# EUROPEAN PATENT APPLICATION

(11) **EP 1 591 448 A2**
(43) Date of publication of application: **02.11.2005**
(21) Application number: 05005147.3
(22) Date of filing: 16.04.2001
(51) Int. Cl.: C07H 21/04, C07K 14/47

(54) **Novel nucleic acids and polypeptides**

(30) Priority: 18.04.2000 US 552929; 22.09.2000 US 668317; 24.10.2000 US 695783; 01.12.2000 US 728628; 26.01.2001 US 770160; 13.02.2001 US 783066; 22.03.2001 US 816828
(62) Divisional of application: 01926385.4
(71) Applicant: Nuvelo, Inc., Sunnyvale, CA 94085 (US)
(72) Inventor: Tang, Y. Tom, San Jose, CA 95118 (US); Asundi, Vinod, Foster City, CA 94404 (US); Zhou, Ping, Cupertino, CA 95014 (US); Xue, Aidong J., Sunnyvale, CA 94087 (US); Ren, Feiyan, Cupertino, CA 95014 (US); Zhang, Jie, Campbell, CA 95008 (US); Wang, Jian-Rui, San José, CA 95128 (US); Yang, Yonghong, San Jose, CA 95118 (US); Zhao, Qing A., San Jose, CA 95118 (US); Goodrich, Ryle W., US/Stateline, NV 89449 (US); Liu, Chenghua, San Jose, CA 95117 (US); Drmanac, Radoje T., Los Altos Hills, CA 94022 (US); Ma, Yunqing, Santa Clara, CA 95051 (US); Wang, Zhiwei, Sunnyvale, CA 94085 (US); Wehrma, Tom, Stanford, CA 94305 (US)
(74) Representative: Forstmeyer, Dietmar

(57) **Abstract**

The present invention provides novel nucleic acids, novel polypeptide sequences encoded by these nucleic acids and uses thereof.

### Annex to the application documents - subsequently filed sequences listing

SEQUENCE LISTING
<110> Hyseq, Inc

<120> NOVEL MACROPHAGE NUCLEIC ACIDS AND POLYPEPTIDES
<130> 21272-065

<140> NOT YET ASSIGNED
<141> 2001-04-16
<150> 09/522,929
<151> 2000-04-18

<150> 09/770,160
<151> 2001-01-26

<150> 09/668,317
<151> 2000-09-22

<150> 09/695,783
<151> 2000-10-24

<150> 09/728,628
<151> 2000-12-01

<150> 09/783,066
<151> 2001-02-13

<150> 09/816,828
<151> 2001-03-22

<160> 584
<170> Custom

<210> 1
<211> 2584
<212> DNA
<213> Homo sapiens

<220>
<221> CDS
<222> (365)..(1981)

<400> 1
aaagccctcc tcccgggcca aggtagagga agttgggctc ccgcctggct gggaggcggg 60

agggatcccg ctcctgttgt tttccgccgg caggagtagg ctggcgggcg cagggggcgg 120

ggtgcgccct ccctccccgg ccagggcgct cgggagcggg gacccgagcc tgcagccgag 180

ctccgctgcc ggccctggac actcggctca gccaagcatc cttcctgggg gccgaggaag 240

tggggccact ctgccgttcc gaggacctgg gaggagccct cggtaccccg ggccccgggg 300

ccctggggca cacacgtcca gcccagcccg agcctgcgtt tcctgagccg ggatctgggg 360

cgag atg gcc gca ggc ggc agt gcg ccc gag ccc cgc gtc ctc gtc tgc 409
Met Ala Ala Gly Gly Ser Ala Pro Glu Pro Arg Val Leu Val Cys
1               5                   10                  15

ctc ggg gcg ctc ctg gcc ggc tgg gtc acc gta gga ttg gag gct gtt 457
Leu Gly Ala Leu Leu Ala Gly Trp Val Thr Val Gly Leu Glu Ala Val

20                  25                  30

gtc att tgg aga agt tat gag aat gtt act ctg cac tgt ggc aac atc      505
Val Ile Trp Arg Ser Tyr Glu Asn Val Thr Leu His Cys Gly Asn Ile
                35                  40                  45

tcg gga ctg agg ggc cag gtg acc tgg tac cgg aac aac tcg gag cct      553
Ser Gly Leu Arg Gly Gln Val Thr Trp Tyr Arg Asn Asn Ser Glu Pro
            50                  55                  60

gtc ttc ctt ctc tcg tcc aac tct agc ctc cgg cca gct gag cct cgc      601
Val Phe Leu Leu Ser Ser Asn Ser Ser Leu Arg Pro Ala Glu Pro Arg
        65                  70                  75

ttc tct cta gtg gat gcc acc tcc ctg cac att gaa tcg ctg agc ctg      649
Phe Ser Leu Val Asp Ala Thr Ser Leu His Ile Glu Ser Leu Ser Leu
 80                  85                  90                  95

gga gat gag gga atc tac acc tgc cag gag atc ctg aat gtg act cag      697
Gly Asp Glu Gly Ile Tyr Thr Cys Gln Glu Ile Leu Asn Val Thr Gln
                100                 105                 110

tgg ttc caa gtg tgg ctg cag gtg gcc agc ggc ccc tat cag att gag      745
Trp Phe Gln Val Trp Leu Gln Val Ala Ser Gly Pro Tyr Gln Ile Glu
            115                 120                 125

gtc cac atc gtg gcc acc ggc aca ctc ccc aac ggc acc ctc tac gca      793
Val His Ile Val Ala Thr Gly Thr Leu Pro Asn Gly Thr Leu Tyr Ala
        130                 135                 140

gcc agg ggc tcc cag gtg gac ttc agc tgc aac agc agc tcc agg cca      841
Ala Arg Gly Ser Gln Val Asp Phe Ser Cys Asn Ser Ser Ser Arg Pro
    145                 150                 155

cca ccc gtg gtt gaa tgg tgg ttc cag gcc ctg aat tcc agc agc gag      889
Pro Pro Val Val Glu Trp Trp Phe Gln Ala Leu Asn Ser Ser Ser Glu
160                 165                 170                 175

tcc ttt ggc cac aac ctg aca gtc aac ttt ttc tca ctg tta ctg ata      937
Ser Phe Gly His Asn Leu Thr Val Asn Phe Phe Ser Leu Leu Leu Ile
                180                 185                 190

tcg cca aac ctc caa ggg aac tac acc tgt tta gcc ttg aat cag ctc      985
Ser Pro Asn Leu Gln Gly Asn Tyr Thr Cys Leu Ala Leu Asn Gln Leu
            195                 200                 205

agc aag aga cat cga aag gtg acc acc gag ctc ctg gtc tac tat ccc      1033
Ser Lys Arg His Arg Lys Val Thr Thr Glu Leu Leu Val Tyr Tyr Pro
        210                 215                 220

cct cca tca gct ccc cag tgc tgg gca cag atg gca tca gga tcg ttc      1081
Pro Pro Ser Ala Pro Gln Cys Trp Ala Gln Met Ala Ser Gly Ser Phe
    225                 230                 235

atg ttg cag ctt acc tgt cgc tgg gat ggg gga tac cct gac cct gac      1129
Met Leu Gln Leu Thr Cys Arg Trp Asp Gly Gly Tyr Pro Asp Pro Asp
240                 245                 250                 255

ttc ctg tgg ata gaa gag cca gga ggt gta atc gtg ggg aag tca aag      1177
Phe Leu Trp Ile Glu Glu Pro Gly Gly Val Ile Val Gly Lys Ser Lys

                260                 265                 270

ctg ggg gtg gaa atg ctg agc gag tcc cag ctg tcg gat ggc aag aag 1225
Leu Gly Val Glu Met Leu Ser Glu Ser Gln Leu Ser Asp Gly Lys Lys
            275                 280                 285

ttc aag tgt gtt aca agc cac ata gtt ggg cca gag tcg ggc gcc agc 1273
Phe Lys Cys Val Thr Ser His Ile Val Gly Pro Glu Ser Gly Ala Ser
        290                 295                 300

tgc atg gtg cag atc agg ggt ccc tcc ctt ctc tct gag ccc atg aag 1321
Cys Met Val Gln Ile Arg Gly Pro Ser Leu Leu Ser Glu Pro Met Lys
    305                 310                 315

act tgc ttc act ggg ggc aat gtg acg ctt aca tgc cag gtg tct ggg 1369
Thr Cys Phe Thr Gly Gly Asn Val Thr Leu Thr Cys Gln Val Ser Gly
320                 325                 330                 335

gcc tac ccc cct gcc aag atc ctg tgg ctg agg aac ctt acc cag ccc 1417
Ala Tyr Pro Pro Ala Lys Ile Leu Trp Leu Arg Asn Leu Thr Gln Pro
                340                 345                 350

gag gtg atc atc cag cct agc agc cgc cat ctc att acc cag gat ggc 1465
Glu Val Ile Ile Gln Pro Ser Ser Arg His Leu Ile Thr Gln Asp Gly
            355                 360                 365

cag aac tcc acc ctc act atc cac aac tgc tcc cag gac ctg gat gag 1513
Gln Asn Ser Thr Leu Thr Ile His Asn Cys Ser Gln Asp Leu Asp Glu
        370                 375                 380

ggc tac tac atc tgc cga gct gac agc cct gta ggg gtg agg gag atg 1561
Gly Tyr Tyr Ile Cys Arg Ala Asp Ser Pro Val Gly Val Arg Glu Met
    385                 390                 395

gaa atc tgg ctg agt gtg aaa gaa cct tta aat atc ggg ggg att gtg 1609
Glu Ile Trp Leu Ser Val Lys Glu Pro Leu Asn Ile Gly Gly Ile Val
400                 405                 410                 415

gga acc att gtg agc ctc ctt ctg ctg gga ctg gcc att atc tca ggg 1657
Gly Thr Ile Val Ser Leu Leu Leu Leu Gly Leu Ala Ile Ile Ser Gly
                420                 425                 430

ctt ctg ttg cat tat agc cct gtg ttc tgc tgg aaa gga aac act tcc 1705
Leu Leu Leu His Tyr Ser Pro Val Phe Cys Trp Lys Gly Asn Thr Ser
            435                 440                 445

agg gga caa tac atg gat gat gtc atg gtt ttg gtg gat tca gaa gag 1753
Arg Gly Gln Tyr Met Asp Asp Val Met Val Leu Val Asp Ser Glu Glu
        450                 455                 460

gaa gag gag gag gag gag gag gag gag gaa gat gct gca gta ggg gaa 1801
Glu Glu Glu Glu Glu Glu Glu Glu Glu Glu Asp Ala Ala Val Gly Glu
    465                 470                 475

cag gag gga gca cgt gag aga gag gag ttg cca aaa gaa ata cct aag 1849
Gln Glu Gly Ala Arg Glu Arg Glu Glu Leu Pro Lys Glu Ile Pro Lys
480                 485                 490                 495

cag gac cac att cac aga gtg acc gcc ttg gtg aat ggg aac ata gaa 1897
Gln Asp His Ile His Arg Val Thr Ala Leu Val Asn Gly Asn Ile Glu

                    500                 505                 510

cag atg gga aat gga ttc cag gat cta caa gat gac agc agt gag gag      1945
Gln Met Gly Asn Gly Phe Gln Asp Leu Gln Asp Asp Ser Ser Glu Glu
            515                 520                 525

caa agt gac att gtt caa gaa gaa gac agg cca gtc tgaa gaagaggatg      1995
Gln Ser Asp Ile Val Gln Glu Glu Asp Arg Pro Val
        530                 535

gtccatggtt gtcttgctct gaaagcttgg agagctacat tgaagacgag ctcttcattc   2055

agctttgact ccacctgcac ccctggcggg ggcttgcact aacaatgttt gggtctcagc   2115

aaaaaacaaa accaagcaca cacatctttc cttccatgta ttgaaaaaca ttggtttgat   2175

ttgctctaag ttttcccaat gatgtttaaa agctttgaga aggaaagctg ctttggtgtc   2235

tgaggtgcca cttctgctgt gaatcctggc tttatccagg ttgatctact gtgatagatg   2295

ctgatttaga gggaacagag gtcagggaag cacaagatgg aaaattgcaa taacccatgc   2355

actgagactt agaaaatcat ccttactagg taaaatgtat tatgatgcaa taagtgccaa   2415

ctgatatttc tcacgttggg actggccagg aactgctgca aagaaaaata agcagctcct   2475

tctccattat ttacatttta agatgtggtg gggggaggtt gggagaaatt agttctgagg   2535

ttatcatatg ccttttttaa aagataatgg aataaagcta tttttaagt                2584

<210> 2
<211> 1288
<212> DNA
<213> Homo sapiens

<220>
<221> CDS
<222> (79)..(1242)

<400> 2
ctcgggtggt tacgcaccct ggaagggaat aaacattagg accatagagg acgctctagg     60
actaatgctc atcagaaa atg act agg agt gct ggc atc cct atg ttc ttt      111
ttt cag atg gga aac att ccc ccc aag gca aaa acg ccc cta aga tgt      159
att ctg gag aat tgg gac caa ttt gac cct cag atg cta aga aag aaa      207
cta ctt ata ttc ttc tgc agt act gcc tgg cca caa tat cct ctt caa      255
ggg gga gaa acc tgg cct cct aag aga agt ata aat tat aac agc atc 303
tta cag cta gac ctc ttt tgt aga aag gag ggc aaa tgg agt gaa gtg 351
cca tat gtg caa act ttc ttt tca tta aga gac aac tca caa tta tgt 399
aaa aag tgt ggt tta tgc cct aca gga agc cct cag agt cca cct ccc 447
tac ccc agc gtc ccc ccc ccg act cct tcc tca act aat aag gac ccc 495
cct tta acc caa acg gtc caa aag gag ata gac aaa ggg gta aac aat 543
gaa cca aag agt gcc aat att ccc cga tta tgc ccc ctc caa gca gtg 591
aga gga gga gaa ttc ggc cca gcc aga gtg cct gta cct ttt tct ctc 639
tca gac tta aag caa att aaa ata gac cta ggt aaa ttc tca gat aac 687
cct gac ggc tat att gat gtt tta caa ggg tta gga caa tcc ttt gat 735
ctg aca tgg aga gat ata atg tta cta cta aat cag aca cta acc cca 783
aat gag aga agt gcc acc ata act gca gcc cga gag ctt ggc gat ctc 831
tgg tat ctc agt cag gtc aat gat agg atg aca aca gag gaa aga gaa 879
cga ttc cat aca ggc cag cag gca gtt ccc agt gca gac cct cat tgg 927
gac aca gaa tca gaa cat gga gat tgg tgc tgc aga cat tta cta act 975
tgc gtg ctg gaa gga cta agg aaa acc agg aag aag gct gtg aat ttt 1023
tca gtg atg tcc act gta aca cag gga aag gaa gaa aat cct act gcc 1071
ttt ctg gag aga cta agg gag gca ctg agg aag cat acc tcc ctg tca 1119
cct gac tct att gaa ggc caa cta atc tta aag att aag ttt atc act 1167
cgg tca gct gca gac att aga aaa caa act tca aaa gtc cac ctt agg 1215
cct gga gca aaa ctt aag aca ccc tat tgaac ttggcaacct cggtttttta 1267
taatagagat caggaggagc a 1288

<210> 3
<211> 1676
<212> DNA
<213> Homo sapiens

<220>
<221> CDS
<222> (1331)..(1549)

<220>
<221> misc_feature
<222> (1)...(1676)
<223> n = a,t,c or g

<400> 3
tctttttttt tttttttttt ttttgggtgt ggtgatgtat ttattcataa tatattttca 60
gaacacatta ataatggaga ataacactta ttcatatact gaatataact tttcctggag 120
cactctagag cttgtttgga gttggagaat actgccaggc ttttcctaat ctctttggtc 180
tttggaagtg ggcagggttt ctcaaaccaa gtgtcttcca tgggccattg gcaaaggctt 240
cccttcatca gcttggaggg gcagaaagac catggcttca gcacttccat tttggaaaga 300
agtaacaaaa aagtgaatta atgagcaatc ggaaagactc aaagcatttt gtactccaca 360
gttcatttct tcacacaaac gtccattact gcagcgggca tgaaaaccgg cagggtgtta 420
ggctcatggc ctgaagagaa gtcacatcac cagccgatgt tttcatgcaa aaggcaatcg 480
tgatgattca gaacctggtt ctgaatttct ccaggtgtgc tcgtgagctg aaggtcatgc 540
ccattctgtg catcctgtgt ctttctactg gtaaacttgg atgtcatttt agagtatctt 600
ggcaaaatgg tgagaggatc tcactttccc aatatcactc tgtaatgaag ctgcttgggg 660
gatcctccat ttgattgtgg agagaccaag gcaccgtggg ctgaagcaac gaatattaac 720
aacactaaca acaataagga taaagtctaa gtctcttcca aaaaaggcat atccaaaaat 780
aagggtaaag agctggtctt atttcatcca agtgattctg tgcactggaa attgtccttc 840
cgttcctccc tttcattaaa agtgtttata tttctgagtc ggatgatcat ttaaatacat 900
acaatattag caccaaggca gcatattttt ttcccagaga tcacatgaaa ctgaatactt 960
atttaacata gttaatagtt ggtacaatct gtgatatcac taacatactt attggctgat 1020
acttgcctag aaatgccaaa aatatctttt atacaactct tatagcacct tgatgatggc 1080
tagattgttt taaaaaaatt cattttaaaa aagtaacaat aaaacattta cagatgaaaa 1140
gtaaacaata gtgtaccgtg ataatgaagg atcactttgt catattacta catgcaaagg 1200
cagtcataat agaatcattt tacgtacaaa aatattacat cacaataaat aatttcaaac 1260
ataaatatta aactttacat cattaggata gtctacatat ctacatacac acatatatgt 1320
gtatatatgt atg tgt aga tat gta tat atg tat atg tac aca cat tta 1369
cac aca caa gca tac aag tca cac aca aac aca cac aca cat aca cat 1417
gca ccc aaa tta agg tgg agg tat tgc att act atc ctt cat cca tcc 1465
ata atg gct tcc tct tca aag cac ctt agc tat aaa act cgc acc tcc 1513
agg cca gta cca gag gat gga gtg cat ttt cca cag taac caccgcattt 1563
ccctacgact cgggtaccat ccggcgactt cttgatgtca gagacagcat aattcccttg 1623
tgatatccgt ctggaattac gcgnctctcg atagtgttcg atgnnggcac tct 1676

<210> 4
<211> 1289
<212> DNA
<213> Homo sapiens

<220>
<221> CDS
<222> (40)..(336)

<400> 4
aaaagagaca aattacccag aaacccctcc cttccccac atg gag gcc ttg gca 54
Met Glu Ala Leu Ala
1               5

aat gtt aat ttt cct aga aaa tcc ttc aga cct gaa gac gca gga aaa 102
Asn Val Asn Phe Pro Arg Lys Ser Phe Arg Pro Glu Asp Ala Gly Lys
                10                  15                  20

gaa tct ggc tct cag ggt ggc ttc tgc gtc ccc gcc gcc agg ccc cag 150
Glu Ser Gly Ser Gln Gly Gly Phe Cys Val Pro Ala Ala Arg Pro Gln
            25                  30                  35

act atg gtc aca ggg ccg tcc tgt tcc tcc ccg gga ctc cag aat ttc 198
Thr Met Val Thr Gly Pro Ser Cys Ser Ser Pro Gly Leu Gln Asn Phe
        40                  45                  50

tct cct caa agg aaa gaa aac agg gca tgc gct tgt tgg caa aac gca 246
Ser Pro Gln Arg Lys Glu Asn Arg Ala Cys Ala Cys Trp Gln Asn Ala
    55                  60                  65

ggg ccg gct ccc aaa aac ccc atg tgt gta cga tta aaa gtt ggc cgt 294
Gly Pro Ala Pro Lys Asn Pro Met Cys Val Arg Leu Lys Val Gly Arg
70                  75                  80                  85

ccc cag gcc tcc cag cgc aaa ctt aaa gag aca ggg ctt tgc tgaaaac 343
Pro Gln Ala Ser Gln Arg Lys Leu Lys Glu Thr Gly Leu Cys
                90                  95

caaacatggg ccagctgggc tttttaacaa cctagagact ttccggagct gcctggaaca 403

gagcctgcgg gaaacggggc ttgccagaga cactcacagt ttccttcatg gcctgttttg 463

gtcccctaag aatctccaca tcattgtctt tcttgtgcct tttccttggt gagcaacaga 523

aagggaaggg ttccaagcct ctaaaaatgt gctttgtgat caggagtgcg ctccaaacca 583

aatacgcgcg ctgccctttc gaggccagtg agctcagcct ccaaggcttt aaagccacat 643

ttcagcaaga gaaagcgctg agagctcgca ggttcattaa agaaggcaaa gcactggttt 703

ctctccttag aaaagtaggt ttcttggctt gatgtagact ggcttgcttt gatttttagt 763

gaagggaatg tacgtaaaac aaaatagggc ttggctggtc aaaggagaca agcaggatgg 823

atggatggat ggatgaatag atagatggtg tttgcatgta aattgcagag aaaacaaaac 883

caaagctgat tggaaacaat taattgtggg tgtctgaggg ggaaggtcgc agctttgggc 943

agctttgaga agcggtacaa gagctctgtg cctgtgtgtc cagccctgga gccagccagt 1003

gcatttattt taagctctta gaagcaactc cttggcccag gaatgcgtga cccctgagat 1063

gggtccacgc atctctctac acgtccttct ctccgtggga tactggactc gtgcctctgc 1123

gcccattctc ttctcacgca tatccatgag ctttaatttc actttctgat cacggtacgt 1183

ccataaagcc agtattacac ttaaatgaag tattcttttt tgtaatcgtt tttttagaa 1243

ggtaaacaaa tttaataaag ctaccaataa tgttaaaaaa aaaaaa 1289

<210> 5
<211> 2307
<212> DNA
<213> Homo sapiens

<220>
<221> CDS
<222> (1612)..(2181)

<400> 5
gcaatttcat aatatggtaa tttttaatat taaaagtaaa ttgtaaatgt gtctttataa 60

acaatatgca taaaatagat catattctaa acccttttct ttttaaaacg ctttttcaca 120

gtaaatttct tatggtcaga ttttccctca gaatagtgca actggaagaa gggaaatgtt 180

ggcagtggct caggactctc tgctagtaca agtccttgta gatctcctgc aggagcgggt 240

gaagactcat gtctgtctcc gtcttcttga tcacctgcag tagctgcacg tgttccgtga 300

caatctgtct gaggtctgtc attttctgga gcagcttggc aaacagctgt gaggactcag 360

ggtggttcag cttcagctgg agctccaggg cttgtagcag gttgtcttga atgtcttcaa 420

tgggcttcac attcagcaaa cctgggcggt ctccactgag aataatgaca gcaataaata 480

ttgccaagtc gctgtcatct aattccagtg cattgaactt cacagcaaac tcaaacttgg 540

gctccataaa gtcaccaaaa ggctttcgca ggctctttag aaactccctt gtcatgaagc 600

cttggccctc ggatatgaga accccatctt tattcatcaa ggaggccagc attgtgtaaa 660

tgatctcgtg gactccatat ttgaggagag ttacttggtc gttcaagtca agatttacaa 720

aaccaggaat gcttttggca tactctgtga tctcctgcac agcctccacg gagcgaaact 780

ggcagccctg aaagatgcgg atggccacct ctttgctctg ctcctgcagg ggggtgatgt 840

gtttgaactt gattttatct tctcccatca ttaaggaatt catgtcatag ataacgaatg 900

gtgatttgtc tgttgtcttt cctgtcaaga tcgccctcgc ctttgctttg gtcagcggga 960

aggactttat gtatgagtca tacaaatgtt ttgccagggc ctgacggagg tcagcggact 1020

ctggattcag ctggtcgata tcactggaga tctccgccaa cagcttctcc ttctcggcct 1080

gtgcgatccg cccaaacctg atggcattat gagacatccc cactgcaagg catttctgaa 1140

accgacagta ctgacattta tttctacttt ttttgtggat ccgacagtta agatcacatc 1200

tgtcatagat aagcttcaat ctgattgttc tccggaagaa acccttgcat ccttcacaag 1260

catgaactcc atagtgaaat ccagaagctt tatctccaca gacacgacat tcaattgcca 1320

tgagggagtt ggaaggctct tcatgaggct tattgtagag ctgagtcttc tcagaataat 1380

aaggtggaga tgcaggctcc actttgattg cactttggta ctcttgaagt ttcaggtcat 1440

acttgtaatc tgcaaccact ggatctgttc ttgtgaatgg aatgtcttcg taatgtggag 1500

tagaaatgct ggagaagtca acagtagtga agggcttgat atcaaaggag tgggagtggt 1560

cttccattac ggagagatcc acggagctga tcccaaagtt ggtgggccag a atg cga 1617
Met Arg
1

tct ctg tgt caa cca tgg tca ttt ctg cgg cca cgg cgg cgt ggc gcc 1665
Ser Leu Cys Gln Pro Trp Ser Phe Leu Arg Pro Arg Arg Arg Gly Ala
        5                   10                  15

cct ctc cgg tgt cct cga ggc cga ccc aag ccc ccc agg cgg cgg ctg 1713
Pro Leu Arg Cys Pro Arg Gly Arg Pro Lys Pro Pro Arg Arg Arg Leu
    20                  25                  30

cgg ctc ggg ctc ggg aat tcc cag act cag aga gaa ccc acc atg gtg 1761
Arg Leu Gly Leu Gly Asn Ser Gln Thr Gln Arg Glu Pro Thr Met Val
35                  40                  45                  50

ctg tct cct gcc gac aag acc aac gtc aag gcc gcc tgg ggt aag gtc 1809
Leu Ser Pro Ala Asp Lys Thr Asn Val Lys Ala Ala Trp Gly Lys Val
                55                  60                  65

ggc gcg cac gct ggc gag tat ggt gcg gag gcc ctg gag agg atg ttc 1857
Gly Ala His Ala Gly Glu Tyr Gly Ala Glu Ala Leu Glu Arg Met Phe
            70                  75                  80

ctg tcc ttc ccc acc acc aag acc tac ttc ccg cac ttc gac ctg agc 1905
Leu Ser Phe Pro Thr Thr Lys Thr Tyr Phe Pro His Phe Asp Leu Ser
        85                  90                  95

cac ggc tct gcc cag gtt aag ggc cac ggc aag aag gtg gcc gac gcg 1953
His Gly Ser Ala Gln Val Lys Gly His Gly Lys Lys Val Ala Asp Ala
    100                 105                 110

ctg acc aac gcc gtg gcg cac gtg gac gac atg ccc aac gcg ctg tcc 2001
Leu Thr Asn Ala Val Ala His Val Asp Asp Met Pro Asn Ala Leu Ser
115                 120                 125                 130

gcc ctg agc gac ctg cac gcg cac aag ctt cgg gtg gac ccg gtc aac 2049
Ala Leu Ser Asp Leu His Ala His Lys Leu Arg Val Asp Pro Val Asn
                135                 140                 145

ttc aag ctc cta agc cac tgc ctg ctg gtg acc ctg gcc gcc cac ctc 2097
Phe Lys Leu Leu Ser His Cys Leu Leu Val Thr Leu Ala Ala His Leu
            150                 155                 160

ccc gcc gag ttc acc cct gcg gtg cac gcc tcc ctg gac aag ttc ctg 2145
Pro Ala Glu Phe Thr Pro Ala Val His Ala Ser Leu Asp Lys Phe Leu
        165                 170                 175

gct tct gtg agc acc gtg ctg acc tcc aaa tac cgt taag ctggagcctc 2195

Ala Ser Val Ser Thr Val Leu Thr Ser Lys Tyr Arg
    180                 185                 190

ggtggccatg cttcttgccc cttgggcctc cccccagccc ctcctcccct tcctgcaccc 2255

gtacccccgt ggtctttgaa taaagtctga gtgggcggca aaaaaaaaaa aa 2307

<210> 6
<211> 912
<212> DNA
<213> Homo sapiens

<220>
<221> CDS
<222> (3)..(866)

<400> 6
gg atg tcc agg ctg cgg gcg ctg ctg ggc ctc ggg ctg ctg gtt gcg 47
Met Ser Arg Leu Arg Ala Leu Leu Gly Leu Gly Leu Leu Val Ala
1               5                   10                  15

ggc tcg cgc ctg ccg cgg atc aaa agc cag acc atc gcc tgt cgc tcg 95
Gly Ser Arg Leu Pro Arg Ile Lys Ser Gln Thr Ile Ala Cys Arg Ser
                20                  25                  30

gga ccc acc tgg tgg gga ccg cag cgg ctg aac tcg ggt ggc cgc tgg 143
Gly Pro Thr Trp Trp Gly Pro Gln Arg Leu Asn Ser Gly Gly Arg Trp
            35                  40                  45

gac tca gag gtc atg gcg agc acg gtg gtg aag tac ctg agc cag gag 191
Asp Ser Glu Val Met Ala Ser Thr Val Val Lys Tyr Leu Ser Gln Glu
        50                  55                  60

gag gcc cag gcc gtg gac cag gag cta ttt aac gaa tac cag ttc agc 239
Glu Ala Gln Ala Val Asp Gln Glu Leu Phe Asn Glu Tyr Gln Phe Ser
    65                  70                  75

gtg gac caa ctt atg gaa ctg gcc ggg ctg agc tgt gct aca gcc atc 287
Val Asp Gln Leu Met Glu Leu Ala Gly Leu Ser Cys Ala Thr Ala Ile
80                  85                  90                  95

gcc aag gca tat ccc ccc acg tcc atg tcc agg agc ccc cct act gtc 335
Ala Lys Ala Tyr Pro Pro Thr Ser Met Ser Arg Ser Pro Pro Thr Val
                100                 105                 110

ctg gtc atc tgt ggc ccg ggg aat aat gga gga gat ggt ctg gtc tgt 383
Leu Val Ile Cys Gly Pro Gly Asn Asn Gly Gly Asp Gly Leu Val Cys
            115                 120                 125

gct cga cac ctc aaa ctc ttt ggc tac gag cca acc atc tat tac ccc 431
Ala Arg His Leu Lys Leu Phe Gly Tyr Glu Pro Thr Ile Tyr Tyr Pro
        130                 135                 140

aaa agg cct aac aag ccc ctc ttc act gca ttg gtg acc cag tgt cag 479
Lys Arg Pro Asn Lys Pro Leu Phe Thr Ala Leu Val Thr Gln Cys Gln
    145                 150                 155

aaa atg gac atc cct ttc ctt ggg gaa atg ccc gca gag ccc atg acg 527
Lys Met Asp Ile Pro Phe Leu Gly Glu Met Pro Ala Glu Pro Met Thr
160                 165                 170                 175

att gat gaa ctg tat gag ctg gtg gtg gat gcc atc ttt ggc ttc agc 575
Ile Asp Glu Leu Tyr Glu Leu Val Val Asp Ala Ile Phe Gly Phe Ser
                180                 185                 190

ttc aag ggc gat gtt cgg gaa ccg ttc cac agc atc ctg agt gtc ctg 623
Phe Lys Gly Asp Val Arg Glu Pro Phe His Ser Ile Leu Ser Val Leu
            195                 200                 205

aag gga ctc act gtg ccc att gcc agc atc gac att ccc tca gga tgg 671
Lys Gly Leu Thr Val Pro Ile Ala Ser Ile Asp Ile Pro Ser Gly Trp
        210                 215                 220

gac gtg gag aag gga aat gct gga ggg atc cag cca gac ttg ctc atc 719
Asp Val Glu Lys Gly Asn Ala Gly Gly Ile Gln Pro Asp Leu Leu Ile
    225                 230                 235

tcc ctc aca gcc ccc aaa aaa tct gca acc cag ttt acc ggt cgc tac 767
Ser Leu Thr Ala Pro Lys Lys Ser Ala Thr Gln Phe Thr Gly Arg Tyr
240                 245                 250                 255

cat tac ctg ggg ggt cgt ttt gtg cca cct gct ctg gaa aag aag tac 815
His Tyr Leu Gly Gly Arg Phe Val Pro Pro Ala Leu Glu Lys Lys Tyr
                260                 265                 270

cag ctg aac ctg cca ccc tac cct gac act gag tgt gtc tat cgt ctg 863
Gln Leu Asn Leu Pro Pro Tyr Pro Asp Thr Glu Cys Val Tyr Arg Leu
            275                 280                 285

cag tgagggaagg tgggtgggta ttctccccaa taaagactta gagccc 912
Gln

<210> 7
<211> 1949
<212> DNA
<213> Homo sapiens

<220>
<221> CDS
<222> (1441)..(1776)

<400> 7
tttttttttt tttttaaaaa ttactctttt acttttatta caataaataa ttatcaataa 60

tagaattaaa caattttcaa ttaaaaccta ctgcattact ttggggtttc acagcagcag 120

aaacaaacat aaatccagtt gaaagggcaa ggcttccaga atccagtgac aagaaacagt 180

ctggtcttga ttattcgggc tagcaatggg aaacactgat acagataatg caaaaacaat 240

gaaatgcatc ggcatactct ctttgtacat cacattatct gacactttaa aatattccag 300

ctaagtaatt taagcaagcc atgaagctct gtttctgcaa cagttagagt tctcccccag 360

caagcccgca gcacaactgc tcccagagcc accctctggc cagaaacggg ccccatcatc 420

atgcagacct agaagcctcc accctggtgg aagcagccat ctttctcaaa gctctttctg 480

gctcacacaa cccatggtta aaggaaaatg ggaaagtgac atatttaaag ggcctttctc 540

gaggactgag atgaactgga agacagacag actgtggctc cagatcagga agaggcttca 600

gagtaagggc cacatgcaga tgaactccac atgaagaaac tatctgataa aggctgacca 660

ttttgctgat aatcaagcag actgcatgta tatatgaata tatcatacat acatatgtag 720

tgaatatatg tgtacatatt aaataggcat acacacacat cacacacaac cagataaaat 780

aaatggtaca tacattgcta ctatgaacac tgcacgtccc agacagacca atgactaata 840

tgttcaatta aactacttga attaaactac tcttcctctc cccaaaatat tctgcttgca 900

gcaaacatat tccagttgct caagcggtgt tgtttctagg ttcctgagaa ggaatggaat 960

cgtcctcttg tttcagaaca ctacagaaag ccaactgcaa caggaccaag ctaaacaatt 1020

catatttgat ttggccatga ttcctccacc tattcttagt cacagggcat gaaaccttac 1080

tcagaaagat atcaatttct ttttctacaa ctggctacaa atttactgta ttatgttaga 1140

tggaggggac tgggggaagg cgggcagcag ggagtttaca tgttggcaat cattcattgc 1200

ctttcccttt acccagagaa aatgctactt taaaaaaaaa aacacccaga aatttagagt 1260

tcaataatga atttgctttt cttggcacat ttttgaaatg atcatatcat tacatgtagc 1320

taggtcttta tctcctgaat gctcctctag ggaggcaagt tatctaaaca acaaaataac 1380

ctacaaaaaa agttaatatt gtgagcagtc tcaatttgta gttattaaac tcaaagtcta 1440

atg ctg aaa ggc aac agt aat tta aac aga ttt gtt tat ttt tat tat 1488
Met Leu Lys Gly Asn Ser Asn Leu Asn Arg Phe Val Tyr Phe Tyr Tyr
1               5                   10                  15

tat ttt ttg aga tgg agt ctt gct ctg tca ctc tgg ctg gag tgc agt 1536
Tyr Phe Leu Arg Trp Ser Leu Ala Leu Ser Leu Trp Leu Glu Cys Ser
            20                  25                  30

ggc aca atc tcc gct cac tgc aat ctc cat ctc cca ggt tca agt gat 1584
Gly Thr Ile Ser Ala His Cys Asn Leu His Leu Pro Gly Ser Ser Asp
        35                  40                  45

tct cct gcc tca gcc tcc cca gta act ggg att aca ggc aca tgc cac 1632
Ser Pro Ala Ser Ala Ser Pro Val Thr Gly Ile Thr Gly Thr Cys His
    50                  55                  60

cat gcc cag cta att ttt ttg tat ttt tat tac aga cgg ggt ttc gcc 1680
His Ala Gln Leu Ile Phe Leu Tyr Phe Tyr Tyr Arg Arg Gly Phe Ala
65                  70                  75                  80

atg ttg ctc agg ttg gtg tcg aac tcc tgg gct caa gca atc cac ctg 1728
Met Leu Leu Arg Leu Val Ser Asn Ser Trp Ala Gln Ala Ile His Leu

                85                  90                  95

cct tgg ctt ccc aaa gtg ctg gga tta cag gca cct gcc cca gat tta 1776
Pro Trp Leu Pro Lys Val Leu Gly Leu Gln Ala Pro Ala Pro Asp Leu
            100                 105                 110

taatgctatt cacagatctt gcagaattat aacaggctcc ttctacaact tggttagtga 1836

catcaagtac taactatata tgttccaggt caagaactta ctcttgagat attcaaaaag 1896

tgcttgtcaa gaatatcagg aatctagaat ttatggcaaa ttgtatatat ttt 1949

<210> 8
<211> 1225
<212> DNA
<213> Homo sapiens

<220>
<221> CDS
<222> (90)..(1172)

<400> 8
aaggatcctt aattaaatta atcccccccc cccggtgagc cgcaaccttt ccaagggagt 60

ggttgtgtga tcgccatctt agggaaaag atg ttc tcg tcc gtg gcg cac ctg 113
Met Phe Ser Ser Val Ala His Leu
1               5

gcg cgg gcg aac ccc ttc aac acg cca cat ctg cag ctg gtg cac gat 161
Ala Arg Ala Asn Pro Phe Asn Thr Pro His Leu Gln Leu Val His Asp
    10                  15                  20

ggt ctc ggg gac ctc cgc agc agc tcc cca ggg ccc acg ggc cag ccc 209
Gly Leu Gly Asp Leu Arg Ser Ser Ser Pro Gly Pro Thr Gly Gln Pro
25                  30                  35                  40

cgc cgc cct cgc aac ctg gca gcc gcc gcc gtg gaa gag tac agt tgt 257
Arg Arg Pro Arg Asn Leu Ala Ala Ala Ala Val Glu Glu Tyr Ser Cys
                45                  50                  55

gaa ttt ggc tcc gcg aag tat tat gca ctg tgt ggc ttt ggt ggg gtc 305
Glu Phe Gly Ser Ala Lys Tyr Tyr Ala Leu Cys Gly Phe Gly Gly Val
            60                  65                  70

tta agt tgt ggt ctg aca cac act gct gtg gtt ccc ctg gat tta gtg 353
Leu Ser Cys Gly Leu Thr His Thr Ala Val Val Pro Leu Asp Leu Val
        75                  80                  85

aaa tgc cgt atg cag gtg gac ccc caa aag tac aag ggc ata ttt aac 401
Lys Cys Arg Met Gln Val Asp Pro Gln Lys Tyr Lys Gly Ile Phe Asn
    90                  95                  100

gga ttc tca gtt aca ctt aaa gag gat ggt gtt cgt ggt ttg gct aaa 449
Gly Phe Ser Val Thr Leu Lys Glu Asp Gly Val Arg Gly Leu Ala Lys
105                 110                 115                 120

gga tgg gct ccg act ttc ctt ggc tac tcc atg cag gga ctc tgc aag 497

Gly Trp Ala Pro Thr Phe Leu Gly Tyr Ser Met Gln Gly Leu Cys Lys
                125                 130                 135

ttt ggc ttt tat gaa gtc ttt aaa gtc ttg tat agc aat atg ctt gga 545
Phe Gly Phe Tyr Glu Val Phe Lys Val Leu Tyr Ser Asn Met Leu Gly
            140                 145                 150

gag gag aat act tat ctc tgg cgc aca tca cta tat ttg gct gcc tct 593
Glu Glu Asn Thr Tyr Leu Trp Arg Thr Ser Leu Tyr Leu Ala Ala Ser
        155                 160                 165

gcc agt gct gaa ttc ttt gct gac att gcc ctg gct cct atg gaa gct 641
Ala Ser Ala Glu Phe Phe Ala Asp Ile Ala Leu Ala Pro Met Glu Ala
    170                 175                 180

gct aag gtt cga att caa acc cag cca ggt tat gcc aac act ttg agg 689
Ala Lys Val Arg Ile Gln Thr Gln Pro Gly Tyr Ala Asn Thr Leu Arg
185                 190                 195                 200

gat gca gct ccc aaa atg tat aag gaa gaa ggc cta aaa gca ttc tac 737
Asp Ala Ala Pro Lys Met Tyr Lys Glu Glu Gly Leu Lys Ala Phe Tyr
                205                 210                 215

aag ggg gtt gct cct ctc tgg atg aga cag ata cca tac acc atg atg 785
Lys Gly Val Ala Pro Leu Trp Met Arg Gln Ile Pro Tyr Thr Met Met
            220                 225                 230

aag ttc gcc tgc ttt gaa cgt act gtt gaa gca ctg tac aag ttt gtg 833
Lys Phe Ala Cys Phe Glu Arg Thr Val Glu Ala Leu Tyr Lys Phe Val
        235                 240                 245

gtt cct aag ccc cgc agt gaa tgt tca aag cca gag cag ctg gtt gta 881
Val Pro Lys Pro Arg Ser Glu Cys Ser Lys Pro Glu Gln Leu Val Val
    250                 255                 260

aca ttt gta gca ggt tac ata gct gga gtc ttt tgt gca att gtt tct 929
Thr Phe Val Ala Gly Tyr Ile Ala Gly Val Phe Cys Ala Ile Val Ser
265                 270                 275                 280

cac cct gct gat tct gtg gta tct gtg ttg aat aaa gaa aaa ggt agc 977
His Pro Ala Asp Ser Val Val Ser Val Leu Asn Lys Glu Lys Gly Ser
                285                 290                 295

agt gct tct ctg gtc ctc aag aga ctt gga ttt aaa ggt gta tgg aag 1025
Ser Ala Ser Leu Val Leu Lys Arg Leu Gly Phe Lys Gly Val Trp Lys
            300                 305                 310

gga ctg ttt gcc cgt atc atc atg att ggt acc ctg act gca cta cag 1073
Gly Leu Phe Ala Arg Ile Ile Met Ile Gly Thr Leu Thr Ala Leu Gln
        315                 320                 325

tgg ttt atc tat gac tcc gtg aag gtc tac ttc aga ctt cct cgc cct 1121
Trp Phe Ile Tyr Asp Ser Val Lys Val Tyr Phe Arg Leu Pro Arg Pro
    330                 335                 340

cct cca ccc gag atg cca gag tct ctg aag aag aag ctt ggg tta act 1169
Pro Pro Pro Glu Met Pro Glu Ser Leu Lys Lys Lys Leu Gly Leu Thr
345                 350                 355                 360

cag tagttagatc aaagcaaatg tggactgaat ctgcttgttg atcagtgttg aag 1225

Gln

<210> 9
<211> 894
<212> DNA
<213> Homo sapiens

<220>
<221> CDS
<222> (364)..(600)

<400> 9
cagattcttt gtcctactga ccaaattccc ccaagttcag aatttgcaga ttttatagca 60

ggtggtattc ttttccaaag atatcttgca ttattcatgt cattatggag caaatataaa 120

gttagaagct gaccataccc tgggggtgta gcaattcctc caggggcctc gagctcctgg 180

ttctcgcact gatccagcaa ctttttgaaa ctaaaggcgc tttccgccat caccgccact 240

ggcatcttcg cggccggcct cgccgttgca ccgtccggac agccaaacct gggctggacg 300

tggttttgtc tgctgcgccc gctcttcgcg ctctcgtttc attttctgca gcgcgccagc 360

agg atg gcc cac aag cag atc tac tac tcg gac aag tac ttc gac gaa 408
Met Ala His Lys Gln Ile Tyr Tyr Ser Asp Lys Tyr Phe Asp Glu
1               5                   10                  15

cac tac gag tac cgg cat gtt atg tta ccc aga gaa ctt tcc aaa caa 456
His Tyr Glu Tyr Arg His Val Met Leu Pro Arg Glu Leu Ser Lys Gln
                20                  25                  30

gta cct aaa act cat ctg atg tct gaa gag gag tgg agg aga ctt ggt 504
Val Pro Lys Thr His Leu Met Ser Glu Glu Glu Trp Arg Arg Leu Gly
            35                  40                  45

gtc caa cag agt cta ggc tgg gtt cat tac atg att cat gag cca gaa 552
Val Gln Gln Ser Leu Gly Trp Val His Tyr Met Ile His Glu Pro Glu
        50                  55                  60

cca cat att ctt ctc ttt aga cga cct ctt cca aaa gat caa caa aaa 600
Pro His Ile Leu Leu Phe Arg Arg Pro Leu Pro Lys Asp Gln Gln Lys
    65                  70                  75

tgaagtttat ctggggatcg tcaaatcttt ttcaaattta atgtatatgt gtatataagg 660

tagtattcag tgaatacttg agaaatgtac aaatctttca tccatacctg tgcatgagct 720

gtattcttca cagcaacaga gctcagttaa atgcaactgc aagtaggtta ctgtaagatg 780

tttaagataa aagttcttcc agtcagtttt tctcttaagt gcctgtttga gtttactgaa 840

acagtttact tttgttcaat aaagtttgta tgttgcattt aaaaaaaaaa aaaa 894

<210> 10
<211> 2248
<212> DNA
<213> Homo sapiens

<220>
<221> CDS
<222> (190)..(924)

<400> 10
aaggatcctt aattaaatta atcccccccc cccccccccg agctaaagcc aaaagcagat 60

caaagtggtg ggactcgcgt cgcggccgcg gagacgtgaa gctctcgatg ctcctcccgc 120

tgcgggtcgg cgctcgccct cgctctcctc gccctccgcc ccggccccgg ccccggcccc 180

gcgcccgcc atg gag aag act gag ctg atc cag aag gcc aag ctg gcc 228
Met Glu Lys Thr Glu Leu Ile Gln Lys Ala Lys Leu Ala
1               5                   10

gag cag gcc gag cgc tac gac gac atg gcc acc tgc atg aag gca gtg 276
Glu Gln Ala Glu Arg Tyr Asp Asp Met Ala Thr Cys Met Lys Ala Val
    15                  20                  25

acc gag cag ggc gcc gag ctg tcc aac gag gag cgc aac ctg ctc tcc 324
Thr Glu Gln Gly Ala Glu Leu Ser Asn Glu Glu Arg Asn Leu Leu Ser
30                  35                  40                  45

gtg gcc tac aag aac gtg gtc ggg ggc cgc agg tcc gcc tgg agg gtc 372
Val Ala Tyr Lys Asn Val Val Gly Gly Arg Arg Ser Ala Trp Arg Val
                50                  55                  60

atc tct agc atc gag cag aag acc gac acc tcc gac aag aag ttg cag 420
Ile Ser Ser Ile Glu Gln Lys Thr Asp Thr Ser Asp Lys Lys Leu Gln
            65                  70                  75

ctg att aag gac tat cgg gag aaa gtg gag tcc gag ctg aga tcc atc 468
Leu Ile Lys Asp Tyr Arg Glu Lys Val Glu Ser Glu Leu Arg Ser Ile
        80                  85                  90

tgc acc acg gtg ctg gaa ttg ttg gat aaa tat tta ata gcc aat gca 516
Cys Thr Thr Val Leu Glu Leu Leu Asp Lys Tyr Leu Ile Ala Asn Ala
    95                  100                 105

act aat cca gag agt aag gtc ttc tat ctg aaa atg aag ggt gat tac 564
Thr Asn Pro Glu Ser Lys Val Phe Tyr Leu Lys Met Lys Gly Asp Tyr
110                 115                 120                 125

ttc cgg tac ctt gct gaa gtt gcg tgt ggt gat gat cga aaa caa acg 612
Phe Arg Tyr Leu Ala Glu Val Ala Cys Gly Asp Asp Arg Lys Gln Thr
                130                 135                 140

ata gat aat tcc caa gga gct tac caa gag gca ttt gat ata agc aag 660
Ile Asp Asn Ser Gln Gly Ala Tyr Gln Glu Ala Phe Asp Ile Ser Lys
            145                 150                 155

aaa gag atg caa ccc aca cac cca atc cgc ctg ggg ctt gct ctt aac 708
Lys Glu Met Gln Pro Thr His Pro Ile Arg Leu Gly Leu Ala Leu Asn

160                 165                 170

ttt tct gta ttt tac tat gag att ctt aat aac cca gag ctt gcc tgc      756
Phe Ser Val Phe Tyr Tyr Glu Ile Leu Asn Asn Pro Glu Leu Ala Cys
    175                 180                 185

acg ctg gct aaa acg gct ttt gat gag gcc att gct gaa ctt gat aca      804
Thr Leu Ala Lys Thr Ala Phe Asp Glu Ala Ile Ala Glu Leu Asp Thr
190                 195                 200                 205

ctg aat gaa gac tca tac aaa gac agc acc ctc atc atg cag ttg ctt      852
Leu Asn Glu Asp Ser Tyr Lys Asp Ser Thr Leu Ile Met Gln Leu Leu
                210                 215                 220

aga gac aac cta aca ctt tgg aca tca gac agt gca gga gaa gaa tgt      900
Arg Asp Asn Leu Thr Leu Trp Thr Ser Asp Ser Ala Gly Glu Glu Cys
            225                 230                 235

gat gcg gca gaa ggg gct gaa aac taaatccata cagggtgtca tccttctttc    954
Asp Ala Ala Glu Gly Ala Glu Asn
        240                 245

cttcaagaaa cctttttaca catctccatt ccttattcca cttggatttc ctatagcaaa   1014

gaaacccatt catgtgtatg gaatcaactg tttatagtct tttcacactg cagctttggg   1074

aaaacttcat tccttgattt gtgtttgtct tggccttcct ggtgtgcagt actgctgtag   1134

aaaagtatta atagcttcat ttcatataaa cataagtaac tcccaaacac ttatgtagag   1194

gactaaaaat gtatctggta tttaagtaat ctgaaccagt tctgcaagtg actgtgtttt   1254

gtattactgt gaaaataaga aaatgtagtt aattacaatt taaagagtat tccacataac   1314

ttcttaattt ctacattccc tcccttactc ttcgggggtt tcctttcagt aagcaacttt   1374

tccatgctct taatgtattc ctttttagta ggaatccgga agtattagat tgaatggaaa   1434

agcacttgcc atctctgtct aggggtcaca aattgaaatg gctcctgtat cacatacgga   1494

ggtcttgtgt atctgtggca acagggagtt tccttattca ctctttattt gctgctgttt   1554

aagttgccaa cctcccctcc caataaaaat tcacttacac ctcctgcctt tgtagttctg   1614

gtattcactt tactatgtga tagaagtagc atgttgctgc cagaatacaa gcattgcttt   1674

tggcaaatta aagtgcatgt catttcttaa tacactagaa aggggaaata aattaaagta   1734

cacaagtcca agtctaaaac tttagtactt ttccatgcag atttgtgcac atgtgagagg   1794

gtgtccagtt tgtctagtga ttgttattta gagagttgga ccactattgt gtgttgctaa   1854

tcattgactg tagtcccaaa aaagccttgt gaaaatgtta tgccctatgt aacagcagag   1914

taacataaaa taaaagtaca ttttataaac catttactat ggctttgtaa caattgcata   1974

cccatatttt aagggacagg tgaatttact actttctaaa gtttattgat acttcccttt   2034

tatgtaaaat gtagtagtga tacctatatt tccacattgt gcattgtgac acacttgtct   2094

agggatgcct ggaagtgtat aaaattggac tgcatttctt agagtgtttt actatagatc 2154

agtctcatgg gccatctctt cctcagatgt aaatgatatc tggttaagtg ttatatggaa 2214

taaagtggac attttaaaac taaaaaaaaa aaaa 2248

<210> 11
<211> 1655
<212> DNA
<213> Homo sapiens

<220>
<221> CDS
<222> (382)..(1479)

<400> 11
tgattctgac tgaatacagg ccctggaccc ttccctcaag tctcaccagt tctgctctcc 60

catcaagctt cagatgccat gttgtactgg gggaatgtag cccttgtgct ccccaccccc 120

tacctccacc tgagcctcac cctgctgttg agccctgagt ggctagggga aatgggaaga 180

ggattgccat ggcctggcca tcttgttgct gcttggttag atcatatagc taatgaatta 240

ggcaggggag ctattttttg aagatgatga actaaatgtt gaagacaagt ttgagatctg 300

taaaattttt tggctttcac ccccaaccag tgaccaaaga cttgaccact caaagtccag 360

ctccccagaa cactgctcga c atg gac acc ggt gtg att gaa ggt gga tta 411
Met Asp Thr Gly Val Ile Glu Gly Gly Leu
1               5                   10

aat gtc act ctc acc atc cgg cta ctt atg cat gga aag gaa gtt ggc 459
Asn Val Thr Leu Thr Ile Arg Leu Leu Met His Gly Lys Glu Val Gly
                15                  20                  25

agt atc atc gga aag aaa gga gaa tca gtt aag aag atg cgc gag gag 507
Ser Ile Ile Gly Lys Lys Gly Glu Ser Val Lys Lys Met Arg Glu Glu
            30                  35                  40

agt ggt gca cgt atc aac atc tca gaa ggg aat tgt cct gag aga att 555
Ser Gly Ala Arg Ile Asn Ile Ser Glu Gly Asn Cys Pro Glu Arg Ile
        45                  50                  55

atc act ttg gct gga ccc act aat gcc atc ttc aaa gcc ttt gct atg 603
Ile Thr Leu Ala Gly Pro Thr Asn Ala Ile Phe Lys Ala Phe Ala Met
    60                  65                  70

atc att gac aaa ctg gaa gag gac ata agc agc tct atg acc aat agc 651
Ile Ile Asp Lys Leu Glu Glu Asp Ile Ser Ser Ser Met Thr Asn Ser
75                  80                  85                  90

aca gct gcc agt aga ccc ccg gtc acc ctg agg ctg gtg gtc cct gct 699
Thr Ala Ala Ser Arg Pro Pro Val Thr Leu Arg Leu Val Val Pro Ala
                95                  100                 105

agt cag tgt ggc tct ctc att gga aaa ggt gga tgc aag atc aag gaa 747

Ser Gln Cys Gly Ser Leu Ile Gly Lys Gly Gly Cys Lys Ile Lys Glu
            110                 115                 120

ata cga gag agt aca ggg gct cag gtc cag gtg gca ggg gat atg cta      795
Ile Arg Glu Ser Thr Gly Ala Gln Val Gln Val Ala Gly Asp Met Leu
        125                 130                 135

ccc aac tca act gag cgg gcc atc act att gct ggc att cca caa tcc      843
Pro Asn Ser Thr Glu Arg Ala Ile Thr Ile Ala Gly Ile Pro Gln Ser
    140                 145                 150

atc att gag tgt gtc aaa cag atc tgc gtg gtc atg ttg gag act ctc      891
Ile Ile Glu Cys Val Lys Gln Ile Cys Val Val Met Leu Glu Thr Leu
155                 160                 165                 170

tcc cag tcc ccc ccg aag ggc gtg acc atc ccg tac cgg ccc aag ccg      939
Ser Gln Ser Pro Pro Lys Gly Val Thr Ile Pro Tyr Arg Pro Lys Pro
                175                 180                 185

tcc agc tct ccg gtc atc ttt gca ggt ggt cag gac agg tac agc aca      987
Ser Ser Ser Pro Val Ile Phe Ala Gly Gly Gln Asp Arg Tyr Ser Thr
            190                 195                 200

ggc agc gac agt gcg agc ttt ccc cac acc acc ccg tcc atg tgc ctc     1035
Gly Ser Asp Ser Ala Ser Phe Pro His Thr Thr Pro Ser Met Cys Leu
        205                 210                 215

aac cct gac ctg gag gga cca cct cta gag gcc tat acc att caa gga     1083
Asn Pro Asp Leu Glu Gly Pro Pro Leu Glu Ala Tyr Thr Ile Gln Gly
    220                 225                 230

cag tat gcc att cca cag cca gat ttg acc aag ctg cac cag ttg gca     1131
Gln Tyr Ala Ile Pro Gln Pro Asp Leu Thr Lys Leu His Gln Leu Ala
235                 240                 245                 250

atg caa cag tct cat ttt ccc atg acg cat ggc aac acc gga ttc agt     1179
Met Gln Gln Ser His Phe Pro Met Thr His Gly Asn Thr Gly Phe Ser
                255                 260                 265

ggc att gaa tcc agc tct cca gag gtg aaa ggc tat tgg gca ggt ttg     1227
Gly Ile Glu Ser Ser Ser Pro Glu Val Lys Gly Tyr Trp Ala Gly Leu
            270                 275                 280

gat gca tct gct cag act act tct cat gaa ctc acc att cca aac gat     1275
Asp Ala Ser Ala Gln Thr Thr Ser His Glu Leu Thr Ile Pro Asn Asp
        285                 290                 295

ttg att ggc tgc ata atc ggg cgt caa ggc gcc aaa atc aat gag atc     1323
Leu Ile Gly Cys Ile Ile Gly Arg Gln Gly Ala Lys Ile Asn Glu Ile
    300                 305                 310

cgt cag atg tct ggg gcg cag atc aaa att gcg aac cca gtg gaa gga     1371
Arg Gln Met Ser Gly Ala Gln Ile Lys Ile Ala Asn Pro Val Glu Gly
315                 320                 325                 330

tct act gat agg cag gtt acc atc act gga tct gct gcc agc att agc     1419
Ser Thr Asp Arg Gln Val Thr Ile Thr Gly Ser Ala Ala Ser Ile Ser
                335                 340                 345

ctg gct caa tat cta atc aat gtc agg ctt tcc tcg gag acg ggt ggc     1467

Leu Ala Gln Tyr Leu Ile Asn Val Arg Leu Ser Ser Glu Thr Gly Gly
            350                 355                 360

atg ggg agc agc tag aacaatgcag attcatccat aatccctttc tgctgttcac      1522
Met Gly Ser Ser
        365

caccacccat gatccatctg tgtagtttct gaacagtcag cgattccagg ttttaaatag      1582

tttgtaaatt ttcagtttct acacacttta tcatccactc gtgatttttt aattaaagcg      1642

ttttaattcc ttt                                                          1655

<210> 12
<211> 794
<212> DNA
<213> Homo sapiens

<220>
<221> CDS
<222> (230)..(748)

<400> 12
caaagttgtc atgaatactg acttgggcgt gggacccatc cgagatgtgc tggaccacat      60

ctacagtgcg ctgtatgtgg agctggtggt gaagaatccc ctgtgcccgc tgggccaaac      120

tgtgcaaagt gagctctttc gctcccgact ggactcctat gttcgctctc tgcccttctt      180

ctccgcccgg gctggctgaa gcaacctacc tcaagtctca ggagaattc atg tct      235
                                                      Met Ser
                                                      1

gcc tgg ggc cct tcc ggg aac ccg agc cca agg agt ggg ggc ggc ccc      283
Ala Trp Gly Pro Ser Gly Asn Pro Ser Pro Arg Ser Gly Gly Gly Pro
        5                   10                  15

gga gcg agg ctc aca ctc cct gcc ctg cag atg act gtc cac aac ctg      331
Gly Ala Arg Leu Thr Leu Pro Ala Leu Gln Met Thr Val His Asn Leu
    20                  25                  30

tac ctg ttt gac cgg aat gga gtg tgt ctg cac tac agc gaa tgg cac      379
Tyr Leu Phe Asp Arg Asn Gly Val Cys Leu His Tyr Ser Glu Trp His
35                  40                  45                  50

cgc aag aag caa gca ggg att ccc aag gag gag gag tat aag ctg atg      427
Arg Lys Lys Gln Ala Gly Ile Pro Lys Glu Glu Glu Tyr Lys Leu Met
                55                  60                  65

tac ggg atg ctc ttc tct atc cgc tcg ttt gtc agc aag atg tcc ccg      475
Tyr Gly Met Leu Phe Ser Ile Arg Ser Phe Val Ser Lys Met Ser Pro
            70                  75                  80

cta gac atg aag gat ggc ttc ctg gcc ttc caa act agc cgt tac aaa      523
Leu Asp Met Lys Asp Gly Phe Leu Ala Phe Gln Thr Ser Arg Tyr Lys
        85                  90                  95

ctc cat tac tac gag acg ccc act ggg atc aaa gtt gtc atg aat act 571
Leu His Tyr Tyr Glu Thr Pro Thr Gly Ile Lys Val Val Met Asn Thr
    100                 105                 110

gac ttg ggc gtg gga ccc atc cga gat gtg ctg cac cac atc tac agt 619
Asp Leu Gly Val Gly Pro Ile Arg Asp Val Leu His His Ile Tyr Ser
115                 120                 125                 130

gcg ctg tat gtg gag ctg gtg gtg aag aat ccc ctg tgc ccg ctg ggc 667
Ala Leu Tyr Val Glu Leu Val Val Lys Asn Pro Leu Cys Pro Leu Gly
                135                 140                 145

caa act gtg caa agt gag ctc ttt cgc tcc cga ctg gac tcc tat gtt 715
Gln Thr Val Gln Ser Glu Leu Phe Arg Ser Arg Leu Asp Ser Tyr Val
            150                 155                 160

cgc tct ctg ccc ttc ttc tcc gcc cgg gct ggc tgaagcaa cctacctcaa 766
Arg Ser Leu Pro Phe Phe Ser Ala Arg Ala Gly
        165                 170

gtctcaggag aattcatgtc tgcctggg 794

<210> 13
<211> 709
<212> DNA
<213> Homo sapiens

<220>
<221> CDS
<222> (85)..(528)

<400> 13
aaggatcctt aattaaatta atcccccccc cccgctctag cagctgccgc tgagccgccg 60

gacggacgct cgtcttcgcc cgcc atg gcc gag agc gac tgg gac acg gtg 111
Met Ala Glu Ser Asp Trp Asp Thr Val
1               5

acg gtg ctg cgc aag aag ggc cct acg gcc gcc cag gcc aaa tcc aag 159
Thr Val Leu Arg Lys Lys Gly Pro Thr Ala Ala Gln Ala Lys Ser Lys
10                  15                  20                  25

cag gct atc tta gcg gca cag aga cga gga gaa gat gtg gag act tcc 207
Gln Ala Ile Leu Ala Ala Gln Arg Arg Gly Glu Asp Val Glu Thr Ser
                30                  35                  40

aag aaa tgg gct gct ggc cag aac aaa caa cat tct att acc aag aac 255
Lys Lys Trp Ala Ala Gly Gln Asn Lys Gln His Ser Ile Thr Lys Asn
            45                  50                  55

acg gcc aag ctg gac cgg gag aca gag gag ctg cac cat gac agg gtg 303
Thr Ala Lys Leu Asp Arg Glu Thr Glu Glu Leu His His Asp Arg Val
        60                  65                  70

acc ctg gag gtg ggc aag gtg atc cag caa ggt cgg cag agc aag ggg 351
Thr Leu Glu Val Gly Lys Val Ile Gln Gln Gly Arg Gln Ser Lys Gly
    75                  80                  85

ctt acg cag aag gac ctg gcc acg aaa atc aat gag aag cca cag gtg     399
Leu Thr Gln Lys Asp Leu Ala Thr Lys Ile Asn Glu Lys Pro Gln Val
 90                 95                 100                 105

atc gcg gac tat gag agc gga cgg gcc ata ccc aat aac cag gtg ctt     447
Ile Ala Asp Tyr Glu Ser Gly Arg Ala Ile Pro Asn Asn Gln Val Leu
                110                 115                 120

ggc aaa atc gag cgg gcc att ggc ctc aag ctc cgg gga aag gac att     495
Gly Lys Ile Glu Arg Ala Ile Gly Leu Lys Leu Arg Gly Lys Asp Ile
            125                 130                 135

gga aag ccc atc gag aag ggg cct agg gcg aaa tgaacaca aagcctcgaa     546
Gly Lys Pro Ile Glu Lys Gly Pro Arg Ala Lys
        140                 145

atcagtgcgc tccagctgat ctcgttccgc cggttcccct tggccgccag ttccgttctc     606

ctcacgggcc gaacggaaca aggggtccag cttgcggggg accctcccca gcccattcct     666

gctgtcaaac aaacaaaacc ttgcaaagcg aaaaaaaaaa aaa     709

<210> 14
<211> 627
<212> DNA
<213> Homo sapiens

<220>
<221> CDS
<222> (11)..(349)

<400> 14
cggggctggc atg acc ccg ggg gtc ttc cat gcc agt ccg cct cag tcg     49
           Met Thr Pro Gly Val Phe His Ala Ser Pro Pro Gln Ser
            1               5                  10

cag agg gtc cct cgg caa gcg ccc tgt gag tgg gcc att cgg aac att     97
Gln Arg Val Pro Arg Gln Ala Pro Cys Glu Trp Ala Ile Arg Asn Ile
    15                  20                  25

gga cag aag ccc aaa gag cca aat tgt cac aat tgt gga acc cac att     145
Gly Gln Lys Pro Lys Glu Pro Asn Cys His Asn Cys Gly Thr His Ile
 30                 35                  40                  45

ggc ctg aga tcc aaa acg ctt cga ggc acc cca aat tac ctg ccc att     193
Gly Leu Arg Ser Lys Thr Leu Arg Gly Thr Pro Asn Tyr Leu Pro Ile
                50                  55                  60

cgt cag gac acc cac cca ccc agt gtt ata ttc tgc ctc gcc gga gtg     241
Arg Gln Asp Thr His Pro Pro Ser Val Ile Phe Cys Leu Ala Gly Val
            65                  70                  75

ggt gtt ccc ggg ggc act tgc cga cca gcc cct tgc gtc ccc agg ttt     289
Gly Val Pro Gly Gly Thr Cys Arg Pro Ala Pro Cys Val Pro Arg Phe
        80                  85                  90

gca gct ctc ccc tgg gcc act aac cat cct ggc ccg ggc tgc ctg tct 337
Ala Ala Leu Pro Trp Ala Thr Asn His Pro Gly Pro Gly Cys Leu Ser
    95                  100                 105

gac ctc cgt gcc tag tcgtggctct ccatcttgtc tcctccccgt gtccccaatg 392
Asp Leu Arg Ala
110

tcttcagtgg ggggcccctc ttgggtcccc tcctctgcca tcacctgaag acccccacgc 452

caaacactga atgtcacctg tgcctgccgc ctcggtccac cttgcggccc gtgtttgact 512

caactcagct cctttaacgc taatatttcc ggcaaaatcc catgcttggg ttttgtcttt 572

aaccttgtaa cgcttgcaat cccaataaag cattaaaagt catgaaaaaa aaaaa 627

<210> 15
<211> 325
<212> DNA
<213> Homo sapiens

<220>
<221> CDS
<222> (86)..(268)

<400> 15
ggctgttggg gtccaccacg ccttccacct gccccactgc ttcttcgctt ctctcttgga 60

aagtccagtc tctcctcggc ttgca atg gac ccc aac tgc tcc tgc gcc gct 112
Met Asp Pro Asn Cys Ser Cys Ala Ala
1               5

ggt gtc tcc tgc acc tgc gct ggt tcc tgc aag tgc aaa gag tgc aaa 160
Gly Val Ser Cys Thr Cys Ala Gly Ser Cys Lys Cys Lys Glu Cys Lys
10                  15                  20                  25

tgc acc tcc tgc aag aag agc tgc tgc tcc tgc tgc ccc gtg ggc tgt 208
Cys Thr Ser Cys Lys Lys Ser Cys Cys Ser Cys Cys Pro Val Gly Cys
                30                  35                  40

agc aag tgt gcc cag ggc tgt gtt tgc aaa ggg gcg tca gag aag tgc 256
Ser Lys Cys Ala Gln Gly Cys Val Cys Lys Gly Ala Ser Glu Lys Cys
            45                  50                  55

agc tgc tgc gac tga tgccaggaca acctttctcc cagatgtaaa cagagagaca 311
Ser Cys Cys Asp
        60

tgtacaaacc tgga 325

<210> 16
<211> 791
<212> DNA
<213> Homo sapiens

<220>
<221> CDS
<222> (223)..(738)

<400> 16
cggaggtgtc taccccgccg gtgatggcgt tgaacgccac tggcttcccg gccttccgtc 60

cgctgcctcc gtccgattct gcgtctgctt gctgaggagg cggattaggg gggcgcggag 120

tctcttccct tgagtgcata ggtcccggtt ggtagagggt ttgagtccgc atcgccacag 180

ctgaaggctg cgagggacta agagcagaat atatctttag aa atg agt tgc aca 234
Met Ser Cys Thr
1

att gag aag gca ctt gcc gac gct aaa gct ctt gtt gaa aga tta aga 282
Ile Glu Lys Ala Leu Ala Asp Ala Lys Ala Leu Val Glu Arg Leu Arg
5                   10                  15                  20

gat cat gac gat gca gca gaa tct ctg att gag caa acc aca gct ctc 330
Asp His Asp Asp Ala Ala Glu Ser Leu Ile Glu Gln Thr Thr Ala Leu
                25                  30                  35

aac aag cga gta gaa gcc atg aaa cag tat cag gaa gaa att caa gaa 378
Asn Lys Arg Val Glu Ala Met Lys Gln Tyr Gln Glu Glu Ile Gln Glu
            40                  45                  50

ctt aat gaa gtc gcg aga cat cgg cca cgg tcc acg tta gtt atg gga 426
Leu Asn Glu Val Ala Arg His Arg Pro Arg Ser Thr Leu Val Met Gly
        55                  60                  65

atc cag caa gaa aac aga caa atc aga gag ttg caa caa gaa aac aaa 474
Ile Gln Gln Glu Asn Arg Gln Ile Arg Glu Leu Gln Gln Glu Asn Lys
    70                  75                  80

gaa tta cgt aca tct ctg gaa gaa cat cag tcg gcc ttg gaa ctt ata 522
Glu Leu Arg Thr Ser Leu Glu Glu His Gln Ser Ala Leu Glu Leu Ile
85                  90                  95                  100

atg agc aag tac cga gaa caa atg ttt aga ttg cta atg gct agc aaa 570
Met Ser Lys Tyr Arg Glu Gln Met Phe Arg Leu Leu Met Ala Ser Lys
                105                 110                 115

aaa gat gat ccg ggt ata ata atg aag tta aaa gag cag cac tcc aag 618
Lys Asp Asp Pro Gly Ile Ile Met Lys Leu Lys Glu Gln His Ser Lys
            120                 125                 130

att gac atg gta cat cgt aac aag tcc gaa gga ttc ttc ctt gat gca 666
Ile Asp Met Val His Arg Asn Lys Ser Glu Gly Phe Phe Leu Asp Ala
        135                 140                 145

tct cga cac atc ctt gaa gca cct caa cat gga ctg gag aga agg cac 714
Ser Arg His Ile Leu Glu Ala Pro Gln His Gly Leu Glu Arg Arg His
    150                 155                 160

ttg gaa gca aat cag aat gta cac taaataaaca gtcaactttt ggggtgtgga 768
Leu Glu Ala Asn Gln Asn Val His
165                 170

tggaaggggg gtccatttta aaa                                              791

<210> 17
<211> 1297
<212> DNA
<213> Homo sapiens

<220>
<221> CDS
<222> (223)..(1251)

<400> 17
taagcttgcg gccgcccgcg agcggggcgg tgccacgtac ggctccggaa gaagcgacgg      60
aatctgctag ggcacaagag agacgggcgc tcgggctctc gcagtcctct tccgtcagtg     120
tcttttgctt cgactcccgg cggagcgcgc aacgtggagt gacgtgcagg ggccaagtgc     180
aacccaggca gccacggctg tttcggagct caggactcta aa atg gca gag cag        234
ctt tct cca gga aag gcg gtg gat cag gtg tgc acc ttc ctt ttc aaa       282
aag cct ggg cgg aaa ggg gct gct gga cgc aga aag cgc ccg gcc tgc       330
gac cca gag ccc gga gaa agc ggc agc agt agc gac gaa ggc tgc act       378
gtg gtt cga ccg gaa aag aag cgg gtg acc cac aat cca atg ata cag       426
aag acc cgt gac agt ggt aaa cag aag gcg gct tac ggc gac ttg agc       474
agc gaa gag gaa gag gaa aat gag ccc gag agt ctc ggc gtg gtt tat       522
aaa tcc acc cgt tcg gcg aaa ccc gtg gga cca gag gat atg gga gcg       570
aca gct gtc tat gag ctg gac aca gag aaa gag cgc gat gca caa gcc       618
atc ttt gag cgc agc cag aag atc cag gag gag ctg agg ggc aag gag       666
gat gac aag atc tat cgg gga atc aac aat tat cag aaa tac atg aag 714
ccc aag gat acg tct atg ggc aat gcc tct tcc ggg atg gtg agg aag 762
ggc ccc atc cga gcg ccc gag cat cta cgt gcc acc gtg cgc tgg gat 810
tac cag ccc gac atc tgt aag gac tac aaa gag act ggc ttc tgc ggc 858
ttc gga gac agc tgc aaa ttc ctc cat gac cgt tca gat tac aag cat 906
ggg tgg cag atc gaa cgt gag ctt gat gag ggt cgc tat ggt gtc tat 954
gag gat gaa aac tat gaa gtg gga agc gat gat gag gaa ata cca ttc 1002
aag tgt ttc atc tgt cgc cag agc ttc caa aac cca gtt gtc acc aag 1050
tgc agg cat tat ttc tgc gag agc tgt gca ctg cag cat ttc cgc acc 1098
acc ccg cgc tgc tat gtc tgt gac cag cag acc aat ggc gtc ttc aat 1146
cca gcg aaa gaa ttg att gct aaa cta gag aag cat cga gct aca gga 1194
gag ggt ggt gct tcc gac ttg cca gaa gac ccc gat gag gat gca att 1242
ccc att act taggtttccc ataattctta aatttaaaaa ataaacgttt tgttct 1297

<210> 18
<211> 1454
<212> DNA

<213> Homo sapiens

<220>
<221> CDS
<222> (16)..(1401)

<400> 18
cctctgcagc tcagc atg gct agg gta ctg gga gca ccc gtt gca ctg ggg 51
Met Ala Arg Val Leu Gly Ala Pro Val Ala Leu Gly
1               5                   10

ttg tgg agc cta tgc tgg tct ctg gcc att gcc acc cct ctt cct ccg 99
Leu Trp Ser Leu Cys Trp Ser Leu Ala Ile Ala Thr Pro Leu Pro Pro
        15                  20                  25

act agt gcc cat ggg aat gtt gct gaa ggc gag acc aag cca gac cca 147
Thr Ser Ala His Gly Asn Val Ala Glu Gly Glu Thr Lys Pro Asp Pro
    30                  35                  40

gac gtg act gaa cgc tgc tca gat ggc tgg agc ttt gat gct acc acc 195
Asp Val Thr Glu Arg Cys Ser Asp Gly Trp Ser Phe Asp Ala Thr Thr
45                  50                  55                  60

ctg gat gac aat gga acc atg ctg ttt ttt aaa ggg gag ttt gtg tgg 243
Leu Asp Asp Asn Gly Thr Met Leu Phe Phe Lys Gly Glu Phe Val Trp
                65                  70                  75

aag agt cac aaa tgg gac cgg gag tta atc tca gag aga tgg aag aat 291
Lys Ser His Lys Trp Asp Arg Glu Leu Ile Ser Glu Arg Trp Lys Asn
            80                  85                  90

ttc ccc agc cct gtg gat gct gca ttc cgt caa ggt cac aac agt gtc 339
Phe Pro Ser Pro Val Asp Ala Ala Phe Arg Gln Gly His Asn Ser Val
        95                  100                 105

ttt ctg atc aag ggg gac aaa gtc tgg gta tac cct cct gaa aag aag 387
Phe Leu Ile Lys Gly Asp Lys Val Trp Val Tyr Pro Pro Glu Lys Lys
    110                 115                 120

gag aaa gga tac cca aag ttg ctc caa gat gaa ttt cct gga atc cca 435
Glu Lys Gly Tyr Pro Lys Leu Leu Gln Asp Glu Phe Pro Gly Ile Pro
125                 130                 135                 140

tcc cca ctg gat gca gct gtg gaa tgt cac cgt gga gaa tgt caa gct 483
Ser Pro Leu Asp Ala Ala Val Glu Cys His Arg Gly Glu Cys Gln Ala
                145                 150                 155

gaa ggc gtc ctc ttc ttc caa ggt gac cgc gag tgg ttc tgg gac ttg 531
Glu Gly Val Leu Phe Phe Gln Gly Asp Arg Glu Trp Phe Trp Asp Leu
            160                 165                 170

gct acg gga acc atg aag gag cgt tcc tgg cca gct gtt ggg aac tgc 579
Ala Thr Gly Thr Met Lys Glu Arg Ser Trp Pro Ala Val Gly Asn Cys
        175                 180                 185

tcc tct gcc ctg aga tgg ctg ggc cgc tac tac tgc ttc cag ggt aac 627
Ser Ser Ala Leu Arg Trp Leu Gly Arg Tyr Tyr Cys Phe Gln Gly Asn
    190                 195                 200

caa ttc ctg cgc ttc gac cct gtc agg gga gag gtg cct ccc agg tac 675

Gln Phe Leu Arg Phe Asp Pro Val Arg Gly Glu Val Pro Pro Arg Tyr
205                 210                 215                 220

ccg cgg gat gtc cga gac tac ttc atg ccc tgc cct ggc aga ggc cat     723
Pro Arg Asp Val Arg Asp Tyr Phe Met Pro Cys Pro Gly Arg Gly His
                225                 230                 235

gga cac agg aat ggg act ggc cat ggg aac agt acc cac cat ggc cct     771
Gly His Arg Asn Gly Thr Gly His Gly Asn Ser Thr His His Gly Pro
            240                 245                 250

gag tat atg cgc tgt agc cca cat cta gtc ttg tct gca ctg acg tct     819
Glu Tyr Met Arg Cys Ser Pro His Leu Val Leu Ser Ala Leu Thr Ser
        255                 260                 265

gac aac cat ggt gcc acc tat gcc ttc agt ggg acc cac tac tgg cgt     867
Asp Asn His Gly Ala Thr Tyr Ala Phe Ser Gly Thr His Tyr Trp Arg
    270                 275                 280

ctg gac acc agc cgg gat ggc tgg cat agc tgg ccc att gct cat cag     915
Leu Asp Thr Ser Arg Asp Gly Trp His Ser Trp Pro Ile Ala His Gln
285                 290                 295                 300

tgg ccc cag ggt cct tca gca gtg gat gct gcc ttt tcc tgg gaa gaa     963
Trp Pro Gln Gly Pro Ser Ala Val Asp Ala Ala Phe Ser Trp Glu Glu
                305                 310                 315

aaa ctc tat ctg gtc cag ggc acc cag gta tat gtc ttc ctg aca aag     1011
Lys Leu Tyr Leu Val Gln Gly Thr Gln Val Tyr Val Phe Leu Thr Lys
            320                 325                 330

gga ggc tat acc cta gta agc ggt tat ccg aag cgg ctg gag aag gaa     1059
Gly Gly Tyr Thr Leu Val Ser Gly Tyr Pro Lys Arg Leu Glu Lys Glu
        335                 340                 345

gtc ggg acc cct cat ggg att atc ctg gac tct gtg gat gcg gcc ttt     1107
Val Gly Thr Pro His Gly Ile Ile Leu Asp Ser Val Asp Ala Ala Phe
    350                 355                 360

atc tgc cct ggg tct tct cgg ctc cat atc atg gca gga cgg cgg ctg     1155
Ile Cys Pro Gly Ser Ser Arg Leu His Ile Met Ala Gly Arg Arg Leu
365                 370                 375                 380

tgg tgg ctg gac ctg aag tca gga gcc caa gcc acg tgg aca gag ctt     1203
Trp Trp Leu Asp Leu Lys Ser Gly Ala Gln Ala Thr Trp Thr Glu Leu
                385                 390                 395

cct tgg ccc cat gag aag gta gac gga gcc ttg tgt atg gaa aag tcc     1251
Pro Trp Pro His Glu Lys Val Asp Gly Ala Leu Cys Met Glu Lys Ser
            400                 405                 410

ctt ggc cct aac tca tgt tcc gcc aat ggt ccc ggc ttg tac ctc atc     1299
Leu Gly Pro Asn Ser Cys Ser Ala Asn Gly Pro Gly Leu Tyr Leu Ile
        415                 420                 425

cat ggt ccc aat ttg tac tgc tac agt gat gtg gag aaa ctg aat gca     1347
His Gly Pro Asn Leu Tyr Cys Tyr Ser Asp Val Glu Lys Leu Asn Ala
    430                 435                 440

gcc aag gcc ctt ccg caa ccc cag aat gtg acc agt ctc ctg ggc tgc     1395

Ala Lys Ala Leu Pro Gln Pro Gln Asn Val Thr Ser Leu Leu Gly Cys
445                 450                 455                 460

act cac tgaggggcct tctgacatga gtctggcctg gccccacctc ctagttcctc      1451
Thr His

ata                                                                 1454

<210> 19
<211> 1764
<212> DNA
<213> Homo sapiens

<220>
<221> CDS
<222> (203)..(1042)

<400> 19
gatttgctct gccagcagct gtcggtgccg cgctcgacac cgagtcctag ctaggcgctc      60

acagaatacg cgctccctcc ctcccccttc tctgtccccc gcctctcgct caccccggcc      120

cactccagcg gcgactttga gggattccct ctctggcggc ctctgcagca gcacagccgg      180

cctcattcgg ggcactgcga gt atg gat ctc caa gga aga ggg gtc ccc agc      232
                         Met Asp Leu Gln Gly Arg Gly Val Pro Ser
                         1               5                   10

atc gac aga ctt cga gtt ctc ctg atg ttg ttc cat aca atg gct caa      280
Ile Asp Arg Leu Arg Val Leu Leu Met Leu Phe His Thr Met Ala Gln
                15                  20                  25

atc atg gca gaa caa gaa gtg gaa aat ctc tca ggc ctt tcc act aac      328
Ile Met Ala Glu Gln Glu Val Glu Asn Leu Ser Gly Leu Ser Thr Asn
            30                  35                  40

cct gaa aaa gat ata ttt gtg gtg cgg gaa aat ggg acg acg tgt ctc      376
Pro Glu Lys Asp Ile Phe Val Val Arg Glu Asn Gly Thr Thr Cys Leu
        45                  50                  55

atg gca gag ttt gca gcc aaa ttt att gta cct tat gat gtg tgg gcc      424
Met Ala Glu Phe Ala Ala Lys Phe Ile Val Pro Tyr Asp Val Trp Ala
    60                  65                  70

agc aac tac gta gat ctg atc aca gaa cag gcc gat atc gca ttg acc      472
Ser Asn Tyr Val Asp Leu Ile Thr Glu Gln Ala Asp Ile Ala Leu Thr
75                  80                  85                  90

cgg gga gct gag gtg aag ggc cgc tgt ggc cac agc cag tcg gag ctg      520
Arg Gly Ala Glu Val Lys Gly Arg Cys Gly His Ser Gln Ser Glu Leu
                95                  100                 105

caa gtg ttc tgg gtg gat cgc gca tat gca ctc aaa atg ctc ttt gta      568
Gln Val Phe Trp Val Asp Arg Ala Tyr Ala Leu Lys Met Leu Phe Val
            110                 115                 120

aag gaa agc cac aac atg tcc aag gga cct gag gcg act tgg agg ctg 616
Lys Glu Ser His Asn Met Ser Lys Gly Pro Glu Ala Thr Trp Arg Leu
        125                 130                 135

agc aaa gtg cag ttt gtc tac gac tcc tcg gag aaa acc cac ttc aaa 664
Ser Lys Val Gln Phe Val Tyr Asp Ser Ser Glu Lys Thr His Phe Lys
    140                 145                 150

gac gca gtc agt gct ggg aag cac aca gcc aac tcg cac cac ctc tct 712
Asp Ala Val Ser Ala Gly Lys His Thr Ala Asn Ser His His Leu Ser
155                 160                 165                 170

gcc ttg gtc acc ccc gct ggg aag tcc tat gag tgt caa gct caa caa 760
Ala Leu Val Thr Pro Ala Gly Lys Ser Tyr Glu Cys Gln Ala Gln Gln
                175                 180                 185

acc att tca ctg gcc tct agt gat ccg cag aag acg gtc acc atg atc 808
Thr Ile Ser Leu Ala Ser Ser Asp Pro Gln Lys Thr Val Thr Met Ile
            190                 195                 200

ctg tct gcg gtc cac atc caa cct ttt gac att atc tca gat ttt gtc 856
Leu Ser Ala Val His Ile Gln Pro Phe Asp Ile Ile Ser Asp Phe Val
        205                 210                 215

ttc agt gaa gag cat aaa tgc cca gtg gat gag cgg gag caa ctg gaa 904
Phe Ser Glu Glu His Lys Cys Pro Val Asp Glu Arg Glu Gln Leu Glu
    220                 225                 230

gaa acc ttg ccc ctg att ttg ggg ctc atc ttg ggc ctc gtc atc atg 952
Glu Thr Leu Pro Leu Ile Leu Gly Leu Ile Leu Gly Leu Val Ile Met
235                 240                 245                 250

gta aca ctc gcg att tac cac gtc cac cac aaa atg act gcc aac cag 1000
Val Thr Leu Ala Ile Tyr His Val His His Lys Met Thr Ala Asn Gln
                255                 260                 265

gtg cag atc cct cgg gac aga tcc cag tat aag cac atg ggc tagaggc 1049
Val Gln Ile Pro Arg Asp Arg Ser Gln Tyr Lys His Met Gly
            270                 275                 280

cgttaggcag gcacccccta ttcctgctcc cccaactgga tcaggtagaa caacaaaagc 1109

acttttccat cttgtacacg agatacacca acatagctac aatcaaacag gcctgggtat 1169

ctgaggcttg cttggcttgt gtccatgctt aaacccacgg aagggggaga ctctttcgga 1229

tttgtagggt gaaatggcaa ttattctctc catgctgggg aggaggggag gagggtctca 1289

gacagctttc gtgctcatgg tggcttggct ttgactctcc aaagagcaat aaatgccact 1349

tggagctgta tctggcccca aagtttaggg attgaaaaca tgcttctttg aggaggaaac 1409

ccctttaggt tcagaagaat atggggtgct ttgctccctt ggacacagct ggcttatcct 1469

atacagttgt caatgcacac agaatacaac ctcatgctcc ctgcagcaag acccctgaaa 1529

gtgattcatg cttctggctg gcattctgca tgtttagtga ttgtcttggg aatgtttcac 1589

tgctacccgc atccagcgac tgcagcacca gaaaacgact aatgtaacta tgcagagttg 1649

tttggacttc ttcctgtgcc aggtccaagt cgggggacct gaagaatcaa tctgtgtgag 1709

tctgtttttc aaaatgaaat aaaacacact attctctggc aaaaaaaaaa aaaaa 1764

<210> 20
<211> 1872
<212> DNA
<213> Homo sapiens

<220>
<221> CDS
<222> (232)..(1713)

<400> 20
ttggacacta gtgcagaaat atgtcccgag agcaaaatag agaggccaca agtgacagaa 60

agattttaca catacatgtg cgagatacaa aaacagtgaa ggatgtacag aagccaaaaa 120

atgtgaacaa gacagctgaa aaagttagaa ttataaaata tttgttggga gagctcatgg 180

cccctggtag cagaacaaga gatttcagaa attcagaggt tgattacaga a atg gag 237
Met Glu
1

gca tgt ata tct gta ctt cca aca gta agt gga aac aca gat att caa 285
Ala Cys Ile Ser Val Leu Pro Thr Val Ser Gly Asn Thr Asp Ile Gln
        5                   10                  15

gtt gag ata gca ctg gcc atg caa cca tta aga agt gag aat gct cag 333
Val Glu Ile Ala Leu Ala Met Gln Pro Leu Arg Ser Glu Asn Ala Gln
    20                  25                  30

tta cga agg cag ttg aga att ttg aac cag caa ctc aga gaa caa cag 381
Leu Arg Arg Gln Leu Arg Ile Leu Asn Gln Gln Leu Arg Glu Gln Gln
35                  40                  45                  50

aaa act caa aaa cca tct ggt gct gtg gat tgc aac ctt gaa ttg ttt 429
Lys Thr Gln Lys Pro Ser Gly Ala Val Asp Cys Asn Leu Glu Leu Phe
                55                  60                  65

tct ctt cag tca ttg aat atg tca ctg caa aat caa ttg gag gag tca 477
Ser Leu Gln Ser Leu Asn Met Ser Leu Gln Asn Gln Leu Glu Glu Ser
            70                  75                  80

cta aag agc cag gaa tta ctg cag agt aaa aat gaa gag ctg tta aaa 525
Leu Lys Ser Gln Glu Leu Leu Gln Ser Lys Asn Glu Glu Leu Leu Lys
        85                  90                  95

gtg att gaa aat cag aaa gat gaa aac aaa aaa ttt agt agt ata ttt 573
Val Ile Glu Asn Gln Lys Asp Glu Asn Lys Lys Phe Ser Ser Ile Phe
    100                 105                 110

aaa gac aaa gat caa act ata ctt gaa aat aaa cag caa tat gat att 621
Lys Asp Lys Asp Gln Thr Ile Leu Glu Asn Lys Gln Gln Tyr Asp Ile
115                 120                 125                 130

gag ata aca aga ata aaa att gaa ttg gag gaa gcc cta gtc aat gtg 669

Glu Ile Thr Arg Ile Lys Ile Glu Leu Glu Glu Ala Leu Val Asn Val
                135                 140                 145

aaa agc tcc cag ttt aag tta gaa act gct gaa aag gaa aac cag ata 717
Lys Ser Ser Gln Phe Lys Leu Glu Thr Ala Glu Lys Glu Asn Gln Ile
            150                 155                 160

ttg ggg ata aca tta cgt cag cgt gat gct gag gtg act cga cta aga 765
Leu Gly Ile Thr Leu Arg Gln Arg Asp Ala Glu Val Thr Arg Leu Arg
        165                 170                 175

gaa tta acc aga act tta cag act agc atg gca aag ctt ctc tcc gat 813
Glu Leu Thr Arg Thr Leu Gln Thr Ser Met Ala Lys Leu Leu Ser Asp
    180                 185                 190

ctt agt gtg gac agt gct cgc tgc aag cct ggg aat aac ctt acc aaa 861
Leu Ser Val Asp Ser Ala Arg Cys Lys Pro Gly Asn Asn Leu Thr Lys
195                 200                 205                 210

tca ctc ttg aac att cat gat aaa caa ctt caa cat gac cca gct cct 909
Ser Leu Leu Asn Ile His Asp Lys Gln Leu Gln His Asp Pro Ala Pro
                215                 220                 225

gct cac act tcc ata atg agc tat cta aat aag tta gaa aca aat tac 957
Ala His Thr Ser Ile Met Ser Tyr Leu Asn Lys Leu Glu Thr Asn Tyr
            230                 235                 240

agt ttt aca cat tca gag cca ctt tct aca att aaa aat gag gaa acc 1005
Ser Phe Thr His Ser Glu Pro Leu Ser Thr Ile Lys Asn Glu Glu Thr
        245                 250                 255

ata gag cca gac aaa acc tat gaa aat gtt ctg tcc tcc aga ggc cct 1053
Ile Glu Pro Asp Lys Thr Tyr Glu Asn Val Leu Ser Ser Arg Gly Pro
    260                 265                 270

caa aat agt aac act agg ggc atg gag gaa gca tct gca cct gga att 1101
Gln Asn Ser Asn Thr Arg Gly Met Glu Glu Ala Ser Ala Pro Gly Ile
275                 280                 285                 290

att tct gcc ctt tca aaa cag gat tct gat gaa ggg agt gaa act atg 1149
Ile Ser Ala Leu Ser Lys Gln Asp Ser Asp Glu Gly Ser Glu Thr Met
                295                 300                 305

gct tta ata gaa gat gag cat aat ttg gat aat aca att tac att cct 1197
Ala Leu Ile Glu Asp Glu His Asn Leu Asp Asn Thr Ile Tyr Ile Pro
            310                 315                 320

ttt gct aga agc act cct gaa aag aaa tca cca ctt tct aag aga cta 1245
Phe Ala Arg Ser Thr Pro Glu Lys Lys Ser Pro Leu Ser Lys Arg Leu
        325                 330                 335

tcc cct cag cca caa ata aga gca gct aca aca cag cta gtc agc aac 1293
Ser Pro Gln Pro Gln Ile Arg Ala Ala Thr Thr Gln Leu Val Ser Asn
    340                 345                 350

agt gga ctt gct gtc tct gga aaa gaa aat aaa ctg tgt aca cct gta 1341
Ser Gly Leu Ala Val Ser Gly Lys Glu Asn Lys Leu Cys Thr Pro Val
355                 360                 365                 370

atc tgt tcc tct tca aca aag gaa gca gaa gat gca cct gaa aaa ctt 1389

Ile Cys Ser Ser Ser Thr Lys Glu Ala Glu Asp Ala Pro Glu Lys Leu
                375                 380                 385

tcc aga gca tct gat atg aag gac aca cag ctc ctc aag aaa ata aag      1437
Ser Arg Ala Ser Asp Met Lys Asp Thr Gln Leu Leu Lys Lys Ile Lys
            390                 395                 400

gaa gca att ggt aag atc cct gct gcc acc aag gag cca gag gaa caa      1485
Glu Ala Ile Gly Lys Ile Pro Ala Ala Thr Lys Glu Pro Glu Glu Gln
        405                 410                 415

act gca tgt cat ggc cca tca ggt tgt ctt agc aac agc ctt caa gtg      1533
Thr Ala Cys His Gly Pro Ser Gly Cys Leu Ser Asn Ser Leu Gln Val
    420                 425                 430

aaa ggc aat act gtc tgt gat ggt agt gtt ttc act tct gac ttg atg      1581
Lys Gly Asn Thr Val Cys Asp Gly Ser Val Phe Thr Ser Asp Leu Met
435                 440                 445                 450

tct gac tgg agc atc tct tcg ttt tca acg ttc act tct cgt gat gaa      1629
Ser Asp Trp Ser Ile Ser Ser Phe Ser Thr Phe Thr Ser Arg Asp Glu
                455                 460                 465

caa gac ttc aga aat ggc ctt gcg gca tta gat gcc aac ata gct aga      1677
Gln Asp Phe Arg Asn Gly Leu Ala Ala Leu Asp Ala Asn Ile Ala Arg
            470                 475                 480

ctc cag aag tct tta agg act ggt ctt ctg gag aaa tgaa ttcagaagaa      1727
Leu Gln Lys Ser Leu Arg Thr Gly Leu Leu Glu Lys
        485                 490

aattcatcag gtgcttcttt ttaaaactag aacttggcta tattgaatgt gtatttttct   1787

ttagtgaaat gatgttttat gttattatgt gtgaagtaat atattgtaca agtaataaat   1847

gtattgttga gataaaaaaa aaaaa                                          1872

<210> 21
<211> 877
<212> DNA
<213> Homo sapiens

<220>
<221> CDS
<222> (21)..(797)

<400> 21
gaggcctgga ggacaccaac    atg aac aag ttg aaa tca tcg cag aag gat      50
                         Met Asn Lys Leu Lys Ser Ser Gln Lys Asp
                         1               5                   10

aaa gtt cgt cag ttt atg atc ttc aca caa tct agt gaa aaa aca gca      98
Lys Val Arg Gln Phe Met Ile Phe Thr Gln Ser Ser Glu Lys Thr Ala
                15                  20                  25

gta agt tgt ctt tct caa aat gac tgg aag tta gat gtt gca aca gat      146
Val Ser Cys Leu Ser Gln Asn Asp Trp Lys Leu Asp Val Ala Thr Asp

            30                  35                  40

aat ttt ttc caa aat cct gaa ctt tat ata cga gag agt gta aaa gga 194
Asn Phe Phe Gln Asn Pro Glu Leu Tyr Ile Arg Glu Ser Val Lys Gly
        45                  50                  55

tca ttg gac agg aag aag tta gaa cag ctg tac aat aga tac aaa gac 242
Ser Leu Asp Arg Lys Lys Leu Glu Gln Leu Tyr Asn Arg Tyr Lys Asp
    60                  65                  70

cct caa gat gag aat aaa att gga ata gat ggc ata cag cag ttc tgt 290
Pro Gln Asp Glu Asn Lys Ile Gly Ile Asp Gly Ile Gln Gln Phe Cys
75                  80                  85                  90

gat gac ctg gca ctc gat cca gcc agc att agt gtg ttg att att gca 338
Asp Asp Leu Ala Leu Asp Pro Ala Ser Ile Ser Val Leu Ile Ile Ala
                95                  100                 105

tgg aag ttc aga gca gca aca cag tgc gag ttc tcc aaa cag gag ttc 386
Trp Lys Phe Arg Ala Ala Thr Gln Cys Glu Phe Ser Lys Gln Glu Phe
            110                 115                 120

atg gat ggc atg aca gaa tta gga tgt gac agc ata gaa aaa cta aag 434
Met Asp Gly Met Thr Glu Leu Gly Cys Asp Ser Ile Glu Lys Leu Lys
        125                 130                 135

gcc cag ata ccc aag atg gaa caa gaa ttg aaa gaa cca gga cga ttt 482
Ala Gln Ile Pro Lys Met Glu Gln Glu Leu Lys Glu Pro Gly Arg Phe
    140                 145                 150

aag gat ttt tac cag ttt act ttt aat ttt gca aag aat cca gga caa 530
Lys Asp Phe Tyr Gln Phe Thr Phe Asn Phe Ala Lys Asn Pro Gly Gln
155                 160                 165                 170

aaa gga tta gat cta gaa atg gcc att gcc tac tgg aac tta gtg ctt 578
Lys Gly Leu Asp Leu Glu Met Ala Ile Ala Tyr Trp Asn Leu Val Leu
                175                 180                 185

aat gga aga ttt aaa ttc tta gac tta tgg aat aaa ttt ttg ttg gaa 626
Asn Gly Arg Phe Lys Phe Leu Asp Leu Trp Asn Lys Phe Leu Leu Glu
            190                 195                 200

cat cat aaa cga tca ata cca aaa gac act tgg aat ctt ctt tta gac 674
His His Lys Arg Ser Ile Pro Lys Asp Thr Trp Asn Leu Leu Leu Asp
        205                 210                 215

ttc agt acg atg att gca gat gac atg tct aat tat gat gaa gaa gga 722
Phe Ser Thr Met Ile Ala Asp Asp Met Ser Asn Tyr Asp Glu Glu Gly
    220                 225                 230

gca tgg cct gtt ctt att gat gac ttt gtg gaa ttt gca cgc cct caa 770
Ala Trp Pro Val Leu Ile Asp Asp Phe Val Glu Phe Ala Arg Pro Gln
235                 240                 245                 250

att gct ggg aca aaa agt aca aca gtg tagca ctaaaggaac cttctagaat 822
Ile Ala Gly Thr Lys Ser Thr Thr Val
                255

gtacatagtc tgtacaataa atacaacaga aaattgcaca gtcaaaaaaa aaaaa 877

<210> 22
<211> 993
<212> DNA
<213> Homo sapiens

<220>
<221> CDS
<222> (301)..(879)

<400> 22
ggcctgcaat ccctcggcgc ggggcaggtt ccgggctgct taggttggca ccggtccgtg      60

gtccccgggg gcgcagtcgc agcgctcccg ccctccaggc gtcagcgagt gcgcggtcca     120

gtgcggccgg aacctggcgc aactcctaga gcggtccttg gggagacgcg ggtcccagtc     180

ctgcggctcc tactggggag tgcgctggtc ggaagattgc tggactcgct gaagagagac     240

tacgcaggaa agccccagcc acccatcaaa tcagagagaa ggaatccacc ttcttacgct     300

atg gca ggt aag aaa gta ctc att gtc tat gca cac cag gaa ccc aag       348
Met Ala Gly Lys Lys Val Leu Ile Val Tyr Ala His Gln Glu Pro Lys
1               5                   10                  15

tct ttc aac gga tcc ttg aag aat gtg gct gta gat gaa ctg agc agg       396
Ser Phe Asn Gly Ser Leu Lys Asn Val Ala Val Asp Glu Leu Ser Arg
            20                  25                  30

cag ggc tgc acc gtc aca gtg tct gat ttg tat gcc atg aac ctt gag       444
Gln Gly Cys Thr Val Thr Val Ser Asp Leu Tyr Ala Met Asn Leu Glu
        35                  40                  45

ccg agg gcc aca gac aaa gat atc act ggt act ctt tct aat cct gag       492
Pro Arg Ala Thr Asp Lys Asp Ile Thr Gly Thr Leu Ser Asn Pro Glu
    50                  55                  60

gtt ttc aat tat gga gtg gaa acc cac gaa gcc tac aag caa agg tct       540
Val Phe Asn Tyr Gly Val Glu Thr His Glu Ala Tyr Lys Gln Arg Ser
65                  70                  75                  80

ctg gct agc gac atc act gat gag cag aaa aag gtt cgg gag gct gac       588
Leu Ala Ser Asp Ile Thr Asp Glu Gln Lys Lys Val Arg Glu Ala Asp
                85                  90                  95

cta gtg ata ttt cag ggt aaa cta gcg ctc ctt tcc gta acc acg gga       636
Leu Val Ile Phe Gln Gly Lys Leu Ala Leu Leu Ser Val Thr Thr Gly
            100                 105                 110

ggc acg gcc gag atg tac acg aag aca gga gtc aat gga gat tct cga       684
Gly Thr Ala Glu Met Tyr Thr Lys Thr Gly Val Asn Gly Asp Ser Arg
        115                 120                 125

tac ttc ctg tgg cca ctc cag cat ggc aca tta cac ttc tgt gga ttt       732
Tyr Phe Leu Trp Pro Leu Gln His Gly Thr Leu His Phe Cys Gly Phe
    130                 135                 140

aaa gtc ctt gcc cct cag atc agc ttt gct cct gaa att gca tcc gaa       780

Lys Val Leu Ala Pro Gln Ile Ser Phe Ala Pro Glu Ile Ala Ser Glu
145                 150                 155                 160

gaa gaa aga aag ggg atg gtg gct gcg tgg tcc cag agg ctg cag acc      828
Glu Glu Arg Lys Gly Met Val Ala Ala Trp Ser Gln Arg Leu Gln Thr
                165                 170                 175

atc tgg aag gaa gag ccc atc ccc tgc aca gcc cac tgg cac ttc ggg      876
Ile Trp Lys Glu Glu Pro Ile Pro Cys Thr Ala His Trp His Phe Gly
            180                 185                 190

caa taac tctgtggcac gtgggcatca cgtaagcagc acactaggag gcccaggcgc      933
Gln

aggcaaagag aagatggtgc tgtcatgaaa taaaattaca acatagctaa aaaaaaaaaa      993

<210> 23
<211> 2076
<212> DNA
<213> Homo sapiens

<220>
<221> CDS
<222> (491)..(1666)

<400> 23
cgaacctggc ttcgctaacg ccctcccagc tccctcgggc ctgacttccg gtttcctcgc      60

gcgtccctgg cgccgagccc gcggacagcg gcagcccctt ttccggctga gagctcatcc      120

acacttccaa tcactttccg gagtgcttcc cctccctccg gcccgtgctg gtcccgacgg      180

cgggcctggg tctcgcgcgc gtattgctgg gtaacgggcc ttctcccgcg tcggcccggc      240

ccctcctgcc tcggctcgtc cctccttcca gaacgtcccg ggctcctgcc gagtcagaag      300

aaatgggact ccctccgcga cgtgcccgga gcagctccct tcgctgtgga agcggcggtg      360

tcttcgaaga aaccggaagc ccgtggtgac ccctggcgac ccggtttgtt ttcggtccgt      420

ttccaaacac taaggaatcg aaactcggcg gccttggggg cggccctacg tagcctggct      480

tctggttgtc atg gat gca ctg gta gaa gat gat atc tgt att ctg aat      529
           Met Asp Ala Leu Val Glu Asp Asp Ile Cys Ile Leu Asn
           1               5                   10

cat gaa aaa gcc cat aag aga gat aca gtg act cca gtt tca ata tat      577
His Glu Lys Ala His Lys Arg Asp Thr Val Thr Pro Val Ser Ile Tyr
    15                  20                  25

tca gga gat gaa tct gtt gct tcc cat ttt gct ctt gtc act gca tat      625
Ser Gly Asp Glu Ser Val Ala Ser His Phe Ala Leu Val Thr Ala Tyr
30                  35                  40                  45

gaa gac atc aaa aaa cga ctt aag gat tca gag aaa gag aac tct ttg      673
Glu Asp Ile Lys Lys Arg Leu Lys Asp Ser Glu Lys Glu Asn Ser Leu

                50                  55                  60

tta aag aag aga ata aga ttt ttg gaa gaa aag cta ata gct cga ttt 721
Leu Lys Lys Arg Ile Arg Phe Leu Glu Glu Lys Leu Ile Ala Arg Phe
            65                  70                  75

gaa gaa gaa aca agt tcc gtg gga cga gaa caa gta aat aag gcc tat 769
Glu Glu Glu Thr Ser Ser Val Gly Arg Glu Gln Val Asn Lys Ala Tyr
        80                  85                  90

cat gca tat cga gag gtt tgc att gat aga gat aat ttg aag agc aaa 817
His Ala Tyr Arg Glu Val Cys Ile Asp Arg Asp Asn Leu Lys Ser Lys
    95                  100                 105

ctg gac aaa atg aat aaa gac aac tct gaa tct ttg aaa gta ttg aat 865
Leu Asp Lys Met Asn Lys Asp Asn Ser Glu Ser Leu Lys Val Leu Asn
110                 115                 120                 125

gag cag cta caa tct aaa gaa gta gaa ctc ctc cag ctg agg aca gag 913
Glu Gln Leu Gln Ser Lys Glu Val Glu Leu Leu Gln Leu Arg Thr Glu
                130                 135                 140

gtg gaa act cag cag gtg atg agg aat tta aat cca cct tca tca aac 961
Val Glu Thr Gln Gln Val Met Arg Asn Leu Asn Pro Pro Ser Ser Asn
            145                 150                 155

tgg gag gtg gaa aag ttg agc tgt gac ctg aag atc cat ggt ttg gaa 1009
Trp Glu Val Glu Lys Leu Ser Cys Asp Leu Lys Ile His Gly Leu Glu
        160                 165                 170

caa gag ctg gaa ctg atg agg aaa gaa tgt agc gat ctc aaa ata gaa 1057
Gln Glu Leu Glu Leu Met Arg Lys Glu Cys Ser Asp Leu Lys Ile Glu
    175                 180                 185

cta cag aaa gcc aaa caa acg gat cca tat cag gaa gac aat ctg aag 1105
Leu Gln Lys Ala Lys Gln Thr Asp Pro Tyr Gln Glu Asp Asn Leu Lys
190                 195                 200                 205

agc aga gat ctc caa aaa cta agc att tca agt gat aat atg cag cat 1153
Ser Arg Asp Leu Gln Lys Leu Ser Ile Ser Ser Asp Asn Met Gln His
                210                 215                 220

gca tac tgg gaa ctg aag aga gaa atg tct aat tta cat ctg gtg act 1201
Ala Tyr Trp Glu Leu Lys Arg Glu Met Ser Asn Leu His Leu Val Thr
            225                 230                 235

caa gta caa gct gaa cta cta aga aaa ctg aaa acc tca act gca atc 1249
Gln Val Gln Ala Glu Leu Leu Arg Lys Leu Lys Thr Ser Thr Ala Ile
        240                 245                 250

aag aaa gcc tgt gcc cct gta gga tgc agt gaa gac ctt gga aga gac 1297
Lys Lys Ala Cys Ala Pro Val Gly Cys Ser Glu Asp Leu Gly Arg Asp
    255                 260                 265

agc aca aaa ctg cac ttg atg aat ttt act gca aca tac aca aga cat 1345
Ser Thr Lys Leu His Leu Met Asn Phe Thr Ala Thr Tyr Thr Arg His
270                 275                 280                 285

ccc cct ctc tta cca aat ggc aaa gct ctt tgt cat acc aca tct tcc 1393
Pro Pro Leu Leu Pro Asn Gly Lys Ala Leu Cys His Thr Thr Ser Ser

290                 295                 300

cct tta cca gga gat gta aag gtt tta tca gag aaa gca atc ctc caa      1441
Pro Leu Pro Gly Asp Val Lys Val Leu Ser Glu Lys Ala Ile Leu Gln
            305                 310                 315

tca tgg aca gac aat gag aga tcc att cct aat gat ggt aca tgc ttt      1489
Ser Trp Thr Asp Asn Glu Arg Ser Ile Pro Asn Asp Gly Thr Cys Phe
        320                 325                 330

cag gaa cac agt tct tat ggc aga aat tct ctg gaa gac aat tcc tgg      1537
Gln Glu His Ser Ser Tyr Gly Arg Asn Ser Leu Glu Asp Asn Ser Trp
    335                 340                 345

gta ttt cca agt cct cct aaa tca agt gag aca gca ttt ggg gaa act      1585
Val Phe Pro Ser Pro Pro Lys Ser Ser Glu Thr Ala Phe Gly Glu Thr
350                 355                 360                 365

aaa act aaa act ttg cct tta ccc aac ctt cca cca ctg cat tac ttg      1633
Lys Thr Lys Thr Leu Pro Leu Pro Asn Leu Pro Pro Leu His Tyr Leu
                370                 375                 380

gat caa cat aat cag aac tgc ctt tat aag aat taatttgg aagagattca      1684
Asp Gln His Asn Gln Asn Cys Leu Tyr Lys Asn
            385                 390

cgatttcacc atgaggacac ttatctcttt cagtggtcct cccaagaaat tatttaacaa      1744

actgaaagga gattttgatt aaaattttgc agaggtcttc agtatctata tttgaacaca      1804

ctgtacaata gtacaaaaac caacatagtt ggttttctag tatgaaagag caccctctag      1864

ctccatattc taagaatctg aaatatgcta ctatactaat taataagtaa acttaaggtg      1924

tttaaaaaac tctgccttct atattaattg taaaattttg cctctcagaa gaatggaatt      1984

ggagattgta gacgtggttt tacaaaatgt gaaatgtcta aatatctgtt cataaaaata      2044

aaaggaaaac atgtttcttc aaaaaaaaaa aa                                  2076

<210> 24
<211> 734
<212> DNA
<213> Homo sapiens

<220>
<221> CDS
<222> (43)..(606)

<400> 24
   gcagttaagg cacacagagc accagctccc tcctgcctga ag atg ttc cac caa      54
                                                  Met Phe His Gln
                                                  1
   att tgg gca gct ctg ctc tac ttc tat ggt att atc ctt aac tcc atc      102
   Ile Trp Ala Ala Leu Leu Tyr Phe Tyr Gly Ile Ile Leu Asn Ser Ile
   5                   10                  15                  20
   tac cag tgc cct gag cac agt caa ctg aca act ctg ggc gtg gat ggg 150
   Tyr Gln Cys Pro Glu His Ser Gln Leu Thr Thr Leu Gly Val Asp Gly
   25 30 35
   aag gag ttc cca gag gtc cac ttg ggc cag tgg tac ttt atc gca ggg 198
   Lys Glu Phe Pro Glu Val His Leu Gly Gln Trp Tyr Phe Ile Ala Gly
   40 45 50
   gca gct ccc acc aag gag gag ttg gca act ttt gac cct gtg gac aac 246
   Ala Ala Pro Thr Lys Glu Glu Leu Ala Thr Phe Asp Pro Val Asp Asn
   55 60 65
   att gtc ttc aat atg gct gct ggc tct gcc ccg atg cag ctc cac ctt 294
   Ile Val Phe Asn Met Ala Ala Gly Ser Ala Pro Met Gln Leu His Leu
   70 75 80
   cgt gct acc atc cgc atg aaa gat ggg ctc tgt gtg ccc cgg aaa tgg 342
   Arg Ala Thr Ile Arg Met Lys Asp Gly Leu Cys Val Pro Arg Lys Trp
   85 90 95 100
   atc tac cac ctg act gaa ggg agc aca gat ctc aga act gaa ggc cgc 390
   Ile Tyr His Leu Thr Glu Gly Ser Thr Asp Leu Arg Thr Glu Gly Arg
   105 110 115
   cct gac atg aag act gag ctc ttt tcc agc tca tgc cca ggt gga atc 438
   Pro Asp Met Lys Thr Glu Leu Phe Ser Ser Ser Cys Pro Gly Gly Ile
   120 125 130
   atg ctg aat gag aca ggc cag ggt tac cag cgc ttt ctc ctc tac aat 486
   Met Leu Asn Glu Thr Gly Gln Gly Tyr Gln Arg Phe Leu Leu Tyr Asn
   135 140 145
   cgc tca cca cat cct ccc gaa aag tgt gtg gag gaa ttc aag tcc ctg 534
   Arg Ser Pro His Pro Pro Glu Lys Cys Val Glu Glu Phe Lys Ser Leu
   150 155 160
   act tcc tgc ctg gac tcc aaa gcc ttc tta ttg act cct agg aat caa 582
   Thr Ser Cys Leu Asp Ser Lys Ala Phe Leu Leu Thr Pro Arg Asn Gln
   165 170 175 180
   gag gcc tgt gag ctg tcc aat aac tgacctgtaa cttcatctaa gtccccagat 636
   Glu Ala Cys Glu Leu Ser Asn Asn
   185
   gggtacaatg ggagctgagt tgttggaggg agaagctgga gacttccagc tccagctccc 696
   actcaagata ataaagataa tttttcaaaa aaaaaaaa 734

<210> 25
<211> 2112
<212> DNA
<213> Homo sapiens

<220>
<221> CDS
<222> (452)..(2062)

<400> 25
tttaacctgg aatgacttaa atgtcatcct aacttctacc ctctccccaa atgaacggga 60

aagagttttt tctctagccc aatttcacac tgataactgc cagcttcatg agccagacct 120

ccatgaaggc attagagcag ttccccgaga agatcccaat ggaactatca ggcaaattcc 180

ccagcagctg aagactgaca ctgcccgatc gcctcagaag cctcctggac catcgcggat 240

gctttgggta actcttacag tggaggagct ccgtttccct ctgacctggg tcgggcgggc 300

agctgcggct gctgaggctc ggtggggccc ctccaagacg cgtgtccgca tctgcccgcc 360

gggcgtctgc ggggtgcagc gtccactgga gcgcgacagc ccctgggaca gaggaggaca 420

gtggcctcgc ttcccttctc cctcccgcgc g atg gcc tcg gcg ctg agc tat 472
Met Ala Ser Ala Leu Ser Tyr
1               5

gtc tcc aag ttc aag tcc ttc gtg atc ttg ttc gtc acc ccg ctc ctg 520
Val Ser Lys Phe Lys Ser Phe Val Ile Leu Phe Val Thr Pro Leu Leu
        10                  15                  20

ctg ctg cca ctc gtc att ctg atg ccc gcc aag ttt gtc agg tgt gcc 568
Leu Leu Pro Leu Val Ile Leu Met Pro Ala Lys Phe Val Arg Cys Ala
    25                  30                  35

tac gtc atc atc ctc atg gcc att tac tgg tgc aca gaa gtc atc cct 616
Tyr Val Ile Ile Leu Met Ala Ile Tyr Trp Cys Thr Glu Val Ile Pro
40                  45                  50                  55

ctg gct gtc acc tct ctc atg cct gtc ttg ctt ttc cca ctc ttc cag 664
Leu Ala Val Thr Ser Leu Met Pro Val Leu Leu Phe Pro Leu Phe Gln
                60                  65                  70

att ctg gac tcc agg cag gtg tgt gtc cag tac atg aag gac acc aac 712
Ile Leu Asp Ser Arg Gln Val Cys Val Gln Tyr Met Lys Asp Thr Asn
            75                  80                  85

atg ctg ttc ctg ggc ggc ctc atc gtg gcc gtg gct gtg gag cgc tgg 760
Met Leu Phe Leu Gly Gly Leu Ile Val Ala Val Ala Val Glu Arg Trp
        90                  95                  100

aac ctg cac aag agg atc gcc ctg cgc acg ctc ctc tgg gtg ggg gcc 808
Asn Leu His Lys Arg Ile Ala Leu Arg Thr Leu Leu Trp Val Gly Ala
    105                 110                 115

aag cct gca cgg ctg atg ctg ggc ttc atg ggc gtc aca gcc ctc ctg 856
Lys Pro Ala Arg Leu Met Leu Gly Phe Met Gly Val Thr Ala Leu Leu
120                 125                 130                 135

tcc atg tgg atc agt aac acg gca acc acg gcc atg atg gtg ccc atc 904
Ser Met Trp Ile Ser Asn Thr Ala Thr Thr Ala Met Met Val Pro Ile
                140                 145                 150

gtg gag gcc ata ttg cag cag atg gaa gcc aca agc gca gcc acc gag 952
Val Glu Ala Ile Leu Gln Gln Met Glu Ala Thr Ser Ala Ala Thr Glu
            155                 160                 165

gcc ggc ctg gag ctg gtg gac aag ggc aag gcc aag gag ctg cca ggg 1000
Ala Gly Leu Glu Leu Val Asp Lys Gly Lys Ala Lys Glu Leu Pro Gly
        170                 175                 180

agt caa gtg att ttt gaa ggc ccc act ctg ggg cag cag gaa gac caa 1048
Ser Gln Val Ile Phe Glu Gly Pro Thr Leu Gly Gln Gln Glu Asp Gln
    185                 190                 195

gag cgg aag agg ttg tgt aag gcc atg acc ctg tgc atc tgc tac gcg 1096
Glu Arg Lys Arg Leu Cys Lys Ala Met Thr Leu Cys Ile Cys Tyr Ala
200                 205                 210                 215

gcc agc atc ggg ggc acc gcc acc ctg acc ggg acg gga ccc aac gtg 1144
Ala Ser Ile Gly Gly Thr Ala Thr Leu Thr Gly Thr Gly Pro Asn Val
                220                 225                 230

gtg ctc ctg ggc cag atg aac gag ttg ttt cct gac agc aag gac ctc 1192
Val Leu Leu Gly Gln Met Asn Glu Leu Phe Pro Asp Ser Lys Asp Leu
            235                 240                 245

gtg aac ttt gct tcc tgg ttt gca ttt gcc ttt ccc aac atg ctg gtg 1240
Val Asn Phe Ala Ser Trp Phe Ala Phe Ala Phe Pro Asn Met Leu Val
        250                 255                 260

atg ctg ctg ttc gcc tgg ctg tgg ctc cag ttt gtt tac atg aga ttc 1288
Met Leu Leu Phe Ala Trp Leu Trp Leu Gln Phe Val Tyr Met Arg Phe
    265                 270                 275

aat ttt aaa aag tcc tgg ggc tgc ggg cta gag agc aag aaa aac gag 1336
Asn Phe Lys Lys Ser Trp Gly Cys Gly Leu Glu Ser Lys Lys Asn Glu
280                 285                 290                 295

aag gct gcc ctc aag gtg ctg cag gag gag tac cgg aag ctg ggg ccc 1384
Lys Ala Ala Leu Lys Val Leu Gln Glu Glu Tyr Arg Lys Leu Gly Pro
                300                 305                 310

ttg tcc ttc gcg gag atc aac gtg ctg atc tgc ttc ttc ctg ctg gtc 1432
Leu Ser Phe Ala Glu Ile Asn Val Leu Ile Cys Phe Phe Leu Leu Val
            315                 320                 325

atc ctg tgg ttc tcc cga gac ccc ggc ttc atg ccc ggc tgg ctg act 1480
Ile Leu Trp Phe Ser Arg Asp Pro Gly Phe Met Pro Gly Trp Leu Thr
        330                 335                 340

gtt gcc tgg gtg gag ggt gag aca aag tat gtc tcc gat gcc act gtg 1528
Val Ala Trp Val Glu Gly Glu Thr Lys Tyr Val Ser Asp Ala Thr Val
    345                 350                 355

gcc atc ttt gtg gcc acc ctg cta ttc att gtg cct tca cag aag ccc 1576
Ala Ile Phe Val Ala Thr Leu Leu Phe Ile Val Pro Ser Gln Lys Pro
360                 365                 370                 375

aag ttt aac ttc cgc agc cag act gag gaa gaa agg aaa act cca ttt 1624
Lys Phe Asn Phe Arg Ser Gln Thr Glu Glu Glu Arg Lys Thr Pro Phe
                380                 385                 390

tat ccc cct ccc ctg ctg gat tgg aag gta acc cag gag aaa gtg ccc 1672
Tyr Pro Pro Pro Leu Leu Asp Trp Lys Val Thr Gln Glu Lys Val Pro
            395                 400                 405

tgg ggc atc gtg ctg cta cta ggg ggc gga ttt gct ctg gct aaa gga     1720
Trp Gly Ile Val Leu Leu Leu Gly Gly Gly Phe Ala Leu Ala Lys Gly
        410                 415                 420

tcc gag gcc tcg ggg ctg tcc gtg tgg atg ggg aag cag atg gag ccc     1768
Ser Glu Ala Ser Gly Leu Ser Val Trp Met Gly Lys Gln Met Glu Pro
    425                 430                 435

ttg cac gca gtg ccc ccg gca gcc atc acc ttg atc ttg tcc ttg ctc     1816
Leu His Ala Val Pro Pro Ala Ala Ile Thr Leu Ile Leu Ser Leu Leu
440                 445                 450                 455

gtt gcc gtg ttc act gag tgc aca agc aac gtg gcc acc acc acc ttg     1864
Val Ala Val Phe Thr Glu Cys Thr Ser Asn Val Ala Thr Thr Thr Leu
                460                 465                 470

ttc ctg ccc atc ttt gcc tcc atg tct cgc tcc atc ggc ctc aat ccg     1912
Phe Leu Pro Ile Phe Ala Ser Met Ser Arg Ser Ile Gly Leu Asn Pro
            475                 480                 485

ctg tac atc atg ctg ccc tgt acc ctg agt gcc tcc ttt gcc ttc atg     1960
Leu Tyr Ile Met Leu Pro Cys Thr Leu Ser Ala Ser Phe Ala Phe Met
        490                 495                 500

ttg cct gtg gcc acc cct cca aat gcc atc gtg ttc acc tat ggg cac     2008
Leu Pro Val Ala Thr Pro Pro Asn Ala Ile Val Phe Thr Tyr Gly His
    505                 510                 515

ctc aag gtt gct gac atg gta aca cag ctg ttt tta ttt act ccc gtc     2056
Leu Lys Val Ala Asp Met Val Thr Gln Leu Phe Leu Phe Thr Pro Val
520                 525                 530                 535

gga cta taacgctgtt gtcataaggg atgccccatt tatgaatgac agagtttcaa     2112
Gly Leu

<210> 26
<211> 1961
<212> DNA
<213> Homo sapiens

<220>
<221> CDS
<222> (143)..(1843)

<400> 26
tgtgacgggc ggcccttcgt ctcaccttcc gtcctcgggc ccgcaggtcg cagggcggcc     60

tgcagctggg ccgcggccga ggaggcagcg cgacctccgc actattcttt caacttttaa     120

gaacaaatgc accttatagc tc atg gaa gaa aaa aca caa atc aag aca ttt     172
                         Met Glu Glu Lys Thr Gln Ile Lys Thr Phe
                         1               5                   10

ttg ggt tcc aag ttg cca aag tat gga aca aaa tct gta aga agt aca     220
Leu Gly Ser Lys Leu Pro Lys Tyr Gly Thr Lys Ser Val Arg Ser Thr

                    15                  20                  25

ttg cag cca atg cca aat ggg aca cct gtt aat tta tta gga act tcc      268
Leu Gln Pro Met Pro Asn Gly Thr Pro Val Asn Leu Leu Gly Thr Ser
                30                  35                  40

aag aat agt aat gtc aaa agt tac atc aaa aat aat ggc tct gat tgt      316
Lys Asn Ser Asn Val Lys Ser Tyr Ile Lys Asn Asn Gly Ser Asp Cys
            45                  50                  55

cca tca tct cat tca ttt aat tgg agg aaa gca aat aaa tat cag ctt      364
Pro Ser Ser His Ser Phe Asn Trp Arg Lys Ala Asn Lys Tyr Gln Leu
        60                  65                  70

tgt gca caa ggt gtc gaa gag cct aac aat act caa aat tca cat gat      412
Cys Ala Gln Gly Val Glu Glu Pro Asn Asn Thr Gln Asn Ser His Asp
 75                  80                  85                  90

aaa ata att gat cct gaa aaa cgt gtt cct act caa gga atg ttt gat      460
Lys Ile Ile Asp Pro Glu Lys Arg Val Pro Thr Gln Gly Met Phe Asp
                 95                 100                 105

aaa aat ggg ata aag gga ggt ttg aaa agt gtt tct tta ttc aca tca      508
Lys Asn Gly Ile Lys Gly Gly Leu Lys Ser Val Ser Leu Phe Thr Ser
            110                 115                 120

aag tta gca aag cca tcc act atg ttt gtg tca tct aca gag gag tta      556
Lys Leu Ala Lys Pro Ser Thr Met Phe Val Ser Ser Thr Glu Glu Leu
        125                 130                 135

aac caa aag tct ttt tct gga cca tct aat ttg ggt aaa ttc acc aaa      604
Asn Gln Lys Ser Phe Ser Gly Pro Ser Asn Leu Gly Lys Phe Thr Lys
    140                 145                 150

ggc aca tta tta gga agg act tca tat tct tcg atc aat act cca aaa      652
Gly Thr Leu Leu Gly Arg Thr Ser Tyr Ser Ser Ile Asn Thr Pro Lys
155                 160                 165                 170

tca cag ttg aat gga ttt tat gga aac cga tca gct ggt agc atg caa      700
Ser Gln Leu Asn Gly Phe Tyr Gly Asn Arg Ser Ala Gly Ser Met Gln
                175                 180                 185

agg cct aga gcg aac tcc tgt gcc acc aga agc agt tct gga gaa agc      748
Arg Pro Arg Ala Asn Ser Cys Ala Thr Arg Ser Ser Ser Gly Glu Ser
            190                 195                 200

tta gct caa tcc cca gac agt agt aaa tct att aat tgt gaa aaa atg      796
Leu Ala Gln Ser Pro Asp Ser Ser Lys Ser Ile Asn Cys Glu Lys Met
        205                 210                 215

gta agg tca caa agt ttt tca cat tcc att cag aat tca ttc ctt cca      844
Val Arg Ser Gln Ser Phe Ser His Ser Ile Gln Asn Ser Phe Leu Pro
    220                 225                 230

cct tca tct ata acc aga tca cat tcc ttt aat aga gct gtg gat ctt      892
Pro Ser Ser Ile Thr Arg Ser His Ser Phe Asn Arg Ala Val Asp Leu
235                 240                 245                 250

aca aag cct tat cag aac caa cag cta tcc att aga gtg cct cta cgg      940
Thr Lys Pro Tyr Gln Asn Gln Gln Leu Ser Ile Arg Val Pro Leu Arg

                255                 260                 265

tca agt atg cta aca aga aat tcc cgg cag cca gaa gta ctc aat ggg 988
Ser Ser Met Leu Thr Arg Asn Ser Arg Gln Pro Glu Val Leu Asn Gly
            270                 275                 280

aat gaa cat ttg ggg tat gga ttt aat agg cct tat gct gct ggt gga 1036
Asn Glu His Leu Gly Tyr Gly Phe Asn Arg Pro Tyr Ala Ala Gly Gly
        285                 290                 295

aag aag ttg gct tta cca aat ggc cca ggt gta act tct act tta ggt 1084
Lys Lys Leu Ala Leu Pro Asn Gly Pro Gly Val Thr Ser Thr Leu Gly
    300                 305                 310

tat aga atg gtt cat ccc tct cta ctg aaa tct agc cga tct cca ttt 1132
Tyr Arg Met Val His Pro Ser Leu Leu Lys Ser Ser Arg Ser Pro Phe
315                 320                 325                 330

tct ggg act atg aca gtt gat gga aat aaa aat tca cct gct gac aca 1180
Ser Gly Thr Met Thr Val Asp Gly Asn Lys Asn Ser Pro Ala Asp Thr
                335                 340                 345

tgt gta gag gaa gat gct aca gtt ttg gct aag gac aga gct gct aat 1228
Cys Val Glu Glu Asp Ala Thr Val Leu Ala Lys Asp Arg Ala Ala Asn
            350                 355                 360

aag gac caa gaa ctg att gaa aat gaa agt tat aga aca aaa aac aac 1276
Lys Asp Gln Glu Leu Ile Glu Asn Glu Ser Tyr Arg Thr Lys Asn Asn
        365                 370                 375

cag acc atg aaa cat gat gct aaa atg aga tac ctg agt gat gat gtg 1324
Gln Thr Met Lys His Asp Ala Lys Met Arg Tyr Leu Ser Asp Asp Val
    380                 385                 390

gat gac att tcc ttg tcg tct ttg tca tct tct gat aag aat gat tta 1372
Asp Asp Ile Ser Leu Ser Ser Leu Ser Ser Ser Asp Lys Asn Asp Leu
395                 400                 405                 410

agt gaa gac ttt agt gat gat ttt ata gat ata gaa gac tcc aac aga 1420
Ser Glu Asp Phe Ser Asp Asp Phe Ile Asp Ile Glu Asp Ser Asn Arg
                415                 420                 425

act aga ata act cca gag gaa atg tct ctc aaa gaa gag aaa cat gaa 1468
Thr Arg Ile Thr Pro Glu Glu Met Ser Leu Lys Glu Glu Lys His Glu
            430                 435                 440

aat ggg cca cca cag gat atg ttt gat tcc ccc aag gaa aat gaa aaa 1516
Asn Gly Pro Pro Gln Asp Met Phe Asp Ser Pro Lys Glu Asn Glu Lys
        445                 450                 455

gcc ttc agt aaa act gat gaa tgg ata gat ata agt gtc tct gac agg 1564
Ala Phe Ser Lys Thr Asp Glu Trp Ile Asp Ile Ser Val Ser Asp Arg
    460                 465                 470

agt gaa tgt aca aaa cat act tct ggg aat aat ttg gtt tca cca gat 1612
Ser Glu Cys Thr Lys His Thr Ser Gly Asn Asn Leu Val Ser Pro Asp
475                 480                 485                 490

aca gac tac aga gct ggt tct tcg ttt gaa ctc tct cca tct gat agc 1660
Thr Asp Tyr Arg Ala Gly Ser Ser Phe Glu Leu Ser Pro Ser Asp Ser

495                    500                    505

tct gat gga aca tac atg tgg gat gaa gaa ggc ttg gaa ccc att gga      1708
Ser Asp Gly Thr Tyr Met Trp Asp Glu Glu Gly Leu Glu Pro Ile Gly
            510                    515                    520

aat gtc cat cca gtt ggg agc tat gag tcc tct gaa atg aac agc ata      1756
Asn Val His Pro Val Gly Ser Tyr Glu Ser Ser Glu Met Asn Ser Ile
        525                    530                    535

gta tgt atg gat tta tat act ctt gga ata ttt tgt tta ccc tac tat      1804
Val Cys Met Asp Leu Tyr Thr Leu Gly Ile Phe Cys Leu Pro Tyr Tyr
    540                    545                    550

aga gag act tgt gat atg att gat ttt gta aaa aat tta tgaattaact      1853
Arg Glu Thr Cys Asp Met Ile Asp Phe Val Lys Asn Leu
555                    560                    565

ttactacttt gagaaatgga aattcatatt tgttaagagc ctataaaata tctgactcta   1913

atatgaaata aagcatggag aatatatgac atttgaaaaa aaaaaaaa                1961

<210> 27
<211> 751
<212> DNA
<213> Homo sapiens

<220>
<221> CDS
<222> (44)..(520)

<400> 27
cgcgtccgca cgtaacggag tggccaacgg cctgcagagc aac atg ccc aag ttt      55
                                                Met Pro Lys Phe
                                                1

tat tgt gac tac tgc gat aca tac ctc acc cat gac tct cca tct gtg      103
Tyr Cys Asp Tyr Cys Asp Thr Tyr Leu Thr His Asp Ser Pro Ser Val
5                   10                  15                  20

aga aag aca cac tgc agt gga agg aaa cac aaa gag aat gtg aaa gac      151
Arg Lys Thr His Cys Ser Gly Arg Lys His Lys Glu Asn Val Lys Asp
                25                  30                  35

tat tat cag aaa tgg atg gaa gag cag gct cag agc ctg att gac aaa      199
Tyr Tyr Gln Lys Trp Met Glu Glu Gln Ala Gln Ser Leu Ile Asp Lys
            40                  45                  50

aca acg gct gca ttt caa caa gga aag ata cct cct act cca ttc tct      247
Thr Thr Ala Ala Phe Gln Gln Gly Lys Ile Pro Pro Thr Pro Phe Ser
        55                  60                  65

gct cct cct cct gca ggg gcg atg ata cca cct ccc ccc agc ctt ccg      295
Ala Pro Pro Pro Ala Gly Ala Met Ile Pro Pro Pro Pro Ser Leu Pro
    70                  75                  80

ggt cct cct cgc cct ggt atg atg cca gca ccc cat atg ggg ggc cct      343

Gly Pro Pro Arg Pro Gly Met Met Pro Ala Pro His Met Gly Gly Pro
85                  90                  95                  100

ccc atg atg cca atg atg ggc cct cct cct cct ggg atg atg cca gtg      391
Pro Met Met Pro Met Met Gly Pro Pro Pro Pro Gly Met Met Pro Val
                105                 110                 115

gga cct gct cct gga atg agg ccg ccc atg gga ggc cat atg cca atg      439
Gly Pro Ala Pro Gly Met Arg Pro Pro Met Gly Gly His Met Pro Met
            120                 125                 130

atg cct ggg ccc cca atg atg aga cct cct gcc cgt ccc atg atg gtg      487
Met Pro Gly Pro Pro Met Met Arg Pro Pro Ala Arg Pro Met Met Val
        135                 140                 145

ccc act cgg ccc gga atg act cga cca gac aga taaggata gaggggaggc      538
Pro Thr Arg Pro Gly Met Thr Arg Pro Asp Arg
    150                 155

cttattgtat cggttttata ttacctgttc tgcttcacca ggagatcatg ctgctgtgat      598

actgagtttt ctaaacagca taaggaagac ttgctcccct gtcctatgaa agagaatagt      658

tttggagggg agaagtggga caaaaaagat gcagttttcc tttgtattgg gaaatgtgaa      718

aataaaattg tcaactcttt caaaaaaaaa aaa                                 751

<210> 28
<211> 3075
<212> DNA
<213> Homo sapiens

<220>
<221> CDS
<222> (69)..(2786)

<400> 28
ggtgggttta tctcaaggcc tgagtagccg gtaacaaacg agggttcccg ggattggacc      60

gacgcacc atg cct ctg cga ctt gat atc aaa aga aag cta act gct aga      110
         Met Pro Leu Arg Leu Asp Ile Lys Arg Lys Leu Thr Ala Arg
         1               5                   10

tct gat cga gtt aag agt gtg gat ctg cat cct aca gag cca tgg atg      158
Ser Asp Arg Val Lys Ser Val Asp Leu His Pro Thr Glu Pro Trp Met
15                  20                  25                  30

ttg gca agt ctt tac aat ggc agt gtg tgt gtt tgg aat cat gaa aca      206
Leu Ala Ser Leu Tyr Asn Gly Ser Val Cys Val Trp Asn His Glu Thr
                35                  40                  45

cag aca ctg gtg aag aca ttt gaa gta tgt gat ctt cct gtt cga gct      254
Gln Thr Leu Val Lys Thr Phe Glu Val Cys Asp Leu Pro Val Arg Ala
            50                  55                  60

gca aag ttt gtt gca agg aag aat tgg gtt gtg aca gga gcg gat gac      302
Ala Lys Phe Val Ala Arg Lys Asn Trp Val Val Thr Gly Ala Asp Asp

        65                  70                  75

atg cag att aga gtg ttc aat tac aat act ctg gag aga gtt cat atg 350
Met Gln Ile Arg Val Phe Asn Tyr Asn Thr Leu Glu Arg Val His Met
    80                  85                  90

ttt gaa gca cac tca gac tac att cgc tgt att gct gtt cat cca acc 398
Phe Glu Ala His Ser Asp Tyr Ile Arg Cys Ile Ala Val His Pro Thr
95                  100                 105                 110

cag cct ttc att cta act agc agt gat gac atg ctt att aag ctc tgg 446
Gln Pro Phe Ile Leu Thr Ser Ser Asp Asp Met Leu Ile Lys Leu Trp
                115                 120                 125

gac tgg gat aaa aaa tgg tct tgc tca caa gtg ttt gaa gga cac acc 494
Asp Trp Asp Lys Lys Trp Ser Cys Ser Gln Val Phe Glu Gly His Thr
            130                 135                 140

cat tat gtt atg cag att gtg atc aac ccc aaa gat aac aat cag ttt 542
His Tyr Val Met Gln Ile Val Ile Asn Pro Lys Asp Asn Asn Gln Phe
        145                 150                 155

gcc agt gcc tct ttg gac agg act atc aag gtg tgg cag ttg ggc tct 590
Ala Ser Ala Ser Leu Asp Arg Thr Ile Lys Val Trp Gln Leu Gly Ser
    160                 165                 170

tcg tca cca aac ttc act ttg gaa gga cat gag aaa ggc gtg aat tgc 638
Ser Ser Pro Asn Phe Thr Leu Glu Gly His Glu Lys Gly Val Asn Cys
175                 180                 185                 190

att gat tac tac agt ggt ggg gac aag cca tac ctc att tca ggt gca 686
Ile Asp Tyr Tyr Ser Gly Gly Asp Lys Pro Tyr Leu Ile Ser Gly Ala
                195                 200                 205

gat gac cgt ctt gtt aaa ata tgg gat tat cag aat aaa aca tgt gtg 734
Asp Asp Arg Leu Val Lys Ile Trp Asp Tyr Gln Asn Lys Thr Cys Val
            210                 215                 220

cag aca ctg gaa gga cat gcc caa aat gtg tct tgt gcc agc ttt cat 782
Gln Thr Leu Glu Gly His Ala Gln Asn Val Ser Cys Ala Ser Phe His
        225                 230                 235

cct gag ttg cca atc att atc aca ggt tca gaa gat gga aca gta cgt 830
Pro Glu Leu Pro Ile Ile Ile Thr Gly Ser Glu Asp Gly Thr Val Arg
    240                 245                 250

att tgg cat tca agc acc tac cgg ctt gag agc aca ctg aat tat gga 878
Ile Trp His Ser Ser Thr Tyr Arg Leu Glu Ser Thr Leu Asn Tyr Gly
255                 260                 265                 270

atg gag agg gta tgg tgc gtg gcc agt cta aga ggg tca aac aat gtc 926
Met Glu Arg Val Trp Cys Val Ala Ser Leu Arg Gly Ser Asn Asn Val
                275                 280                 285

gct ttg ggc tat gat gaa ggg agc atc att gtt aag ctt ggt cgg gag 974
Ala Leu Gly Tyr Asp Glu Gly Ser Ile Ile Val Lys Leu Gly Arg Glu
            290                 295                 300

gaa cct gcc atg tcc atg gat gcc aat gga aag ata att tgg gcc aag 1022
Glu Pro Ala Met Ser Met Asp Ala Asn Gly Lys Ile Ile Trp Ala Lys

        305                 310                 315

cat tca gaa gtc cag cag gcc aac cta aaa gca atg gga gat gct gaa 1070
His Ser Glu Val Gln Gln Ala Asn Leu Lys Ala Met Gly Asp Ala Glu
    320                 325                 330

att aaa gat ggt gaa aga ttg cca ctg gca gta aag gat atg ggc agt 1118
Ile Lys Asp Gly Glu Arg Leu Pro Leu Ala Val Lys Asp Met Gly Ser
335                 340                 345                 350

tgt gaa ata tac cct cag act att cag cac aat cct aat ggg cgg ttt 1166
Cys Glu Ile Tyr Pro Gln Thr Ile Gln His Asn Pro Asn Gly Arg Phe
                355                 360                 365

gtg gtg gtg tgt ggt gat ggg gag tat atc atc tac aca gca atg gca 1214
Val Val Val Cys Gly Asp Gly Glu Tyr Ile Ile Tyr Thr Ala Met Ala
            370                 375                 380

ttg aga aac aag agc ttt gga tct gct cag gag ttt gca tgg gcc cac 1262
Leu Arg Asn Lys Ser Phe Gly Ser Ala Gln Glu Phe Ala Trp Ala His
        385                 390                 395

gat tct tca gag tat gca ata aga gag agc aac agc att gta aag ata 1310
Asp Ser Ser Glu Tyr Ala Ile Arg Glu Ser Asn Ser Ile Val Lys Ile
    400                 405                 410

ttt aag aac ttt aag gaa aaa aaa tca ttt aaa cca gat ttt gga gca 1358
Phe Lys Asn Phe Lys Glu Lys Lys Ser Phe Lys Pro Asp Phe Gly Ala
415                 420                 425                 430

gaa agt atc tac ggc ggc ttc tta ttg gga gtc aga tct gta aat ggc 1406
Glu Ser Ile Tyr Gly Gly Phe Leu Leu Gly Val Arg Ser Val Asn Gly
                435                 440                 445

tta gcc ttc tat gac tgg gac aat aca gaa ctc ata cga aga att gaa 1454
Leu Ala Phe Tyr Asp Trp Asp Asn Thr Glu Leu Ile Arg Arg Ile Glu
            450                 455                 460

att cag ccc aaa cat att ttc tgg tct gac tct gga gag cta gtc tgt 1502
Ile Gln Pro Lys His Ile Phe Trp Ser Asp Ser Gly Glu Leu Val Cys
        465                 470                 475

att gct act gag gaa tca ttt ttt atc ctt aag tat ctg tca gaa aaa 1550
Ile Ala Thr Glu Glu Ser Phe Phe Ile Leu Lys Tyr Leu Ser Glu Lys
    480                 485                 490

gtc ttg gct gca cag gaa aca cat gag gga gtt act gaa gat ggc att 1598
Val Leu Ala Ala Gln Glu Thr His Glu Gly Val Thr Glu Asp Gly Ile
495                 500                 505                 510

gaa gat gcc ttt gag gtt ctt ggt gag att cag gaa att gtg aaa aca 1646
Glu Asp Ala Phe Glu Val Leu Gly Glu Ile Gln Glu Ile Val Lys Thr
                515                 520                 525

ggg ctt tgg gta ggc gat tgc ttc att tac aca agt tct gtg aac aga 1694
Gly Leu Trp Val Gly Asp Cys Phe Ile Tyr Thr Ser Ser Val Asn Arg
            530                 535                 540

tta aat tat tat gtt gga gga gaa ata gtc acc att gcc cac ttg gac 1742
Leu Asn Tyr Tyr Val Gly Gly Glu Ile Val Thr Ile Ala His Leu Asp

545                 550                 555

agg acg atg tat ctc cta ggc tac att cct aaa gac aac agg ctt tat      1790
Arg Thr Met Tyr Leu Leu Gly Tyr Ile Pro Lys Asp Asn Arg Leu Tyr
    560                 565                 570

ctg ggg gat aaa gaa ttg aac atc att agc tat tcc ctg ctg gtt tca      1838
Leu Gly Asp Lys Glu Leu Asn Ile Ile Ser Tyr Ser Leu Leu Val Ser
575                 580                 585                 590

gtc ctg gaa tac cag aca gct gtc atg cgg agg gac ttt agc atg gct      1886
Val Leu Glu Tyr Gln Thr Ala Val Met Arg Arg Asp Phe Ser Met Ala
                595                 600                 605

gat aag gtc ctt cct acc att cca aaa gaa cag agg acc aga gtt gca      1934
Asp Lys Val Leu Pro Thr Ile Pro Lys Glu Gln Arg Thr Arg Val Ala
            610                 615                 620

cac ttt ttg gaa aag cag ggc ttc aag cag caa gct ctt aca gta tcc      1982
His Phe Leu Glu Lys Gln Gly Phe Lys Gln Gln Ala Leu Thr Val Ser
        625                 630                 635

aca gat cct gag cat cgt ttt gag ctt gct ctt cag ctt gga gag tta      2030
Thr Asp Pro Glu His Arg Phe Glu Leu Ala Leu Gln Leu Gly Glu Leu
    640                 645                 650

aaa att gca tac cag tta gca gtg gaa gca gag tca gaa cag aag tgg      2078
Lys Ile Ala Tyr Gln Leu Ala Val Glu Ala Glu Ser Glu Gln Lys Trp
655                 660                 665                 670

aaa caa ctt gct gaa ctt gcc att agt aaa tgt cag ttt ggc cta gcc      2126
Lys Gln Leu Ala Glu Leu Ala Ile Ser Lys Cys Gln Phe Gly Leu Ala
                675                 680                 685

cag gag tgc ctg cat cat gca cag gat tat ggg ggc ctg ctg ctt ttg      2174
Gln Glu Cys Leu His His Ala Gln Asp Tyr Gly Gly Leu Leu Leu Leu
            690                 695                 700

gcc act gcc tct gga aat gct aat atg gtg aac aag cta gca gag ggt      2222
Ala Thr Ala Ser Gly Asn Ala Asn Met Val Asn Lys Leu Ala Glu Gly
        705                 710                 715

gcg gag aga gat ggc aaa aat aat gtg gca ttc atg agc tac ttt tta      2270
Ala Glu Arg Asp Gly Lys Asn Asn Val Ala Phe Met Ser Tyr Phe Leu
    720                 725                 730

cag ggc aag gtt gat gcc tgc cta gag ctc tta att aga act gga cgg      2318
Gln Gly Lys Val Asp Ala Cys Leu Glu Leu Leu Ile Arg Thr Gly Arg
735                 740                 745                 750

ctg cca gaa gct gcc ttc ttg gcc cga act tac tta ccc agt cag gtt      2366
Leu Pro Glu Ala Ala Phe Leu Ala Arg Thr Tyr Leu Pro Ser Gln Val
                755                 760                 765

tca agg gta gtg aaa ctc tgg aga gag aat ctc tca aaa gtc aat cag      2414
Ser Arg Val Val Lys Leu Trp Arg Glu Asn Leu Ser Lys Val Asn Gln
            770                 775                 780

aaa gca gca gaa tcc ctt gct gac cca aca gag tat gaa aac ctg ttc      2462
Lys Ala Ala Glu Ser Leu Ala Asp Pro Thr Glu Tyr Glu Asn Leu Phe

        785                 790                 795

cct gga tta aaa gaa gcc ttt gtt gtt gaa gaa tgg gtg aag gaa aca 2510
Pro Gly Leu Lys Glu Ala Phe Val Val Glu Glu Trp Val Lys Glu Thr
    800                 805                 810

cat gct gat ctg tgg cca gcc aaa caa tac cca ctt gtc acg cca aat 2558
His Ala Asp Leu Trp Pro Ala Lys Gln Tyr Pro Leu Val Thr Pro Asn
815                 820                 825                 830

gaa gag aga aat gtc atg gaa gag gga aaa gac ttt cag ccc tca aga 2606
Glu Glu Arg Asn Val Met Glu Glu Gly Lys Asp Phe Gln Pro Ser Arg
                835                 840                 845

tct aca gct caa cag gaa ctt gat ggg aaa cct gct tct cct act ccg 2654
Ser Thr Ala Gln Gln Glu Leu Asp Gly Lys Pro Ala Ser Pro Thr Pro
            850                 855                 860

gtt att gtg gcc tcc cac aca gcc aac aaa gaa gaa aag agt tta ctc 2702
Val Ile Val Ala Ser His Thr Ala Asn Lys Glu Glu Lys Ser Leu Leu
        865                 870                 875

gaa cta gaa gta gat ttg gat aat ttg gaa tta gaa gat att gac aca 2750
Glu Leu Glu Val Asp Leu Asp Asn Leu Glu Leu Glu Asp Ile Asp Thr
    880                 885                 890

aca gat atc aat ctg gat gaa gat att ttg gat gat tgac tgtaatgctt 2800
Thr Asp Ile Asn Leu Asp Glu Asp Ile Leu Asp Asp
895                 900                 905

tccatttacc tgactaaaca gatcattatt atatataggt attgattgct accctgacca 2860

cagtgctttg gactatgaga aacttcttag atttttatat gtaaatgctg tggaccactg 2920

ggagcacaat gcccacatca tcttaagaag agtttatgtg cagcatttaa atcactgtgt 2980

tttccttgtt aactaaaaca gacatgggct ttgatttttt tcatactatt agaccatatc 3040

tcataaaacc ttttgaatta ataaaaaaaa aaaaa 3075

<210> 29
<211> 2695
<212> DNA
<213> Homo sapiens

<220>
<221> CDS
<222> (1)..(2649)

<400> 29
atg aat aca tca caa tta tta gaa ata gcc aac cag gtg ttt gta aac 48
agg gct gca gta agc ctt gag gaa aac cgc aaa gag aat gga cat cag 96
gcc cgg cga aac acc gac ctg gtt gtc agc tgc agc aat cag agg ggt 144
cag gag tca ctg gaa aag ttg tta ggc cgg tat ttc tac atc tcg cat 192
ttg tca gcc ctc gcc aaa acc atg agg cag cgg ttt gtt acc tgc cga 240
cac cat aat gcg agg caa ggt cca gct gtt ccg ccc ggc ata caa gct 288
tat gca gca gcc ccc att gaa gat ctg cag gcc att agg aac aat atc 336
aca gcg ggt gtt tac aca ccc tgc gat att gga ggt aat atc atc ctc 384
tgc ccc ctg gca tat tac caa cga tat caa aca ggg gtg gtg tac acc 432
cct tgt gat att ggg agt att atc atc ctc tcc acc tcc ggg tgt cca 480
agt cac aca gag cca cgg aat ctc aca ggt gtc tca gaa ttc ctc ctc 528
ctg gga ctc tca gag gat cca gaa ctg cag cct gtc ctc cct ggg ctg 576
tcc ctg tcc atg tat ctg ctc acg gtg ctg agg aac ctg ctc atc atc 624
ctg gct gtc agc tct gac tcc cac ctc cac acc ccc atg tac ttc ttc 672
ctc tcc aac ccg tca tgg gct gac atc gct ttc acc tcg gcc aca gtt 720
ccc aag atg att gtg gac atg cag agt ggt gtg gtg gtg tca gtg atg 768
tac act gtg gtc acc ccc atg ctg aac cct ttc atc tac tgc ctg aga 816
aac agg gac att caa agc gcc ctg tgg agg ctg cgc agc aga aca gtc 864
gaa tct cat gat ctg ttc cat cct ttt tct tgt gtg ggt atc cac tgt 912
cag gtc tct gag ctt gat gct gtc atg tcc ctt gac acc gct tcc ggg 960
gct ttt cgg tgg atg gct ttt ggg gtg gaa gtg gca gcc cca gct tac 1008
cag cca tca tgc tca tgt ccg gga tct cag tcc tct ggg gaa ggg gcc 1056
aga aaa ggc tgg agc agc cga agt ctt tcg gaa aca gta tct gcg gta 1104
tat tca ggg ctg act cag gaa aga aga tgc cag ccc cag agt ccc acc 1152
tcc acc gtt ggt tcc atc ata atg aat gga tcc aat atg gca aat aca 1200
tca ccg agt gta aaa tcc aaa gag gac cag ggg tta agt ggg cac gat 1248
gaa aag gaa aac cca ttt gca gag tac atg tgg atg gag aat gaa gag 1296
gat ttc aac aga cag gtg gag gag gaa ctg cag gag caa gac ttc ttg 1344
gac cgc tgc ttc caa gag atg ctg gat gaa gaa gac caa gac tgg ttt 1392
att ccc tca cga gac ctg cct cag gcc atg gga cag ttg caa cag cag 1440
tta aat gga ctg tca gtc agt gaa ggt cat gat tct gaa gat att ttg 1488
gtc cat ggg cgc agg tcc ctg agc cca cag ctt cgc ggg ggt ctg gcc 1536
gag cat gcg tca caa cct acg cgg cca aga gca gtg cgc acg cgc aac     1584
cta acg cgg gac tgc cag caa ctt ccg ggc gtt tac agg cag gca gga     1632
tcc agg agg acg gga ggg gcc gct gcg gac cgc agt cgc tcc acc tgg     1680
agg aga cac cag aag gaa gac agc ctg agg gac gca gcc atc ccc ggc     1728
tcc tac cgg cgc ccc gcc ccg cgc atg cgc acg cgc aca ggg agt cag     1776
ctg gct gcg cgg gag gtc acg gga agt ggg gcg gtg ccc aga cag ctg     1824
gag gga agg agg tgt cag gcg ggg aga gac gca aac ggc ggg acc agc     1872
agc gac ggt agc agc agc atg gcc gcg atc tat ggg ggt gta gag ggg     1920
gga ggc aca cga tcc gag gtc ctt tta gtc tca gag gat ggg aag atc     1968
ctg gca gaa gca gat gga ctg agc aca aac cac tgg ctg atc ggg aca     2016
gac aag tgt gtg gag agg atc aat gag atg gtg aac agg gcc aaa cgg     2064
aaa gca ggg gtg gat cct ctg gta ccg ctg cga agc ttg ggc cta tct     2112
ctg agc ggt ggg gac cag gag gac gcg ggg agg atc ctg atc gag gag     2160
ctg agg gac cga ttt ccc tac ctg agt gaa agc tac tta atc acc acc     2208
gat gcc gcc ggc tcc atc gcc aca gct aca ccg gat ggt gga gtt gtg     2256
ctc ata tct gga aca ggc tcc aac tgc agg ctc atc aac cct gat ggc 2304
tcc gag agt ggc tgc ggc ggc tgg ggc cat atg atg ggt gat gag ggt 2352
tca gcc tac tgg atc gca cac caa gca gtg aaa ata gtg ttt gac tcc 2400
att gac aac cta gag gcg gct cct cat gat atc ggc tac gtc aaa cag 2448
gcc atg ttc cac tat ttc cag gtg cca gat cgg cta ggg ata ctc act 2496
cac ctg tat agg gac ttt gat aaa tgc agg ttt gct ggg ttt tgc cgg 2544
aaa att gca gaa ggt gct cag cag gga gac ccc ctt tcc cgc tat atc 2592
ttc agg aag agc tgg gga gat gct ggg cag gca cat cgt agc agt gtt 2640
gcc cga gat tgacccggtc ttgttccagg gcaagattgg actccccatc ctgtgc 2695

<210> 30
<211> 742
<212> DNA
<213> Homo sapiens

<220>
<221> CDS
<222> (338)..(532)

<400> 30
acggagggtg ggagcagtgg atggtgggag ggaatggaga taataaatgg aacaacaact 60

atcttattaa aataagataa taacagtcaa aactaataca aagcatataa aaccaggtaa 120

gatgataaac atgaatgccg aaaactgctt aagaaaaggg tagcagggag ttattttctg 180

agtagatgac atttatgcta aacgtggaac aaggagatgg agccaaccct gaaaattctg 240

ggagaagagg acagaaggca gagggaagag caagagcaaa aattctgaaa caggagaaat 300

tgtgagcata tcagcattaa gtaccactga ggtggca atg cac act tca acc tct     355
                                      Met His Thr Ser Thr Ser
                                      1               5

tct tca gtc aca aag agt tac atc tca tca cag aca aat gga gaa acg     403
Ser Ser Val Thr Lys Ser Tyr Ile Ser Ser Gln Thr Asn Gly Glu Thr
            10                  15                  20

gga caa ctt gtc cat cgt ttc act gta cca gct cct gta gtg ata ata     451
Gly Gln Leu Val His Arg Phe Thr Val Pro Ala Pro Val Val Ile Ile
        25                  30                  35

ctc att att ttg tgt gtg atg gct ggt att att gga acg atc ctc tta     499
Leu Ile Ile Leu Cys Val Met Ala Gly Ile Ile Gly Thr Ile Leu Leu
    40                  45                  50

att tct tac agt att cgc cga ctg ata aag gca tgaggatg tggcctgcat     550
Ile Ser Tyr Ser Ile Arg Arg Leu Ile Lys Ala
55                  60                  65

gctgcctgat cttgcctaga accggctgca cctgctgttc tcttgtttat gcaaactggc     610

tgcacctgct attcctttgc ttatgcccct acccctggct atcctaattc cctgttctcc     670

tgcctcacta ttactgtatt ctctacttct aaataaaaat aaaacaaaat acaaaccgtt     730

aaaaaaaaaa aa                                                         742

<210> 31
<211> 1474
<212> DNA
<213> Homo sapiens

<220>
<221> CDS
<222> (274)..(930)

<400> 31
aaggatcctt aattaaatta atccccctcc cccggagctt tctcgcgggc ttgcagctgc      60

ggcaagtgct ggcggcggct gctcgcgcaa gtcagctggc gtgggaacta ccctttgtag     120

ctgagaacgg cttgtttatt gctacaaaga ctctattgac attggtagct tcagcggcag     180

cagcttctta cggtataaag ctgttgcttc ctgaagaggc tacaagcatc cttccctagg     240

actgctgtaa gctttgagcc tctagcagga gac atg cct cgg gga cga aag agt     294
                                     Met Pro Arg Gly Arg Lys Ser
                                     1               5

cgg cgc cgc cgt aat gcg aga gcc gca gaa gag aac cgc aac aat cgc     342
Arg Arg Arg Arg Asn Ala Arg Ala Ala Glu Glu Asn Arg Asn Asn Arg
        10                  15                  20

aaa atc cag gcc tca gag gcc tcc gag acc cct atg gcc gcc tct gtg     390
Lys Ile Gln Ala Ser Glu Ala Ser Glu Thr Pro Met Ala Ala Ser Val

25                  30                  35

gta gcg agc acc ccc gaa gac gac ctg agc ggc ccc gag gaa gac ccg      438
Val Ala Ser Thr Pro Glu Asp Asp Leu Ser Gly Pro Glu Glu Asp Pro
 40                  45                  50                  55

agc act cca gag gag gcc tct acc acc cct gaa gaa gcc tcg agc act      486
Ser Thr Pro Glu Glu Ala Ser Thr Thr Pro Glu Glu Ala Ser Ser Thr
                 60                  65                  70

gcc caa gca caa aag cct tca gtg ccc cgg agc aat ttt cag ggc acc      534
Ala Gln Ala Gln Lys Pro Ser Val Pro Arg Ser Asn Phe Gln Gly Thr
             75                  80                  85

aag aaa agt ctc ctg atg tct ata tta gcg ctc atc ttc atc atg ggc      582
Lys Lys Ser Leu Leu Met Ser Ile Leu Ala Leu Ile Phe Ile Met Gly
         90                  95                 100

aac agc gcc aag gaa gct ctg gtc tgg aaa gtg ctg ggg aag tta gga      630
Asn Ser Ala Lys Glu Ala Leu Val Trp Lys Val Leu Gly Lys Leu Gly
    105                 110                 115

atg cag cct gga cgt cag cac agc atc ttt gga gat ccg aag aag atc      678

120                 125                 130                 135

gtc aca gaa gag ttt gtg cgc aga ggg tac ctg att tat aaa ccg gtg      726

                140                 145                 150

ccc cgt agc agt ccg gtg gag tat gag ttc ttc tgg ggg ccc cga gca      774

            155                 160                 165

cac gtg gaa tcg agc aaa ctg aaa gtc atg cat ttt gtg gca agg gtt      822

        170                 175                 180

cgt aac cga tgc tct aaa gac tgg cct tgt aat tat gac tgg gat tcg      870

    185                 190                 195

gac gat gat gca gag gtt gag gct atc ctc aat tca ggt gct agg ggt      918

200                 205                 210                 215

tat tcc gcc cct taa gtagatctga ggcagacccct tgggggtgta aaagagagtc      973


acaggtaccc caaggagtag atgccagggt cctaagttga aaatgatgtc gattgggggc      1033

gggggacact gtatttgata tttgtgatca gtgatcattg ttcaactgcg aaatagagtg      1093

tttgcttttg ataatggaaa attgtattcg ttttaaaatt ccgtttgttg agaataacaa      1153

tatgtttaaa aatataattg aacaaatttg tttctttgtt gcctgtcagg aacagttagt      1213

agaacagttt tgctagcgtt ctaaaatgaa gtcgttccat cataatctat gatcttgtac      1273

ggggggggga ggggtaagct gttcttttga agttgaaata cccagtaaaa tgttgaagaa   1333

ggatggagga tttcttcata tctgacgttt ctgaaaccct ttgtgtctgc tgttgtgtga   1393

agattgacat ttaccatgat tttccttagt tactgcagaa catagagaaa aataaaagcc   1453

taacgaataa aaaaaaaaa a                                              1474

<210> 32
<211> 592
<212> DNA
<213> Homo sapiens

<220>
<221> CDS
<222> (86)..(460)

<400> 32
gtgtcgccgc tgtgccgcta gcggtgcccc gcctgctgcg gtggcaccag ccaggaggcg    60

gagtggaagt ggccgtgggg cgggt atg gga cta gct ggc gtg tgc gcc ctg     112
                            Met Gly Leu Ala Gly Val Cys Ala Leu
                            1               5

aga cgc tca gcg ggc tat ata ctc gtc ggt ggg gcc ggc ggt cag tct     160
Arg Arg Ser Ala Gly Tyr Ile Leu Val Gly Gly Ala Gly Gly Gln Ser
10                  15                  20                  25

gcg gca gcg gca gca aga cgg tgc agt gaa gga gag tgg gcg tct ggc     208
Ala Ala Ala Ala Ala Arg Arg Cys Ser Glu Gly Glu Trp Ala Ser Gly
                30                  35                  40

ggg gtc cgc agt ttc agc aga gcc gct gca gcc atg gcc cca atc aag     256
Gly Val Arg Ser Phe Ser Arg Ala Ala Ala Ala Met Ala Pro Ile Lys
            45                  50                  55

gtt cgg ctc ctg gct gat ccc act ggg gcc ttt ggg aag gag aca gac     304
Val Arg Leu Leu Ala Asp Pro Thr Gly Ala Phe Gly Lys Glu Thr Asp
        60                  65                  70

tta tta cta gat gat tcg ctg gtg tcc atc ttt ggg aat cga cgt ctc     352
Leu Leu Leu Asp Asp Ser Leu Val Ser Ile Phe Gly Asn Arg Arg Leu
    75                  80                  85

aag agg ttc tcc atg gtg gta cag gat ggc ata gtg aag gcc ctg aat     400
Lys Arg Phe Ser Met Val Val Gln Asp Gly Ile Val Lys Ala Leu Asn
90                  95                  100                 105

gtg gaa cca gat ggc aca ggc ctc acc tgc agc ctg gca ccc aat atc     448
Val Glu Pro Asp Gly Thr Gly Leu Thr Cys Ser Leu Ala Pro Asn Ile
                110                 115                 120

atc tca cag ctc tga ggccctgggc cagattactt cctccacccc tccctatctc     503
Ile Ser Gln Leu
            125

acctgcccag ccctgtgctg gggccctgca attggaatgt tggccagatt tctgcaataa   563

acacttgtgg tttgcggcca aaaaaaaa                                    592

<210> 33
<211> 779
<212> DNA
<213> Homo sapiens

<220>
<221> CDS
<222> (16)..(726)

<400> 33
gacgcgtggg cagcg atg gcg gct gcg tcg ggg tcg gtt ctg cag cgc tgt      51
                 Met Ala Ala Ala Ser Gly Ser Val Leu Gln Arg Cys
                 1               5                   10

atc gtg tcg ccg gca ggg agg cat agc gcc tct ctg atc ttc ctg cat      99
Ile Val Ser Pro Ala Gly Arg His Ser Ala Ser Leu Ile Phe Leu His
        15                  20                  25

ggc tca ggt gat tct gga caa gga tta aga atg tgg atc aag cag gtt      147
Gly Ser Gly Asp Ser Gly Gln Gly Leu Arg Met Trp Ile Lys Gln Val
    30                  35                  40

tta aat caa gat tta aca ttc caa cac ata aaa att att tat cca aca      195
Leu Asn Gln Asp Leu Thr Phe Gln His Ile Lys Ile Ile Tyr Pro Thr
45                  50                  55                  60

gct cct ccc aga tca tat act cct atg aaa gga gga atc tcc aat gta      243
Ala Pro Pro Arg Ser Tyr Thr Pro Met Lys Gly Gly Ile Ser Asn Val
                65                  70                  75

tgg ttt gac aga ttt aaa ata acc aat gac tgc cca gaa cac ctt gaa      291
Trp Phe Asp Arg Phe Lys Ile Thr Asn Asp Cys Pro Glu His Leu Glu
            80                  85                  90

tca att gat gtc atg tgt caa gtg ctt act gat ttg att gat gaa gaa      339
Ser Ile Asp Val Met Cys Gln Val Leu Thr Asp Leu Ile Asp Glu Glu
        95                  100                 105

gta aaa agt ggc atc aag aag aac agg ata tta ata gga gga ttc tct      387
Val Lys Ser Gly Ile Lys Lys Asn Arg Ile Leu Ile Gly Gly Phe Ser
    110                 115                 120

atg gga gga tgc atg gca atg cat tta gca tat aga aat cat caa gat      435
Met Gly Gly Cys Met Ala Met His Leu Ala Tyr Arg Asn His Gln Asp
125                 130                 135                 140

gtg gca gga gta ttt gct ctt tct agt ttt ctg aat aaa gca tct gct      483
Val Ala Gly Val Phe Ala Leu Ser Ser Phe Leu Asn Lys Ala Ser Ala
                145                 150                 155

gtt tac cag gct ctt cag aag agt aat ggt gta ctt cct gaa tta ttt      531
Val Tyr Gln Ala Leu Gln Lys Ser Asn Gly Val Leu Pro Glu Leu Phe
            160                 165                 170

cag tgt cat ggt act gca gat gag tta gtt ctt cat tct tgg gca gaa 579
Gln Cys His Gly Thr Ala Asp Glu Leu Val Leu His Ser Trp Ala Glu
        175                 180                 185

gag aca aac tca atg tta aaa tct cta gga gtg acc acg aag ttt cat 627
Glu Thr Asn Ser Met Leu Lys Ser Leu Gly Val Thr Thr Lys Phe His
    190                 195                 200

agt ttt cca aat gtt tac cat gag cta agc aaa act gag tta gac ata 675
Ser Phe Pro Asn Val Tyr His Glu Leu Ser Lys Thr Glu Leu Asp Ile
205                 210                 215                 220

ttg aag tta tgg att ctt aca aag ctg cca gga gaa atg gaa aaa caa 723
Leu Lys Leu Trp Ile Leu Thr Lys Leu Pro Gly Glu Met Glu Lys Gln
                225                 230                 235

aaa tgaatgaatc aagagtgatt tgttaatgta agtgtaatgt ctttgtgaaa agt 779
Lys

<210> 34
<211> 567
<212> DNA
<213> Homo sapiens

<220>
<221> CDS
<222> (56)..(514)

<400> 34
   agccgggctg gtcctgctgc gagccggcgg cccggagtgg ggcggcggag caaac atg 58
   Met
   1
   aac gtt gga gtt gcc cac agt gaa gtg aat cca aat acc cgt gtc atg 106
   Asn Val Gly Val Ala His Ser Glu Val Asn Pro Asn Thr Arg Val Met
   5 10 15
   aac agc cgg ggt atg tgg ctg aca tat gca ttg gga gtt ggc ttg ctt 154
   Asn Ser Arg Gly Met Trp Leu Thr Tyr Ala Leu Gly Val Gly Leu Leu
   20 25 30
   cat att gtc tta ctc agc att ccc ttc ttc agt gtt cct gtt gct tgg 202
   His Ile Val Leu Leu Ser Ile Pro Phe Phe Ser Val Pro Val Ala Trp
   35 40 45
   act tta aca aat att ata cat aat ctg ggg atg tac gta ttt ttg cat 250
   Thr Leu Thr Asn Ile Ile His Asn Leu Gly Met Tyr Val Phe Leu His
   50 55 60 65
   gca gtg aaa gga aca cct ttc gaa act cct gac cag ggt aaa gca agg 298
   Ala Val Lys Gly Thr Pro Phe Glu Thr Pro Asp Gln Gly Lys Ala Arg
   70 75 80
   ctc cta act cat tgg gaa caa ctg gac tat gga gta cag ttt aca tct 346
   Leu Leu Thr His Trp Glu Gln Leu Asp Tyr Gly Val Gln Phe Thr Ser
   85 90 95
   tca cgg aag ttt ttc aca att tct cca ata att cta tat ttt ctg gca 394
   Ser Arg Lys Phe Phe Thr Ile Ser Pro Ile Ile Leu Tyr Phe Leu Ala
   100 105 110
   agt ttc tat acg aag tat gat cca act cac ttc atc cta aac aca gct 442
   Ser Phe Tyr Thr Lys Tyr Asp Pro Thr His Phe Ile Leu Asn Thr Ala
   115 120 125
   tct ctc ctg agt gta cta att ccc aaa atg cca caa cta cat ggt gtt 490
   Ser Leu Leu Ser Val Leu Ile Pro Lys Met Pro Gln Leu His Gly Val
   130 135 140 145
   cgg atc ttt gga att aat aag tat tgaaatgttt tgaaactgaa aaaaaatttt 544
   Arg Ile Phe Gly Ile Asn Lys Tyr
   150
   acagctactg aatttcttat aag 567

<210> 35
<211> 1518
<212> DNA
<213> Homo sapiens

<220>
<221> CDS
<222> (252)..(566)

<400> 35
aaggatcctt aattaaatta atcccccccc cccccgactc ttgtttcgct ccttgacaac 60

cctggcgggg gttcgctggc tgcggccccg gctccggccc ccgcaggagc agcacccccc 120

ggggaaagac attttctgct cccaccgagt tggcagggcc tgcttcctga atctcctggg 180

tgtgtcttaa ctgccagtcc cagcacctcc tgaaagcccc actctcctcc agtggtcaca 240

gtggaaggat c atg gga gaa aca gaa ggg aag aaa gat gag gct gac tat 290
Met Gly Glu Thr Glu Gly Lys Lys Asp Glu Ala Asp Tyr
1               5                   10

aag cga ctg cag acc ttc cct ctg gtc agg cac tcg gac atg cca gag 338
Lys Arg Leu Gln Thr Phe Pro Leu Val Arg His Ser Asp Met Pro Glu
    15                  20                  25

gag atg cgc gtg gag acc atg gag cta tgt gtc aca gcc tgt gag aaa 386
Glu Met Arg Val Glu Thr Met Glu Leu Cys Val Thr Ala Cys Glu Lys
30                  35                  40                  45

ttc tcc aac aac aac gag agc gcc gcc aag atg atc aaa gag aca atg 434
Phe Ser Asn Asn Asn Glu Ser Ala Ala Lys Met Ile Lys Glu Thr Met
                50                  55                  60

gac aag aag ttc ggc tcc tcc tgg cac gtg gtg atc ggc gag ggc ttt 482
Asp Lys Lys Phe Gly Ser Ser Trp His Val Val Ile Gly Glu Gly Phe
            65                  70                  75

ggg ttt gag atc acc cac gag gtg aag aac ctc ctc tac ctg tac ttc 530
Gly Phe Glu Ile Thr His Glu Val Lys Asn Leu Leu Tyr Leu Tyr Phe
        80                  85                  90

ggg ggc acc ctg gct gtg tgc gtc tgg aag tgc tcc tgac actctgtccc 580
Gly Gly Thr Leu Ala Val Cys Val Trp Lys Cys Ser
    95                  100                 105

ctgccccgtc ccctgcaggg ccttttcctg ccactcatct ggggtgggga gcagccctag 640

gcaggtcctg gtttttccaa ggagagttgg ggtcttttct ttttgtcttt gtgtaccagt 700

ttcctgagcc acgcccagtg tgtgaacttg acatctccat ccccaggctc tcaactgtct 760

ccctcggagt ctcagggtgt ggacggggca gcgggcatgg gtctgtgtgg gagacgtggg 820

gtggggcggt gtgacagggt agaggaggtg ggagatgaga tcttccgcac aggaacacgc 880

cagtccccct ttctccaggg ctgccttccc cttgcatcct gggagcccca ctgccctgcc 940

atccccagta ctgccgggaa gtgtcggccg tccttgtcat tagtggtcat atgaaaatgg 1000

ccccaagaag gagatgattc tttcaaggga cacaggcagc ttctctcctt gtcctctggg 1060

gaggtgctga cccctcagaa accccttccc ccaacttgac cccaggctga acagaccact 1120

gcatctcact gggccagcag cccccccagc ccccagcctt ggtggggacc aagcagcctt 1180

tcccgtcccc tcctcgaccc gtacagttga gagccagggg ctggtgtgtg ggagctgcta 1240

cctggcagtt tctcgagggg tcaccgagcc tctggtggga cacctgggca ggagtgctct 1300

caccacgagg ctgcttccgc agggaaccct ggcctgcccg cgacttcgca tcagggaccg 1360

catgctgatt tgtactgctc tctgctgggt tttctatgtt cttttcgagt gtgggaaaag 1420

ggttttagta gaagggtgaa tcgtatttta cacagcggtc ttatttatat aaatgtcttg 1480

gtttttacaa ttaaaatgac caaaaactga aaaaaaaa 1518

<210> 36
<211> 1056
<212> DNA
<213> Homo sapiens

<220>
<221> CDS
<222> (186)..(395)

<400> 36
gccgggcccc accggacggc ttgctctgct ctttacctgg ggttgctgtc gaggaccctg 60

tgcaagactc ggccggtttt tctttctccc tgatggacag acccaaacat agccgcgcag 120

catcgtgaag ggctggggcc ttcactcctc tgtggctctg gaagagcccg atttcctcag 180

gaggc    atg tcg ggc ccc agg cct gtg gtg ctg agc ggg cct tcg gga    227
         Met Ser Gly Pro Arg Pro Val Val Leu Ser Gly Pro Ser Gly
         1               5                   10

gct ggg aag agc acc ctg ctg aag agg ctg ctc cag gag cac agc ggc    275
Ala Gly Lys Ser Thr Leu Leu Lys Arg Leu Leu Gln Glu His Ser Gly
15                  20                  25                  30

atc ttt ggc ttc agc gtg tcc cat acc agg gct ctc gtg gag gga tac    323
Ile Phe Gly Phe Ser Val Ser His Thr Arg Ala Leu Val Glu Gly Tyr
                35                  40                  45

cac gag gaa ccc gag gcc cgg cga gga gaa cgg caa aga tta cta ctt    371
His Glu Glu Pro Glu Ala Arg Arg Gly Glu Arg Gln Arg Leu Leu Leu
            50                  55                  60

tgt aac cag gga ggt gat gca gcg tgacatagca gccggcgact tcatcgagca    425
Cys Asn Gln Gly Gly Asp Ala Ala
        65                  70

tgccgagttc tcggggaacc tgtatggcac gagcaaggtg gcggtgcagg ccgtgcaggc    485

catgaaccgc atctgtgtgc tggacgtgga cctgcagggt gtgcggaaca tcaaggccac    545

cgatctgcgg cccatctaca tctctgtgca gccgccttca ctgcacgtgc tggagcagcg    605

gctgcggcag cgcaacactg aaaccgagga gagcctggtg aagcggctgg ctgctgccca    665

ggccgacatg gagagcagca aggagcccgg cctgtttgat gtggtcatca ttaacgacag    725

cctggaccag gcctacgcag agctgaagga ggcgctctct gaggaaatca agaaagctca    785

aaggaccggc gcctgaggct tgctgtctgt tctcggcacc ccgggcccat acaggaccag    845

ggcagcagca ttgagccacc cccttggcag gcgatacggc agctctgtgc ccttggccag    905

catgtggagt ggaggagatg ctgcccctgt ggttggaaca tcctggggtg acccccgacc    965

cagcctcgct gggctgtccc ctgtccctat ctctcactct ggacccaggg ctgacatcct    1025

aataaaataa ctgttggatt agaaaaaaaa a                                   1056

<210> 37
<211> 456
<212> DNA
<213> Homo sapiens

<220>
<221> CDS
<222> (51)..(401)

<400> 37
ctactaaatt cgcggccgcg tcgacgggac gttgtctgca ggcactcaga atg gtc    56
cag cgt ttg aca tac cga cgt agg ctt tcc tac aat aca gcc tct aac    104
aaa act agg ctg tcc cga acc cct ggt aat aga att gtt tac ctt tat      152
acc aag aag gtt ggg aaa gca cca aaa tct gca tgt ggt gtg tgc cca      200
ggc aga ctt cga ggg gtt cgt gct gta aga cct aaa gtt ctt atg aga      248
ttg tcc aaa aca aag aaa cat gtc agc agg gcc tat ggt ggt tcc atg      296
tgt gct aaa tgt gtt cgt gac agg atc aag cgt gct ttc ctt atc gag      344
gag cag aaa atc gtt gtg aaa gtg ttg aag gca caa gca cag agt cag      392
aaa gct aaa taaaaaa atgaaacttt tttgagtaat aaaaatgaaa agacgctgta      448
aaaaaaaa                                                              456

<210> 38
<211> 2950
<212> DNA
<213> Homo sapiens

<220>
<221> CDS
<222> (222)..(1634)

<400> 38
   gcaggcaggg caactacctg ctgtgctcac tgctgctggg caacgtgctg gtcaacacca      60
   cgctcaccat cctgctcgac gacatcgccg gctcgggcct cgtggccgtg gtagtctcca     120
   ccatcggtat cgtcatcttc ggagagatcg tgccccaggc catctgctcc cggcatggcc     180
   tggctgtggg ggccaacacc atcttcctca ccaagttttt c    atg atg atg acc      233
                                                    Met Met Met Thr
                                                    1
   ttc ccc gct tcc tac ccg gtc agc aag ctg ctg gac tgc gtc ctg ggc      281
   Phe Pro Ala Ser Tyr Pro Val Ser Lys Leu Leu Asp Cys Val Leu Gly
   5                   10                  15                  20
   cag gag ata ggc acc gtc tat aac cgg gaa aaa ctg ctg gag atg ctc 329
   Gln Glu Ile Gly Thr Val Tyr Asn Arg Glu Lys Leu Leu Glu Met Leu
   25 30 35
   cgg gtc acc gat ccc tac aac gac ctc gtt aag gag gag ctg aac atc 377
   Arg Val Thr Asp Pro Tyr Asn Asp Leu Val Lys Glu Glu Leu Asn Ile
   40 45 50
   atc caa ggg gcg ctg gag ctc cgc acc aag acg gtg gag gac gtg atg 425
   Ile Gln Gly Ala Leu Glu Leu Arg Thr Lys Thr Val Glu Asp Val Met
   55 60 65
   acc cca ctc cgg gac tgc ttc atg atc acc ggc gaa gcc atc ctg gac 473
   Thr Pro Leu Arg Asp Cys Phe Met Ile Thr Gly Glu Ala Ile Leu Asp
   70 75 80
   ttc aac acc atg tct gag atc atg gag agc ggc tac acc cgc att cca 521
   Phe Asn Thr Met Ser Glu Ile Met Glu Ser Gly Tyr Thr Arg Ile Pro
   85 90 95 100
   gtg ttt gaa ggg gag cgc tcc aat atc gtg gac ctg ctg ttt gtc aaa 569
   Val Phe Glu Gly Glu Arg Ser Asn Ile Val Asp Leu Leu Phe Val Lys
   105 110 115
   gac ttg gcc ttc gtg gat ccc gat gac tgt acc ccc ctg aaa acc atc 617
   Asp Leu Ala Phe Val Asp Pro Asp Asp Cys Thr Pro Leu Lys Thr Ile
   120 125 130
   acc aaa ttt tat aac cac ccc ttg cac ttt gtt ttc aat gac acc aag 665
   Thr Lys Phe Tyr Asn His Pro Leu His Phe Val Phe Asn Asp Thr Lys
   135 140 145
   ttg gac gct atg ctg gaa gaa ttt aag aaa ggt aaa tct cac ctg gct 713
   Leu Asp Ala Met Leu Glu Glu Phe Lys Lys Gly Lys Ser His Leu Ala
   150 155 160
   atc gtg cag cgg gta aac aat gag gga gaa ggg gat cca ttt tat gaa 761
   Ile Val Gln Arg Val Asn Asn Glu Gly Glu Gly Asp Pro Phe Tyr Glu
   165 170 175 180
   gtt ctg gga atc gtc acc tta gaa gat gtg att gaa gaa atc atc aaa 809
   Val Leu Gly Ile Val Thr Leu Glu Asp Val Ile Glu Glu Ile Ile Lys
   185 190 195
   tct gag att ctt gat gaa aca gat tta tac act gac aac aga acg aaa 857
   Ser Glu Ile Leu Asp Glu Thr Asp Leu Tyr Thr Asp Asn Arg Thr Lys
   200 205 210
   aag aaa gtg gct cac cgg gaa cga aag caa gat ttt tct gcc ttt aag 905
   Lys Lys Val Ala His Arg Glu Arg Lys Gln Asp Phe Ser Ala Phe Lys
   215 220 225
   cag aca gac agt gag atg aag gtt aaa ata tca cca cag ctc ctc ctg 953
   Gln Thr Asp Ser Glu Met Lys Val Lys Ile Ser Pro Gln Leu Leu Leu
   230 235 240
   gcc atg cac cgt ttc cta gca aca gaa gta gaa gca ttt agc cca tcc 1001
   Ala Met His Arg Phe Leu Ala Thr Glu Val Glu Ala Phe Ser Pro Ser
   245 250 255 260
   cag atg tca gag aag atc ctt cta agg ctg cta aag cac ccc aat gtc     1049
   Gln Met Ser Glu Lys Ile Leu Leu Arg Leu Leu Lys His Pro Asn Val
                   265                 270                 275
   atc cag gaa ctg aaa tat gat gag aag aac aag aaa gcc ccc gaa tac     1097
   Ile Gln Glu Leu Lys Tyr Asp Glu Lys Asn Lys Lys Ala Pro Glu Tyr
               280                 285                 290
   tac ctc tac cag cgc aac aag cca gta gac tac ttc gtt ctc att ctg     1145
   Tyr Leu Tyr Gln Arg Asn Lys Pro Val Asp Tyr Phe Val Leu Ile Leu
           295                 300                 305
   cag ggg aaa gtg gaa gtt gaa gct ggg aaa gaa ggt atg aag ttt gaa     1193
   Gln Gly Lys Val Glu Val Glu Ala Gly Lys Glu Gly Met Lys Phe Glu
       310                 315                 320
   gcg agc gcc ttc tca tac tat ggc gtg atg gcc ctg aca gcc tct cca     1241
   Ala Ser Ala Phe Ser Tyr Tyr Gly Val Met Ala Leu Thr Ala Ser Pro
   325                 330                 335                 340
   ggt gaa aat aag tcc cct cct cgc cca tgt ggc ttg aat cac tca gac     1289
   Gly Glu Asn Lys Ser Pro Pro Arg Pro Cys Gly Leu Asn His Ser Asp
                   345                 350                 355
   tct ctc agt cga agc gac cgg att gac gcc gtc aca cca aca ctg ggg     1337
   Ser Leu Ser Arg Ser Asp Arg Ile Asp Ala Val Thr Pro Thr Leu Gly
               360                 365                 370
   agc agc aat aac cag ctc aat tct tcg ctc ctc caa gtc tac atc ccc     1385
   Ser Ser Asn Asn Gln Leu Asn Ser Ser Leu Leu Gln Val Tyr Ile Pro
           375                 380                 385
   gat tac tcg gtg cga gcc ctt tcg gat ctg cag ttt gtt aag atc tca     1433
   Asp Tyr Ser Val Arg Ala Leu Ser Asp Leu Gln Phe Val Lys Ile Ser
       390                 395                 400
   aga cag caa tac caa aat gcc ttg atg gca tcc cgg atg gac aaa acc     1481
   Arg Gln Gln Tyr Gln Asn Ala Leu Met Ala Ser Arg Met Asp Lys Thr
   405                 410                 415                 420
   ccc cag tct tca gac agt gaa aac act aaa atc gaa ttg act ctt acg     1529
   Pro Gln Ser Ser Asp Ser Glu Asn Thr Lys Ile Glu Leu Thr Leu Thr
                   425                 430                 435
   gag ctg cat gac ggg ttg cca gac gag aca gcc aac ctg ctc aac gaa     1577
   Glu Leu His Asp Gly Leu Pro Asp Glu Thr Ala Asn Leu Leu Asn Glu
               440                 445                 450
   cag aac tgt gtg acg cac agt aag gcc aac cac agc ctg cac aac gaa     1625
   Gln Asn Cys Val Thr His Ser Lys Ala Asn His Ser Leu His Asn Glu
           455                 460                 465
   ggc gcc atc taggccg cgctggctgc acccgcccag gcccgcaccc gcccagtccc     1681
   Gly Ala Ile
       470
   gagggcccgg ccctgtctgc ccatgacttc actggtgtga gcttgtccgc catgctgtac     1741
   cctgcaacat cctgagacca aagaccttgt gcccttccca ggagccgcgg aggaggacag     1801
   tgagggagga atggaaacga gagatgtgaa gttggcagcc ggggcatggc gttcaagatt 1861
   ttggagatga actgattccg cccaaataga atcatgttta ttttttcagc tctccctttt 1921
   atcattattc acactcctct gccctcgatt tgcatgaagt tgaaaattgt tgcgatttat 1981
   tttttcaaga gatcatgttt ttaaagtgtc ttttgcagag ttttaagttg ttctgtctga 2041
   actctgctgt gatcccatga tgtgaccctg atgggctgga cttgcccctc cggtagcctt 2101
   ccttggccct cccagcgagg ggcacccttc ctctgtgccc cagtgggcat caccgtcgat 2161
   ctcgctggct gaatgaagaa gaccgtgtta ctgcagaacc tgccaagtct gtcatcactg 2221
   tggggtgtag cctgcctcag agggacctgc aatcacctct ctgagctcag tggtattttg 2281
   agaatttaat gtttaactgt acccctttcc ctcaggaaga tttaacattt gcttgggaat 2341
   gtgattttgc tcccacccta aggaattttt atcaccaaaa tgaatgttaa tgaatttaaa 2401
   acccatggtt tatcattggc aagaggcaag ttgacttcat cctgtcattc cagcctgggg 2461
   tctgccagcc agccctcctc cccgacccag cgcctaagct cacagagtgt cgtcacccac 2521
   ctccctggtc cttgctcttg ttaacccaag atgctgctac acagatgcca aatggaaatc 2581
   ttccacaggg ctttttcagt aaacacgtga tgtggagtgc aagctcctcc ccttcccact 2641
   agaacatact ttaacagaaa acgagtcgga ccttctagct gcactctgta ctgtgtgccg 2701
   agaaggcatt tctagacccg tgtttttaaa ggagggaact ttggggattg ccagcccctg 2761
   ctcctcctcc cagggagcca actgtccctc ctcccctgtc cctgggccca tggggcccgg 2821
   cagtggctgt gtcccctgcc tgagggctct gtgcctcctg cctcagatgc ggcctgtgcc 2881
   agagaggctg cctgtacagc cagggtcagt ttggccccaa acagggattc agaaaccaaa 2941
   aaaaaaaaa 2950

<210> 39
<211> 412
<212> DNA
<213> Homo sapiens

<220>
<221> CDS
<222> (204)..(326)

<400> 39
taacttcagg aaccagctca tgatctcagg atgtatggaa aaataatctt tgtattacta 60

ttgtcaggag aaacgggaca acttgtccat cgtttcactg taccagctcc tgtagtgata 120

atactcatta ttttgtgtgt gatggctggt attattggaa cgatcctctt aatttcttac 180

agtattcgcc gactgataaa ggc atg agg atg tgg cct gca tgc tgc ctg 230

Met Arg Met Trp Pro Ala Cys Cys Leu
1               5

atc ttg cct aga acc gtc tgc acc tgc tgt tct ctt gtt tat gca aac      278
Ile Leu Pro Arg Thr Val Cys Thr Cys Cys Ser Leu Val Tyr Ala Asn
10                  15                  20                  25

tgg ctg cac ctg cta ttc ctt tgc tta tgc ccc tac ccc tgg cta tcc      326
Trp Leu His Leu Leu Phe Leu Cys Leu Cys Pro Tyr Pro Trp Leu Ser
                30                  35                  40

taattccctg ttctcctgcc tcactattac tgtattctct acttctaaat aaaaataaaa      386

caaaatacaa accgttaaaa aaaaaa                                          412

<210> 40
<211> 441
<212> DNA
<213> Homo sapiens

<220>
<221> CDS
<222> (9)..(308)

<400> 40
gagacacc atg aga gcc ctc aca ctc ctc gcc cta ttg gcc ctg gcc gca      50
         Met Arg Ala Leu Thr Leu Leu Ala Leu Leu Ala Leu Ala Ala
           1               5                  10

ctt tgc atc gct ggc cag gca ggt gcg aag ccc agc ggt gca gag tcc      98
Leu Cys Ile Ala Gly Gln Ala Gly Ala Lys Pro Ser Gly Ala Glu Ser
15                  20                  25                  30

agc aaa ggt gca gcc ttt gtg tcc aag cag gag ggc agc gag gta gtg      146
Ser Lys Gly Ala Ala Phe Val Ser Lys Gln Glu Gly Ser Glu Val Val
                35                  40                  45

aag aga ccc agg cgc tac ctg tat caa tgg ctg gga gcc cca gtc ccc      194
Lys Arg Pro Arg Arg Tyr Leu Tyr Gln Trp Leu Gly Ala Pro Val Pro
            50                  55                  60

tac ccg gat ccc ctg gag ccc agg agg gag gtg tgt gag ctc aat ccg      242
Tyr Pro Asp Pro Leu Glu Pro Arg Arg Glu Val Cys Glu Leu Asn Pro
        65                  70                  75

gac tgt gac gag ttg gct gac cac atc ggc ttt cag gag gcc tat cgg      290
Asp Cys Asp Glu Leu Ala Asp His Ile Gly Phe Gln Glu Ala Tyr Arg
    80                  85                  90

cgc ttc tac ggc ccg gtc tagggt gtcgctctgc tggcctggcc ggcaacccca      344
Arg Phe Tyr Gly Pro Val
95                  100

gttctgctcc tctccaggca cccttctttc ctcttcccct tgcccttgcc ctgacctccc      404

agccctatgg atgtggggtc cccatcatcc cagctgc                              441

<210> 41
<211> 724
<212> DNA
<213> Homo sapiens

<220>
<221> CDS
<222> (1)..(639)

<400> 41
atg gct gtc cca gca gtg tcc ggg ctc tcc cgg cag gtg cga tgc ttc 48
Met Ala Val Pro Ala Val Ser Gly Leu Ser Arg Gln Val Arg Cys Phe
1               5                   10                  15

agt acc tct gtg gtc aga cca ttt gcc aag ctt gtg agg cct cct gtt 96
Ser Thr Ser Val Val Arg Pro Phe Ala Lys Leu Val Arg Pro Pro Val
            20                  25                  30

cag gta tac ggt att gaa ggt cgc tat gcc aca gct ctt tat tct gct 144
Gln Val Tyr Gly Ile Glu Gly Arg Tyr Ala Thr Ala Leu Tyr Ser Ala
        35                  40                  45

gca tca aaa cag aat aag ctg gag caa gta gaa aag gag ttg ttg aga 192
Ala Ser Lys Gln Asn Lys Leu Glu Gln Val Glu Lys Glu Leu Leu Arg
    50                  55                  60

gta gca caa atc ctg aag gaa ccc aaa gtg gct gct tct gtt ttg aat 240
Val Ala Gln Ile Leu Lys Glu Pro Lys Val Ala Ala Ser Val Leu Asn
65                  70                  75                  80

ccc tat gtg aag cgt tcc att aaa gtg aaa agc cta aat gac atc aca 288
Pro Tyr Val Lys Arg Ser Ile Lys Val Lys Ser Leu Asn Asp Ile Thr
                85                  90                  95

gca aaa gag agg ttc tct ccc ctc act acc aat ctg atc aat ttg ctt 336
Ala Lys Glu Arg Phe Ser Pro Leu Thr Thr Asn Leu Ile Asn Leu Leu
            100                 105                 110

gct gaa aat ggt cga tta agc aat acc caa gga gtc gtt tct gcc ttt 384
Ala Glu Asn Gly Arg Leu Ser Asn Thr Gln Gly Val Val Ser Ala Phe
        115                 120                 125

tct acc atg atg agt gtc cat cgc gga gag gta cct tgc aca gtg acc 432
Ser Thr Met Met Ser Val His Arg Gly Glu Val Pro Cys Thr Val Thr
    130                 135                 140

tct gca tct cct tta gaa gaa gcc aca ctc tct gaa tta aaa act gtc 480
Ser Ala Ser Pro Leu Glu Glu Ala Thr Leu Ser Glu Leu Lys Thr Val
145                 150                 155                 160

ctc aag agc ttc cta agt caa ggc caa gta ttg aaa ttg gag gct aag 528
Leu Lys Ser Phe Leu Ser Gln Gly Gln Val Leu Lys Leu Glu Ala Lys
                165                 170                 175

act gat ccg tca atc ttg ggt gga atg att gtg cgc att ggc gag aaa 576
Thr Asp Pro Ser Ile Leu Gly Gly Met Ile Val Arg Ile Gly Glu Lys
            180                 185                 190

tat gtt gac atg tct gtc aag acc aag att cag aag ctg ggc agg gct 624
Tyr Val Asp Met Ser Val Lys Thr Lys Ile Gln Lys Leu Gly Arg Ala
        195                 200                 205

atg cgg gag att gtc taaaagtgtt ggttttctgc catcagtgaa aattcttaaa 679
Met Arg Glu Ile Val
    210

cttggagcaa caataaaaag cttccagaac agaaaaaaaa aaaaa 724

<210> 42
<211> 742
<212> DNA
<213> Homo sapiens

<220>
<221> CDS
<222> (68)..(655)

<400> 42
aaggatcctt aattaaatta atcccccccc cccccccctc tttcctttcg ctgctgcggc 60
cgcagcc atg agt atg ctc agg ctt cag aag agg ctc gcc tct agt gtc 109
ctc cgc tgt ggc aag aag aag gtc tgg tta gac ccc aat gag acc aat 157
gaa atc gcc aat gcc aac tcc cgt cag cag atc cgg aag ctc atc aaa 205
gat ggg ctg atc atc cgc aag cct gtg acg gtc cat tcc cgg gct cga 253
tgc cgg aaa aac acc ttg gcc cgc cgg aag ggc agg cac atg ggc ata 301
ggt aag cgg aag ggt aca gcc aat gcc cga atg cca gag aag gtc aca 349
tgg atg agg aga atg agg att ttg cgc cgg ctg ctc aga aga tac cgt 397
gaa tct aag aag atc gat cgc cac atg tat cac agc ctg tac ctg aag 445
gtg aag ggg aat gtg ttc aaa aac aag cgg att ctc atg gaa cac atc 493
cac aag ctg aag gca gac aag gcc cgc aag aag ctc ctg gct gac cag      541
gct gag gcc cgc agg tct aag acc aag gaa gca cgc aag cgc cgt gaa      589
gag cgc ctc cag gcc aag aag gag gag atc atc aag act tta tcc aag      637
gag gaa gag acc aag aaa taaaac ctcccacttt gtctgtacat actggcctct      691
gtgattacat agatcagcca ttaaaataaa acaagcctta aaaaaaaaaa a              742

<210> 43
<211> 405
<212> DNA
<213> Homo sapiens

<220>
<221> CDS
<222> (153)..(299)

<400> 43
tgcttcatat taacatagaa aagaaatttc ttcacctatc tcctgagtct tttgaaatct      60
tcacatatat tattttctaa attattatta tttaaaattt tcttaaagat ggagtcttgc      120
tctgtctccc aggctggata gagtacagtg gc atg atc tta gct cac tgt aac      173
ctc aga ctt ctg gga tca aac aat tct ccc ctc agc ctc ctg aat agc      221
tgg ggc tac agg tgc atg cca tca tgc atg gct aat ttt tta aaa ttt      269
ttc gta gag atg ggg tct cgc tac gta gtc t aggctgttct caaactcctg      320
ggctcaagca atcctcccac ctcagcctcc caaggcgctg ggattatagg tgtgagtcac      380
catccctggc ttctatttta ttact                                            405

<210> 44
<211> 1921
<212> DNA
<213> Homo sapiens

<220>
<221> CDS
<222> (701)..(1312)

<400> 44
atgaatgcag gctgggagtc cccgctcaca gtacaggaag gtttggtgtc tggtgagggc      60

tgctctgtgc ttccaagatg gcactgggtt gttgtgtcct ctggagaggg ggaaaactgg     120

gtctcgtgtg ctcctgtggc gagcgggcgc cccggaggtt tggcaggggt agtgccaacg     180

gacacctcat ggtgtggtag cttaaactca gagcctaaac gcaggaccag ggctaggagc     240

ccaggaccag aaggcaaaac ttcctacctg atttatggat gggaacttgc agcccctgga     300

ggggaatggc ttcggtctgg agctctacgc actagttctc tgggaggaca gagggcagga     360

aaggggtgca gccaaaatca aactcggttc cacttggacg agcagtgggg agattcgctg     420

tctgaactac taaggagagt ttttaaaacc acttaccaga atctagagtg ggaattaaaa     480

tctgcgtggg cttcttcaga ttccacagaa atgaggactg ccacaccttc tccaactttt     540

gcaggctcca cccaggatgc gaaggcacta gaatttccca aattaagaac gaagaggaag     600

tttggacctt ttcggccacc gctcgcttca atatggctgc ccccagggag agacgaggct     660

accatgaagg agccgagcgc agaccctgag tccgtcaccc atg gat cgc agc gcg      715
                                          Met Asp Arg Ser Ala
                                          1               5

gag ttc agg aaa tgg aag gcg caa tgt ttg agc aaa gcg gac ctc agc      763
Glu Phe Arg Lys Trp Lys Ala Gln Cys Leu Ser Lys Ala Asp Leu Ser
                10                  15                  20

cgg aag ggc agt gtt gac gag gat gtg gta gag ctt gtg cag ttt ctg      811
Arg Lys Gly Ser Val Asp Glu Asp Val Val Glu Leu Val Gln Phe Leu
            25                  30                  35

aac atg cga gat cag ttt ttc acc acc agc tcc tgc gct ggc cgc atc      859
Asn Met Arg Asp Gln Phe Phe Thr Thr Ser Ser Cys Ala Gly Arg Ile
        40                  45                  50

cta ctc ctt gac cgg ggt ata aat ggt ttt gag gtt cag aaa caa aac      907
Leu Leu Leu Asp Arg Gly Ile Asn Gly Phe Glu Val Gln Lys Gln Asn
    55                  60                  65

tgt tgc tgg cta ctg gtt aca cac aaa ctt tgt gta aaa gat gat gtg      955
Cys Cys Trp Leu Leu Val Thr His Lys Leu Cys Val Lys Asp Asp Val
70                  75                  80                  85

att gta gct ctg aag aaa gca aat ggt gat gcc act ttg aaa ttt gaa     1003
Ile Val Ala Leu Lys Lys Ala Asn Gly Asp Ala Thr Leu Lys Phe Glu
                90                  95                  100

cca ttt gtt ctt cat gtg cag tgt cga caa ttg cag gat gca cag att     1051
Pro Phe Val Leu His Val Gln Cys Arg Gln Leu Gln Asp Ala Gln Ile
            105                 110                 115

ctg cat tcc atg gca ata gat tct ggt ttc agg aac tct ggc ata acg     1099
Leu His Ser Met Ala Ile Asp Ser Gly Phe Arg Asn Ser Gly Ile Thr
        120                 125                 130

gtg gga aag aga gga aaa act atg ttg gct gtc cgg agt aca cat ggc     1147
Val Gly Lys Arg Gly Lys Thr Met Leu Ala Val Arg Ser Thr His Gly
    135                 140                 145

tta gaa gtt cca tta agc cat aag gga aaa ctg atg gtg aca gag gaa     1195
Leu Glu Val Pro Leu Ser His Lys Gly Lys Leu Met Val Thr Glu Glu
150                 155                 160                 165

tat att gac ttc ctg tta aat gtg gca aat caa aaa atg gag gaa aac     1243
Tyr Ile Asp Phe Leu Leu Asn Val Ala Asn Gln Lys Met Glu Glu Asn
                170                 175                 180

aag aaa aga att gag aga gat ggg gtt ttg cca tgt tgc cta ggc tgg     1291
Lys Lys Arg Ile Glu Arg Asp Gly Val Leu Pro Cys Cys Leu Gly Trp
            185                 190                 195

tct caa act cct gga ctc aag tg attgcccgcc ttggcctccc aaagtgttgg     1344
Ser Gln Thr Pro Gly Leu Lys
        200

gattacatgc ataagccacc gcacctgtct gacatatgac ttgactccca agaaacttag     1404

gttttacaac tgcctacagc atgctttgga aagggaaacg atgactaact tacatcccaa     1464

gatcaaagag aaaaataact catcatatat tcataagaaa aaaagaaacc cagaaaaaac     1524

acgtgcccag tgtattacta aagaaagtga tgaagaactt gaaaatgatg atgatgatga     1584

tctaggaatc aatgttacca tcttccctga agattactaa gctttggttc tgatgtgtct     1644

tggccgtaat gtttctagta ggttttataa agctgctctt cataagagta ttttagtttg     1704

ttgagtgtat cagccattca taagccagta atgacaagtg cagagcttca aactataact     1764

ttgttgccca gaggatgtgc agttgtcatc taagctctca gcagtacccg gcttatccta     1824

cgacttcacc tgaaatgcta tagttatccc tactttttta ccagtttctc ccagaagcac     1884

ctgcttaata aatcaaagat gtttgaaaaa aaaaaaa                              1921

<210> 45
<211> 1026
<212> DNA
<213> Homo sapiens

<220>
<221> CDS
<222> (91)..(402)

<400> 45
ggctgccaac agatcatgag ccatcagctc ctctggggcc agctatagga caacagaact      60

ctcaccaaag gaccagacac agtgagcacc     atg gga cag tgt cgg tca gcc      111
                                     Met Gly Gln Cys Arg Ser Ala
                                      1               5

aac gca gag gat gct cag gaa ttc agt gat gtg gag agg gcc att gag      159
Asn Ala Glu Asp Ala Gln Glu Phe Ser Asp Val Glu Arg Ala Ile Glu
        10                  15                  20

acc ctc atc aag aac ttt cac cag tac tcc gtg gag ggt ggg aag gag      207
Thr Leu Ile Lys Asn Phe His Gln Tyr Ser Val Glu Gly Gly Lys Glu
    25                  30                  35

acg ctg acc cct tct gag cta cgg gac ctg gtc acc cag cag ctg ccc      255
Thr Leu Thr Pro Ser Glu Leu Arg Asp Leu Val Thr Gln Gln Leu Pro
40                  45                  50                  55

cat ctc atg ccg agc aac tgt ggc ctg gaa gag aaa att gcc aac ctg      303
His Leu Met Pro Ser Asn Cys Gly Leu Glu Glu Lys Ile Ala Asn Leu
                60                  65                  70

ggc agc tgc aat gac tct aaa ctg gag ttc agg agt ttc tgg gag ctg      351
Gly Ser Cys Asn Asp Ser Lys Leu Glu Phe Arg Ser Phe Trp Glu Leu
            75                  80                  85

att gga gaa gcg gcc aag agt gtg aag ctg gag agg cct gtc cgg ggg      399
Ile Gly Glu Ala Ala Lys Ser Val Lys Leu Glu Arg Pro Val Arg Gly
        90                  95                  100

cac tgag aactccctct ggaattcttg gggggtgttg gggagagact gtgggcctgg      456
His

aaataaaact tgtctcctct accaccaccc tgtaccctag ccctgcacct gtccacatct      516

ctgcaaagtt cagcttcctt ccccaggtct ctgtgcactc tgtcttggat gctctgggga      576

gctcatgggt ggaggagtct ccaccagagg gaggctcagg ggactggttg ggccagggat      636

gaatatttga gggataaaaa ttgtgtaaga gccaaagaat tggtagtagg gggagaacag      696

agaggagctg ggctatggga aatgatttga ataatggagc tgggaatatg gctggatatc      756

tggtactaaa aaagggtctt taagaaccta cttcctaatc tcttccccaa tccaaaccat      816

agctgtctgt ccagtgctct cttcctgcct ccagctctgc cccaggctcc tcctagactc      876

tgtccctggg ctagggcagg ggaggaggga gagcagggtt gggggagagg ctgaggagag      936

tgtgacatgt ggggagagga ccagctgggt gcttgggcat tgacagaatg atggttgttt      996

tgtatcattt gattaataaa aaaaaaaaaa                                    1026

<210> 46
<211> 874

<212> DNA
<213> Homo sapiens

<220>
<221> CDS
<222> (277)..(591)

<400> 46
gctctggaat cgagttacgc gcgaaagggc agagtttctg gaggaaaccg cagcctctca 60

accgctgacc gggtctcaga aggcccccgg caggcccgct tggcgggaac tgaccacgcg 120

ccagtcaggc tctccaggga cctgcgcagg cgcgtgtggg cggagtcgtg cgcagggggc 180

ggggcttcgg gaaggagcca cagagagggc ggggcgtagg acctgcgctt cgggggtgga 240

gtcggagcgg cgcggcggcg gtcatgcggg acgcgg atg cag acg cag gcg gag 294
Met Gln Thr Gln Ala Glu
1               5

gcg ctg acg gcg ggg atg gcc ggg gtg gcc aca gct gcc gcg ggg gcg 342
Ala Leu Thr Ala Gly Met Ala Gly Val Ala Thr Ala Ala Ala Gly Ala
            10                  15                  20

tgg aca cag ccg cag ctc cgg ccg gtg gag ctc ccc cag cgc acg cgc 390
Trp Thr Gln Pro Gln Leu Arg Pro Val Glu Leu Pro Gln Arg Thr Arg
        25                  30                  35

cag gtc cgg gca gag acg ccg cgt ctg cgc cag ggg gtc acg aat gcg 438
Gln Val Arg Ala Glu Thr Pro Arg Leu Arg Gln Gly Val Thr Asn Ala
    40                  45                  50

gcc gca cat att cac cct cag cgt gcc ttt ccc gac ccc ctt gga ggc 486
Ala Ala His Ile His Pro Gln Arg Ala Phe Pro Asp Pro Leu Gly Gly
55                  60                  65                  70

gga aat cgc cca tgg gtc cct ggc acc aga tgc cga gcc cca cca aag 534
Gly Asn Arg Pro Trp Val Pro Gly Thr Arg Cys Arg Ala Pro Pro Lys
                75                  80                  85

ggt ggt tgg gaa gga tct cac agt gag tgg cag gat cct ggt cgt ccg 582
Gly Gly Trp Glu Gly Ser His Ser Glu Trp Gln Asp Pro Gly Arg Pro
            90                  95                  100

ctg gaa agc tgaagac tgtcgcctgc tccgaatttc cgtcatcaac tttcttgacc 638
Leu Glu Ser
        105

agctttccct ggtggtgcgg accatgcagc gctttgggcc ccccgtttcc cgctaagcct 698

ggcctgggca aatggagcga ggtcccactt tgcgtctcct tgtaggcagt gcgtccatcc 758

ttccctaggg caggaattcc cacagttgct actttcctgg gagggcctca tgttttatct 818

ggttcttaaa tgtttgttac tacagaaaat aaaactgcgc tactaaaaaa aaaaaa 874

<210> 47

<211> 1117
<212> DNA
<213> Homo sapiens

<220>
<221> CDS
<222> (93)..(896)

<400> 47
agctgttgtg catccagagg tggaattggg gcccggcatt ccctcctcgt cccgggctgg 60

cccttgcccc caccctgcaa ctcctggttg ag atg ggc tca gcc aag agc gtc 113
Met Gly Ser Ala Lys Ser Val
1               5

cca gtc aca cca gcg cgg cct ccg ccg cac aac aag cat ctg gct cga 161
Pro Val Thr Pro Ala Arg Pro Pro Pro His Asn Lys His Leu Ala Arg
        10                  15                  20

gtg gcg gac ccc cgt tca cct agt gct ggc atc ctg cgc act ccc atc 209
Val Ala Asp Pro Arg Ser Pro Ser Ala Gly Ile Leu Arg Thr Pro Ile
    25                  30                  35

cag gtg gag agc tct cca cag cca ggc cta cca gca ggg gag caa ctg 257
Gln Val Glu Ser Ser Pro Gln Pro Gly Leu Pro Ala Gly Glu Gln Leu
40                  45                  50                  55

gag ggt ctt aaa cat gcc cag gac tca gat ccc cgc tct cct act ctt 305
Glu Gly Leu Lys His Ala Gln Asp Ser Asp Pro Arg Ser Pro Thr Leu
                60                  65                  70

ggt att gca cgg aca cct atg aag acc agc agt gga gac ccc cca agc 353
Gly Ile Ala Arg Thr Pro Met Lys Thr Ser Ser Gly Asp Pro Pro Ser
            75                  80                  85

cca ctg gtg aaa cag ctg agt gaa gta ttt gaa act gaa gac tct aaa 401
Pro Leu Val Lys Gln Leu Ser Glu Val Phe Glu Thr Glu Asp Ser Lys
        90                  95                  100

tca aat ctt ccc cca gag cct gtt ctg ccc cca gag gca cct tta tct 449
Ser Asn Leu Pro Pro Glu Pro Val Leu Pro Pro Glu Ala Pro Leu Ser
    105                 110                 115

tct gaa ttg gac ttg cct ctg ggt acc cag tta tct gtt gag gaa cag 497
Ser Glu Leu Asp Leu Pro Leu Gly Thr Gln Leu Ser Val Glu Glu Gln
120                 125                 130                 135

atg cca cct tgg aac cag act gag ttc ccc tcc aaa cag gtg ttt tcc 545
Met Pro Pro Trp Asn Gln Thr Glu Phe Pro Ser Lys Gln Val Phe Ser
                140                 145                 150

aag gag gaa gca aga cag ccc aca gaa acc cct gtg gcc agc cag agc 593
Lys Glu Glu Ala Arg Gln Pro Thr Glu Thr Pro Val Ala Ser Gln Ser
            155                 160                 165

tcc gac aag ccc tca agg gac cct gag act ccc aga tct tca ggt tct 641
Ser Asp Lys Pro Ser Arg Asp Pro Glu Thr Pro Arg Ser Ser Gly Ser
        170                 175                 180

atg cgc aat aga tgg aaa cca aac agc agc aag gta cta ggg aga tcc 689

Met Arg Asn Arg Trp Lys Pro Asn Ser Ser Lys Val Leu Gly Arg Ser
    185                 190                 195

ccc ctc acc atc ctg cag gat gac aac tcc cct ggc acc ctg aca cta      737
Pro Leu Thr Ile Leu Gln Asp Asp Asn Ser Pro Gly Thr Leu Thr Leu
200                 205                 210                 215

cga cag ggt aag cgg cct tca ccc cta agt gaa aat gtt agt gaa cta      785
Arg Gln Gly Lys Arg Pro Ser Pro Leu Ser Glu Asn Val Ser Glu Leu
                220                 225                 230

aag gaa gga gcc att ctt gga act gga cga ctt ctg aaa act gga gga      833
Lys Glu Gly Ala Ile Leu Gly Thr Gly Arg Leu Leu Lys Thr Gly Gly
            235                 240                 245

cga gca tgg gag caa ggc cag gac cat gac aag gaa aat cag cac ttt      881
Arg Ala Trp Glu Gln Gly Gln Asp His Asp Lys Glu Asn Gln His Phe
        250                 255                 260

ccc ttg gtg gag agc taggccctgc atggccccag caatgcagtc acccagggcc      936
Pro Leu Val Glu Ser
    265

tggtgatatc tgtgtcctct caccccttct ttcccaggga tactgaggaa tggcttgttt      996

tcttagactc ctcctcagct accaaactgg gactcacagc tttattgggc tttctttgtg     1056

tcttgtgtgt ttcttttata ttaaaggaag taattttaaa tgttacttta aaaaaaaaaa     1116

a                                                                     1117

<210> 48
<211> 6607
<212> DNA
<213> Homo sapiens

<220>
<221> CDS
<222> (81)..(1544)

<400> 48
acacgaaagg caccccggag attgctttct ggggaagcaa tgctggggtg aaaacaacac       60

ggctagaagc tcattctgaa atg ggg agc act gaa att ttg gaa aag gag          110
                      Met Gly Ser Thr Glu Ile Leu Glu Lys Glu
                      1               5                   10

acc cca gaa aat ctc agt aat ggt acc agc agc aat gtg gaa gca gcc      158
Thr Pro Glu Asn Leu Ser Asn Gly Thr Ser Ser Asn Val Glu Ala Ala
                15                  20                  25

aaa agg ttg gcc aaa cgc ctt tat cag ctg gac aga ttc aaa aga tca      206
Lys Arg Leu Ala Lys Arg Leu Tyr Gln Leu Asp Arg Phe Lys Arg Ser
            30                  35                  40

gat gtt gca aaa cac ctt ggc aag aac aac gaa ttt agc aaa cta gtt      254
Asp Val Ala Lys His Leu Gly Lys Asn Asn Glu Phe Ser Lys Leu Val

45                  50                  55

gca gaa gaa tat ctg aag ttt ttt gat ttt aca gga atg acg ctg gat        302
Ala Glu Glu Tyr Leu Lys Phe Phe Asp Phe Thr Gly Met Thr Leu Asp
     60                  65                  70

cag tca ctc agg tat ttc ttt aaa gca ttc tct ctt gtg gga gaa act        350
Gln Ser Leu Arg Tyr Phe Phe Lys Ala Phe Ser Leu Val Gly Glu Thr
 75                  80                  85                  90

caa gaa cga gag aga gtt tta ata cac ttc tcc aat aga tat ttt tat        398
Gln Glu Arg Glu Arg Val Leu Ile His Phe Ser Asn Arg Tyr Phe Tyr
                 95                 100                 105

tgt aac cca gat acc att gct tca caa gat gga gtc cat tgc ctt acc        446
Cys Asn Pro Asp Thr Ile Ala Ser Gln Asp Gly Val His Cys Leu Thr
            110                 115                 120

tgt gca ata atg ctt ctt aat acc gat cta cat ggc cac aat att gga        494
Cys Ala Ile Met Leu Leu Asn Thr Asp Leu His Gly His Asn Ile Gly
        125                 130                 135

aag aag atg acc tgt cag gag ttc att gca aat ctg caa ggg gta aat        542
Lys Lys Met Thr Cys Gln Glu Phe Ile Ala Asn Leu Gln Gly Val Asn
    140                 145                 150

gag ggt gtt gat ttc tcc aag gat ctg ctg aaa gct ctg tac aac tca        590
Glu Gly Val Asp Phe Ser Lys Asp Leu Leu Lys Ala Leu Tyr Asn Ser
155                 160                 165                 170

atc aag aat gag aag ctt gaa tgg gca gta gat gat gaa gag aaa aaa        638
Ile Lys Asn Glu Lys Leu Glu Trp Ala Val Asp Asp Glu Glu Lys Lys
                175                 180                 185

aag tct ccc tca gaa agt act gag gag aaa gct aac gga aca cat cca        686
Lys Ser Pro Ser Glu Ser Thr Glu Glu Lys Ala Asn Gly Thr His Pro
            190                 195                 200

aag acc atc agt cgt att gga agt act act aac cca ttt ttg gac att        734
Lys Thr Ile Ser Arg Ile Gly Ser Thr Thr Asn Pro Phe Leu Asp Ile
        205                 210                 215

cct cat gat cca aat gct gct gtg tac aaa agt gga ttc ttg gct cgg        782
Pro His Asp Pro Asn Ala Ala Val Tyr Lys Ser Gly Phe Leu Ala Arg
    220                 225                 230

aaa att cat gca gat atg gat gga aag aag act cca aga gga aaa cga        830
Lys Ile His Ala Asp Met Asp Gly Lys Lys Thr Pro Arg Gly Lys Arg
235                 240                 245                 250

gga tgg aaa acc ttt tat gct gta ctg aag gga aca gtt ctt tac ttg        878
Gly Trp Lys Thr Phe Tyr Ala Val Leu Lys Gly Thr Val Leu Tyr Leu
                255                 260                 265

caa aag gat gaa tac aag cca gaa aag gcc ttg tct gaa gag gac ttg        926
Gln Lys Asp Glu Tyr Lys Pro Glu Lys Ala Leu Ser Glu Glu Asp Leu
            270                 275                 280

aaa aac gct gtg agt gtg cac cac gca ttg gca tcc aag gcc acg gac        974
Lys Asn Ala Val Ser Val His His Ala Leu Ala Ser Lys Ala Thr Asp

        285                 290                 295

tat gag aag aaa cca aac gtg ttt aaa ctt aaa act gcc gac tgg agg 1022
Tyr Glu Lys Lys Pro Asn Val Phe Lys Leu Lys Thr Ala Asp Trp Arg
    300                 305                 310

gtc ttg ctt ttt caa act cag agc cca gag gaa atg caa ggg tgg ata 1070
Val Leu Leu Phe Gln Thr Gln Ser Pro Glu Glu Met Gln Gly Trp Ile
315                 320                 325                 330

aac aaa atc aat tgt gtg gca gct gta ttt tct gca cca cca ttt cca 1118
Asn Lys Ile Asn Cys Val Ala Ala Val Phe Ser Ala Pro Pro Phe Pro
                335                 340                 345

gca gca atc ggc tct cag aag aag ttt agc cgc cca ctt ctg cct gcc 1166
Ala Ala Ile Gly Ser Gln Lys Lys Phe Ser Arg Pro Leu Leu Pro Ala
            350                 355                 360

act aca aca aaa ctg tct cag gag gag caa ctg aag tca cat gaa agt 1214
Thr Thr Thr Lys Leu Ser Gln Glu Glu Gln Leu Lys Ser His Glu Ser
        365                 370                 375

aag ctg aag cag atc acc acc gag ctg gcc gag cac cgc tca tat ccc 1262
Lys Leu Lys Gln Ile Thr Thr Glu Leu Ala Glu His Arg Ser Tyr Pro
    380                 385                 390

ccc gac aag aag gtc aaa gcc aag gac gtc gat gag tac aaa ctg aaa 1310
Pro Asp Lys Lys Val Lys Ala Lys Asp Val Asp Glu Tyr Lys Leu Lys
395                 400                 405                 410

gac cac tat ctg gag ttt gag aaa acc cgc tat gaa atg tat gtc agc 1358
Asp His Tyr Leu Glu Phe Glu Lys Thr Arg Tyr Glu Met Tyr Val Ser
                415                 420                 425

att ctc aag gaa gga ggc aaa gag cta ctg agt aac gat gaa agc gag 1406
Ile Leu Lys Glu Gly Gly Lys Glu Leu Leu Ser Asn Asp Glu Ser Glu
            430                 435                 440

gct gca gga ctg aag aag tcg cac tcg agt cct tcg ctg aac ccg gat 1454
Ala Ala Gly Leu Lys Lys Ser His Ser Ser Pro Ser Leu Asn Pro Asp
        445                 450                 455

act tct cca atc act gcc aaa gtc aag cgt aac gtg tca gag agg aag 1502
Thr Ser Pro Ile Thr Ala Lys Val Lys Arg Asn Val Ser Glu Arg Lys
    460                 465                 470

gat cac cga cct gaa aca cca agc att aag caa aaa gtt act tagagtc 1551
Asp His Arg Pro Glu Thr Pro Ser Ile Lys Gln Lys Val Thr
475                 480                 485

catctgcggc caggaagtgc tggtcatgga gcaaaatagg gtttttcaag atctttctgg 1611

taatccgtga atatatttaa aaaaaaaaaa gtctgtgaca aaacggtgca ttagtaattt 1671

tttctattgt atatttttgt tagtttctgt acagattgtc tttgctcttg atttcttttg 1731

ctttgatgat ttttgcaact tgatagctaa tgcacctttt ctgtgaggag gaggggatcg 1791

tgatttcaga atgaattatg tatcccttct cttttggttt tctcttgttt gcagtctgct 1851

cagttgtttt atgtattctc atatcaactg ttaaactttt ttttaaggtt aaagaattta 1911

atccattgtg aaacacttaa ctggacaaac tgtagtttta gtaaattcta gctggagtta 1971

atatacgcct ttatatgtga aatcttgccc agtcacagag gtagaattga gcactcacag 2031

atgctccagt aagaatcaca gtgctgggaa tctagttgct ccaatatgag gcagcttcat 2091

gtgcagctta gcacttgttg ttgagatcgg accctgctgg aagcagggaa aagaagcgtg 2151

aagatcgtag gattgagaac ttagggaagc acattagctt gcttgaagtg ctgattccat 2211

ttcagccaag caagggaaag aggaagtgga gtcattttgc ctttgaaggc tgaggaaaga 2271

ttgataccca gttaattttg tttgctaaag gatgggggca ataatcggcc cttgaggagc 2331

tgcagcagta ggcatgtgct cagtctgcag gaattgttac ctcactccca cagggtctag 2391

actagaaatc catcatctct atcgttgata tccttccatc caggaataga tttttcttac 2451

tctacatatg tgtgtgtgcg tgcgtgtgtg tgcgtgtgtg ggcatggggt tgtgtcctgg 2511

ttgtgatatt gaggtcttcc ttcctaacaa attaatacta aaatgaaaca gcttttcttg 2571

tgtccttaag acaaaataag gaaggaaaac gtagctgcag ttgtccacga tggatattgg 2631

ttctttaaaa tatatctgaa agtagtagtc agaatgaatt atggttggaa aactgaggaa 2691

tcttctggtt gcaggtgcaa agtgactttg tttattcttg tctcagtctc cttgatagcc 2751

acttcactct gctactactc aactttctcc taaaaatact tcatctattt tcagtccttt 2811

ctttctgtct actcaaaatg gttctattaa ctttgcagtc atgagcttgt tccagttaca 2871

gtccctttga agttcagggt gataaacaga atattcttct gtagaggaag agaaaggagt 2931

gaaagtttag cccactgaga cctagagctt tgtgatttcc taaccttgaa actctgtaat 2991

ccctaaagtt aaaatctccg caagtggcac aacttcagaa ctaatagtat cactttgatt 3051

tttctttttc ctcccttaga aagtttctct agttctatag tttatttgtt gaaggtacta 3111

tgaccaaaga atcagctgct ctacaggaat agcatggttc cagtgaatta gagaaaacct 3171

gctgtaaagc catggtagtg tctaagtggt atgttattat gatgtactag catttattta 3231

cagaattatt tattaacgtt tacttccttc ccctctgtaa atgtccatga ctattgccca 3291

gagaaggctt acccctctct agggttgcag ttgctttctt tgtaataagt attttgccac 3351

acctgtaaaa aaaaaaaacc tcacttttaa ctctctgcct tgtttgggta aaggcagtaa 3411

ctaagtttat gtttcagaac tgcaaaacaa acaggatagt taccaatatg gcccatgtgt 3471

cagattgatt tttgtagcct ctcactgaat ccaacatatc cacaagcaag ttatctgtct 3531

ttctacctga taatctaaat tatcaggata tttgttttct gcctaaatgt ttatactaag 3591

ccgaggggag agaggtacct agaccatgtc atctacgagc ttcagtaact aaagaaaaag 3651

gaacttccct gagtggcttg aatgtgtttg cccacagtct atatctatgt atatagaatg 3711
tctgtatgta ttttacttat ttaatataca ttgaatggta ccttgctaca gtatttctga 3771
catttagagt agtgttgaaa tactcggcta gcatcagcac cactatagca ctgtccgtgt 3831
catatgagtc actaatatta actccaggga cttctggata ggctaataga tcattggata 3891
cgaagggctc ttttgaagct tcagtatacc atgtttgcat agtttatctt taaaaacaac 3951
tttaaaggtt cttttgtgag ccaggatctc agactgccgt agcatgatgc tgtccatctt 4011
tagcgcatgg gctgagaaca cctcttccct gaggcttctg aaggttgctg tctgtcatga 4071
gtgcatgaag gaggccaaga gtttatgcta tgggaggaaa cagtcactga tttgcctaga 4131
ttctgagagt ctggcccata gccaaccaca ttttcctttg ggataattta tttcctgtgg 4191
catctagcca gaagaaattg aggatgtttc ctttcacagc tgctccaagc ctgttgccca 4251
attcacggta caagggagca ccccttccct ttcctctgaa ggtacgccac ccacctccgt 4311
cgcccacctc agcgcccagg agccttggga cttccttcca tatgataaat cattcttctt 4371
cacgtcaata cacttcatat taatttctag tacagaaaat cttgacagct atcagaatgc 4431
cttggtcata gtgttgttgc aaaattgacc atacaggtgg cccatgtata aaatctgaat 4491
tttaggggtt tgtccccacc tcgcatgctg gcttttacag ggaggtgtct gggattcctc 4551
attagcaatc aaaacttaat tactgggatg cagagtcctt actttatcgc cagcccgtag 4611
gcatttctga agtgcacttt tttgaaacat cattttgcta actctcagca gtgtctaatt 4671
aaactgagca atacttttgt gaattttaat taatctcagc aaaaccatga tgggagagag 4731
tcctctgatg gaaatgtagt ccctggatta tgtgtaacct ttttattgct cttagatgca 4791
gaggatagaa agcatttttt ggtgcagtgg tcttgtggca aacacaagac cctctatgcg 4851
tctccaactg ttatcctaat ctagaaaatg aggactggcc cctgggcaaa agtgacatga 4911
ggaatttact ctggaagagg aaaatctggg tggctttcca aggctaagat aggtttgtat 4971
ttcaccctgt ggccaagcta cagaacttct gagattgtgg aagaattttt gcaaccagca 5031
gggaaagagg cctcttactg cctaaacaca aagttacact gagcttttct actgtccttt 5091
gcctattgct ccctctatca tgtaaagatc tgggaaggat gagaggcagg gcctgcttgt 5151
catgagctgc actcttttct ttttaactaa tcattgacaa ttggaagaaa attgacgtta 5211
aagaagtttc tccattgtct tactaacaaa accttttggg tttcattaat tgtccttgaa 5271
attgagttcc tttggcattt ttccttgcag tcatcagtta agcatgttgc atcctgaatt 5331
cacagaagtt tagctttgca ggtttgaatc tctgtaattt aactcccgtg gacttggtcg 5391
agttttcagc aggttgggag ccacctctct tcatttcagc agtgagtcat cccttgactt 5451

ttcaaatgac agaatttttt ccaattgtaa aattagcact gtaaaacaaa gaaccaaagt 5511

ggcatcctaa gagttgttaa acctgaagtc tagtttatga ggaattgtcc aagttggagt 5571

ttaaatagta tctgcttttg tctcaaagca tctaagttat tctgacagaa aatggtaagt 5631

cagctttgca ggcagatgcg cctctgggcc tcctaccttg ctccacagct ttctggccat 5691

cttgtctccc aggccatgcc actgctctgc cacatgtcag caaatttctt tccaccagtc 5751

ttatagcatc ttacatgatc aaatcatcac agaataaccc cgtgatagat tattgatagc 5811

aatagagagg ggcggctttg tcactgattt ttctctcaga ttccttttcc atctctcatc 5871

cataaaggaa ggactgaaat ccaaaggcat tctccttttg tacctacagt atccagaacc 5931

cacgtgggca gccttctgct tatgacaata attggcccat tgcatgcaga gagaatgtct 5991

tcatagagag aatgtcatta aatacttgaa tctgcatgac agtttgactt gaatgcaaca 6051

gcaggaaaat tttgcaagtt acataattgt atatacagta ggttttctta agtctcttcg 6111

gttcatcctt tgtaatttgt gtgtgtatct gtagtattgc aggcttttgg agactattct 6171

tacaggcagt atgtcagtca tcaaagaaaa tgctgtcacc tgccattgtt gtatttgtgg 6231

gtatttatag ttgtatgtat gtaaatgcat cagtgtgtag attgcatatc agtgtatggt 6291

acatgtacat caaaattatt tttgtcctta atcagtgtga tatgaaaagc aagtacaacc 6351

tcataggact gattatataa tgaagttgtt gagagtatat atagtggtat tgttttatta 6411

aacttaaact caaataatat tttgattaaa atttttaata agactttatg ctagaaaatt 6471

ctttgagctt tgaatcacca gggcaaaaat gactttcaac taaccttgtg aatcttttgc 6531

agtgtactgt gtgcaatacc aagggcatag ctccctgtaa tttgggaaat acagaaagaa 6591

aagaaaaaaa aaaaaa 6607

<210> 49
<211> 4173
<212> DNA
<213> Homo sapiens

<220>
<221> CDS
<222> (3)..(704)

<400> 49
aa atg gcg gct tcc agg tgg gcg cgc aag gcc gtg gtc ctg ctt tgt 47
Met Ala Ala Ser Arg Trp Ala Arg Lys Ala Val Val Leu Leu Cys
1               5                   10                  15

gcc tct gac ctg ctg ctg ctg ctg cta ctg cta cca ccg cct ggg tcc 95
Ala Ser Asp Leu Leu Leu Leu Leu Leu Leu Leu Pro Pro Pro Gly Ser
                20                  25                  30

tgc gcg gcc gaa ggc tcg ccc ggg acg ccc gac gag tct acc cca cct     143
Cys Ala Ala Glu Gly Ser Pro Gly Thr Pro Asp Glu Ser Thr Pro Pro
            35                  40                  45

ccc cgg aag aag aag aag gat att cgc gat tac aat gat gca gac atg     191
Pro Arg Lys Lys Lys Lys Asp Ile Arg Asp Tyr Asn Asp Ala Asp Met
        50                  55                  60

gcg cgt ctt ctg gag caa tgg gag aaa gat gat gac att gaa gaa gga     239
Ala Arg Leu Leu Glu Gln Trp Glu Lys Asp Asp Asp Ile Glu Glu Gly
    65                  70                  75

gat ctt cca gag cac aag aga cct tca gca cct gtc gac ttc tca aag     287
Asp Leu Pro Glu His Lys Arg Pro Ser Ala Pro Val Asp Phe Ser Lys
80                  85                  90                  95

ata gac cca agc aag cct gaa agc ata ttg aaa atg acg aaa aaa ggg     335
Ile Asp Pro Ser Lys Pro Glu Ser Ile Leu Lys Met Thr Lys Lys Gly
                100                 105                 110

aag act ctc atg atg ttt gtc act gta tca gga agc cct act gag aag     383
Lys Thr Leu Met Met Phe Val Thr Val Ser Gly Ser Pro Thr Glu Lys
            115                 120                 125

gag aca gag gaa att acg agc ctc tgg cag ggc agc ctt ttc aat gcc     431
Glu Thr Glu Glu Ile Thr Ser Leu Trp Gln Gly Ser Leu Phe Asn Ala
        130                 135                 140

aac tat gac gtc cag agg ttc att gtg gga tca gac cgt gct atc ttc     479
Asn Tyr Asp Val Gln Arg Phe Ile Val Gly Ser Asp Arg Ala Ile Phe
    145                 150                 155

atg ctt cgc gat ggg agc tac gcc tgg gag atc aag gac ttt ttg gtc     527
Met Leu Arg Asp Gly Ser Tyr Ala Trp Glu Ile Lys Asp Phe Leu Val
160                 165                 170                 175

ggt caa gac agg tgt gct gat gta act ctg gag ggc cag gtg tac ccc     575
Gly Gln Asp Arg Cys Ala Asp Val Thr Leu Glu Gly Gln Val Tyr Pro
                180                 185                 190

ggc aaa gga gga gga agc aaa gag aaa aat aaa aca aag caa gac aag     623
Gly Lys Gly Gly Gly Ser Lys Glu Lys Asn Lys Thr Lys Gln Asp Lys
            195                 200                 205

ggc aaa aaa aag aag gaa gga gat ctg aaa tct cgg tct tcc aag gaa     671
Gly Lys Lys Lys Lys Glu Gly Asp Leu Lys Ser Arg Ser Ser Lys Glu
        210                 215                 220

gaa aat cga gct ggg aat aaa aga gaa gac ctg tgatgggg cagcagtgac     722
Glu Asn Arg Ala Gly Asn Lys Arg Glu Asp Leu
    225                 230

gcgctgtggg gggacaggtg gacgtggaga gctctttgcc cagctcctgg ggtgggagtg     782

gtctcaggca actgcacacc ggatgacatt ctagtgtctt ctagaaaggg tctgccacat     842

gaccagtttg tggtcaaaga attactgctt aataggcttc aagtaagaag acagatgttt     902

tctaattaat actggacact gacaaattca tgtttactat aaaatctcct tacatggaaa     962

tgtgactgtg ttgctttttc ccatttacac ttggtgagtc atcaactcta ctgagattcc 1022

actcccctcc aagcacctgc tgtgattggg tggcctgctc tgatcagata gcaaattctg 1082

atcagagaag actttaaaac tcttgactta attgagtaaa ctcttcatgc catatacatc 1142

attttcatta tgttaaaggt aaaatatgct ttgtgaactc agatgtctgt agccaggaag 1202

ccagggtgtg taaatccaaa atctatgcag gaaatgcgga gaatagaaaa tatgtcactt 1262

gaaatcctaa gtagttttga atttctttga cttgaatctt actcatcagt aagagaactc 1322

ttggtgtctg tcaggtttta tgtggtctgt aaagttaggg gttctgtttt gtttccttat 1382

ttaggaaaga gtactgctgg tgtcgagggg ttatatgttc catttaatgt gacagtttta 1442

aaggatttaa gtagggaatc agagtccttt gcagagtgtg acagacgact caataacctc 1502

atttgtttct aaacattttt ctttgataaa gtgcctaaat ctgtgctttc gtatagagta 1562

acatgatgtg ctactgttga tgtctgattt tgccgttcat gttagagcct actgtgaata 1622

agagttagaa catttatata cagatgtcat ttctaagaac taaaattctt tgggaaaaac 1682

cctcaattgt gattttaata aattaaaagt agcacattac atggttaaaa aatgtcagtg 1742

ttaaagaatg gtacaaagtg aaaagtgtat ccctctcttg ccgccggtgg tagcttgtcc 1802

cagtggaagc tgctgttaac aatttgtgcc cccacatccc cctccctgcc catccaccaa 1862

aaaaaagtac atttacttat gtaaatgtac ttatggtgat gtatgtttgt tttggcctca 1922

cagcatctgt ttccccttaa tttggtagct gctcacattt ccctcgaaag aaccacaccc 1982

tctgcattct cagttctttg ctttggatgg gacatttgcc ctgcagtccc cccaccctcc 2042

aggccatgcc ctctccaggg tgaggcctgt gtgatctacc gtactagggt actaggccct 2102

gaaagaggct tttcttgttc ctcttgcatc ttgaacctgg agcgggagct gttgtaggcc 2162

ccgcccttgg agaagagaac tgtctgacag tggggagaga gcgccacacc ctggtggcat 2222

aaacgagtcc ctgaatcatg ccgtggctga accaagccct gtctgtgggc tttttctgtt 2282

gtactcaggg cagtttgatg gggttactgt cctgcatagc cataatggcc cagtataaag 2342

cagctgtttt gatgagataa ttgctttaat taagcaaaag gtagcaaagc tttcactccg 2402

ccctgtacct tctgtttcca cttaggagcc ttcccatgtc agaatgtgca gatctgtctc 2462

attgtttcct gtgcagtgtg cccccacttc acccagtagt ttctgtgtgt ctgttatgta 2522

ctaggtacta caaggtgcca ggacggtgta gatacagcct ctgctatcgt aaaactcaat 2582

gattcggtgg gggaagacaa atgtcagtaa tgtacaaagt aaaatggcag ctgttagaag 2642

tatgaaaggg gcagggtagg gggaggtaga atcttccctg accaggttaa gaaaaccaga 2702

ggccttctct gagggcaaga ggaggagagg agaaatagag taaggcaggc agaggaaaca 2762

gtctgagcta agaccctgtg gctagaagtg gcagagggag aggcagcagg aaggccagcg 2822

gggaggctgg ggcccagtgc aggcccaggt tggaggagcg tagcacatgg agtttggtag 2882

gagtttggga cgccctggtg gatcttaatt gtgatggggt gggtgtgaaa ggcagtccag 2942

gttgcactgg ttgcacagga gaagtgatca gaagaggacc ccagcaggtg tgagccgtga 3002

gctgggaggt gcttcagtag tgcaggccat agctgaaggt gtcctacatc agcagggtga 3062

tggtgaggtt tgaaccactg tttcactgca tagtccctgc tgatggacac ttgagtgttc 3122

agattttttg ctggtatatt cagtgctgca gtggacattt tcatacaaaa tatttcggta 3182

cacttttgtt tatatctgaa aggtaaattc ctagcagtag aattattaga gcaaacggaa 3242

tttaacattt tggtgtgtat tgccaaattg ccctcccaag tggtttagtc agcttaccct 3302

tgccaacaat agatctatcc ttgccagcct tgggcatcac atttaccagt ttaatagatt 3362

gtaaaaccat atcttaattg gctaccctga agccaccata ctggagaggc tgcgtacagt 3422

gtttcacgta gagagaggga tacccaggag gcccacctgc tccaacccca gctgcatgag 3482

tcttcccagc ccaggcacag acatgtggat aagatttaaa catttccagc cccagccttc 3542

aagcaatcct agttgacact gaggggagcc aacataagct gagctgagaa acagtctgcc 3602

cagtctgcag attcatgagc aaaagaaatg ttgggctggg tacagtggct cacgcctgta 3662

atcccagtac tttgggaggc cgaggtgggt ggatcagttg aggtcaggag tttgagacca 3722

gcctggccaa catggtgaag ccctgtctct actaaaaatt agccgagtgt ggtggtgcgg 3782

gcctgtaatc ccagctactc aggtggctga ggcaggagaa tggcttgaac ccgggaggcg 3842

gaggttgcag tgagccaaga tcaggccact gcactccagc ctggatgacg ggatgagact 3902

ctgtctcaaa aaaacgaaac aaaaattttt taagagaaat gtcatttgtt tttgtttttg 3962

agacagggtc tcactctgtt gccctcacta gagtgcagta gggatcacgg ctcactgaag 4022

tctctaccta ccggctcaat tgatcttccc accacagcct cccaaatagc tgggagaaat 4082

gtcctgtttt taatgaattt gtcttccttt ttgtcttgtt tgttttaata tctagtgatc 4142

taataaattt ggatgatatc ttttgactat c 4173

<210> 50
<211> 911
<212> DNA
<213> Homo sapiens

<220>
<221> CDS
<222> (94)..(525)

<400> 50
cgcgggctgt ctcggaaact cagagccggg ttcctcccgg gtttctgccg ggtttctccc      60

tgcggctcct gggttgttga gactcttgtg aag atg gct tgc gct gcc gcg cgg      114
                                     Met Ala Cys Ala Ala Ala Arg
                                      1               5

tcc ccg gcc gac cag gac agg ttt att tgt atc tat cct gct tat tta      162
Ser Pro Ala Asp Gln Asp Arg Phe Ile Cys Ile Tyr Pro Ala Tyr Leu
        10                  15                  20

aat aat aag aag acc atc gca gag gga agg cga atc ccc ata agt aag      210
Asn Asn Lys Lys Thr Ile Ala Glu Gly Arg Arg Ile Pro Ile Ser Lys
    25                  30                  35

gct gtt gaa aat cct aca gct aca gag att caa gat gta tgt tca gca      258
Ala Val Glu Asn Pro Thr Ala Thr Glu Ile Gln Asp Val Cys Ser Ala
40                  45                  50                  55

gtt gga ctt aac gta ttt ctt gag aaa aat aaa atg tac tct aga gaa      306
Val Gly Leu Asn Val Phe Leu Glu Lys Asn Lys Met Tyr Ser Arg Glu
                60                  65                  70

tgg aat cgt gat gtc caa tac aga ggc aga gtc cgg gtc cag ctc aaa      354
Trp Asn Arg Asp Val Gln Tyr Arg Gly Arg Val Arg Val Gln Leu Lys
            75                  80                  85

cag gaa gat ggg agc ctc tgc ctt gta cag ttc cca tca cgt aag tca      402
Gln Glu Asp Gly Ser Leu Cys Leu Val Gln Phe Pro Ser Arg Lys Ser
        90                  95                  100

gta atg ttg tat gca gca gaa atg ata cct aaa cta aaa aca agg aca      450
Val Met Leu Tyr Ala Ala Glu Met Ile Pro Lys Leu Lys Thr Arg Thr
    105                 110                 115

caa aaa aca gga ggt gct gac caa agt ctt caa caa gga gag gga agt      498
Gln Lys Thr Gly Gly Ala Asp Gln Ser Leu Gln Gln Gly Glu Gly Ser
120                 125                 130                 135

aaa aaa ggg aaa gga aag aaa aag aag taacc tagtatcagc atcaagtatg      550
Lys Lys Gly Lys Gly Lys Lys Lys Lys
                140

tggtactact gtaagagaca tgaatggaga cttctaattt gtatcggagg gaaacagaag      610

ctttttgttt gcatcattta actgaactgt gaacccttgt gcctctcatc tttatcatcg      670

gagttgacag tgaaacaaat ttacatcaga agtttgcatc tcgcgtatat gccgtataaa      730

agaatttttt tgtctttcaa tgcagttttt tggaagaaaa tatttttaaa tggacaatgg      790

actgtacaat aagttacttg aaataagttg tttcagataa atttcaatta gatttaaaat      850

aaacattatg tccacctttt aagttaatga aataaaattt gaaactgaca aaaaaaaaaa      910

a                                                                     911

<210> 51
<211> 2346
<212> DNA
<213> Homo sapiens

<220>
<221> CDS
<222> (86)..(1561)

<400> 51
tctgacgacg caattcggca cgagggtggc gctggcggtt gctgtcagct gattcccggg 60

gttggtggca gcggcggtag cagca atg gac ttt ctc ctg ggg aac ccg ttc 112
Met Asp Phe Leu Leu Gly Asn Pro Phe
1               5

agc tct cca gtg gga cag cgc atc gag aaa gcc aca gat ggc tcc ctg 160
Ser Ser Pro Val Gly Gln Arg Ile Glu Lys Ala Thr Asp Gly Ser Leu
10                  15                  20                  25

cag agc gag gac tgg gcc ctc aac atg gag atc tgc gac atc atc aac 208
Gln Ser Glu Asp Trp Ala Leu Asn Met Glu Ile Cys Asp Ile Ile Asn
                30                  35                  40

gag acg gag gaa ggt ccc aaa gat gcc ctc cga gca gta aag aag aga 256
Glu Thr Glu Glu Gly Pro Lys Asp Ala Leu Arg Ala Val Lys Lys Arg
            45                  50                  55

atc gtg ggg aat aag aac ttc cac gag gtg atg ctg gct ctc aca gtc 304
Ile Val Gly Asn Lys Asn Phe His Glu Val Met Leu Ala Leu Thr Val
        60                  65                  70

tta gaa acc tgt gtc aag aac tgc ggg cac cgc ttc cac gtg ctg gtg 352
Leu Glu Thr Cys Val Lys Asn Cys Gly His Arg Phe His Val Leu Val
    75                  80                  85

gcc agc cag gac ttc gtg gag agt gtg ctg gtg agg acc atc ctg ccc 400
Ala Ser Gln Asp Phe Val Glu Ser Val Leu Val Arg Thr Ile Leu Pro
90                  95                  100                 105

aag aac aac cca ccc acc atc gtg cat gac aaa gtg ctc aac ctc atc 448
Lys Asn Asn Pro Pro Thr Ile Val His Asp Lys Val Leu Asn Leu Ile
                110                 115                 120

cag tcc tgg gct gac gcg ttc cgc agc tcg ccc gat ctg aca ggt gtg 496
Gln Ser Trp Ala Asp Ala Phe Arg Ser Ser Pro Asp Leu Thr Gly Val
            125                 130                 135

gtc acc atc tat gag gac ctg cgg agg aaa ggc ctg gag ttc ccc atg 544
Val Thr Ile Tyr Glu Asp Leu Arg Arg Lys Gly Leu Glu Phe Pro Met
        140                 145                 150

act gac ctg gac atg ctg tca ccc atc cac aca ccc cag agg acc gtg 592
Thr Asp Leu Asp Met Leu Ser Pro Ile His Thr Pro Gln Arg Thr Val
    155                 160                 165

ttc aac tca gag aca caa tca gga cag gat tct gtg ggc act gac tcc 640
Phe Asn Ser Glu Thr Gln Ser Gly Gln Asp Ser Val Gly Thr Asp Ser
170                 175                 180                 185

agc cag caa gag gac tct ggc cag cat gct gcc cct ctg ccc gcc ccg 688
Ser Gln Gln Glu Asp Ser Gly Gln His Ala Ala Pro Leu Pro Ala Pro
                190                 195                 200

ccc ata ctc tcc ggt gac acg ccc ata gca cca acc ccg gaa cag att 736
Pro Ile Leu Ser Gly Asp Thr Pro Ile Ala Pro Thr Pro Glu Gln Ile
            205                 210                 215

ggg aag ctg cgc agt gag ctg gag atg gtg agt ggg aac gtg agg gtg 784
Gly Lys Leu Arg Ser Glu Leu Glu Met Val Ser Gly Asn Val Arg Val
        220                 225                 230

atg tcg gag atg ctg acg gag ctg gtg ccc acc cag gcc gag ccc gca 832
Met Ser Glu Met Leu Thr Glu Leu Val Pro Thr Gln Ala Glu Pro Ala
    235                 240                 245

gac ctg gag ctg ctg cag gag ctc aac cgc acg tgc cga gcc atg cag 880
Asp Leu Glu Leu Leu Gln Glu Leu Asn Arg Thr Cys Arg Ala Met Gln
250                 255                 260                 265

cag cgg gtc ctg gag ctc atc cct cag atc gcc aat gag cag ctg aca 928
Gln Arg Val Leu Glu Leu Ile Pro Gln Ile Ala Asn Glu Gln Leu Thr
                270                 275                 280

gag gag ctg ctc atc gtc aat gac aat ctc aac aat gtg ttc ctg cgc 976
Glu Glu Leu Leu Ile Val Asn Asp Asn Leu Asn Asn Val Phe Leu Arg
            285                 290                 295

cat gaa cgg ttt gaa cgg ttc cga aca ggc cag acc acc aag gcc cca 1024
His Glu Arg Phe Glu Arg Phe Arg Thr Gly Gln Thr Thr Lys Ala Pro
        300                 305                 310

agt gag gcc gag ccg gca gct gac ctg atc gac atg ggc cct gac cca 1072
Ser Glu Ala Glu Pro Ala Ala Asp Leu Ile Asp Met Gly Pro Asp Pro
    315                 320                 325

gca gcc acc ggc aac ctc tca tcc cag ctg gca gga atg aac ctg ggc 1120
Ala Ala Thr Gly Asn Leu Ser Ser Gln Leu Ala Gly Met Asn Leu Gly
330                 335                 340                 345

tcc agc agt gtg aga gct ggc ctg cag tct ctg gag gcc tct ggt cga 1168
Ser Ser Ser Val Arg Ala Gly Leu Gln Ser Leu Glu Ala Ser Gly Arg
                350                 355                 360

ctg gaa gat gag ttt gac atg ttt gcg ctg aca cgg ggc agc tca ctg 1216
Leu Glu Asp Glu Phe Asp Met Phe Ala Leu Thr Arg Gly Ser Ser Leu
            365                 370                 375

gct gac caa cgg aaa gag gta aaa tac gaa gcc ccc caa gca aca gac 1264
Ala Asp Gln Arg Lys Glu Val Lys Tyr Glu Ala Pro Gln Ala Thr Asp
        380                 385                 390

ggc ctg gct gga gcc ctg gac gcc cgg cag cag agc act ggc gcg atc 1312
Gly Leu Ala Gly Ala Leu Asp Ala Arg Gln Gln Ser Thr Gly Ala Ile
    395                 400                 405

cca gtc acc cag gcc tgc ctc atg gag gac atc gag cag tgg ctg tcc 1360
Pro Val Thr Gln Ala Cys Leu Met Glu Asp Ile Glu Gln Trp Leu Ser
410                 415                 420                 425

act gac gtg ggt aat gat gcg gaa gag cct aag ggg gtc acc agc gaa     1408
Thr Asp Val Gly Asn Asp Ala Glu Glu Pro Lys Gly Val Thr Ser Glu
            430                 435                 440

gaa ttt gac aaa ttc ctg gaa gaa cgg gcc aaa gcc gcg gac cga ttg     1456
Glu Phe Asp Lys Phe Leu Glu Glu Arg Ala Lys Ala Ala Asp Arg Leu
        445                 450                 455

ccc aac ctc tcc agc ccc tca gct gag ggg ccc ccg ggt ccc cca tct     1504
Pro Asn Leu Ser Ser Pro Ser Ala Glu Gly Pro Pro Gly Pro Pro Ser
    460                 465                 470

ggc cca gcg ccc cgg aag aag acc cag gag aaa gat gat gac atg ctg     1552
Gly Pro Ala Pro Arg Lys Lys Thr Gln Glu Lys Asp Asp Asp Met Leu
475                 480                 485

ttt gcc tta tgagtgt ggggtctggc accctgcagc ccaggtcccc actgctctca     1608
Phe Ala Leu
490

cacccttagg ctgggacctc cctccctcct ctggtgttaa ggctgctttg ggggtggctt     1668

gttaccccct tttcctcctc tttgaagacg gagctgcccc agctgtggct gggggtgtgg     1728

aggcagtggg atgaactggg ggacaggtct gcgctgcagt gggatctggc tgctctgcct     1788

cctttcccac cccagctgac catgagactt tgctgagaag tggaggcccc aggacaggct     1848

ggctggctgg ctggctgctt gacccagtgt gactctcctt cactgagtga taccctgctc     1908

cgggcccatg ccccaaggag cccttcagag cccacactgc cagtcgaggc ctggctggag     1968

gctggccaca gtggaaattc tgccgagcct cttgtccctt ccctgctctg ctgcatgggg     2028

ccccatggct ttggctggcc actgagggta gggtgtggag gtgtggaggc cccctgagga     2088

gctgcggcgg cccaggtacg aagctgcaac tctgcgcgca gtgggcgaga tctcatcagc     2148

cccaggctgc aggtgaggct tcaggggatg ctgggcccca ctgcccctcc gctgccttgc     2208

cctccatcct tcctctgttc cttctggccg ggcaccacag cactggggct cacctcttgg     2268

ttgatcctct tgtactggga gaggtgcctt ttgtatcccc aattaaaggt agaaaaccac     2328

cctgctaaaa aaaaaaaa                                                  2346

<210> 52
<211> 762
<212> DNA
<213> Homo sapiens

<220>
<221> CDS
<222> (313)..(489)

<400> 52

aagtattgaa atgtcttttt aaaattttaa ccaccgtatt tattggttca aaaactagaa 60

tttatagttt caggcagatt tcaaccaaag agtcaccaaa ttaaatacac agggtagctt 120

gtgaggcata gacacagccc atgtgttttc ctctacattg tatattcatt tctctttggc 180

gatttgacat tatagccatt ctctggaagt cctaaagcaa actagtattt tatgtgccat 240

attaagttaa aatttcttat gtgagatacc actaatactg gttttgattt aggccatcct 300

tcttgcctgg at atg aca atg gag ctt gtt tct atg att aag acc tac 348
Met Thr Met Glu Leu Val Ser Met Ile Lys Thr Tyr
1               5                   10

cca tgg cag tgt atg gaa tgt aaa aca tgc att ata tgt gga caa ccc 396
Pro Trp Gln Cys Met Glu Cys Lys Thr Cys Ile Ile Cys Gly Gln Pro
        15                  20                  25

cac cat gaa gaa gaa atg atg ttc tgt gat atg tgt gac aga ggt tat 444
His His Glu Glu Glu Met Met Phe Cys Asp Met Cys Asp Arg Gly Tyr
    30                  35                  40

cat act ttt tgt gtg ggc ctt ggt gct att cca tca ggt aaa gat taa 492
His Thr Phe Cys Val Gly Leu Gly Ala Ile Pro Ser Gly Lys Asp
45                  50                  55

aaaaagagca aacattatct ttgggtgatg agactctccc agcactccaa aaggctgcat 552

aaacgctatc atgacatgaa gagcttgtgg actcttatgt gcaaatatga ccaaagctgt 612

gattatacag gtcatgtcca ccagcttttc atccctagac aattccaact aaaggataag 672

tgaaaggcca gaatgcccct gctaagtttt ggaagtatag ttctatagaa tgagctggtg 732

tttttctact gaaatactaa agtaacaaag 762

<210> 53
<211> 537
<212> DNA
<213> Homo sapiens

<220>
<221> CDS
<222> (208)..(321)

<400> 53
aattagtttc agtggcttga ttctaggcca agattctggc aacagattgt agtcttacct 60

tgttttcttc aatctcactg gatctctctc tcttttaccc cccttaggc tgagggtaaa 120

aagctgggat tggtaggctg ggtccagaac actgaccggg gcacagtgca aggacaattg 180

caaggtccca tctccaaggt gcgtcat atg cag gaa tgg ctt gaa aca aga 231
Met Gln Glu Trp Leu Glu Thr Arg
1               5

gga agt cct aaa tca cac atc gac aaa gca aac ttc aac aat gaa aaa 279

Gly Ser Pro Lys Ser His Ile Asp Lys Ala Asn Phe Asn Asn Glu Lys
10                  15                  20

gtc atc ttg aag ttg gat tac tca gac ttc caa att gta aaa taatggc     328
Val Ile Leu Lys Leu Asp Tyr Ser Asp Phe Gln Ile Val Lys
25                  30                  35

ctgaatttaa gttttctaag ataaactcag tggtttggtt tttattatta atagagatag     388

aactattgtg tgttaatgtt agcattagtc aataagttat tttaatgtca gatttttgaa     448

tgttattata tattacctgt atgatggaag gattaccact gtacacaaat ctaatcaata     508

aaaacgttag aaccttcaaa aaaaaaaaa                                        537

<210> 54
<211> 509
<212> DNA
<213> Homo sapiens

<220>
<221> CDS
<222> (201)..(413)

<400> 54
ttcttgttaa agccaaaaaa actttccaga cagaataaaa cataaatcaa actatatgcc      60

caagagccac atctgaaaca ggctggacgc aaaatgatga aagacacatt ggtcaaattc     120

caaccaaatg actatcatat gaagtacact ttgttttatc tcatttttat tattcttatc     180

ttttatatat atttttgag atg gag tct cac tct gtc acc cag gct gca     230
                     
                     1               5                   10

gtg cag tgg cac cat ctc ggc tca ctg caa cct ctg cct ccc ggg ttc     278

                15                  20                  25

aag caa ttc tct gcc tca gcc tgc cga gta gct ggg att aca gat gcc     326

            30                  35                  40

cgc cac cat gcc tgg cta att ttt gta ttt tta gta gag acg ggg ttt     374

        45                  50                  55

cac cat ctt ggg cag tct ggt ctt gaa ctc ctg atc tca tgatccaccc     423

    60                  65                  70

gccttggcct ccctaagtgc tgggattaca ggcatgagcc accacaccca gccatgaagt     483

ataggtcgac cggccgcgaa tttagt                                          509

<210> 55
<211> 2993
<212> DNA
<213> Homo sapiens

<220>
<221> CDS
<222> (1)..(1641)

<400> 55
atg atc gct gat gat aag cca gct gcg atg gct ggt gag tca gct gga      48
Met Ile Ala Asp Asp Lys Pro Ala Ala Met Ala Gly Glu Ser Ala Gly
1               5                   10                  15

cag tca tct gag tct gga gtt ggg gcc aac ttc ttt ggc atc aca ttc      96
Gln Ser Ser Glu Ser Gly Val Gly Ala Asn Phe Phe Gly Ile Thr Phe
            20                  25                  30

cag aca aca gaa aca ctg atg agc aca ggg cat ctg aat ggg gcc gaa      144
Gln Thr Thr Glu Thr Leu Met Ser Thr Gly His Leu Asn Gly Ala Glu
        35                  40                  45

tgc aaa gca ggt cca ggc act gtg aag acc ctg gcg gtg gag gaa gag      192
Cys Lys Ala Gly Pro Gly Thr Val Lys Thr Leu Ala Val Glu Glu Glu
    50                  55                  60

gct tcc cgg ctg tgg agg aag cca gac cct tac aac aca aga cga gaa      240
Ala Ser Arg Leu Trp Arg Lys Pro Asp Pro Tyr Asn Thr Arg Arg Glu
65                  70                  75                  80

cca gac ctg cgt ggg gga gct ctg gat gct aca ggg gct caa gga ggg      288
Pro Asp Leu Arg Gly Gly Ala Leu Asp Ala Thr Gly Ala Gln Gly Gly
                85                  90                  95

cca ctg gac cga gcg cgc aaa gaa cct gag acc gct tgc tct cac cgc      336
Pro Leu Asp Arg Ala Arg Lys Glu Pro Glu Thr Ala Cys Ser His Arg
            100                 105                 110

cgc aag tcg gtc gca gga cag aca cca gtg ggc agc aac aaa aaa aga      384
Arg Lys Ser Val Ala Gly Gln Thr Pro Val Gly Ser Asn Lys Lys Arg
        115                 120                 125

aac cgg gtt ccg gga cac gtg ccg gcg gct gga cta acc tca gcg gct      432
Asn Arg Val Pro Gly His Val Pro Ala Ala Gly Leu Thr Ser Ala Ala
    130                 135                 140

gca acc aag gag cgc gca caa tgc tcc gat aca ggg ggt ctg gat ccc      480
Ala Thr Lys Glu Arg Ala Gln Cys Ser Asp Thr Gly Gly Leu Asp Pro
145                 150                 155                 160

tac tct gcg ggc cat ttc tcc aga gcg act ttg ctc ttc tgt cct ccc      528
Tyr Ser Ala Gly His Phe Ser Arg Ala Thr Leu Leu Phe Cys Pro Pro
                165                 170                 175

cac act cac cgc tgc atc tcc ctc acc aaa agc gag aag tcg gag cga      576
His Thr His Arg Cys Ile Ser Leu Thr Lys Ser Glu Lys Ser Glu Arg
            180                 185                 190

caa cag ctc ttt ctg ccc aag ccc cag tca gct gtt ttc ggg tcc gag      624

Gln Gln Leu Phe Leu Pro Lys Pro Gln Ser Ala Val Phe Gly Ser Glu
        195                 200                 205

gga agg agg acc ctg cga aag ctg cga cga cta tct tcc cct ggg gcc 672
Gly Arg Arg Thr Leu Arg Lys Leu Arg Arg Leu Ser Ser Pro Gly Ala
    210                 215                 220

atg gac tcg gac gcc agc ctg gtg tcc agc cgc ccg tcg tcg cca gag 720
Met Asp Ser Asp Ala Ser Leu Val Ser Ser Arg Pro Ser Ser Pro Glu
225                 230                 235                 240

ccc gat gac ctt ttt ctg ccg gcc cgg agt aag ggc agc agc ggc agc 768
Pro Asp Asp Leu Phe Leu Pro Ala Arg Ser Lys Gly Ser Ser Gly Ser
                245                 250                 255

gcc ttc act ggg ggc acc gtg tcc tcg tcc acc ccg agt gac tgc ccg 816
Ala Phe Thr Gly Gly Thr Val Ser Ser Ser Thr Pro Ser Asp Cys Pro
            260                 265                 270

ccg gag ctg agc gcc gag ctg cgc ggc gct atg ggc tct gcg ggc gcg 864
Pro Glu Leu Ser Ala Glu Leu Arg Gly Ala Met Gly Ser Ala Gly Ala
        275                 280                 285

cat cct ggg gac aag cta gga ggc agt ggc ttc aag tca tcc tcg tcc 912
His Pro Gly Asp Lys Leu Gly Gly Ser Gly Phe Lys Ser Ser Ser Ser
    290                 295                 300

agc acc tcg tcg tct acg tcg tcg gcg gct gcg tcg tcc acc aag aag 960
Ser Thr Ser Ser Ser Thr Ser Ser Ala Ala Ala Ser Ser Thr Lys Lys
305                 310                 315                 320

gac aag aag caa atg aca gag ccg gag ctg cag cag ctg cgt ctc aag 1008
Asp Lys Lys Gln Met Thr Glu Pro Glu Leu Gln Gln Leu Arg Leu Lys
                325                 330                 335

atc aac agc cgc gag cgc aag cgc atg cac gac ctc aac atc gcc atg 1056
Ile Asn Ser Arg Glu Arg Lys Arg Met His Asp Leu Asn Ile Ala Met
            340                 345                 350

gat ggc ctc cgc gag gtc atg ccg tac gca cac ggc cct tcg gtg cgc 1104
Asp Gly Leu Arg Glu Val Met Pro Tyr Ala His Gly Pro Ser Val Arg
        355                 360                 365

aag ctt tcc aag atc gcc acg ctg ctg ctg gcg cgc aac tac atc ctc 1152
Lys Leu Ser Lys Ile Ala Thr Leu Leu Leu Ala Arg Asn Tyr Ile Leu
    370                 375                 380

atg ctc acc aac tcg ctg gag gag atg aag cga ctg gtg agc gag atc 1200
Met Leu Thr Asn Ser Leu Glu Glu Met Lys Arg Leu Val Ser Glu Ile
385                 390                 395                 400

tac ggg ggc cac cac gct ggc ttc cac ccg tcg gcc tgc ggc ggc ctg 1248
Tyr Gly Gly His His Ala Gly Phe His Pro Ser Ala Cys Gly Gly Leu
                405                 410                 415

gcg cac tcc gcg ccc ctg ccc gcc gcc acc gcg cac ccg gca gca gca 1296
Ala His Ser Ala Pro Leu Pro Ala Ala Thr Ala His Pro Ala Ala Ala
            420                 425                 430

gcg cac gcc gca cat cac ccc gcg gtg cac cac ccc atc ctg ccg ccc 1344

Ala His Ala Ala His His Pro Ala Val His His Pro Ile Leu Pro Pro
        435                 440                 445

gcc gcc gca gcg gct gct gcc gcc gct gca gcc gcg gct gtg tcc agc      1392
Ala Ala Ala Ala Ala Ala Ala Ala Ala Ala Ala Ala Ala Val Ser Ser
    450                 455                 460

gcc tct ctg ccc gga tcc ggg ctg ccg tcg gtc ggc tcc atc cgt cca      1440
Ala Ser Leu Pro Gly Ser Gly Leu Pro Ser Val Gly Ser Ile Arg Pro
465                 470                 475                 480

ccg cac ggc cta ctc aag tct ccg tct gct gcc gcg gcc gcc ccg ctg      1488
Pro His Gly Leu Leu Lys Ser Pro Ser Ala Ala Ala Ala Ala Pro Leu
                485                 490                 495

ggg ggc ggg ggc ggc ggc agt ggg gcg agc ggg ggc ttc cag cac tgg      1536
Gly Gly Gly Gly Gly Gly Ser Gly Ala Ser Gly Gly Phe Gln His Trp
            500                 505                 510

ggc ggc atg ccc tgc ccc tgc agc atg tgc cag gtg ccg ccg ccg cac      1584
Gly Gly Met Pro Cys Pro Cys Ser Met Cys Gln Val Pro Pro Pro His
        515                 520                 525

cac cac gtg tcg gct atg ggc gcc ggc agc ctg ccg cgc ctc acc tcc      1632
His His Val Ser Ala Met Gly Ala Gly Ser Leu Pro Arg Leu Thr Ser
    530                 535                 540

gac gcc aag tgagcct actggcgccg gcgcgttctg gcgacagggg agccaggggc      1688
Asp Ala Lys
545

cgcggggaag cgaggactgg cctgcgctgg gctcgggagc tctgtcgcga ggaggggcgc      1748

aggaccatgg actgggggtg gggcatggtg gggattccag catctgcgaa cccaagcaat      1808

gggggcgccc acagagcagt ggggagtgag gggatgttct ctccgggacc tgatcgagcg      1868

ctgtctggct ttaacctgag ctggtccagt agacatcgtt ttatgaaaag gtaccgctgt      1928

gtgcattcct cactagaact catccgaccc ccgaccccca cctccgggaa aagattctaa      1988

aaacttcttt ccctgagagc gtggcctgac ttgcagactc ggcttgggca gcacttcggg      2048

gggggagggg gttttatggg agggggacac attggggcct tgctcctctt cctcctttct      2108

tggcgggtgg gagactccgg gtagccgcac tgcagaagca acagcccgac cgcgccctcc      2168

agggtcgtcc ctggcccaag gccaggggcc acaagttagt tggaagccgg cgttcggtat      2228

cagaagcgct gatggtcata tccaatctca atatctgggt caatccacac cctcttagaa      2288

ctgtggccgt tcctccctgt ctctcgttga tttgggagaa tatggttttc taataaatct      2348

gtggatgttc cttcttcaac agtatgagca agtttataga cattcagagt agaaccactt      2408

gtggattgga ataacccaaa actgccgatt tcaggggcgg gtgcattgta gttattattt      2468

taaaatagaa actaccccac cgactcatct ttccttctct aagcacaaag tgatttggtt      2528

attttggtac ctgagaacgt aacagaatta aaaggcagtt gctgtggaaa cagtttgggt      2588

tatttggggg ttctgttggc tttttaaaat tttctttttt ggatgtgtaa atttatcaat 2648

gatgaggtaa gtgcgcaatg ctaagctgtt tgctcacgtg actgccagcc ccatcggagt 2708

ctaagccggc tttcctctat tttggtttat ttttgccacg tttaacacaa atggtaaact 2768

cctccacgtg cttcctgcgt tccgtgcaag ccgcctcggc gctgcctgcg ttgcaaactg 2828

ggctttgtag cgtctgccgt gtaacaccct tcctctgatc gcaccgcccc tcgcagagag 2888

tgtatcatct gttttatttt tgtaaaaaca aagtgctaaa taatatttat tacttgtttg 2948

gttgcaaaaa cggaataaat gactgagtgt tgagatttta aaaaa 2993

<210> 56
<211> 866
<212> DNA
<213> Homo sapiens

<220>
<221> CDS
<222> (310)..(510)

<400> 56
cccggtcgac ccacgcgtcc gcggacgcgt ggggcaatat atacactatt atatacccac 60

aaaaaataaa aataaaggct gggtgccatg gctcacacct gtaatcccac cacttcagga 120

ggcctgaggt cgggagtttg agaccaacct ggccaacatg gagaaaccac gtctctactg 180

aaaatacaaa atcagctggg catggtggta catgcctata atcccagcta ctcgggaggc 240

tgaggcagga caatcgcctg aaccctggag gtggaggcta cggtgagccg agatcgtgct 300

gagagcaag atg ggt cac cag cag ctg tac tgg agc cac ccg cga aaa 348
Met Gly His Gln Gln Leu Tyr Trp Ser His Pro Arg Lys
1               5                   10

ttc ggc cag ggt tct cgc tct tgt cgt gtc tgt tca aac cgg cac ggt 396
Phe Gly Gln Gly Ser Arg Ser Cys Arg Val Cys Ser Asn Arg His Gly
    15                  20                  25

ctg atc cgg aaa tat ggc ctc aat atg tgc cgc cag tgt ttc cgt cag 444
Leu Ile Arg Lys Tyr Gly Leu Asn Met Cys Arg Gln Cys Phe Arg Gln
30                  35                  40                  45

tac gcg aag gat atc ggt ttc att aag aaa gac ctg agc tgt ctt cct 492
Tyr Ala Lys Asp Ile Gly Phe Ile Lys Lys Asp Leu Ser Cys Leu Pro
                50                  55                  60

tgg cac tgc cta tgg agg tgacac ccatctcctc catcatggcc atcctgagac 546
Trp His Cys Leu Trp Arg
            65

cgctcgcgaa gcccaagatc atcaaaaaga gcaccaagtt cactgggaac cagtcagact 606

gatatgtcaa aattaagggt aactggtgga aacacagagg tattgacaac agggttcata    666

gaaggtttga gggccagatc tatgcccaac attggttatg ggagaaacaa aaagacaaag    726

cacatactgc ccagtggctt ctggaagttc ctggtccaca acgttaagga gctggaagta    786

ctgctggtga gcaacaaatc ttactgtgtt gagatcactc atgatgtttc ttccaagaac    846

tgcaaagcca tcttggaaag                                                866

<210> 57
<211> 676
<212> DNA
<213> Homo sapiens

<220>
<221> CDS
<222> (175)..(396)

<400> 57
atttggccct cgagcagcaa attcggcacg aggagcggct ggagaggtgg tcggagaagt     60

aggaacctcc tgccgggctc gtggcggctt ctgtccgctc cgcgagggaa gcgccttccc    120

cacaggacat caatgcaagc ttgaataaga aaaacaaatt cttcctccta agcc atg      177
                                                            Met
                                                            1

gca tat cag tta tac aga aat act act ttg gga aac agt ctt cag gag      225
Ala Tyr Gln Leu Tyr Arg Asn Thr Thr Leu Gly Asn Ser Leu Gln Glu
            5                   10                  15

agc cta gat gag ctc ata cag ggc tct cta aat acg tac aga ttc tgc      273
Ser Leu Asp Glu Leu Ile Gln Gly Ser Leu Asn Thr Tyr Arg Phe Cys
        20                  25                  30

gat aat gtg tgg act ttt gta ctg aat gat gtt gaa ttc aga gag gtg      321
Asp Asn Val Trp Thr Phe Val Leu Asn Asp Val Glu Phe Arg Glu Val
    35                  40                  45

aca gaa ctt att aaa gtg gat aaa gtg aaa att gta gcc tgt gat ggt      369
Thr Glu Leu Ile Lys Val Asp Lys Val Lys Ile Val Ala Cys Asp Gly
50                  55                  60                  65

aaa aat act ggc tcc aat act aca gaa tgaat agaaaaaata tgactttttt      421
Lys Asn Thr Gly Ser Asn Thr Thr Glu
                70

acaccatctt ctgttattca ttgcttttga agagaagcat agaagagact ttttatttat     481

tctagaattg cagaaatgac tacactgtgc tataccagag aattccagta gaaagaaact    541

tgtaactctg tagcctctta catcaccttt attatacagc atgaaaaacc ataacttttt    601

tttaaggaca aaagttgttg ccttcctaag aaccttcttt aataaactca ttttaaaact    661

ctgaaaaaaa aaaaa                                                     676

<210> 58
<211> 1915
<212> DNA
<213> Homo sapiens

<220>
<221> CDS
<222> (518)..(1021)

<400> 58
tgcgcggctt cccggcgctc gtggaatctt ctggagccct tttctccact cagcgagcct 60

gagagtgcgg aagtctccgg ctggtggggc atggcccagg agcactagtg atgatgcggg 120

tgtgctggtt ggtgagacag gacagccggc accagcgaat cagacttcca catttggaag 180

cagttgtgat tgggcgtggc ccagagacca agatcactga taagaaatgt tctcgacagc 240

aagtacagtt gaaagcagag tgtaacaagg gatatgtcaa ggtaaagcag agtttgagga 300

agaggcaaag aaccctggcc tggaaacaca caggaagaga aagagatcag gcaacagtga 360

ttctatagaa agggatgctg ctcaggaagc tgaggctggg acagggctgg aacctgggag 420

caactctggc caatgctctg tgcccctaaa gaagggaaaa gatgcaccta tcaaaaagga 480

atccctgggc cactggagtc aaggcttgaa gatttct atg cag gac ccc aaa atg 535
Met Gln Asp Pro Lys Met
1               5

cag gtt tac aaa gat gag cag gtg gtg gtg ata aag gat aaa tac cca 583
Gln Val Tyr Lys Asp Glu Gln Val Val Val Ile Lys Asp Lys Tyr Pro
            10                  15                  20

aag gcc cgt tac cat tgg ctg gtc tta ccg tgg acc tcc att tcc agt 631
Lys Ala Arg Tyr His Trp Leu Val Leu Pro Trp Thr Ser Ile Ser Ser
        25                  30                  35

ctg aag gct gtg gcc agg gaa cac ctt gaa ctc ctt aag cat atg cac 679
Leu Lys Ala Val Ala Arg Glu His Leu Glu Leu Leu Lys His Met His
    40                  45                  50

act gtg ggg gaa aag gtg att gta gat ttt gct ggg tcc agc aaa ctc 727
Thr Val Gly Glu Lys Val Ile Val Asp Phe Ala Gly Ser Ser Lys Leu
55                  60                  65                  70

cgc ttc cga ttg ggc tac cac gcc att ccg agt atg agc cat gta cat 775
Arg Phe Arg Leu Gly Tyr His Ala Ile Pro Ser Met Ser His Val His
                75                  80                  85

ctt cat gtg atc agc cag gat ttt gat tct cct tgc ctt aaa aac aaa 823
Leu His Val Ile Ser Gln Asp Phe Asp Ser Pro Cys Leu Lys Asn Lys
            90                  95                  100

aaa cat tgg aat tct ttc aat aca gaa tac ttc cta gaa tca caa gct 871
Lys His Trp Asn Ser Phe Asn Thr Glu Tyr Phe Leu Glu Ser Gln Ala

105                 110                 115

gtg atc gag atg gta caa gag gct ggt aga gta act gtc cga gat ggg     919
Val Ile Glu Met Val Gln Glu Ala Gly Arg Val Thr Val Arg Asp Gly
    120                 125                 130

atg cct gag ctc ttg aag ctg ccc ctt cgt tgt cat gag tgc cag cag     967
Met Pro Glu Leu Leu Lys Leu Pro Leu Arg Cys His Glu Cys Gln Gln
135                 140                 145                 150

ctg ctg cct tcc att cct cag ctg aaa gaa cat ctc agg aag cac tgg     1015
Leu Leu Pro Ser Ile Pro Gln Leu Lys Glu His Leu Arg Lys His Trp
                155                 160                 165

aca cag tgattctgca gagcctgagc tgctgctgtg gtgtggccca ctggagcaaa     1071
Thr Gln

ctgctggcac ctattctggg ttgcttgtga acttctactc atttcctaaa ttaaaacatg   1131

cagctttttc acaaatttat tctattattg agtggccaca atgtagagtg gctcaaagta   1191

cttcaggatt aggaatttgg gtttgtcata gatgtattct ctggtgaggg tggctgggat   1251

atacctgacc caccatcttc agaaggaccc atgtcaggtc tgaccattgg gagcaaagcc   1311

atgttcacac tgacctaatg cagagtatgg aagcattggg ctggttatac atttctgttt   1371

cttagattta tcctccgcct ctgtaggcat ggacaacctt taatcagagc atctagagtg   1431

gcctcttgtt tatcctgaag atactgatgg gtcttgtttt ctgttagtct gttttgtaat   1491

attcttttcc cttccttcat ggggaggctt agtttgtcca gtccttccat gcccttctat   1551

cccagattac ctaaatgttc ccttctcagg aattctgtct catcagttct tcacagtgag   1611

aaaagaggct agatgatggt gtggggggtt ggagttttct tctaataccg agggttcctg   1671

gctgtgagga aacagccaca tgttcgtcat gattgagctg tgaagtcttc ttggacctgt   1731

tgtctgaaaa taaagttaat ttgtttgagg catctctctt aagtaggtgg aaactattga   1791

agttcagcta acaatcacag cataggttct gatgcatgga aaggtggttg gtgaatgaaa   1851

aagttgcgta gagccactac tttctttttc cctgagaata aatttggata aaacaaaaaa   1911

aaaa                                                                1915

<210> 59
<211> 400
<212> DNA
<213> Homo sapiens

<220>
<221> CDS
<222> (50)..(343)

<400> 59
ttccggccgc gtcgacgcat cctgtccagc aaagaagcaa tcagccaaa atg ata 55
Met Ile
1

cct gga ggc tta tct gag gcc aaa ccc gcc act cca gaa atc cag gag 103
Pro Gly Gly Leu Ser Glu Ala Lys Pro Ala Thr Pro Glu Ile Gln Glu
        5                   10                  15

att gtt gat aag gtt aaa cca cag ctt gaa gaa aaa aca aat gag act 151
Ile Val Asp Lys Val Lys Pro Gln Leu Glu Glu Lys Thr Asn Glu Thr
    20                  25                  30

tac gga aaa ttg gaa gct gtg cag tat aaa act caa gtt gtt gct gga 199
Tyr Gly Lys Leu Glu Ala Val Gln Tyr Lys Thr Gln Val Val Ala Gly
35                  40                  45                  50

aca aat tac tac att aag gta cga gca ggt gat aat aaa tat atg cac 247
Thr Asn Tyr Tyr Ile Lys Val Arg Ala Gly Asp Asn Lys Tyr Met His
                55                  60                  65

ttg aaa gta ttc aaa agt ctt ccc gga caa aat gag gac ttg gta ctt 295
Leu Lys Val Phe Lys Ser Leu Pro Gly Gln Asn Glu Asp Leu Val Leu
            70                  75                  80

act gga tac cag gtt gac aaa aac aag gat gac gag ctg acg ggc ttt 343
Thr Gly Tyr Gln Val Asp Lys Asn Lys Asp Asp Glu Leu Thr Gly Phe
        85                  90                  95

tagcagcatg tacccaaagt gttctgattc cttcaactgg ctactgagtc atgatcc 400

<210> 60
<211> 3724
<212> DNA
<213> Homo sapiens

<220>
<221> CDS
<222> (351)..(3674)

<400> 60
gcggggcact ttgaagaatc acgtgtgttg aggccctctt aaagagatag gcacctactt 60

ttccctccac cctaaacacc gccctgaggg cggcggcgac cgtggtaatt gcaactgctc 120

aggggcaggg cttgccccag cgccgagtag tggcaacggc gtggttgcgt cgggggtgcc 180

tgggagcctg gagtcccggg ggcctgaaat cggcagcttc ccgggcagac actctctccc 240

tcaggaagag gtgccgccga gtcagcgcgg ggcagtgtga gcgccccgag gtgctttctc 300

agttgaggag aggtggggtt acagggcaca ggtgacaggg ccggagaaag atg gag 356
Met Glu
1

cag ccc ggg gcg gcg gcg tcg gga gcg gga ggc ggc agc gag gaa ccc 404
Gln Pro Gly Ala Ala Ala Ser Gly Ala Gly Gly Gly Ser Glu Glu Pro

5                   10                  15

ggt ggg ggc cgg agc aac aag cgg agc gcg ggg aac cgg gcc gcc aat      452
Gly Gly Gly Arg Ser Asn Lys Arg Ser Ala Gly Asn Arg Ala Ala Asn
    20                  25                  30

gaa gag gaa acg aaa aac aaa ccc aaa ttg aac att caa ata aaa act      500
Glu Glu Glu Thr Lys Asn Lys Pro Lys Leu Asn Ile Gln Ile Lys Thr
 35                  40                  45                  50

ttg gca gat gat gtg cgt gac cga att aca agt ttt aga aaa tct act      548
Leu Ala Asp Asp Val Arg Asp Arg Ile Thr Ser Phe Arg Lys Ser Thr
                 55                  60                  65

gtc aaa aaa gaa aaa cct ctt att caa cat cct att gat tct caa gtc      596
Val Lys Lys Glu Lys Pro Leu Ile Gln His Pro Ile Asp Ser Gln Val
             70                  75                  80

gcg atg agt gag ttt cct gca gct cag cca tta tat gat gaa cga tct      644
Ala Met Ser Glu Phe Pro Ala Ala Gln Pro Leu Tyr Asp Glu Arg Ser
         85                  90                  95

ttg aat ttg tca gaa aag gaa gta ttg gat ctc ttt gaa aaa atg atg      692
Leu Asn Leu Ser Glu Lys Glu Val Leu Asp Leu Phe Glu Lys Met Met
    100                 105                 110

gag gac atg aac ctt aac gaa gag aaa aaa gct cct tta cga aac aaa      740
Glu Asp Met Asn Leu Asn Glu Glu Lys Lys Ala Pro Leu Arg Asn Lys
115                 120                 125                 130

gac ttt acc acc aaa cgt gag atg gtt gtc cag tat att tct gcc act      788
Asp Phe Thr Thr Lys Arg Glu Met Val Val Gln Tyr Ile Ser Ala Thr
                135                 140                 145

gcc aaa tct ata gtt gga agt aaa gtt acg ggt ggg ctg aaa aac agc      836
Ala Lys Ser Ile Val Gly Ser Lys Val Thr Gly Gly Leu Lys Asn Ser
            150                 155                 160

aaa cat gaa tgc acc ctg tct tca caa gaa tat gtt cat gaa tta cga      884
Lys His Glu Cys Thr Leu Ser Ser Gln Glu Tyr Val His Glu Leu Arg
        165                 170                 175

tcg ggt ata tca gat gag aaa ctt ctt aat tgc cta gaa tcc ctc agg      932
Ser Gly Ile Ser Asp Glu Lys Leu Leu Asn Cys Leu Glu Ser Leu Arg
    180                 185                 190

gtt tct tta acc agc aat ccg gtc agc tgg gtt aac aac ttt ggc cat      980
Val Ser Leu Thr Ser Asn Pro Val Ser Trp Val Asn Asn Phe Gly His
195                 200                 205                 210

gaa ggt ctt gga ctc tta ttg gat gag ctg gaa aag ctt ctg gac aaa     1028
Glu Gly Leu Gly Leu Leu Leu Asp Glu Leu Glu Lys Leu Leu Asp Lys
                215                 220                 225

aaa cag caa gaa aat att gac aag aag aat cag tat aaa ctt att caa     1076
Lys Gln Gln Glu Asn Ile Asp Lys Lys Asn Gln Tyr Lys Leu Ile Gln
            230                 235                 240

tgc ctc aaa gca ttt atg aat aat aag ttt gga tta caa agg att cta     1124
Cys Leu Lys Ala Phe Met Asn Asn Lys Phe Gly Leu Gln Arg Ile Leu

245                 250                 255

gga gat gaa aga agt ctt tta cta ttg gca aga gca att gac ccc aaa     1172
Gly Asp Glu Arg Ser Leu Leu Leu Leu Ala Arg Ala Ile Asp Pro Lys
    260                 265                 270

caa ccc aac atg atg act gaa ata gta aaa ata ctt tct gct att tgc     1220
Gln Pro Asn Met Met Thr Glu Ile Val Lys Ile Leu Ser Ala Ile Cys
275                 280                 285                 290

att gtt gga gaa gag aac att cta gat aaa ctt tta ggg gct ata aca     1268
Ile Val Gly Glu Glu Asn Ile Leu Asp Lys Leu Leu Gly Ala Ile Thr
                295                 300                 305

aca gca gca gaa aga aat aac agg gaa cga ttt tca cca att gtg gaa     1316
Thr Ala Ala Glu Arg Asn Asn Arg Glu Arg Phe Ser Pro Ile Val Glu
            310                 315                 320

ggt tta gaa aat cag gaa gcc ttg caa tta cag gtg gcc tgc atg cag     1364
Gly Leu Glu Asn Gln Glu Ala Leu Gln Leu Gln Val Ala Cys Met Gln
        325                 330                 335

ttt ata aat gcc ctt gtc act tct cct tat gag ctt gat ttt cga ata     1412
Phe Ile Asn Ala Leu Val Thr Ser Pro Tyr Glu Leu Asp Phe Arg Ile
    340                 345                 350

cat tta agg aat gaa ttc ctc cgt tca gga cta aaa aca atg tta cca     1460
His Leu Arg Asn Glu Phe Leu Arg Ser Gly Leu Lys Thr Met Leu Pro
355                 360                 365                 370

gat cta aaa gaa aaa gag aat gat gag ctt gat att cag ttg aaa gta     1508
Asp Leu Lys Glu Lys Glu Asn Asp Glu Leu Asp Ile Gln Leu Lys Val
                375                 380                 385

ttt gat gaa aac aaa gaa gat gac cta act gaa tta tca cac cgt ctc     1556
Phe Asp Glu Asn Lys Glu Asp Asp Leu Thr Glu Leu Ser His Arg Leu
            390                 395                 400

aat gac att cga gca gaa atg gat gat atg aat gaa gtc tac cat ctt     1604
Asn Asp Ile Arg Ala Glu Met Asp Asp Met Asn Glu Val Tyr His Leu
        405                 410                 415

cta tat aat atg ctg aag gac act gct gct gaa aat tac ttc tta tct     1652
Leu Tyr Asn Met Leu Lys Asp Thr Ala Ala Glu Asn Tyr Phe Leu Ser
    420                 425                 430

att cta caa cat ttt ttg ctt atc aga aat gat tat tat atc agg cca     1700
Ile Leu Gln His Phe Leu Leu Ile Arg Asn Asp Tyr Tyr Ile Arg Pro
435                 440                 445                 450

caa tat tat aaa ata att gag gaa tgt gtt tca cag ata gtg cta cac     1748
Gln Tyr Tyr Lys Ile Ile Glu Glu Cys Val Ser Gln Ile Val Leu His
                455                 460                 465

tgc agt ggt atg gat cca gac ttc aaa tac agg caa aga tta gac atc     1796
Cys Ser Gly Met Asp Pro Asp Phe Lys Tyr Arg Gln Arg Leu Asp Ile
            470                 475                 480

gat tta act cat ctg ata gat tct tgt gtg aac aag gcg aaa gtt gaa     1844
Asp Leu Thr His Leu Ile Asp Ser Cys Val Asn Lys Ala Lys Val Glu

        485                 490                 495

gaa agt gaa caa aaa gct gca gag ttt tca aag aag ttc gat gaa gaa 1892
Glu Ser Glu Gln Lys Ala Ala Glu Phe Ser Lys Lys Phe Asp Glu Glu
    500                 505                 510

ttc aca gct cga cag gaa gct caa gca gag ctt caa aaa aga gat gag 1940
Phe Thr Ala Arg Gln Glu Ala Gln Ala Glu Leu Gln Lys Arg Asp Glu
515                 520                 525                 530

aaa atc aaa gaa ctt gaa gca gaa atc cag caa ctt cga acc cag gca 1988
Lys Ile Lys Glu Leu Glu Ala Glu Ile Gln Gln Leu Arg Thr Gln Ala
                535                 540                 545

caa gta ctc tca agt tca tca gga att cca ggt cct cct gca gca cct 2036
Gln Val Leu Ser Ser Ser Ser Gly Ile Pro Gly Pro Pro Ala Ala Pro
            550                 555                 560

cca ttg cca ggt gta ggg ccg cct cca cca ccc gcg cca cct cta 2084
Pro Leu Pro Gly Val Gly Pro Pro Pro Pro Pro Ala Pro Pro Leu
        565                 570                 575

ccc gga gga gct cct ctt cct cct cca cca cct cct tta cct gga atg 2132
Pro Gly Gly Ala Pro Leu Pro Pro Pro Pro Pro Pro Leu Pro Gly Met
        580                 585                 590

atg ggg ata cca cca cca ccc cca cca cca ctt tta ttt ggg gga cct 2180
Met Gly Ile Pro Pro Pro Pro Pro Pro Pro Leu Leu Phe Gly Gly Pro
595 600 605 610

cct cca cca cca ccc ctt gga gga gtt cct cct ccc cca gga ata tca 2228
Pro Pro Pro Pro Pro Leu Gly Gly Val Pro Pro Pro Pro Gly Ile Ser
615 620 625

ctt aat cta cct tat gga atg aag cag aaa aaa atg tat aaa cct gaa 2276
Leu Asn Leu Pro Tyr Gly Met Lys Gln Lys Lys Met Tyr Lys Pro Glu
                630                 635                 640

gtg tcc atg aag aga atc aat tgg tca aag att gaa ccc aca gaa tta 2324
Val Ser Met Lys Arg Ile Asn Trp Ser Lys Ile Glu Pro Thr Glu Leu
            645                 650                 655

tct gag aac tgt ttc tgg tta aga gtc aaa gaa gac aag ttt gag aat 2372
Ser Glu Asn Cys Phe Trp Leu Arg Val Lys Glu Asp Lys Phe Glu Asn
        660                 665                 670

cca gat ctc ttt gcc aaa ttg gca ttg aat ttt gct act cag ata aaa 2420
Pro Asp Leu Phe Ala Lys Leu Ala Leu Asn Phe Ala Thr Gln Ile Lys
675 680 685 690

gtt caa aag aac gca gaa gca tta gaa gaa aag aag act ggg cct aca 2468
Val Gln Lys Asn Ala Glu Ala Leu Glu Glu Lys Lys Thr Gly Pro Thr
695 700 705

aag aag aaa gtg aaa gaa ctg aga att ttg gat ccc aaa aca gct cag 2516
Lys Lys Lys Val Lys Glu Leu Arg Ile Leu Asp Pro Lys Thr Ala Gln
                710                 715                 720

aat ctg tcc atc ttt ctg gga tca tat cgc atg cca tat gaa gac ata 2564
Asn Leu Ser Ile Phe Leu Gly Ser Tyr Arg Met Pro Tyr Glu Asp Ile

725                 730                 735

aga aac gtt att ctg gag gtt aat gaa gac atg ctg agt gag gct tta          2612
Arg Asn Val Ile Leu Glu Val Asn Glu Asp Met Leu Ser Glu Ala Leu
    740                 745                 750

att cag aac ctt gtg aaa cat ctt cct gag cag aag ata ctc aac gaa          2660
Ile Gln Asn Leu Val Lys His Leu Pro Glu Gln Lys Ile Leu Asn Glu
755                 760                 765                 770

tta gca gag ctt aag aat gaa tat gat gac ctc tgt gag cct gaa caa          2708
Leu Ala Glu Leu Lys Asn Glu Tyr Asp Asp Leu Cys Glu Pro Glu Gln
                775                 780                 785

ttt gga gtt gtg atg agc tct gtg aaa atg tta cag cct cgt ctc agt          2756
Phe Gly Val Val Met Ser Ser Val Lys Met Leu Gln Pro Arg Leu Ser
            790                 795                 800

agt atc ctg ttc aag ctc aca ttt gaa gaa cac ata aac aac atc aaa          2804
Ser Ile Leu Phe Lys Leu Thr Phe Glu Glu His Ile Asn Asn Ile Lys
        805                 810                 815

cca agc atc ata gca gta act ctt gcc tgt gaa gaa ctg aag aaa agt          2852
Pro Ser Ile Ile Ala Val Thr Leu Ala Cys Glu Glu Leu Lys Lys Ser
    820                 825                 830

gaa agc ttt aac aga ctt tta gag tta gtt ctt ctt gtt gga aac tac          2900
Glu Ser Phe Asn Arg Leu Leu Glu Leu Val Leu Leu Val Gly Asn Tyr
835                 840                 845                 850

atg aac tca ggc tca aga aat gcc cag tct ttg gga ttt aag atc aac          2948
Met Asn Ser Gly Ser Arg Asn Ala Gln Ser Leu Gly Phe Lys Ile Asn
                855                 860                 865

ttc ctt tgt aag atc aga gat act aaa tca gcg gat caa aaa aca acc          2996
Phe Leu Cys Lys Ile Arg Asp Thr Lys Ser Ala Asp Gln Lys Thr Thr
            870                 875                 880

ctt ttg cat ttt att gcc gac att tgt gag gaa aaa tat cga gat atc          3044
Leu Leu His Phe Ile Ala Asp Ile Cys Glu Glu Lys Tyr Arg Asp Ile
        885                 890                 895

cta aaa ttt cct gaa gaa ctg gaa cac gta gaa agt gca agc aaa gtt          3092
Leu Lys Phe Pro Glu Glu Leu Glu His Val Glu Ser Ala Ser Lys Val
    900                 905                 910

tca gct caa att ctc aag agc aac ctt gca tca atg gaa caa caa att          3140
Ser Ala Gln Ile Leu Lys Ser Asn Leu Ala Ser Met Glu Gln Gln Ile
915                 920                 925                 930

gtt cat ctg gaa cgt gac atc aag aaa ttc ccc caa gca gaa aat caa          3188
Val His Leu Glu Arg Asp Ile Lys Lys Phe Pro Gln Ala Glu Asn Gln
                935                 940                 945

cac gat aag ttt gtg gaa aag atg acc agc ttt aca aag act gcc cga          3236
His Asp Lys Phe Val Glu Lys Met Thr Ser Phe Thr Lys Thr Ala Arg
            950                 955                 960

gaa cag tat gaa aaa ctc tcc acc atg cac aac aac atg atg aag ctc          3284
Glu Gln Tyr Glu Lys Leu Ser Thr Met His Asn Asn Met Met Lys Leu

            965                 970                 975

tat gag aat ctt gga gaa tac ttc att ttt gac tca aag aca gtg agc 3332
Tyr Glu Asn Leu Gly Glu Tyr Phe Ile Phe Asp Ser Lys Thr Val Ser
        980                 985                 990

ata gaa gag ttc ttt ggt gat ctc aac aac ttc cga act ttg ttt ttg 3380
Ile Glu Glu Phe Phe Gly Asp Leu Asn Asn Phe Arg Thr Leu Phe Leu
995 1000 1005 1010

gaa gca gtg aga gaa aac aat aag aga aga gaa atg gaa gag aag acc 3428
Glu Ala Val Arg Glu Asn Asn Lys Arg Arg Glu Met Glu Glu Lys Thr
1015 1020 1025

agg agg gca aaa ctt gca aaa gag aaa gct gaa caa gaa aag tta gaa 3476
Arg Arg Ala Lys Leu Ala Lys Glu Lys Ala Glu Gln Glu Lys Leu Glu
                1030                1035                1040

cgc cag aag aaa aag aaa caa ctc att gat ata aac aaa gag ggt gat 3524
Arg Gln Lys Lys Lys Lys Gln Leu Ile Asp Ile Asn Lys Glu Gly Asp
            1045                1050                1055

gag act ggt gtg atg gat aat ctt cta gaa gcc cta caa tca ggt gca 3572
Glu Thr Gly Val Met Asp Asn Leu Leu Glu Ala Leu Gln Ser Gly Ala
        1060                1065                1070

gca ttc aga gac cgt cga aag cgg att cca agg aat cca gat aac aga 3620
Ala Phe Arg Asp Arg Arg Lys Arg Ile Pro Arg Asn Pro Asp Asn Arg
1075 1080 1085 1090

cga gta cct ttg gaa agg tca cgc tct cgc cac aat gga gct atc tca 3668
Arg Val Pro Leu Glu Arg Ser Arg Ser Arg His Asn Gly Ala Ile Ser
1095 1100 1105

tct aag tgattcctga tgccaaagaa tatgaaaata tttaagtaaa aacaaaatga 3724
Ser Lys

<210> 61
<211> 417
<212> DNA
<213> Homo sapiens

<220>
<221> CDS
<222> (28)..(402)

<400> 61
aaattcgcgg ccgcgtcgac cgcagcc atg gct cgt ggt ccc aag aag cat 51
Met Ala Arg Gly Pro Lys Lys His
1               5

ctg aag cgg gtg gca gct cca aag cat tgg atg ctg gat aaa ttg acc 99
Leu Lys Arg Val Ala Ala Pro Lys His Trp Met Leu Asp Lys Leu Thr
    10                  15                  20

ggt gtg ttt gct cct cgt cca tcc acc ggt ccc cac aag ttg aga gag 147
Gly Val Phe Ala Pro Arg Pro Ser Thr Gly Pro His Lys Leu Arg Glu
25                  30                  35                  40

tgt ctc ccc ctc atc att ttc ctg agg aac aga ctt aag tat gcc ctg 195
Cys Leu Pro Leu Ile Ile Phe Leu Arg Asn Arg Leu Lys Tyr Ala Leu
                45                  50                  55

aca gga gat gaa gta aag aag att tgc atg cag cgg ttc att aaa atc 243
Thr Gly Asp Glu Val Lys Lys Ile Cys Met Gln Arg Phe Ile Lys Ile
            60                  65                  70

gat ggc aag gtc cga act gat ata acc tac cct gct gga ttc atg gat 291
Asp Gly Lys Val Arg Thr Asp Ile Thr Tyr Pro Ala Gly Phe Met Asp
        75                  80                  85

gtc atc agc att gac aag acg gga gag aat ttc cgt ctg atc tat gac 339
Val Ile Ser Ile Asp Lys Thr Gly Glu Asn Phe Arg Leu Ile Tyr Asp
    90                  95                  100

acc aag ggt cgc ttt gct gta cat cgt att aca cct gag gag gcc aag 387
Thr Lys Gly Arg Phe Ala Val His Arg Ile Thr Pro Glu Glu Ala Lys
105                 110                 115                 120

tac aat ttg tgc aag tgagaagatt ttttt 417
Tyr Asn Leu Cys Lys
                125

<210> 62
<211> 482
<212> DNA
<213> Homo sapiens

<220>
<221> CDS
<222> (14)..(424)

<400> 62
ggaaggctta gcc atg tcg tcc ttg atc aga agg gtg atc agc acc gcg 49
Met Ser Ser Leu Ile Arg Arg Val Ile Ser Thr Ala
1               5                   10

aaa gcc cca ggg gcc att gga ccc tac agt caa gct gta tta gtc gac 97
Lys Ala Pro Gly Ala Ile Gly Pro Tyr Ser Gln Ala Val Leu Val Asp
        15                  20                  25

agg acc att tac att tca gga cag ata ggc atg gac cct tca agt gga 145
Arg Thr Ile Tyr Ile Ser Gly Gln Ile Gly Met Asp Pro Ser Ser Gly
    30                  35                  40

cag ctt gtg tca gga ggg gta gca gaa gaa gct aaa caa gct ctt aaa 193
Gln Leu Val Ser Gly Gly Val Ala Glu Glu Ala Lys Gln Ala Leu Lys
45                  50                  55                  60

aac atg ggt gaa att ctg aaa gct gca ggc tgt gac ttc act aac gtg 241
Asn Met Gly Glu Ile Leu Lys Ala Ala Gly Cys Asp Phe Thr Asn Val

65                  70                  75

gtg aaa aca act gtt ctt ctg gct gac ata aat gac ttc aat act gtc      289
Val Lys Thr Thr Val Leu Leu Ala Asp Ile Asn Asp Phe Asn Thr Val
            80                  85                  90

aat gaa atc tac aaa cag tat ttc aag agt aat ttt cct gct aga gct      337
Asn Glu Ile Tyr Lys Gln Tyr Phe Lys Ser Asn Phe Pro Ala Arg Ala
        95                  100                 105

gct tac caa gtt gct gct tta ccc aaa ggc agc cga att gaa att gaa      385
Ala Tyr Gln Val Ala Ala Leu Pro Lys Gly Ser Arg Ile Glu Ile Glu
    110                 115                 120

gca gta gct atc caa gga cca ctg aca acg gca tca cta taagtgggcc      434
Ala Val Ala Ile Gln Gly Pro Leu Thr Thr Ala Ser Leu
125                 130                 135

cagtgctgtg tagtctggaa ttgttaacat tttaattttt acaattga                 482

<210> 63
<211> 1242
<212> DNA
<213> Homo sapiens

<220>
<221> CDS
<222> (7)..(1083)

<400> 63
cgagcc atg cgg acc gcg gac cgg gag gcg cgc ccg ggg ctt ccg tcc       48
ctg ctg ctg ctg ctg ctg gcc ggg gcc ggg ctg tca gcc gcc tcg ccc      96
cca gca gcg ccg cgc ttc aac gtg agc ctg gac tcg gtc ccc gag ctg      144
cgc tgg ctg ccc gtg ctg cgg cac tac gac ttg gac ttg gtg cgc gcc      192
gcg atg gcg caa gtc atc ggg gac aga gtc ccc aag tgg gtg cac gtg      240
tta atc gga aaa gtg gtc ctg gag ctg gag cgc ttc ctg ccc cag ccc      288
ttc acc ggc gag atc cgc ggc atg tgt gac ttc atg aac ctc agc ctg      336
gcg gac tgc ctt ctg gtc aac ctg gcc tac gag tcc tcc gtg ttc tgc     384
acc agt att gtg gct caa gac tcc aga ggc cac att tac cat ggt cgg     432
aat ttg gat tat cct ttt ggg aat gtc tta cgc aag ctg aca gtg gat     480
gtg caa ttc tta aag aat ggg cag att gca ttc aca gga act act ttt     528
att ggc tat gta gga tta tgg act ggc cag agc cca cac aag ttt aca     576
gtt tct ggt gat gaa cga gat aaa ggc tgg tgg tgg gag aat gct atc     624
gct gcc ctg ttt cgg aga cac att ccc gtc agc tgg ctg atc cgc gct     672
acc ctg agt gag tcg gaa aac ttc gaa gca gct gtt ggc aag ttg gcc     720
aag act ccc ctt att gct gat gtt tat tac att gtt ggt ggc acg tcc     768
ccc cgg gag ggg gtg gtc atc acg agg aac aga gat ggc cca gca gac     816
att tgg cct cta gat cct ttg aat gga gcg tgg ttc cga gtt gag aca     864
aat tac gac cac tgg aag cca gca ccc aag gaa gat gac cgg aga aca     912
tct gcc atc aag gcc ctt aat gct aca gga caa gca aac ctc agc ctg     960
gag gca ctt ttc cag att ttg tcg gtg gtt cca gtt tat aac aac ctc     1008
aca att tat act acg gta atg agc gcc ggt agc cca gac aag tac atg     1056
act agg atc aga aac ccg agt aga aag taagt cagcagaaga gcgagttcgc     1108
ccgtgctgtg aaagatgatt ttttaaaaaa tgaaattctt gaagagctgc accttaaaaa     1168
ataagacaaa gtgaaagtat tggattatgt tacacacaat gcaggctcct tcctcattga     1228
actttacaac cttg                                                       1242

<210> 64
<211> 1170
<212> DNA
<213> Homo sapiens

<220>
<221> CDS
<222> (101)..(754)

<400> 64
aagcccagta catttcaagt tggtcgcggc ttgggctccg ctttggggag gggcagcagg      60

tttattcact ggatctctga atacccaggc cccctccacc atg gcc agc cgg ggt       115
                                            Met Ala Ser Arg Gly
                                            1               5

ggg ggc cgg ggt cgt ggc cgg ggc cag ttg acc ttc aac gtg gag gcc       163
Gly Gly Arg Gly Arg Gly Arg Gly Gln Leu Thr Phe Asn Val Glu Ala
                10                  15                  20

gtg ggc att ggg aaa ggg gat gct ttg ccc cca ccc acc ctg cag cct       211
Val Gly Ile Gly Lys Gly Asp Ala Leu Pro Pro Pro Thr Leu Gln Pro
            25                  30                  35

tct cca ctc ttc cct ccc ttg gag ttc cgc cca gta cct ttg ccc tca       259
Ser Pro Leu Phe Pro Pro Leu Glu Phe Arg Pro Val Pro Leu Pro Ser
        40                  45                  50

ggc gag gaa ggg gaa tat gtc ctg gca ctg aag caa gag cta cga gga       307
Gly Glu Glu Gly Glu Tyr Val Leu Ala Leu Lys Gln Glu Leu Arg Gly
    55                  60                  65

gcc atg agg cag ctc ccc tac ttc atc cgg cca gct gtc ccc aag aga       355
Ala Met Arg Gln Leu Pro Tyr Phe Ile Arg Pro Ala Val Pro Lys Arg
70                  75                  80                  85

gat gtg gag cgt tat tca gac aaa tat cag atg tca ggt ccg att gac       403
Asp Val Glu Arg Tyr Ser Asp Lys Tyr Gln Met Ser Gly Pro Ile Asp
                90                  95                  100

aat gcc atc gat tgg aac cct gat tgg cgg cgt cta ccc cgg gag cta       451
Asn Ala Ile Asp Trp Asn Pro Asp Trp Arg Arg Leu Pro Arg Glu Leu
            105                 110                 115

aag atc cga gtg cgg aag cta cag aag gaa cgg att aca att ctg ctc       499
Lys Ile Arg Val Arg Lys Leu Gln Lys Glu Arg Ile Thr Ile Leu Leu

120                 125                 130

ccc aag agg ccc cct aag acc aca gaa gat aag gag gaa aca ata cag          547
Pro Lys Arg Pro Pro Lys Thr Thr Glu Asp Lys Glu Glu Thr Ile Gln
    135                 140                 145

aaa cta gag acc ctg gag aag aag gaa gaa gaa gta act tca gag gag          595
Lys Leu Glu Thr Leu Glu Lys Lys Glu Glu Glu Val Thr Ser Glu Glu
150                 155                 160                 165

gat gag gag aaa gaa gaa gaa gaa gag aag gaa gag gag gaa gaa gaa          643
Asp Glu Glu Lys Glu Glu Glu Glu Glu Lys Glu Glu Glu Glu Glu Glu
                170                 175                 180

gag tat gat gaa gaa gaa cat gaa gag gaa act gat tac atc atg tca          691
Glu Tyr Asp Glu Glu Glu His Glu Glu Glu Thr Asp Tyr Ile Met Ser
            185                 190                 195

tat ttt gac aat gga gag gac ttt ggt ggt gac agt gat gac aat atg          739
Tyr Phe Asp Asn Gly Glu Asp Phe Gly Gly Asp Ser Asp Asp Asn Met
        200                 205                 210

gac gag gct ata tac tgaagaagga ctctggaccc tcgtgtcttt ctttaggata          794
Asp Glu Ala Ile Tyr
    215

cagagagtaa ctgtacctat tatttgtttc ttcagacaag caaatcattt ggtcagagtt          854

catataatct gtctgttccc tggagatggg aatagaggat gatgacagtt tattttctac          914

acttcccctc cttccacatt tgtatcacct ttgctatctt ggggaaagtg caaaggacaa          974

acatctcaat tgtatgaagg gagaaaggag aattgaaaga agaactgggg ttgttagagc          1034

tgagatgact gtacacatac ccctgcccaa tttatatagc tctttgtgga gataattagg          1094

ggtgggagca gtttgaagga gtaagcctgg ttttatactt ttaaataaag tgtttttatc          1154

tgtcaaaaaa aaaaaa                                                          1170

<210> 65
<211> 396
<212> DNA
<213> Homo sapiens

<220>
<221> CDS
<222> (54)..(236)

<400> 65
cccgctgcgt gttttcctct tgatcgggaa ctcctgcttc tccttgcctc gaa atg          56
                                                           Met
                                                           1

gac ccc aac tgc tcc tgc tcg cct gtt ggc tcc tgt gcc tgt gcc ggc          104
Asp Pro Asn Cys Ser Cys Ser Pro Val Gly Ser Cys Ala Cys Ala Gly
            5                   10                  15

tcc tgc aaa tgc aaa gag tgc aaa tgc acc tcc tgc aag aag agc tgc 152
Ser Cys Lys Cys Lys Glu Cys Lys Cys Thr Ser Cys Lys Lys Ser Cys
        20                  25                  30

tgc tcc tgc tgc cct gtg ggc tgt gcc aag tgt gcc cag ggc tgc atc 200
Cys Ser Cys Cys Pro Val Gly Cys Ala Lys Cys Ala Gln Gly Cys Ile
    35                  40                  45

tgc aaa ggg acg tca gac aag tgc agc tgc tgt gcc tgat gccaggacag 250
Cys Lys Gly Thr Ser Asp Lys Cys Ser Cys Cys Ala
50                  55                  60

ctgtgctctc agatgtaaat agagcaacct atataaacct ggattttttt tttttttttt 310

ttgtacaacc ctgacccgtt tgctacatct ttttttctat gaaatatgtg aatggcaata 370

aattcatcta gactaaaaaa aaaaaa 396

<210> 66
<211> 2410
<212> DNA
<213> Homo sapiens

<220>
<221> CDS
<222> (121)..(1113)

<400> 66
gtcccctcag agggttcctg ctgctgccgg tgccttggac cctccccctc gcttctcgtt 60

ctactgcccc aggagcccgg cgggtccggg actcccgtcc gtgccggtgc gggcgccggc 120

atg tgg ctg tgg gag gac cag ggc ggc ctc ctg ggc cct ttc tcc ttc 168
Met Trp Leu Trp Glu Asp Gln Gly Gly Leu Leu Gly Pro Phe Ser Phe
1               5                   10                  15

ctg ctg cta gtg ctg ctg ctg gtg acg cgg agc ccg gtc aat gcc tgc 216
Leu Leu Leu Val Leu Leu Leu Val Thr Arg Ser Pro Val Asn Ala Cys
            20                  25                  30

ctc ctc acc ggc agc ctc ttc gtt cta ctg cgc gtc ttc agc ttt gag 264
Leu Leu Thr Gly Ser Leu Phe Val Leu Leu Arg Val Phe Ser Phe Glu
        35                  40                  45

ccg gtg ccc tct tgc agg gcc ctg cag gtg ctc aag ccc cgg gac cgc 312
Pro Val Pro Ser Cys Arg Ala Leu Gln Val Leu Lys Pro Arg Asp Arg
    50                  55                  60

att tct gcc atc gcc cac cgt ggc ggc agc cac gac gcg ccc gag aac 360
Ile Ser Ala Ile Ala His Arg Gly Gly Ser His Asp Ala Pro Glu Asn
65                  70                  75                  80

acg ctg gcg gcc att cgg cag gca gct aag aat gga gca aca ggc gtg 408
Thr Leu Ala Ala Ile Arg Gln Ala Ala Lys Asn Gly Ala Thr Gly Val
                85                  90                  95

gag ttg gac att gag ttt act tct gac ggg att cct gtc tta atg cac 456
Glu Leu Asp Ile Glu Phe Thr Ser Asp Gly Ile Pro Val Leu Met His
            100                 105                 110

gat aac aca gta gat agg acg act gat ggg act ggg cga ttg tgt gat 504
Asp Asn Thr Val Asp Arg Thr Thr Asp Gly Thr Gly Arg Leu Cys Asp
        115                 120                 125

ttg aca ttt gaa caa att agg aag ctg aat cct gca gca aac cac aga 552
Leu Thr Phe Glu Gln Ile Arg Lys Leu Asn Pro Ala Ala Asn His Arg
    130                 135                 140

ctc agg aat gat ttc cct gat gaa aag atc cct acc cta agg gaa gct 600
Leu Arg Asn Asp Phe Pro Asp Glu Lys Ile Pro Thr Leu Arg Glu Ala
145                 150                 155                 160

gtt gca gag tgc cta aac cat aac ctc aca atc ttc ttt gat gtc aaa 648
Val Ala Glu Cys Leu Asn His Asn Leu Thr Ile Phe Phe Asp Val Lys
                165                 170                 175

ggc cat gca cac aag gct act gag gct cta aag aaa atg tat atg gaa 696
Gly His Ala His Lys Ala Thr Glu Ala Leu Lys Lys Met Tyr Met Glu
            180                 185                 190

ttt cct caa ctg tat aat aat agt gtg gtc tgt tct ttc ttg cca gaa 744
Phe Pro Gln Leu Tyr Asn Asn Ser Val Val Cys Ser Phe Leu Pro Glu
        195                 200                 205

gtt atc tac aag atg aga caa aca gat cgg gat gta ata aca gca tta 792
Val Ile Tyr Lys Met Arg Gln Thr Asp Arg Asp Val Ile Thr Ala Leu
    210                 215                 220

act cac aga cct tgg agc cta agc cat aca gga gat ggg aaa cca cgc 840
Thr His Arg Pro Trp Ser Leu Ser His Thr Gly Asp Gly Lys Pro Arg
225                 230                 235                 240

tat gat act ttc tgg aaa cat ttt ata ttt gtt atg atg gac att ttg 888
Tyr Asp Thr Phe Trp Lys His Phe Ile Phe Val Met Met Asp Ile Leu
                245                 250                 255

ctc gat tgg agc atg cat aat atc ttg tgg tac ctg tgt gga att tca 936
Leu Asp Trp Ser Met His Asn Ile Leu Trp Tyr Leu Cys Gly Ile Ser
            260                 265                 270

gct ttc ctc atg caa aag gat ttt gta tcc ccg gcc tac ttg aag aag 984
Ala Phe Leu Met Gln Lys Asp Phe Val Ser Pro Ala Tyr Leu Lys Lys
        275                 280                 285

tgg tca gct aaa gga atc cag gtt gtt ggt tgg act gtt aat acc ttt 1032
Trp Ser Ala Lys Gly Ile Gln Val Val Gly Trp Thr Val Asn Thr Phe
    290                 295                 300

gat gaa aag agt tac tac gaa tcc cat ctt ggt tcc agc tat atc act 1080
Asp Glu Lys Ser Tyr Tyr Glu Ser His Leu Gly Ser Ser Tyr Ile Thr
305                 310                 315                 320

gac agc atg gta gaa gac tgc gaa cct cac ttc tagacttt cacggtggga 1131
Asp Ser Met Val Glu Asp Cys Glu Pro His Phe
                325                 330

cgaaacgggt tcagaaactg ccaggggcct catacaggga tatcaaaata ccctttgtgc 1191

tagcccaggc cctggggaat caggtgactc acacaaatgc aatagttggt cactgcattt 1251

ttacctgaac caaagctaaa cccggtgttg ccaccatgca ccatggcatg ccagagttca 1311

acactgttgc tcttgaaaat ctgggtctga aaaaacgcac aagagcccct gccctgccct 1371

agctgaggca cacagggaga cccagtgagg ataagcacag attgaattgt acagtttgca 1431

gatgcagatg taaatgcatg ggacatgcat gataactcag agttgacatt ttaaaacttg 1491

ccacacttat ttcaaatatt tgtactcagc tatgttaaca tgtactgtag acatcaaact 1551

tgtggccata ctaataaaat tattaaaagg agcactaaag gaaaactgtg tgccaagcat 1611

catagcctaa gccatacagg agatgggaaa ccacgctatg atactttctg gaaacatttt 1671

atatttgtta tgatggacat tttgctcgat tggagcatgc ataatatctt gtggtacctg 1731

tgtggaattt cagctttcct catgcaaaag gattttgtat ccccggccta cttgaagaag 1791

tggtcagcta aaggaatcca ggttgttggt tggactgtta atacctttga tgaaaagagt 1851

tactacgaat cccatcttgg ttccagctat atcactgaca gcatggtaga agactgcgaa 1911

cctcacttct agactttcac ggtgggacga aacgggttca gaaactgcca ggggcctcat 1971

acagggatat caaaataccc tttgtgctag cccaggccct ggggaatcag gtgactcaca 2031

caaatgcaat agttggtcac tgcattttta cctgaaccaa agctaaaccc ggtgttgcca 2091

ccatgcacca tggcatgcca gagttcaaca ctgttgctct tgaaaatctg ggtctgaaaa 2151

aacgcacaag agcccctgcc ctgccctagc tgaggcacac agggagaccc agtgaggata 2211

agcacagatt gaattgtaca gtttgcagat gcagatgtaa atgcatggga catgcatgat 2271

aactcagagt tgacatttta aaacttgcca cacttatttc aaatatttgt actcagctat 2331

gttaacatgt actgtagaca tcaaacttgt ggccatacta ataaaattat taaaaggagc 2391

actaaaggaa aaaaaaaaa 2410

<210> 67
<211> 1464
<212> DNA
<213> Homo sapiens

<220>
<221> CDS
<222> (373)..(1461)

<400> 67
atggcccccg ctccgccccc cgccgcctcc ttctcgccct ccgaggtcca gcggcgcctg 60

gcggccggcg cgtgctgggt ccgccgcggg gcccgcctct acgacctctc cagcttcgtg 120

cggcaccacc cggggggcga gcagctgctg cgggccaggg cgggccagga catcagcgcc 180

gacctggacg ggccgccgca caggcactcg gccaacgcgc gccgctggct ggagcagtac 240

tacgtgggag agctccgcgg ggagcagcag acaggtgata agcatcccat gcgctctgaa 300

acccaccaca tcacagaaac agcccttgct ggcgttacca ggaccttcgc cttcctccac 360

ccggtgggct cc atg gag aac gag cct gta gcc ctt gag gaa act cag 408
Met Glu Asn Glu Pro Val Ala Leu Glu Glu Thr Gln
1               5                   10

aag aca gat cct gct atg gaa cca cgg ttc aaa gtg gtg gat tgg gac 456
Lys Thr Asp Pro Ala Met Glu Pro Arg Phe Lys Val Val Asp Trp Asp
        15                  20                  25

aag aac aca gcc agt ggc tgc ctg act ccc cgt gct gaa gat tgg ctg 504
Lys Asn Thr Ala Ser Gly Cys Leu Thr Pro Arg Ala Glu Asp Trp Leu
    30                  35                  40

agc atc cac cga gac acc ctc atc ctc act ccg att gtg tgt ggc aga 552
Ser Ile His Arg Asp Thr Leu Ile Leu Thr Pro Ile Val Cys Gly Arg
45                  50                  55                  60

ttg ggg ccc acc agc atc aca gat gag ggc act gag ggg ctc cgg gag 600
Leu Gly Pro Thr Ser Ile Thr Asp Glu Gly Thr Glu Gly Leu Arg Glu
                65                  70                  75

gaa aag atg cac gtc cag gat ctc aca gct ggt caa agg cac agc cag 648
Glu Lys Met His Val Gln Asp Leu Thr Ala Gly Gln Arg His Ser Gln
            80                  85                  90

gct cta atg gac ctg gtg gac tgg cga aag cct ctc ctg tgg cag gtg 696
Ala Leu Met Asp Leu Val Asp Trp Arg Lys Pro Leu Leu Trp Gln Val
        95                  100                 105

ggc cac ttg gga gag aag tac gat gag tgg gtt cac cag ccg gtg acc 744
Gly His Leu Gly Glu Lys Tyr Asp Glu Trp Val His Gln Pro Val Thr
    110                 115                 120

agg ccc atc cgc ctc ttc cac tca gac ctc att gag ggc ctc tct aag 792
Arg Pro Ile Arg Leu Phe His Ser Asp Leu Ile Glu Gly Leu Ser Lys
125                 130                 135                 140

act gtc tgg tac agt gtc ccc atc atc tgg gtg ccc ctg gtg ctg tat 840
Thr Val Trp Tyr Ser Val Pro Ile Ile Trp Val Pro Leu Val Leu Tyr
                145                 150                 155

ctc agc tgg tcc tac tac cga acc ttt gcc cag ggc aac gtc cga ctc 888
Leu Ser Trp Ser Tyr Tyr Arg Thr Phe Ala Gln Gly Asn Val Arg Leu
            160                 165                 170

ttc acg tca ttt aca aca gag tac acg gtg gca gtg ccc aag tcc atg 936
Phe Thr Ser Phe Thr Thr Glu Tyr Thr Val Ala Val Pro Lys Ser Met
        175                 180                 185

ttc ccc ggg ctc ttc atg ctg ggg aca ttc ctc tgg agc ctc atc gag 984
Phe Pro Gly Leu Phe Met Leu Gly Thr Phe Leu Trp Ser Leu Ile Glu
    190                 195                 200

tac ctc atc cac cgc ttc ctg ttc cac atg aag ccc ccc agc gac agc      1032
Tyr Leu Ile His Arg Phe Leu Phe His Met Lys Pro Pro Ser Asp Ser
205                 210                 215                 220

tat tac ctc atc atg ctg cac ttc gtc atg cac ggc cag cac cac aag      1080
Tyr Tyr Leu Ile Met Leu His Phe Val Met His Gly Gln His His Lys
                225                 230                 235

gca ccc ttc gac ggc tcc cgc ctg gtc ttc ccc cct gtg cca gcc tcc      1128
Ala Pro Phe Asp Gly Ser Arg Leu Val Phe Pro Pro Val Pro Ala Ser
            240                 245                 250

ctg gtg atc ggc gtc ttc tac ttg tgc atg cag ctc atc ctg ccc gag      1176
Leu Val Ile Gly Val Phe Tyr Leu Cys Met Gln Leu Ile Leu Pro Glu
        255                 260                 265

gca gta ggg ggc act gtg ttt gcg ggg ggc ctc ctg ggc tac gtc ctc      1224
Ala Val Gly Gly Thr Val Phe Ala Gly Gly Leu Leu Gly Tyr Val Leu
    270                 275                 280

tat gac atg acc cat tac tac ctg cac ttt ggc tcg ccg cac aag ggc      1272
Tyr Asp Met Thr His Tyr Tyr Leu His Phe Gly Ser Pro His Lys Gly
285                 290                 295                 300

tcc tac ctg tac agc ctg aag gcc cac cac gtc aag cac cac ttt gca      1320
Ser Tyr Leu Tyr Ser Leu Lys Ala His His Val Lys His His Phe Ala
                305                 310                 315

cat cag aag tca gga ggg tca cat cca ctt ggt ggc cag gtg gcc ctt      1368
His Gln Lys Ser Gly Gly Ser His Pro Leu Gly Gly Gln Val Ala Leu
            320                 325                 330

ggt gac cca ctt ctt cct gga gcg tcc ctg cct aga gct cag ccc aca      1416
Gly Asp Pro Leu Leu Pro Gly Ala Ser Leu Pro Arg Ala Gln Pro Thr
        335                 340                 345

gga ctg ctt cag gcc gtg gcc aca ggt agc agc cgc aag ggg aaa tga      1464
Gly Leu Leu Gln Ala Val Ala Thr Gly Ser Ser Arg Lys Gly Lys
    350                 355                 360

<210> 68
<211> 886
<212> DNA
<213> Homo sapiens

<220>
<221> CDS
<222> (40)..(618)

<400> 68
ccgagccgag cgcgcggagc agctggggcc ggggcgcgg atg ctg gaa gtt cac      54
                                          Met Leu Glu Val His
                                          1               5

atc ccg tcg gtg ggg ccc gag gcc gag ggg ccc agg cag agc ccg gag      102

Ile Pro Ser Val Gly Pro Glu Ala Glu Gly Pro Arg Gln Ser Pro Glu
                10                  15                  20

aaa agc cac atg gtg ttc cga gtg gag gtg ctg tgc agc ggg cgc aga 150
Lys Ser His Met Val Phe Arg Val Glu Val Leu Cys Ser Gly Arg Arg
            25                  30                  35

cac acg gtg cca agg cgc tac agc gag ttc cac gcg ctg cac aag cgg 198
His Thr Val Pro Arg Arg Tyr Ser Glu Phe His Ala Leu His Lys Arg
        40                  45                  50

atc aag aag ctg tac aaa gtg ccc gac ttc ccc tcg aaa cgc ctg ccc 246
Ile Lys Lys Leu Tyr Lys Val Pro Asp Phe Pro Ser Lys Arg Leu Pro
    55                  60                  65

aac tgg agg acc aga ggg ttg gaa cag cgc cgg cag ggc ttg gag gct 294
Asn Trp Arg Thr Arg Gly Leu Glu Gln Arg Arg Gln Gly Leu Glu Ala
70                  75                  80                  85

tac atc cag ggc atc ctg tac ctg aac cag gag gtg ccc aag gag tta 342
Tyr Ile Gln Gly Ile Leu Tyr Leu Asn Gln Glu Val Pro Lys Glu Leu
                90                  95                  100

ctg gaa ttc ctg aga ctt cgg cac ttc ccc aca gac ccc aag gct agc 390
Leu Glu Phe Leu Arg Leu Arg His Phe Pro Thr Asp Pro Lys Ala Ser
            105                 110                 115

aac tgg ggc acc ctg agg gag ttc ctg cct ggc gac agc agc tcc cag 438
Asn Trp Gly Thr Leu Arg Glu Phe Leu Pro Gly Asp Ser Ser Ser Gln
        120                 125                 130

cag cac cag cgg cct gtc ctg agc ttc cat gtg gat ccc tat gtt tgc 486
Gln His Gln Arg Pro Val Leu Ser Phe His Val Asp Pro Tyr Val Cys
    135                 140                 145

aac ccc tcc cca gag tcg ctg ccc aac gtg gtg gtg aat ggt gtg ctc 534
Asn Pro Ser Pro Glu Ser Leu Pro Asn Val Val Val Asn Gly Val Leu
150                 155                 160                 165

cag ggc ctc tac agc ttc agc atc agc cca gat aaa gcc cag cca aag 582
Gln Gly Leu Tyr Ser Phe Ser Ile Ser Pro Asp Lys Ala Gln Pro Lys
                170                 175                 180

gcg gcc tgt cac cct gct cct ctg cca ccg atg ccc tgat cagtccagag 632
Ala Ala Cys His Pro Ala Pro Leu Pro Pro Met Pro
            185                 190

gcctttggct gcctcctaag aaagtcatgt gcctctgtcc tatgaactcc atataaggct 692

gggtcctcct ttggcctgga cccaggactt aattacccag tgcccagttg tgccacattc 752

ccactcaagg ctcagaactt ggctcgcatt ggtagctgga ggtggtagaa tttgtatgct 812

cttagagccc aacagccaag gcagggtcaa gaagataagt aataaaagag gaagtcagcc 872

aaaaaaaaaa aaaa 886

<210> 69
<211> 4181
<212> DNA
<213> Homo sapiens

<220>
<221> CDS
<222> (229)..(1560)

<400> 69
agtgcggatc ccagcggcgg ccgtgtagct gagcagccct ggggcttggt tctatgtccc 60

tgtggctatg tttccagtgt cctctgggtg tttccaagag caacaagaaa cgaataaatc 120

tctggcaaag gcagctcgag catctcatct gacgtgagtt caagtacaga tcacacgccc 180

actaaagccc agaagaatgt ggctaccagc gaagactccg acctgagc atg cgc aca 237
Met Arg Thr
1

ctg agc acg ccc agc ccg gcc ctg ata tgt cca ccg aat ctc cca gga 285
Leu Ser Thr Pro Ser Pro Ala Leu Ile Cys Pro Pro Asn Leu Pro Gly
    5                   10                  15

ttt cag aat gga agg ggc tcg tcc acc tcc tcg tcc tcc atc acc ggg 333
Phe Gln Asn Gly Arg Gly Ser Ser Thr Ser Ser Ser Ser Ile Thr Gly
20                  25                  30                  35

gag acg gtg gcc atg gtg cac tcc ccg ccc ccg acc cgc ctc aca cac 381
Glu Thr Val Ala Met Val His Ser Pro Pro Pro Thr Arg Leu Thr His
                40                  45                  50

ccg ctc atc cgg ctc gcc tcc aga ccc cag aag gag cag gcc agc ata 429
Pro Leu Ile Arg Leu Ala Ser Arg Pro Gln Lys Glu Gln Ala Ser Ile
            55                  60                  65

gac cgg ctc ccg gac cac tcc atg gtg cag atc ttc tcc ttc ctg ccc 477
Asp Arg Leu Pro Asp His Ser Met Val Gln Ile Phe Ser Phe Leu Pro
        70                  75                  80

acc aac cag ctg tgc cgc tgc gcg cga gtg tgc cgc cgc tgg tac aac 525
Thr Asn Gln Leu Cys Arg Cys Ala Arg Val Cys Arg Arg Trp Tyr Asn
    85                  90                  95

ctg gcc tgg gac ccg cgg ctc tgg agg act atc cgc ctg acg ggc gag 573
Leu Ala Trp Asp Pro Arg Leu Trp Arg Thr Ile Arg Leu Thr Gly Glu
100                 105                 110                 115

acc atc aac gtg gac cgc gcc ctc aag gtg ctg acc cgc aga ctc tgc 621
Thr Ile Asn Val Asp Arg Ala Leu Lys Val Leu Thr Arg Arg Leu Cys
                120                 125                 130

cag gac acc ccc aac gtg tgt ctc atg ctg gaa acc gta act gtc agt 669
Gln Asp Thr Pro Asn Val Cys Leu Met Leu Glu Thr Val Thr Val Ser
            135                 140                 145

ggc tgc agg cgg ctc aca gac cga ggg ctg tac acc atc gcc cag tgc 717
Gly Cys Arg Arg Leu Thr Asp Arg Gly Leu Tyr Thr Ile Ala Gln Cys
        150                 155                 160

tgc ccc gaa ctg agg cga ctg gaa gtc tca ggc tgt tac aat atc tcc 765
Cys Pro Glu Leu Arg Arg Leu Glu Val Ser Gly Cys Tyr Asn Ile Ser
    165                 170                 175

aac gag gcc gtc ttt gat gtg gtg tcc ctc tgc cct aat ctg gag cac 813
Asn Glu Ala Val Phe Asp Val Val Ser Leu Cys Pro Asn Leu Glu His
180                 185                 190                 195

ctg gat gtg tca gga tgc tcc aaa gtg acc tgc atc agc ttg acc cgg 861
Leu Asp Val Ser Gly Cys Ser Lys Val Thr Cys Ile Ser Leu Thr Arg
                200                 205                 210

gag gcc tcc att aaa ctg tca ccc ttg cat ggc aaa cag att tcc atc 909
Glu Ala Ser Ile Lys Leu Ser Pro Leu His Gly Lys Gln Ile Ser Ile
            215                 220                 225

cgc tac ctg gac atg acg gac tgc ttc gtg ctg gag gac gaa ggc ctg 957
Arg Tyr Leu Asp Met Thr Asp Cys Phe Val Leu Glu Asp Glu Gly Leu
        230                 235                 240

cac acc atc gcg gcg cac tgc acg cag ctc acc cac ctc tac ctg cgc 1005
His Thr Ile Ala Ala His Cys Thr Gln Leu Thr His Leu Tyr Leu Arg
    245                 250                 255

cgc tgc gtc cgc ctg acc gac gaa ggc ctg cgc tac ctg gtg atc tac 1053
Arg Cys Val Arg Leu Thr Asp Glu Gly Leu Arg Tyr Leu Val Ile Tyr
260                 265                 270                 275

tgc gcc tcc atc aag gag ctg agc gtc agc gac tgc cgc ttc gtc agc 1101
Cys Ala Ser Ile Lys Glu Leu Ser Val Ser Asp Cys Arg Phe Val Ser
                280                 285                 290

gac ttc ggc ctg cgg gag atc gcc aag ctg gag tcc cgc ctg cgg tac 1149
Asp Phe Gly Leu Arg Glu Ile Ala Lys Leu Glu Ser Arg Leu Arg Tyr
            295                 300                 305

ctg agc atc gcg cac tgc ggc cgg gtc acc gac gtg ggc atc cgc tac 1197
Leu Ser Ile Ala His Cys Gly Arg Val Thr Asp Val Gly Ile Arg Tyr
        310                 315                 320

gtg gcc aag tac tgc agc aag ctg cgc tac ctc aac gcg agg ggc tgc 1245
Val Ala Lys Tyr Cys Ser Lys Leu Arg Tyr Leu Asn Ala Arg Gly Cys
    325                 330                 335

gag ggc atc acg gac cac ggt gtg gag tac ctc gcc aag aac tgc acc 1293
Glu Gly Ile Thr Asp His Gly Val Glu Tyr Leu Ala Lys Asn Cys Thr
340                 345                 350                 355

aaa ctc aaa tcc ctg gat atc ggc aaa tgc cct ttg gta tcc gac acg 1341
Lys Leu Lys Ser Leu Asp Ile Gly Lys Cys Pro Leu Val Ser Asp Thr
                360                 365                 370

ggc ctg gag tgc ctg gcc ctg aac tgc ttc aac ctc aag cgg ctc agc 1389
Gly Leu Glu Cys Leu Ala Leu Asn Cys Phe Asn Leu Lys Arg Leu Ser
            375                 380                 385

ctc aag tcc tgc gag agc atc acc ggc cag ggc ttg cag atc gtg gcc 1437
Leu Lys Ser Cys Glu Ser Ile Thr Gly Gln Gly Leu Gln Ile Val Ala
        390                 395                 400

gcc aac tgc ttt gac ctc cag acg ctg aat gtc cag gac tgc gag gtc     1485
Ala Asn Cys Phe Asp Leu Gln Thr Leu Asn Val Gln Asp Cys Glu Val
    405                 410                 415

tcc gtg gag gcc ctg cgc ttt gtc aaa cgc cac tgc aag cgc tgc gtc     1533
Ser Val Glu Ala Leu Arg Phe Val Lys Arg His Cys Lys Arg Cys Val
420                 425                 430                 435

atc gag cac acc aac ccg gct ttc ttc tgaag ggacagagtt catccggcgt     1585
Ile Glu His Thr Asn Pro Ala Phe Phe
                440

tgtattcaca caaacctgaa caaagcaaat ttttttaaaa gcagcgtatg taagcaccga     1645

cacccactca aaacagctct ttcttccggg aaggttatta ggaatctggc ctttattttt     1705

cctcatttct catgggcaac agaggccaaa gaaacgaagc aagacaaaca gcaaacaggc     1765

attttggtca ggtcatttgt aggcagtttc tcttctcaca aaagatgtac ttaagcaggc     1825

tgatcgctgt tccttgagca aggcgcttac tctcctccgc tcaggccccc aaggccgccc     1885

tttccctcgc acacaggccc cacccccaca gttccacgcc ccccccacca aggccacacc     1945

ctccctccct agagcagcag cgaggatcca tcatcagaat cacagtgctc tccagacctc     2005

ctctctaaac tgcttcattg acctaagtca ctctcttcaa tcccacaccc atggacattc     2065

ttgtcaactc aataccatag cactttgcat aggcaaaata cttttcaggc ctttttaaaa     2125

aattcattac agcaaacagc tggggaagga catgcagtcc tcccccagct ctgtcaatga     2185

ctatgacctt ggccaaagca cttcactgct ctgggctgca gcttccagca ctgaatcaga     2245

ggccacacag cccaaagatt agcttcatgt ccattatagc attgagggag cagagatacc     2305

catacacaga agcaccttgg catagagcac ccaggcatcg acctcttcca ggagaactga     2365

ttctgtggat ggatgtgatt tcaggagatt gtgcagtgcc agcatcagtg cataaagggt     2425

cctgtatgtc ctttggctgc aaatcaccca cttccctgtg tttcagtggg agaatttcct     2485

ctcccacctc ctcacatcct cttttgccag gctggatgct gtcgtctctg tacacaaata     2545

ctttctgcat tcccccctcc acaccatcct agcgaggcac cagcacacct aatcacagca     2605

aagcccagat ccccccatca gttgctttta ctcagtgttt tcaaatagga gtaaaggccc     2665

ttgcaatttt taattaacaa gcaaggccca agggaacaca tgtcctcaaa agtttttctg     2725

atccctcgcc ttgcacacct ggcatgcatc aggcacatct gtcctacagc tggcagagac     2785

agatgcctcg gttctttgtc attcagattg catttgacct cttctcatct atttatttct     2845

ttatacatcc agacttcatc acatgaagcc tattggggtt aagtttgtaa gtgtttaatt     2905

gtgcaaattg ccaccctgtg tacctcctcc atgtctgtct gcgtgttttc caccaaagaa     2965

tgcaaagcag acttccaggt gtttaaattc tgttcactca acaatgccag atgaatggaa     3025

gagggaacac actgagatga cttagactct ggtccaccaa ccagaccctt ggaaaggaat 3085

actaaaatca ttacaaggta tggattttaa atggatgaaa cttcaaatta tcttatttgg 3145

atagaagtct atattctagc ctcatttgca tgaagtcaga tagccagaag aaattccatt 3205

gctggttttc acgaaattca cttgtctttt gctaataaac acatggccct ttcccagatt 3265

attctctagc caagccccac ctttgttacg ttgaaatccc tcatttattt tcttctcaaa 3325

atgcccatta tccaaatgca gaacctctgc atctccaagc cagttatgct gaatttgtca 3385

aacttagaca cccttgacaa ctgcactcct actgtaggct cctgtgcata ctgtcgtctt 3445

ctgtggggga tggagaggtt agtgtgatga ggtggtgtct gcccaggagg tttctttcaa 3505

acatcatggc ctcccatcca atcaacatca tcaaattaca tgtgtaatca aggctctgtg 3565

ccatggggga aatgaatcat ttagctaggc caggatctag tgaaagccac agagtttaaa 3625

accatgaaag aagttgaagg cagcattcct cagctctgtg acttgtgacc ctatttgaag 3685

tttcaggatt tgggtgtcac aaaggattgt ccctaatcct tggccctggg gtcttccgag 3745

tgagctggtt taatactctg agaatgagca gggagatcca gagaatgaat ccctgaccgc 3805

atcacctaaa ctgtcttcca aacatgagac aaagctgact gttcacactg attgcccagc 3865

acataccgtc ttgccagttt cttcttttct cccagtctcc tgttcatcca ttctgttctc 3925

ccttggggtg ggaatctatg atggaggtta ctggggaaac agctcagcag atttttggag 3985

accaaaccaa aggtctcact aggaaattta tctgttttaa aacattgctt ccttcctggc 4045

tctgctaaat tgaatgctca ttgtttgttg ttgttgtttt ttaattctaa tgttcaaatc 4105

actgcgtgct gtatgaatct agaaagcctt aatttactac caagaaataa agcaatatgt 4165

tcgtaaaaaa aaaaaa 4181

<210> 70
<211> 519
<212> DNA
<213> Homo sapiens

<220>
<221> CDS
<222> (132)..(428)

<400> 70
ggaattccta gggatgcaga tgggggaagg ggaagctgag aaagcagtga agacagaatg 60

agctggggaa gaggcagatg gacggggctg cagcatagga gtctcagctg cttacatcca 120

ggtccaggat t atg tct gct aac aga cgc tgg tgg gta cca cct gac gat 170
Met Ser Ala Asn Arg Arg Trp Trp Val Pro Pro Asp Asp
1               5                   10

gaa gac tgt gtg tct gag aag ctc ctg agg aag act cgg gaa tct cca 218
Glu Asp Cys Val Ser Glu Lys Leu Leu Arg Lys Thr Arg Glu Ser Pro
    15                  20                  25

ctg gtg cct ata ggc tta gga ggc tgc ttg gtg gta gca gca tac agg 266
Leu Val Pro Ile Gly Leu Gly Gly Cys Leu Val Val Ala Ala Tyr Arg
30                  35                  40                  45

att tac cgg ctg agg tct cgt ggt tcc acc aag atg tcc ata cac ctg 314
Ile Tyr Arg Leu Arg Ser Arg Gly Ser Thr Lys Met Ser Ile His Leu
                50                  55                  60

att cac acc cga gtg gca gcg cag gcc tgt gca gtg ggt gca atc atg 362
Ile His Thr Arg Val Ala Ala G[?][?] [?]la Cys Ala Val Gly Ala Ile Met
65 70 75

cta ggt gct gtg tac aca atg tac agc gat tac gtc aag agg atg gca 410
Leu Gly Ala Val Tyr Thr Met Tyr Ser Asp Tyr Val Lys Arg Met Ala
80 85 90

cag gat gct gga gag aag taggac tccaatagga gccggggctg tccaactccc 464
Gln Asp Ala Gly Glu Lys
95

ctaactcaat ccctggtaca ttcctaataa agcagttttg aggaaaaaaa aaaaa 519

<210> 71
<211> 439
<212> DNA
<213> Homo sapiens

<220>
<221> CDS
<222> (10)..(399)

<400> 71
gccgccaca atg gtg cgc atg aat gtc ctg gca gat gct ctc aag agt 48

1               5                   10

atc aac aat gcc gaa aag aga ggc aaa cgc cag gtg ctt att agg ccg 96

    15                  20                  25

tgc tcc aaa gtc atc gtc cgg ttt ctc act gtg atg atg aag cat ggt 144

30                  35                  40                  45

tac att ggc gaa ttt gaa atc att gat gac cac aga gct ggg aaa att 192

                50                  55                  60

gtt gtg aac ctc aca ggc agg cta aac aag tgt ggg gtg atc agc ccc 240

            65                  70                  75

aga ttt gac gtg caa ctc aaa gac ctg gaa aaa tgg cag aat aat ctg 288

        80                  85                  90

ctt cca tcc cgc cag ttt ggt ttc att gta ctg aca acc tca gct ggc 336

    95                  100                 105

atc atg gac cat gaa gaa gca aga cga aaa cac aca gga ggg aaa atc 384

110                 115                 120                 125

ctg gga ttc ttt ttc tagggatgta atacatatat ttacaaataa aaaaaaaaa 439

                130

<210> 72
<211> 645
<212> DNA
<213> Homo sapiens

<220>
<221> CDS
<222> (52)..(459)

<400> 72
attcccagct gtttctgctt gctgatcagg actgcacaca gagaactcac c atg gag 57
Met Glu
1

ttt ggg ctg agc tgg gtt ttc ctt gtt gct att tta aaa ggt gtc cag 105
Phe Gly Leu Ser Trp Val Phe Leu Val Ala Ile Leu Lys Gly Val Gln
        5                   10                  15

tgt gag gtg cag ctg gtg gag tcc ggg gga ggc tta gtt cag cct ggg 153
Cys Glu Val Gln Leu Val Glu Ser Gly Gly Gly Leu Val Gln Pro Gly
    20                  25                  30

ggg tcc ctg aga ctc tcc tgt gca gcc tct gga ttc acc ttc agt agc 201
Gly Ser Leu Arg Leu Ser Cys Ala Ala Ser Gly Phe Thr Phe Ser Ser
35                  40                  45                  50

tac tgg atg cac tgg gtc cgc caa gct cca ggg aag ggg ctg gtg tgg 249
Tyr Trp Met His Trp Val Arg Gln Ala Pro Gly Lys Gly Leu Val Trp
                55                  60                  65

gtc tca cgt att aat agt gat ggg agt agc aca agc tac gcg gac tcc 297
Val Ser Arg Ile Asn Ser Asp Gly Ser Ser Thr Ser Tyr Ala Asp Ser
            70                  75                  80

gtg aag ggc cga ttc acc atc tcc aga gac aac gcc aag aac acg ctg 345
Val Lys Gly Arg Phe Thr Ile Ser Arg Asp Asn Ala Lys Asn Thr Leu
        85                  90                  95

tat ctg caa atg aac agt ctg aga gcc gag gac acg gct gtg tat tac 393
Tyr Leu Gln Met Asn Ser Leu Arg Ala Glu Asp Thr Ala Val Tyr Tyr

100                 105                 110

tgt gca aga gac aca gtg agg gga agt caa tgt gag ccc aga cac aaa      441
Cys Ala Arg Asp Thr Val Arg Gly Ser Gln Cys Glu Pro Arg His Lys
115                 120                 125                 130

cct cgc tgc agg ggc atc tgagac cacgaggggg tgtcctgggc cctgtgaact      495
Pro Arg Cys Arg Gly Ile
                135

gggctgctct ccgtggcagc ggctggtggt gctaaaggct gattttctct cagcatctgg    555

ggctgattca tcaagtttcc tcagagaacc tttcagattt acaattctgt acttacgttt    615

aatgtctctg aatgtgacaa aaaaaaaaaa                                     645

<210> 73
<211> 1532
<212> DNA
<213> Homo sapiens

<220>
<221> CDS
<222> (158)..(1486)

<400> 73
gaggcagggg aggctcggcg accctgtcag gcgctgcggg acgccgagga ctggggcggc     60

gtgctggaga tccgcggcca accgtggggt gctgctgtcg ggactcatag gtgtcgtctc    120

ctggaagagg cctctctccc ttgtgataac ctttttc atg ctg ctt tct gca gtg     175
                                         Met Leu Leu Ser Ala Val
                                         1               5

tgt gta atg ctg aat ttg gct ggt tca att ctc tct tgt cag aat gct      223
Cys Val Met Leu Asn Leu Ala Gly Ser Ile Leu Ser Cys Gln Asn Ala
            10                  15                  20

cag cta gtc aac tcc cta gaa ggc tgc cag ttg att aag ttt gac agt      271
Gln Leu Val Asn Ser Leu Glu Gly Cys Gln Leu Ile Lys Phe Asp Ser
        25                  30                  35

gtg gag gtg tgt gtc tgc tgt gag ctg cag cac cag tcg tcc ggc tgc      319
Val Glu Val Cys Val Cys Cys Glu Leu Gln His Gln Ser Ser Gly Cys
    40                  45                  50

agc aac ctc ggg gag acg ctg aag ctg aac ccg ctg cag gag aac tgc      367
Ser Asn Leu Gly Glu Thr Leu Lys Leu Asn Pro Leu Gln Glu Asn Cys
55                  60                  65                  70

aac gct gtg agg ctg acc ttg aag gat ctc ctc ttc agc gtg tgt gcc      415
Asn Ala Val Arg Leu Thr Leu Lys Asp Leu Leu Phe Ser Val Cys Ala
                75                  80                  85

ctc aac gtc ctg tcc act atc gtg tgt gcg ctg gcc aca gcc atg tgc      463
Leu Asn Val Leu Ser Thr Ile Val Cys Ala Leu Ala Thr Ala Met Cys
            90                  95                  100

tgt atg cag atg gtc tcc tcc gat gtc ctg cag atg ttc ctt ccg cag 511
Cys Met Gln Met Val Ser Ser Asp Val Leu Gln Met Phe Leu Pro Gln
        105                 110                 115

agg tca cat cct gcc aac ccc acc tgc gtg act cct cac ggc acc gtt 559
Arg Ser His Pro Ala Asn Pro Thr Cys Val Thr Pro His Gly Thr Val
    120                 125                 130

ctc cac cag acc ctg gat ttc gac gag ttc atc ccc cca ctc ccg cct 607
Leu His Gln Thr Leu Asp Phe Asp Glu Phe Ile Pro Pro Leu Pro Pro
135                 140                 145                 150

cca ccc tat tac ccc cca gag tac acc tgc aca ccg tcc act gag gcc 655
Pro Pro Tyr Tyr Pro Pro Glu Tyr Thr Cys Thr Pro Ser Thr Glu Ala
                155                 160                 165

cag agg ggc ctc cac ctg gac ttt gct ccg tct cca ttc ggc act ttg 703
Gln Arg Gly Leu His Leu Asp Phe Ala Pro Ser Pro Phe Gly Thr Leu
            170                 175                 180

tat gac gtg gcc atc aat agc ccc ggc ctg ctc tat cct gct gag ctc 751
Tyr Asp Val Ala Ile Asn Ser Pro Gly Leu Leu Tyr Pro Ala Glu Leu
        185                 190                 195

ccc cct ccg tac gag gcg gtg gtg ggc cag ccc cct gcc agc cag gtt 799
Pro Pro Pro Tyr Glu Ala Val Val Gly Gln Pro Pro Ala Ser Gln Val
    200                 205                 210

aca agt ata ggt cag cag gtg gcc gag tcc agc tcc ggg gac cca aac 847
Thr Ser Ile Gly Gln Gln Val Ala Glu Ser Ser Ser Gly Asp Pro Asn
215                 220                 225                 230

acc agt gct ggc ttc agc act cca gta cca gct gac agc aca agc ctc 895
Thr Ser Ala Gly Phe Ser Thr Pro Val Pro Ala Asp Ser Thr Ser Leu
                235                 240                 245

ctg gtg tcc gag ggc act gct acg cca ggt tcc agc cca tcc ccc gat 943
Leu Val Ser Glu Gly Thr Ala Thr Pro Gly Ser Ser Pro Ser Pro Asp
            250                 255                 260

ggc cct gtg ggc gcc cca gca ccc tcc gaa cct gcc ctt ccc cct ggc 991
Gly Pro Val Gly Ala Pro Ala Pro Ser Glu Pro Ala Leu Pro Pro Gly
        265                 270                 275

cgc gtg tct cca gag gat cct ggc atg ggc tca cag gtg cag cca ggt 1039
Arg Val Ser Pro Glu Asp Pro Gly Met Gly Ser Gln Val Gln Pro Gly
    280                 285                 290

ccc ggg cat gtg tcc cgc tcc acc agc gac ccc acc ttg tgc aca tct 1087
Pro Gly His Val Ser Arg Ser Thr Ser Asp Pro Thr Leu Cys Thr Ser
295                 300                 305                 310

agc atg gca ggc gat gcc tct tca cac agg ccg tcg tgt agc cag gac 1135
Ser Met Ala Gly Asp Ala Ser Ser His Arg Pro Ser Cys Ser Gln Asp
                315                 320                 325

ctg gaa gcg gga ctg tct gag gct gtg cct ggg agc gct tcc atg tct 1183
Leu Glu Ala Gly Leu Ser Glu Ala Val Pro Gly Ser Ala Ser Met Ser
            330                 335                 340

cgc tct gcc acg gct gcc tgt cgg gcc cag ctt tca cca gcg ggg gac     1231
Arg Ser Ala Thr Ala Ala Cys Arg Ala Gln Leu Ser Pro Ala Gly Asp
        345                 350                 355

cca gat acc tgg aaa act gac caa cgg cca aca cca gag ccc ttc cca     1279
Pro Asp Thr Trp Lys Thr Asp Gln Arg Pro Thr Pro Glu Pro Phe Pro
    360                 365                 370

gcg acc tcc aaa gag cgg cca cgc tct tta gtg gac agc aag gcc tat     1327
Ala Thr Ser Lys Glu Arg Pro Arg Ser Leu Val Asp Ser Lys Ala Tyr
375                 380                 385                 390

gcg gac gcc agg gtt ttg gtg gcc aag ttt ttg gaa cac tca cac tgt     1375
Ala Asp Ala Arg Val Leu Val Ala Lys Phe Leu Glu His Ser His Cys
                395                 400                 405

gcc ctc ccc acc gag gca cag cac atg gtg ggt gcc atg cgc ttg gct     1423
Ala Leu Pro Thr Glu Ala Gln His Met Val Gly Ala Met Arg Leu Ala
            410                 415                 420

gtc acc aac gag gag cgc ctc gag gag gag gcc gtc ttc ggc gct gat     1471
Val Thr Asn Glu Glu Arg Leu Glu Glu Glu Ala Val Phe Gly Ala Asp
        425                 430                 435

gtt ctg gac cag gta tgaaggagcc acatttctga ccgtggacac caacgcctgg     1526
Val Leu Asp Gln Val
    440

cgccac                                                              1532

<210> 74
<211> 1529
<212> DNA
<213> Homo sapiens

<220>
<221> CDS
<222> (1)..(1392)

<400> 74
atg ccg gcc acc tgg gaa gac ttt aag cag ata ata aag tgt gaa ata     48
aca gtt att tat aga cat ggc ctt ccc ttg gta aca ctt acc ttg cca     96
tct aga aaa gaa cgt tgt caa ttc gta gtc aaa cca atg ttg tca aca     144
gtt ggt tca ttc ctt cag gac cta caa aat gaa gat aag ggt atc aaa     192
act gca gcc atc ttc aca gca gtt tat tca cct act gaa gga cat ctg 240
gga tac ttc caa gtt ttg gca att atg aat gga gtt gct ata aat aca 288
cat gcg cag gct ttt gtt tgc acg gag att ctt caa gca atc act atg 336
tca gca gac aca ggt gtt tct ctt cct tca tat gag gaa tat cag gga 384
tcc aaa ctt att cga aaa gct aaa gag gca cca ttc gta ccc att gga 432
ata gca ggt ttt gca gca gtt gtt gca tat gga tta cac aaa ctg aag 480
agc agg gga aat act aaa atg tcc att cat ctg atc cac atg cgt gtg 528
gta gcc caa ggc ttt gtt gta gga gca atg act gtt ggt tct ctt aac 576
agt gat aat gaa caa tta cag ctc tca gaa ggg tat caa tct gag gga 624
ctt ctc ata gca gag cct aat ttt ata tgg act tat aaa ccc atg gag 672
gag gca ctt agc cgt gca gga aca aat ggc aag cct tta gcc cga tct 720
gga gcg gca aag ggg tgc ctc gct gga tca gga gca cag tgg aca ccc 768
tgc tgg atc cgg aag aat gta agt cag cag cga gtc tgc tac ggc ggc 816
aaa aca gca gtg gtg gac aaa aaa cca agt aat gag cac act gct gag 864
atg gaa cac atg aaa tct ttg gtt cac aga cta ttt aca atc ttg cat 912
tta gaa gag tct cag aaa aag aga gag cac cat tta ctg gag aaa att      960
gac cac ctg aag gaa cag ctg cag ccc ctt gaa cag gtg aaa gct gga      1008
ata gaa gct cat tcg gaa gcc aaa acc agt gga ctc ctg tgg gct gga      1056
ttg gca ctg ctg tcc att cag ggt ggg gca ctg gcc tgg ctc acg tgg      1104
tgg gtg tac tcc tgg gat atc atg gag cca gtt aca tac ttc atc aca      1152
ttt gca aat tct atg gtc ttt ttt gca tac ttt ata gtc act cga cag      1200
gat tat act tac tca gct gtt aag agt agg caa ttt ctt cag ttc ttc      1248
cac aag aaa tca aag caa cag cac ttt gat gtg cag caa tac aac aag      1296
tta aaa gaa gac ctt gct aag gct aaa gaa tcc ctg aaa cag gcg cgt      1344
cat tct ctc tgt ttg caa atg caa gta gaa gaa ctc aat gaa aag aat      1392
taatcttaca gttttaaatg tcgtcagatt ttccattatg tattgatttt gcaacttagg      1452
atgtttttga gtcccatggt tcattttgat tgtttaatct ttgttattaa attcttgtaa      1512
aacagaaaaa aaaaaaa                                                     1529

<210> 75
<211> 1340
<212> DNA
<213> Homo sapiens

<220>
<221> CDS
<222> (86)..(1294)

<400> 75
gagccaagac ggtcggggct gcttgctaac tccaggaaca ggtttaagtt tttgaaactg      60

aagtaggtct acacagtagg aactc atg tca ttt ctt acc aat gat gcg agc 112
Met Ser Phe Leu Thr Asn Asp Ala Ser
1               5

tca gag tca ata gca tcc ttc tct aaa cag gag gtc atg agt agc ttt 160
Ser Glu Ser Ile Ala Ser Phe Ser Lys Gln Glu Val Met Ser Ser Phe
10                  15                  20                  25

ctg cca gag gga ggg tgt tac gag ctg ctc act gtg ata ggc aaa gga 208
Leu Pro Glu Gly Gly Cys Tyr Glu Leu Leu Thr Val Ile Gly Lys Gly
                30                  35                  40

ttt gag gac ctg atg act gtg aat cta gca agg tac aaa cca aca gga 256
Phe Glu Asp Leu Met Thr Val Asn Leu Ala Arg Tyr Lys Pro Thr Gly
            45                  50                  55

gag tac gtg act gta cgg agg att aac cta gaa gct tgt tcc aat gag 304
Glu Tyr Val Thr Val Arg Arg Ile Asn Leu Glu Ala Cys Ser Asn Glu
        60                  65                  70

atg gta aca ttc ttg cag ggc gag ctg cat gtc tcc aaa ctc ttc aac 352
Met Val Thr Phe Leu Gln Gly Glu Leu His Val Ser Lys Leu Phe Asn
    75                  80                  85

cat ccc aat atc gtg cca tat cga gcc act ttt att gca gac aat gag 400
His Pro Asn Ile Val Pro Tyr Arg Ala Thr Phe Ile Ala Asp Asn Glu
90                  95                  100                 105

ctg tgg gtt gtc aca tca ttc atg gca tac ggt tct gca aaa gat ctc 448
Leu Trp Val Val Thr Ser Phe Met Ala Tyr Gly Ser Ala Lys Asp Leu
                110                 115                 120

atc tgt aca cac ttc atg gat ggc atg aat gag ctg gcg att gct tac 496
Ile Cys Thr His Phe Met Asp Gly Met Asn Glu Leu Ala Ile Ala Tyr
            125                 130                 135

atc ctg cag ggg gtg ctg aag gcc ctc gac tac atc cac cac atg gga 544
Ile Leu Gln Gly Val Leu Lys Ala Leu Asp Tyr Ile His His Met Gly
        140                 145                 150

tat gta cac agg agt gtc aaa gcc agc cac atc ctg atc tct gtg gat 592
Tyr Val His Arg Ser Val Lys Ala Ser His Ile Leu Ile Ser Val Asp
    155                 160                 165

ggg aag gtc tac ctg tct ggt ttg cgc agc aac ctc agc atg ata agc 640
Gly Lys Val Tyr Leu Ser Gly Leu Arg Ser Asn Leu Ser Met Ile Ser
170                 175                 180                 185

cat ggg cag cgg cag cga gtg gtc cac gat ttt ccc aag tac agt gtc 688
His Gly Gln Arg Gln Arg Val Val His Asp Phe Pro Lys Tyr Ser Val
                190                 195                 200

aag gtt ctg ccg tgg ctc agc ccc gag gtc ctc cag cag aat ctc cag 736
Lys Val Leu Pro Trp Leu Ser Pro Glu Val Leu Gln Gln Asn Leu Gln
            205                 210                 215

ggt tat gat gcc aag tct gac atc tac agt gtg gga atc aca gcc tgt 784
Gly Tyr Asp Ala Lys Ser Asp Ile Tyr Ser Val Gly Ile Thr Ala Cys
        220                 225                 230

gaa ctg gcc aac ggc cat gtc ccc ttt aag gat atg cct gcc acc cag          832
Glu Leu Ala Asn Gly His Val Pro Phe Lys Asp Met Pro Ala Thr Gln
    235                 240                 245

ccc agg tcc tgg ttc tgt cct ccc cag atg ctg cta gag aaa ctg aac          880
Pro Arg Ser Trp Phe Cys Pro Pro Gln Met Leu Leu Glu Lys Leu Asn
250                 255                 260                 265

ggc aca gtg ccc tgc ctg ttg gat acc agc acc atc ccc gct gag gag          928
Gly Thr Val Pro Cys Leu Leu Asp Thr Ser Thr Ile Pro Ala Glu Glu
                270                 275                 280

ctg acc atg agc cct tcg cgc tca gtg gcc aac tct ggc ctg agt gac          976
Leu Thr Met Ser Pro Ser Arg Ser Val Ala Asn Ser Gly Leu Ser Asp
            285                 290                 295

agc ctg acc acc agc acc ccc cgg ccc tcc aac ggt gac tcg ccc tcc         1024
Ser Leu Thr Thr Ser Thr Pro Arg Pro Ser Asn Gly Asp Ser Pro Ser
        300                 305                 310

cac ccc tac cac cga acc ttc tcc ccc cac ttc cac cac ttt gtg gag         1072
His Pro Tyr His Arg Thr Phe Ser Pro His Phe His His Phe Val Glu
    315                 320                 325

cag tgc ctt cag cgc aac ccg gat gcc agg ccc agt gcc agc acc ctc         1120
Gln Cys Leu Gln Arg Asn Pro Asp Ala Arg Pro Ser Ala Ser Thr Leu
330                 335                 340                 345

ctg aac cac tct ttc ttc aag cag atc aag cga cgt gcc tca gag gct         1168
Leu Asn His Ser Phe Phe Lys Gln Ile Lys Arg Arg Ala Ser Glu Ala
                350                 355                 360

ttg ccc gaa ttg ctt cgt cct gtc acc ccc atc acc aat ttt gag ggc         1216
Leu Pro Glu Leu Leu Arg Pro Val Thr Pro Ile Thr Asn Phe Glu Gly
            365                 370                 375

agc cag tct cag gac cac agt gga atc ttt ggc ctg gta aca aac ctg         1264
Ser Gln Ser Gln Asp His Ser Gly Ile Phe Gly Leu Val Thr Asn Leu
        380                 385                 390

gaa gag ctg gag gtg gac gat tgg gag ttc t gagcctctgc aaactgtgcg         1315
Glu Glu Leu Glu Val Asp Asp Trp Glu Phe
    395                 400

cattctccag ccagggatgc agagg                                             1340

<210> 76
<211> 4536
<212> DNA
<213> Homo sapiens

<220>
<221> CDS
<222> (300)..(3173)

<400> 76
   aggggacaaa aagagagcta gaaaaaatag ggggaggggg tcatattgat tcctgtggga 60
   gatgaaattc ggatattaaa caactgacct aagtcgctta cagttcttcc tcccaccttt 120
   ctattttgcc tcttaatcag cccaggcgtc ctttccctga gattaaaaaa aagagagagg 180
   ggagcgttaa caattaaatg taataagcaa ctccaaagat ggggatgctg tcgaggtggt 240
   agttccgtgc aaaccttgct attaccgttg caaatagacg cggagcccaa ggaggtaaa 299
   atg cac act tgc tgc ccc cca gta act ttg gaa cag gac ctt cac aga 347
   Met His Thr Cys Cys Pro Pro Val Thr Leu Glu Gln Asp Leu His Arg
   1 5 10 15
   aaa atg cat agc tgg atg ctg cag act cta gcg ttt gct gta aca tct 395
   Lys Met His Ser Trp Met Leu Gln Thr Leu Ala Phe Ala Val Thr Ser
   20 25 30
   ctc gtc ctt tcg tgt gca gaa acc atc gat tat tat ggg gaa atc tgt 443
   Leu Val Leu Ser Cys Ala Glu Thr Ile Asp Tyr Tyr Gly Glu Ile Cys
   35 40 45
   gac aat gca tgt cct tgt gag gaa aag gac ggc att tta act gtg agc 491
   Asp Asn Ala Cys Pro Cys Glu Glu Lys Asp Gly Ile Leu Thr Val Ser
   50 55 60
   tgt gaa aac cgg ggg atc atc agt ctc tct gaa att agc cct ccc cgt 539
   Cys Glu Asn Arg Gly Ile Ile Ser Leu Ser Glu Ile Ser Pro Pro Arg
   65 70 75 80
   ttc cca atc tac cac ctc ttg ttg tcc gga aac ctt ttg aac cgt ctc 587
   Phe Pro Ile Tyr His Leu Leu Leu Ser Gly Asn Leu Leu Asn Arg Leu
   85 90 95
   tat ccc aat gag ttt gtc aat tac act ggg gct tca att ttg cat cta 635
   Tyr Pro Asn Glu Phe Val Asn Tyr Thr Gly Ala Ser Ile Leu His Leu
   100 105 110
   ggt agc aat gtt atc cag gac att gag acc ggg gct ttc cat ggg cta 683
   Gly Ser Asn Val Ile Gln Asp Ile Glu Thr Gly Ala Phe His Gly Leu
   115 120 125
   cgg ggt ttg agg aga ttg cat cta aac aat aat aaa ctg gaa ctt ctg 731
   Arg Gly Leu Arg Arg Leu His Leu Asn Asn Asn Lys Leu Glu Leu Leu
   130 135 140
   cga gat gat acc ttc ctt ggc ttg gag aac ctg gag tac cta cag gtc 779
   Arg Asp Asp Thr Phe Leu Gly Leu Glu Asn Leu Glu Tyr Leu Gln Val
   145 150 155 160
   gat tac aac tac atc agc gtc att gaa ccc aat gct ttt ggg aaa ctg 827
   Asp Tyr Asn Tyr Ile Ser Val Ile Glu Pro Asn Ala Phe Gly Lys Leu
   165 170 175
   cat ttg ttg cag gtg ctt atc ctc aat gac aat ctt ttg tcc agt tta 875
   His Leu Leu Gln Val Leu Ile Leu Asn Asp Asn Leu Leu Ser Ser Leu
   180 185 190
   ccc aac aat ctt ttc cgt ttt gtg ccc tta acg cac ttg gac ctc cgg 923
   Pro Asn Asn Leu Phe Arg Phe Val Pro Leu Thr His Leu Asp Leu Arg
   195 200 205
   ggg aac cgg ctg aaa ctt ctg ccc tac gtg ggg ctc ttg cag cac atg 971
   Gly Asn Arg Leu Lys Leu Leu Pro Tyr Val Gly Leu Leu Gln His Met
   210 215 220
   gat aaa gtt gtg gag cta cag ctg gag gaa aac cct tgg aat tgt tct 1019
   Asp Lys Val Val Glu Leu Gln Leu Glu Glu Asn Pro Trp Asn Cys Ser
   225 230 235 240
   tgt gag ctg atc tct cta aag gat tgg ttg gac agc atc tcc tat tca 1067
   Cys Glu Leu Ile Ser Leu Lys Asp Trp Leu Asp Ser Ile Ser Tyr Ser
   245 250 255
   gcc ctg gtg ggg gat gta gtt tgt gag acc ccc ttc cgc tta cac gga 1115
   Ala Leu Val Gly Asp Val Val Cys Glu Thr Pro Phe Arg Leu His Gly
   260 265 270
   agg gac ttg gac gag gta tcc aag cag gaa ctt tgc cca agg aga ctt 1163
   Arg Asp Leu Asp Glu Val Ser Lys Gln Glu Leu Cys Pro Arg Arg Leu
   275 280 285
   att tct gac tac gag atg agg ccg cag acg cct ttg agc acc acg ggg 1211
   Ile Ser Asp Tyr Glu Met Arg Pro Gln Thr Pro Leu Ser Thr Thr Gly
   290 295 300
   tat tta cac acc acc ccg gcg tca gtg aat tct gtg gcc act tct tcc 1259
   Tyr Leu His Thr Thr Pro Ala Ser Val Asn Ser Val Ala Thr Ser Ser
   305 310 315 320
   tct gct gtt tac aaa ccc cct ttg aag ccc cct aag ggg act cgc caa 1307
   Ser Ala Val Tyr Lys Pro Pro Leu Lys Pro Pro Lys Gly Thr Arg Gln
   325 330 335
   ccc aac aag ccc agg gtg cgc ccc acc tct cgg cag ccc tct aag gac 1355
   Pro Asn Lys Pro Arg Val Arg Pro Thr Ser Arg Gln Pro Ser Lys Asp
   340 345 350
   ttg ggc tac agc aac tat ggc ccc agc atc gcc tat cag acc aaa tcc 1403
   Leu Gly Tyr Ser Asn Tyr Gly Pro Ser Ile Ala Tyr Gln Thr Lys Ser
   355 360 365
   ccg gtg cct ttg gag tgt ccc acc gcg tgc tct tgc aac ctg cag atc 1451
   Pro Val Pro Leu Glu Cys Pro Thr Ala Cys Ser Cys Asn Leu Gln Ile
   370 375 380
   tct gat ctg ggc ctc aac gta aac tgc cag gag cga aag atc gag agc 1499
   Ser Asp Leu Gly Leu Asn Val Asn Cys Gln Glu Arg Lys Ile Glu Ser
   385 390 395 400
   atc gct gaa ctg cag ccc aag ccc tac aat ccc aag aaa atg tat ctg 1547
   Ile Ala Glu Leu Gln Pro Lys Pro Tyr Asn Pro Lys Lys Met Tyr Leu
   405 410 415
   aca gag aac tac atc gct gtc gtg cgc agg aca gac ctc ctg gag gcc 1595
   Thr Glu Asn Tyr Ile Ala Val Val Arg Arg Thr Asp Leu Leu Glu Ala
   420 425 430
   acg ggg ctg gac ctc ctg cac ctg ggg aat aac cgc atc tcg atg atc 1643
   Thr Gly Leu Asp Leu Leu His Leu Gly Asn Asn Arg Ile Ser Met Ile
   435 440 445
   cag gac cgc gct ttc ggg gat ctc acc aac ctg agg cgc ctc tac ctg 1691
   Gln Asp Arg Ala Phe Gly Asp Leu Thr Asn Leu Arg Arg Leu Tyr Leu
   450 455 460
   aat ggc aac agg atc gag agg ctg agc ccg gag tta ttc tat ggc ctg 1739
   Asn Gly Asn Arg Ile Glu Arg Leu Ser Pro Glu Leu Phe Tyr Gly Leu
   465 470 475 480
   cag agc ctg cag tat ctc ttc ctc cag tac aat ctc atc cgc gag att 1787
   Gln Ser Leu Gln Tyr Leu Phe Leu Gln Tyr Asn Leu Ile Arg Glu Ile
   485 490 495
   cag tct gga act ttt gac ccg gtc cca aac ctc cag ctg cta ttc ttg 1835
   Gln Ser Gly Thr Phe Asp Pro Val Pro Asn Leu Gln Leu Leu Phe Leu
   500 505 510
   aat aac aac ctc ctg cag gcc atg ccc tca ggc gtc ttc tct ggc ttg 1883
   Asn Asn Asn Leu Leu Gln Ala Met Pro Ser Gly Val Phe Ser Gly Leu
   515 520 525
   acc ctc ctc agg cta aac ctg agg agt aac cac ttc acc tcc ttg cca 1931
   Thr Leu Leu Arg Leu Asn Leu Arg Ser Asn His Phe Thr Ser Leu Pro
   530 535 540
   gtg agt gga gtt ttg gac cag ctg aag tca ctc atc caa atc gac ctg 1979
   Val Ser Gly Val Leu Asp Gln Leu Lys Ser Leu Ile Gln Ile Asp Leu
   545 550 555 560
   cat gac aat cct tgg gat tgt acc tgt gac att gtg ggc atg aag ctg 2027
   His Asp Asn Pro Trp Asp Cys Thr Cys Asp Ile Val Gly Met Lys Leu
   565 570 575
   tgg gtg gag cag ctc aaa gtg ggc gtc cta gtg gac gag gtg atc tgt 2075
   Trp Val Glu Gln Leu Lys Val Gly Val Leu Val Asp Glu Val Ile Cys
   580 585 590
   aag gcg ccc aaa aaa ttc gct gag acc gac atg cgc tcc att aag tcg 2123
   Lys Ala Pro Lys Lys Phe Ala Glu Thr Asp Met Arg Ser Ile Lys Ser
   595 600 605
   gag ctg ctg tgc cct gac tat tca gat gta gta gtt tcc acg ccc aca 2171
   Glu Leu Leu Cys Pro Asp Tyr Ser Asp Val Val Val Ser Thr Pro Thr
   610 615 620
   ccc tcc tct atc cag gtc cct gcg agg acc agc gcc gtg act cct gcg 2219
   Pro Ser Ser Ile Gln Val Pro Ala Arg Thr Ser Ala Val Thr Pro Ala
   625 630 635 640
   gtc cgg ttg aat agc acc ggg gcc ccc gcg agc ttg ggc gca ggc gga 2267
   Val Arg Leu Asn Ser Thr Gly Ala Pro Ala Ser Leu Gly Ala Gly Gly
   645 650 655
   ggg gcg tcg tcg gtg ccc ttg tct gtg tta att ctc agc ctc ctg ctg 2315
   Gly Ala Ser Ser Val Pro Leu Ser Val Leu Ile Leu Ser Leu Leu Leu
   660 665 670
   gtt ttc atc atg tcc gtc ttc gtg gcc gcc ggg ctc ttc gtg ctg gtc 2363
   Val Phe Ile Met Ser Val Phe Val Ala Ala Gly Leu Phe Val Leu Val
   675 680 685
   atg aag cgc agg aag aag aac cag agc gac cac acc agc acc aac aac 2411
   Met Lys Arg Arg Lys Lys Asn Gln Ser Asp His Thr Ser Thr Asn Asn
   690 695 700
   tcc gac gtg agc tcc ttt aac atg cag tac agc gtg tac ggc ggc ggc 2459
   Ser Asp Val Ser Ser Phe Asn Met Gln Tyr Ser Val Tyr Gly Gly Gly
   705 710 715 720
   ggc ggc acg ggc ggc cac cca cac gcg cac gtg cat cac cgc ggg ccc 2507
   Gly Gly Thr Gly Gly His Pro His Ala His Val His His Arg Gly Pro
   725 730 735
   gcg ctg ccc aag gtg aag acg ccc gcg ggc cac gtg tat gaa tac atc 2555
   Ala Leu Pro Lys Val Lys Thr Pro Ala Gly His Val Tyr Glu Tyr Ile
   740 745 750
   ccc cac cca ctg ggc cac atg tgc aaa aac ccc atc tac cgc tcc cga 2603
   Pro His Pro Leu Gly His Met Cys Lys Asn Pro Ile Tyr Arg Ser Arg
   755 760 765
   gag ggc aac tcc gta gag gat tac aaa gac ctg cac gag ctc aag gtc 2651
   Glu Gly Asn Ser Val Glu Asp Tyr Lys Asp Leu His Glu Leu Lys Val
   770 775 780
   acc tac agc agc aac cac cac ctg cag cag cag cag cag ccg ccg ccg 2699
   Thr Tyr Ser Ser Asn His His Leu Gln Gln Gln Gln Gln Pro Pro Pro
   785 790 795 800
   cca ccg cag cag cca cag cag cag ccc ccg ccg cag ctg cag ctg cag 2747
   Pro Pro Gln Gln Pro Gln Gln Gln Pro Pro Pro Gln Leu Gln Leu Gln
   805 810 815
   cct ggg gag gag gag agg cgg gaa agc cac cac ttg cgg agc ccc gcc 2795
   Pro Gly Glu Glu Glu Arg Arg Glu Ser His His Leu Arg Ser Pro Ala
   820 825 830
   tac agc gtc agc acc atc gag ccc cgg gag gac ctg ctg tcg ccg gtg 2843
   Tyr Ser Val Ser Thr Ile Glu Pro Arg Glu Asp Leu Leu Ser Pro Val
   835 840 845
   cag gac gcc gac cgc ttt tac agg ggc att tta gaa cca gac aaa cac 2891
   Gln Asp Ala Asp Arg Phe Tyr Arg Gly Ile Leu Glu Pro Asp Lys His
   850 855 860
   tgc tcc acc acc ccc gcc ggc aat agc ctc ccg gaa tat ccc aaa ttc 2939
   Cys Ser Thr Thr Pro Ala Gly Asn Ser Leu Pro Glu Tyr Pro Lys Phe
   865 870 875 880
   ccg tgc agc ccc gct gct tac act ttc tcc ccc aac tat gac ctg aga 2987
   Pro Cys Ser Pro Ala Ala Tyr Thr Phe Ser Pro Asn Tyr Asp Leu Arg
   885 890 895
   cgc ccc cat cag tat ttg cac ccg ggg gca ggg gac agc agg cta cgg 3035
   Arg Pro His Gln Tyr Leu His Pro Gly Ala Gly Asp Ser Arg Leu Arg
   900 905 910
   gaa ccg gtg ctc tac agc ccc ccg agt gct gtc ttt gta gaa ccc aac 3083
   Glu Pro Val Leu Tyr Ser Pro Pro Ser Ala Val Phe Val Glu Pro Asn
   915 920 925
   cgg aac gaa tat ctg gag tta aaa gca aaa cta aac gtt gag ccg gac 3131
   Arg Asn Glu Tyr Leu Glu Leu Lys Ala Lys Leu Asn Val Glu Pro Asp
   930 935 940
   tac ctc gaa gtg ctg gaa aaa cag acc acg ttt agc cag ttc taaaagc 3180
   Tyr Leu Glu Val Leu Glu Lys Gln Thr Thr Phe Ser Gln Phe
   945 950 955
   aaagaaactc tcttggagct tttgcattta aaacaaacaa gcaagcagac acacacagtg 3240
   aacacatttg attaattgtg ttgtttcaac gtttagggtg aagtgccttg gcacgggatt 3300
   tctcagcttc ggtggaagat acgaaaaggg tgtgcaattt cctttaaaat ttacacgtgg 3360
   gaaacatttg tgtaaactgg gcacatcact ttctcttctt gcgtgtgggg caggtgtgga 3420
   gaagggcttt aaggaggcca atttgctgcg cgggtgacct gtgaaaggtc acagtcattt 3480
   ttgtagtggt tggaagtgct aagaatggtg gatgatggca gagcatagat tctactcttc 3540
   ctcttttgct tcctccccct cccccgcccc tgccccacct ctctttctcc ccttttaagc 3600
   catgggtggg tctaactggc ttttgtggag aaattagcac accccaactt taataggaaa 3660
   tttgttctct ttttccgccc ctctccttct ctcctcccct cccctccctt ctcattcctt 3720
   ttctttgttt ttaaaggatg tgtttggatg cattctggac atttgaatta aaaaaaaagt 3780
   attgtgatcc tgtaaaggat caccatagat gtggacaaat cattaaaatt acagagctat 3840
   atgatccata attgattagt caaaataact tattgatgaa atatacaaat attttattgt 3900
   agcacctatt tttatatgca catttagcat tcctctttcc ttcactattt agcctatgat 3960
   tttgcagagg tgtcacactg tattaggatc tgcatttcta aaactgacgt ggtatcagga 4020
   aggcattttc aatcattcaa aatgtggaga atttaatggc taaatcttta aaagccaatg 4080
   caacccaccc aattgaatct gcattttctt ttaagaaaac agagctgatt gtatcccaat 4140
   gtattttaaa aaatagggca attgattggg ccattccgag agaattgttt gcaagttttg 4200
   ggttttatta gaaaatattt gaaagtattt ttattaatga accaaaatga catgttcatt 4260
   tgactactat tgtagccgat tttcgattgt ttaaccaaac ccagttgcat ttgtacagat 4320
   ccacgtgtac tggcacctca gaagaccaaa tcatggactg tacaagtctc tatacaatgt 4380
   ctttatccct gtgggcagca agcaatgatg ataatgacaa acaggatatc tgtaagatgg 4440
   ggctactgtt gttacagtct catatgtatc ccagcacatg taatttttta aatagtttct 4500
   gaataaacac ttgataacta tgtcaaaaaa aaaaaa 4536

<210> 77
<211> 1251

<212> DNA
<213> Homo sapiens

<220>
<221> CDS
<222> (1)..(1140)

<400> 77
atg gtt gat gtc gaa ttc ttt ggc aac ttt tta tgt agt tgt aag cag 48
atc agc ttc aat gat tgt tct caa ttg gtt gtc gtc aac ttc caa tgg 96
cag gcc act atg ctc ctc atc ttc aag ggt ctc gtc tcc ttt gca gaa 144
ctt cca gaa cca cca ctg cac tgc cca aga acc ctc ttg aga tgc tct 192
tgc aga ata aga gat tat cat ctt caa gaa cgc aac tat gga acc tgc 240
acc tcc gca cat gac gct ggt tta tgg cag ata ccc cga cca ttg cca 288
tac cgc ttc ctt agc tca ggc ttt gtt gag tac tct gca gat aac cca 336
ata aga cgg tat ttt gtg acc cca gcc ata agg agc ctt gcc ctc ctt 384
gct gcc att ctc ctg gtg gcc ctg cag gct cgg gcg gag cca ctc cag 432
gca att gct gat gag gct aca gcc cag gag cag cct gga gca gat gat 480
cag gaa gtg gtt gat tcc ttt gca tgg gat gaa aga gct cct ctt cag 528
gtt tca ggg aag tcc tct cct gtt tgt gca cgg ctg ctc ttg cta cag 576
gag acc cgg gac aga gga ctg ctg ttt gcc ctc cct ctt cac tct gcc 624
tac ctt gag gat ctg ctc agg cag agc cac ttc agg caa gag ctg atg 672
aag ctg cag ccc agg agc agc ctg gag cag atg atc agg aaa tgg ctc 720
atg cct tta cat ggc atg aaa gtg ccg ctc ttc cgc ttt cag cct gat 768
aaa atc att gtc ctc tcc acc ctg att cct aca gga gac tac tca ccc 816
cat aac ctc aaa aac ctc ttc atg agg atg gtg acc cca gcc atg agg 864
acc ctc gcc atc ctt gct gcc att ctc ctg gtg gcc ctg cag gcc cag 912
gct gag cca ctc cag gca aga gct gat gag gtt gct gca gcc ccg gag 960
cag att gca gcg gac atc cca gaa gtg gtt gtt tcc ctt gca tgg gac 1008
gaa agc ttg gct cca aag cat cca ggc tca agg aaa aac atg gac tgc 1056
tat tgc aga ata cca gcg tgc att gca gga gaa cgt cgc tat gga acc 1104
tgc atc tac cag gga aga ctc tgg gca ttc tgc tgc tgag cttgcagaaa 1154
aagaaaaatg agctcaaaat ttgctttgag agctacaggg aattgctatt actcctgtac 1214
cttctgctca atttcctttc ctcatctcaa ataaatg 1251

<210> 78
<211> 520
<212> DNA
<213> Homo sapiens

<220>
<221> CDS
<222> (7)..(234)

<400> 78
tacacc atg agc aaa gct cac cct ccc gag ttg aaa aaa ttt atg gac 48
Met Ser Lys Ala His Pro Pro Glu Leu Lys Lys Phe Met Asp
1               5                   10

aag aag tta tca ttg aaa tta aat ggt ggc aga cat gtc caa gga ata 96
Lys Lys Leu Ser Leu Lys Leu Asn Gly Gly Arg His Val Gln Gly Ile
15                  20                  25                  30

ttg cgg gga ttt gat ccc ttt atg aac ctt gtg ata gat gaa tgt gtg 144
Leu Arg Gly Phe Asp Pro Phe Met Asn Leu Val Ile Asp Glu Cys Val
                35                  40                  45

gag atg gcg act agt gga caa cag aac aat att gga atg gtg gta ata 192
Glu Met Ala Thr Ser Gly Gln Gln Asn Asn Ile Gly Met Val Val Ile
            50                  55                  60

cga gga aat agt atc atc atg tta gaa gcc ttg gaa cga gta taaataa 241
Arg Gly Asn Ser Ile Ile Met Leu Glu Ala Leu Glu Arg Val
        65                  70                  75

tggctgttca gcagagaaac ccatgtcctc tctccatagg gcctgtttta ctatgatgta 301

aaaattaggt catgtacatt ttcatattag actttttgtt aaataaactt ttgtaatagt 361

caaaaatgct ttttcagatg ttttgaatat agaaaatcag ctctcattcc agtttttttt 421

aacatgaatt ttcctggttg acattgattt caaagggttt tatgcattaa agtgaaagaa 481

ttttattaaa tgtgaaacat ggcaaggaaa aaaaaaaaa 520

<210> 79
<211> 2083
<212> DNA
<213> Homo sapiens

<220>
<221> CDS
<222> (82)..(1818)

<400> 79
aattcccggg tcgacgattt cgtgcggccg ggagcccgcc gcgctggtag cgatattaat 60

aaggcagcgg aaagaagaaa t atg aat acg gct cca tca aga ccc agc ccc 111
Met Asn Thr Ala Pro Ser Arg Pro Ser Pro
1               5                   10

aca cga agg gat cca tat ggc ttt gga gac agt cga gat tca agg cgt 159
Thr Arg Arg Asp Pro Tyr Gly Phe Gly Asp Ser Arg Asp Ser Arg Arg
                15                  20                  25

gat cga tcc cca att cga gga agt cca agg aga gag ccc agg gat ggc 207
Asp Arg Ser Pro Ile Arg Gly Ser Pro Arg Arg Glu Pro Arg Asp Gly
            30                  35                  40

aga aat ggc cgg gat gcc cgg gac agc aga gac att cga gac ccc cga 255
Arg Asn Gly Arg Asp Ala Arg Asp Ser Arg Asp Ile Arg Asp Pro Arg

45                    50                    55

gac ttg cgg gac cac aga cat agt aga gat ttg cgg gat cac aga gac          303
Asp Leu Arg Asp His Arg His Ser Arg Asp Leu Arg Asp His Arg Asp
    60                  65                  70

agc agg agt gtg cgc gac gtt cgg gac gtg agg gat ctt aga gac ttt          351
Ser Arg Ser Val Arg Asp Val Arg Asp Val Arg Asp Leu Arg Asp Phe
75                  80                  85                  90

cgt gat cta aga gac tct agg gat ttt cga gat cag cga gac ccc atg          399
Arg Asp Leu Arg Asp Ser Arg Asp Phe Arg Asp Gln Arg Asp Pro Met
                95                  100                 105

tac gac aga tac aga gac atg aga gac tcc cga gat cct atg tac agg          447
Tyr Asp Arg Tyr Arg Asp Met Arg Asp Ser Arg Asp Pro Met Tyr Arg
            110                 115                 120

aga gaa ggc tct tat gac cga tac cta cga atg gat gac tat tgc agg          495
Arg Glu Gly Ser Tyr Asp Arg Tyr Leu Arg Met Asp Asp Tyr Cys Arg
        125                 130                 135

aga aag gat gac tct tat ttt gac cgt tac aga gat agc ttt gat gga          543
Arg Lys Asp Asp Ser Tyr Phe Asp Arg Tyr Arg Asp Ser Phe Asp Gly
    140                 145                 150

cgg ggc cct cca ggc cca gaa agt cag tct cgt gca aaa gag cgt ttg          591
Arg Gly Pro Pro Gly Pro Glu Ser Gln Ser Arg Ala Lys Glu Arg Leu
155                 160                 165                 170

aaa cgt gag gaa cgg cgt aga gaa gag ctt tat cgt caa tat ttt gag          639
Lys Arg Glu Glu Arg Arg Arg Glu Glu Leu Tyr Arg Gln Tyr Phe Glu
                175                 180                 185

gaa atc cag aga cgc ttt gat gcc gaa agg ccc gtt gat tgt tct gtg          687
Glu Ile Gln Arg Arg Phe Asp Ala Glu Arg Pro Val Asp Cys Ser Val
            190                 195                 200

att gtg gtc aac aaa cag aca aaa gac tat gct gag tct gtg ggg cgg          735
Ile Val Val Asn Lys Gln Thr Lys Asp Tyr Ala Glu Ser Val Gly Arg
        205                 210                 215

aag gtg cga gac ctg ggc atg gta gtg gac ttg atc ttc ctt aac aca          783
Lys Val Arg Asp Leu Gly Met Val Val Asp Leu Ile Phe Leu Asn Thr
    220                 225                 230

gaa gtg tca ctg tca caa gcc ttg gag gat gtt agc agg gga ggt tct          831
Glu Val Ser Leu Ser Gln Ala Leu Glu Asp Val Ser Arg Gly Gly Ser
235                 240                 245                 250

cct ttt gct att gtc atc acc cag caa cac cag att cac cgc tcc tgc          879
Pro Phe Ala Ile Val Ile Thr Gln Gln His Gln Ile His Arg Ser Cys
                255                 260                 265

aca gtc aac atc atg ttt gga acc ccg caa gag cat cgc aac atg ccc          927
Thr Val Asn Ile Met Phe Gly Thr Pro Gln Glu His Arg Asn Met Pro
            270                 275                 280

caa gca gat gcc atg gtg ctg gtg gcc aga aat tat gag cgt tac aag          975
Gln Ala Asp Ala Met Val Leu Val Ala Arg Asn Tyr Glu Arg Tyr Lys

        285                 290                 295

aat gag tgc cgg gag aag gaa cgt gag gag att gcc aga cag gca gcc 1023
Asn Glu Cys Arg Glu Lys Glu Arg Glu Glu Ile Ala Arg Gln Ala Ala
    300                 305                 310

aag atg gcc gat gaa gcc atc ctg cag gaa aga gag aga gga ggc cct 1071
Lys Met Ala Asp Glu Ala Ile Leu Gln Glu Arg Glu Arg Gly Gly Pro
315                 320                 325                 330

gag gag gga gtg cgt ggg ggc cac cct cca gcc atc cag agc ctc atc 1119
Glu Glu Gly Val Arg Gly Gly His Pro Pro Ala Ile Gln Ser Leu Ile
                335                 340                 345

aac ctg ctg gca gac aac agg tac ctc act gct gaa gag act gac aag 1167
Asn Leu Leu Ala Asp Asn Arg Tyr Leu Thr Ala Glu Glu Thr Asp Lys
            350                 355                 360

atc atc aac tac ctg cga gag cgg aag gag cgg ctg atg agg agc agc 1215
Ile Ile Asn Tyr Leu Arg Glu Arg Lys Glu Arg Leu Met Arg Ser Ser
        365                 370                 375

acc gac tct ctg cct ggc ccg att tcc cgc caa cca ctc ggg gcg acc 1263
Thr Asp Ser Leu Pro Gly Pro Ile Ser Arg Gln Pro Leu Gly Ala Thr
    380                 385                 390

tcg ggt gcc tcg ctg aag aca cag cca agc tcc caa ccg ctc cag agc 1311
Ser Gly Ala Ser Leu Lys Thr Gln Pro Ser Ser Gln Pro Leu Gln Ser
395                 400                 405                 410

ggc caa gtg ctc ccc tct gct aca ccc act cca tct gca ccc ccc acc 1359
Gly Gln Val Leu Pro Ser Ala Thr Pro Thr Pro Ser Ala Pro Pro Thr
                415                 420                 425

tcc cag caa gag ctt cag gcc aaa atc ctc agc ctc ttc aat agt ggc 1407
Ser Gln Gln Glu Leu Gln Ala Lys Ile Leu Ser Leu Phe Asn Ser Gly
            430                 435                 440

aca gtg acg gcc aat agc agc tct gca tcc ccc tcg gtt gct gcc gga 1455
Thr Val Thr Ala Asn Ser Ser Ser Ala Ser Pro Ser Val Ala Ala Gly
        445                 450                 455

aac acc cca aac cag aat ttt tcc aca gca gca aac agc cag cct caa 1503
Asn Thr Pro Asn Gln Asn Phe Ser Thr Ala Ala Asn Ser Gln Pro Gln
    460                 465                 470

caa aga tca cag gct tct ggc aat cag cct cca agc att ttg gga cag 1551
Gln Arg Ser Gln Ala Ser Gly Asn Gln Pro Pro Ser Ile Leu Gly Gln
475                 480                 485                 490

gga gga tct gct cag aac atg ggc ccc aga cct ggg gct cct tcc caa 1599
Gly Gly Ser Ala Gln Asn Met Gly Pro Arg Pro Gly Ala Pro Ser Gln
                495                 500                 505

ggg ctt ttt ggc cag cct tcc agt cgc ctg gca cct gct agc aac atg 1647
Gly Leu Phe Gly Gln Pro Ser Ser Arg Leu Ala Pro Ala Ser Asn Met
            510                 515                 520

act agc cag agg cct gtg tct tcc aca ggt atc aac ttt gac aat cca 1695
Thr Ser Gln Arg Pro Val Ser Ser Thr Gly Ile Asn Phe Asp Asn Pro

525                    530                    535

agt gta cag aag gct ctg gat acc ctg atc cag agt ggc cct gct ctc      1743
Ser Val Gln Lys Ala Leu Asp Thr Leu Ile Gln Ser Gly Pro Ala Leu
    540                    545                    550

tcc cac ctg gtt agc cag acc aca gca cag atg ggg cag cca cag gcc      1791
Ser His Leu Val Ser Gln Thr Thr Ala Gln Met Gly Gln Pro Gln Ala
555                    560                    565                    570

ccc atg gga tct tac cag agg cat tac tgaag ctaaatcttt caactctccc      1843
Pro Met Gly Ser Tyr Gln Arg His Tyr
                575

cagtcccctc atcccctggc ctcctcccac ttacttgttc taaatagagc tgtttggagg    1903

atgttctctg cgctcccagg ccggcatcga gtgtcatcaa tttctaccac ctgctctctc    1963

ttctgcccaa ggctgtgttg cttattcctt acaaagttta tactgcattt ggggctgtat    2023

ctttttttgt tttttgtttt gtagaaaata aatatctccg ggggcagtaa aaaaaaaaaa    2083

<210> 80
<211> 438
<212> DNA
<213> Homo sapiens

<220>
<221> CDS
<222> (186)..(338)

<400> 80
ccctgttctt ctttacctaa ctctatagca gtaattggta catttcttcc atcaaagttt      60

catattttta gcaaagtatc aagccttacc gtacggaaag ctgcaaccac aagattggag     120

ggaaacaggt ttctgttgga aaagaaagca ttctgtcagt attcttttaa aacaattttt     180

tagag atg agg tcc ctg ttg ccc agg ctg gag tgc agt ggt gtg att      227
      Met Arg Ser Leu Leu Pro Arg Leu Glu Cys Ser Gly Val Ile
      1               5                   10

gca gct cac tgc agc ctt gaa ctt ttg ggc tca agt gat cct cct gcc      275
Ala Ala His Cys Ser Leu Glu Leu Leu Gly Ser Ser Asp Pro Pro Ala
15                  20                  25                  30

tca gcc tcc caa agt gtt ggg act aca ggc gtg agc cat ggt acc tgt      323
Ser Ala Ser Gln Ser Val Gly Thr Thr Gly Val Ser His Gly Thr Cys
                35                  40                  45

ctt cat cag tat tct taaaattcag tgtgcaaagt atctgctttg agatgtccac      378
Leu His Gln Tyr Ser
            50

ttctaaaacc ctaacacagt gttctctctc cacccctagc tgacactgca ccttttgctt    438

<210> 81
<211> 2693
<212> DNA
<213> Homo sapiens

<220>
<221> CDS
<222> (79)..(2334)

<400> 81
gacagatctg cgcgtatcct ggagccggcc cagttgtgaa ctaggagagc tttgggacct 60

ctgtcccaag caagagag atg aat gga gag tat aga ggc aga gga ttt gga 111
Met Asn Gly Glu Tyr Arg Gly Arg Gly Phe Gly
1               5                   10

cga gga aga ttt caa agc tgg aaa agg gga aga ggt ggt ggg aac ttc 159
Arg Gly Arg Phe Gln Ser Trp Lys Arg Gly Arg Gly Gly Gly Asn Phe
            15                  20                  25

tca gga aaa tgg aga gaa aga gaa cac aga cct gat ctg agt aaa acc 207
Ser Gly Lys Trp Arg Glu Arg Glu His Arg Pro Asp Leu Ser Lys Thr
        30                  35                  40

aca gga aaa cgt act tct ggg gac tta ata caa gaa att gga tac aaa 255
Thr Gly Lys Arg Thr Ser Gly Asp Leu Ile Gln Glu Ile Gly Tyr Lys
    45                  50                  55

aat gga aga ctg aaa gag cag aaa gga aat att gag gta act ccc cag 303
Asn Gly Arg Leu Lys Glu Gln Lys Gly Asn Ile Glu Val Thr Pro Gln
60                  65                  70                  75

aaa gta gta att cag gaa gca gca acc atc ctt atg gct aag aga cta 351
Lys Val Val Ile Gln Glu Ala Ala Thr Ile Leu Met Ala Lys Arg Leu
                80                  85                  90

aaa gaa gtt ctg agg tta cca gaa tgt ggg aat tca aag gat gaa ata 399
Lys Glu Val Leu Arg Leu Pro Glu Cys Gly Asn Ser Lys Asp Glu Ile
            95                  100                 105

gaa aga aag gga agt att ttg gta gat ttt aaa gaa ctg aca gaa ggt 447
Glu Arg Lys Gly Ser Ile Leu Val Asp Phe Lys Glu Leu Thr Glu Gly
        110                 115                 120

ggt gaa gta act aac ttg ata cca gat ata gca act gaa cta aga gat 495
Gly Glu Val Thr Asn Leu Ile Pro Asp Ile Ala Thr Glu Leu Arg Asp
    125                 130                 135

gca cct gag aaa acc ttg gct tgc atg ggt ttg gca ata cat cag gtg 543
Ala Pro Glu Lys Thr Leu Ala Cys Met Gly Leu Ala Ile His Gln Val
140                 145                 150                 155

tta act aag gac ctt gaa agg cat gca gct gag tta caa gcc cag gaa 591
Leu Thr Lys Asp Leu Glu Arg His Ala Ala Glu Leu Gln Ala Gln Glu
                160                 165                 170

gga ttg tct aat gat gga gaa aca atg gta aat gtg cca cat att cat 639
Gly Leu Ser Asn Asp Gly Glu Thr Met Val Asn Val Pro His Ile His

            175                 180                 185

gca agg gtg tac aac tat gag cct ttg aca cag ctc aag aat gtc aga 687
Ala Arg Val Tyr Asn Tyr Glu Pro Leu Thr Gln Leu Lys Asn Val Arg
        190                 195                 200

gca aat tac tat gga aaa tac att gct cta aga ggg aca gtg gtt cgt 735
Ala Asn Tyr Tyr Gly Lys Tyr Ile Ala Leu Arg Gly Thr Val Val Arg
    205                 210                 215

gtc agt aat ata aag cct ctt tgc acc aag atg gct ttt ctt tgt gct 783
Val Ser Asn Ile Lys Pro Leu Cys Thr Lys Met Ala Phe Leu Cys Ala
220                 225                 230                 235

gca tgt gga gaa att cag agc ttt cct ctt cca gat gga aaa tac agt 831
Ala Cys Gly Glu Ile Gln Ser Phe Pro Leu Pro Asp Gly Lys Tyr Ser
                240                 245                 250

ctt ccc aca aag tgt cct gtg cct gtg tgt cga ggc agg tca ttt act 879
Leu Pro Thr Lys Cys Pro Val Pro Val Cys Arg Gly Arg Ser Phe Thr
            255                 260                 265

gct ctc cgc agc tct cct ctc aca gtt acg atg gac tgg cag tca atc 927
Ala Leu Arg Ser Ser Pro Leu Thr Val Thr Met Asp Trp Gln Ser Ile
        270                 275                 280

aaa atc cag gaa ttg atg tct gat gat cag aga gaa gca ggt cgg att 975
Lys Ile Gln Glu Leu Met Ser Asp Asp Gln Arg Glu Ala Gly Arg Ile
    285                 290                 295

cca cga aca ata gaa tgt gag ctt gtt cat gat ctt gtg gat agc tgt 1023
Pro Arg Thr Ile Glu Cys Glu Leu Val His Asp Leu Val Asp Ser Cys
300                 305                 310                 315

gtc ccg gga gac aca gtg act att act gga att gtc aaa gtc tca aat 1071
Val Pro Gly Asp Thr Val Thr Ile Thr Gly Ile Val Lys Val Ser Asn
                320                 325                 330

gcg gaa gaa ggt ttg gca tta gca ctc ttt gga gga agc cag aaa tac 1119
Ala Glu Glu Gly Leu Ala Leu Ala Leu Phe Gly Gly Ser Gln Lys Tyr
            335                 340                 345

gca gat gac aaa aac aga att cca att cgg gga gac ccc cac atc ctt 1167
Ala Asp Asp Lys Asn Arg Ile Pro Ile Arg Gly Asp Pro His Ile Leu
        350                 355                 360

gtt gtt gga gat cca ggc cta gga aaa agt caa atg cta cag gca gcg 1215
Val Val Gly Asp Pro Gly Leu Gly Lys Ser Gln Met Leu Gln Ala Ala
    365                 370                 375

tgc aat gtt gcc cca cgt ggc gtg tat gtt tgt ggt aac acc acg acc 1263
Cys Asn Val Ala Pro Arg Gly Val Tyr Val Cys Gly Asn Thr Thr Thr
380                 385                 390                 395

acc tct ggt ctg acg gta act ctt tca aaa gat agt tcc tct gga gat 1311
Thr Ser Gly Leu Thr Val Thr Leu Ser Lys Asp Ser Ser Ser Gly Asp
                400                 405                 410

ttt gct ttg gaa gct ggt gcc ctg gta ctt ggt gat caa ggt att tgt 1359
Phe Ala Leu Glu Ala Gly Ala Leu Val Leu Gly Asp Gln Gly Ile Cys

415                 420                 425

gga atc gat gaa ttt gat aag atg ggg aat caa cat caa gcc ttg ttg     1407
Gly Ile Asp Glu Phe Asp Lys Met Gly Asn Gln His Gln Ala Leu Leu
        430                 435                 440

gaa gcc atg gag cag caa agt att agt ctt gct aag gct ggt gtg gtt     1455
Glu Ala Met Glu Gln Gln Ser Ile Ser Leu Ala Lys Ala Gly Val Val
    445                 450                 455

tgt agc ctt cct gca aga act tcc att att gct gct gca aat cca gtt     1503
Cys Ser Leu Pro Ala Arg Thr Ser Ile Ile Ala Ala Ala Asn Pro Val
460                 465                 470                 475

gga gga cat tac aat aaa gcc aaa aca gtt tct gag aat tta aaa atg     1551
Gly Gly His Tyr Asn Lys Ala Lys Thr Val Ser Glu Asn Leu Lys Met
                480                 485                 490

ggg agt gca cta cta tcc aga ttt gat ttg gtc ttt atc ctg tta gat     1599
Gly Ser Ala Leu Leu Ser Arg Phe Asp Leu Val Phe Ile Leu Leu Asp
            495                 500                 505

act cca aat gag cat cat gat cac tta ctc tct gaa cat gtg att gca     1647
Thr Pro Asn Glu His His Asp His Leu Leu Ser Glu His Val Ile Ala
        510                 515                 520

ata aga gct gga aag cag aga acc att agc agt gcc aca gta gct cgt     1695
Ile Arg Ala Gly Lys Gln Arg Thr Ile Ser Ser Ala Thr Val Ala Arg
    525                 530                 535

atg aat agt caa gat tca aat act tcc gta ctt gaa gta gtt tct gag     1743
Met Asn Ser Gln Asp Ser Asn Thr Ser Val Leu Glu Val Val Ser Glu
540                 545                 550                 555

aag cca tta tca gaa aga cta aag gtg gtt cct gga gaa aca ata gat     1791
Lys Pro Leu Ser Glu Arg Leu Lys Val Val Pro Gly Glu Thr Ile Asp
                560                 565                 570

ccc att ccc cac cag cta ttg aga aag tac att ggc tat gct cgg cag     1839
Pro Ile Pro His Gln Leu Leu Arg Lys Tyr Ile Gly Tyr Ala Arg Gln
            575                 580                 585

tat gtg tac cca agg cta tcc aca gaa gct gct cga gtt ctt caa gat     1887
Tyr Val Tyr Pro Arg Leu Ser Thr Glu Ala Ala Arg Val Leu Gln Asp
        590                 595                 600

ttt tac ctt gag ctc cgg aaa cag agc cag agg tta aat agc tca cca     1935
Phe Tyr Leu Glu Leu Arg Lys Gln Ser Gln Arg Leu Asn Ser Ser Pro
    605                 610                 615

atc act acc agg cag ctg gaa tct ttg att cgt ctg aca gag gca cga     1983
Ile Thr Thr Arg Gln Leu Glu Ser Leu Ile Arg Leu Thr Glu Ala Arg
620                 625                 630                 635

gca agg ttg gaa ttg aga gag gaa gca acc aaa gaa gac gct gag gat     2031
Ala Arg Leu Glu Leu Arg Glu Glu Ala Thr Lys Glu Asp Ala Glu Asp
                640                 645                 650

ata gtg gaa att atg aaa tat agc atg cta gga act tac tct gat gaa     2079
Ile Val Glu Ile Met Lys Tyr Ser Met Leu Gly Thr Tyr Ser Asp Glu

            655                 660                 665

ttt ggg aac cta gat ttt gag cga tcc cag cat ggt tct gga atg agc 2127
Phe Gly Asn Leu Asp Phe Glu Arg Ser Gln His Gly Ser Gly Met Ser
        670                 675                 680

aac agg tca aca gcg aaa aga ttt att tct gct ctc aac aac gtt gct 2175
Asn Arg Ser Thr Ala Lys Arg Phe Ile Ser Ala Leu Asn Asn Val Ala
    685                 690                 695

gaa aga act tat aat aat ata ttt caa ttt cat caa ctt cgg cag att 2223
Glu Arg Thr Tyr Asn Asn Ile Phe Gln Phe His Gln Leu Arg Gln Ile
700                 705                 710                 715

gcc aaa gaa cta aac att cag gtt gct gat ttt gaa aat ttt att gga 2271
Ala Lys Glu Leu Asn Ile Gln Val Ala Asp Phe Glu Asn Phe Ile Gly
                720                 725                 730

tca cta aat gac cag ggt tac ctc ttg aaa aaa ggc cca aaa gtt tac 2319
Ser Leu Asn Asp Gln Gly Tyr Leu Leu Lys Lys Gly Pro Lys Val Tyr
            735                 740                 745

cag ctt caa act atg taaaaggact tcaccaagtt agggcctcct gggtttattg 2374
Gln Leu Gln Thr Met
        750

cagattaaag ccatctcagt gaagatatgc gtgcacgcac agacagacag acacacacac 2434

acacacacac acacacacac acacacacac acacagtcaa atactgttct ctgaaaaatg 2494

atgtcccaaa agtattataa taggaaaaaa gcattaaata taataaacta atttaagaag 2554

tgataaagtc tccagatgca gtagctcaca ctgtaatcac agtgactcag gaggctgagg 2614

tgagaggatt ccttgaggcc agggttcgag accaaccttg ggcaacatag caagacccca 2674

tttcttaaaa aaaaaaaaa 2693

<210> 82
<211> 1141
<212> DNA
<213> Homo sapiens

<220>
<221> CDS
<222> (556)..(864)

<400> 82
taagcttgcg gccgcccttt tgagtacctc tgtccaggac tgaagacgaa ccttggccgc 60
agtccttgcg aactgttcgc aaaacagtgc gggctgcggt gggtcagcgt cacatgctaa 120
tgacaggatg ttcctcgtag ctttttattt tgtgcgtctt atgtttgtat agtgtgttgt 180
catttgattt tttttttttt tttaagtata aaagctgctt agaatagttc tatcatgaag 240
ggcacttttc agattgtggg ctggaaaggg ttattttatc tgtgggggag ggagggagac 300
gtgaagccta tttaaaatga gcctgtgacg attatgcaca tgaccagagg cggccggtaa 360
tcagggcaga gtctggtgtg gaccgccggg cgcagagcgg ctccgcggcg ggagctgagc 420
ggaaggcgcg ggctgcgatc tgctctctgt tcccctggat ccgcctatgc acattaccct 480
tgtttcatta ctttttcatg tcattttttt atcccaaaaa atatttttta aaagttaaaa 540
aaagacatat caaac atg caa ata ctt gaa tcc agc agg cca ata gtg aaa 591
tgt gaa cac ttt cta att cta cac ttc cag gtt gac atc agt aca cag 639
aaa cag gct cta gga aaa att tct aag ttc ata gcc tac gtg tgt gta 687
tgt gtg cgt gtt gaa aca gaa gtg tgt gtg tgt gtg cat gtg tgg cgc 735
gtg ctc ctg cac ggc gtg cag ttt tct gtg ttt gtg tgt ttg aaa gcg 783
tgt gag gat tta act gtg ggt ttt ccc ttg tat tca gta tac gct ttt 831
ttt ttg gcg cat tgg tca aac ggt tgt tat tat tagcttct aactttgcac 882
tgagtgtcct cgcccttcct ttagctaatc ctatacatta cagagaattc ctaggacagg 942
gctggcgaca attcatgtgt aaatgtttac tcaaggactc tgtgactgcg tttaaaaagt 1002
acagtgtata tctctggaga aataacatgt atacctacct ttagcagctt tttattgtgg 1062
actaatgcaa gaaaattatt accagagtat tgcaccattt ttccatttgg aatataaagt 1122
taaagagaaa aaaaaaaaa 1141

<210> 83
<211> 2197
<212> DNA
<213> Homo sapiens

<220>
<221> CDS
<222> (1263)..(1979)

<400> 83
   acctggcatg gttttaagtg atattaagag tattggctta tatttaagaa gtcaaaagat 60
   accactttat gaggaatgcc agcttttggt gagaaaagga tttgattttc agagaaaaca 120
   gtatggcaaa ctaaagaagt ttactactgt aaatcctgag ttttataatg aaccaaaaac 180
   caaactttat cttaagctaa gtcggaagga aagatcttca gcttatagca aaaatgatct 240
   ttgggtggtt tcaaaaaccc tagactttga gctggatact tttatcgcat gtagtgcttt 300
   ctttggacca tcatctatca atgagataga aatactgcct ttgaaaggct atttcccttc 360
   taattggccc actaacatgg ttgtccatgc gttattggtt tgtaatgcta gcacagaact 420
   gactactttg aaaaacattc aggactactt taatccagct actctacctc taacacagta 480
   cctgttaaca acgtcttcgc caactatagt tagtaacaaa agagtcagta agagaaaatt 540
   tatcccacca gccttcacaa atgtcagtac aaaatttgaa ctactcagcc taggagcaac 600
   attgaagtta gctagtgagt tgattcaggt acacaagtta aacaaggatc aagctacagc 660
   tctaattcaa atagctcaaa tgatggcatc acatgaaagc attgaagaag tgaaggaact 720
   gcaaactcat accttcccta tcacaatcat acatggtgtg tttggagcag gaaagagtta 780
   cttgctggca gtggtgattt tgttctttgt acagctgttt gaaaagagtg aagctcccac 840
   cattggaaat gcaaggccgt ggaaacttct gatttcttct tctactaatg tggctgttga 900
   cagagtactt cttgggcttc tcagtcttgg atttgaaaac tttatcagag ttgggagtgt 960
   taggaagatt gccaaaccaa ttttacctta tagcttgcat gctggctcaa aaaatgaaag 1020
   tgaacagtta aaagaactac atgcactaat gaaagaagac ctgactccta cggaaagagt 1080
   ctatgtgaga aaaagcattg agcagcataa actggggacc aatagaaccc tgctgaagca 1140
   ggttcgagtg tgaaaagctg attcttgttg gggatcccaa acagctacct cctactattc 1200
   agggttctga tgcagctcat gaaaatggat tggaacaaac tctttttgat cgactttgct 1260
   ta atg ggt cac aag cca att cta ttg aga act caa tac cgt tgt cat 1307
   Met Gly His Lys Pro Ile Leu Leu Arg Thr Gln Tyr Arg Cys His
   1 5 10 15
   cct gca atc agt gct att gct aat gat ctg ttt tac aaa gga gcc ctc 1355
   Pro Ala Ile Ser Ala Ile Ala Asn Asp Leu Phe Tyr Lys Gly Ala Leu
   20 25 30
   atg aat ggt gta aca gaa ata gag cgg agc cct tta ttg gaa tgg cta 1403
   Met Asn Gly Val Thr Glu Ile Glu Arg Ser Pro Leu Leu Glu Trp Leu
   35 40 45
   cca acc ctg tgt ttt tat aat gtt aaa gga cta gaa cag ata gaa aga 1451
   Pro Thr Leu Cys Phe Tyr Asn Val Lys Gly Leu Glu Gln Ile Glu Arg
   50 55 60
   gat aac agc ttt cat aat gtg gca gaa gct acg ttt aca ctc aag ctg 1499
   Asp Asn Ser Phe His Asn Val Ala Glu Ala Thr Phe Thr Leu Lys Leu
   65                  70                  75
   att caa tca ctg att gca agt gga ata gca ggc tct atg att ggt gtg      1547
   Ile Gln Ser Leu Ile Ala Ser Gly Ile Ala Gly Ser Met Ile Gly Val
    80                  85                  90                  95
   ata aca tta tac aaa tcc cag atg tac aag ctt tgt cat tta ctc agt      1595
   Ile Thr Leu Tyr Lys Ser Gln Met Tyr Lys Leu Cys His Leu Leu Ser
                   100                 105                 110
   gct gtg gac ttt cac cat cct gat att aaa act gtg cag gtg tcc aca      1643
   Ala Val Asp Phe His His Pro Asp Ile Lys Thr Val Gln Val Ser Thr
               115                 120                 125
   gta gat gct ttt cag gga gct gaa aag gag atc att att ctg tcc tgt      1691
   Val Asp Ala Phe Gln Gly Ala Glu Lys Glu Ile Ile Ile Leu Ser Cys
           130                 135                 140
   gta agg aca aga caa gta gga ttc att gat tca gaa aaa aga atg aat      1739
   Val Arg Thr Arg Gln Val Gly Phe Ile Asp Ser Glu Lys Arg Met Asn
       145                 150                 155
   gtt gca ttg act aga gga aag agg cat ttg ttg att gtg gga aat tta      1787
   Val Ala Leu Thr Arg Gly Lys Arg His Leu Leu Ile Val Gly Asn Leu
   160                 165                 170                 175
   gcc tgt ttg agg aaa aat caa ctt tgg gga cga gtg atc caa cac tgc      1835
   Ala Cys Leu Arg Lys Asn Gln Leu Trp Gly Arg Val Ile Gln His Cys
                   180                 185                 190
   gaa gga agg gaa gat gga ttg caa cat gca aac cag tat gaa cca cag      1883
   Glu Gly Arg Glu Asp Gly Leu Gln His Ala Asn Gln Tyr Glu Pro Gln
               195                 200                 205
   ctg aac cat ctc ctt aaa gat tat ttt gaa aaa caa gtg gaa gaa aaa      1931
   Leu Asn His Leu Leu Lys Asp Tyr Phe Glu Lys Gln Val Glu Glu Lys
           210                 215                 220
   cag aag aaa aag agt gaa aaa gag aaa tct aaa gat aaa tct cat tca      1979
   Gln Lys Lys Lys Ser Glu Lys Glu Lys Ser Lys Asp Lys Ser His Ser
       225                 230                 235
   taaaaagaca tggtgtaaat attttgtatt tatgtaaatt cagactcatt ttacatgata      2039
   tattttttat atttttatta ctctaaaccc tcttattaaa aatatgatat ttaaataaca      2099
   tagtaaacac atgtaaaaat tttgttcttc aaaaaagtgt acaaaaggta gtataaaatc      2159
   ctactaataa aaataagctt ttttctaaaa aaaaaaaa                              2197

<210> 84
<211> 796
<212> DNA
<213> Homo sapiens

<220>
<221> CDS

<222> (242)..(739)

<400> 84
gattagcgcc aacagctcag agaaaacgtg acgaaaacca gtctgtaaaa cccgagcctg      60

ggagaggggc ttcggtgcgc ggggggaatt tgcagacgct ccctgctggc ggagatttcc      120

tgacctgtcc ttcggcgcgg gactttcggc gggtcccggc cgggcagacc caagtgccgg      180

cggcggagac tgcagtggag ccagtaccgg ctgtagtggc cggggccgtg gcgggagagt      240

c atg tca gag ccg cag ccg cgg ggc gca gag cgc gat ctc tac cgg      286
  Met Ser Glu Pro Gln Pro Arg Gly Ala Glu Arg Asp Leu Tyr Arg
  1               5                   10                  15

gac acg tgg gtg cga tac ctg ggc tat gcc aat gag gtg ggc gag gct      334
Asp Thr Trp Val Arg Tyr Leu Gly Tyr Ala Asn Glu Val Gly Glu Ala
                20                  25                  30

ttc cgc tct ctt gtg cca gcg gcg gtg gtg tgg ctg agc tat ggc gtg      382
Phe Arg Ser Leu Val Pro Ala Ala Val Val Trp Leu Ser Tyr Gly Val
            35                  40                  45

gcc agc tcc tac gtg ctg gcg gat gcc att gac aaa ggc aag aag gct      430
Ala Ser Ser Tyr Val Leu Ala Asp Ala Ile Asp Lys Gly Lys Lys Ala
        50                  55                  60

gga gag gtg ccc agc cct gaa gca ggc cgc agc gcc agg gtg acc gtg      478
Gly Glu Val Pro Ser Pro Glu Ala Gly Arg Ser Ala Arg Val Thr Val
    65                  70                  75

gct gtg gtg gac acc ttt gta tgg cag gct cta gcc tct gtg gcc att      526
Ala Val Val Asp Thr Phe Val Trp Gln Ala Leu Ala Ser Val Ala Ile
80                  85                  90                  95

ccg ggc ttc acc atc aac cgc gtg tgt gct gcc tct ctc tat gtc ctg      574
Pro Gly Phe Thr Ile Asn Arg Val Cys Ala Ala Ser Leu Tyr Val Leu
                100                 105                 110

ggc act gcc acc cgc tgg ccc ctg gct gtc cgc aag tgg acc acc acc      622
Gly Thr Ala Thr Arg Trp Pro Leu Ala Val Arg Lys Trp Thr Thr Thr
            115                 120                 125

gcg ctt ggg ctg ttg acc atc ccc atc att atc cac ccc att gac agg      670
Ala Leu Gly Leu Leu Thr Ile Pro Ile Ile Ile His Pro Ile Asp Arg
        130                 135                 140

tcg gtg gat ttc ctc ctg gac tcc agc ctg cgc aag ctc tac cca aca      718
Ser Val Asp Phe Leu Leu Asp Ser Ser Leu Arg Lys Leu Tyr Pro Thr
    145                 150                 155

gtg ggg aag ccc agc tcc tcc tg atcatactct ggtacctggc ctgtgcatcg      771
Val Gly Lys Pro Ser Ser Ser
160                 165

gcctcctgct tcatgtcaac ctcct                                          796

<210> 85
<211> 1872
<212> DNA
<213> Homo sapiens

<220>
<221> CDS
<222> (44)..(1063)

<400> 85
acgcgtccgg gaacttggag cagccagaag aagtcagtat tac atg aaa tat ggg 55
Met Lys Tyr Gly
1

aat cca aat tat gga ggc atg aaa gga att ctt agc aat tca tgg aag 103
Asn Pro Asn Tyr Gly Gly Met Lys Gly Ile Leu Ser Asn Ser Trp Lys
5                   10                  15                  20

cga aga tat cat tcc cgt cgt att cag cgg gac gtg atc aag aag aga 151
Arg Arg Tyr His Ser Arg Arg Ile Gln Arg Asp Val Ile Lys Lys Arg
                25                  30                  35

gcc ctg att ggg gat gac gtt ggc ttg acg tcg tat aaa cat cga cat 199
Ala Leu Ile Gly Asp Asp Val Gly Leu Thr Ser Tyr Lys His Arg His
            40                  45                  50

tct ggg cta gtg aat gtt ccc gag gaa ccc att gaa gag gag gaa gag 247
Ser Gly Leu Val Asn Val Pro Glu Glu Pro Ile Glu Glu Glu Glu Glu
        55                  60                  65

gag gag gag gag gaa gag gaa gag gaa gaa gaa gac cag gac atg gat 295
Glu Glu Glu Glu Glu Glu Glu Glu Glu Glu Glu Asp Gln Asp Met Asp
    70                  75                  80

gca gat gac aga gtg gtg gta gag tac cac gag gag ctc ccg gct ctc 343
Ala Asp Asp Arg Val Val Val Glu Tyr His Glu Glu Leu Pro Ala Leu
85                  90                  95                  100

aag cag ccc cgg gag cgg agc gcg tct aga cga tcc agt gcc agc agc 391
Lys Gln Pro Arg Glu Arg Ser Ala Ser Arg Arg Ser Ser Ala Ser Ser
                105                 110                 115

tca gac tca gat gaa atg gac tat gat cta gaa ctg aaa atg att tcc 439
Ser Asp Ser Asp Glu Met Asp Tyr Asp Leu Glu Leu Lys Met Ile Ser
            120                 125                 130

acg cct tca cca aag aaa agc atg aaa atg act atg tat gct gac gaa 487
Thr Pro Ser Pro Lys Lys Ser Met Lys Met Thr Met Tyr Ala Asp Glu
        135                 140                 145

gtg gaa tct cag ttg aaa aat att agg aac tcc atg agg gca gat agt 535
Val Glu Ser Gln Leu Lys Asn Ile Arg Asn Ser Met Arg Ala Asp Ser
    150                 155                 160

gta tct tca agc aat atc aaa aac cga att ggt aac aaa tta cca cct 583
Val Ser Ser Ser Asn Ile Lys Asn Arg Ile Gly Asn Lys Leu Pro Pro
165                 170                 175                 180

gag aaa ttt gca gat gtc cga cat cta tta gat gag aaa cgt cag cac 631
Glu Lys Phe Ala Asp Val Arg His Leu Leu Asp Glu Lys Arg Gln His

                185                 190                 195

tcc cgt cca cgg cca cca gtc agc agt act aaa tca gat ata cgc cag 679
Ser Arg Pro Arg Pro Pro Val Ser Ser Thr Lys Ser Asp Ile Arg Gln
            200                 205                 210

cgg tta gga aaa aga cca cat tct ccg gaa aag gct ttt agt agt aac 727
Arg Leu Gly Lys Arg Pro His Ser Pro Glu Lys Ala Phe Ser Ser Asn
        215                 220                 225

ccc gtc gtt cgg aga gag ccc tct tct gat gtg cat agt agg cta ggt 775
Pro Val Val Arg Arg Glu Pro Ser Ser Asp Val His Ser Arg Leu Gly
    230                 235                 240

gtt ccc agg cag gat agt aaa ggc ctc tac gcc gat act cgg gag aag 823
Val Pro Arg Gln Asp Ser Lys Gly Leu Tyr Ala Asp Thr Arg Glu Lys
245                 250                 255                 260

aaa tca ggt aat tta tgg act cgc cta gga tct gca ccc aag acc aaa 871
Lys Ser Gly Asn Leu Trp Thr Arg Leu Gly Ser Ala Pro Lys Thr Lys
                265                 270                 275

gaa aag aat acg aag aaa gtg gat cac agg gcg cct ggc gct gag gaa 919
Glu Lys Asn Thr Lys Lys Val Asp His Arg Ala Pro Gly Ala Glu Glu
            280                 285                 290

gac gac tct gag ctg caa agg gca tgg ggg gct ctg att aag gag aaa 967
Asp Asp Ser Glu Leu Gln Arg Ala Trp Gly Ala Leu Ile Lys Glu Lys
        295                 300                 305

gag cag tct cgc caa aag aag agc cgg tta gat aac tta cca tct ctc 1015
Glu Gln Ser Arg Gln Lys Lys Ser Arg Leu Asp Asn Leu Pro Ser Leu
    310                 315                 320

cag att gaa gtt agt cgg gaa agc agc tct ggt tca gag gca gag tcc 1063
Gln Ile Glu Val Ser Arg Glu Ser Ser Ser Gly Ser Glu Ala Glu Ser
325                 330                 335                 340

tgatgcccct ggggcctatg gcagctgccc taaagcctga cattcttgca cagggtgggg 1123

cgcgcagtag gaacctcccc cgcaggagct ggcgccctct cgctcctgct cacacagtca 1183

ccctccgctc ttgctacttc ggcaagacat cttaagaatg tgacacattt tcaagggcat 1243

ctctatcttt cgctgcagag ttgtagttct gggccctcga ttcctttccc accaccccac 1303

aggctttacc ctttgagaaa aaggcagccc tccagatttt gtagacattt ttcttaatat 1363

ttttaacatt gtgtctttta aaagaaatgt tttacacagt tcatccaaag agcagagaac 1423

tgaacttctc actattgcct tggccctgac agctgttacc agcacccttt tcccaagaaa 1483

agtcaattcc caggtgctta ttggacatcc ttcgaggggg aagaggaggg aagcggccag 1543

ctcacccttc cgggacccta gtgtggggcg aatctcacgg acctgacctc agaggtgcac 1603

cagtgccgcc caggtagata agagctgcag cattgactgt gcttccgttc ttccctcggg 1663

gattgctcat ggcatgggcc tcttacgtgc tgtcagcttg atgtgaagat caagttcagt 1723

gctgtgggat ttttaggaac aaaaaactgt gacgtctgga tttggggttg atttttgtgc 1783

tggggtggga ttatgggtca atgttgaaga atttttaagt ctgtattatg tttaaaacat 1843

gaatgatttg aaagtttaaa aaaaaaaaa 1872

<210> 86
<211> 1819
<212> DNA
<213> Homo sapiens

<220>
<221> CDS
<222> (984)..(1406)

<400> 86
gcacgagccc ttcctcaccc cgtcccctga gtcccctgac cagtggtcca gctcgtcccc 60

gcattccaac gtctccgact ggtccgaggg cgtctccagc cctcccacca gcatgcagtc 120

ccagatcgcc cgcattccgg aggccttcaa gtaaacggcg cgccccacga gaccccggct 180

tcctttccca agccttcggg cgtctgtgtg cgctctgtgg atgccagggc cgaccagagg 240

agccttttta aaacacatgt ttttatacaa aataagaaca aggattttaa ttttttttag 300

tatttattta tgtactttta ttttacacag aaacactgcc tttttattta tatgtactgt 360

tttatctggc cccaggtaga aacttttatc tattctgaga aaacaagcaa gttctgagag 420

ccagggtttt cctacgtagg atgaaaagat tcttctgtgt ttataaaata taaacaaaga 480

ttcatgattt ataaatgcca tttatttatt gattcctttt ttcaaaatcc aaaaagaaat 540

gatgttggag aagggaagtt gaacgagcat agtccaaaaa gctcctgggg cgtccaggcc 600

gcgccctttc cccgacgccc acccaacccc aagccagccc ggccgctcca ccagcatcac 660

ctgcctgtta ggagaagctg catccagagg caaacggagg caaagctggc tcaccttccg 720

cacgcggatt aatttgcatc tgaaatagga aacaagtgaa agcatatggg ttagatgttg 780

ccatgtgttt tagatggttt cttgcaagca tgcttgtgaa aatgtgttct cggagtgtgt 840

atgccaagag tgcacccatg gtaccaatca tgaatctttg tttcaggttc agtattatgt 900

agttgttcgt tggttataca agttcttggt ccctccagaa ccaccccggc cccctgcccg 960

ttcttgaaat gtaggcatca tgc atg tca aac atg aga tgt gtg gac tgt 1010
Met Ser Asn Met Arg Cys Val Asp Cys
1               5

ggc act tgc ctg ggt cac aca cgg agg cat cct acc ctt ttc tgg gga 1058
Gly Thr Cys Leu Gly His Thr Arg Arg His Pro Thr Leu Phe Trp Gly
10                  15                  20                  25

aag aca ctg cct ggg ctg acc ccg gtg gcg gcc cca gca cct cag cct 1106

Lys Thr Leu Pro Gly Leu Thr Pro Val Ala Ala Pro Ala Pro Gln Pro
                30                  35                  40

gca cag tgt ccc cca ggt tcc gaa gaa gat gct cca gca aca cag cct      1154
Ala Gln Cys Pro Pro Gly Ser Glu Glu Asp Ala Pro Ala Thr Gln Pro
            45                  50                  55

ggg ccc cag ctc gcg gga ccc gac ccc ccg tgg gct ccc gtg ttt tgt      1202
Gly Pro Gln Leu Ala Gly Pro Asp Pro Pro Trp Ala Pro Val Phe Cys
        60                  65                  70

agg aga ctt gcc aga gcc ggg cac att gag ctg tgc aac gcc gtg ggc      1250
Arg Arg Leu Ala Arg Ala Gly His Ile Glu Leu Cys Asn Ala Val Gly
    75                  80                  85

tgc gtc ctt tgg tcc tgt ccc cgc agc cct ggc agg ggg cat gcg gtc      1298
Cys Val Leu Trp Ser Cys Pro Arg Ser Pro Gly Arg Gly His Ala Val
90                  95                  100                 105

ggg cag ggg ctg gag gga ggc ggg ggc tgc cct tgg gcc acc cct cct      1346
Gly Gln Gly Leu Glu Gly Gly Gly Gly Cys Pro Trp Ala Thr Pro Pro
                110                 115                 120

agt ttg gga gga gca gat ttt tgc aat acc aag tat agc cta tgg cag      1394
Ser Leu Gly Gly Ala Asp Phe Cys Asn Thr Lys Tyr Ser Leu Trp Gln
            125                 130                 135

aaa aaa tgt ctg taa atatgttttt aaaggtggat tttgtttaaa aaatcttaat      1449
Lys Lys Cys Leu
        140

gaatgagtct gttgtgtgtc atgccagtga gggacgtcag acttggctca gctcggggag   1509

ccttagccgc ccatgcactg gggacgctcc gctgccgtgc cgcctgcact cctcagggca   1569

gcctcccccg gctctacggg ggccgcgtgg tgccatcccc agggggcatg accagatgcg   1629

tcccaagatg ttgattttta ctgtgtttta taaaatagag tgtagtttac agaaaaagac   1689

tttaaaagtg atctacatga ggaactgtag atgatgtatt tttttcatct tttttgttaa   1749

ctgatttgca ataaaaatga tactgatggt gaaaaaaaaa aaaaaaaaaa cctatgcggc   1809

cgcaagctta                                                          1819

<210> 87
<211> 4070
<212> DNA
<213> Homo sapiens

<220>
<221> CDS
<222> (12)..(1997)

<400> 87
tgagtgatgc t atg gag gag atc gac atg caa caa ggc acc tcg tca gta      50
             Met Glu Glu Ile Asp Met Gln Gln Gly Thr Ser Ser Val

1                   5                   10

aaa cca cag gct aat ggt gtt ttg gat gaa aaa tct caa att cag gag          98
Lys Pro Gln Ala Asn Gly Val Leu Asp Glu Lys Ser Gln Ile Gln Glu
     15                  20                  25

cca tgt tgt tca gac ctc ttc ctg ttt cct gac gag agt ggg aat gta         146
Pro Cys Cys Ser Asp Leu Phe Leu Phe Pro Asp Glu Ser Gly Asn Val
 30                  35                  40                  45

tcc cag gag tcc ggc ccc acc tat gcc tca ttc tct cac cat ttc atc         194
Ser Gln Glu Ser Gly Pro Thr Tyr Ala Ser Phe Ser His His Phe Ile
                 50                  55                  60

agt gat gca atg aca ggt gtg ccc act gag aat gat gac ttt tgc att         242
Ser Asp Ala Met Thr Gly Val Pro Thr Glu Asn Asp Asp Phe Cys Ile
             65                  70                  75

ctt ttt gca cca aaa gca gcc atg cag gag aag gaa gaa gaa cca gtt         290
Leu Phe Ala Pro Lys Ala Ala Met Gln Glu Lys Glu Glu Glu Pro Val
         80                  85                  90

ata aaa atc atg gtt gat gat gca att gtg ata aga gac aat tat ttc         338
Ile Lys Ile Met Val Asp Asp Ala Ile Val Ile Arg Asp Asn Tyr Phe
     95                 100                 105

agt ctg ccc gtt aat aag acc gat acg agc aaa gcc ccc tta cac ttt         386
Ser Leu Pro Val Asn Lys Thr Asp Thr Ser Lys Ala Pro Leu His Phe
110                 115                 120                 125

ccc att cct gtg att cgc tat gtg gtg aag gag gtc tct ctt gtc tgg         434
Pro Ile Pro Val Ile Arg Tyr Val Val Lys Glu Val Ser Leu Val Trp
                130                 135                 140

cat ctt tat gga gga aag gat ttt gga aca gtc cct ccc act tct ccg         482
His Leu Tyr Gly Gly Lys Asp Phe Gly Thr Val Pro Pro Thr Ser Pro
            145                 150                 155

gct aaa agt tat att agt ccc cac agt tcg cct tct cac aca ccc acg         530
Ala Lys Ser Tyr Ile Ser Pro His Ser Ser Pro Ser His Thr Pro Thr
        160                 165                 170

aga cat gga cgt aat aca gta tgt ggg gga aaa gga agg aac cat gac         578
Arg His Gly Arg Asn Thr Val Cys Gly Gly Lys Gly Arg Asn His Asp
    175                 180                 185

ttt tta atg gaa ata cag cta agc aag gtg aag ttt cag cat gaa gtc         626
Phe Leu Met Glu Ile Gln Leu Ser Lys Val Lys Phe Gln His Glu Val
190                 195                 200                 205

tac ccg cca tgc aaa cct gat tgt gat tcc agc ctc tca gaa cac cca         674
Tyr Pro Pro Cys Lys Pro Asp Cys Asp Ser Ser Leu Ser Glu His Pro
                210                 215                 220

gtc tcc cgg cag gtg ttc att gtt cag gat ctt gag att cga gat cgt         722
Val Ser Arg Gln Val Phe Ile Val Gln Asp Leu Glu Ile Arg Asp Arg
            225                 230                 235

ttg gca aca tca caa atg aat aaa ttt tta tac ctg tat tgc agt aaa         770
Leu Ala Thr Ser Gln Met Asn Lys Phe Leu Tyr Leu Tyr Cys Ser Lys

240                 245                 250

gaa atg cct cga aaa gct cac tcc aac atg ttg aca gtg aaa gcc tta        818
Glu Met Pro Arg Lys Ala His Ser Asn Met Leu Thr Val Lys Ala Leu
    255                 260                 265

cac gtg tgt cca gaa tct ggc agg tcc cca cag gag tgc tgc ttg aga        866
His Val Cys Pro Glu Ser Gly Arg Ser Pro Gln Glu Cys Cys Leu Arg
270                 275                 280                 285

gtg tcg ctg atg ccg ctc cgc ctc aat att gac cag gat gct ttg ttc        914
Val Ser Leu Met Pro Leu Arg Leu Asn Ile Asp Gln Asp Ala Leu Phe
                290                 295                 300

ttc ctg aag gat ttc ttc aca agt ctt tct gca gaa gta gag ctt caa        962
Phe Leu Lys Asp Phe Phe Thr Ser Leu Ser Ala Glu Val Glu Leu Gln
            305                 310                 315

atg act cca gat cca gaa gtt aaa aag tct cct gga gct gat gtc acc       1010
Met Thr Pro Asp Pro Glu Val Lys Lys Ser Pro Gly Ala Asp Val Thr
        320                 325                 330

tgc agt ttg cca agg cat ttg agt acc tca aag gag cca aat ctg gtt       1058
Cys Ser Leu Pro Arg His Leu Ser Thr Ser Lys Glu Pro Asn Leu Val
    335                 340                 345

att tct ttc tct ggg cca aaa cag cct tcc caa aat gat agt gcc aat       1106
Ile Ser Phe Ser Gly Pro Lys Gln Pro Ser Gln Asn Asp Ser Ala Asn
350                 355                 360                 365

tca gtg gaa gtg gtt aat ggc atg gaa gag aag aac ttc tct gct gaa       1154
Ser Val Glu Val Val Asn Gly Met Glu Glu Lys Asn Phe Ser Ala Glu
                370                 375                 380

gaa gca tct ttt agg gat cag cct gtg ttt ttt aga gaa ttt aga ttc       1202
Glu Ala Ser Phe Arg Asp Gln Pro Val Phe Phe Arg Glu Phe Arg Phe
            385                 390                 395

acg tca gaa gtt ccc att cga ctt gat tat cat ggc aaa cat gta tca       1250
Thr Ser Glu Val Pro Ile Arg Leu Asp Tyr His Gly Lys His Val Ser
        400                 405                 410

atg gat cag ggt acg cta gct ggg att ttg att ggt ctg gct cag tta       1298
Met Asp Gln Gly Thr Leu Ala Gly Ile Leu Ile Gly Leu Ala Gln Leu
    415                 420                 425

aac tgc tct gaa cta aag ctc aag agg ctt tcc tat cga cat ggt tta       1346
Asn Cys Ser Glu Leu Lys Leu Lys Arg Leu Ser Tyr Arg His Gly Leu
430                 435                 440                 445

cta ggc gtt gac aaa tta ttc tca tat gca atc act gag tgg ctt aat       1394
Leu Gly Val Asp Lys Leu Phe Ser Tyr Ala Ile Thr Glu Trp Leu Asn
                450                 455                 460

gac att aag aag aac cag cta cca gga atc ctg gga ggt gtt gga cct       1442
Asp Ile Lys Lys Asn Gln Leu Pro Gly Ile Leu Gly Gly Val Gly Pro
            465                 470                 475

atg cat tca cta gta caa tta gta caa ggc cta aag gac ttg gtc tgg       1490
Met His Ser Leu Val Gln Leu Val Gln Gly Leu Lys Asp Leu Val Trp

480                    485                    490

ctc cca ata gag cag tac cgg aag gat ggc cgc att gtc aga ggg ttt     1538
Leu Pro Ile Glu Gln Tyr Arg Lys Asp Gly Arg Ile Val Arg Gly Phe
    495                 500                 505

cag aga ggc gct gct tcc ttt ggt acc tcg aca gcg atg gct gct cta     1586
Gln Arg Gly Ala Ala Ser Phe Gly Thr Ser Thr Ala Met Ala Ala Leu
510                 515                 520                 525

gaa ctc aca aac aga atg gtt caa acc ata cag gca gct gca gag act     1634
Glu Leu Thr Asn Arg Met Val Gln Thr Ile Gln Ala Ala Ala Glu Thr
                530                 535                 540

gct tat gat atg gtg tct cct ggt acc ctt tct atc gag ccc aag aag     1682
Ala Tyr Asp Met Val Ser Pro Gly Thr Leu Ser Ile Glu Pro Lys Lys
            545                 550                 555

acc aaa agg ttt cct cat cac cgg tta gcc cac cag cca gta gac ctg     1730
Thr Lys Arg Phe Pro His His Arg Leu Ala His Gln Pro Val Asp Leu
        560                 565                 570

agg gaa ggt gtg gcc aag gcc tac agt gtt gtg aaa gag gga atc aca     1778
Arg Glu Gly Val Ala Lys Ala Tyr Ser Val Val Lys Glu Gly Ile Thr
    575                 580                 585

gac acg gct cag acc att tat gaa act gcg gct cga gaa cac gag agc     1826
Asp Thr Ala Gln Thr Ile Tyr Glu Thr Ala Ala Arg Glu His Glu Ser
590                 595                 600                 605

aga ggg gtg act ggt gcc gtg ggc gag gtt ctg cgc cag att cct ccg     1874
Arg Gly Val Thr Gly Ala Val Gly Glu Val Leu Arg Gln Ile Pro Pro
                610                 615                 620

gca gtg gtg aaa cct ctg att gtt gcc aca gaa gca acg tca aac gtg     1922
Ala Val Val Lys Pro Leu Ile Val Ala Thr Glu Ala Thr Ser Asn Val
            625                 630                 635

ctg ggt ggc atg aga aac caa att agg cca gat gtc cgg caa gac gag     1970
Leu Gly Gly Met Arg Asn Gln Ile Arg Pro Asp Val Arg Gln Asp Glu
        640                 645                 650

tca cag aaa tgg cgc cac ggg gat gac tgatg gcttggaact gacagtgtga     2022
Ser Gln Lys Trp Arg His Gly Asp Asp
    655                 660

agataaggag agtggaacca ggagctcaga gtcctgacag cagcttcaga ggaagctcgt     2082

ttaattttat tgtgctcatc tcaggaacaa aagcattttt tagttaaata atttaacatc     2142

aaaacaacat gcaaccaaaa acttctgaca tttatagttg atacttgcct atagaaatgt     2202

ttggtggctg gtgtcaaagg tccttaaagc atttgctgcc aagttagtgg aaggctcact     2262

tttgttaaga tgactgtaat tctccttgtt accgacgaga gatcattgga agctgccttc     2322

taacactttg tgtagctctg tggagttgga ttttcttaag gtttaaaaag aatcacagct     2382

tcggaacttt taactgaaaa tgagagacag aagccacagg ggaagcaaag caaataggat     2442

tttcaatata aatatcagtg tggaaaaata acctattctg ttgaatttag tgttcatgca 2502
cttgagaaca acattatttc catttactcc gaaaatcctt ctgtgggggt ttgagaaagt 2562
gaatgttgca gacatgttct gttgtgttgc actttatcct gtgtttatgt gtatgtgttt 2622
ttagattaat tcaagttgtg tgctatattt cttgtataat ttacaaagtt acacaaaata 2682
taaagagcag taaacttgtc tgaaagtttt tggcaaagga aggtaacttc aatgtaatag 2742
cttcctttaa gagtacagga aaatgcattc tgtaatgaag tggggcccat gtaattgttt 2802
atattttcag ttttaagcag gtatagtgca ggcttgttag gaatgtgtgg aagggaagat 2862
tggaagtgat ttttcctctt ttaaaagtaa acaaaattct tcaaatatgc cctagttaac 2922
tatttcagca taccattttt acttggttaa cagtgtacat tttgataacc tatcaggaat 2982
gaataaagta tttttattta aaggtgatat tgttttatgc ttccatgaat attttgcttt 3042
tcttagagca tattcataga tgcagtactg tgatttaccg caagataatt gggaacactt 3102
tctcaaaatc gattttggtt aatatcaggt aaatcattac ctaggagtaa gagggaaatt 3162
gttgtcaatt cctgacttta gattgatcca gcagaaagaa aaggtggaac tttgtatata 3222
tccactgttg aaagttgcat gacctgactt cctgttttct aaagttgggg gaatattatt 3282
gaacttcaat ttcagaactg catttcaatt tggggcttgg aaaatgtagt taaatcaatt 3342
tcgttctcta aattttctaa gcaggaaagc aatgtaaacc aaaaatcaaa atgtttagaa 3402
tttataaaaa tggggttata tagcttttaa ttttagtttc ctttttactg ccaaaaaact 3462
taaaaagaca acagcgtaac cacctgttta agcaccgtgg gaccatctct tattcgggta 3522
gagcctggtc ctggcgacac actaacagga aggcgtgctg tgttaccggg aaggcgcctc 3582
tggctgtggg gtgcgttctg accaccccgc tcagatgcca gcattttcca tttgccctta 3642
aggaaacttg ctatgtgtgt taacccagtt atgattagtc aaactggaaa acacttgatc 3702
aaacttaaaa ttgtaaaatc tgtctaactt ttaaatatac tttccacagt tgtacttaag 3762
ttcaattaac tacagtatag agaaaaataa ttattctctt ctttgcaccc ggagttgcaa 3822
acaaattaag tttacaaatc tcatttaaac atatatcttt gtaatgtaat ctccgcggtt 3882
ctttctttgt tctaccctcc tgcacctgtt gtcgatgtaa tcattttggg aacagaaacg 3942
ttgtgtctca aaaagattcg ttgtcagttc agccaagatg ttctttacct gagattctgg 4002
aaagatgttt atctatatcg gaacacttct acattattaa atgtccttaa gctttaaaaa 4062
aaaaaaaa 4070

<210> 88

<211> 2734
<212> DNA
<213> Homo sapiens

<220>
<221> CDS
<222> (576)..(1919)

<400> 88
cgtctcaccc cagctccttt ggctccgcac cccacccaca cctgctgccc accaccccgg 60

cagcaccttt ccctgcccag gcttcagagt gccctgttgc tgctgccact gccccccaca 120

ctccagggcc atgtcagagc tcccatctac cctccaccag catgccgctc ctgaagatgc 180

ccccaccatt ctcggggtgc agccacccct gcagcgggca ctgtggtggg cactgcagtg 240

ggcctctcct cccacccccg agctctcagc cactccctag cactcacagg gatcccgggt 300

gcaaggggca caagtttgca cacagtggcc tggcttgcca gctgccccag ccctgcgagg 360

cagatgaggg gctgggtgag gaagaggata gcagctctga gcgaagctcc tgcacctcat 420

cctccaccca ccagagagat gggaagttct gtgactgctg ctactgtgag ttcttcggcc 480

acaatgcgcc acccgctgcc ccgacgagtc ggaactatac cgagatccgg gagaagctcc 540

gctcgaggct gaccaggcgg aaagaggagc tgccc atg aag ggg ggc acc ctg 593
Met Lys Gly Gly Thr Leu
1               5

ggc ggg atc cct ggg gag ccc gcc gtg gac cac cga gat gtg gat gag 641
Gly Gly Ile Pro Gly Glu Pro Ala Val Asp His Arg Asp Val Asp Glu
            10                  15                  20

ctg ctg gaa ttc atc aac agc acg gag ccc aaa gtc ccc aac agc gcc 689
Leu Leu Glu Phe Ile Asn Ser Thr Glu Pro Lys Val Pro Asn Ser Ala
        25                  30                  35

agg gcc gcc aag cgg gcc cgg cac aag ctg aaa aag aag gaa aag gag 737
Arg Ala Ala Lys Arg Ala Arg His Lys Leu Lys Lys Lys Glu Lys Glu
    40                  45                  50

aag gcc cag ttg gca gca gaa gct cta aag cag gca aat cgt gtt tct 785
Lys Ala Gln Leu Ala Ala Glu Ala Leu Lys Gln Ala Asn Arg Val Ser
55                  60                  65                  70

gga agc cgg gag cca agg cct gcc agg gag agg ctc ttg gag tgg ccc 833
Gly Ser Arg Glu Pro Arg Pro Ala Arg Glu Arg Leu Leu Glu Trp Pro
                75                  80                  85

gac cgg gaa ctg gat cgg gtc aac agc ttc ctg agc agc cgt ctg cag 881
Asp Arg Glu Leu Asp Arg Val Asn Ser Phe Leu Ser Ser Arg Leu Gln
            90                  95                  100

gag atc aaa aac act gtc aaa gac tcc atc cgt gcc agc ttc agt gtg 929
Glu Ile Lys Asn Thr Val Lys Asp Ser Ile Arg Ala Ser Phe Ser Val
        105                 110                 115

tgt gag ctc agc atg gac agc aat ggc ttc tct aag gag ggg gct gct 977

Cys Glu Leu Ser Met Asp Ser Asn Gly Phe Ser Lys Glu Gly Ala Ala
    120                 125                 130

gag cct gag cct cag agt cta ccc ccc tca aac ctc agt ggc tcc tca 1025
Glu Pro Glu Pro Gln Ser Leu Pro Pro Ser Asn Leu Ser Gly Ser Ser
135                 140                 145                 150

gag cag cag cct gac atc aac ctt gac ctg tcc cct ttg act ttg ggc 1073
Glu Gln Gln Pro Asp Ile Asn Leu Asp Leu Ser Pro Leu Thr Leu Gly
                155                 160                 165

tcc cct cag aac cac acg tta caa gct cca ggc gag cca gcc cca cca 1121
Ser Pro Gln Asn His Thr Leu Gln Ala Pro Gly Glu Pro Ala Pro Pro
            170                 175                 180

tgg gca gaa atg aga ggc ccc cac cca cca tgg aca gag gtg agg ggg 1169
Trp Ala Glu Met Arg Gly Pro His Pro Pro Trp Thr Glu Val Arg Gly
        185                 190                 195

ccc cct ccc ggt atc gtc ccc gag aac ggg ctc gtg agg aga ctc aac 1217
Pro Pro Pro Gly Ile Val Pro Glu Asn Gly Leu Val Arg Arg Leu Asn
    200                 205                 210

acc gtg ccc aac cta tcc cgg gtg atc tgg gtc aag aca ccc aag ccg 1265
Thr Val Pro Asn Leu Ser Arg Val Ile Trp Val Lys Thr Pro Lys Pro
215                 220                 225                 230

ggc tac ccc agc tcc gag gag cca agc tca aag gaa gtt ccc agt tgc 1313
Gly Tyr Pro Ser Ser Glu Glu Pro Ser Ser Lys Glu Val Pro Ser Cys
                235                 240                 245

aag cag gag ctg cct gag cct gtg tcc tca ggt ggg aag cca cag aag 1361
Lys Gln Glu Leu Pro Glu Pro Val Ser Ser Gly Gly Lys Pro Gln Lys
            250                 255                 260

ggc aag agg cag ggc agt cag gcc aag aag agc gag gca agc cca gcc 1409
Gly Lys Arg Gln Gly Ser Gln Ala Lys Lys Ser Glu Ala Ser Pro Ala
        265                 270                 275

ccc cgg ccc cca gcc agc cta gag gtt ccc agt gcc aag ggc cag gtc 1457
Pro Arg Pro Pro Ala Ser Leu Glu Val Pro Ser Ala Lys Gly Gln Val
    280                 285                 290

gct ggc ccc aag cag cca ggc agg gtc cta gag ctt ccc aaa gta ggc 1505
Ala Gly Pro Lys Gln Pro Gly Arg Val Leu Glu Leu Pro Lys Val Gly
295                 300                 305                 310

agc tgt gct gag gct gga gag ggg agc cgg ggg agc cgg cca gga cca 1553
Ser Cys Ala Glu Ala Gly Glu Gly Ser Arg Gly Ser Arg Pro Gly Pro
                315                 320                 325

ggt tgg gct ggc agt ccc aaa act gag aag gag aag ggc agc tcc tgg 1601
Gly Trp Ala Gly Ser Pro Lys Thr Glu Lys Glu Lys Gly Ser Ser Trp
            330                 335                 340

cga aac tgg cca ggc gag gcc aag gca cgg cct cag gag cag gag tct 1649
Arg Asn Trp Pro Gly Glu Ala Lys Ala Arg Pro Gln Glu Gln Glu Ser
        345                 350                 355

gtg cag ccc cca ggc cca gca agg cca cag agc ttg ccc cag ggc aag 1697

Val Gln Pro Pro Gly Pro Ala Arg Pro Gln Ser Leu Pro Gln Gly Lys
    360                 365                 370

ggc cgc agc cgc cgg agc cgc aac aag cag gag aag cca gcc tcc tcc      1745
Gly Arg Ser Arg Arg Ser Arg Asn Lys Gln Glu Lys Pro Ala Ser Ser
375                 380                 385                 390

ttg gac gat gtg ttc ctg ccc aag gac atg gac ggg gtg gag atg gat      1793
Leu Asp Asp Val Phe Leu Pro Lys Asp Met Asp Gly Val Glu Met Asp
                395                 400                 405

gag act gac cga gag gtg gag tac ttt aag agg ttc tgt ttg gat tct      1841
Glu Thr Asp Arg Glu Val Glu Tyr Phe Lys Arg Phe Cys Leu Asp Ser
            410                 415                 420

gca aag cag act cgt cag aaa gtt gct gtg aac tgg acc aac ttc agc      1889
Ala Lys Gln Thr Arg Gln Lys Val Ala Val Asn Trp Thr Asn Phe Ser
        425                 430                 435

ctc aag aaa acc act cct agc aca gct cag t gaggccctgc ccaggctgag      1940
Leu Lys Lys Thr Thr Pro Ser Thr Ala Gln
    440                 445

ctgcttcagg gcatcctgag gccctgactg ccagctgaag gcgtataatt tttccctccg   2000

tgtgccccac ctacccgtcc aaaactgaga aggagaaggg cagctcctgg cgaaactggc   2060

caggcgaggc caaggcacgg cctcaggagc aggagtctgt gcagccccca ggcccagcaa   2120

ggccacagag cttgccccag ggccagggcc gcagccgccg gagccgcaac aagcaggaga   2180

agccagcctc ctccttggac gatgtgttcc tgcccaagga catggacggg gtggagatgg   2240

atgagactga ccgagaggca gggcagtcag gccaagaaga gctggcccaa cactgctctc   2300

tttgtgtttg gtttttttgt ttttgttttt attttgtttt tttccaattc tttacttttg   2360

atactgtgaa gatctttcgt gccgaaagat aaagcaacat ttggacacag agttggcatg   2420

ttggtgattt gtgggtctgg gcggggaggg agttgagggt gtgctcagaa gcatgggctc   2480

tcccttagtc actaacccta gcatgcacca tgcatcagca gtttggtgct attgtcccat   2540

tttacaggtg aggaaactaa agccgatagg aaagtgactc agctcaactt agtctggctg   2600

attagcattg agcgcagact ggtatgatta tggcatctgt gcccttttct ccttttggga   2660

ggtctctggt atgtgagcct ggggcagccc cttccccact ctttcctgtt tccctctttg   2720

caagatgggt gagt                                                      2734

<210> 89
<211> 3813
<212> DNA
<213> Homo sapiens

<220>

<221> CDS
<222> (183)..(2492)

<400> 89
agagtaaagt gcagcctctc cagacactgg ggccccagtg gctgtgggcg aaggtaatcc 60

aggcctgggt acgattccgg gccctccttc gacttcccag cggttgctgg taggaggagt 120

tggcggaacg acttggaact cctttataag tgtcagctgt gagattttaa tttgatttga 180

aa atg agt aag tgc aga aag aca cca gtt cag cag cta gca agt ccc 227
Met Ser Lys Cys Arg Lys Thr Pro Val Gln Gln Leu Ala Ser Pro
1               5                   10                  15

gcg tca ttc agc cca gat att ctt gct gac att ttt gaa ctc ttt gcc 275
Ala Ser Phe Ser Pro Asp Ile Leu Ala Asp Ile Phe Glu Leu Phe Ala
                20                  25                  30

aag aac ttt tct tat ggc aag cca ctt aat aat gag tgg cag tta cca 323
Lys Asn Phe Ser Tyr Gly Lys Pro Leu Asn Asn Glu Trp Gln Leu Pro
            35                  40                  45

gat ccc agt gag att ttc acc tgt gac cac act gaa ttt aat gca ttt 371
Asp Pro Ser Glu Ile Phe Thr Cys Asp His Thr Glu Phe Asn Ala Phe
        50                  55                  60

ctt gat ttg aag aac tcc cta aat gaa gta aaa aac cta ctg agt gat 419
Leu Asp Leu Lys Asn Ser Leu Asn Glu Val Lys Asn Leu Leu Ser Asp
    65                  70                  75

aag aaa ctg gat gag tgg cat gag cac act gct ttc act aat aaa gca 467
Lys Lys Leu Asp Glu Trp His Glu His Thr Ala Phe Thr Asn Lys Ala
80                  85                  90                  95

ggg aaa atc att tct cat gtt aga aaa tct gtg aat gct gaa ctt tgt 515
Gly Lys Ile Ile Ser His Val Arg Lys Ser Val Asn Ala Glu Leu Cys
                100                 105                 110

act caa gca tgg tgt aag ttc cat gag att ttg tgc agc ttt cca ctt 563
Thr Gln Ala Trp Cys Lys Phe His Glu Ile Leu Cys Ser Phe Pro Leu
            115                 120                 125

att cca cag gaa gct ttt cag aat gga aaa ctg aat tct cta cac ctt 611
Ile Pro Gln Glu Ala Phe Gln Asn Gly Lys Leu Asn Ser Leu His Leu
        130                 135                 140

tgt gaa gct cca gga gct ttt ata gct agt ctc aac cac tac tta aaa 659
Cys Glu Ala Pro Gly Ala Phe Ile Ala Ser Leu Asn His Tyr Leu Lys
    145                 150                 155

tcc cat cgg ttt cct tgt cat tgg agt tgg gta gcg aat act ctg aat 707
Ser His Arg Phe Pro Cys His Trp Ser Trp Val Ala Asn Thr Leu Asn
160                 165                 170                 175

cca tac cat gaa gca aat gac gac ctc atg atg att atg gat gac cgg 755
Pro Tyr His Glu Ala Asn Asp Asp Leu Met Met Ile Met Asp Asp Arg
                180                 185                 190

ctt att gca aat acc ttg cac tgg tgg tac ttt ggt cca gat aac act 803
Leu Ile Ala Asn Thr Leu His Trp Trp Tyr Phe Gly Pro Asp Asn Thr

            195                 200                 205

ggt gat atc atg acc ctg aaa ttc ttg act gga ctt cag aat ttc ata 851
Gly Asp Ile Met Thr Leu Lys Phe Leu Thr Gly Leu Gln Asn Phe Ile
        210                 215                 220

agc agc atg gct act gtt cac ttg gtc act gca gat ggg agt ttt gat 899
Ser Ser Met Ala Thr Val His Leu Val Thr Ala Asp Gly Ser Phe Asp
    225                 230                 235

tgc caa gga aac cca ggt gaa caa gaa gct tta gtt tct tct ttg cat 947
Cys Gln Gly Asn Pro Gly Glu Gln Glu Ala Leu Val Ser Ser Leu His
240                 245                 250                 255

tac tgt gaa gtt gtc act gct ctg acc act ctt gga aac ggt ggc tct 995
Tyr Cys Glu Val Val Thr Ala Leu Thr Thr Leu Gly Asn Gly Gly Ser
                260                 265                 270

ttt gtt cta aag atg ttt act atg ttt gaa cat tgt tcc ata aac ttg 1043
Phe Val Leu Lys Met Phe Thr Met Phe Glu His Cys Ser Ile Asn Leu
            275                 280                 285

atg tac ctg cta aac tgt tgt ttt gac caa gtc cat gtt ttc aaa cct 1091
Met Tyr Leu Leu Asn Cys Cys Phe Asp Gln Val His Val Phe Lys Pro
        290                 295                 300

gct act agc aag gca gga aac tcc gaa gtc tat gtg gtt tgc ctc cac 1139
Ala Thr Ser Lys Ala Gly Asn Ser Glu Val Tyr Val Val Cys Leu His
    305                 310                 315

tat aag ggg aga gag gcc atc cat cct ctg tta tct aag atg acc ttg 1187
Tyr Lys Gly Arg Glu Ala Ile His Pro Leu Leu Ser Lys Met Thr Leu
320                 325                 330                 335

aat ttt ggg act gaa atg aaa agg aaa gcc ctt ttt ccc cat cat gtg 1235
Asn Phe Gly Thr Glu Met Lys Arg Lys Ala Leu Phe Pro His His Val
                340                 345                 350

att cct gat tct ttt ctt aag aga cat gaa gaa tgt tgt gtg ttc ttt 1283
Ile Pro Asp Ser Phe Leu Lys Arg His Glu Glu Cys Cys Val Phe Phe
            355                 360                 365

cat aaa tat cag cta gag act att tct gaa aac att cgt cta ttt gag 1331
His Lys Tyr Gln Leu Glu Thr Ile Ser Glu Asn Ile Arg Leu Phe Glu
        370                 375                 380

tgc atg gga aag gcg gaa caa gaa aag ctg aat aat tta agg gat tgt 1379
Cys Met Gly Lys Ala Glu Gln Glu Lys Leu Asn Asn Leu Arg Asp Cys
    385                 390                 395

gct ata caa tat ttt atg caa aaa ttt caa ctg aaa cat ctt tcc aga 1427
Ala Ile Gln Tyr Phe Met Gln Lys Phe Gln Leu Lys His Leu Ser Arg
400                 405                 410                 415

aat aat tgg cta gta aaa aaa tct agt att ggt tgt agt aca aat aca 1475
Asn Asn Trp Leu Val Lys Lys Ser Ser Ile Gly Cys Ser Thr Asn Thr
                420                 425                 430

aaa tgg ttt ggg cag agg aac aaa tat ttt aaa act tat aat gaa agg 1523
Lys Trp Phe Gly Gln Arg Asn Lys Tyr Phe Lys Thr Tyr Asn Glu Arg

            435                 440                 445

aag atg cta gaa gcc ctt tca tgg aaa gat aaa gta gcc aaa gga tac 1571
Lys Met Leu Glu Ala Leu Ser Trp Lys Asp Lys Val Ala Lys Gly Tyr
        450                 455                 460

ttt aat agt tgg gct gaa gaa cat ggt gta tat cat cct ggg cag agt 1619
Phe Asn Ser Trp Ala Glu Glu His Gly Val Tyr His Pro Gly Gln Ser
    465                 470                 475

tct att tta gaa gga aca gct tcc aat ctt gag tgt cac tta tgg cat 1667
Ser Ile Leu Glu Gly Thr Ala Ser Asn Leu Glu Cys His Leu Trp His
480                 485                 490                 495

att ttg gag gga aag aaa ctg cca aag gta aaa tgt tct cct ttt tgc 1715
Ile Leu Glu Gly Lys Lys Leu Pro Lys Val Lys Cys Ser Pro Phe Cys
                500                 505                 510

aat ggt gaa att tta aaa act ctt aat gaa gca att gaa aag tca tta 1763
Asn Gly Glu Ile Leu Lys Thr Leu Asn Glu Ala Ile Glu Lys Ser Leu
            515                 520                 525

gga gga gct ttt aat ttg gat tcc aag ttt agg cca aaa cag cag tat 1811
Gly Gly Ala Phe Asn Leu Asp Ser Lys Phe Arg Pro Lys Gln Gln Tyr
        530                 535                 540

tct tgt tct tgt cat gtt ttt tct gaa gaa ctg ata ttt tcc gag ttg 1859
Ser Cys Ser Cys His Val Phe Ser Glu Glu Leu Ile Phe Ser Glu Leu
    545                 550                 555

tgt agc ctt act gag tgc ctt cag gat gag cag gtt gta gta ccc agc 1907
Cys Ser Leu Thr Glu Cys Leu Gln Asp Glu Gln Val Val Val Pro Ser
560                 565                 570                 575

aat caa ata aag tgc ctg ctg gtg ggc ttt tcg act ctc cgt aat atc 1955
Asn Gln Ile Lys Cys Leu Leu Val Gly Phe Ser Thr Leu Arg Asn Ile
                580                 585                 590

aaa atg cat ata ccg ttg gaa gtt cga ctc cta gaa tca gct gaa ctc 2003
Lys Met His Ile Pro Leu Glu Val Arg Leu Leu Glu Ser Ala Glu Leu
            595                 600                 605

aca act ttt agc tgt tca ttg ctt cat gat gga gat cca act tac cag 2051
Thr Thr Phe Ser Cys Ser Leu Leu His Asp Gly Asp Pro Thr Tyr Gln
        610                 615                 620

cgt tta ttt ttg gac tgc ctt cta cat tca ttg cgg gag ctt cat aca 2099
Arg Leu Phe Leu Asp Cys Leu Leu His Ser Leu Arg Glu Leu His Thr
    625                 630                 635

gga gat gtt atg att ttg cct gta ctt tct tgc ttc aca aga ttt atg 2147
Gly Asp Val Met Ile Leu Pro Val Leu Ser Cys Phe Thr Arg Phe Met
640                 645                 650                 655

gct ggt ttg atc ttt gta ctc cac agt tgt ttt aga ttc atc act ttt 2195
Ala Gly Leu Ile Phe Val Leu His Ser Cys Phe Arg Phe Ile Thr Phe
                660                 665                 670

gtt tgt ccc aca tcc tct gat ccc ctg agg acc tgc gca gtc ctg cta 2243
Val Cys Pro Thr Ser Ser Asp Pro Leu Arg Thr Cys Ala Val Leu Leu

            675                 680                 685

tgt gtt ggt tat cag gac ctt cca aat cca gtt ttc cga tat ttg cag 2291
Cys Val Gly Tyr Gln Asp Leu Pro Asn Pro Val Phe Arg Tyr Leu Gln
        690                 695                 700

agt gtg aat gaa ttg ttg agc act ttg ctc aac tct gac tca ccc cag 2339
Ser Val Asn Glu Leu Leu Ser Thr Leu Leu Asn Ser Asp Ser Pro Gln
    705                 710                 715

cag gtt tta cag ttt gtg cca atg gag gta ctc ctt aag ggg gcc ctg 2387
Gln Val Leu Gln Phe Val Pro Met Glu Val Leu Leu Lys Gly Ala Leu
720                 725                 730                 735

ctt gat ttt ttg tgg gat ttg aat gct gcc att gct aaa agg cat ttg 2435
Leu Asp Phe Leu Trp Asp Leu Asn Ala Ala Ile Ala Lys Arg His Leu
                740                 745                 750

cat ttc att att caa aga gag aga gaa gaa att atc aac agc ctt cag 2483
His Phe Ile Ile Gln Arg Glu Arg Glu Glu Ile Ile Asn Ser Leu Gln
            755                 760                 765

tta caa aac tgaacat atgctttctg agattcaact ttatgatttc ttataatttg 2539
Leu Gln Asn
        770

cccagtattt gcatcctgtt gctctattaa tttaaaaacc ttttattttg gggaaaggcc 2599

aacatttgca tcattcaaag tctcattaat tctggaaaac catccattct gatctctagg 2659

gtatatacac ccacaggcat agagctcttc cacgtggtgg aatctatgca atgatagata 2719

ttcacactct aaatatgagg tgtgtgtatg tgtatgggtg gccacagcca tgcttaccta 2779

tgccatttag ttggtcttac ttaatctgct taagatttgc atctgtgtac ctttgttcag 2839

attagttttt tttttttcca gccgatttcc tcttagtggc taatgctgtt agtgaatttt 2899

ccaactaatt tcctctcatt ggttaatgtt gttaatgaat tgagagaggt aattgaggaa 2959

aggaaatgag taaatcactg ttcagcaaca ctgatttccg ttaacacatc agttatgaat 3019

ttcagggaat tcatctcgcc agattcttga taacatgcca ttcattgccc ttaggtgatt 3079

gaccctattt tcttacatgg ctcaaataaa actagtatgc tgttgtatga atcttttact 3139

gaccacacca tccaactata aaaatataac gggacagctt taaaccaaag atcatgttta 3199

gaacaatgaa aaattatttg ttgtatctaa tacacgcctg tattgtgaaa agcttcattt 3259

agcaatgatg taataatttt taacttccag gaaataatct gtgaatggaa agatttttta 3319

agattttgag atagtgttta gtctcatgtt gggaacacat gaatgtgatg aacatagtga 3379

atactaaaga aaacgcttca gactttcaga atgatggttc agaatttaaa atttttaatc 3439

ttttctaatt tctttttttc agtgtgaaaa tagcacttta ccaaaagatt agccatgaaa 3499

tggttatttt gccagttaca tttgatttct tttgtatctg caatgtaatg agttatttta 3559

tttcttctgt atttgcagtg taatgagttt ttgtggcaaa gtgtattaag caatttttca 3619

ttatcttgaa gttccacaaa gtggagaata tttatattct cacatgcatt ttaggcactt 3679

ttgatatgtg aaaatagatg tattttctga tgcatttggt taataaatat taatctgaac 3739

attttcatgt tctttgctat tttgaattcc attatagatt catgaataaa gtcattacta 3799

gagaaaaaaa aaaa 3813

<210> 90
<211> 3251
<212> DNA
<213> Homo sapiens

<220>
<221> CDS
<222> (105)..(2174)

<400> 90
cggtcgacga tttcgtcaaa tgttgtttat caggaattct tattggttta tggaagtaac 60

accggttggt gattggctat accttgttga gctataagaa ccac atg ccc tgg gaa 116
Met Pro Trp Glu
1

gaa cca gca ggt gag aag ccc agt tgc tct cac agt cag aag gcg ttc 164
Glu Pro Ala Gly Glu Lys Pro Ser Cys Ser His Ser Gln Lys Ala Phe
5                   10                  15                  20

cac atg gag cct gcc cag aag ccc tgc ttc acc act gac atg gtg aca 212
His Met Glu Pro Ala Gln Lys Pro Cys Phe Thr Thr Asp Met Val Thr
                25                  30                  35

tgg gcc ctc ctc tgc atc tct gca gag act gtg cgt ggg gag gct cct 260
Trp Ala Leu Leu Cys Ile Ser Ala Glu Thr Val Arg Gly Glu Ala Pro
            40                  45                  50

tca cag cct agg ggc atc cct cac cgc tcg ccc gtc agt gtg gat gac 308
Ser Gln Pro Arg Gly Ile Pro His Arg Ser Pro Val Ser Val Asp Asp
        55                  60                  65

ctg tgg ctg gag aag aca cag aga aag aag ttg cag aag cag gcc cac 356
Leu Trp Leu Glu Lys Thr Gln Arg Lys Lys Leu Gln Lys Gln Ala His
    70                  75                  80

atc gaa agg agg ctg cac ata ggg gca gtg cac aaa gat gga gtc aag 404
Ile Glu Arg Arg Leu His Ile Gly Ala Val His Lys Asp Gly Val Lys
85                  90                  95                  100

tgc tgg aga aag acg atc att acc tct cca gag tct ttg aat ctc cct 452
Cys Trp Arg Lys Thr Ile Ile Thr Ser Pro Glu Ser Leu Asn Leu Pro
                105                 110                 115

aga aga agc cat cca ctc tcc cag agt gct cca acg gga ctg aac cac 500
Arg Arg Ser His Pro Leu Ser Gln Ser Ala Pro Thr Gly Leu Asn His
            120                 125                 130

atg ggc tgg cca gag cac aca cca ggc act gcc atg cct gat gga gct 548
Met Gly Trp Pro Glu His Thr Pro Gly Thr Ala Met Pro Asp Gly Ala
        135                 140                 145

ctg gac aca gct gtc tgc gct gac gaa gtg ggg agc gag gag gac ctg 596
Leu Asp Thr Ala Val Cys Ala Asp Glu Val Gly Ser Glu Glu Asp Leu
    150                 155                 160

tat gat gac ctg cac agc tcc agc cac cac tac agc cac cct gga ggg 644
Tyr Asp Asp Leu His Ser Ser Ser His His Tyr Ser His Pro Gly Gly
165                 170                 175                 180

ggt ggg gag cag ctg gct atc aat gag ctc atc agc gat ggc agt gtg 692
Gly Gly Glu Gln Leu Ala Ile Asn Glu Leu Ile Ser Asp Gly Ser Val
                185                 190                 195

gtc tgc gct gaa gca ctc tgg gac cat gtc acc atg gac gac cag gag 740
Val Cys Ala Glu Ala Leu Trp Asp His Val Thr Met Asp Asp Gln Glu
            200                 205                 210

ctg ggc ttc aaa gct ggg gac gtc atc gaa gtg atg gat gcc acc aac 788
Leu Gly Phe Lys Ala Gly Asp Val Ile Glu Val Met Asp Ala Thr Asn
        215                 220                 225

aga gag tgg tgg tgg ggc cgg gtc gcc gat ggc gag ggc tgg ttt cca 836
Arg Glu Trp Trp Trp Gly Arg Val Ala Asp Gly Glu Gly Trp Phe Pro
    230                 235                 240

gcc agc ttc gtt cgg ctg agg gtg aat cag gac gag ccc gcg gat gac 884
Ala Ser Phe Val Arg Leu Arg Val Asn Gln Asp Glu Pro Ala Asp Asp
245                 250                 255                 260

gac gcc cct ctg gcc ggg aac agc gga gcg gag gac ggc ggg gcg gag 932
Asp Ala Pro Leu Ala Gly Asn Ser Gly Ala Glu Asp Gly Gly Ala Glu
                265                 270                 275

gcg cag agc agc aag gac cag atg cgg acc aac gtc atc aac gag atc 980
Ala Gln Ser Ser Lys Asp Gln Met Arg Thr Asn Val Ile Asn Glu Ile
            280                 285                 290

ctc agc act gag cgg gac tac atc aag cac ctg cgc gac atc tgc gag 1028
Leu Ser Thr Glu Arg Asp Tyr Ile Lys His Leu Arg Asp Ile Cys Glu
        295                 300                 305

ggc tac gtc cgg cag tgc cgc aag cgc gca gac atg ttc agc gag gag 1076
Gly Tyr Val Arg Gln Cys Arg Lys Arg Ala Asp Met Phe Ser Glu Glu
    310                 315                 320

cag ctg cgt acc atc ttc ggg aac atc gag gac atc tac cgc tgc cag 1124
Gln Leu Arg Thr Ile Phe Gly Asn Ile Glu Asp Ile Tyr Arg Cys Gln
325                 330                 335                 340

aag gcc ttc gtg aag gcc ctg gag cag agg ttc aac cgc gag cgc cca 1172
Lys Ala Phe Val Lys Ala Leu Glu Gln Arg Phe Asn Arg Glu Arg Pro
                345                 350                 355

cac ctg agc gag ctg ggt gcc tgc ttc ctg gag cat caa gcc gac ttc 1220
His Leu Ser Glu Leu Gly Ala Cys Phe Leu Glu His Gln Ala Asp Phe
            360                 365                 370

cag atc tac tcg gag tac tgc aat aac cac ccc aac gcc tgc gtg gag 1268
Gln Ile Tyr Ser Glu Tyr Cys Asn Asn His Pro Asn Ala Cys Val Glu
        375                 380                 385

ctc tcc cgg ctc acc aag ctc agc aag tac gtg tac ttc ttc gag gcc 1316
Leu Ser Arg Leu Thr Lys Leu Ser Lys Tyr Val Tyr Phe Phe Glu Ala
    390                 395                 400

tgc cgg ctg ctg cag aag atg att gac atc tcc ctg gat ggc ttc ctg 1364
Cys Arg Leu Leu Gln Lys Met Ile Asp Ile Ser Leu Asp Gly Phe Leu
405                 410                 415                 420

ctg act ccg gtg cag aag atc tgc aag tac cct ctg cag ctg gcc gag 1412
Leu Thr Pro Val Gln Lys Ile Cys Lys Tyr Pro Leu Gln Leu Ala Glu
                425                 430                 435

ctg ctc aaa tac acg cac ccc cag cac agg gac ttc aag gat gtt gaa 1460
Leu Leu Lys Tyr Thr His Pro Gln His Arg Asp Phe Lys Asp Val Glu
            440                 445                 450

gcc gcc ttg cat gcc atg aag aac gtg gcc cag ctc atc aac gag cgg 1508
Ala Ala Leu His Ala Met Lys Asn Val Ala Gln Leu Ile Asn Glu Arg
        455                 460                 465

aag cgg aga ctt gag aac atc gac aag att gct cag tgg cag agc tcc 1556
Lys Arg Arg Leu Glu Asn Ile Asp Lys Ile Ala Gln Trp Gln Ser Ser
    470                 475                 480

ata gag gac tgg gag gga gaa gat ctc ttg gtc agg agc tca gaa ctc 1604
Ile Glu Asp Trp Glu Gly Glu Asp Leu Leu Val Arg Ser Ser Glu Leu
485                 490                 495                 500

atc tac tcg ggg gag ctg act cga gtt aca cag cct caa gcc aaa agc 1652
Ile Tyr Ser Gly Glu Leu Thr Arg Val Thr Gln Pro Gln Ala Lys Ser
                505                 510                 515

cag cag cga atg ttc ttt ctc ttt gac cac cag ctc atc tac tgt aag 1700
Gln Gln Arg Met Phe Phe Leu Phe Asp His Gln Leu Ile Tyr Cys Lys
            520                 525                 530

aag gac ctg ctc cgc cgc gac gtg ttg tac tac aag ggc cgg ctg gac 1748
Lys Asp Leu Leu Arg Arg Asp Val Leu Tyr Tyr Lys Gly Arg Leu Asp
        535                 540                 545

atg gac ggc ctg gag gtg gtg gac ctg gag gac ggg aag gac aga gac 1796
Met Asp Gly Leu Glu Val Val Asp Leu Glu Asp Gly Lys Asp Arg Asp
    550                 555                 560

ctc cat gtg agc atc aag aac gcc ttc cgg ctg cac cgt ggc gcc aca 1844
Leu His Val Ser Ile Lys Asn Ala Phe Arg Leu His Arg Gly Ala Thr
565                 570                 575                 580

ggg gac agc cac ctg ctg tgc acc agg aag cct gag cag aag cag cgc 1892
Gly Asp Ser His Leu Leu Cys Thr Arg Lys Pro Glu Gln Lys Gln Arg
                585                 590                 595

tgg ctc aag gcc ttt gcc agg gag agg gag cag gtg cag ctg gac cag 1940
Trp Leu Lys Ala Phe Ala Arg Glu Arg Glu Gln Val Gln Leu Asp Gln
            600                 605                 610

gag aca ggc ttc tcc atc act gaa ctg cag agg aag cag gcc atg ctg     1988
Glu Thr Gly Phe Ser Ile Thr Glu Leu Gln Arg Lys Gln Ala Met Leu
        615                 620                 625

aat gcc agc aag cag cag gtc aca ggg aag ccc aaa gct gtt ggc cgg     2036
Asn Ala Ser Lys Gln Gln Val Thr Gly Lys Pro Lys Ala Val Gly Arg
    630                 635                 640

ccc tgc tac ctg acg cgc cag aag cac cca gcc ctg ccc agc aac cgg     2084
Pro Cys Tyr Leu Thr Arg Gln Lys His Pro Ala Leu Pro Ser Asn Arg
645                 650                 655                 660

ccc cag cag cag gtc ctg gtg ctg gcg gag ccc agg cgc aag cca tct     2132
Pro Gln Gln Gln Val Leu Val Leu Ala Glu Pro Arg Arg Lys Pro Ser
                665                 670                 675

acc ttc tgg cac agc atc agc cgg ctg gca ccc ttc cgc aag tgaactg     2181
Thr Phe Trp His Ser Ile Ser Arg Leu Ala Pro Phe Arg Lys
            680                 685                 690

gtccctgcct gacagcacct gctgggcctt cctgccagtg gcccccagtt tttcttcccc     2241

gaggcccact cggcctggcc ttcctctgcc tgcaagtgag cagggatggg ctggggagtt     2301

gcttgtgcca ccaagacgtg ccaggtctgt actcctgttg tctttttccc tgctcctggt     2361

gccctgaaga gaccagcaag gggcagaccc ccgcactcgc cacaccgccg ctgcagcttg     2421

ggccccatcc gccctctgga cctgtgtagg gcctcactgc tggagcgggg aaaccgcagc     2481

tcagcccagg cccagctggg gagaaggcgc tacctgcgtg ggaccctctt ctctggaaac     2541

ctaatcctcc tttcatttcc tctgggcagg actctctggc cttctgtggc ctgcaatgcc     2601

aggccatgtg cccctctgcc ctctagttct ccaagtcccc agcccggcca gtggtgccag     2661

gcagcttgcc acttgggagg gcagaagcca ggaattccac acccttgtgt tgcgcccgga     2721

gcccgccctt cgcctcccag cccctcaaga caccgctggc tgctggacac cctcttcact     2781

tgtgtgtgtg tgtgtagcgg aaaaggacaa gacggtgcag tcggctgcat actcccagtc     2841

gggagtgtgg tcagtctgcc tgctgctgtg cggtagctcc agaaccacct cgttcctggt     2901

tttgtttgga ttttggcatc ttgtttttct aacaacaaac aatggagaaa aagaattgat     2961

tcttagtgac acagaagatt gccttacgct cgtgagcgtg agaagccata agagagagac     3021

cgaattctgt ggctcagcac acaggactga cccacagccc aggcagcggg tgtgtggaga     3081

tggcgccctg tcctgccaag gggcgccagg agcagagcca gggcctggcg agctggcgtg     3141

gagcccacag gattcagcag catggacagt cactcttgca ctattccttc tccaagccag     3201

aaaccacatt taatttcata aataaattta tgaaaagtaa aaaaaaaaaa               3251

<210> 91
<211> 361
<212> DNA
<213> Homo sapiens

<220>
<221> CDS
<222> (42)..(299)

<400> 91
cgatccatag ctaagacgcc aggtcccccg gaaacctaga a atg gaa cca ttg 53
Met Glu Pro Leu
1

aaa ttt agg gat gtg gcc ata gaa ttc tct ctg gag gag tgg caa tgc 101
Lys Phe Arg Asp Val Ala Ile Glu Phe Ser Leu Glu Glu Trp Gln Cys
5                   10                  15                  20

ctg gac act ata cag cag aat tta tat agg aat gtg atg tta gag aac 149
Leu Asp Thr Ile Gln Gln Asn Leu Tyr Arg Asn Val Met Leu Glu Asn
                25                  30                  35

tac aga aac ctg gtc ttc ttg ggt att gtt gtc tct aag cca gac ctg 197
Tyr Arg Asn Leu Val Phe Leu Gly Ile Val Val Ser Lys Pro Asp Leu
            40                  45                  50

atc acc tgt ctg gaa caa gaa aaa gag cct tgg act aga aag aga cat 245
Ile Thr Cys Leu Glu Gln Glu Lys Glu Pro Trp Thr Arg Lys Arg His
        55                  60                  65

agg atg gtg gcc gaa ccc cca gga tct ctt tct gtt acc cag gct gga 293
Arg Met Val Ala Glu Pro Pro Gly Ser Leu Ser Val Thr Gln Ala Gly
    70                  75                  80

gtg cag tgactgcagc ttcaaccttc agggctcaag tgctcctttc acctcagcct 349
Val Gln
85

cctgactagc tg 361

<210> 92
<211> 1561
<212> DNA
<213> Homo sapiens

<220>
<221> CDS
<222> (48)..(1343)

<400> 92
gcgtccgccc acgcgtccgc ccacgcgtcc ggtgagggga ggcagaa atg gag gaa 56
Met Glu Glu
1

cgg gcg ttc gtc aac ccc ttc ccg gac tac gag gcc gcc gcc ggg gcg 104
Arg Ala Phe Val Asn Pro Phe Pro Asp Tyr Glu Ala Ala Ala Gly Ala
    5                   10                  15

ctg ctc gcc tcc gga gcg gcc gaa gag aca ggc tgt gtt cgt ccc ccg 152
Leu Leu Ala Ser Gly Ala Ala Glu Glu Thr Gly Cys Val Arg Pro Pro
20                  25                  30                  35

gcg acc acg gat gag ccc ggc ctc cct ttt cat cag gac ggg aag atc 200
Ala Thr Thr Asp Glu Pro Gly Leu Pro Phe His Gln Asp Gly Lys Ile
                40                  45                  50

att cat aat ttc ata aga cgg atc cag acc aaa att aaa gat ctt ctg 248
Ile His Asn Phe Ile Arg Arg Ile Gln Thr Lys Ile Lys Asp Leu Leu
            55                  60                  65

cag caa atg gaa gaa ggg ctg aag acg gct gat ccc cat gac tgc tct 296
Gln Gln Met Glu Glu Gly Leu Lys Thr Ala Asp Pro His Asp Cys Ser
        70                  75                  80

gct tat act ggc tgg aca ggc ata gcc ctt ttg tac ctg cag ttg tac 344
Ala Tyr Thr Gly Trp Thr Gly Ile Ala Leu Leu Tyr Leu Gln Leu Tyr
    85                  90                  95

cgg gtc aca tgt gac caa acc tac ctg ctc cga tcc ctg gat tac gta 392
Arg Val Thr Cys Asp Gln Thr Tyr Leu Leu Arg Ser Leu Asp Tyr Val
100                 105                 110                 115

aaa aga aca ctt cgg aat ctg aat ggc cgc agg gtc acc ttc ctc tgt 440
Lys Arg Thr Leu Arg Asn Leu Asn Gly Arg Arg Val Thr Phe Leu Cys
                120                 125                 130

ggg gat gct ggc ccc ctg gct gtt gga gct gtg att tat cac aaa ctc 488
Gly Asp Ala Gly Pro Leu Ala Val Gly Ala Val Ile Tyr His Lys Leu
            135                 140                 145

aga agt gac tgt gag tcc cag gaa tgt gtc aca aaa ctt ttg cag ctc 536
Arg Ser Asp Cys Glu Ser Gln Glu Cys Val Thr Lys Leu Leu Gln Leu
        150                 155                 160

cag aga tcg gtt gtt tgc caa gaa tca gac ctt cct gat gag ctg ctt 584
Gln Arg Ser Val Val Cys Gln Glu Ser Asp Leu Pro Asp Glu Leu Leu
    165                 170                 175

tat gga cgg gca ggt tat ctg tat gcc tta ctg tac ctg aac aca gag 632
Tyr Gly Arg Ala Gly Tyr Leu Tyr Ala Leu Leu Tyr Leu Asn Thr Glu
180                 185                 190                 195

ata ggt cca ggc acc gtg tgt gag tca gct att aaa gag gta gtc aat 680
Ile Gly Pro Gly Thr Val Cys Glu Ser Ala Ile Lys Glu Val Val Asn
                200                 205                 210

gct att att gaa tcg ggt aag act ttg tca agg gaa gaa aga aaa acg 728
Ala Ile Ile Glu Ser Gly Lys Thr Leu Ser Arg Glu Glu Arg Lys Thr
            215                 220                 225

gag cgc tgc ccg ctg ttg tac cag tgg cac cgg aag cag tac gtt gga 776
Glu Arg Cys Pro Leu Leu Tyr Gln Trp His Arg Lys Gln Tyr Val Gly
        230                 235                 240

gca gcc cat ggc atg gct gga att tac tat atg tta atg cag ccg gca 824
Ala Ala His Gly Met Ala Gly Ile Tyr Tyr Met Leu Met Gln Pro Ala
    245                 250                 255

gca aaa gtg gac caa gaa acc ttg aca gaa atg gtg aaa ccc agt att 872
Ala Lys Val Asp Gln Glu Thr Leu Thr Glu Met Val Lys Pro Ser Ile
260                 265                 270                 275

gat tat gtg cgc cac aaa aaa ttc cga tct ggg aat tac cca tca tca 920
Asp Tyr Val Arg His Lys Lys Phe Arg Ser Gly Asn Tyr Pro Ser Ser
                280                 285                 290

tta agc aat gaa aca gac cgg ctg gtg cac tgg tgc cac ggc gcc ccg 968
Leu Ser Asn Glu Thr Asp Arg Leu Val His Trp Cys His Gly Ala Pro
            295                 300                 305

ggg gtc atc cac atg ctc atg cag gcg tac aag gtc ttt aag gag gag 1016
Gly Val Ile His Met Leu Met Gln Ala Tyr Lys Val Phe Lys Glu Glu
        310                 315                 320

aag tac ttg aaa gag gcc atg gag tgt agc gat gtg att tgg cag cga 1064
Lys Tyr Leu Lys Glu Ala Met Glu Cys Ser Asp Val Ile Trp Gln Arg
    325                 330                 335

ggt ttg ctg cgg aag ggc tac ggg ata tgc cat ggg act gct ggc aac 1112
Gly Leu Leu Arg Lys Gly Tyr Gly Ile Cys His Gly Thr Ala Gly Asn
340                 345                 350                 355

ggc tat tcc ttc ctg tcc ctt tac cgt ctc acg cag gat aag aag tac 1160
Gly Tyr Ser Phe Leu Ser Leu Tyr Arg Leu Thr Gln Asp Lys Lys Tyr
                360                 365                 370

ctc tac cga gct tgc aag ttt gca gag tgg tgt cta gat tac gga gca 1208
Leu Tyr Arg Ala Cys Lys Phe Ala Glu Trp Cys Leu Asp Tyr Gly Ala
            375                 380                 385

cac ggg tgc cgc att cct gac aga ccc tat tcg ctc ttt gaa ggc atg 1256
His Gly Cys Arg Ile Pro Asp Arg Pro Tyr Ser Leu Phe Glu Gly Met
        390                 395                 400

gct ggc gct att cac ttt ctc tct gat gtc ctg gga cca gag aca tca 1304
Ala Gly Ala Ile His Phe Leu Ser Asp Val Leu Gly Pro Glu Thr Ser
    405                 410                 415

cgg ttt cca gca ttt gaa ctt gac tct tcg aag agg gat taaaaggtgc 1353
Arg Phe Pro Ala Phe Glu Leu Asp Ser Ser Lys Arg Asp
420                 425                 430

aaaaagacaa ctaaaatacc catttggacc aaaagccgcc agattgctta gtgcctgaca 1413

cagaaacaac tgggaatcct gaaagagaag cagacaccgt cacaggcccc tctggttaga 1473

ctagcatgag tgaccgaagc catccatcaa cattttctaa cagcaccctc atcaatataa 1533

aatatgactt cttcacaaaa aaaaaaaa 1561

<210> 93
<211> 2438
<212> DNA
<213> Homo sapiens

<220>
<221> CDS
<222> (205)..(2124)

<400> 93
   ggtggaattc cgcggagtcc tgcggatggc gcacgcgcaa acctcctgtg atgcctgcgc 60
   ctcctggccc cgccttcggc acgagggagt tccggatgtt tagcgttacc atggatcctg 120
   gaggtgcccg cgaacactgc ttgtcgcctg ggcaaccgga gaggacgaag caggacctag 180
   gtggcggcgg tggtaccggc tgca atg gtg tcc aat ccc gtg cat ggc ttg 231
   Met Val Ser Asn Pro Val His Gly Leu
   1 5
   ccc ttt ctt ccg ggc acg tcc ttt aag gac tct acg aaa aca gcc ttc 279
   Pro Phe Leu Pro Gly Thr Ser Phe Lys Asp Ser Thr Lys Thr Ala Phe
   10 15 20 25
   cac aga agt cag acg ctg agc tac agg aac ggc tat gca att gtt cga 327
   His Arg Ser Gln Thr Leu Ser Tyr Arg Asn Gly Tyr Ala Ile Val Arg
   30 35 40
   cgt cca aca gtt ggg ata ggc gga gac cgg ctc cag ttc aac cag ctg 375
   Arg Pro Thr Val Gly Ile Gly Gly Asp Arg Leu Gln Phe Asn Gln Leu
   45 50 55
   tcc cag gct gag ctg gat gag ttg gcc agt aag gca cca gtc tta act 423
   Ser Gln Ala Glu Leu Asp Glu Leu Ala Ser Lys Ala Pro Val Leu Thr
   60 65 70
   tat ggc caa cct aaa caa gcc cca cct gcg gat ttt att cct gcg cat 471
   Tyr Gly Gln Pro Lys Gln Ala Pro Pro Ala Asp Phe Ile Pro Ala His
   75 80 85
   gtg gcc ttt gac aaa aag gta ctg aaa ttt gat gcc tat ttc caa gaa 519
   Val Ala Phe Asp Lys Lys Val Leu Lys Phe Asp Ala Tyr Phe Gln Glu
   90 95 100 105
   gat gtt cct atg tca act gag gaa cag tat agg atc cgt cag gtg aac 567
   Asp Val Pro Met Ser Thr Glu Glu Gln Tyr Arg Ile Arg Gln Val Asn
   110 115 120
   att tac tat tat cta gaa gat gac agc atg tct gtc ata gag cct gtt 615
   Ile Tyr Tyr Tyr Leu Glu Asp Asp Ser Met Ser Val Ile Glu Pro Val
   125 130 135
   gta gaa aat tct gga atc ctt caa ggc aag tta ata aaa cgc cag cgg 663
   Val Glu Asn Ser Gly Ile Leu Gln Gly Lys Leu Ile Lys Arg Gln Arg
   140 145 150
   cta gcc aag aat gac cgg ggt gac cat tac cat tgg aaa gac cta aat 711
   Leu Ala Lys Asn Asp Arg Gly Asp His Tyr His Trp Lys Asp Leu Asn
   155 160 165
   cga gga ata aac atc aca att tat ggc aaa act ttc cgc gtt gtt gac 759
   Arg Gly Ile Asn Ile Thr Ile Tyr Gly Lys Thr Phe Arg Val Val Asp
   170 175 180 185
   tgt gac caa ttc aca cag gta ttt tta gaa agc caa gga att gag tta     807
   Cys Asp Gln Phe Thr Gln Val Phe Leu Glu Ser Gln Gly Ile Glu Leu
               190                 195                 200
   aat cca cca gag aag atg gct ctt gat cct tac act gaa ctc cga aaa     855
   Asn Pro Pro Glu Lys Met Ala Leu Asp Pro Tyr Thr Glu Leu Arg Lys
           205                 210                 215
   cag cct ctt cgt aag tat gtc acc cca tca gac ttt gat caa ctc aag     903
   Gln Pro Leu Arg Lys Tyr Val Thr Pro Ser Asp Phe Asp Gln Leu Lys
       220                 225                 230
   caa ttt ctc acc ttt gac aaa cag gtc ctt cga ttc tat gca atc tgg     951
   Gln Phe Leu Thr Phe Asp Lys Gln Val Leu Arg Phe Tyr Ala Ile Trp
       235                 240                 245
   gat gat aca gac agc atg tat ggt gaa tgt cgg acc tac atc att cat     999
   Asp Asp Thr Asp Ser Met Tyr Gly Glu Cys Arg Thr Tyr Ile Ile His
   250                 255                 260                 265
   tac tat ctt atg gat gat acg gtg gaa att cga gag gtc cac gaa cgg     1047
   Tyr Tyr Leu Met Asp Asp Thr Val Glu Ile Arg Glu Val His Glu Arg
               270                 275                 280
   aat gat ggg aga gat cct ttc cca ctc cta atg aac cgc cag cgt gtg     1095
   Asn Asp Gly Arg Asp Pro Phe Pro Leu Leu Met Asn Arg Gln Arg Val
           285                 290                 295
   ccc aaa gtt ttg gtg gaa aat gca aag aac ttc cct cag tgt gtg cta     1143
   Pro Lys Val Leu Val Glu Asn Ala Lys Asn Phe Pro Gln Cys Val Leu
       300                 305                 310
   gaa atc tct gac caa gaa gtg ttg gaa tgg tat act gct aaa gac ttc     1191
   Glu Ile Ser Asp Gln Glu Val Leu Glu Trp Tyr Thr Ala Lys Asp Phe
       315                 320                 325
   att gtt ggg aag tca ctc act atc ctt ggg aga act ttc ttc att tat     1239
   Ile Val Gly Lys Ser Leu Thr Ile Leu Gly Arg Thr Phe Phe Ile Tyr
   330                 335                 340                 345
   gat tgt gat cca ttt act cga cgg tat tac aaa gag aag ttt gga atc     1287
   Asp Cys Asp Pro Phe Thr Arg Arg Tyr Tyr Lys Glu Lys Phe Gly Ile
               350                 355                 360
   act gat tta cca cgt att gat gtg agc aag cgg gaa cca cct cca gta     1335
   Thr Asp Leu Pro Arg Ile Asp Val Ser Lys Arg Glu Pro Pro Pro Val
           365                 370                 375
   aaa cag gag ttg cct cct tat aac ggt ttt gga cta gtg gaa gat tct     1383
   Lys Gln Glu Leu Pro Pro Tyr Asn Gly Phe Gly Leu Val Glu Asp Ser
       380                 385                 390
   gct cag aat tgt ttt gct ctc att cca aaa gct cca aaa aaa gac gtt     1431
   Ala Gln Asn Cys Phe Ala Leu Ile Pro Lys Ala Pro Lys Lys Asp Val
       395                 400                 405
   att aaa atg ctg gtg aat gat aac aag gtg ctt cgt tat ttg gct gta     1479
   Ile Lys Met Leu Val Asn Asp Asn Lys Val Leu Arg Tyr Leu Ala Val
   410                 415                 420                 425
   ctg gaa tcc ccc atc cca gaa gac aaa gac cgc aga ttt gtc ttc tct     1527
   Leu Glu Ser Pro Ile Pro Glu Asp Lys Asp Arg Arg Phe Val Phe Ser
                   430                 435                 440
   tac ttt cta gct acc gac atg atc agt atc ttt gag cct cct gtt cgc     1575
   Tyr Phe Leu Ala Thr Asp Met Ile Ser Ile Phe Glu Pro Pro Val Arg
                   445                 450                 455
   aat tct ggt atc att ggg ggc aag tac ctt ggc agg act aaa gtt gtt     1623
   Asn Ser Gly Ile Ile Gly Gly Lys Tyr Leu Gly Arg Thr Lys Val Val
           460                 465                 470
   aaa cca tac tct aca gtg gac aac cct gtc tac tat ggc ccc agt gac     1671
   Lys Pro Tyr Ser Thr Val Asp Asn Pro Val Tyr Tyr Gly Pro Ser Asp
       475                 480                 485
   ttc ttc att ggt gct gtg att gaa gtg ttt ggt cac cgg ttc atc atc     1719
   Phe Phe Ile Gly Ala Val Ile Glu Val Phe Gly His Arg Phe Ile Ile
   490                 495                 500                 505
   ctt gat aca gac gag tat gtt ttg aaa tac atg gag agc aac gct gcc     1767
   Leu Asp Thr Asp Glu Tyr Val Leu Lys Tyr Met Glu Ser Asn Ala Ala
                   510                 515                 520
   cag tat tca cca gaa gca ctc gcg tca att cag aac cat gtc cga aag     1815
   Gln Tyr Ser Pro Glu Ala Leu Ala Ser Ile Gln Asn His Val Arg Lys
               525                 530                 535
   cga gaa gcg cct gct cca gaa gca gaa agc aag caa act gaa aag gat     1863
   Arg Glu Ala Pro Ala Pro Glu Ala Glu Ser Lys Gln Thr Glu Lys Asp
           540                 545                 550
   cca ggc gtg cag gaa ttg gaa gca tta ata gac aca att cag aag caa     1911
   Pro Gly Val Gln Glu Leu Glu Ala Leu Ile Asp Thr Ile Gln Lys Gln
       555                 560                 565
   ctg aaa gat cac tca tgc aaa gac aac att cgt gag gca ttt caa att     1959
   Leu Lys Asp His Ser Cys Lys Asp Asn Ile Arg Glu Ala Phe Gln Ile
   570                 575                 580                 585
   tat gac aag gaa gct tca gga tat gtg gac aga gac atg ttc ttt aaa     2007
   Tyr Asp Lys Glu Ala Ser Gly Tyr Val Asp Arg Asp Met Phe Phe Lys
                   590                 595                 600
   atc tgt gaa tcg ctt aac gtc cca gtg gat gac tcc ttg gtt aag gag     2055
   Ile Cys Glu Ser Leu Asn Val Pro Val Asp Asp Ser Leu Val Lys Glu
               605                 610                 615
   tta atc agg atg tgc tct cat gga gaa ggc aaa att aac tac tat aac     2103
   Leu Ile Arg Met Cys Ser His Gly Glu Gly Lys Ile Asn Tyr Tyr Asn
           620                 625                 630
   ttt gtt cgt gct ttc tca aac tg acctgctgat gagaaaatgc aagacaattt     2156
   Phe Val Arg Ala Phe Ser Asn
       635                 640
   ttgatactgg aactatgctt tgaaatacac cttacactct tcatagaggc atttacaggg     2216
   ttcctgaagt tttatttctg ttttggttct tatttcactc ctactgaagt cgaaactaaa     2276
   ttggatctaa taggatctaa gattggtgcc ttatttaggg tgataggggt atagcaatgt 2336
   ctaattttgt gtgtcaaatt gacttggcca caggggcccc aaatatttcc tttctttctt 2396
   tttaaaaaaa taaatttttt tagagatggg aaaaaaaaaa aa 2438

<210> 94
<211> 1474
<212> DNA
<213> Homo sapiens

<220>
<221> CDS
<222> (59)..(1078)

<400> 94
cgccgggttt ccctcatgat cccgggcggg cggcggcggc ggcagaggcg gcgggagg 58
atg acc tct tac cgg gag cgg agt gcc gac ctg gcc cgt ttc tac act 106
Met Thr Ser Tyr Arg Glu Arg Ser Ala Asp Leu Ala Arg Phe Tyr Thr
1               5                   10                  15

gtc acc gag ccc cag cga cac ccg agg ggc tac aca gta tat aag gtc 154
Val Thr Glu Pro Gln Arg His Pro Arg Gly Tyr Thr Val Tyr Lys Val
            20                  25                  30

acc gcc cgg gtt gtt tca cga aga aat cca cag gat gtc cag gag ata 202
Thr Ala Arg Val Val Ser Arg Arg Asn Pro Gln Asp Val Gln Glu Ile
        35                  40                  45

att gta tgg aat aga tac acc gtt ttt aac aaa cta cac cta gaa cta 250
Ile Val Trp Asn Arg Tyr Thr Val Phe Asn Lys Leu His Leu Glu Leu
    50                  55                  60

tgg cta att cac aaa aac tta ttt cgt cat tca gag ttg ttt cct cca 298
Trp Leu Ile His Lys Asn Leu Phe Arg His Ser Glu Leu Phe Pro Pro
65                  70                  75                  80

ttt gct aaa gga ata gtg ttt ggg cga ttt gat gaa act gtt ata gaa 346
Phe Ala Lys Gly Ile Val Phe Gly Arg Phe Asp Glu Thr Val Ile Glu
                85                  90                  95

gag aga aga caa tgt gct gaa gac ctg cta cag ttc tct gcc aat att 394
Glu Arg Arg Gln Cys Ala Glu Asp Leu Leu Gln Phe Ser Ala Asn Ile
            100                 105                 110

cct gct ctt tac aat agt aaa cag ctt gaa gac ttt ttc aag ggt gga 442
Pro Ala Leu Tyr Asn Ser Lys Gln Leu Glu Asp Phe Phe Lys Gly Gly
        115                 120                 125

ata att aat gat agt tct gaa tta att ggt cct gct gaa gct cac tca 490
Ile Ile Asn Asp Ser Ser Glu Leu Ile Gly Pro Ala Glu Ala His Ser
    130                 135                 140

gat tcc ctc att gat acc ttt cct gag tgt agt acg gaa ggc ttc tcc 538
Asp Ser Leu Ile Asp Thr Phe Pro Glu Cys Ser Thr Glu Gly Phe Ser
145                 150                 155                 160

agc gac agt gat ctg gta tct ctt act gtt gat gtg gat tct ctt gct 586
Ser Asp Ser Asp Leu Val Ser Leu Thr Val Asp Val Asp Ser Leu Ala
                165                 170                 175

gag tta gat gat gga atg gct tcc aat caa aat tct ccc att aga act 634
Glu Leu Asp Asp Gly Met Ala Ser Asn Gln Asn Ser Pro Ile Arg Thr
            180                 185                 190

ttt ggt ctc aat ctt tct tcg gat tct tca gca cta ggg gct gtt gct 682
Phe Gly Leu Asn Leu Ser Ser Asp Ser Ser Ala Leu Gly Ala Val Ala
        195                 200                 205

tct gac agt gaa cag agc aaa aca gaa gaa gaa cgg gaa agt cgt agc 730
Ser Asp Ser Glu Gln Ser Lys Thr Glu Glu Glu Arg Glu Ser Arg Ser
    210                 215                 220

ctc ttt cct ggc agt tta aag ccg aag ctt ggc aag aga gat tat ttg 778
Leu Phe Pro Gly Ser Leu Lys Pro Lys Leu Gly Lys Arg Asp Tyr Leu
225                 230                 235                 240

gag aaa gca gga gaa tta ata aag ctg gct tta aaa aag gaa gaa gaa 826
Glu Lys Ala Gly Glu Leu Ile Lys Leu Ala Leu Lys Lys Glu Glu Glu
                245                 250                 255

gac gac tat gaa gct gct tct gat ttt tat agg aag gga gtt gat tta 874
Asp Asp Tyr Glu Ala Ala Ser Asp Phe Tyr Arg Lys Gly Val Asp Leu
            260                 265                 270

ctc cta gaa ggt gtt caa gga gag tca agc cct acc cgt cga gaa gct 922
Leu Leu Glu Gly Val Gln Gly Glu Ser Ser Pro Thr Arg Arg Glu Ala
        275                 280                 285

gtg aag aga aga aca gcc gag tac ctc atg cgg gca gaa agt atc tct 970
Val Lys Arg Arg Thr Ala Glu Tyr Leu Met Arg Ala Glu Ser Ile Ser
    290                 295                 300

agt ctt tat ggg aaa cct cag ctt gat gat gta tct cag gta tgt ctc 1018
Ser Leu Tyr Gly Lys Pro Gln Leu Asp Asp Val Ser Gln Val Cys Leu
305                 310                 315                 320

ata ttt tgt tgt gtg ttt tct tca gtg att ttt tgc tgt ctg gtc tct 1066
Ile Phe Cys Cys Val Phe Ser Ser Val Ile Phe Cys Cys Leu Val Ser
                325                 330                 335

ctg ctt tat ttc taa ttagaatgtt ataggatttt gatttttga attttattaa 1121
Leu Leu Tyr Phe
            340

tgattttcat aaaggttggt ggtagaaaga aatgcattat agcaaaccac caaatgtgga 1181

ataaaataag acttgctttc tccttttctt ggacttttat tagattctta ctgtaccctt 1241

agtctcctca tctggagatg agacagcaac ttatactggc atgatatcta tttggatttg 1301

gacgttttac cgttatattg ggactcttgg ataactgctc tccaaatgta ctgctttatg 1361

ttcttagcta tacatgataa gcctaaaatg tgcgcgctat accaatctgc gcccttaaat 1421

acaaaaccat aattgtatca tagattatta tgatattatg cacatcctct aca 1474

<210> 95
<211> 1288
<212> DNA
<213> Homo sapiens

<220>
<221> CDS
<222> (773)..(1015)

<400> 95
ggaattccct agcagataga ggacttggtc tgaccttcag ggttgtgatg gggcaggttg 60

ggaggtgggc ggggagtctc tgtggtctgg gtgctgaggt tgacccgatg ggcaggtgaa 120

gaaaatcacc ttctccattc cccttgattt ctttatcgtg tgccgcacga ggcatgctga 180

agggacacac acctttcccc ctttttattt acaaagtaga gagctgagaa agacagctca 240

tgttctgttt tgttttgtac acattatctt tctcaagaga tgtgatgtcc ttaagtaaac 300

tgacctgcct tcatggcttg tcctgtaatg aatgcgttca agacagtgtt ttggggacag 360

gaaagaggca ttcttaggta agcggcggtg ggggatgggg tggggtggag aggaaaccct 420

tctaggaagt agttcaatga aaaacctaaa aatgcccaaa taaacattcg cttacatatt 480

atatatcttg ggcttctttt tatgacagtt cattcaggga cccagtcagt aaacaggtag 540

ttaacttcgt gtaaccaagc aagggttttg tttatctttt ttaaagtaga aaaaagtgag 600

gctcaaactc tctctttctc cttgtcataa ctattggttt acagtcttta tttgtttaaa 660

agtaaagcac attgtatgta tttatttggc aatacatgag gccattaaaa ccctgagcct 720

aaggtaccac agttagtctc atttgcctct tgtcctgtga actccactta ga atg 775
Met
1

tca ttg aac ttg ggc aga cat aat tct agt gtc tgt tcc aaa cgc act 823
Ser Leu Asn Leu Gly Arg His Asn Ser Ser Val Cys Ser Lys Arg Thr
            5                   10                  15

gtg tca cag aag cta gaa tta cca tta gag gca caa acc cct gag aat 871
Val Ser Gln Lys Leu Glu Leu Pro Leu Glu Ala Gln Thr Pro Glu Asn
        20                  25                  30

aca caa ggg ggc acg ctt cca gta gat gtg ttg ggg aag gag gag ggc 919
Thr Gln Gly Gly Thr Leu Pro Val Asp Val Leu Gly Lys Glu Glu Gly
    35                  40                  45

aga ggg gac agg gga cag gat tca gct ttg tgg tgg gtc ctg agg gtt 967
Arg Gly Asp Arg Gly Gln Asp Ser Ala Leu Trp Trp Val Leu Arg Val
50                  55                  60                  65

cct acc agg ggt agc cag gat ctg gga aac aga tca gcg act cta gtc 1015
Pro Thr Arg Gly Ser Gln Asp Leu Gly Asn Arg Ser Ala Thr Leu Val
                70                  75                  80

tgaagtggct gcctggttcg ggggctgcct tcagcaagat tcaggcagga gagacggaaa 1075

tagccacctt ccaggcgtga gtcctggaga taaaaatgga ttttaaccta ggactgccgg 1135

gagctggccc tccgcggctg ctcagactag ggctgtgtgg gctggctctc gcctgtttcc 1195

ggaggataac tggcttggtt ctctttatgg cttggcttca ttccgaccct tgggtggggc 1255

cacatccaac ccactgccca ctgggtgtgc gtg 1288

<210> 96
<211> 1888
<212> DNA
<213> Homo sapiens

<220>
<221> CDS
<222> (505)..(960)

<400> 96
agtgcggcgc agcgcagctc ctgaggtttt ggttatgaac aggattcgga ttcacgtctt 60

gccaaccaat cgggggagga tcactccagt gcccaggtct caggaacctc tgtcttgtgc 120

attcactcat cgtccatgct ctcaccctcg tctggagggg caggagtttt gcattaagca 180

tatccttgaa gacaagaatg cacccttcaa gcagtgtagt tatatatcga cgaagaatgg 240

aaaaagatgt cccaatgctg ccccaaagcc agagaagaaa gatggggtgt ccttctgtgc 300

tgaacatgtc cgtaggaatg ccctggcact tcatgctcaa atgaagaaga ccaacctagg 360

gcctgtgggt gaaacactcc tgtgccagct gagctcatat gctaagacag agctggggtc 420

tcagactcca gaaagtagtc gcagtgaagc cagccgaata ctagacatgc tggtgtctac 480

acggcagaag aagtggccct gatt atg cgt gaa aag cta att cgt ttg cag 531
Met Arg Glu Lys Leu Ile Arg Leu Gln
1               5

tcg ttg tat att gat cag ttt aaa cga ctt cag cat ctg ctc aag gag 579
Ser Leu Tyr Ile Asp Gln Phe Lys Arg Leu Gln His Leu Leu Lys Glu
10                  15                  20                  25

aag aag cgc cga tac tta cat aat cgc aaa gtg gaa cat gaa gct cta 627
Lys Lys Arg Arg Tyr Leu His Asn Arg Lys Val Glu His Glu Ala Leu
                30                  35                  40

ggc agt agt ctc ctg act ggc cca gag gga ctt ttg gcc aaa gaa cga 675
Gly Ser Ser Leu Leu Thr Gly Pro Glu Gly Leu Leu Ala Lys Glu Arg
            45                  50                  55

gag aac tta aag cga tta aaa tgt ctg cga cga tac cgc cag cgc tat 723
Glu Asn Leu Lys Arg Leu Lys Cys Leu Arg Arg Tyr Arg Gln Arg Tyr
        60                  65                  70

gga gtg gaa gcc tta ctg cat agg cag ttg aag gaa cgg aga atg ctg     771
Gly Val Glu Ala Leu Leu His Arg Gln Leu Lys Glu Arg Arg Met Leu
    75                  80                  85

gcc aca gat ggt gct gcc caa cag gcc cat acc act cgt tcc agt cag     819
Ala Thr Asp Gly Ala Ala Gln Gln Ala His Thr Thr Arg Ser Ser Gln
90                  95                  100                 105

agg tgc ttg gcc ttt gtg gat gat gtt cgt tgt tcc aat cag tct ctt     867
Arg Cys Leu Ala Phe Val Asp Asp Val Arg Cys Ser Asn Gln Ser Leu
                110                 115                 120

cca atg acc aga cac tgc ctt acc cag aaa gat gcg act gcc att ggt     915
Pro Met Thr Arg His Cys Leu Thr Gln Lys Asp Ala Thr Ala Ile Gly
            125                 130                 135

gac ctg tta act ctt tac tgt ggt cta atg gag aca gca ctc gac tga     963
Asp Leu Leu Thr Leu Tyr Cys Gly Leu Met Glu Thr Ala Leu Asp
        140                 145                 150

aagtgaagga cccagactct agttgacttg gatgttgtgg gtgatggtat gcagtgtcct   1023

ccttcacctt tgctttttga tccttcacta acccttgaag atcatttagt caaagaaatt   1083

gctgaagaca cagtggatat tttgggccag atgcagatgg ctggagatgg gtgcagatcc   1143

cagggatctc gaaattctga gaaagcctct gcaccattgt ctcagagtgg attggctact   1203

gcaaatggga agccagaacc cacttctatt agttgatagt ttggggaacc attttacttt   1263

ggtggattta aatttctcgt tcttcaaaga agtatttctg atcatctctc acaaaacaca   1323

ggcaacccag actaccttgt ttttcttctg gattgagtat tatagtcaaa cgtaaagcca   1383

aatttgaggg acgtggaccg tgggtttctt cccactcagg gcaaggaaga gaaagagaga   1443

taatccctat caaagcttct gggattattt gggactatac ctaaaaggaa agtgacatct   1503

ttgtaatggg atttcccaag tcatggatag aaatccactt aagtagctag gtgggagctg   1563

tctttgtgag tggtgtcttt gagatactga tggccctaat atcggttatc tctatctgaa   1623

gacctgcagt ttagcttttc ttcacctcta cttaattggg cagtctttaa cccctttccc   1683

tggtgaactc tggcttttta tcctctgaga aaacagccca caggactggg tcctccttat   1743

ccgtcttgct tttaacctat gtaaatttct gtggttgcta tcttatgtaa aatatagtaa   1803

aaatttattg tatatagttt ctattaaatg ttaaagaaaa tgttatgaaa atatcaaaca   1863

gtcacctgaa ttgaaaaaaa aaaaa                                         1888

<210> 97
<211> 1488
<212> DNA
<213> Homo sapiens

<220>
<221> CDS
<222> (63)..(1364)

<400> 97
gagcccggcg agggcgccgg tgctttgttc tgtctgaggc caggaagttt gaccgcgctg      60
cc   atg ccg aac cgc aag gcc agc cgg aat gct tac tat ttc ttc gtg      107
cag gag aag atc ccc gaa cta cgg cga cga ggc ctg cct gtg gct cgc      155
gtt gct gat gcc atc cct tac tgc tcc gca gac tgg gcg ctt ctg agg      203
gag gaa gaa aag gag aaa tac gca gaa atg gct cga gaa tgg agg gcc      251
gct cag gga aag gac cct ggg ccc tca gag aag cag aaa cct gtt ttc      299
aca cca ctg agg agg cca ggc atg ctt gta cca aag cag aat gtt tca      347
cct cca gat atg tca gct ttg tct tta aaa ggt gat caa gct ctc ctt      395
gga ggc att ttt tat ttt ttg aac att ttt agc cat ggc gag cta cct      443
cct cat tgt gaa cag cgc ttc ctc cct tgt gaa att ggc tgt gtt aag      491
tat tct ctc caa gaa ggt att atg gca gat ttc cac agt ttt ata aat      539
cct ggt gaa att cca cga gga ttt cga ttt cat tgt cag gct gca agt      587
gat tct agt cac aag att cct att tca aat ttt gaa cgt ggg cat aac      635
caa gca act gtg tta caa aac ctt tat aga ttt att cat ccc aac cca      683
ggg aac tgg cca cct atc tac tgc aag tct gat gat aga acc aga gtc      731
aac tgg tgt ttg aag cat atg gca aag gca tca gaa atc agg caa gat 779
cta caa ctt ctc act gta gag gac ctt gta gtg ggg atc tac caa caa 827
aaa ttt ctc aag gag ccc tct aag act tgg att cga agc ctc cta gat 875
gtg gcc atg tgg gat tat tct agc aac aca agg tgc aag tgg cat gaa 923
gaa aat gat att ctc ttc tgt gct tta gct gtt tgc aag aag att gcg 971
tac tgc atc agt aat tct ctg gcc act ctc ttt gga atc cag ctc aca 1019
gag gct cat gta cca cta caa gat tat gag gcc agc aat agt gtg aca 1067
ccc aaa atg gtt gta ttg gat gca ggg cgt tac cag aag cta agg gtt 1115
ggg agt tca gga ttc tct cat ttc aac tct tct aat gag gaa caa aga 1163
tca aac aca ccc att ggt gac tac cca tct agg gca aaa att tct ggc 1211
caa aac agc agc gtt cgg gga aga gga att acc cgc tta cta gag agc 1259
att tcc aat tct tcc agc aat atc cac aaa ttc tcc aac tgt gac act 1307
tca ctc tca cct tac atg tcc caa aaa gat gga tac aaa tct ttc tct 1355
tcc tta tct taatgat ggtactcttt tcaatttctg aaaacagtaa caggcccaac 1411
ttccttctta ctacagtcat attaaacaga tcacatcaat gacaaatgtc actactataa 1471
aaactaaaaa aaaaaaa                                                    1488

<210> 98
<211> 2208
<212> DNA
<213> Homo sapiens

<220>
<221> CDS
<222> (73)..(2112)

<400> 98
gatgctgata aagagaaaga tttgcagaaa tttcttaaaa atgtggatga aatctccaat      60

ttaattcagg ag atg aat tct gat gac cca gtt gtg caa cag aaa gct         108
              Met Asn Ser Asp Asp Pro Val Val Gln Gln Lys Ala
              1               5                   10

gtc ctg gag aca gaa aag aga cta ctg ctt atg gag gaa gac cag gag       156
Val Leu Glu Thr Glu Lys Arg Leu Leu Leu Met Glu Glu Asp Gln Glu
        15                  20                  25

gag gat gaa tgc agg acc acc ttg aac aag act atg atc agt cct cca       204
Glu Asp Glu Cys Arg Thr Thr Leu Asn Lys Thr Met Ile Ser Pro Pro
    30                  35                  40

caa act gct atg aag agt gca gaa gaa ata aac tca gag gcc ttc ttg       252
Gln Thr Ala Met Lys Ser Ala Glu Glu Ile Asn Ser Glu Ala Phe Leu
45                  50                  55                  60

gca tct gtg gag aag gat gca aag gaa cga gcc aag aga aga agg gaa       300
Ala Ser Val Glu Lys Asp Ala Lys Glu Arg Ala Lys Arg Arg Arg Glu
                65                  70                  75

aac aaa gtc ttg gcg gat gcc cta aaa gaa aaa ggg aat gaa gca ttt       348
Asn Lys Val Leu Ala Asp Ala Leu Lys Glu Lys Gly Asn Glu Ala Phe
            80                  85                  90

gct gaa ggc aat tat gaa aca gct atc ctg cgc tac agt gag ggt ttg       396
Ala Glu Gly Asn Tyr Glu Thr Ala Ile Leu Arg Tyr Ser Glu Gly Leu
        95                  100                 105

gag aag ctg aag gac atg aaa gtg ctg tac acc aac cga gcc cag gct       444
Glu Lys Leu Lys Asp Met Lys Val Leu Tyr Thr Asn Arg Ala Gln Ala
    110                 115                 120

tat atg aaa ctt gag gac tat gag aag gca ctg gtg gat tgt gag tgg       492
Tyr Met Lys Leu Glu Asp Tyr Glu Lys Ala Leu Val Asp Cys Glu Trp
125                 130                 135                 140

gct ctc aag tgt gat gaa aaa tgc aca aaa gca tat ttt cac atg gga       540
Ala Leu Lys Cys Asp Glu Lys Cys Thr Lys Ala Tyr Phe His Met Gly
                145                 150                 155

aaa gcc aac ctg gcc ctg aag aac tac agt gtg tct aga gag tgt tat       588
Lys Ala Asn Leu Ala Leu Lys Asn Tyr Ser Val Ser Arg Glu Cys Tyr

            160                 165                 170

aag aag atc tta gaa ata aac ccc aag ctg caa acc cag gtg aaa ggt 636
Lys Lys Ile Leu Glu Ile Asn Pro Lys Leu Gln Thr Gln Val Lys Gly
        175                 180                 185

tac ctg aat caa gta gat ctt cag gaa aaa gca gac ctt caa gaa aag 684
Tyr Leu Asn Gln Val Asp Leu Gln Glu Lys Ala Asp Leu Gln Glu Lys
    190                 195                 200

gaa gcc cac gaa ctg ctg gat tca gga aag aac aca gcc gtg acc acc 732
Glu Ala His Glu Leu Leu Asp Ser Gly Lys Asn Thr Ala Val Thr Thr
205                 210                 215                 220

aag aac ctc ctg gag acc ctt tcc aag cct gac cag atc ccc ttg ttc 780
Lys Asn Leu Leu Glu Thr Leu Ser Lys Pro Asp Gln Ile Pro Leu Phe
                225                 230                 235

tat gct ggg ggg att gag atc ctg act gaa atg ata aat gag tgc aca 828
Tyr Ala Gly Gly Ile Glu Ile Leu Thr Glu Met Ile Asn Glu Cys Thr
            240                 245                 250

gaa caa act tta ttc aga atg cac aat gga ttt agt atc atc agt gac 876
Glu Gln Thr Leu Phe Arg Met His Asn Gly Phe Ser Ile Ile Ser Asp
        255                 260                 265

aac gag gtc ata aga agg tgt ttt tcc aca gca gga aat gat gca gtt 924
Asn Glu Val Ile Arg Arg Cys Phe Ser Thr Ala Gly Asn Asp Ala Val
    270                 275                 280

gaa gaa atg gtc tgt gtg tct gtt ctc aag ctc tgg caa gca gtg tgc 972
Glu Glu Met Val Cys Val Ser Val Leu Lys Leu Trp Gln Ala Val Cys
285                 290                 295                 300

agc agg aac gag gaa aac cag cgt gtg cta gtg ata cac cat gac agg 1020
Ser Arg Asn Glu Glu Asn Gln Arg Val Leu Val Ile His His Asp Arg
                305                 310                 315

gcc agg ctg ttg gcc gcc ctc ttg tcc tcc aag gtc ctg gcc atc cgg 1068
Ala Arg Leu Leu Ala Ala Leu Leu Ser Ser Lys Val Leu Ala Ile Arg
            320                 325                 330

cag cag agc tat gcc ctg ctg ctg cat ctc gcc cag act gag agc gga 1116
Gln Gln Ser Tyr Ala Leu Leu Leu His Leu Ala Gln Thr Glu Ser Gly
        335                 340                 345

cgg agc ctg atc atc aac cac ctt gac ctg acc aga tta ttg gaa gcg 1164
Arg Ser Leu Ile Ile Asn His Leu Asp Leu Thr Arg Leu Leu Glu Ala
    350                 355                 360

ctg gtg tca ttt ctt gat ttc tcg gat aag gag gcc aac act gct atg 1212
Leu Val Ser Phe Leu Asp Phe Ser Asp Lys Glu Ala Asn Thr Ala Met
365                 370                 375                 380

gga ctg ttc aca gac ttg gct ctg gaa gaa aga ttc caa gtc tgg ttc 1260
Gly Leu Phe Thr Asp Leu Ala Leu Glu Glu Arg Phe Gln Val Trp Phe
                385                 390                 395

cag gcc aac ctt cca ggt gtt ctc cct gca ctc aca ggc gtt ctg aag 1308
Gln Ala Asn Leu Pro Gly Val Leu Pro Ala Leu Thr Gly Val Leu Lys

400                 405                 410

aca gat ccc aag gta agc agc tcc tcg gct ctg tgc cag tgc att gcc      1356
Thr Asp Pro Lys Val Ser Ser Ser Ser Ala Leu Cys Gln Cys Ile Ala
        415                 420                 425

atc atg gga aac ctc agt gct gag ccc act acc cga aga cac atg gcg      1404
Ile Met Gly Asn Leu Ser Ala Glu Pro Thr Thr Arg Arg His Met Ala
    430                 435                 440

gcc tgt gag gaa ttt ggg gat ggc tgc ttg agc ctc ctg gcc agg tgt      1452
Ala Cys Glu Glu Phe Gly Asp Gly Cys Leu Ser Leu Leu Ala Arg Cys
445                 450                 455                 460

gag gag gat gtg gac ctg ttc aga gag gtt atc tac aca ctc ctg gga      1500
Glu Glu Asp Val Asp Leu Phe Arg Glu Val Ile Tyr Thr Leu Leu Gly
                465                 470                 475

ctc atg atg aac ctg tgt ctt cag gct ccc ttt gtc tct gag gtt tgg      1548
Leu Met Met Asn Leu Cys Leu Gln Ala Pro Phe Val Ser Glu Val Trp
            480                 485                 490

gct gtg gag gtg agc aga agg tgc ctg tct tta cta aac agc cag gat      1596
Ala Val Glu Val Ser Arg Arg Cys Leu Ser Leu Leu Asn Ser Gln Asp
        495                 500                 505

gga gga atc ctg aca aga gct gct ggt gtt ctg agc cgg acc ctt tct      1644
Gly Gly Ile Leu Thr Arg Ala Ala Gly Val Leu Ser Arg Thr Leu Ser
    510                 515                 520

tcc tct ctg aaa att gtt gag gag gcc ttg cga gca gga gtg gta aag      1692
Ser Ser Leu Lys Ile Val Glu Glu Ala Leu Arg Ala Gly Val Val Lys
525                 530                 535                 540

aaa atg atg aaa ttc ctg aag aca gga ggt gag act gca tca cgt tat      1740
Lys Met Met Lys Phe Leu Lys Thr Gly Gly Glu Thr Ala Ser Arg Tyr
                545                 550                 555

gct ata aag ata cta gct atc tgc acg aat agt tat cat gaa gct cgg      1788
Ala Ile Lys Ile Leu Ala Ile Cys Thr Asn Ser Tyr His Glu Ala Arg
            560                 565                 570

gaa gaa gta ata aga ctg gat aaa aag ttg agc gtt atg atg aag ctg      1836
Glu Glu Val Ile Arg Leu Asp Lys Lys Leu Ser Val Met Met Lys Leu
        575                 580                 585

ctc agc tcg gag gat gag gtt ctg gtg ggc aac gct gcc ctc tgc ctt      1884
Leu Ser Ser Glu Asp Glu Val Leu Val Gly Asn Ala Ala Leu Cys Leu
    590                 595                 600

ggt aac tgc atg gag gtg ccc aac gtt gcg tct tcc ctg cta aag acg      1932
Gly Asn Cys Met Glu Val Pro Asn Val Ala Ser Ser Leu Leu Lys Thr
605                 610                 615                 620

gac ctt ttg cag gtc ttg tta aag ctt gca ggc agt gac aca cag aag      1980
Asp Leu Leu Gln Val Leu Leu Lys Leu Ala Gly Ser Asp Thr Gln Lys
                625                 630                 635

acg gcc gtg cag gtg aac gca ggc att gct ctg ggg aag ctg tgc aca      2028
Thr Ala Val Gln Val Asn Ala Gly Ile Ala Leu Gly Lys Leu Cys Thr

640                 645                 650

gct gag ccc aga ttt gct gct caa ctg aga aag ctt cat ggc cta gaa      2076
Ala Glu Pro Arg Phe Ala Ala Gln Leu Arg Lys Leu His Gly Leu Glu
        655                 660                 665

att ctc aac tct acg atg aaa tac atc agt gat tct tgag agagacaggg      2126
Ile Leu Asn Ser Thr Met Lys Tyr Ile Ser Asp Ser
    670                 675                 680

tttgtgtgca tttggggaac acacagatgc acaccgtgtg ttgttcctat gctaataaag    2186

acctttgatg tatccacttc ag                                             2208

<210> 99
<211> 1686
<212> DNA
<213> Homo sapiens

<220>
<221> CDS
<222> (117)..(539)

<400> 99
ccggaccctt cttcagattg cacaggagga ggcggtagcg gaacctttct cctccgcctc      60

gttctcgctt ccacccgggc ccagttgggg acggacgcac tagaggcgac ctaaac        116
atg gag aca gcg ggc gct gca act ggg cag ccg gcc tct ggg ctg gag      164
Met Glu Thr Ala Gly Ala Ala Thr Gly Gln Pro Ala Ser Gly Leu Glu
1               5                   10                  15

gct ccg ggg tcc acg aat gac cgg ctt ttc ctg gtt aaa ggt gga att      212
Ala Pro Gly Ser Thr Asn Asp Arg Leu Phe Leu Val Lys Gly Gly Ile
            20                  25                  30

ttc ctt ggt acc gtt gct gca gcg gga atg cta gct gga ttt att aca      260
Phe Leu Gly Thr Val Ala Ala Ala Gly Met Leu Ala Gly Phe Ile Thr
        35                  40                  45

aca tta tca ttg gct aaa aag aaa agc cct gaa tgg ttc aat aag gga      308
Thr Leu Ser Leu Ala Lys Lys Lys Ser Pro Glu Trp Phe Asn Lys Gly
    50                  55                  60

agt atg gcc acg gct gca tta ccg gaa agc ggg tct tcc ctt gcc ttg      356
Ser Met Ala Thr Ala Ala Leu Pro Glu Ser Gly Ser Ser Leu Ala Leu
65                  70                  75                  80

cga gct ctg ggc tgg ggc tcc ctg tat gca tgg tgt ggg gtt ggt gtg      404
Arg Ala Leu Gly Trp Gly Ser Leu Tyr Ala Trp Cys Gly Val Gly Val
                85                  90                  95

att agc ttc gca gtc tgg aaa gct tta gga gtt cac agt atg aac gac      452
Ile Ser Phe Ala Val Trp Lys Ala Leu Gly Val His Ser Met Asn Asp
            100                 105                 110

ttt cga agt aaa atg caa tca ata ttt cca aca att ccc aag aac tcc      500
Phe Arg Ser Lys Met Gln Ser Ile Phe Pro Thr Ile Pro Lys Asn Ser

115                 120                 125

gaa tcg gct gtt gag tgg gag gaa aca ttg aaa tcc aaa tgagatgagc      549
Glu Ser Ala Val Glu Trp Glu Glu Thr Leu Lys Ser Lys
    130                 135                 140

atggatgaat ttcaaaatgc ttgttacaga aaggggtggc tctggagaca ccatgacagc      609

aaaaggactg ggactgattt ctcccaggaa catgggcaga ttgctgactg aaccagtgca      669

ctggatagca ttcagcctca tcacaggaga gtatgtgtgt gcgtgctggg ggagggtaaa      729

gttttcccac agttaagaag actatttaaa aatagtaatt acaggaataa cttccttatg      789

tggaggggac cccataggaa atgattctgt ttgtaacagt tgaagcaaat ttcatactaa      849

aaaagtttat aataaaagta tgataagaaa atatttataa aacagacccc ctaatagcaa      909

tagtactgca atgtgtctaa ttaaaggagt ttcaagagcc tttcttgtca atatatgaca      969

gaacttggaa gtcttattct gattagccag actacaaagt gacagtcacc tggtaagtcc     1029

tgtatggtag gaagtgggtg tggctctcgg gtcctaaatg tggtctgtgc tttacagcca     1089

tcacacacat gctagttatt tacctgatgc ccttgcttga aggccctata aattcttata     1149

cttaaaacaa aaagccagta atgagttgtg atcaccccaa aaggcacagt tgggctaaaa     1209

taatgatgat atcaggggta aacatatttt tttgagaagt tggtctggat ctctgaacac     1269

tgacaatttg tcattttgtc tcagttgtca aagtaaaaaa agcccagttg tcatccgtct     1329

tcaatatttt taaccagaaa gtcacctgag tacggagtcc caatataaag tatttgggac     1389

cacctcccta acaaagctgt tagggtaaat tctgtgtgca cgcccaactc cctcctgtct     1449

tccacagtct cctacaaaaa aaacagatgg agaataatgt ctacatgcaa ttgtttttac     1509

tgcaattgta ttttaaaata ttttcaagaa gagaaatact ttaatatgtc aatatgtcca     1569

gagaaaaaac ctgtagttaa atatcagttg tgcaatttct tcctacttgc ctaggctcta     1629

attagtcagc aatcaggttt aataaaggac atttgaaatt tctgtaaaaa aaaaaaa        1686

<210> 100
<211> 2602
<212> DNA
<213> Homo sapiens

<220>
<221> CDS
<222> (278)..(1615)

<400> 100
   ccggtgtgtg ggtctgtgac agggtccaac agggcctggt ccgtgtccgg tcccccaaat       60
   ctgtcgtccc tgcccccagg cattggcatc aacaaaagtc agaattcccg ggaacttgaa      120
   cagaggctgc taaattccca gtaattgctc ctttggcctt ctagggactg acttcaaaga 180
   aggaaggaaa gaatcaggca gtgcttcctc attctctttt aaaacccgct tcccgctgag 240
   tctgcaccca ggagaccaga gagcaccttg cccttcc atg gaa act cag gct gat 295
   Met Glu Thr Gln Ala Asp
   1 5
   ctc gta tct cag gaa cct cag gcc ctg ctt gac agt gct ctt cct tca 343
   Leu Val Ser Gln Glu Pro Gln Ala Leu Leu Asp Ser Ala Leu Pro Ser
   10 15 20
   aaa gtt cct gcc ttt tcc gac aag gac agc ctg ggg gat gag atg ttg 391
   Lys Val Pro Ala Phe Ser Asp Lys Asp Ser Leu Gly Asp Glu Met Leu
   25 30 35
   gcg gct gcg ctc cta aag gcc aag tcc cag gag ctg gta acc ttt gag 439
   Ala Ala Ala Leu Leu Lys Ala Lys Ser Gln Glu Leu Val Thr Phe Glu
   40 45 50
   gat gta gct gtg tac ttc atc cgg aag gag tgg aag cgt ttg gaa cct 487
   Asp Val Ala Val Tyr Phe Ile Arg Lys Glu Trp Lys Arg Leu Glu Pro
   55 60 65 70
   gct cag agg gac ctc tat aga gat gtg atg ctg gag aat tac ggg aat 535
   Ala Gln Arg Asp Leu Tyr Arg Asp Val Met Leu Glu Asn Tyr Gly Asn
   75 80 85
   gtg ttc tca ctg gat cgt gag acc agg act gaa aat gat caa gaa att 583
   Val Phe Ser Leu Asp Arg Glu Thr Arg Thr Glu Asn Asp Gln Glu Ile
   90 95 100
   tct gaa gac aca aga tca cat ggg gtc cta ctg gga aga ttt caa aag 631
   Ser Glu Asp Thr Arg Ser His Gly Val Leu Leu Gly Arg Phe Gln Lys
   105 110 115
   gat att tct cag ggt ctc aag ttt aaa gaa gcc tat gaa cga gaa gtc 679
   Asp Ile Ser Gln Gly Leu Lys Phe Lys Glu Ala Tyr Glu Arg Glu Val
   120 125 130
   agt ctg aaa agg ccg ctg ggg aac tcc cct gga gaa aga ctg aac agg 727
   Ser Leu Lys Arg Pro Leu Gly Asn Ser Pro Gly Glu Arg Leu Asn Arg
   135 140 145 150
   aaa atg cca gat ttt ggt caa gtg aca gtt gag gag aag cta acc ccc 775
   Lys Met Pro Asp Phe Gly Gln Val Thr Val Glu Glu Lys Leu Thr Pro
   155 160 165
   agg gga gag aga agc gag aaa tat aat gat ttt ggg aac agc ttc act 823
   Arg Gly Glu Arg Ser Glu Lys Tyr Asn Asp Phe Gly Asn Ser Phe Thr
   170 175 180
   gtg aat tcc aac ctt atc tca cat cag aga ctc ccc gtg gga gac aga 871
   Val Asn Ser Asn Leu Ile Ser His Gln Arg Leu Pro Val Gly Asp Arg
   185 190 195
   ccc cat aag tgt gat gaa tgt agc aag agc ttt aat cga act tca gac 919
   Pro His Lys Cys Asp Glu Cys Ser Lys Ser Phe Asn Arg Thr Ser Asp
   200 205 210
   ctt att caa cat cag aga atc cac act ggg gaa aag ccc tat gaa tgt 967
   Leu Ile Gln His Gln Arg Ile His Thr Gly Glu Lys Pro Tyr Glu Cys
   215 220 225 230
   aat gag tgt ggg aag gcc ttc agc cag agc tca cac ctt att cag cat 1015
   Asn Glu Cys Gly Lys Ala Phe Ser Gln Ser Ser His Leu Ile Gln His
   235 240 245
   cag aga atc cac act ggg gaa aaa cct tat gaa tgt agt gat tgt ggg 1063
   Gln Arg Ile His Thr Gly Glu Lys Pro Tyr Glu Cys Ser Asp Cys Gly
   250 255 260
   aaa acc ttc agc tgt agc tct gcc ctc att ctg cat cgg agg atc cac 1111
   Lys Thr Phe Ser Cys Ser Ser Ala Leu Ile Leu His Arg Arg Ile His
   265 270 275
   acg ggg gag aaa ccc tat gaa tgt aat gag tgt ggg aag acc ttc agc 1159
   Thr Gly Glu Lys Pro Tyr Glu Cys Asn Glu Cys Gly Lys Thr Phe Ser
   280 285 290
   tgg agc tcc acc ctc acc cac cat cag aga atc cac act ggt gag aaa 1207
   Trp Ser Ser Thr Leu Thr His His Gln Arg Ile His Thr Gly Glu Lys
   295 300 305 310
   ccc tac gcc tgc aat gaa tgt ggg aag gcc ttc agc agg agc tca acc 1255
   Pro Tyr Ala Cys Asn Glu Cys Gly Lys Ala Phe Ser Arg Ser Ser Thr
   315 320 325
   ctt att cac cat cag aga atc cac act gga gaa aaa ccc tat gaa tgt 1303
   Leu Ile His His Gln Arg Ile His Thr Gly Glu Lys Pro Tyr Glu Cys
   330 335 340
   aat gaa tgt ggg aaa gcc ttc agc cag agc tca cac ctc tat cag cac 1351
   Asn Glu Cys Gly Lys Ala Phe Ser Gln Ser Ser His Leu Tyr Gln His
   345 350 355
   cag aga atc cac act gga gag aag ccc tac gaa tgt atg gaa tgt gga 1399
   Gln Arg Ile His Thr Gly Glu Lys Pro Tyr Glu Cys Met Glu Cys Gly
   360 365 370
   gga aag ttt acc tac agt tca ggc ctt att cag cat caa aga atc cac 1447
   Gly Lys Phe Thr Tyr Ser Ser Gly Leu Ile Gln His Gln Arg Ile His
   375 380 385 390
   acc ggg gag aac ccc tat gaa tgt agt gag tgt ggg aaa gcc ttc agg 1495
   Thr Gly Glu Asn Pro Tyr Glu Cys Ser Glu Cys Gly Lys Ala Phe Arg
   395 400 405
   tac agc tcg gct ctt gtt cgc cat cag aga att cac act gga gag aag 1543
   Tyr Ser Ser Ala Leu Val Arg His Gln Arg Ile His Thr Gly Glu Lys
   410 415 420
   cct ttg aat ggg atc ggc atg agc aaa agc tcc ctc aga gtt acg acc 1591
   Pro Leu Asn Gly Ile Gly Met Ser Lys Ser Ser Leu Arg Val Thr Thr
   425 430 435
   gag tta aat atc aga gag tcc acg tgaaagagcc acacacccat tttcctcact 1645
   Glu Leu Asn Ile Arg Glu Ser Thr
   440 445
   ttccctgagt ctcaagagct cttgccttac cctataaatc tcaacagctt aggatgtgtc 1705
   cctttcaact cagacttttc attttagaga atggggcaga tggggcaaat cgttgaattt 1765
   tcccagaaat cacaccagcc ttagaaagcg tcaaggcaag tggatggcgt gctgggaata 1825
   gaaagcagct ctgggaccag ttaccccatt taggaaagga gtttgcacta aactgttttt 1885
   ctcacagcag aggaaccttt ccaaggtggg gatggaagca cagtcgggac agaattcgat 1945
   ggattccttt agttggagtc cgcgtagtca gcacagacag cagtggaaag gacgctctgg 2005
   gtcctgttat ttgctaggga gggtaaaggg agactatttc aaagctactg ttcctagtcc 2065
   agctttaagt ttcggtaaga aacatgctgt tttgtttcat gatttcgtta attatggaaa 2125
   tttggcattg agggattatt ttattgaggg tagaagagat tccagaatca tcatctgtga 2185
   tgatggtgtc ctttagggct cttggagcag ccagaccatg tttccaagag aaacctggtg 2245
   atattgccag cagaccccct gccatccccc ccagttgtcc tggggctgaa tgggcaaatc 2305
   tgtccaaaca gctagtaacc ggctgtgagg gagagggtca gaagcactta gcgttggcct 2365
   ctgattgctg tcctctcttg tcctcttccc actccaatga tgaaaatgat tttctctaaa 2425
   tgcctgggta aggatgcttt caaggagctc acttggcctg cttgccctgc cctctcacct 2485
   ctgacaccca gccccaggag ccagaccact cctgcctcca cctctgactc ttcagcagct 2545
   gaagattaat gcagagaaag agcaaagccc aaaagggaga aaaaaaaaac aaaaaaa 2602

<210> 101
<211> 2411
<212> DNA
<213> Homo sapiens

<220>
<221> CDS
<222> (17)..(1747)

<400> 101
tgcgtccggt gcggcg atg ctg acc ccg gcg ttc gac ctc agc cag gat 49
Met Leu Thr Pro Ala Phe Asp Leu Ser Gln Asp
1               5                   10

ccg gac ttc ctg act atc gcc atc cgc gtg ccc tac gcc cgg gtc tcc 97
Pro Asp Phe Leu Thr Ile Ala Ile Arg Val Pro Tyr Ala Arg Val Ser
            15                  20                  25

gag ttc gac gtc tac ttc gag ggg tct gac ttc aag ttc tac gcc aag 145
Glu Phe Asp Val Tyr Phe Glu Gly Ser Asp Phe Lys Phe Tyr Ala Lys
        30                  35                  40

cca tac ttt ctc aga tta acc ctt cct gga aga att gta gaa aat gga 193
Pro Tyr Phe Leu Arg Leu Thr Leu Pro Gly Arg Ile Val Glu Asn Gly

45                  50                  55

agt gag caa ggg tcc tat gat gca gat aaa gga att ttt acc att cgc          241
Ser Glu Gln Gly Ser Tyr Asp Ala Asp Lys Gly Ile Phe Thr Ile Arg
 60                  65                  70                  75

ctg ccc aaa gaa acc cct ggc cag cat ttt gag ggg ctg aac atg tta          289
Leu Pro Lys Glu Thr Pro Gly Gln His Phe Glu Gly Leu Asn Met Leu
                 80                  85                  90

act gct ctt ctg gca cca aga aaa tcc agg aca gca aaa cca ctt gtg          337
Thr Ala Leu Leu Ala Pro Arg Lys Ser Arg Thr Ala Lys Pro Leu Val
             95                 100                 105

gaa gaa ata ggt gct tct gag att cct gag gaa gta gtt gac gat gaa          385
Glu Glu Ile Gly Ala Ser Glu Ile Pro Glu Glu Val Val Asp Asp Glu
        110                 115                 120

gag ttt gat tgg gaa att gag cag aca ccc tgt gaa gag gta tca gaa          433
Glu Phe Asp Trp Glu Ile Glu Gln Thr Pro Cys Glu Glu Val Ser Glu
    125                 130                 135

agt gct ttg aat ccg cag tgc cac tat gga ttt gga aac tta cga tca          481
Ser Ala Leu Asn Pro Gln Cys His Tyr Gly Phe Gly Asn Leu Arg Ser
140                 145                 150                 155

gga gtg ttg caa cgg tta cag gat gaa ctg agt gat gtt att gat att          529
Gly Val Leu Gln Arg Leu Gln Asp Glu Leu Ser Asp Val Ile Asp Ile
                160                 165                 170

aag gat cca gat ttc acc cct gca gct gaa cga aga cag aag cgc ctg          577
Lys Asp Pro Asp Phe Thr Pro Ala Ala Glu Arg Arg Gln Lys Arg Leu
            175                 180                 185

gcc gct gag ctg gcc aag ttt gat cct gat cat tat cta gct gac ttt          625
Ala Ala Glu Leu Ala Lys Phe Asp Pro Asp His Tyr Leu Ala Asp Phe
        190                 195                 200

ttt gaa gat gag gcg att gaa cag att ttg aag tat aat cct tgg tgg          673
Phe Glu Asp Glu Ala Ile Glu Gln Ile Leu Lys Tyr Asn Pro Trp Trp
    205                 210                 215

act gac aaa tat tca aaa atg atg gcc ttt ttg gaa aag agt cag gaa          721
Thr Asp Lys Tyr Ser Lys Met Met Ala Phe Leu Glu Lys Ser Gln Glu
220                 225                 230                 235

caa gaa aat cat gct aca tta gtg tct ttt tct gaa gaa gag aag tat          769
Gln Glu Asn His Ala Thr Leu Val Ser Phe Ser Glu Glu Glu Lys Tyr
                240                 245                 250

cag cta cga aaa ttt gtc aat aaa tct tat ctg ctg gac aag aga gcc          817
Gln Leu Arg Lys Phe Val Asn Lys Ser Tyr Leu Leu Asp Lys Arg Ala
            255                 260                 265

tgt cgt caa gtg tgc tac agt ttg att gat atc ctt ctg gca tat tgc          865
Cys Arg Gln Val Cys Tyr Ser Leu Ile Asp Ile Leu Leu Ala Tyr Cys
        270                 275                 280

tat gaa acc cgt gtc act gaa gga gag aag aat gtt gaa tct gca tgg          913
Tyr Glu Thr Arg Val Thr Glu Gly Glu Lys Asn Val Glu Ser Ala Trp

285                 290                 295

aat atc agg aaa ctg agt cca aca cta tgc tgg ttt gag act tgg act        961
Asn Ile Arg Lys Leu Ser Pro Thr Leu Cys Trp Phe Glu Thr Trp Thr
300                 305                 310                 315

aac gtt cat gat atc atg gtg tct ttt gga aga agg gtg ttg tgt tac        1009
Asn Val His Asp Ile Met Val Ser Phe Gly Arg Arg Val Leu Cys Tyr
                320                 325                 330

cca ctc tat cgc cat ttc aag ctg gtg atg aag gcc tac agg gac act        1057
Pro Leu Tyr Arg His Phe Lys Leu Val Met Lys Ala Tyr Arg Asp Thr
            335                 340                 345

ata aag ata ttg caa ctg ggt aaa agt gca gtt tta aag tgt ctc ctg        1105
Ile Lys Ile Leu Gln Leu Gly Lys Ser Ala Val Leu Lys Cys Leu Leu
        350                 355                 360

gat att cac aaa att ttt cag gaa aat gac cca gcg tac ata ctg aat        1153
Asp Ile His Lys Ile Phe Gln Glu Asn Asp Pro Ala Tyr Ile Leu Asn
    365                 370                 375

gat ctc tac ata aca gac tac tgt gtg tac att cag aaa gtc aaa tcc        1201
Asp Leu Tyr Ile Thr Asp Tyr Cys Val Tyr Ile Gln Lys Val Lys Ser
380                 385                 390                 395

aaa aag ttg gca gct ctt gca gaa gcc tta aag gaa gtc tcc ctt aca        1249
Lys Lys Leu Ala Ala Leu Ala Glu Ala Leu Lys Glu Val Ser Leu Thr
                400                 405                 410

aag gcc cag ctg ggg tta gaa ctg gaa gaa cta gaa gca gca gca ctg        1297
Lys Ala Gln Leu Gly Leu Glu Leu Glu Glu Leu Glu Ala Ala Ala Leu
            415                 420                 425

ctt gtc cag gag gaa gaa act gca tta aaa gca gcc cat tca gtt tct        1345
Leu Val Gln Glu Glu Glu Thr Ala Leu Lys Ala Ala His Ser Val Ser
        430                 435                 440

ggg cag cag aca ctt tgc tcc agc tct gag gca agt gat tcg gag gac        1393
Gly Gln Gln Thr Leu Cys Ser Ser Ser Glu Ala Ser Asp Ser Glu Asp
    445                 450                 455

tca gac agc agc gtg tca tct gga aac gaa gac tca ggc tca gat tca        1441
Ser Asp Ser Ser Val Ser Ser Gly Asn Glu Asp Ser Gly Ser Asp Ser
460                 465                 470                 475

gaa caa gat gaa ctc aaa gat agt cca tct gag aca gtc agt tct ttg        1489
Glu Gln Asp Glu Leu Lys Asp Ser Pro Ser Glu Thr Val Ser Ser Leu
                480                 485                 490

caa ggt ccc ttt ctt gaa gaa agc agt gcc ttt ctt att gtt gat ggt        1537
Gln Gly Pro Phe Leu Glu Glu Ser Ser Ala Phe Leu Ile Val Asp Gly
            495                 500                 505

gga gta cgc aga aac aca gcc atc cag gag tct gat gcc agt cag gga        1585
Gly Val Arg Arg Asn Thr Ala Ile Gln Glu Ser Asp Ala Ser Gln Gly
        510                 515                 520

aag cca ctt gcc tct tcc tgg cct ctt gga gtg tct ggg cct ctg ata        1633
Lys Pro Leu Ala Ser Ser Trp Pro Leu Gly Val Ser Gly Pro Leu Ile

525                 530                 535

gag gag ctt ggg gaa caa ctg aag act aca gtt cag gtt tct gaa ccc     1681
Glu Glu Leu Gly Glu Gln Leu Lys Thr Thr Val Gln Val Ser Glu Pro
540                 545                 550                 555

aag ggc acc act gct gta aac cgc agc aat att cag gag aga gac ggc     1729
Lys Gly Thr Thr Ala Val Asn Arg Ser Asn Ile Gln Glu Arg Asp Gly
                560                 565                 570

tgt cag aca cca aat aat tgactc ttaggtggtt ttattcattg ttgagaaata     1783
Cys Gln Thr Pro Asn Asn
            575

tggtagattg ggtttcattt accgaatgag aattcttcat tttcactttg taatttttct     1843

tagtatatag tcagcccact gtatttgtgt gttccacatc tgtggattca accaactgca     1903

gatcaaaaat attgaagaaa aaatcgcatc tgtaccaaac atgtacagac ttttttcttg     1963

ttattattct ctaaataata cagtatgaca actatttcca cagcatttac attgtattgg     2023

gtaatataag taatctagtg atgatttaaa ctgtgcagga ggatgtgggt aggttatatg     2083

caaatactgc accatttttt atcaggggct tgagcatctg aggattttag tatcctcagg     2143

agtcctggaa ccaacccccc acagatacgg ggacaacttt atgacattgt ttttcaacca     2203

atgaatgttt ataccttttg ttttccttgc cgcgactgtg aagataaagt tcaaaagtat     2263

ttttaccaaa gtgtagctaa tatttcaagc tgaaaataat agttctactg cccgtgtctc     2323

cagaatgtag agcccatcaa tatttttatt ttaggaggtg tacttgacac ccaataaact     2383

gcacgtatca aaagtgaaaa aaaaaaaa                                        2411

<210> 102
<211> 2206
<212> DNA
<213> Homo sapiens

<220>
<221> CDS
<222> (200)..(1612)

<400> 102
caggacttcc gcacgagaga agagagattt aaataggctt aaaaagttag gtgacagcag       60

caaaaattca gactgttaat ctgtcagctc taacactgat gcagatacca ctcaggaaaa      120

aaacaatgca acttctaacc gaaaatcttc agttggcgta aaaaagaata gcaagagcag      180

aacgttaacg aggcaatct atg tca aga att cca gct tct tcc aac tct acc      232
                    Met Ser Arg Ile Pro Ala Ser Ser Asn Ser Thr
                    1               5                   10

tca tct aag cta act cat ata aat aat tcc agg gta cca aag aaa ctg      280

Ser Ser Lys Leu Thr His Ile Asn Asn Ser Arg Val Pro Lys Lys Leu
            15                  20                  25

aag aag cct gca aag cct tta ctt tca aag ata aaa ttg aga aat cat 328
Lys Lys Pro Ala Lys Pro Leu Leu Ser Lys Ile Lys Leu Arg Asn His
        30                  35                  40

tgc aag cgg ctg gag caa aag aat gct tca aga aaa ctc gaa atg gga 376
Cys Lys Arg Leu Glu Gln Lys Asn Ala Ser Arg Lys Leu Glu Met Gly
    45                  50                  55

aac tta gta ctg aaa gag cct aaa gta gtt ctg tat aaa aat ttg ccc 424
Asn Leu Val Leu Lys Glu Pro Lys Val Val Leu Tyr Lys Asn Leu Pro
60                  65                  70                  75

att aaa aaa gat aag gag cca gag gga cca gcc caa gcc gca gtt gcc 472
Ile Lys Lys Asp Lys Glu Pro Glu Gly Pro Ala Gln Ala Ala Val Ala
                80                  85                  90

agc ggg tgc ttg act aga cac gcg gcg aga gaa cac aga cag aat cct 520
Ser Gly Cys Leu Thr Arg His Ala Ala Arg Glu His Arg Gln Asn Pro
            95                  100                 105

gtg aga ggt gct cat tcg cag ggg gag agc tcg ccc tgc acc tac ata 568
Val Arg Gly Ala His Ser Gln Gly Glu Ser Ser Pro Cys Thr Tyr Ile
        110                 115                 120

act cgg cgg tca gtg agg aca aga aca aat ctg aag gag gcc tct gac 616
Thr Arg Arg Ser Val Arg Thr Arg Thr Asn Leu Lys Glu Ala Ser Asp
    125                 130                 135

atc aag ctt gaa cca aat acg ttg aat ggc tat aaa agc agt gtg acg 664
Ile Lys Leu Glu Pro Asn Thr Leu Asn Gly Tyr Lys Ser Ser Val Thr
140                 145                 150                 155

gaa cct tgc ccc gac agt ggt gaa cag ctg cag cca gct cct gtg ctg 712
Glu Pro Cys Pro Asp Ser Gly Glu Gln Leu Gln Pro Ala Pro Val Leu
                160                 165                 170

cag gag gaa gaa ctg gct cat gag act gca caa aaa ggg gag gca aag 760
Gln Glu Glu Glu Leu Ala His Glu Thr Ala Gln Lys Gly Glu Ala Lys
            175                 180                 185

tgt cat aag agt gac aca ggc atg tcc aaa aag aag tca cga caa gga 808
Cys His Lys Ser Asp Thr Gly Met Ser Lys Lys Lys Ser Arg Gln Gly
        190                 195                 200

aaa ctt gtg aaa cag ttt gca aaa ata gag gaa tct act cca gtg cac 856
Lys Leu Val Lys Gln Phe Ala Lys Ile Glu Glu Ser Thr Pro Val His
    205                 210                 215

gat tct cct gga aaa gac gac gcg gta cca gat ttg atg ggt ccc cat 904
Asp Ser Pro Gly Lys Asp Asp Ala Val Pro Asp Leu Met Gly Pro His
220                 225                 230                 235

tct gac cag ggt gag cac agt ggc act gtg ggc gtg cct gtg agc tac 952
Ser Asp Gln Gly Glu His Ser Gly Thr Val Gly Val Pro Val Ser Tyr
                240                 245                 250

aca gac tgt gct cct tca ccc gtc ggt tgt tca gtt gtg aca tca gat 1000

Thr Asp Cys Ala Pro Ser Pro Val Gly Cys Ser Val Val Thr Ser Asp
            255                 260                 265

agc ttc aaa aca aaa gac agc ttt aga act gca aaa agt aaa aag aag 1048
Ser Phe Lys Thr Lys Asp Ser Phe Arg Thr Ala Lys Ser Lys Lys Lys
        270                 275                 280

agg cga atc aca agg tat gat gca cag tta atc cta gaa aat aac tct 1096
Arg Arg Ile Thr Arg Tyr Asp Ala Gln Leu Ile Leu Glu Asn Asn Ser
    285                 290                 295

ggg att ccc aaa ttg act ctt cgt agg cgt cat gat agc agc agc aaa 1144
Gly Ile Pro Lys Leu Thr Leu Arg Arg Arg His Asp Ser Ser Ser Lys
300                 305                 310                 315

aca aat gac caa gag aat gat gga atg aac tct tcc aaa ata agc atc 1192
Thr Asn Asp Gln Glu Asn Asp Gly Met Asn Ser Ser Lys Ile Ser Ile
                320                 325                 330

aag tta agc aaa gac cat gac aac gat aac aat ctc tat gta gca aag 1240
Lys Leu Ser Lys Asp His Asp Asn Asp Asn Asn Leu Tyr Val Ala Lys
            335                 340                 345

ctt aat aat gga ttt aac tca gga tca ggc agt agt tct aca aaa tta 1288
.Leu Asn Asn Gly Phe Asn Ser Gly Ser Gly Ser Ser Ser Thr Lys Leu
350 355 360

aaa atc cag cta aaa cga gat gag gaa aat agg ggg tct tat aca gag 1336
Lys Ile Gln Leu Lys Arg Asp Glu Glu Asn Arg Gly Ser Tyr Thr Glu
365 370 375

ggg ctt cat gaa aat ggg gtg tgc tgc agt gat cct ctt tct ctc ttg 1384
Gly Leu His Glu Asn Gly Val Cys Cys Ser Asp Pro Leu Ser Leu Leu
380 385 390 395

gag tct cga atg gag gtg gat gac tat agt cag tat gag gaa gaa agt 1432
Glu Ser Arg Met Glu Val Asp Asp Tyr Ser Gln Tyr Glu Glu Glu Ser
400 405 410

aca gat gat tcc tcc tct tct gag ggc gat gaa gag gag gat gac tat 1480
Thr Asp Asp Ser Ser Ser Ser Glu Gly Asp Glu Glu Glu Asp Asp Tyr
415 420 425

gat gat gac ttt gaa gac gat ttt att cct ctt cct cca gct aag cgc 1528
Asp Asp Asp Phe Glu Asp Asp Phe Ile Pro Leu Pro Pro Ala Lys Arg
430 435 440

ttg agg tta ata gtt gga aaa gac tct ata gat att gac att tct tca 1576
Leu Arg Leu Ile Val Gly Lys Asp Ser Ile Asp Ile Asp Ile Ser Ser
445 450 455

agg aga aga gaa gat cag tct tta agg ctt aat gcc taag ctcttggtct 1626
Arg Arg Arg Glu Asp Gln Ser Leu Arg Leu Asn Ala
460 465 470

taacttgacc tgggataact actttaaaga aataaaaaat tccagtcaat tattcctcaa 1686

ctgaaagttt agtggcagca cttctattgt cccttcactt atcagcatac tattgtagaa 1746

agtgtacagc atactgactc aattcttaag tctgatttgt gcaaattttt atcgtacttt 1806

ttaaatagcc ttcttacgtg caattctgag ttagaggtaa agccctgttg taaaataaag 1866

gctcaagcaa aattgtacag tgatagcaac tttccacaca ggacgttgaa aacagtaatg 1926

tggctacaca gtttttttaa ctgtaagagc atcagctggc tctttaatat atgactaaac 1986

aataatttaa aacaaatcat agtagcagca tattaagggt ttctagtatg ctaatatcac 2046

cagcaatgat ctttggcttt ttgatttatt tgctagatgt ttcccccttg gagttttgtc 2106

agtttcacac tgtttgctgg cccaggtgta ctgtttgtgg cctttgttaa tatcgcaaac 2166

cattggttgg gagtcagatt ggtttcttaa aaaaaaaaaa 2206

<210> 103
<211> 1300
<212> DNA
<213> Homo sapiens

<220>
<221> CDS
<222> (165)..(662)

<400> 103
gttttcaggc tcttggcggt gaaggtcaga gtgcagacct gagaccactg ctcaccgact 60

tcagactcca gtttatctgt gccccagctt cctcacttag tctcagaaga cttaggctga 120

ggcctcagaa ggagatctcc atcctttgtc cagagcagaa cagg atg gcc atg tcc 176
Met Ala Met Ser
1

cag gaa tca ttg acc ttc aag gac gtg ttt gtg gac ttc acc ctg gag 224
Gln Glu Ser Leu Thr Phe Lys Asp Val Phe Val Asp Phe Thr Leu Glu
5                   10                  15                  20

gag tgg cag caa ctg gac tct gcc cag aag aac ctc tac agg gat gtc 272
Glu Trp Gln Gln Leu Asp Ser Ala Gln Lys Asn Leu Tyr Arg Asp Val
                25                  30                  35

atg ctt gag aac tac agc cac ctg gtg tcc gtg ggg tat cta gtt gcg 320
Met Leu Glu Asn Tyr Ser His Leu Val Ser Val Gly Tyr Leu Val Ala
            40                  45                  50

aag cct gat gtg atc ttc agg ttg gga cca ggt gga gag tcc tgg atg 368
Lys Pro Asp Val Ile Phe Arg Leu Gly Pro Gly Gly Glu Ser Trp Met
        55                  60                  65

gca gat ggg ggg acc ccg gta cgg acc tgt gca gaa gtc tgg caa gtt 416
Ala Asp Gly Gly Thr Pro Val Arg Thr Cys Ala Glu Val Trp Gln Val
    70                  75                  80

gat gag cag ata gat cac tac aag gaa agc caa gac aaa ctt cct tgg 464
Asp Glu Gln Ile Asp His Tyr Lys Glu Ser Gln Asp Lys Leu Pro Trp
85                  90                  95                  100

caa gct gca ttc ata ggc aag gaa aca ctg aag gat gaa agc ggt caa      512
Gln Ala Ala Phe Ile Gly Lys Glu Thr Leu Lys Asp Glu Ser Gly Gln
                105                 110                 115

gaa tcc aga aca tgt aga aaa agc att tat ctg agc aca gaa ttt gat      560
Glu Ser Arg Thr Cys Arg Lys Ser Ile Tyr Leu Ser Thr Glu Phe Asp
            120                 125                 130

tct gta agg caa aga ctc cct aaa tat tat tcg tgg gaa aag gca ttc      608
Ser Val Arg Gln Arg Leu Pro Lys Tyr Tyr Ser Trp Glu Lys Ala Phe
        135                 140                 145

aaa aca tca ttt aaa ctt tct tgg tca aaa tgg aag cta tgt aag aaa      656
Lys Thr Ser Phe Lys Leu Ser Trp Ser Lys Trp Lys Leu Cys Lys Lys
    150                 155                 160

gaa aga tgatggatgt aaagcatatt ggaaagtatg cttccattat aatcttcata      712
Glu Arg
165

aagctcaacc tgcagagaga ttttttgacc ctaatcaacg agggaaagcc ctccaccaaa      772

agcaagccct tagaaaaagt cagagaagtc aaactgggga gaaactctac aaatgtactg      832

aatgtggaaa agtgtttatc cagaaagcaa acttagttgt acatcaaaga actcacaccg      892

gagagaaacc ttatgaatgc tgcgaatgtg caaaagcctt cagccagaag tcaaccctca      952

tagcacacca gagaactcac acaggggaga agccctatga atgcagtgaa tgtggaaaaa     1012

cctttatcca gaagtcaact ctgattaaac ataagaaaat tcatatggag agagacccta     1072

taaatgcagt gtctgtgaga aagccttcag taggaagtca actctcatta aacatcagat     1132

aattcatatg ggagaaacct tatgaatgta ataaatgtgg gaaatctttt agtgttaaat     1192

caactctcat tgtatgtcac agaacataaa tgcataagtt gcatgctatt gtgaatagtg     1252

tgatgaaatt ttgcactgtc ctgctctttc ctgctcagga tgcaaatc                 1300

<210> 104
<211> 4004
<212> DNA
<213> Homo sapiens

<220>
<221> CDS
<222> (1051)..(3174)

<220>
<221> misc_feature
<222> (1)...(4004)
<223> n = a,t,c or g

<400> 104
tcggcgctcc cgggtcgacc cacgcgtccg gtgagtactc tgttttgaaa catatgcttg       60

aactataatg tgctttgttt tagttttaaa gttaaaaaaa ggaaccaatg atttttcttt 120

ttagtcaaaa ctctaacctt tacagacgtt ttctgttaat cttcaggtca caattaagat 180

attattactt cattgactgt gtatgggggg gggtgacatt ttaatctgga ctccaaatct 240

aaaaatgtac atgttcgctt ccattaccag gtacgatttg atagtgttgg tggcctgtct 300

aatcatatag cagctctaaa agagatggtg gtgtttccat tactttatcc agaagtcttt 360

gaaaaattta aaattcaacc cccaagaggt tgtttgtttt atgggccacc tggaactgga 420

aagactctgg ttgccagagc acttgccaat gagtgcagtc aaggggataa aagagtagca 480

tttttcatga ggaaaggtgc tgattgtcta agtaaatggg taggagaatc tgaaagacag 540

ctacgattgc tgtttgatca ggcctatcag atgcgcccat caattatttt ttttgacgaa 600

attgatggtc tggctccagt acggtcaagc aggcaagatc agattcacag ttctattgtt 660

tccaccctgc tagctcttat ggatggattg gacagcagag gggaaattgt ggtcattggt 720

gctacgaaca ggctagattc tatagatcct gctttacgaa ggcctggtcg ctttgataga 780

gaattcctct ttagcctgcc tgataaagag gcttgaaaag agattctaaa gattcacacc 840

agggattgga atcccaaacc actggacaca tttttagaag agctagcaga aaactgtgtt 900

ggatactgtg gagcagatat taaatcaata tgtgctgaag ctgctttatg tgctttacga 960

cgacgctacc cacagatcta taccactagt gagaaactgc agttggatct ctcttcaatt 1020

aatatctcag ctaaggattt cgaggtagct atg caa aag atg ata cca gcc 1071
Met Gln Lys Met Ile Pro Ala
1               5

tcc caa aga gct gtg aca tca cct ggg cag gca ctg tcc acc gtt gtg 1119
Ser Gln Arg Ala Val Thr Ser Pro Gly Gln Ala Leu Ser Thr Val Val
        10                  15                  20

aaa cca ctc ctg caa aac act gtt gac aag att tta gaa gcc ctg cag 1167
Lys Pro Leu Leu Gln Asn Thr Val Asp Lys Ile Leu Glu Ala Leu Gln
    25                  30                  35

aga gta ttt cca cat gca gaa ttc aga aca aat aaa aca tta gac tca 1215
Arg Val Phe Pro His Ala Glu Phe Arg Thr Asn Lys Thr Leu Asp Ser
40                  45                  50                  55

gat att tct tgt cct ctg cta gaa agt gac ttg gct tac agt gat gat 1263
Asp Ile Ser Cys Pro Leu Leu Glu Ser Asp Leu Ala Tyr Ser Asp Asp
                60                  65                  70

gat gtt cca tca gtt tat gaa aat gga ctt tct cag aaa tct tct cat 1311
Asp Val Pro Ser Val Tyr Glu Asn Gly Leu Ser Gln Lys Ser Ser His
            75                  80                  85

aag gca aaa gac aat ttt aat ttt ctt cat ttg aat aga aat gct tgt 1359
Lys Ala Lys Asp Asn Phe Asn Phe Leu His Leu Asn Arg Asn Ala Cys
        90                  95                  100

tac caa cct atg tct ttt cga cca aga ata ttg ata gta gga gaa cca 1407
Tyr Gln Pro Met Ser Phe Arg Pro Arg Ile Leu Ile Val Gly Glu Pro
    105                 110                 115

gga ttt ggg caa ggt tct cac ttg gca cca gct gtc att cat gct ttg 1455
Gly Phe Gly Gln Gly Ser His Leu Ala Pro Ala Val Ile His Ala Leu
120                 125                 130                 135

gaa aag ttt act gta tat aca tta gac att cct gtt ctt ttt gga gtt 1503
Glu Lys Phe Thr Val Tyr Thr Leu Asp Ile Pro Val Leu Phe Gly Val
                140                 145                 150

agt act aca tcc cct gaa gaa aca tgt gcc cag gtg att cgt gaa gct 1551
Ser Thr Thr Ser Pro Glu Glu Thr Cys Ala Gln Val Ile Arg Glu Ala
            155                 160                 165

aag aga aca gca cca agt ata gtg tat gtt cct cat atc cac gtg tgg 1599
Lys Arg Thr Ala Pro Ser Ile Val Tyr Val Pro His Ile His Val Trp
        170                 175                 180

tgg gaa ata gtt gga ccg aca ctt aaa gcc aca ttt acc aca tta tta 1647
Trp Glu Ile Val Gly Pro Thr Leu Lys Ala Thr Phe Thr Thr Leu Leu
    185                 190                 195

cag aat att cct tca ttt gct cca gtt tta cta ctt gca act tct gac 1695
Gln Asn Ile Pro Ser Phe Ala Pro Val Leu Leu Leu Ala Thr Ser Asp
200                 205                 210                 215

aaa ccc cat tcc gct ttg cca gaa gag gtg caa gaa ttg ttt atc cgt 1743
Lys Pro His Ser Ala Leu Pro Glu Glu Val Gln Glu Leu Phe Ile Arg
                220                 225                 230

gat tat gga gag att ttt aat gtc cag tta ccg gat aaa gaa gaa cgg 1791
Asp Tyr Gly Glu Ile Phe Asn Val Gln Leu Pro Asp Lys Glu Glu Arg
            235                 240                 245

aca aaa ttt ttt gaa gat tta att cta aaa caa gct gct aag cct cct 1839
Thr Lys Phe Phe Glu Asp Leu Ile Leu Lys Gln Ala Ala Lys Pro Pro
        250                 255                 260

ata tca aaa aag aaa gca gtt ttg cag gct ttg gag gta ctc cca gta 1887
Ile Ser Lys Lys Lys Ala Val Leu Gln Ala Leu Glu Val Leu Pro Val
    265                 270                 275

gca cca cca cct gag cca aga tca ctg aca gca gaa gaa gtg aaa cga 1935
Ala Pro Pro Pro Glu Pro Arg Ser Leu Thr Ala Glu Glu Val Lys Arg
280                 285                 290                 295

cta gaa gaa caa gaa gaa gat aca ttt aga gaa ctg agg att ttc tta 1983
Leu Glu Glu Gln Glu Glu Asp Thr Phe Arg Glu Leu Arg Ile Phe Leu
                300                 305                 310

aga aat gtt aca cat agg ctt gct att gac aag cga ttc cga gtg ttt 2031
Arg Asn Val Thr His Arg Leu Ala Ile Asp Lys Arg Phe Arg Val Phe
            315                 320                 325

act aag cct gtt gac cct gat gag gtt cct gat tat gtc act gta ata 2079
Thr Lys Pro Val Asp Pro Asp Glu Val Pro Asp Tyr Val Thr Val Ile
        330                 335                 340

aag caa cca atg gac ctt tca tct gta atc agt aaa att gat cta cac 2127
Lys Gln Pro Met Asp Leu Ser Ser Val Ile Ser Lys Ile Asp Leu His
    345                 350                 355

aag tat ctg act gtg aaa gac tat ttg aga gat att gat cta atc tgt 2175
Lys Tyr Leu Thr Val Lys Asp Tyr Leu Arg Asp Ile Asp Leu Ile Cys
360                 365                 370                 375

agt aat gcc tta gaa tac aat cca gat aga gat cct gga gat cgt ctt 2223
Ser Asn Ala Leu Glu Tyr Asn Pro Asp Arg Asp Pro Gly Asp Arg Leu
                380                 385                 390

att agg cat aga gcc tgt gct tta aga gat act gcc tat gcc ata att 2271
Ile Arg His Arg Ala Cys Ala Leu Arg Asp Thr Ala Tyr Ala Ile Ile
            395                 400                 405

aaa gaa gaa ctt gat gaa gac ttt gag cag ctc tgt gaa gaa att cag 2319
Lys Glu Glu Leu Asp Glu Asp Phe Glu Gln Leu Cys Glu Glu Ile Gln
        410                 415                 420

gaa tct aga aag aaa aga ggt tgt agc tcc tcc aaa tat gcc ccg tct 2367
Glu Ser Arg Lys Lys Arg Gly Cys Ser Ser Ser Lys Tyr Ala Pro Ser
    425                 430                 435

tac tac cat gtg atg cca aag caa aat tcc act ctt gtt ggt gat aaa 2415
Tyr Tyr His Val Met Pro Lys Gln Asn Ser Thr Leu Val Gly Asp Lys
440                 445                 450                 455

aga tca gac cca gag cag aat gaa aag cta aag aca ccg agt act cct 2463
Arg Ser Asp Pro Glu Gln Asn Glu Lys Leu Lys Thr Pro Ser Thr Pro
                460                 465                 470

gtg gct tgc agc act cct gct cag ttg aag agg aaa att cgc aaa aag 2511
Val Ala Cys Ser Thr Pro Ala Gln Leu Lys Arg Lys Ile Arg Lys Lys
            475                 480                 485

tca aac tgg tac tta ggc acc ata aaa aag cga agg aag att tca cag 2559
Ser Asn Trp Tyr Leu Gly Thr Ile Lys Lys Arg Arg Lys Ile Ser Gln
        490                 495                 500

gca aag gat gat agc cag aat gcc ata gat cac aaa att gag agt gat 2607
Ala Lys Asp Asp Ser Gln Asn Ala Ile Asp His Lys Ile Glu Ser Asp
    505                 510                 515

aca gag gaa act caa gac aca agt gta gat cat aat gag acc gga aac 2655
Thr Glu Glu Thr Gln Asp Thr Ser Val Asp His Asn Glu Thr Gly Asn
520                 525                 530                 535

aca gga gag tct tcg gtg gaa gaa aat gaa aaa cag caa aat gcc tct 2703
Thr Gly Glu Ser Ser Val Glu Glu Asn Glu Lys Gln Gln Asn Ala Ser
                540                 545                 550

gaa agc aaa ctg gaa ttg aga aat aat tca aat act tgt aat ata gag 2751
Glu Ser Lys Leu Glu Leu Arg Asn Asn Ser Asn Thr Cys Asn Ile Glu
            555                 560                 565

aat gag ctt gaa gac tct agg aag act aca gca tgt aca gaa ttg aga 2799
Asn Glu Leu Glu Asp Ser Arg Lys Thr Thr Ala Cys Thr Glu Leu Arg
        570                 575                 580

gac aag att gct tgt aat gga gat gct tct agc tct cag ata ata cat 2847
Asp Lys Ile Ala Cys Asn Gly Asp Ala Ser Ser Ser Gln Ile Ile His
    585                 590                 595

att tct gat gaa aat gaa gga aaa gaa atg tgt gtt ctg cga atg act 2895
Ile Ser Asp Glu Asn Glu Gly Lys Glu Met Cys Val Leu Arg Met Thr
600                 605                 610                 615

cga gct aga cgt tcc cag gta gaa cag cag cag ctc atc act gtt gaa 2943
Arg Ala Arg Arg Ser Gln Val Glu Gln Gln Gln Leu Ile Thr Val Glu
                620                 625                 630

aag gct ttg gca att ctt tct cag cct aca ccc tca ctt gtt gtg gat 2991
Lys Ala Leu Ala Ile Leu Ser Gln Pro Thr Pro Ser Leu Val Val Asp
            635                 640                 645

cat gag cga tta aaa aat ctt ttg aag act gtt gtt aaa aaa agt caa 3039
His Glu Arg Leu Lys Asn Leu Leu Lys Thr Val Val Lys Lys Ser Gln
        650                 655                 660

aac tac aac ata ttt cag ttg gaa aat ttg tat gca gta atc agc caa 3087
Asn Tyr Asn Ile Phe Gln Leu Glu Asn Leu Tyr Ala Val Ile Ser Gln
    665                 670                 675

tgt att tat cgg cat cgc aag gac cat gat aaa aca tca ctt att cag 3135
Cys Ile Tyr Arg His Arg Lys Asp His Asp Lys Thr Ser Leu Ile Gln
680                 685                 690                 695

aaa atg gag caa gag gta gaa aac ttc agt tgt tcc aga tgatgatgtc 3184
Lys Met Glu Gln Glu Val Glu Asn Phe Ser Cys Ser Arg
                700                 705

atggtatcga gtattcttta tattcagttc ctatttaagt catttttgtc atgtccgcct 3244

aattgatgta gtatgaaacc ctgcatcttt aaggaaaaga ttaaaatagt aaaataaagt 3304

attttaactt cctgactatt atggtacata taaagataaa tgtcatgtgt aagatactga 3364

taaatatgga acttgctaga caagaccctg tagtaatagt ataatagttg agtttgggca 3424

actctaataa agttattttg gtaacaatgt ttatggcact ttagaaataa ttagcagcgt 3484

taaattttgt ntgtattaag cacttttaat tttatccttc ctaaaaatag tttattgtat 3544

ctgacaagaa acttacttaa ccattgtgtc cttcccatct tttttgtcat cttttttttc 3604

ttcaaatgcc ctcctcccat ctgccttgag attcccttgt cttcacttaa aagccagagt 3664

gcaagtcatg atttgcggga gggctcttga accacttctg gctgcaccac aattctgtac 3724

ttgagtatca cagtcattgt ttttgagaca aacattttta taattctaat ttgggttaat 3784

aaagatttta aatatttctt ggtttacttt tgtaattata tacacaacaa atgtattaat 3844

aactaccttg ttaaacacct tttaatagca caaggttttt atatttgcaa gctgttgata 3904

tctttctaaa actgtttagg ttatagtcta ttgatacttt ttatatacaa ttttataaat 3964

ataaatatta taattttata ttaatggtaa aaaaaaaaaa 4004

<210> 105
<211> 2707
<212> DNA
<213> Homo sapiens

<220>
<221> CDS
<222> (161)..(1714)

<400> 105
   gtggttggtt accgcctttt gcactagcag tagcaaggaa gggggtgggg cgctctttct 60
   ttttctctta gaagagggtt tagcacaggt tttttcgttc tcacttccac accaccttac 120
   cgcctcccga ccccccctct ccccctcccc acctatcgtc atg acg gcc tct ccg 175
   Met Thr Ala Ser Pro
   1 5
   gat tac ttg gtg gtg ctt ttt ggg atc act gct ggg gcc acc ggg gcc 223
   Asp Tyr Leu Val Val Leu Phe Gly Ile Thr Ala Gly Ala Thr Gly Ala
   10 15 20
   aag cta ggc tcg gat gag aag gag ttg atc ctg ctg ttc tgg aaa gtc 271
   Lys Leu Gly Ser Asp Glu Lys Glu Leu Ile Leu Leu Phe Trp Lys Val
   25 30 35
   gtg gat ctg gcc aac aag aag gtg gga cag ttg cac gaa gtg cta gtt 319
   Val Asp Leu Ala Asn Lys Lys Val Gly Gln Leu His Glu Val Leu Val
   40 45 50
   aga ccg gat cag ttg gaa ctg acg gag gac tgc aaa gaa gaa act aaa 367
   Arg Pro Asp Gln Leu Glu Leu Thr Glu Asp Cys Lys Glu Glu Thr Lys
   55 60 65
   ata gac gtc gaa agc ctg tcc tcg gcg tcg cag ctg gac caa gcc ctc 415
   Ile Asp Val Glu Ser Leu Ser Ser Ala Ser Gln Leu Asp Gln Ala Leu
   70 75 80 85
   cga cag ttt aac cag tca gtg agc aat gaa ctg aat att gga gta ggg 463
   Arg Gln Phe Asn Gln Ser Val Ser Asn Glu Leu Asn Ile Gly Val Gly
   90 95 100
   act tcc ttc tgt ctc tgt act gat ggg cag ctt cat gtc agg caa atc 511
   Thr Ser Phe Cys Leu Cys Thr Asp Gly Gln Leu His Val Arg Gln Ile
   105 110 115
   ctg cat cct gag gct tcc aag aag aat gta cta tta cct gaa tgc ttc 559
   Leu His Pro Glu Ala Ser Lys Lys Asn Val Leu Leu Pro Glu Cys Phe
   120 125 130
   tat tcc ttt ttt gat ctt cga aaa gaa ttc aag aaa tgt tgc cct ggt 607
   Tyr Ser Phe Phe Asp Leu Arg Lys Glu Phe Lys Lys Cys Cys Pro Gly
   135 140 145
   tca cct gat att gac aaa ctg gac gtt gcc aca atg aca gag tat tta 655
   Ser Pro Asp Ile Asp Lys Leu Asp Val Ala Thr Met Thr Glu Tyr Leu
   150 155 160 165
   aat ttt gag aag agt agt tca gtc tct cga tat gga gcc tct caa gtt 703
   Asn Phe Glu Lys Ser Ser Ser Val Ser Arg Tyr Gly Ala Ser Gln Val
   170 175 180
   gaa gat atg ggg aat ata att tta gca atg att tca gag cct tat aat 751
   Glu Asp Met Gly Asn Ile Ile Leu Ala Met Ile Ser Glu Pro Tyr Asn
   185 190 195
   cac agg ttt tca gat cca gag aga gtg aat tac aag ttt gaa agt gga 799
   His Arg Phe Ser Asp Pro Glu Arg Val Asn Tyr Lys Phe Glu Ser Gly
   200 205 210
   act tgc agc aag atg gaa ctt att gat gat aac acc gta gtc agg gca 847
   Thr Cys Ser Lys Met Glu Leu Ile Asp Asp Asn Thr Val Val Arg Ala
   215 220 225
   cga ggt tta cca tgg cag tct tca gat caa gat att gca aga ttc ttc 895
   Arg Gly Leu Pro Trp Gln Ser Ser Asp Gln Asp Ile Ala Arg Phe Phe
   230 235 240 245
   aaa gga ctc aat att gcc aag gga ggt gca gca ctt tgt ctg aat gct 943
   Lys Gly Leu Asn Ile Ala Lys Gly Gly Ala Ala Leu Cys Leu Asn Ala
   250 255 260
   cag ggt cga agg aac gga gaa gct ctg gtt agg ttt gta agt gag gag 991
   Gln Gly Arg Arg Asn Gly Glu Ala Leu Val Arg Phe Val Ser Glu Glu
   265 270 275
   cac cga gac cta gca cta cag agg cac aaa cat cac atg ggg acc cgg 1039
   His Arg Asp Leu Ala Leu Gln Arg His Lys His His Met Gly Thr Arg
   280 285 290
   tat att gag gtt tac aaa gca aca ggt gaa gat ttc ctt aaa att gct 1087
   Tyr Ile Glu Val Tyr Lys Ala Thr Gly Glu Asp Phe Leu Lys Ile Ala
   295 300 305
   ggt ggt act tcc aat gag gta gcc cag ttt ctc tcc aag gaa aat caa 1135
   Gly Gly Thr Ser Asn Glu Val Ala Gln Phe Leu Ser Lys Glu Asn Gln
   310 315 320 325
   gtc att gtc cgc atg cgg ggg ctc cct ttc acg gcc aca gct gaa gaa 1183
   Val Ile Val Arg Met Arg Gly Leu Pro Phe Thr Ala Thr Ala Glu Glu
   330 335 340
   gtg gtg gcc ttc ttt gga cag cat tgc cct att act ggg gga aag gaa 1231
   Val Val Ala Phe Phe Gly Gln His Cys Pro Ile Thr Gly Gly Lys Glu
   345 350 355
   ggc atc ctc ttt gtc acc tac cca gat ggt agg cca aca ggg gac gct 1279
   Gly Ile Leu Phe Val Thr Tyr Pro Asp Gly Arg Pro Thr Gly Asp Ala
   360 365 370
   ttt gtc ctc ttt gcc tgt gag gaa tat gca cag aat gcg ttg agg aag 1327
   Phe Val Leu Phe Ala Cys Glu Glu Tyr Ala Gln Asn Ala Leu Arg Lys
   375 380 385
   cat aaa gac ttg ttg ggt aaa aga tac att gaa ctc ttc agg agc aca 1375
   His Lys Asp Leu Leu Gly Lys Arg Tyr Ile Glu Leu Phe Arg Ser Thr
   390 395 400 405
   gca gct gaa gtt cag cag gtg ctg aat cga ttc tcc tcg gcc cct ctc 1423
   Ala Ala Glu Val Gln Gln Val Leu Asn Arg Phe Ser Ser Ala Pro Leu
   410 415 420
   att cca ctt cca acc cct ccc att att cca gta cta cct cag caa ttt 1471
   Ile Pro Leu Pro Thr Pro Pro Ile Ile Pro Val Leu Pro Gln Gln Phe
   425 430 435
   gtg ccc cct aca aat gtt aga gac tgt ata cgc ctt cga ggt ctt ccc 1519
   Val Pro Pro Thr Asn Val Arg Asp Cys Ile Arg Leu Arg Gly Leu Pro
   440 445 450
   tat gca gcc aca att gag gac atc ctg gat ttc ctg ggg gag ttc gcc 1567
   Tyr Ala Ala Thr Ile Glu Asp Ile Leu Asp Phe Leu Gly Glu Phe Ala
   455 460 465
   aca gat att cgt act cat ggg gtt cac atg gtt ttg aat cac cag ggc 1615
   Thr Asp Ile Arg Thr His Gly Val His Met Val Leu Asn His Gln Gly
   470 475 480 485
   cgc cca tca gga gat gcc ttt atc cag atg aag tct gcg gac aga gca 1663
   Arg Pro Ser Gly Asp Ala Phe Ile Gln Met Lys Ser Ala Asp Arg Ala
   490 495 500
   ttt atg gct gca cag aag tgt cat aaa aaa aaa cat gaa gga cag ata 1711
   Phe Met Ala Ala Gln Lys Cys His Lys Lys Lys His Glu Gly Gln Ile
   505 510 515
   tgt tgaa gtctttcagt gttcagctga ggagatgaac tttgtgttaa tggggggcac 1768
   Cys
   tttaaatcga aatggcttat ccccaccgcc atgtaagtta ccatgcctgt ctcctccctc 1828
   ctacacattt ccagctcctg ctgcagttat tcctacagaa gctgccattt accagccctc 1888
   tgtgattttg aatccacgag cactgcagcc ctccacagcg tactacccag caggcactca 1948
   gctcttcatg aactacacag cgtactatcc cagcccccca ggttcgccta atagtcttgg 2008
   ctacttccct acagctgcta atcttagcgg tgtccctcca cagcctggca cggtggtcag 2068
   aatgcagggc ctggcctaca atactggagt taaggaaatt cttaacttct tccaaggtta 2128
   ccagtgtttg aaagatgtat ggtgatcttg aaacctccag acacaagaaa acttctagca 2188
   aattcagggg aagtttgtct acactcaggc tgcagtattt tcagcaaact tgattggaca 2248
   aacggggcct gtgccttatc ttttggtgga gtgaaaaagt ttgagctagt gaagccaaat 2308
   cgtaacttac agcaagcagc atgcagcata cctggctctt tgctgattgc aaataggcat 2368
   ttaaaatgtg aatttggaat cagatgtctc cattacttcc agttaaagtg gcatcatagg 2428
   tgtttcctaa gttttaagtc ttggataaaa actccaccag tgtctaccat ctccaccatg 2488
   aactctgtta aggaagcttc atttttgtat attcccgctc ttttctcttc atttccctgt 2548
   cttctgcata atcatgcctt cttgctaagt aattcaagca taagatcttg gaataataaa 2608
   atcacaatct taggagaaag aataaaattg ttattttccc agtctcttgg ccatgatgat 2668
   atcttatgat taaaaacaaa ttaaatttta aaacacctg 2707

<210> 106
<211> 711
<212> DNA
<213> Homo sapiens

<220>
<221> CDS
<222> (258)..(524)

<400> 106
ggaaaatctc attttactta tgataaaaac tgatgagtta ttatgtccta ttggccctgt 60

atttcccagg gtgggaacaa tgtctctgcc aaagcttagg ggctccctct cctaatgctt 120

cacatccgtg ttttctgttg ccctcacctt ctatactccc ctcttgcact gagaccccac 180

acccagcagc taatgttcac tgcccagagc aaggagttgt cagccttacc tctctgctcc 240

ttctgtagtg tcacacc atg tct gac cgg aag gca gtg atc aag aac gca 290
Met Ser Asp Arg Lys Ala Val Ile Lys Asn Ala
1               5                   10

gac atg tct gag gac atg caa cag gat gcc gtt gac tgc gcc acg cag 338
Asp Met Ser Glu Asp Met Gln Gln Asp Ala Val Asp Cys Ala Thr Gln
            15                  20                  25

gcc atg gag aag tac aat ata gag aag gac att gct gcc tat atc aag 386
Ala Met Glu Lys Tyr Asn Ile Glu Lys Asp Ile Ala Ala Tyr Ile Lys
        30                  35                  40

aag gaa ttt gac aag aaa tat aac cct acc tgg cat tgt atc gtg ggc 434
Lys Glu Phe Asp Lys Lys Tyr Asn Pro Thr Trp His Cys Ile Val Gly
    45                  50                  55

cga aat ttt ggc agc tac gtc aca cac gag aca aag cac ttc atc tat 482
Arg Asn Phe Gly Ser Tyr Val Thr His Glu Thr Lys His Phe Ile Tyr
60                  65                  70                  75

ttt tac ttg ggt caa gtt gca atc ctc ctc ttc aag tca ggc taggtgg 531
Phe Tyr Leu Gly Gln Val Ala Ile Leu Leu Phe Lys Ser Gly
                80                  85

ccatggtgaa ggtgtcagtg gcggcggcag cgatggcaag caggcggcgt tgctgggact 591

gttttgcact ggagccagca tcaggatgtc ctctcgaatg gctgtgctac tgcatggact 651

gtatactcga tttcatgtgt atgtcgcagt aaacaaaacc aaacctcaaa aaaaaaaaaa 711

<210> 107

<211> 1062
<212> DNA
<213> Homo sapiens

<220>
<221> CDS
<222> (654)..(935)

<400> 107
ctggtacaac aggtctctac aacgccaaga tctaactaag ctttaaaagg tcaagaagtt 60
ttatggctga caaaggactc gcgcaacgca gaaggccttt cccaccttaa gcttccgggg 120
atctgggaat tttaccccca ttctcttctg tttgtctgag tctcatctct ctgcaagcaa 180
gggctgaaat catttgtttt ggttgttttg agggagagag gcggggtggg ggggtgcaaa 240
tctgccagca gctcttacgt aaggcatgtt ttattgggga gggctgagct tttattttct 300
cctctccagt ggggttggct tttattgttt cttgtttggg tttggaatgg aaatatggat 360
agcagcataa agtactttta ttttgacaaa attcattttt ttcaacaatg gagacataga 420
tttgacccac aataacttct ccccctctct ttttactctg ctcaaaaagc atctctcctc 480
ccattaccca accttggtca taagtgtgcc tggctggttt gcagatattt gttctgcttt 540
gtaaaaattg gccattagtg catttattga gatgatctct aaagagctat gccctgacct 600
acccctgatt ctatgacatt ggggcccttc ttttgctgaa actgccttac gta atg 656
gtt tta ctc ctt gaa aga gat ttg acg gaa tcc att tta tgc caa gtg 704
ctg ccc tgc act gtt tct gca ata tgt ggt gta tgc tgt ggt gat ctt 752
gct ggg aat gat tat aag tgt gtg tgt ggt ggg gga gtg ggt att aca 800
tgc att gct gaa gag tca tcc tgg tgt tcc tca ttc ctc cca cct tcc 848
cgt ggt cat ttt aat tac ggg gca gtg tca ccg caa agg gag gaa act 896
caa agc cga aag caa aat tcc agg cct gat tct ggc ttt tgaggttcct 945
ggttcttgaa gccaggcctg acccgactct cagatggggt cagtcccgtc gctttgcaga 1005
ctgaccctgg aaatctacaa aatgcagatt ttcctgattt cctcttctct tgcccag 1062

<210> 108
<211> 901
<212> DNA
<213> Homo sapiens

<220>
<221> CDS
<222> (76)..(696)

<400> 108
tttcgtgctg gagcggctgt gcgggctgcg tagcggtgct gggtcgggcc gacgtgccac     60

ccacccggag ccggg atg cca gaa ggg ccg ttg gtg agg aaa ttt cac cat     111
                 Met Pro Glu Gly Pro Leu Val Arg Lys Phe His His
                 1               5                   10

ttg gtc tcc ccc ctt gtg ggt cag cag gtg gtc aag aca ggg ggc agc     159
Leu Val Ser Pro Leu Val Gly Gln Gln Val Val Lys Thr Gly Gly Ser
        15                  20                  25

agt aag aag cta cag ccc gcc agc ctg cag tct ctg tgg ctc cag gac     207
Ser Lys Lys Leu Gln Pro Ala Ser Leu Gln Ser Leu Trp Leu Gln Asp
    30                  35                  40

acc cag gtc cat gga aag aaa tta ttc ctt aga ttt gat cta gat gaa     255
Thr Gln Val His Gly Lys Lys Leu Phe Leu Arg Phe Asp Leu Asp Glu
45                  50                  55                  60

gaa atg ggg ccc cct ggc agc agc cca aca cca gag cct cca caa aaa     303
Glu Met Gly Pro Pro Gly Ser Ser Pro Thr Pro Glu Pro Pro Gln Lys
                65                  70                  75

gaa gtg cag aag gaa ggg gct gcg gac cca aag cag gtc ggg gag ccc     351
Glu Val Gln Lys Glu Gly Ala Ala Asp Pro Lys Gln Val Gly Glu Pro
            80                  85                  90

agc ggg cag aag acc ctt gat gga tcc tca cgg tct gca gag ctc gtc     399
Ser Gly Gln Lys Thr Leu Asp Gly Ser Ser Arg Ser Ala Glu Leu Val
        95                  100                 105

ccc cag ggc gag gat gat tct gag tat ttg gag aga gac gcc cct gca     447
Pro Gln Gly Glu Asp Asp Ser Glu Tyr Leu Glu Arg Asp Ala Pro Ala
    110                 115                 120

gga gat gct ggg agg tgg ctg cgt gtc agc ttt ggt ttg ttt ggc agc     495
Gly Asp Ala Gly Arg Trp Leu Arg Val Ser Phe Gly Leu Phe Gly Ser
125                 130                 135                 140

gtt tgg gtg aac gat ttc tcc aga gcc aag aaa gcc aac aag agg ggg     543
Val Trp Val Asn Asp Phe Ser Arg Ala Lys Lys Ala Asn Lys Arg Gly
                145                 150                 155

gac tgg agg gac cct tcc ccg agg ttg gtc ctg cac ttt ggt ggt ggt     591
Asp Trp Arg Asp Pro Ser Pro Arg Leu Val Leu His Phe Gly Gly Gly
            160                 165                 170

ggc ttc ctg gca ttt tat aat tgt cag ttg tct tgg agc tct tcc cca     639
Gly Phe Leu Ala Phe Tyr Asn Cys Gln Leu Ser Trp Ser Ser Ser Pro
        175                 180                 185

gtg gtc aca ccc acc tgt gac atc ctg tct gag aag ttc cat cga gga     687
Val Val Thr Pro Thr Cys Asp Ile Leu Ser Glu Lys Phe His Arg Gly
    190                 195                 200

caa gcc tta taagctc taggccaggc tcagcctgtc tgctatacac tgctggacca     743
Gln Ala Leu
205

gagatacttc tcagggctag ggaacatcat taagaatgaa gccttgtaca gagctgggat     803

ccatcccctt tctctcggtt cagtcctgag tgcctcgcgt cgggaggtcc tggtggatca     863

cgtggtggag ttcagtacag cctggctgca tggcaagt                             901

<210> 109
<211> 986
<212> DNA
<213> Homo sapiens

<220>
<221> CDS
<222> (100)..(936)

<400> 109
cccgaggctg gcgggttttg gcagtagctg tggctgcggc tgccgggcct ggggacgcgg      60

gcggcgaggc cgcgtcgcag cctcctcgtc tcgccggct atg gct gcg ctc ggc     114
                                          Met Ala Ala Leu Gly
                                          1               5

cgg ccc ttc agc ggc ctc cct ctg agc ggc ggc tcg gac ttc ctg cag     162
Arg Pro Phe Ser Gly Leu Pro Leu Ser Gly Gly Ser Asp Phe Leu Gln
                10                  15                  20

ccg ccg cag ccg gcc ttc ccc ggc cgg gcc ttc ccg ccg ggg gct gac     210
Pro Pro Gln Pro Ala Phe Pro Gly Arg Ala Phe Pro Pro Gly Ala Asp
            25                  30                  35

ggc gcc gag ttg gcc ccg cgg ccg gga cct cgc gca gtc cct agc agt     258
Gly Ala Glu Leu Ala Pro Arg Pro Gly Pro Arg Ala Val Pro Ser Ser
        40                  45                  50

ccc gct ggg agt gcg gcg cgc gga cgt gtt tct gtt cac tgt aaa aag     306
Pro Ala Gly Ser Ala Ala Arg Gly Arg Val Ser Val His Cys Lys Lys
    55                  60                  65

aaa cac aag cga gag gag gag gag gat gat gat tgt cca gta aga aag     354
Lys His Lys Arg Glu Glu Glu Glu Asp Asp Asp Cys Pro Val Arg Lys
70                  75                  80                  85

aaa agg ata act gaa gca gag ctc tgt gct ggt cct aat gac tgg att     402
Lys Arg Ile Thr Glu Ala Glu Leu Cys Ala Gly Pro Asn Asp Trp Ile
                90                  95                  100

ctt tgt gca cat cag gat gta gag ggg cat gga gta aat ccc agt gtt 450
Leu Cys Ala His Gln Asp Val Glu Gly His Gly Val Asn Pro Ser Val
            105                 110                 115

agt ggc ctt tcc ata cct ggg ata tta gat gtt att tgt gaa gaa atg 498
Ser Gly Leu Ser Ile Pro Gly Ile Leu Asp Val Ile Cys Glu Glu Met
        120                 125                 130

gat cag aca act gga gaa cca cag tgt gaa gtt gcc cga agg aag ctt 546
Asp Gln Thr Thr Gly Glu Pro Gln Cys Glu Val Ala Arg Arg Lys Leu
    135                 140                 145

cag gag att gag gac agg ata att gat gaa gat gaa gaa gtt gaa gct 594
Gln Glu Ile Glu Asp Arg Ile Ile Asp Glu Asp Glu Glu Val Glu Ala
150                 155                 160                 165

gac aga aat gtt aac cat ctc ccc agt ctt gtc ctt tct gat acc atg 642
Asp Arg Asn Val Asn His Leu Pro Ser Leu Val Leu Ser Asp Thr Met
                170                 175                 180

aaa aca ggt ttg aag agg gaa ttt gat gaa gtt ttt aca aag aaa atg 690
Lys Thr Gly Leu Lys Arg Glu Phe Asp Glu Val Phe Thr Lys Lys Met
            185                 190                 195

att gag tct atg agc cgt cct tcc atg gag ctt gtt ctc tgg aaa ccc 738
Ile Glu Ser Met Ser Arg Pro Ser Met Glu Leu Val Leu Trp Lys Pro
        200                 205                 210

ctc cct gaa ctc ctt tct gat aag cca aag cca tcc tct aat act aag 786
Leu Pro Glu Leu Leu Ser Asp Lys Pro Lys Pro Ser Ser Asn Thr Lys
    215                 220                 225

aac tat aca gga gag agc caa gct aag cat gta gct gct ggc act gcc 834
Asn Tyr Thr Gly Glu Ser Gln Ala Lys His Val Ala Ala Gly Thr Ala
230                 235                 240                 245

ttc cct cag aga act gaa ctg ttt tcg gaa cct cgg cca aca ggg atg 882
Phe Pro Gln Arg Thr Glu Leu Phe Ser Glu Pro Arg Pro Thr Gly Met
                250                 255                 260

tct ctt tat aat agt ttg gag aca gct act agc aca gaa gaa gag atg 930
Ser Leu Tyr Asn Ser Leu Glu Thr Ala Thr Ser Thr Glu Glu Glu Met
            265                 270                 275

gaa ctc tagaaaccaa tttctacact aaagttgtca aatgttagaa aaaaaaaaaa 986
Glu Leu

<210> 110
<211> 1893
<212> DNA
<213> Homo sapiens

<220>
<221> CDS

<222> (760)..(1182)

<400> 110
tggtttaatc tggataagct gaaagataga ttgctatcaa aaaagttccc caagatgtgc 60

atagccaaac tgggatagaa ggcaaactcc ccaaagctac ctgctggttt tgagaggggt 120

ggtaagacat ggcaattccc aggagtagta gaaaataata tgcctgacta ccaacagctc 180

aagtatgctt atttgcacat cctagacttg gtgtctgtaa gactcagtta ccacttttat 240

tttcctgtag ctaggagtta gcaaaaggaa ctggggcctt ccagccgagc cactaaacct 300

gtcttatttg gaatggggat tgtccagcaa agggagcaaa catgaattag atgttaagct 360

attgagctga agaaaagaaa gcagttcaca tttaggtgaa atagatgatg ttatcaggaa 420

gccaggttcc caccagagtc ggtgcttggt acctggtctc tccagtctca acagactcag 480

gtcaggtctc tcacccagga agcaaccact caataaaata gagaacatct gagaattaca 540

aatgtctatg cttgattgct cctctaaatc cagtgcatag gttaaccctg catgcccatt 600

tcttcctggg cttcttgatg gcaatgtgtt cttaaataac tggtcttgtg ttcatgctaa 660

agacaaactt acatgaagtt tttcagttta agacattcta gtgaatggct gctatgtgtt 720

tctggcactc attcctaacc aagtctttag agatttcag atg acc tta aag atg 774
Met Thr Leu Lys Met
1               5

caa tat ctt ttt ctt tct ttc ttt ctt tct ttt ttt ctg aga caa gag 822
Gln Tyr Leu Phe Leu Ser Phe Phe Leu Ser Phe Phe Leu Arg Gln Glu
                10                  15                  20

ttg cgc cct gtc gcc cag gct ggg gtg ctg gag tgc agt ggt gcg atc 870
Leu Arg Pro Val Ala Gln Ala Gly Val Leu Glu Cys Ser Gly Ala Ile
            25                  30                  35

tca gct cac tgc agc ctc tgc ttc cca ggt tca agt gtt tct cct gcc 918
Ser Ala His Cys Ser Leu Cys Phe Pro Gly Ser Ser Val Ser Pro Ala
        40                  45                  50

tca gcc ttc cga gta gct ggg att aca ggc atg tac cac tat gcc tgg 966
Ser Ala Phe Arg Val Ala Gly Ile Thr Gly Met Tyr His Tyr Ala Trp
    55                  60                  65

cta att ttt att tct ata ttt tta gta gag atg ggg ttt cat cat gtt 1014
Leu Ile Phe Ile Ser Ile Phe Leu Val Glu Met Gly Phe His His Val
70                  75                  80                  85

tgc cag gct ggt ctc gaa ctc ctg gcc tca agt gat ccg ccc acc tca 1062
Cys Gln Ala Gly Leu Glu Leu Leu Ala Ser Ser Asp Pro Pro Thr Ser
                90                  95                  100

gcc tcc caa agt gct ggg att aca ggc gcg agc cac cgc gcc tgg cca 1110
Ala Ser Gln Ser Ala Gly Ile Thr Gly Ala Ser His Arg Ala Trp Pro
            105                 110                 115

aag atg caa att ctt gtt tgg att tat gct ctg cct ctt ccc agc att 1158

Lys Met Gln Ile Leu Val Trp Ile Tyr Ala Leu Pro Leu Pro Ser Ile
        120                 125                 130

ttc tta tct gta gcc ctg ctt gct tgagagtata cttggataag aagtattgct    1212
Phe Leu Ser Val Ala Leu Leu Ala
    135                 140

gttgagggag ctataagaaa aggattcttc ttccagaagt aaagaactca tctttagagt    1272

acctttaaat gaattttgtt tttctttctt attttgaggt ggattggtct tctctttttt    1332

tgggtttcca gctcactggg actctcagac cttacctttc cagctcaaac accattagtt    1392

aaattccttc attctcatta gaatgcagcc tgctgagtat gtgggtttca ctgccggagt    1452

ccatcattta gccagtatac atagaggaac tgcttcgaat caaggcaact ggtgaagggc    1512

ttagcatgtt ggcagcaata tcccagagat tgaatctgtt tgcattttcc tcatctagga    1572

taacagctgc ttgaagccag ggctcttagc cctttgcatt ccccttgagc gaggaagcca    1632

cactgccttt ctgtgtctgg ttcagagctc ttccttcttg gcatgttttc tggactacat    1692

gcacatgggc agctatagat taatctgcaa aacctagtca cttacctacc cataatatct    1752

gggaaggtgt ggtatttgtt ttaaagaaac attgtttctt tgggagggca gtttctgtct    1812

ggactttgag gtggacttag ttatccctac agttctttaa ctctcagctt ttaataaaag    1872

atgaaatcag aaaaaaaaaa a                                              1893

<210> 111
<211> 783
<212> DNA
<213> Homo sapiens

<220>
<221> CDS
<222> (278)..(451)

<400> 111
atgatgctct tggaaaacac acacaggaag gcggaagtta aatgtgtacc caactacgaa      60

gtcagatgag gcaggatcac accctggctg cccctctggt cactagatgc cttgggcagg     120

taagctcacc tcttctggcc tcagtttctc catctgtgaa atgagtgtaa catctcatag     180

gctgtggtac ggactcatga attgatgcat ataaagcgtt cagtaagcct tttctcttaa     240

taaaaaaccc acaaaaccat catcagctca agattct atg caa aga gaa gac ggc     295
                                         Met Gln Arg Glu Asp Gly
                                         1               5

aca ccc agc atg ggg tgt ccc ttg acg ctg cca gac tca agt gac ttg     343
Thr Pro Ser Met Gly Cys Pro Leu Thr Leu Pro Asp Ser Ser Asp Leu
            10                  15                  20

agg ttc ctg gcc ttt gag ccc gcc cct ccc act ctt ccc tcg gcc agg     391
Arg Phe Leu Ala Phe Glu Pro Ala Pro Pro Thr Leu Pro Ser Ala Arg
        25                  30                  35

cca agg ctc ctc caa gcc tca gtt acc ctg tct tta aaa gag cgc gct     439
Pro Arg Leu Leu Gln Ala Ser Val Thr Leu Ser Leu Lys Glu Arg Ala
    40                  45                  50

cct ctg att cta tag aatcctgagg ctcaaagcca aagatgaaca gcctggaagc     494
Pro Leu Ile Leu
55

agagccatca gagcggcaaa gctgcccagg gatccccgag aactcgctgg ggttagagat     554

caggaccctg agattcaggc tctacacctg attcctcgtg cccccgtccg ctacggggct     614

gagttatggg catcgtttga catgggccac tcggattccc ttccaggccc ttgcccgtgc     674

cgttccctct gcctggccac cggatgaagc aggaagacgg actgcaagta gaccatttct     734

agccctgcct agataaaggc agagggcgca gtgaggtcct gcgacgaaa     783

<210> 112
<211> 6339
<212> DNA
<213> Homo sapiens

<220>
<221> CDS
<222> (533)..(4864)

<400> 112
ggaaaaaaag gaaggcgtga gggcgggcag cagcgacagg atgcttgttt ttcgctctac      60

caaagtcgtc tgaaggcgag acagcgggcc cagggcgcag gacccaccgc agccccctgg     120

gcagtctcct cgccccgcgt ccgcgtcgtc tccggggcac ttagtaaggg gtggggagag     180

cttgccctcc ctcttaagct gaggagaaac acccgaagac accgcaggag cctgtgaaag     240

tccctaggac tccaagtgag gaagtgacac tcccaggcga gccggcccgc ggctgccagt     300

ctgcacggcc tcggcacggc ggccccggag cggcgcgggg tggatctcag gctctgccgg     360

cccgcggccc gcggggtcca tgcgcagggc ccccagccca agttcttcca tcttccgatg     420

cggcccccca gagccgcggg gcagccggtg atctagcccg ggagcccatc ttacagcggt     480

gccaagcaga ggggcggcag agacggaggg gcagcctctt tgggactaac tc atg     535
                                                          Met
                                                          1

aag aac aag ggt gct aag cag aag ctg aaa cga aag gga gcc gcc agc     583
Lys Asn Lys Gly Ala Lys Gln Lys Leu Lys Arg Lys Gly Ala Ala Ser
            5                   10                  15

gcg ttt ggc tgt gac ctg acg gag tat ctg gaa agc tcg gga cag gat     631

Ala Phe Gly Cys Asp Leu Thr Glu Tyr Leu Glu Ser Ser Gly Gln Asp
        20                  25                  30

gtt cca tac gtt ttg aag agc tgt gca gaa ttt ata gag act cac ggc 679
Val Pro Tyr Val Leu Lys Ser Cys Ala Glu Phe Ile Glu Thr His Gly
    35                  40                  45

atc gtg gat gga atc tat cgg ctt tca gga gtc acc tca aac ata caa 727
Ile Val Asp Gly Ile Tyr Arg Leu Ser Gly Val Thr Ser Asn Ile Gln
50                  55                  60                  65

cgg cta agg caa gag ttt ggc tca gat caa tgt cca gat ctg aca agg 775
Arg Leu Arg Gln Glu Phe Gly Ser Asp Gln Cys Pro Asp Leu Thr Arg
                70                  75                  80

gaa gtg tac ctc cag gac atc cac tgt gtg ggc tcg ctt tgc aag ctc 823
Glu Val Tyr Leu Gln Asp Ile His Cys Val Gly Ser Leu Cys Lys Leu
            85                  90                  95

tac ttt agg gag ctg ccc aac ccc ctc ctg act tat gag ctc tat gag 871
Tyr Phe Arg Glu Leu Pro Asn Pro Leu Leu Thr Tyr Glu Leu Tyr Glu
        100                 105                 110

aaa ttc acg gag gca gtg tcg cat tgc cct gaa gaa ggc caa ctg gcc 919
Lys Phe Thr Glu Ala Val Ser His Cys Pro Glu Glu Gly Gln Leu Ala
    115                 120                 125

cga atc caa aat gtt atc cag gag ctt cct cca tcc cac tat agg acc 967
Arg Ile Gln Asn Val Ile Gln Glu Leu Pro Pro Ser His Tyr Arg Thr
130                 135                 140                 145

ttg gaa tac ctg att cga cac ctg gcc cat atc gcc tcc ttc agc agc 1015
Leu Glu Tyr Leu Ile Arg His Leu Ala His Ile Ala Ser Phe Ser Ser
                150                 155                 160

aag acc aac atg cac gcc cgg aac ctg gcc ctg gtg tgg gcg cca aac 1063
Lys Thr Asn Met His Ala Arg Asn Leu Ala Leu Val Trp Ala Pro Asn
            165                 170                 175

ctc ctc agg tct aaa gaa att gaa gcc act ggt tgc aat gga gat gca 1111
Leu Leu Arg Ser Lys Glu Ile Glu Ala Thr Gly Cys Asn Gly Asp Ala
        180                 185                 190

gcc ttc ctt gca gtc cgg gtc cag cag gtg gtg att gag ttc ata ttg 1159
Ala Phe Leu Ala Val Arg Val Gln Gln Val Val Ile Glu Phe Ile Leu
    195                 200                 205

aat cat gta gat caa atc ttt aac aac ggt gca cct ggg tct ctg gag 1207
Asn His Val Asp Gln Ile Phe Asn Asn Gly Ala Pro Gly Ser Leu Glu
210                 215                 220                 225

aat gat gaa aac cgg ccc atc atg aag agc ctg acc ttg cca gcc ctc 1255
Asn Asp Glu Asn Arg Pro Ile Met Lys Ser Leu Thr Leu Pro Ala Leu
                230                 235                 240

tcc ctg ccc atg aag ctg gtg agc ctt gag gaa gct caa gcc cgc agc 1303
Ser Leu Pro Met Lys Leu Val Ser Leu Glu Glu Ala Gln Ala Arg Ser
            245                 250                 255

ctg gcc act aac cat cct gct cgc aag gaa agg agg gag aac agc ctg 1351

Leu Ala Thr Asn His Pro Ala Arg Lys Glu Arg Arg Glu Asn Ser Leu
        260                 265                 270

cct gag att gtc cct ccc atg ggc acc ctc ttc cac act gtc ctt gag 1399
Pro Glu Ile Val Pro Pro Met Gly Thr Leu Phe His Thr Val Leu Glu
    275                 280                 285

tta cca gac aac aag cga aag ctc tcc agt aaa tca aag aag tgg aaa 1447
Leu Pro Asp Asn Lys Arg Lys Leu Ser Ser Lys Ser Lys Lys Trp Lys
290                 295                 300                 305

tca ata ttt aac ctg gga cgt tct gga tca gac tcc aaa tca aaa ctg 1495
Ser Ile Phe Asn Leu Gly Arg Ser Gly Ser Asp Ser Lys Ser Lys Leu
                310                 315                 320

agt aga aat ggg agt gta ttt gtg aga gga cag agg ctc tcg gtg gaa 1543
Ser Arg Asn Gly Ser Val Phe Val Arg Gly Gln Arg Leu Ser Val Glu
            325                 330                 335

aag gct act atc cga cca gct aaa agc atg gac tca cta tgt tca gtg 1591
Lys Ala Thr Ile Arg Pro Ala Lys Ser Met Asp Ser Leu Cys Ser Val
        340                 345                 350

cct gtg gaa gga aaa gaa acc aag gga aat ttc aat cga aca gtt acc 1639
Pro Val Glu Gly Lys Glu Thr Lys Gly Asn Phe Asn Arg Thr Val Thr
    355                 360                 365

acc ggt gga ttt ttc att cca gca aca aag atg cac tcc acc ggc acc 1687
Thr Gly Gly Phe Phe Ile Pro Ala Thr Lys Met His Ser Thr Gly Thr
370                 375                 380                 385

ggc agc tca tgt gac ctc acc aag cag gag ggc gaa tgg ggc cag gag 1735
Gly Ser Ser Cys Asp Leu Thr Lys Gln Glu Gly Glu Trp Gly Gln Glu
                390                 395                 400

ggg atg cct ccc ggg gct gag ggt ggc ttt gat gtg agc agt gat cgc 1783
Gly Met Pro Pro Gly Ala Glu Gly Gly Phe Asp Val Ser Ser Asp Arg
            405                 410                 415

agc cat ctc cag ggc gct cag gcc cgg ccc cca ccg gaa cag ctg aag 1831
Ser His Leu Gln Gly Ala Gln Ala Arg Pro Pro Pro Glu Gln Leu Lys
        420                 425                 430

gtt ttc cgg cct gtt gag gat ccg gag agc gag caa aca gcc cca aag 1879
Val Phe Arg Pro Val Glu Asp Pro Glu Ser Glu Gln Thr Ala Pro Lys
    435                 440                 445

atg ttg ggt atg ttc tac act tcg aac gac agc cct agc aaa tcc gtc 1927
Met Leu Gly Met Phe Tyr Thr Ser Asn Asp Ser Pro Ser Lys Ser Val
450                 455                 460                 465

ttc acc agc agc ctc ttc ccg atg gag ccc tcg ccg cgt aac cag cgc 1975
Phe Thr Ser Ser Leu Phe Pro Met Glu Pro Ser Pro Arg Asn Gln Arg
                470                 475                 480

aag gcg ctg aac atc tcc gag ccc ttt gcg gta tct gtg ccg ctc cgc 2023
Lys Ala Leu Asn Ile Ser Glu Pro Phe Ala Val Ser Val Pro Leu Arg
            485                 490                 495

gtg tcc gca gtc atc agc acc aac agc acg ccg tgc aga aca ccc ccg 2071

Val Ser Ala Val Ile Ser Thr Asn Ser Thr Pro Cys Arg Thr Pro Pro
        500                 505                 510

aag gag ctg cag tct ctt tcc agc ctg gaa gag ttt tct ttt cat gga      2119
Lys Glu Leu Gln Ser Leu Ser Ser Leu Glu Glu Phe Ser Phe His Gly
    515                 520                 525

tca gag agc gga ggc tgg cca gaa gaa gag aaa ccg ctg gga gct gag      2167
Ser Glu Ser Gly Gly Trp Pro Glu Glu Glu Lys Pro Leu Gly Ala Glu
530                 535                 540                 545

act tct gca gct tct gta cct aag aag gca ggt ctt gag gat gcc aag      2215
Thr Ser Ala Ala Ser Val Pro Lys Lys Ala Gly Leu Glu Asp Ala Lys
                550                 555                 560

gca gta cct gaa gca cca ggg aca gtg gaa tgc agc aaa ggc ctg tcc      2263
Ala Val Pro Glu Ala Pro Gly Thr Val Glu Cys Ser Lys Gly Leu Ser
            565                 570                 575

cag gag cca ggc gcc cac ctg gag gag aag aaa acc cca gaa agc tcc      2311
Gln Glu Pro Gly Ala His Leu Glu Glu Lys Lys Thr Pro Glu Ser Ser
        580                 585                 590

ttg agc tct caa cat tta aat gaa tta gag aag agg cca aat ccg gag      2359
Leu Ser Ser Gln His Leu Asn Glu Leu Glu Lys Arg Pro Asn Pro Glu
    595                 600                 605

aag gtg gtg gag gag gga cga gag gct ggt gag atg gag tcc agc acc      2407
Lys Val Val Glu Glu Gly Arg Glu Ala Gly Glu Met Glu Ser Ser Thr
610                 615                 620                 625

ctg cag gag agc ccc agg gcc aga gcc gaa gct gtg ctt ctc cat gag      2455
Leu Gln Glu Ser Pro Arg Ala Arg Ala Glu Ala Val Leu Leu His Glu
                630                 635                 640

atg gat gaa gat gat ctg gcc aat gcc ctg atc tgg cct gag att caa      2503
Met Asp Glu Asp Asp Leu Ala Asn Ala Leu Ile Trp Pro Glu Ile Gln
            645                 650                 655

cag gag ctg aaa atc att gaa tct gag gag gag ctc tca tcg ttg cca      2551
Gln Glu Leu Lys Ile Ile Glu Ser Glu Glu Glu Leu Ser Ser Leu Pro
        660                 665                 670

cct cct gct ctg aag acc agc cca att cag cct att ctc gag tcg agt      2599
Pro Pro Ala Leu Lys Thr Ser Pro Ile Gln Pro Ile Leu Glu Ser Ser
    675                 680                 685

ctg ggg ccc ttt att ccc tca gag cct cct ggg agc ttg cct tgt ggc      2647
Leu Gly Pro Phe Ile Pro Ser Glu Pro Pro Gly Ser Leu Pro Cys Gly
690                 695                 700                 705

tcc ttc cct gct cca gtc tcc acc cct ctg gag gtg tgg act agg gat      2695
Ser Phe Pro Ala Pro Val Ser Thr Pro Leu Glu Val Trp Thr Arg Asp
                710                 715                 720

cca gcc aat cag agc aca cag ggg gct tcc aca gca gcc agc aga gag      2743
Pro Ala Asn Gln Ser Thr Gln Gly Ala Ser Thr Ala Ala Ser Arg Glu
            725                 730                 735

aag ccg gaa cct gag cag ggc ctg cac cca gac ctc gcc agc ctg gct      2791

Lys Pro Glu Pro Glu Gln Gly Leu His Pro Asp Leu Ala Ser Leu Ala
        740                 745                 750

cct ctg gaa ata gtt cct ttt gag aag gca tct cca caa gca aca gtg 2839
Pro Leu Glu Ile Val Pro Phe Glu Lys Ala Ser Pro Gln Ala Thr Val
    755                 760                 765

gaa gta gga ggc cca ggc aat ctg tct cct cca ctc cca cct gct cct 2887
Glu Val Gly Gly Pro Gly Asn Leu Ser Pro Pro Leu Pro Pro Ala Pro
770                 775                 780                 785

ccc cct cca act cct ctg gag gag tca act cca gtc ctg ctt tca aag 2935
Pro Pro Pro Thr Pro Leu Glu Glu Ser Thr Pro Val Leu Leu Ser Lys
                790                 795                 800

ggc agc ccg gaa aga gaa gac tca tcc agg aaa ttg agg aca gat ctc 2983
Gly Ser Pro Glu Arg Glu Asp Ser Ser Arg Lys Leu Arg Thr Asp Leu
            805                 810                 815

tac ata gac cag ctg aag tcc caa gac agc cct gag atc tct agc ctc 3031
Tyr Ile Asp Gln Leu Lys Ser Gln Asp Ser Pro Glu Ile Ser Ser Leu
        820                 825                 830

tgt cag gga gag gag gca acc cca aga cac agt gac aag caa aat tca 3079
Cys Gln Gly Glu Glu Ala Thr Pro Arg His Ser Asp Lys Gln Asn Ser
    835                 840                 845

aag aat gct gct tct gag ggg aaa ggc tgt ggt ttt cca agc cca acc 3127
Lys Asn Ala Ala Ser Glu Gly Lys Gly Cys Gly Phe Pro Ser Pro Thr
850                 855                 860                 865

agg gag gtt gag atc gtc tca caa gaa gag gag gat gta acc cat tca 3175
Arg Glu Val Glu Ile Val Ser Gln Glu Glu Glu Asp Val Thr His Ser
                870                 875                 880

gta cag gag cct tca gac tgt gac gaa gat gac act gtg aca gac att 3223
Val Gln Glu Pro Ser Asp Cys Asp Glu Asp Asp Thr Val Thr Asp Ile
            885                 890                 895

gcc cag cat ggc ctg gag atg gtg gag ccc tgg gag gaa ccc cag tgg 3271
Ala Gln His Gly Leu Glu Met Val Glu Pro Trp Glu Glu Pro Gln Trp
        900                 905                 910

gtg acg agt ccc ctt cac tct ccc acc ctg aaa gac gcg cac aag gcc 3319
Val Thr Ser Pro Leu His Ser Pro Thr Leu Lys Asp Ala His Lys Ala
    915                 920                 925

cag gta cag ggc ctt cag ggt cac cag ttg gag aag agg ctt tcc cac 3367
Gln Val Gln Gly Leu Gln Gly His Gln Leu Glu Lys Arg Leu Ser His
930                 935                 940                 945

agg ccc agc ctt cgc cag agc cat tct cta gat agc aaa ccc acg gtt 3415
Arg Pro Ser Leu Arg Gln Ser His Ser Leu Asp Ser Lys Pro Thr Val
                950                 955                 960

aaa agc cag tgg act ctc gag gtt ccc tcc tcc agc agc tgt gct aat 3463
Lys Ser Gln Trp Thr Leu Glu Val Pro Ser Ser Ser Ser Cys Ala Asn
            965                 970                 975

ctt gaa aca gag agg aat tct gac cct ctt cag ccc cag gca ccc agg 3511

Leu Glu Thr Glu Arg Asn Ser Asp Pro Leu Gln Pro Gln Ala Pro Arg
        980                 985                 990

aga gag att act gga tgg gat gag aaa gcc ctg agg tcc ttc aga gag 3559
Arg Glu Ile Thr Gly Trp Asp Glu Lys Ala Leu Arg Ser Phe Arg Glu
    995                 1000                1005

ttc tct ggc ctg aaa ggg gca gag gct cct ccc aac cag aag gga cca 3607
Phe Ser Gly Leu Lys Gly Ala Glu Ala Pro Pro Asn Gln Lys Gly Pro
1010                1015                1020                1025

agt ggt gtg caa ccc aac cca gca gaa acc agc ccc atc agc cta gca 3655
Ser Gly Val Gln Pro Asn Pro Ala Glu Thr Ser Pro Ile Ser Leu Ala
                1030                1035                1040

gag gga aag gag cta ggg aca cac ctg ggg cac agc agt cca cag att 3703
Glu Gly Lys Glu Leu Gly Thr His Leu Gly His Ser Ser Pro Gln Ile
            1045                1050                1055

agg caa ggt ggt gtt cct ggg cca gag agc agc aag gag agt tca ccc 3751
Arg Gln Gly Gly Val Pro Gly Pro Glu Ser Ser Lys Glu Ser Ser Pro
        1060                1065                1070

agc gtg cag gac agc act tcg cct gga gag cac ccc gca aag tta cag 3799
Ser Val Gln Asp Ser Thr Ser Pro Gly Glu His Pro Ala Lys Leu Gln
    1075                1080                1085

cta aag agc aca gag tgt ggg ccc cca aaa ggg aaa aac agg cct tct 3847
Leu Lys Ser Thr Glu Cys Gly Pro Pro Lys Gly Lys Asn Arg Pro Ser
1090                1095                1100                1105

tcc ctc aac ttg gac cct gcc att ccc att gct gac ctc ttc tgg ttt 3895
Ser Leu Asn Leu Asp Pro Ala Ile Pro Ile Ala Asp Leu Phe Trp Phe
                1110                1115                1120

gag aat gtg gcc tca ttt agt tca cct gga atg cag gtc tct gag cca 3943
Glu Asn Val Ala Ser Phe Ser Ser Pro Gly Met Gln Val Ser Glu Pro
            1125                1130                1135

gga gac cca aag gtc aca tgg atg acc tca tct tac tgt aaa gca gac 3991
Gly Asp Pro Lys Val Thr Trp Met Thr Ser Ser Tyr Cys Lys Ala Asp
        1140                1145                1150

ccc tgg agg gtt tac tcc cag gac ccc cag gac ctg gac att gtt gct 4039
Pro Trp Arg Val Tyr Ser Gln Asp Pro Gln Asp Leu Asp Ile Val Ala
    1155                1160                1165

cat gca ctg aca ggc cgc cgt aac tca gct cct gtg agt gtg tca gct 4087
His Ala Leu Thr Gly Arg Arg Asn Ser Ala Pro Val Ser Val Ser Ala
1170                1175                1180                1185

gtg aga acc tcc ttc atg gtc aaa atg tgc cag gcc agg gcg gtc cca 4135
Val Arg Thr Ser Phe Met Val Lys Met Cys Gln Ala Arg Ala Val Pro
                1190                1195                1200

gtc atc cct ccc aag att cag tac acc cag atc cca cag ccc ctg ccc 4183
Val Ile Pro Pro Lys Ile Gln Tyr Thr Gln Ile Pro Gln Pro Leu Pro
            1205                1210                1215

tct cag agc tca ggg gag aat ggg gtt cag cct ctg gag agg agc cag 4231

Ser Gln Ser Ser Gly Glu Asn Gly Val Gln Pro Leu Glu Arg Ser Gln
        1220                1225                1230

gag gga ccc agc tca acc agt ggg acc act cag aaa cct gcc aaa gat 4279
Glu Gly Pro Ser Ser Thr Ser Gly Thr Thr Gln Lys Pro Ala Lys Asp
    1235                1240                1245

gat tct ccc tcc tcc ctg gaa agc tca aag gaa gaa aaa cca aag caa 4327
Asp Ser Pro Ser Ser Leu Glu Ser Ser Lys Glu Glu Lys Pro Lys Gln
1250                1255                1260                1265

gat ccc gga gcc att aag tcc tca cca gtg gat gcc act gca ccc tgc 4375
Asp Pro Gly Ala Ile Lys Ser Ser Pro Val Asp Ala Thr Ala Pro Cys
                1270                1275                1280

atg tgc gag gga cct acc ctt tct cca gaa cca ggc tcg tct aac ctg 4423
Met Cys Glu Gly Pro Thr Leu Ser Pro Glu Pro Gly Ser Ser Asn Leu
            1285                1290                1295

ctc tcc acc cag gat gca gta gtg caa tgc aga aag cgc atg tca gag 4471
Leu Ser Thr Gln Asp Ala Val Val Gln Cys Arg Lys Arg Met Ser Glu
        1300                1305                1310

aca gag cca tct ggg gac aac ctt ctt tct tca aaa cta gag cga cca 4519
Thr Glu Pro Ser Gly Asp Asn Leu Leu Ser Ser Lys Leu Glu Arg Pro
    1315                1320                1325

tct ggg ggt tct aag cct ttc cac agg tca agg cca gga aga cct cag 4567
Ser Gly Gly Ser Lys Pro Phe His Arg Ser Arg Pro Gly Arg Pro Gln
1330                1335                1340                1345

agc cta atc tta ttc agt cct cct ttc ccc att atg gac cac ctg ccc 4615
Ser Leu Ile Leu Phe Ser Pro Pro Phe Pro Ile Met Asp His Leu Pro
                1350                1355                1360

cct tca tcc aca gtg aca gat tcc aag gtc ctg ctg tcc cct atc aga 4663
Pro Ser Ser Thr Val Thr Asp Ser Lys Val Leu Leu Ser Pro Ile Arg
            1365                1370                1375

agt ccc acc cag aca gtt tcc cct ggc ctt ctt tgt gga gag ttg gca 4711
Ser Pro Thr Gln Thr Val Ser Pro Gly Leu Leu Cys Gly Glu Leu Ala
        1380                1385                1390

gaa aac aca tgg gtc aca cca gaa ggg gtt aca ctt agg aat aaa atg 4759
Glu Asn Thr Trp Val Thr Pro Glu Gly Val Thr Leu Arg Asn Lys Met
    1395                1400                1405

acc atc cct aag aat ggc cag aga cta gag acc tca acc agc tgt ttt 4807
Thr Ile Pro Lys Asn Gly Gln Arg Leu Glu Thr Ser Thr Ser Cys Phe
1410                1415                1420                1425

tac cag cct cag cgg aga tca gta att ctg gat gga aga agt ggg agg 4855
Tyr Gln Pro Gln Arg Arg Ser Val Ile Leu Asp Gly Arg Ser Gly Arg
                1430                1435                1440

caa ata gaa tgatttc ggttcacctg ctggtgtctg aaaaaaaccg tgattcatct 4911
Gln Ile Glu

ggaagttatt acagggccag cttgccatat tccaggcaca cgttatcaag tttgggccta 4971

ttgtggcctc tgacttctct ttcttcagcc ttttgaccac ttattaatta gtccatttgc 5031

tagaagagtg gtcaagggaa aaacgagaga tgaaatttag ttaagtctat gtgagcaagt 5091

gagagaaggt taggtaaggg gagaggatgg aatgcttgcc tccaatgaac tttggagctt 5151

gtatgtgagt cagattgctc ccctattgct attatctatt actcttgaga gctggctgtc 5211

ctttgaaaga aagaagtaat gttctttgaa agaaagaaaa atctcttgct gtgtcaaacc 5271

tcaaaatgtt gctattgggg ttagaaggcc tcctctttat gctttttaat gctctttcaa 5331

acgtgttctt ttagaccagt tttctaataa gctttgtaaa atgtactatc caaattagaa 5391

gcggatttgg aaatgcaaac taacgtgcac ttagatatcc aagtgggtga gcttagccac 5451

tcttacccat gctctttccc tggaatccct ggagacctgt ccaagatgat ttccatatac 5511

cagcatagaa aatcagaatc aagagcaaac tctgagactg gcacaatcca agaagatttc 5571

ctggctctgg cttttagtaa tttgggactc caactgccac tgtactggac tgtaatttat 5631

aaatccagta gctacgcagg gtggaggctg ggctgaggat taccataatg aaatgtacta 5691

aatcttcatt taggtatgca attgtgaagt gaaggcatct gctttcttta cagtatcaga 5751

gtccaagaac aggatgtcac catagataaa agcctcatac aaaggcagaa ctacactcca 5811

aatttaatgt gtttaaattg gtggggcacc agcagaaaat acttctagct cagctttact 5871

cttcttccac actaggctgg gcccagcaat acaggagagg atgaagggag gagctccagg 5931

aggcgaggga agagccctag cagggcggcc atcacaacca ctcactgaga gttgcccttc 5991

ttaaaaatgt attttatttt agccagtggg tcccttcctt tctcctttcc tctctactgc 6051

tcaagaacag atttgaggcc aggtgcggtg cctcacatct gtaatcccaa cactttggga 6111

ggctgagatg ggtggattgc ttgagcccag gagttcaaga ccagcctggg caacacagcg 6171

agaccccatc tcttaaaaaa taacagactt gaggaacccc tctcccttcc ataattcccc 6231

tcatccaccg cccactccag gcactcactc aaacttgctc ttcaactctg tatacaagca 6291

gaagcaataa accaatctga ttttcttttc aattaaaaaa aaaaaaaa 6339

<210> 113
<211> 6593
<212> DNA
<213> Homo sapiens

<220>
<221> CDS
<222> (3200)..(3700)

<400> 113

ttttcgtcta tgtttctgat ctgctttcag gaagaattat tcatttttat cttcccaatc 60
tgaatgtttt attgaatttt tattttccaa gttaataaaa cctttggtta ttatcctgtc 120
tttgttttac agtgtcctgc tcttgatgcg tggatgcaga ttttgtctct ctgtggacgt 180
taatgattgt ggtctgaggg gaagtctttt ctgctccctg tgttgccctc attttctctg 240
agttctcttt attttggtgg tctcagtctc tatctttcac gttgtgaatt tttctcaaat 300
atatggtgat cctatggatc tgttcatgtt ttaagagtga ggcatccaaa agctgattgg 360
aagttgtgtg tgccaactgg tgagcttttc cactagggtc accaggtggg cacctggact 420
catcattgga gaacactgcc tgtcagtatt tgcacgtgtt ttctctgggg ctcattctgt 480
ttcttgagag atattcccac tactctcctt cctgggaaac gggcatacac agggctttta 540
gcctatgctg agtactcatg tggtttcatc aaatgttgtc ccactctcgg cagaagtccc 600
catgagcact tggcttgatc tggccacagg acaccttttg ccccttcctt cagacatacc 660
cagctctgag cttggacaat gctcgaggaa caatgaaaag gctagatttt aactcctaat 720
tgccctcttt agccaagtgc ctctgtgcaa tgcacaactg taggaccata tccagagacc 780
ctggtctata ctcagtgtgc ccgagtcctg accgctgagc aggcagcctc atcacccgtg 840
agtatttaga ctttcattaa gttgcttcaa cctttgctgt gcctggtgtc cctgagttgg 900
agctgctctg atttctccat ggagtaggag cacagtggtc atctggctgc atgggaagga 960
caggggacct cagagccaat aaatgccctg gtcttcagcc ccatgtctca ccccatccct 1020
cctattccaa tccccaagcc tctcctggag gccacagggc atctctgctg accactctgt 1080
ggcctcccct ctgcaggcat gtggttttag gatccccctc cctgctcact tttccatcag 1140
ttccattcct gcataaatag gttgctagct cttgcttact gttctcttct gtattctgtg 1200
ttcatggttg tattaatata actttcattc ctctgctcct attttcatgg ggtttcatga 1260
gggaagaaac aggctcatgt gttcagtata ccatcttgat ccataaaccc catttgagtt 1320
atctcacagg ttgcttaggg ggcttgctgc tctgactggt ttgtgaacgt gccctgagtc 1380
cagctgggac tgcagcaaca tccatcctgt tgagctgtgg tctgctaagt gattttgtgt 1440
aatgctgtgc caagtgcagg agagggatac atagcatggc taagctctgc ttgaatgcca 1500
tgattagtta ttaccttcct tccttccccc tggttttgct gcctattgtc agtaaatgat 1560
cttagatctc ctgcctcccc aaagatatag tctatgtggt ggtctgggtt ctaggttttc 1620
tttaccaccc ttgcttcact taagaaacag aaaactggct aggcacagta gtggctcaca 1680
cctataatcc cagccctttg ggaggccaag gcaggaggat cgcttgaggc caggaattca 1740
agaccagcct gcacaacata gcaagactcc atctctacaa aaagttaaat ttaaattagt 1800

ggtggcatgc acttatagtc ccagctactc gggaggatga ggcaggagaa ttgtttgagc 1860

ccatgacttt gaggttactg tgagctgtga tcctgctatt ttactccacc tgagtgacag 1920

agcgagacgc tgtctcaaaa ataaagaaac aagtagtcca cacaggtgtt tgccagcaaa 1980

caaacagcgc ggattctctg ttgaacgtgg ccaacattgc ctcgtcgaag tttgtatctt 2040

gccatagttg atgatcaggt ggtcggggct tcctggtcag acctgacctt tgtggggaga 2100

tgttctccca ccaaggctca gaagcaggac actcttgctg cccctcgggc agccacccag 2160

aacatgggcg tctccatttc tgaggttcag catagttccc cccaccctct tcccatgcag 2220

tgcatgcctg ctctttatca ggatggaaca gagggaaggc agaggcactg ggggcagcct 2280

gtgttgttga tctctgtaag tcacagactg ttcgaaaacc tcaggaatta tgtaagcgtt 2340

cagtgcctca tttaagagat gaaaaactga agattagaaa gaggaaacga ctagtcgccc 2400

tgctagttga tgacctccag atagccatct tcattcccag ttaagtttac tacaaaacca 2460

cagcctcagc tgctgaagct aacctcccta cccaccatcg ctagggactc agaacaccat 2520

ggagctcatc tctccaacaa aagtcagaat tagcaagtac agatgtgtct tacgaaaatg 2580

tcagaatcca tcatggctgc tttggtgagg agctgttctc cttccgtggt catgccattg 2640

gaaggcagtt gtatgacaag aatgctttca catgagctcc gatttcttta actctccaga 2700

aggacttcag aagcctctgg agttttgaga cacggcctct ctctcccttc tgcttggaga 2760

caccttcctg ggggtctcca ctgcattccc cgccggtatt cctctgtgga gccttgcgcc 2820

ctcaggtccc agaaatgggc tgtgtgggag cttccagtca gcctggtatc tcgggacctt 2880

ctcagccagg aaatgagact gcacaaagcc ctgtgaggcc cagctgtgtt atttctcctt 2940

ccccgacatt gcataatcac tgctgacaca gcatttgggg aagatttaac gtcttgggcc 3000

agcagctgcc atctggaacc atcaccacat agcaggttgt tctgtcccat ccaactgatg 3060

agggggctgc cctaatgaca aggtccagga cctgggttgg gtggtggaag gctgggagct 3120

ggagtgggca ttattgtgct tctgtgctga aaattgctat ggctcttttc ccctaacagc 3180

cagcgtggga gaaccaaac atg aga cac aag cat ccc ctt gag ctc cac aga 3232
Met Arg His Lys His Pro Leu Glu Leu His Arg
1               5                   10

tct tgc gcc ttg ctc tca tca ggc att tcc ttg gag aac agc agt cag 3280
Ser Cys Ala Leu Leu Ser Ser Gly Ile Ser Leu Glu Asn Ser Ser Gln
            15                  20                  25

cag ctc atg gaa gtg agc ccg gtg cac aga ctt tgc atg gac ttt gca 3328
Gln Leu Met Glu Val Ser Pro Val His Arg Leu Cys Met Asp Phe Ala
        30                  35                  40

cag gtt cca ttt cca tcg tgt gca gac acc tgt atc ttt ggg ctc cac 3376
Gln Val Pro Phe Pro Ser Cys Ala Asp Thr Cys Ile Phe Gly Leu His

45                                50                                55

gtg agc acc tgc cct ctt cac tct gat ttt ttt ttt aag aga cag cat      3424
Val Ser Thr Cys Pro Leu His Ser Asp Phe Phe Phe Lys Arg Gln His
60                      65                      70                      75

ctt act ctg tca ccg agg ctg gag tac agt ggt gtg atc aca gct cac      3472
Leu Thr Leu Ser Pro Arg Leu Glu Tyr Ser Gly Val Ile Thr Ala His
                        80                      85                      90

tgc agc ctg aaa ctc ctg ggc tca ggc aat cct cct gcc tca gcc tcc      3520
Cys Ser Leu Lys Leu Leu Gly Ser Gly Asn Pro Pro Ala Ser Ala Ser
            95                      100                     105

cga gtg gct ggt agt aca ggt gca tgc cac cac gcc cac cta att ttt      3568
Arg Val Ala Gly Ser Thr Gly Ala Cys His His Ala His Leu Ile Phe
        110                     115                     120

tta tct ttt ata gat aca ggt ctc act gtt gct cag gct ggt ctc gaa      3616
Leu Ser Phe Ile Asp Thr Gly Leu Thr Val Ala Gln Ala Gly Leu Glu
    125                     130                     135

ctc ctg gcc tca agt gat cct cct gcc ttg gcc tac caa aat gct ggg      3664
Leu Leu Ala Ser Ser Asp Pro Pro Ala Leu Ala Tyr Gln Asn Ala Gly
140                     145                     150                     155

att ata ggc atg aac cac tgt gcc tgg cct tca ctc tgat tttcttaag      3714
Ile Ile Gly Met Asn His Cys Ala Trp Pro Ser Leu
                160                     165

agtcctacag gaccttcagg tcttgtcact tctctaccat tgtgtgtgag acagggcatg      3774

gggtgagctt ggtcaggtgt gggtcaaaag cctggagact cagagctgct ggttccccaa      3834

gagcccacaa aagttatcag agtttctgag ctgagtgttc tgcagttgcc agaccccaca      3894

atgctgggaa tactggcttc tttgtgttta gaacttgttc tgagaacctt gatgagaaca      3954

catcaagcaa gcagttcctc ccagccaggg ctggagcaac atgccaaaaa acattcttgt      4014

ctatgggaaa aagacaacca gtattttgta ctgtttgacc aagttgtttt taaatataca      4074

catgcctcaa caacccacgg cctcaatcgc actgctaatt tgtttaaatc cctgacactc      4134

agatgtcaag aatagcttca tctagcagtg ttctctttaa aggaaccctt tttttgtttt      4194

tcattttaat cactgccttc ttctctggga ggtattgtgg gctagagaaa gaagtcaggt      4254

ttgggagtca gccagattta gattggagtc ccagctctgt caccagatct ctgggtgatc      4314

ttggcaaact aacttctgag cttctgtgca ttataggcat tcagcacata tttgttggat      4374

gaaaaatact tttcttgaaa gtttatgaag ttgtaggatg cccagcagcc cccttttctc      4434

tatttccttc ctcttgaagc aagcgttgac tatacctctt tccctaaccc ttagtgtacc      4494

atagcttctt ccaacctctc tccaatccat tacttttttc tgagctaccc tgtctcaaat      4554

ctaactcagg cctttttgca agaggaagtt taagccattg tttactcatt aactcacttg      4614

acccttagaa caatcgtggg aggtgtttat ttcggaaagg aagctaaagc acaggacaga 4674

aaaataaggt catagcgtga actagtaagt agcagaactg ggaacatagg actgttattt 4734

tcaaacactc taaacttcac tgcagttgca aatttgcaac caaaatggcc gccttgaaag 4794

ggtcttttgc agtaaaatct cagtgtccaa catcaaagac agttcttcag ggcctccaat 4854

taactataca tcctgccccc agctgctcat ctctgcacct ctaacacgta cttcacagag 4914

taggaaattg ggctttctcc ttcaaaaaga aaagtagaga aggcctgagg gcagtgtaat 4974

taaaatggag aagtagagac cacgaattac ttggatgaat tcataactgg ttggtctctg 5034

aggcagggat tattattttt cctgtatttt aagcttaaaa gtagtgattt ttgcattgtt 5094

acaaaaaaca aaatagcaaa atatcatttg atacttgtat tctgtttgca atagctacca 5154

cttcctttag ccccagcaaa aatcaatagg tgtgtattct ctacaagccc atgctgtgcc 5214

agaatgccag tattgctaaa tgtcatgcaa ggatttagac acttttttaa aggaaactct 5274

gggagtgcct gttgtctgga gagttgacat tgtccaaagc aggccaacac tggtatgggt 5334

atggagaacc cacgtcctag ctgtggctca cctgcagatg ccttgaattt acctctctgg 5394

gttggaagtg ggaagacttc ttttaaaagt cctttctaag tttaaaaatc caaaggttac 5454

gaaggtagtt tttatttatc tcaataagtg gcctatttgc ttaaagcaag tgtacctctg 5514

tatatcagcc caaagcttag ggcagtgctt cttggcttta gtatctgaga aaggtgcctt 5574

tgggaagagt ttggtcagac agaaggtagt ctgaatctta aaggtttcag atgaatgttt 5634

gaagtagaac ctgggaaatc tgcttcctag ggctgttaga aggaaattgg tctccttaaa 5694

ttacgtcagc atctgaggca gtttcctaag ggaatttctt ttaaattcaa aagttcaact 5754

gcaggaataa atagctgtac aggactagat cttcccatcc ctcatctttt ccccttcccc 5814

gagtaacgac tcaaaaaggc ttctgaaatt ctactcagaa tcggagccgt ttgcattcca 5874

acaaggatcc cttaagccaa atgctggtgt gtttgtgtag aatgtccctg gggtaggggc 5934

ggtagctgtg gggagggaat gtggtgcagg ggacacctgc cctctgccct tgaaattgca 5994

ccataagatg ctgcctatgt cactcacagt ggtgctgata ctatccgcca aagacaaatt 6054

gtgaacacgg aaaaatggtc gttccctact gtacatcctc aggacagact taactaaact 6114

tggagacagg agattgttca gaccatattg tagtgggtgg tgatcattgt tttattttgg 6174

gggagtctgg tctattgagc agattgaatg tttccttatt gtgcagggct taattgacta 6234

tgtctgaaag tttttactga gagctctaag aaaactattg aggaaaatga aatgttattt 6294

tgtagtacaa ggattatttt tgtctattta ggatatacat agctgtagct ttgtaaaaaa 6354

aaagatcttt ataaacaata tatgaatgtg ccgtcttatt tattgattac tgtaaattaa 6414

gatataaatg gctatttgca taatttatac ctgtgggaat taactggagt atttgttatt 6474

tgactgtttt ctattaagga atattaggct tggtgctatg atgaatgatc ttgtaaaatc 6534

atgtgtattc ttaagaaaat ttttgaatat aaatttcttg aactgaaaaa aaaaaaaaa 6593

<210> 114
<211> 2994
<212> DNA
<213> Homo sapiens

<220>
<221> CDS
<222> (27)..(908)

<400> 114
tgcgaggctc cgcttcttct acaagt atg gag aag gca aaa ggc aag gag tgg 53
Met Glu Lys Ala Lys Gly Lys Glu Trp
1               5

acc tcc aca gag aag tcg agg gaa gag gat cag cag gct tct aat caa 101
Thr Ser Thr Glu Lys Ser Arg Glu Glu Asp Gln Gln Ala Ser Asn Gln
10                  15                  20                  25

cca aat tca att gct ttg cca gga aca tca gca aag aga acc aaa gaa 149
Pro Asn Ser Ile Ala Leu Pro Gly Thr Ser Ala Lys Arg Thr Lys Glu
                30                  35                  40

aaa atg tct gtc aaa ggc agt aaa gtg ctc tgc cct aag aaa aag gca 197
Lys Met Ser Val Lys Gly Ser Lys Val Leu Cys Pro Lys Lys Lys Ala
            45                  50                  55

gag cac act gac aac ccc aga cct cag aag aag ata cca atc cct cca 245
Glu His Thr Asp Asn Pro Arg Pro Gln Lys Lys Ile Pro Ile Pro Pro
        60                  65                  70

tta cct tct aaa ctg cca cct gtt aat ctg att cac cgg gac att ctg 293
Leu Pro Ser Lys Leu Pro Pro Val Asn Leu Ile His Arg Asp Ile Leu
    75                  80                  85

cgg gcc tgg tgc caa caa ttg aag ctg agc tcc aaa ggc cag aaa ttg 341
Arg Ala Trp Cys Gln Gln Leu Lys Leu Ser Ser Lys Gly Gln Lys Leu
90                  95                  100                 105

gat gca tat aag cgc ctg tgt gcc ttt gcc tac cca aat caa aag gat 389
Asp Ala Tyr Lys Arg Leu Cys Ala Phe Ala Tyr Pro Asn Gln Lys Asp
                110                 115                 120

ttt cct agc aca gca aaa gag gcc aaa atc cgg aaa tca ttg caa aaa 437
Phe Pro Ser Thr Ala Lys Glu Ala Lys Ile Arg Lys Ser Leu Gln Lys
            125                 130                 135

aaa tta aag gtg gaa aag ggg gaa acg tcc ctg caa agt tct gag aca 485
Lys Leu Lys Val Glu Lys Gly Glu Thr Ser Leu Gln Ser Ser Glu Thr
        140                 145                 150

cat cct cct gaa gtg gct ctt cct cct gtg ggg gag ccg cct gcc ctg 533

His Pro Pro Glu Val Ala Leu Pro Pro Val Gly Glu Pro Pro Ala Leu
    155                 160                 165

gaa aat tcc act gct ctc ctt gag gga gtt aat aca gtt gtg gtg aca 581
Glu Asn Ser Thr Ala Leu Leu Glu Gly Val Asn Thr Val Val Val Thr
170                 175                 180                 185

act tct gcc cca gag gct ttg ctg gcc tcc tgg gcg aga att tca gcc 629
Thr Ser Ala Pro Glu Ala Leu Leu Ala Ser Trp Ala Arg Ile Ser Ala
                190                 195                 200

agg gcg agg aca cca gag gca gtg gaa tct cca caa gag gcc tct ggt 677
Arg Ala Arg Thr Pro Glu Ala Val Glu Ser Pro Gln Glu Ala Ser Gly
            205                 210                 215

gtc agg tgg tgt gtg gtc cat ggg aaa agt ctc cct gca gac aca gat 725
Val Arg Trp Cys Val Val His Gly Lys Ser Leu Pro Ala Asp Thr Asp
        220                 225                 230

ggt tgg gtt cac ctg cag ttt cat gct ggt caa gcc tgg gtt cca gaa 773
Gly Trp Val His Leu Gln Phe His Ala Gly Gln Ala Trp Val Pro Glu
    235                 240                 245

aag caa gaa ggg aga gtg agt gca ctc ttc ttg ctt cct gcc tcc aat 821
Lys Gln Glu Gly Arg Val Ser Ala Leu Phe Leu Leu Pro Ala Ser Asn
250                 255                 260                 265

ttt cca ccc ccg cac ctt gaa gac aat atg ttg tgc ccc aaa tgt gtt 869
Phe Pro Pro Pro His Leu Glu Asp Asn Met Leu Cys Pro Lys Cys Val
                270                 275                 280

cac agg aac aag gtc tta ata aaa agc ctc caa tgg gaa tagaatatca 918
His Arg Asn Lys Val Leu Ile Lys Ser Leu Gln Trp Glu
            285                 290

ggaaaaaggc cacatctatg gtaattaatg gcagaaaagc tggagagttg gattctgcgg 978

tgctgctgac aggtgaactc tggtcctctg cacctgttta tgggccatgc agactggtgg 1038

ggtggcagat gttagcctaa gacccctagc agtgcctgtt gctttgtgag tggagataga 1098

gactcttaca tttaaaaatg gaaaaacatt tcacaaatta ccataaattg tagttaatat 1158

gtagaaaaac tcattcatac tacttttcta aaatagacat gacttcagca gcagcttttt 1218

tttgttgtat tttgagacag tgtctcactg ttgcccaggc tggagtgcag tggtgcaatc 1278

tcagttcagt gcaatctccg cctcctgggt tcaaatgatt ctcctgcctc agcctcttga 1338

gtagctaggt acaggcacct gccaccacac ccagctaatt ttttgtattt ttagtagaga 1398

tggggtttca ccatgttggc caggttggtc tcaaactcct ggactcaagt gatcaccctc 1458

ctcagcctcc caaaatgctg ggactatggg catgagcccc tgcgcctgac cttcaacagc 1518

tcttttaagt gagttcttca gctaagcatt gtgatggact tgagtaaaat ggtagttggc 1578

tcttgtgctc aattttctct tcctctgaac actgactact ttaggagctg cttcattcca 1638

attgcaattt cataaaacgt aaagtatttt aaggcaaaga aaggctgtta attccctccc 1698

tcccccaaac acatgatttt taatattcta aacaatattt ttcaaagttc tcttaataac 1758

ctgagatttc tatggtttga ctccaggatc aaaacacaag ggactttgta ttatttcact 1818

tataattgtt ttgtatattt ctggagttta aaatgtttaa ggttgcttcc cgctcataaa 1878

tacataatat attgaattta aaatgtgttt attaaccgat tctccataaa taaaaataag 1938

atgtgtatgt aaaataattc atctgttgta tttagagaac catattcatt gcatgcaaat 1998

cttattgtta gtgttcttaa ctcaagtagg agtaaaccaa aaagtgtgat ttttcttttg 2058

tatgactcgt ttgttcttta ttagttggtg gtatgggttg gatcatttgt ttttaaaact 2118

acttaggtat gattcacata caaaaagctg cacatattta atgtatccta ttgtgtaatt 2178

aatttttaat tttttttgtg tacttcctaa acttatagtc ctgcgagtct gggaacagat 2238

ctgtttttca cttatcctga tttaatgaca gtttccaaca ttgttttgtt attacaagta 2298

ggggatcttt ttttttgccc gtttaatgaa gatactaaaa ataatgcact ggaaggagtg 2358

gaagagttgg aaaatttgta accatcataa tacaggtgta ataggtttgg gaaagaatcc 2418

tcaaaaatgt taaagcaagg gaggaaagtt tgttgagaag caagatgttc ttctctcctg 2478

cccgcccccg ccgttggttg ttggtggtca gaattattgt gtaataaata atagacattt 2538

tttcttatac tatgtgtatt gttccttttg tttccttttt aaacttctcc cctgctttat 2598

ttggatgggt caagtttctg ttctgtttcc ttcctttcta ttaatttgga aatgtccttg 2658

gctttacgat tctgcttgta gatacttccc ctgtttctaa cacatttcaa taaacttaaa 2718

tttctctata tacaaaataa attaataatt ggagtctacc agtaagacag tttatttact 2778

catcttcctg ccccagcaag ataaaaccta aggttacata cttgccaaat caccctaatt 2838

ttcacaggcc tcccccaaag ttcctatcaa tcacattcag tcccccactc cctgtctccc 2898

tgctatgctc ccagaatgct aagaggaaga cagttggagc tataaactcc ctgtgtctca 2958

gatgagttcc ctggcagagg catgacatcc tcgtgc 2994

<210> 115
<211> 499
<212> DNA
<213> Homo sapiens

<220>
<221> CDS
<222> (303)..(446)

<400> 115
gcagggagga gggagagctg ctgatggcct gagaggtcca accgggccct ggctgcagag 60

ctcaggtggt ggggcttgca gggcagggct gtggccaggg caaagagcgg ccatcacagc 120

ttacgcatga ctcgaaggtg tttatgagga cctactgtgt gccatgtgcc tactgaggct 180

tacacattca aggcgttatc ggaccctgac cagtgctccc aggtgccctg taacacagtc 240

tgctggcctc tgggagggca gtctcctaag ttctttatgg ggtctgagca gggattgcag 300

gg atg atg cct cca gga gac caa ggt gga cca gga gct cct gcc ttc 347
Met Met Pro Pro Gly Asp Gln Gly Gly Pro Gly Ala Pro Ala Phe
1               5                   10                  15

tgt gag aac cac ctt ggg cag tct ttc cga ggt ggt ggc aag agc ctg 395
Cys Glu Asn His Leu Gly Gln Ser Phe Arg Gly Gly Gly Lys Ser Leu
                20                  25                  30

ggg ccc agc ctc cca ggg tcc cct ctc caa ggc cat ggc ttt act aac 443
Gly Pro Ser Leu Pro Gly Ser Pro Leu Gln Gly His Gly Phe Thr Asn
            35                  40                  45

atg taaagtgacc aaggcctttg tagtgtctga gtgcaggcac cacaggcctg tgt 499
Met

<210> 116
<211> 1113
<212> DNA
<213> Homo sapiens

<220>
<221> CDS
<222> (35)..(505)

<400> 116
ccgggccgtc gtgggctccg gcttgcgtgc ggag atg agc ggg tcc ctc ggc 52
Met Ser Gly Ser Leu Gly
1               5

cga gct gcg gcg gct ctg ctc cgc tgg ggg cgc ggc gcg ggc ggc ggt 100
Arg Ala Ala Ala Ala Leu Leu Arg Trp Gly Arg Gly Ala Gly Gly Gly
            10                  15                  20

ggc ctt tgg ggt ccg ggc gtg cgg gcg gcg ggc tcg ggc gcg ggc ggc 148
Gly Leu Trp Gly Pro Gly Val Arg Ala Ala Gly Ser Gly Ala Gly Gly
        25                  30                  35

ggc ggc tcg gcg gag cag ttg gac gcg ctg gtg aag aag gac aag gtg 196
Gly Gly Ser Ala Glu Gln Leu Asp Ala Leu Val Lys Lys Asp Lys Val
    40                  45                  50

gtg gtc ttc ctc aag ggg acg ccg gag cag ccc cag tgc ggc ttc agc 244
Val Val Phe Leu Lys Gly Thr Pro Glu Gln Pro Gln Cys Gly Phe Ser
55                  60                  65                  70

aac gcc gtg gtg cag atc ctg cgg ctg cac ggc gtc cgc gat tac gcg 292
Asn Ala Val Val Gln Ile Leu Arg Leu His Gly Val Arg Asp Tyr Ala

                75                  80                  85

gcc tac aac gtg ctg gac gac ccg gag ctc cga caa ggc att aaa gac 340
Ala Tyr Asn Val Leu Asp Asp Pro Glu Leu Arg Gln Gly Ile Lys Asp
            90                  95                  100

tat tcc aac tgg ccc acc atc ccg caa gtg tac ctc aat ggc gag ttt 388
Tyr Ser Asn Trp Pro Thr Ile Pro Gln Val Tyr Leu Asn Gly Glu Phe
        105                 110                 115

gta ggg ggc tgt gac att ctt ctg cag atg cac cag aat ggg gac ttg 436
Val Gly Gly Cys Asp Ile Leu Leu Gln Met His Gln Asn Gly Asp Leu
    120                 125                 130

gtg gaa gaa ctg aaa aag ctg ggg atc cac tcc gcc ctt tta gat gaa 484
Val Glu Glu Leu Lys Lys Leu Gly Ile His Ser Ala Leu Leu Asp Glu
135                 140                 145                 150

aag aaa gac caa gac tcc aag tg agggcggcca agtcctcgct gagcagagag 537
Lys Lys Asp Gln Asp Ser Lys
                155

ggagccgttc atgtcagaga ctcactgcca gaaaagcctt acccattttg gttttcacta 597

ttgagaccgc aactgcttgc actgatcatt ttggttcgtg agcagttggt gattttagtt 657

ggtctggtgt tcgggctaag aatattttat tgtggactta attacaacca ctgcactgta 717

atgattcaat gctgtattat gatattgctg taaacaaaat tcattcttat attgtcactt 777

attctttgcc tgattcagaa gttaaatagg agctttggaa tcattattca tgacccctct 837

gcaaatgtgt cagtctccaa agagagtatc tccccccaaa ttttgtgtag cttcttttgt 897

tatggaaaat ggtggacaaa aaaagaaact gtgataactg gggcgttgtt ttttaaaata 957

aactccagca cagggatgct gtgcatgcct gagttgattc cgaagtgcat atgtctgtaa 1017

ggatttggag tgcctgcagt gttttatgtg tgggaagtaa gggtgagtct catattcttc 1077

tattaaattt gccacaagaa ttgcaaaaaa aaaaaa 1113

<210> 117
<211> 3528
<212> DNA
<213> Homo sapiens

<220>
<221> CDS
<222> (213)..(3203)

<400> 117
gttgactgtg acacgggtgt tacatatctt cctgtgcccc ttctccctgt agggtgaagc 60

tctgggcctc ggcttttggt ggggagataa aatccattgc tgctaagtac tccggttccc 120

agcttctgca aaagaaatac aaagagtatg agaaagacgt tgccatagaa gaaatcgatg 180

gcctccaact ggtaaagaag ctggcaaaga ac atg gaa gag atg ttt cac aag 233
Met Glu Glu Met Phe His Lys
1               5

aag tct gag gcc gtc agg cgt ctg gtg gag gct gca gaa gaa gca cac 281
Lys Ser Glu Ala Val Arg Arg Leu Val Glu Ala Ala Glu Glu Ala His
        10                  15                  20

ctg aaa cat gaa ttt gat gca gac tta cag tat gaa tac ttc aat gct 329
Leu Lys His Glu Phe Asp Ala Asp Leu Gln Tyr Glu Tyr Phe Asn Ala
    25                  30                  35

gtg ctg ata aat gaa agg gac aaa gac ggg aat ttt ttg gag ctg gga 377
Val Leu Ile Asn Glu Arg Asp Lys Asp Gly Asn Phe Leu Glu Leu Gly
40                  45                  50                  55

aag gaa ttc atc tta gcc cca aat gac cat ttt aat aat ttg cct gtg 425
Lys Glu Phe Ile Leu Ala Pro Asn Asp His Phe Asn Asn Leu Pro Val
                60                  65                  70

aac atc agt cta agt gac gtc caa gta cca acg aac atg tac aac aaa 473
Asn Ile Ser Leu Ser Asp Val Gln Val Pro Thr Asn Met Tyr Asn Lys
            75                  80                  85

gac cct gca att gtc aat ggg gtt tat tgg tct gaa tct cta aac aaa 521
Asp Pro Ala Ile Val Asn Gly Val Tyr Trp Ser Glu Ser Leu Asn Lys
        90                  95                  100

gtt ttt gta gat aac ttt gac cgt gac cca tct ctc ata tgg cag tac 569
Val Phe Val Asp Asn Phe Asp Arg Asp Pro Ser Leu Ile Trp Gln Tyr
    105                 110                 115

ttt gga agt gca aag ggc ttt ttt agg cag tat ccg ggg att aaa tgg 617
Phe Gly Ser Ala Lys Gly Phe Phe Arg Gln Tyr Pro Gly Ile Lys Trp
120                 125                 130                 135

gaa cca gat gag aat gga gtc att gcc ttc gac tgc agg aac cga aaa 665
Glu Pro Asp Glu Asn Gly Val Ile Ala Phe Asp Cys Arg Asn Arg Lys
                140                 145                 150

tgg tac atc cag gca gca act tct ccg aaa gac gtg gtc att tta gtt 713
Trp Tyr Ile Gln Ala Ala Thr Ser Pro Lys Asp Val Val Ile Leu Val
            155                 160                 165

gac gtc agt ggc agc atg aaa gga ctc cgt ctg act atc gcg aag caa 761
Asp Val Ser Gly Ser Met Lys Gly Leu Arg Leu Thr Ile Ala Lys Gln
        170                 175                 180

aca gtc tca tcc att ttg gat aca ctt ggg gat gat gac ttc ttc aac 809
Thr Val Ser Ser Ile Leu Asp Thr Leu Gly Asp Asp Asp Phe Phe Asn
    185                 190                 195

ata att gct tat aat gag gag ctt cac tat gtg gaa cct tgc ctg aat 857
Ile Ile Ala Tyr Asn Glu Glu Leu His Tyr Val Glu Pro Cys Leu Asn
200                 205                 210                 215

gga act ttg gtg caa gcc gac agg aca aac aaa gag cac ttc agg gag 905
Gly Thr Leu Val Gln Ala Asp Arg Thr Asn Lys Glu His Phe Arg Glu
                220                 225                 230

cat ctg gac aaa ctt ttc gcc aaa gga att gga atg ttg gat ata gct 953
His Leu Asp Lys Leu Phe Ala Lys Gly Ile Gly Met Leu Asp Ile Ala
            235                 240                 245

ctg aat gag gcc ttc aac att ctg agt gat ttc aac cac acg gga caa 1001
Leu Asn Glu Ala Phe Asn Ile Leu Ser Asp Phe Asn His Thr Gly Gln
        250                 255                 260

gga agt atc tgc agt cag gcc atc atg ctc ata act gat ggg gcg gtg 1049
Gly Ser Ile Cys Ser Gln Ala Ile Met Leu Ile Thr Asp Gly Ala Val
    265                 270                 275

gac acc tat gat aca atc ttt gca aaa tac aat tgg cca gat cga aag 1097
Asp Thr Tyr Asp Thr Ile Phe Ala Lys Tyr Asn Trp Pro Asp Arg Lys
280                 285                 290                 295

gtt cgc atc ttc aca tac ctc att gga cga gag gct gcg ttt gca gac 1145
Val Arg Ile Phe Thr Tyr Leu Ile Gly Arg Glu Ala Ala Phe Ala Asp
                300                 305                 310

aat cta aag tgg atg gcc tgt gcc aac aaa gga ttt ttt acc caa atc 1193
Asn Leu Lys Trp Met Ala Cys Ala Asn Lys Gly Phe Phe Thr Gln Ile
            315                 320                 325

tcc acc ttg gct gat gtg cag gag aat gtc atg gaa tac ctt cac gtg 1241
Ser Thr Leu Ala Asp Val Gln Glu Asn Val Met Glu Tyr Leu His Val
        330                 335                 340

ctt agc cgg ccc aaa gtc atc gac cag gag cat gat gtg gtg tgg acc 1289
Leu Ser Arg Pro Lys Val Ile Asp Gln Glu His Asp Val Val Trp Thr
    345                 350                 355

gaa gct tac att gac agc act ctc cct cag gca caa aag ctg act gat 1337
Glu Ala Tyr Ile Asp Ser Thr Leu Pro Gln Ala Gln Lys Leu Thr Asp
360                 365                 370                 375

gat cag ggc ccc gtc ctg atg acc act gta gcc atg cct gtg ttt agt 1385
Asp Gln Gly Pro Val Leu Met Thr Thr Val Ala Met Pro Val Phe Ser
                380                 385                 390

aag cag aac gaa acc aga tcg aag ggc att ctt ctg gga gtg gtt ggc 1433
Lys Gln Asn Glu Thr Arg Ser Lys Gly Ile Leu Leu Gly Val Val Gly
            395                 400                 405

aca gat gtc cca gtg aaa gaa ctt ctg aag acc atc ccc aaa tac aag 1481
Thr Asp Val Pro Val Lys Glu Leu Leu Lys Thr Ile Pro Lys Tyr Lys
        410                 415                 420

tta ggg att cac ggt tat gcc ttt gca atc aca aat aat gga tat atc 1529
Leu Gly Ile His Gly Tyr Ala Phe Ala Ile Thr Asn Asn Gly Tyr Ile
    425                 430                 435

ctg acg cat ccg gaa ctc agg ctg ctg tac gaa gaa gga aaa aag cga 1577
Leu Thr His Pro Glu Leu Arg Leu Leu Tyr Glu Glu Gly Lys Lys Arg
440                 445                 450                 455

agg aaa cct aac tat agt agc gtt gac ctc tct gag gtg gag tgg gaa 1625
Arg Lys Pro Asn Tyr Ser Ser Val Asp Leu Ser Glu Val Glu Trp Glu
                460                 465                 470

gac cga gat gac gtg ttg aga aat gct atg gtg aat cga aag acg ggg 1673
Asp Arg Asp Asp Val Leu Arg Asn Ala Met Val Asn Arg Lys Thr Gly
            475                 480                 485

aag ttt tcc atg gag gtg aag aag aca gtg gac aaa ggg aaa cgg gtt 1721
Lys Phe Ser Met Glu Val Lys Lys Thr Val Asp Lys Gly Lys Arg Val
        490                 495                 500

ttg gtg atg aca aat gac tac tat tat aca gac atc aag ggt act cct 1769
Leu Val Met Thr Asn Asp Tyr Tyr Tyr Thr Asp Ile Lys Gly Thr Pro
    505                 510                 515

ttc agt tta ggt gtg gcg ctt tcc aga ggt cat ggg aaa tat ttc ttc 1817
Phe Ser Leu Gly Val Ala Leu Ser Arg Gly His Gly Lys Tyr Phe Phe
520                 525                 530                 535

cga ggg aat gta acc atc gaa gaa ggc ctg cat gac tta gaa cat ccc 1865
Arg Gly Asn Val Thr Ile Glu Glu Gly Leu His Asp Leu Glu His Pro
                540                 545                 550

gat gtg tcc ttg gca gat gaa tgg tcc tac tgc aac act gac cta cac 1913
Asp Val Ser Leu Ala Asp Glu Trp Ser Tyr Cys Asn Thr Asp Leu His
            555                 560                 565

cct gag cac cgc cat ctg tct cag tta gaa gcg att aag ctc tac cta 1961
Pro Glu His Arg His Leu Ser Gln Leu Glu Ala Ile Lys Leu Tyr Leu
        570                 575                 580

aaa ggc aaa gaa cct ctg ctc cag tgt gat aaa gaa ttg atc caa gaa 2009
Lys Gly Lys Glu Pro Leu Leu Gln Cys Asp Lys Glu Leu Ile Gln Glu
    585                 590                 595

gtc ctt ttt gac gcg gtg gtg agt gcc ccc att gaa gcg tat tgg acc 2057
Val Leu Phe Asp Ala Val Val Ser Ala Pro Ile Glu Ala Tyr Trp Thr
600                 605                 610                 615

agc ctg gcc ctc aac aaa tct gaa aat tct gac aag ggc gtg gag gtt 2105
Ser Leu Ala Leu Asn Lys Ser Glu Asn Ser Asp Lys Gly Val Glu Val
                620                 625                 630

gcc ttc ctc ggc act cgc acg ggc ctc tcc aga atc aac ctg ttt gtc 2153
Ala Phe Leu Gly Thr Arg Thr Gly Leu Ser Arg Ile Asn Leu Phe Val
            635                 640                 645

ggg gct gag cag ctc acc aat cag gac ttc ctg aaa gct ggt gac aag 2201
Gly Ala Glu Gln Leu Thr Asn Gln Asp Phe Leu Lys Ala Gly Asp Lys
        650                 655                 660

gag aac att ttt aac gca gac cat ttc cct ctc tgg tac cga aga gcc 2249
Glu Asn Ile Phe Asn Ala Asp His Phe Pro Leu Trp Tyr Arg Arg Ala
    665                 670                 675

gct gag cag att cca ggg agc ttc gtc tac tcg atc cca ttc agc act 2297
Ala Glu Gln Ile Pro Gly Ser Phe Val Tyr Ser Ile Pro Phe Ser Thr
680                 685                 690                 695

gga cca gtc aat aaa agc aat gtg gtg aca gca agt aca tcc atc cag 2345
Gly Pro Val Asn Lys Ser Asn Val Val Thr Ala Ser Thr Ser Ile Gln
                700                 705                 710

ctc ctg gat gaa cgg aaa tct cct gtg gtg gca gct gta ggc att cag 2393
Leu Leu Asp Glu Arg Lys Ser Pro Val Val Ala Ala Val Gly Ile Gln
            715                 720                 725

atg aaa ctt gaa ttt ttc caa agg aag ttc tgg act gcc agc aga cag 2441
Met Lys Leu Glu Phe Phe Gln Arg Lys Phe Trp Thr Ala Ser Arg Gln
        730                 735                 740

tgt gct tcc ctg gat ggc aaa tgc tcc atc agc tgt gat gat gag act 2489
Cys Ala Ser Leu Asp Gly Lys Cys Ser Ile Ser Cys Asp Asp Glu Thr
    745                 750                 755

gtg aat tgt tac ctc ata gac aat aat gga ttt att ttg gtg tct gaa 2537
Val Asn Cys Tyr Leu Ile Asp Asn Asn Gly Phe Ile Leu Val Ser Glu
760                 765                 770                 775

gac tac aca cag act gga gac ttt ttt ggt gag atc gag gga gct gtg 2585
Asp Tyr Thr Gln Thr Gly Asp Phe Phe Gly Glu Ile Glu Gly Ala Val
                780                 785                 790

atg aac aaa ttg cta aca atg ggc tcc ttt aaa aga att acc ctt tat 2633
Met Asn Lys Leu Leu Thr Met Gly Ser Phe Lys Arg Ile Thr Leu Tyr
            795                 800                 805

gac tac caa gcc atg tgt aga gcc aac aag gaa agc agc gat ggc gcc 2681
Asp Tyr Gln Ala Met Cys Arg Ala Asn Lys Glu Ser Ser Asp Gly Ala
        810                 815                 820

cat ggc ctc ctg gat cct tat aat gcc ttc ctc tct gca gta aaa tgg 2729
His Gly Leu Leu Asp Pro Tyr Asn Ala Phe Leu Ser Ala Val Lys Trp
    825                 830                 835

atc atg aca gaa ctt gtc ttg ttc ctg gtg gaa ttt aac ctc tgc agt 2777
Ile Met Thr Glu Leu Val Leu Phe Leu Val Glu Phe Asn Leu Cys Ser
840                 845                 850                 855

tgg tgg cac tcc gat atg aca gct aaa gcc cag aaa ttg aaa cag acc 2825
Trp Trp His Ser Asp Met Thr Ala Lys Ala Gln Lys Leu Lys Gln Thr
                860                 865                 870

ctg gag cct tgt gat act gaa tat cca gca ttc gtc tct gag cgc acc 2873
Leu Glu Pro Cys Asp Thr Glu Tyr Pro Ala Phe Val Ser Glu Arg Thr
            875                 880                 885

atc aag gag act aca ggg aat att gct tgt gaa gac tgc tcc aag tcc 2921
Ile Lys Glu Thr Thr Gly Asn Ile Ala Cys Glu Asp Cys Ser Lys Ser
        890                 895                 900

ttt gtc atc cag caa atc cca agc agc aac ctg ttc atg gtg gtg gtg 2969
Phe Val Ile Gln Gln Ile Pro Ser Ser Asn Leu Phe Met Val Val Val
    905                 910                 915

gac agc agc tgc ctc tgt gaa tct gtg gcc ccc atc acc atg gca ccc 3017
Asp Ser Ser Cys Leu Cys Glu Ser Val Ala Pro Ile Thr Met Ala Pro
920                 925                 930                 935

att gaa atc agg tat aat gaa tcc ctt aag tgt gaa cgt cta aag gcc 3065
Ile Glu Ile Arg Tyr Asn Glu Ser Leu Lys Cys Glu Arg Leu Lys Ala
                940                 945                 950

cag aag atc aga agg cgc cca gaa tct tgt cat ggc ttc cat cct gag     3113
Gln Lys Ile Arg Arg Arg Pro Glu Ser Cys His Gly Phe His Pro Glu
            955                 960                 965

gag aat gca agg gag tgt ggg ggt gcg ccg agt ctc caa gcc cag aca     3161
Glu Asn Ala Arg Glu Cys Gly Gly Ala Pro Ser Leu Gln Ala Gln Thr
        970                 975                 980

gtc ctc ctt ctg ctc cct ctg ctt ttg atg ctc ttc tca agg tgacact     3210
Val Leu Leu Leu Leu Pro Leu Leu Leu Met Leu Phe Ser Arg
    985                 990                 995

gactgagatg ttctcttggc atgctaaatc atggataaac tgtgaaccaa aatatggtgc   3270

aacatacgag acatgaatat agtccaacca tcagcatctc atcatgattt taaactgtgc   3330

gtgatataaa ctcttaaaga tatgttgaca aaaagttatc tttttacttt gccagtcatg   3390

caaatgtgag tttgccacat gataatcacc cttcatcaga aatgggaccg caagtggtag   3450

gcagtgtccc ttctgcttga aacctattga aaccaattta aaactgtgta ctttttaaat   3510

aaagtatatt aaaatcat                                                 3528

<210> 118
<211> 1957
<212> DNA
<213> Homo sapiens

<220>
<221> CDS
<222> (124)..(1470)

<400> 118
gagatgggtt ggctgcagta gtgagaggct gggggtgcgg ctctttccct gcagtcctgc     60

cgaggaagcg tgcgtccctg gcgcttcctt cttctcttcc ggcggagagc ttgggatgtg    120

gta atg cca gcc aca ctc ctc aga gcc gtg gcc aga tct cat cat ata      168
    Met Pro Ala Thr Leu Leu Arg Ala Val Ala Arg Ser His His Ile
    1               5                   10                  15

tta tca aaa gca cat cag tgc cga aga atc ggt cat cta atg tta aaa      216
Leu Ser Lys Ala His Gln Cys Arg Arg Ile Gly His Leu Met Leu Lys
                20                  25                  30

cca ctt aag gaa ttt gaa aat aca aca tgc agc aca ctg aca ata cgt      264
Pro Leu Lys Glu Phe Glu Asn Thr Thr Cys Ser Thr Leu Thr Ile Arg
            35                  40                  45

caa agc ttg gat ttg ttc ctt cct gat aaa aca gct agt ggt ttg aat      312
Gln Ser Leu Asp Leu Phe Leu Pro Asp Lys Thr Ala Ser Gly Leu Asn
        50                  55                  60

aag tct cag atc ctg gaa atg aac caa aaa aag tca gat acc agc atg      360
Lys Ser Gln Ile Leu Glu Met Asn Gln Lys Lys Ser Asp Thr Ser Met

65                  70                  75

ctg tct cca tta aat gct gct cgt tgc caa gat gaa aag gca cac ctt        408
Leu Ser Pro Leu Asn Ala Ala Arg Cys Gln Asp Glu Lys Ala His Leu
 80                  85                  90                  95

cca acc atg aaa tcc ttt ggt act cac agg aga gtg acc cac aaa cca        456
Pro Thr Met Lys Ser Phe Gly Thr His Arg Arg Val Thr His Lys Pro
                100                 105                 110

aat ctg ttg ggt tct aaa tgg ttt ata aaa ata tta aag agg cat ttc        504
Asn Leu Leu Gly Ser Lys Trp Phe Ile Lys Ile Leu Lys Arg His Phe
            115                 120                 125

tca tct gta tca acg gaa aca ttt gtt cca aaa caa gac ttc cca cag        552
Ser Ser Val Ser Thr Glu Thr Phe Val Pro Lys Gln Asp Phe Pro Gln
        130                 135                 140

gtg aag aga cca cta aaa gca tcc agg acc aga cag cca tcc agg acc        600
Val Lys Arg Pro Leu Lys Ala Ser Arg Thr Arg Gln Pro Ser Arg Thr
    145                 150                 155

aac ctt cca gtt ctg tct gtg aac gag gac cca atg cac tgc aca gca        648
Asn Leu Pro Val Leu Ser Val Asn Glu Asp Pro Met His Cys Thr Ala
160                 165                 170                 175

ttt gca acg gca gat gag tat cat ctg gga aat ctg tct caa gat ctg        696
Phe Ala Thr Ala Asp Glu Tyr His Leu Gly Asn Leu Ser Gln Asp Leu
                180                 185                 190

gcc tcc cac gga tat gtt gaa gta aca agc ttg cct aga gat gca gca        744
Ala Ser His Gly Tyr Val Glu Val Thr Ser Leu Pro Arg Asp Ala Ala
            195                 200                 205

aat att ttg gtg atg ggt gtg gaa aat tct gca aaa gaa ggt gat cct        792
Asn Ile Leu Val Met Gly Val Glu Asn Ser Ala Lys Glu Gly Asp Pro
        210                 215                 220

gga aca ata ttc ttc ttc agg gaa gga gct gct gtg ttt tgg aat gtg        840
Gly Thr Ile Phe Phe Phe Arg Glu Gly Ala Ala Val Phe Trp Asn Val
    225                 230                 235

aaa gac aaa act atg aag cat gtg atg aaa gtt cta gaa aaa cat gaa        888
Lys Asp Lys Thr Met Lys His Val Met Lys Val Leu Glu Lys His Glu
240                 245                 250                 255

att cag ccc tat gaa atc gca ctg gta cac tgg gaa aat gaa gaa ctt        936
Ile Gln Pro Tyr Glu Ile Ala Leu Val His Trp Glu Asn Glu Glu Leu
                260                 265                 270

aac tac ata aaa ata gag gga cag tca aaa ctt cac agg ggg gaa atc        984
Asn Tyr Ile Lys Ile Glu Gly Gln Ser Lys Leu His Arg Gly Glu Ile
            275                 280                 285

aag tta aat tca gag ctg gat tta gat gat gcc att cta gag aag ttt       1032
Lys Leu Asn Ser Glu Leu Asp Leu Asp Asp Ala Ile Leu Glu Lys Phe
        290                 295                 300

gct ttc tcc aat gct cta tgc ctt tct gta aaa ctg gca att tgg gaa       1080
Ala Phe Ser Asn Ala Leu Cys Leu Ser Val Lys Leu Ala Ile Trp Glu

305                 310                 315

gca tca ctg gat aaa ttt att gaa tct att cag tca att cct gag gct      1128
Ala Ser Leu Asp Lys Phe Ile Glu Ser Ile Gln Ser Ile Pro Glu Ala
320                 325                 330                 335

tta aaa gct ggg aag aaa gtg aaa cta tct cat gaa gaa gtt atg cag      1176
Leu Lys Ala Gly Lys Lys Val Lys Leu Ser His Glu Glu Val Met Gln
                340                 345                 350

aaa atc ggt gaa ctc ttt gct cta agg cac cgt ata aac ttg agt tca      1224
Lys Ile Gly Glu Leu Phe Ala Leu Arg His Arg Ile Asn Leu Ser Ser
            355                 360                 365

gac ttc ctg att act cct gat ttc tac tgg gac aga gaa aac ctg gaa      1272
Asp Phe Leu Ile Thr Pro Asp Phe Tyr Trp Asp Arg Glu Asn Leu Glu
        370                 375                 380

gga ctt tac gat aaa acg tgt caa ttc ctt agc att ggc cga aga gtt      1320
Gly Leu Tyr Asp Lys Thr Cys Gln Phe Leu Ser Ile Gly Arg Arg Val
    385                 390                 395

aag gtc atg aat gaa aaa ctt cag cac tgc atg gaa cta aca gat cta      1368
Lys Val Met Asn Glu Lys Leu Gln His Cys Met Glu Leu Thr Asp Leu
400                 405                 410                 415

atg cgg aat cac ctg aat gag aag agg gca ctc cgc ttg gag tgg atg      1416
Met Arg Asn His Leu Asn Glu Lys Arg Ala Leu Arg Leu Glu Trp Met
                420                 425                 430

att gtc atc ctc att acc ata gag gta atg ttt gag ctg gga cga gta      1464
Ile Val Ile Leu Ile Thr Ile Glu Val Met Phe Glu Leu Gly Arg Val
            435                 440                 445

ttt ttc tgatcaagtg ataaccaaag tgtcactgca agagatattc aagttctaca      1520
Phe Phe

atcaaaaatt aaatgttcgg cccggcgcgg tgcctcatgc ctgtaatccc agcactttcg   1580

gaggccaaga agggtggctt gagatgagat caggagctca agacaagcct ggccaacatg   1640

gtgaaacccc atctctacta aaaataccaa aattagccag gtgtgttggc acacgcccgt   1700

catctcagct actcaggagg ctgaggcagg agaatctctt gaacttggga ggcggaggtt   1760

gcagtgagct aagatcacac cactgcactc cagccagggc aacagtgaga ctcagtctca   1820

aaaataaaca ataaaataaa taaataaatg ttcactactg ggtgatcatt taataggtgt   1880

ttttttaatc aagaaattat ctttttcagc ccagtatatc gtgtgaataa aattatgaag   1940

aatctaaaaa aaaaaaa                                                  1957

<210> 119
<211> 2665
<212> DNA

<213> Homo sapiens

<220>
<221> CDS
<222> (95)..(2608)

<400> 119
gcacgagctg ggccggcagc ggttgtgagg agttagctcg cggcattgca ggctctgaga      60

ggaggggacc cggttcccgg gtgagtgtcc aggc atg cca gcg gaa cgg ccc      112
                                      Met Pro Ala Glu Arg Pro
                                      1               5

gcg ggc agc ggc ggc tcg gag gct cca gca atg gtt gaa caa ctg gac      160
Ala Gly Ser Gly Gly Ser Glu Ala Pro Ala Met Val Glu Gln Leu Asp
            10                  15                  20

act gct gtg att acc ccg gcc atg cta gaa gag gaa gaa cag ctt gaa      208
Thr Ala Val Ile Thr Pro Ala Met Leu Glu Glu Glu Glu Gln Leu Glu
        25                  30                  35

gct gct gga cta gag aga gag cgg aag atg ctg gaa aag gct cgc atg      256
Ala Ala Gly Leu Glu Arg Glu Arg Lys Met Leu Glu Lys Ala Arg Met
    40                  45                  50

tct tgg gat aga gag tcg aca gaa att cgg tac cgt aga ctt caa cat      304
Ser Trp Asp Arg Glu Ser Thr Glu Ile Arg Tyr Arg Arg Leu Gln His
55                  60                  65                  70

ttg ctt gaa aaa agc aat atc tac tcc aaa ttt tta ttg acg aaa atg      352
Leu Leu Glu Lys Ser Asn Ile Tyr Ser Lys Phe Leu Leu Thr Lys Met
                75                  80                  85

gaa cag caa caa tta gag gaa cag aag aag aaa gaa aaa ttg gag aga      400
Glu Gln Gln Gln Leu Glu Glu Gln Lys Lys Lys Glu Lys Leu Glu Arg
            90                  95                  100

aaa aag gag tct tta aaa gtt aaa aag ggt aaa aat tca att gat gca      448
Lys Lys Glu Ser Leu Lys Val Lys Lys Gly Lys Asn Ser Ile Asp Ala
        105                 110                 115

agt gaa gag aag cca gtt atg agg aaa aaa aga gga aga gaa gat gaa      496
Ser Glu Glu Lys Pro Val Met Arg Lys Lys Arg Gly Arg Glu Asp Glu
    120                 125                 130

tca tac aat att tca gag gtc atg tca aaa gag gaa att ttg tct gtg      544
Ser Tyr Asn Ile Ser Glu Val Met Ser Lys Glu Glu Ile Leu Ser Val
135                 140                 145                 150

gct aaa aaa aat aaa aag gag aat gag gat gaa aac tcc tcc tct act      592
Ala Lys Lys Asn Lys Lys Glu Asn Glu Asp Glu Asn Ser Ser Ser Thr
                155                 160                 165

aat ctc tgt gtg gaa gat ctt cag aaa aat aaa gat tcg aat agt ata      640
Asn Leu Cys Val Glu Asp Leu Gln Lys Asn Lys Asp Ser Asn Ser Ile
            170                 175                 180

att aaa gat aga ttg tct gaa acg gtt agg cag aat act aaa ttc ttt      688
Ile Lys Asp Arg Leu Ser Glu Thr Val Arg Gln Asn Thr Lys Phe Phe
        185                 190                 195

ttt gac cca gtc cgg aag tgt aat ggt cag cca gta cct ttt caa caa 736
Phe Asp Pro Val Arg Lys Cys Asn Gly Gln Pro Val Pro Phe Gln Gln
    200                 205                 210

cca aag cac ttc act gga gga gtg atg cga tgg tac caa gta gaa ggc 784
Pro Lys His Phe Thr Gly Gly Val Met Arg Trp Tyr Gln Val Glu Gly
215                 220                 225                 230

atg gaa tgg ctt agg atg ctt tgg gaa aat gga att aat ggc att tta 832
Met Glu Trp Leu Arg Met Leu Trp Glu Asn Gly Ile Asn Gly Ile Leu
                235                 240                 245

gca gat gaa atg gga ttg ggt aag aca gtt cag tgc att gct act att 880
Ala Asp Glu Met Gly Leu Gly Lys Thr Val Gln Cys Ile Ala Thr Ile
            250                 255                 260

gca ttg atg att cag aga gga gta cca gga cct ttt ctt gtc tgt ggc 928
Ala Leu Met Ile Gln Arg Gly Val Pro Gly Pro Phe Leu Val Cys Gly
        265                 270                 275

cct ttg tct aca ctt cct aac tgg atg gct gaa ttc aaa aga ttt aca 976
Pro Leu Ser Thr Leu Pro Asn Trp Met Ala Glu Phe Lys Arg Phe Thr
    280                 285                 290

cca gat atc cct aca atg tta tat cat gga acc cag gag gaa cgt caa 1024
Pro Asp Ile Pro Thr Met Leu Tyr His Gly Thr Gln Glu Glu Arg Gln
295                 300                 305                 310

aaa ttg gta aga aat att tac aaa cgg aaa ggg act ttg cag att cat 1072
Lys Leu Val Arg Asn Ile Tyr Lys Arg Lys Gly Thr Leu Gln Ile His
                315                 320                 325

cct gtg gta atc acg tca ttt gaa ata gcc atg aga gac cga aat gcg 1120
Pro Val Val Ile Thr Ser Phe Glu Ile Ala Met Arg Asp Arg Asn Ala
            330                 335                 340

tta cag cat tgc tat tgg aaa tac tta ata gta gat gaa gga cac agg 1168
Leu Gln His Cys Tyr Trp Lys Tyr Leu Ile Val Asp Glu Gly His Arg
        345                 350                 355

att aag aat atg aag tgc cgt cta atc agg gag tta aaa cga ttc aat 1216
Ile Lys Asn Met Lys Cys Arg Leu Ile Arg Glu Leu Lys Arg Phe Asn
    360                 365                 370

gct gat aac aaa ctt ctt ttg act ggt act ccc ttg caa aac aat tta 1264
Ala Asp Asn Lys Leu Leu Leu Thr Gly Thr Pro Leu Gln Asn Asn Leu
375                 380                 385                 390

tca gaa ctt tgg tca ttg cta aac ttt ttg ttg cca gat gta ttt gat 1312
Ser Glu Leu Trp Ser Leu Leu Asn Phe Leu Leu Pro Asp Val Phe Asp
                395                 400                 405

gac ttg aaa agc ttt gag tct tgg ttt gac atc act agt ctt tct gaa 1360
Asp Leu Lys Ser Phe Glu Ser Trp Phe Asp Ile Thr Ser Leu Ser Glu
            410                 415                 420

act gct gaa gat att att gct aaa gaa aga gaa cag aat gta ttg cat 1408
Thr Ala Glu Asp Ile Ile Ala Lys Glu Arg Glu Gln Asn Val Leu His
        425                 430                 435

atg ctg cac cag att tta aca cct ttc tta ttg aga aga ctg aag tct 1456
Met Leu His Gln Ile Leu Thr Pro Phe Leu Leu Arg Arg Leu Lys Ser
    440                 445                 450

gat gtt gct ctt gaa gtt cct cct aaa cga gaa gta gtc gtt tat gct 1504
Asp Val Ala Leu Glu Val Pro Pro Lys Arg Glu Val Val Val Tyr Ala
455                 460                 465                 470

cca ctt tca aag aag cag gag atc ttt tat aca gcc att gtg aac cgt 1552
Pro Leu Ser Lys Lys Gln Glu Ile Phe Tyr Thr Ala Ile Val Asn Arg
                475                 480                 485

aca att gca aac atg tat gga tcc agt gag aaa gaa aca att gag tta 1600
Thr Ile Ala Asn Met Tyr Gly Ser Ser Glu Lys Glu Thr Ile Glu Leu
            490                 495                 500

agt cct act ggt aga cca aaa cga cga act aga aaa tca ata aat aac 1648
Ser Pro Thr Gly Arg Pro Lys Arg Arg Thr Arg Lys Ser Ile Asn Asn
        505                 510                 515

agc aaa ata gat gaa ttc cct aat gaa ttg gaa aaa ctg atc agt caa 1696
Ser Lys Ile Asp Glu Phe Pro Asn Glu Leu Glu Lys Leu Ile Ser Gln
    520                 525                 530

ata cag cca gag gtg gac cga gaa aga gct gtt gtg gaa gtg aat atc 1744
Ile Gln Pro Glu Val Asp Arg Glu Arg Ala Val Val Glu Val Asn Ile
535                 540                 545                 550

cct gta gaa tct gaa gtt aat ctg aag ctg cag aat ata atg atg cta 1792
Pro Val Glu Ser Glu Val Asn Leu Lys Leu Gln Asn Ile Met Met Leu
                555                 560                 565

ctt cgt aaa tgt tgt aat cat cca tat ttg att gaa tat cct ata gac 1840
Leu Arg Lys Cys Cys Asn His Pro Tyr Leu Ile Glu Tyr Pro Ile Asp
            570                 575                 580

cct gtt aca caa gaa ttt aag atc gat gaa gaa ttg gta aca aat tct 1888
Pro Val Thr Gln Glu Phe Lys Ile Asp Glu Glu Leu Val Thr Asn Ser
        585                 590                 595

ggg aag ttc ttg att ttg gat cga atg ctg cca gaa cta aaa aaa aga 1936
Gly Lys Phe Leu Ile Leu Asp Arg Met Leu Pro Glu Leu Lys Lys Arg
    600                 605                 610

ggt cac aag gtg ctg ctt ttt tca caa atg aca agc atg ttg gac att 1984
Gly His Lys Val Leu Leu Phe Ser Gln Met Thr Ser Met Leu Asp Ile
615                 620                 625                 630

ttg atg gat tac tgc cat ctc aga gat ttc aac ttc agc agg ctt gat 2032
Leu Met Asp Tyr Cys His Leu Arg Asp Phe Asn Phe Ser Arg Leu Asp
                635                 640                 645

ggg tcc atg tct tac tca gag aga gaa aaa aac atg cac agc ttc aac 2080
Gly Ser Met Ser Tyr Ser Glu Arg Glu Lys Asn Met His Ser Phe Asn
            650                 655                 660

acg gat cca gag gtg ttt atc ttc tta gtg agt aca cga gct ggt ggc 2128
Thr Asp Pro Glu Val Phe Ile Phe Leu Val Ser Thr Arg Ala Gly Gly
        665                 670                 675

ctg ggc att aat ctg act gca gca gat aca gtt atc att tat gat agt     2176
Leu Gly Ile Asn Leu Thr Ala Ala Asp Thr Val Ile Ile Tyr Asp Ser
    680                 685                 690

gat tgg aac ccc cag tcg gat ctt cag gcc cag gat aga tgt cat aga     2224
Asp Trp Asn Pro Gln Ser Asp Leu Gln Ala Gln Asp Arg Cys His Arg
695                 700                 705                 710

att ggt cag aca aag cca gtt gtt gtt tat cgc ctt gtt aca gca aat     2272
Ile Gly Gln Thr Lys Pro Val Val Val Tyr Arg Leu Val Thr Ala Asn
                715                 720                 725

act atc gat cag aaa att gtg gaa aga gca gct gct aaa agg aaa ctg     2320
Thr Ile Asp Gln Lys Ile Val Glu Arg Ala Ala Ala Lys Arg Lys Leu
            730                 735                 740

gaa aag ttg atc atc cat aaa aat cat ttc aaa ggt ggt cag tct gga     2368
Glu Lys Leu Ile Ile His Lys Asn His Phe Lys Gly Gly Gln Ser Gly
        745                 750                 755

tta aat ctg tct aag aat ttc tta gat cct aag gaa tta atg gaa tta     2416
Leu Asn Leu Ser Lys Asn Phe Leu Asp Pro Lys Glu Leu Met Glu Leu
    760                 765                 770

tta aaa tct aga gat tat gaa agg gaa ata aaa gga tca aga gag aag     2464
Leu Lys Ser Arg Asp Tyr Glu Arg Glu Ile Lys Gly Ser Arg Glu Lys
775                 780                 785                 790

gtc att agt gat aaa gat cta gag ttg ttg tta gat cga agt gat ctt     2512
Val Ile Ser Asp Lys Asp Leu Glu Leu Leu Leu Asp Arg Ser Asp Leu
                795                 800                 805

att gat caa atg aat gct tca gga cca att aaa gag aag atg ggg ata     2560
Ile Asp Gln Met Asn Ala Ser Gly Pro Ile Lys Glu Lys Met Gly Ile
            810                 815                 820

ttc aag ata tta gaa aat tct gaa gat tcc agt cct gaa tgt ttg ttt     2608
Phe Lys Ile Leu Glu Asn Ser Glu Asp Ser Ser Pro Glu Cys Leu Phe
        825                 830                 835

taaagtggag ctcaagaata gcttttaaaa gttcttattt acatctagtg atttccc      2665

<210> 120
<211> 2274
<212> DNA
<213> Homo sapiens

<220>
<221> CDS
<222> (1016)..(2221)

<400> 120
cactaggtga tttactctta gctgattcga aatcagaaac ataatcagca aggactgggg      60

aacctcccac agtactttct ctcgctgctg ttttctccac ccacacaagt ccaccacttt     120

taaaagcatt tcttccatct cgctggccac atcttcgtgg atgacagcgc tgccactgtg 180

cttcagaatc ctctatctct cccagatctt tcctggtgtt tctctcaggg tcagtggcat 240

ctaatctttg tgactctact cctggtttga gtcctcggtt aacagccttg gggaaactgt 300

aacataaact gcaacgctgt tggtgccgtt tcttcttaga cagggtggca gcgatcgcct 360

ccccccgcag ccggccttcc actgcgccag caagaccccc tcccaccaaa acgccgaggg 420

ctcccagagc gcaggcgccc cgggccccgc ccctttccga agactcggct cctattggcc 480

caggggccgg ggttggaacg ccgaagggag ttgctcctag gtcttgtccg tgccgggttc 540

gcttttcttt ccttcccgaa gcctggccct tagatctact atcgcagagt ctgcggccgc 600

caggaagccc agattcgagt gtccggagag taaccggaag tgctgtcccc aggcctaggg 660

gcggcgccgg cggctgccag ggagaggcaa gaattgagtg ttgtgaatag ttctgaacta 720

gagacctttt gaaaccaaaa ggaagatggt cttgagtctt tcttgttacg aatgctatct 780

tctttgagaa gtcaaattgt caggatcagg aatgtatgtt ttcttccatc tggattctag 840

ggttggaaat accaagtaag gaattaacat atatgatgct ggaaacttgt agaggctcat 900

gattgaaagg cctgaaacag atgtatcttc caagttctta tcaagaaaat gactgtgagg 960

gcaacgatgg gtcaagaaga ccagcggaaa actccctagg agagagccat agaaa atg 1018
Met
1

tac act tca gaa gag aaa tgt aat cag aga act caa aaa agg aaa ata 1066
Tyr Thr Ser Glu Glu Lys Cys Asn Gln Arg Thr Gln Lys Arg Lys Ile
            5                   10                  15

tat aat gta tgc cct cgg aag ggt aaa aag att ttt att cat atg cat 1114
Tyr Asn Val Cys Pro Arg Lys Gly Lys Lys Ile Phe Ile His Met His
        20                  25                  30

gag att att cag ata gat ggt cat ata tac cag tgc ctt gaa tgc aag 1162
Glu Ile Ile Gln Ile Asp Gly His Ile Tyr Gln Cys Leu Glu Cys Lys
    35                  40                  45

caa aac ttc tgt gaa aac tta gct ctt att atg tgt gag aga acc cat 1210
Gln Asn Phe Cys Glu Asn Leu Ala Leu Ile Met Cys Glu Arg Thr His
50                  55                  60                  65

act ggg gag aaa cct tat aaa tgt gat atg tgt gag aaa acc ttt gtc 1258
Thr Gly Glu Lys Pro Tyr Lys Cys Asp Met Cys Glu Lys Thr Phe Val
                70                  75                  80

caa agc tca gat ctt act tca cac cag agg atc cac aat tac gag aaa 1306
Gln Ser Ser Asp Leu Thr Ser His Gln Arg Ile His Asn Tyr Glu Lys
            85                  90                  95

cct tat aaa tgt agc aaa tgt gag aag agc ttt tgg cat cac tta gcg 1354
Pro Tyr Lys Cys Ser Lys Cys Glu Lys Ser Phe Trp His His Leu Ala
        100                 105                 110

ctt tca gga cat cag aga aca cat gca ggt aaa aaa ttc tat aca tgt 1402
Leu Ser Gly His Gln Arg Thr His Ala Gly Lys Lys Phe Tyr Thr Cys
    115                 120                 125

gac att tgt ggc aag aat ttt ggt cag agt tct gat ctg ctt gtc cac 1450
Asp Ile Cys Gly Lys Asn Phe Gly Gln Ser Ser Asp Leu Leu Val His
130                 135                 140                 145

cag cga agc cat act ggc gag aaa cca tat cta tgt agt gag tgt gac 1498
Gln Arg Ser His Thr Gly Glu Lys Pro Tyr Leu Cys Ser Glu Cys Asp
                150                 155                 160

aaa tgc ttc agt aga agt aca aac ctc ata agg cat cga aga act cac 1546
Lys Cys Phe Ser Arg Ser Thr Asn Leu Ile Arg His Arg Arg Thr His
            165                 170                 175

aca ggt gag aaa cca ttt aag tgt ctc gag tgt gaa aaa gct ttt agt 1594
Thr Gly Glu Lys Pro Phe Lys Cys Leu Glu Cys Glu Lys Ala Phe Ser
        180                 185                 190

ggg aaa tca gat ctt att agc cac cag aga act cac act ggg gaa agg 1642
Gly Lys Ser Asp Leu Ile Ser His Gln Arg Thr His Thr Gly Glu Arg
    195                 200                 205

ccc tac aaa tgt aat aag tgt gag aaa agt tac cga cac cgt tca gcc 1690
Pro Tyr Lys Cys Asn Lys Cys Glu Lys Ser Tyr Arg His Arg Ser Ala
210                 215                 220                 225

ttc att gta cat aaa aga gtt cat act ggg gag aag ccc tat aag tgt 1738
Phe Ile Val His Lys Arg Val His Thr Gly Glu Lys Pro Tyr Lys Cys
                230                 235                 240

ggt gcc tgt gaa aaa tgc ttt ggc cag aaa tca gac ctt atc gtg cac 1786
Gly Ala Cys Glu Lys Cys Phe Gly Gln Lys Ser Asp Leu Ile Val His
            245                 250                 255

cag aga gtc cac aca ggt gag aag ccg tat aaa tgc ctg gaa tgt atg 1834
Gln Arg Val His Thr Gly Glu Lys Pro Tyr Lys Cys Leu Glu Cys Met
        260                 265                 270

aga agt ttt act cgg agt gcc aac cta att agg cac cag gca act cac 1882
Arg Ser Phe Thr Arg Ser Ala Asn Leu Ile Arg His Gln Ala Thr His
    275                 280                 285

act cac act ttt aaa tgc ctt gaa tat gaa aaa agc ttt aac tgt agc 1930
Thr His Thr Phe Lys Cys Leu Glu Tyr Glu Lys Ser Phe Asn Cys Ser
290                 295                 300                 305

tca gat ctt att gta cat cag aga att cac atg gaa gag aaa cca cat 1978
Ser Asp Leu Ile Val His Gln Arg Ile His Met Glu Glu Lys Pro His
                310                 315                 320

cag tgg tct gcg tgt gag agt ggc ttc ctc cta gga atg gac ttt gtt 2026
Gln Trp Ser Ala Cys Glu Ser Gly Phe Leu Leu Gly Met Asp Phe Val
            325                 330                 335

gcc caa cag aaa atg aga act caa aca gag gag cta cac tat aaa tac 2074
Ala Gln Gln Lys Met Arg Thr Gln Thr Glu Glu Leu His Tyr Lys Tyr
        340                 345                 350

act gta tgt gat aaa agc ttc cac cag agt tca gcc ctt ctt caa cat     2122
Thr Val Cys Asp Lys Ser Phe His Gln Ser Ser Ala Leu Leu Gln His
    355                 360                 365

cag aca gta cac att ggt gaa aaa ccg ttt gtc tgt aat gtg agt gaa     2170
Gln Thr Val His Ile Gly Glu Lys Pro Phe Val Cys Asn Val Ser Glu
370                 375                 380                 385

aaa ggt ctt gag ctt agc cct ccc cat gcg tca gaa gcc tca cag atg     2218
Lys Gly Leu Glu Leu Ser Pro Pro His Ala Ser Glu Ala Ser Gln Met
                390                 395                 400

tct tgaccaggcg agaagctgta ataccaatat taaaaattat ttatgtatca gag     2274
Ser

<210> 121
<211> 2126
<212> DNA
<213> Homo sapiens

<220>
<221> CDS
<222> (90)..(1826)

<400> 121
ataaatgcta caaatgtgtg gtaaagcagg gacatacctt atttgttcct acagggtgga     60

tccatgctgt gctcacttct caggactgt atg gct ttt ggg ggg aac ttc ctg     113
                                Met Ala Phe Gly Gly Asn Phe Leu
                                1               5

cac aac ctt aac att ggc atg cag ctc agg tgt tat gag atg gag aaa     161
His Asn Leu Asn Ile Gly Met Gln Leu Arg Cys Tyr Glu Met Glu Lys
    10                  15                  20

agg cta aaa aca cca gat ctt ttc aaa ttc cct ttc ttt gaa gcc ata     209
Arg Leu Lys Thr Pro Asp Leu Phe Lys Phe Pro Phe Phe Glu Ala Ile
25                  30                  35                  40

tgt tgg ttt gta gcc aaa aac ttg ctg gaa acc ctg aaa gaa ctg aga     257
Cys Trp Phe Val Ala Lys Asn Leu Leu Glu Thr Leu Lys Glu Leu Arg
                45                  50                  55

gaa gat ggt ttc cag cct caa act tac cta gta cag gga gtg aaa gca     305
Glu Asp Gly Phe Gln Pro Gln Thr Tyr Leu Val Gln Gly Val Lys Ala
            60                  65                  70

ctg cat act gct tta aaa tta tgg atg aaa aaa gaa ctt gta tct gaa     353
Leu His Thr Ala Leu Lys Leu Trp Met Lys Lys Glu Leu Val Ser Glu
        75                  80                  85

cat gcc ttt gaa att cca gac aat gtt aga cct gga cac ctt att aaa     401
His Ala Phe Glu Ile Pro Asp Asn Val Arg Pro Gly His Leu Ile Lys
    90                  95                  100

gaa ctt tct aaa gta att cga gca ata gag gag gaa aac ggc aaa cca 449
Glu Leu Ser Lys Val Ile Arg Ala Ile Glu Glu Glu Asn Gly Lys Pro
105                 110                 115                 120

gtt aaa tct cag gga att cct att gtg tgt cca gtt tca cga tcc tca 497
Val Lys Ser Gln Gly Ile Pro Ile Val Cys Pro Val Ser Arg Ser Ser
                125                 130                 135

aat gaa gca act tcc cca tac cat tcc cga aga aag atg agg aaa ctt 545
Asn Glu Ala Thr Ser Pro Tyr His Ser Arg Arg Lys Met Arg Lys Leu
            140                 145                 150

cga gat cat aat gtc cga act cct tct aac cta gac atc cta gag ctc 593
Arg Asp His Asn Val Arg Thr Pro Ser Asn Leu Asp Ile Leu Glu Leu
        155                 160                 165

cac aca agg gag gtc ctc aaa cga tta gag atg tgt cca tgg gaa gag 641
His Thr Arg Glu Val Leu Lys Arg Leu Glu Met Cys Pro Trp Glu Glu
    170                 175                 180

gac atc ttg agc tct aaa ctg aat gga aaa ttc aac aaa cat ctc caa 689
Asp Ile Leu Ser Ser Lys Leu Asn Gly Lys Phe Asn Lys His Leu Gln
185                 190                 195                 200

cca tcc tcc aca gta cct gaa tgg aga gcg aaa gat aat gat cta cga 737
Pro Ser Ser Thr Val Pro Glu Trp Arg Ala Lys Asp Asn Asp Leu Arg
                205                 210                 215

tta ctg ctg aca aat gga aga ata att aaa gat gaa agg cag ccc ttt 785
Leu Leu Leu Thr Asn Gly Arg Ile Ile Lys Asp Glu Arg Gln Pro Phe
            220                 225                 230

gca gat caa agt ctt tat aca gca gat agt gaa aat gaa gag gat aaa 833
Ala Asp Gln Ser Leu Tyr Thr Ala Asp Ser Glu Asn Glu Glu Asp Lys
        235                 240                 245

aga agg aca aaa aag gca aaa atg aag ata gaa gag agt tca gga gta 881
Arg Arg Thr Lys Lys Ala Lys Met Lys Ile Glu Glu Ser Ser Gly Val
    250                 255                 260

gag gga gtg gaa cat gaa gaa tct caa aaa cca ctg aat ggg ttt ttt 929
Glu Gly Val Glu His Glu Glu Ser Gln Lys Pro Leu Asn Gly Phe Phe
265                 270                 275                 280

aca cgt gtg aaa tca gaa ctc agg agt aga tca tca gga tat tct gat 977
Thr Arg Val Lys Ser Glu Leu Arg Ser Arg Ser Ser Gly Tyr Ser Asp
                285                 290                 295

att tct gag tca gaa gac tcc gga ccc gag tgc act gca ctg aaa agt 1025
Ile Ser Glu Ser Glu Asp Ser Gly Pro Glu Cys Thr Ala Leu Lys Ser
            300                 305                 310

atc ttt acc act gaa gag tct gaa agt tca ggt gat gaa aag aaa caa 1073
Ile Phe Thr Thr Glu Glu Ser Glu Ser Ser Gly Asp Glu Lys Lys Gln
        315                 320                 325

gaa ata aca tcc aac ttt aag gag gaa tct aat gtg atg agg aac ttc 1121
Glu Ile Thr Ser Asn Phe Lys Glu Glu Ser Asn Val Met Arg Asn Phe
    330                 335                 340

ctt caa aag agc cag aag cca tct aga agt gaa att cca att aaa agg 1169
Leu Gln Lys Ser Gln Lys Pro Ser Arg Ser Glu Ile Pro Ile Lys Arg
345                 350                 355                 360

gaa tgt cct acc tcg acg agc aca gag gaa gaa gct att cag ggc atg 1217
Glu Cys Pro Thr Ser Thr Ser Thr Glu Glu Glu Ala Ile Gln Gly Met
                365                 370                 375

ctg tct atg gca ggg ttg cac tat tcc acg tgt tta caa agg caa ata 1265
Leu Ser Met Ala Gly Leu His Tyr Ser Thr Cys Leu Gln Arg Gln Ile
            380                 385                 390

caa agc aca gac tgc agt ggt gaa aga aac tct ctc cag gat ccc agc 1313
Gln Ser Thr Asp Cys Ser Gly Glu Arg Asn Ser Leu Gln Asp Pro Ser
        395                 400                 405

agc tgc cat ggc agt aac cat gag gtt agg cag ttg tat cgc tat gat 1361
Ser Cys His Gly Ser Asn His Glu Val Arg Gln Leu Tyr Arg Tyr Asp
    410                 415                 420

aaa cca gtg gaa tgt gga tac cat gtc aag act gaa gat cca gac ttg 1409
Lys Pro Val Glu Cys Gly Tyr His Val Lys Thr Glu Asp Pro Asp Leu
425                 430                 435                 440

agg act tcc tcc tgg att aaa cag ttt gat act tcc aga ttt cat cct 1457
Arg Thr Ser Ser Trp Ile Lys Gln Phe Asp Thr Ser Arg Phe His Pro
                445                 450                 455

cag gat cta agt aga agc cag aaa tgc atc aga aag gaa ggt tca tca 1505
Gln Asp Leu Ser Arg Ser Gln Lys Cys Ile Arg Lys Glu Gly Ser Ser
            460                 465                 470

gaa att agt cag agg gta caa agt agg aat tat gtg gac agc agc ggc 1553
Glu Ile Ser Gln Arg Val Gln Ser Arg Asn Tyr Val Asp Ser Ser Gly
        475                 480                 485

tca agc ctt cag aat gga aag tat atg cag aat tca aac ctg act tcg 1601
Ser Ser Leu Gln Asn Gly Lys Tyr Met Gln Asn Ser Asn Leu Thr Ser
    490                 495                 500

ggg gcg tgc cag ata agt aat ggc agt cta agc cca gaa agg cca gtt 1649
Gly Ala Cys Gln Ile Ser Asn Gly Ser Leu Ser Pro Glu Arg Pro Val
505                 510                 515                 520

ggt gaa act tcc ttc tcg gtg ccc ctt cac ccc acc aag aga ccg gca 1697
Gly Glu Thr Ser Phe Ser Val Pro Leu His Pro Thr Lys Arg Pro Ala
                525                 530                 535

tca aat cca cca cct atc agc aac cag gca aca aaa ggt aaa cgt cca 1745
Ser Asn Pro Pro Pro Ile Ser Asn Gln Ala Thr Lys Gly Lys Arg Pro
            540                 545                 550

aaa aaa gga atg gca aca gcc aaa caa cgt ctt ggg aag atc ctt aag 1793
Lys Lys Gly Met Ala Thr Ala Lys Gln Arg Leu Gly Lys Ile Leu Lys
        555                 560                 565

ttg aac aga aat ggc cat gca cgt ttc ttt gtg tgacagag ctgctgttgc 1844
Leu Asn Arg Asn Gly His Ala Arg Phe Phe Val
    570                 575

agccattctt ccctttggag accagtctag gggtgcagga gcctggagct tccgctgtcc 1904

ccctgcctgg agcagtttgt gtgtatagta agaacactgc ccgaagaaca gaatgaacct 1964

gatgctgcat tttcactgtg ccacacccac tcagcaataa ccattttgga cctggtgggg 2024

gagaggaaga aggagggtag aaccttaaaa agagaccttg aactggaaag ggtctcttgt 2084

cagggcttga attttatttt gttggtggta gtgtcttgat gt 2126

<210> 122
<211> 2298
<212> DNA
<213> Homo sapiens

<220>
<221> CDS
<222> (96)..(1784)

<400> 122
tttcgtcggc cgcgtccccg ccggccctgc agacgtggtg gggcgggacc cgctgggcac 60

tcccgcgcgt actcggccgc ctgagcgata ctaaa atg tct gat gat gct ggt 113
Met Ser Asp Asp Ala Gly
1               5

gac acc tta gcc act gga gac aaa gca gaa gtt act gag atg cct aat 161
Asp Thr Leu Ala Thr Gly Asp Lys Ala Glu Val Thr Glu Met Pro Asn
            10                  15                  20

agt gat tct tta cct gag gat gca gaa gtg cat tgt gat tca gct gca 209
Ser Asp Ser Leu Pro Glu Asp Ala Glu Val His Cys Asp Ser Ala Ala
        25                  30                  35

gtt tca cat gag cca aca cca gct gac ccc aga ggg gag ggg cat gaa 257
Val Ser His Glu Pro Thr Pro Ala Asp Pro Arg Gly Glu Gly His Glu
    40                  45                  50

aat gca gct gtg cag ggt gca ggg gct gcc gcc att ggg ccc cct gtg 305
Asn Ala Ala Val Gln Gly Ala Gly Ala Ala Ala Ile Gly Pro Pro Val
55                  60                  65                  70

cag cct cag gat gcc aac gcc ctg gag ccc cct ctc aat gga gac gtg 353
Gln Pro Gln Asp Ala Asn Ala Leu Glu Pro Pro Leu Asn Gly Asp Val
                75                  80                  85

act gag gat aca ctt gct gaa tgt att gat tcc gtc agc ctt gag gca 401
Thr Glu Asp Thr Leu Ala Glu Cys Ile Asp Ser Val Ser Leu Glu Ala
            90                  95                  100

gaa ccc aga tcc gaa ata ccc ctg caa gaa cag aat tat ctg gct gtg 449
Glu Pro Arg Ser Glu Ile Pro Leu Gln Glu Gln Asn Tyr Leu Ala Val
        105                 110                 115

gat tcc cct cca agt gga gga gga tgg gca ggc tgg gga tcc tgg ggc 497
Asp Ser Pro Pro Ser Gly Gly Gly Trp Ala Gly Trp Gly Ser Trp Gly
    120                 125                 130

aaa tct ctg ctg tcg tca gca tct gcc aca gta ggt cat gga ttg acg 545
Lys Ser Leu Leu Ser Ser Ala Ser Ala Thr Val Gly His Gly Leu Thr
135                 140                 145                 150

gca gtc aag gaa aaa gca gga gcc act cta cgg att cat ggt gta aat 593
Ala Val Lys Glu Lys Ala Gly Ala Thr Leu Arg Ile His Gly Val Asn
                155                 160                 165

tct gga tct tct gaa gga gcc caa cca aat act gaa aac gga gtc cct 641
Ser Gly Ser Ser Glu Gly Ala Gln Pro Asn Thr Glu Asn Gly Val Pro
            170                 175                 180

gaa ata aca gat gca gcc aca gat cag ggc cct gca gaa agc cca ccc 689
Glu Ile Thr Asp Ala Ala Thr Asp Gln Gly Pro Ala Glu Ser Pro Pro
        185                 190                 195

act tcc cct tca tca gcc tct cgg ggt atg ctg tct gcc atc acc aat 737
Thr Ser Pro Ser Ser Ala Ser Arg Gly Met Leu Ser Ala Ile Thr Asn
    200                 205                 210

gtg gtt caa aac aca ggt aaa agt gtc tta act gga ggc ctt gat gcg 785
Val Val Gln Asn Thr Gly Lys Ser Val Leu Thr Gly Gly Leu Asp Ala
215                 220                 225                 230

ttg gaa ttc atc ggc aag aaa acc atg aat gtc ctt gca gaa agt gac 833
Leu Glu Phe Ile Gly Lys Lys Thr Met Asn Val Leu Ala Glu Ser Asp
                235                 240                 245

ccg ggc ttt aag cgg acc aag acg ctc atg gag aga act gtt tcc ttg 881
Pro Gly Phe Lys Arg Thr Lys Thr Leu Met Glu Arg Thr Val Ser Leu
            250                 255                 260

tct cag atg tta agg gaa gct aag gag aag gag aag cag aga ctg gca 929
Ser Gln Met Leu Arg Glu Ala Lys Glu Lys Glu Lys Gln Arg Leu Ala
        265                 270                 275

cag cag ctc acg atg gag aga acc gcg cac tac ggg atg ctg ttt gat 977
Gln Gln Leu Thr Met Glu Arg Thr Ala His Tyr Gly Met Leu Phe Asp
    280                 285                 290

gaa tat caa ggc ttg tca cac ctg gaa gcc ctg gaa att ctg tcc aat 1025
Glu Tyr Gln Gly Leu Ser His Leu Glu Ala Leu Glu Ile Leu Ser Asn
295                 300                 305                 310

gaa agc gaa agc aag gtt cag tca ttt tta gca tca ctt gat gga gag 1073
Glu Ser Glu Ser Lys Val Gln Ser Phe Leu Ala Ser Leu Asp Gly Glu
                315                 320                 325

aag ctg gaa ctc tta aaa aat gac cta att tcc att aaa gac atc ttt 1121
Lys Leu Glu Leu Leu Lys Asn Asp Leu Ile Ser Ile Lys Asp Ile Phe
            330                 335                 340

gca gcc aaa gaa tta gag aat gaa gaa aat caa gaa gaa caa ggc tta 1169
Ala Ala Lys Glu Leu Glu Asn Glu Glu Asn Gln Glu Glu Gln Gly Leu
        345                 350                 355

gaa gaa aag gga gaa gaa ttt gct cgc atg ctt aca gag ctt ctc ttt 1217
Glu Glu Lys Gly Glu Glu Phe Ala Arg Met Leu Thr Glu Leu Leu Phe
    360                 365                 370

gaa tta cat gtg gcg gcc aca cct gac aaa ctc aat aag gcc atg aag     1265
Glu Leu His Val Ala Ala Thr Pro Asp Lys Leu Asn Lys Ala Met Lys
375                 380                 385                 390

agg gct cat gac tgg gtg gaa gag gat caa acc gtg gtg tca gta gat     1313
Arg Ala His Asp Trp Val Glu Glu Asp Gln Thr Val Val Ser Val Asp
                395                 400                 405

gtg gca aaa gtg tcc gaa gaa gaa aca aag aag gaa gaa aag gaa gag     1361
Val Ala Lys Val Ser Glu Glu Glu Thr Lys Lys Glu Glu Lys Glu Glu
            410                 415                 420

aaa tct caa gac cct caa gaa gac aaa aag gag gaa aag aaa act aag     1409
Lys Ser Gln Asp Pro Gln Glu Asp Lys Lys Glu Glu Lys Lys Thr Lys
        425                 430                 435

acc ata gag gaa gta tac atg tcg tcc att gaa agt ctg gcg gag gta     1457
Thr Ile Glu Glu Val Tyr Met Ser Ser Ile Glu Ser Leu Ala Glu Val
    440                 445                 450

aca gcg cgc tgt att gag cag ctt cat aaa gta gca gaa tta att ctt     1505
Thr Ala Arg Cys Ile Glu Gln Leu His Lys Val Ala Glu Leu Ile Leu
455                 460                 465                 470

cat gga caa gaa gag gaa aaa cca gct cag gac caa gca aaa gtt cta     1553
His Gly Gln Glu Glu Glu Lys Pro Ala Gln Asp Gln Ala Lys Val Leu
                475                 480                 485

ata aaa tta act act gca atg tgc aat gaa gtg gcc tct tta tca aag     1601
Ile Lys Leu Thr Thr Ala Met Cys Asn Glu Val Ala Ser Leu Ser Lys
            490                 495                 500

aag ttt acg aat tct tta acc act gtt ggg agc aac aag aag gcc gag     1649
Lys Phe Thr Asn Ser Leu Thr Thr Val Gly Ser Asn Lys Lys Ala Glu
        505                 510                 515

gtc ctt aac ccc atg atc agt agt gta ttg tta gag ggc tgc aac agt     1697
Val Leu Asn Pro Met Ile Ser Ser Val Leu Leu Glu Gly Cys Asn Ser
    520                 525                 530

aca acg tac ata cag gat gcc ttc cag ctg ctg ctg cct gtt ctg cag     1745
Thr Thr Tyr Ile Gln Asp Ala Phe Gln Leu Leu Leu Pro Val Leu Gln
535                 540                 545                 550

gtc tca cat atc cag acc agt tgt ttg aaa gca cag ccg tgacctggcc      1794
Val Ser His Ile Gln Thr Ser Cys Leu Lys Ala Gln Pro
                555                 560

agactccatc tagttaaagg agacagctgg ccgccttgcc tcaatatgta ccatttaagg   1854

ggatgttctc tgtgcgcctg gccacagaca tccatttgag gacactacaa gcaattttgc   1914

acagacaata ttgagaatgc aaatttagag agagttatca tttctctcaa tgtgtataat   1974

tgtttttaca aacaattgtg ttttctttat gttaatttaa acttacacag cttatattga   2034

aaatttcctt tcatctgaaa tttatttaca aatattcctg ttcattttcc tggttaagca   2094

tgctatattt agaaactcat ggggagacct tagacttttg tttaatcctt tatgtttcaa   2154

cctttaaatg ttccattctt atagtattac tttaaatcaa ttctaaaact gaactttgtt 2214

ttgttacata aatgtcgcag gcaaaaataa cactacttat agattttacc tattatggta 2274

aaaaatagga acatattgtc attc 2298

<210> 123
<211> 546
<212> DNA
<213> Homo sapiens

<220>
<221> CDS
<222> (216)..(443)

<400> 123
ccttaatcta gcagtaattt atcaaaaacc catatgataa gagaattatt ttataatagt 60
gttggtaaag aaaatctaaa atagaaaaaa catgccaaca attgattggc taatgaggtg 120
ggtgatgcca gatgcaagac tgctgggcct accacaggag caagagagaa gccatgcatt 180
ccatctcaag gcatctgtga gcccatgtag acacc atg agc aaa gct cac cct 233
cct gag ttg aaa aaa ttt ata gac aag aag tta tca ttg aaa tta aat 281
ggt ggc aga cat gtc caa gga ata ttg tgg gga ttt gat ccc tta atg 329
aat ctt gtg ata gat aag tgt gtg gag atg gca acc agc ggg caa cag 377
aag aat att gga atg gtg gtg ata cga aga aat agt atc atc atg tta 425
gaa acc ttg gaa caa gaa taaata atggctgttc agcagagaaa ttccacgtcc 479
cctctccaaa gagcctgttt tactatgatg taaaaattag gtcatgtaca tttttatgtt 539
ggacttt 546

<210> 124
<211> 3107
<212> DNA

<213> Homo sapiens

<220>
<221> CDS
<222> (16)..(2847)

<400> 124
gtcaacttgg tggtg atg ctg gcc gag ttg ttc atg tgt ttt gag gtg ctc 51
Met Leu Ala Glu Leu Phe Met Cys Phe Glu Val Leu
1               5                   10

aag ccc gac ttt gtg caa gtg aag gac ttg ccc gat ggt cac gct gcc 99
Lys Pro Asp Phe Val Gln Val Lys Asp Leu Pro Asp Gly His Ala Ala
        15                  20                  25

tcc ccc cgg ggc act gag gcc tcc cca cct cag aac aac agc ggc agt 147
Ser Pro Arg Gly Thr Glu Ala Ser Pro Pro Gln Asn Asn Ser Gly Ser
    30                  35                  40

agt tct cct gtc ttc acc ttc cgc cac ccg ctt ctg tca tct ggt ggc 195
Ser Ser Pro Val Phe Thr Phe Arg His Pro Leu Leu Ser Ser Gly Gly
45                  50                  55                  60

ccc cag tcc cca ctc cga gga tcc aca ggc tcc ctg aag tct tcc ccg 243
Pro Gln Ser Pro Leu Arg Gly Ser Thr Gly Ser Leu Lys Ser Ser Pro
                65                  70                  75

tcc atg tcc cat atg gag gcc ctg ggc aag gcc tgg aac cgg cag ctc 291
Ser Met Ser His Met Glu Ala Leu Gly Lys Ala Trp Asn Arg Gln Leu
            80                  85                  90

agc cgt ccc ctc tcc cag gct gtg tca ttc agc acc ccc ttt ggc ctg 339
Ser Arg Pro Leu Ser Gln Ala Val Ser Phe Ser Thr Pro Phe Gly Leu
        95                  100                 105

gac agc gac gtg gat gtc gtc atg gga gac cct gtg ctc ctc cgc tct 387
Asp Ser Asp Val Asp Val Val Met Gly Asp Pro Val Leu Leu Arg Ser
    110                 115                 120

gtg agc tcg gac agc ctg ggc ccc ccg cgt ccc gcg ccg gcc agg acc 435
Val Ser Ser Asp Ser Leu Gly Pro Pro Arg Pro Ala Pro Ala Arg Thr
125                 130                 135                 140

ccc acc cag cca ccc ccg gag cct ggt gac ctg ccc acc atc gag gaa 483
Pro Thr Gln Pro Pro Pro Glu Pro Gly Asp Leu Pro Thr Ile Glu Glu
                145                 150                 155

gct ctg cag atc atc cac agt gcc gag ccc cgg ctc ctc cca gat ggg 531
Ala Leu Gln Ile Ile His Ser Ala Glu Pro Arg Leu Leu Pro Asp Gly
            160                 165                 170

gcg gcc gac ggc agc ttc tac ctc cac tcc cct gag ggg ccc tcc aag 579
Ala Ala Asp Gly Ser Phe Tyr Leu His Ser Pro Glu Gly Pro Ser Lys
        175                 180                 185

cca tcc ctg gcc tcc ccc tac ctg ccc gag ggg acc tcc aaa cca ctg 627
Pro Ser Leu Ala Ser Pro Tyr Leu Pro Glu Gly Thr Ser Lys Pro Leu
    190                 195                 200

tcc gac agg ccc acc aaa gca cca gtg tac atg cca cac ccc gag acc 675

Ser Asp Arg Pro Thr Lys Ala Pro Val Tyr Met Pro His Pro Glu Thr
205                 210                 215                 220

ccc tcg aaa cca tct ccc tgt ctg gtg ggg gag gca tcg aaa ccg cca     723
Pro Ser Lys Pro Ser Pro Cys Leu Val Gly Glu Ala Ser Lys Pro Pro
                225                 230                 235

gcc cca tcc gag ggg tcc ccg aag gcg gtg gct tcg tcc cca gca gcc     771
Ala Pro Ser Glu Gly Ser Pro Lys Ala Val Ala Ser Ser Pro Ala Ala
            240                 245                 250

acc aac tcc gag gtg aaa atg acc agc ttt gca gaa cgc aag aaa cag     819
Thr Asn Ser Glu Val Lys Met Thr Ser Phe Ala Glu Arg Lys Lys Gln
        255                 260                 265

ctg gtg aag gca gag gct gag gcc gga gcg ggg tcc ccc acg tcc act     867
Leu Val Lys Ala Glu Ala Glu Ala Gly Ala Gly Ser Pro Thr Ser Thr
    270                 275                 280

ccg gcc ccg ccg gag gcc ctg agc tcg gag atg agt gag ctc agc gcc     915
Pro Ala Pro Pro Glu Ala Leu Ser Ser Glu Met Ser Glu Leu Ser Ala
285                 290                 295                 300

cgg ctg gag gag aaa cgc aga gcc atc gag gct cag aag cga cgg att     963
Arg Leu Glu Glu Lys Arg Arg Ala Ile Glu Ala Gln Lys Arg Arg Ile
                305                 310                 315

gag gcc ata ttc gcc aag cac cgc cag cgg ctg ggc aaa agc gcc ttc     1011
Glu Ala Ile Phe Ala Lys His Arg Gln Arg Leu Gly Lys Ser Ala Phe
            320                 325                 330

ctg cag gtg cag ccg cgg gaa gcc tct ggg gag gcg gaa gca gag gcg     1059
Leu Gln Val Gln Pro Arg Glu Ala Ser Gly Glu Ala Glu Ala Glu Ala
        335                 340                 345

gag gag gcc gat tcc ggt cca gtc cct ggt ggg gag cgg ccc gca ggc     1107
Glu Glu Ala Asp Ser Gly Pro Val Pro Gly Gly Glu Arg Pro Ala Gly
    350                 355                 360

gag ggc cag ggt gag cca acc tca cgg ccc aag gca gtg acc ttc tcg     1155
Glu Gly Gln Gly Glu Pro Thr Ser Arg Pro Lys Ala Val Thr Phe Ser
365                 370                 375                 380

cca gac ctg ggc ccg gtg ccc cac gag ggg ctg ggg gaa tac aat cga     1203
Pro Asp Leu Gly Pro Val Pro His Glu Gly Leu Gly Glu Tyr Asn Arg
                385                 390                 395

gcg gtc agc aag ctg agt gcc gcc ttg agc tcg ctg cag cgg gac atg     1251
Ala Val Ser Lys Leu Ser Ala Ala Leu Ser Ser Leu Gln Arg Asp Met
            400                 405                 410

cag agg ctc acg gac cag cag cag cgg ctc ctg gcc ccg ccc gag gcc     1299
Gln Arg Leu Thr Asp Gln Gln Gln Arg Leu Leu Ala Pro Pro Glu Ala
        415                 420                 425

ccc gga tcc gcc cca cca cct gct gcg tgg gtc atc cct ggc ccc acg     1347
Pro Gly Ser Ala Pro Pro Pro Ala Ala Trp Val Ile Pro Gly Pro Thr
    430                 435                 440

acg ggg ccc aaa gct gca tcc ccc agc ccc gcc cgg cga gtc ccg gcc     1395

Thr Gly Pro Lys Ala Ala Ser Pro Ser Pro Ala Arg Arg Val Pro Ala
445                 450                 455                 460

acc cgg cgc agc cct ggg ccc ggg ccc agc cag tca ccc cgc agc ccg      1443
Thr Arg Arg Ser Pro Gly Pro Gly Pro Ser Gln Ser Pro Arg Ser Pro
                465                 470                 475

aaa cac acg cgg cca gcg gag ctg cgg ctg gca ccc ttg acc agg gtg      1491
Lys His Thr Arg Pro Ala Glu Leu Arg Leu Ala Pro Leu Thr Arg Val
            480                 485                 490

ctt acg cca ccc cac gac gta gac agc ctc ccc cac ctg cgc aag ttc      1539
Leu Thr Pro Pro His Asp Val Asp Ser Leu Pro His Leu Arg Lys Phe
        495                 500                 505

tcg ccg agc cag gtg ccc gtg cag acg cgc tct tcc atc ctc ctg gcg      1587
Ser Pro Ser Gln Val Pro Val Gln Thr Arg Ser Ser Ile Leu Leu Ala
    510                 515                 520

gag gag acg ccc ccc gag gag cca gcc gcc cgg ccg ggc ctc atc gag      1635
Glu Glu Thr Pro Pro Glu Glu Pro Ala Ala Arg Pro Gly Leu Ile Glu
525                 530                 535                 540

atc ccg ctg ggc agc ctg gca gat ccc gcc gcc gag gac gag gga gac      1683
Ile Pro Leu Gly Ser Leu Ala Asp Pro Ala Ala Glu Asp Glu Gly Asp
                545                 550                 555

ggg agc ccc gct ggt gct gag gat tcc ttg gag gag gag gcg tct tcg      1731
Gly Ser Pro Ala Gly Ala Glu Asp Ser Leu Glu Glu Glu Ala Ser Ser
            560                 565                 570

gag ggg gag ccc cgg gtg ggg ctg ggg ttc ttc tac aag gat gaa gac      1779
Glu Gly Glu Pro Arg Val Gly Leu Gly Phe Phe Tyr Lys Asp Glu Asp
        575                 580                 585

aag cct gag gac gag atg gcc caa aag cgg gcc agc ctg ctg gag cgg      1827
Lys Pro Glu Asp Glu Met Ala Gln Lys Arg Ala Ser Leu Leu Glu Arg
    590                 595                 600

cag cag cgg cga gca gag gag gcg cgg cgg cgc aag cag tgg cag gag      1875
Gln Gln Arg Arg Ala Glu Glu Ala Arg Arg Arg Lys Gln Trp Gln Glu
605                 610                 615                 620

gtg gag aag gaa cag cgg agg gag gag gcc gcg agg ctg gcc caa gag      1923
Val Glu Lys Glu Gln Arg Arg Glu Glu Ala Ala Arg Leu Ala Gln Glu
                625                 630                 635

gag gcc ccg ggc cca gcc ccg ctt gtg tcc gca gtc ccg atg gcg act      1971
Glu Ala Pro Gly Pro Ala Pro Leu Val Ser Ala Val Pro Met Ala Thr
            640                 645                 650

cca gcc cct gct gcc cgg gct cca gcc gag gag gag gtg ggc ccc cgg      2019
Pro Ala Pro Ala Ala Arg Ala Pro Ala Glu Glu Glu Val Gly Pro Arg
        655                 660                 665

aag ggg gac ttc acg cgg cag gag tac gag cgc cgg gcc cag ctg aag      2067
Lys Gly Asp Phe Thr Arg Gln Glu Tyr Glu Arg Arg Ala Gln Leu Lys
    670                 675                 680

ctg atg gac gac ctc gat aag gtg ctg cgg ccc cgg gct gcg ggg tcc      2115

Leu Met Asp Asp Leu Asp Lys Val Leu Arg Pro Arg Ala Ala Gly Ser
685                 690                 695                 700

ggg ggt cca ggt cgg ggc ggg cgg agg gcc acc cgg cct cgc tcg ggt      2163
Gly Gly Pro Gly Arg Gly Gly Arg Arg Ala Thr Arg Pro Arg Ser Gly
                705                 710                 715

tgc tgt gac gac tca gcc ctg gca cga agc cca gcc cgc ggc ctg ctg      2211
Cys Cys Asp Asp Ser Ala Leu Ala Arg Ser Pro Ala Arg Gly Leu Leu
            720                 725                 730

ggc tct cgg ctg agc aaa atc tat tcc cag tcc acc ctg tca ctg tcc      2259
Gly Ser Arg Leu Ser Lys Ile Tyr Ser Gln Ser Thr Leu Ser Leu Ser
        735                 740                 745

act gtg gcc aac gag gcc cac aat aac ctc ggg gtg aag agg ccc acg      2307
Thr Val Ala Asn Glu Ala His Asn Asn Leu Gly Val Lys Arg Pro Thr
    750                 755                 760

tct cgg gct ccc tcc ccg tca ggt ctc atg tcc cca agc cgc ctg cct      2355
Ser Arg Ala Pro Ser Pro Ser Gly Leu Met Ser Pro Ser Arg Leu Pro
765                 770                 775                 780

gga agc cgc gaa cgg gac tgg gaa aat ggc agc aat gcc tcc tcc cca      2403
Gly Ser Arg Glu Arg Asp Trp Glu Asn Gly Ser Asn Ala Ser Ser Pro
                785                 790                 795

gcg tca gtg ccc gag tac aca ggt cca cgg ctg tac aaa gaa ccc agc      2451
Ala Ser Val Pro Glu Tyr Thr Gly Pro Arg Leu Tyr Lys Glu Pro Ser
            800                 805                 810

gcc aag tcc aac aag ttc atc atc cac aat gcc cta tca cac tgc tgc      2499
Ala Lys Ser Asn Lys Phe Ile Ile His Asn Ala Leu Ser His Cys Cys
        815                 820                 825

ctg gcg ggc aag gtg aac gaa ccg cag aag aat cgc att ctg gag gaa      2547
Leu Ala Gly Lys Val Asn Glu Pro Gln Lys Asn Arg Ile Leu Glu Glu
    830                 835                 840

att gag aaa agc aag gcc aac cac ttc ctg atc ctc ttt cgc gac tcg      2595
Ile Glu Lys Ser Lys Ala Asn His Phe Leu Ile Leu Phe Arg Asp Ser
845                 850                 855                 860

agc tgc cag ttc cgg gcg ctc tac acg ctg tcg ggg gag aca gag gag      2643
Ser Cys Gln Phe Arg Ala Leu Tyr Thr Leu Ser Gly Glu Thr Glu Glu
                865                 870                 875

ctg tcg cgg ctg gca ggg tat ggg ccc cgg acc gtc acg ccc gcc atg      2691
Leu Ser Arg Leu Ala Gly Tyr Gly Pro Arg Thr Val Thr Pro Ala Met
            880                 885                 890

gtg gaa ggc atc tac aag tac aac tcg gac cgc aag cgc ttc acc cag      2739
Val Glu Gly Ile Tyr Lys Tyr Asn Ser Asp Arg Lys Arg Phe Thr Gln
        895                 900                 905

atc ccc gcc aag acc atg tcc atg agc gtc gat gcc ttc acc atc cag      2787
Ile Pro Ala Lys Thr Met Ser Met Ser Val Asp Ala Phe Thr Ile Gln
    910                 915                 920

gga cac ctc tgg cag ggc aag aaa ccc acc act ccc aag aag ggc ggc      2835

Gly His Leu Trp Gln Gly Lys Lys Pro Thr Thr Pro Lys Lys Gly Gly
925                 930                 935                 940

ggc acc ccc aaa tag ccccacccgg gcggtccacg ggccgggccc tgtgtgctgc     2890
Gly Thr Pro Lys

ggccgccatc ccctggagga cagtcagtcg gtattcctgg gtcctgtctg tccccaaccg   2950

tgtctgggtg gggctggagt ctccaccctc tgactttgag tccagtcctg ctgtgggggc    3010

tgagctggga ggttcaggga ctcagggctc agctcagtcc ccttgtctgt cctcccccac   3070

ttcttgaata aaataattta aagagaaaaa aaaaaaa                            3107

<210> 125
<211> 5559
<212> DNA
<213> Homo sapiens

<220>
<221> CDS
<222> (1)..(3636)

<400> 125
atg gcg tta atg ttt aca gga cat tta cta ttc tta gca tta ttg atg      48
Met Ala Leu Met Phe Thr Gly His Leu Leu Phe Leu Ala Leu Leu Met
1               5                   10                  15

ttt gct ttc tct act ttt gag gaa tct gtg agc aat tat tcc gaa tgg      96
Phe Ala Phe Ser Thr Phe Glu Glu Ser Val Ser Asn Tyr Ser Glu Trp
            20                  25                  30

gca gtt ttc aca gat gat ata gat cag ttt aaa aca cag aaa gtg caa     144
Ala Val Phe Thr Asp Asp Ile Asp Gln Phe Lys Thr Gln Lys Val Gln
        35                  40                  45

gat ttc aga ccc aac caa aag ctg aag aaa agt atg ctt cat cca agt     192
Asp Phe Arg Pro Asn Gln Lys Leu Lys Lys Ser Met Leu His Pro Ser
    50                  55                  60

tta tat ttt gat gct gga gaa atc caa gca atg aga caa aag tct cgt     240
Leu Tyr Phe Asp Ala Gly Glu Ile Gln Ala Met Arg Gln Lys Ser Arg
65                  70                  75                  80

gca agc cat ttg cat ctt ttt aga gct atc aga agt gca gtg aca gtt     288
Ala Ser His Leu His Leu Phe Arg Ala Ile Arg Ser Ala Val Thr Val
                85                  90                  95

atg ctg tcc aac cca aca tac tac cta cct cca cca aag cat gct gat     336
Met Leu Ser Asn Pro Thr Tyr Tyr Leu Pro Pro Pro Lys His Ala Asp
            100                 105                 110

ttt gct gcc aag tgg aat gaa att tat ggt aac aat ctg cct cct tta     384
Phe Ala Ala Lys Trp Asn Glu Ile Tyr Gly Asn Asn Leu Pro Pro Leu
        115                 120                 125

gca ttg tac tgt ttg tta tgc cca gaa gac aaa gtt gcc ttt gaa ttt 432
Ala Leu Tyr Cys Leu Leu Cys Pro Glu Asp Lys Val Ala Phe Glu Phe
    130                 135                 140

gtc ttg gaa tat atg gac agg atg gtt ggc tac aaa gac tgg cta gta 480
Val Leu Glu Tyr Met Asp Arg Met Val Gly Tyr Lys Asp Trp Leu Val
145                 150                 155                 160

gag aat gca cca gga gat gag gtt cca att ggc cat tcc tta aca ggt 528
Glu Asn Ala Pro Gly Asp Glu Val Pro Ile Gly His Ser Leu Thr Gly
                165                 170                 175

ttt gcc act gcc ttt gac ttt tta tat aac tta tta gat aat cat cga 576
Phe Ala Thr Ala Phe Asp Phe Leu Tyr Asn Leu Leu Asp Asn His Arg
            180                 185                 190

aga caa aaa tac ctg gaa aaa ata tgg gtt att act gag gaa atg tac 624
Arg Gln Lys Tyr Leu Glu Lys Ile Trp Val Ile Thr Glu Glu Met Tyr
        195                 200                 205

gag tat tcc aag gtc cgc tca tgg ggc aaa cag ctt ctc cat aac cac 672
Glu Tyr Ser Lys Val Arg Ser Trp Gly Lys Gln Leu Leu His Asn His
    210                 215                 220

caa gcc act aat atg ata gca tta ctc aca ggg gcc ttg gtg act gga 720
Gln Ala Thr Asn Met Ile Ala Leu Leu Thr Gly Ala Leu Val Thr Gly
225                 230                 235                 240

gta gat aaa gga tct aaa gca aat ata tgg aaa cag gct gta gtg gat 768
Val Asp Lys Gly Ser Lys Ala Asn Ile Trp Lys Gln Ala Val Val Asp
                245                 250                 255

gtc atg gaa aag aca atg ttt cta ttg aat cat att gtt gat ggt tct 816
Val Met Glu Lys Thr Met Phe Leu Leu Asn His Ile Val Asp Gly Ser
            260                 265                 270

ttg gat gaa ggt gtg gcc tat gga agc tac aca gct aaa tcc gtc aca 864
Leu Asp Glu Gly Val Ala Tyr Gly Ser Tyr Thr Ala Lys Ser Val Thr
        275                 280                 285

cag tat gtt ttt ctg gcc cag cgc cat ttt aat atc aac aac ttg gat 912
Gln Tyr Val Phe Leu Ala Gln Arg His Phe Asn Ile Asn Asn Leu Asp
    290                 295                 300

aat aac tgg tta aag atg cac ttt tgg ttc tat tat gcc acc ctt tta 960
Asn Asn Trp Leu Lys Met His Phe Trp Phe Tyr Tyr Ala Thr Leu Leu
305                 310                 315                 320

cct ggc ttc caa aga act gtg ggt ata gca gat tcc aat tat aat tgg 1008
Pro Gly Phe Gln Arg Thr Val Gly Ile Ala Asp Ser Asn Tyr Asn Trp
                325                 330                 335

ttt tat ggt cca gaa agc cag cta gtt ttc ttg gat aag ttc atc tta 1056
Phe Tyr Gly Pro Glu Ser Gln Leu Val Phe Leu Asp Lys Phe Ile Leu
            340                 345                 350

aag aat gga gct gga aat tgg tta gct cag caa att aga aag cac cga 1104
Lys Asn Gly Ala Gly Asn Trp Leu Ala Gln Gln Ile Arg Lys His Arg
        355                 360                 365

cct aaa gat gga ccg atg gtt cct tca act gcc caa agg tgg agt act 1152
Pro Lys Asp Gly Pro Met Val Pro Ser Thr Ala Gln Arg Trp Ser Thr
    370                 375                 380

ctt cac act gaa tac atc tgg tat gat ccc cag ctc aca cca cag cca 1200
Leu His Thr Glu Tyr Ile Trp Tyr Asp Pro Gln Leu Thr Pro Gln Pro
385                 390                 395                 400

cct gct gat tat ggt act gca aaa ata cac aca ttc cct aac tgg ggt 1248
Pro Ala Asp Tyr Gly Thr Ala Lys Ile His Thr Phe Pro Asn Trp Gly
                405                 410                 415

gtg gtt act tat ggg gct ggg ttg cca aac aca cag acc aac acc ttt 1296
Val Val Thr Tyr Gly Ala Gly Leu Pro Asn Thr Gln Thr Asn Thr Phe
            420                 425                 430

gtg tct ttt aaa tct ggg aag ctg ggg gga cga gct gtg tat gac ata 1344
Val Ser Phe Lys Ser Gly Lys Leu Gly Gly Arg Ala Val Tyr Asp Ile
        435                 440                 445

gtt cat ttt cag cca tat tcc tgg att gat ggg tgg aga agt ttt aac 1392
Val His Phe Gln Pro Tyr Ser Trp Ile Asp Gly Trp Arg Ser Phe Asn
    450                 455                 460

cca gga cat gag cat cca gat cag aac tca ttt act ttt gcc ccc aat 1440
Pro Gly His Glu His Pro Asp Gln Asn Ser Phe Thr Phe Ala Pro Asn
465                 470                 475                 480

gga caa gta ttt gtt tct gaa gct ctc tat gga ccc aag ttg agc cac 1488
Gly Gln Val Phe Val Ser Glu Ala Leu Tyr Gly Pro Lys Leu Ser His
                485                 490                 495

ctt aac aat gta ttg gtg ttt gct cca tca ccc tca agc cag tgt aat 1536
Leu Asn Asn Val Leu Val Phe Ala Pro Ser Pro Ser Ser Gln Cys Asn
            500                 505                 510

aag ccc tgg gaa ggt caa ctg gga gaa tgt gcg cag tgg ctt aag tgg 1584
Lys Pro Trp Glu Gly Gln Leu Gly Glu Cys Ala Gln Trp Leu Lys Trp
        515                 520                 525

act ggc gag gag gtt ggt gat gca gct ggg gaa ata atc act gcc tct 1632
Thr Gly Glu Glu Val Gly Asp Ala Ala Gly Glu Ile Ile Thr Ala Ser
    530                 535                 540

caa cat ggg gaa atg gta ttt gtg agt ggg gaa gcc gtg tct gct tat 1680
Gln His Gly Glu Met Val Phe Val Ser Gly Glu Ala Val Ser Ala Tyr
545                 550                 555                 560

tct tca gca atg aga ctg aaa agt gta tat cgt gct ttg ctt ctc tta 1728
Ser Ser Ala Met Arg Leu Lys Ser Val Tyr Arg Ala Leu Leu Leu Leu
                565                 570                 575

aat tcc caa act ctg cta gtt gtt gat cat att gag agg caa gaa gat 1776
Asn Ser Gln Thr Leu Leu Val Val Asp His Ile Glu Arg Gln Glu Asp
            580                 585                 590

tcc cca ata aat tct gtc agt gcc ttc ttt cat aat ttg gat att gat 1824
Ser Pro Ile Asn Ser Val Ser Ala Phe Phe His Asn Leu Asp Ile Asp
        595                 600                 605

ttt aaa tat atc cca tat aag ttt atg aat agg tat aat ggt gcc atg 1872
Phe Lys Tyr Ile Pro Tyr Lys Phe Met Asn Arg Tyr Asn Gly Ala Met
    610                 615                 620

atg gat gtg tgg gat gca cat tac aaa atg ttt tgg ttt gat cat cat 1920
Met Asp Val Trp Asp Ala His Tyr Lys Met Phe Trp Phe Asp His His
625                 630                 635                 640

ggc aat agt ccc atg gcc agt ata cag gaa gca gag caa gct gct gaa 1968
Gly Asn Ser Pro Met Ala Ser Ile Gln Glu Ala Glu Gln Ala Ala Glu
                645                 650                 655

ttt aaa aaa cga tgg act caa ttt gtt aat gtt act ttt cag atg gaa 2016
Phe Lys Lys Arg Trp Thr Gln Phe Val Asn Val Thr Phe Gln Met Glu
            660                 665                 670

ccc aca atc aca aga att gca tat gtc ttt tat ggg cca tat atc aat 2064
Pro Thr Ile Thr Arg Ile Ala Tyr Val Phe Tyr Gly Pro Tyr Ile Asn
        675                 680                 685

gtc tcc agc tgc aga ttt att gat agt tcc aat cct gga ctt cag att 2112
Val Ser Ser Cys Arg Phe Ile Asp Ser Ser Asn Pro Gly Leu Gln Ile
    690                 695                 700

tct ctc aat gtc aat aat act gaa cat gtt gtt tct att gta act gat 2160
Ser Leu Asn Val Asn Asn Thr Glu His Val Val Ser Ile Val Thr Asp
705                 710                 715                 720

tac cat aac ctg aag aca aga ttc aat tat ctg gga ttc ggt ggc ttt 2208
Tyr His Asn Leu Lys Thr Arg Phe Asn Tyr Leu Gly Phe Gly Gly Phe
                725                 730                 735

gcc agt gtg gct gat caa ggc caa ata acc cga ttt ggt ttg ggc act 2256
Ala Ser Val Ala Asp Gln Gly Gln Ile Thr Arg Phe Gly Leu Gly Thr
            740                 745                 750

caa gca ata gta aag cct gta aga cat gat agg att att ttc ccc ttt 2304
Gln Ala Ile Val Lys Pro Val Arg His Asp Arg Ile Ile Phe Pro Phe
        755                 760                 765

gga ttt aaa ttt aat ata gca gtt gga tta att ttg tgc att agc ttg 2352
Gly Phe Lys Phe Asn Ile Ala Val Gly Leu Ile Leu Cys Ile Ser Leu
    770                 775                 780

gtg att tta act ttc caa tgg cgt ttt tac ctt tct ttt aga aaa cta 2400
Val Ile Leu Thr Phe Gln Trp Arg Phe Tyr Leu Ser Phe Arg Lys Leu
785                 790                 795                 800

atg cga tgg ata tta ata ctt gtt att gcc ttg tgg ttt att gag ctt 2448
Met Arg Trp Ile Leu Ile Leu Val Ile Ala Leu Trp Phe Ile Glu Leu
                805                 810                 815

ttg gat gtg tgg agc act tgt agt cag ccc att tgt gca aaa tgg aca 2496
Leu Asp Val Trp Ser Thr Cys Ser Gln Pro Ile Cys Ala Lys Trp Thr
            820                 825                 830

agg aca gag gct gag gga agc aag aag tct ttg tct tct gaa ggg cac 2544
Arg Thr Glu Ala Glu Gly Ser Lys Lys Ser Leu Ser Ser Glu Gly His
        835                 840                 845

cac atg gat ctt cct gat gtt gtc att acc tca ctt cct ggt tca gga 2592
His Met Asp Leu Pro Asp Val Val Ile Thr Ser Leu Pro Gly Ser Gly
    850                 855                 860

gct gaa att ctc aaa caa ctt ttt ttc aac agt agt gat ttt ctc tac 2640
Ala Glu Ile Leu Lys Gln Leu Phe Phe Asn Ser Ser Asp Phe Leu Tyr
865                 870                 875                 880

atc agg gtt cct aca gcc tac att gat att cct gaa act gag ttg gaa 2688
Ile Arg Val Pro Thr Ala Tyr Ile Asp Ile Pro Glu Thr Glu Leu Glu
                885                 890                 895

atc gac tca ttt gta gat gct tgt gaa tgg aag gtg tca gat atc cgc 2736
Ile Asp Ser Phe Val Asp Ala Cys Glu Trp Lys Val Ser Asp Ile Arg
            900                 905                 910

agt ggg cat ttt cgt tta ctc cga ggc tgg ttg cag tct tta gtc cag 2784
Ser Gly His Phe Arg Leu Leu Arg Gly Trp Leu Gln Ser Leu Val Gln
        915                 920                 925

gac aca aaa tta cat ttg caa aac atc cat ctg cat gaa ccc aat agg 2832
Asp Thr Lys Leu His Leu Gln Asn Ile His Leu His Glu Pro Asn Arg
    930                 935                 940

ggt aaa ctg gcc caa tat ttt gca atg aat aag gac aaa aaa aga aaa 2880
Gly Lys Leu Ala Gln Tyr Phe Ala Met Asn Lys Asp Lys Lys Arg Lys
945                 950                 955                 960

ttt aaa agg aga gag tct ttg cca gaa caa aga agt caa atg aaa ggc 2928
Phe Lys Arg Arg Glu Ser Leu Pro Glu Gln Arg Ser Gln Met Lys Gly
                965                 970                 975

gcc ttt gat aga gat gct gaa tat att agg gct ttg agg aga cac ctg 2976
Ala Phe Asp Arg Asp Ala Glu Tyr Ile Arg Ala Leu Arg Arg His Leu
            980                 985                 990

gtt tac tat cca agt gca cgt cct gtg ctc agt tta agc agt gga agc 3024
Val Tyr Tyr Pro Ser Ala Arg Pro Val Leu Ser Leu Ser Ser Gly Ser
        995                 1000                1005

tgg acg tta aag ctt cat ttt ttt cag gaa gtt tta gga gct tcg atg 3072
Trp Thr Leu Lys Leu His Phe Phe Gln Glu Val Leu Gly Ala Ser Met
    1010                1015                1020

agg gca ttg tac ata gta aga gac cct cgg gca tgg att tat tca atg 3120
Arg Ala Leu Tyr Ile Val Arg Asp Pro Arg Ala Trp Ile Tyr Ser Met
1025                1030                1035                1040

ttg tac aat agt aaa cca agt ctt tat tct ttg aag aat gta cca gag 3168
Leu Tyr Asn Ser Lys Pro Ser Leu Tyr Ser Leu Lys Asn Val Pro Glu
                1045                1050                1055

cat tta gca aaa ttg ttt aaa ata gag gga ggt aaa ggc aaa tgt aac 3216
His Leu Ala Lys Leu Phe Lys Ile Glu Gly Gly Lys Gly Lys Cys Asn
            1060                1065                1070

tta aat tcg ggt tat gct ttc gag tat gaa cca ttg agg aaa gaa tta 3264
Leu Asn Ser Gly Tyr Ala Phe Glu Tyr Glu Pro Leu Arg Lys Glu Leu
        1075                1080                1085

tca aaa tcc aaa tca aat gca gtg tcc ctc ttg tct cac ttg tgg cta 3312
Ser Lys Ser Lys Ser Asn Ala Val Ser Leu Leu Ser His Leu Trp Leu
    1090                1095                1100

gca aat aca gca gca gcc ttg aga ata aat aca gat ttg ctg cct act 3360
Ala Asn Thr Ala Ala Ala Leu Arg Ile Asn Thr Asp Leu Leu Pro Thr
1105                1110                1115                1120

agc tac cag ctg gtc aag ttt gaa gat att gtg cat ttt cct cag aaa 3408
Ser Tyr Gln Leu Val Lys Phe Glu Asp Ile Val His Phe Pro Gln Lys
                1125                1130                1135

act act gaa agg att ttt gcc ttt ctt gga att cct ttg tct cct gct 3456
Thr Thr Glu Arg Ile Phe Ala Phe Leu Gly Ile Pro Leu Ser Pro Ala
            1140                1145                1150

agt tta aac caa ata ttg ttt gcc acc tct aca aac ctt ttt tac ctt 3504
Ser Leu Asn Gln Ile Leu Phe Ala Thr Ser Thr Asn Leu Phe Tyr Leu
        1155                1160                1165

ccc tat gaa ggg gaa ata tca cca act aat act aat gtt tgg aaa cag 3552
Pro Tyr Glu Gly Glu Ile Ser Pro Thr Asn Thr Asn Val Trp Lys Gln
    1170                1175                1180

aac ttg cct aga gat gaa att aaa cta att gaa aac atc tgc tgg act 3600
Asn Leu Pro Arg Asp Glu Ile Lys Leu Ile Glu Asn Ile Cys Trp Thr
1185                1190                1195                1200

ctg atg gat cgc cta gga tat cca aag ttt atg gac taaa tgctgcaggt 3650
Leu Met Asp Arg Leu Gly Tyr Pro Lys Phe Met Asp
                1205                1210

cagcagaaat ttgcactaat aatacttacc aacccacttt gtggatatga atcagaagag 3710

tttgtttatt ctttagtgtg tgtgtgtgtg tgcacgcgtg tatgtgttca gtgttgtttg 3770

cacagagaga ttgttttaaa aaatggcacc atatttggcc tagcaggatt tatttttatg 3830

tcatcacctc ccttgccttt gtttctgaaa attttgtctg ctaaaaagtt tctgctacag 3890

agtggtagat gaagttatat catggggtca ggggagatgg gaaaatttta agtttttgtc 3950

taactcccct tcatctgtaa ctgtgctaat ctatctagag acctcaaaca ctgctaaagg 4010

ccttgcaatt gctgctttac ccacgcatct cttgctttca agatggacta caaaagttcc 4070

ttatcctttt gaaaaggtct tctgacacac ttatcttgca caaagaaaaa gaaaatttct 4130

tttactgtgt ttaatgttca gtgatatcac tgaggaaatg gtgaaagctc ctatcagaac 4190

tataggattt cttctgggaa atacagatgg aaatacagaa tgaatatgtt tttttgaggt 4250

cggaaactga ctttaaaagc ctccttgaag ttttttactt agaaatataa ggaataagtc 4310

tttgaacaat ctgggtggca agggctggta gattatttta gacatgattg tctgtttaaa 4370

actctccttt cactttttat cctccctgga gctacagctg ttcgccatca catcactccc 4430

atcctatcct ttctgtcact gtcaagcaaa acaatcagta gttactaatc gctgaactct 4490

caatattgtg gggcattttc cccccagttg attaattttg ctttaaagac tgacacagac 4550

ttagaatcaa atttattttt ctggaattaa cactctgtga ctcaaagtag tgccactgca 4610

gtgtcttttt aaactggaaa cagaattgga aaactgcctg acttatcttg catccctttg 4670

aatgagttta cagactgcca gtgtctgcaa aagttgaaag caaatgggag atgatgtcag 4730

aggcatctgt ttcctttacc atctgcatct tattataaat gtagtcgtca taaagtgtgg 4790

tttattttat tttggtaggc tctgaaatca aaatgctacg ccattataag ccagtggagt 4850

aattacaatg tattggatga aaacataagg cagtgtggag acttgatgaa aatctctgta 4910

cagattgcag tcttcttcct gatgtttcaa actgtggttc ccccaagctc tctaacactt 4970

ggaagtctgt cattctgacc tagataaaag tggttctttc tcagtagtta ttattatgtc 5030

aaaatgtgcc tccagagtga taaagctctg tatatgttag attccagcta aacctaactt 5090

ggctgtcatt tttcttccat tatagagtga gtggagactg ccccccctcc ccaacatatt 5150

ccttcccata tctctcatga ttgtccctct gtaatttcaa aatgaatgaa attcatgtga 5210

atgtaggttg agagggcact gaagacctga atctacacta gtaatctcaa gaaagattat 5270

tcattctatc tcagagttac cggcaagcat ataagatgct acttggataa tatctacatg 5330

aatattgcat gctacatggt tgataacact atttccatta ttgggcagaa tctcagtgtt 5390

tactttcaat tcctaggata tgtgatcgtg aatcagatca catataaaaa gtctggattg 5450

tcagtagtat tagatctgat caaggtagga attacaattg catgcaggta gcaagcaaga 5510

aagcagaaac tactgttccc tttattttaa cattgtacag acaatacag 5559

<210> 126
<211> 5772
<212> DNA
<213> Homo sapiens

<220>
<221> CDS
<222> (86)..(2122)

<400> 126
cagaaacatc catcagcatt cagtgaacag caaatatgga ctatcttttg agttagcgac 60

tgggatctgg ttggagagtc ctctt atg tcc tct cag gtc cat gtg caa gag 112
Met Ser Ser Gln Val His Val Gln Glu
1               5

gct tat tat ggt cct cag gca gac ctg gcg cac tgt cat gcc tac ctg 160
Ala Tyr Tyr Gly Pro Gln Ala Asp Leu Ala His Cys His Ala Tyr Leu
10                  15                  20                  25

gta gaa atg agc cag ctc ctg caa agc atg gac gtc ctg cat cgg aca 208

Val Glu Met Ser Gln Leu Leu Gln Ser Met Asp Val Leu His Arg Thr
                30                  35                  40

tac tcg gca cca gct atc aac gcc atc cag ggt gga tct ttt gaa agt      256
Tyr Ser Ala Pro Ala Ile Asn Ala Ile Gln Gly Gly Ser Phe Glu Ser
            45                  50                  55

ccc aaa aag gaa aaa aga tcg cac agg agg tgg cgg tcc aga gct att      304
Pro Lys Lys Glu Lys Arg Ser His Arg Arg Trp Arg Ser Arg Ala Ile
        60                  65                  70

ggc aaa gat gct aaa gga aca ctg cag gtc ccg aaa cct ttt tct ggc      352
Gly Lys Asp Ala Lys Gly Thr Leu Gln Val Pro Lys Pro Phe Ser Gly
    75                  80                  85

cca gta aga cta cac tcc tcc aat cct aat ttg tca aca cta gat ttt      400
Pro Val Arg Leu His Ser Ser Asn Pro Asn Leu Ser Thr Leu Asp Phe
90                  95                  100                 105

gga gaa gag aaa aat tat tct gat ggc tct gaa acc tca tca gag ttt      448
Gly Glu Glu Lys Asn Tyr Ser Asp Gly Ser Glu Thr Ser Ser Glu Phe
                110                 115                 120

tct aaa atg caa gaa gat ctg tgt cat att gcc cat aaa gtt tac ttc      496
Ser Lys Met Gln Glu Asp Leu Cys His Ile Ala His Lys Val Tyr Phe
            125                 130                 135

act tta agg tca gct ttt aat atc atg tca gcg gag aga gag aaa ctg      544
Thr Leu Arg Ser Ala Phe Asn Ile Met Ser Ala Glu Arg Glu Lys Leu
        140                 145                 150

aag cag ctg atg gag cag gat gcc tcc tcc tcc ccg tct gct cag gtc      592
Lys Gln Leu Met Glu Gln Asp Ala Ser Ser Ser Pro Ser Ala Gln Val
    155                 160                 165

att ggt ctg aag aac gcc ctg tca tcc gcc cta gca caa aac aca gat      640
Ile Gly Leu Lys Asn Ala Leu Ser Ser Ala Leu Ala Gln Asn Thr Asp
170                 175                 180                 185

ctt aaa gaa cgc tta cgc aga atc cat gcc gag tct ctg ctc ctc gac      688
Leu Lys Glu Arg Leu Arg Arg Ile His Ala Glu Ser Leu Leu Leu Asp
                190                 195                 200

tcc ccc gct gtc gcc aag tcg ggt gac aat ctg gca gag gaa aac tcc      736
Ser Pro Ala Val Ala Lys Ser Gly Asp Asn Leu Ala Glu Glu Asn Ser
            205                 210                 215

aga gat gaa aac cga gct cta gtt cat cag ctt tct aat gaa agt aga      784
Arg Asp Glu Asn Arg Ala Leu Val His Gln Leu Ser Asn Glu Ser Arg
        220                 225                 230

ctc tcc atc act gac tcc ctt tct gag ttt ttt gat gct cag gaa gtt      832
Leu Ser Ile Thr Asp Ser Leu Ser Glu Phe Phe Asp Ala Gln Glu Val
    235                 240                 245

ctg tta tct cca agc tct tca gaa aac gag att tct gat gat gac tca      880
Leu Leu Ser Pro Ser Ser Ser Glu Asn Glu Ile Ser Asp Asp Asp Ser
250                 255                 260                 265

tat gtc agt gac ata agt gat aat ctt tcc tta gat aat ctc agt aat      928

Tyr Val Ser Asp Ile Ser Asp Asn Leu Ser Leu Asp Asn Leu Ser Asn
                270                 275                 280

gat tta gat aat gag aga cag acc ttg ggg cct gtc ctt gat agt ggt      976
Asp Leu Asp Asn Glu Arg Gln Thr Leu Gly Pro Val Leu Asp Ser Gly
            285                 290                 295

cgg gaa gcg aag tcc cgg aga aga acg tgc ctg ccg gcg ccc tgc ccg     1024
Arg Glu Ala Lys Ser Arg Arg Arg Thr Cys Leu Pro Ala Pro Cys Pro
        300                 305                 310

agc agc agt aac atc agc ctg tgg aac atc ctg agg aac aac atc ggg     1072
Ser Ser Ser Asn Ile Ser Leu Trp Asn Ile Leu Arg Asn Asn Ile Gly
    315                 320                 325

aag gac ctg tcc aag gtg gcc atg ccg gtg gag ctg aac gag ccc ctg     1120
Lys Asp Leu Ser Lys Val Ala Met Pro Val Glu Leu Asn Glu Pro Leu
330                 335                 340                 345

aac acg ctg cag agg ctc tgc gag gag ctg gag tac agc gag ctc ctg     1168
Asn Thr Leu Gln Arg Leu Cys Glu Glu Leu Glu Tyr Ser Glu Leu Leu
                350                 355                 360

gac aag gcc gcg cag att ccc agc ccc ctg gaa agg atg gta tat gtg     1216
Asp Lys Ala Ala Gln Ile Pro Ser Pro Leu Glu Arg Met Val Tyr Val
            365                 370                 375

gca gcc ttt gcc ata tca gcg tat gca tct agc tac tac cga gct gga     1264
Ala Ala Phe Ala Ile Ser Ala Tyr Ala Ser Ser Tyr Tyr Arg Ala Gly
        380                 385                 390

agc aag cca ttt aat ccg gtt ctt gga gaa aca tat gaa tgt att cgg     1312
Ser Lys Pro Phe Asn Pro Val Leu Gly Glu Thr Tyr Glu Cys Ile Arg
    395                 400                 405

gag gac aag ggc ttc cag ttt ttt tca gaa cag gtc agc cac cat ccg     1360
Glu Asp Lys Gly Phe Gln Phe Phe Ser Glu Gln Val Ser His His Pro
410                 415                 420                 425

cct atc tct gcg tgt cat gct gag tct aga aat ttt gtt ttc tgg caa     1408
Pro Ile Ser Ala Cys His Ala Glu Ser Arg Asn Phe Val Phe Trp Gln
                430                 435                 440

gat gtg aga tgg aaa aac aaa ttc tgg ggc aaa tcc atg gaa att gtt     1456
Asp Val Arg Trp Lys Asn Lys Phe Trp Gly Lys Ser Met Glu Ile Val
            445                 450                 455

cca att ggc aca acc cat gtg act ctg cca gtt ttt ggg gat cat ttt     1504
Pro Ile Gly Thr Thr His Val Thr Leu Pro Val Phe Gly Asp His Phe
        460                 465                 470

gag tgg aac aaa gtg acc tct tgc atc cat aac atc tta agc ggg cag     1552
Glu Trp Asn Lys Val Thr Ser Cys Ile His Asn Ile Leu Ser Gly Gln
    475                 480                 485

agg tgg att gag cac tat gga gag att gtc atc aag aac ctg cat gat     1600
Arg Trp Ile Glu His Tyr Gly Glu Ile Val Ile Lys Asn Leu His Asp
490                 495                 500                 505

gat tcc tgc tac tgc aaa gtg aat ttt ata aag gca aaa tac tgg agc     1648

Asp Ser Cys Tyr Cys Lys Val Asn Phe Ile Lys Ala Lys Tyr Trp Ser
                510                 515                 520

act aat gcc cat gag att gaa ggc aca gtg ttt gac agg agt gga aaa 1696
Thr Asn Ala His Glu Ile Glu Gly Thr Val Phe Asp Arg Ser Gly Lys
            525                 530                 535

gcg gtt cat cgg ctg ttt ggg aaa tgg cat gaa agc atc tac tgt ggc 1744
Ala Val His Arg Leu Phe Gly Lys Trp His Glu Ser Ile Tyr Cys Gly
        540                 545                 550

ggc ggc tcc tct tct gcc tgt gta tgg aga gca aat cct atg ccg aaa 1792
Gly Gly Ser Ser Ser Ala Cys Val Trp Arg Ala Asn Pro Met Pro Lys
    555                 560                 565

ggc tac gag caa tac tat agc ttc aca cag ttt gcg ctg gaa tta aat 1840
Gly Tyr Glu Gln Tyr Tyr Ser Phe Thr Gln Phe Ala Leu Glu Leu Asn
570                 575                 580                 585

gaa atg gat cca tca tca aag tct tta ttg cca cct act gac act cga 1888
Glu Met Asp Pro Ser Ser Lys Ser Leu Leu Pro Pro Thr Asp Thr Arg
                590                 595                 600

ttt agg cca gac cag agg ttt cta gag gaa ggg aac tta gaa gaa gct 1936
Phe Arg Pro Asp Gln Arg Phe Leu Glu Glu Gly Asn Leu Glu Glu Ala
            605                 610                 615

gaa ata caa aag cag agg att gaa caa ctg cag aga gaa agg cgg cgg 1984
Glu Ile Gln Lys Gln Arg Ile Glu Gln Leu Gln Arg Glu Arg Arg Arg
        620                 625                 630

gtc tta gaa gaa aat cat gtg gag cac cag cct cgg ttt ttc agg aaa 2032
Val Leu Glu Glu Asn His Val Glu His Gln Pro Arg Phe Phe Arg Lys
    635                 640                 645

tcc gac gat gac tct tgg gtg agc aac ggc acc tat ttg gaa ctt aga 2080
Ser Asp Asp Asp Ser Trp Val Ser Asn Gly Thr Tyr Leu Glu Leu Arg
650                 655                 660                 665

aaa gat ctt ggt ttt tcc aaa ctg gac cat cct gtc tta tgg tgaaaaa 2129
Lys Asp Leu Gly Phe Ser Lys Leu Asp His Pro Val Leu Trp
                670                 675

gtaaagaaga aagataacat tagtgtattt ctcctgtgct tgccttctga agtggcacaa 2189

acctgtgttt atatatttaa aagatactct aggatgatca cttgtgctta gcttagcatt 2249

gtaactcttt aagtctatat tttcctcagt gcgtttcttt acaatttcaa atgttaccct 2309

gattgtttat atgaatgtag aacaccttga catttctttt tatatataaa ctatttaata 2369

aaaatgaaag attgaatgtt catgtgtggg ttaaaaaaag aagctttaac actaattttc 2429

caaaggttag ggaagattcc aattaaattt atgccttata aaattatgtt gtagaaaaaa 2489

aatcaacctc tcccaggtgc attaagaaat aagaattccc agggttactc acccatgcgt 2549

aagctaccca agtttaattt ggtagctgaa atatcttttt gcctcagaca gctcttgaat 2609

tgctcataca gaacaattct gctggtgctg gagtctgaag aatattttca tttgcatttt 2669

agtggttagg gagaggatat aagattaatg gaatgtatat ttttatataa gacgtatacg 2729
gcaccttctt gaaaaggaag catttgaact tgttcctccc tatagttcta ttgccttata 2789
tgcaaaattg taccctgttg ctcagagaaa ttattcataa gagaaaagaa ttccaattaa 2849
ttaaatatca caatagcatc ccagagagac agtaggaaat ttctccttag tgagagctga 2909
gtccttgaga agttaagaga ctgtttcctg cttcccaccc caccccgttt tttgtccctc 2969
cgtttgatca cctccctgtt gagtatggaa tgtcccagtt taatataaaa catacagttt 3029
attccacaca gcagtttgac tttgtaaaag tttagcaaaa taactgattg ttttggataa 3089
ctcaacatgt ttttcttaaa tgctgtttta gtattttcta cctcttaata agcaccatat 3149
tttagatctt gtataaaaag ttgccatctg ccttatagac aaatgtagag aattgatgtt 3209
cttgctttgt gttgtgttct ggtaaagctt taagtgagtg tcttacccct ttcctatact 3269
ttcttgttat ccatatcatt cttttgtgag ttttgcagca gtcttgaata atacagatta 3329
taactactga aaggggaacc ttcgtctttt taaatgtgtc atttcacatt acaataatat 3389
gtgtagtagt tgaattgtgt tatcaaagca tgtccaactt ctgtccagat gacattaaaa 3449
ccaaagccta attgttaagc ctcttattct aaagtccata gcaatgctat ggaatatcaa 3509
gtataatgat gtagtaaaaa gatttctcca gaaaacactt tgagatactt aaagaaattc 3569
agagcatatc gaatgttatg aatgaagagt aaaataatta caggaaaaga atgcatgatt 3629
tttcattgtc acaccaaata accagttgga caatattgca ttttcaaaat ggagagttat 3689
ttaaagtgga tgtgtagcct tctggaatct gcacgtgact ggcattttaa aacaactcat 3749
taaatattgg attcatttta tcgagatgcc aagatgaaca caatgtgaat atcatttcca 3809
tttctaagca gttgaatgac acagtcagat tctttggtgt atgctttgtt ctttccatct 3869
caagcattta tccaaattct gctcacagac atgaggaagc aggctgtaga tttaagggca 3929
ctcaagggga aaacaaatgt agtttccaca gtgtgaggat aaatgtagaa tcgtggtaat 3989
attggctgtt aaattggcct gctccagaga ctggctcaaa aaagaaagaa gcaacgaaaa 4049
caaaatgcac agcctgaatg gacgtagcac tgtcatgtgg catttggaaa atctttaatt 4109
atcctaatgt tatttcactt gcccttatgc tttattttag agtcccaggg acaactggat 4169
gaaacattac gtctgtagtc atcttatttg agatgggggt gggaacgaat taaaatatga 4229
tagagaacta tatgcagatt ttaaaatcta gtcagtacta gcatctataa agggcttttc 4289
tgaaatttaa acagcttggt gatgcatcct gatattataa gcttaaaact atcttgaggg 4349
ggaaatgact tcttttctcc ttctgtccct tttctcaaaa gcatctttcc cccaaatgta 4409
tgtctctagg acattttata gttttaagag gaagaaaaca gaaacgtggc atcatttggg 4469

aagaaccaag gtagaattta attttctgga ctttaaatct cctggttaaa taatgctaat 4529

cactgtacca tttgagttac atgttttaac ctcttttttt tttctaatta ttccatccaa 4589

aatttctggt gaattacaca gaaatttttg gaaatgggac tttgtgcagg taaccaccct 4649

gcacttcagg taatgttcct gtcacagctg tcgactaccc cacgctgcag gaaatccatt 4709

cagaaatgag ctaacagtct ccagtgtagc aggagagcca gtcacttccc tccccaggcc 4769

ccaaagcacc ccaaggctgg tattccttga gtcataggta ttaataataa aagcctcaat 4829

gcagcttctc catgtagttc ctctcctaca agccaggtgg attctggtcc taactaaaga 4889

gatgggagtt cacctgaggg caagaagtac ccaggatgcc caaacagccg cacaggtgtc 4949

ctgtgcttcc tgtgagactt cctgggggaa atatacaaac taatatattt tttaatgttt 5009

acgtcattta cactgctgct tttctcatat tctgctttta attacttgta gtaaatcatt 5069

tgtttgggct tcaagcactg tcttccatct gcatcttcca gatttatcat gagtgatctt 5129

gttctactgt taaagatgtc aagcctaaaa agtaataaag tttttatttg gctgttgaac 5189

cttgatgtag cccctactac atacactaca agttatgcct tctggctact acagaatctc 5249

cgcagacctt ttagttatga gtagaagcaa taagaaggtg tccttatggt cttaggaata 5309

atagttccaa tgtattggca taatttgttt gaatgtcttt attttgatat taacaccggc 5369

ataaatctat ttttgctacc agatgatagc tattttcatt tgctctattt tcctctgcat 5429

tgtactaatc tgttactcag tgttagtctt catttttctg tgttggaaac ctgcgtcatg 5489

taaatatctg caaatcatga cagtctaaag tgcaaaccat tttcagagac tttggggaat 5549

gcagtgatca gaagtggcta atttatttta tttctgatta ttttatccag agggtacttt 5609

tttaatggat atttgtaaat cttccactta accactgaaa attattttgt tttaatccca 5669

cccttccatc catacctctg cctccccaaa aagctcctat taatttgctt atccccctca 5729

tgtagctagt tgaatgtgaa taaataacat ggaaaaaaaa aaa 5772

<210> 127
<211> 4682
<212> DNA
<213> Homo sapiens

<220>
<221> CDS
<222> (310)..(4443)

<400> 127
   tttcgtgggt tagacacact cgaataatcc ctctggctgg gctgaatttg tgggaattta 60
   ggaagccaga gtgctggaaa tacagcagcc tttgaagtac cctctgttaa tttggatgga 120
   tctcagtgtg ccccgttcga gacctctcca ccaacccctt ctgatcttgc gatttgctct 180
   tcttgacttt aattagtatc taggaaagtc taaactttgg acctacctct ttttttgata 240
   ctcatttttg tacttttgct ctctgggatt ggtttcttaa agaatctgga tcctttttaa 300
   tatgtcaaa atg agt ctg ctg atg ttt aca caa cta ctg ctc tgt gga 348
   Met Ser Leu Leu Met Phe Thr Gln Leu Leu Leu Cys Gly
   1 5 10
   ttt tta tat gtt cgg gtt gat gga tcg cgt ctt cgc cag gag gac ttt 396
   Phe Leu Tyr Val Arg Val Asp Gly Ser Arg Leu Arg Gln Glu Asp Phe
   15 20 25
   ccc ccg cgg att gtg gag cat cct tcc gat gtc atc gtc tct aag ggc 444
   Pro Pro Arg Ile Val Glu His Pro Ser Asp Val Ile Val Ser Lys Gly
   30 35 40 45
   gag ccc acg act ctg aac tgc aag gcg gag ggc cgg cca acg ccc acc 492
   Glu Pro Thr Thr Leu Asn Cys Lys Ala Glu Gly Arg Pro Thr Pro Thr
   50 55 60
   att gag tgg tac aaa gat ggg gag cga gtg gag act gac aag gac gat 540
   Ile Glu Trp Tyr Lys Asp Gly Glu Arg Val Glu Thr Asp Lys Asp Asp
   65 70 75
   ccc cgg tcc cac agg atg ctt ctg ccc agc gga tcc tta ttc ttc ttg 588
   Pro Arg Ser His Arg Met Leu Leu Pro Ser Gly Ser Leu Phe Phe Leu
   80 85 90
   cgc atc gtg cac ggg cgc agg agt aaa cct gat gaa gga agc tac gtt 636
   Arg Ile Val His Gly Arg Arg Ser Lys Pro Asp Glu Gly Ser Tyr Val
   95 100 105
   tgt gtt gcg agg aac tat ctt ggt gaa gca gtg agt cga aat gcg tct 684
   Cys Val Ala Arg Asn Tyr Leu Gly Glu Ala Val Ser Arg Asn Ala Ser
   110 115 120 125
   ctg gaa gtg gca ttg tta cga gat gac ttc cga caa aac ccc aca gat 732
   Leu Glu Val Ala Leu Leu Arg Asp Asp Phe Arg Gln Asn Pro Thr Asp
   130 135 140
   gtt gta gtg gca gct gga gag cct gca atc ctg gag tgc cag cct ccc 780
   Val Val Val Ala Ala Gly Glu Pro Ala Ile Leu Glu Cys Gln Pro Pro
   145 150 155
   cgg gga cac cca gaa ccc acc atc tac tgg aaa aaa gac aaa gtt cga 828
   Arg Gly His Pro Glu Pro Thr Ile Tyr Trp Lys Lys Asp Lys Val Arg
   160 165 170
   att gat gac aag gaa gaa aga ata agt atc cgt ggt gga aaa ctg atg 876
   Ile Asp Asp Lys Glu Glu Arg Ile Ser Ile Arg Gly Gly Lys Leu Met
   175 180 185
   atc tcc aat acc agg aaa agt gat gca ggg atg tat act tgt gtt ggt 924
   Ile Ser Asn Thr Arg Lys Ser Asp Ala Gly Met Tyr Thr Cys Val Gly
   190 195 200 205
   acc aat atg gtg gga gaa agg gac agt gac cca gca gag ctg act gtc 972
   Thr Asn Met Val Gly Glu Arg Asp Ser Asp Pro Ala Glu Leu Thr Val
   210 215 220
   ttt gaa cga ccc aca ttt ctc agg agg cca att aac cag gtg gta ctg 1020
   Phe Glu Arg Pro Thr Phe Leu Arg Arg Pro Ile Asn Gln Val Val Leu
   225 230 235
   gag gaa gaa gct gta gaa ttt cgt tgt caa gtc caa gga gat cct caa 1068
   Glu Glu Glu Ala Val Glu Phe Arg Cys Gln Val Gln Gly Asp Pro Gln
   240 245 250
   cca act gtg agg tgg aaa aag gat gat gca gac ttg cca aga gga agg 1116
   Pro Thr Val Arg Trp Lys Lys Asp Asp Ala Asp Leu Pro Arg Gly Arg
   255 260 265
   tat gac atc aaa gac gat tac aca cta aga att aaa aag acc atg agt 1164
   Tyr Asp Ile Lys Asp Asp Tyr Thr Leu Arg Ile Lys Lys Thr Met Ser
   270 275 280 285
   aca gat gaa ggc acc tat atg tgt att gct gag aat cgg gtt gga aaa 1212
   Thr Asp Glu Gly Thr Tyr Met Cys Ile Ala Glu Asn Arg Val Gly Lys
   290 295 300
   atg gaa gcc tct gct aca ctc acc gtc cga gct ccc cca cag ttt gtg 1260
   Met Glu Ala Ser Ala Thr Leu Thr Val Arg Ala Pro Pro Gln Phe Val
   305 310 315
   gtt cgg cca aga gat cag att gtt gct caa ggt cga aca gtg aca ttt 1308
   Val Arg Pro Arg Asp Gln Ile Val Ala Gln Gly Arg Thr Val Thr Phe
   320 325 330
   ccc tgt gaa act aaa gga aac cca cag cca gct gtt ttt tgg cag aaa 1356
   Pro Cys Glu Thr Lys Gly Asn Pro Gln Pro Ala Val Phe Trp Gln Lys
   335 340 345
   gaa ggc agc cag aac cta ctt ttc cca aac caa ccc cag cag ccc aac 1404
   Glu Gly Ser Gln Asn Leu Leu Phe Pro Asn Gln Pro Gln Gln Pro Asn
   350 355 360 365
   agt aga tgc tca gtg tca cca act gga gac ctc aca atc acc aac att 1452
   Ser Arg Cys Ser Val Ser Pro Thr Gly Asp Leu Thr Ile Thr Asn Ile
   370 375 380
   caa cgt tcc gac gcg ggt tac tac atc tgc cag gct tta act gtg gca 1500
   Gln Arg Ser Asp Ala Gly Tyr Tyr Ile Cys Gln Ala Leu Thr Val Ala
   385 390 395
   gga agc att tta gca aaa gct caa ctg gag gtt act gat gtt ttg aca 1548
   Gly Ser Ile Leu Ala Lys Ala Gln Leu Glu Val Thr Asp Val Leu Thr
   400 405 410
   gat aga cct cca cct ata att cta caa ggc cca gcc aac caa acg ctg 1596
   Asp Arg Pro Pro Pro Ile Ile Leu Gln Gly Pro Ala Asn Gln Thr Leu
   415 420 425
   gca gtg gat ggt aca gcg tta ctg aaa tgt aaa gcc act ggt gat cct 1644
   Ala Val Asp Gly Thr Ala Leu Leu Lys Cys Lys Ala Thr Gly Asp Pro
   430 435 440 445
   ctt cct gta att agc tgg tta aag gag gga ttt act ttt ccg ggt aga 1692
   Leu Pro Val Ile Ser Trp Leu Lys Glu Gly Phe Thr Phe Pro Gly Arg
   450 455 460
   gat cca aga gca aca att caa gag caa ggc aca ctg cag att aag aat 1740
   Asp Pro Arg Ala Thr Ile Gln Glu Gln Gly Thr Leu Gln Ile Lys Asn
   465 470 475
   tta cgg att tct gat act ggc act tat act tgt gtg gct aca agt tca 1788
   Leu Arg Ile Ser Asp Thr Gly Thr Tyr Thr Cys Val Ala Thr Ser Ser
   480 485 490
   agt gga gag act tcc tgg agt gca gtg ctg gat gtg aca gag tct gga 1836
   Ser Gly Glu Thr Ser Trp Ser Ala Val Leu Asp Val Thr Glu Ser Gly
   495 500 505
   gca aca atc agt aaa aac tat gat tta agt gac ctg cca ggg cca cca 1884
   Ala Thr Ile Ser Lys Asn Tyr Asp Leu Ser Asp Leu Pro Gly Pro Pro
   510 515 520 525
   tcc aaa ccg cag gtc act gat gtt act aag aac agt gtc acc ttg tcc 1932
   Ser Lys Pro Gln Val Thr Asp Val Thr Lys Asn Ser Val Thr Leu Ser
   530 535 540
   tgg cag cca ggt acc cct gga acc ctt cca gca agt gca tat atc att 1980
   Trp Gln Pro Gly Thr Pro Gly Thr Leu Pro Ala Ser Ala Tyr Ile Ile
   545 550 555
   gag gct ttc agc caa tca gtg agc aac agc tgg cag acc gtg gca aac 2028
   Glu Ala Phe Ser Gln Ser Val Ser Asn Ser Trp Gln Thr Val Ala Asn
   560 565 570
   cat gta aag acc acc ctc tat act gta aga gga ctg cgg ccc aat aca 2076
   His Val Lys Thr Thr Leu Tyr Thr Val Arg Gly Leu Arg Pro Asn Thr
   575 580 585
   atc tac tta ttc atg gtc aga gcg atc aac ccc caa ggt ctc agt gac 2124
   Ile Tyr Leu Phe Met Val Arg Ala Ile Asn Pro Gln Gly Leu Ser Asp
   590 595 600 605
   cca agt ccc atg tca gat cct gtg cgc aca caa gat atc agc cca cca 2172
   Pro Ser Pro Met Ser Asp Pro Val Arg Thr Gln Asp Ile Ser Pro Pro
   610 615 620
   gca caa gga gtg gac cac agg caa gtg cag aaa gag cta gga gat gtc 2220
   Ala Gln Gly Val Asp His Arg Gln Val Gln Lys Glu Leu Gly Asp Val
   625 630 635
   ctt gtc cgt ctt cat aat cca gtt gtg ctg act ccc acc acg gtt cag 2268
   Leu Val Arg Leu His Asn Pro Val Val Leu Thr Pro Thr Thr Val Gln
   640 645 650
   gtc aca tgg acg gtt gat cgc caa ccc cag ttt atc caa ggc tac cga 2316
   Val Thr Trp Thr Val Asp Arg Gln Pro Gln Phe Ile Gln Gly Tyr Arg
   655 660 665
   gtg atg tat cgt cag act tca ggt ctg cag gcg aca tct tcg tgg cag 2364
   Val Met Tyr Arg Gln Thr Ser Gly Leu Gln Ala Thr Ser Ser Trp Gln
   670 675 680 685
   aat tta gat gcc aaa gtc ccg act gaa cga agt gct gtc tta gtc aac     2412
   Asn Leu Asp Ala Lys Val Pro Thr Glu Arg Ser Ala Val Leu Val Asn
                   690                 695                 700
   ctg aaa aag ggg gtg act tat gaa att aaa gta cgg cca tat ttt aat     2460
   Leu Lys Lys Gly Val Thr Tyr Glu Ile Lys Val Arg Pro Tyr Phe Asn
               705                 710                 715
   gag ttc caa gga atg gat agt gaa tct aaa acg gtt cgt act act gaa     2508
   Glu Phe Gln Gly Met Asp Ser Glu Ser Lys Thr Val Arg Thr Thr Glu
           720                 725                 730
   gaa gcc cca agt gcc cca cca cag tct gtc act gta ctg aca gtt gga     2556
   Glu Ala Pro Ser Ala Pro Pro Gln Ser Val Thr Val Leu Thr Val Gly
       735                 740                 745
   agc tac aat agc aca agt att agt gtt tcc tgg gat cct cct cct cca     2604
   Ser Tyr Asn Ser Thr Ser Ile Ser Val Ser Trp Asp Pro Pro Pro Pro
   750                 755                 760                 765
   gat cac cag aat gga att atc caa gaa tac aag atc tgg tgt cta gga     2652
   Asp His Gln Asn Gly Ile Ile Gln Glu Tyr Lys Ile Trp Cys Leu Gly
                   770                 775                 780
   aat gaa acg cga ttc cat atc aac aaa act gtg gat gca gcc att cgg     2700
   Asn Glu Thr Arg Phe His Ile Asn Lys Thr Val Asp Ala Ala Ile Arg
               785                 790                 795
   tcc gta ata att ggt gga tta ttc cca ggt att caa tac cgg gta gag     2748
   Ser Val Ile Ile Gly Gly Leu Phe Pro Gly Ile Gln Tyr Arg Val Glu
           800                 805                 810
   gtt gca gct agt acc agt gca ggg gtt gga gta aag agt gag cca cag     2796
   Val Ala Ala Ser Thr Ser Ala Gly Val Gly Val Lys Ser Glu Pro Gln
       815                 820                 825
   cca ata ata atc ggg aga cgc aat gaa gtt gtc att act gaa aac aat     2844
   Pro Ile Ile Ile Gly Arg Arg Asn Glu Val Val Ile Thr Glu Asn Asn
   830                 835                 840                 845
   aac agc ata act gag caa atc act gat gtg gtg aag caa cca gcc ttt     2892
   Asn Ser Ile Thr Glu Gln Ile Thr Asp Val Val Lys Gln Pro Ala Phe
                   850                 855                 860
   ata gct ggt att ggt ggt gcc tgc tgg gta att ctg atg ggt ttt agc     2940
   Ile Ala Gly Ile Gly Gly Ala Cys Trp Val Ile Leu Met Gly Phe Ser
               865                 870                 875
   ata tgg ttg tat tgg cga aga aag aag agg aag gga ctc agt aat tat     2988
   Ile Trp Leu Tyr Trp Arg Arg Lys Lys Arg Lys Gly Leu Ser Asn Tyr
           880                 885                 890
   gct gtt acg ttt caa aga gga gat gga gga cta atg agc aat gga agc     3036
   Ala Val Thr Phe Gln Arg Gly Asp Gly Gly Leu Met Ser Asn Gly Ser
       895                 900                 905
   cgt cca ggt ctt ctc aat gct ggt gat ccc agc tat cca tgg ctt gct     3084
   Arg Pro Gly Leu Leu Asn Ala Gly Asp Pro Ser Tyr Pro Trp Leu Ala
   910                 915                 920                 925
   gat tct tgg cca gcc acg agc ttg cca gta aat aat agc aac agt ggc     3132
   Asp Ser Trp Pro Ala Thr Ser Leu Pro Val Asn Asn Ser Asn Ser Gly
                   930                 935                 940
   cca aat gag att gga aat ttt ggc cgt gga gat gtg ctg cca cca gtt     3180
   Pro Asn Glu Ile Gly Asn Phe Gly Arg Gly Asp Val Leu Pro Pro Val
               945                 950                 955
   cca ggc caa ggg gat aaa aca gca acg atg ctc tca gat gga gcc att     3228
   Pro Gly Gln Gly Asp Lys Thr Ala Thr Met Leu Ser Asp Gly Ala Ile
           960                 965                 970
   tat agt agc att gac ttc act acc aaa acc agt tac aac agt tcc agc     3276
   Tyr Ser Ser Ile Asp Phe Thr Thr Lys Thr Ser Tyr Asn Ser Ser Ser
       975                 980                 985
   caa ata aca cag gct acc cca tat gcc acg aca cag atc ttg cat tcc     3324
   Gln Ile Thr Gln Ala Thr Pro Tyr Ala Thr Thr Gln Ile Leu His Ser
   990                 995                 1000                1005
   aac agc ata cat gaa ttg gct gtc gat ctg cct gat cca caa tgg aaa     3372
   Asn Ser Ile His Glu Leu Ala Val Asp Leu Pro Asp Pro Gln Trp Lys
                   1010                1015                1020
   agc tca att cag caa aaa aca gat ctg atg gga ttt ggt tat tct cta     3420
   Ser Ser Ile Gln Gln Lys Thr Asp Leu Met Gly Phe Gly Tyr Ser Leu
               1025                1030                1035
   cct gat cag aac aaa ggt aac aat ggt ggg aaa ggt gaa aaa aag aag     3468
   Pro Asp Gln Asn Lys Gly Asn Asn Gly Gly Lys Gly Glu Lys Lys Lys
           1040                1045                1050
   aaa aat aaa aac tct tct aaa cca cag aaa aac aat gga tcc act tgg     3516
   Lys Asn Lys Asn Ser Ser Lys Pro Gln Lys Asn Asn Gly Ser Thr Trp
       1055                1060                1065
   gcc aat gtc cct cta cct ccc ccc cca gtc cag ccc ctt cct ggc acg     3564
   Ala Asn Val Pro Leu Pro Pro Pro Pro Val Gln Pro Leu Pro Gly Thr
   1070                1075                1080                1085
   gag ctg gaa cac tat gca gtg gaa caa caa gaa aat ggc tat gac agt     3612
   Glu Leu Glu His Tyr Ala Val Glu Gln Gln Glu Asn Gly Tyr Asp Ser
                   1090                1095                1100
   gat agc tgg tgc cca cca ttg cca gta caa act tac tta cac caa ggt     3660
   Asp Ser Trp Cys Pro Pro Leu Pro Val Gln Thr Tyr Leu His Gln Gly
               1105                1110                1115
   ctg gaa gat gaa ctg gaa gaa gat gat gat agg gtc cca aca cct cct     3708
   Leu Glu Asp Glu Leu Glu Glu Asp Asp Asp Arg Val Pro Thr Pro Pro
           1120                1125                1130
   gtt cga ggc gtg gct tct tct cct gct atc tcc ttt gga cag cag tcc     3756
   Val Arg Gly Val Ala Ser Ser Pro Ala Ile Ser Phe Gly Gln Gln Ser
       1135                1140                1145
   act gca act ctt act cca tcc cca cgg gaa gag atg caa ccc atg ctg     3804
   Thr Ala Thr Leu Thr Pro Ser Pro Arg Glu Glu Met Gln Pro Met Leu
   1150                1155                1160                1165
   cag gct cac ctg gat gag ttg aca aga gcc tat cag ttt gat ata gca 3852
   Gln Ala His Leu Asp Glu Leu Thr Arg Ala Tyr Gln Phe Asp Ile Ala
   1170 1175 1180
   aaa caa aca tgg cac att caa agc aat aat caa cct cca cag cct cca 3900
   Lys Gln Thr Trp His Ile Gln Ser Asn Asn Gln Pro Pro Gln Pro Pro
   1185 1190 1195
   gtt cca ccg tta ggt tat gtg tct gga gcc ttg att tct gat ttg gaa 3948
   Val Pro Pro Leu Gly Tyr Val Ser Gly Ala Leu Ile Ser Asp Leu Glu
   1200 1205 1210
   acg gat gtt gca gat gat gat gcc gac gac gaa gag gaa gct tta gaa 3996
   Thr Asp Val Ala Asp Asp Asp Ala Asp Asp Glu Glu Glu Ala Leu Glu
   1215 1220 1225
   atc ccc agg ccc ctg aga gca ctg gac cag act cct gga tcc agc atg 4044
   Ile Pro Arg Pro Leu Arg Ala Leu Asp Gln Thr Pro Gly Ser Ser Met
   1230 1235 1240 1245
   gac aat cta gac agc tct gtg aca gga aaa gcc ttt acc tcc tct caa 4092
   Asp Asn Leu Asp Ser Ser Val Thr Gly Lys Ala Phe Thr Ser Ser Gln
   1250 1255 1260
   aga cct cga cct acc agc cca ttt tct act gac agt aac acc agt gca 4140
   Arg Pro Arg Pro Thr Ser Pro Phe Ser Thr Asp Ser Asn Thr Ser Ala
   1265 1270 1275
   gcc ctg agt caa agt cag agg cct cgg ccc act aaa aaa cac aag gga 4188
   Ala Leu Ser Gln Ser Gln Arg Pro Arg Pro Thr Lys Lys His Lys Gly
   1280 1285 1290
   ggg cgg atg gac caa caa cca gca ttg cct cat cga agg gaa gga atg 4236
   Gly Arg Met Asp Gln Gln Pro Ala Leu Pro His Arg Arg Glu Gly Met
   1295 1300 1305
   aca gat gag gag gcc ttg gtg ccc tat agc aag ccc agt ttc cca tct 4284
   Thr Asp Glu Glu Ala Leu Val Pro Tyr Ser Lys Pro Ser Phe Pro Ser
   1310 1315 1320 1325
   cca ggt ggc cac agc tca tca gga aca gct tct tct aag gga tcc act 4332
   Pro Gly Gly His Ser Ser Ser Gly Thr Ala Ser Ser Lys Gly Ser Thr
   1330 1335 1340
   gga cct agg aaa acc gag gtg ttg aga gca ggc cac cag cgc aat gcc 4380
   Gly Pro Arg Lys Thr Glu Val Leu Arg Ala Gly His Gln Arg Asn Ala
   1345 1350 1355
   agc gac ctt ctt gac ata gga tat atg ggc tcc aac agt caa gga cag 4428
   Ser Asp Leu Leu Asp Ile Gly Tyr Met Gly Ser Asn Ser Gln Gly Gln
   1360 1365 1370
   ttt aca ggt gaa tta tagtaaatga gaggagacat acaaagctgc tctgaaggac 4483
   Phe Thr Gly Glu Leu
   1375
   catcaggtcc ggactcatgg aagtgatgac tctaaacagt gcaatgaaca atttatttat 4543
   gtactattaa aagaactgta aatgcaatgt aaagacacac agccacacat atcccacaga 4603
   tattttcatt gtgttcttct cttaagtaca ccacccacct taactctttc ttgtcaggag 4663
   tatataaaaa aaaaaaaaa 4682

<210> 128
<211> 4794
<212> DNA
<213> Homo sapiens

<220>
<221> CDS
<222> (226)..(4791)

<400> 128
atggagccac tcgccaggct ccagtcaaag agcaaagccg cggggaccca ctggatcctt 60

accatgacca cagaggggtg ggccgaaggc gtggggagga gctccccatc caggtcctct 120

gtcccttccg ccagcccctc gacctcggtc accgtcagca gcatggtcaa acacttcaga 180

gctttgcaag agcaggcaag gacctaccta gatctccttt gctcc atg tgt gac 234
Met Cys Asp
1

ctg tca aat gct tcg gtg aaa acc aca gca aaa gac att caa caa aca 282
Leu Ser Asn Ala Ser Val Lys Thr Thr Ala Lys Asp Ile Gln Gln Thr
    5                   10                  15

gag caa acg att gaa caa aag ctt gtc cag gcc cag aac tta act cag 330
Glu Gln Thr Ile Glu Gln Lys Leu Val Gln Ala Gln Asn Leu Thr Gln
20                  25                  30                  35

ggc tgg gaa gag atc aag cac ctg aag tct gag ctc tgg att tac ctg 378
Gly Trp Glu Glu Ile Lys His Leu Lys Ser Glu Leu Trp Ile Tyr Leu
                40                  45                  50

caa gat gct gat cag caa ctg cag aac atg aag agg agg cac tct gag 426
Gln Asp Ala Asp Gln Gln Leu Gln Asn Met Lys Arg Arg His Ser Glu
            55                  60                  65

ctg gag ctg aac att gca cag aac atg gtt tca caa gtt aag gat ttt 474
Leu Glu Leu Asn Ile Ala Gln Asn Met Val Ser Gln Val Lys Asp Phe
        70                  75                  80

gtt aag aaa cta cag agc aaa cag gca tcc gtg aac acc ata ata gaa 522
Val Lys Lys Leu Gln Ser Lys Gln Ala Ser Val Asn Thr Ile Ile Glu
    85                  90                  95

aag gtg aat aag tta aca aag aag gag gaa tcg cct gaa cac aag gaa 570
Lys Val Asn Lys Leu Thr Lys Lys Glu Glu Ser Pro Glu His Lys Glu
100                 105                 110                 115

ata aat cat tta aat gat cag tgg ctc gat ttg tgc cgt cag tct aac 618
Ile Asn His Leu Asn Asp Gln Trp Leu Asp Leu Cys Arg Gln Ser Asn
                120                 125                 130

aac ctg tgc ttg caa agg gaa gag gat ctt cag aga aca aga gat tac 666

Asn Leu Cys Leu Gln Arg Glu Glu Asp Leu Gln Arg Thr Arg Asp Tyr
            135                 140                 145

cat gac tgt atg aat gtt gtt gaa gtg ttc cta gaa aaa ttt act aca 714
His Asp Cys Met Asn Val Val Glu Val Phe Leu Glu Lys Phe Thr Thr
        150                 155                 160

gaa tgg gat aac ttg gcc aga tct gat gca gag agt aca gct gtc cac 762
Glu Trp Asp Asn Leu Ala Arg Ser Asp Ala Glu Ser Thr Ala Val His
    165                 170                 175

ctg gaa gct ttg aaa aag tta gca ttg gca ttg cag gag aga aag tat 810
Leu Glu Ala Leu Lys Lys Leu Ala Leu Ala Leu Gln Glu Arg Lys Tyr
180                 185                 190                 195

gct att gaa gat ctg aaa gat caa aag cag aaa atg ata gag cat ctg 858
Ala Ile Glu Asp Leu Lys Asp Gln Lys Gln Lys Met Ile Glu His Leu
                200                 205                 210

aat tta gat gac aag gag tta gtc aaa gaa cag acg agt cat tta gag 906
Asn Leu Asp Asp Lys Glu Leu Val Lys Glu Gln Thr Ser His Leu Glu
            215                 220                 225

caa cgt tgg ttt cag ctt gag gac ctc att aaa agg aaa atc caa gtg 954
Gln Arg Trp Phe Gln Leu Glu Asp Leu Ile Lys Arg Lys Ile Gln Val
        230                 235                 240

tca gtc acc aac ttg gag gag tta aat gtg gtg cag tcc aga ttt cag 1002
Ser Val Thr Asn Leu Glu Glu Leu Asn Val Val Gln Ser Arg Phe Gln
    245                 250                 255

gag cta atg gag tgg gca gaa gag caa caa ccc aac atc gcc gag gcc 1050
Glu Leu Met Glu Trp Ala Glu Glu Gln Gln Pro Asn Ile Ala Glu Ala
260                 265                 270                 275

ctt aag cag agc cct cct cca gat atg gct cag aac ctt ctc atg gat 1098
Leu Lys Gln Ser Pro Pro Pro Asp Met Ala Gln Asn Leu Leu Met Asp
                280                 285                 290

cac ctg gcc atc tgc agt gaa ctg gag gcc aag cag atg ctc ctg aaa 1146
His Leu Ala Ile Cys Ser Glu Leu Glu Ala Lys Gln Met Leu Leu Lys
            295                 300                 305

tcg ctt ata aag gac gca gac agg gtc atg gca gat ctt ggt ctc aat 1194
Ser Leu Ile Lys Asp Ala Asp Arg Val Met Ala Asp Leu Gly Leu Asn
        310                 315                 320

gag cga cag gtc atc cag aag gct ctc tct gat gca caa agc cac gtg 1242
Glu Arg Gln Val Ile Gln Lys Ala Leu Ser Asp Ala Gln Ser His Val
    325                 330                 335

aat tgt ctc agt gac tta gtg ggc cag cga aga aag tac tta aac aaa 1290
Asn Cys Leu Ser Asp Leu Val Gly Gln Arg Arg Lys Tyr Leu Asn Lys
340                 345                 350                 355

gcc ttg tcc gag aaa acc cag ttt ctc atg gca gtg ttc cag gcc acc 1338
Ala Leu Ser Glu Lys Thr Gln Phe Leu Met Ala Val Phe Gln Ala Thr
                360                 365                 370

agc caa att cag caa cat gag cga aag ata atg ttc cgt gaa cac atc 1386

Ser Gln Ile Gln Gln His Glu Arg Lys Ile Met Phe Arg Glu His Ile
            375                 380                 385

tgt ctg tta cca gat gat gtg agc aaa caa gtc aaa aca tgt aag agt      1434
Cys Leu Leu Pro Asp Asp Val Ser Lys Gln Val Lys Thr Cys Lys Ser
        390                 395                 400

gca caa gcc agc ctc aag act tac caa aat gaa gtc act gga ctt tgg      1482
Ala Gln Ala Ser Leu Lys Thr Tyr Gln Asn Glu Val Thr Gly Leu Trp
    405                 410                 415

gcc cag ggt cgc gaa cta atg aag gaa gtc aca gag cag gaa aag agt      1530
Ala Gln Gly Arg Glu Leu Met Lys Glu Val Thr Glu Gln Glu Lys Ser
420                 425                 430                 435

gaa gtg ctg ggg aag ctt cag gaa ttg cag agt gtc tat gac agt gtt      1578
Glu Val Leu Gly Lys Leu Gln Glu Leu Gln Ser Val Tyr Asp Ser Val
                440                 445                 450

tta caa aag tgc agt cac cgg tta caa gaa cta gag aag aat ttg gtt      1626
Leu Gln Lys Cys Ser His Arg Leu Gln Glu Leu Glu Lys Asn Leu Val
            455                 460                 465

tct agg aag cat ttt aag gaa gat ttt gat aaa gct tgc cac tgg cta      1674
Ser Arg Lys His Phe Lys Glu Asp Phe Asp Lys Ala Cys His Trp Leu
        470                 475                 480

aaa caa gca gat att gtt aca ttt cct gaa atc aac cta atg aat gag      1722
Lys Gln Ala Asp Ile Val Thr Phe Pro Glu Ile Asn Leu Met Asn Glu
    485                 490                 495

agt tct gag ctt cat aca caa ctg gct aaa tac caa aac att ctt gaa      1770
Ser Ser Glu Leu His Thr Gln Leu Ala Lys Tyr Gln Asn Ile Leu Glu
500                 505                 510                 515

caa tct cca gaa tat gaa aat ctt cta ctt acg ctg cag aga act ggg      1818
Gln Ser Pro Glu Tyr Glu Asn Leu Leu Leu Thr Leu Gln Arg Thr Gly
                520                 525                 530

cag acc ata tta cca tcg ctg aat gaa gtc gat cat tcc tac ctc agt      1866
Gln Thr Ile Leu Pro Ser Leu Asn Glu Val Asp His Ser Tyr Leu Ser
            535                 540                 545

gaa aag cta aat gct ttg cct cga caa ttt aat gta att gtt gcc ttg      1914
Glu Lys Leu Asn Ala Leu Pro Arg Gln Phe Asn Val Ile Val Ala Leu
        550                 555                 560

gct aaa gac aag ttc tat aaa gtc cag gaa gca att ctt gct cgg aag      1962
Ala Lys Asp Lys Phe Tyr Lys Val Gln Glu Ala Ile Leu Ala Arg Lys
    565                 570                 575

gaa tat gct tcc ttg att gag ttg aca acc cag tct ctg agt gaa ctt      2010
Glu Tyr Ala Ser Leu Ile Glu Leu Thr Thr Gln Ser Leu Ser Glu Leu
580                 585                 590                 595

gaa gcc caa ttc ttg agg atg agc aaa gtt ccc acc gac ctg gcc gtt      2058
Glu Ala Gln Phe Leu Arg Met Ser Lys Val Pro Thr Asp Leu Ala Val
                600                 605                 610

gag gag gct ctt tct ctg caa gat ggt tgc aga gcc att ctg gac gag      2106

Glu Glu Ala Leu Ser Leu Gln Asp Gly Cys Arg Ala Ile Leu Asp Glu
            615                 620                 625

gtg gcg ggc ctt ggg gag gcg gtg gat gaa ctg aac cag aaa aaa gaa 2154
Val Ala Gly Leu Gly Glu Ala Val Asp Glu Leu Asn Gln Lys Lys Glu
        630                 635                 640

ggt ttt cgc agc aca ggt cag cct tgg cag cca gac aag atg ctg cac 2202
Gly Phe Arg Ser Thr Gly Gln Pro Trp Gln Pro Asp Lys Met Leu His
    645                 650                 655

ctt gtc acc tta tat cac agg ctg aag cga caa aca gaa cag agg gtt 2250
Leu Val Thr Leu Tyr His Arg Leu Lys Arg Gln Thr Glu Gln Arg Val
660                 665                 670                 675

agc tta tta gaa gac acc acc agt gct tac caa gaa cac gag aag atg 2298
Ser Leu Leu Glu Asp Thr Thr Ser Ala Tyr Gln Glu His Glu Lys Met
                680                 685                 690

tgc caa cag ctg gag aga caa ctg aag tct gta aaa gag gag cag tcc 2346
Cys Gln Gln Leu Glu Arg Gln Leu Lys Ser Val Lys Glu Glu Gln Ser
            695                 700                 705

aaa gtg aat gag gaa acg ctg cct gca gag gag aag ctc aaa atg tat 2394
Lys Val Asn Glu Glu Thr Leu Pro Ala Glu Glu Lys Leu Lys Met Tyr
        710                 715                 720

cac tcc ctg gca gga agt ctc cag gac tca ggg att gta ctg aaa cga 2442
His Ser Leu Ala Gly Ser Leu Gln Asp Ser Gly Ile Val Leu Lys Arg
    725                 730                 735

gta acc ata cat ctt gaa gat ctt gcc cca cac ctt gac ccc ttg gct 2490
Val Thr Ile His Leu Glu Asp Leu Ala Pro His Leu Asp Pro Leu Ala
740                 745                 750                 755

tat gag aaa gcc agg cat cag atc cag tcc tgg caa ggg gag tta aaa 2538
Tyr Glu Lys Ala Arg His Gln Ile Gln Ser Trp Gln Gly Glu Leu Lys
                760                 765                 770

ctg ttg act tct gcc att ggt gag acg gtg aca gaa tgt gag agc cga 2586
Leu Leu Thr Ser Ala Ile Gly Glu Thr Val Thr Glu Cys Glu Ser Arg
            775                 780                 785

atg gtg cag agt ata gac ttc cag act gag atg agt cgc tcc ctg gac 2634
Met Val Gln Ser Ile Asp Phe Gln Thr Glu Met Ser Arg Ser Leu Asp
        790                 795                 800

tgg ctg agg aga gtg aag gca gag ctc agt ggg ccg gtg tac cta gac 2682
Trp Leu Arg Arg Val Lys Ala Glu Leu Ser Gly Pro Val Tyr Leu Asp
    805                 810                 815

ctc aac ctg cag gac atc caa gag gaa atc aga aaa atc caa att cat 2730
Leu Asn Leu Gln Asp Ile Gln Glu Glu Ile Arg Lys Ile Gln Ile His
820                 825                 830                 835

cag gaa gag gtc cag tcc agc ttg aga atc atg aat gcg ctg agt cac 2778
Gln Glu Glu Val Gln Ser Ser Leu Arg Ile Met Asn Ala Leu Ser His
                840                 845                 850

aag gaa aag gag aag ttc aca aag gcc aag gag ctg att tct gcg gat 2826

Lys Glu Lys Glu Lys Phe Thr Lys Ala Lys Glu Leu Ile Ser Ala Asp
            855                 860                 865

tta gaa cac agc ctc gct gag ctc tca gag ctg gat gga gac atc cag 2874
Leu Glu His Ser Leu Ala Glu Leu Ser Glu Leu Asp Gly Asp Ile Gln
        870                 875                 880

gaa gcc tta cgc acc aga cag gct acc ttg act gaa ata tat agc cag 2922
Glu Ala Leu Arg Thr Arg Gln Ala Thr Leu Thr Glu Ile Tyr Ser Gln
    885                 890                 895

tgt caa agg tat tat cag gta ttt caa gca gcc aat gac tgg ctt gag 2970
Cys Gln Arg Tyr Tyr Gln Val Phe Gln Ala Ala Asn Asp Trp Leu Glu
900                 905                 910                 915

gat gcc caa gaa atg tta cag ctg gca ggc aat ggc cta gac gtg gag 3018
Asp Ala Gln Glu Met Leu Gln Leu Ala Gly Asn Gly Leu Asp Val Glu
                920                 925                 930

agc gca gag gaa aat ctc aaa agc cac atg gaa ttt ttc agt aca gag 3066
Ser Ala Glu Glu Asn Leu Lys Ser His Met Glu Phe Phe Ser Thr Glu
            935                 940                 945

gat cag ttc cat agt aac ctg gag gag ctc cac agc ctg gta gcc acc 3114
Asp Gln Phe His Ser Asn Leu Glu Glu Leu His Ser Leu Val Ala Thr
        950                 955                 960

ctg gac cca ctc atc aag cca acc ggc aaa gaa gac cta gaa cag aaa 3162
Leu Asp Pro Leu Ile Lys Pro Thr Gly Lys Glu Asp Leu Glu Gln Lys
    965                 970                 975

gtg gct tct ctg gaa ctc agg agc cag agg atg agc cgg gac tct ggt 3210
Val Ala Ser Leu Glu Leu Arg Ser Gln Arg Met Ser Arg Asp Ser Gly
980                 985                 990                 995

gcc caa gtg gat ctc ttg cag aga tgc aca gct caa tgg cac gat tac 3258
Ala Gln Val Asp Leu Leu Gln Arg Cys Thr Ala Gln Trp His Asp Tyr
                1000                1005                1010

cag aaa gca agg gaa gag gtt att gaa ttg atg aat gat aca gaa aag 3306
Gln Lys Ala Arg Glu Glu Val Ile Glu Leu Met Asn Asp Thr Glu Lys
            1015                1020                1025

aaa ttg tct gag ttt tct ttg ttg aag act tcg tct agt cat gaa gcg 3354
Lys Leu Ser Glu Phe Ser Leu Leu Lys Thr Ser Ser Ser His Glu Ala
        1030                1035                1040

gaa gaa aaa ttg tca gaa cac aag gct tta gtg tca gtg gtt aac tct 3402
Glu Glu Lys Leu Ser Glu His Lys Ala Leu Val Ser Val Val Asn Ser
    1045                1050                1055

ttc cat gag aaa att gtg gcc ctt gag gaa aaa gct tca caa ctg gag 3450
Phe His Glu Lys Ile Val Ala Leu Glu Glu Lys Ala Ser Gln Leu Glu
1060                1065                1070                1075

aaa acc gga aat gat gcc agc aaa gcc acc ctg agc agg tca atg acc 3498
Lys Thr Gly Asn Asp Ala Ser Lys Ala Thr Leu Ser Arg Ser Met Thr
                1080                1085                1090

acc gtc tgg cag cgc tgg aca cgc ctt cga gct gtg gcc cag gac cag 3546

Thr Val Trp Gln Arg Trp Thr Arg Leu Arg Ala Val Ala Gln Asp Gln
            1095                1100                1105

gag aag atc ctg gaa gat gca gtg gat gag tgg acg ggc ttt aac aac 3594
Glu Lys Ile Leu Glu Asp Ala Val Asp Glu Trp Thr Gly Phe Asn Asn
        1110                1115                1120

aag gtt aaa aag gcc act gaa atg att gat cag ctg caa gat aag tta 3642
Lys Val Lys Lys Ala Thr Glu Met Ile Asp Gln Leu Gln Asp Lys Leu
    1125                1130                1135

cct gga agt tca gca gag aaa gca tcg aaa gca gag ctc tta act ctt 3690
Pro Gly Ser Ser Ala Glu Lys Ala Ser Lys Ala Glu Leu Leu Thr Leu
1140                1145                1150                1155

ctt gaa tac cac gac acg ttc gtt ctg gag ctg gag cag cag cag tcg 3738
Leu Glu Tyr His Asp Thr Phe Val Leu Glu Leu Glu Gln Gln Gln Ser
                1160                1165                1170

gcc ttg ggc atg ctg cgg cag caa acc ctg agc atg ctc cag gat gga 3786
Ala Leu Gly Met Leu Arg Gln Gln Thr Leu Ser Met Leu Gln Asp Gly
            1175                1180                1185

gcc gcc cca acc cct ggg gaa gag cct ccg ctc atg cag gaa atc acc 3834
Ala Ala Pro Thr Pro Gly Glu Glu Pro Pro Leu Met Gln Glu Ile Thr
        1190                1195                1200

gcc atg caa gat cgg tgc ctg aac atg cag gag aaa gtg aag act aat 3882
Ala Met Gln Asp Arg Cys Leu Asn Met Gln Glu Lys Val Lys Thr Asn
    1205                1210                1215

gga aag ttg gtg aag caa gag ctg aag gac cga gaa atg gtg gag act 3930
Gly Lys Leu Val Lys Gln Glu Leu Lys Asp Arg Glu Met Val Glu Thr
1220                1225                1230                1235

cag atc aat tct gtg aaa tgt tgg gtt cag gaa acg aaa gaa tat tta 3978
Gln Ile Asn Ser Val Lys Cys Trp Val Gln Glu Thr Lys Glu Tyr Leu
                1240                1245                1250

ggg aat cca aca ata gaa ata gat gct caa ctt gaa gaa ctt cag att 4026
Gly Asn Pro Thr Ile Glu Ile Asp Ala Gln Leu Glu Glu Leu Gln Ile
            1255                1260                1265

ctc cta aca gaa gcc aca aat cac cga cag aac att gaa aaa atg gca 4074
Leu Leu Thr Glu Ala Thr Asn His Arg Gln Asn Ile Glu Lys Met Ala
        1270                1275                1280

gaa gaa cag aag gag aag tac tta ggt ctt tat acc ata tta cct tct 4122
Glu Glu Gln Lys Glu Lys Tyr Leu Gly Leu Tyr Thr Ile Leu Pro Ser
    1285                1290                1295

gaa ctc tcc ctt cag ttg gct gaa gtg gcg tta gat cta aag atc cga 4170
Glu Leu Ser Leu Gln Leu Ala Glu Val Ala Leu Asp Leu Lys Ile Arg
1300                1305                1310                1315

gat cag atc caa gac aaa ata aaa gaa gtt gag cag agc aag gcc acg 4218
Asp Gln Ile Gln Asp Lys Ile Lys Glu Val Glu Gln Ser Lys Ala Thr
                1320                1325                1330

agc cag gaa ctc agc cgg caa att cag aag tta gct aaa gac ctc aca 4266

Ser Gln Glu Leu Ser Arg Gln Ile Gln Lys Leu Ala Lys Asp Leu Thr
        1335                1340                1345

act att cta act aag ctg aaa gcg aag aca gat aat gta gtt caa gct      4314
Thr Ile Leu Thr Lys Leu Lys Ala Lys Thr Asp Asn Val Val Gln Ala
    1350                1355                1360

aaa act gac caa aag gtg ctg gga gag gaa tta gat ggc tgt aat tca      4362
Lys Thr Asp Gln Lys Val Leu Gly Glu Glu Leu Asp Gly Cys Asn Ser
1365                1370                1375

aag tta atg gaa tta gat gca gca gta cag aaa ttc ttg gaa cag aat      4410
Lys Leu Met Glu Leu Asp Ala Ala Val Gln Lys Phe Leu Glu Gln Asn
1380                1385                1390                1395

ggc caa ctg ggt aag cca ctg gcc aag aag ata gga aaa ctg act gaa      4458
Gly Gln Leu Gly Lys Pro Leu Ala Lys Lys Ile Gly Lys Leu Thr Glu
                1400                1405                1410

ctt cac cag cag acc att aga caa gct gag aat cgg ctc tcc aag ctc      4506
Leu His Gln Gln Thr Ile Arg Gln Ala Glu Asn Arg Leu Ser Lys Leu
            1415                1420                1425

aat cag gca gca tca cat tta gaa gaa tac aat gaa atg ctt gaa tta      4554
Asn Gln Ala Ala Ser His Leu Glu Glu Tyr Asn Glu Met Leu Glu Leu
        1430                1435                1440

att ttg aag tgg att gaa aaa gct aaa gtc ttg gct cat gga act att      4602
Ile Leu Lys Trp Ile Glu Lys Ala Lys Val Leu Ala His Gly Thr Ile
    1445                1450                1455

gca tgg aat tct gca agc cag ctt cgg gaa caa tat att ttg cat cag      4650
Ala Trp Asn Ser Ala Ser Gln Leu Arg Glu Gln Tyr Ile Leu His Gln
1460                1465                1470                1475

gag gtc ggg cag ccc aga gca agc cat gag tcc aca tca cat gct ggc      4698
Glu Val Gly Gln Pro Arg Ala Ser His Glu Ser Thr Ser His Ala Gly
                1480                1485                1490

tat gtt agt tca ttt cct ctg aag tta cat gag aaa aat gtt cct ttt      4746
Tyr Val Ser Ser Phe Pro Leu Lys Leu His Glu Lys Asn Val Pro Phe
            1495                1500                1505

ctg tca gtc acg tca tcc agg aaa tta ttt cat cct ttt gta act taa      4794
Leu Ser Val Thr Ser Ser Arg Lys Leu Phe His Pro Phe Val Thr
        1510                1515                1520

<210> 129
<211> 2115
<212> DNA
<213> Homo sapiens

<220>
<221> CDS
<222> (725)..(1696)

<400> 129
cggccgctgc agccagcccc gcggctccct cagacccgcg ggcgcagccg ccgggggtga      60

ggcgcttggg gaccgcgggc cgagcggcgg ggatccccga gcaccatgct ggacccgtct     120

tccagcgaag aggagtcgga cgaggggctg gaagaggaaa gccgcgatgt gctggtggca     180

gccggcagct cgcagcgagc tcctccagcc ccgactcggg aagggcggcg ggacgcgccg     240

gggcgcgcgg gcggcggcgg cgcggccaga tctgtgagcc cgagcccctc tgtgctcagc     300

gaggggcgag acgagcccca gcggcagctg gacgatgagc aggagcggag gatccgcctg     360

cagctctacg tcttcgtcgt gaggtgcatc gcgtacccct tcaacgccaa gcagcccacc     420

gacatggccc ggaggcagca gaagcttaac aaacaacagt tgcagttact gaaagaacgg     480

ttccaggcct tcctcaatgg ggaaacccaa attgtagctg acgaagcatt ttgcaacgca     540

gttcggagtt attatgaggt ttttctaaag agtgaccgag tggccagaat ggtacagagt     600

ggagggtgtt ctgctaatga cttcagagaa gtatttaaga aaaacataga aaaacgtgtg     660

cggagtttgc cagaaataga tggcttgagc aaagagacag tgttgagctc atggatagcc     720

aaat atg atg cca ttt aca gag gtg aag agg act tgt gca aac agc caa     769
     Met Met Pro Phe Thr Glu Val Lys Arg Thr Cys Ala Asn Ser Gln
     1               5                   10                  15

act aga atg gcc cta agt gca gtg tct gaa ctt att acg agc aag gaa     817
Thr Arg Met Ala Leu Ser Ala Val Ser Glu Leu Ile Thr Ser Lys Glu
                20                  25                  30

caa ctc tat gaa atg ttt cag cag att ctg ggt att aaa aaa cta gaa     865
Gln Leu Tyr Glu Met Phe Gln Gln Ile Leu Gly Ile Lys Lys Leu Glu
            35                  40                  45

cac cag ctc ctt tat aat gca tgt cag ctg gat aac gca gat gaa caa     913
His Gln Leu Leu Tyr Asn Ala Cys Gln Leu Asp Asn Ala Asp Glu Gln
        50                  55                  60

gca gcc cag atc aga agg gaa ctt gat ggc cgg ctg caa ttg gca gat     961
Ala Ala Gln Ile Arg Arg Glu Leu Asp Gly Arg Leu Gln Leu Ala Asp
    65                  70                  75

aaa atg gca aag aaa tat gtg aat gtt ata ttg gag gag aag aaa agc    1009
Lys Met Ala Lys Lys Tyr Val Asn Val Ile Leu Glu Glu Lys Lys Ser
80                  85                  90                  95

agg tgg cct gag act ctg gga ctg atc tgt gaa gtg gtg cca gat gtt    1057
Arg Trp Pro Glu Thr Leu Gly Leu Ile Cys Glu Val Val Pro Asp Val
                100                 105                 110

cct tcc aat ccc atg aat gaa ctg gtg caa gaa aga aaa ttc ccc aaa    1105
Pro Ser Asn Pro Met Asn Glu Leu Val Gln Glu Arg Lys Phe Pro Lys
            115                 120                 125

ttt ata gca aaa gat atg gag aat atg tat ata gaa gag ttg cgg tct    1153
Phe Ile Ala Lys Asp Met Glu Asn Met Tyr Ile Glu Glu Leu Arg Ser
        130                 135                 140

tca gtg aat ttg cta atg gcc aat ttg gaa agt ctt cca gtt tcg aaa 1201
Ser Val Asn Leu Leu Met Ala Asn Leu Glu Ser Leu Pro Val Ser Lys
    145                 150                 155

ggt ggt ccg gaa ttt aaa tta caa aaa tta aaa cgt tca cag aac tct 1249
Gly Gly Pro Glu Phe Lys Leu Gln Lys Leu Lys Arg Ser Gln Asn Ser
160                 165                 170                 175

gca ttt ttg gac ata gga gat gag aat gag att cag ctg tca aag tcc 1297
Ala Phe Leu Asp Ile Gly Asp Glu Asn Glu Ile Gln Leu Ser Lys Ser
                180                 185                 190

gac gtg gta ctg tca ttc acc tta gag att gtc ata atg gaa gtg caa 1345
Asp Val Val Leu Ser Phe Thr Leu Glu Ile Val Ile Met Glu Val Gln
            195                 200                 205

ggc ctg aag tca gtt gct ccc aat cga att gtt tac tgt aca atg gaa 1393
Gly Leu Lys Ser Val Ala Pro Asn Arg Ile Val Tyr Cys Thr Met Glu
        210                 215                 220

gtg gaa gga gaa aaa ctt cag aca gac cag gcc gaa gcc tca agg cca 1441
Val Glu Gly Glu Lys Leu Gln Thr Asp Gln Ala Glu Ala Ser Arg Pro
    225                 230                 235

caa tgg ggg act caa gga gat ttc acc acc acc cat cct cgg cct gtg 1489
Gln Trp Gly Thr Gln Gly Asp Phe Thr Thr Thr His Pro Arg Pro Val
240                 245                 250                 255

gtc aaa gtg aaa ctc ttc aca gaa agc act gga gtt ctg gcc ctg gaa 1537
Val Lys Val Lys Leu Phe Thr Glu Ser Thr Gly Val Leu Ala Leu Glu
                260                 265                 270

gat aaa gaa ctg gga agg gtg ata tta tac cca act tct aat agc tcc 1585
Asp Lys Glu Leu Gly Arg Val Ile Leu Tyr Pro Thr Ser Asn Ser Ser
            275                 280                 285

aaa tca gct gaa tta cac cga atg gta gtt cca aaa aat agc cag gat 1633
Lys Ser Ala Glu Leu His Arg Met Val Val Pro Lys Asn Ser Gln Asp
        290                 295                 300

tct gac tta aaa atc aaa ctg gca gtg cga atg gat aaa cca gca cat 1681
Ser Asp Leu Lys Ile Lys Leu Ala Val Arg Met Asp Lys Pro Ala His
    305                 310                 315

atg aag cat agt ggg tgagtataaa tttgaggatt tttttcctga attattgaat 1736
Met Lys His Ser Gly
320

ttcatttcag tgtattattt ttgtcagttt agtgagagaa ccagtatagc aggctttcac 1796

atggagtttt ctcaaaaagt gacatacgta agttatctta ctcaatgttt ttatacccat 1856

agacggcttt atagctcaat acatgagtcc ttagttaaga taactgtatg cgagctacca 1916

cgatattgga acttgattgt gttagtatta ctcatcagtc atattgatac agggcaccag 1976

ggcactgata ccttctaaga aacattcttc aaattacaat aatagttgaa acaagcttag 2036

cattgaggtt cctagaaata aacttccata gactgtataa aaactaaaac atagtgtaga 2096

tgtaacccaa cgaggctat 2115

<210> 130
<211> 824
<212> DNA
<213> Homo sapiens

<220>
<221> CDS
<222> (264)..(734)

<400> 130
cgaccgccaa ttcggcacga gagagcccgg ccgctcgccc tgtctagtct ctccccggcc 60

ccttgattct ccgcgtcttc ctcgcaggcc agcctaagct cagcctgcat tgcgccctcc 120

cggcctaggg tgtcgctgta ccggcctccg agctccacat agacaagtcc ggacctcggg 180

cgggggcagg agacggagct gagcggcggg gggcagtgtc gcctcggtct agggacggcg 240

gcggagtcgg tggtccctgc gcg atg gcc acc tcg gtg ttg tgc tgc ctg 290
Met Ala Thr Ser Val Leu Cys Cys Leu
1               5

cgg tgc tgc aga gac ggg ggg act ggc cac atc cct ctg aag gag atg 338
Arg Cys Cys Arg Asp Gly Gly Thr Gly His Ile Pro Leu Lys Glu Met
10                  15                  20                  25

ccg gcc gtg cag ctg gac acg cag cac atg gga atc tgg ggt att ggt 386
Pro Ala Val Gln Leu Asp Thr Gln His Met Gly Ile Trp Gly Ile Gly
                30                  35                  40

gtt gca act cag aag gtt aac ttg aat cag att cct ctt ggc cga gat 434
Val Ala Thr Gln Lys Val Asn Leu Asn Gln Ile Pro Leu Gly Arg Asp
            45                  50                  55

atg cac agt ctg gtg atg aga aat gat gga gcc ctt tac cac aac aat 482
Met His Ser Leu Val Met Arg Asn Asp Gly Ala Leu Tyr His Asn Asn
        60                  65                  70

gaa gag aaa aat agg ctg cca gca aac agt ctt ccg cag gaa gga gat 530
Glu Glu Lys Asn Arg Leu Pro Ala Asn Ser Leu Pro Gln Glu Gly Asp
    75                  80                  85

gtg gtg ggt att act tat gac cat gtc gaa tta aat gta tac ttg aat 578
Val Val Gly Ile Thr Tyr Asp His Val Glu Leu Asn Val Tyr Leu Asn
90                  95                  100                 105

gga aaa aac atg cat tgt cca gca tca ggt ata cga ggg aca gtg tat 626
Gly Lys Asn Met His Cys Pro Ala Ser Gly Ile Arg Gly Thr Val Tyr
                110                 115                 120

cca gtt gtt tat gtt gat gac agt gca att ttg gat tgc cag ttc agt 674
Pro Val Val Tyr Val Asp Asp Ser Ala Ile Leu Asp Cys Gln Phe Ser
            125                 130                 135

gag ttt tat cat acg cct cca cct ggt ttt gaa aaa ata tta ttt gaa     722
Glu Phe Tyr His Thr Pro Pro Pro Gly Phe Glu Lys Ile Leu Phe Glu
        140                 145                 150

cag caa atc ttc tga atgtatttgt ttttaaaact tgtatttctg cactgttaaa     777
Gln Gln Ile Phe
    155

aaatgttcat catttaataa aactttacct ggcctaaaaa aaaaaaa                 824

<210> 131
<211> 1646
<212> DNA
<213> Homo sapiens

<220>
<221> CDS
<222> (418)..(1446)

<400> 131
tccttaatta aattaatccc cccccccccc cccccggttt tccgggcgga agtgaggcgc      60

ccagcggaac ggcctctggt gacattttct tgggggcggc gcggacgact gaagggactt     120

ggtggtagag gctgcggagg cggcgaggtt cactttggtc ccggcagggg ctctgaccgc     180

gacccactgc tcgctgccgg ggcttgttcc gaggaggaga aggcggcggt ggccgtcgcg     240

tcctgagtca ccgcagcggg gaagagtccc ggccgggggt tcggtgcgtg ccccgacggg     300

gcgcgcgcga agcgagcgag aaggcacgga gtcgccgcgg agcgcaggtt gctttcctgt     360

tgcatctttg atatcatttc ctagactcta ctccatgatt taacttgaaa ttctgaa       417
atg aag atg gag gag gca gtg gga aaa gtt gaa gaa ctc att gag tcc     465
Met Lys Met Glu Glu Ala Val Gly Lys Val Glu Glu Leu Ile Glu Ser
1               5                   10                  15

gaa gcc cca cca aaa gca tct gaa caa gag aca gcc aag gag gaa gat     513
Glu Ala Pro Pro Lys Ala Ser Glu Gln Glu Thr Ala Lys Glu Glu Asp
            20                  25                  30

gga tct gta gaa ctg gaa tct caa gtt cag aaa gat ggt gta gcg gat     561
Gly Ser Val Glu Leu Glu Ser Gln Val Gln Lys Asp Gly Val Ala Asp
        35                  40                  45

tct aca gtt att tct tca atg ccc tgc ttg ttg atg gaa ctg aga agg     609
Ser Thr Val Ile Ser Ser Met Pro Cys Leu Leu Met Glu Leu Arg Arg
    50                  55                  60

gac tct tct gag tct cag tta gca tcc aca gag agt gac aag cct aca     657
Asp Ser Ser Glu Ser Gln Leu Ala Ser Thr Glu Ser Asp Lys Pro Thr
65                  70                  75                  80

act ggc cga gtt tat gag agt gac tcc tct aat cac tgc atg ctt tcc     705
Thr Gly Arg Val Tyr Glu Ser Asp Ser Ser Asn His Cys Met Leu Ser
                85                  90                  95

cct tcc tct agt ggt cac ctg gct gat tca gat acg ttg tct tcc gca 753
Pro Ser Ser Ser Gly His Leu Ala Asp Ser Asp Thr Leu Ser Ser Ala
            100                 105                 110

gaa gag aat gaa ccc tct cag gca gaa acg gcg gta gaa gga gac cct 801
Glu Glu Asn Glu Pro Ser Gln Ala Glu Thr Ala Val Glu Gly Asp Pro
        115                 120                 125

tca gga gtg tct ggt gcc aca gtt ggg cgc aag tct agg cgg tcc cga 849
Ser Gly Val Ser Gly Ala Thr Val Gly Arg Lys Ser Arg Arg Ser Arg
    130                 135                 140

tct gaa agt gaa act tcc act atg gct gcc aag aaa aac cgg caa tcc 897
Ser Glu Ser Glu Thr Ser Thr Met Ala Ala Lys Lys Asn Arg Gln Ser
145                 150                 155                 160

agt gat aaa cag aat ggc cga gtc gcc aag gtt aaa ggt cat cgg agc 945
Ser Asp Lys Gln Asn Gly Arg Val Ala Lys Val Lys Gly His Arg Ser
                165                 170                 175

caa aag cac aag gag agg atc agg cta ctg agg cag aaa cgg gag gct 993
Gln Lys His Lys Glu Arg Ile Arg Leu Leu Arg Gln Lys Arg Glu Ala
            180                 185                 190

gct gca agg aag aaa tat aac ctg ctg cag gac agt agt acc agt gat 1041
Ala Ala Arg Lys Lys Tyr Asn Leu Leu Gln Asp Ser Ser Thr Ser Asp
        195                 200                 205

agt gac ctg act tgt gac tca agc acg agc tca tca gat gat gat gaa 1089
Ser Asp Leu Thr Cys Asp Ser Ser Thr Ser Ser Ser Asp Asp Asp Glu
    210                 215                 220

gag gtt tca ggg agc agc aag aca atc act gca gag ata cca ggt cat 1137
Glu Val Ser Gly Ser Ser Lys Thr Ile Thr Ala Glu Ile Pro Gly His
225                 230                 235                 240

ttg gat cca gga ttc cta gca agt gac aaa aca tct gct ggc aat gcg 1185
Leu Asp Pro Gly Phe Leu Ala Ser Asp Lys Thr Ser Ala Gly Asn Ala
                245                 250                 255

cca ctc aat gaa gaa att aac att gcg tct tca gat agt gaa gta gag 1233
Pro Leu Asn Glu Glu Ile Asn Ile Ala Ser Ser Asp Ser Glu Val Glu
            260                 265                 270

att gtg gga gtt cag gaa cat gca agg tgt gtt cat cct cga ggt ggt 1281
Ile Val Gly Val Gln Glu His Ala Arg Cys Val His Pro Arg Gly Gly
        275                 280                 285

gtg att cag agt gtt tct tca tgg aag cat ggc tcg ggc acg cag tat 1329
Val Ile Gln Ser Val Ser Ser Trp Lys His Gly Ser Gly Thr Gln Tyr
    290                 295                 300

gtt agc acc agg caa aca cag tca tgg act gct gtg act ccc cag cag 1377
Val Ser Thr Arg Gln Thr Gln Ser Trp Thr Ala Val Thr Pro Gln Gln
305                 310                 315                 320

act tgg gct tca cca gca gaa gtt gtt gac ctt acc ttg gat gag gat 1425
Thr Trp Ala Ser Pro Ala Glu Val Val Asp Leu Thr Leu Asp Glu Asp
                325                 330                 335

agc agg cgt aaa tac cta ctg ta atacaatgtc actgtgtttc ctctgcactg     1478
Ser Arg Arg Lys Tyr Leu Leu
            340

ttcccttcca cttcctcatc ctctttgtga catggaagtt cattgtcata gcttcagctt   1538

cagaagctgt ttgtggcatt tgtaggattc aaactcatgg aaaattccct cctcttcccc   1598

ccttttttta attaaaagaa gtcacttagg aaaataaaaa aaaaaaaa                1646

<210> 132
<211> 680
<212> DNA
<213> Homo sapiens

<220>
<221> CDS
<222> (185)..(610)

<400> 132
atgggcggaa gcacccctcc taccaggacc cggaaacaga tggccacccc gagccggcct     60

atccggaaac tggctcgaag aagacgggga ttggccatcg cttccggaag ttgtgagctt    120

tgtttcggag cgggagctct tccgagacgc actgggggcc ggatgaatcc tgcttatctg    180

tgaa atg cag tta aca cat cag ctg gac cta ttt ccc gaa tgc agg att     229
     Met Gln Leu Thr His Gln Leu Asp Leu Phe Pro Glu Cys Arg Ile
     1               5                   10                  15

gtt gat cca ttt cag ata ctt gtg gca gca aac aaa gca gtt cac ctc     277
Val Asp Pro Phe Gln Ile Leu Val Ala Ala Asn Lys Ala Val His Leu
                20                  25                  30

tac aaa ctg gga aaa atg aag aca aga act cta tct act gaa att att     325
Tyr Lys Leu Gly Lys Met Lys Thr Arg Thr Leu Ser Thr Glu Ile Ile
            35                  40                  45

ttc aac ctt tcc cca aat aac aat att tca gag gct ttg aaa aaa ttt     373
Phe Asn Leu Ser Pro Asn Asn Asn Ile Ser Glu Ala Leu Lys Lys Phe
        50                  55                  60

ggt atc tca gca aat gac act tca att cta att gtt tac att gaa gag     421
Gly Ile Ser Ala Asn Asp Thr Ser Ile Leu Ile Val Tyr Ile Glu Glu
    65                  70                  75

gga gaa aaa caa ata aat caa gaa tac cta ata tct caa gta gaa ggt     469
Gly Glu Lys Gln Ile Asn Gln Glu Tyr Leu Ile Ser Gln Val Glu Gly
80                  85                  90                  95

cat cag gtt tct ctg aaa aat ctt cct gaa ata atg aat att aca gaa     517
His Gln Val Ser Leu Lys Asn Leu Pro Glu Ile Met Asn Ile Thr Glu
                100                 105                 110

gtc aaa aag ata tat aaa ctc tct tca caa gaa gaa agt att ggg aca     565
Val Lys Lys Ile Tyr Lys Leu Ser Ser Gln Glu Glu Ser Ile Gly Thr
            115                 120                 125

tta ttg gat gct atc att tgt aga atg tca aca aaa gat gtt tta tga 613
Leu Leu Asp Ala Ile Ile Cys Arg Met Ser Thr Lys Asp Val Leu
        130                 135                 140

aatgtcagaa atattaacaa aaattctcag cattaaagaa aacattgatt ttcaaaaaaa 673

aaaaaaa 680

<210> 133
<211> 1188
<212> DNA
<213> Homo sapiens

<220>
<221> CDS
<222> (174)..(731)

<400> 133
cggcttttta aatgacccag gcgtgggtaa agcatcggtc caggcatcgg tggcggggac 60

aggcctaggc agcccgggcc acccagactg gcccctaatg agctcccaag tggcctgtgg 120

ggacttggcc tcagttttgc cagctgtgaa ctgggaccgt cgctgctaag gcc atg 176
Met
1

gac acc ttc agc acc aag agc ctg gct ctg cag gcg cag aag aag ctc 224
Asp Thr Phe Ser Thr Lys Ser Leu Ala Leu Gln Ala Gln Lys Lys Leu
            5                   10                  15

ctg agt aag atg gcg tcc aag gca gtg gtg gcc gtg ctg gtg gat gac 272
Leu Ser Lys Met Ala Ser Lys Ala Val Val Ala Val Leu Val Asp Asp
        20                  25                  30

acc agc agt gag gtg ctg gat gag ctg tac cgc gcc acc agg gag ttc 320
Thr Ser Ser Glu Val Leu Asp Glu Leu Tyr Arg Ala Thr Arg Glu Phe
    35                  40                  45

acg cgc agc cgc aag gag gcc cag aag atg ctc aag aac ctg gtc aag 368
Thr Arg Ser Arg Lys Glu Ala Gln Lys Met Leu Lys Asn Leu Val Lys
50                  55                  60                  65

gtg gcc ctg aag ctg gga ctg ctg ctg cgt ggg gac cag ctg ggc ggt 416
Val Ala Leu Lys Leu Gly Leu Leu Leu Arg Gly Asp Gln Leu Gly Gly
                70                  75                  80

gag gag ctg gcg ctg ctg cgg cgc ttc cgc cac cgg gcg cgc tgc ctg 464
Glu Glu Leu Ala Leu Leu Arg Arg Phe Arg His Arg Ala Arg Cys Leu
            85                  90                  95

gcc atg acg gcc gtc agc ttc cac cag gtg gac ttc acc ttc gac cgg 512
Ala Met Thr Ala Val Ser Phe His Gln Val Asp Phe Thr Phe Asp Arg
        100                 105                 110

cgc gtg ctg gcc gtc ggg ctg ctc gag tgc cgc gac ctg ctg cac cag 560
Arg Val Leu Ala Val Gly Leu Leu Glu Cys Arg Asp Leu Leu His Gln

115                 120                 125

gcc gtg ggt ccc cac ctg acc gcc aag tcc cac ggc cgc atc aac cac      608
Ala Val Gly Pro His Leu Thr Ala Lys Ser His Gly Arg Ile Asn His
130                 135                 140                 145

gtg ttc ggc cac cta gcc gac tgc gac ttc ctg gct gcg ctc tac ggc      656
Val Phe Gly His Leu Ala Asp Cys Asp Phe Leu Ala Ala Leu Tyr Gly
                150                 155                 160

ccc gcc gag ccc tac cgc tcc cac ctg cgc agg atc tgc gag ggc ctg      704
Pro Ala Glu Pro Tyr Arg Ser His Leu Arg Arg Ile Cys Glu Gly Leu
            165                 170                 175

ggc cgg atg ctg gac gag ggc agc ctc tgaac cccggcgccg cccaaccgcg      756
Gly Arg Met Leu Asp Glu Gly Ser Leu
        180                 185

cccctcgcgc cttttggggc tctcctgctg ggcgcgggtg gggtttgtgg gtttttttcc      816

acctcttttc tcccaatcgg actccggcca aactccccta gacagatggg tgacctgtct      876

cctttgagag gatgctgagg catctgtagc agctgtttca aacaccaatg tcacctctcc      936

tcctggcccc cgcccaatgg ggagaggaat ttggggccct actctgggga ccacctttca      996

cccgtttgta ctttctgggc cacgccgacc cctgggtcgc ttgatgtaaa agccaaaagc     1056

tgctgcctcc cacttggatc atgtcgcctg ggattttcat ccctcgcaca aggactacgg     1116

gttcacacgg tgaactgggg gaagggaagt gttagggggc aagtcgcggc accccccctt     1176

ccataaactc ac                                                         1188

<210> 134
<211> 1887
<212> DNA
<213> Homo sapiens

<220>
<221> CDS
<222> (369)..(1568)

<400> 134
cgttgtggct gctgtttcct tcggctttcc tcctcctgct ccaccatgtg gagccgacgg       60

cagggccgcc tcaggcccac ggtctgcggg gtggaggagc tacggcgccg ccggcgggag      120

cgggagcact gcggaaggcg cggagggagc agcagctggt cagcaagagg ctgctgagaa      180

acgacgcccc agaggaagct ggagagggat gtgtggctgc gatcctcggg gaaaccgagg      240

tgcagcagtt cctgcggcaa gcccagcggg ggacagagga aaaggagaga gagggggctc      300

tggtcagcct tcgtcgaggc ttgcagcacc ctgaaacaca gcaaaccttc atccggctgg      360

agggcagc atg cgg acc ctg gtc ggg ctc ctg acc agc aac cag gcc ctg      410

Met Arg Thr Leu Val Gly Leu Leu Thr Ser Asn Gln Ala Leu
1               5                   10

ctg cag ctt gag gcg gct cgg tgc ctg cat gag ctc tct cac tcc gag     458
Leu Gln Leu Glu Ala Ala Arg Cys Leu His Glu Leu Ser His Ser Glu
15                  20                  25                  30

cag tcc act gtt gct gag gcc tgc ctg cca gcc act tct tac ctc ctc     506
Gln Ser Thr Val Ala Glu Ala Cys Leu Pro Ala Thr Ser Tyr Leu Leu
                35                  40                  45

acc tac ctc tcc agt cac agc tca gac ttc ata cct ggc atg aaa cag     554
Thr Tyr Leu Ser Ser His Ser Ser Asp Phe Ile Pro Gly Met Lys Gln
            50                  55                  60

gcc aag ccc agt gct ttt gtt gcc tgt tct cag gag ctg tgt ctg tat     602
Ala Lys Pro Ser Ala Phe Val Ala Cys Ser Gln Glu Leu Cys Leu Tyr
        65                  70                  75

aca ctg ggt aac ctg atc gtg gag agt gag gct gtg aga agg cag ctc     650
Thr Leu Gly Asn Leu Ile Val Glu Ser Glu Ala Val Arg Arg Gln Leu
    80                  85                  90

ctg cca cag ggc att gtt cca gcc ttg gct gcc tgc atc cag tcc ccc     698
Leu Pro Gln Gly Ile Val Pro Ala Leu Ala Ala Cys Ile Gln Ser Pro
95                  100                 105                 110

cat gtg gct gtg ctg gaa gct ctc gga tat gcc ttg tcc cag ctt cta     746
His Val Ala Val Leu Glu Ala Leu Gly Tyr Ala Leu Ser Gln Leu Leu
                115                 120                 125

cag gct gag gaa gct cca gag aag atc att ccc tcc atc ttg gcc tcc     794
Gln Ala Glu Glu Ala Pro Glu Lys Ile Ile Pro Ser Ile Leu Ala Ser
            130                 135                 140

act ctc cct cag cac atg cta caa atg ttg caa cct ggc cca aag ctc     842
Thr Leu Pro Gln His Met Leu Gln Met Leu Gln Pro Gly Pro Lys Leu
        145                 150                 155

aac cct ggg gtc gct gtg gag ttt gcc tgg tgc ctt cat tac atc atc     890
Asn Pro Gly Val Ala Val Glu Phe Ala Trp Cys Leu His Tyr Ile Ile
    160                 165                 170

tgc agc cag gtc agc aat cct ctg ctc att ggc cat ggg gct ctg tct     938
Cys Ser Gln Val Ser Asn Pro Leu Leu Ile Gly His Gly Ala Leu Ser
175                 180                 185                 190

act ctg ggg ttg ctg ctg ttg gac ttg gct ggg gct gtc cag aaa agc     986
Thr Leu Gly Leu Leu Leu Leu Asp Leu Ala Gly Ala Val Gln Lys Ser
                195                 200                 205

gag gat gca gga ctg gag ctg ctg gca tgc ccc gtg ctt cga tgt cta     1034
Glu Asp Ala Gly Leu Glu Leu Leu Ala Cys Pro Val Leu Arg Cys Leu
            210                 215                 220

agc aac ctg cta act gag gca gca gtg gag act gtg gga ggg caa atg     1082
Ser Asn Leu Leu Thr Glu Ala Ala Val Glu Thr Val Gly Gly Gln Met
        225                 230                 235

cag ctc aga gat gag cgt gtt gtg gca gcc tta ttt atc ctt ctg cag     1130

Gln Leu Arg Asp Glu Arg Val Val Ala Ala Leu Phe Ile Leu Leu Gln
    240                 245                 250

ttc ttt ttc cag aaa cag ccc agt ctg ctc cct gag ggc ctt tgg ctc      1178
Phe Phe Phe Gln Lys Gln Pro Ser Leu Leu Pro Glu Gly Leu Trp Leu
255                 260                 265                 270

ctc aac aac ctc act gca aac agt cct agt ttc tgt acc tcc ttg ctc      1226
Leu Asn Asn Leu Thr Ala Asn Ser Pro Ser Phe Cys Thr Ser Leu Leu
                275                 280                 285

tcc ctg gat ctg att gag cct ctc tta cag ctg ttg cca gta tct aac      1274
Ser Leu Asp Leu Ile Glu Pro Leu Leu Gln Leu Leu Pro Val Ser Asn
            290                 295                 300

gtg gtg agc gta atg gtg ctc aca gtt ctg tgc aat gtt gca gaa aag      1322
Val Val Ser Val Met Val Leu Thr Val Leu Cys Asn Val Ala Glu Lys
        305                 310                 315

ggt cct gct tac tgc cag cgg ctg tgg cca ggg ccc ctg ctt ccc gcc      1370
Gly Pro Ala Tyr Cys Gln Arg Leu Trp Pro Gly Pro Leu Leu Pro Ala
    320                 325                 330

ttg ctg cac aca cta gcc ttt tct gac act gaa gta gta ggc cag agt      1418
Leu Leu His Thr Leu Ala Phe Ser Asp Thr Glu Val Val Gly Gln Ser
335                 340                 345                 350

ttg gag ctg ctg cat ctg ctg ttc ctg tat cag cca gag gct gtt cag      1466
Leu Glu Leu Leu His Leu Leu Phe Leu Tyr Gln Pro Glu Ala Val Gln
                355                 360                 365

gtc ttc ctg cag cag tca ggg ctg caa gcc ctg gaa agg cat cag gaa      1514
Val Phe Leu Gln Gln Ser Gly Leu Gln Ala Leu Glu Arg His Gln Glu
            370                 375                 380

gag gcc cag ctc cag gat cgt gtg tat gct ctc cag cag aca gct ctt      1562
Glu Ala Gln Leu Gln Asp Arg Val Tyr Ala Leu Gln Gln Thr Ala Leu
        385                 390                 395

caa ggg tgatcttgtt tctcaatgtc actcattccc ctctctctta acatcaagct      1618
Gln Gly
    400

tgtttgtcca gtagagcctt tggagattta ggaccataat gaggtctcat gttctctgct      1678

cccacaccta agccaagacc tttgggtccc agctcctccc cttccactca gcactatcca      1738

ggcaggagga ccaaaaggga ctcagtgtgg tctacttact ctggggccct agaatccctg      1798

cccccccgcc acccttcatg tttgcttcag cagctggtag cttttgatga gacagaataa      1858

agttttattt ttatattaaa aaaaaaaaa                                        1887

<210> 135
<211> 1061
<212> DNA
<213> Homo sapiens

<220>
<221> CDS
<222> (386)..(616)

<400> 135
gccaaccatg gtgaacttgg gtctgtcccg ggtgggggac gcggtggctg ccaagcaccc 60
ggtgagaagg ccggtctggg acgcgggcct caaaccccag aagctgacct gagcgaggcg 120
gggggccggg gcgtgggggt gccctcgtca gggaagagtg gcggaggctg ggggcactct 180
cttgctaagg ggctcagggc gtccgggtgg gcatagggga ggtgggacgt gtccaagcgc 240
ccagggtctc actcctccac agccagggtg tgcccagagg agggacagga gaggcggtga 300
aggaagaggg caggggatcg agccttcacc cgcctctgcc accccgggac tcggggagta 360
tgccgcatgc cagtcacacg ccttc atg aag ggc gtt ttc acc ttc gtc aca 412
ggc acc ggc atg gcc ttt ggc ttg cag atg ttc att cag agg aag ttt 460
cca tac cct ttg cag tgg agc ctc cta gtg gcc gtg gtt gca ggc tct 508
gtg gtc agc tac ggg gtg acg aga gtg gag tcg gag aaa tgc aac aac 556
ctc tgg ctc ttc ctg gag acc ggg cag ctc ccc aaa gac agg agc aca 604
gat cag aga agc tag gagagctcca gcaggggcac agaggattgg gggcaggagg 659
agtctggaac acagccttca tgccccctga ccccaggccg accctcccca caccctaggg 719
taccccagtc gtatcctctg tccgcatgtg tggccaggcc tgacaaacac ctgcagatgg 779
ctgctgcccc aacctgggac ctgcccagga ggttggagca gaaagggctc tccctggggt 839
ggtgtttctc ctctagggta ttgggatgca tgttctgcac tgccagcaga gagggtgtgt 899
ctgggggcca ccacctatgg gacacggggt cgaaggggcc tgtacactct gtcatttcct 959
ttctagcccc tgcatctcca acaagtccaa ggtgacagct ggtgctaggg gcgtggggtt 1019
aataaatggc ttatccttct ctccaaaaaa aaaaaaaaaa aa 1061

<210> 136

<211> 2260
<212> DNA
<213> Homo sapiens

<220>
<221> CDS
<222> (1612)..(2142)

<400> 136
gcaatttcat aatatggtaa tttttaatat taaaagtaaa ttgtaaatgt gtctttataa 60

acaatatgca taaaatagat catattctaa acccttttct ttttaaaacg ctttttcaca 120

gtaaatttct tatggtcaga ttttccctca gaatagtgca actggaagaa gggaaatgtt 180

ggcagtggct caggactctc tgctagtaca agtccttgta gatctcctgc aggagcgggt 240

gaagactcat gtctgtctcc gtcttcttga tcacctgcag tagctgcacg tgttccgtga 300

caatctgtct gaggtctgtc attttctgga gcagcttggc aaacagctgt gaggactcag 360

ggtggttcag cttcagctgg agctccaggg cttgtagcag gttgtcttga atgtcttcaa 420

tgggcttcac attcagcaaa cctgggcggt ctccactgag aataatgaca gcaataaata 480

ttgccaagtc gctgtcatct aattccagtg cattgaactt cacagcaaac tcaaacttgg 540

gctccataaa gtcaccaaaa ggctttcgca ggctctttag aaactccctt gtcatgaagc 600

cttggccctc ggatatgaga accccatctt tattcatcaa ggaggccagc attgtgtaaa 660

tgatctcgtg gactccatat ttgaggagag ttacttggtc gttcaagtca agatttacaa 720

aaccaggaat gcttttggca tactctgtga tctcctgcac agcctccacg gagcgaaact 780

ggcagccctg aaagatgcgg atggccacct ctttgctctg ctcctgcagg ggggtgatgt 840

gtttgaactt gattttatct tctcccatca ttaaggaatt catgtcatag ataacgaatg 900

gtgatttgtc tgttgtcttt cctgtcaaga tcgccctcgc ctttgctttg gtcagcggga 960

aggactttat gtatgagtca tacaaatgtt ttgccagggc ctgacggagg tcagcggact 1020

ctggattcag ctggtcgata tcactggaga tctccgccaa cagcttctcc ttctcggcct 1080

gtgcgatccg cccaaacctg atggcattat gagacatccc cactgcaagg catttctgaa 1140

accgacagta ctgacattta tttctacttt ttttgtggat ccgacagtta agatcacatc 1200

tgtcatagat aagcttcaat ctgattgttc tccggaagaa acccttgcat ccttcacaag 1260

catgaactcc atagtgaaat ccagaagctt tatctccaca gacacgacat tcaattgcca 1320

tgagggagtt ggaaggctct tcatgaggct tattgtagag ctgagtcttc tcagaataat 1380

aaggtggaga tgcaggctcc actttgattg cactttggta ctcttgaagt ttcaggtcat 1440

acttgtaatc tgcaaccact ggatctgttc ttgtgaatgg aatgtcttcg taatgtggag 1500

tagaaatgct ggagaagtca acagtagtga agggcttgat atcaaaggag tgggagtggt 1560

cttccattac ggagagatcc acggagctga tcccaaagtt ggtgggccag a atg cga 1617
Met Arg
1

tct ctg tgt caa cca tgg tca ttt ctg cgg cca cgg cgg cgt ggc gcc 1665
Ser Leu Cys Gln Pro Trp Ser Phe Leu Arg Pro Arg Arg Arg Gly Ala
        5                   10                  15

cct ctc cgg tgt cct cga ggc cga ccc aag ccc ccc agg cgg cgg ctg 1713
Pro Leu Arg Cys Pro Arg Gly Arg Pro Lys Pro Pro Arg Arg Arg Leu
    20                  25                  30

cgg ctc ggg ctc ggg aat tcc cag act cag aga gaa ccc acc atg gtg 1761
Arg Leu Gly Leu Gly Asn Ser Gln Thr Gln Arg Glu Pro Thr Met Val
35                  40                  45                  50

ctg tct cct gcc gac aag acc aac gtc aag gcc gcc tgg ggt aag gtc 1809
Leu Ser Pro Ala Asp Lys Thr Asn Val Lys Ala Ala Trp Gly Lys Val
                55                  60                  65

ggc gcg cac gct ggc gag tat ggt gcg gag gcc ctg gag agg cac ttc 1857
Gly Ala His Ala Gly Glu Tyr Gly Ala Glu Ala Leu Glu Arg His Phe
            70                  75                  80

gac ctg agc cac ggc tct gcc cag gtt aag ggc cac ggc aag aag gtg 1905
Asp Leu Ser His Gly Ser Ala Gln Val Lys Gly His Gly Lys Lys Val
        85                  90                  95

gcc gac gcg ctg acc aac gcc gtg gcg cac gtg gac gac atg ccc aac 1953
Ala Asp Ala Leu Thr Asn Ala Val Ala His Val Asp Asp Met Pro Asn
    100                 105                 110

gcg ctg tcc gcc ctg agc gac ctg cac gcg cac aag ctt cgg gtg gac 2001
Ala Leu Ser Ala Leu Ser Asp Leu His Ala His Lys Leu Arg Val Asp
115                 120                 125                 130

ccg gtc aac ttc aag ctc cta agc cac tgc ctg ctg gtg acc ctg gcc 2049
Pro Val Asn Phe Lys Leu Leu Ser His Cys Leu Leu Val Thr Leu Ala
                135                 140                 145

gcc cac ctc ccc gcc gag ttc acc cct gcg gtg cac gcc tcc ctg gac 2097
Ala His Leu Pro Ala Glu Phe Thr Pro Ala Val His Ala Ser Leu Asp
            150                 155                 160

aag ttc ctg gct tct gtg agc acc gtg ctg acc tcc aaa tac cgt taa 2145
Lys Phe Leu Ala Ser Val Ser Thr Val Leu Thr Ser Lys Tyr Arg
        165                 170                 175

gctggagcct cggtggccat gcttcttgcc ccttgggcct ccccccagcc cctcctcccc 2205

ttcctgcacc cgtacccccg tggtctttga ataaagtctg agtgggcggc aaaaa 2260

<210> 137
<211> 2329
<212> DNA
<213> Homo sapiens

<220>
<221> CDS
<222> (1612)..(2211)

<400> 137
gcaatttcat aatatggtaa tttttaatat taaaagtaaa ttgtaaatgt gtctttataa 60
acaatatgca taaaatagat catattctaa acccttttct ttttaaaacg ctttttcaca 120
gtaaatttct tatggtcaga ttttccctca gaatagtgca actggaagaa gggaaatgtt 180
ggcagtggct caggactctc tgctagtaca agtccttgta gatctcctgc aggagcgggt 240
gaagactcat gtctgtctcc gtcttcttga tcacctgcag tagctgcacg tgttccgtga 300
caatctgtct gaggtctgtc attttctgga gcagcttggc aaacagctgt gaggactcag 360
ggtggttcag cttcagctgg agctccaggg cttgtagcag gttgtcttga atgtcttcaa 420
tgggcttcac attcagcaaa cctgggcggt ctccactgag aataatgaca gcaataaata 480
ttgccaagtc gctgtcatct aattccagtg cattgaactt cacagcaaac tcaaacttgg 540
gctccataaa gtcaccaaaa ggctttcgca ggctctttag aaactccctt gtcatgaagc 600
cttggccctc ggatatgaga accccatctt tattcatcaa ggaggccagc attgtgtaaa 660
tgatctcgtg gactccatat ttgaggagag ttacttggtc gttcaagtca agatttacaa 720
aaccaggaat gcttttggca tactctgtga tctcctgcac agcctccacg gagcgaaact 780
ggcagccctg aaagatgcgg atggccacct ctttgctctg ctcctgcagg ggggtgatgt 840
gtttgaactt gattttatct tctcccatca ttaaggaatt catgtcatag ataacgaatg 900
gtgatttgtc tgttgtcttt cctgtcaaga tcgccctcgc ctttgctttg gtcagcggga 960
aggactttat gtatgagtca tacaaatgtt ttgccagggc ctgacggagg tcagcggact 1020
ctggattcag ctggtcgata tcactggaga tctccgccaa cagcttctcc ttctcggcct 1080
gtgcgatccg cccaaacctg atggcattat gagacatccc cactgcaagg catttctgaa 1140
accgacagta ctgacattta tttctacttt ttttgtggat ccgacagtta agatcacatc 1200
tgtcatagat aagcttcaat ctgattgttc tccggaagaa acccttgcat ccttcacaag 1260
catgaactcc atagtgaaat ccagaagctt tatctccaca gacacgacat tcaattgcca 1320
tgagggagtt ggaaggctct tcatgaggct tattgtagag ctgagtcttc tcagaataat 1380
aaggtggaga tgcaggctcc actttgattg cactttggta ctcttgaagt ttcaggtcat 1440
acttgtaatc tgcaaccact ggatctgttc ttgtgaatgg aatgtcttcg taatgtggag 1500
tagaaatgct ggagaagtca acagtagtga agggcttgat atcaaaggag tgggagtggt 1560
cttccattac ggagagatcc acggagctga tcccaaagtt ggtgggccag a atg cga 1617
tct ctg tgt caa cca tgg tca ttt ctg cgg cca cgg cgg cgt ggc gcc 1665
cct ctc cgg tgt cct cga ggc cga ccc aag ccc ccc agg cgg cgg ctg 1713
cgg ctc ggg ctc ggg aat tcc cag act cag aga gaa ccc acc atg gtg 1761
ctg tct cct gcc gac aag acc aac gtc aag gcc gcc tgg ggt aag gtc 1809
ggc gcg cac gct ggc gag tat ggt gcg gag gcc ctg gag agg atg ttc 1857
ctg tcc ttc ccc acc acc aag acc tac ttc ccg cac ttc gac ctg agc 1905
cac ggc tct gcc cag gtt aag ggc cac ggc aag aag gtg gcc gac gcg 1953
ctg acc aac gcc gtg gcg cac gtg gac gac atg ccc aac gcc gtg gcg 2001
cac gtg gac gac atg ccc aac gcg ctg tcc gcc ctg agc gac ctg cac 2049
gcg cac aag ctt cgg gtg gac ccg gtc aac ttc aag ctc cta agc cac 2097
tgc ctg ctg gtg acc ctg gcc gcc cac ctc ccc gcc gag ttc acc cct 2145
gcg gtg cac gcc tcc ctg gac aag ttc ctg gct tct gtg agc acc gtg 2193
ctg acc tcc aaa tac cgt taagct ggagcctcgg tggccatgct tcttgcccct 2247
tgggcctccc cccagcccct cctccccttc ctgcacccgt acccccgtgg tctttgaata 2307
aagtctgagt gggcggcaaa aa 2329

<210> 138
<211> 1050
<212> DNA
<213> Homo sapiens

<220>
<221> CDS
<222> (649)..(789)

<400> 138
atggggagag gtgcaccctg ccacctgcta acttctcgct agacacagtg tttctgccca 60

ggtgacctgt tcagcagcag aacaagccag ggccatgggg acgggggaag ttttcacttg 120

gagatggaca ccaagacaat gaagatttgt tgtccaaata ggtcaataat tctgggagac 180

tcttggaaaa aactgaatat attcaggacc aactctctcc ctcccctcat cccacatctc 240

aaagcagaca atgtaaagag agaacatctc acacacccag ctcgccatgc ctactcattc 300

ctgaatttca ggtgccatca ctgctctttc tttcttcttt gtcatttgag aaaggatgca 360

ggaggacaat tcccacagat aatctgagga atgcagaaaa accagggcag gacagttatc 420

gacaatgcat tagaacttgg tgagcatcct ctgtagaggg actccacccc tgctcaacag 480

cttggcttcc aggcaagacc aaccacatct ggtctctgcc ttcggtggcc cacacaccta 540

agcgtcatcg tcattgccat agcatcatga tgcaacacat ctacgtgtag cactacgacg 600

ttatgtttgg gtaatgtggg gatgaactgc atgaggctct gattaagg atg tgg gga 657
Met Trp Gly
1

agt ggg ctg cgg tca ctg tcg gcc ttg caa ggc cac ctg gag gcc tgt 705
Ser Gly Leu Arg Ser Leu Ser Ala Leu Gln Gly His Leu Glu Ala Cys
    5                   10                  15

ctg tta gcc agt ggt gga gga gca agg ctt cag gaa ggg cca gcc aca 753
Leu Leu Ala Ser Gly Gly Gly Ala Arg Leu Gln Glu Gly Pro Ala Thr
20                  25                  30                  35

tgc cat ctt ccc tgc gat cag gca aaa aag tgg aat taaa aagtcaaacc 803
Cys His Leu Pro Cys Asp Gln Ala Lys Lys Trp Asn
                40                  45

tttatatgca tgtgttatgt ccattttgca ggatgaactg agtttaaaag aatttttttt 863

tctcttcaag ttgctttgtc ttttccatcc tcatcacaag cccttgtttg agtgtcttat 923

ccctgagcaa tctttcgatg gatggagatg atcattaggt acttttgttt caacctttat 983

tcctgtaaat atttctgtga aaactaggag aacagagatg agatttgaca aaaaaaaatt 1043

gaattaa 1050

<210> 139
<211> 711
<212> DNA
<213> Homo sapiens

<220>
<221> CDS
<222> (125)..(448)

<400> 139
acatttacca atcaccaagc atgcaccaaa cactgcttta ggcactgggg acacaaaggg 60

gacagagcca tcctcctttg acacctggtc ttcagttctg tgcccaacgt atatagtttt 120

gaca atg acc agg ttg gac tgt tta atg tct ttc aac tta cca cgt aat 169
Met Thr Arg Leu Asp Cys Leu Met Ser Phe Asn Leu Pro Arg Asn
1               5                   10                  15

cct ctt gta ggg atc aca tct ttc ttt atg ata ttg tat ttc tct acc 217
Pro Leu Val Gly Ile Thr Ser Phe Phe Met Ile Leu Tyr Phe Ser Thr
                20                  25                  30

tct aac agt aaa aat tcc att caa ccc tta aag ctc act tca aat tct 265
Ser Asn Ser Lys Asn Ser Ile Gln Pro Leu Lys Leu Thr Ser Asn Ser
            35                  40                  45

tct ttg aga agt ttt tcc ttt ctc cgc aac cag atg tac ata ttt gaa 313
Ser Leu Arg Ser Phe Ser Phe Leu Arg Asn Gln Met Tyr Ile Phe Glu
        50                  55                  60

ctc tct ttg tac ttg gag ggc act tct ttc gtg gta gtt ctt tta ttt 361
Leu Ser Leu Tyr Leu Glu Gly Thr Ser Phe Val Val Val Leu Leu Phe
    65                  70                  75

tta tta atc tct gta tcc tta gat agt cct cca aca acc aaa ggt tgg 409
Leu Leu Ile Ser Val Ser Leu Asp Ser Pro Pro Thr Thr Lys Gly Trp
80                  85                  90                  95

gac tct gtc tta cat atc tgg gtg ccc ctc ata gtg cag taataagtaa 458
Asp Ser Val Leu His Ile Trp Val Pro Leu Ile Val Gln
                100                 105

gttgattata tacgagctat gtaacttata ttttttaatg gttggatatc actgagtttt 518

tttttttaag aattttttta ttgaggtaaa cttcacataa cataaaatta actattttaa 578

agtgagaagt tcagtgccac ttagtattgt taacaatgtt gcataaccac cacctttatt 638

taaagttcca aaaaaaatgt tctcctctaa aaggaaaccc catcccatta agcagatact 698

ttccattcct tcc 711

<210> 140
<211> 202
<212> DNA
<213> Homo sapiens

<220>
<221> CDS
<222> (21)..(152)

<400> 140
aaagaatcta aattgtaatc atg ttt cat gta ttt gtt tta tta ctt act 50
Met Phe His Val Phe Val Leu Leu Leu Thr
1               5                   10

ttt ata gca ctt agt ccc agt ggt att aga ctg cta ttt ggt ttc ata 98
Phe Ile Ala Leu Ser Pro Ser Gly Ile Arg Leu Leu Phe Gly Phe Ile
                15                  20                  25

caa aaa gga tta aat tta aat tca ttc atg ttt aga ctt gag tta tta 146
Gln Lys Gly Leu Asn Leu Asn Ser Phe Met Phe Arg Leu Glu Leu Leu
            30                  35                  40

cat ttt taaaactatc atcttgcctt taatgtttgt ggtcctacac aaactattag 202
His Phe

<210> 141
<211> 499
<212> DNA
<213> Homo sapiens

<220>
<221> CDS
<222> (66)..(452)

<400> 141
tgcggatccc agcggcggtc gtgtagctga gcagccctgg ggcttggttc tatgtccctg 60

tggct atg ttt cca gtg tcc tct ggg tgt ttc caa gag caa caa gaa 107
Met Phe Pro Val Ser Ser Gly Cys Phe Gln Glu Gln Gln Glu
1               5                   10

acg aat aaa tct ctg ccc cac agc gcc tcc acc cca gag acc cgg acc 155
Thr Asn Lys Ser Leu Pro His Ser Ala Ser Thr Pro Glu Thr Arg Thr
15                  20                  25                  30

aag ttc aca cag gac aat ctg tgc cac gcc cag cgc gag cgc ctg gac 203
Lys Phe Thr Gln Asp Asn Leu Cys His Ala Gln Arg Glu Arg Leu Asp
                35                  40                  45

tcg gcc aac ctg tgg gtg ctg gtg gac tgc atc ctt cgc gac acc tcc 251
Ser Ala Asn Leu Trp Val Leu Val Asp Cys Ile Leu Arg Asp Thr Ser
            50                  55                  60

gag gac ctg gga ctc cag tgt gac gcc gtg aac ctg gcc ttc ggg cgc 299
Glu Asp Leu Gly Leu Gln Cys Asp Ala Val Asn Leu Ala Phe Gly Arg
        65                  70                  75

cgc tgt gag gaa ctg gag gac gcg cgg cac aag ctg cag cac cac ctg 347
Arg Cys Glu Glu Leu Glu Asp Ala Arg His Lys Leu Gln His His Leu
    80                  85                  90

cac aag atg ctg cgg gaa atc aca gat cag gaa cac aac gtg gtg gca 395
His Lys Met Leu Arg Glu Ile Thr Asp Gln Glu His Asn Val Val Ala
95                  100                 105                 110

ctg aag gag gcc atc aag gac aag gag gaa cct ctg cac ata gcc cag 443
Leu Lys Glu Ala Ile Lys Asp Lys Glu Glu Pro Leu His Ile Ala Gln
                115                 120                 125

acc cgg ctg tagctgc tctcacaccg gcccaacatg cagctgtgcc gcgatgcagc 499
Thr Arg Leu

<210> 142
<211> 685
<212> DNA
<213> Homo sapiens

<220>
<221> CDS
<222> (59)..(559)

<400> 142
ggatcgacac atctcgtagc ggcggtcgtg tagctgagca gccctggggc ttggttct 58
atg tcc ctg tgg cta tgt ttc cag tgt cct ctg ggt gtt tcc aag agc 106
Met Ser Leu Trp Leu Cys Phe Gln Cys Pro Leu Gly Val Ser Lys Ser
1               5                   10                  15

aac aag aaa cga ata aat ctc tgc gcc tcc acc ccg gag acc tgg acc 154
Asn Lys Lys Arg Ile Asn Leu Cys Ala Ser Thr Pro Glu Thr Trp Thr
            20                  25                  30

aag ttc acg cag gac aat ctg tgc cgc tcc cag cgc gag cac ctg gac 202
Lys Phe Thr Gln Asp Asn Leu Cys Arg Ser Gln Arg Glu His Leu Asp
        35                  40                  45

tcg gcc aac ctg tgg gtg ctg gtg gac tgc atc ttt cgc gac acc tcc 250
Ser Ala Asn Leu Trp Val Leu Val Asp Cys Ile Phe Arg Asp Thr Ser
    50                  55                  60

gag gac ctg gga ctc cag tgt gac gct gtg aac ctg gcc ttc ggg cgc 298
Glu Asp Leu Gly Leu Gln Cys Asp Ala Val Asn Leu Ala Phe Gly Arg
65                  70                  75                  80

cgc tgt gag gag ctg gag gac acg cgg cac aag ctg cag cac cac ctg 346
Arg Cys Glu Glu Leu Glu Asp Thr Arg His Lys Leu Gln His His Leu
                85                  90                  95

cac aag atg ctg cgg gaa atc aca gat cag aaa caa cgt ggc ggc act 394
His Lys Met Leu Arg Glu Ile Thr Asp Gln Lys Gln Arg Gly Gly Thr
            100                 105                 110

gaa gga ggc cat caa gga caa gga ggc acc tct gca cat agc cca gac 442
Glu Gly Gly His Gln Gly Gln Gly Gly Thr Ser Ala His Ser Pro Asp
        115                 120                 125

cct tct gta cct gcg ctt gca ctg gcc caa cat gga gct gtg tgg caa     490
Pro Ser Val Pro Ala Leu Ala Leu Ala Gln His Gly Ala Val Trp Gln
    130                 135                 140

tgc agc cca gtt cag gtg ctg cct ggg cct ctg agg cag tcc cac ttg     538
Cys Ser Pro Val Gln Val Leu Pro Gly Pro Leu Arg Gln Ser His Leu
145                 150                 155                 160

ccc ccg tcc acc tcc tcg ctg tg accctttcca gtaaacactc caatctccac     591
Pro Pro Ser Thr Ser Ser Leu
                165

acacacatcc cccgcaggtg ctgagagggg agggagactt tgcggccaca cgcaacatgc     651

aagcacacct tatacacaca gatgatatag acat                                685

<210> 143
<211> 1251
<212> DNA
<213> Homo sapiens

<220>
<221> CDS
<222> (343)..(978)

<220>
<221> misc_feature
<222> (1)...(1251)
<223> n = a,t,c or g

<400> 143
cagggaaaaa aataaagttt atttcttgtc caaaagtaat tccaattcag gttttaagtc      60

tgaaccttca aacttgggaa gtttttttcc aagtcatcag caagcccctt atcaacttca     120

cgtaggattt taataaaatc ttccccccca gggctttaaa aaggaaaaag gttttgaaat     180

gcaaaggaac acttttgttt gctcaactgg ccttccttta ctccctgagc caccggctnc     240

tttggtaatg cctttttcta tctttagata ctcttgctca gtctgggagg cataaatgcc     300

aaacaagtcc gttctgaaaa tccaaaatgg cttgatgttt ac   atg ccc att tta     354
                                                 Met Pro Ile Leu
                                                   1

caa ctg ctt act aca gtg gat gat gga att caa gca att gta cat tgt     402
Gln Leu Leu Thr Thr Val Asp Asp Gly Ile Gln Ala Ile Val His Cys
  5                  10                  15                  20

cct gac act gga aaa gac att tgg aat tta ctt ttt gac ctg gtc tgc     450
Pro Asp Thr Gly Lys Asp Ile Trp Asn Leu Leu Phe Asp Leu Val Cys
                 25                  30                  35

cat gaa ttc tgc cag tct gat gat cca ccc atc att ctt caa gaa cag     498
His Glu Phe Cys Gln Ser Asp Asp Pro Pro Ile Ile Leu Gln Glu Gln
             40                  45                  50

aaa aca gtg cta gcc tct gtt ttt tca gtg ttg tct gcc atc tat gcc     546
Lys Thr Val Leu Ala Ser Val Phe Ser Val Leu Ser Ala Ile Tyr Ala
        55                  60                  65

tca cag act gag caa gag tat cta aag ata gaa aaa gta gat ctt cct     594
Ser Gln Thr Glu Gln Glu Tyr Leu Lys Ile Glu Lys Val Asp Leu Pro
    70                  75                  80

cta att gac agc ctc att cgg gtc tta caa aat atg gaa cag tgt cag     642
Leu Ile Asp Ser Leu Ile Arg Val Leu Gln Asn Met Glu Gln Cys Gln
85                  90                  95                  100

aaa aaa cca gag aac tcg gca gag tct aac aca gag gaa act aaa agg     690
Lys Lys Pro Glu Asn Ser Ala Glu Ser Asn Thr Glu Glu Thr Lys Arg
                105                 110                 115

act gat tta acc caa gat gat ttc cac ttg aaa atc tta aag gat att     738
Thr Asp Leu Thr Gln Asp Asp Phe His Leu Lys Ile Leu Lys Asp Ile
            120                 125                 130

tta tgt gaa ttt ctt tct aat att ttt cag gca tta aca aag gag acg     786
Leu Cys Glu Phe Leu Ser Asn Ile Phe Gln Ala Leu Thr Lys Glu Thr
        135                 140                 145

gtg gct cag gga gta aag gaa ggc cag ttg agc aaa cag aag tgt tcc     834
Val Ala Gln Gly Val Lys Glu Gly Gln Leu Ser Lys Gln Lys Cys Ser
    150                 155                 160

tct gca ttt caa aac ctt ctt cct ttc tat agc cct gtg gtg gaa gat     882
Ser Ala Phe Gln Asn Leu Leu Pro Phe Tyr Ser Pro Val Val Glu Asp
165                 170                 175                 180

ttt att aaa atc cta cgt gaa gtt gat aag gcg ctt gct gat gac ttg     930
Phe Ile Lys Ile Leu Arg Glu Val Asp Lys Ala Leu Ala Asp Asp Leu
                185                 190                 195

gaa aaa aac ttc cca agt ttg aag gaa cct tat gca aaa atc cag atg     978
Glu Lys Asn Phe Pro Ser Leu Lys Glu Pro Tyr Ala Lys Ile Gln Met
            200                 205                 210

taatgaggtg gctcctttca cccttcacgt ggtctgccag gaagttgcct acttgctaaa     1038

aggtgtaccc tgagcatttg tgactccgag ggaaaccagg aacatcatgc tgcagaaacc     1098

ctttggagga acgtgccaac tgctccgtaa ccatgagctg gctctccttt gatggagaga     1158

ttaatagttt ccataattat atcatcctgg ttgttctggg gccagagttt acttagactt     1218

gaaagaaatt gaagttaatt taaaaaaaaa aaa                                  1251

<210> 144
<211> 726
<212> DNA
<213> Homo sapiens

<220>
<221> CDS

<222> (151)..(522)

<400> 144
ttctgcaagc tcagcagcag aatgcccaga gtcttccctg gtagatgcag gttccatagc 60

gacgttctcc tgcaatgcac gctggtattc tgcaatagca gtccatgttt ttccttgagc 120

ctggatgctt tggagccaag ctttcgtccc atg caa ggg aaa caa cca ctt 171
Met Gln Gly Lys Gln Pro Leu
1               5

ctg gga cag agg act gct gtc tgc cct ctc tgg tca ccc tgc cta gct 219
Leu Gly Gln Arg Thr Ala Val Cys Pro Leu Trp Ser Pro Cys Leu Ala
        10                  15                  20

aga gga tct gtg acc cca gcc atg agg acc ctc gcc atc ctt gct gcc 267
Arg Gly Ser Val Thr Pro Ala Met Arg Thr Leu Ala Ile Leu Ala Ala
    25                  30                  35

att ctc ctg gtg gcc ctg cag gcc cag gct gag cca ctc cag gca aga 315
Ile Leu Leu Val Ala Leu Gln Ala Gln Ala Glu Pro Leu Gln Ala Arg
40                  45                  50                  55

gct gat gag gtt gct gca gcc ccg gag cag att gca gcg gac atc cca 363
Ala Asp Glu Val Ala Ala Ala Pro Glu Gln Ile Ala Ala Asp Ile Pro
                60                  65                  70

gaa gtg gtt gtt tcc ctt gca tgg gac gaa agc ttg gct cca aag cat 411
Glu Val Val Val Ser Leu Ala Trp Asp Glu Ser Leu Ala Pro Lys His
            75                  80                  85

cca ggc tca agg aaa aac atg gac tgc tat tgc aga ata cca gcg tgc 459
Pro Gly Ser Arg Lys Asn Met Asp Cys Tyr Cys Arg Ile Pro Ala Cys
        90                  95                  100

att gca gga gaa cgt cgc tat gga acc tgc atc tac cag gga aga ctc 507
Ile Ala Gly Glu Arg Arg Tyr Gly Thr Cys Ile Tyr Gln Gly Arg Leu
    105                 110                 115

tgg gca ttc tgc tgc tgagcttgca gaaaaagaaa aatgagctca aaatttgctt 562
Trp Ala Phe Cys Cys
120

tgagagctac agggaattgc tattactcct gtaccttctg ctcaatttcc tttcctcatc 622

tcaaataaat gccttgttac aaaaaaaaat atttataata ttagaattta gaaaatatca 682

gaggcactta aaaaagcttt acttaaaact attgtagtag taaa 726

<210> 145
<211> 1838
<212> DNA
<213> Homo sapiens

<220>
<221> CDS
<222> (228)..(1799)

<400> 145
   tgcctctcca ggagcccagc gcaggccgca gagccggggc cgctgtgagc cgagaccgcg      60
   ggccgcggag ctcgggcggc cggcgcggag aatttgttcc tgttgaagag tggctcctct     120
   tctaatttcc agactccttg aggttttagg agtctggtag gtgaaatttt ctacctctaa     180
   ggagaaacag tacctgctcc ttcctcaagc gcaagccctc cattgct atg gat acc       236
   gaa tcc act tat tct gga tat tct tac tat tca agt cat tcg aaa aaa       284
   tct cac aga caa ggg gaa aga act aga gag aga cac aag tca ccc cgg       332
   aat aaa gac ggc aga ggg tca gaa aag tct gtc acc att caa cct ccc       380
   act gga gag ccc ctg ttg gga aat gat tct act cgg aca gag gaa gtt       428
   cag gat gac aac tgg gga gag acc acc acg gcc att aca ggc acc tcg       476
   gag cac agc ata tcc caa gag gac att gcc agg atc agc aag gac atg       524
   gag gac agc gtg ggg ctg gat tgc aaa cgc tac ctg ggc ctc acc gtc       572
   gcc tct ttt ctt gga ctt cta gtt ttc ctc acc cct att gcc ttc atc       620
   ctt tta cct ccg atc ctg tgg agg gat gag ctg gag cct tgt ggc aca       668
   att tgt gag ggg ctc ttt atc tcc atg gca ttc aaa ctc ctc att ctg       716
   ctc ata ggg acc tgg gca ctt ttt ttc cgc aag cgg aga gct gac atg       764
   cca cgg gtg ttt gtg ttt cgt gcc ctt ttg ttg gtc ctc atc ttt ctc       812
   ttt gtg gtt tcc tat tgg ctt ttt tac ggg gtc cgc att ttg gac tct 860
   cgg gac cgg aat tac cag ggc att gtg caa tat gca gtc tcc ctt gtg 908
   gat gcc ctc ctc ttc atc cat tac ctg gcc atc gtc ctg ctg gag ctc 956
   agg cag ctg cag ccc atg ttc acg ctg cag gtg gtc cgc tcc acc gat 1004
   ggc gag tcc cgc ttc tac agc ctg gga cac ctg agt atc cag cga gca 1052
   gca ttg gtg gtc cta gaa aat tac tac aaa gat ttc acc atc tat aac 1100
   cca aac ctc cta aca gcc tcc aaa ttc cga gca gcc aag cat atg gcc 1148
   ggg ctg aaa gtc tac aat gta gat ggc ccc agt aac aat gcc act ggc 1196
   cag tcc cgg gcc atg att gct gca gct gct cgg cgc agg gac tca agc 1244
   cac aac gag ttg tat tat gaa gag gcc gaa cat gaa cgg cga gta aag 1292
   aag cgg aaa gca agg ctg gtg gtt gca gtg gaa gag gcc ttc atc cac 1340
   att cag cgt ctc cag gct gag gag cag cag aaa gcc cca ggg gag gtg 1388
   atg gac cct agg gag gcc gcc cag gcc att ttc ccc tcc atg gcc agg 1436
   gct ctc cag aag tac ctg cgc atc acc cgg cag cag aac tac cac agc 1484
   atg gag agc atc ctg cag cac ctg gcc ttc tgc atc acc aac ggc atg 1532
   acc ccc aag gcc ttc cta gaa cgg tac ctc agt gcg ggc ccc acc ctg 1580
   caa tat gac aag gac cgc tgg ctc tct aca cag tgg agg ctt gtc agt 1628
   gat gag gct gtg act aat gga tta cgg gat gga att gtg ttc gtc ctt 1676
   aag tgc ttg gac ttc agc ctc gta gtc aat gtg aag aaa att cca ttc 1724
   atc ata ctc tct gaa gag ttc ata gac ccc aaa tct cac aaa ttt gtc 1772
   ctt cgc tta cag tct gag aca tcc gtt taaaa gttctatatt tgtggcttta 1824
   ttaaaaaaaa aaaa 1838

<210> 146
<211> 3485
<212> DNA
<213> Homo sapiens

<220>
<221> CDS
<222> (4)..(2838)

<400> 146
agc atg ggg gcc cgg agc gga gct cgg ggc gcg ctg ctg ctg gca ctg 48
Met Gly Ala Arg Ser Gly Ala Arg Gly Ala Leu Leu Leu Ala Leu
1               5                   10                  15

ctg ctc tgc tgg gac ccg agg ctg agc caa gca ggc act gat tct ggc 96
Leu Leu Cys Trp Asp Pro Arg Leu Ser Gln Ala Gly Thr Asp Ser Gly
                20                  25                  30

agc gag gtg ctc cct gac tcc ttc ccg tca gcg cca gca gag ccg ctg 144
Ser Glu Val Leu Pro Asp Ser Phe Pro Ser Ala Pro Ala Glu Pro Leu
            35                  40                  45

ccc tac ttc ctg cag gag cca cag gac gcc tac att gtg aag aac aag 192
Pro Tyr Phe Leu Gln Glu Pro Gln Asp Ala Tyr Ile Val Lys Asn Lys
        50                  55                  60

cct gtg gag ctc cgc tgc cgc gcc ttc ccc gcc aca cag atc tac ttc 240
Pro Val Glu Leu Arg Cys Arg Ala Phe Pro Ala Thr Gln Ile Tyr Phe
    65                  70                  75

aag tgc aac ggc gag tgg gtc agc cag aac gac cac gtc aca cag gaa 288

Lys Cys Asn Gly Glu Trp Val Ser Gln Asn Asp His Val Thr Gln Glu
80                  85                  90                  95

ggc ctg gat gag gcc acc ggc ctg cgg gtg cgc gag gtg cag atc gag      336
Gly Leu Asp Glu Ala Thr Gly Leu Arg Val Arg Glu Val Gln Ile Glu
                100                 105                 110

gtg tcg cgg cag cag gtg gag gag ctc ttt ggg ctg gag gat tac tgg      384
Val Ser Arg Gln Gln Val Glu Glu Leu Phe Gly Leu Glu Asp Tyr Trp
            115                 120                 125

tgc cag tgc gtg gcc tgg agc tcc gcg ggc acc acc aag agt cgc cga      432
Cys Gln Cys Val Ala Trp Ser Ser Ala Gly Thr Thr Lys Ser Arg Arg
        130                 135                 140

gcc tac gtc cgc atc gcc tac ctg cgc aag aac ttc gat cag gag cct      480
Ala Tyr Val Arg Ile Ala Tyr Leu Arg Lys Asn Phe Asp Gln Glu Pro
    145                 150                 155

ctg ggc aag gag gtg ccc ctg gac cat gag gtt ctc ctg cag tgc cgc      528
Leu Gly Lys Glu Val Pro Leu Asp His Glu Val Leu Leu Gln Cys Arg
160                 165                 170                 175

ccg ccg gag ggg gtg cct gtg gcc gag gtg gaa tgg ctc aag aat gag      576
Pro Pro Glu Gly Val Pro Val Ala Glu Val Glu Trp Leu Lys Asn Glu
                180                 185                 190

gat gtc atc gac ccc acc cag gac acc aac ttc ctg ctc acc atc gac      624
Asp Val Ile Asp Pro Thr Gln Asp Thr Asn Phe Leu Leu Thr Ile Asp
            195                 200                 205

cac aac ctc atc atc cgc cag gcc cgc ctg tcg gac act gcc aac tat      672
His Asn Leu Ile Ile Arg Gln Ala Arg Leu Ser Asp Thr Ala Asn Tyr
        210                 215                 220

acc tgc gtg gcc aag aac atc gtg gcc aaa cgc cgg agc acc act gcc      720
Thr Cys Val Ala Lys Asn Ile Val Ala Lys Arg Arg Ser Thr Thr Ala
    225                 230                 235

acc gtc atc gtc tac gtg aat ggc ggc tgg tcc agc tgg gca gag tgg      768
Thr Val Ile Val Tyr Val Asn Gly Gly Trp Ser Ser Trp Ala Glu Trp
240                 245                 250                 255

tca ccc tgc tcc aac cgc tgt ggc cga ggc tgg cag aag cgc acc cgg      816
Ser Pro Cys Ser Asn Arg Cys Gly Arg Gly Trp Gln Lys Arg Thr Arg
                260                 265                 270

acc tgc acc aac ccc gct cca ctc aac gga ggg gcc ttc tgc gag ggc      864
Thr Cys Thr Asn Pro Ala Pro Leu Asn Gly Gly Ala Phe Cys Glu Gly
            275                 280                 285

cag gca ttc cag aag acc gcc tgc acc acc atc tgc cca gtc gat ggg      912
Gln Ala Phe Gln Lys Thr Ala Cys Thr Thr Ile Cys Pro Val Asp Gly
        290                 295                 300

gcg tgg acg gag tgg agc aag tgg tca gcc tgc agc act gag tgt gcc      960
Ala Trp Thr Glu Trp Ser Lys Trp Ser Ala Cys Ser Thr Glu Cys Ala
    305                 310                 315

cac tgg cgt agc cgc gag tgc atg gcg ccc cca ccc cag aac gga ggc     1008

His Trp Arg Ser Arg Glu Cys Met Ala Pro Pro Pro Gln Asn Gly Gly
320                 325                 330                 335

cgt gac tgc agc ggg acg ctg ctc gac tct aag aac tgc aca gat ggg     1056
Arg Asp Cys Ser Gly Thr Leu Leu Asp Ser Lys Asn Cys Thr Asp Gly
                340                 345                 350

ctg tgc atg caa aat aag aaa act cta agc gac ccc aac agc cag gtg     1104
Leu Cys Met Gln Asn Lys Lys Thr Leu Ser Asp Pro Asn Ser Gln Val
            355                 360                 365

ctg gag gcc tca ggg gat gcg gcg ctg tat gcg ggg ctc gtg gtg gcc     1152
Leu Glu Ala Ser Gly Asp Ala Ala Leu Tyr Ala Gly Leu Val Val Ala
        370                 375                 380

atc ttc gtg gtc gtg gca atc ctc atg gcg gtg ggg gtg gtg gtg tac     1200
Ile Phe Val Val Val Ala Ile Leu Met Ala Val Gly Val Val Val Tyr
    385                 390                 395

cgc cgc aac tgc cgt gac ttc gac aca gac atc act gac tca tct gct     1248
Arg Arg Asn Cys Arg Asp Phe Asp Thr Asp Ile Thr Asp Ser Ser Ala
400                 405                 410                 415

gcc ctg act ggt ggt ttc cac ccc gtc aac ttt aag acg gca agg ccc     1296
Ala Leu Thr Gly Gly Phe His Pro Val Asn Phe Lys Thr Ala Arg Pro
                420                 425                 430

agc aac ccg cag ctc cta cac ccc tct gtg cct cct gac ctg aca gcc     1344
Ser Asn Pro Gln Leu Leu His Pro Ser Val Pro Pro Asp Leu Thr Ala
            435                 440                 445

agc gcc ggc atc tac cgc gga ccc gtg tat gcc ctg cag gac tcc acc     1392
Ser Ala Gly Ile Tyr Arg Gly Pro Val Tyr Ala Leu Gln Asp Ser Thr
        450                 455                 460

gac aaa atc ccc atg acc aac tct cct ctg ctg gac ccc tta ccc agc     1440
Asp Lys Ile Pro Met Thr Asn Ser Pro Leu Leu Asp Pro Leu Pro Ser
    465                 470                 475

ctt aag gtc aag gtc tac agc tcc agc acc acg ggc tct ggg cca ggc     1488
Leu Lys Val Lys Val Tyr Ser Ser Ser Thr Thr Gly Ser Gly Pro Gly
480                 485                 490                 495

ctg gca gat ggg gct gac ctg ctg ggg gtc ttg ccg cct ggc aca tac     1536
Leu Ala Asp Gly Ala Asp Leu Leu Gly Val Leu Pro Pro Gly Thr Tyr
                500                 505                 510

cct agc gat ttc gcc cgg gac acc cac ttc ctg cac ctg cgc agc gcc     1584
Pro Ser Asp Phe Ala Arg Asp Thr His Phe Leu His Leu Arg Ser Ala
            515                 520                 525

agc ctc ggt tcc cag cag ctc ttg ggc ctg ccc cga gac cca ggg agc     1632
Ser Leu Gly Ser Gln Gln Leu Leu Gly Leu Pro Arg Asp Pro Gly Ser
        530                 535                 540

agc gtc agc ggc acc ttt ggc tgc ctg ggt ggg agg ctc agc atc ccc     1680
Ser Val Ser Gly Thr Phe Gly Cys Leu Gly Gly Arg Leu Ser Ile Pro
    545                 550                 555

ggc aca ggg gtc agc ttg ctg gtg ccc aat gga gcc att ccc cag ggc     1728

Gly Thr Gly Val Ser Leu Leu Val Pro Asn Gly Ala Ile Pro Gln Gly
560                 565                 570                 575

aag ttc tac gag atg tat cta ctc atc aac aag gca gaa agt acc ctc      1776
Lys Phe Tyr Glu Met Tyr Leu Leu Ile Asn Lys Ala Glu Ser Thr Leu
                580                 585                 590

ccg ctt tca gaa ggg acc cag aca gta ttg agc ccc tcg gtg acc tgt      1824
Pro Leu Ser Glu Gly Thr Gln Thr Val Leu Ser Pro Ser Val Thr Cys
            595                 600                 605

gga ccc aca ggc ctc ctg ctg tgc cgc ccc gtc atc ctc acc atg ccc      1872
Gly Pro Thr Gly Leu Leu Leu Cys Arg Pro Val Ile Leu Thr Met Pro
        610                 615                 620

cac tgt gcc gaa gtc agt gcc cgt gac tgg atc ttt cag ctc aag acc      1920
His Cys Ala Glu Val Ser Ala Arg Asp Trp Ile Phe Gln Leu Lys Thr
    625                 630                 635

cag gcc cac cag ggc cac tgg gag gag gtg gtg acc ctg gat gag gag      1968
Gln Ala His Gln Gly His Trp Glu Glu Val Val Thr Leu Asp Glu Glu
640                 645                 650                 655

acc ctg aac aca ccc tgc tac tgc cag ctg gag ccc agg gcc tgt cac      2016
Thr Leu Asn Thr Pro Cys Tyr Cys Gln Leu Glu Pro Arg Ala Cys His
                660                 665                 670

atc ctg ctg gac cag ctg ggc acc tac gtg ttc acg ggc gag tcc tat      2064
Ile Leu Leu Asp Gln Leu Gly Thr Tyr Val Phe Thr Gly Glu Ser Tyr
            675                 680                 685

tcc cgc tca gca gtc aag cgg ctc cag ctg gcc gtc ttc gcc ccc gcc      2112
Ser Arg Ser Ala Val Lys Arg Leu Gln Leu Ala Val Phe Ala Pro Ala
        690                 695                 700

ctc tgc acc tcc ctg gag tac agc ctc cgg gtc tac tgc ctg gag gac      2160
Leu Cys Thr Ser Leu Glu Tyr Ser Leu Arg Val Tyr Cys Leu Glu Asp
    705                 710                 715

acg cct gta gca ctg aag gag gtg ctg gag ctg gag cgg act ctg ggc      2208
Thr Pro Val Ala Leu Lys Glu Val Leu Glu Leu Glu Arg Thr Leu Gly
720                 725                 730                 735

gga tac ttg gtg gag gag ccg aaa ccg cta atg ttc aag gac agt tac      2256
Gly Tyr Leu Val Glu Glu Pro Lys Pro Leu Met Phe Lys Asp Ser Tyr
                740                 745                 750

cac aac ctg cgc ctc tcc ctc cat gac ctc ccc cat gcc cat tgg agg      2304
His Asn Leu Arg Leu Ser Leu His Asp Leu Pro His Ala His Trp Arg
            755                 760                 765

agc aag ctg ctg gcc aaa tac cag gag atc ccc ttc tat cac att tgg      2352
Ser Lys Leu Leu Ala Lys Tyr Gln Glu Ile Pro Phe Tyr His Ile Trp
        770                 775                 780

agt ggc agc cag aag gcc ctc cac tgc act ttc acc ctg gag agg cac      2400
Ser Gly Ser Gln Lys Ala Leu His Cys Thr Phe Thr Leu Glu Arg His
    785                 790                 795

agc ttg gcc tcc aca gag ctc acc tgc aag atc tgc gtg cgg caa gtg      2448

Ser Leu Ala Ser Thr Glu Leu Thr Cys Lys Ile Cys Val Arg Gln Val
800                 805                 810                 815

gaa ggg gag ggc cag ata ttc cag ctg cat acc act ctg gca gag aca      2496
Glu Gly Glu Gly Gln Ile Phe Gln Leu His Thr Thr Leu Ala Glu Thr
                820                 825                 830

cct gct ggc tcc ctg gac act ctc tgc tct gcc cct ggc agc act gtc      2544
Pro Ala Gly Ser Leu Asp Thr Leu Cys Ser Ala Pro Gly Ser Thr Val
            835                 840                 845

acc acc cag ctg gga cct tat gcc ttc aag atc cca ctg tcc atc cgc      2592
Thr Thr Gln Leu Gly Pro Tyr Ala Phe Lys Ile Pro Leu Ser Ile Arg
        850                 855                 860

cag aag ata tgc aac agc cta gat gcc ccc aac tca cgg ggc aat gac      2640
Gln Lys Ile Cys Asn Ser Leu Asp Ala Pro Asn Ser Arg Gly Asn Asp
    865                 870                 875

tgg cgg atg tta gca cag aag ctc tct atg gac cgg tac ctg aat tac      2688
Trp Arg Met Leu Ala Gln Lys Leu Ser Met Asp Arg Tyr Leu Asn Tyr
880                 885                 890                 895

ttt gcc acc aaa gcg agc ccc acg ggt gtg atc ctg gac ctc tgg gaa      2736
Phe Ala Thr Lys Ala Ser Pro Thr Gly Val Ile Leu Asp Leu Trp Glu
                900                 905                 910

gct ctg cag cag gac gat ggg gac ctc aac agc ctg gcg agt gcc ttg      2784
Ala Leu Gln Gln Asp Asp Gly Asp Leu Asn Ser Leu Ala Ser Ala Leu
            915                 920                 925

gag gag atg ggc aag agt gag atg ctg gtg gct gtg gcc acc gac ggg      2832
Glu Glu Met Gly Lys Ser Glu Met Leu Val Ala Val Ala Thr Asp Gly
        930                 935                 940

gac tgc tgagcctcct gggacagcgg gctggcaggg actggcagga ggcaggtgca      2888
Asp Cys
    945

gggaggcctg gggcagcctc ctgatgggga tgtttggcct ctgcttcctc ccagttcaca      2948

gccagagttg cctctcctcc tcctcttccc caacccccag accatgacca gccttagaaa      3008

atccatgtac tctgttgtta gagggcccag agttccttct ccacccccgc tctctctctc      3068

ttggcctgag atctctgtgc aggaaccaag atggggctga agcctctgga ggcagttggt      3128

tgggggcggg caggcaggag gccctccctc caccccccca ccctcagccc ggcaacttct      3188

gggttccatg ggttttagtt ccgttctcgt tttcttcctc cgttattgat ttctcctttc      3248

tccctaagcc cccttctgct tccacgccct tttcctcttt gaagagtcaa gtacaattca      3308

gacaaactgc tttctcctgt ccaaaagcaa aaaggcaaag gaaagaaaga aagcttcaga      3368

ccgctagtaa ggctcaaaga agaagaaaaa caccaaaacc acaaggaaaa agaaaaaccc      3428

agtttcttag gaaacgcaaa cgatttatta tccagattat ttggataagt ccttttt      3485

<210> 147
<211> 539
<212> PRT
<213> Homo sapiens

<400> 147
Met Ala Ala Gly Gly Ser Ala Pro Glu Pro Arg Val Leu Val Cys Leu
1               5                   10                  15
Gly Ala Leu Leu Ala Gly Trp Val Thr Val Gly Leu Glu Ala Val Val
            20                  25                  30
Ile Trp Arg Ser Tyr Glu Asn Val Thr Leu His Cys Gly Asn Ile Ser
        35                  40                  45
Gly Leu Arg Gly Gln Val Thr Trp Tyr Arg Asn Asn Ser Glu Pro Val
    50                  55                  60
Phe Leu Leu Ser Ser Asn Ser Ser Leu Arg Pro Ala Glu Pro Arg Phe
65                  70                  75                  80
Ser Leu Val Asp Ala Thr Ser Leu His Ile Glu Ser Leu Ser Leu Gly
                85                  90                  95
Asp Glu Gly Ile Tyr Thr Cys Gln Glu Ile Leu Asn Val Thr Gln Trp
            100                 105                 110
Phe Gln Val Trp Leu Gln Val Ala Ser Gly Pro Tyr Gln Ile Glu Val
        115                 120                 125
His Ile Val Ala Thr Gly Thr Leu Pro Asn Gly Thr Leu Tyr Ala Ala
    130                 135                 140
Arg Gly Ser Gln Val Asp Phe Ser Cys Asn Ser Ser Ser Arg Pro Pro
145                 150                 155                 160
Pro Val Val Glu Trp Trp Phe Gln Ala Leu Asn Ser Ser Ser Glu Ser
                165                 170                 175
Phe Gly His Asn Leu Thr Val Asn Phe Phe Ser Leu Leu Leu Ile Ser
            180                 185                 190
Pro Asn Leu Gln Gly Asn Tyr Thr Cys Leu Ala Leu Asn Gln Leu Ser
        195                 200                 205
Lys Arg His Arg Lys Val Thr Thr Glu Leu Leu Val Tyr Tyr Pro Pro
    210                 215                 220
Pro Ser Ala Pro Gln Cys Trp Ala Gln Met Ala Ser Gly Ser Phe Met
225                 230                 235                 240
Leu Gln Leu Thr Cys Arg Trp Asp Gly Gly Tyr Pro Asp Pro Asp Phe
                245                 250                 255
Leu Trp Ile Glu Glu Pro Gly Gly Val Ile Val Gly Lys Ser Lys Leu
            260                 265                 270
Gly Val Glu Met Leu Ser Glu Ser Gln Leu Ser Asp Gly Lys Lys Phe
        275                 280                 285
Lys Cys Val Thr Ser His Ile Val Gly Pro Glu Ser Gly Ala Ser Cys
    290                 295                 300
Met Val Gln Ile Arg Gly Pro Ser Leu Leu Ser Glu Pro Met Lys Thr
305                 310                 315                 320
Cys Phe Thr Gly Gly Asn Val Thr Leu Thr Cys Gln Val Ser Gly Ala
                325                 330                 335
Tyr Pro Pro Ala Lys Ile Leu Trp Leu Arg Asn Leu Thr Gln Pro Glu
            340                 345                 350
Val Ile Ile Gln Pro Ser Ser Arg His Leu Ile Thr Gln Asp Gly Gln
        355                 360                 365
Asn Ser Thr Leu Thr Ile His Asn Cys Ser Gln Asp Leu Asp Glu Gly
    370                 375                 380
Tyr Tyr Ile Cys Arg Ala Asp Ser Pro Val Gly Val Arg Glu Met Glu
385                 390                 395                 400
Ile Trp Leu Ser Val Lys Glu Pro Leu Asn Ile Gly Gly Ile Val Gly
                405                 410                 415
Thr Ile Val Ser Leu Leu Leu Leu Gly Leu Ala Ile Ile Ser Gly Leu

            420                 425                 430
Leu Leu His Tyr Ser Pro Val Phe Cys Trp Lys Gly Asn Thr Ser Arg
        435                 440                 445
Gly Gln Tyr Met Asp Asp Val Met Val Leu Val Asp Ser Glu Glu Glu
    450                 455                 460
Glu Glu Glu Glu Glu Glu Glu Glu Glu Asp Ala Ala Val Gly Glu Gln
465                 470                 475                 480
Glu Gly Ala Arg Glu Arg Glu Glu Leu Pro Lys Glu Ile Pro Lys Gln
                485                 490                 495
Asp His Ile His Arg Val Thr Ala Leu Val Asn Gly Asn Ile Glu Gln
            500                 505                 510
Met Gly Asn Gly Phe Gln Asp Leu Gln Asp Asp Ser Ser Glu Glu Gln
        515                 520                 525
Ser Asp Ile Val Gln Glu Glu Asp Arg Pro Val
    530                 535

<210> 148
<211> 388
<212> PRT
<213> Homo sapiens

<400> 148

<210> 149
<211> 73
<212> PRT
<213> Homo sapiens

<400> 149

<210> 150
<211> 99
<212> PRT
<213> Homo sapiens

<400> 150
Met Glu Ala Leu Ala Asn Val Asn Phe Pro Arg Lys Ser Phe Arg Pro
1               5                   10                  15
Glu Asp Ala Gly Lys Glu Ser Gly Ser Gln Gly Gly Phe Cys Val Pro
            20                  25                  30
Ala Ala Arg Pro Gln Thr Met Val Thr Gly Pro Ser Cys Ser Ser Pro
        35                  40                  45
Gly Leu Gln Asn Phe Ser Pro Gln Arg Lys Glu Asn Arg Ala Cys Ala
    50                  55                  60
Cys Trp Gln Asn Ala Gly Pro Ala Pro Lys Asn Pro Met Cys Val Arg
65                  70                  75                  80
Leu Lys Val Gly Arg Pro Gln Ala Ser Gln Arg Lys Leu Lys Glu Thr
                85                  90                  95
Gly Leu Cys

<210> 151
<211> 190
<212> PRT
<213> Homo sapiens

<400> 151
Met Arg Ser Leu Cys Gln Pro Trp Ser Phe Leu Arg Pro Arg Arg Arg
1               5                   10                  15
Gly Ala Pro Leu Arg Cys Pro Arg Gly Arg Pro Lys Pro Pro Arg Arg
            20                  25                  30
Arg Leu Arg Leu Gly Leu Gly Asn Ser Gln Thr Gln Arg Glu Pro Thr
        35                  40                  45
Met Val Leu Ser Pro Ala Asp Lys Thr Asn Val Lys Ala Ala Trp Gly
    50                  55                  60
Lys Val Gly Ala His Ala Gly Glu Tyr Gly Ala Glu Ala Leu Glu Arg
65                  70                  75                  80
Met Phe Leu Ser Phe Pro Thr Thr Lys Thr Tyr Phe Pro His Phe Asp
                85                  90                  95
Leu Ser His Gly Ser Ala Gln Val Lys Gly His Gly Lys Lys Val Ala
            100                 105                 110
Asp Ala Leu Thr Asn Ala Val Ala His Val Asp Asp Met Pro Asn Ala
        115                 120                 125
Leu Ser Ala Leu Ser Asp Leu His Ala His Lys Leu Arg Val Asp Pro
    130                 135                 140
Val Asn Phe Lys Leu Leu Ser His Cys Leu Leu Val Thr Leu Ala Ala
145                 150                 155                 160
His Leu Pro Ala Glu Phe Thr Pro Ala Val His Ala Ser Leu Asp Lys
                165                 170                 175
Phe Leu Ala Ser Val Ser Thr Val Leu Thr Ser Lys Tyr Arg
            180                 185                 190

<210> 152
<211> 288
<212> PRT
<213> Homo sapiens

<400> 152
Met Ser Arg Leu Arg Ala Leu Leu Gly Leu Gly Leu Leu Val Ala Gly
1               5                   10                  15
Ser Arg Leu Pro Arg Ile Lys Ser Gln Thr Ile Ala Cys Arg Ser Gly
            20                  25                  30
Pro Thr Trp Trp Gly Pro Gln Arg Leu Asn Ser Gly Gly Arg Trp Asp
        35                  40                  45
Ser Glu Val Met Ala Ser Thr Val Val Lys Tyr Leu Ser Gln Glu Glu
    50                  55                  60
Ala Gln Ala Val Asp Gln Glu Leu Phe Asn Glu Tyr Gln Phe Ser Val
65                  70                  75                  80
Asp Gln Leu Met Glu Leu Ala Gly Leu Ser Cys Ala Thr Ala Ile Ala
                85                  90                  95
Lys Ala Tyr Pro Pro Thr Ser Met Ser Arg Ser Pro Pro Thr Val Leu
            100                 105                 110
Val Ile Cys Gly Pro Gly Asn Asn Gly Gly Asp Gly Leu Val Cys Ala
        115                 120                 125
Arg His Leu Lys Leu Phe Gly Tyr Glu Pro Thr Ile Tyr Tyr Pro Lys

130                 135                 140
Arg Pro Asn Lys Pro Leu Phe Thr Ala Leu Val Thr Gln Cys Gln Lys
145                 150                 155                 160
Met Asp Ile Pro Phe Leu Gly Glu Met Pro Ala Glu Pro Met Thr Ile
                165                 170                 175
Asp Glu Leu Tyr Glu Leu Val Val Asp Ala Ile Phe Gly Phe Ser Phe
            180                 185                 190
Lys Gly Asp Val Arg Glu Pro Phe His Ser Ile Leu Ser Val Leu Lys
        195                 200                 205
Gly Leu Thr Val Pro Ile Ala Ser Ile Asp Ile Pro Ser Gly Trp Asp
    210                 215                 220
Val Glu Lys Gly Asn Ala Gly Gly Ile Gln Pro Asp Leu Leu Ile Ser
225                 230                 235                 240
Leu Thr Ala Pro Lys Lys Ser Ala Thr Gln Phe Thr Gly Arg Tyr His
                245                 250                 255
Tyr Leu Gly Gly Arg Phe Val Pro Pro Ala Leu Glu Lys Lys Tyr Gln
            260                 265                 270
Leu Asn Leu Pro Pro Tyr Pro Asp Thr Glu Cys Val Tyr Arg Leu Gln
        275                 280                 285

<210> 153
<211> 112
<212> PRT
<213> Homo sapiens

<400> 153
Met Leu Lys Gly Asn Ser Asn Leu Asn Arg Phe Val Tyr Phe Tyr Tyr
1               5                   10                  15
Tyr Phe Leu Arg Trp Ser Leu Ala Leu Ser Leu Trp Leu Glu Cys Ser
            20                  25                  30
Gly Thr Ile Ser Ala His Cys Asn Leu His Leu Pro Gly Ser Ser Asp
        35                  40                  45
Ser Pro Ala Ser Ala Ser Pro Val Thr Gly Ile Thr Gly Thr Cys His
    50                  55                  60
His Ala Gln Leu Ile Phe Leu Tyr Phe Tyr Tyr Arg Arg Gly Phe Ala
65                  70                  75                  80
Met Leu Leu Arg Leu Val Ser Asn Ser Trp Ala Gln Ala Ile His Leu
                85                  90                  95
Pro Trp Leu Pro Lys Val Leu Gly Leu Gln Ala Pro Ala Pro Asp Leu
            100                 105                 110

<210> 154
<211> 361
<212> PRT
<213> Homo sapiens

<400> 154
Met Phe Ser Ser Val Ala His Leu Ala Arg Ala Asn Pro Phe Asn Thr
1               5                   10                  15
Pro His Leu Gln Leu Val His Asp Gly Leu Gly Asp Leu Arg Ser Ser

            20                  25                  30
Ser Pro Gly Pro Thr Gly Gln Pro Arg Arg Pro Arg Asn Leu Ala Ala
        35                  40                  45
Ala Ala Val Glu Glu Tyr Ser Cys Glu Phe Gly Ser Ala Lys Tyr Tyr
    50                  55                  60
Ala Leu Cys Gly Phe Gly Gly Val Leu Ser Cys Gly Leu Thr His Thr
65                  70                  75                  80
Ala Val Val Pro Leu Asp Leu Val Lys Cys Arg Met Gln Val Asp Pro
                85                  90                  95
Gln Lys Tyr Lys Gly Ile Phe Asn Gly Phe Ser Val Thr Leu Lys Glu
            100                 105                 110
Asp Gly Val Arg Gly Leu Ala Lys Gly Trp Ala Pro Thr Phe Leu Gly
        115                 120                 125
Tyr Ser Met Gln Gly Leu Cys Lys Phe Gly Phe Tyr Glu Val Phe Lys
    130                 135                 140
Val Leu Tyr Ser Asn Met Leu Gly Glu Glu Asn Thr Tyr Leu Trp Arg
145                 150                 155                 160
Thr Ser Leu Tyr Leu Ala Ala Ser Ala Ser Ala Glu Phe Phe Ala Asp
                165                 170                 175
Ile Ala Leu Ala Pro Met Glu Ala Ala Lys Val Arg Ile Gln Thr Gln
            180                 185                 190
Pro Gly Tyr Ala Asn Thr Leu Arg Asp Ala Ala Pro Lys Met Tyr Lys
        195                 200                 205
Glu Glu Gly Leu Lys Ala Phe Tyr Lys Gly Val Ala Pro Leu Trp Met
    210                 215                 220
Arg Gln Ile Pro Tyr Thr Met Met Lys Phe Ala Cys Phe Glu Arg Thr
225                 230                 235                 240
Val Glu Ala Leu Tyr Lys Phe Val Val Pro Lys Pro Arg Ser Glu Cys
                245                 250                 255
Ser Lys Pro Glu Gln Leu Val Val Thr Phe Val Ala Gly Tyr Ile Ala
            260                 265                 270
Gly Val Phe Cys Ala Ile Val Ser His Pro Ala Asp Ser Val Val Ser
        275                 280                 285
Val Leu Asn Lys Glu Lys Gly Ser Ser Ala Ser Leu Val Leu Lys Arg
    290                 295                 300
Leu Gly Phe Lys Gly Val Trp Lys Gly Leu Phe Ala Arg Ile Ile Met
305                 310                 315                 320
Ile Gly Thr Leu Thr Ala Leu Gln Trp Phe Ile Tyr Asp Ser Val Lys
                325                 330                 335
Val Tyr Phe Arg Leu Pro Arg Pro Pro Pro Pro Glu Met Pro Glu Ser
            340                 345                 350
Leu Lys Lys Lys Leu Gly Leu Thr Gln
        355                 360

<210> 155
<211> 79
<212> PRT
<213> Homo sapiens

<400> 155
Met Ala His Lys Gln Ile Tyr Tyr Ser Asp Lys Tyr Phe Asp Glu His
1               5                   10                  15
Tyr Glu Tyr Arg His Val Met Leu Pro Arg Glu Leu Ser Lys Gln Val
            20                  25                  30
Pro Lys Thr His Leu Met Ser Glu Glu Glu Trp Arg Arg Leu Gly Val
        35                  40                  45
Gln Gln Ser Leu Gly Trp Val His Tyr Met Ile His Glu Pro Glu Pro

50                  55                  60
His Ile Leu Leu Phe Arg Arg Pro Leu Pro Lys Asp Gln Gln Lys
65                  70                  75

<210> 156
<211> 245
<212> PRT
<213> Homo sapiens

<400> 156
Met Glu Lys Thr Glu Leu Ile Gln Lys Ala Lys Leu Ala Glu Gln Ala
1               5                   10                  15
Glu Arg Tyr Asp Asp Met Ala Thr Cys Met Lys Ala Val Thr Glu Gln
            20                  25                  30
Gly Ala Glu Leu Ser Asn Glu Glu Arg Asn Leu Leu Ser Val Ala Tyr
        35                  40                  45
Lys Asn Val Val Gly Gly Arg Arg Ser Ala Trp Arg Val Ile Ser Ser
    50                  55                  60
Ile Glu Gln Lys Thr Asp Thr Ser Asp Lys Lys Leu Gln Leu Ile Lys
65                  70                  75                  80
Asp Tyr Arg Glu Lys Val Glu Ser Glu Leu Arg Ser Ile Cys Thr Thr
                85                  90                  95
Val Leu Glu Leu Leu Asp Lys Tyr Leu Ile Ala Asn Ala Thr Asn Pro
            100                 105                 110
Glu Ser Lys Val Phe Tyr Leu Lys Met Lys Gly Asp Tyr Phe Arg Tyr
        115                 120                 125
Leu Ala Glu Val Ala Cys Gly Asp Asp Arg Lys Gln Thr Ile Asp Asn
    130                 135                 140
Ser Gln Gly Ala Tyr Gln Glu Ala Phe Asp Ile Ser Lys Lys Glu Met
145                 150                 155                 160
Gln Pro Thr His Pro Ile Arg Leu Gly Leu Ala Leu Asn Phe Ser Val
                165                 170                 175
Phe Tyr Tyr Glu Ile Leu Asn Asn Pro Glu Leu Ala Cys Thr Leu Ala
            180                 185                 190
Lys Thr Ala Phe Asp Glu Ala Ile Ala Glu Leu Asp Thr Leu Asn Glu
        195                 200                 205
Asp Ser Tyr Lys Asp Ser Thr Leu Ile Met Gln Leu Leu Arg Asp Asn
    210                 215                 220
Leu Thr Leu Trp Thr Ser Asp Ser Ala Gly Glu Glu Cys Asp Ala Ala
225                 230                 235                 240
Glu Gly Ala Glu Asn
                245

<210> 157
<211> 366
<212> PRT
<213> Homo sapiens

<400> 157
Met Asp Thr Gly Val Ile Glu Gly Gly Leu Asn Val Thr Leu Thr Ile
1               5                   10                  15
Arg Leu Leu Met His Gly Lys Glu Val Gly Ser Ile Ile Gly Lys Lys
            20                  25                  30
Gly Glu Ser Val Lys Lys Met Arg Glu Glu Ser Gly Ala Arg Ile Asn

35                  40                  45
Ile Ser Glu Gly Asn Cys Pro Glu Arg Ile Ile Thr Leu Ala Gly Pro
    50                  55                  60
Thr Asn Ala Ile Phe Lys Ala Phe Ala Met Ile Ile Asp Lys Leu Glu
65                  70                  75                  80
Glu Asp Ile Ser Ser Ser Met Thr Asn Ser Thr Ala Ala Ser Arg Pro
                85                  90                  95
Pro Val Thr Leu Arg Leu Val Val Pro Ala Ser Gln Cys Gly Ser Leu
            100                 105                 110
Ile Gly Lys Gly Gly Cys Lys Ile Lys Glu Ile Arg Glu Ser Thr Gly
        115                 120                 125
Ala Gln Val Gln Val Ala Gly Asp Met Leu Pro Asn Ser Thr Glu Arg
    130                 135                 140
Ala Ile Thr Ile Ala Gly Ile Pro Gln Ser Ile Ile Glu Cys Val Lys
145                 150                 155                 160
Gln Ile Cys Val Val Met Leu Glu Thr Leu Ser Gln Ser Pro Pro Lys
                165                 170                 175
Gly Val Thr Ile Pro Tyr Arg Pro Lys Pro Ser Ser Ser Pro Val Ile
            180                 185                 190
Phe Ala Gly Gly Gln Asp Arg Tyr Ser Thr Gly Ser Asp Ser Ala Ser
        195                 200                 205
Phe Pro His Thr Thr Pro Ser Met Cys Leu Asn Pro Asp Leu Glu Gly
    210                 215                 220
Pro Pro Leu Glu Ala Tyr Thr Ile Gln Gly Gln Tyr Ala Ile Pro Gln
225                 230                 235                 240
Pro Asp Leu Thr Lys Leu His Gln Leu Ala Met Gln Gln Ser His Phe
                245                 250                 255
Pro Met Thr His Gly Asn Thr Gly Phe Ser Gly Ile Glu Ser Ser Ser
            260                 265                 270
Pro Glu Val Lys Gly Tyr Trp Ala Gly Leu Asp Ala Ser Ala Gln Thr
        275                 280                 285
Thr Ser His Glu Leu Thr Ile Pro Asn Asp Leu Ile Gly Cys Ile Ile
    290                 295                 300
Gly Arg Gln Gly Ala Lys Ile Asn Glu Ile Arg Gln Met Ser Gly Ala
305                 310                 315                 320
Gln Ile Lys Ile Ala Asn Pro Val Glu Gly Ser Thr Asp Arg Gln Val
                325                 330                 335
Thr Ile Thr Gly Ser Ala Ala Ser Ile Ser Leu Ala Gln Tyr Leu Ile
            340                 345                 350
Asn Val Arg Leu Ser Ser Glu Thr Gly Gly Met Gly Ser Ser
        355                 360                 365

<210> 158
<211> 173
<212> PRT
<213> Homo sapiens

<400> 158
Met Ser Ala Trp Gly Pro Ser Gly Asn Pro Ser Pro Arg Ser Gly Gly
1               5                   10                  15
Gly Pro Gly Ala Arg Leu Thr Leu Pro Ala Leu Gln Met Thr Val His
            20                  25                  30
Asn Leu Tyr Leu Phe Asp Arg Asn Gly Val Cys Leu His Tyr Ser Glu
        35                  40                  45
Trp His Arg Lys Lys Gln Ala Gly Ile Pro Lys Glu Glu Glu Tyr Lys
    50                  55                  60
Leu Met Tyr Gly Met Leu Phe Ser Ile Arg Ser Phe Val Ser Lys Met

65                  70                  75                  80
Ser Pro Leu Asp Met Lys Asp Gly Phe Leu Ala Phe Gln Thr Ser Arg
                85                  90                  95
Tyr Lys Leu His Tyr Tyr Glu Thr Pro Thr Gly Ile Lys Val Val Met
            100                 105                 110
Asn Thr Asp Leu Gly Val Gly Pro Ile Arg Asp Val Leu His His Ile
        115                 120                 125
Tyr Ser Ala Leu Tyr Val Glu Leu Val Val Lys Asn Pro Leu Cys Pro
    130                 135                 140
Leu Gly Gln Thr Val Gln Ser Glu Leu Phe Arg Ser Arg Leu Asp Ser
145                 150                 155                 160
Tyr Val Arg Ser Leu Pro Phe Phe Ser Ala Arg Ala Gly
                165                 170

<210> 159
<211> 148
<212> PRT
<213> Homo sapiens

<400> 159
Met Ala Glu Ser Asp Trp Asp Thr Val Thr Val Leu Arg Lys Lys Gly
1               5                   10                  15
Pro Thr Ala Ala Gln Ala Lys Ser Lys Gln Ala Ile Leu Ala Ala Gln
            20                  25                  30
Arg Arg Gly Glu Asp Val Glu Thr Ser Lys Lys Trp Ala Ala Gly Gln
        35                  40                  45
Asn Lys Gln His Ser Ile Thr Lys Asn Thr Ala Lys Leu Asp Arg Glu
    50                  55                  60
Thr Glu Glu Leu His His Asp Arg Val Thr Leu Glu Val Gly Lys Val
65                  70                  75                  80
Ile Gln Gln Gly Arg Gln Ser Lys Gly Leu Thr Gln Lys Asp Leu Ala
                85                  90                  95
Thr Lys Ile Asn Glu Lys Pro Gln Val Ile Ala Asp Tyr Glu Ser Gly
            100                 105                 110
Arg Ala Ile Pro Asn Asn Gln Val Leu Gly Lys Ile Glu Arg Ala Ile
        115                 120                 125
Gly Leu Lys Leu Arg Gly Lys Asp Ile Gly Lys Pro Ile Glu Lys Gly
    130                 135                 140
Pro Arg Ala Lys
145

<210> 160
<211> 113
<212> PRT
<213> Homo sapiens

<400> 160
Met Thr Pro Gly Val Phe His Ala Ser Pro Pro Gln Ser Gln Arg Val
1               5                   10                  15
Pro Arg Gln Ala Pro Cys Glu Trp Ala Ile Arg Asn Ile Gly Gln Lys
            20                  25                  30
Pro Lys Glu Pro Asn Cys His Asn Cys Gly Thr His Ile Gly Leu Arg
        35                  40                  45
Ser Lys Thr Leu Arg Gly Thr Pro Asn Tyr Leu Pro Ile Arg Gln Asp

50                  55                  60
Thr His Pro Pro Ser Val Ile Phe Cys Leu Ala Gly Val Gly Val Pro
65                  70                  75                  80
Gly Gly Thr Cys Arg Pro Ala Pro Cys Val Pro Arg Phe Ala Ala Leu
                85                  90                  95
Pro Trp Ala Thr Asn His Pro Gly Pro Gly Cys Leu Ser Asp Leu Arg
            100                 105                 110
Ala

<210> 161
<211> 61
<212> PRT
<213> Homo sapiens

<400> 161
Met Asp Pro Asn Cys Ser Cys Ala Ala Gly Val Ser Cys Thr Cys Ala
1               5                   10                  15
Gly Ser Cys Lys Cys Lys Glu Cys Lys Cys Thr Ser Cys Lys Lys Ser
            20                  25                  30
Cys Cys Ser Cys Cys Pro Val Gly Cys Ser Lys Cys Ala Gln Gly Cys
        35                  40                  45
Val Cys Lys Gly Ala Ser Glu Lys Cys Ser Cys Cys Asp
    50                  55                  60

<210> 162
<211> 172
<212> PRT
<213> Homo sapiens

<400> 162
Met Ser Cys Thr Ile Glu Lys Ala Leu Ala Asp Ala Lys Ala Leu Val
1               5                   10                  15
Glu Arg Leu Arg Asp His Asp Asp Ala Ala Glu Ser Leu Ile Glu Gln
            20                  25                  30
Thr Thr Ala Leu Asn Lys Arg Val Glu Ala Met Lys Gln Tyr Gln Glu
        35                  40                  45
Glu Ile Gln Glu Leu Asn Glu Val Ala Arg His Arg Pro Arg Ser Thr
    50                  55                  60
Leu Val Met Gly Ile Gln Gln Glu Asn Arg Gln Ile Arg Glu Leu Gln
65                  70                  75                  80
Gln Glu Asn Lys Glu Leu Arg Thr Ser Leu Glu Glu His Gln Ser Ala
                85                  90                  95
Leu Glu Leu Ile Met Ser Lys Tyr Arg Glu Gln Met Phe Arg Leu Leu
            100                 105                 110
Met Ala Ser Lys Lys Asp Asp Pro Gly Ile Ile Met Lys Leu Lys Glu
        115                 120                 125
Gln His Ser Lys Ile Asp Met Val His Arg Asn Lys Ser Glu Gly Phe
    130                 135                 140
Phe Leu Asp Ala Ser Arg His Ile Leu Glu Ala Pro Gln His Gly Leu
145                 150                 155                 160
Glu Arg Arg His Leu Glu Ala Asn Gln Asn Val His
                165                 170

<210> 163
<211> 343
<212> PRT
<213> Homo sapiens

<400> 163

<210> 164
<211> 462
<212> PRT
<213> Homo sapiens

<400> 164
Met Ala Arg Val Leu Gly Ala Pro Val Ala Leu Gly Leu Trp Ser Leu
1               5                   10                  15
Cys Trp Ser Leu Ala Ile Ala Thr Pro Leu Pro Pro Thr Ser Ala His
            20                  25                  30
Gly Asn Val Ala Glu Gly Glu Thr Lys Pro Asp Pro Asp Val Thr Glu
        35                  40                  45
Arg Cys Ser Asp Gly Trp Ser Phe Asp Ala Thr Thr Leu Asp Asp Asn
    50                  55                  60
Gly Thr Met Leu Phe Phe Lys Gly Glu Phe Val Trp Lys Ser His Lys
65                  70                  75                  80
Trp Asp Arg Glu Leu Ile Ser Glu Arg Trp Lys Asn Phe Pro Ser Pro
                85                  90                  95
Val Asp Ala Ala Phe Arg Gln Gly His Asn Ser Val Phe Leu Ile Lys
            100                 105                 110
Gly Asp Lys Val Trp Val Tyr Pro Pro Glu Lys Lys Glu Lys Gly Tyr
        115                 120                 125
Pro Lys Leu Leu Gln Asp Glu Phe Pro Gly Ile Pro Ser Pro Leu Asp
    130                 135                 140
Ala Ala Val Glu Cys His Arg Gly Glu Cys Gln Ala Glu Gly Val Leu
145                 150                 155                 160
Phe Phe Gln Gly Asp Arg Glu Trp Phe Trp Asp Leu Ala Thr Gly Thr
                165                 170                 175
Met Lys Glu Arg Ser Trp Pro Ala Val Gly Asn Cys Ser Ser Ala Leu
            180                 185                 190
Arg Trp Leu Gly Arg Tyr Tyr Cys Phe Gln Gly Asn Gln Phe Leu Arg
        195                 200                 205
Phe Asp Pro Val Arg Gly Glu Val Pro Pro Arg Tyr Pro Arg Asp Val
    210                 215                 220
Arg Asp Tyr Phe Met Pro Cys Pro Gly Arg Gly His Gly His Arg Asn
225                 230                 235                 240
Gly Thr Gly His Gly Asn Ser Thr His His Gly Pro Glu Tyr Met Arg
                245                 250                 255
Cys Ser Pro His Leu Val Leu Ser Ala Leu Thr Ser Asp Asn His Gly
            260                 265                 270
Ala Thr Tyr Ala Phe Ser Gly Thr His Tyr Trp Arg Leu Asp Thr Ser
        275                 280                 285
Arg Asp Gly Trp His Ser Trp Pro Ile Ala His Gln Trp Pro Gln Gly
    290                 295                 300
Pro Ser Ala Val Asp Ala Ala Phe Ser Trp Glu Glu Lys Leu Tyr Leu
305                 310                 315                 320
Val Gln Gly Thr Gln Val Tyr Val Phe Leu Thr Lys Gly Gly Tyr Thr
                325                 330                 335
Leu Val Ser Gly Tyr Pro Lys Arg Leu Glu Lys Glu Val Gly Thr Pro
            340                 345                 350
His Gly Ile Ile Leu Asp Ser Val Asp Ala Ala Phe Ile Cys Pro Gly
        355                 360                 365
Ser Ser Arg Leu His Ile Met Ala Gly Arg Arg Leu Trp Trp Leu Asp
    370                 375                 380
Leu Lys Ser Gly Ala Gln Ala Thr Trp Thr Glu Leu Pro Trp Pro His
385                 390                 395                 400
Glu Lys Val Asp Gly Ala Leu Cys Met Glu Lys Ser Leu Gly Pro Asn
                405                 410                 415
Ser Cys Ser Ala Asn Gly Pro Gly Leu Tyr Leu Ile His Gly Pro Asn
            420                 425                 430
Leu Tyr Cys Tyr Ser Asp Val Glu Lys Leu Asn Ala Ala Lys Ala Leu
        435                 440                 445
Pro Gln Pro Gln Asn Val Thr Ser Leu Leu Gly Cys Thr His

450                 455                 460

<210> 165
<211> 280
<212> PRT
<213> Homo sapiens

<400> 165
Met Asp Leu Gln Gly Arg Gly Val Pro Ser Ile Asp Arg Leu Arg Val
1               5                   10                  15
Leu Leu Met Leu Phe His Thr Met Ala Gln Ile Met Ala Glu Gln Glu
            20                  25                  30
Val Glu Asn Leu Ser Gly Leu Ser Thr Asn Pro Glu Lys Asp Ile Phe
        35                  40                  45
Val Val Arg Glu Asn Gly Thr Thr Cys Leu Met Ala Glu Phe Ala Ala
    50                  55                  60
Lys Phe Ile Val Pro Tyr Asp Val Trp Ala Ser Asn Tyr Val Asp Leu
65                  70                  75                  80
Ile Thr Glu Gln Ala Asp Ile Ala Leu Thr Arg Gly Ala Glu Val Lys
                85                  90                  95
Gly Arg Cys Gly His Ser Gln Ser Glu Leu Gln Val Phe Trp Val Asp
            100                 105                 110
Arg Ala Tyr Ala Leu Lys Met Leu Phe Val Lys Glu Ser His Asn Met
        115                 120                 125
Ser Lys Gly Pro Glu Ala Thr Trp Arg Leu Ser Lys Val Gln Phe Val
    130                 135                 140
Tyr Asp Ser Ser Glu Lys Thr His Phe Lys Asp Ala Val Ser Ala Gly
145                 150                 155                 160
Lys His Thr Ala Asn Ser His His Leu Ser Ala Leu Val Thr Pro Ala
                165                 170                 175
Gly Lys Ser Tyr Glu Cys Gln Ala Gln Gln Thr Ile Ser Leu Ala Ser
            180                 185                 190
Ser Asp Pro Gln Lys Thr Val Thr Met Ile Leu Ser Ala Val His Ile
        195                 200                 205
Gln Pro Phe Asp Ile Ile Ser Asp Phe Val Phe Ser Glu Glu His Lys
    210                 215                 220
Cys Pro Val Asp Glu Arg Glu Gln Leu Glu Glu Thr Leu Pro Leu Ile
225                 230                 235                 240
Leu Gly Leu Ile Leu Gly Leu Val Ile Met Val Thr Leu Ala Ile Tyr
                245                 250                 255
His Val His His Lys Met Thr Ala Asn Gln Val Gln Ile Pro Arg Asp
            260                 265                 270
Arg Ser Gln Tyr Lys His Met Gly
        275                 280

<210> 166
<211> 494
<212> PRT
<213> Homo sapiens

<400> 166
Met Glu Ala Cys Ile Ser Val Leu Pro Thr Val Ser Gly Asn Thr Asp
1               5                   10                  15
Ile Gln Val Glu Ile Ala Leu Ala Met Gln Pro Leu Arg Ser Glu Asn

                    20                  25                  30
Ala Gln Leu Arg Arg Gln Leu Arg Ile Leu Asn Gln Gln Leu Arg Glu
            35                  40                  45
Gln Gln Lys Thr Gln Lys Pro Ser Gly Ala Val Asp Cys Asn Leu Glu
        50                  55                  60
Leu Phe Ser Leu Gln Ser Leu Asn Met Ser Leu Gln Asn Gln Leu Glu
65                  70                  75                  80
Glu Ser Leu Lys Ser Gln Glu Leu Leu Gln Ser Lys Asn Glu Glu Leu
                85                  90                  95
Leu Lys Val Ile Glu Asn Gln Lys Asp Glu Asn Lys Lys Phe Ser Ser
            100                 105                 110
Ile Phe Lys Asp Lys Asp Gln Thr Ile Leu Glu Asn Lys Gln Gln Tyr
        115                 120                 125
Asp Ile Glu Ile Thr Arg Ile Lys Ile Glu Leu Glu Glu Ala Leu Val
    130                 135                 140
Asn Val Lys Ser Ser Gln Phe Lys Leu Glu Thr Ala Glu Lys Glu Asn
145                 150                 155                 160
Gln Ile Leu Gly Ile Thr Leu Arg Gln Arg Asp Ala Glu Val Thr Arg
                165                 170                 175
Leu Arg Glu Leu Thr Arg Thr Leu Gln Thr Ser Met Ala Lys Leu Leu
            180                 185                 190
Ser Asp Leu Ser Val Asp Ser Ala Arg Cys Lys Pro Gly Asn Asn Leu
        195                 200                 205
Thr Lys Ser Leu Leu Asn Ile His Asp Lys Gln Leu Gln His Asp Pro
    210                 215                 220
Ala Pro Ala His Thr Ser Ile Met Ser Tyr Leu Asn Lys Leu Glu Thr
225                 230                 235                 240
Asn Tyr Ser Phe Thr His Ser Glu Pro Leu Ser Thr Ile Lys Asn Glu
                245                 250                 255
Glu Thr Ile Glu Pro Asp Lys Thr Tyr Glu Asn Val Leu Ser Ser Arg
            260                 265                 270
Gly Pro Gln Asn Ser Asn Thr Arg Gly Met Glu Glu Ala Ser Ala Pro
        275                 280                 285
Gly Ile Ile Ser Ala Leu Ser Lys Gln Asp Ser Asp Glu Gly Ser Glu
    290                 295                 300
Thr Met Ala Leu Ile Glu Asp Glu His Asn Leu Asp Asn Thr Ile Tyr
305                 310                 315                 320
Ile Pro Phe Ala Arg Ser Thr Pro Glu Lys Lys Ser Pro Leu Ser Lys
                325                 330                 335
Arg Leu Ser Pro Gln Pro Gln Ile Arg Ala Ala Thr Thr Gln Leu Val
            340                 345                 350
Ser Asn Ser Gly Leu Ala Val Ser Gly Lys Glu Asn Lys Leu Cys Thr
        355                 360                 365
Pro Val Ile Cys Ser Ser Ser Thr Lys Glu Ala Glu Asp Ala Pro Glu
    370                 375                 380
Lys Leu Ser Arg Ala Ser Asp Met Lys Asp Thr Gln Leu Leu Lys Lys
385                 390                 395                 400
Ile Lys Glu Ala Ile Gly Lys Ile Pro Ala Ala Thr Lys Glu Pro Glu
                405                 410                 415
Glu Gln Thr Ala Cys His Gly Pro Ser Gly Cys Leu Ser Asn Ser Leu
            420                 425                 430
Gln Val Lys Gly Asn Thr Val Cys Asp Gly Ser Val Phe Thr Ser Asp
        435                 440                 445
Leu Met Ser Asp Trp Ser Ile Ser Ser Phe Ser Thr Phe Thr Ser Arg
    450                 455                 460
Asp Glu Gln Asp Phe Arg Asn Gly Leu Ala Ala Leu Asp Ala Asn Ile
465                 470                 475                 480
Ala Arg Leu Gln Lys Ser Leu Arg Thr Gly Leu Leu Glu Lys
                485                 490

<210> 167
<211> 259
<212> PRT
<213> Homo sapiens

<400> 167
Met Asn Lys Leu Lys Ser Ser Gln Lys Asp Lys Val Arg Gln Phe Met
1               5                   10                  15
Ile Phe Thr Gln Ser Ser Glu Lys Thr Ala Val Ser Cys Leu Ser Gln
            20                  25                  30
Asn Asp Trp Lys Leu Asp Val Ala Thr Asp Asn Phe Phe Gln Asn Pro
        35                  40                  45
Glu Leu Tyr Ile Arg Glu Ser Val Lys Gly Ser Leu Asp Arg Lys Lys
    50                  55                  60
Leu Glu Gln Leu Tyr Asn Arg Tyr Lys Asp Pro Gln Asp Glu Asn Lys
65                  70                  75                  80
Ile Gly Ile Asp Gly Ile Gln Gln Phe Cys Asp Asp Leu Ala Leu Asp
                85                  90                  95
Pro Ala Ser Ile Ser Val Leu Ile Ile Ala Trp Lys Phe Arg Ala Ala
            100                 105                 110
Thr Gln Cys Glu Phe Ser Lys Gln Glu Phe Met Asp Gly Met Thr Glu
        115                 120                 125
Leu Gly Cys Asp Ser Ile Glu Lys Leu Lys Ala Gln Ile Pro Lys Met
    130                 135                 140
Glu Gln Glu Leu Lys Glu Pro Gly Arg Phe Lys Asp Phe Tyr Gln Phe
145                 150                 155                 160
Thr Phe Asn Phe Ala Lys Asn Pro Gly Gln Lys Gly Leu Asp Leu Glu
                165                 170                 175
Met Ala Ile Ala Tyr Trp Asn Leu Val Leu Asn Gly Arg Phe Lys Phe
            180                 185                 190
Leu Asp Leu Trp Asn Lys Phe Leu Leu Glu His His Lys Arg Ser Ile
        195                 200                 205
Pro Lys Asp Thr Trp Asn Leu Leu Leu Asp Phe Ser Thr Met Ile Ala
    210                 215                 220
Asp Asp Met Ser Asn Tyr Asp Glu Glu Gly Ala Trp Pro Val Leu Ile
225                 230                 235                 240
Asp Asp Phe Val Glu Phe Ala Arg Pro Gln Ile Ala Gly Thr Lys Ser
                245                 250                 255
Thr Thr Val

<210> 168
<211> 193
<212> PRT
<213> Homo sapiens

<400> 168
Met Ala Gly Lys Lys Val Leu Ile Val Tyr Ala His Gln Glu Pro Lys
1               5                   10                  15
Ser Phe Asn Gly Ser Leu Lys Asn Val Ala Val Asp Glu Leu Ser Arg
            20                  25                  30
Gln Gly Cys Thr Val Thr Val Ser Asp Leu Tyr Ala Met Asn Leu Glu
        35                  40                  45
Pro Arg Ala Thr Asp Lys Asp Ile Thr Gly Thr Leu Ser Asn Pro Glu

50                  55                  60
Val Phe Asn Tyr Gly Val Glu Thr His Glu Ala Tyr Lys Gln Arg Ser
65                  70                  75                  80
Leu Ala Ser Asp Ile Thr Asp Glu Gln Lys Lys Val Arg Glu Ala Asp
                85                  90                  95
Leu Val Ile Phe Gln Gly Lys Leu Ala Leu Leu Ser Val Thr Thr Gly
            100                 105                 110
Gly Thr Ala Glu Met Tyr Thr Lys Thr Gly Val Asn Gly Asp Ser Arg
        115                 120                 125
Tyr Phe Leu Trp Pro Leu Gln His Gly Thr Leu His Phe Cys Gly Phe
    130                 135                 140
Lys Val Leu Ala Pro Gln Ile Ser Phe Ala Pro Glu Ile Ala Ser Glu
145                 150                 155                 160
Glu Glu Arg Lys Gly Met Val Ala Ala Trp Ser Gln Arg Leu Gln Thr
                165                 170                 175
Ile Trp Lys Glu Glu Pro Ile Pro Cys Thr Ala His Trp His Phe Gly
            180                 185                 190
Gln

<210> 169
<211> 392
<212> PRT
<213> Homo sapiens

<400> 169
Met Asp Ala Leu Val Glu Asp Asp Ile Cys Ile Leu Asn His Glu Lys
1               5                   10                  15
Ala His Lys Arg Asp Thr Val Thr Pro Val Ser Ile Tyr Ser Gly Asp
            20                  25                  30
Glu Ser Val Ala Ser His Phe Ala Leu Val Thr Ala Tyr Glu Asp Ile
        35                  40                  45
Lys Lys Arg Leu Lys Asp Ser Glu Lys Glu Asn Ser Leu Leu Lys Lys
    50                  55                  60
Arg Ile Arg Phe Leu Glu Glu Lys Leu Ile Ala Arg Phe Glu Glu Glu
65                  70                  75                  80
Thr Ser Ser Val Gly Arg Glu Gln Val Asn Lys Ala Tyr His Ala Tyr
                85                  90                  95
Arg Glu Val Cys Ile Asp Arg Asp Asn Leu Lys Ser Lys Leu Asp Lys
            100                 105                 110
Met Asn Lys Asp Asn Ser Glu Ser Leu Lys Val Leu Asn Glu Gln Leu
        115                 120                 125
Gln Ser Lys Glu Val Glu Leu Leu Gln Leu Arg Thr Glu Val Glu Thr
    130                 135                 140
Gln Gln Val Met Arg Asn Leu Asn Pro Pro Ser Ser Asn Trp Glu Val
145                 150                 155                 160
Glu Lys Leu Ser Cys Asp Leu Lys Ile His Gly Leu Glu Gln Glu Leu
                165                 170                 175
Glu Leu Met Arg Lys Glu Cys Ser Asp Leu Lys Ile Glu Leu Gln Lys
            180                 185                 190
Ala Lys Gln Thr Asp Pro Tyr Gln Glu Asp Asn Leu Lys Ser Arg Asp
        195                 200                 205
Leu Gln Lys Leu Ser Ile Ser Ser Asp Asn Met Gln His Ala Tyr Trp
    210                 215                 220
Glu Leu Lys Arg Glu Met Ser Asn Leu His Leu Val Thr Gln Val Gln
225                 230                 235                 240
Ala Glu Leu Leu Arg Lys Leu Lys Thr Ser Thr Ala Ile Lys Lys Ala

245                 250                 255
Cys Ala Pro Val Gly Cys Ser Glu Asp Leu Gly Arg Asp Ser Thr Lys
            260                 265                 270
Leu His Leu Met Asn Phe Thr Ala Thr Tyr Thr Arg His Pro Pro Leu
        275                 280                 285
Leu Pro Asn Gly Lys Ala Leu Cys His Thr Thr Ser Ser Pro Leu Pro
    290                 295                 300
Gly Asp Val Lys Val Leu Ser Glu Lys Ala Ile Leu Gln Ser Trp Thr
305                 310                 315                 320
Asp Asn Glu Arg Ser Ile Pro Asn Asp Gly Thr Cys Phe Gln Glu His
                325                 330                 335
Ser Ser Tyr Gly Arg Asn Ser Leu Glu Asp Asn Ser Trp Val Phe Pro
            340                 345                 350
Ser Pro Pro Lys Ser Ser Glu Thr Ala Phe Gly Glu Thr Lys Thr Lys
        355                 360                 365
Thr Leu Pro Leu Pro Asn Leu Pro Pro Leu His Tyr Leu Asp Gln His
    370                 375                 380
Asn Gln Asn Cys Leu Tyr Lys Asn
385                 390

<210> 170
<211> 188
<212> PRT
<213> Homo sapiens

<400> 170
Met Phe His Gln Ile Trp Ala Ala Leu Leu Tyr Phe Tyr Gly Ile Ile
1               5                   10                  15
Leu Asn Ser Ile Tyr Gln Cys Pro Glu His Ser Gln Leu Thr Thr Leu
            20                  25                  30
Gly Val Asp Gly Lys Glu Phe Pro Glu Val His Leu Gly Gln Trp Tyr
        35                  40                  45
Phe Ile Ala Gly Ala Ala Pro Thr Lys Glu Glu Leu Ala Thr Phe Asp
    50                  55                  60
Pro Val Asp Asn Ile Val Phe Asn Met Ala Ala Gly Ser Ala Pro Met
65                  70                  75                  80
Gln Leu His Leu Arg Ala Thr Ile Arg Met Lys Asp Gly Leu Cys Val
                85                  90                  95
Pro Arg Lys Trp Ile Tyr His Leu Thr Glu Gly Ser Thr Asp Leu Arg
            100                 105                 110
Thr Glu Gly Arg Pro Asp Met Lys Thr Glu Leu Phe Ser Ser Ser Cys
        115                 120                 125
Pro Gly Gly Ile Met Leu Asn Glu Thr Gly Gln Gly Tyr Gln Arg Phe
    130                 135                 140
Leu Leu Tyr Asn Arg Ser Pro His Pro Pro Glu Lys Cys Val Glu Glu
145                 150                 155                 160
Phe Lys Ser Leu Thr Ser Cys Leu Asp Ser Lys Ala Phe Leu Leu Thr
                165                 170                 175
Pro Arg Asn Gln Glu Ala Cys Glu Leu Ser Asn Asn
            180                 185

<210> 171
<211> 537
<212> PRT
<213> Homo sapiens

<400> 171
Met Ala Ser Ala Leu Ser Tyr Val Ser Lys Phe Lys Ser Phe Val Ile
1               5                   10                  15
Leu Phe Val Thr Pro Leu Leu Leu Leu Pro Leu Val Ile Leu Met Pro
            20                  25                  30
Ala Lys Phe Val Arg Cys Ala Tyr Val Ile Ile Leu Met Ala Ile Tyr
        35                  40                  45
Trp Cys Thr Glu Val Ile Pro Leu Ala Val Thr Ser Leu Met Pro Val
    50                  55                  60
Leu Leu Phe Pro Leu Phe Gln Ile Leu Asp Ser Arg Gln Val Cys Val
65                  70                  75                  80
Gln Tyr Met Lys Asp Thr Asn Met Leu Phe Leu Gly Gly Leu Ile Val
                85                  90                  95
Ala Val Ala Val Glu Arg Trp Asn Leu His Lys Arg Ile Ala Leu Arg
            100                 105                 110
Thr Leu Leu Trp Val Gly Ala Lys Pro Ala Arg Leu Met Leu Gly Phe
        115                 120                 125
Met Gly Val Thr Ala Leu Leu Ser Met Trp Ile Ser Asn Thr Ala Thr
    130                 135                 140
Thr Ala Met Met Val Pro Ile Val Glu Ala Ile Leu Gln Gln Met Glu
145                 150                 155                 160
Ala Thr Ser Ala Ala Thr Glu Ala Gly Leu Glu Leu Val Asp Lys Gly
                165                 170                 175
Lys Ala Lys Glu Leu Pro Gly Ser Gln Val Ile Phe Glu Gly Pro Thr
            180                 185                 190
Leu Gly Gln Gln Glu Asp Gln Glu Arg Lys Arg Leu Cys Lys Ala Met
        195                 200                 205
Thr Leu Cys Ile Cys Tyr Ala Ala Ser Ile Gly Gly Thr Ala Thr Leu
    210                 215                 220
Thr Gly Thr Gly Pro Asn Val Val Leu Leu Gly Gln Met Asn Glu Leu
225                 230                 235                 240
Phe Pro Asp Ser Lys Asp Leu Val Asn Phe Ala Ser Trp Phe Ala Phe
                245                 250                 255
Ala Phe Pro Asn Met Leu Val Met Leu Leu Phe Ala Trp Leu Trp Leu
            260                 265                 270
Gln Phe Val Tyr Met Arg Phe Asn Phe Lys Lys Ser Trp Gly Cys Gly
        275                 280                 285
Leu Glu Ser Lys Lys Asn Glu Lys Ala Ala Leu Lys Val Leu Gln Glu
    290                 295                 300
Glu Tyr Arg Lys Leu Gly Pro Leu Ser Phe Ala Glu Ile Asn Val Leu
305                 310                 315                 320
Ile Cys Phe Phe Leu Leu Val Ile Leu Trp Phe Ser Arg Asp Pro Gly
                325                 330                 335
Phe Met Pro Gly Trp Leu Thr Val Ala Trp Val Glu Gly Glu Thr Lys
            340                 345                 350
Tyr Val Ser Asp Ala Thr Val Ala Ile Phe Val Ala Thr Leu Leu Phe
        355                 360                 365
Ile Val Pro Ser Gln Lys Pro Lys Phe Asn Phe Arg Ser Gln Thr Glu
    370                 375                 380
Glu Glu Arg Lys Thr Pro Phe Tyr Pro Pro Pro Leu Leu Asp Trp Lys
385                 390                 395                 400
Val Thr Gln Glu Lys Val Pro Trp Gly Ile Val Leu Leu Leu Gly Gly
                405                 410                 415
Gly Phe Ala Leu Ala Lys Gly Ser Glu Ala Ser Gly Leu Ser Val Trp
            420                 425                 430
Met Gly Lys Gln Met Glu Pro Leu His Ala Val Pro Pro Ala Ala Ile
        435                 440                 445
Thr Leu Ile Leu Ser Leu Leu Val Ala Val Phe Thr Glu Cys Thr Ser

450                 455                 460
Asn Val Ala Thr Thr Thr Leu Phe Leu Pro Ile Phe Ala Ser Met Ser
465                 470                 475                 480
Arg Ser Ile Gly Leu Asn Pro Leu Tyr Ile Met Leu Pro Cys Thr Leu
                485                 490                 495
Ser Ala Ser Phe Ala Phe Met Leu Pro Val Ala Thr Pro Pro Asn Ala
            500                 505                 510
Ile Val Phe Thr Tyr Gly His Leu Lys Val Ala Asp Met Val Thr Gln
        515                 520                 525
Leu Phe Leu Phe Thr Pro Val Gly Leu
    530                 535

<210> 172
<211> 567
<212> PRT
<213> Homo sapiens

<400> 172
Met Glu Glu Lys Thr Gln Ile Lys Thr Phe Leu Gly Ser Lys Leu Pro
1               5                   10                  15
Lys Tyr Gly Thr Lys Ser Val Arg Ser Thr Leu Gln Pro Met Pro Asn
            20                  25                  30
Gly Thr Pro Val Asn Leu Leu Gly Thr Ser Lys Asn Ser Asn Val Lys
        35                  40                  45
Ser Tyr Ile Lys Asn Asn Gly Ser Asp Cys Pro Ser Ser His Ser Phe
    50                  55                  60
Asn Trp Arg Lys Ala Asn Lys Tyr Gln Leu Cys Ala Gln Gly Val Glu
65                  70                  75                  80
Glu Pro Asn Asn Thr Gln Asn Ser His Asp Lys Ile Ile Asp Pro Glu
                85                  90                  95
Lys Arg Val Pro Thr Gln Gly Met Phe Asp Lys Asn Gly Ile Lys Gly
            100                 105                 110
Gly Leu Lys Ser Val Ser Leu Phe Thr Ser Lys Leu Ala Lys Pro Ser
        115                 120                 125
Thr Met Phe Val Ser Ser Thr Glu Glu Leu Asn Gln Lys Ser Phe Ser
    130                 135                 140
Gly Pro Ser Asn Leu Gly Lys Phe Thr Lys Gly Thr Leu Leu Gly Arg
145                 150                 155                 160
Thr Ser Tyr Ser Ser Ile Asn Thr Pro Lys Ser Gln Leu Asn Gly Phe
                165                 170                 175
Tyr Gly Asn Arg Ser Ala Gly Ser Met Gln Arg Pro Arg Ala Asn Ser
            180                 185                 190
Cys Ala Thr Arg Ser Ser Ser Gly Glu Ser Leu Ala Gln Ser Pro Asp
        195                 200                 205
Ser Ser Lys Ser Ile Asn Cys Glu Lys Met Val Arg Ser Gln Ser Phe
    210                 215                 220
Ser His Ser Ile Gln Asn Ser Phe Leu Pro Pro Ser Ser Ile Thr Arg
225                 230                 235                 240
Ser His Ser Phe Asn Arg Ala Val Asp Leu Thr Lys Pro Tyr Gln Asn
                245                 250                 255
Gln Gln Leu Ser Ile Arg Val Pro Leu Arg Ser Ser Met Leu Thr Arg
            260                 265                 270
Asn Ser Arg Gln Pro Glu Val Leu Asn Gly Asn Glu His Leu Gly Tyr
        275                 280                 285
Gly Phe Asn Arg Pro Tyr Ala Ala Gly Gly Lys Lys Leu Ala Leu Pro
    290                 295                 300
Asn Gly Pro Gly Val Thr Ser Thr Leu Gly Tyr Arg Met Val His Pro

305                 310                 315                 320
Ser Leu Leu Lys Ser Ser Arg Ser Pro Phe Ser Gly Thr Met Thr Val
                325                 330                 335
Asp Gly Asn Lys Asn Ser Pro Ala Asp Thr Cys Val Glu Glu Asp Ala
            340                 345                 350
Thr Val Leu Ala Lys Asp Arg Ala Ala Asn Lys Asp Gln Glu Leu Ile
        355                 360                 365
Glu Asn Glu Ser Tyr Arg Thr Lys Asn Asn Gln Thr Met Lys His Asp
    370                 375                 380
Ala Lys Met Arg Tyr Leu Ser Asp Asp Val Asp Asp Ile Ser Leu Ser
385                 390                 395                 400
Ser Leu Ser Ser Ser Asp Lys Asn Asp Leu Ser Glu Asp Phe Ser Asp
                405                 410                 415
Asp Phe Ile Asp Ile Glu Asp Ser Asn Arg Thr Arg Ile Thr Pro Glu
            420                 425                 430
Glu Met Ser Leu Lys Glu Glu Lys His Glu Asn Gly Pro Pro Gln Asp
        435                 440                 445
Met Phe Asp Ser Pro Lys Glu Asn Glu Lys Ala Phe Ser Lys Thr Asp
    450                 455                 460
Glu Trp Ile Asp Ile Ser Val Ser Asp Arg Ser Glu Cys Thr Lys His
465                 470                 475                 480
Thr Ser Gly Asn Asn Leu Val Ser Pro Asp Thr Asp Tyr Arg Ala Gly
                485                 490                 495
Ser Ser Phe Glu Leu Ser Pro Ser Asp Ser Ser Asp Gly Thr Tyr Met
            500                 505                 510
Trp Asp Glu Glu Gly Leu Glu Pro Ile Gly Asn Val His Pro Val Gly
        515                 520                 525
Ser Tyr Glu Ser Ser Glu Met Asn Ser Ile Val Cys Met Asp Leu Tyr
    530                 535                 540
Thr Leu Gly Ile Phe Cys Leu Pro Tyr Tyr Arg Glu Thr Cys Asp Met
545                 550                 555                 560
Ile Asp Phe Val Lys Asn Leu
                565

<210> 173
<211> 159
<212> PRT
<213> Homo sapiens

<400> 173
Met Pro Lys Phe Tyr Cys Asp Tyr Cys Asp Thr Tyr Leu Thr His Asp
1               5                   10                  15
Ser Pro Ser Val Arg Lys Thr His Cys Ser Gly Arg Lys His Lys Glu
            20                  25                  30
Asn Val Lys Asp Tyr Tyr Gln Lys Trp Met Glu Glu Gln Ala Gln Ser
        35                  40                  45
Leu Ile Asp Lys Thr Thr Ala Ala Phe Gln Gln Gly Lys Ile Pro Pro
    50                  55                  60
Thr Pro Phe Ser Ala Pro Pro Pro Ala Gly Ala Met Ile Pro Pro Pro
65                  70                  75                  80
Pro Ser Leu Pro Gly Pro Pro Arg Pro Gly Met Met Pro Ala Pro His
                85                  90                  95
Met Gly Gly Pro Pro Met Met Pro Met Met Gly Pro Pro Pro Pro Gly
            100                 105                 110
Met Met Pro Val Gly Pro Ala Pro Gly Met Arg Pro Pro Met Gly Gly
        115                 120                 125
His Met Pro Met Met Pro Gly Pro Pro Met Met Arg Pro Pro Ala Arg

130                 135                 140
Pro Met Met Val Pro Thr Arg Pro Gly Met Thr Arg Pro Asp Arg
145                 150                 155

<210> 174
<211> 906
<212> PRT
<213> Homo sapiens

<400> 174
Met Pro Leu Arg Leu Asp Ile Lys Arg Lys Leu Thr Ala Arg Ser Asp
1               5                   10                  15
Arg Val Lys Ser Val Asp Leu His Pro Thr Glu Pro Trp Met Leu Ala
            20                  25                  30
Ser Leu Tyr Asn Gly Ser Val Cys Val Trp Asn His Glu Thr Gln Thr
        35                  40                  45
Leu Val Lys Thr Phe Glu Val Cys Asp Leu Pro Val Arg Ala Ala Lys
    50                  55                  60
Phe Val Ala Arg Lys Asn Trp Val Val Thr Gly Ala Asp Asp Met Gln
65                  70                  75                  80
Ile Arg Val Phe Asn Tyr Asn Thr Leu Glu Arg Val His Met Phe Glu
                85                  90                  95
Ala His Ser Asp Tyr Ile Arg Cys Ile Ala Val His Pro Thr Gln Pro
            100                 105                 110
Phe Ile Leu Thr Ser Ser Asp Asp Met Leu Ile Lys Leu Trp Asp Trp
        115                 120                 125
Asp Lys Lys Trp Ser Cys Ser Gln Val Phe Glu Gly His Thr His Tyr
    130                 135                 140
Val Met Gln Ile Val Ile Asn Pro Lys Asp Asn Asn Gln Phe Ala Ser
145                 150                 155                 160
Ala Ser Leu Asp Arg Thr Ile Lys Val Trp Gln Leu Gly Ser Ser Ser
                165                 170                 175
Pro Asn Phe Thr Leu Glu Gly His Glu Lys Gly Val Asn Cys Ile Asp
            180                 185                 190
Tyr Tyr Ser Gly Gly Asp Lys Pro Tyr Leu Ile Ser Gly Ala Asp Asp
        195                 200                 205
Arg Leu Val Lys Ile Trp Asp Tyr Gln Asn Lys Thr Cys Val Gln Thr
    210                 215                 220
Leu Glu Gly His Ala Gln Asn Val Ser Cys Ala Ser Phe His Pro Glu
225                 230                 235                 240
Leu Pro Ile Ile Ile Thr Gly Ser Glu Asp Gly Thr Val Arg Ile Trp
                245                 250                 255
His Ser Ser Thr Tyr Arg Leu Glu Ser Thr Leu Asn Tyr Gly Met Glu
            260                 265                 270
Arg Val Trp Cys Val Ala Ser Leu Arg Gly Ser Asn Asn Val Ala Leu
        275                 280                 285
Gly Tyr Asp Glu Gly Ser Ile Ile Val Lys Leu Gly Arg Glu Glu Pro
    290                 295                 300
Ala Met Ser Met Asp Ala Asn Gly Lys Ile Ile Trp Ala Lys His Ser
305                 310                 315                 320
Glu Val Gln Gln Ala Asn Leu Lys Ala Met Gly Asp Ala Glu Ile Lys
                325                 330                 335
Asp Gly Glu Arg Leu Pro Leu Ala Val Lys Asp Met Gly Ser Cys Glu
            340                 345                 350
Ile Tyr Pro Gln Thr Ile Gln His Asn Pro Asn Gly Arg Phe Val Val
        355                 360                 365
Val Cys Gly Asp Gly Glu Tyr Ile Ile Tyr Thr Ala Met Ala Leu Arg

    370                 375                 380
Asn Lys Ser Phe Gly Ser Ala Gln Glu Phe Ala Trp Ala His Asp Ser
385                 390                 395                 400
Ser Glu Tyr Ala Ile Arg Glu Ser Asn Ser Ile Val Lys Ile Phe Lys
                405                 410                 415
Asn Phe Lys Glu Lys Lys Ser Phe Lys Pro Asp Phe Gly Ala Glu Ser
            420                 425                 430
Ile Tyr Gly Gly Phe Leu Leu Gly Val Arg Ser Val Asn Gly Leu Ala
        435                 440                 445
Phe Tyr Asp Trp Asp Asn Thr Glu Leu Ile Arg Arg Ile Glu Ile Gln
    450                 455                 460
Pro Lys His Ile Phe Trp Ser Asp Ser Gly Glu Leu Val Cys Ile Ala
465                 470                 475                 480
Thr Glu Glu Ser Phe Phe Ile Leu Lys Tyr Leu Ser Glu Lys Val Leu
                485                 490                 495
Ala Ala Gln Glu Thr His Glu Gly Val Thr Glu Asp Gly Ile Glu Asp
            500                 505                 510
Ala Phe Glu Val Leu Gly Glu Ile Gln Glu Ile Val Lys Thr Gly Leu
        515                 520                 525
Trp Val Gly Asp Cys Phe Ile Tyr Thr Ser Ser Val Asn Arg Leu Asn
    530                 535                 540
Tyr Tyr Val Gly Gly Glu Ile Val Thr Ile Ala His Leu Asp Arg Thr
545                 550                 555                 560
Met Tyr Leu Leu Gly Tyr Ile Pro Lys Asp Asn Arg Leu Tyr Leu Gly
                565                 570                 575
Asp Lys Glu Leu Asn Ile Ile Ser Tyr Ser Leu Leu Val Ser Val Leu
            580                 585                 590
Glu Tyr Gln Thr Ala Val Met Arg Arg Asp Phe Ser Met Ala Asp Lys
        595                 600                 605
Val Leu Pro Thr Ile Pro Lys Glu Gln Arg Thr Arg Val Ala His Phe
    610                 615                 620
Leu Glu Lys Gln Gly Phe Lys Gln Gln Ala Leu Thr Val Ser Thr Asp
625                 630                 635                 640
Pro Glu His Arg Phe Glu Leu Ala Leu Gln Leu Gly Glu Leu Lys Ile
                645                 650                 655
Ala Tyr Gln Leu Ala Val Glu Ala Glu Ser Glu Gln Lys Trp Lys Gln
            660                 665                 670
Leu Ala Glu Leu Ala Ile Ser Lys Cys Gln Phe Gly Leu Ala Gln Glu
        675                 680                 685
Cys Leu His His Ala Gln Asp Tyr Gly Gly Leu Leu Leu Leu Ala Thr
    690                 695                 700
Ala Ser Gly Asn Ala Asn Met Val Asn Lys Leu Ala Glu Gly Ala Glu
705                 710                 715                 720
Arg Asp Gly Lys Asn Asn Val Ala Phe Met Ser Tyr Phe Leu Gln Gly
                725                 730                 735
Lys Val Asp Ala Cys Leu Glu Leu Leu Ile Arg Thr Gly Arg Leu Pro
            740                 745                 750
Glu Ala Ala Phe Leu Ala Arg Thr Tyr Leu Pro Ser Gln Val Ser Arg
        755                 760                 765
Val Val Lys Leu Trp Arg Glu Asn Leu Ser Lys Val Asn Gln Lys Ala
    770                 775                 780
Ala Glu Ser Leu Ala Asp Pro Thr Glu Tyr Glu Asn Leu Phe Pro Gly
785                 790                 795                 800
Leu Lys Glu Ala Phe Val Val Glu Glu Trp Val Lys Glu Thr His Ala
                805                 810                 815
Asp Leu Trp Pro Ala Lys Gln Tyr Pro Leu Val Thr Pro Asn Glu Glu
            820                 825                 830
Arg Asn Val Met Glu Glu Gly Lys Asp Phe Gln Pro Ser Arg Ser Thr
        835                 840                 845
Ala Gln Gln Glu Leu Asp Gly Lys Pro Ala Ser Pro Thr Pro Val Ile

850                 855                 860
Val Ala Ser His Thr Ala Asn Lys Glu Glu Lys Ser Leu Leu Glu Leu
865                 870                 875                 880
Glu Val Asp Leu Asp Asn Leu Glu Leu Glu Asp Ile Asp Thr Thr Asp
                885                 890                 895
Ile Asn Leu Asp Glu Asp Ile Leu Asp Asp
            900                 905

<210> 175
<211> 883
<212> PRT
<213> Homo sapiens

<400> 175

<210> 176
<211> 65
<212> PRT
<213> Homo sapiens

<400> 176
Met His Thr Ser Thr Ser Ser Ser Val Thr Lys Ser Tyr Ile Ser Ser
1               5                   10                  15
Gln Thr Asn Gly Glu Thr Gly Gln Leu Val His Arg Phe Thr Val Pro
            20                  25                  30
Ala Pro Val Val Ile Ile Leu Ile Ile Leu Cys Val Met Ala Gly Ile
        35                  40                  45
Ile Gly Thr Ile Leu Leu Ile Ser Tyr Ser Ile Arg Arg Leu Ile Lys
    50                  55                  60
Ala
65

<210> 177
<211> 219
<212> PRT
<213> Homo sapiens

<400> 177
Met Pro Arg Gly Arg Lys Ser Arg Arg Arg Arg Asn Ala Arg Ala Ala
1               5                   10                  15
Glu Glu Asn Arg Asn Asn Arg Lys Ile Gln Ala Ser Glu Ala Ser Glu
            20                  25                  30
Thr Pro Met Ala Ala Ser Val Val Ala Ser Thr Pro Glu Asp Asp Leu
        35                  40                  45
Ser Gly Pro Glu Glu Asp Pro Ser Thr Pro Glu Glu Ala Ser Thr Thr
    50                  55                  60
Pro Glu Glu Ala Ser Ser Thr Ala Gln Ala Gln Lys Pro Ser Val Pro
65                  70                  75                  80
Arg Ser Asn Phe Gln Gly Thr Lys Lys Ser Leu Leu Met Ser Ile Leu
                85                  90                  95
Ala Leu Ile Phe Ile Met Gly Asn Ser Ala Lys Glu Ala Leu Val Trp
            100                 105                 110
Lys Val Leu Gly Lys Leu Gly Met Gln Pro Gly Arg Gln His Ser Ile
        115                 120                 125
Phe Gly Asp Pro Lys Lys Ile Val Thr Glu Glu Phe Val Arg Arg Gly
    130                 135                 140
Tyr Leu Ile Tyr Lys Pro Val Pro Arg Ser Ser Pro Val Glu Tyr Glu
145                 150                 155                 160
Phe Phe Trp Gly Pro Arg Ala His Val Glu Ser Ser Lys Leu Lys Val

                165                 170                 175
Met His Phe Val Ala Arg Val Arg Asn Arg Cys Ser Lys Asp Trp Pro
            180                 185                 190
Cys Asn Tyr Asp Trp Asp Ser Asp Asp Asp Ala Glu Val Glu Ala Ile
        195                 200                 205
Leu Asn Ser Gly Ala Arg Gly Tyr Ser Ala Pro
    210                 215

<210> 178
<211> 125
<212> PRT
<213> Homo sapiens

<400> 178
Met Gly Leu Ala Gly Val Cys Ala Leu Arg Arg Ser Ala Gly Tyr Ile
1               5                   10                  15
Leu Val Gly Gly Ala Gly Gly Gln Ser Ala Ala Ala Ala Ala Arg Arg
            20                  25                  30
Cys Ser Glu Gly Glu Trp Ala Ser Gly Gly Val Arg Ser Phe Ser Arg
        35                  40                  45
Ala Ala Ala Ala Met Ala Pro Ile Lys Val Arg Leu Leu Ala Asp Pro
    50                  55                  60
Thr Gly Ala Phe Gly Lys Glu Thr Asp Leu Leu Leu Asp Asp Ser Leu
65                  70                  75                  80
Val Ser Ile Phe Gly Asn Arg Arg Leu Lys Arg Phe Ser Met Val Val
                85                  90                  95
Gln Asp Gly Ile Val Lys Ala Leu Asn Val Glu Pro Asp Gly Thr Gly
            100                 105                 110
Leu Thr Cys Ser Leu Ala Pro Asn Ile Ile Ser Gln Leu
        115                 120                 125

<210> 179
<211> 237
<212> PRT
<213> Homo sapiens

<400> 179
Met Ala Ala Ala Ser Gly Ser Val Leu Gln Arg Cys Ile Val Ser Pro
1               5                   10                  15
Ala Gly Arg His Ser Ala Ser Leu Ile Phe Leu His Gly Ser Gly Asp
            20                  25                  30
Ser Gly Gln Gly Leu Arg Met Trp Ile Lys Gln Val Leu Asn Gln Asp
        35                  40                  45
Leu Thr Phe Gln His Ile Lys Ile Ile Tyr Pro Thr Ala Pro Pro Arg
    50                  55                  60
Ser Tyr Thr Pro Met Lys Gly Gly Ile Ser Asn Val Trp Phe Asp Arg
65                  70                  75                  80
Phe Lys Ile Thr Asn Asp Cys Pro Glu His Leu Glu Ser Ile Asp Val
                85                  90                  95
Met Cys Gln Val Leu Thr Asp Leu Ile Asp Glu Glu Val Lys Ser Gly
            100                 105                 110
Ile Lys Lys Asn Arg Ile Leu Ile Gly Gly Phe Ser Met Gly Gly Cys
        115                 120                 125
Met Ala Met His Leu Ala Tyr Arg Asn His Gln Asp Val Ala Gly Val

130                 135                 140
Phe Ala Leu Ser Ser Phe Leu Asn Lys Ala Ser Ala Val Tyr Gln Ala
145                 150                 155                 160
Leu Gln Lys Ser Asn Gly Val Leu Pro Glu Leu Phe Gln Cys His Gly
                165                 170                 175
Thr Ala Asp Glu Leu Val Leu His Ser Trp Ala Glu Glu Thr Asn Ser
            180                 185                 190
Met Leu Lys Ser Leu Gly Val Thr Thr Lys Phe His Ser Phe Pro Asn
        195                 200                 205
Val Tyr His Glu Leu Ser Lys Thr Glu Leu Asp Ile Leu Lys Leu Trp
    210                 215                 220
Ile Leu Thr Lys Leu Pro Gly Glu Met Glu Lys Gln Lys
225                 230                 235

<210> 180
<211> 153
<212> PRT
<213> Homo sapiens

<400> 180
Met Asn Val Gly Val Ala His Ser Glu Val Asn Pro Asn Thr Arg Val
1               5                   10                  15
Met Asn Ser Arg Gly Met Trp Leu Thr Tyr Ala Leu Gly Val Gly Leu
            20                  25                  30
Leu His Ile Val Leu Leu Ser Ile Pro Phe Phe Ser Val Pro Val Ala
        35                  40                  45
Trp Thr Leu Thr Asn Ile Ile His Asn Leu Gly Met Tyr Val Phe Leu
    50                  55                  60
His Ala Val Lys Gly Thr Pro Phe Glu Thr Pro Asp Gln Gly Lys Ala
65                  70                  75                  80
Arg Leu Leu Thr His Trp Glu Gln Leu Asp Tyr Gly Val Gln Phe Thr
                85                  90                  95
Ser Ser Arg Lys Phe Phe Thr Ile Ser Pro Ile Ile Leu Tyr Phe Leu
            100                 105                 110
Ala Ser Phe Tyr Thr Lys Tyr Asp Pro Thr His Phe Ile Leu Asn Thr
        115                 120                 125
Ala Ser Leu Leu Ser Val Leu Ile Pro Lys Met Pro Gln Leu His Gly
    130                 135                 140
Val Arg Ile Phe Gly Ile Asn Lys Tyr
145                 150

<210> 181
<211> 105
<212> PRT
<213> Homo sapiens

<400> 181
Met Gly Glu Thr Glu Gly Lys Lys Asp Glu Ala Asp Tyr Lys Arg Leu
1               5                   10                  15
Gln Thr Phe Pro Leu Val Arg His Ser Asp Met Pro Glu Glu Met Arg
            20                  25                  30
Val Glu Thr Met Glu Leu Cys Val Thr Ala Cys Glu Lys Phe Ser Asn
        35                  40                  45
Asn Asn Glu Ser Ala Ala Lys Met Ile Lys Glu Thr Met Asp Lys Lys

50                  55                  60
Phe Gly Ser Ser Trp His Val Val Ile Gly Glu Gly Phe Gly Phe Glu
65                  70                  75                  80
Ile Thr His Glu Val Lys Asn Leu Leu Tyr Leu Tyr Phe Gly Gly Thr
                85                  90                  95
Leu Ala Val Cys Val Trp Lys Cys Ser
            100                 105

<210> 182
<211> 70
<212> PRT
<213> Homo sapiens

<400> 182
Met Ser Gly Pro Arg Pro Val Val Leu Ser Gly Pro Ser Gly Ala Gly
1               5                   10                  15
Lys Ser Thr Leu Leu Lys Arg Leu Leu Gln Glu His Ser Gly Ile Phe
            20                  25                  30
Gly Phe Ser Val Ser His Thr Arg Ala Leu Val Glu Gly Tyr His Glu
        35                  40                  45
Glu Pro Glu Ala Arg Arg Gly Glu Arg Gln Arg Leu Leu Leu Cys Asn
    50                  55                  60
Gln Gly Gly Asp Ala Ala
65                  70

<210> 183
<211> 117
<212> PRT
<213> Homo sapiens

<400> 183

<210> 184
<211> 471
<212> PRT
<213> Homo sapiens

<400> 184
Met Met Met Thr Phe Pro Ala Ser Tyr Pro Val Ser Lys Leu Leu Asp
1               5                   10                  15
Cys Val Leu Gly Gln Glu Ile Gly Thr Val Tyr Asn Arg Glu Lys Leu
            20                  25                  30
Leu Glu Met Leu Arg Val Thr Asp Pro Tyr Asn Asp Leu Val Lys Glu
        35                  40                  45
Glu Leu Asn Ile Ile Gln Gly Ala Leu Glu Leu Arg Thr Lys Thr Val
    50                  55                  60
Glu Asp Val Met Thr Pro Leu Arg Asp Cys Phe Met Ile Thr Gly Glu
65                  70                  75                  80
Ala Ile Leu Asp Phe Asn Thr Met Ser Glu Ile Met Glu Ser Gly Tyr
                85                  90                  95
Thr Arg Ile Pro Val Phe Glu Gly Glu Arg Ser Asn Ile Val Asp Leu
            100                 105                 110
Leu Phe Val Lys Asp Leu Ala Phe Val Asp Pro Asp Asp Cys Thr Pro
        115                 120                 125
Leu Lys Thr Ile Thr Lys Phe Tyr Asn His Pro Leu His Phe Val Phe
    130                 135                 140
Asn Asp Thr Lys Leu Asp Ala Met Leu Glu Glu Phe Lys Lys Gly Lys
145                 150                 155                 160
Ser His Leu Ala Ile Val Gln Arg Val Asn Asn Glu Gly Glu Gly Asp
                165                 170                 175
Pro Phe Tyr Glu Val Leu Gly Ile Val Thr Leu Glu Asp Val Ile Glu
            180                 185                 190
Glu Ile Ile Lys Ser Glu Ile Leu Asp Glu Thr Asp Leu Tyr Thr Asp
        195                 200                 205
Asn Arg Thr Lys Lys Lys Val Ala His Arg Glu Arg Lys Gln Asp Phe
    210                 215                 220
Ser Ala Phe Lys Gln Thr Asp Ser Glu Met Lys Val Lys Ile Ser Pro
225                 230                 235                 240
Gln Leu Leu Leu Ala Met His Arg Phe Leu Ala Thr Glu Val Glu Ala
                245                 250                 255
Phe Ser Pro Ser Gln Met Ser Glu Lys Ile Leu Leu Arg Leu Leu Lys
            260                 265                 270
His Pro Asn Val Ile Gln Glu Leu Lys Tyr Asp Glu Lys Asn Lys Lys
        275                 280                 285
Ala Pro Glu Tyr Tyr Leu Tyr Gln Arg Asn Lys Pro Val Asp Tyr Phe
    290                 295                 300
Val Leu Ile Leu Gln Gly Lys Val Glu Val Glu Ala Gly Lys Glu Gly
305                 310                 315                 320
Met Lys Phe Glu Ala Ser Ala Phe Ser Tyr Tyr Gly Val Met Ala Leu
                325                 330                 335
Thr Ala Ser Pro Gly Glu Asn Lys Ser Pro Pro Arg Pro Cys Gly Leu
            340                 345                 350
Asn His Ser Asp Ser Leu Ser Arg Ser Asp Arg Ile Asp Ala Val Thr
        355                 360                 365
Pro Thr Leu Gly Ser Ser Asn Asn Gln Leu Asn Ser Ser Leu Leu Gln
    370                 375                 380
Val Tyr Ile Pro Asp Tyr Ser Val Arg Ala Leu Ser Asp Leu Gln Phe
385                 390                 395                 400
Val Lys Ile Ser Arg Gln Gln Tyr Gln Asn Ala Leu Met Ala Ser Arg
                405                 410                 415
Met Asp Lys Thr Pro Gln Ser Ser Asp Ser Glu Asn Thr Lys Ile Glu
            420                 425                 430
Leu Thr Leu Thr Glu Leu His Asp Gly Leu Pro Asp Glu Thr Ala Asn
        435                 440                 445
Leu Leu Asn Glu Gln Asn Cys Val Thr His Ser Lys Ala Asn His Ser

450                 455                 460
Leu His Asn Glu Gly Ala Ile
465                 470

<210> 185
<211> 41
<212> PRT
<213> Homo sapiens

<400> 185
Met Arg Met Trp Pro Ala Cys Cys Leu Ile Leu Pro Arg Thr Val Cys
1               5                   10                  15
Thr Cys Cys Ser Leu Val Tyr Ala Asn Trp Leu His Leu Leu Phe Leu
            20                  25                  30
Cys Leu Cys Pro Tyr Pro Trp Leu Ser
        35                  40

<210> 186
<211> 100
<212> PRT
<213> Homo sapiens

<400> 186
Met Arg Ala Leu Thr Leu Leu Ala Leu Leu Ala Leu Ala Ala Leu Cys
1               5                   10                  15
Ile Ala Gly Gln Ala Gly Ala Lys Pro Ser Gly Ala Glu Ser Ser Lys
            20                  25                  30
Gly Ala Ala Phe Val Ser Lys Gln Glu Gly Ser Glu Val Val Lys Arg
        35                  40                  45
Pro Arg Arg Tyr Leu Tyr Gln Trp Leu Gly Ala Pro Val Pro Tyr Pro
    50                  55                  60
Asp Pro Leu Glu Pro Arg Arg Glu Val Cys Glu Leu Asn Pro Asp Cys
65                  70                  75                  80
Asp Glu Leu Ala Asp His Ile Gly Phe Gln Glu Ala Tyr Arg Arg Phe
                85                  90                  95
Tyr Gly Pro Val
            100

<210> 187
<211> 213
<212> PRT
<213> Homo sapiens

<400> 187
Met Ala Val Pro Ala Val Ser Gly Leu Ser Arg Gln Val Arg Cys Phe
1               5                   10                  15
Ser Thr Ser Val Val Arg Pro Phe Ala Lys Leu Val Arg Pro Pro Val
            20                  25                  30
Gln Val Tyr Gly Ile Glu Gly Arg Tyr Ala Thr Ala Leu Tyr Ser Ala
        35                  40                  45
Ala Ser Lys Gln Asn Lys Leu Glu Gln Val Glu Lys Glu Leu Leu Arg

50                  55                  60
Val Ala Gln Ile Leu Lys Glu Pro Lys Val Ala Ala Ser Val Leu Asn
65                  70                  75                  80
Pro Tyr Val Lys Arg Ser Ile Lys Val Lys Ser Leu Asn Asp Ile Thr
                85                  90                  95
Ala Lys Glu Arg Phe Ser Pro Leu Thr Thr Asn Leu Ile Asn Leu Leu
            100                 105                 110
Ala Glu Asn Gly Arg Leu Ser Asn Thr Gln Gly Val Val Ser Ala Phe
        115                 120                 125
Ser Thr Met Met Ser Val His Arg Gly Glu Val Pro Cys Thr Val Thr
    130                 135                 140
Ser Ala Ser Pro Leu Glu Glu Ala Thr Leu Ser Glu Leu Lys Thr Val
145                 150                 155                 160
Leu Lys Ser Phe Leu Ser Gln Gly Gln Val Leu Lys Leu Glu Ala Lys
                165                 170                 175
Thr Asp Pro Ser Ile Leu Gly Gly Met Ile Val Arg Ile Gly Glu Lys
            180                 185                 190
Tyr Val Asp Met Ser Val Lys Thr Lys Ile Gln Lys Leu Gly Arg Ala
        195                 200                 205
Met Arg Glu Ile Val
    210

<210> 188
<211> 196
<212> PRT
<213> Homo sapiens

<400> 188

<210> 189
<211> 49
<212> PRT
<213> Homo sapiens

<400> 189

<210> 190
<211> 204
<212> PRT
<213> Homo sapiens

<400> 190
Met Asp Arg Ser Ala Glu Phe Arg Lys Trp Lys Ala Gln Cys Leu Ser
1               5                   10                  15
Lys Ala Asp Leu Ser Arg Lys Gly Ser Val Asp Glu Asp Val Val Glu
            20                  25                  30
Leu Val Gln Phe Leu Asn Met Arg Asp Gln Phe Phe Thr Thr Ser Ser
        35                  40                  45
Cys Ala Gly Arg Ile Leu Leu Leu Asp Arg Gly Ile Asn Gly Phe Glu
    50                  55                  60
Val Gln Lys Gln Asn Cys Cys Trp Leu Leu Val Thr His Lys Leu Cys
65                  70                  75                  80
Val Lys Asp Asp Val Ile Val Ala Leu Lys Lys Ala Asn Gly Asp Ala
                85                  90                  95
Thr Leu Lys Phe Glu Pro Phe Val Leu His Val Gln Cys Arg Gln Leu
            100                 105                 110
Gln Asp Ala Gln Ile Leu His Ser Met Ala Ile Asp Ser Gly Phe Arg
        115                 120                 125
Asn Ser Gly Ile Thr Val Gly Lys Arg Gly Lys Thr Met Leu Ala Val
    130                 135                 140
Arg Ser Thr His Gly Leu Glu Val Pro Leu Ser His Lys Gly Lys Leu
145                 150                 155                 160
Met Val Thr Glu Glu Tyr Ile Asp Phe Leu Leu Asn Val Ala Asn Gln
                165                 170                 175
Lys Met Glu Glu Asn Lys Lys Arg Ile Glu Arg Asp Gly Val Leu Pro
            180                 185                 190
Cys Cys Leu Gly Trp Ser Gln Thr Pro Gly Leu Lys
        195                 200

<210> 191
<211> 104
<212> PRT
<213> Homo sapiens

<400> 191
Met Gly Gln Cys Arg Ser Ala Asn Ala Glu Asp Ala Gln Glu Phe Ser
1               5                   10                  15
Asp Val Glu Arg Ala Ile Glu Thr Leu Ile Lys Asn Phe His Gln Tyr
            20                  25                  30
Ser Val Glu Gly Gly Lys Glu Thr Leu Thr Pro Ser Glu Leu Arg Asp
        35                  40                  45
Leu Val Thr Gln Gln Leu Pro His Leu Met Pro Ser Asn Cys Gly Leu
    50                  55                  60
Glu Glu Lys Ile Ala Asn Leu Gly Ser Cys Asn Asp Ser Lys Leu Glu
65                  70                  75                  80
Phe Arg Ser Phe Trp Glu Leu Ile Gly Glu Ala Ala Lys Ser Val Lys
                85                  90                  95
Leu Glu Arg Pro Val Arg Gly His
            100

<210> 192
<211> 105
<212> PRT
<213> Homo sapiens

<400> 192
Met Gln Thr Gln Ala Glu Ala Leu Thr Ala Gly Met Ala Gly Val Ala
1               5                   10                  15
Thr Ala Ala Ala Gly Ala Trp Thr Gln Pro Gln Leu Arg Pro Val Glu
            20                  25                  30
Leu Pro Gln Arg Thr Arg Gln Val Arg Ala Glu Thr Pro Arg Leu Arg
        35                  40                  45
Gln Gly Val Thr Asn Ala Ala Ala His Ile His Pro Gln Arg Ala Phe
    50                  55                  60
Pro Asp Pro Leu Gly Gly Gly Asn Arg Pro Trp Val Pro Gly Thr Arg
65                  70                  75                  80
Cys Arg Ala Pro Pro Lys Gly Gly Trp Glu Gly Ser His Ser Glu Trp
                85                  90                  95
Gln Asp Pro Gly Arg Pro Leu Glu Ser
            100                 105

<210> 193
<211> 268
<212> PRT
<213> Homo sapiens

<400> 193
Met Gly Ser Ala Lys Ser Val Pro Val Thr Pro Ala Arg Pro Pro Pro
1               5                   10                  15
His Asn Lys His Leu Ala Arg Val Ala Asp Pro Arg Ser Pro Ser Ala
            20                  25                  30
Gly Ile Leu Arg Thr Pro Ile Gln Val Glu Ser Ser Pro Gln Pro Gly
        35                  40                  45
Leu Pro Ala Gly Glu Gln Leu Glu Gly Leu Lys His Ala Gln Asp Ser
    50                  55                  60
Asp Pro Arg Ser Pro Thr Leu Gly Ile Ala Arg Thr Pro Met Lys Thr
65                  70                  75                  80
Ser Ser Gly Asp Pro Pro Ser Pro Leu Val Lys Gln Leu Ser Glu Val

                    85                  90                  95
Phe Glu Thr Glu Asp Ser Lys Ser Asn Leu Pro Pro Glu Pro Val Leu
            100                 105                 110
Pro Pro Glu Ala Pro Leu Ser Ser Glu Leu Asp Leu Pro Leu Gly Thr
        115                 120                 125
Gln Leu Ser Val Glu Glu Gln Met Pro Pro Trp Asn Gln Thr Glu Phe
    130                 135                 140
Pro Ser Lys Gln Val Phe Ser Lys Glu Glu Ala Arg Gln Pro Thr Glu
145                 150                 155                 160
Thr Pro Val Ala Ser Gln Ser Ser Asp Lys Pro Ser Arg Asp Pro Glu
                165                 170                 175
Thr Pro Arg Ser Ser Gly Ser Met Arg Asn Arg Trp Lys Pro Asn Ser
            180                 185                 190
Ser Lys Val Leu Gly Arg Ser Pro Leu Thr Ile Leu Gln Asp Asp Asn
        195                 200                 205
Ser Pro Gly Thr Leu Thr Leu Arg Gln Gly Lys Arg Pro Ser Pro Leu
    210                 215                 220
Ser Glu Asn Val Ser Glu Leu Lys Glu Gly Ala Ile Leu Gly Thr Gly
225                 230                 235                 240
Arg Leu Leu Lys Thr Gly Gly Arg Ala Trp Glu Gln Gly Gln Asp His
                245                 250                 255
Asp Lys Glu Asn Gln His Phe Pro Leu Val Glu Ser
            260                 265

<210> 194
<211> 488
<212> PRT
<213> Homo sapiens

<400> 194
Met Gly Ser Thr Glu Ile Leu Glu Lys Glu Thr Pro Glu Asn Leu Ser
1               5                   10                  15
Asn Gly Thr Ser Ser Asn Val Glu Ala Ala Lys Arg Leu Ala Lys Arg
            20                  25                  30
Leu Tyr Gln Leu Asp Arg Phe Lys Arg Ser Asp Val Ala Lys His Leu
        35                  40                  45
Gly Lys Asn Asn Glu Phe Ser Lys Leu Val Ala Glu Glu Tyr Leu Lys
    50                  55                  60
Phe Phe Asp Phe Thr Gly Met Thr Leu Asp Gln Ser Leu Arg Tyr Phe
65                  70                  75                  80
Phe Lys Ala Phe Ser Leu Val Gly Glu Thr Gln Glu Arg Glu Arg Val
                85                  90                  95
Leu Ile His Phe Ser Asn Arg Tyr Phe Tyr Cys Asn Pro Asp Thr Ile
            100                 105                 110
Ala Ser Gln Asp Gly Val His Cys Leu Thr Cys Ala Ile Met Leu Leu
        115                 120                 125
Asn Thr Asp Leu His Gly His Asn Ile Gly Lys Lys Met Thr Cys Gln
    130                 135                 140
Glu Phe Ile Ala Asn Leu Gln Gly Val Asn Glu Gly Val Asp Phe Ser
145                 150                 155                 160
Lys Asp Leu Leu Lys Ala Leu Tyr Asn Ser Ile Lys Asn Glu Lys Leu
                165                 170                 175
Glu Trp Ala Val Asp Asp Glu Glu Lys Lys Lys Ser Pro Ser Glu Ser
            180                 185                 190
Thr Glu Glu Lys Ala Asn Gly Thr His Pro Lys Thr Ile Ser Arg Ile
        195                 200                 205
Gly Ser Thr Thr Asn Pro Phe Leu Asp Ile Pro His Asp Pro Asn Ala

210                 215                 220
Ala Val Tyr Lys Ser Gly Phe Leu Ala Arg Lys Ile His Ala Asp Met
225                 230                 235                 240
Asp Gly Lys Lys Thr Pro Arg Gly Lys Arg Gly Trp Lys Thr Phe Tyr
                245                 250                 255
Ala Val Leu Lys Gly Thr Val Leu Tyr Leu Gln Lys Asp Glu Tyr Lys
            260                 265                 270
Pro Glu Lys Ala Leu Ser Glu Glu Asp Leu Lys Asn Ala Val Ser Val
        275                 280                 285
His His Ala Leu Ala Ser Lys Ala Thr Asp Tyr Glu Lys Lys Pro Asn
    290                 295                 300
Val Phe Lys Leu Lys Thr Ala Asp Trp Arg Val Leu Leu Phe Gln Thr
305                 310                 315                 320
Gln Ser Pro Glu Glu Met Gln Gly Trp Ile Asn Lys Ile Asn Cys Val
                325                 330                 335
Ala Ala Val Phe Ser Ala Pro Pro Phe Pro Ala Ala Ile Gly Ser Gln
            340                 345                 350
Lys Lys Phe Ser Arg Pro Leu Leu Pro Ala Thr Thr Thr Lys Leu Ser
        355                 360                 365
Gln Glu Glu Gln Leu Lys Ser His Glu Ser Lys Leu Lys Gln Ile Thr
    370                 375                 380
Thr Glu Leu Ala Glu His Arg Ser Tyr Pro Pro Asp Lys Lys Val Lys
385                 390                 395                 400
Ala Lys Asp Val Asp Glu Tyr Lys Leu Lys Asp His Tyr Leu Glu Phe
                405                 410                 415
Glu Lys Thr Arg Tyr Glu Met Tyr Val Ser Ile Leu Lys Glu Gly Gly
            420                 425                 430
Lys Glu Leu Leu Ser Asn Asp Glu Ser Glu Ala Ala Gly Leu Lys Lys
        435                 440                 445
Ser His Ser Ser Pro Ser Leu Asn Pro Asp Thr Ser Pro Ile Thr Ala
    450                 455                 460
Lys Val Lys Arg Asn Val Ser Glu Arg Lys Asp His Arg Pro Glu Thr
465                 470                 475                 480
Pro Ser Ile Lys Gln Lys Val Thr
                485

<210> 195
<211> 234
<212> PRT
<213> Homo sapiens

<400> 195
Met Ala Ala Ser Arg Trp Ala Arg Lys Ala Val Val Leu Leu Cys Ala
1               5                   10                  15
Ser Asp Leu Leu Leu Leu Leu Leu Leu Leu Pro Pro Pro Gly Ser Cys
            20                  25                  30
Ala Ala Glu Gly Ser Pro Gly Thr Pro Asp Glu Ser Thr Pro Pro Pro
        35                  40                  45
Arg Lys Lys Lys Lys Asp Ile Arg Asp Tyr Asn Asp Ala Asp Met Ala
    50                  55                  60
Arg Leu Leu Glu Gln Trp Glu Lys Asp Asp Asp Ile Glu Glu Gly Asp
65                  70                  75                  80
Leu Pro Glu His Lys Arg Pro Ser Ala Pro Val Asp Phe Ser Lys Ile
                85                  90                  95
Asp Pro Ser Lys Pro Glu Ser Ile Leu Lys Met Thr Lys Lys Gly Lys
            100                 105                 110
Thr Leu Met Met Phe Val Thr Val Ser Gly Ser Pro Thr Glu Lys Glu

115                 120                 125
Thr Glu Glu Ile Thr Ser Leu Trp Gln Gly Ser Leu Phe Asn Ala Asn
    130                 135                 140
Tyr Asp Val Gln Arg Phe Ile Val Gly Ser Asp Arg Ala Ile Phe Met
145                 150                 155                 160
Leu Arg Asp Gly Ser Tyr Ala Trp Glu Ile Lys Asp Phe Leu Val Gly
                165                 170                 175
Gln Asp Arg Cys Ala Asp Val Thr Leu Glu Gly Gln Val Tyr Pro Gly
            180                 185                 190
Lys Gly Gly Gly Ser Lys Glu Lys Asn Lys Thr Lys Gln Asp Lys Gly
        195                 200                 205
Lys Lys Lys Lys Glu Gly Asp Leu Lys Ser Arg Ser Ser Lys Glu Glu
    210                 215                 220
Asn Arg Ala Gly Asn Lys Arg Glu Asp Leu
225                 230

<210> 196
<211> 144
<212> PRT
<213> Homo sapiens

<400> 196
Met Ala Cys Ala Ala Ala Arg Ser Pro Ala Asp Gln Asp Arg Phe Ile
1               5                   10                  15
Cys Ile Tyr Pro Ala Tyr Leu Asn Asn Lys Lys Thr Ile Ala Glu Gly
            20                  25                  30
Arg Arg Ile Pro Ile Ser Lys Ala Val Glu Asn Pro Thr Ala Thr Glu
        35                  40                  45
Ile Gln Asp Val Cys Ser Ala Val Gly Leu Asn Val Phe Leu Glu Lys
    50                  55                  60
Asn Lys Met Tyr Ser Arg Glu Trp Asn Arg Asp Val Gln Tyr Arg Gly
65                  70                  75                  80
Arg Val Arg Val Gln Leu Lys Gln Glu Asp Gly Ser Leu Cys Leu Val
                85                  90                  95
Gln Phe Pro Ser Arg Lys Ser Val Met Leu Tyr Ala Ala Glu Met Ile
            100                 105                 110
Pro Lys Leu Lys Thr Arg Thr Gln Lys Thr Gly Gly Ala Asp Gln Ser
        115                 120                 125
Leu Gln Gln Gly Glu Gly Ser Lys Lys Gly Lys Gly Lys Lys Lys Lys
    130                 135                 140

<210> 197
<211> 492
<212> PRT
<213> Homo sapiens

<400> 197
Met Asp Phe Leu Leu Gly Asn Pro Phe Ser Ser Pro Val Gly Gln Arg
1               5                   10                  15
Ile Glu Lys Ala Thr Asp Gly Ser Leu Gln Ser Glu Asp Trp Ala Leu
            20                  25                  30
Asn Met Glu Ile Cys Asp Ile Ile Asn Glu Thr Glu Glu Gly Pro Lys

35                  40                  45
Asp Ala Leu Arg Ala Val Lys Lys Arg Ile Val Gly Asn Lys Asn Phe
        50                  55                  60
His Glu Val Met Leu Ala Leu Thr Val Leu Glu Thr Cys Val Lys Asn
 65                  70                  75                  80
Cys Gly His Arg Phe His Val Leu Val Ala Ser Gln Asp Phe Val Glu
                 85                  90                  95
Ser Val Leu Val Arg Thr Ile Leu Pro Lys Asn Asn Pro Pro Thr Ile
            100                 105                 110
Val His Asp Lys Val Leu Asn Leu Ile Gln Ser Trp Ala Asp Ala Phe
        115                 120                 125
Arg Ser Ser Pro Asp Leu Thr Gly Val Val Thr Ile Tyr Glu Asp Leu
    130                 135                 140
Arg Arg Lys Gly Leu Glu Phe Pro Met Thr Asp Leu Asp Met Leu Ser
145                 150                 155                 160
Pro Ile His Thr Pro Gln Arg Thr Val Phe Asn Ser Glu Thr Gln Ser
                165                 170                 175
Gly Gln Asp Ser Val Gly Thr Asp Ser Ser Gln Gln Glu Asp Ser Gly
            180                 185                 190
Gln His Ala Ala Pro Leu Pro Ala Pro Pro Ile Leu Ser Gly Asp Thr
        195                 200                 205
Pro Ile Ala Pro Thr Pro Glu Gln Ile Gly Lys Leu Arg Ser Glu Leu
    210                 215                 220
Glu Met Val Ser Gly Asn Val Arg Val Met Ser Glu Met Leu Thr Glu
225                 230                 235                 240
Leu Val Pro Thr Gln Ala Glu Pro Ala Asp Leu Glu Leu Leu Gln Glu
                245                 250                 255
Leu Asn Arg Thr Cys Arg Ala Met Gln Gln Arg Val Leu Glu Leu Ile
            260                 265                 270
Pro Gln Ile Ala Asn Glu Gln Leu Thr Glu Glu Leu Leu Ile Val Asn
        275                 280                 285
Asp Asn Leu Asn Asn Val Phe Leu Arg His Glu Arg Phe Glu Arg Phe
    290                 295                 300
Arg Thr Gly Gln Thr Thr Lys Ala Pro Ser Glu Ala Glu Pro Ala Ala
305                 310                 315                 320
Asp Leu Ile Asp Met Gly Pro Asp Pro Ala Ala Thr Gly Asn Leu Ser
                325                 330                 335
Ser Gln Leu Ala Gly Met Asn Leu Gly Ser Ser Ser Val Arg Ala Gly
            340                 345                 350
Leu Gln Ser Leu Glu Ala Ser Gly Arg Leu Glu Asp Glu Phe Asp Met
        355                 360                 365
Phe Ala Leu Thr Arg Gly Ser Ser Leu Ala Asp Gln Arg Lys Glu Val
    370                 375                 380
Lys Tyr Glu Ala Pro Gln Ala Thr Asp Gly Leu Ala Gly Ala Leu Asp
385                 390                 395                 400
Ala Arg Gln Gln Ser Thr Gly Ala Ile Pro Val Thr Gln Ala Cys Leu
                405                 410                 415
Met Glu Asp Ile Glu Gln Trp Leu Ser Thr Asp Val Gly Asn Asp Ala
            420                 425                 430
Glu Glu Pro Lys Gly Val Thr Ser Glu Glu Phe Asp Lys Phe Leu Glu
        435                 440                 445
Glu Arg Ala Lys Ala Ala Asp Arg Leu Pro Asn Leu Ser Ser Pro Ser
    450                 455                 460
Ala Glu Gly Pro Pro Gly Pro Pro Ser Gly Pro Ala Pro Arg Lys Lys
465                 470                 475                 480
Thr Gln Glu Lys Asp Asp Asp Met Leu Phe Ala Leu
                485                 490

<210> 198
<211> 59
<212> PRT
<213> Homo sapiens

<400> 198
Met Thr Met Glu Leu Val Ser Met Ile Lys Thr Tyr Pro Trp Gln Cys
1               5                   10                  15
Met Glu Cys Lys Thr Cys Ile Ile Cys Gly Gln Pro His His Glu Glu
            20                  25                  30
Glu Met Met Phe Cys Asp Met Cys Asp Arg Gly Tyr His Thr Phe Cys
        35                  40                  45
Val Gly Leu Gly Ala Ile Pro Ser Gly Lys Asp
    50                  55

<210> 199
<211> 38
<212> PRT
<213> Homo sapiens

<400> 199
Met Gln Glu Trp Leu Glu Thr Arg Gly Ser Pro Lys Ser His Ile Asp
1               5                   10                  15
Lys Ala Asn Phe Asn Asn Glu Lys Val Ile Leu Lys Leu Asp Tyr Ser
            20                  25                  30
Asp Phe Gln Ile Val Lys
        35

<210> 200
<211> 71
<212> PRT
<213> Homo sapiens

<400> 200

<210> 201
<211> 547
<212> PRT
<213> Homo sapiens

<400> 201
Met Ile Ala Asp Asp Lys Pro Ala Ala Met Ala Gly Glu Ser Ala Gly
1               5                   10                  15
Gln Ser Ser Glu Ser Gly Val Gly Ala Asn Phe Phe Gly Ile Thr Phe
            20                  25                  30
Gln Thr Thr Glu Thr Leu Met Ser Thr Gly His Leu Asn Gly Ala Glu
        35                  40                  45
Cys Lys Ala Gly Pro Gly Thr Val Lys Thr Leu Ala Val Glu Glu Glu
    50                  55                  60
Ala Ser Arg Leu Trp Arg Lys Pro Asp Pro Tyr Asn Thr Arg Arg Glu
65                  70                  75                  80
Pro Asp Leu Arg Gly Gly Ala Leu Asp Ala Thr Gly Ala Gln Gly Gly
                85                  90                  95
Pro Leu Asp Arg Ala Arg Lys Glu Pro Glu Thr Ala Cys Ser His Arg
            100                 105                 110
Arg Lys Ser Val Ala Gly Gln Thr Pro Val Gly Ser Asn Lys Lys Arg
        115                 120                 125
Asn Arg Val Pro Gly His Val Pro Ala Ala Gly Leu Thr Ser Ala Ala
    130                 135                 140
Ala Thr Lys Glu Arg Ala Gln Cys Ser Asp Thr Gly Gly Leu Asp Pro
145                 150                 155                 160
Tyr Ser Ala Gly His Phe Ser Arg Ala Thr Leu Leu Phe Cys Pro Pro
                165                 170                 175
His Thr His Arg Cys Ile Ser Leu Thr Lys Ser Glu Lys Ser Glu Arg
            180                 185                 190
Gln Gln Leu Phe Leu Pro Lys Pro Gln Ser Ala Val Phe Gly Ser Glu
        195                 200                 205
Gly Arg Arg Thr Leu Arg Lys Leu Arg Arg Leu Ser Ser Pro Gly Ala
    210                 215                 220
Met Asp Ser Asp Ala Ser Leu Val Ser Ser Arg Pro Ser Ser Pro Glu
225                 230                 235                 240
Pro Asp Asp Leu Phe Leu Pro Ala Arg Ser Lys Gly Ser Ser Gly Ser
                245                 250                 255
Ala Phe Thr Gly Gly Thr Val Ser Ser Ser Thr Pro Ser Asp Cys Pro
            260                 265                 270
Pro Glu Leu Ser Ala Glu Leu Arg Gly Ala Met Gly Ser Ala Gly Ala
        275                 280                 285
His Pro Gly Asp Lys Leu Gly Gly Ser Gly Phe Lys Ser Ser Ser Ser
    290                 295                 300
Ser Thr Ser Ser Ser Thr Ser Ser Ala Ala Ala Ser Ser Thr Lys Lys
305                 310                 315                 320
Asp Lys Lys Gln Met Thr Glu Pro Glu Leu Gln Gln Leu Arg Leu Lys
                325                 330                 335
Ile Asn Ser Arg Glu Arg Lys Arg Met His Asp Leu Asn Ile Ala Met
            340                 345                 350
Asp Gly Leu Arg Glu Val Met Pro Tyr Ala His Gly Pro Ser Val Arg
        355                 360                 365
Lys Leu Ser Lys Ile Ala Thr Leu Leu Leu Ala Arg Asn Tyr Ile Leu
    370                 375                 380
Met Leu Thr Asn Ser Leu Glu Glu Met Lys Arg Leu Val Ser Glu Ile
385                 390                 395                 400
Tyr Gly Gly His His Ala Gly Phe His Pro Ser Ala Cys Gly Gly Leu
                405                 410                 415
Ala His Ser Ala Pro Leu Pro Ala Ala Thr Ala His Pro Ala Ala Ala
            420                 425                 430
Ala His Ala Ala His His Pro Ala Val His His Pro Ile Leu Pro Pro
        435                 440                 445
Ala Ala Ala Ala Ala Ala Ala Ala Ala Ala Ala Ala Ala Val Ser Ser
    450                 455                 460
Ala Ser Leu Pro Gly Ser Gly Leu Pro Ser Val Gly Ser Ile Arg Pro

465                 470                 475                 480
Pro His Gly Leu Leu Lys Ser Pro Ser Ala Ala Ala Ala Ala Pro Leu
                485                 490                 495
Gly Gly Gly Gly Gly Gly Ser Gly Ala Ser Gly Gly Phe Gln His Trp
            500                 505                 510
Gly Gly Met Pro Cys Pro Cys Ser Met Cys Gln Val Pro Pro Pro His
        515                 520                 525
His His Val Ser Ala Met Gly Ala Gly Ser Leu Pro Arg Leu Thr Ser
    530                 535                 540
Asp Ala Lys
545

<210> 202
<211> 67
<212> PRT
<213> Homo sapiens

<400> 202
Met Gly His Gln Gln Leu Tyr Trp Ser His Pro Arg Lys Phe Gly Gln
 1               5                  10                  15
Gly Ser Arg Ser Cys Arg Val Cys Ser Asn Arg His Gly Leu Ile Arg
            20                  25                  30
Lys Tyr Gly Leu Asn Met Cys Arg Gln Cys Phe Arg Gln Tyr Ala Lys
        35                  40                  45
Asp Ile Gly Phe Ile Lys Lys Asp Leu Ser Cys Leu Pro Trp His Cys
    50                  55                  60
Leu Trp Arg
65

<210> 203
<211> 74
<212> PRT
<213> Homo sapiens

<400> 203
Met Ala Tyr Gln Leu Tyr Arg Asn Thr Thr Leu Gly Asn Ser Leu Gln
 1               5                  10                  15
Glu Ser Leu Asp Glu Leu Ile Gln Gly Ser Leu Asn Thr Tyr Arg Phe
            20                  25                  30
Cys Asp Asn Val Trp Thr Phe Val Leu Asn Asp Val Glu Phe Arg Glu
        35                  40                  45
Val Thr Glu Leu Ile Lys Val Asp Lys Val Lys Ile Val Ala Cys Asp
    50                  55                  60
Gly Lys Asn Thr Gly Ser Asn Thr Thr Glu
 65                  70

<210> 204
<211> 168
<212> PRT
<213> Homo sapiens

<400> 204
Met Gln Asp Pro Lys Met Gln Val Tyr Lys Asp Glu Gln Val Val Val
1               5                   10                  15
Ile Lys Asp Lys Tyr Pro Lys Ala Arg Tyr His Trp Leu Val Leu Pro
            20                  25                  30
Trp Thr Ser Ile Ser Ser Leu Lys Ala Val Ala Arg Glu His Leu Glu
        35                  40                  45
Leu Leu Lys His Met His Thr Val Gly Glu Lys Val Ile Val Asp Phe
    50                  55                  60
Ala Gly Ser Ser Lys Leu Arg Phe Arg Leu Gly Tyr His Ala Ile Pro
65                  70                  75                  80
Ser Met Ser His Val His Leu His Val Ile Ser Gln Asp Phe Asp Ser
                85                  90                  95
Pro Cys Leu Lys Asn Lys Lys His Trp Asn Ser Phe Asn Thr Glu Tyr
            100                 105                 110
Phe Leu Glu Ser Gln Ala Val Ile Glu Met Val Gln Glu Ala Gly Arg
        115                 120                 125
Val Thr Val Arg Asp Gly Met Pro Glu Leu Leu Lys Leu Pro Leu Arg
    130                 135                 140
Cys His Glu Cys Gln Gln Leu Leu Pro Ser Ile Pro Gln Leu Lys Glu
145                 150                 155                 160
His Leu Arg Lys His Trp Thr Gln
                165

<210> 205
<211> 98
<212> PRT
<213> Homo sapiens

<400> 205
Met Ile Pro Gly Gly Leu Ser Glu Ala Lys Pro Ala Thr Pro Glu Ile
1               5                   10                  15
Gln Glu Ile Val Asp Lys Val Lys Pro Gln Leu Glu Glu Lys Thr Asn
            20                  25                  30
Glu Thr Tyr Gly Lys Leu Glu Ala Val Gln Tyr Lys Thr Gln Val Val
        35                  40                  45
Ala Gly Thr Asn Tyr Tyr Ile Lys Val Arg Ala Gly Asp Asn Lys Tyr
    50                  55                  60
Met His Leu Lys Val Phe Lys Ser Leu Pro Gly Gln Asn Glu Asp Leu
65                  70                  75                  80
Val Leu Thr Gly Tyr Gln Val Asp Lys Asn Lys Asp Asp Glu Leu Thr
                85                  90                  95
Gly Phe

<210> 206
<211> 1108
<212> PRT
<213> Homo sapiens

<400> 206

<210> 207
<211> 125
<212> PRT
<213> Homo sapiens

<400> 207
Met Ala Arg Gly Pro Lys Lys His Leu Lys Arg Val Ala Ala Pro Lys
1               5                   10                  15
His Trp Met Leu Asp Lys Leu Thr Gly Val Phe Ala Pro Arg Pro Ser
            20                  25                  30
Thr Gly Pro His Lys Leu Arg Glu Cys Leu Pro Leu Ile Ile Phe Leu
        35                  40                  45
Arg Asn Arg Leu Lys Tyr Ala Leu Thr Gly Asp Glu Val Lys Lys Ile
    50                  55                  60
Cys Met Gln Arg Phe Ile Lys Ile Asp Gly Lys Val Arg Thr Asp Ile
65                  70                  75                  80
Thr Tyr Pro Ala Gly Phe Met Asp Val Ile Ser Ile Asp Lys Thr Gly
                85                  90                  95
Glu Asn Phe Arg Leu Ile Tyr Asp Thr Lys Gly Arg Phe Ala Val His
            100                 105                 110
Arg Ile Thr Pro Glu Glu Ala Lys Tyr Asn Leu Cys Lys
        115                 120                 125

<210> 208
<211> 137
<212> PRT
<213> Homo sapiens

<400> 208
Met Ser Ser Leu Ile Arg Arg Val Ile Ser Thr Ala Lys Ala Pro Gly
1               5                   10                  15
Ala Ile Gly Pro Tyr Ser Gln Ala Val Leu Val Asp Arg Thr Ile Tyr
            20                  25                  30
Ile Ser Gly Gln Ile Gly Met Asp Pro Ser Ser Gly Gln Leu Val Ser
        35                  40                  45
Gly Gly Val Ala Glu Glu Ala Lys Gln Ala Leu Lys Asn Met Gly Glu

        50                  55                  60
Ile Leu Lys Ala Ala Gly Cys Asp Phe Thr Asn Val Val Lys Thr Thr
 65                  70                  75                  80
Val Leu Leu Ala Asp Ile Asn Asp Phe Asn Thr Val Asn Glu Ile Tyr
                 85                  90                  95
Lys Gln Tyr Phe Lys Ser Asn Phe Pro Ala Arg Ala Ala Tyr Gln Val
            100                 105                 110
Ala Ala Leu Pro Lys Gly Ser Arg Ile Glu Ile Glu Ala Val Ala Ile
        115                 120                 125
Gln Gly Pro Leu Thr Thr Ala Ser Leu
    130                 135

<210> 209
<211> 359
<212> PRT
<213> Homo sapiens

<400> 209

<210> 210
<211> 218
<212> PRT
<213> Homo sapiens

<400> 210
   Met Ala Ser Arg Gly Gly Gly Arg Gly Arg Gly Arg Gly Gln Leu Thr
   1               5                   10                  15
   Phe Asn Val Glu Ala Val Gly Ile Gly Lys Gly Asp Ala Leu Pro Pro
               20                  25                  30
   Pro Thr Leu Gln Pro Ser Pro Leu Phe Pro Pro Leu Glu Phe Arg Pro
           35                  40                  45
   Val Pro Leu Pro Ser Gly Glu Glu Gly Glu Tyr Val Leu Ala Leu Lys
       50                  55                  60
   Gln Glu Leu Arg Gly Ala Met Arg Gln Leu Pro Tyr Phe Ile Arg Pro
   65                  70                  75                  80
   Ala Val Pro Lys Arg Asp Val Glu Arg Tyr Ser Asp Lys Tyr Gln Met
                   85                  90                  95
   Ser Gly Pro Ile Asp Asn Ala Ile Asp Trp Asn Pro Asp Trp Arg Arg
               100                 105                 110
   Leu Pro Arg Glu Leu Lys Ile Arg Val Arg Lys Leu Gln Lys Glu Arg
           115                 120                 125
   Ile Thr Ile Leu Leu Pro Lys Arg Pro Pro Lys Thr Thr Glu Asp Lys
       130                 135                 140
   Glu Glu Thr Ile Gln Lys Leu Glu Thr Leu Glu Lys Lys Glu Glu Glu
   145                 150                 155                 160
   Val Thr Ser Glu Glu Asp Glu Glu Lys Glu Glu Glu Glu Glu Lys Glu
                   165                 170                 175
   Glu Glu Glu Glu Glu Glu Tyr Asp Glu Glu Glu His Glu Glu Glu Thr
               180                 185                 190
   Asp Tyr Ile Met Ser Tyr Phe Asp Asn Gly Glu Asp Phe Gly Gly Asp
           195                 200                 205
   Ser Asp Asp Asn Met Asp Glu Ala Ile Tyr
       210                 215

<210> 211
<211> 61
<212> PRT
<213> Homo sapiens

<400> 211
Met Asp Pro Asn Cys Ser Cys Ser Pro Val Gly Ser Cys Ala Cys Ala
1               5                   10                  15
Gly Ser Cys Lys Cys Lys Glu Cys Lys Cys Thr Ser Cys Lys Lys Ser
            20                  25                  30
Cys Cys Ser Cys Cys Pro Val Gly Cys Ala Lys Cys Ala Gln Gly Cys

35                  40                  45
Ile Cys Lys Gly Thr Ser Asp Lys Cys Ser Cys Cys Ala
    50                  55                  60

<210> 212
<211> 331
<212> PRT
<213> Homo sapiens

<400> 212
Met Trp Leu Trp Glu Asp Gln Gly Gly Leu Leu Gly Pro Phe Ser Phe
1               5                   10                  15
Leu Leu Leu Val Leu Leu Leu Val Thr Arg Ser Pro Val Asn Ala Cys
            20                  25                  30
Leu Leu Thr Gly Ser Leu Phe Val Leu Leu Arg Val Phe Ser Phe Glu
        35                  40                  45
Pro Val Pro Ser Cys Arg Ala Leu Gln Val Leu Lys Pro Arg Asp Arg
    50                  55                  60
Ile Ser Ala Ile Ala His Arg Gly Gly Ser His Asp Ala Pro Glu Asn
65                  70                  75                  80
Thr Leu Ala Ala Ile Arg Gln Ala Ala Lys Asn Gly Ala Thr Gly Val
                85                  90                  95
Glu Leu Asp Ile Glu Phe Thr Ser Asp Gly Ile Pro Val Leu Met His
            100                 105                 110
Asp Asn Thr Val Asp Arg Thr Thr Asp Gly Thr Gly Arg Leu Cys Asp
        115                 120                 125
Leu Thr Phe Glu Gln Ile Arg Lys Leu Asn Pro Ala Ala Asn His Arg
    130                 135                 140
Leu Arg Asn Asp Phe Pro Asp Glu Lys Ile Pro Thr Leu Arg Glu Ala
145                 150                 155                 160
Val Ala Glu Cys Leu Asn His Asn Leu Thr Ile Phe Phe Asp Val Lys
                165                 170                 175
Gly His Ala His Lys Ala Thr Glu Ala Leu Lys Lys Met Tyr Met Glu
            180                 185                 190
Phe Pro Gln Leu Tyr Asn Asn Ser Val Val Cys Ser Phe Leu Pro Glu
        195                 200                 205
Val Ile Tyr Lys Met Arg Gln Thr Asp Arg Asp Val Ile Thr Ala Leu
    210                 215                 220
Thr His Arg Pro Trp Ser Leu Ser His Thr Gly Asp Gly Lys Pro Arg
225                 230                 235                 240
Tyr Asp Thr Phe Trp Lys His Phe Ile Phe Val Met Met Asp Ile Leu
                245                 250                 255
Leu Asp Trp Ser Met His Asn Ile Leu Trp Tyr Leu Cys Gly Ile Ser
            260                 265                 270
Ala Phe Leu Met Gln Lys Asp Phe Val Ser Pro Ala Tyr Leu Lys Lys
        275                 280                 285
Trp Ser Ala Lys Gly Ile Gln Val Val Gly Trp Thr Val Asn Thr Phe
    290                 295                 300
Asp Glu Lys Ser Tyr Tyr Glu Ser His Leu Gly Ser Ser Tyr Ile Thr
305                 310                 315                 320
Asp Ser Met Val Glu Asp Cys Glu Pro His Phe
                325                 330

<210> 213
<211> 363

<212> PRT
<213> Homo sapiens

<400> 213
Met Glu Asn Glu Pro Val Ala Leu Glu Glu Thr Gln Lys Thr Asp Pro
1               5                   10                  15
Ala Met Glu Pro Arg Phe Lys Val Val Asp Trp Asp Lys Asn Thr Ala
            20                  25                  30
Ser Gly Cys Leu Thr Pro Arg Ala Glu Asp Trp Leu Ser Ile His Arg
        35                  40                  45
Asp Thr Leu Ile Leu Thr Pro Ile Val Cys Gly Arg Leu Gly Pro Thr
    50                  55                  60
Ser Ile Thr Asp Glu Gly Thr Glu Gly Leu Arg Glu Glu Lys Met His
65                  70                  75                  80
Val Gln Asp Leu Thr Ala Gly Gln Arg His Ser Gln Ala Leu Met Asp
                85                  90                  95
Leu Val Asp Trp Arg Lys Pro Leu Leu Trp Gln Val Gly His Leu Gly
            100                 105                 110
Glu Lys Tyr Asp Glu Trp Val His Gln Pro Val Thr Arg Pro Ile Arg
        115                 120                 125
Leu Phe His Ser Asp Leu Ile Glu Gly Leu Ser Lys Thr Val Trp Tyr
    130                 135                 140
Ser Val Pro Ile Ile Trp Val Pro Leu Val Leu Tyr Leu Ser Trp Ser
145                 150                 155                 160
Tyr Tyr Arg Thr Phe Ala Gln Gly Asn Val Arg Leu Phe Thr Ser Phe
                165                 170                 175
Thr Thr Glu Tyr Thr Val Ala Val Pro Lys Ser Met Phe Pro Gly Leu
            180                 185                 190
Phe Met Leu Gly Thr Phe Leu Trp Ser Leu Ile Glu Tyr Leu Ile His
        195                 200                 205
Arg Phe Leu Phe His Met Lys Pro Pro Ser Asp Ser Tyr Tyr Leu Ile
    210                 215                 220
Met Leu His Phe Val Met His Gly Gln His His Lys Ala Pro Phe Asp
225                 230                 235                 240
Gly Ser Arg Leu Val Phe Pro Pro Val Pro Ala Ser Leu Val Ile Gly
                245                 250                 255
Val Phe Tyr Leu Cys Met Gln Leu Ile Leu Pro Glu Ala Val Gly Gly
            260                 265                 270
Thr Val Phe Ala Gly Gly Leu Leu Gly Tyr Val Leu Tyr Asp Met Thr
        275                 280                 285
His Tyr Tyr Leu His Phe Gly Ser Pro His Lys Gly Ser Tyr Leu Tyr
    290                 295                 300
Ser Leu Lys Ala His His Val Lys His His Phe Ala His Gln Lys Ser
305                 310                 315                 320
Gly Gly Ser His Pro Leu Gly Gly Gln Val Ala Leu Gly Asp Pro Leu
                325                 330                 335
Leu Pro Gly Ala Ser Leu Pro Arg Ala Gln Pro Thr Gly Leu Leu Gln
            340                 345                 350
Ala Val Ala Thr Gly Ser Ser Arg Lys Gly Lys
        355                 360

<210> 214
<211> 193
<212> PRT
<213> Homo sapiens

<400> 214
Met Leu Glu Val His Ile Pro Ser Val Gly Pro Glu Ala Glu Gly Pro
1               5                   10                  15
Arg Gln Ser Pro Glu Lys Ser His Met Val Phe Arg Val Glu Val Leu
            20                  25                  30
Cys Ser Gly Arg Arg His Thr Val Pro Arg Arg Tyr Ser Glu Phe His
        35                  40                  45
Ala Leu His Lys Arg Ile Lys Lys Leu Tyr Lys Val Pro Asp Phe Pro
    50                  55                  60
Ser Lys Arg Leu Pro Asn Trp Arg Thr Arg Gly Leu Glu Gln Arg Arg
65                  70                  75                  80
Gln Gly Leu Glu Ala Tyr Ile Gln Gly Ile Leu Tyr Leu Asn Gln Glu
                85                  90                  95
Val Pro Lys Glu Leu Leu Glu Phe Leu Arg Leu Arg His Phe Pro Thr
            100                 105                 110
Asp Pro Lys Ala Ser Asn Trp Gly Thr Leu Arg Glu Phe Leu Pro Gly
        115                 120                 125
Asp Ser Ser Ser Gln Gln His Gln Arg Pro Val Leu Ser Phe His Val
    130                 135                 140
Asp Pro Tyr Val Cys Asn Pro Ser Pro Glu Ser Leu Pro Asn Val Val
145                 150                 155                 160
Val Asn Gly Val Leu Gln Gly Leu Tyr Ser Phe Ser Ile Ser Pro Asp
                165                 170                 175
Lys Ala Gln Pro Lys Ala Ala Cys His Pro Ala Pro Leu Pro Pro Met
            180                 185                 190
Pro

<210> 215
<211> 444
<212> PRT
<213> Homo sapiens

<400> 215
Met Arg Thr Leu Ser Thr Pro Ser Pro Ala Leu Ile Cys Pro Pro Asn
1               5                   10                  15
Leu Pro Gly Phe Gln Asn Gly Arg Gly Ser Ser Thr Ser Ser Ser Ser
            20                  25                  30
Ile Thr Gly Glu Thr Val Ala Met Val His Ser Pro Pro Pro Thr Arg
        35                  40                  45
Leu Thr His Pro Leu Ile Arg Leu Ala Ser Arg Pro Gln Lys Glu Gln
    50                  55                  60
Ala Ser Ile Asp Arg Leu Pro Asp His Ser Met Val Gln Ile Phe Ser
65                  70                  75                  80
Phe Leu Pro Thr Asn Gln Leu Cys Arg Cys Ala Arg Val Cys Arg Arg
                85                  90                  95
Trp Tyr Asn Leu Ala Trp Asp Pro Arg Leu Trp Arg Thr Ile Arg Leu
            100                 105                 110
Thr Gly Glu Thr Ile Asn Val Asp Arg Ala Leu Lys Val Leu Thr Arg
        115                 120                 125
Arg Leu Cys Gln Asp Thr Pro Asn Val Cys Leu Met Leu Glu Thr Val
    130                 135                 140
Thr Val Ser Gly Cys Arg Arg Leu Thr Asp Arg Gly Leu Tyr Thr Ile
145                 150                 155                 160
Ala Gln Cys Cys Pro Glu Leu Arg Arg Leu Glu Val Ser Gly Cys Tyr
                165                 170                 175
Asn Ile Ser Asn Glu Ala Val Phe Asp Val Val Ser Leu Cys Pro Asn

            180                 185                 190
Leu Glu His Leu Asp Val Ser Gly Cys Ser Lys Val Thr Cys Ile Ser
        195                 200                 205
Leu Thr Arg Glu Ala Ser Ile Lys Leu Ser Pro Leu His Gly Lys Gln
    210                 215                 220
Ile Ser Ile Arg Tyr Leu Asp Met Thr Asp Cys Phe Val Leu Glu Asp
225                 230                 235                 240
Glu Gly Leu His Thr Ile Ala Ala His Cys Thr Gln Leu Thr His Leu
                245                 250                 255
Tyr Leu Arg Arg Cys Val Arg Leu Thr Asp Glu Gly Leu Arg Tyr Leu
            260                 265                 270
Val Ile Tyr Cys Ala Ser Ile Lys Glu Leu Ser Val Ser Asp Cys Arg
        275                 280                 285
Phe Val Ser Asp Phe Gly Leu Arg Glu Ile Ala Lys Leu Glu Ser Arg
    290                 295                 300
Leu Arg Tyr Leu Ser Ile Ala His Cys Gly Arg Val Thr Asp Val Gly
305                 310                 315                 320
Ile Arg Tyr Val Ala Lys Tyr Cys Ser Lys Leu Arg Tyr Leu Asn Ala
                325                 330                 335
Arg Gly Cys Glu Gly Ile Thr Asp His Gly Val Glu Tyr Leu Ala Lys
            340                 345                 350
Asn Cys Thr Lys Leu Lys Ser Leu Asp Ile Gly Lys Cys Pro Leu Val
        355                 360                 365
Ser Asp Thr Gly Leu Glu Cys Leu Ala Leu Asn Cys Phe Asn Leu Lys
    370                 375                 380
Arg Leu Ser Leu Lys Ser Cys Glu Ser Ile Thr Gly Gln Gly Leu Gln
385                 390                 395                 400
Ile Val Ala Ala Asn Cys Phe Asp Leu Gln Thr Leu Asn Val Gln Asp
                405                 410                 415
Cys Glu Val Ser Val Glu Ala Leu Arg Phe Val Lys Arg His Cys Lys
            420                 425                 430
Arg Cys Val Ile Glu His Thr Asn Pro Ala Phe Phe
        435                 440

<210> 216
<211> 99
<212> PRT
<213> Homo sapiens

<400> 216
Met Ser Ala Asn Arg Arg Trp Trp Val Pro Pro Asp Asp Glu Asp Cys
1               5                   10                  15
Val Ser Glu Lys Leu Leu Arg Lys Thr Arg Glu Ser Pro Leu Val Pro
            20                  25                  30
Ile Gly Leu Gly Gly Cys Leu Val Val Ala Ala Tyr Arg Ile Tyr Arg
        35                  40                  45
Leu Arg Ser Arg Gly Ser Thr Lys Met Ser Ile His Leu Ile His Thr
    50                  55                  60
Arg Val Ala Ala Gln Ala Cys Ala Val Gly Ala Ile Met Leu Gly Ala
65                  70                  75                  80
Val Tyr Thr Met Tyr Ser Asp Tyr Val Lys Arg Met Ala Gln Asp Ala
                85                  90                  95
Gly Glu Lys

<210> 217
<211> 130
<212> PRT
<213> Homo sapiens

<400> 217

<210> 218
<211> 136
<212> PRT
<213> Homo sapiens

<400> 218
Met Glu Phe Gly Leu Ser Trp Val Phe Leu Val Ala Ile Leu Lys Gly
1               5                   10                  15
Val Gln Cys Glu Val Gln Leu Val Glu Ser Gly Gly Gly Leu Val Gln
            20                  25                  30
Pro Gly Gly Ser Leu Arg Leu Ser Cys Ala Ala Ser Gly Phe Thr Phe
        35                  40                  45
Ser Ser Tyr Trp Met His Trp Val Arg Gln Ala Pro Gly Lys Gly Leu
    50                  55                  60
Val Trp Val Ser Arg Ile Asn Ser Asp Gly Ser Ser Thr Ser Tyr Ala
65                  70                  75                  80
Asp Ser Val Lys Gly Arg Phe Thr Ile Ser Arg Asp Asn Ala Lys Asn
                85                  90                  95
Thr Leu Tyr Leu Gln Met Asn Ser Leu Arg Ala Glu Asp Thr Ala Val
            100                 105                 110
Tyr Tyr Cys Ala Arg Asp Thr Val Arg Gly Ser Gln Cys Glu Pro Arg
        115                 120                 125
His Lys Pro Arg Cys Arg Gly Ile
    130                 135

<210> 219
<211> 443
<212> PRT
<213> Homo sapiens

<400> 219
Met Leu Leu Ser Ala Val Cys Val Met Leu Asn Leu Ala Gly Ser Ile
1               5                   10                  15
Leu Ser Cys Gln Asn Ala Gln Leu Val Asn Ser Leu Glu Gly Cys Gln
            20                  25                  30
Leu Ile Lys Phe Asp Ser Val Glu Val Cys Val Cys Cys Glu Leu Gln
        35                  40                  45
His Gln Ser Ser Gly Cys Ser Asn Leu Gly Glu Thr Leu Lys Leu Asn
    50                  55                  60
Pro Leu Gln Glu Asn Cys Asn Ala Val Arg Leu Thr Leu Lys Asp Leu
65                  70                  75                  80
Leu Phe Ser Val Cys Ala Leu Asn Val Leu Ser Thr Ile Val Cys Ala
                85                  90                  95
Leu Ala Thr Ala Met Cys Cys Met Gln Met Val Ser Ser Asp Val Leu
            100                 105                 110
Gln Met Phe Leu Pro Gln Arg Ser His Pro Ala Asn Pro Thr Cys Val
        115                 120                 125
Thr Pro His Gly Thr Val Leu His Gln Thr Leu Asp Phe Asp Glu Phe
    130                 135                 140
Ile Pro Pro Leu Pro Pro Pro Pro Tyr Tyr Pro Pro Glu Tyr Thr Cys
145                 150                 155                 160
Thr Pro Ser Thr Glu Ala Gln Arg Gly Leu His Leu Asp Phe Ala Pro
                165                 170                 175
Ser Pro Phe Gly Thr Leu Tyr Asp Val Ala Ile Asn Ser Pro Gly Leu
            180                 185                 190
Leu Tyr Pro Ala Glu Leu Pro Pro Pro Tyr Glu Ala Val Val Gly Gln
        195                 200                 205
Pro Pro Ala Ser Gln Val Thr Ser Ile Gly Gln Gln Val Ala Glu Ser
    210                 215                 220
Ser Ser Gly Asp Pro Asn Thr Ser Ala Gly Phe Ser Thr Pro Val Pro
225                 230                 235                 240
Ala Asp Ser Thr Ser Leu Leu Val Ser Glu Gly Thr Ala Thr Pro Gly
                245                 250                 255
Ser Ser Pro Ser Pro Asp Gly Pro Val Gly Ala Pro Ala Pro Ser Glu
            260                 265                 270
Pro Ala Leu Pro Pro Gly Arg Val Ser Pro Glu Asp Pro Gly Met Gly
        275                 280                 285
Ser Gln Val Gln Pro Gly Pro Gly His Val Ser Arg Ser Thr Ser Asp
    290                 295                 300
Pro Thr Leu Cys Thr Ser Ser Met Ala Gly Asp Ala Ser Ser His Arg
305                 310                 315                 320
Pro Ser Cys Ser Gln Asp Leu Glu Ala Gly Leu Ser Glu Ala Val Pro
                325                 330                 335
Gly Ser Ala Ser Met Ser Arg Ser Ala Thr Ala Ala Cys Arg Ala Gln
            340                 345                 350
Leu Ser Pro Ala Gly Asp Pro Asp Thr Trp Lys Thr Asp Gln Arg Pro
        355                 360                 365
Thr Pro Glu Pro Phe Pro Ala Thr Ser Lys Glu Arg Pro Arg Ser Leu
    370                 375                 380
Val Asp Ser Lys Ala Tyr Ala Asp Ala Arg Val Leu Val Ala Lys Phe
385                 390                 395                 400
Leu Glu His Ser His Cys Ala Leu Pro Thr Glu Ala Gln His Met Val
                405                 410                 415
Gly Ala Met Arg Leu Ala Val Thr Asn Glu Glu Arg Leu Glu Glu Glu
            420                 425                 430
Ala Val Phe Gly Ala Asp Val Leu Asp Gln Val
        435                 440

<210> 220
<211> 464
<212> PRT
<213> Homo sapiens

<400> 220

<210> 221
<211> 403
<212> PRT
<213> Homo sapiens

<400> 221
Met Ser Phe Leu Thr Asn Asp Ala Ser Ser Glu Ser Ile Ala Ser Phe
1               5                   10                  15
Ser Lys Gln Glu Val Met Ser Ser Phe Leu Pro Glu Gly Gly Cys Tyr
            20                  25                  30
Glu Leu Leu Thr Val Ile Gly Lys Gly Phe Glu Asp Leu Met Thr Val
        35                  40                  45
Asn Leu Ala Arg Tyr Lys Pro Thr Gly Glu Tyr Val Thr Val Arg Arg
    50                  55                  60
Ile Asn Leu Glu Ala Cys Ser Asn Glu Met Val Thr Phe Leu Gln Gly
65                  70                  75                  80
Glu Leu His Val Ser Lys Leu Phe Asn His Pro Asn Ile Val Pro Tyr
                85                  90                  95
Arg Ala Thr Phe Ile Ala Asp Asn Glu Leu Trp Val Val Thr Ser Phe
            100                 105                 110
Met Ala Tyr Gly Ser Ala Lys Asp Leu Ile Cys Thr His Phe Met Asp
        115                 120                 125
Gly Met Asn Glu Leu Ala Ile Ala Tyr Ile Leu Gln Gly Val Leu Lys
    130                 135                 140
Ala Leu Asp Tyr Ile His His Met Gly Tyr Val His Arg Ser Val Lys
145                 150                 155                 160
Ala Ser His Ile Leu Ile Ser Val Asp Gly Lys Val Tyr Leu Ser Gly
                165                 170                 175
Leu Arg Ser Asn Leu Ser Met Ile Ser His Gly Gln Arg Gln Arg Val
            180                 185                 190
Val His Asp Phe Pro Lys Tyr Ser Val Lys Val Leu Pro Trp Leu Ser
        195                 200                 205
Pro Glu Val Leu Gln Gln Asn Leu Gln Gly Tyr Asp Ala Lys Ser Asp
    210                 215                 220
Ile Tyr Ser Val Gly Ile Thr Ala Cys Glu Leu Ala Asn Gly His Val
225                 230                 235                 240
Pro Phe Lys Asp Met Pro Ala Thr Gln Pro Arg Ser Trp Phe Cys Pro
                245                 250                 255
Pro Gln Met Leu Leu Glu Lys Leu Asn Gly Thr Val Pro Cys Leu Leu
            260                 265                 270
Asp Thr Ser Thr Ile Pro Ala Glu Glu Leu Thr Met Ser Pro Ser Arg
        275                 280                 285
Ser Val Ala Asn Ser Gly Leu Ser Asp Ser Leu Thr Thr Ser Thr Pro
    290                 295                 300
Arg Pro Ser Asn Gly Asp Ser Pro Ser His Pro Tyr His Arg Thr Phe
305                 310                 315                 320
Ser Pro His Phe His His Phe Val Glu Gln Cys Leu Gln Arg Asn Pro

                325                 330                 335
Asp Ala Arg Pro Ser Ala Ser Thr Leu Leu Asn His Ser Phe Phe Lys
            340                 345                 350
Gln Ile Lys Arg Arg Ala Ser Glu Ala Leu Pro Glu Leu Leu Arg Pro
        355                 360                 365
Val Thr Pro Ile Thr Asn Phe Glu Gly Ser Gln Ser Gln Asp His Ser
    370                 375                 380
Gly Ile Phe Gly Leu Val Thr Asn Leu Glu Glu Leu Glu Val Asp Asp
385                 390                 395                 400
Trp Glu Phe

<210> 222
<211> 958
<212> PRT
<213> Homo sapiens

<400> 222
Met His Thr Cys Cys Pro Pro Val Thr Leu Glu Gln Asp Leu His Arg
1               5                   10                  15
Lys Met His Ser Trp Met Leu Gln Thr Leu Ala Phe Ala Val Thr Ser
            20                  25                  30
Leu Val Leu Ser Cys Ala Glu Thr Ile Asp Tyr Tyr Gly Glu Ile Cys
        35                  40                  45
Asp Asn Ala Cys Pro Cys Glu Glu Lys Asp Gly Ile Leu Thr Val Ser
    50                  55                  60
Cys Glu Asn Arg Gly Ile Ile Ser Leu Ser Glu Ile Ser Pro Pro Arg
65                  70                  75                  80
Phe Pro Ile Tyr His Leu Leu Leu Ser Gly Asn Leu Leu Asn Arg Leu
                85                  90                  95
Tyr Pro Asn Glu Phe Val Asn Tyr Thr Gly Ala Ser Ile Leu His Leu
            100                 105                 110
Gly Ser Asn Val Ile Gln Asp Ile Glu Thr Gly Ala Phe His Gly Leu
        115                 120                 125
Arg Gly Leu Arg Arg Leu His Leu Asn Asn Asn Lys Leu Glu Leu Leu
    130                 135                 140
Arg Asp Asp Thr Phe Leu Gly Leu Glu Asn Leu Glu Tyr Leu Gln Val
145                 150                 155                 160
Asp Tyr Asn Tyr Ile Ser Val Ile Glu Pro Asn Ala Phe Gly Lys Leu
                165                 170                 175
His Leu Leu Gln Val Leu Ile Leu Asn Asp Asn Leu Leu Ser Ser Leu
            180                 185                 190
Pro Asn Asn Leu Phe Arg Phe Val Pro Leu Thr His Leu Asp Leu Arg
        195                 200                 205
Gly Asn Arg Leu Lys Leu Leu Pro Tyr Val Gly Leu Leu Gln His Met
    210                 215                 220
Asp Lys Val Val Glu Leu Gln Leu Glu Glu Asn Pro Trp Asn Cys Ser
225                 230                 235                 240
Cys Glu Leu Ile Ser Leu Lys Asp Trp Leu Asp Ser Ile Ser Tyr Ser
                245                 250                 255
Ala Leu Val Gly Asp Val Val Cys Glu Thr Pro Phe Arg Leu His Gly
            260                 265                 270
Arg Asp Leu Asp Glu Val Ser Lys Gln Glu Leu Cys Pro Arg Arg Leu
        275                 280                 285
Ile Ser Asp Tyr Glu Met Arg Pro Gln Thr Pro Leu Ser Thr Thr Gly
    290                 295                 300
Tyr Leu His Thr Thr Pro Ala Ser Val Asn Ser Val Ala Thr Ser Ser

305                 310                 315                 320
Ser Ala Val Tyr Lys Pro Pro Leu Lys Pro Pro Lys Gly Thr Arg Gln
                325                 330                 335
Pro Asn Lys Pro Arg Val Arg Pro Thr Ser Arg Gln Pro Ser Lys Asp
            340                 345                 350
Leu Gly Tyr Ser Asn Tyr Gly Pro Ser Ile Ala Tyr Gln Thr Lys Ser
        355                 360                 365
Pro Val Pro Leu Glu Cys Pro Thr Ala Cys Ser Cys Asn Leu Gln Ile
    370                 375                 380
Ser Asp Leu Gly Leu Asn Val Asn Cys Gln Glu Arg Lys Ile Glu Ser
385                 390                 395                 400
Ile Ala Glu Leu Gln Pro Lys Pro Tyr Asn Pro Lys Lys Met Tyr Leu
                405                 410                 415
Thr Glu Asn Tyr Ile Ala Val Val Arg Arg Thr Asp Leu Leu Glu Ala
            420                 425                 430
Thr Gly Leu Asp Leu Leu His Leu Gly Asn Asn Arg Ile Ser Met Ile
        435                 440                 445
Gln Asp Arg Ala Phe Gly Asp Leu Thr Asn Leu Arg Arg Leu Tyr Leu
    450                 455                 460
Asn Gly Asn Arg Ile Glu Arg Leu Ser Pro Glu Leu Phe Tyr Gly Leu
465                 470                 475                 480
Gln Ser Leu Gln Tyr Leu Phe Leu Gln Tyr Asn Leu Ile Arg Glu Ile
                485                 490                 495
Gln Ser Gly Thr Phe Asp Pro Val Pro Asn Leu Gln Leu Leu Phe Leu
            500                 505                 510
Asn Asn Asn Leu Leu Gln Ala Met Pro Ser Gly Val Phe Ser Gly Leu
        515                 520                 525
Thr Leu Leu Arg Leu Asn Leu Arg Ser Asn His Phe Thr Ser Leu Pro
    530                 535                 540
Val Ser Gly Val Leu Asp Gln Leu Lys Ser Leu Ile Gln Ile Asp Leu
545                 550                 555                 560
His Asp Asn Pro Trp Asp Cys Thr Cys Asp Ile Val Gly Met Lys Leu
                565                 570                 575
Trp Val Glu Gln Leu Lys Val Gly Val Leu Val Asp Glu Val Ile Cys
            580                 585                 590
Lys Ala Pro Lys Lys Phe Ala Glu Thr Asp Met Arg Ser Ile Lys Ser
        595                 600                 605
Glu Leu Leu Cys Pro Asp Tyr Ser Asp Val Val Val Ser Thr Pro Thr
    610                 615                 620
Pro Ser Ser Ile Gln Val Pro Ala Arg Thr Ser Ala Val Thr Pro Ala
625                 630                 635                 640
Val Arg Leu Asn Ser Thr Gly Ala Pro Ala Ser Leu Gly Ala Gly Gly
                645                 650                 655
Gly Ala Ser Ser Val Pro Leu Ser Val Leu Ile Leu Ser Leu Leu Leu
            660                 665                 670
Val Phe Ile Met Ser Val Phe Val Ala Ala Gly Leu Phe Val Leu Val
        675                 680                 685
Met Lys Arg Arg Lys Lys Asn Gln Ser Asp His Thr Ser Thr Asn Asn
    690                 695                 700
Ser Asp Val Ser Ser Phe Asn Met Gln Tyr Ser Val Tyr Gly Gly Gly
705                 710                 715                 720
Gly Gly Thr Gly Gly His Pro His Ala His Val His His Arg Gly Pro
                725                 730                 735
Ala Leu Pro Lys Val Lys Thr Pro Ala Gly His Val Tyr Glu Tyr Ile
            740                 745                 750
Pro His Pro Leu Gly His Met Cys Lys Asn Pro Ile Tyr Arg Ser Arg
        755                 760                 765
Glu Gly Asn Ser Val Glu Asp Tyr Lys Asp Leu His Glu Leu Lys Val
    770                 775                 780
Thr Tyr Ser Ser Asn His His Leu Gln Gln Gln Gln Pro Pro Pro

785                 790                 795                 800
Pro Pro Gln Gln Pro Gln Gln Gln Pro Pro Pro Gln Leu Gln Leu Gln
                805                 810                 815
Pro Gly Glu Glu Glu Arg Arg Glu Ser His His Leu Arg Ser Pro Ala
            820                 825                 830
Tyr Ser Val Ser Thr Ile Glu Pro Arg Glu Asp Leu Leu Ser Pro Val
        835                 840                 845
Gln Asp Ala Asp Arg Phe Tyr Arg Gly Ile Leu Glu Pro Asp Lys His
    850                 855                 860
Cys Ser Thr Thr Pro Ala Gly Asn Ser Leu Pro Glu Tyr Pro Lys Phe
865                 870                 875                 880
Pro Cys Ser Pro Ala Ala Tyr Thr Phe Ser Pro Asn Tyr Asp Leu Arg
                885                 890                 895
Arg Pro His Gln Tyr Leu His Pro Gly Ala Gly Asp Ser Arg Leu Arg
            900                 905                 910
Glu Pro Val Leu Tyr Ser Pro Pro Ser Ala Val Phe Val Glu Pro Asn
        915                 920                 925
Arg Asn Glu Tyr Leu Glu Leu Lys Ala Lys Leu Asn Val Glu Pro Asp
    930                 935                 940
Tyr Leu Glu Val Leu Glu Lys Gln Thr Thr Phe Ser Gln Phe
945                 950                 955

<210> 223
<211> 380
<212> PRT
<213> Homo sapiens

<400> 223

<210> 224
<211> 76
<212> PRT
<213> Homo sapiens

<400> 224
Met Ser Lys Ala His Pro Pro Glu Leu Lys Lys Phe Met Asp Lys Lys
1               5                   10                  15
Leu Ser Leu Lys Leu Asn Gly Gly Arg His Val Gln Gly Ile Leu Arg
            20                  25                  30
Gly Phe Asp Pro Phe Met Asn Leu Val Ile Asp Glu Cys Val Glu Met
        35                  40                  45
Ala Thr Ser Gly Gln Gln Asn Asn Ile Gly Met Val Val Ile Arg Gly
    50                  55                  60
Asn Ser Ile Ile Met Leu Glu Ala Leu Glu Arg Val
65                  70                  75

<210> 225
<211> 579
<212> PRT
<213> Homo sapiens

<400> 225
Met Asn Thr Ala Pro Ser Arg Pro Ser Pro Thr Arg Arg Asp Pro Tyr
1               5                   10                  15
Gly Phe Gly Asp Ser Arg Asp Ser Arg Arg Asp Arg Ser Pro Ile Arg
            20                  25                  30
Gly Ser Pro Arg Arg Glu Pro Arg Asp Gly Arg Asn Gly Arg Asp Ala
        35                  40                  45
Arg Asp Ser Arg Asp Ile Arg Asp Pro Arg Asp Leu Arg Asp His Arg
    50                  55                  60
His Ser Arg Asp Leu Arg Asp His Arg Asp Ser Arg Ser Val Arg Asp
65                  70                  75                  80
Val Arg Asp Val Arg Asp Leu Arg Asp Phe Arg Asp Leu Arg Asp Ser

                85                  90                  95
Arg Asp Phe Arg Asp Gln Arg Asp Pro Met Tyr Asp Arg Tyr Arg Asp
            100                 105                 110
Met Arg Asp Ser Arg Asp Pro Met Tyr Arg Arg Glu Gly Ser Tyr Asp
        115                 120                 125
Arg Tyr Leu Arg Met Asp Asp Tyr Cys Arg Arg Lys Asp Asp Ser Tyr
    130                 135                 140
Phe Asp Arg Tyr Arg Asp Ser Phe Asp Gly Arg Gly Pro Pro Gly Pro
145                 150                 155                 160
Glu Ser Gln Ser Arg Ala Lys Glu Arg Leu Lys Arg Glu Glu Arg Arg
                165                 170                 175
Arg Glu Glu Leu Tyr Arg Gln Tyr Phe Glu Glu Ile Gln Arg Arg Phe
            180                 185                 190
Asp Ala Glu Arg Pro Val Asp Cys Ser Val Ile Val Val Asn Lys Gln
        195                 200                 205
Thr Lys Asp Tyr Ala Glu Ser Val Gly Arg Lys Val Arg Asp Leu Gly
    210                 215                 220
Met Val Val Asp Leu Ile Phe Leu Asn Thr Glu Val Ser Leu Ser Gln
225                 230                 235                 240
Ala Leu Glu Asp Val Ser Arg Gly Gly Ser Pro Phe Ala Ile Val Ile
                245                 250                 255
Thr Gln Gln His Gln Ile His Arg Ser Cys Thr Val Asn Ile Met Phe
            260                 265                 270
Gly Thr Pro Gln Glu His Arg Asn Met Pro Gln Ala Asp Ala Met Val
        275                 280                 285
Leu Val Ala Arg Asn Tyr Glu Arg Tyr Lys Asn Glu Cys Arg Glu Lys
    290                 295                 300
Glu Arg Glu Glu Ile Ala Arg Gln Ala Ala Lys Met Ala Asp Glu Ala
305                 310                 315                 320
Ile Leu Gln Glu Arg Glu Arg Gly Gly Pro Glu Glu Gly Val Arg Gly
                325                 330                 335
Gly His Pro Pro Ala Ile Gln Ser Leu Ile Asn Leu Leu Ala Asp Asn
            340                 345                 350
Arg Tyr Leu Thr Ala Glu Glu Thr Asp Lys Ile Ile Asn Tyr Leu Arg
        355                 360                 365
Glu Arg Lys Glu Arg Leu Met Arg Ser Ser Thr Asp Ser Leu Pro Gly
    370                 375                 380
Pro Ile Ser Arg Gln Pro Leu Gly Ala Thr Ser Gly Ala Ser Leu Lys
385                 390                 395                 400
Thr Gln Pro Ser Ser Gln Pro Leu Gln Ser Gly Gln Val Leu Pro Ser
                405                 410                 415
Ala Thr Pro Thr Pro Ser Ala Pro Pro Thr Ser Gln Gln Glu Leu Gln
            420                 425                 430
Ala Lys Ile Leu Ser Leu Phe Asn Ser Gly Thr Val Thr Ala Asn Ser
        435                 440                 445
Ser Ser Ala Ser Pro Ser Val Ala Ala Gly Asn Thr Pro Asn Gln Asn
    450                 455                 460
Phe Ser Thr Ala Ala Asn Ser Gln Pro Gln Gln Arg Ser Gln Ala Ser
465                 470                 475                 480
Gly Asn Gln Pro Pro Ser Ile Leu Gly Gln Gly Gly Ser Ala Gln Asn
                485                 490                 495
Met Gly Pro Arg Pro Gly Ala Pro Ser Gln Gly Leu Phe Gly Gln Pro
            500                 505                 510
Ser Ser Arg Leu Ala Pro Ala Ser Asn Met Thr Ser Gln Arg Pro Val
        515                 520                 525
Ser Ser Thr Gly Ile Asn Phe Asp Asn Pro Ser Val Gln Lys Ala Leu
    530                 535                 540
Asp Thr Leu Ile Gln Ser Gly Pro Ala Leu Ser His Leu Val Ser Gln
545                 550                 555                 560
Thr Thr Ala Gln Met Gly Gln Pro Gln Ala Pro Met Gly Ser Tyr Gln

                565                 570                 575
Arg His Tyr

<210> 226
<211> 51
<212> PRT
<213> Homo sapiens

<400> 226
Met Arg Ser Leu Leu Pro Arg Leu Glu Cys Ser Gly Val Ile Ala Ala
1               5                   10                  15
His Cys Ser Leu Glu Leu Leu Gly Ser Ser Asp Pro Pro Ala Ser Ala
            20                  25                  30
Ser Gln Ser Val Gly Thr Thr Gly Val Ser His Gly Thr Cys Leu His
        35                  40                  45
Gln Tyr Ser
    50

<210> 227
<211> 752
<212> PRT
<213> Homo sapiens

<400> 227
Met Asn Gly Glu Tyr Arg Gly Arg Gly Phe Gly Arg Gly Arg Phe Gln
1               5                   10                  15
Ser Trp Lys Arg Gly Arg Gly Gly Gly Asn Phe Ser Gly Lys Trp Arg
            20                  25                  30
Glu Arg Glu His Arg Pro Asp Leu Ser Lys Thr Thr Gly Lys Arg Thr
        35                  40                  45
Ser Gly Asp Leu Ile Gln Glu Ile Gly Tyr Lys Asn Gly Arg Leu Lys
    50                  55                  60
Glu Gln Lys Gly Asn Ile Glu Val Thr Pro Gln Lys Val Val Ile Gln
65                  70                  75                  80
Glu Ala Ala Thr Ile Leu Met Ala Lys Arg Leu Lys Glu Val Leu Arg
                85                  90                  95
Leu Pro Glu Cys Gly Asn Ser Lys Asp Glu Ile Glu Arg Lys Gly Ser
            100                 105                 110
Ile Leu Val Asp Phe Lys Glu Leu Thr Glu Gly Gly Glu Val Thr Asn
        115                 120                 125
Leu Ile Pro Asp Ile Ala Thr Glu Leu Arg Asp Ala Pro Glu Lys Thr
    130                 135                 140
Leu Ala Cys Met Gly Leu Ala Ile His Gln Val Leu Thr Lys Asp Leu
145                 150                 155                 160
Glu Arg His Ala Ala Glu Leu Gln Ala Gln Glu Gly Leu Ser Asn Asp
                165                 170                 175
Gly Glu Thr Met Val Asn Val Pro His Ile His Ala Arg Val Tyr Asn
            180                 185                 190
Tyr Glu Pro Leu Thr Gln Leu Lys Asn Val Arg Ala Asn Tyr Tyr Gly
        195                 200                 205
Lys Tyr Ile Ala Leu Arg Gly Thr Val Val Arg Val Ser Asn Ile Lys
    210                 215                 220
Pro Leu Cys Thr Lys Met Ala Phe Leu Cys Ala Ala Cys Gly Glu Ile

225                 230                 235                 240
Gln Ser Phe Pro Leu Pro Asp Gly Lys Tyr Ser Leu Pro Thr Lys Cys
                245                 250                 255
Pro Val Pro Val Cys Arg Gly Arg Ser Phe Thr Ala Leu Arg Ser Ser
            260                 265                 270
Pro Leu Thr Val Thr Met Asp Trp Gln Ser Ile Lys Ile Gln Glu Leu
        275                 280                 285
Met Ser Asp Asp Gln Arg Glu Ala Gly Arg Ile Pro Arg Thr Ile Glu
    290                 295                 300
Cys Glu Leu Val His Asp Leu Val Asp Ser Cys Val Pro Gly Asp Thr
305                 310                 315                 320
Val Thr Ile Thr Gly Ile Val Lys Val Ser Asn Ala Glu Glu Gly Leu
                325                 330                 335
Ala Leu Ala Leu Phe Gly Gly Ser Gln Lys Tyr Ala Asp Asp Lys Asn
            340                 345                 350
Arg Ile Pro Ile Arg Gly Asp Pro His Ile Leu Val Val Gly Asp Pro
        355                 360                 365
Gly Leu Gly Lys Ser Gln Met Leu Gln Ala Ala Cys Asn Val Ala Pro
    370                 375                 380
Arg Gly Val Tyr Val Cys Gly Asn Thr Thr Thr Thr Ser Gly Leu Thr
385                 390                 395                 400
Val Thr Leu Ser Lys Asp Ser Ser Ser Gly Asp Phe Ala Leu Glu Ala
                405                 410                 415
Gly Ala Leu Val Leu Gly Asp Gln Gly Ile Cys Gly Ile Asp Glu Phe
            420                 425                 430
Asp Lys Met Gly Asn Gln His Gln Ala Leu Leu Glu Ala Met Glu Gln
        435                 440                 445
Gln Ser Ile Ser Leu Ala Lys Ala Gly Val Val Cys Ser Leu Pro Ala
    450                 455                 460
Arg Thr Ser Ile Ile Ala Ala Ala Asn Pro Val Gly Gly His Tyr Asn
465                 470                 475                 480
Lys Ala Lys Thr Val Ser Glu Asn Leu Lys Met Gly Ser Ala Leu Leu
                485                 490                 495
Ser Arg Phe Asp Leu Val Phe Ile Leu Leu Asp Thr Pro Asn Glu His
            500                 505                 510
His Asp His Leu Leu Ser Glu His Val Ile Ala Ile Arg Ala Gly Lys
        515                 520                 525
Gln Arg Thr Ile Ser Ser Ala Thr Val Ala Arg Met Asn Ser Gln Asp
    530                 535                 540
Ser Asn Thr Ser Val Leu Glu Val Val Ser Glu Lys Pro Leu Ser Glu
545                 550                 555                 560
Arg Leu Lys Val Val Pro Gly Glu Thr Ile Asp Pro Ile Pro His Gln
                565                 570                 575
Leu Leu Arg Lys Tyr Ile Gly Tyr Ala Arg Gln Tyr Val Tyr Pro Arg
            580                 585                 590
Leu Ser Thr Glu Ala Ala Arg Val Leu Gln Asp Phe Tyr Leu Glu Leu
        595                 600                 605
Arg Lys Gln Ser Gln Arg Leu Asn Ser Ser Pro Ile Thr Thr Arg Gln
    610                 615                 620
Leu Glu Ser Leu Ile Arg Leu Thr Glu Ala Arg Ala Arg Leu Glu Leu
625                 630                 635                 640
Arg Glu Glu Ala Thr Lys Glu Asp Ala Glu Asp Ile Val Glu Ile Met
                645                 650                 655
Lys Tyr Ser Met Leu Gly Thr Tyr Ser Asp Glu Phe Gly Asn Leu Asp
            660                 665                 670
Phe Glu Arg Ser Gln His Gly Ser Gly Met Ser Asn Arg Ser Thr Ala
        675                 680                 685
Lys Arg Phe Ile Ser Ala Leu Asn Asn Val Ala Glu Arg Thr Tyr Asn
    690                 695                 700
Asn Ile Phe Gln Phe His Gln Leu Arg Gln Ile Ala Lys Glu Leu Asn

705                 710                 715                 720
Ile Gln Val Ala Asp Phe Glu Asn Phe Ile Gly Ser Leu Asn Asp Gln
                725                 730                 735
Gly Tyr Leu Leu Lys Lys Gly Pro Lys Val Tyr Gln Leu Gln Thr Met
            740                 745                 750

<210> 228
<211> 103
<212> PRT
<213> Homo sapiens

<400> 228

<210> 229
<211> 239
<212> PRT
<213> Homo sapiens

<400> 229
Met Gly His Lys Pro Ile Leu Leu Arg Thr Gln Tyr Arg Cys His Pro
1               5                   10                  15
Ala Ile Ser Ala Ile Ala Asn Asp Leu Phe Tyr Lys Gly Ala Leu Met
            20                  25                  30
Asn Gly Val Thr Glu Ile Glu Arg Ser Pro Leu Leu Glu Trp Leu Pro
        35                  40                  45
Thr Leu Cys Phe Tyr Asn Val Lys Gly Leu Glu Gln Ile Glu Arg Asp
    50                  55                  60
Asn Ser Phe His Asn Val Ala Glu Ala Thr Phe Thr Leu Lys Leu Ile
65                  70                  75                  80
Gln Ser Leu Ile Ala Ser Gly Ile Ala Gly Ser Met Ile Gly Val Ile
                85                  90                  95
Thr Leu Tyr Lys Ser Gln Met Tyr Lys Leu Cys His Leu Leu Ser Ala
            100                 105                 110
Val Asp Phe His His Pro Asp Ile Lys Thr Val Gln Val Ser Thr Val
        115                 120                 125
Asp Ala Phe Gln Gly Ala Glu Lys Glu Ile Ile Ile Leu Ser Cys Val
    130                 135                 140
Arg Thr Arg Gln Val Gly Phe Ile Asp Ser Glu Lys Arg Met Asn Val

145                 150                 155                 160
Ala Leu Thr Arg Gly Lys Arg His Leu Leu Ile Val Gly Asn Leu Ala
                165                 170                 175
Cys Leu Arg Lys Asn Gln Leu Trp Gly Arg Val Ile Gln His Cys Glu
            180                 185                 190
Gly Arg Glu Asp Gly Leu Gln His Ala Asn Gln Tyr Glu Pro Gln Leu
        195                 200                 205
Asn His Leu Leu Lys Asp Tyr Phe Glu Lys Gln Val Glu Glu Lys Gln
    210                 215                 220
Lys Lys Lys Ser Glu Lys Glu Lys Ser Lys Asp Lys Ser His Ser
225                 230                 235

<210> 230
<211> 166
<212> PRT
<213> Homo sapiens

<400> 230
Met Ser Glu Pro Gln Pro Arg Gly Ala Glu Arg Asp Leu Tyr Arg Asp
1               5                   10                  15
Thr Trp Val Arg Tyr Leu Gly Tyr Ala Asn Glu Val Gly Glu Ala Phe
            20                  25                  30
Arg Ser Leu Val Pro Ala Ala Val Val Trp Leu Ser Tyr Gly Val Ala
        35                  40                  45
Ser Ser Tyr Val Leu Ala Asp Ala Ile Asp Lys Gly Lys Lys Ala Gly
    50                  55                  60
Glu Val Pro Ser Pro Glu Ala Gly Arg Ser Ala Arg Val Thr Val Ala
65                  70                  75                  80
Val Val Asp Thr Phe Val Trp Gln Ala Leu Ala Ser Val Ala Ile Pro
                85                  90                  95
Gly Phe Thr Ile Asn Arg Val Cys Ala Ala Ser Leu Tyr Val Leu Gly
            100                 105                 110
Thr Ala Thr Arg Trp Pro Leu Ala Val Arg Lys Trp Thr Thr Thr Ala
        115                 120                 125
Leu Gly Leu Leu Thr Ile Pro Ile Ile Ile His Pro Ile Asp Arg Ser
    130                 135                 140
Val Asp Phe Leu Leu Asp Ser Ser Leu Arg Lys Leu Tyr Pro Thr Val
145                 150                 155                 160
Gly Lys Pro Ser Ser Ser
                165

<210> 231
<211> 340
<212> PRT
<213> Homo sapiens

<400> 231
Met Lys Tyr Gly Asn Pro Asn Tyr Gly Gly Met Lys Gly Ile Leu Ser
1               5                   10                  15
Asn Ser Trp Lys Arg Arg Tyr His Ser Arg Arg Ile Gln Arg Asp Val
            20                  25                  30
Ile Lys Lys Arg Ala Leu Ile Gly Asp Asp Val Gly Leu Thr Ser Tyr
        35                  40                  45
Lys His Arg His Ser Gly Leu Val Asn Val Pro Glu Glu Pro Ile Glu

    50                  55                  60
Glu Glu Glu Glu Glu Glu Glu Glu Glu Glu Glu Glu Glu Glu Glu Asp
65                  70                  75                  80
Gln Asp Met Asp Ala Asp Asp Arg Val Val Val Glu Tyr His Glu Glu
                85                  90                  95
Leu Pro Ala Leu Lys Gln Pro Arg Glu Arg Ser Ala Ser Arg Arg Ser
            100                 105                 110
Ser Ala Ser Ser Ser Asp Ser Asp Glu Met Asp Tyr Asp Leu Glu Leu
        115                 120                 125
Lys Met Ile Ser Thr Pro Ser Pro Lys Lys Ser Met Lys Met Thr Met
    130                 135                 140
Tyr Ala Asp Glu Val Glu Ser Gln Leu Lys Asn Ile Arg Asn Ser Met
145                 150                 155                 160
Arg Ala Asp Ser Val Ser Ser Ser Asn Ile Lys Asn Arg Ile Gly Asn
                165                 170                 175
Lys Leu Pro Pro Glu Lys Phe Ala Asp Val Arg His Leu Leu Asp Glu
            180                 185                 190
Lys Arg Gln His Ser Arg Pro Arg Pro Pro Val Ser Ser Thr Lys Ser
        195                 200                 205
Asp Ile Arg Gln Arg Leu Gly Lys Arg Pro His Ser Pro Glu Lys Ala
    210                 215                 220
Phe Ser Ser Asn Pro Val Val Arg Arg Glu Pro Ser Ser Asp Val His
225                 230                 235                 240
Ser Arg Leu Gly Val Pro Arg Gln Asp Ser Lys Gly Leu Tyr Ala Asp
                245                 250                 255
Thr Arg Glu Lys Lys Ser Gly Asn Leu Trp Thr Arg Leu Gly Ser Ala
            260                 265                 270
Pro Lys Thr Lys Glu Lys Asn Thr Lys Lys Val Asp His Arg Ala Pro
        275                 280                 285
Gly Ala Glu Glu Asp Asp Ser Glu Leu Gln Arg Ala Trp Gly Ala Leu
    290                 295                 300
Ile Lys Glu Lys Glu Gln Ser Arg Gln Lys Lys Ser Arg Leu Asp Asn
305                 310                 315                 320
Leu Pro Ser Leu Gln Ile Glu Val Ser Arg Glu Ser Ser Ser Gly Ser
                325                 330                 335
Glu Ala Glu Ser
            340

<210> 232
<211> 141
<212> PRT
<213> Homo sapiens

<400> 232
Met Ser Asn Met Arg Cys Val Asp Cys Gly Thr Cys Leu Gly His Thr
1               5                   10                  15
Arg Arg His Pro Thr Leu Phe Trp Gly Lys Thr Leu Pro Gly Leu Thr
            20                  25                  30
Pro Val Ala Ala Pro Ala Pro Gln Pro Ala Gln Cys Pro Pro Gly Ser
        35                  40                  45
Glu Glu Asp Ala Pro Ala Thr Gln Pro Gly Pro Gln Leu Ala Gly Pro
    50                  55                  60
Asp Pro Pro Trp Ala Pro Val Phe Cys Arg Arg Leu Ala Arg Ala Gly
65                  70                  75                  80
His Ile Glu Leu Cys Asn Ala Val Gly Cys Val Leu Trp Ser Cys Pro
                85                  90                  95
Arg Ser Pro Gly Arg Gly His Ala Val Gly Gln Gly Leu Glu Gly Gly

                100                 105                 110
Gly Gly Cys Pro Trp Ala Thr Pro Pro Ser Leu Gly Gly Ala Asp Phe
        115                 120                 125
Cys Asn Thr Lys Tyr Ser Leu Trp Gln Lys Lys Cys Leu
    130                 135                 140

<210> 233
<211> 662
<212> PRT
<213> Homo sapiens

<400> 233
Met Glu Glu Ile Asp Met Gln Gln Gly Thr Ser Ser Val Lys Pro Gln
1               5                   10                  15
Ala Asn Gly Val Leu Asp Glu Lys Ser Gln Ile Gln Glu Pro Cys Cys
            20                  25                  30
Ser Asp Leu Phe Leu Phe Pro Asp Glu Ser Gly Asn Val Ser Gln Glu
        35                  40                  45
Ser Gly Pro Thr Tyr Ala Ser Phe Ser His His Phe Ile Ser Asp Ala
    50                  55                  60
Met Thr Gly Val Pro Thr Glu Asn Asp Asp Phe Cys Ile Leu Phe Ala
65                  70                  75                  80
Pro Lys Ala Ala Met Gln Glu Lys Glu Glu Glu Pro Val Ile Lys Ile
                85                  90                  95
Met Val Asp Asp Ala Ile Val Ile Arg Asp Asn Tyr Phe Ser Leu Pro
            100                 105                 110
Val Asn Lys Thr Asp Thr Ser Lys Ala Pro Leu His Phe Pro Ile Pro
        115                 120                 125
Val Ile Arg Tyr Val Val Lys Glu Val Ser Leu Val Trp His Leu Tyr
    130                 135                 140
Gly Gly Lys Asp Phe Gly Thr Val Pro Pro Thr Ser Pro Ala Lys Ser
145                 150                 155                 160
Tyr Ile Ser Pro His Ser Ser Pro Ser His Thr Pro Thr Arg His Gly
                165                 170                 175
Arg Asn Thr Val Cys Gly Gly Lys Gly Arg Asn His Asp Phe Leu Met
            180                 185                 190
Glu Ile Gln Leu Ser Lys Val Lys Phe Gln His Glu Val Tyr Pro Pro
        195                 200                 205
Cys Lys Pro Asp Cys Asp Ser Ser Leu Ser Glu His Pro Val Ser Arg
    210                 215                 220
Gln Val Phe Ile Val Gln Asp Leu Glu Ile Arg Asp Arg Leu Ala Thr
225                 230                 235                 240
Ser Gln Met Asn Lys Phe Leu Tyr Leu Tyr Cys Ser Lys Glu Met Pro
                245                 250                 255
Arg Lys Ala His Ser Asn Met Leu Thr Val Lys Ala Leu His Val Cys
            260                 265                 270
Pro Glu Ser Gly Arg Ser Pro Gln Glu Cys Cys Leu Arg Val Ser Leu
        275                 280                 285
Met Pro Leu Arg Leu Asn Ile Asp Gln Asp Ala Leu Phe Phe Leu Lys
    290                 295                 300
Asp Phe Phe Thr Ser Leu Ser Ala Glu Val Glu Leu Gln Met Thr Pro
305                 310                 315                 320
Asp Pro Glu Val Lys Lys Ser Pro Gly Ala Asp Val Thr Cys Ser Leu
                325                 330                 335
Pro Arg His Leu Ser Thr Ser Lys Glu Pro Asn Leu Val Ile Ser Phe
            340                 345                 350
Ser Gly Pro Lys Gln Pro Ser Gln Asn Asp Ser Ala Asn Ser Val Glu

        355                 360                 365
Val Val Asn Gly Met Glu Glu Lys Asn Phe Ser Ala Glu Glu Ala Ser
    370                 375                 380
Phe Arg Asp Gln Pro Val Phe Phe Arg Glu Phe Arg Phe Thr Ser Glu
385                 390                 395                 400
Val Pro Ile Arg Leu Asp Tyr His Gly Lys His Val Ser Met Asp Gln
                405                 410                 415
Gly Thr Leu Ala Gly Ile Leu Ile Gly Leu Ala Gln Leu Asn Cys Ser
            420                 425                 430
Glu Leu Lys Leu Lys Arg Leu Ser Tyr Arg His Gly Leu Leu Gly Val
        435                 440                 445
Asp Lys Leu Phe Ser Tyr Ala Ile Thr Glu Trp Leu Asn Asp Ile Lys
    450                 455                 460
Lys Asn Gln Leu Pro Gly Ile Leu Gly Gly Val Gly Pro Met His Ser
465                 470                 475                 480
Leu Val Gln Leu Val Gln Gly Leu Lys Asp Leu Val Trp Leu Pro Ile
                485                 490                 495
Glu Gln Tyr Arg Lys Asp Gly Arg Ile Val Arg Gly Phe Gln Arg Gly
            500                 505                 510
Ala Ala Ser Phe Gly Thr Ser Thr Ala Met Ala Ala Leu Glu Leu Thr
        515                 520                 525
Asn Arg Met Val Gln Thr Ile Gln Ala Ala Ala Glu Thr Ala Tyr Asp
    530                 535                 540
Met Val Ser Pro Gly Thr Leu Ser Ile Glu Pro Lys Lys Thr Lys Arg
545                 550                 555                 560
Phe Pro His His Arg Leu Ala His Gln Pro Val Asp Leu Arg Glu Gly
                565                 570                 575
Val Ala Lys Ala Tyr Ser Val Val Lys Glu Gly Ile Thr Asp Thr Ala
            580                 585                 590
Gln Thr Ile Tyr Glu Thr Ala Ala Arg Glu His Glu Ser Arg Gly Val
        595                 600                 605
Thr Gly Ala Val Gly Glu Val Leu Arg Gln Ile Pro Pro Ala Val Val
    610                 615                 620
Lys Pro Leu Ile Val Ala Thr Glu Ala Thr Ser Asn Val Leu Gly Gly
625                 630                 635                 640
Met Arg Asn Gln Ile Arg Pro Asp Val Arg Gln Asp Glu Ser Gln Lys
                645                 650                 655
Trp Arg His Gly Asp Asp
            660

<210> 234
<211> 448
<212> PRT
<213> Homo sapiens

<400> 234
Met Lys Gly Gly Thr Leu Gly Gly Ile Pro Gly Glu Pro Ala Val Asp
1               5                   10                  15
His Arg Asp Val Asp Glu Leu Leu Glu Phe Ile Asn Ser Thr Glu Pro
            20                  25                  30
Lys Val Pro Asn Ser Ala Arg Ala Ala Lys Arg Ala Arg His Lys Leu
        35                  40                  45
Lys Lys Lys Glu Lys Glu Lys Ala Gln Leu Ala Ala Glu Ala Leu Lys
    50                  55                  60
Gln Ala Asn Arg Val Ser Gly Ser Arg Glu Pro Arg Pro Ala Arg Glu
65                  70                  75                  80
Arg Leu Leu Glu Trp Pro Asp Arg Glu Leu Asp Arg Val Asn Ser Phe

                        85                  90                  95
Leu Ser Ser Arg Leu Gln Glu Ile Lys Asn Thr Val Lys Asp Ser Ile
            100                 105                 110
Arg Ala Ser Phe Ser Val Cys Glu Leu Ser Met Asp Ser Asn Gly Phe
        115                 120                 125
Ser Lys Glu Gly Ala Ala Glu Pro Glu Pro Gln Ser Leu Pro Pro Ser
    130                 135                 140
Asn Leu Ser Gly Ser Ser Glu Gln Gln Pro Asp Ile Asn Leu Asp Leu
145                 150                 155                 160
Ser Pro Leu Thr Leu Gly Ser Pro Gln Asn His Thr Leu Gln Ala Pro
                165                 .170                175
Gly Glu Pro Ala Pro Pro Trp Ala Glu Met Arg Gly Pro His Pro Pro
            180                 185                 190
Trp Thr Glu Val Arg Gly Pro Pro Pro Gly Ile Val Pro Glu Asn Gly
        195                 200                 205
Leu Val Arg Arg Leu Asn Thr Val Pro Asn Leu Ser Arg Val Ile Trp
    210                 215                 220
Val Lys Thr Pro Lys Pro Gly Tyr Pro Ser Ser Glu Glu Pro Ser Ser
225                 230                 235                 240
Lys Glu Val Pro Ser Cys Lys Gln Glu Leu Pro Glu Pro Val Ser Ser
                245                 250                 255
Gly Gly Lys Pro Gln Lys Gly Lys Arg Gln Gly Ser Gln Ala Lys Lys
            260                 265                 270
Ser Glu Ala Ser Pro Ala Pro Arg Pro Pro Ala Ser Leu Glu Val Pro
        275                 280                 285
Ser Ala Lys Gly Gln Val Ala Gly Pro Lys Gln Pro Gly Arg Val Leu
    290                 295                 300
Glu Leu Pro Lys Val Gly Ser Cys Ala Glu Ala Gly Glu Gly Ser Arg
305                 310                 315                 320
Gly Ser Arg Pro Gly Pro Gly Trp Ala Gly Ser Pro Lys Thr Glu Lys
                325                 330                 335
Glu Lys Gly Ser Ser Trp Arg Asn Trp Pro Gly Glu Ala Lys Ala Arg
            340                 345                 350
Pro Gln Glu Gln Glu Ser Val Gln Pro Pro Gly Pro Ala Arg Pro Gln
        355                 360                 365
Ser Leu Pro Gln Gly Lys Gly Arg Ser Arg Arg Ser Arg Asn Lys Gln
    370                 375                 380
Glu Lys Pro Ala Ser Ser Leu Asp Asp Val Phe Leu Pro Lys Asp Met
385                 390                 395                 400
Asp Gly Val Glu Met Asp Glu Thr Asp Arg Glu Val Glu Tyr Phe Lys
                405                 410                 415
Arg Phe Cys Leu Asp Ser Ala Lys Gln Thr Arg Gln Lys Val Ala Val
            420                 425                 430
Asn Trp Thr Asn Phe Ser Leu Lys Lys Thr Thr Pro Ser Thr Ala Gln
        435                 440                 445

<210> 235
<211> 770
<212> PRT
<213> Homo sapiens

<400> 235
Met Ser Lys Cys Arg Lys Thr Pro Val Gln Gln Leu Ala Ser Pro Ala
1               5                   10                  15
Ser Phe Ser Pro Asp Ile Leu Ala Asp Ile Phe Glu Leu Phe Ala Lys

            20                  25                  30
Asn Phe Ser Tyr Gly Lys Pro Leu Asn Asn Glu Trp Gln Leu Pro Asp
        35                  40                  45
Pro Ser Glu Ile Phe Thr Cys Asp His Thr Glu Phe Asn Ala Phe Leu
    50                  55                  60
Asp Leu Lys Asn Ser Leu Asn Glu Val Lys Asn Leu Leu Ser Asp Lys
65                  70                  75                  80
Lys Leu Asp Glu Trp His Glu His Thr Ala Phe Thr Asn Lys Ala Gly
                85                  90                  95
Lys Ile Ile Ser His Val Arg Lys Ser Val Asn Ala Glu Leu Cys Thr
            100                 105                 110
Gln Ala Trp Cys Lys Phe His Glu Ile Leu Cys Ser Phe Pro Leu Ile
        115                 120                 125
Pro Gln Glu Ala Phe Gln Asn Gly Lys Leu Asn Ser Leu His Leu Cys
    130                 135                 140
Glu Ala Pro Gly Ala Phe Ile Ala Ser Leu Asn His Tyr Leu Lys Ser
145                 150                 155                 160
His Arg Phe Pro Cys His Trp Ser Trp Val Ala Asn Thr Leu Asn Pro
                165                 170                 175
Tyr His Glu Ala Asn Asp Asp Leu Met Met Ile Met Asp Asp Arg Leu
            180                 185                 190
Ile Ala Asn Thr Leu His Trp Trp Tyr Phe Gly Pro Asp Asn Thr Gly
        195                 200                 205
Asp Ile Met Thr Leu Lys Phe Leu Thr Gly Leu Gln Asn Phe Ile Ser
    210                 215                 220
Ser Met Ala Thr Val His Leu Val Thr Ala Asp Gly Ser Phe Asp Cys
225                 230                 235                 240
Gln Gly Asn Pro Gly Glu Gln Glu Ala Leu Val Ser Ser Leu His Tyr
                245                 250                 255
Cys Glu Val Val Thr Ala Leu Thr Thr Leu Gly Asn Gly Gly Ser Phe
            260                 265                 270
Val Leu Lys Met Phe Thr Met Phe Glu His Cys Ser Ile Asn Leu Met
        275                 280                 285
Tyr Leu Leu Asn Cys Cys Phe Asp Gln Val His Val Phe Lys Pro Ala
    290                 295                 300
Thr Ser Lys Ala Gly Asn Ser Glu Val Tyr Val Val Cys Leu His Tyr
305                 310                 315                 320
Lys Gly Arg Glu Ala Ile His Pro Leu Leu Ser Lys Met Thr Leu Asn
                325                 330                 335
Phe Gly Thr Glu Met Lys Arg Lys Ala Leu Phe Pro His His Val Ile
            340                 345                 350
Pro Asp Ser Phe Leu Lys Arg His Glu Glu Cys Cys Val Phe Phe His
        355                 360                 365
Lys Tyr Gln Leu Glu Thr Ile Ser Glu Asn Ile Arg Leu Phe Glu Cys
    370                 375                 380
Met Gly Lys Ala Glu Gln Glu Lys Leu Asn Asn Leu Arg Asp Cys Ala
385                 390                 395                 400
Ile Gln Tyr Phe Met Gln Lys Phe Gln Leu Lys His Leu Ser Arg Asn
                405                 410                 415
Asn Trp Leu Val Lys Lys Ser Ser Ile Gly Cys Ser Thr Asn Thr Lys
            420                 425                 430
Trp Phe Gly Gln Arg Asn Lys Tyr Phe Lys Thr Tyr Asn Glu Arg Lys
        435                 440                 445
Met Leu Glu Ala Leu Ser Trp Lys Asp Lys Val Ala Lys Gly Tyr Phe
    450                 455                 460
Asn Ser Trp Ala Glu Glu His Gly Val Tyr His Pro Gly Gln Ser Ser
465                 470                 475                 480
Ile Leu Glu Gly Thr Ala Ser Asn Leu Glu Cys His Leu Trp His Ile
                485                 490                 495
Leu Glu Gly Lys Lys Leu Pro Lys Val Lys Cys Ser Pro Phe Cys Asn

                    500                         505                         510
Gly Glu Ile Leu Lys Thr Leu Asn Glu Ala Ile Glu Lys Ser Leu Gly
            515                         520                         525
Gly Ala Phe Asn Leu Asp Ser Lys Phe Arg Pro Lys Gln Gln Tyr Ser
        530                         535                         540
Cys Ser Cys His Val Phe Ser Glu Glu Leu Ile Phe Ser Glu Leu Cys
545                         550                         555                         560
Ser Leu Thr Glu Cys Leu Gln Asp Glu Gln Val Val Val Pro Ser Asn
                        565                         570                         575
Gln Ile Lys Cys Leu Leu Val Gly Phe Ser Thr Leu Arg Asn Ile Lys
                580                         585                         590
Met His Ile Pro Leu Glu Val Arg Leu Leu Glu Ser Ala Glu Leu Thr
            595                         600                         605
Thr Phe Ser Cys Ser Leu Leu His Asp Gly Asp Pro Thr Tyr Gln Arg
        610                         615                         620
Leu Phe Leu Asp Cys Leu Leu His Ser Leu Arg Glu Leu His Thr Gly
625                         630                         635                         640
Asp Val Met Ile Leu Pro Val Leu Ser Cys Phe Thr Arg Phe Met Ala
                        645                         650                         655
Gly Leu Ile Phe Val Leu His Ser Cys Phe Arg Phe Ile Thr Phe Val
                660                         665                         670
Cys Pro Thr Ser Ser Asp Pro Leu Arg Thr Cys Ala Val Leu Leu Cys
            675                         680                         685
Val Gly Tyr Gln Asp Leu Pro Asn Pro Val Phe Arg Tyr Leu Gln Ser
        690                         695                         700
Val Asn Glu Leu Leu Ser Thr Leu Leu Asn Ser Asp Ser Pro Gln Gln
705                         710                         715                         720
Val Leu Gln Phe Val Pro Met Glu Val Leu Leu Lys Gly Ala Leu Leu
                        725                         730                         735
Asp Phe Leu Trp Asp Leu Asn Ala Ala Ile Ala Lys Arg His Leu His
                740                         745                         750
Phe Ile Ile Gln Arg Glu Arg Glu Glu Ile Ile Asn Ser Leu Gln Leu
            755                         760                         765
Gln Asn
        770

<210> 236
<211> 690
<212> PRT
<213> Homo sapiens

<400> 236
Met Pro Trp Glu Glu Pro Ala Gly Glu Lys Pro Ser Cys Ser His Ser
1                   5                           10                          15
Gln Lys Ala Phe His Met Glu Pro Ala Gln Lys Pro Cys Phe Thr Thr
                20                          25                          30
Asp Met Val Thr Trp Ala Leu Leu Cys Ile Ser Ala Glu Thr Val Arg
            35                          40                          45
Gly Glu Ala Pro Ser Gln Pro Arg Gly Ile Pro His Arg Ser Pro Val
        50                          55                          60
Ser Val Asp Asp Leu Trp Leu Glu Lys Thr Gln Arg Lys Lys Leu Gln
65                          70                          75                          80
Lys Gln Ala His Ile Glu Arg Arg Leu His Ile Gly Ala Val His Lys
                        85                          90                          95
Asp Gly Val Lys Cys Trp Arg Lys Thr Ile Ile Thr Ser Pro Glu Ser
                100                         105                         110
Leu Asn Leu Pro Arg Arg Ser His Pro Leu Ser Gln Ser Ala Pro Thr

        115                 120                 125
Gly Leu Asn His Met Gly Trp Pro Glu His Thr Pro Gly Thr Ala Met
    130                 135                 140
Pro Asp Gly Ala Leu Asp Thr Ala Val Cys Ala Asp Glu Val Gly Ser
145                 150                 155                 160
Glu Glu Asp Leu Tyr Asp Asp Leu His Ser Ser Ser His His Tyr Ser
                165                 170                 175
His Pro Gly Gly Gly Gly Glu Gln Leu Ala Ile Asn Glu Leu Ile Ser
            180                 185                 190
Asp Gly Ser Val Val Cys Ala Glu Ala Leu Trp Asp His Val Thr Met
        195                 200                 205
Asp Asp Gln Glu Leu Gly Phe Lys Ala Gly Asp Val Ile Glu Val Met
    210                 215                 220
Asp Ala Thr Asn Arg Glu Trp Trp Trp Gly Arg Val Ala Asp Gly Glu
225                 230                 235                 240
Gly Trp Phe Pro Ala Ser Phe Val Arg Leu Arg Val Asn Gln Asp Glu
                245                 250                 255
Pro Ala Asp Asp Asp Ala Pro Leu Ala Gly Asn Ser Gly Ala Glu Asp
            260                 265                 270
Gly Gly Ala Glu Ala Gln Ser Ser Lys Asp Gln Met Arg Thr Asn Val
        275                 280                 285
Ile Asn Glu Ile Leu Ser Thr Glu Arg Asp Tyr Ile Lys His Leu Arg
    290                 295                 300
Asp Ile Cys Glu Gly Tyr Val Arg Gln Cys Arg Lys Arg Ala Asp Met
305                 310                 315                 320
Phe Ser Glu Glu Gln Leu Arg Thr Ile Phe Gly Asn Ile Glu Asp Ile
                325                 330                 335
Tyr Arg Cys Gln Lys Ala Phe Val Lys Ala Leu Glu Gln Arg Phe Asn
            340                 345                 350
Arg Glu Arg Pro His Leu Ser Glu Leu Gly Ala Cys Phe Leu Glu His
        355                 360                 365
Gln Ala Asp Phe Gln Ile Tyr Ser Glu Tyr Cys Asn Asn His Pro Asn
    370                 375                 380
Ala Cys Val Glu Leu Ser Arg Leu Thr Lys Leu Ser Lys Tyr Val Tyr
385                 390                 395                 400
Phe Phe Glu Ala Cys Arg Leu Leu Gln Lys Met Ile Asp Ile Ser Leu
                405                 410                 415
Asp Gly Phe Leu Leu Thr Pro Val Gln Lys Ile Cys Lys Tyr Pro Leu
            420                 425                 430
Gln Leu Ala Glu Leu Leu Lys Tyr Thr His Pro Gln His Arg Asp Phe
        435                 440                 445
Lys Asp Val Glu Ala Ala Leu His Ala Met Lys Asn Val Ala Gln Leu
    450                 455                 460
Ile Asn Glu Arg Lys Arg Arg Leu Glu Asn Ile Asp Lys Ile Ala Gln
465                 470                 475                 480
Trp Gln Ser Ser Ile Glu Asp Trp Glu Gly Glu Asp Leu Leu Val Arg
                485                 490                 495
Ser Ser Glu Leu Ile Tyr Ser Gly Glu Leu Thr Arg Val Thr Gln Pro
            500                 505                 510
Gln Ala Lys Ser Gln Gln Arg Met Phe Phe Leu Phe Asp His Gln Leu
        515                 520                 525
Ile Tyr Cys Lys Lys Asp Leu Leu Arg Arg Asp Val Leu Tyr Tyr Lys
    530                 535                 540
Gly Arg Leu Asp Met Asp Gly Leu Glu Val Val Asp Leu Glu Asp Gly
545                 550                 555                 560
Lys Asp Arg Asp Leu His Val Ser Ile Lys Asn Ala Phe Arg Leu His
                565                 570                 575
Arg Gly Ala Thr Gly Asp Ser His Leu Leu Cys Thr Arg Lys Pro Glu
            580                 585                 590
Gln Lys Gln Arg Trp Leu Lys Ala Phe Ala Arg Glu Arg Glu Gln Val

595                 600                 605
Gln Leu Asp Gln Glu Thr Gly Phe Ser Ile Thr Glu Leu Gln Arg Lys
    610                 615                 620
Gln Ala Met Leu Asn Ala Ser Lys Gln Gln Val Thr Gly Lys Pro Lys
625                 630                 635                 640
Ala Val Gly Arg Pro Cys Tyr Leu Thr Arg Gln Lys His Pro Ala Leu
                645                 650                 655
Pro Ser Asn Arg Pro Gln Gln Gln Val Leu Val Leu Ala Glu Pro Arg
            660                 665                 670
Arg Lys Pro Ser Thr Phe Trp His Ser Ile Ser Arg Leu Ala Pro Phe
        675                 680                 685
Arg Lys
    690

<210> 237
<211> 86
<212> PRT
<213> Homo sapiens

<400> 237
Met Glu Pro Leu Lys Phe Arg Asp Val Ala Ile Glu Phe Ser Leu Glu
  1               5                  10                  15
Glu Trp Gln Cys Leu Asp Thr Ile Gln Gln Asn Leu Tyr Arg Asn Val
             20                  25                  30
Met Leu Glu Asn Tyr Arg Asn Leu Val Phe Leu Gly Ile Val Val Ser
         35                  40                  45
Lys Pro Asp Leu Ile Thr Cys Leu Glu Gln Glu Lys Glu Pro Trp Thr
     50                  55                  60
Arg Lys Arg His Arg Met Val Ala Glu Pro Pro Gly Ser Leu Ser Val
 65                  70                  75                  80
Thr Gln Ala Gly Val Gln
                 85

<210> 238
<211> 432
<212> PRT
<213> Homo sapiens

<400> 238
Met Glu Glu Arg Ala Phe Val Asn Pro Phe Pro Asp Tyr Glu Ala Ala
  1               5                  10                  15
Ala Gly Ala Leu Leu Ala Ser Gly Ala Ala Glu Glu Thr Gly Cys Val
             20                  25                  30
Arg Pro Pro Ala Thr Thr Asp Glu Pro Gly Leu Pro Phe His Gln Asp
         35                  40                  45
Gly Lys Ile Ile His Asn Phe Ile Arg Arg Ile Gln Thr Lys Ile Lys
     50                  55                  60
Asp Leu Leu Gln Gln Met Glu Glu Gly Leu Lys Thr Ala Asp Pro His
 65                  70                  75                  80
Asp Cys Ser Ala Tyr Thr Gly Trp Thr Gly Ile Ala Leu Leu Tyr Leu
                 85                  90                  95
Gln Leu Tyr Arg Val Thr Cys Asp Gln Thr Tyr Leu Leu Arg Ser Leu
            100                 105                 110
Asp Tyr Val Lys Arg Thr Leu Arg Asn Leu Asn Gly Arg Arg Val Thr

115                 120                 125
Phe Leu Cys Gly Asp Ala Gly Pro Leu Ala Val Gly Ala Val Ile Tyr
    130                 135                 140
His Lys Leu Arg Ser Asp Cys Glu Ser Gln Glu Cys Val Thr Lys Leu
145                 150                 155                 160
Leu Gln Leu Gln Arg Ser Val Val Cys Gln Glu Ser Asp Leu Pro Asp
                165                 170                 175
Glu Leu Leu Tyr Gly Arg Ala Gly Tyr Leu Tyr Ala Leu Leu Tyr Leu
            180                 185                 190
Asn Thr Glu Ile Gly Pro Gly Thr Val Cys Glu Ser Ala Ile Lys Glu
        195                 200                 205
Val Val Asn Ala Ile Ile Glu Ser Gly Lys Thr Leu Ser Arg Glu Glu
    210                 215                 220
Arg Lys Thr Glu Arg Cys Pro Leu Leu Tyr Gln Trp His Arg Lys Gln
225                 230                 235                 240
Tyr Val Gly Ala Ala His Gly Met Ala Gly Ile Tyr Tyr Met Leu Met
                245                 250                 255
Gln Pro Ala Ala Lys Val Asp Gln Glu Thr Leu Thr Glu Met Val Lys
            260                 265                 270
Pro Ser Ile Asp Tyr Val Arg His Lys Lys Phe Arg Ser Gly Asn Tyr
        275                 280                 285
Pro Ser Ser Leu Ser Asn Glu Thr Asp Arg Leu Val His Trp Cys His
    290                 295                 300
Gly Ala Pro Gly Val Ile His Met Leu Met Gln Ala Tyr Lys Val Phe
305                 310                 315                 320
Lys Glu Glu Lys Tyr Leu Lys Glu Ala Met Glu Cys Ser Asp Val Ile
                325                 330                 335
Trp Gln Arg Gly Leu Leu Arg Lys Gly Tyr Gly Ile Cys His Gly Thr
            340                 345                 350
Ala Gly Asn Gly Tyr Ser Phe Leu Ser Leu Tyr Arg Leu Thr Gln Asp
        355                 360                 365
Lys Lys Tyr Leu Tyr Arg Ala Cys Lys Phe Ala Glu Trp Cys Leu Asp
    370                 375                 380
Tyr Gly Ala His Gly Cys Arg Ile Pro Asp Arg Pro Tyr Ser Leu Phe
385                 390                 395                 400
Glu Gly Met Ala Gly Ala Ile His Phe Leu Ser Asp Val Leu Gly Pro
                405                 410                 415
Glu Thr Ser Arg Phe Pro Ala Phe Glu Leu Asp Ser Ser Lys Arg Asp
            420                 425                 430

<210> 239
<211> 640
<212> PRT
<213> Homo sapiens

<400> 239
Met Val Ser Asn Pro Val His Gly Leu Pro Phe Leu Pro Gly Thr Ser
1               5                   10                  15
Phe Lys Asp Ser Thr Lys Thr Ala Phe His Arg Ser Gln Thr Leu Ser
            20                  25                  30
Tyr Arg Asn Gly Tyr Ala Ile Val Arg Arg Pro Thr Val Gly Ile Gly
        35                  40                  45
Gly Asp Arg Leu Gln Phe Asn Gln Leu Ser Gln Ala Glu Leu Asp Glu
    50                  55                  60
Leu Ala Ser Lys Ala Pro Val Leu Thr Tyr Gly Gln Pro Lys Gln Ala

65                  70                  75                  80
Pro Pro Ala Asp Phe Ile Pro Ala His Val Ala Phe Asp Lys Lys Val
                85                  90                  95
Leu Lys Phe Asp Ala Tyr Phe Gln Glu Asp Val Pro Met Ser Thr Glu
            100                 105                 110
Glu Gln Tyr Arg Ile Arg Gln Val Asn Ile Tyr Tyr Tyr Leu Glu Asp
        115                 120                 125
Asp Ser Met Ser Val Ile Glu Pro Val Val Glu Asn Ser Gly Ile Leu
    130                 135                 140
Gln Gly Lys Leu Ile Lys Arg Gln Arg Leu Ala Lys Asn Asp Arg Gly
145                 150                 155                 160
Asp His Tyr His Trp Lys Asp Leu Asn Arg Gly Ile Asn Ile Thr Ile
                165                 170                 175
Tyr Gly Lys Thr Phe Arg Val Val Asp Cys Asp Gln Phe Thr Gln Val
            180                 185                 190
Phe Leu Glu Ser Gln Gly Ile Glu Leu Asn Pro Pro Glu Lys Met Ala
        195                 200                 205
Leu Asp Pro Tyr Thr Glu Leu Arg Lys Gln Pro Leu Arg Lys Tyr Val
    210                 215                 220
Thr Pro Ser Asp Phe Asp Gln Leu Lys Gln Phe Leu Thr Phe Asp Lys
225                 230                 235                 240
Gln Val Leu Arg Phe Tyr Ala Ile Trp Asp Asp Thr Asp Ser Met Tyr
                245                 250                 255
Gly Glu Cys Arg Thr Tyr Ile Ile His Tyr Tyr Leu Met Asp Asp Thr
            260                 265                 270
Val Glu Ile Arg Glu Val His Glu Arg Asn Asp Gly Arg Asp Pro Phe
        275                 280                 285
Pro Leu Leu Met Asn Arg Gln Arg Val Pro Lys Val Leu Val Glu Asn
    290                 295                 300
Ala Lys Asn Phe Pro Gln Cys Val Leu Glu Ile Ser Asp Gln Glu Val
305                 310                 315                 320
Leu Glu Trp Tyr Thr Ala Lys Asp Phe Ile Val Gly Lys Ser Leu Thr
                325                 330                 335
Ile Leu Gly Arg Thr Phe Phe Ile Tyr Asp Cys Asp Pro Phe Thr Arg
            340                 345                 350
Arg Tyr Tyr Lys Glu Lys Phe Gly Ile Thr Asp Leu Pro Arg Ile Asp
        355                 360                 365
Val Ser Lys Arg Glu Pro Pro Pro Val Lys Gln Glu Leu Pro Pro Tyr
    370                 375                 380
Asn Gly Phe Gly Leu Val Glu Asp Ser Ala Gln Asn Cys Phe Ala Leu
385                 390                 395                 400
Ile Pro Lys Ala Pro Lys Lys Asp Val Ile Lys Met Leu Val Asn Asp
                405                 410                 415
Asn Lys Val Leu Arg Tyr Leu Ala Val Leu Glu Ser Pro Ile Pro Glu
            420                 425                 430
Asp Lys Asp Arg Arg Phe Val Phe Ser Tyr Phe Leu Ala Thr Asp Met
        435                 440                 445
Ile Ser Ile Phe Glu Pro Pro Val Arg Asn Ser Gly Ile Ile Gly Gly
    450                 455                 460
Lys Tyr Leu Gly Arg Thr Lys Val Val Lys Pro Tyr Ser Thr Val Asp
465                 470                 475                 480
Asn Pro Val Tyr Tyr Gly Pro Ser Asp Phe Phe Ile Gly Ala Val Ile
                485                 490                 495
Glu Val Phe Gly His Arg Phe Ile Ile Leu Asp Thr Asp Glu Tyr Val
            500                 505                 510
Leu Lys Tyr Met Glu Ser Asn Ala Ala Gln Tyr Ser Pro Glu Ala Leu
        515                 520                 525
Ala Ser Ile Gln Asn His Val Arg Lys Arg Glu Ala Pro Ala Pro Glu
    530                 535                 540
Ala Glu Ser Lys Gln Thr Glu Lys Asp Pro Gly Val Gln Glu Leu Glu

545                 550                 555                 560
Ala Leu Ile Asp Thr Ile Gln Lys Gln Leu Lys Asp His Ser Cys Lys
                565                 570                 575
Asp Asn Ile Arg Glu Ala Phe Gln Ile Tyr Asp Lys Glu Ala Ser Gly
            580                 585                 590
Tyr Val Asp Arg Asp Met Phe Phe Lys Ile Cys Glu Ser Leu Asn Val
        595                 600                 605
Pro Val Asp Asp Ser Leu Val Lys Glu Leu Ile Arg Met Cys Ser His
    610                 615                 620
Gly Glu Gly Lys Ile Asn Tyr Tyr Asn Phe Val Arg Ala Phe Ser Asn
625                 630                 635                 640

<210> 240
<211> 340
<212> PRT
<213> Homo sapiens

<400> 240
Met Thr Ser Tyr Arg Glu Arg Ser Ala Asp Leu Ala Arg Phe Tyr Thr
1               5                   10                  15
Val Thr Glu Pro Gln Arg His Pro Arg Gly Tyr Thr Val Tyr Lys Val
            20                  25                  30
Thr Ala Arg Val Val Ser Arg Arg Asn Pro Gln Asp Val Gln Glu Ile
        35                  40                  45
Ile Val Trp Asn Arg Tyr Thr Val Phe Asn Lys Leu His Leu Glu Leu
    50                  55                  60
Trp Leu Ile His Lys Asn Leu Phe Arg His Ser Glu Leu Phe Pro Pro
65                  70                  75                  80
Phe Ala Lys Gly Ile Val Phe Gly Arg Phe Asp Glu Thr Val Ile Glu
                85                  90                  95
Glu Arg Arg Gln Cys Ala Glu Asp Leu Leu Gln Phe Ser Ala Asn Ile
            100                 105                 110
Pro Ala Leu Tyr Asn Ser Lys Gln Leu Glu Asp Phe Phe Lys Gly Gly
        115                 120                 125
Ile Ile Asn Asp Ser Ser Glu Leu Ile Gly Pro Ala Glu Ala His Ser
    130                 135                 140
Asp Ser Leu Ile Asp Thr Phe Pro Glu Cys Ser Thr Glu Gly Phe Ser
145                 150                 155                 160
Ser Asp Ser Asp Leu Val Ser Leu Thr Val Asp Val Asp Ser Leu Ala
                165                 170                 175
Glu Leu Asp Asp Gly Met Ala Ser Asn Gln Asn Ser Pro Ile Arg Thr
            180                 185                 190
Phe Gly Leu Asn Leu Ser Ser Asp Ser Ser Ala Leu Gly Ala Val Ala
        195                 200                 205
Ser Asp Ser Glu Gln Ser Lys Thr Glu Glu Glu Arg Glu Ser Arg Ser
    210                 215                 220
Leu Phe Pro Gly Ser Leu Lys Pro Lys Leu Gly Lys Arg Asp Tyr Leu
225                 230                 235                 240
Glu Lys Ala Gly Glu Leu Ile Lys Leu Ala Leu Lys Lys Glu Glu Glu
                245                 250                 255
Asp Asp Tyr Glu Ala Ala Ser Asp Phe Tyr Arg Lys Gly Val Asp Leu
            260                 265                 270
Leu Leu Glu Gly Val Gln Gly Glu Ser Ser Pro Thr Arg Arg Glu Ala
        275                 280                 285
Val Lys Arg Arg Thr Ala Glu Tyr Leu Met Arg Ala Glu Ser Ile Ser

290                 295                 300
Ser Leu Tyr Gly Lys Pro Gln Leu Asp Asp Val Ser Gln Val Cys Leu
305                 310                 315                 320
Ile Phe Cys Cys Val Phe Ser Ser Val Ile Phe Cys Cys Leu Val Ser
                325                 330                 335
Leu Leu Tyr Phe
            340

<210> 241
<211> 81
<212> PRT
<213> Homo sapiens

<400> 241
Met Ser Leu Asn Leu Gly Arg His Asn Ser Ser Val Cys Ser Lys Arg
1               5                   10                  15
Thr Val Ser Gln Lys Leu Glu Leu Pro Leu Glu Ala Gln Thr Pro Glu
            20                  25                  30
Asn Thr Gln Gly Gly Thr Leu Pro Val Asp Val Leu Gly Lys Glu Glu
        35                  40                  45
Gly Arg Gly Asp Arg Gly Gln Asp Ser Ala Leu Trp Trp Val Leu Arg
    50                  55                  60
Val Pro Thr Arg Gly Ser Gln Asp Leu Gly Asn Arg Ser Ala Thr Leu
65                  70                  75                  80
Val

<210> 242
<211> 152
<212> PRT
<213> Homo sapiens

<400> 242
Met Arg Glu Lys Leu Ile Arg Leu Gln Ser Leu Tyr Ile Asp Gln Phe
1               5                   10                  15
Lys Arg Leu Gln His Leu Leu Lys Glu Lys Lys Arg Arg Tyr Leu His
            20                  25                  30
Asn Arg Lys Val Glu His Glu Ala Leu Gly Ser Ser Leu Leu Thr Gly
        35                  40                  45
Pro Glu Gly Leu Leu Ala Lys Glu Arg Glu Asn Leu Lys Arg Leu Lys
    50                  55                  60
Cys Leu Arg Arg Tyr Arg Gln Arg Tyr Gly Val Glu Ala Leu Leu His
65                  70                  75                  80
Arg Gln Leu Lys Glu Arg Arg Met Leu Ala Thr Asp Gly Ala Ala Gln
                85                  90                  95
Gln Ala His Thr Thr Arg Ser Ser Gln Arg Cys Leu Ala Phe Val Asp
            100                 105                 110
Asp Val Arg Cys Ser Asn Gln Ser Leu Pro Met Thr Arg His Cys Leu
        115                 120                 125
Thr Gln Lys Asp Ala Thr Ala Ile Gly Asp Leu Leu Thr Leu Tyr Cys
    130                 135                 140
Gly Leu Met Glu Thr Ala Leu Asp
145                 150

<210> 243
<211> 434
<212> PRT
<213> Homo sapiens

<400> 243

<210> 244
<211> 680
<212> PRT
<213> Homo sapiens

<400> 244
Met Asn Ser Asp Asp Pro Val Val Gln Gln Lys Ala Val Leu Glu Thr
1               5                   10                  15
Glu Lys Arg Leu Leu Leu Met Glu Glu Asp Gln Glu Glu Asp Glu Cys
            20                  25                  30
Arg Thr Thr Leu Asn Lys Thr Met Ile Ser Pro Pro Gln Thr Ala Met
        35                  40                  45
Lys Ser Ala Glu Glu Ile Asn Ser Glu Ala Phe Leu Ala Ser Val Glu
    50                  55                  60
Lys Asp Ala Lys Glu Arg Ala Lys Arg Arg Arg Glu Asn Lys Val Leu
65                  70                  75                  80
Ala Asp Ala Leu Lys Glu Lys Gly Asn Glu Ala Phe Ala Glu Gly Asn
                85                  90                  95
Tyr Glu Thr Ala Ile Leu Arg Tyr Ser Glu Gly Leu Glu Lys Leu Lys
            100                 105                 110
Asp Met Lys Val Leu Tyr Thr Asn Arg Ala Gln Ala Tyr Met Lys Leu
        115                 120                 125
Glu Asp Tyr Glu Lys Ala Leu Val Asp Cys Glu Trp Ala Leu Lys Cys
    130                 135                 140
Asp Glu Lys Cys Thr Lys Ala Tyr Phe His Met Gly Lys Ala Asn Leu
145                 150                 155                 160
Ala Leu Lys Asn Tyr Ser Val Ser Arg Glu Cys Tyr Lys Lys Ile Leu
                165                 170                 175
Glu Ile Asn Pro Lys Leu Gln Thr Gln Val Lys Gly Tyr Leu Asn Gln
            180                 185                 190
Val Asp Leu Gln Glu Lys Ala Asp Leu Gln Glu Lys Glu Ala His Glu
        195                 200                 205
Leu Leu Asp Ser Gly Lys Asn Thr Ala Val Thr Thr Lys Asn Leu Leu
    210                 215                 220
Glu Thr Leu Ser Lys Pro Asp Gln Ile Pro Leu Phe Tyr Ala Gly Gly
225                 230                 235                 240
Ile Glu Ile Leu Thr Glu Met Ile Asn Glu Cys Thr Glu Gln Thr Leu
                245                 250                 255
Phe Arg Met His Asn Gly Phe Ser Ile Ile Ser Asp Asn Glu Val Ile
            260                 265                 270
Arg Arg Cys Phe Ser Thr Ala Gly Asn Asp Ala Val Glu Glu Met Val
        275                 280                 285
Cys Val Ser Val Leu Lys Leu Trp Gln Ala Val Cys Ser Arg Asn Glu
    290                 295                 300
Glu Asn Gln Arg Val Leu Val Ile His His Asp Arg Ala Arg Leu Leu
305                 310                 315                 320
Ala Ala Leu Leu Ser Ser Lys Val Leu Ala Ile Arg Gln Gln Ser Tyr
                325                 330                 335
Ala Leu Leu Leu His Leu Ala Gln Thr Glu Ser Gly Arg Ser Leu Ile
            340                 345                 350
Ile Asn His Leu Asp Leu Thr Arg Leu Leu Glu Ala Leu Val Ser Phe

        355                 360                 365
Leu Asp Phe Ser Asp Lys Glu Ala Asn Thr Ala Met Gly Leu Phe Thr
    370                 375                 380
Asp Leu Ala Leu Glu Glu Arg Phe Gln Val Trp Phe Gln Ala Asn Leu
385                 390                 395                 400
Pro Gly Val Leu Pro Ala Leu Thr Gly Val Leu Lys Thr Asp Pro Lys
                405                 410                 415
Val Ser Ser Ser Ser Ala Leu Cys Gln Cys Ile Ala Ile Met Gly Asn
            420                 425                 430
Leu Ser Ala Glu Pro Thr Thr Arg Arg His Met Ala Ala Cys Glu Glu
        435                 440                 445
Phe Gly Asp Gly Cys Leu Ser Leu Leu Ala Arg Cys Glu Glu Asp Val
    450                 455                 460
Asp Leu Phe Arg Glu Val Ile Tyr Thr Leu Leu Gly Leu Met Met Asn
465                 470                 475                 480
Leu Cys Leu Gln Ala Pro Phe Val Ser Glu Val Trp Ala Val Glu Val
                485                 490                 495
Ser Arg Arg Cys Leu Ser Leu Leu Asn Ser Gln Asp Gly Gly Ile Leu
            500                 505                 510
Thr Arg Ala Ala Gly Val Leu Ser Arg Thr Leu Ser Ser Ser Leu Lys
        515                 520                 525
Ile Val Glu Glu Ala Leu Arg Ala Gly Val Val Lys Lys Met Met Lys
    530                 535                 540
Phe Leu Lys Thr Gly Gly Glu Thr Ala Ser Arg Tyr Ala Ile Lys Ile
545                 550                 555                 560
Leu Ala Ile Cys Thr Asn Ser Tyr His Glu Ala Arg Glu Glu Val Ile
                565                 570                 575
Arg Leu Asp Lys Lys Leu Ser Val Met Met Lys Leu Leu Ser Ser Glu
            580                 585                 590
Asp Glu Val Leu Val Gly Asn Ala Ala Leu Cys Leu Gly Asn Cys Met
        595                 600                 605
Glu Val Pro Asn Val Ala Ser Ser Leu Leu Lys Thr Asp Leu Leu Gln
    610                 615                 620
Val Leu Leu Lys Leu Ala Gly Ser Asp Thr Gln Lys Thr Ala Val Gln
625                 630                 635                 640
Val Asn Ala Gly Ile Ala Leu Gly Lys Leu Cys Thr Ala Glu Pro Arg
                645                 650                 655
Phe Ala Ala Gln Leu Arg Lys Leu His Gly Leu Glu Ile Leu Asn Ser
            660                 665                 670
Thr Met Lys Tyr Ile Ser Asp Ser
        675                 680

<210> 245
<211> 141
<212> PRT
<213> Homo sapiens

<400> 245
Met Glu Thr Ala Gly Ala Ala Thr Gly Gln Pro Ala Ser Gly Leu Glu
1               5                   10                  15
Ala Pro Gly Ser Thr Asn Asp Arg Leu Phe Leu Val Lys Gly Gly Ile
            20                  25                  30
Phe Leu Gly Thr Val Ala Ala Ala Gly Met Leu Ala Gly Phe Ile Thr
        35                  40                  45
Thr Leu Ser Leu Ala Lys Lys Lys Ser Pro Glu Trp Phe Asn Lys Gly
    50                  55                  60
Ser Met Ala Thr Ala Ala Leu Pro Glu Ser Gly Ser Ser Leu Ala Leu

65                  70                  75                  80
Arg Ala Leu Gly Trp Gly Ser Leu Tyr Ala Trp Cys Gly Val Gly Val
                85                  90                  95
Ile Ser Phe Ala Val Trp Lys Ala Leu Gly Val His Ser Met Asn Asp
            100                 105                 110
Phe Arg Ser Lys Met Gln Ser Ile Phe Pro Thr Ile Pro Lys Asn Ser
        115                 120                 125
Glu Ser Ala Val Glu Trp Glu Glu Thr Leu Lys Ser Lys
    130                 135                 140

<210> 246
<211> 446
<212> PRT
<213> Homo sapiens

<400> 246
Met Glu Thr Gln Ala Asp Leu Val Ser Gln Glu Pro Gln Ala Leu Leu
1               5                   10                  15
Asp Ser Ala Leu Pro Ser Lys Val Pro Ala Phe Ser Asp Lys Asp Ser
            20                  25                  30
Leu Gly Asp Glu Met Leu Ala Ala Ala Leu Leu Lys Ala Lys Ser Gln
        35                  40                  45
Glu Leu Val Thr Phe Glu Asp Val Ala Val Tyr Phe Ile Arg Lys Glu
    50                  55                  60
Trp Lys Arg Leu Glu Pro Ala Gln Arg Asp Leu Tyr Arg Asp Val Met
65                  70                  75                  80
Leu Glu Asn Tyr Gly Asn Val Phe Ser Leu Asp Arg Glu Thr Arg Thr
                85                  90                  95
Glu Asn Asp Gln Glu Ile Ser Glu Asp Thr Arg Ser His Gly Val Leu
            100                 105                 110
Leu Gly Arg Phe Gln Lys Asp Ile Ser Gln Gly Leu Lys Phe Lys Glu
        115                 120                 125
Ala Tyr Glu Arg Glu Val Ser Leu Lys Arg Pro Leu Gly Asn Ser Pro
    130                 135                 140
Gly Glu Arg Leu Asn Arg Lys Met Pro Asp Phe Gly Gln Val Thr Val
145                 150                 155                 160
Glu Glu Lys Leu Thr Pro Arg Gly Glu Arg Ser Glu Lys Tyr Asn Asp
                165                 170                 175
Phe Gly Asn Ser Phe Thr Val Asn Ser Asn Leu Ile Ser His Gln Arg
            180                 185                 190
Leu Pro Val Gly Asp Arg Pro His Lys Cys Asp Glu Cys Ser Lys Ser
        195                 200                 205
Phe Asn Arg Thr Ser Asp Leu Ile Gln His Gln Arg Ile His Thr Gly
    210                 215                 220
Glu Lys Pro Tyr Glu Cys Asn Glu Cys Gly Lys Ala Phe Ser Gln Ser
225                 230                 235                 240
Ser His Leu Ile Gln His Gln Arg Ile His Thr Gly Glu Lys Pro Tyr
                245                 250                 255
Glu Cys Ser Asp Cys Gly Lys Thr Phe Ser Cys Ser Ser Ala Leu Ile
            260                 265                 270
Leu His Arg Arg Ile His Thr Gly Glu Lys Pro Tyr Glu Cys Asn Glu
        275                 280                 285
Cys Gly Lys Thr Phe Ser Trp Ser Ser Thr Leu Thr His His Gln Arg
    290                 295                 300
Ile His Thr Gly Glu Lys Pro Tyr Ala Cys Asn Glu Cys Gly Lys Ala
305                 310                 315                 320
Phe Ser Arg Ser Ser Thr Leu Ile His His Gln Arg Ile His Thr Gly

                325                 330                 335
Glu Lys Pro Tyr Glu Cys Asn Glu Cys Gly Lys Ala Phe Ser Gln Ser
            340                 345                 350
Ser His Leu Tyr Gln His Gln Arg Ile His Thr Gly Glu Lys Pro Tyr
        355                 360                 365
Glu Cys Met Glu Cys Gly Gly Lys Phe Thr Tyr Ser Ser Gly Leu Ile
    370                 375                 380
Gln His Gln Arg Ile His Thr Gly Glu Asn Pro Tyr Glu Cys Ser Glu
385                 390                 395                 400
Cys Gly Lys Ala Phe Arg Tyr Ser Ser Ala Leu Val Arg His Gln Arg
                405                 410                 415
Ile His Thr Gly Glu Lys Pro Leu Asn Gly Ile Gly Met Ser Lys Ser
            420                 425                 430
Ser Leu Arg Val Thr Thr Glu Leu Asn Ile Arg Glu Ser Thr
        435                 440                 445

<210> 247
<211> 577
<212> PRT
<213> Homo sapiens

<400> 247
Met Leu Thr Pro Ala Phe Asp Leu Ser Gln Asp Pro Asp Phe Leu Thr
1               5                   10                  15
Ile Ala Ile Arg Val Pro Tyr Ala Arg Val Ser Glu Phe Asp Val Tyr
            20                  25                  30
Phe Glu Gly Ser Asp Phe Lys Phe Tyr Ala Lys Pro Tyr Phe Leu Arg
        35                  40                  45
Leu Thr Leu Pro Gly Arg Ile Val Glu Asn Gly Ser Glu Gln Gly Ser
    50                  55                  60
Tyr Asp Ala Asp Lys Gly Ile Phe Thr Ile Arg Leu Pro Lys Glu Thr
65                  70                  75                  80
Pro Gly Gln His Phe Glu Gly Leu Asn Met Leu Thr Ala Leu Leu Ala
                85                  90                  95
Pro Arg Lys Ser Arg Thr Ala Lys Pro Leu Val Glu Glu Ile Gly Ala
            100                 105                 110
Ser Glu Ile Pro Glu Glu Val Val Asp Asp Glu Glu Phe Asp Trp Glu
        115                 120                 125
Ile Glu Gln Thr Pro Cys Glu Glu Val Ser Glu Ser Ala Leu Asn Pro
    130                 135                 140
Gln Cys His Tyr Gly Phe Gly Asn Leu Arg Ser Gly Val Leu Gln Arg
145                 150                 155                 160
Leu Gln Asp Glu Leu Ser Asp Val Ile Asp Ile Lys Asp Pro Asp Phe
                165                 170                 175
Thr Pro Ala Ala Glu Arg Arg Gln Lys Arg Leu Ala Ala Glu Leu Ala
            180                 185                 190
Lys Phe Asp Pro Asp His Tyr Leu Ala Asp Phe Phe Glu Asp Glu Ala
        195                 200                 205
Ile Glu Gln Ile Leu Lys Tyr Asn Pro Trp Trp Thr Asp Lys Tyr Ser
    210                 215                 220
Lys Met Met Ala Phe Leu Glu Lys Ser Gln Glu Gln Glu Asn His Ala
225                 230                 235                 240
Thr Leu Val Ser Phe Ser Glu Glu Glu Lys Tyr Gln Leu Arg Lys Phe
                245                 250                 255
Val Asn Lys Ser Tyr Leu Leu Asp Lys Arg Ala Cys Arg Gln Val Cys
            260                 265                 270
Tyr Ser Leu Ile Asp Ile Leu Leu Ala Tyr Cys Tyr Glu Thr Arg Val

        275                 280                 285
Thr Glu Gly Glu Lys Asn Val Glu Ser Ala Trp Asn Ile Arg Lys Leu
    290                 295                 300
Ser Pro Thr Leu Cys Trp Phe Glu Thr Trp Thr Asn Val His Asp Ile
305                 310                 315                 320
Met Val Ser Phe Gly Arg Arg Val Leu Cys Tyr Pro Leu Tyr Arg His
                325                 330                 335
Phe Lys Leu Val Met Lys Ala Tyr Arg Asp Thr Ile Lys Ile Leu Gln
            340                 345                 350
Leu Gly Lys Ser Ala Val Leu Lys Cys Leu Leu Asp Ile His Lys Ile
        355                 360                 365
Phe Gln Glu Asn Asp Pro Ala Tyr Ile Leu Asn Asp Leu Tyr Ile Thr
    370                 375                 380
Asp Tyr Cys Val Tyr Ile Gln Lys Val Lys Ser Lys Lys Leu Ala Ala
385                 390                 395                 400
Leu Ala Glu Ala Leu Lys Glu Val Ser Leu Thr Lys Ala Gln Leu Gly
                405                 410                 415
Leu Glu Leu Glu Glu Leu Glu Ala Ala Ala Leu Leu Val Gln Glu Glu
            420                 425                 430
Glu Thr Ala Leu Lys Ala Ala His Ser Val Ser Gly Gln Gln Thr Leu
        435                 440                 445
Cys Ser Ser Ser Glu Ala Ser Asp Ser Glu Asp Ser Asp Ser Ser Val
    450                 455                 460
Ser Ser Gly Asn Glu Asp Ser Gly Ser Asp Ser Glu Gln Asp Glu Leu
465                 470                 475                 480
Lys Asp Ser Pro Ser Glu Thr Val Ser Ser Leu Gln Gly Pro Phe Leu
                485                 490                 495
Glu Glu Ser Ser Ala Phe Leu Ile Val Asp Gly Gly Val Arg Arg Asn
            500                 505                 510
Thr Ala Ile Gln Glu Ser Asp Ala Ser Gln Gly Lys Pro Leu Ala Ser
        515                 520                 525
Ser Trp Pro Leu Gly Val Ser Gly Pro Leu Ile Glu Glu Leu Gly Glu
    530                 535                 540
Gln Leu Lys Thr Thr Val Gln Val Ser Glu Pro Lys Gly Thr Thr Ala
545                 550                 555                 560
Val Asn Arg Ser Asn Ile Gln Glu Arg Asp Gly Cys Gln Thr Pro Asn
                565                 570                 575
Asn

<210> 248
<211> 471
<212> PRT
<213> Homo sapiens

<400> 248
Met Ser Arg Ile Pro Ala Ser Ser Asn Ser Thr Ser Ser Lys Leu Thr
1               5                   10                  15
His Ile Asn Asn Ser Arg Val Pro Lys Lys Leu Lys Lys Pro Ala Lys
            20                  25                  30
Pro Leu Leu Ser Lys Ile Lys Leu Arg Asn His Cys Lys Arg Leu Glu
        35                  40                  45
Gln Lys Asn Ala Ser Arg Lys Leu Glu Met Gly Asn Leu Val Leu Lys
    50                  55                  60
Glu Pro Lys Val Val Leu Tyr Lys Asn Leu Pro Ile Lys Lys Asp Lys
65                  70                  75                  80
Glu Pro Glu Gly Pro Ala Gln Ala Ala Val Ala Ser Gly Cys Leu Thr

                85                  90                  95
Arg His Ala Ala Arg Glu His Arg Gln Asn Pro Val Arg Gly Ala His
            100                 105                 110
Ser Gln Gly Glu Ser Ser Pro Cys Thr Tyr Ile Thr Arg Arg Ser Val
        115                 120                 125
Arg Thr Arg Thr Asn Leu Lys Glu Ala Ser Asp Ile Lys Leu Glu Pro
    130                 135                 140
Asn Thr Leu Asn Gly Tyr Lys Ser Ser Val Thr Glu Pro Cys Pro Asp
145                 150                 155                 160
Ser Gly Glu Gln Leu Gln Pro Ala Pro Val Leu Gln Glu Glu Glu Leu
                165                 170                 175
Ala His Glu Thr Ala Gln Lys Gly Glu Ala Lys Cys His Lys Ser Asp
            180                 185                 190
Thr Gly Met Ser Lys Lys Lys Ser Arg Gln Gly Lys Leu Val Lys Gln
        195                 200                 205
Phe Ala Lys Ile Glu Glu Ser Thr Pro Val His Asp Ser Pro Gly Lys
    210                 215                 220
Asp Asp Ala Val Pro Asp Leu Met Gly Pro His Ser Asp Gln Gly Glu
225                 230                 235                 240
His Ser Gly Thr Val Gly Val Pro Val Ser Tyr Thr Asp Cys Ala Pro
                245                 250                 255
Ser Pro Val Gly Cys Ser Val Val Thr Ser Asp Ser Phe Lys Thr Lys
            260                 265                 270
Asp Ser Phe Arg Thr Ala Lys Ser Lys Lys Lys Arg Arg Ile Thr Arg
        275                 280                 285
Tyr Asp Ala Gln Leu Ile Leu Glu Asn Asn Ser Gly Ile Pro Lys Leu
    290                 295                 300
Thr Leu Arg Arg Arg His Asp Ser Ser Ser Lys Thr Asn Asp Gln Glu
305                 310                 315                 320
Asn Asp Gly Met Asn Ser Ser Lys Ile Ser Ile Lys Leu Ser Lys Asp
                325                 330                 335
His Asp Asn Asp Asn Asn Leu Tyr Val Ala Lys Leu Asn Asn Gly Phe
            340                 345                 350
Asn Ser Gly Ser Gly Ser Ser Ser Thr Lys Leu Lys Ile Gln Leu Lys
        355                 360                 365
Arg Asp Glu Glu Asn Arg Gly Ser Tyr Thr Glu Gly Leu His Glu Asn
    370                 375                 380
Gly Val Cys Cys Ser Asp Pro Leu Ser Leu Leu Glu Ser Arg Met Glu
385                 390                 395                 400
Val Asp Asp Tyr Ser Gln Tyr Glu Glu Glu Ser Thr Asp Asp Ser Ser
                405                 410                 415
Ser Ser Glu Gly Asp Glu Glu Glu Asp Asp Tyr Asp Asp Asp Phe Glu
            420                 425                 430
Asp Asp Phe Ile Pro Leu Pro Pro Ala Lys Arg Leu Arg Leu Ile Val
        435                 440                 445
Gly Lys Asp Ser Ile Asp Ile Asp Ile Ser Ser Arg Arg Arg Glu Asp
    450                 455                 460
Gln Ser Leu Arg Leu Asn Ala
465                 470

<210> 249
<211> 166
<212> PRT
<213> Homo sapiens

<400> 249
Met Ala Met Ser Gln Glu Ser Leu Thr Phe Lys Asp Val Phe Val Asp

1                   5                   10                  15
Phe Thr Leu Glu Glu Trp Gln Gln Leu Asp Ser Ala Gln Lys Asn Leu
                20                  25                  30
Tyr Arg Asp Val Met Leu Glu Asn Tyr Ser His Leu Val Ser Val Gly
            35                  40                  45
Tyr Leu Val Ala Lys Pro Asp Val Ile Phe Arg Leu Gly Pro Gly Gly
    50                  55                  60
Glu Ser Trp Met Ala Asp Gly Gly Thr Pro Val Arg Thr Cys Ala Glu
65                  70                  75                  80
Val Trp Gln Val Asp Glu Gln Ile Asp His Tyr Lys Glu Ser Gln Asp
                85                  90                  95
Lys Leu Pro Trp Gln Ala Ala Phe Ile Gly Lys Glu Thr Leu Lys Asp
            100                 105                 110
Glu Ser Gly Gln Glu Ser Arg Thr Cys Arg Lys Ser Ile Tyr Leu Ser
        115                 120                 125
Thr Glu Phe Asp Ser Val Arg Gln Arg Leu Pro Lys Tyr Tyr Ser Trp
    130                 135                 140
Glu Lys Ala Phe Lys Thr Ser Phe Lys Leu Ser Trp Ser Lys Trp Lys
145                 150                 155                 160
Leu Cys Lys Lys Glu Arg
                165

<210> 250
<211> 708
<212> PRT
<213> Homo sapiens

<400> 250
Met Gln Lys Met Ile Pro Ala Ser Gln Arg Ala Val Thr Ser Pro Gly
1               5                   10                  15
Gln Ala Leu Ser Thr Val Val Lys Pro Leu Leu Gln Asn Thr Val Asp
                20                  25                  30
Lys Ile Leu Glu Ala Leu Gln Arg Val Phe Pro His Ala Glu Phe Arg
            35                  40                  45
Thr Asn Lys Thr Leu Asp Ser Asp Ile Ser Cys Pro Leu Leu Glu Ser
    50                  55                  60
Asp Leu Ala Tyr Ser Asp Asp Asp Val Pro Ser Val Tyr Glu Asn Gly
65                  70                  75                  80
Leu Ser Gln Lys Ser Ser His Lys Ala Lys Asp Asn Phe Asn Phe Leu
                85                  90                  95
His Leu Asn Arg Asn Ala Cys Tyr Gln Pro Met Ser Phe Arg Pro Arg
            100                 105                 110
Ile Leu Ile Val Gly Glu Pro Gly Phe Gly Gln Gly Ser His Leu Ala
        115                 120                 125
Pro Ala Val Ile His Ala Leu Glu Lys Phe Thr Val Tyr Thr Leu Asp
    130                 135                 140
Ile Pro Val Leu Phe Gly Val Ser Thr Thr Ser Pro Glu Glu Thr Cys
145                 150                 155                 160
Ala Gln Val Ile Arg Glu Ala Lys Arg Thr Ala Pro Ser Ile Val Tyr
                165                 170                 175
Val Pro His Ile His Val Trp Trp Glu Ile Val Gly Pro Thr Leu Lys
            180                 185                 190
Ala Thr Phe Thr Thr Leu Leu Gln Asn Ile Pro Ser Phe Ala Pro Val
        195                 200                 205
Leu Leu Leu Ala Thr Ser Asp Lys Pro His Ser Ala Leu Pro Glu Glu
    210                 215                 220
Val Gln Glu Leu Phe Ile Arg Asp Tyr Gly Glu Ile Phe Asn Val Gln

225                 230                 235                 240
Leu Pro Asp Lys Glu Glu Arg Thr Lys Phe Phe Glu Asp Leu Ile Leu
                245                 250                 255
Lys Gln Ala Ala Lys Pro Pro Ile Ser Lys Lys Lys Ala Val Leu Gln
            260                 265                 270
Ala Leu Glu Val Leu Pro Val Ala Pro Pro Pro Glu Pro Arg Ser Leu
        275                 280                 285
Thr Ala Glu Glu Val Lys Arg Leu Glu Glu Gln Glu Glu Asp Thr Phe
    290                 295                 300
Arg Glu Leu Arg Ile Phe Leu Arg Asn Val Thr His Arg Leu Ala Ile
305                 310                 315                 320
Asp Lys Arg Phe Arg Val Phe Thr Lys Pro Val Asp Pro Asp Glu Val
                325                 330                 335
Pro Asp Tyr Val Thr Val Ile Lys Gln Pro Met Asp Leu Ser Ser Val
            340                 345                 350
Ile Ser Lys Ile Asp Leu His Lys Tyr Leu Thr Val Lys Asp Tyr Leu
        355                 360                 365
Arg Asp Ile Asp Leu Ile Cys Ser Asn Ala Leu Glu Tyr Asn Pro Asp
    370                 375                 380
Arg Asp Pro Gly Asp Arg Leu Ile Arg His Arg Ala Cys Ala Leu Arg
385                 390                 395                 400
Asp Thr Ala Tyr Ala Ile Ile Lys Glu Glu Leu Asp Glu Asp Phe Glu
                405                 410                 415
Gln Leu Cys Glu Glu Ile Gln Glu Ser Arg Lys Lys Arg Gly Cys Ser
            420                 425                 430
Ser Ser Lys Tyr Ala Pro Ser Tyr Tyr His Val Met Pro Lys Gln Asn
        435                 440                 445
Ser Thr Leu Val Gly Asp Lys Arg Ser Asp Pro Glu Gln Asn Glu Lys
    450                 455                 460
Leu Lys Thr Pro Ser Thr Pro Val Ala Cys Ser Thr Pro Ala Gln Leu
465                 470                 475                 480
Lys Arg Lys Ile Arg Lys Lys Ser Asn Trp Tyr Leu Gly Thr Ile Lys
                485                 490                 495
Lys Arg Arg Lys Ile Ser Gln Ala Lys Asp Asp Ser Gln Asn Ala Ile
            500                 505                 510
Asp His Lys Ile Glu Ser Asp Thr Glu Glu Thr Gln Asp Thr Ser Val
        515                 520                 525
Asp His Asn Glu Thr Gly Asn Thr Gly Glu Ser Ser Val Glu Glu Asn
    530                 535                 540
Glu Lys Gln Gln Asn Ala Ser Glu Ser Lys Leu Glu Leu Arg Asn Asn
545                 550                 555                 560
Ser Asn Thr Cys Asn Ile Glu Asn Glu Leu Glu Asp Ser Arg Lys Thr
                565                 570                 575
Thr Ala Cys Thr Glu Leu Arg Asp Lys Ile Ala Cys Asn Gly Asp Ala
            580                 585                 590
Ser Ser Ser Gln Ile Ile His Ile Ser Asp Glu Asn Glu Gly Lys Glu
        595                 600                 605
Met Cys Val Leu Arg Met Thr Arg Ala Arg Arg Ser Gln Val Glu Gln
    610                 615                 620
Gln Gln Leu Ile Thr Val Glu Lys Ala Leu Ala Ile Leu Ser Gln Pro
625                 630                 635                 640
Thr Pro Ser Leu Val Val Asp His Glu Arg Leu Lys Asn Leu Leu Lys
                645                 650                 655
Thr Val Val Lys Lys Ser Gln Asn Tyr Asn Ile Phe Gln Leu Glu Asn
            660                 665                 670
Leu Tyr Ala Val Ile Ser Gln Cys Ile Tyr Arg His Arg Lys Asp His
        675                 680                 685
Asp Lys Thr Ser Leu Ile Gln Lys Met Glu Gln Glu Val Glu Asn Phe
    690                 695                 700
Ser Cys Ser Arg

705

<210> 251
<211> 518
<212> PRT
<213> Homo sapiens

<400> 251
Met Thr Ala Ser Pro Asp Tyr Leu Val Val Leu Phe Gly Ile Thr Ala
1               5                   10                  15
Gly Ala Thr Gly Ala Lys Leu Gly Ser Asp Glu Lys Glu Leu Ile Leu
            20                  25                  30
Leu Phe Trp Lys Val Val Asp Leu Ala Asn Lys Lys Val Gly Gln Leu
        35                  40                  45
His Glu Val Leu Val Arg Pro Asp Gln Leu Glu Leu Thr Glu Asp Cys
    50                  55                  60
Lys Glu Glu Thr Lys Ile Asp Val Glu Ser Leu Ser Ser Ala Ser Gln
65                  70                  75                  80
Leu Asp Gln Ala Leu Arg Gln Phe Asn Gln Ser Val Ser Asn Glu Leu
                85                  90                  95
Asn Ile Gly Val Gly Thr Ser Phe Cys Leu Cys Thr Asp Gly Gln Leu
            100                 105                 110
His Val Arg Gln Ile Leu His Pro Glu Ala Ser Lys Lys Asn Val Leu
        115                 120                 125
Leu Pro Glu Cys Phe Tyr Ser Phe Phe Asp Leu Arg Lys Glu Phe Lys
    130                 135                 140
Lys Cys Cys Pro Gly Ser Pro Asp Ile Asp Lys Leu Asp Val Ala Thr
145                 150                 155                 160
Met Thr Glu Tyr Leu Asn Phe Glu Lys Ser Ser Ser Val Ser Arg Tyr
                165                 170                 175
Gly Ala Ser Gln Val Glu Asp Met Gly Asn Ile Ile Leu Ala Met Ile
            180                 185                 190
Ser Glu Pro Tyr Asn His Arg Phe Ser Asp Pro Glu Arg Val Asn Tyr
        195                 200                 205
Lys Phe Glu Ser Gly Thr Cys Ser Lys Met Glu Leu Ile Asp Asp Asn
    210                 215                 220
Thr Val Val Arg Ala Arg Gly Leu Pro Trp Gln Ser Ser Asp Gln Asp
225                 230                 235                 240
Ile Ala Arg Phe Phe Lys Gly Leu Asn Ile Ala Lys Gly Gly Ala Ala
                245                 250                 255
Leu Cys Leu Asn Ala Gln Gly Arg Arg Asn Gly Glu Ala Leu Val Arg
            260                 265                 270
Phe Val Ser Glu Glu His Arg Asp Leu Ala Leu Gln Arg His Lys His
        275                 280                 285
His Met Gly Thr Arg Tyr Ile Glu Val Tyr Lys Ala Thr Gly Glu Asp
    290                 295                 300
Phe Leu Lys Ile Ala Gly Gly Thr Ser Asn Glu Val Ala Gln Phe Leu
305                 310                 315                 320
Ser Lys Glu Asn Gln Val Ile Val Arg Met Arg Gly Leu Pro Phe Thr
                325                 330                 335
Ala Thr Ala Glu Glu Val Val Ala Phe Phe Gly Gln His Cys Pro Ile
            340                 345                 350
Thr Gly Gly Lys Glu Gly Ile Leu Phe Val Thr Tyr Pro Asp Gly Arg
        355                 360                 365
Pro Thr Gly Asp Ala Phe Val Leu Phe Ala Cys Glu Glu Tyr Ala Gln
    370                 375                 380
Asn Ala Leu Arg Lys His Lys Asp Leu Leu Gly Lys Arg Tyr Ile Glu

385                 390                 395                 400
Leu Phe Arg Ser Thr Ala Ala Glu Val Gln Gln Val Leu Asn Arg Phe
                405                 410                 415
Ser Ser Ala Pro Leu Ile Pro Leu Pro Thr Pro Pro Ile Ile Pro Val
            420                 425                 430
Leu Pro Gln Gln Phe Val Pro Pro Thr Asn Val Arg Asp Cys Ile Arg
        435                 440                 445
Leu Arg Gly Leu Pro Tyr Ala Ala Thr Ile Glu Asp Ile Leu Asp Phe
    450                 455                 460
Leu Gly Glu Phe Ala Thr Asp Ile Arg Thr His Gly Val His Met Val
465                 470                 475                 480
Leu Asn His Gln Gly Arg Pro Ser Gly Asp Ala Phe Ile Gln Met Lys
                485                 490                 495
Ser Ala Asp Arg Ala Phe Met Ala Ala Gln Lys Cys His Lys Lys Lys
            500                 505                 510
His Glu Gly Gln Ile Cys
        515

<210> 252
<211> 89
<212> PRT
<213> Homo sapiens

<400> 252
Met Ser Asp Arg Lys Ala Val Ile Lys Asn Ala Asp Met Ser Glu Asp
1               5                   10                  15
Met Gln Gln Asp Ala Val Asp Cys Ala Thr Gln Ala Met Glu Lys Tyr
            20                  25                  30
Asn Ile Glu Lys Asp Ile Ala Ala Tyr Ile Lys Lys Glu Phe Asp Lys
        35                  40                  45
Lys Tyr Asn Pro Thr Trp His Cys Ile Val Gly Arg Asn Phe Gly Ser
    50                  55                  60
Tyr Val Thr His Glu Thr Lys His Phe Ile Tyr Phe Tyr Leu Gly Gln
65                  70                  75                  80
Val Ala Ile Leu Leu Phe Lys Ser Gly
                85

<210> 253
<211> 94
<212> PRT
<213> Homo sapiens

<400> 253

<210> 254
<211> 207
<212> PRT
<213> Homo sapiens

<400> 254
Met Pro Glu Gly Pro Leu Val Arg Lys Phe His His Leu Val Ser Pro
1               5                   10                  15
Leu Val Gly Gln Gln Val Val Lys Thr Gly Gly Ser Ser Lys Lys Leu
            20                  25                  30
Gln Pro Ala Ser Leu Gln Ser Leu Trp Leu Gln Asp Thr Gln Val His
        35                  40                  45
Gly Lys Lys Leu Phe Leu Arg Phe Asp Leu Asp Glu Glu Met Gly Pro
    50                  55                  60
Pro Gly Ser Ser Pro Thr Pro Glu Pro Pro Gln Lys Glu Val Gln Lys
65                  70                  75                  80
Glu Gly Ala Ala Asp Pro Lys Gln Val Gly Glu Pro Ser Gly Gln Lys
                85                  90                  95
Thr Leu Asp Gly Ser Ser Arg Ser Ala Glu Leu Val Pro Gln Gly Glu
            100                 105                 110
Asp Asp Ser Glu Tyr Leu Glu Arg Asp Ala Pro Ala Gly Asp Ala Gly
        115                 120                 125
Arg Trp Leu Arg Val Ser Phe Gly Leu Phe Gly Ser Val Trp Val Asn
    130                 135                 140
Asp Phe Ser Arg Ala Lys Lys Ala Asn Lys Arg Gly Asp Trp Arg Asp
145                 150                 155                 160
Pro Ser Pro Arg Leu Val Leu His Phe Gly Gly Gly Gly Phe Leu Ala
                165                 170                 175
Phe Tyr Asn Cys Gln Leu Ser Trp Ser Ser Ser Pro Val Val Thr Pro
            180                 185                 190
Thr Cys Asp Ile Leu Ser Glu Lys Phe His Arg Gly Gln Ala Leu
        195                 200                 205

<210> 255
<211> 279
<212> PRT
<213> Homo sapiens

<400> 255
Met Ala Ala Leu Gly Arg Pro Phe Ser Gly Leu Pro Leu Ser Gly Gly
1               5                   10                  15
Ser Asp Phe Leu Gln Pro Pro Gln Pro Ala Phe Pro Gly Arg Ala Phe
            20                  25                  30
Pro Pro Gly Ala Asp Gly Ala Glu Leu Ala Pro Arg Pro Gly Pro Arg
        35                  40                  45
Ala Val Pro Ser Ser Pro Ala Gly Ser Ala Ala Arg Gly Arg Val Ser
    50                  55                  60
Val His Cys Lys Lys Lys His Lys Arg Glu Glu Glu Glu Asp Asp Asp
65                  70                  75                  80
Cys Pro Val Arg Lys Lys Arg Ile Thr Glu Ala Glu Leu Cys Ala Gly
                85                  90                  95
Pro Asn Asp Trp Ile Leu Cys Ala His Gln Asp Val Glu Gly His Gly

                100                 105                 110
Val Asn Pro Ser Val Ser Gly Leu Ser Ile Pro Gly Ile Leu Asp Val
        115                 120                 125
Ile Cys Glu Glu Met Asp Gln Thr Thr Gly Glu Pro Gln Cys Glu Val
    130                 135                 140
Ala Arg Arg Lys Leu Gln Glu Ile Glu Asp Arg Ile Ile Asp Glu Asp
145                 150                 155                 160
Glu Glu Val Glu Ala Asp Arg Asn Val Asn His Leu Pro Ser Leu Val
                165                 170                 175
Leu Ser Asp Thr Met Lys Thr Gly Leu Lys Arg Glu Phe Asp Glu Val
            180                 185                 190
Phe Thr Lys Lys Met Ile Glu Ser Met Ser Arg Pro Ser Met Glu Leu
        195                 200                 205
Val Leu Trp Lys Pro Leu Pro Glu Leu Leu Ser Asp Lys Pro Lys Pro
    210                 215                 220
Ser Ser Asn Thr Lys Asn Tyr Thr Gly Glu Ser Gln Ala Lys His Val
225                 230                 235                 240
Ala Ala Gly Thr Ala Phe Pro Gln Arg Thr Glu Leu Phe Ser Glu Pro
                245                 250                 255
Arg Pro Thr Gly Met Ser Leu Tyr Asn Ser Leu Glu Thr Ala Thr Ser
            260                 265                 270
Thr Glu Glu Glu Met Glu Leu
        275

<210> 256
<211> 141
<212> PRT
<213> Homo sapiens

<400> 256
Met Thr Leu Lys Met Gln Tyr Leu Phe Leu Ser Phe Phe Leu Ser Phe
  1               5                  10                  15
Phe Leu Arg Gln Glu Leu Arg Pro Val Ala Gln Ala Gly Val Leu Glu
             20                  25                  30
Cys Ser Gly Ala Ile Ser Ala His Cys Ser Leu Cys Phe Pro Gly Ser
         35                  40                  45
Ser Val Ser Pro Ala Ser Ala Phe Arg Val Ala Gly Ile Thr Gly Met
     50                  55                  60
Tyr His Tyr Ala Trp Leu Ile Phe Ile Ser Ile Phe Leu Val Glu Met
 65                  70                  75                  80
Gly Phe His His Val Cys Gln Ala Gly Leu Glu Leu Leu Ala Ser Ser
                 85                  90                  95
Asp Pro Pro Thr Ser Ala Ser Gln Ser Ala Gly Ile Thr Gly Ala Ser
            100                 105                 110
His Arg Ala Trp Pro Lys Met Gln Ile Leu Val Trp Ile Tyr Ala Leu
        115                 120                 125
Pro Leu Pro Ser Ile Phe Leu Ser Val Ala Leu Leu Ala
    130                 135                 140

<210> 257
<211> 58
<212> PRT
<213> Homo sapiens

<400> 257
Met Gln Arg Glu Asp Gly Thr Pro Ser Met Gly Cys Pro Leu Thr Leu
1               5                   10                  15
Pro Asp Ser Ser Asp Leu Arg Phe Leu Ala Phe Glu Pro Ala Pro Pro
            20                  25                  30
Thr Leu Pro Ser Ala Arg Pro Arg Leu Leu Gln Ala Ser Val Thr Leu
        35                  40                  45
Ser Leu Lys Glu Arg Ala Pro Leu Ile Leu
    50                  55

<210> 258
<211> 1444
<212> PRT
<213> Homo sapiens

<400> 258
Met Lys Asn Lys Gly Ala Lys Gln Lys Leu Lys Arg Lys Gly Ala Ala
1               5                   10                  15
Ser Ala Phe Gly Cys Asp Leu Thr Glu Tyr Leu Glu Ser Ser Gly Gln
            20                  25                  30
Asp Val Pro Tyr Val Leu Lys Ser Cys Ala Glu Phe Ile Glu Thr His
        35                  40                  45
Gly Ile Val Asp Gly Ile Tyr Arg Leu Ser Gly Val Thr Ser Asn Ile
    50                  55                  60
Gln Arg Leu Arg Gln Glu Phe Gly Ser Asp Gln Cys Pro Asp Leu Thr
65                  70                  75                  80
Arg Glu Val Tyr Leu Gln Asp Ile His Cys Val Gly Ser Leu Cys Lys
                85                  90                  95
Leu Tyr Phe Arg Glu Leu Pro Asn Pro Leu Leu Thr Tyr Glu Leu Tyr
            100                 105                 110
Glu Lys Phe Thr Glu Ala Val Ser His Cys Pro Glu Glu Gly Gln Leu
        115                 120                 125
Ala Arg Ile Gln Asn Val Ile Gln Glu Leu Pro Pro Ser His Tyr Arg
    130                 135                 140
Thr Leu Glu Tyr Leu Ile Arg His Leu Ala His Ile Ala Ser Phe Ser
145                 150                 155                 160
Ser Lys Thr Asn Met His Ala Arg Asn Leu Ala Leu Val Trp Ala Pro
                165                 170                 175
Asn Leu Leu Arg Ser Lys Glu Ile Glu Ala Thr Gly Cys Asn Gly Asp
            180                 185                 190
Ala Ala Phe Leu Ala Val Arg Val Gln Gln Val Val Ile Glu Phe Ile
        195                 200                 205
Leu Asn His Val Asp Gln Ile Phe Asn Asn Gly Ala Pro Gly Ser Leu
    210                 215                 220
Glu Asn Asp Glu Asn Arg Pro Ile Met Lys Ser Leu Thr Leu Pro Ala
225                 230                 235                 240
Leu Ser Leu Pro Met Lys Leu Val Ser Leu Glu Glu Ala Gln Ala Arg
                245                 250                 255
Ser Leu Ala Thr Asn His Pro Ala Arg Lys Glu Arg Arg Glu Asn Ser
            260                 265                 270
Leu Pro Glu Ile Val Pro Pro Met Gly Thr Leu Phe His Thr Val Leu
        275                 280                 285
Glu Leu Pro Asp Asn Lys Arg Lys Leu Ser Ser Lys Ser Lys Lys Trp
    290                 295                 300
Lys Ser Ile Phe Asn Leu Gly Arg Ser Gly Ser Asp Ser Lys Ser Lys
305                 310                 315                 320
Leu Ser Arg Asn Gly Ser Val Phe Val Arg Gly Gln Arg Leu Ser Val

                325                 330                 335
Glu Lys Ala Thr Ile Arg Pro Ala Lys Ser Met Asp Ser Leu Cys Ser
            340                 345                 350
Val Pro Val Glu Gly Lys Glu Thr Lys Gly Asn Phe Asn Arg Thr Val
        355                 360                 365
Thr Thr Gly Gly Phe Phe Ile Pro Ala Thr Lys Met His Ser Thr Gly
    370                 375                 380
Thr Gly Ser Ser Cys Asp Leu Thr Lys Gln Glu Gly Glu Trp Gly Gln
385                 390                 395                 400
Glu Gly Met Pro Pro Gly Ala Glu Gly Gly Phe Asp Val Ser Ser Asp
                405                 410                 415
Arg Ser His Leu Gln Gly Ala Gln Ala Arg Pro Pro Pro Glu Gln Leu
            420                 425                 430
Lys Val Phe Arg Pro Val Glu Asp Pro Glu Ser Glu Gln Thr Ala Pro
        435                 440                 445
Lys Met Leu Gly Met Phe Tyr Thr Ser Asn Asp Ser Pro Ser Lys Ser
    450                 455                 460
Val Phe Thr Ser Ser Leu Phe Pro Met Glu Pro Ser Pro Arg Asn Gln
465                 470                 475                 480
Arg Lys Ala Leu Asn Ile Ser Glu Pro Phe Ala Val Ser Val Pro Leu
                485                 490                 495
Arg Val Ser Ala Val Ile Ser Thr Asn Ser Thr Pro Cys Arg Thr Pro
            500                 505                 510
Pro Lys Glu Leu Gln Ser Leu Ser Ser Leu Glu Glu Phe Ser Phe His
        515                 520                 525
Gly Ser Glu Ser Gly Gly Trp Pro Glu Glu Glu Lys Pro Leu Gly Ala
    530                 535                 540
Glu Thr Ser Ala Ala Ser Val Pro Lys Lys Ala Gly Leu Glu Asp Ala
545                 550                 555                 560
Lys Ala Val Pro Glu Ala Pro Gly Thr Val Glu Cys Ser Lys Gly Leu
                565                 570                 575
Ser Gln Glu Pro Gly Ala His Leu Glu Glu Lys Lys Thr Pro Glu Ser
            580                 585                 590
Ser Leu Ser Ser Gln His Leu Asn Glu Leu Glu Lys Arg Pro Asn Pro
        595                 600                 605
Glu Lys Val Val Glu Glu Gly Arg Glu Ala Gly Glu Met Glu Ser Ser
    610                 615                 620
Thr Leu Gln Glu Ser Pro Arg Ala Arg Ala Glu Ala Val Leu Leu His
625                 630                 635                 640
Glu Met Asp Glu Asp Asp Leu Ala Asn Ala Leu Ile Trp Pro Glu Ile
                645                 650                 655
Gln Gln Glu Leu Lys Ile Ile Glu Ser Glu Glu Glu Leu Ser Ser Leu
            660                 665                 670
Pro Pro Pro Ala Leu Lys Thr Ser Pro Ile Gln Pro Ile Leu Glu Ser
        675                 680                 685
Ser Leu Gly Pro Phe Ile Pro Ser Glu Pro Pro Gly Ser Leu Pro Cys
    690                 695                 700
Gly Ser Phe Pro Ala Pro Val Ser Thr Pro Leu Glu Val Trp Thr Arg
705                 710                 715                 720
Asp Pro Ala Asn Gln Ser Thr Gln Gly Ala Ser Thr Ala Ala Ser Arg
                725                 730                 735
Glu Lys Pro Glu Pro Glu Gln Gly Leu His Pro Asp Leu Ala Ser Leu
            740                 745                 750
Ala Pro Leu Glu Ile Val Pro Phe Glu Lys Ala Ser Pro Gln Ala Thr
        755                 760                 765
Val Glu Val Gly Gly Pro Gly Asn Leu Ser Pro Pro Leu Pro Pro Ala
    770                 775                 780
Pro Pro Pro Pro Thr Pro Leu Glu Glu Ser Thr Pro Val Leu Leu Ser
785                 790                 795                 800
Lys Gly Ser Pro Glu Arg Glu Asp Ser Ser Arg Lys Leu Arg Thr Asp

                805                 810                 815
Leu Tyr Ile Asp Gln Leu Lys Ser Gln Asp Ser Pro Glu Ile Ser Ser
            820                 825                 830
Leu Cys Gln Gly Glu Glu Ala Thr Pro Arg His Ser Asp Lys Gln Asn
        835                 840                 845
Ser Lys Asn Ala Ala Ser Glu Gly Lys Gly Cys Gly Phe Pro Ser Pro
    850                 855                 860
Thr Arg Glu Val Glu Ile Val Ser Gln Glu Glu Glu Asp Val Thr His
865                 870                 875                 880
Ser Val Gln Glu Pro Ser Asp Cys Asp Glu Asp Asp Thr Val Thr Asp
                885                 890                 895
Ile Ala Gln His Gly Leu Glu Met Val Glu Pro Trp Glu Glu Pro Gln
            900                 905                 910
Trp Val Thr Ser Pro Leu His Ser Pro Thr Leu Lys Asp Ala His Lys
        915                 920                 925
Ala Gln Val Gln Gly Leu Gln Gly His Gln Leu Glu Lys Arg Leu Ser
    930                 935                 940
His Arg Pro Ser Leu Arg Gln Ser His Ser Leu Asp Ser Lys Pro Thr
945                 950                 955                 960
Val Lys Ser Gln Trp Thr Leu Glu Val Pro Ser Ser Ser Ser Cys Ala
                965                 970                 975
Asn Leu Glu Thr Glu Arg Asn Ser Asp Pro Leu Gln Pro Gln Ala Pro
            980                 985                 990
Arg Arg Glu Ile Thr Gly Trp Asp Glu Lys Ala Leu Arg Ser Phe Arg
        995                 1000                1005
Glu Phe Ser Gly Leu Lys Gly Ala Glu Ala Pro Pro Asn Gln Lys Gly
    1010                1015                1020
Pro Ser Gly Val Gln Pro Asn Pro Ala Glu Thr Ser Pro Ile Ser Leu
1025                1030                1035                1040
Ala Glu Gly Lys Glu Leu Gly Thr His Leu Gly His Ser Ser Pro Gln
                1045                1050                1055
Ile Arg Gln Gly Gly Val Pro Gly Pro Glu Ser Ser Lys Glu Ser Ser
            1060                1065                1070
Pro Ser Val Gln Asp Ser Thr Ser Pro Gly Glu His Pro Ala Lys Leu
        1075                1080                1085
Gln Leu Lys Ser Thr Glu Cys Gly Pro Pro Lys Gly Lys Asn Arg Pro
    1090                1095                1100
Ser Ser Leu Asn Leu Asp Pro Ala Ile Pro Ile Ala Asp Leu Phe Trp
1105                1110                1115                1120
Phe Glu Asn Val Ala Ser Phe Ser Ser Pro Gly Met Gln Val Ser Glu
                1125                1130                1135
Pro Gly Asp Pro Lys Val Thr Trp Met Thr Ser Ser Tyr Cys Lys Ala
            1140                1145                1150
Asp Pro Trp Arg Val Tyr Ser Gln Asp Pro Gln Asp Leu Asp Ile Val
        1155                1160                1165
Ala His Ala Leu Thr Gly Arg Arg Asn Ser Ala Pro Val Ser Val Ser
    1170                1175                1180
Ala Val Arg Thr Ser Phe Met Val Lys Met Cys Gln Ala Arg Ala Val
1185                1190                1195                1200
Pro Val Ile Pro Pro Lys Ile Gln Tyr Thr Gln Ile Pro Gln Pro Leu
                1205                1210                1215
Pro Ser Gln Ser Ser Gly Glu Asn Gly Val Gln Pro Leu Glu Arg Ser
            1220                1225                1230
Gln Glu Gly Pro Ser Ser Thr Ser Gly Thr Thr Gln Lys Pro Ala Lys
        1235                1240                1245
Asp Asp Ser Pro Ser Ser Leu Glu Ser Ser Lys Glu Glu Lys Pro Lys
    1250                1255                1260
Gln Asp Pro Gly Ala Ile Lys Ser Ser Pro Val Asp Ala Thr Ala Pro
1265                1270                1275                1280
Cys Met Cys Glu Gly Pro Thr Leu Ser Pro Glu Pro Gly Ser Ser Asn

1285                    1290                    1295
Leu Leu Ser Thr Gln Asp Ala Val Val Gln Cys Arg Lys Arg Met Ser
        1300                    1305                    1310
Glu Thr Glu Pro Ser Gly Asp Asn Leu Leu Ser Ser Lys Leu Glu Arg
    1315                    1320                    1325
Pro Ser Gly Gly Ser Lys Pro Phe His Arg Ser Arg Pro Gly Arg Pro
1330                    1335                    1340
Gln Ser Leu Ile Leu Phe Ser Pro Pro Phe Pro Ile Met Asp His Leu
1345                    1350                    1355                    1360
Pro Pro Ser Ser Thr Val Thr Asp Ser Lys Val Leu Leu Ser Pro Ile
                1365                    1370                    1375
Arg Ser Pro Thr Gln Thr Val Ser Pro Gly Leu Leu Cys Gly Glu Leu
        1380                    1385                    1390
Ala Glu Asn Thr Trp Val Thr Pro Glu Gly Val Thr Leu Arg Asn Lys
    1395                    1400                    1405
Met Thr Ile Pro Lys Asn Gly Gln Arg Leu Glu Thr Ser Thr Ser Cys
1410                    1415                    1420
Phe Tyr Gln Pro Gln Arg Arg Ser Val Ile Leu Asp Gly Arg Ser Gly
1425                    1430                    1435                    1440
Arg Gln Ile Glu

<210> 259
<211> 167
<212> PRT
<213> Homo sapiens

<400> 259
Met Arg His Lys His Pro Leu Glu Leu His Arg Ser Cys Ala Leu Leu
1               5                   10                  15
Ser Ser Gly Ile Ser Leu Glu Asn Ser Ser Gln Gln Leu Met Glu Val
            20                  25                  30
Ser Pro Val His Arg Leu Cys Met Asp Phe Ala Gln Val Pro Phe Pro
        35                  40                  45
Ser Cys Ala Asp Thr Cys Ile Phe Gly Leu His Val Ser Thr Cys Pro
    50                  55                  60
Leu His Ser Asp Phe Phe Phe Lys Arg Gln His Leu Thr Leu Ser Pro
65                  70                  75                  80
Arg Leu Glu Tyr Ser Gly Val Ile Thr Ala His Cys Ser Leu Lys Leu
                85                  90                  95
Leu Gly Ser Gly Asn Pro Pro Ala Ser Ala Ser Arg Val Ala Gly Ser
            100                 105                 110
Thr Gly Ala Cys His His Ala His Leu Ile Phe Leu Ser Phe Ile Asp
        115                 120                 125
Thr Gly Leu Thr Val Ala Gln Ala Gly Leu Glu Leu Leu Ala Ser Ser
    130                 135                 140
Asp Pro Pro Ala Leu Ala Tyr Gln Asn Ala Gly Ile Ile Gly Met Asn
145                 150                 155                 160
His Cys Ala Trp Pro Ser Leu
                165

<210> 260
<211> 294
<212> PRT
<213> Homo sapiens

<400> 260
Met Glu Lys Ala Lys Gly Lys Glu Trp Thr Ser Thr Glu Lys Ser Arg
1               5                   10                  15
Glu Glu Asp Gln Gln Ala Ser Asn Gln Pro Asn Ser Ile Ala Leu Pro
            20                  25                  30
Gly Thr Ser Ala Lys Arg Thr Lys Glu Lys Met Ser Val Lys Gly Ser
        35                  40                  45
Lys Val Leu Cys Pro Lys Lys Lys Ala Glu His Thr Asp Asn Pro Arg
    50                  55                  60
Pro Gln Lys Lys Ile Pro Ile Pro Pro Leu Pro Ser Lys Leu Pro Pro
65                  70                  75                  80
Val Asn Leu Ile His Arg Asp Ile Leu Arg Ala Trp Cys Gln Gln Leu
                85                  90                  95
Lys Leu Ser Ser Lys Gly Gln Lys Leu Asp Ala Tyr Lys Arg Leu Cys
            100                 105                 110
Ala Phe Ala Tyr Pro Asn Gln Lys Asp Phe Pro Ser Thr Ala Lys Glu
        115                 120                 125
Ala Lys Ile Arg Lys Ser Leu Gln Lys Lys Leu Lys Val Glu Lys Gly
    130                 135                 140
Glu Thr Ser Leu Gln Ser Ser Glu Thr His Pro Pro Glu Val Ala Leu
145                 150                 155                 160
Pro Pro Val Gly Glu Pro Pro Ala Leu Glu Asn Ser Thr Ala Leu Leu
                165                 170                 175
Glu Gly Val Asn Thr Val Val Val Thr Thr Ser Ala Pro Glu Ala Leu
            180                 185                 190
Leu Ala Ser Trp Ala Arg Ile Ser Ala Arg Ala Arg Thr Pro Glu Ala
        195                 200                 205
Val Glu Ser Pro Gln Glu Ala Ser Gly Val Arg Trp Cys Val Val His
    210                 215                 220
Gly Lys Ser Leu Pro Ala Asp Thr Asp Gly Trp Val His Leu Gln Phe
225                 230                 235                 240
His Ala Gly Gln Ala Trp Val Pro Glu Lys Gln Glu Gly Arg Val Ser
                245                 250                 255
Ala Leu Phe Leu Leu Pro Ala Ser Asn Phe Pro Pro Pro His Leu Glu
            260                 265                 270
Asp Asn Met Leu Cys Pro Lys Cys Val His Arg Asn Lys Val Leu Ile
        275                 280                 285
Lys Ser Leu Gln Trp Glu
    290

<210> 261
<211> 48
<212> PRT
<213> Homo sapiens

<400> 261
Met Met Pro Pro Gly Asp Gln Gly Gly Pro Gly Ala Pro Ala Phe Cys
1               5                   10                  15
Glu Asn His Leu Gly Gln Ser Phe Arg Gly Gly Gly Lys Ser Leu Gly
            20                  25                  30
Pro Ser Leu Pro Gly Ser Pro Leu Gln Gly His Gly Phe Thr Asn Met
        35                  40                  45

<210> 262
<211> 157
<212> PRT
<213> Homo sapiens

<400> 262
Met Ser Gly Ser Leu Gly Arg Ala Ala Ala Ala Leu Leu Arg Trp Gly
1               5                   10                  15
Arg Gly Ala Gly Gly Gly Gly Leu Trp Gly Pro Gly Val Arg Ala Ala
            20                  25                  30
Gly Ser Gly Ala Gly Gly Gly Gly Ser Ala Glu Gln Leu Asp Ala Leu
        35                  40                  45
Val Lys Lys Asp Lys Val Val Val Phe Leu Lys Gly Thr Pro Glu Gln
    50                  55                  60
Pro Gln Cys Gly Phe Ser Asn Ala Val Val Gln Ile Leu Arg Leu His
65                  70                  75                  80
Gly Val Arg Asp Tyr Ala Ala Tyr Asn Val Leu Asp Asp Pro Glu Leu
                85                  90                  95
Arg Gln Gly Ile Lys Asp Tyr Ser Asn Trp Pro Thr Ile Pro Gln Val
            100                 105                 110
Tyr Leu Asn Gly Glu Phe Val Gly Gly Cys Asp Ile Leu Leu Gln Met
        115                 120                 125
His Gln Asn Gly Asp Leu Val Glu Glu Leu Lys Lys Leu Gly Ile His
    130                 135                 140
Ser Ala Leu Leu Asp Glu Lys Lys Asp Gln Asp Ser Lys
145                 150                 155

<210> 263
<211> 997
<212> PRT
<213> Homo sapiens

<400> 263
Met Glu Glu Met Phe His Lys Lys Ser Glu Ala Val Arg Arg Leu Val
1               5                   10                  15
Glu Ala Ala Glu Glu Ala His Leu Lys His Glu Phe Asp Ala Asp Leu
            20                  25                  30
Gln Tyr Glu Tyr Phe Asn Ala Val Leu Ile Asn Glu Arg Asp Lys Asp
        35                  40                  45
Gly Asn Phe Leu Glu Leu Gly Lys Glu Phe Ile Leu Ala Pro Asn Asp
    50                  55                  60
His Phe Asn Asn Leu Pro Val Asn Ile Ser Leu Ser Asp Val Gln Val
65                  70                  75                  80
Pro Thr Asn Met Tyr Asn Lys Asp Pro Ala Ile Val Asn Gly Val Tyr
                85                  90                  95
Trp Ser Glu Ser Leu Asn Lys Val Phe Val Asp Asn Phe Asp Arg Asp
            100                 105                 110
Pro Ser Leu Ile Trp Gln Tyr Phe Gly Ser Ala Lys Gly Phe Phe Arg
        115                 120                 125
Gln Tyr Pro Gly Ile Lys Trp Glu Pro Asp Glu Asn Gly Val Ile Ala
    130                 135                 140
Phe Asp Cys Arg Asn Arg Lys Trp Tyr Ile Gln Ala Ala Thr Ser Pro
145                 150                 155                 160
Lys Asp Val Val Ile Leu Val Asp Val Ser Gly Ser Met Lys Gly Leu

                165                 170                 175
Arg Leu Thr Ile Ala Lys Gln Thr Val Ser Ser Ile Leu Asp Thr Leu
            180                 185                 190
Gly Asp Asp Asp Phe Phe Asn Ile Ile Ala Tyr Asn Glu Glu Leu His
        195                 200                 205
Tyr Val Glu Pro Cys Leu Asn Gly Thr Leu Val Gln Ala Asp Arg Thr
    210                 215                 220
Asn Lys Glu His Phe Arg Glu His Leu Asp Lys Leu Phe Ala Lys Gly
225                 230                 235                 240
Ile Gly Met Leu Asp Ile Ala Leu Asn Glu Ala Phe Asn Ile Leu Ser
                245                 250                 255
Asp Phe Asn His Thr Gly Gln Gly Ser Ile Cys Ser Gln Ala Ile Met
            260                 265                 270
Leu Ile Thr Asp Gly Ala Val Asp Thr Tyr Asp Thr Ile Phe Ala Lys
        275                 280                 285
Tyr Asn Trp Pro Asp Arg Lys Val Arg Ile Phe Thr Tyr Leu Ile Gly
    290                 295                 300
Arg Glu Ala Ala Phe Ala Asp Asn Leu Lys Trp Met Ala Cys Ala Asn
305                 310                 315                 320
Lys Gly Phe Phe Thr Gln Ile Ser Thr Leu Ala Asp Val Gln Glu Asn
                325                 330                 335
Val Met Glu Tyr Leu His Val Leu Ser Arg Pro Lys Val Ile Asp Gln
            340                 345                 350
Glu His Asp Val Val Trp Thr Glu Ala Tyr Ile Asp Ser Thr Leu Pro
        355                 360                 365
Gln Ala Gln Lys Leu Thr Asp Asp Gln Gly Pro Val Leu Met Thr Thr
    370                 375                 380
Val Ala Met Pro Val Phe Ser Lys Gln Asn Glu Thr Arg Ser Lys Gly
385                 390                 395                 400
Ile Leu Leu Gly Val Val Gly Thr Asp Val Pro Val Lys Glu Leu Leu
                405                 410                 415
Lys Thr Ile Pro Lys Tyr Lys Leu Gly Ile His Gly Tyr Ala Phe Ala
            420                 425                 430
Ile Thr Asn Asn Gly Tyr Ile Leu Thr His Pro Glu Leu Arg Leu Leu
        435                 440                 445
Tyr Glu Glu Gly Lys Lys Arg Arg Lys Pro Asn Tyr Ser Ser Val Asp
    450                 455                 460
Leu Ser Glu Val Glu Trp Glu Asp Arg Asp Asp Val Leu Arg Asn Ala
465                 470                 475                 480
Met Val Asn Arg Lys Thr Gly Lys Phe Ser Met Glu Val Lys Lys Thr
                485                 490                 495
Val Asp Lys Gly Lys Arg Val Leu Val Met Thr Asn Asp Tyr Tyr Tyr
            500                 505                 510
Thr Asp Ile Lys Gly Thr Pro Phe Ser Leu Gly Val Ala Leu Ser Arg
        515                 520                 525
Gly His Gly Lys Tyr Phe Phe Arg Gly Asn Val Thr Ile Glu Glu Gly
    530                 535                 540
Leu His Asp Leu Glu His Pro Asp Val Ser Leu Ala Asp Glu Trp Ser
545                 550                 555                 560
Tyr Cys Asn Thr Asp Leu His Pro Glu His Arg His Leu Ser Gln Leu
                565                 570                 575
Glu Ala Ile Lys Leu Tyr Leu Lys Gly Lys Glu Pro Leu Leu Gln Cys
            580                 585                 590
Asp Lys Glu Leu Ile Gln Glu Val Leu Phe Asp Ala Val Val Ser Ala
        595                 600                 605
Pro Ile Glu Ala Tyr Trp Thr Ser Leu Ala Leu Asn Lys Ser Glu Asn
    610                 615                 620
Ser Asp Lys Gly Val Glu Val Ala Phe Leu Gly Thr Arg Thr Gly Leu
625                 630                 635                 640
Ser Arg Ile Asn Leu Phe Val Gly Ala Glu Gln Leu Thr Asn Gln Asp

645                 650                 655
Phe Leu Lys Ala Gly Asp Lys Glu Asn Ile Phe Asn Ala Asp His Phe
            660                 665                 670
Pro Leu Trp Tyr Arg Arg Ala Ala Glu Gln Ile Pro Gly Ser Phe Val
        675                 680                 685
Tyr Ser Ile Pro Phe Ser Thr Gly Pro Val Asn Lys Ser Asn Val Val
    690                 695                 700
Thr Ala Ser Thr Ser Ile Gln Leu Leu Asp Glu Arg Lys Ser Pro Val
705                 710                 715                 720
Val Ala Ala Val Gly Ile Gln Met Lys Leu Glu Phe Phe Gln Arg Lys
                725                 730                 735
Phe Trp Thr Ala Ser Arg Gln Cys Ala Ser Leu Asp Gly Lys Cys Ser
            740                 745                 750
Ile Ser Cys Asp Asp Glu Thr Val Asn Cys Tyr Leu Ile Asp Asn Asn
        755                 760                 765
Gly Phe Ile Leu Val Ser Glu Asp Tyr Thr Gln Thr Gly Asp Phe Phe
    770                 775                 780
Gly Glu Ile Glu Gly Ala Val Met Asn Lys Leu Leu Thr Met Gly Ser
785                 790                 795                 800
Phe Lys Arg Ile Thr Leu Tyr Asp Tyr Gln Ala Met Cys Arg Ala Asn
                805                 810                 815
Lys Glu Ser Ser Asp Gly Ala His Gly Leu Leu Asp Pro Tyr Asn Ala
            820                 825                 830
Phe Leu Ser Ala Val Lys Trp Ile Met Thr Glu Leu Val Leu Phe Leu
        835                 840                 845
Val Glu Phe Asn Leu Cys Ser Trp Trp His Ser Asp Met Thr Ala Lys
    850                 855                 860
Ala Gln Lys Leu Lys Gln Thr Leu Glu Pro Cys Asp Thr Glu Tyr Pro
865                 870                 875                 880
Ala Phe Val Ser Glu Arg Thr Ile Lys Glu Thr Thr Gly Asn Ile Ala
                885                 890                 895
Cys Glu Asp Cys Ser Lys Ser Phe Val Ile Gln Gln Ile Pro Ser Ser
            900                 905                 910
Asn Leu Phe Met Val Val Val Asp Ser Ser Cys Leu Cys Glu Ser Val
        915                 920                 925
Ala Pro Ile Thr Met Ala Pro Ile Glu Ile Arg Tyr Asn Glu Ser Leu
    930                 935                 940
Lys Cys Glu Arg Leu Lys Ala Gln Lys Ile Arg Arg Arg Pro Glu Ser
945                 950                 955                 960
Cys His Gly Phe His Pro Glu Glu Asn Ala Arg Glu Cys Gly Gly Ala
                965                 970                 975
Pro Ser Leu Gln Ala Gln Thr Val Leu Leu Leu Leu Pro Leu Leu Leu
            980                 985                 990
Met Leu Phe Ser Arg
        995

<210> 264
<211> 449
<212> PRT
<213> Homo sapiens

<400> 264
Met Pro Ala Thr Leu Leu Arg Ala Val Ala Arg Ser His His Ile Leu
1               5                   10                  15
Ser Lys Ala His Gln Cys Arg Arg Ile Gly His Leu Met Leu Lys Pro
            20                  25                  30
Leu Lys Glu Phe Glu Asn Thr Thr Cys Ser Thr Leu Thr Ile Arg Gln

        35                  40                  45
Ser Leu Asp Leu Phe Leu Pro Asp Lys Thr Ala Ser Gly Leu Asn Lys
    50                  55                  60
Ser Gln Ile Leu Glu Met Asn Gln Lys Lys Ser Asp Thr Ser Met Leu
65                  70                  75                  80
Ser Pro Leu Asn Ala Ala Arg Cys Gln Asp Glu Lys Ala His Leu Pro
                85                  90                  95
Thr Met Lys Ser Phe Gly Thr His Arg Arg Val Thr His Lys Pro Asn
            100                 105                 110
Leu Leu Gly Ser Lys Trp Phe Ile Lys Ile Leu Lys Arg His Phe Ser
        115                 120                 125
Ser Val Ser Thr Glu Thr Phe Val Pro Lys Gln Asp Phe Pro Gln Val
    130                 135                 140
Lys Arg Pro Leu Lys Ala Ser Arg Thr Arg Gln Pro Ser Arg Thr Asn
145                 150                 155                 160
Leu Pro Val Leu Ser Val Asn Glu Asp Pro Met His Cys Thr Ala Phe
                165                 170                 175
Ala Thr Ala Asp Glu Tyr His Leu Gly Asn Leu Ser Gln Asp Leu Ala
            180                 185                 190
Ser His Gly Tyr Val Glu Val Thr Ser Leu Pro Arg Asp Ala Ala Asn
        195                 200                 205
Ile Leu Val Met Gly Val Glu Asn Ser Ala Lys Glu Gly Asp Pro Gly
    210                 215                 220
Thr Ile Phe Phe Phe Arg Glu Gly Ala Ala Val Phe Trp Asn Val Lys
225                 230                 235                 240
Asp Lys Thr Met Lys His Val Met Lys Val Leu Glu Lys His Glu Ile
                245                 250                 255
Gln Pro Tyr Glu Ile Ala Leu Val His Trp Glu Asn Glu Glu Leu Asn
            260                 265                 270
Tyr Ile Lys Ile Glu Gly Gln Ser Lys Leu His Arg Gly Glu Ile Lys
        275                 280                 285
Leu Asn Ser Glu Leu Asp Leu Asp Asp Ala Ile Leu Glu Lys Phe Ala
    290                 295                 300
Phe Ser Asn Ala Leu Cys Leu Ser Val Lys Leu Ala Ile Trp Glu Ala
305                 310                 315                 320
Ser Leu Asp Lys Phe Ile Glu Ser Ile Gln Ser Ile Pro Glu Ala Leu
                325                 330                 335
Lys Ala Gly Lys Lys Val Lys Leu Ser His Glu Glu Val Met Gln Lys
            340                 345                 350
Ile Gly Glu Leu Phe Ala Leu Arg His Arg Ile Asn Leu Ser Ser Asp
        355                 360                 365
Phe Leu Ile Thr Pro Asp Phe Tyr Trp Asp Arg Glu Asn Leu Glu Gly
    370                 375                 380
Leu Tyr Asp Lys Thr Cys Gln Phe Leu Ser Ile Gly Arg Arg Val Lys
385                 390                 395                 400
Val Met Asn Glu Lys Leu Gln His Cys Met Glu Leu Thr Asp Leu Met
                405                 410                 415
Arg Asn His Leu Asn Glu Lys Arg Ala Leu Arg Leu Glu Trp Met Ile
            420                 425                 430
Val Ile Leu Ile Thr Ile Glu Val Met Phe Glu Leu Gly Arg Val Phe
        435                 440                 445
Phe

<210> 265
<211> 838
<212> PRT
<213> Homo sapiens

<400> 265
Met Pro Ala Glu Arg Pro Ala Gly Ser Gly Gly Ser Glu Ala Pro Ala
1               5                   10                  15
Met Val Glu Gln Leu Asp Thr Ala Val Ile Thr Pro Ala Met Leu Glu
            20                  25                  30
Glu Glu Glu Gln Leu Glu Ala Ala Gly Leu Glu Arg Glu Arg Lys Met
        35                  40                  45
Leu Glu Lys Ala Arg Met Ser Trp Asp Arg Glu Ser Thr Glu Ile Arg
    50                  55                  60
Tyr Arg Arg Leu Gln His Leu Leu Glu Lys Ser Asn Ile Tyr Ser Lys
65                  70                  75                  80
Phe Leu Leu Thr Lys Met Glu Gln Gln Gln Leu Glu Glu Gln Lys Lys
                85                  90                  95
Lys Glu Lys Leu Glu Arg Lys Lys Glu Ser Leu Lys Val Lys Lys Gly
            100                 105                 110
Lys Asn Ser Ile Asp Ala Ser Glu Glu Lys Pro Val Met Arg Lys Lys
        115                 120                 125
Arg Gly Arg Glu Asp Glu Ser Tyr Asn Ile Ser Glu Val Met Ser Lys
    130                 135                 140
Glu Glu Ile Leu Ser Val Ala Lys Lys Asn Lys Lys Glu Asn Glu Asp
145                 150                 155                 160
Glu Asn Ser Ser Ser Thr Asn Leu Cys Val Glu Asp Leu Gln Lys Asn
                165                 170                 175
Lys Asp Ser Asn Ser Ile Ile Lys Asp Arg Leu Ser Glu Thr Val Arg
            180                 185                 190
Gln Asn Thr Lys Phe Phe Phe Asp Pro Val Arg Lys Cys Asn Gly Gln
        195                 200                 205
Pro Val Pro Phe Gln Gln Pro Lys His Phe Thr Gly Gly Val Met Arg
    210                 215                 220
Trp Tyr Gln Val Glu Gly Met Glu Trp Leu Arg Met Leu Trp Glu Asn
225                 230                 235                 240
Gly Ile Asn Gly Ile Leu Ala Asp Glu Met Gly Leu Gly Lys Thr Val
                245                 250                 255
Gln Cys Ile Ala Thr Ile Ala Leu Met Ile Gln Arg Gly Val Pro Gly
            260                 265                 270
Pro Phe Leu Val Cys Gly Pro Leu Ser Thr Leu Pro Asn Trp Met Ala
        275                 280                 285
Glu Phe Lys Arg Phe Thr Pro Asp Ile Pro Thr Met Leu Tyr His Gly
    290                 295                 300
Thr Gln Glu Glu Arg Gln Lys Leu Val Arg Asn Ile Tyr Lys Arg Lys
305                 310                 315                 320
Gly Thr Leu Gln Ile His Pro Val Val Ile Thr Ser Phe Glu Ile Ala
                325                 330                 335
Met Arg Asp Arg Asn Ala Leu Gln His Cys Tyr Trp Lys Tyr Leu Ile
            340                 345                 350
Val Asp Glu Gly His Arg Ile Lys Asn Met Lys Cys Arg Leu Ile Arg
        355                 360                 365
Glu Leu Lys Arg Phe Asn Ala Asp Asn Lys Leu Leu Leu Thr Gly Thr
    370                 375                 380
Pro Leu Gln Asn Asn Leu Ser Glu Leu Trp Ser Leu Leu Asn Phe Leu
385                 390                 395                 400
Leu Pro Asp Val Phe Asp Asp Leu Lys Ser Phe Glu Ser Trp Phe Asp
                405                 410                 415
Ile Thr Ser Leu Ser Glu Thr Ala Glu Asp Ile Ile Ala Lys Glu Arg
            420                 425                 430
Glu Gln Asn Val Leu His Met Leu His Gln Ile Leu Thr Pro Phe Leu
        435                 440                 445
Leu Arg Arg Leu Lys Ser Asp Val Ala Leu Glu Val Pro Pro Lys Arg

450                 455                 460
Glu Val Val Val Tyr Ala Pro Leu Ser Lys Lys Gln Glu Ile Phe Tyr
465                 470                 475                 480
Thr Ala Ile Val Asn Arg Thr Ile Ala Asn Met Tyr Gly Ser Ser Glu
                485                 490                 495
Lys Glu Thr Ile Glu Leu Ser Pro Thr Gly Arg Pro Lys Arg Arg Thr
            500                 505                 510
Arg Lys Ser Ile Asn Asn Ser Lys Ile Asp Glu Phe Pro Asn Glu Leu
        515                 520                 525
Glu Lys Leu Ile Ser Gln Ile Gln Pro Glu Val Asp Arg Glu Arg Ala
    530                 535                 540
Val Val Glu Val Asn Ile Pro Val Glu Ser Glu Val Asn Leu Lys Leu
545                 550                 555                 560
Gln Asn Ile Met Met Leu Leu Arg Lys Cys Cys Asn His Pro Tyr Leu
                565                 570                 575
Ile Glu Tyr Pro Ile Asp Pro Val Thr Gln Glu Phe Lys Ile Asp Glu
            580                 585                 590
Glu Leu Val Thr Asn Ser Gly Lys Phe Leu Ile Leu Asp Arg Met Leu
        595                 600                 605
Pro Glu Leu Lys Lys Arg Gly His Lys Val Leu Leu Phe Ser Gln Met
    610                 615                 620
Thr Ser Met Leu Asp Ile Leu Met Asp Tyr Cys His Leu Arg Asp Phe
625                 630                 635                 640
Asn Phe Ser Arg Leu Asp Gly Ser Met Ser Tyr Ser Glu Arg Glu Lys
                645                 650                 655
Asn Met His Ser Phe Asn Thr Asp Pro Glu Val Phe Ile Phe Leu Val
            660                 665                 670
Ser Thr Arg Ala Gly Gly Leu Gly Ile Asn Leu Thr Ala Ala Asp Thr
        675                 680                 685
Val Ile Ile Tyr Asp Ser Asp Trp Asn Pro Gln Ser Asp Leu Gln Ala
    690                 695                 700
Gln Asp Arg Cys His Arg Ile Gly Gln Thr Lys Pro Val Val Val Tyr
705                 710                 715                 720
Arg Leu Val Thr Ala Asn Thr Ile Asp Gln Lys Ile Val Glu Arg Ala
                725                 730                 735
Ala Ala Lys Arg Lys Leu Glu Lys Leu Ile Ile His Lys Asn His Phe
            740                 745                 750
Lys Gly Gly Gln Ser Gly Leu Asn Leu Ser Lys Asn Phe Leu Asp Pro
        755                 760                 765
Lys Glu Leu Met Glu Leu Leu Lys Ser Arg Asp Tyr Glu Arg Glu Ile
    770                 775                 780
Lys Gly Ser Arg Glu Lys Val Ile Ser Asp Lys Asp Leu Glu Leu Leu
785                 790                 795                 800
Leu Asp Arg Ser Asp Leu Ile Asp Gln Met Asn Ala Ser Gly Pro Ile
                805                 810                 815
Lys Glu Lys Met Gly Ile Phe Lys Ile Leu Glu Asn Ser Glu Asp Ser
            820                 825                 830
Ser Pro Glu Cys Leu Phe
        835

<210> 266
<211> 402
<212> PRT
<213> Homo sapiens

<400> 266
Met Tyr Thr Ser Glu Glu Lys Cys Asn Gln Arg Thr Gln Lys Arg Lys

1               5                   10                  15
Ile Tyr Asn Val Cys Pro Arg Lys Gly Lys Lys Ile Phe Ile His Met
                20                  25                  30
His Glu Ile Ile Gln Ile Asp Gly His Ile Tyr Gln Cys Leu Glu Cys
            35                  40                  45
Lys Gln Asn Phe Cys Glu Asn Leu Ala Leu Ile Met Cys Glu Arg Thr
    50                  55                  60
His Thr Gly Glu Lys Pro Tyr Lys Cys Asp Met Cys Glu Lys Thr Phe
65                  70                  75                  80
Val Gln Ser Ser Asp Leu Thr Ser His Gln Arg Ile His Asn Tyr Glu
                85                  90                  95
Lys Pro Tyr Lys Cys Ser Lys Cys Glu Lys Ser Phe Trp His His Leu
            100                 105                 110
Ala Leu Ser Gly His Gln Arg Thr His Ala Gly Lys Lys Phe Tyr Thr
        115                 120                 125
Cys Asp Ile Cys Gly Lys Asn Phe Gly Gln Ser Ser Asp Leu Leu Val
    130                 135                 140
His Gln Arg Ser His Thr Gly Glu Lys Pro Tyr Leu Cys Ser Glu Cys
145                 150                 155                 160
Asp Lys Cys Phe Ser Arg Ser Thr Asn Leu Ile Arg His Arg Arg Thr
                165                 170                 175
His Thr Gly Glu Lys Pro Phe Lys Cys Leu Glu Cys Glu Lys Ala Phe
            180                 185                 190
Ser Gly Lys Ser Asp Leu Ile Ser His Gln Arg Thr His Thr Gly Glu
        195                 200                 205
Arg Pro Tyr Lys Cys Asn Lys Cys Glu Lys Ser Tyr Arg His Arg Ser
    210                 215                 220
Ala Phe Ile Val His Lys Arg Val His Thr Gly Glu Lys Pro Tyr Lys
225                 230                 235                 240
Cys Gly Ala Cys Glu Lys Cys Phe Gly Gln Lys Ser Asp Leu Ile Val
                245                 250                 255
His Gln Arg Val His Thr Gly Glu Lys Pro Tyr Lys Cys Leu Glu Cys
            260                 265                 270
Met Arg Ser Phe Thr Arg Ser Ala Asn Leu Ile Arg His Gln Ala Thr
        275                 280                 285
His Thr His Thr Phe Lys Cys Leu Glu Tyr Glu Lys Ser Phe Asn Cys
    290                 295                 300
Ser Ser Asp Leu Ile Val His Gln Arg Ile His Met Glu Glu Lys Pro
305                 310                 315                 320
His Gln Trp Ser Ala Cys Glu Ser Gly Phe Leu Leu Gly Met Asp Phe
                325                 330                 335
Val Ala Gln Gln Lys Met Arg Thr Gln Thr Glu Glu Leu His Tyr Lys
            340                 345                 350
Tyr Thr Val Cys Asp Lys Ser Phe His Gln Ser Ser Ala Leu Leu Gln
        355                 360                 365
His Gln Thr Val His Ile Gly Glu Lys Pro Phe Val Cys Asn Val Ser
    370                 375                 380
Glu Lys Gly Leu Glu Leu Ser Pro Pro His Ala Ser Glu Ala Ser Gln
385                 390                 395                 400
Met Ser

<210> 267
<211> 579
<212> PRT
<213> Homo sapiens

<400> 267
Met Ala Phe Gly Gly Asn Phe Leu His Asn Leu Asn Ile Gly Met Gln
1               5                   10                  15
Leu Arg Cys Tyr Glu Met Glu Lys Arg Leu Lys Thr Pro Asp Leu Phe
            20                  25                  30
Lys Phe Pro Phe Phe Glu Ala Ile Cys Trp Phe Val Ala Lys Asn Leu
        35                  40                  45
Leu Glu Thr Leu Lys Glu Leu Arg Glu Asp Gly Phe Gln Pro Gln Thr
    50                  55                  60
Tyr Leu Val Gln Gly Val Lys Ala Leu His Thr Ala Leu Lys Leu Trp
65                  70                  75                  80
Met Lys Lys Glu Leu Val Ser Glu His Ala Phe Glu Ile Pro Asp Asn
                85                  90                  95
Val Arg Pro Gly His Leu Ile Lys Glu Leu Ser Lys Val Ile Arg Ala
            100                 105                 110
Ile Glu Glu Glu Asn Gly Lys Pro Val Lys Ser Gln Gly Ile Pro Ile
        115                 120                 125
Val Cys Pro Val Ser Arg Ser Ser Asn Glu Ala Thr Ser Pro Tyr His
    130                 135                 140
Ser Arg Arg Lys Met Arg Lys Leu Arg Asp His Asn Val Arg Thr Pro
145                 150                 155                 160
Ser Asn Leu Asp Ile Leu Glu Leu His Thr Arg Glu Val Leu Lys Arg
                165                 170                 175
Leu Glu Met Cys Pro Trp Glu Glu Asp Ile Leu Ser Ser Lys Leu Asn
            180                 185                 190
Gly Lys Phe Asn Lys His Leu Gln Pro Ser Ser Thr Val Pro Glu Trp
        195                 200                 205
Arg Ala Lys Asp Asn Asp Leu Arg Leu Leu Leu Thr Asn Gly Arg Ile
    210                 215                 220
Ile Lys Asp Glu Arg Gln Pro Phe Ala Asp Gln Ser Leu Tyr Thr Ala
225                 230                 235                 240
Asp Ser Glu Asn Glu Glu Asp Lys Arg Arg Thr Lys Lys Ala Lys Met
                245                 250                 255
Lys Ile Glu Glu Ser Ser Gly Val Glu Gly Val Glu His Glu Glu Ser
            260                 265                 270
Gln Lys Pro Leu Asn Gly Phe Phe Thr Arg Val Lys Ser Glu Leu Arg
        275                 280                 285
Ser Arg Ser Ser Gly Tyr Ser Asp Ile Ser Glu Ser Glu Asp Ser Gly
    290                 295                 300
Pro Glu Cys Thr Ala Leu Lys Ser Ile Phe Thr Thr Glu Glu Ser Glu
305                 310                 315                 320
Ser Ser Gly Asp Glu Lys Lys Gln Glu Ile Thr Ser Asn Phe Lys Glu
                325                 330                 335
Glu Ser Asn Val Met Arg Asn Phe Leu Gln Lys Ser Gln Lys Pro Ser
            340                 345                 350
Arg Ser Glu Ile Pro Ile Lys Arg Glu Cys Pro Thr Ser Thr Ser Thr
        355                 360                 365
Glu Glu Glu Ala Ile Gln Gly Met Leu Ser Met Ala Gly Leu His Tyr
    370                 375                 380
Ser Thr Cys Leu Gln Arg Gln Ile Gln Ser Thr Asp Cys Ser Gly Glu
385                 390                 395                 400
Arg Asn Ser Leu Gln Asp Pro Ser Ser Cys His Gly Ser Asn His Glu
                405                 410                 415
Val Arg Gln Leu Tyr Arg Tyr Asp Lys Pro Val Glu Cys Gly Tyr His
            420                 425                 430
Val Lys Thr Glu Asp Pro Asp Leu Arg Thr Ser Ser Trp Ile Lys Gln
        435                 440                 445
Phe Asp Thr Ser Arg Phe His Pro Gln Asp Leu Ser Arg Ser Gln Lys
    450                 455                 460
Cys Ile Arg Lys Glu Gly Ser Ser Glu Ile Ser Gln Arg Val Gln Ser

465                 470                 475                 480
Arg Asn Tyr Val Asp Ser Ser Gly Ser Ser Leu Gln Asn Gly Lys Tyr
                485                 490                 495
Met Gln Asn Ser Asn Leu Thr Ser Gly Ala Cys Gln Ile Ser Asn Gly
            500                 505                 510
Ser Leu Ser Pro Glu Arg Pro Val Gly Glu Thr Ser Phe Ser Val Pro
        515                 520                 525
Leu His Pro Thr Lys Arg Pro Ala Ser Asn Pro Pro Pro Ile Ser Asn
    530                 535                 540
Gln Ala Thr Lys Gly Lys Arg Pro Lys Lys Gly Met Ala Thr Ala Lys
545                 550                 555                 560
Gln Arg Leu Gly Lys Ile Leu Lys Leu Asn Arg Asn Gly His Ala Arg
                565                 570                 575
Phe Phe Val

<210> 268
<211> 563
<212> PRT
<213> Homo sapiens

<400> 268
Met Ser Asp Asp Ala Gly Asp Thr Leu Ala Thr Gly Asp Lys Ala Glu
1               5                   10                  15
Val Thr Glu Met Pro Asn Ser Asp Ser Leu Pro Glu Asp Ala Glu Val
            20                  25                  30
His Cys Asp Ser Ala Ala Val Ser His Glu Pro Thr Pro Ala Asp Pro
        35                  40                  45
Arg Gly Glu Gly His Glu Asn Ala Ala Val Gln Gly Ala Gly Ala Ala
    50                  55                  60
Ala Ile Gly Pro Pro Val Gln Pro Gln Asp Ala Asn Ala Leu Glu Pro
65                  70                  75                  80
Pro Leu Asn Gly Asp Val Thr Glu Asp Thr Leu Ala Glu Cys Ile Asp
                85                  90                  95
Ser Val Ser Leu Glu Ala Glu Pro Arg Ser Glu Ile Pro Leu Gln Glu
            100                 105                 110
Gln Asn Tyr Leu Ala Val Asp Ser Pro Pro Ser Gly Gly Gly Trp Ala
        115                 120                 125
Gly Trp Gly Ser Trp Gly Lys Ser Leu Leu Ser Ser Ala Ser Ala Thr
    130                 135                 140
Val Gly His Gly Leu Thr Ala Val Lys Glu Lys Ala Gly Ala Thr Leu
145                 150                 155                 160
Arg Ile His Gly Val Asn Ser Gly Ser Ser Glu Gly Ala Gln Pro Asn
                165                 170                 175
Thr Glu Asn Gly Val Pro Glu Ile Thr Asp Ala Ala Thr Asp Gln Gly
            180                 185                 190
Pro Ala Glu Ser Pro Pro Thr Ser Pro Ser Ser Ala Ser Arg Gly Met
        195                 200                 205
Leu Ser Ala Ile Thr Asn Val Val Gln Asn Thr Gly Lys Ser Val Leu
    210                 215                 220
Thr Gly Gly Leu Asp Ala Leu Glu Phe Ile Gly Lys Lys Thr Met Asn
225                 230                 235                 240
Val Leu Ala Glu Ser Asp Pro Gly Phe Lys Arg Thr Lys Thr Leu Met
                245                 250                 255
Glu Arg Thr Val Ser Leu Ser Gln Met Leu Arg Glu Ala Lys Glu Lys
            260                 265                 270
Glu Lys Gln Arg Leu Ala Gln Gln Leu Thr Met Glu Arg Thr Ala His

275                 280                 285
Tyr Gly Met Leu Phe Asp Glu Tyr Gln Gly Leu Ser His Leu Glu Ala
    290                 295                 300
Leu Glu Ile Leu Ser Asn Glu Ser Glu Ser Lys Val Gln Ser Phe Leu
305                 310                 315                 320
Ala Ser Leu Asp Gly Glu Lys Leu Glu Leu Leu Lys Asn Asp Leu Ile
                325                 330                 335
Ser Ile Lys Asp Ile Phe Ala Ala Lys Glu Leu Glu Asn Glu Glu Asn
            340                 345                 350
Gln Glu Glu Gln Gly Leu Glu Glu Lys Gly Glu Glu Phe Ala Arg Met
        355                 360                 365
Leu Thr Glu Leu Leu Phe Glu Leu His Val Ala Ala Thr Pro Asp Lys
    370                 375                 380
Leu Asn Lys Ala Met Lys Arg Ala His Asp Trp Val Glu Glu Asp Gln
385                 390                 395                 400
Thr Val Val Ser Val Asp Val Ala Lys Val Ser Glu Glu Glu Thr Lys
                405                 410                 415
Lys Glu Glu Lys Glu Glu Lys Ser Gln Asp Pro Gln Glu Asp Lys Lys
            420                 425                 430
Glu Glu Lys Lys Thr Lys Thr Ile Glu Glu Val Tyr Met Ser Ser Ile
        435                 440                 445
Glu Ser Leu Ala Glu Val Thr Ala Arg Cys Ile Glu Gln Leu His Lys
    450                 455                 460
Val Ala Glu Leu Ile Leu His Gly Gln Glu Glu Glu Lys Pro Ala Gln
465                 470                 475                 480
Asp Gln Ala Lys Val Leu Ile Lys Leu Thr Thr Ala Met Cys Asn Glu
                485                 490                 495
Val Ala Ser Leu Ser Lys Lys Phe Thr Asn Ser Leu Thr Thr Val Gly
            500                 505                 510
Ser Asn Lys Lys Ala Glu Val Leu Asn Pro Met Ile Ser Ser Val Leu
        515                 520                 525
Leu Glu Gly Cys Asn Ser Thr Thr Tyr Ile Gln Asp Ala Phe Gln Leu
    530                 535                 540
Leu Leu Pro Val Leu Gln Val Ser His Ile Gln Thr Ser Cys Leu Lys
545                 550                 555                 560
Ala Gln Pro

<210> 269
<211> 76
<212> PRT
<213> Homo sapiens

<400> 269

<210> 270
<211> 944
<212> PRT
<213> Homo sapiens

<400> 270
Met Leu Ala Glu Leu Phe Met Cys Phe Glu Val Leu Lys Pro Asp Phe
1               5                   10                  15
Val Gln Val Lys Asp Leu Pro Asp Gly His Ala Ala Ser Pro Arg Gly
            20                  25                  30
Thr Glu Ala Ser Pro Pro Gln Asn Asn Ser Gly Ser Ser Ser Pro Val
        35                  40                  45
Phe Thr Phe Arg His Pro Leu Leu Ser Ser Gly Gly Pro Gln Ser Pro
    50                  55                  60
Leu Arg Gly Ser Thr Gly Ser Leu Lys Ser Ser Pro Ser Met Ser His
65                  70                  75                  80
Met Glu Ala Leu Gly Lys Ala Trp Asn Arg Gln Leu Ser Arg Pro Leu
                85                  90                  95
Ser Gln Ala Val Ser Phe Ser Thr Pro Phe Gly Leu Asp Ser Asp Val
            100                 105                 110
Asp Val Val Met Gly Asp Pro Val Leu Leu Arg Ser Val Ser Ser Asp
        115                 120                 125
Ser Leu Gly Pro Pro Arg Pro Ala Pro Ala Arg Thr Pro Thr Gln Pro
    130                 135                 140
Pro Pro Glu Pro Gly Asp Leu Pro Thr Ile Glu Glu Ala Leu Gln Ile
145                 150                 155                 160
Ile His Ser Ala Glu Pro Arg Leu Leu Pro Asp Gly Ala Ala Asp Gly
                165                 170                 175
Ser Phe Tyr Leu His Ser Pro Glu Gly Pro Ser Lys Pro Ser Leu Ala
            180                 185                 190
Ser Pro Tyr Leu Pro Glu Gly Thr Ser Lys Pro Leu Ser Asp Arg Pro
        195                 200                 205
Thr Lys Ala Pro Val Tyr Met Pro His Pro Glu Thr Pro Ser Lys Pro
    210                 215                 220
Ser Pro Cys Leu Val Gly Glu Ala Ser Lys Pro Pro Ala Pro Ser Glu
225                 230                 235                 240
Gly Ser Pro Lys Ala Val Ala Ser Ser Pro Ala Ala Thr Asn Ser Glu
                245                 250                 255
Val Lys Met Thr Ser Phe Ala Glu Arg Lys Lys Gln Leu Val Lys Ala
            260                 265                 270
Glu Ala Glu Ala Gly Ala Gly Ser Pro Thr Ser Thr Pro Ala Pro Pro
        275                 280                 285
Glu Ala Leu Ser Ser Glu Met Ser Glu Leu Ser Ala Arg Leu Glu Glu
    290                 295                 300
Lys Arg Arg Ala Ile Glu Ala Gln Lys Arg Arg Ile Glu Ala Ile Phe
305                 310                 315                 320
Ala Lys His Arg Gln Arg Leu Gly Lys Ser Ala Phe Leu Gln Val Gln
                325                 330                 335
Pro Arg Glu Ala Ser Gly Glu Ala Glu Ala Glu Ala Glu Glu Ala Asp
            340                 345                 350
Ser Gly Pro Val Pro Gly Gly Glu Arg Pro Ala Gly Glu Gly Gln Gly
        355                 360                 365
Glu Pro Thr Ser Arg Pro Lys Ala Val Thr Phe Ser Pro Asp Leu Gly
    370                 375                 380
Pro Val Pro His Glu Gly Leu Gly Glu Tyr Asn Arg Ala Val Ser Lys
385                 390                 395                 400
Leu Ser Ala Ala Leu Ser Ser Leu Gln Arg Asp Met Gln Arg Leu Thr
                405                 410                 415
Asp Gln Gln Gln Arg Leu Leu Ala Pro Pro Glu Ala Pro Gly Ser Ala

420                     425                     430
Pro Pro Pro Ala Ala Trp Val Ile Pro Gly Pro Thr Thr Gly Pro Lys
        435                     440                     445
Ala Ala Ser Pro Ser Pro Ala Arg Arg Val Pro Ala Thr Arg Arg Ser
    450                     455                     460
Pro Gly Pro Gly Pro Ser Gln Ser Pro Arg Ser Pro Lys His Thr Arg
465                     470                     475                     480
Pro Ala Glu Leu Arg Leu Ala Pro Leu Thr Arg Val Leu Thr Pro Pro
                    485                     490                     495
His Asp Val Asp Ser Leu Pro His Leu Arg Lys Phe Ser Pro Ser Gln
                500                     505                     510
Val Pro Val Gln Thr Arg Ser Ser Ile Leu Leu Ala Glu Glu Thr Pro
        515                     520                     525
Pro Glu Glu Pro Ala Ala Arg Pro Gly Leu Ile Glu Ile Pro Leu Gly
    530                     535                     540
Ser Leu Ala Asp Pro Ala Ala Glu Asp Glu Gly Asp Gly Ser Pro Ala
545                     550                     555                     560
Gly Ala Glu Asp Ser Leu Glu Glu Glu Ala Ser Ser Glu Gly Glu Pro
                    565                     570                     575
Arg Val Gly Leu Gly Phe Phe Tyr Lys Asp Glu Asp Lys Pro Glu Asp
                580                     585                     590
Glu Met Ala Gln Lys Arg Ala Ser Leu Leu Glu Arg Gln Gln Arg Arg
        595                     600                     605
Ala Glu Glu Ala Arg Arg Arg Lys Gln Trp Gln Glu Val Glu Lys Glu
    610                     615                     620
Gln Arg Arg Glu Glu Ala Ala Arg Leu Ala Gln Glu Glu Ala Pro Gly
625                     630                     635                     640
Pro Ala Pro Leu Val Ser Ala Val Pro Met Ala Thr Pro Ala Pro Ala
                    645                     650                     655
Ala Arg Ala Pro Ala Glu Glu Glu Val Gly Pro Arg Lys Gly Asp Phe
                660                     665                     670
Thr Arg Gln Glu Tyr Glu Arg Arg Ala Gln Leu Lys Leu Met Asp Asp
        675                     680                     685
Leu Asp Lys Val Leu Arg Pro Arg Ala Ala Gly Ser Gly Gly Pro Gly
    690                     695                     700
Arg Gly Gly Arg Arg Ala Thr Arg Pro Arg Ser Gly Cys Cys Asp Asp
705                     710                     715                     720
Ser Ala Leu Ala Arg Ser Pro Ala Arg Gly Leu Leu Gly Ser Arg Leu
                    725                     730                     735
Ser Lys Ile Tyr Ser Gln Ser Thr Leu Ser Leu Ser Thr Val Ala Asn
                740                     745                     750
Glu Ala His Asn Asn Leu Gly Val Lys Arg Pro Thr Ser Arg Ala Pro
        755                     760                     765
Ser Pro Ser Gly Leu Met Ser Pro Ser Arg Leu Pro Gly Ser Arg Glu
    770                     775                     780
Arg Asp Trp Glu Asn Gly Ser Asn Ala Ser Ser Pro Ala Ser Val Pro
785                     790                     795                     800
Glu Tyr Thr Gly Pro Arg Leu Tyr Lys Glu Pro Ser Ala Lys Ser Asn
                    805                     810                     815
Lys Phe Ile Ile His Asn Ala Leu Ser His Cys Cys Leu Ala Gly Lys
                820                     825                     830
Val Asn Glu Pro Gln Lys Asn Arg Ile Leu Glu Glu Ile Glu Lys Ser
        835                     840                     845
Lys Ala Asn His Phe Leu Ile Leu Phe Arg Asp Ser Ser Cys Gln Phe
    850                     855                     860
Arg Ala Leu Tyr Thr Leu Ser Gly Glu Thr Glu Glu Leu Ser Arg Leu
865                     870                     875                     880
Ala Gly Tyr Gly Pro Arg Thr Val Thr Pro Ala Met Val Glu Gly Ile
                    885                     890                     895
Tyr Lys Tyr Asn Ser Asp Arg Lys Arg Phe Thr Gln Ile Pro Ala Lys

900                 905                 910
Thr Met Ser Met Ser Val Asp Ala Phe Thr Ile Gln Gly His Leu Trp
        915                 920                 925
Gln Gly Lys Lys Pro Thr Thr Pro Lys Lys Gly Gly Gly Thr Pro Lys
    930                 935                 940

<210> 271
<211> 1212
<212> PRT
<213> Homo sapiens

<400> 271
Met Ala Leu Met Phe Thr Gly His Leu Leu Phe Leu Ala Leu Leu Met
1               5                   10                  15
Phe Ala Phe Ser Thr Phe Glu Glu Ser Val Ser Asn Tyr Ser Glu Trp
            20                  25                  30
Ala Val Phe Thr Asp Asp Ile Asp Gln Phe Lys Thr Gln Lys Val Gln
        35                  40                  45
Asp Phe Arg Pro Asn Gln Lys Leu Lys Lys Ser Met Leu His Pro Ser
    50                  55                  60
Leu Tyr Phe Asp Ala Gly Glu Ile Gln Ala Met Arg Gln Lys Ser Arg
65                  70                  75                  80
Ala Ser His Leu His Leu Phe Arg Ala Ile Arg Ser Ala Val Thr Val
                85                  90                  95
Met Leu Ser Asn Pro Thr Tyr Tyr Leu Pro Pro Pro Lys His Ala Asp
            100                 105                 110
Phe Ala Ala Lys Trp Asn Glu Ile Tyr Gly Asn Asn Leu Pro Pro Leu
        115                 120                 125
Ala Leu Tyr Cys Leu Leu Cys Pro Glu Asp Lys Val Ala Phe Glu Phe
    130                 135                 140
Val Leu Glu Tyr Met Asp Arg Met Val Gly Tyr Lys Asp Trp Leu Val
145                 150                 155                 160
Glu Asn Ala Pro Gly Asp Glu Val Pro Ile Gly His Ser Leu Thr Gly
                165                 170                 175
Phe Ala Thr Ala Phe Asp Phe Leu Tyr Asn Leu Leu Asp Asn His Arg
            180                 185                 190
Arg Gln Lys Tyr Leu Glu Lys Ile Trp Val Ile Thr Glu Glu Met Tyr
        195                 200                 205
Glu Tyr Ser Lys Val Arg Ser Trp Gly Lys Gln Leu Leu His Asn His
    210                 215                 220
Gln Ala Thr Asn Met Ile Ala Leu Leu Thr Gly Ala Leu Val Thr Gly
225                 230                 235                 240
Val Asp Lys Gly Ser Lys Ala Asn Ile Trp Lys Gln Ala Val Val Asp
                245                 250                 255
Val Met Glu Lys Thr Met Phe Leu Leu Asn His Ile Val Asp Gly Ser
            260                 265                 270
Leu Asp Glu Gly Val Ala Tyr Gly Ser Tyr Thr Ala Lys Ser Val Thr
        275                 280                 285
Gln Tyr Val Phe Leu Ala Gln Arg His Phe Asn Ile Asn Asn Leu Asp
    290                 295                 300
Asn Asn Trp Leu Lys Met His Phe Trp Phe Tyr Tyr Ala Thr Leu Leu
305                 310                 315                 320
Pro Gly Phe Gln Arg Thr Val Gly Ile Ala Asp Ser Asn Tyr Asn Trp
                325                 330                 335
Phe Tyr Gly Pro Glu Ser Gln Leu Val Phe Leu Asp Lys Phe Ile Leu

340                 345                 350
Lys Asn Gly Ala Gly Asn Trp Leu Ala Gln Gln Ile Arg Lys His Arg
        355                 360                 365
Pro Lys Asp Gly Pro Met Val Pro Ser Thr Ala Gln Arg Trp Ser Thr
    370                 375                 380
Leu His Thr Glu Tyr Ile Trp Tyr Asp Pro Gln Leu Thr Pro Gln Pro
385                 390                 395                 400
Pro Ala Asp Tyr Gly Thr Ala Lys Ile His Thr Phe Pro Asn Trp Gly
                405                 410                 415
Val Val Thr Tyr Gly Ala Gly Leu Pro Asn Thr Gln Thr Asn Thr Phe
            420                 425                 430
Val Ser Phe Lys Ser Gly Lys Leu Gly Gly Arg Ala Val Tyr Asp Ile
        435                 440                 445
Val His Phe Gln Pro Tyr Ser Trp Ile Asp Gly Trp Arg Ser Phe Asn
    450                 455                 460
Pro Gly His Glu His Pro Asp Gln Asn Ser Phe Thr Phe Ala Pro Asn
465                 470                 475                 480
Gly Gln Val Phe Val Ser Glu Ala Leu Tyr Gly Pro Lys Leu Ser His
                485                 490                 495
Leu Asn Asn Val Leu Val Phe Ala Pro Ser Pro Ser Ser Gln Cys Asn
            500                 505                 510
Lys Pro Trp Glu Gly Gln Leu Gly Glu Cys Ala Gln Trp Leu Lys Trp
        515                 520                 525
Thr Gly Glu Glu Val Gly Asp Ala Ala Gly Glu Ile Ile Thr Ala Ser
    530                 535                 540
Gln His Gly Glu Met Val Phe Val Ser Gly Glu Ala Val Ser Ala Tyr
545                 550                 555                 560
Ser Ser Ala Met Arg Leu Lys Ser Val Tyr Arg Ala Leu Leu Leu Leu
                565                 570                 575
Asn Ser Gln Thr Leu Leu Val Val Asp His Ile Glu Arg Gln Glu Asp
            580                 585                 590
Ser Pro Ile Asn Ser Val Ser Ala Phe Phe His Asn Leu Asp Ile Asp
        595                 600                 605
Phe Lys Tyr Ile Pro Tyr Lys Phe Met Asn Arg Tyr Asn Gly Ala Met
    610                 615                 620
Met Asp Val Trp Asp Ala His Tyr Lys Met Phe Trp Phe Asp His His
625                 630                 635                 640
Gly Asn Ser Pro Met Ala Ser Ile Gln Glu Ala Glu Gln Ala Ala Glu
                645                 650                 655
Phe Lys Lys Arg Trp Thr Gln Phe Val Asn Val Thr Phe Gln Met Glu
            660                 665                 670
Pro Thr Ile Thr Arg Ile Ala Tyr Val Phe Tyr Gly Pro Tyr Ile Asn
        675                 680                 685
Val Ser Ser Cys Arg Phe Ile Asp Ser Ser Asn Pro Gly Leu Gln Ile
    690                 695                 700
Ser Leu Asn Val Asn Asn Thr Glu His Val Val Ser Ile Val Thr Asp
705                 710                 715                 720
Tyr His Asn Leu Lys Thr Arg Phe Asn Tyr Leu Gly Phe Gly Gly Phe
                725                 730                 735
Ala Ser Val Ala Asp Gln Gly Gln Ile Thr Arg Phe Gly Leu Gly Thr
            740                 745                 750
Gln Ala Ile Val Lys Pro Val Arg His Asp Arg Ile Ile Phe Pro Phe
        755                 760                 765
Gly Phe Lys Phe Asn Ile Ala Val Gly Leu Ile Leu Cys Ile Ser Leu
    770                 775                 780
Val Ile Leu Thr Phe Gln Trp Arg Phe Tyr Leu Ser Phe Arg Lys Leu
785                 790                 795                 800
Met Arg Trp Ile Leu Ile Leu Val Ile Ala Leu Trp Phe Ile Glu Leu
                805                 810                 815
Leu Asp Val Trp Ser Thr Cys Ser Gln Pro Ile Cys Ala Lys Trp Thr

            820                 825                 830
Arg Thr Glu Ala Glu Gly Ser Lys Lys Ser Leu Ser Ser Glu Gly His
        835                 840                 845
His Met Asp Leu Pro Asp Val Val Ile Thr Ser Leu Pro Gly Ser Gly
    850                 855                 860
Ala Glu Ile Leu Lys Gln Leu Phe Phe Asn Ser Ser Asp Phe Leu Tyr
865                 870                 875                 880
Ile Arg Val Pro Thr Ala Tyr Ile Asp Ile Pro Glu Thr Glu Leu Glu
                885                 890                 895
Ile Asp Ser Phe Val Asp Ala Cys Glu Trp Lys Val Ser Asp Ile Arg
            900                 905                 910
Ser Gly His Phe Arg Leu Leu Arg Gly Trp Leu Gln Ser Leu Val Gln
        915                 920                 925
Asp Thr Lys Leu His Leu Gln Asn Ile His Leu His Glu Pro Asn Arg
    930                 935                 940
Gly Lys Leu Ala Gln Tyr Phe Ala Met Asn Lys Asp Lys Lys Arg Lys
945                 950                 955                 960
Phe Lys Arg Arg Glu Ser Leu Pro Glu Gln Arg Ser Gln Met Lys Gly
                965                 970                 975
Ala Phe Asp Arg Asp Ala Glu Tyr Ile Arg Ala Leu Arg Arg His Leu
            980                 985                 990
Val Tyr Tyr Pro Ser Ala Arg Pro Val Leu Ser Leu Ser Ser Gly Ser
        995                 1000                1005
Trp Thr Leu Lys Leu His Phe Phe Gln Glu Val Leu Gly Ala Ser Met
    1010                1015                1020
Arg Ala Leu Tyr Ile Val Arg Asp Pro Arg Ala Trp Ile Tyr Ser Met
1025                1030                1035                1040
Leu Tyr Asn Ser Lys Pro Ser Leu Tyr Ser Leu Lys Asn Val Pro Glu
                1045                1050                1055
His Leu Ala Lys Leu Phe Lys Ile Glu Gly Gly Lys Gly Lys Cys Asn
            1060                1065                1070
Leu Asn Ser Gly Tyr Ala Phe Glu Tyr Glu Pro Leu Arg Lys Glu Leu
        1075                1080                1085
Ser Lys Ser Lys Ser Asn Ala Val Ser Leu Leu Ser His Leu Trp Leu
    1090                1095                1100
Ala Asn Thr Ala Ala Ala Leu Arg Ile Asn Thr Asp Leu Leu Pro Thr
1105                1110                1115                1120
Ser Tyr Gln Leu Val Lys Phe Glu Asp Ile Val His Phe Pro Gln Lys
                1125                1130                1135
Thr Thr Glu Arg Ile Phe Ala Phe Leu Gly Ile Pro Leu Ser Pro Ala
            1140                1145                1150
Ser Leu Asn Gln Ile Leu Phe Ala Thr Ser Thr Asn Leu Phe Tyr Leu
        1155                1160                1165
Pro Tyr Glu Gly Glu Ile Ser Pro Thr Asn Thr Asn Val Trp Lys Gln
    1170                1175                1180
Asn Leu Pro Arg Asp Glu Ile Lys Leu Ile Glu Asn Ile Cys Trp Thr
1185                1190                1195                1200
Leu Met Asp Arg Leu Gly Tyr Pro Lys Phe Met Asp
                1205                1210

<210> 272
<211> 679
<212> PRT
<213> Homo sapiens

<400> 272
Met Ser Ser Gln Val His Val Gln Glu Ala Tyr Tyr Gly Pro Gln Ala

1                   5                   10                  15
Asp Leu Ala His Cys His Ala Tyr Leu Val Glu Met Ser Gln Leu Leu
                20                  25                  30
Gln Ser Met Asp Val Leu His Arg Thr Tyr Ser Ala Pro Ala Ile Asn
            35                  40                  45
Ala Ile Gln Gly Gly Ser Phe Glu Ser Pro Lys Lys Glu Lys Arg Ser
    50                  55                  60
His Arg Arg Trp Arg Ser Arg Ala Ile Gly Lys Asp Ala Lys Gly Thr
65                  70                  75                  80
Leu Gln Val Pro Lys Pro Phe Ser Gly Pro Val Arg Leu His Ser Ser
                85                  90                  95
Asn Pro Asn Leu Ser Thr Leu Asp Phe Gly Glu Glu Lys Asn Tyr Ser
                100                 105                 110
Asp Gly Ser Glu Thr Ser Ser Glu Phe Ser Lys Met Gln Glu Asp Leu
            115                 120                 125
Cys His Ile Ala His Lys Val Tyr Phe Thr Leu Arg Ser Ala Phe Asn
    130                 135                 140
Ile Met Ser Ala Glu Arg Glu Lys Leu Lys Gln Leu Met Glu Gln Asp
145                 150                 155                 160
Ala Ser Ser Ser Pro Ser Ala Gln Val Ile Gly Leu Lys Asn Ala Leu
                165                 170                 175
Ser Ser Ala Leu Ala Gln Asn Thr Asp Leu Lys Glu Arg Leu Arg Arg
                180                 185                 190
Ile His Ala Glu Ser Leu Leu Leu Asp Ser Pro Ala Val Ala Lys Ser
            195                 200                 205
Gly Asp Asn Leu Ala Glu Glu Asn Ser Arg Asp Glu Asn Arg Ala Leu
    210                 215                 220
Val His Gln Leu Ser Asn Glu Ser Arg Leu Ser Ile Thr Asp Ser Leu
225                 230                 235                 240
Ser Glu Phe Phe Asp Ala Gln Glu Val Leu Leu Ser Pro Ser Ser Ser
                245                 250                 255
Glu Asn Glu Ile Ser Asp Asp Asp Ser Tyr Val Ser Asp Ile Ser Asp
                260                 265                 270
Asn Leu Ser Leu Asp Asn Leu Ser Asn Asp Leu Asp Asn Glu Arg Gln
            275                 280                 285
Thr Leu Gly Pro Val Leu Asp Ser Gly Arg Glu Ala Lys Ser Arg Arg
    290                 295                 300
Arg Thr Cys Leu Pro Ala Pro Cys Pro Ser Ser Ser Asn Ile Ser Leu
305                 310                 315                 320
Trp Asn Ile Leu Arg Asn Asn Ile Gly Lys Asp Leu Ser Lys Val Ala
                325                 330                 335
Met Pro Val Glu Leu Asn Glu Pro Leu Asn Thr Leu Gln Arg Leu Cys
                340                 345                 350
Glu Glu Leu Glu Tyr Ser Glu Leu Leu Asp Lys Ala Ala Gln Ile Pro
            355                 360                 365
Ser Pro Leu Glu Arg Met Val Tyr Val Ala Ala Phe Ala Ile Ser Ala
    370                 375                 380
Tyr Ala Ser Ser Tyr Tyr Arg Ala Gly Ser Lys Pro Phe Asn Pro Val
385                 390                 395                 400
Leu Gly Glu Thr Tyr Glu Cys Ile Arg Glu Asp Lys Gly Phe Gln Phe
                405                 410                 415
Phe Ser Glu Gln Val Ser His His Pro Pro Ile Ser Ala Cys His Ala
                420                 425                 430
Glu Ser Arg Asn Phe Val Phe Trp Gln Asp Val Arg Trp Lys Asn Lys
            435                 440                 445
Phe Trp Gly Lys Ser Met Glu Ile Val Pro Ile Gly Thr Thr His Val
    450                 455                 460
Thr Leu Pro Val Phe Gly Asp His Phe Glu Trp Asn Lys Val Thr Ser
465                 470                 475                 480
Cys Ile His Asn Ile Leu Ser Gly Gln Arg Trp Ile Glu His Tyr Gly

                    485                     490                     495
Glu Ile Val Ile Lys Asn Leu His Asp Asp Ser Cys Tyr Cys Lys Val
                500                     505                     510
Asn Phe Ile Lys Ala Lys Tyr Trp Ser Thr Asn Ala His Glu Ile Glu
            515                     520                     525
Gly Thr Val Phe Asp Arg Ser Gly Lys Ala Val His Arg Leu Phe Gly
    530                     535                     540
Lys Trp His Glu Ser Ile Tyr Cys Gly Gly Gly Ser Ser Ser Ala Cys
545                     550                     555                     560
Val Trp Arg Ala Asn Pro Met Pro Lys Gly Tyr Glu Gln Tyr Tyr Ser
                565                     570                     575
Phe Thr Gln Phe Ala Leu Glu Leu Asn Glu Met Asp Pro Ser Ser Lys
                580                     585                     590
Ser Leu Leu Pro Pro Thr Asp Thr Arg Phe Arg Pro Asp Gln Arg Phe
        595                     600                     605
Leu Glu Glu Gly Asn Leu Glu Glu Ala Glu Ile Gln Lys Gln Arg Ile
    610                     615                     620
Glu Gln Leu Gln Arg Glu Arg Arg Arg Val Leu Glu Glu Asn His Val
625.                    630                     635                     640
Glu His Gln Pro Arg Phe Phe Arg Lys Ser Asp Asp Asp Ser Trp Val
                645                     650                     655
Ser Asn Gly Thr Tyr Leu Glu Leu Arg Lys Asp Leu Gly Phe Ser Lys
                660                     665                     670
Leu Asp His Pro Val Leu Trp
        675

<210> 273
<211> 1378
<212> PRT
<213> Homo sapiens

<400> 273
Met Ser Leu Leu Met Phe Thr Gln Leu Leu Leu Cys Gly Phe Leu Tyr
  1               5                  10                  15
Val Arg Val Asp Gly Ser Arg Leu Arg Gln Glu Asp Phe Pro Pro Arg
                 20                  25                  30
Ile Val Glu His Pro Ser Asp Val Ile Val Ser Lys Gly Glu Pro Thr
         35                  40                  45
Thr Leu Asn Cys Lys Ala Glu Gly Arg Pro Thr Pro Thr Ile Glu Trp
     50                  55                  60
Tyr Lys Asp Gly Glu Arg Val Glu Thr Asp Lys Asp Asp Pro Arg Ser
 65                  70                  75                  80
His Arg Met Leu Leu Pro Ser Gly Ser Leu Phe Phe Leu Arg Ile Val
                 85                  90                  95
His Gly Arg Arg Ser Lys Pro Asp Glu Gly Ser Tyr Val Cys Val Ala
            100                 105                 110
Arg Asn Tyr Leu Gly Glu Ala Val Ser Arg Asn Ala Ser Leu Glu Val
        115                 120                 125
Ala Leu Leu Arg Asp Asp Phe Arg Gln Asn Pro Thr Asp Val Val Val
    130                 135                 140
Ala Ala Gly Glu Pro Ala Ile Leu Glu Cys Gln Pro Pro Arg Gly His
145                 150                 155                 160
Pro Glu Pro Thr Ile Tyr Trp Lys Lys Asp Lys Val Arg Ile Asp Asp
                165                 170                 175
Lys Glu Glu Arg Ile Ser Ile Arg Gly Gly Lys Leu Met Ile Ser Asn
            180                 185                 190
Thr Arg Lys Ser Asp Ala Gly Met Tyr Thr Cys Val Gly Thr Asn Met

195                 200                 205
Val Gly Glu Arg Asp Ser Asp Pro Ala Glu Leu Thr Val Phe Glu Arg
    210                 215                 220
Pro Thr Phe Leu Arg Arg Pro Ile Asn Gln Val Val Leu Glu Glu Glu
225                 230                 235                 240
Ala Val Glu Phe Arg Cys Gln Val Gln Gly Asp Pro Gln Pro Thr Val
                245                 250                 255
Arg Trp Lys Lys Asp Asp Ala Asp Leu Pro Arg Gly Arg Tyr Asp Ile
            260                 265                 270
Lys Asp Asp Tyr Thr Leu Arg Ile Lys Lys Thr Met Ser Thr Asp Glu
        275                 280                 285
Gly Thr Tyr Met Cys Ile Ala Glu Asn Arg Val Gly Lys Met Glu Ala
    290                 295                 300
Ser Ala Thr Leu Thr Val Arg Ala Pro Pro Gln Phe Val Val Arg Pro
305                 310                 315                 320
Arg Asp Gln Ile Val Ala Gln Gly Arg Thr Val Thr Phe Pro Cys Glu
                325                 330                 335
Thr Lys Gly Asn Pro Gln Pro Ala Val Phe Trp Gln Lys Glu Gly Ser
            340                 345                 350
Gln Asn Leu Leu Phe Pro Asn Gln Pro Gln Gln Pro Asn Ser Arg Cys
        355                 360                 365
Ser Val Ser Pro Thr Gly Asp Leu Thr Ile Thr Asn Ile Gln Arg Ser
    370                 375                 380
Asp Ala Gly Tyr Tyr Ile Cys Gln Ala Leu Thr Val Ala Gly Ser Ile
385                 390                 395                 400
Leu Ala Lys Ala Gln Leu Glu Val Thr Asp Val Leu Thr Asp Arg Pro
                405                 410                 415
Pro Pro Ile Ile Leu Gln Gly Pro Ala Asn Gln Thr Leu Ala Val Asp
            420                 425                 430
Gly Thr Ala Leu Leu Lys Cys Lys Ala Thr Gly Asp Pro Leu Pro Val
        435                 440                 445
Ile Ser Trp Leu Lys Glu Gly Phe Thr Phe Pro Gly Arg Asp Pro Arg
    450                 455                 460
Ala Thr Ile Gln Glu Gln Gly Thr Leu Gln Ile Lys Asn Leu Arg Ile
465                 470                 475                 480
Ser Asp Thr Gly Thr Tyr Thr Cys Val Ala Thr Ser Ser Ser Gly Glu
                485                 490                 495
Thr Ser Trp Ser Ala Val Leu Asp Val Thr Glu Ser Gly Ala Thr Ile
            500                 505                 510
Ser Lys Asn Tyr Asp Leu Ser Asp Leu Pro Gly Pro Pro Ser Lys Pro
        515                 520                 525
Gln Val Thr Asp Val Thr Lys Asn Ser Val Thr Leu Ser Trp Gln Pro
    530                 535                 540
Gly Thr Pro Gly Thr Leu Pro Ala Ser Ala Tyr Ile Ile Glu Ala Phe
545                 550                 555                 560
Ser Gln Ser Val Ser Asn Ser Trp Gln Thr Val Ala Asn His Val Lys
                565                 570                 575
Thr Thr Leu Tyr Thr Val Arg Gly Leu Arg Pro Asn Thr Ile Tyr Leu
            580                 585                 590
Phe Met Val Arg Ala Ile Asn Pro Gln Gly Leu Ser Asp Pro Ser Pro
        595                 600                 605
Met Ser Asp Pro Val Arg Thr Gln Asp Ile Ser Pro Pro Ala Gln Gly
    610                 615                 620
Val Asp His Arg Gln Val Gln Lys Glu Leu Gly Asp Val Leu Val Arg
625                 630                 635                 640
Leu His Asn Pro Val Val Leu Thr Pro Thr Thr Val Gln Val Thr Trp
                645                 650                 655
Thr Val Asp Arg Gln Pro Gln Phe Ile Gln Gly Tyr Arg Val Met Tyr
            660                 665                 670
Arg Gln Thr Ser Gly Leu Gln Ala Thr Ser Ser Trp Gln Asn Leu Asp

        675                 680                 685
Ala Lys Val Pro Thr Glu Arg Ser Ala Val Leu Val Asn Leu Lys Lys
    690                 695                 700
Gly Val Thr Tyr Glu Ile Lys Val Arg Pro Tyr Phe Asn Glu Phe Gln
705                 710                 715                 720
Gly Met Asp Ser Glu Ser Lys Thr Val Arg Thr Thr Glu Glu Ala Pro
                725                 730                 735
Ser Ala Pro Pro Gln Ser Val Thr Val Leu Thr Val Gly Ser Tyr Asn
            740                 745                 750
Ser Thr Ser Ile Ser Val Ser Trp Asp Pro Pro Pro Pro Asp His Gln
        755                 760                 765
Asn Gly Ile Ile Gln Glu Tyr Lys Ile Trp Cys Leu Gly Asn Glu Thr
    770                 775                 780
Arg Phe His Ile Asn Lys Thr Val Asp Ala Ala Ile Arg Ser Val Ile
785                 790                 795                 800
Ile Gly Gly Leu Phe Pro Gly Ile Gln Tyr Arg Val Glu Val Ala Ala
                805                 810                 815
Ser Thr Ser Ala Gly Val Gly Val Lys Ser Glu Pro Gln Pro Ile Ile
            820                 825                 830
Ile Gly Arg Arg Asn Glu Val Val Ile Thr Glu Asn Asn Asn Ser Ile
        835                 840                 845
Thr Glu Gln Ile Thr Asp Val Val Lys Gln Pro Ala Phe Ile Ala Gly
    850                 855                 860
Ile Gly Gly Ala Cys Trp Val Ile Leu Met Gly Phe Ser Ile Trp Leu
865                 870                 875                 880
Tyr Trp Arg Arg Lys Lys Arg Lys Gly Leu Ser Asn Tyr Ala Val Thr
                885                 890                 895
Phe Gln Arg Gly Asp Gly Gly Leu Met Ser Asn Gly Ser Arg Pro Gly
            900                 905                 910
Leu Leu Asn Ala Gly Asp Pro Ser Tyr Pro Trp Leu Ala Asp Ser Trp
        915                 920                 925
Pro Ala Thr Ser Leu Pro Val Asn Asn Ser Asn Ser Gly Pro Asn Glu
    930                 935                 940
Ile Gly Asn Phe Gly Arg Gly Asp Val Leu Pro Pro Val Pro Gly Gln
945                 950                 955                 960
Gly Asp Lys Thr Ala Thr Met Leu Ser Asp Gly Ala Ile Tyr Ser Ser
                965                 970                 975
Ile Asp Phe Thr Thr Lys Thr Ser Tyr Asn Ser Ser Ser Gln Ile Thr
            980                 985                 990
Gln Ala Thr Pro Tyr Ala Thr Thr Gln Ile Leu His Ser Asn Ser Ile
        995                 1000                1005
His Glu Leu Ala Val Asp Leu Pro Asp Pro Gln Trp Lys Ser Ser Ile
    1010                1015                1020
Gln Gln Lys Thr Asp Leu Met Gly Phe Gly Tyr Ser Leu Pro Asp Gln
1025                1030                1035                1040
Asn Lys Gly Asn Asn Gly Gly Lys Gly Glu Lys Lys Lys Lys Asn Lys
                1045                1050                1055
Asn Ser Ser Lys Pro Gln Lys Asn Asn Gly Ser Thr Trp Ala Asn Val
            1060                1065                1070
Pro Leu Pro Pro Pro Pro Val Gln Pro Leu Pro Gly Thr Glu Leu Glu
        1075                1080                1085
His Tyr Ala Val Glu Gln Gln Glu Asn Gly Tyr Asp Ser Asp Ser Trp
    1090                1095                1100
Cys Pro Pro Leu Pro Val Gln Thr Tyr Leu His Gln Gly Leu Glu Asp
1105                1110                1115                1120
Glu Leu Glu Glu Asp Asp Asp Arg Val Pro Thr Pro Pro Val Arg Gly
                1125                1130                1135
Val Ala Ser Ser Pro Ala Ile Ser Phe Gly Gln Gln Ser Thr Ala Thr
            1140                1145                1150
Leu Thr Pro Ser Pro Arg Glu Glu Met Gln Pro Met Leu Gln Ala His

1155                1160                1165
Leu Asp Glu Leu Thr Arg Ala Tyr Gln Phe Asp Ile Ala Lys Gln Thr
    1170                1175                1180
Trp His Ile Gln Ser Asn Asn Gln Pro Pro Gln Pro Pro Val Pro Pro
1185                1190                1195                1200
Leu Gly Tyr Val Ser Gly Ala Leu Ile Ser Asp Leu Glu Thr Asp Val
                1205                1210                1215
Ala Asp Asp Asp Ala Asp Asp Glu Glu Glu Ala Leu Glu Ile Pro Arg
            1220                1225                1230
Pro Leu Arg Ala Leu Asp Gln Thr Pro Gly Ser Ser Met Asp Asn Leu
        1235                1240                1245
Asp Ser Ser Val Thr Gly Lys Ala Phe Thr Ser Ser Gln Arg Pro Arg
    1250                1255                1260
Pro Thr Ser Pro Phe Ser Thr Asp Ser Asn Thr Ser Ala Ala Leu Ser
1265                1270                1275                1280
Gln Ser Gln Arg Pro Arg Pro Thr Lys Lys His Lys Gly Gly Arg Met
                1285                1290                1295
Asp Gln Gln Pro Ala Leu Pro His Arg Arg Glu Gly Met Thr Asp Glu
            1300                1305                1310
Glu Ala Leu Val Pro Tyr Ser Lys Pro Ser Phe Pro Ser Pro Gly Gly
        1315                1320                1325
His Ser Ser Ser Gly Thr Ala Ser Ser Lys Gly Ser Thr Gly Pro Arg
    1330                1335                1340
Lys Thr Glu Val Leu Arg Ala Gly His Gln Arg Asn Ala Ser Asp Leu
1345                1350                1355                1360
Leu Asp Ile Gly Tyr Met Gly Ser Asn Ser Gln Gly Gln Phe Thr Gly
                1365                1370                1375
Glu Leu

<210> 274
<211> 1522
<212> PRT
<213> Homo sapiens

<400> 274
Met Cys Asp Leu Ser Asn Ala Ser Val Lys Thr Thr Ala Lys Asp Ile
1               5                   10                  15
Gln Gln Thr Glu Gln Thr Ile Glu Gln Lys Leu Val Gln Ala Gln Asn
            20                  25                  30
Leu Thr Gln Gly Trp Glu Glu Ile Lys His Leu Lys Ser Glu Leu Trp
        35                  40                  45
Ile Tyr Leu Gln Asp Ala Asp Gln Gln Leu Gln Asn Met Lys Arg Arg
    50                  55                  60
His Ser Glu Leu Glu Leu Asn Ile Ala Gln Asn Met Val Ser Gln Val
65                  70                  75                  80
Lys Asp Phe Val Lys Lys Leu Gln Ser Lys Gln Ala Ser Val Asn Thr
                85                  90                  95
Ile Ile Glu Lys Val Asn Lys Leu Thr Lys Lys Glu Glu Ser Pro Glu
            100                 105                 110
His Lys Glu Ile Asn His Leu Asn Asp Gln Trp Leu Asp Leu Cys Arg
        115                 120                 125
Gln Ser Asn Asn Leu Cys Leu Gln Arg Glu Glu Asp Leu Gln Arg Thr
    130                 135                 140
Arg Asp Tyr His Asp Cys Met Asn Val Val Glu Val Phe Leu Glu Lys
145                 150                 155                 160
Phe Thr Thr Glu Trp Asp Asn Leu Ala Arg Ser Asp Ala Glu Ser Thr

                165                 170                 175
Ala Val His Leu Glu Ala Leu Lys Lys Leu Ala Leu Ala Leu Gln Glu
            180                 185                 190
Arg Lys Tyr Ala Ile Glu Asp Leu Lys Asp Gln Lys Gln Lys Met Ile
        195                 200                 205
Glu His Leu Asn Leu Asp Asp Lys Glu Leu Val Lys Glu Gln Thr Ser
    210                 215                 220
His Leu Glu Gln Arg Trp Phe Gln Leu Glu Asp Leu Ile Lys Arg Lys
225                 230                 235                 240
Ile Gln Val Ser Val Thr Asn Leu Glu Glu Leu Asn Val Val Gln Ser
                245                 250                 255
Arg Phe Gln Glu Leu Met Glu Trp Ala Glu Glu Gln Gln Pro Asn Ile
            260                 265                 270
Ala Glu Ala Leu Lys Gln Ser Pro Pro Pro Asp Met Ala Gln Asn Leu
        275                 280                 285
Leu Met Asp His Leu Ala Ile Cys Ser Glu Leu Glu Ala Lys Gln Met
    290                 295                 300
Leu Leu Lys Ser Leu Ile Lys Asp Ala Asp Arg Val Met Ala Asp Leu
305                 310                 315                 320
Gly Leu Asn Glu Arg Gln Val Ile Gln Lys Ala Leu Ser Asp Ala Gln
                325                 330                 335
Ser His Val Asn Cys Leu Ser Asp Leu Val Gly Gln Arg Arg Lys Tyr
            340                 345                 350
Leu Asn Lys Ala Leu Ser Glu Lys Thr Gln Phe Leu Met Ala Val Phe
        355                 360                 365
Gln Ala Thr Ser Gln Ile Gln Gln His Glu Arg Lys Ile Met Phe Arg
    370                 375                 380
Glu His Ile Cys Leu Leu Pro Asp Asp Val Ser Lys Gln Val Lys Thr
385                 390                 395                 400
Cys Lys Ser Ala Gln Ala Ser Leu Lys Thr Tyr Gln Asn Glu Val Thr
                405                 410                 415
Gly Leu Trp Ala Gln Gly Arg Glu Leu Met Lys Glu Val Thr Glu Gln
            420                 425                 430
Glu Lys Ser Glu Val Leu Gly Lys Leu Gln Glu Leu Gln Ser Val Tyr
        435                 440                 445
Asp Ser Val Leu Gln Lys Cys Ser His Arg Leu Gln Glu Leu Glu Lys
    450                 455                 460
Asn Leu Val Ser Arg Lys His Phe Lys Glu Asp Phe Asp Lys Ala Cys
465                 470                 475                 480
His Trp Leu Lys Gln Ala Asp Ile Val Thr Phe Pro Glu Ile Asn Leu
                485                 490                 495
Met Asn Glu Ser Ser Glu Leu His Thr Gln Leu Ala Lys Tyr Gln Asn
            500                 505                 510
Ile Leu Glu Gln Ser Pro Glu Tyr Glu Asn Leu Leu Leu Thr Leu Gln
        515                 520                 525
Arg Thr Gly Gln Thr Ile Leu Pro Ser Leu Asn Glu Val Asp His Ser
    530                 535                 540
Tyr Leu Ser Glu Lys Leu Asn Ala Leu Pro Arg Gln Phe Asn Val Ile
545                 550                 555                 560
Val Ala Leu Ala Lys Asp Lys Phe Tyr Lys Val Gln Glu Ala Ile Leu
                565                 570                 575
Ala Arg Lys Glu Tyr Ala Ser Leu Ile Glu Leu Thr Thr Gln Ser Leu
            580                 585                 590
Ser Glu Leu Glu Ala Gln Phe Leu Arg Met Ser Lys Val Pro Thr Asp
        595                 600                 605
Leu Ala Val Glu Glu Ala Leu Ser Leu Gln Asp Gly Cys Arg Ala Ile
    610                 615                 620
Leu Asp Glu Val Ala Gly Leu Gly Glu Ala Val Asp Glu Leu Asn Gln
625                 630                 635                 640
Lys Lys Glu Gly Phe Arg Ser Thr Gly Gln Pro Trp Gln Pro Asp Lys

                645                 650                 655
Met Leu His Leu Val Thr Leu Tyr His Arg Leu Lys Arg Gln Thr Glu
            660                 665                 670
Gln Arg Val Ser Leu Leu Glu Asp Thr Thr Ser Ala Tyr Gln Glu His
        675                 680                 685
Glu Lys Met Cys Gln Gln Leu Glu Arg Gln Leu Lys Ser Val Lys Glu
    690                 695                 700
Glu Gln Ser Lys Val Asn Glu Glu Thr Leu Pro Ala Glu Glu Lys Leu
705                 710                 715                 720
Lys Met Tyr His Ser Leu Ala Gly Ser Leu Gln Asp Ser Gly Ile Val
                725                 730                 735
Leu Lys Arg Val Thr Ile His Leu Glu Asp Leu Ala Pro His Leu Asp
            740                 745                 750
Pro Leu Ala Tyr Glu Lys Ala Arg His Gln Ile Gln Ser Trp Gln Gly
        755                 760                 765
Glu Leu Lys Leu Leu Thr Ser Ala Ile Gly Glu Thr Val Thr Glu Cys
    770                 775                 780
Glu Ser Arg Met Val Gln Ser Ile Asp Phe Gln Thr Glu Met Ser Arg
785                 790                 795                 800
Ser Leu Asp Trp Leu Arg Arg Val Lys Ala Glu Leu Ser Gly Pro Val
                805                 810                 815
Tyr Leu Asp Leu Asn Leu Gln Asp Ile Gln Glu Glu Ile Arg Lys Ile
            820                 825                 830
Gln Ile His Gln Glu Glu Val Gln Ser Ser Leu Arg Ile Met Asn Ala
        835                 840                 845
Leu Ser His Lys Glu Lys Glu Lys Phe Thr Lys Ala Lys Glu Leu Ile
    850                 855                 860
Ser Ala Asp Leu Glu His Ser Leu Ala Glu Leu Ser Glu Leu Asp Gly
865                 870                 875                 880
Asp Ile Gln Glu Ala Leu Arg Thr Arg Gln Ala Thr Leu Thr Glu Ile
                885                 890                 895
Tyr Ser Gln Cys Gln Arg Tyr Tyr Gln Val Phe Gln Ala Ala Asn Asp
            900                 905                 910
Trp Leu Glu Asp Ala Gln Glu Met Leu Gln Leu Ala Gly Asn Gly Leu
        915                 920                 925
Asp Val Glu Ser Ala Glu Glu Asn Leu Lys Ser His Met Glu Phe Phe
    930                 935                 940
Ser Thr Glu Asp Gln Phe His Ser Asn Leu Glu Glu Leu His Ser Leu
945                 950                 955                 960
Val Ala Thr Leu Asp Pro Leu Ile Lys Pro Thr Gly Lys Glu Asp Leu
                965                 970                 975
Glu Gln Lys Val Ala Ser Leu Glu Leu Arg Ser Gln Arg Met Ser Arg
            980                 985                 990
Asp Ser Gly Ala Gln Val Asp Leu Leu Gln Arg Cys Thr Ala Gln Trp
        995                 1000                1005
His Asp Tyr Gln Lys Ala Arg Glu Glu Val Ile Glu Leu Met Asn Asp
    1010                1015                1020
Thr Glu Lys Lys Leu Ser Glu Phe Ser Leu Leu Lys Thr Ser Ser Ser
1025                1030                1035                1040
His Glu Ala Glu Glu Lys Leu Ser Glu His Lys Ala Leu Val Ser Val
                1045                1050                1055
Val Asn Ser Phe His Glu Lys Ile Val Ala Leu Glu Glu Lys Ala Ser
            1060                1065                1070
Gln Leu Glu Lys Thr Gly Asn Asp Ala Ser Lys Ala Thr Leu Ser Arg
        1075                1080                1085
Ser Met Thr Thr Val Trp Gln Arg Trp Thr Arg Leu Arg Ala Val Ala
    1090                1095                1100
Gln Asp Gln Glu Lys Ile Leu Glu Asp Ala Val Asp Glu Trp Thr Gly
1105                1110                1115                1120
Phe Asn Asn Lys Val Lys Lys Ala Thr Glu Met Ile Asp Gln Leu Gln

1125                1130                1135
Asp Lys Leu Pro Gly Ser Ser Ala Glu Lys Ala Ser Lys Ala Glu Leu
        1140                1145                1150
Leu Thr Leu Leu Glu Tyr His Asp Thr Phe Val Leu Glu Leu Glu Gln
    1155                1160                1165
Gln Gln Ser Ala Leu Gly Met Leu Arg Gln Gln Thr Leu Ser Met Leu
1170                1175                1180
Gln Asp Gly Ala Ala Pro Thr Pro Gly Glu Glu Pro Pro Leu Met Gln
1185                1190                1195                1200
Glu Ile Thr Ala Met Gln Asp Arg Cys Leu Asn Met Gln Glu Lys Val
                1205                1210                1215
Lys Thr Asn Gly Lys Leu Val Lys Gln Glu Leu Lys Asp Arg Glu Met
        1220                1225                1230
Val Glu Thr Gln Ile Asn Ser Val Lys Cys Trp Val Gln Glu Thr Lys
    1235                1240                1245
Glu Tyr Leu Gly Asn Pro Thr Ile Glu Ile Asp Ala Gln Leu Glu Glu
1250                1255                1260
Leu Gln Ile Leu Leu Thr Glu Ala Thr Asn His Arg Gln Asn Ile Glu
1265                1270                1275                1280
Lys Met Ala Glu Glu Gln Lys Glu Lys Tyr Leu Gly Leu Tyr Thr Ile
                1285                1290                1295
Leu Pro Ser Glu Leu Ser Leu Gln Leu Ala Glu Val Ala Leu Asp Leu
        1300                1305                1310
Lys Ile Arg Asp Gln Ile Gln Asp Lys Ile Lys Glu Val Glu Gln Ser
    1315                1320                1325
Lys Ala Thr Ser Gln Glu Leu Ser Arg Gln Ile Gln Lys Leu Ala Lys
1330                1335                1340
Asp Leu Thr Thr Ile Leu Thr Lys Leu Lys Ala Lys Thr Asp Asn Val
1345                1350                1355                1360
Val Gln Ala Lys Thr Asp Gln Lys Val Leu Gly Glu Glu Leu Asp Gly
                1365                1370                1375
Cys Asn Ser Lys Leu Met Glu Leu Asp Ala Ala Val Gln Lys Phe Leu
        1380                1385                1390
Glu Gln Asn Gly Gln Leu Gly Lys Pro Leu Ala Lys Lys Ile Gly Lys
    1395                1400                1405
Leu Thr Glu Leu His Gln Gln Thr Ile Arg Gln Ala Glu Asn Arg Leu
1410                1415                1420
Ser Lys Leu Asn Gln Ala Ala Ser His Leu Glu Glu Tyr Asn Glu Met
1425                1430                1435                1440
Leu Glu Leu Ile Leu Lys Trp Ile Glu Lys Ala Lys Val Leu Ala His
                1445                1450                1455
Gly Thr Ile Ala Trp Asn Ser Ala Ser Gln Leu Arg Glu Gln Tyr Ile
        1460                1465                1470
Leu His Gln Glu Val Gly Gln Pro Arg Ala Ser His Glu Ser Thr Ser
    1475                1480                1485
His Ala Gly Tyr Val Ser Ser Phe Pro Leu Lys Leu His Glu Lys Asn
1490                1495                1500
Val Pro Phe Leu Ser Val Thr Ser Ser Arg Lys Leu Phe His Pro Phe
1505                1510                1515                1520
Val Thr

<210> 275
<211> 324
<212> PRT
<213> Homo sapiens

<400> 275
Met Met Pro Phe Thr Glu Val Lys Arg Thr Cys Ala Asn Ser Gln Thr
1               5                   10                  15
Arg Met Ala Leu Ser Ala Val Ser Glu Leu Ile Thr Ser Lys Glu Gln
            20                  25                  30
Leu Tyr Glu Met Phe Gln Gln Ile Leu Gly Ile Lys Lys Leu Glu His
        35                  40                  45
Gln Leu Leu Tyr Asn Ala Cys Gln Leu Asp Asn Ala Asp Glu Gln Ala
    50                  55                  60
Ala Gln Ile Arg Arg Glu Leu Asp Gly Arg Leu Gln Leu Ala Asp Lys
65                  70                  75                  80
Met Ala Lys Lys Tyr Val Asn Val Ile Leu Glu Glu Lys Lys Ser Arg
                85                  90                  95
Trp Pro Glu Thr Leu Gly Leu Ile Cys Glu Val Val Pro Asp Val Pro
            100                 105                 110
Ser Asn Pro Met Asn Glu Leu Val Gln Glu Arg Lys Phe Pro Lys Phe
        115                 120                 125
Ile Ala Lys Asp Met Glu Asn Met Tyr Ile Glu Glu Leu Arg Ser Ser
    130                 135                 140
Val Asn Leu Leu Met Ala Asn Leu Glu Ser Leu Pro Val Ser Lys Gly
145                 150                 155                 160
Gly Pro Glu Phe Lys Leu Gln Lys Leu Lys Arg Ser Gln Asn Ser Ala
                165                 170                 175
Phe Leu Asp Ile Gly Asp Glu Asn Glu Ile Gln Leu Ser Lys Ser Asp
            180                 185                 190
Val Val Leu Ser Phe Thr Leu Glu Ile Val Ile Met Glu Val Gln Gly
        195                 200                 205
Leu Lys Ser Val Ala Pro Asn Arg Ile Val Tyr Cys Thr Met Glu Val
    210                 215                 220
Glu Gly Glu Lys Leu Gln Thr Asp Gln Ala Glu Ala Ser Arg Pro Gln
225                 230                 235                 240
Trp Gly Thr Gln Gly Asp Phe Thr Thr Thr His Pro Arg Pro Val Val
                245                 250                 255
Lys Val Lys Leu Phe Thr Glu Ser Thr Gly Val Leu Ala Leu Glu Asp
            260                 265                 270
Lys Glu Leu Gly Arg Val Ile Leu Tyr Pro Thr Ser Asn Ser Ser Lys
        275                 280                 285
Ser Ala Glu Leu His Arg Met Val Val Pro Lys Asn Ser Gln Asp Ser
    290                 295                 300
Asp Leu Lys Ile Lys Leu Ala Val Arg Met Asp Lys Pro Ala His Met
305                 310                 315                 320
Lys His Ser Gly

<210> 276
<211> 157
<212> PRT
<213> Homo sapiens

<400> 276
Met Ala Thr Ser Val Leu Cys Cys Leu Arg Cys Cys Arg Asp Gly Gly
1               5                   10                  15
Thr Gly His Ile Pro Leu Lys Glu Met Pro Ala Val Gln Leu Asp Thr
            20                  25                  30
Gln His Met Gly Ile Trp Gly Ile Gly Val Ala Thr Gln Lys Val Asn
        35                  40                  45
Leu Asn Gln Ile Pro Leu Gly Arg Asp Met His Ser Leu Val Met Arg

50                  55                  60
Asn Asp Gly Ala Leu Tyr His Asn Asn Glu Glu Lys Asn Arg Leu Pro
65                  70                  75                  80
Ala Asn Ser Leu Pro Gln Glu Gly Asp Val Val Gly Ile Thr Tyr Asp
                85                  90                  95
His Val Glu Leu Asn Val Tyr Leu Asn Gly Lys Asn Met His Cys Pro
            100                 105                 110
Ala Ser Gly Ile Arg Gly Thr Val Tyr Pro Val Val Tyr Val Asp Asp
        115                 120                 125
Ser Ala Ile Leu Asp Cys Gln Phe Ser Glu Phe Tyr His Thr Pro Pro
    130                 135                 140
Pro Gly Phe Glu Lys Ile Leu Phe Glu Gln Gln Ile Phe
145                 150                 155

<210> 277
<211> 343
<212> PRT
<213> Homo sapiens

<400> 277
Met Lys Met Glu Glu Ala Val Gly Lys Val Glu Glu Leu Ile Glu Ser
1               5                   10                  15
Glu Ala Pro Pro Lys Ala Ser Glu Gln Glu Thr Ala Lys Glu Glu Asp
            20                  25                  30
Gly Ser Val Glu Leu Glu Ser Gln Val Gln Lys Asp Gly Val Ala Asp
        35                  40                  45
Ser Thr Val Ile Ser Ser Met Pro Cys Leu Leu Met Glu Leu Arg Arg
    50                  55                  60
Asp Ser Ser Glu Ser Gln Leu Ala Ser Thr Glu Ser Asp Lys Pro Thr
65                  70                  75                  80
Thr Gly Arg Val Tyr Glu Ser Asp Ser Ser Asn His Cys Met Leu Ser
                85                  90                  95
Pro Ser Ser Ser Gly His Leu Ala Asp Ser Asp Thr Leu Ser Ser Ala
            100                 105                 110
Glu Glu Asn Glu Pro Ser Gln Ala Glu Thr Ala Val Glu Gly Asp Pro
        115                 120                 125
Ser Gly Val Ser Gly Ala Thr Val Gly Arg Lys Ser Arg Arg Ser Arg
    130                 135                 140
Ser Glu Ser Glu Thr Ser Thr Met Ala Ala Lys Lys Asn Arg Gln Ser
145                 150                 155                 160
Ser Asp Lys Gln Asn Gly Arg Val Ala Lys Val Lys Gly His Arg Ser
                165                 170                 175
Gln Lys His Lys Glu Arg Ile Arg Leu Leu Arg Gln Lys Arg Glu Ala
            180                 185                 190
Ala Ala Arg Lys Lys Tyr Asn Leu Leu Gln Asp Ser Ser Thr Ser Asp
        195                 200                 205
Ser Asp Leu Thr Cys Asp Ser Ser Thr Ser Ser Ser Asp Asp Asp Glu
    210                 215                 220
Glu Val Ser Gly Ser Ser Lys Thr Ile Thr Ala Glu Ile Pro Gly His
225                 230                 235                 240
Leu Asp Pro Gly Phe Leu Ala Ser Asp Lys Thr Ser Ala Gly Asn Ala
                245                 250                 255
Pro Leu Asn Glu Glu Ile Asn Ile Ala Ser Ser Asp Ser Glu Val Glu
            260                 265                 270
Ile Val Gly Val Gln Glu His Ala Arg Cys Val His Pro Arg Gly Gly
        275                 280                 285
Val Ile Gln Ser Val Ser Ser Trp Lys His Gly Ser Gly Thr Gln Tyr

290                 295                 300
Val Ser Thr Arg Gln Thr Gln Ser Trp Thr Ala Val Thr Pro Gln Gln
305                 310                 315                 320
Thr Trp Ala Ser Pro Ala Glu Val Val Asp Leu Thr Leu Asp Glu Asp
                325                 330                 335
Ser Arg Arg Lys Tyr Leu Leu
            340

<210> 278
<211> 142
<212> PRT
<213> Homo sapiens

<400> 278
Met Gln Leu Thr His Gln Leu Asp Leu Phe Pro Glu Cys Arg Ile Val
  1               5                  10                  15
Asp Pro Phe Gln Ile Leu Val Ala Ala Asn Lys Ala Val His Leu Tyr
             20                  25                  30
Lys Leu Gly Lys Met Lys Thr Arg Thr Leu Ser Thr Glu Ile Ile Phe
         35                  40                  45
Asn Leu Ser Pro Asn Asn Asn Ile Ser Glu Ala Leu Lys Lys Phe Gly
     50                  55                  60
Ile Ser Ala Asn Asp Thr Ser Ile Leu Ile Val Tyr Ile Glu Glu Gly
 65                  70                  75                  80
Glu Lys Gln Ile Asn Gln Glu Tyr Leu Ile Ser Gln Val Glu Gly His
                 85                  90                  95
Gln Val Ser Leu Lys Asn Leu Pro Glu Ile Met Asn Ile Thr Glu Val
            100                 105                 110
Lys Lys Ile Tyr Lys Leu Ser Ser Gln Glu Glu Ser Ile Gly Thr Leu
        115                 120                 125
Leu Asp Ala Ile Ile Cys Arg Met Ser Thr Lys Asp Val Leu
    130                 135                 140

<210> 279
<211> 186
<212> PRT
<213> Homo sapiens

<400> 279
Met Asp Thr Phe Ser Thr Lys Ser Leu Ala Leu Gln Ala Gln Lys Lys
  1               5                  10                  15
Leu Leu Ser Lys Met Ala Ser Lys Ala Val Val Ala Val Leu Val Asp
             20                  25                  30
Asp Thr Ser Ser Glu Val Leu Asp Glu Leu Tyr Arg Ala Thr Arg Glu
         35                  40                  45
Phe Thr Arg Ser Arg Lys Glu Ala Gln Lys Met Leu Lys Asn Leu Val
     50                  55                  60
Lys Val Ala Leu Lys Leu Gly Leu Leu Leu Arg Gly Asp Gln Leu Gly
 65                  70                  75                  80
Gly Glu Glu Leu Ala Leu Leu Arg Arg Phe Arg His Arg Ala Arg Cys
                 85                  90                  95
Leu Ala Met Thr Ala Val Ser Phe His Gln Val Asp Phe Thr Phe Asp
            100                 105                 110
Arg Arg Val Leu Ala Val Gly Leu Leu Glu Cys Arg Asp Leu Leu His

115                 120                 125
Gln Ala Val Gly Pro His Leu Thr Ala Lys Ser His Gly Arg Ile Asn
    130                 135                 140
His Val Phe Gly His Leu Ala Asp Cys Asp Phe Leu Ala Ala Leu Tyr
145                 150                 155                 160
Gly Pro Ala Glu Pro Tyr Arg Ser His Leu Arg Arg Ile Cys Glu Gly
                165                 170                 175
Leu Gly Arg Met Leu Asp Glu Gly Ser Leu
            180                 185

<210> 280
<211> 400
<212> PRT
<213> Homo sapiens

<400> 280
Met Arg Thr Leu Val Gly Leu Leu Thr Ser Asn Gln Ala Leu Leu Gln
1               5                   10                  15
Leu Glu Ala Ala Arg Cys Leu His Glu Leu Ser His Ser Glu Gln Ser
            20                  25                  30
Thr Val Ala Glu Ala Cys Leu Pro Ala Thr Ser Tyr Leu Leu Thr Tyr
        35                  40                  45
Leu Ser Ser His Ser Ser Asp Phe Ile Pro Gly Met Lys Gln Ala Lys
    50                  55                  60
Pro Ser Ala Phe Val Ala Cys Ser Gln Glu Leu Cys Leu Tyr Thr Leu
65                  70                  75                  80
Gly Asn Leu Ile Val Glu Ser Glu Ala Val Arg Arg Gln Leu Leu Pro
                85                  90                  95
Gln Gly Ile Val Pro Ala Leu Ala Ala Cys Ile Gln Ser Pro His Val
            100                 105                 110
Ala Val Leu Glu Ala Leu Gly Tyr Ala Leu Ser Gln Leu Leu Gln Ala
        115                 120                 125
Glu Glu Ala Pro Glu Lys Ile Ile Pro Ser Ile Leu Ala Ser Thr Leu
    130                 135                 140
Pro Gln His Met Leu Gln Met Leu Gln Pro Gly Pro Lys Leu Asn Pro
145                 150                 155                 160
Gly Val Ala Val Glu Phe Ala Trp Cys Leu His Tyr Ile Ile Cys Ser
                165                 170                 175
Gln Val Ser Asn Pro Leu Leu Ile Gly His Gly Ala Leu Ser Thr Leu
            180                 185                 190
Gly Leu Leu Leu Leu Asp Leu Ala Gly Ala Val Gln Lys Ser Glu Asp
        195                 200                 205
Ala Gly Leu Glu Leu Leu Ala Cys Pro Val Leu Arg Cys Leu Ser Asn
    210                 215                 220
Leu Leu Thr Glu Ala Ala Val Glu Thr Val Gly Gly Gln Met Gln Leu
225                 230                 235                 240
Arg Asp Glu Arg Val Val Ala Ala Leu Phe Ile Leu Leu Gln Phe Phe
                245                 250                 255
Phe Gln Lys Gln Pro Ser Leu Leu Pro Glu Gly Leu Trp Leu Leu Asn
            260                 265                 270
Asn Leu Thr Ala Asn Ser Pro Ser Phe Cys Thr Ser Leu Leu Ser Leu
        275                 280                 285
Asp Leu Ile Glu Pro Leu Leu Gln Leu Leu Pro Val Ser Asn Val Val
    290                 295                 300
Ser Val Met Val Leu Thr Val Leu Cys Asn Val Ala Glu Lys Gly Pro
305                 310                 315                 320
Ala Tyr Cys Gln Arg Leu Trp Pro Gly Pro Leu Leu Pro Ala Leu Leu

                325                 330                 335
His Thr Leu Ala Phe Ser Asp Thr Glu Val Val Gly Gln Ser Leu Glu
            340                 345                 350
Leu Leu His Leu Leu Phe Leu Tyr Gln Pro Glu Ala Val Gln Val Phe
        355                 360                 365
Leu Gln Gln Ser Gly Leu Gln Ala Leu Glu Arg His Gln Glu Glu Ala
    370                 375                 380
Gln Leu Gln Asp Arg Val Tyr Ala Leu Gln Gln Thr Ala Leu Gln Gly
385                 390                 395                 400

<210> 281
<211> 77
<212> PRT
<213> Homo sapiens

<400> 281

<210> 282
<211> 177
<212> PRT
<213> Homo sapiens

<400> 282
Met Arg Ser Leu Cys Gln Pro Trp Ser Phe Leu Arg Pro Arg Arg Arg
1               5                   10                  15
Gly Ala Pro Leu Arg Cys Pro Arg Gly Arg Pro Lys Pro Pro Arg Arg
            20                  25                  30
Arg Leu Arg Leu Gly Leu Gly Asn Ser Gln Thr Gln Arg Glu Pro Thr
        35                  40                  45
Met Val Leu Ser Pro Ala Asp Lys Thr Asn Val Lys Ala Ala Trp Gly
    50                  55                  60
Lys Val Gly Ala His Ala Gly Glu Tyr Gly Ala Glu Ala Leu Glu Arg
65                  70                  75                  80
His Phe Asp Leu Ser His Gly Ser Ala Gln Val Lys Gly His Gly Lys
                85                  90                  95
Lys Val Ala Asp Ala Leu Thr Asn Ala Val Ala His Val Asp Asp Met
            100                 105                 110
Pro Asn Ala Leu Ser Ala Leu Ser Asp Leu His Ala His Lys Leu Arg
        115                 120                 125
Val Asp Pro Val Asn Phe Lys Leu Leu Ser His Cys Leu Leu Val Thr
    130                 135                 140
Leu Ala Ala His Leu Pro Ala Glu Phe Thr Pro Ala Val His Ala Ser

145                 150                 155                 160
Leu Asp Lys Phe Leu Ala Ser Val Ser Thr Val Leu Thr Ser Lys Tyr
                165                 170                 175
Arg

<210> 283
<211> 200
<212> PRT
<213> Homo sapiens

<400> 283

<210> 284
<211> 47
<212> PRT
<213> Homo sapiens

<400> 284
Met Trp Gly Ser Gly Leu Arg Ser Leu Ser Ala Leu Gln Gly His Leu
1               5                   10                  15
Glu Ala Cys Leu Leu Ala Ser Gly Gly Gly Ala Arg Leu Gln Glu Gly
            20                  25                  30
Pro Ala Thr Cys His Leu Pro Cys Asp Gln Ala Lys Lys Trp Asn
        35                  40                  45

<210> 285
<211> 108
<212> PRT
<213> Homo sapiens

<400> 285
Met Thr Arg Leu Asp Cys Leu Met Ser Phe Asn Leu Pro Arg Asn Pro
1               5                   10                  15
Leu Val Gly Ile Thr Ser Phe Phe Met Ile Leu Tyr Phe Ser Thr Ser
            20                  25                  30
Asn Ser Lys Asn Ser Ile Gln Pro Leu Lys Leu Thr Ser Asn Ser Ser
        35                  40                  45
Leu Arg Ser Phe Ser Phe Leu Arg Asn Gln Met Tyr Ile Phe Glu Leu
    50                  55                  60
Ser Leu Tyr Leu Glu Gly Thr Ser Phe Val Val Val Leu Leu Phe Leu
65                  70                  75                  80
Leu Ile Ser Val Ser Leu Asp Ser Pro Pro Thr Thr Lys Gly Trp Asp
                85                  90                  95
Ser Val Leu His Ile Trp Val Pro Leu Ile Val Gln
            100                 105

<210> 286
<211> 44
<212> PRT
<213> Homo sapiens

<400> 286
Met Phe His Val Phe Val Leu Leu Leu Thr Phe Ile Ala Leu Ser Pro
1               5                   10                  15
Ser Gly Ile Arg Leu Leu Phe Gly Phe Ile Gln Lys Gly Leu Asn Leu
            20                  25                  30
Asn Ser Phe Met Phe Arg Leu Glu Leu Leu His Phe
        35                  40

<210> 287
<211> 129
<212> PRT
<213> Homo sapiens

<400> 287
Met Phe Pro Val Ser Ser Gly Cys Phe Gln Glu Gln Gln Glu Thr Asn
1               5                   10                  15
Lys Ser Leu Pro His Ser Ala Ser Thr Pro Glu Thr Arg Thr Lys Phe
            20                  25                  30
Thr Gln Asp Asn Leu Cys His Ala Gln Arg Glu Arg Leu Asp Ser Ala
        35                  40                  45
Asn Leu Trp Val Leu Val Asp Cys Ile Leu Arg Asp Thr Ser Glu Asp
    50                  55                  60
Leu Gly Leu Gln Cys Asp Ala Val Asn Leu Ala Phe Gly Arg Arg Cys
65                  70                  75                  80
Glu Glu Leu Glu Asp Ala Arg His Lys Leu Gln His His Leu His Lys
                85                  90                  95
Met Leu Arg Glu Ile Thr Asp Gln Glu His Asn Val Val Ala Leu Lys

                100                 105                 110
Glu Ala Ile Lys Asp Lys Glu Glu Pro Leu His Ile Ala Gln Thr Arg
        115                 120                 125
Leu

<210> 288
<211> 167
<212> PRT
<213> Homo sapiens

<400> 288
Met Ser Leu Trp Leu Cys Phe Gln Cys Pro Leu Gly Val Ser Lys Ser
1               5                   10                  15
Asn Lys Lys Arg Ile Asn Leu Cys Ala Ser Thr Pro Glu Thr Trp Thr
            20                  25                  30
Lys Phe Thr Gln Asp Asn Leu Cys Arg Ser Gln Arg Glu His Leu Asp
        35                  40                  45
Ser Ala Asn Leu Trp Val Leu Val Asp Cys Ile Phe Arg Asp Thr Ser
    50                  55                  60
Glu Asp Leu Gly Leu Gln Cys Asp Ala Val Asn Leu Ala Phe Gly Arg
65                  70                  75                  80
Arg Cys Glu Glu Leu Glu Asp Thr Arg His Lys Leu Gln His His Leu
                85                  90                  95
His Lys Met Leu Arg Glu Ile Thr Asp Gln Lys Gln Arg Gly Gly Thr
            100                 105                 110
Glu Gly Gly His Gln Gly Gln Gly Gly Thr Ser Ala His Ser Pro Asp
        115                 120                 125
Pro Ser Val Pro Ala Leu Ala Leu Ala Gln His Gly Ala Val Trp Gln
    130                 135                 140
Cys Ser Pro Val Gln Val Leu Pro Gly Pro Leu Arg Gln Ser His Leu
145                 150                 155                 160
Pro Pro Ser Thr Ser Ser Leu
                165

<210> 289
<211> 212
<212> PRT
<213> Homo sapiens

<400> 289
Met Pro Ile Leu Gln Leu Leu Thr Thr Val Asp Asp Gly Ile Gln Ala
1               5                   10                  15
Ile Val His Cys Pro Asp Thr Gly Lys Asp Ile Trp Asn Leu Leu Phe
            20                  25                  30
Asp Leu Val Cys His Glu Phe Cys Gln Ser Asp Asp Pro Pro Ile Ile
        35                  40                  45
Leu Gln Glu Gln Lys Thr Val Leu Ala Ser Val Phe Ser Val Leu Ser
    50                  55                  60
Ala Ile Tyr Ala Ser Gln Thr Glu Gln Glu Tyr Leu Lys Ile Glu Lys
65                  70                  75                  80
Val Asp Leu Pro Leu Ile Asp Ser Leu Ile Arg Val Leu Gln Asn Met
                85                  90                  95
Glu Gln Cys Gln Lys Lys Pro Glu Asn Ser Ala Glu Ser Asn Thr Glu

            100                 105                 110
Glu Thr Lys Arg Thr Asp Leu Thr Gln Asp Asp Phe His Leu Lys Ile
        115                 120                 125
Leu Lys Asp Ile Leu Cys Glu Phe Leu Ser Asn Ile Phe Gln Ala Leu
    130                 135                 140
Thr Lys Glu Thr Val Ala Gln Gly Val Lys Glu Gly Gln Leu Ser Lys
145                 150                 155                 160
Gln Lys Cys Ser Ser Ala Phe Gln Asn Leu Leu Pro Phe Tyr Ser Pro
                165                 170                 175
Val Val Glu Asp Phe Ile Lys Ile Leu Arg Glu Val Asp Lys Ala Leu
            180                 185                 190
Ala Asp Asp Leu Glu Lys Asn Phe Pro Ser Leu Lys Glu Pro Tyr Ala
        195                 200                 205
Lys Ile Gln Met
    210

<210> 290
<211> 124
<212> PRT
<213> Homo sapiens

<400> 290
Met Gln Gly Lys Gln Pro Leu Leu Gly Gln Arg Thr Ala Val Cys Pro
  1               5                  10                  15
Leu Trp Ser Pro Cys Leu Ala Arg Gly Ser Val Thr Pro Ala Met Arg
             20                  25                  30
Thr Leu Ala Ile Leu Ala Ala Ile Leu Leu Val Ala Leu Gln Ala Gln
         35                  40                  45
Ala Glu Pro Leu Gln Ala Arg Ala Asp Glu Val Ala Ala Ala Pro Glu
     50                  55                  60
Gln Ile Ala Ala Asp Ile Pro Glu Val Val Val Ser Leu Ala Trp Asp
 65                  70                  75                  80
Glu Ser Leu Ala Pro Lys His Pro Gly Ser Arg Lys Asn Met Asp Cys
                 85                  90                  95
Tyr Cys Arg Ile Pro Ala Cys Ile Ala Gly Glu Arg Arg Tyr Gly Thr
            100                 105                 110
Cys Ile Tyr Gln Gly Arg Leu Trp Ala Phe Cys Cys
        115                 120

<210> 291
<211> 524
<212> PRT
<213> Homo sapiens

<400> 291

<210> 292
<211> 945
<212> PRT
<213> Homo sapiens

<400> 292
Met Gly Ala Arg Ser Gly Ala Arg Gly Ala Leu Leu Leu Ala Leu Leu
1               5                   10                  15
Leu Cys Trp Asp Pro Arg Leu Ser Gln Ala Gly Thr Asp Ser Gly Ser
            20                  25                  30
Glu Val Leu Pro Asp Ser Phe Pro Ser Ala Pro Ala Glu Pro Leu Pro
        35                  40                  45
Tyr Phe Leu Gln Glu Pro Gln Asp Ala Tyr Ile Val Lys Asn Lys Pro
    50                  55                  60
Val Glu Leu Arg Cys Arg Ala Phe Pro Ala Thr Gln Ile Tyr Phe Lys
65                  70                  75                  80
Cys Asn Gly Glu Trp Val Ser Gln Asn Asp His Val Thr Gln Glu Gly
                85                  90                  95
Leu Asp Glu Ala Thr Gly Leu Arg Val Arg Glu Val Gln Ile Glu Val
            100                 105                 110
Ser Arg Gln Gln Val Glu Glu Leu Phe Gly Leu Glu Asp Tyr Trp Cys
        115                 120                 125
Gln Cys Val Ala Trp Ser Ser Ala Gly Thr Thr Lys Ser Arg Arg Ala
    130                 135                 140
Tyr Val Arg Ile Ala Tyr Leu Arg Lys Asn Phe Asp Gln Glu Pro Leu
145                 150                 155                 160
Gly Lys Glu Val Pro Leu Asp His Glu Val Leu Leu Gln Cys Arg Pro
                165                 170                 175
Pro Glu Gly Val Pro Val Ala Glu Val Glu Trp Leu Lys Asn Glu Asp
            180                 185                 190
Val Ile Asp Pro Thr Gln Asp Thr Asn Phe Leu Leu Thr Ile Asp His
        195                 200                 205
Asn Leu Ile Ile Arg Gln Ala Arg Leu Ser Asp Thr Ala Asn Tyr Thr
    210                 215                 220
Cys Val Ala Lys Asn Ile Val Ala Lys Arg Arg Ser Thr Thr Ala Thr
225                 230                 235                 240
Val Ile Val Tyr Val Asn Gly Gly Trp Ser Ser Trp Ala Glu Trp Ser
                245                 250                 255
Pro Cys Ser Asn Arg Cys Gly Arg Gly Trp Gln Lys Arg Thr Arg Thr
            260                 265                 270
Cys Thr Asn Pro Ala Pro Leu Asn Gly Gly Ala Phe Cys Glu Gly Gln
        275                 280                 285
Ala Phe Gln Lys Thr Ala Cys Thr Thr Ile Cys Pro Val Asp Gly Ala
    290                 295                 300
Trp Thr Glu Trp Ser Lys Trp Ser Ala Cys Ser Thr Glu Cys Ala His
305                 310                 315                 320
Trp Arg Ser Arg Glu Cys Met Ala Pro Pro Pro Gln Asn Gly Gly Arg
                325                 330                 335
Asp Cys Ser Gly Thr Leu Leu Asp Ser Lys Asn Cys Thr Asp Gly Leu
            340                 345                 350
Cys Met Gln Asn Lys Lys Thr Leu Ser Asp Pro Asn Ser Gln Val Leu
        355                 360                 365
Glu Ala Ser Gly Asp Ala Ala Leu Tyr Ala Gly Leu Val Val Ala Ile
    370                 375                 380
Phe Val Val Val Ala Ile Leu Met Ala Val Gly Val Val Val Tyr Arg
385                 390                 395                 400
Arg Asn Cys Arg Asp Phe Asp Thr Asp Ile Thr Asp Ser Ser Ala Ala
                405                 410                 415
Leu Thr Gly Gly Phe His Pro Val Asn Phe Lys Thr Ala Arg Pro Ser

            420                 425                 430
Asn Pro Gln Leu Leu His Pro Ser Val Pro Pro Asp Leu Thr Ala Ser
        435                 440                 445
Ala Gly Ile Tyr Arg Gly Pro Val Tyr Ala Leu Gln Asp Ser Thr Asp
    450                 455                 460
Lys Ile Pro Met Thr Asn Ser Pro Leu Leu Asp Pro Leu Pro Ser Leu
465                 470                 475                 480
Lys Val Lys Val Tyr Ser Ser Ser Thr Thr Gly Ser Gly Pro Gly Leu
                485                 490                 495
Ala Asp Gly Ala Asp Leu Leu Gly Val Leu Pro Pro Gly Thr Tyr Pro
            500                 505                 510
Ser Asp Phe Ala Arg Asp Thr His Phe Leu His Leu Arg Ser Ala Ser
        515                 520                 525
Leu Gly Ser Gln Gln Leu Leu Gly Leu Pro Arg Asp Pro Gly Ser Ser
    530                 535                 540
Val Ser Gly Thr Phe Gly Cys Leu Gly Gly Arg Leu Ser Ile Pro Gly
545                 550                 555                 560
Thr Gly Val Ser Leu Leu Val Pro Asn Gly Ala Ile Pro Gln Gly Lys
                565                 570                 575
Phe Tyr Glu Met Tyr Leu Leu Ile Asn Lys Ala Glu Ser Thr Leu Pro
            580                 585                 590
Leu Ser Glu Gly Thr Gln Thr Val Leu Ser Pro Ser Val Thr Cys Gly
        595                 600                 605
Pro Thr Gly Leu Leu Leu Cys Arg Pro Val Ile Leu Thr Met Pro His
    610                 615                 620
Cys Ala Glu Val Ser Ala Arg Asp Trp Ile Phe Gln Leu Lys Thr Gln
625                 630                 635                 640
Ala His Gln Gly His Trp Glu Glu Val Val Thr Leu Asp Glu Glu Thr
                645                 650                 655
Leu Asn Thr Pro Cys Tyr Cys Gln Leu Glu Pro Arg Ala Cys His Ile
            660                 665                 670
Leu Leu Asp Gln Leu Gly Thr Tyr Val Phe Thr Gly Glu Ser Tyr Ser
        675                 680                 685
Arg Ser Ala Val Lys Arg Leu Gln Leu Ala Val Phe Ala Pro Ala Leu
    690                 695                 700
Cys Thr Ser Leu Glu Tyr Ser Leu Arg Val Tyr Cys Leu Glu Asp Thr
705                 710                 715                 720
Pro Val Ala Leu Lys Glu Val Leu Glu Leu Glu Arg Thr Leu Gly Gly
                725                 730                 735
Tyr Leu Val Glu Glu Pro Lys Pro Leu Met Phe Lys Asp Ser Tyr His
            740                 745                 750
Asn Leu Arg Leu Ser Leu His Asp Leu Pro His Ala His Trp Arg Ser
        755                 760                 765
Lys Leu Leu Ala Lys Tyr Gln Glu Ile Pro Phe Tyr His Ile Trp Ser
    770                 775                 780
Gly Ser Gln Lys Ala Leu His Cys Thr Phe Thr Leu Glu Arg His Ser
785                 790                 795                 800
Leu Ala Ser Thr Glu Leu Thr Cys Lys Ile Cys Val Arg Gln Val Glu
                805                 810                 815
Gly Glu Gly Gln Ile Phe Gln Leu His Thr Thr Leu Ala Glu Thr Pro
            820                 825                 830
Ala Gly Ser Leu Asp Thr Leu Cys Ser Ala Pro Gly Ser Thr Val Thr
        835                 840                 845
Thr Gln Leu Gly Pro Tyr Ala Phe Lys Ile Pro Leu Ser Ile Arg Gln
    850                 855                 860
Lys Ile Cys Asn Ser Leu Asp Ala Pro Asn Ser Arg Gly Asn Asp Trp
865                 870                 875                 880
Arg Met Leu Ala Gln Lys Leu Ser Met Asp Arg Tyr Leu Asn Tyr Phe
                885                 890                 895
Ala Thr Lys Ala Ser Pro Thr Gly Val Ile Leu Asp Leu Trp Glu Ala

900                 905                 910
Leu Gln Gln Asp Asp Gly Asp Leu Asn Ser Leu Ala Ser Ala Leu Glu
        915                 920                 925
Glu Met Gly Lys Ser Glu Met Leu Val Ala Val Ala Thr Asp Gly Asp
    930                 935                 940
Cys
945

<210> 293
<211> 712
<212> DNA
<213> Homo sapiens

<400> 293
gcaccaagac ccagtgcttc cctgacctct ggtccctcta aatcagcatc tatcacagta      60
gatcaacctg gataaagcca ggattcacag cagaagtggc acctcagaca ccaaagcagc     120
tttccttctc aaagctttta aacatcattg ggaaaactta gagcaaatca aaccaatgtt     180
tttcaataca tggaaggaaa gatgtgtgtg cttggttttg ttttttgctg agacccaaac     240
attgttagtg caagcccccg ccaggggtgc aggtggagtc aaagctgaat gaagagctcg     300
tcttcaatgt agctctccaa gctttcagag caagacaacc atgggccatc ctcttgttca     360
gactggcctg tcttcttctt gaccaatgtc actttgctcc tcactgctgt cctcttgtag     420
atcctggaat ccatttccca tctgttatat gttgccatta ggcacaggcg gtcactctgt     480
gaatgtggtc ctgcttaggt atttcttttg gcaactcctc tctctcacgt gctccctcct     540
gttcccctac tgcagcatct tcctcctcct cctcctcctc ctcctcttcc tcttctgaat     600
ccaccaaaac catgacatca tccatgtttt gtatagttag aataagagcg catattttta     660
aagctatgaa ttcctttgcc atagcgttga ctatataata aatacgttgt cc             712

<210> 294
<211> 451
<212> DNA
<213> Homo sapiens

<220>
<221> misc_feature
<222> (1)...(451)
<223> n = a,t,c or g

<400> 294
ggctcgggtg gttacgcacc ctggaaggga ataaacatta ggaccataga ggacgctcta      60
ggactaatgc tcatcagaaa atgactagga gtgctggcat ccctatgttc ttttttcaga     120
tgggaaacat tccccccaag gcaaaaacgc ccctaagatg tattctggag aattgggacc     180
aatttgaccc tcagatgcta agaaagaaac tacttatatt cttctgcagt actgcctggc     240
cacaatatcc tcttcaaggg ggagaaacct ggcctcctaa gagaagtata aattataaca     300
gcatcttaca gctagacctc ttttgtagaa aagaaggcaa atggagtgaa gtgccatatg     360
tgcaaacttt cttttcatta agagaacact ggcaattata taanaagtgt gatttatgcc     420
ctacaggaag ccctcagagt ctacctccct a                                    451

<210> 295
<211> 1389
<212> DNA
<213> Homo sapiens

<400> 295
tttttttttt tgggtgtggt gatgtattta ttcataatat attttcagaa cacattaata 60
atggagaata acacttattc atatactgaa tataactttt cctggagcac ctctaagagc 120
ttgtttggag ttggagaata ctgccagggc ttttcctaat ctctttggtc tttggaagtg 180
ggccaggggt ttctcaaacc aagtgtcttc catgggccat tggcaaaggc ttcccttcat 240
ccagcttgga ggggcagaaa gaccatggct tcagcacttc cattttggaa agaagtaaca 300
aaaaaagtga attaatgagc aatcggaaag actcaaagca ttttgtactc cacagttcat 360
ttcttcacac aaacgtccat tactgcagcg ggcatgaaaa ccaggcaggg gtgttaggct 420
catggcctga agagaagtca catcaccagc cgatgttttc atgcaaaagg caatcgtgat 480
gattcagaac ctggttctga aatttctcca ggtgtgctcg tgagctgaaa ggtccatgcc 540
cattctgtgc atcctgtgtc tttctactgg taaacttgga tgtccatttt agagtatctt 600
ggcaaaatgg tgagaggatc tcactttccc aatatcactc tgtaatgaag ctgcttgggg 660
gatcctccat ttgattgtgg agagaccaag gcaccgtggg ctgaagcaac gaatattaac 720
aacactaaca acaataagga taaagtctaa gtctcttcca aaaaaggcat atccaaaaat 780
aagggtaaag agctggtctt atttcatcca agtgattctg tgcactggaa attgtccttc 840
cgttcctccc tttcagttaa aagtgtttat atttctgagt cggatgatcc atttaaatac 900
ataccaatat tagcaccaag gcagcatatt tttttcccca gagatcacat ggaaactgaa 960
tacttattta acatagttaa tagttggtac aatctgtgat atcactaaca tacttattgg 1020
ctgatacttg cctagaaatg ccaaaaatat cttttataca actcttatag caccttgatg 1080
atggctagat tgttttaaaa aaattcattt taaaaaagta acaataaaac atttacagat 1140
gaaaagtaaa caatagtgta ccgtgataat gaaggatcac tttgtcatat tactacatgc 1200
aaaggcagtc ataatagaat cattttacgt acaaaaatat tacatcacaa taaataattt 1260
caaacataaa tattaaactt tacatcatta ggatagtcta catatctaca tacacacata 1320
tatgtgtata tatgtatgtg tagatatgta tatatgtata tgtacacaca tttacacaca 1380
caagcataa 1389

<210> 296
<211> 2536
<212> DNA
<213> Homo sapiens

<220>
<221> misc_feature
<222> (1)...(2536)
<223> n = a,t,c or g

<400> 296
tgtgtcagca gaatcattac gctttgctgg gcacgagggc agggcagagt gagattgcgt 60
ccatgctgta ttcccgcatg ttcatcaagt gctcgtttgc cccaatggca gatgacgaga 120
gccctacatc atcctcggca gaagagtaac tgtgagggag gcggggacca gccagaccgg 180
gagcaaaccg tcccttgtcc ccgtctcctc cctgttcccg ttcctccctg gcccacccca 240
ctcacactcc ccaaggccca cgggtcaaag gcaagcgagc tctccctctg cttccctggg 300
ggagccccga cggccacagg actccagctc caagtgggtt ttcttggctc ccctgttcaa 360
agtggccaca gcgcagaccg aagcaaaatt cttgtataca ttggcgccag ggctgatgct 420
ggggtggggg gtttatgaag aacattgaga acaatcagct ggtaattatg gatggaggaa 480
gagggagagg aaaaaaatat tgtatttttg aatcattgtt gcaggagggg gtgggaatct 540
taggatttgt tgccagattt gaaagtcact ggaacttgca tattttccat tttaatccta 600
agtgttatta cgccacccag ttggggttca ccctttcatc ccctcacatt taattgtctg 660
atatagaaat agtgttgtgt ccactgcccc gctagacggc tttcttaggg gaattttctt 720
ctggttgttt cacaagacag attctgtcct tgtcaacccg ggacagaaaa cttcagtctt 780
tttcacccgc attcaggatg gaaggggact cagggacagg ctctgtggac ttacagggga 840
cccaatcatt tcacaatgag gaaatttacc ggccaggcgt ggttgactca cgtctgtaat 900
cccagcactt tgggaggcca aggcaagagc ttgagcttga gcctagacgt taaagaccag 960
cctgggcaac acgagcaaga cccatctcta caagaaattt aaaaactagc caggcgtggt 1020
ggtgcgcgcc tgtaagtccc agctacttgg gaggctgagn ccctggaagg tcgaggctac 1080
agtgagctat gatcacacca ttgcacttca gcctgggcga cacagcgaga ccctgtctca 1140

agaaagaaaa aaaaaagaga caaattaccc agaaacccct cccttcccca catggaggcc 1200
ttggcaaatg ttaattttcc tagaaaatcc ttcagacctg gaagacgccg ggaaaagaat 1260
ctggctctcc aggggtggct tctgcgtccc cgccgccagg ccccaaacta tggacacagg 1320
gccgtcctgt tcctccccgg gactccagaa tttctctcct caaaggaaag aaaacagggc 1380
atgcgcttgt tgacaaaacg cagggccggc tcccaaaaac cccatgtgtg tacgattaaa 1440
aagttgggcc gtccccaggg cttccagcgc aaactttaaa gagacagggc tttgctgaaa 1500
accaaacatg ggccagctgg gctttttaac aacctagaga ctttccggag ctgcctggga 1560
acagaaccct gcgggaaaac ggggcttgcc agaggacact tcacagtttc ctttcatggg 1620
cctgtttttg gttcccctta aggaatcttc cacatcattg tctttcttgt gccttttcct 1680
tggtgagcaa cagaaagaga agggttccaa gcctctaaaa atgtgctttg tgatcaggag 1740
tgcgctccaa accaaatacg cgcgctgccc tttcgaggcc agtggagctc agcctccaag 1800
gctttaaagc cacatttcag caagagaaag cgctgagagc tcgcaggttc attaaagaag 1860
gcaaagcact aggtttctct ccttaggaaa agtaggtttc ttgggttgat gtagaatggg 1920
tttgctttgg atttttcagt gaaaggggaa tgttacgtaa aaacaaaata gggcttggct 1980
ggtcaaagga gacaagcagg atggatggat ggatggatgg atggatggat ggatggatgg 2040
atgaatagat agatggtgtt tgccatgtta aattgccaga gaaaacaaaa cccaaagctt 2100
gattggaaaa ccaatttaat tgtggggtgt cctgaggggg gaaggtcgca agctttgggc 2160
aagcctttga ggaagcggta caaagagttc ctgtgccctg tgtgtccagc ccctggaagc 2220
cagccagtgc atttatttta aactctttaa aaaccactcc cttggcccat ggaatgcgtg 2280
accccgtgag atgggtccac gcatctctct acacttcctt ctctccgtgg gatactggac 2340
tcgtgcctct gcgcccattc tctttctcac gcatatccga tgagctttaa tttcactttc 2400
tgatcacggt acgtccataa aagccagtat tacactttaa atgaagtatt tctttttttg 2460
taatcgtttt tttttaggaa ggtaaacaaa ttttaataaa gctaccgaat aatgttgaaa 2520
aaaaaaaaaa aaaaaa 2536

<210> 297
<211> 685
<212> DNA
<213> Homo sapiens

<220>
<221> misc_feature
<222> (1)...(685)
<223> n = a,t,c or g

<400> 297
aggcctagaa ttcggcacga gggcacagac tcagagagag cggaccatgg tgctcgtctc 60
ctgccgacaa gaccaaagcc caacgccccc cgcggctaaa gctcggcgcc acgcctggcg 120
agtatggtgg cgagcccctg gagaggatgt tcctgtcctt ccccaaccaa ccaagaccta 180
cttcccgaaa cttcgaacct taagccaaag ntctgcccca aacggctctg ccccaggttt 240
aagggccacg gccaagaaag gtggccgaac gcgcttgaac caaaagccgt ggcggcaacg 300
tggaacgaac atgccccaaa cggcgctgtc cggccctgaa gcgaccttgc acggcggcac 360
aagctttcgg gtgggacccg gttcaatttt caaaggttcc taaaggccat tgccttgctt 420
gggttgaccc tggggccgtc caccttcccc ggctggagtt tcaacccctg tcggtggcac 480
gccttccctt gggacaagtt tcctgggttt cttgttgaag caccgtgctt gaacctccaa 540
attaccgtta aagctggagc ctcgggtggg ccatgcttct ttgccccttg gggcttcccc 600
ccagggccct ccttcccttt cctggaaccc gtaaccccgt ggtctttgaa taaagtctga 660
gtgggcggca aaaaaaaaaa aaaaa 685

<210> 298
<211> 452
<212> DNA
<213> Homo sapiens

<400> 298
tagtccagcg cggaggaatt cgcgggcgct gctgggcctc gggctgctgg ttgcgggctc 60
gcgcctgccg cggatcaaaa gccagaccat cgcctgtcgc tcgggaccca cctggtgggg 120
accgcagcgg ctgaactcgg gtggccgctg ggactcagag gtcatggcga gcacggtggt 180
gaagtacctg aggcatatcc ccccacgtcc atgtccagga gcccccctac tgtcctggtc 240
atctgtggcc cggggaataa tggaggagat ggtctggtct gtgctcgaca cctcatactc 300
tttggctacg agccaaccat ctattacccc agaaggccta acaagcccct cttcactgca 360
ttggtgaccc agtgtcagaa aatggacatc cctttccttg gggaaatgcc cgcacagccc 420
atgacgattg atgaactgta tgagctggtg gg 452

<210> 299
<211> 1936
<212> DNA
<213> Homo sapiens

<220>
<221> misc_feature
<222> (1)...(1936)
<223> n = a,t,c or g

<400> 299
tttttttttt tttttaaaag gatttaaaaa ttactctttt actttttatt acaatagaat 60
aagtgatcaa ttaatagaat taaacaactt ttcaattaaa acctactgca ttacttttgg 120
ggtttcacag cagcaggaaa caaacataaa tccagttgaa agggcaaggc tttccagaat 180
ccagtgccaa gaaacagtct ggtcttgatt attcgggact agcaatgggg aaatactgga 240
tacaggataa tgccaaaaac caatggaatg catcgggcat actctccttt gtaacatcac 300
attatctgaa cacttttaaa atattccagg ctaaggtaat ttaaggccaa ggcccatgga 360
ggctctggtt tctggcaaca ggttaggagt tctcccccag gcaagcccgg cagcacaatt 420
gctcccagag ccaccctctg gcccagaaaa cggggcccca tcatcatggc agaccttaga 480
agcctccacc ctggtgggaa gcagccatct ttctcaaagc tctttatggc tcacacaacc 540
catggttaaa ggaaaatggg aaagtgacat atgtaaaggg cctttctcga ggactgagat 600
gaactggaag acagacagac tgtggcctcc ccagatcagg aaagaggcct ccagaagtaa 660
ggggcccaca tgccagatga actccccaca tgaaagaaac tatcctgata aaggctgacc 720
cattttgctg ataatcaagc agactgcatg tatatatgaa tatatccata ccataccata 780
tggtaggtga aatatatggt gtacatattt aaaatgggca tacacaccac atcacnacca 840
caacccagat aaaataaatg gtacatacat tgctactatg aacactgcac gtcccagaca 900
gaccaatgac taatatgttc aattaaacta cttgaattaa actactcttc ctctccccaa 960
aatattctgc ttgcagcaaa catattccag ttggctcaag cggtgttgtt tcctaggttc 1020
cctgagaagg aatggaatcg tccctcttgt ttccagaaca cctaacagaa agcccaactg 1080
ccaacaggac ccaagcctaa aacaaattcc atatttgaat ttgggcccat gaattccctc 1140
ccacctattc cttagtccac agggcatgaa accttactca gaaagatatc aatttctttt 1200
tcctacaact ggctacagta tttactgtat tatgttagat ggaggggact gggggaaggc 1260
gggcagcagg cagtttacat gttggcaatc attcatttgc tttccctttt accaaaagaa 1320
aatgctactt taaaaaaaaa aaacacccag aaatttagag ttcaataatg aattttgctt 1380
tcttggcaca tttttgaaat gatcatatca ttacatgtag ctaggtcttt tatcctcctg 1440
gaatgctcct cctagggagg caaggtatct aaacaacaaa ataaccctac aaaaaaagtt 1500
aatattgtga gcagtctcaa tttgtagtta ttaaactcaa agtctaatgc tgaaaggcaa 1560
cagtaattta aacagatttg tttattttta ttattatttt ttgagatgga gtcttgctct 1620
gtcactctgg ctggagtgca gtggcacaat ctctgctcac tgcaatctcc atctcccagg 1680
ttcaagtgat tctcctgcct cagcctcccc agtaactggg attacaggca catgccacca 1740
tgcccagcta atttttttgt atttttatta catgacgggg tttcgccatg ttgctcaggt 1800
tggtgtcgaa ctcctgggct caagcaatcc acctgccttg gcttcccaaa gtgctgggat 1860
tacaggcacc tgccccagat ttataatgct attcacagat cttgcagaat tataacaggc 1920
tccttctaca acttgg 1936

<210> 300
<211> 1413
<212> DNA
<213> Homo sapiens

<400> 300
tgctctctgt gagccgcaac ctttccaagg gagtggttgt gtgatcgcca tcttagggag 60
aaagatgttc tcgtccgtgg cgcactctgg cgcgggcgaa ccccttcaac acgccacatc 120
ctgcagctgg tgcacgatgg tctcggggac ctccgcagca gctccccagg gcccacgggc 180
aagccccgcc gcccctcgca accatggcag ccgccgccgt ggaagagcag tatagctgtg 240
actatggatc tggcagattc tttatccttt gtggacttgg aggaattatt agctgtggca 300
caacacatac aagcattggt tcctctagat cctggttaaa tgcagaatga aggttggacc 360
cccaaaagta caaagggcat atttaacgga tttctcaagt tacacttaaa gaggatggtg 420
ttccgtggtt tggctaaagg attggctccg acttttcctt ggctactcca tgcagggact 480
ctgcaagttt ggcttttatg aagtctttaa aagtcttgta taggcatatg cttggagagg 540
agaatactta tctctggcgc acatcactat aatttggctg cctatgcccg tgctgaattc 600
tttgctgaca ttgccctggc ttcctatgga agctgctaag gttcgaattc aaacccagcc 660
caggttatgc caaacacttt gaggggatgc agctccccaa aatgtattaa ggaagaaggc 720
cttacaagca ttcttacaag aggggtgctc cctctctgga tgagacagat accatacacc 780
atgaattgaa gttccccatg ccttgaacgt actgtttgaa gccactgtac aagtttgtgg 840
ttccctaagc ccccgcagtg aaatgttcaa aagccagagc agcttggttg taaccatttg 900
gtagcaggtt accatagctc gagtcttttg tgccaattgt ttctcacccc gtgctgattc 960
tgtggtatct ggtgttgaat aaaggaaaaa ggtagccagt gctttctctg gtccctcaaa 1020
gagacttggg atttaaaagg tgtaatggaa gggactgttt gcccgtatca tccatgattg 1080
gtacccctga ctgcacttac agtggtttat ctattgactc cgtgaaggtc tacttcagac 1140
cttcctcgcc ctcctccacc ccgagatgca agagtctttt gaagaagaag cttggggtta 1200
actccagtag ttagatcaaa gcaaattgtg gactgaatct gcttgttgat ccagtgtttg 1260
aagaaagtgc aaaaggaact tttatatatt tgacagtgta ggaaattgtc tattcctgat 1320
ataattactg tagtactctt gcttaaggca agaagtttca gatttactgt tgtaaataaa 1380
cccgaactct tcatgaaaaa aaaacaacaa aaa 1413

<210> 301
<211> 495
<212> DNA
<213> Homo sapiens

<400> 301
ggcacgaggt ggttttgtct gctgcgcccg ctcttcgcgc tctcggttca ttttctgcag 60
cgcgccggca ggatggccca caagcagatc tactactcgg acaagtactt cgacgaacac 120
tacgagtacc ggcatgttat gttacccaga gaactttcca aacaagtacc taaaactcat 180
ctgatgtctg aagaggagtg gaggagactt ggtgtccaac agagtctagg ctgggttcat 240
tacatgattc atgagccagg taagctatgc tatgttatag taaagcagta attgttgaaa 300
tattggtggt tatggttgtc aaaattttaa aaatatcatt gacatcaaat gatatctgaa 360
agtaactgtt ctgataagtt ttttttttta atgactaatt tcttgactct ggagagagtt 420
ccaattttat ggtaaataaa ataccacagc agtgaaagta acacattatt ctacttggaa 480
aataatgtaa gaagt 495

<210> 302
<211> 2430
<212> DNA
<213> Homo sapiens

<400> 302

ggcggtggag agggaaattc aagcggccgc gtggtgcgga ggacaaaagc gggcggcggg 60
ccgatgtgtc tgacggcgct ccctctctgc tttttgttct cgcctcgccg ccccgcccct 120
ccgcagctct cgaggctcct cccgctgcgg gtcggcgctc gccctcgctc tcctcgccct 180
ccgccccggc cccggccccg gccccgcgcc cgccatggag aagactgagc tgatccagaa 240
gcgccaagct ggccgatgcg aggcgtagcg tctacgacga catggccacc tggcatgaag 300
gcagtgaccg agcagggcgc ccgagctgtc caacgaggag cgcaacctgc tctccggtgg 360
cctacaagaa cgtggtcggg ggccgcaagt cccgccttgg aagggtcatt ctctagcatc 420
gagcagaaag accgacacct ccgacaagaa gttgcagctg attaaggact atcgggagaa 480
agtggagtcc gagctgagat tccatcttgc accacggtgc tgggaattgt tggataaatt 540
atttaatagc caatgcaaac taatccagag agtaagggtc ttctatcttg aaaatgaagg 600
ggtgatttac tttccgggta cctttgcctg gaagttgcct gttgttgatg atcgaaaaac 660
aaacgataga taattaccca aggagcttac caaggaggca ttttgatatt aaggcaagaa 720
aggaggttgc aacccacaca cccaattccg cctggggggt ttgctcttta actttttctg 780
tattttacta tgagattcct taataaccca gaagcttgcc tgcacgcctg gctaaaaacg 840
gctttttgat ggagggccat tgcctgactt gatacactga atgaaagact catacaaaga 900
cagcacccct catcatgcag ttgctaagag acaaccctac cactttggga catcagacag 960
tgcaggagaa gaatgtgatg cggcagaagg ggctgaaaac taaatccata cagggtgttc 1020
atccttcttt cctttcaagg aaaccttttt tacacatctt ccatttcctt atttccactt 1080
gggattttcc tattaggcca aaggaaaccc ctttcatgtg tttggggatt caactgttta 1140
tagtcttttt cacactgcag ctttggggaa aacttcattt ccttggattt gtgttttgtc 1200
ttggggcttt cctgggggtt gccagtactg ctgtagggaa aagtattaat agcttcaatt 1260
catattaaac ataagtaact ccaaaccact tatggaagag gactaaaaat gtatctggta 1320
tttaagtaat ctgaaccagt tctgcaagtg actgtgtttt gtattactgt gaaaataaga 1380
aaatgtagtt aattacaatt taaagagtat tccacataac ttcttaattt ctacattccc 1440
tccccttact tctacgggga tttcatttaa gatagcaacg tttcccatgc tctaaatgaa 1500
ttcctattct agtaggaatc cgaaaggatt tagatgaaat ggaaaggcac ttgcccatct 1560
ctgtctaggg gttcacaaat tgaaagggtc cttgtatccc aatcgggagt tccttgtgta 1620
acctgtggcc aacagggagg ttcccttatt cactctttat tttgctgctg tttaagttga 1680
caacccaccc ttctcaaata aaaaatacac tatacaccta cctgccatta gtagtttctg 1740
gtattccact ttactatgtg atagaagtag catgttgctg ccagaataca agcattgctt 1800
tgggccaaat taaagtgcat gtcatttctt taatacactc gtaaggggag attaaattaa 1860
ggtacacaag gtcccaaggt ctaaaacctt tagtactttt tccatgcaga tttgtgccac 1920
atgctgagag ggatgtccca gttttttcta gtgattgtta tttagagagg tggacccact 1980
atggtgtgtg gctaatccat tgactgtagt cccgaaaaaa agccttgtga aaatgttatg 2040
ccctatgtaa caggcaggag taacataaaa taaaagtaca ttttataaac ccatttacta 2100
tggctttggt aacaaattgc catacccata ttttaaggga caggtgaaat ttactacttt 2160
ctaaagttta ttgatacttc ccttttatgt aaaatgtagt agtgatacct atatttacca 2220
cattgtgcat tgtgacacac tttgtctagg gatgcctgga agtgtataaa attggactgc 2280
atttcttaga gtgttttact atagatcagt ctcatgggcc atctcttcct cagatgtaaa 2340
tgatatctgg ttaagtgtta tatggccata aagtggacat tttaaaacta gcaaagttaa 2400
aaaaaaaaaa aaaaaaaaaa ggcggccgct 2430

<210> 303
<211> 915
<212> DNA
<213> Homo sapiens

<400> 303
atggaaacca gtgtgattga aggtggatta aatatcactc tcaccatccg actacttatg 60
catggaaagg aagttggcag tatcatcgga aagaaaggag aatcagttaa gaagatgcgc 120
gaggagagtg gtgcacctat caacatctca gaagggaatt gtcctgagag aattatcact 180
tttgctggac ctgctaatgc cgtcttcaaa ggctttgcta tgatcattga caaactggaa 240
gaggacataa gcagctctat gaccagtagc acagctgcca gtagaccccc agtcaccctg 300
aggctggtgg tccctgctag tcagtgtggc tctctcattg gaaaaggtgg ttgcaagatc 360
aaggaaatac gagagagtac aggggctcag gccatcacta ttgctggcat cccacaatcc 420
atcattgagt gtgtcaaaca gatctgtgtg gtcatgttgg agtccccccc gaagggcgtg 480
accatcccgt accagctcaa gccttccagc tctccggtca tctttgcagg cggtcaggcc 540

tatacccttc aaggacagtt tgccattcca cagccagatt tgaccatgct gcaccagttg 600
gcaatgcaac agtctcattt tcccatgacg catggcaaca ctggattcag tgcaggtttg 660
gatacatctg ctcagactac ttctcatgaa cttaccattc caaacgattt gattggctgc 720
ataatcgggc gtcaaggcgc caaaataaat gagatccgtc agatgtctgg ggcacagatc 780
aaaattgcga acccagtgga aggatctact gataggcagg ttaccatcac tggatctgct 840
gccagcatta gcctggctca atatctaatc aatgtcaggc tttcctcgga gacgggtggc 900
atggggagca gctag 915

<210> 304
<211> 1105
<212> DNA
<213> Homo sapiens

<400> 304
ggcacgagca aagttgtcat gaatactgac ttgggcgtgg gacccatccg agatgtgctg 60
gaccacatct acagtgcgct gtatgtggag ctggtggtga agaatcccct gtgcccgctg 120
ggccaaactg tgcaaagtga gctctttcgc tcccgactgg actcctatgt tcgctctctg 180
cccttcttct ccgcccgggc tggctgaagc aacctacctc aagtctcagg agaattcatg 240
tctgcctggg gcccttccgg gaacccgagc ccaaggagtg ggggcggccc cggagcgagg 300
ctcacactcc ccgccctgca gaatgactgt ccacaacctg tacctgtttg accggaatgg 360
agtgtgtctg cactacagcg aatggcaccg cgaggaagca agcaggggat ctcccaagga 420
ggaggagtat aaggctgatg tacggggatg gctcttctct atccgctcgt ttgtcagcaa 480
gatgtccccg ctagacatga aggatgggct tcctgggcct tccaaactta gccgttacaa 540
actccattac tacggagacg cccactgggg atcaaagttt gtcatgaatt actgacttta 600
ggcgttggga cccatcccga gattgttgct gcaccaacat cttacaagtg cgcttgtatg 660
tgggagcttg gtggtgaaag aatccccctt ggtcccgctg gggcccaaaa ctgtgccaaa 720
gtgaagctct tttcggttcc cggacttgga ctccctatgt tcgcttctct gccctttctt 780
tctcccgccc agggctgggc ttgaagcaac cttacctcaa gtcttcaggg agaattcatg 840
gtctgcctgg ggggcccttc cgaaccttgt gccaactaaa ggggccccca ctgtaagccc 900
ccgtaccccc accccttggc ccagagcaac atcctaaaag gcctgccctg gatgctctcc 960
tcctaccaga gtgaagccca caggagtccc tccacctcca caccaaggcc cttctctcca 1020
agttttatcc cctgcctaaa tgctgcgagg aagcacagga ataaaccttt ttgtcactct 1080
caaaaaaaaa aaaaaaaaaa ttcct 1105

<210> 305
<211> 540
<212> DNA
<213> Homo sapiens

<400> 305
caagtgatcc agcaaggtcg gcagagcaag ggcgttacgc agaaggacct ggccacgaaa 60
atcaatgaga agccacaggt gatcgcggac tatgagagcg gacgggccat acccaataac 120
caggtgcttg gcaaaatcga gcgggccatt gatgtgggaa ccaggagtgc tcgtgtgctg 180
agagcccagt gaggcacggg agcggcgtgg ccccccttcc ctgggaagat gttgaagagg 240
ggccagcgtt tgggggagga gggagctggt gctgcagtgg ccagtggctg tggctcattt 300
caggcctcaa gctccgggga aaggacattg gaaagcccat cgagaatggg gcctagggcg 360
aaatgaacac aaagcctcga aatcagtgcg ctccagctga tctcgttccg ccggttcccc 420
ttggccgcca gttccgttca tctcacgggc cgaacggaac aaggggtcca gcttgcgggg 480
gaccctcccc agcccattcc tgctgtcaaa caaacaaaac cttgcaaagc gaaaaaaaaa 540

<210> 306
<211> 449

<212> DNA
<213> Homo sapiens

<400> 306
ttttcatgac ttttaatgct ttattgggat tgcaagcgtt acaaggttaa agacaaaacc 60
caagcatggg attttgccgg aaatattagc gttaaaggag ctgagttgag tcaaacacgg 120
gccgcaagtg gaccgaggcg gcaggcacag gtgacattca gtgtttggcg tgggggtctt 180
caggtgatgg cagaggaggg gacccaagag gccgagtccc tgccgcagcc ctaggcgccg 240
cggtggtggc tgctatggca tcaccagacc cgtgggctcc ggggcccaag caacctggtc 300
gatgggcaga cctggcagct ctcctgctct gtgggctcag acccgcctga cctccctact 360
ccccggcctc accccacctt gcccccaaga ggtcccacag agcttcggac ctcctggaga 420
caaggcaggg tgctgagggg cgggcaaga 449

<210> 307
<211> 417
<212> DNA
<213> Homo sapiens

<220>
<221> misc_feature
<222> (1)...(417)
<223> n = a,t,c or g

<400> 307
gcttcttcgc ttctctcttg gaaagtccag tctctcctcg gcttgcaatg gaccccaact 60
gctcctgcgc cgctggtgtc tcctgcacct gcgctggttc ctgcaagtgc aaagagtgca 120
aatgcacctc ctgcaagaag agtgagtgtg aggccatctc catggtctgg ggctgtggct 180
aaggctgctg ctcctgctgc cccgtgntgc tagcaagtgt gcccagggct gtgtttgcaa 240
aggggcgtca gagaagtgca gctgctgcga ctgatgccac ggacaacctt tctcccagac 300
tgtaaacaga gagacatgta caaacctgga tttttttttt ataccacctt gacccatttg 360
ctacattcct tttcctgtga aatatgtgag tgataattaa acactttaga cctgatt 417

<210> 308
<211> 892
<212> DNA
<213> Homo sapiens

<220>
<221> misc_feature
<222> (1)...(892)
<223> n = a,t,c or g

<400> 308
tttcgtgccg gtcggaggcg gtcggcggag gtgtctaccc cgccggtgat ggcgttgaac 60
gccactggct tcccggcctt ccgtccgctg cctccgtccg attctgcgtc tgcttgctga 120
ggaggcggat taggggggcg cggagtctct tcccttgagt gcataggtcc cggttggtag 180
agggtttgag tccgcatcgc cacagctgaa ggctgcgagg gactaagagc agaatattac 240
cttaggaatt gagtggacca tttgggaagg gcattttccg agcgctaaag ctcttgttga 300
aagattaaga gatcatgacg attgcagcag aatctcctga ttgagcaaac cacagctcct 360
caacaagccg aggtagaagc catgaaacag tatcatggaa gaaattcaag aacttaatga 420
agtcgcgaga catcggccac ggtccaccgt tagttattgg gaatccagca agaaaacaga 480
caaatcagag agttgcaaca agaaaacaaa gaattacgta catctctggg aagaacatca 540
gtccggcctt gggaacttat aatgagcaag taccgagaac aaatggttaa gattgctaat 600

ggctagcaaa aaaagatgat ccgggtataa taatgaagtt aaaaggagca gcactcccan 660
gattgacatt ggtacatcgt aacaagtccg aaggattctt ccttgatgca tctcgacaca 720
tccttgaagc acctcaacat ggactggaga gaaggcactt ggaagcaaat cagaatgtac 780
actaaataaa cagtcaactt ttggggtgtg gatggaaggg gggtccattt taaaagtgct 840
tttacattga atttccctcc cagattagat cagcaaataa atgaaattta tt 892

<210> 309
<211> 512
<212> DNA
<213> Homo sapiens

<400> 309
ggctctcgca gtcctctttc gtcagtgtct tttgcttcga ctcccggcgg agcgcgcaac 60
gtggagtgac gtgcaggggc caagtgcaac ccaggcagcc acggctgttt cggagctcag 120
gactctaaaa tggcagagca gctttctcca ggaaaggcgg tggatcaggt gtgcaccttc 180
cttttcaaaa agcctgggcg gaaaggggct gctggacgca gaaagcgccc ggcctgcgac 240
ccagagcccg gagaaagcgg cagcagtagc gacgaaggct gcactgtggt tcgaccggaa 300
aagaagcggg tgacccacaa tccaatgata cagaagaccc gtgacagtgg taaacagaag 360
gcggcttacg gcgacttgag cagcgaagag gaagaggaaa atgagcccga gagtctcggc 420
gtggtttata aatccacccg ttcggcgaaa cccgtgggac cagaggatat gggagcgaca 480
gctgaggggc aaggaggatg acaagatcta tc 512

<210> 310
<211> 488
<212> DNA
<213> Homo sapiens

<220>
<221> misc_feature
<222> (1)...(488)
<223> n = a,t,c or g

<400> 310
ggaggtgggn caggcagact catgtcagaa ggcccctcag tgagaagcga agaagcaatc 60
tgtctttatt atgaggaact aggaggtggg gccaggcaga ctcatgtcag aaggcccctc 120
agtgagtgca gcccaggaga ctggtcacat tctggggttg cggaagggcc ttgntgcatt 180
cagtttctcc acatcacttc tcatggggcc aaggaagctc tgtccacgtg gcttgggctc 240
ctgacttcag gtccagccac cacagccgcc gtcctgccat gatatgggag ccgagaagac 300
ccagggcaga taaaggccgc atccacagag tccagggnta atcccatgag gggtcccgac 360
tttccttctt ccagccgntt tcggataacc gtttactagg gtatagcctn ccttttttca 420
ggaagacatt ttacctgggg tgccttggga ccagatagan tttttttttn cccggggaaa 480
aggcagca 488

<210> 311
<211> 1776
<212> DNA
<213> Homo sapiens

<220>
<221> misc_feature
<222> (1)...(1776)
<223> n = a,t,c or g

<400> 311
gatttgctct gccagcagct gtcggtgccg cgctcgacac cgagtcctag ctaggcgctc 60
acagaatacg cgctccctcc ctcccccttc tctgtccccc gcctctcgct caccccggcc 120
cactccagcg gcgactttga gggattccct ctctggcggc ctctgcagca gcacagccgg 180
cctcattcgg ggcactgcga gtatggatct ccaaggaaga ggggtcccca gcatcgacag 240
acttcgagtt ctcctgatgt tgttccatac aatggctcaa atcatggcag aacaagaagt 300
ggaaaatctc tcaggccttt ccactaaccc tgaaaaagat atatttgtgg tgcgggaaaa 360
tgggacgacg tgtctcatgg cagagtttgc agccaaattt attgtacctt atgatgtgtg 420
ggccagcaac tacgtagatc tgatcacaga acaggccgat atcgcattga cccggggagc 480
tgaggtgaag ggccgctgtg gccacagcca gtcggagctg caagtgttct gggtggatcg 540
cgcatatgca ctcaaaatgc tctttgtaaa ggaaagccac aacatgtcca agggacctga 600
gggcgacttt ggagggntga gcaaaagtgc agtttgtcta cggattcctc gggagaaaac 660
ccactttcaa agacgcatca gttgctnggg gaagccaaca gccaacttgc accacctctc 720
tgccttggtc acccccgctg ggaagtccta tgagtgtcaa gctcaacaaa ccatttcact 780
ggcctctagt gatccgcaga agacggtcac catgatcctg tctgcggtcc acatccaacc 840
ttttgacatt atctcagatt ttgtcttcag tgaagagcat aaatgcccag tggatgagcg 900
ggagcaactg gaagaaacct tgcccctgat tttggggctc atcttgggcc tcgtcatcat 960
ggtaacactc gcgatttacc acgtccacca caaaatgact gccaaccagg tgcagatccc 1020
tcgggacaga tcccagtata agcacatggg ctagaggccg ttaggcaggc accccctatt 1080
cctgctcccc caactggatc aggtagaaca acaaaagcac ttttccatct tgtacacgag 1140
atacaccaac atagctacaa tcaaacaggc ctgggtatct gaggcttgct tggcttgtgt 1200
ccatgcttaa acccacggaa gggggagact ctttcggatt tgtagggtga aatggcaatt 1260
attctctcca tgctggggag gaggggagga gggtctcaga cagctttcgt gctcatggtg 1320
ggcttggctt tgaactctcc caaagaagcn aatcaaaggc cacttggagc tgtaatctgg 1380
ccccccaaaa gtttagggga ttgaaaaaca tgccatcttt tgaagggagg aaaccccttt 1440
aggttcagaa gaatatgggg tgctttgctc ccttggacac agctggctta tcctatacag 1500
ttgtcaatgc acacagaata caacctcatg ctccctgcag caagacccct gaaagtgatt 1560
catgcttctg gctggcattc tgcatgttta gtgattgtct tgggaatgtt tcactgctac 1620
ccgcatccag cgactgcagc accagaaaac gactaaatgt aactatgcag agttgtttgg 1680
acttcttcct gtgccaggtc caagtcgggg gacctgaaga atcaatctgt gtgagtctgt 1740
ttttcaaaat ggaattaaac cccactattc tctggc 1776

<210> 312
<211> 512
<212> DNA
<213> Homo sapiens

<400> 312
acaaagatca aactatactt gaaaataaac agcaatatga tattgagata acaagaataa 60
aaattgaatt ggaggaagcc ctagtcaatg tgaaaagctc ccagtttaag ttagaaactg 120
ctgaaaagga aaaccagata ttggggataa cattacgtca gcgtgatgct gaggtgactc 180
gactaaaaga attaaccaga ctttttaaaa ctgtggactt aaatgttctg caaagtctta 240
cacaaaagcc caatacagta catgaactac ggaaaagcag aactgctctg aacaaggaag 300
aggtgggaga tctgagtcct ggccccagca cccacttcaa cttcagtcac ctccttggaa 360
tctggggttc tgcagcttga aataatcagt ttggatttta aacttatttg taacataata 420
ataatcctag gaactttaaa ataatatttt gtactaaatt ttagaattat tataataagt 480
gatctaataa aacagtcttt tcctgaaaaa aa 512

<210> 313
<211> 560
<212> DNA
<213> Homo sapiens

<400> 313
tttttttttt tttccaataa aaaataattc aactttatta gtatgaaata ttttgagata 60
attagtgacc caaatgcatg attctccaat atgaaaggtg ttcagcataa gcatacaatc 120
atttagtaaa actgctcttt atgagacccc cagaaaagct ggaggcactt cctttttttg 180
gtggagagag aagacactac ttaactggcc atttccttgc tggagtttat tccgaattcc 240
cttttgtctg attcttcctc ctcaaactcg actaaaggag tgtgtctgtt ggcctgagca 300
ccttctctgt agaacacttt ctttactgtg ccatcctttg gagactttat ggtatgctcc 360
atcttcatgg cgatcataac catgagggaa tctcccgctt tcactttgtc tccagctttg 420
acaaacacct tttcaatggg ttccagtcat aggagctaag ggggccgccc tgagtttctt 480
gtgagctcac agaagataag tatttgggga ctggaatgtc aatctcaata cttccttcct 540
tggaaaatag gtaaatagtg 560

<210> 314
<211> 1590
<212> DNA
<213> Homo sapiens

<400> 314
atgcagctgc tgctggcaga gtgcatgggt cagagtgggc caccaggagc tgtctgccat 60
tgccagaggg tgtggcaggc cagagccgtc cgcagaagca aaaggccagt cccatcgacc 120
acccaagggc tgaagagtgt gggcgcatgg cgtgggagtg gcaggcagct ccacctgcag 180
ccccagtaca ggatccactg ggtgaagcca gctgggctcc tgagtctggt ggggaccatg 240
gagaacattt gtgtctggcc cagcgattgt aaatacacca atcggcactc tgtatctagc 300
tcaagattgc tggactcgct gaagagagac tacgcaggaa agccccagcc acccatcaaa 360
tcagagagaa ggaatccacc ttcttacgct atggcagctg cgcagctccg tgactcagag 420
gagaccgggg gatcagagtt tgtttttgcg gaaaagactt taaggaagtg tgtcaaatgc 480
ccacaggtag agctggaaaa tgtggccttt gctaaagatg cagaagagtc tagagacgca 540
caacggctgg gtcattggtg gccctgcatc atggaaactc tttctaatgc aagtggtact 600
tttgccatac gccttttaaa gatactgtgt caagataacc cttcgcacaa cgtgttctgt 660
tctcctgtga gcatctcctc tgccctggcc atggttctcc taggggcaaa gggaaacacc 720
gcaacccaga tggcccaggc actgtcttta aacacagagg aagacattca tcgggctttc 780
cagtcgcttc tcactgaagt gaacaaggct ggcacacagt acctgctgag aacggccaac 840
aggctctttg gagagaaaac ttgtcagttc ctctcaacgt ttaaggaatc ctgtcttcaa 900
ttctaccatg ctgagctgaa ggagctttcc tttatcagag ctgcagaaga gtccaggaaa 960
cacatcaaca cctgggtctc aaaaaagacc gaaggccagc gacggctgct cggtgacggc 1020
gctgctctgc caccaagaaa aaccccaaac ccactgatcc cacaaagagg ggagaaaaag 1080
gaaggcttca tgctgaagaa ggagaaatac aaacgtacag aagaacttaa ggtcaagttt 1140
tacagagata accaaggaca tcttaaagga gacaggctgt gcgatcattg gaagagggaa 1200
gctgtggatc ttgcattcat gcatttggat gaagatgaca ctggaaactg cacgttgcag 1260
gttgaggtgg ccaagtatca acggaatggg aaatatgagg cttcaggaag gaagtgcgcg 1320
aaccacagga aggctccgtc tctacggcag aagcggccac gccgcagccc atccaaacgc 1380
cgcgacacga gtgagttgtc ctcatcgaac acatttcatc ctgtggattt tgaggatggc 1440
cagagaagac cttcaaggag agtcaagttt ggaccaactc ggaggctcat tgtctttgac 1500
agacacccag ctggtgaacc tgtgtcctgg aggaatgcag gagcagctgc tcattgtatt 1560
caaacctttg atggaaggtg gtttgaatga 1590

<210> 315
<211> 546
<212> DNA
<213> Homo sapiens

<220>
<221> misc_feature
<222> (1)...(546)

<223> n = a,t,c or g

<400> 315
tctggttgtc atggatgcac tggtagaaga tgatatctgt attctgaatc atgaaanagc 60
ccataagaga gatacagtga ctccagtttc aatatattca ggagatgaat ctgttgcttc 120
ccattttgct cttgtcactg catatgaaga catcaaaaaa cgacttaagg attcagagaa 180
agagaactct ttgttaaaga agagaataag atttttggaa gaaaaggtaa tttttatctt 240
ttaccttcat tgcgtttgtg acataaggaa taattcaaaa tacttgaaat tttgaatact 300
ttttgtcctg tcttccatga ttgtactaaa tttccaagaa taactcactg ctaatctgaa 360
aactctgggc attatactta tcctgtgttt cacataccat gtacttggta aatatttgta 420
ggagaggatt taacaacttg gagttatatt ccaaatattt ttctttcatt ttcaggttta 480
cttacctcca tagctgtatg tattttattc agaattttca aatctgggca aaaaaaaaaa 540
aaaaaa 546

<210> 316
<211> 808
<212> DNA
<213> Homo sapiens

<220>
<221> misc_feature
<222> (1)...(808)
<223> n = a,t,c or g

<400> 316
ggcacgagag agcagttaag gcacacagag caccagctcc ctcctgcgct gaagatgttc 60
caccaaattt gggcagctct gctctacttc tatggtatta tccttaactc catctaccag 120
tgccctgagc acagttcaac tgacaactct ggggcgtgga tgggaaggag ttcccagagg 180
tccacttggg ccagtggtac tttatcgcag gggcagctcc cactcaagga ggagttggca 240
actttttgaa ccctgttgga caacattgtc tttcaaaata tgggctgctg gcttctgccc 300
ccgattgcag cttccaacct ttcggtgcta cccatccgca tgtgagtggg aaagatgggg 360
ctcttgtgtg cccccggaaa atgggatctt acccacctgg actgaagggg gaggcacaga 420
tcttcaagaa acttgaaggg gccgcccttg acatggaaga ctgaagctct ttttccagct 480
tcatggcccc agggtgggaa tcatgctgaa atgagaacag gcccagggtt taccagcgct 540
ttctcctcta caatcgctca cccacatcct cccgaaaagt gtgtggagga attcaagtcc 600
ctgacttcct gccctggact tccaaagcct tcttagttga ctcctaggga atcaagaggc 660
ctgtgagctg tccaataact gacctgtaac ttcatctaag ttccccagat ggggnacaat 720
gggagctgag ttgttggagg gaggaagctt ggagacttcc agctccagct cccactcaaa 780
gataataaag ataattttc aaatcctc 808

<210> 317
<211> 406
<212> DNA
<213> Homo sapiens

<400> 317
tttcgtcgat ggcctcggcg ctgagctatg tctccaagtt caagtccttc gtgatcttgt 60
tcgtcacccc gctcctgctg ctgccactcg tcattctgat gcccgccaag attctggact 120
ccaggcaggt gtgtgtccag tacatgaagg acaccaacat gctgttcctg ggcggcctca 180
tcgtggccgt ggctgtggag cgctggaacc tgcacaagag gatcgccctg cgcacgctcc 240
tctgggtggg ggccaagcct gcacggctga tgctgggctt catgggcgtc acagccctcc 300
tgtccatgtg gatcagtaac acggcaacca cggccatgat ggtgcccatc gtggaggcca 360
tattgcagca gatggaagcc acaagcgcag ccaccgaggc cggcct 406

<210> 318
<211> 432
<212> DNA
<213> Homo sapiens

<400> 318
cctgaggatg cacctcttga aaatgtggag tgtgacaata tgaaccgctt tgaccgacca 60
gacagaaatg ttcggcagcc tcaggaaggt ttttggaaaa ggccacccca gaggtggagt 120
ggacaggagc attaccacct cagccaccct gaccactatc atcaccatgg aaaaagtgac 180
ttgagcagcc tcaagtacta cagccttcca gcagccttcc cagacccaca gaccacaccc 240
agggaaaact aataaagcca caacgtatcg aggcccgcag tgaatgctca atccaagaca 300
tgcatcaggg cggtgccatc cggaagaaag ctttacacac gtcttgcacc aagaaagcaa 360
ctatggtttg gaagagcagc ctttttcatc aggcccacaa ttaacaatgg atgtggctaa 420
gagtacacct tc 432

<210> 319
<211> 1131
<212> DNA
<213> Homo sapiens

<400> 319
   gagtggggcc ccgaaatctg agggtgatcg tagtcactag ttgtggcccc catgcaggag 60
   cggagagcga gttctggttt ctgaaacctc gagggatggg agagtatctg ggccgggtgg 120
   acggaggcgg ggagatggag agcgctagac accccagttt tagagtgcag atggatcccg 180
   cttaaggcaa caaaaaaaag cctggtttat gtgtcgacgc tttgatgctt ttgagtcgtg 240
   aggcggtctg gctttttgtt ttgtttgttt gttttgtttt tatcttccat gaacagcgct 300
   tccgtggatg ggcatctcag tggttgtcgt ctttttctct tcctttcacc tcttttcagg 360
   ttttattgtg actactgcga tacatactct caccccatga ctctccatct gtgagacaga 420
   ccacactgca gtggaaagca aacacataga gaatgtgaaa gactattatc agaaattgga 480
   tggaaaaagc agtctcagag cctgatttga caaaaccaac ggcttgcatt tccaacaagg 540
   aaaagaaccc tcctaattcc attctctgct ccttccttcc ttccaggggc cgatgatacc 600
   aaccttcccc cccaagcctt cggggttcct cctcgccctt ggtatgaatg cccaagcacc 660
   cccatattgg gggggccctt cccatggatg gccaatgatt gggcccctcc ctcttcctgg 720
   ggattgattg cccagtgggg acctgcttct ggggaattga ggccggccca tggggaggcc 780
   attattgcca attgattgcc tggggtcccc caatggatgt aggaccttct tgcccgttcc 840
   attgatggtg ccccctcggg cccggaatga cttcgaccag acagataagg atagagggga 900
   ggcccttatt gtatcgggtt ttatattacc tgttctgctt caccaggaga tcatgctgct 960
   gtgatacttg agttttctaa acgagcataa ggaagacttg ctcccctgtc ctatgaaaga 1020
   gaatagtttt ggaggggaga agtgggacaa aaaaagatgc agtttccttt tgtattggga 1080
   aatgtgaaaa taaaattgtc aactctttca gttaaaaaaa aaaaggaatt c 1131

<210> 320
<211> 443
<212> DNA
<213> Homo sapiens

<400> 320
cagaagcagc agatcccttc tgacctacag agtatgaaac ttgttccttg cataaaagaa 60
gcctttttgt tgaagaatgg gtgaaggaaa cacttgctgt tctgtgccag ccaaacaata 120
cccatttgtc accccaattg aagagagaat tctcatggaa gagggaaaag ccttcccccc 180

ctcaagatct acagctaaac agaaacttga tggaaaccct gtttctccta ctccggttta 240
ttggcctctc ccccacacca aacaaagaag aaaagcacct gaatctttgc ccctttgagc 300
cgactggcca tctggatgga gcacgtgata cagctggacc aagttggttg catcatcgat 360
tctgagaatg aaagggttta atgttatcta ggggttttct tcatttgatt cacagtccta 420
taaaagtgtg tgccaacttc aaa 443

<210> 321
<211> 401
<212> DNA
<213> Homo sapiens

<400> 321
aggcaggcag gtcagtgatg tgtcctaagg gtccgaccga cctagatacc cctctttgat 60
tcctcctctt gggattagtg tccatctctg gaagcaggat ccaggaggac gggaggggcc 120
gctgcggacc gcagtcgctc cacctggagg agacaccaga aggaagacag cctgagggac 180
gcagccatcc ccggctccta ccggcgcccc gccccgcgca tgcgcacgcg cacagggagt 240
cagctggctg cgcgggaggt cacgggaagt ggggcggtgc ccacacagct ggagggaagg 300
aggtgtcagg cggggagaga cgcaaacggc gggaccagca gcgacgggga ggcacacgat 360
ccgaggtcct tttagtctca gaggatggga agatcctggc g 401

<210> 322
<211> 381
<212> DNA
<213> Homo sapiens

<220>
<221> misc_feature
<222> (1)...(381)
<223> n = a,t,c or g

<400> 322
aggaaatatt aagattcttc ttatagtatt taacctatta gggtaaaaag gatttaaaat 60
aatatattta catatgttta aannnnnnnn nnnnnnnnnn nnnnnnnnnn nnnnnnnnnn 120
nnnnnnnnnn nnnnnnnatt agggttttta taaaaccaaa agaacttggc aacaccccta 180
ggtacccttt ataagcctcc aattggctac agcctgtgaa ggattggcct gtgaccaatc 240
agaggctgaa gtggagtctt agctcatggt caagcagagg ctgaagtgga aacttcttgt 300
ctttttatca caggcatgag gatgtggcct gcatgctgcc tgatcttgcc tagaaccggc 360
tgcacctgct gttctcttgt t 381

<210> 323
<211> 1549
<212> DNA
<213> Homo sapiens

<400> 323
gtggcaggag aggcggggcc aattttgctg agctttctcg cgggcttgca gctgcggcaa 60
gtgctggcgg cggctgctcg cgcaagtcag ctggcgtggg aactaccctt tgtagctgag 120
aacggcttgg ctatcgatac aaagactcta ttgacattgg taccttcagc gggcagcagc 180
ttcctacggc ataaagctgt agctacctga agaggctaca agcatccttc cctaggactg 240
ctgtaagctc tgagcactct agcaggagac atgcctcggg gacgaaaaga gtcggcgccg 300
ccgtaatgcg agagccgcat aagagaaccg caacaatcgc aaaatccagg cctcagaggc 360

ctccgagacc tctatggccg gctctgtggt aacgagcacc ccccgaagga cgaccctgag 420
cggccccgag gaagtacccg agcactccag aggagggcct cttaccaccc ctgaaggaag 480
cctcgagcac tgcccaagca caaaagcctt tcagttgccg ccggagcaat tttcaggggg 540
caccaagaaa agttttcctg gatgtctata ttacgcgctc atcttcatca tggggcaaca 600
gcgccaagga agctctggtc tggaaagtgc tggggaagtt aggaatgcag cctggacgtc 660
agcacagcat ctttggagat ccgaagaaga tcgtcccaga agagttttgt gcgcagaggg 720
tacctgattt ataaaccggt ggccccgtag cagtccggtg gagtatgagt tcttctgggg 780
ggccccgagc acacgtggga attcgagcaa actgaaagtt catgcatttt tgtgggcaag 840
ggtttcgtaa accgattgct cttaaagact ggccttgtaa ttatgactgg gattcggacg 900
atgatgccag aggttgaggc tatcctccaa ttcaggtgct taggggttat tccgccccct 960
taaagtagat ctggaggcag acccttgggg gtgtaaaaag aggagtcaca ggtaccccca 1020
aaggagtaga tgcccagggt ccctaagttt gaaaatgatg tccgattggg gggcggggga 1080
ccactgtatt tgatatttgt gatccagtga tcattgttcc aactgccgaa atagagtgtt 1140
tgctttttga taaatggaaa attgtattcc gttttaaaat tcccgtttgt tgaggaataa 1200
caatatgttc tcaaaatata attgaacaaa ttttttttct ttgttcccct gtcattgacc 1260
atttagtata acagttttgc ctaacgttcc taaaatgaag tccgttccca tccataatcc 1320
tatgatcctt gtacagcact tatagaaata agctgttctt ttgaagttga aatacccagt 1380
aaaatgttga agaaggatgg aggatttctt catattctga cgtttctgaa accctttgtg 1440
tctgctgttg tgtgaagatt gacatttacc atgattttcc ttacttactg cagaacatag 1500
agaaaaataa aagcctaacg aatagtaaaa aaaaaaaaaa aaaaacact 1549

<210> 324
<211> 412
<212> DNA
<213> Homo sapiens

<400> 324
cgtcggtggg gccggcggtc agtctgcggc agcggcagca agacggtgca gtgaaggaga 60
gtgggcgtct ggcggggtcc gcagtttcag cagagccgct gcagccatgg ccccaatcaa 120
gacacacctg ccagggtttg tggagcaggc tgaggctctg aaggccaagg gagtccaggt 180
ggtggcctgt ctgagtgtta atgatgcctt tgtgactggc gagtggggcc gagcccacaa 240
ggcggaaggc aaggttcggc tcctggctga tcccactggg gcctttggga aggagacaga 300
cttattacta gatgattcgc tggtgtccat ctttgggaat cgacgtctca agaggttctc 360
catggtggta caggatggca tagtgaaggc cctgaatgtg gaaccagatg gc 412

<210> 325
<211> 463
<212> DNA
<213> Homo sapiens

<400> 325
gcagtggcat cagcgatggc ggctgcgtcg gggtcggttc tgcagcgctg tatcgtgtcg 60
ccggcaggga ggcatagcgc ctctctgatc ttcctgcatg gctcaggtga ttctggacaa 120
ggattaagaa tgtggatcaa gcaggtttta aatcaagatt taacattcca acacataaaa 180
attatttatc caacagctcc tcccaggtat gcagtaattt atctcacttg tcaatataac 240
tctgtggcat aaaatatata tagagagatg tgccagctag gtgttcttta agcttaattt 300
tgtatttaac atactttaaa ttagagtact ttgtccccag aatgtattct ttattgcagt 360
tacactgttg gaccaagttt tgccaggtga gtaatttatt ccctttctgt aaagcattcc 420
ctatagctct gaatttaaac tgtgtcccaa gcatggctta tgt 463

<210> 326
<211> 583

<212> DNA
<213> Homo sapiens

<400> 326
cttttcaga aacatacatg gaaatacaga actacacctg taattacaga gggcatatga 60
accctagttt gataagcctt gctttaaagg atgcagtgtg ttctattaaa tgtatttaat 120
acaattgcag aaatggaaaa tgtggacctc cattaaacaa atttttagt tcctaatgag 180
ataaacttgg gaccaaaccg attcctcttg ttttagtgct caaaaatagt ttcattttta 240
ggtatttatg ctttagtggc ctttttttct acctttaaaa ttgtttaagt tccaagtaat 300
tttatttttg gatgttatat atattcttaa cttttatctc tacagatatt ttctggcaag 360
tttctatacg aagtatgatc caactcactt catcctaaac acagcttctc tcctgagtgt 420
actaattccc aaaatgccac aactacatgg tgttcggatc tttggaatta ataagtattg 480
aaatgtttg aaactgaaaa aaaatttac agctactgaa tttcttataa ggaaggagtg 540
gttagtaaac tgcactgttt ctgtgataat gtgaaatgag aag 583

<210> 327
<211> 1539
<212> DNA
<213> Homo sapiens

<400> 327
   taagacactc ttgtttcgct ccttgacaac cctggcgggg gttcgctggc tgcggccccg 60
   gctccggccc ccgcaggagc agcacccccc ggggaaagac attttctgct cccaccgagt 120
   tggcagggcc tgcttcctga atctcctggg tgtgtcttaa ctgccagtcc cagcacctcc 180
   tgaaagcccc actctcctcc agtggtcaca gtggaaggat catggtagaa acacacggga 240
   agaacgatga ctgctgatta taagcgactg cagaccttcc ctctggtcat gcactcggac 300
   atgccacagg agatgcgcgc ggagaccatg gagctatgtg ttcacagcct gtgagaaatt 360
   tttccaacaa caaacggaga gcgcccgcca agattgttca aaagagacaa tggacaagaa 420
   gtttcggctc ctcctgggca cgttgtgatc ggcgagggct ttgggttttg aagatcaccc 480
   acgaggttga aagaacctcc tctaccctgt acttcggggg caccttggct gtgtgcgtct 540
   ggaagttgct ccttgacact ctgtcccctg ccccgtcccc tgcagggcct tttcctgcca 600
   ctcatctggg gtggggagca gccctaggca ggtcctggtt tttccaagga gagttggggt 660
   cttttctttt tgtctttgtg taccagtttc ctgagccacg cccagtgtgt gagacttgac 720
   atctccatcc ccaggctctc aacccgtctc cctcggagtc tcagggtgtg gacggggcag 780
   cgggcatggg tctgtgtggg agacgtgggg tggggccggt gtgacagggt agaggaggtg 840
   ggagatgaga tcttccgcac aggaacacgc cagtccccct ttctccaggg gctgccttcc 900
   ccttggcatc ctggggagcc ccacttgccc tgccatcccc agtacttgcc ggggaagtgt 960
   ccggccgtcc cttgtcatta gtggtcatat tgaaaatggc cccccaagaa ggagatgatt 1020
   cttttcaagg gacacaggca gcttctctcc ttgtcctctg ggaaggtgct tgacccctca 1080
   agaaacccct tccacaaact tgaccccagg ctgaacagac cacttgcatc tcaactgggc 1140
   ccagcagccc cccccagccc cccagccttg ggtggggacc aagcaagcct ttcccgtccc 1200
   ctcctcgacc cgtacagttt gagaagccag gggctggtgt gtgggagctt gctaccctgg 1260
   cagtttctcg aggggtcccc ccgagcctct ggtgggacac cttgggcaag gaagtgctct 1320
   caccacgaag gctgcttccg caaggaaacc cttggcctgc ccgcgacttc gcatcaaggg 1380
   accgcattgc tgatttgtac tggtctctgc ttgggttttc taatgtcttt tcgaagtgtg 1440
   ggaaaagggt tttagtagaa gggtgaatcg tattttacac agcggtctta tttatataaa 1500
   tgtcttggtt tttacaatta aaatgaccaa aaactgaaa 1539

<210> 328
<211> 406
<212> DNA
<213> Homo sapiens

<400> 328
tcgtaagaca caaaccacca gacgtgggcc tgggctctgg gctgggccag gtggatgaag 60
gggaggctgg tggtccaggc ggctgctctt ggctgcaggg tttctgggca cacatccggg 120
tagcacgcat cctggccttc agcaacctcg ttttaaatgg gaccacacca ggagcagcca 180
gggcgctttc attttcactt tctttccccg ggggggacag gaacattcct ttacctcctg 240
aggaaatcgg gctcttccag agccacagag gagtgaaggc cccagccctt cacgatgctg 300
cgcggctatg tttgggtctg tccatcaggg agaaagaaaa accggccgag tcttgcacag 360
ggtcctcgac agcaacccca ggtaaagagc agagcaagcc gtccaa 406

<210> 329
<211> 1108
<212> DNA
<213> Homo sapiens

<400> 329
atggtctacg gggcttctga ggcaatcggg cagcgtcagt cttcagccgc taagccaaga 60
aggagtcagt cagagaacct tgggccagag ttccaggagc tctgggagtg gctgccagga 120
aagcgggact tgccgctaag ggtgaaggag aaggggttga ggggtacttg cccctgcccc 180
aggaaagcgg gacttgctgc taagggtgaa ggagaagggg ttgaggggtt cttgcccctg 240
ccccaggaaa gcagagaagg gaacaggagg acaggagact ggtctcctaa gggaggtcgg 300
tcccccgatc cgagtcacgg caccaaattt catgcgcgtc cgtgtgaaga gaccaccaaa 360
caggctttgt gtgagcaaca tggctgttta tttcacctgg agcatcgaat tgagtgaaaa 420
atgactctgg tttcttctct atatcctcag tgtccacctt cacatccacc atggggttga 480
gattctgagc tcgctccaaa gaggcttcag ccctatttcg gccaacagac ccagtacgaa 540
tcaagaactg agctccggga tcttctggag ttaccttctc ctccaagaaa atgagctggc 600
ccacttccca ttctaggcta accctgtatc cttgacccat tccatcctgc ttcttcaggg 660
atctcattat gtgctgagaa gctggcagcc actcccagag cccctggaac tctggcccaa 720
ggctctccga ctgactcctt ctcggcttgg cggctgaaga ctgacgctgc ccgatcacct 780
cgaaagcccc gtagaccatc acagacgccg agcttcgagt aacttgggca agtgacacct 840
tagggtgagt ctccttgtct gtaaaagggg ctgtggacag tgcctccttc attcggtgac 900
accgtttctg ggcctccagt ccccacaggc ggatctgccg gtcatactgt gccgcctcct 960
cctcgctaat gccgccgcca gcctcctcct tctccaccat ggcgccggct cttcctgcct 1020
cagctccggt cccgctcaag ccaaccgccc gccggacccg cgcgccacct accgccgccg 1080
gcagcacgcc cggagtgctt ctgcgcat 1108

<210> 330
<211> 2035
<212> DNA
<213> Homo sapiens

<220>
<221> misc_feature
<222> (1)...(2035)
<223> n = a,t,c or g

<400> 330
gccaaatcca gttttcatat ttaaagcgaa tgagccccct cctctcagtg ggaaagatga 60
gctgaagctt tactccctcg atagtgttgg gaaaagaaat cagacaaaaa tctaaggaag 120
gaccaaatat tgtacatagt gtgccagtag gcttttgcaa ctggactgaa aatacctgcc 180
ttttctctcc acaggggaaa gtggaagttg aagctgggaa agaagggatg aaggttgaag 240
cgagcgcctt ctcatactat ggcgagatgg ccctgacagc ctctccaggt gaaaataagt 300
cccctcctcg cccatgtggc ttgaatcact cagactctct cagtcgaagc gaccgggatt 360
gacgccgtca caccaacact ggggagcagc aataaccagc tcaattcttc gctcctccaa 420
gtctacatcc ccgattactc cggtgcgagc cctttcggat ctgcagtttg ttaagatctc 480

aagacagcaa taccaaaatg ccttgatggc atcccgggat ggacaaaacc ccccagtctt 540
cagacagtga aaacactaaa atcgaattga ctcttacgga gctgcatgac gggttgccag 600
acgagacagc caacctgctc aacgaacaga actgtgtgac gcacagtaag gccaaccaca 660
gcctgcacaa cgaaggcgcc atcttaggcc gcgctggctg cacccgccca ggcccgcacc 720
cgcccagtcc cgtaggcccg gccctgtctg cccatgactt cactggtgtg agcttgtccg 780
ccatgctgta ccctgcaaca tcctgagacc aaagaccttg tgcccttccc aggagccgcg 840
gaggaggaca gtgagggagg aatggaaacg agagatgtga agttggcagc cggggcatgg 900
cgttcaagat tttggagatg aactgattcc gcccaaatag aatcatgttt atttttcag 960
ctctcccttt tatcattatt cacactcctc tgccctcgat ttgcatgaag ttgaaaattg 1020
ttgcgattta tttttccaa gagaatccat gtttttaaag tgtctttgc cagagtttta 1080
aagttgttct gtctgaactc tgctgtgatc ccatgatgtg gaccctgatg ggctggactt 1140
ncccctccg gtagccttcc ttggccctcc cagcggaggg gcacccttcc tctgtgcccc 1200
agtgggattc accgtcgatc tcgctggctg aatgaagaag accctgttac tgcagaacct 1260
gccaagtctg tcatcactgc ttggggtgta gcctgcctca gagggccctc caatcacctc 1320
tctgagctca atggttattt tgagaattta atgtttaact gtaccccctt tccctcagga 1380
agatttaaca tttgcttggg aatgtgattt tgctcccacc ctaaaggaat tttatcacc 1440
aaaatggaat gttaaatgga attttaaaac ccatggtttt attcattggg caagaggcaa 1500
gttgacttca tcctgtcatt ccagcctggg gtctgccagc cagccctcct ccccgaccca 1560
gcgcctaagc tcacagagtg tcgtcgccca cctccctggg tccttgctct tgttaaccca 1620
aagatgctgc tacacagaat gccaaatggg aaatcttcca cagggctttt tcagtaaaca 1680
cggtgatgtg ggagttgcaa gctcctcccc ttcccactag aacatacttt aacagaaaac 1740
gagtcggacc ttcttagctg cactctgtac tgtgtgcccg agaaggcatt tctagacccg 1800
tgtttttaaa ggagggaact ttggggattg ccagcccctg ctcctcctcc cagggagcca 1860
actgtccctc ctcccctgtc gcctgggccc atggggcccg gcagtggctg tgtcccctgc 1920
ctgagggctc tgtgcctcct gcctcagatg cggcctgtgc cagagaggct gcctgtacag 1980
ccagggtcag tttggcccca aacagggatt cagaatccaa aaaaaaaaaa aaaaa 2035

<210> 331
<211> 381
<212> DNA
<213> Homo sapiens

<220>
<221> misc_feature
<222> (1)...(381)
<223> n = a,t,c or g

<400> 331
aggaaatatt aagattcttc ttatagtatt taacctatta gggtaaaaag gatttaaaat 60
aatatattta catatgttta aannnnnnnn nnnnnnnnnn nnnnnnnnnn nnnnnnnnnn 120
nnnnnnnnnn nnnnnnnatt agggttttta taaaaccaaa agaacttggc aacaccccta 180
ggtacccttt ataagcctcc aattggctac agcctgtgaa ggattggcct gtgaccaatc 240
agaggctgaa gtggagtctt agctcatggt caagcagagg ctgaagtgga aacttcttgt 300
ctttttatca caggcatgag gatgtggcct gcatgctgcc tgatcttgcc tagaaccggc 360
tgcacctgct gttctcttgt t 381

<210> 332
<211> 1092
<212> DNA
<213> Homo sapiens

<400> 332
atgagagccc tcacactcct cgccctattg gccctggccg cactttgcat cgctggccag 60
gcaggtgagt gcccccacct cccctcaggc cgcattgcag tgggggctga gaggaggaag 120

caccatggcc cacctcttct cacccctttg gctggcagtc cctttgcagt ctaaccacct 180
tgttgcaggc tcaatccatt tgccccagct ctgcccttgc agagggagag gagggaagag 240
caagctgccc gagacgcagg ggaaggagga tgagggccct ggggatgagc tggggtgaac 300
caggctccct ttcctttgca ggtgcgaagc ccagcggtgc agagtccagc aaaggtgcag 360
gtatgaggat ggacctgatg ggttcctgga ccctcccctc tcaccctggt ccctcagtct 420
cattccccca ctcctgccac ctcctgtctg gccatcagga aggccagcct gctccccacc 480
tgatcctccc aaacccagag ccacctgatg cctgcccctc tgctccacag cctttgtgtc 540
caagcaggag ggcagcgagg tagtgaagag acccaggcgc tacctgtatc aatggctggg 600
gtgagagaaa aggcagagct gggccaaggc cctgcctctc cgggatggtc tgtgggggag 660
ctgcagcagg gagtggcctc tctgggttgt ggtgggggta caggcagcct gccctggtgg 720
gcaccctgga gccccatgtg tagggagagg agggatgggc attttgcacg ggggctgatg 780
ccaccacgtc gggtgtctca gagccccaag tcccttaccc ggatcccctg gagcccagga 840
gggaggtgtg tgagctcaat ccggactgta acgagttggc tgaccacatc ggctttcagg 900
aggcctatcg gcgcttctac ggcccggtct agggtgtcgc tctgcctggc ctggccggca 960
accccagttt tgctcctctc caggcaccct tctttcctct tccccttgcc cttgccctga 1020
cctcccagcc ctatggatgt ggggtcccca tcatcccagc tgctcccaaa taaactccag 1080
aagaggaaaa aa 1092

<210> 333
<211> 486
<212> DNA
<213> Homo sapiens

<220>
<221> misc_feature
<222> (1)...(486)
<223> n = a,t,c or g

<400> 333
ncattcccac tcgggtttga cctacngccg cccgggagaa gatggctgcc ccagcagtgg 60
tccgggctct cccgcnaggt gcgatgcttc agtacctctg tggtcagacc atttgccaag 120
cttgtgaggc ctcctgttca ggtatacggt attgaaggtc gctatgccac agctctttat 180
tctgttttga atccctatgt gaagcgttcc attaaagtga aaagcctaaa tgacatcaca 240
gcaaaagaga ggttctctcc cctcactacc aacctgatca atttgcttgc tgaaaatggt 300
cgattaagca atacccaagg agtcgtttct gccttttcta ccatgatgag tgtccatcgc 360
ggagaggtac cttgcacagt ggacctctgc atctccttta ggaagaagcc acactctctg 420
gaattaaaaa ctggtccctc aagagcttcc taaggtcaag gncaagtatt ggaaattggg 480
aggcta 486

<210> 334
<211> 510
<212> DNA
<213> Homo sapiens

<400> 334
atgagtatgc tcaggcttca gaagaggctc gcctctagtg tccttcgctg tggcaagaag 60
aaggtgtggt tggaccccaa taagaccaat gaaatcgcca atgccaattc ccatcagcag 120
atctggaagc tgatcaaaga tgggctgatc atccgcaagc ctgtgacggt ccattcccgg 180
gctcgatgcc agaaaaacac cttggcccac tggaagggca ggcacatagg catagataaa 240
agatactgtg aatctaagaa gattgatcgc cacatgtatc acagcctgta cctgaaggtg 300
caggggaatg tgttcacaaa caagccgatt ctcatggaac acagccacaa gctgaaggca 360
gacaaggctc acaagaagct cctggctgac caggctgagg cccgcaggcc taagaccaag 420
gaagcacgga agcgcagtga agagcgcctc caggccaaga aggaggagat catcaagact 480
ttgttcaagg aggaagacac caagaaataa 510

<210> 335
<211> 480
<212> DNA
<213> Homo sapiens

<220>
<221> misc_feature
<222> (1)...(480)
<223> n = a,t,c or g

<400> 335
ggcacgagaa tgcttcatat taacatagaa aagaaatttc ttcacctatc tcctgagtct 60
tttgaaatct tcacatatat tattttctaa attattatta tttaaaattt tcttaaagat 120
ggagtcttgc tctgtctccc aggctggata gagtacagtg gcatgatctt agctcactgt 180
aacctcagac ttctgggatc aaacaattct cccctcagcc tcctgaatag ctggggctac 240
aggtgcatgc catcatgcat ggctaatttt ttaaaatttt tcgtagagat ggggtctcgc 300
tacgtagtct aggctgttct caaactcctg gggctcaagc aatcctccca cctcagcctc 360
ccaaggcgct ggggattatt aggtgttgag tcaccatccc tggggcttct atttttattt 420
attttaaaaa aaaattatta tgtattatat taaatttccn gtgaattgat agggggcagg 480

<210> 336
<211> 403
<212> DNA
<213> Homo sapiens

<400> 336
cagtcctcat ttctgtggaa tctgaataag cccacgcata ttttaattcc cactctagat 60
tctggttcta atcaaggtcc atgcatgtgg tgtcaacccc gtggagacat acattcgctc 120
tggtacttat agtagaaaac cactcttacc ctatactcct ggctcagatg tggctggggt 180
gatagaagct gttggagata atgcatctgc tttcaagaaa ggtgacagag ttttcactag 240
cagcacgatc tctgggggtt atgcagagta tgctcttgca gcagaccaca ctgtttacaa 300
actacctgaa aaactggact ttaaacaagg agctgccatc ggcattccat attttactgc 360
ttatcgagct ctgatccaca gtgcctgtgt gaaagctgga gag 403

<210> 337
<211> 1065
<212> DNA
<213> Homo sapiens

<400> 337
tgtctcagcg gctgccaaca gatcatgagc catcagctcc tctggggcta gctatatgac 60
aacagaactc tcaccaaagg accagacaca gtgggcacca tgggacagtg tcggtcagcc 120
aacgcagagg atgctcagga attcagtgat gtggagaggg ccattgagac cctcatcaag 180
aactttcacc agtactccgt ggagggtggg aaggagacgc tgaccccttc tgagctacgg 240
gacctggtca cccagccagc tgccccatct catgccgagc aactgtggcc tggaagagaa 300
aattgccaac ctgggcagct gcaatgactc taaactggag ttcaggagta tctgggagct 360
gattggagaa gcggccaaga gtgtgaagct ggagaggcct gtccgggggc actgagaact 420
ccctctggga attcttgggg ggtgttgggg agagactgtg ggcctggaaa taaaacttgt 480
ctcctctacc accaccctgt accctagcct gcacctgtcc tcatctctgc aaagttcagc 540
ttccttcccc aggtctctgt gcactctgtc ttggatgctc tggggagctc atgggtggtg 600

gagtctccac cagagggagg ctcaggggac tggttgggcc agggatgaat atttgaggga 660
taaaaattgt gtaagagcca aagaattggt agtaggggga gaacagaaga ggagctgggc 720
tatgggaaat gattttgaat aatggaagct gggaatatgg catggatatc tggtactaaa 780
agaagggtcg tttaagaacc tctttcctta tctctttccg caatccaaac catagctgtc 840
tgtccaagtg ctctcttcct gcctccagct ctgccccagg cctcctccta gactctgtcc 900
ctgggctagg gcaggggagg agggagagcc agggttgggg gagaggctga ggagagtgtg 960
acatgtgggg agaaggacca agctgggtgc ttgggcattg acagaaatga tggtgtgttt 1020
tggtatcatt tgattaataa aaaaaaaatg aaaaaagtga aaaaa 1065

<210> 338
<211> 362
<212> DNA
<213> Homo sapiens

<400> 338
gcggccgcac atattcaccc tcagcgtgcc tttcccgacc cccttggagg cggaaatcgc 60
ccatgggtcc ctggcaccag atgccgagcc ccaccaaagg gtggttggga aggatctcac 120
accgctggaa agctgaagac tgtcgcctgc tccgaatttc cgtcatcaac tttcttgacc 180
agctttccct ggtggtgcgg accatgcagc gctttggccc ccccgtttcc cgctaagcct 240
ggcctgggca aatggagcga ggtcccactt tgcgtctcct tgtaggcagt gcgtccatcc 300
ttccctaggg caggaattcc cacagttgct actttcctgg gagggcctca tgttttatct 360
gg 362

<210> 339
<211> 420
<212> DNA
<213> Homo sapiens

<400> 339
ggacccccgt tcacctagtg ctggcatcct gcgcactccc atccaggtac tctgggccag 60
cgtgggaaag gcagagaatg tctgaattct tgggtctctt cctaacctga ttttgagaga 120
gccgtcatga ccccaccctt atcctagcct tattttctgc aatctcaatc tgtgtggggt 180
aggctggata tctgagggcc ttggcaattc cttcctggaa tatggggagg agaggagaga 240
agagtcaggg cccagacttg ttctagccta tggtcttgac aggtggagag ctctccacag 300
ccaggcctac cagcagggga gcaactggag ggtcttaaac atgcccagga ctcagatccc 360
cgctctccta ctcttggtat tgcacggaca cctatgaaga ccagcagtgg aggtaagcgt 420

<210> 340
<211> 450
<212> DNA
<213> Homo sapiens

<400> 340
gctagaagct cattctgaaa tggggagcac tgaaattttg gaaaaggaga ccccagaaaa 60
tctcagtaat ggtaccagca gcaatgtgga agcagccaaa aggttggcca aacgccttta 120
tcagctggac agattcaaaa gatcagatgt tgcaaaacac cttggcaaga acaacgaatt 180
tagcaaacta gttgcagaag aatatctgaa gttttttgat tttacaggaa tgacgctgga 240
tcagtcactc agagtctcgc tctgtcaccc aggctggagt gcagtagcac catcttggct 300
cactgcaacc tccaccttcc aggttcaagc agttctcctg aatcagcctc ccgaataact 360
gggactacag gaaaatgaat ctacattgtc aagtgagggt catctggatt catctcaaat 420
aaacagtgct cttagtgact ccgagtaaaa 450

<210> 341
<211> 492
<212> DNA
<213> Homo sapiens

<400> 341
cggcccgtaa ttcccggctc gacgatttcg tgggcgctgc gcggcgcagc gaaaatggcg 60
gcttccaggt gggcgcgcaa ggccgtggtc ctgctttgtg cctctgacct gctgctgctg 120
ctgctactgc taccaccgcc tgggtcctgc gcggccgaag gctcgcccgg gacgcccgac 180
gagtctaccc cacctccccg gaagaagaag aaggatattc gcgattacaa tgatgcagac 240
atggcgcgtc ttctggagca atgggaggtc cgcggcgcgc gcacaccctc ccggctcttc 300
tctgtgcggg accctgttgc ctatgacccc ttacctgttc tgtcttggga cctttctgac 360
cccctaacct cctctactca tgccttgaat ccttactgac ccactgaagc cactgttgac 420
cctccattcc ttctctgacc cgcgcgccct gctgaccctc gctccagccc tctgtcattc 480
cagtgccctg tt 492

<210> 342
<211> 439
<212> DNA
<213> Homo sapiens

<220>
<221> misc_feature
<222> (1)...(439)
<223> n = a,t,c or g

<400> 342
tttcgtcaga gccgggttcc tcccgggttt ctgccgggtt tctccctgct gctcctgggt 60
tgttgagact cttgcgaaga tggcttgcgc tgccgcgcgg tccccggccg accaggacag 120
gtttatttgt atctatcctg cttatttaaa taataagaag accatcgcag agggaaggcg 180
aatccccata agtaaggctg ttgaaaatcc tacagctaca gagattcaag atgtatgttc 240
agcagatgga cttaacgtat ttcttgaggt taagaatgtc tgaggggtca ggcggtgctg 300
gcaagatggc tgcacttgag aagatgacgt ttcccaggaa gatgacattt ccagagaagc 360
caagccacaa aaagtacagg gccgccctga agaaggagaa acgaaagata cgtcggcagg 420
aacttgctcg actgatagn 439

<210> 343
<211> 508
<212> DNA
<213> Homo sapiens

<400> 343
atgctgacgg agctggtgcc cacacaggcc gagcccgcag acctggagct gctgcaggag 60
ctcaaccgca cgtgccgagc catgcagcag cgggtcctgg agctcatccc tcagatcgcc 120
aatgagcagc tgacagagga gctgctcatc gtcaatgaca atctcaacaa tgtgttcctg 180
cgccatgaac ggtttgaacg gttccgaaca ggccccatgg ctttggctgg ccactgaggg 240
tagggtgtgg aggtgtgaag gccccctgag gagctgcggc ggcccaggta cgaagctgca 300
actctgcgcg cagtgggcga gatctcatca gccccaggct gcaggtgaga cttcagggga 360
tgctggggcc ccactgcccc tccgctgcct tgccctccat ccttcctctg ttccttctgg 420
ccgggcacca cagcactggg gctcacctct tggttgatcc tcttgtactg ggagaggtgc 480

ctttgtatc cccaattaaa ggtagaaa 508

<210> 344
<211> 336
<212> DNA
<213> Homo sapiens

<400> 344
tcccaaacgt tctgtactgt ccaagtcagt tcctgggtac aagccaaagg tcattccaaa 60
tgctatatgt ggaatttgtc tgaagggtaa ggagtccaac aagaaaggaa aggctgaatc 120
acttatacac tgctcccaat gtgagaatag tggtcgctgg atttgtgact gttgtcagcg 180
ggccccccca acacccagga aagtgggcag aagggggaaa aacagcaaag agggataaaa 240
tagtttttga ctctaatact gtatatgcat ttaagtggaa tatttggtgc catttacaac 300
attattttca tgccaataaa agattttttt tgcaaa 336

<210> 345
<211> 2157
<212> DNA
<213> Homo sapiens

<400> 345
tttgttaaac aggcattacc attgtatatt taaaaacttg ggaagtttta actgaagaag 60
gcagcatgat atgatagctt ttgcttttta caatatgatg ttattagtct tgttctgtgt 120
ggttctggag agcaaaacaa taaagcattg taagttgtat gagtgtaaga acaagtattc 180
actactaatt cccagcacat gaatagtact caatacattt tcaataaatg attgaataca 240
tgtatgataa atgaaaacca gtgggttaaa cttacaagga ggcagaggct tttttttgtt 300
ttgttttgtt tttgagacag agtcttgctc tgtcgcccag gctggagtgc agtggtgcga 360
tctcggctta ctgcagcctc cacttcctgg gttaatttga agcagaagag ggcagttgtt 420
atactgccct gtcagttgga tgcggagtct tactcaaaat tcattctcag cattcttctt 480
ttatggtatc ttctttggca cttagcagcg catcaggtag gcatcttcta tttttcttca 540
ttccttaatt tcctttgtat ccctcaaatg gttatttatt tggctggagt ctgttttgtt 600
cattaagcaa acatgtcttt gctttgaaca tgtctttgat ttgatggata cttaaattcc 660
tcatcaaaca ttttgttgct atgcataacg ttttctttgg ccaactccag caatttccca 720
cattttgaca tgcaatcatg ttaactccca ttttcttttg taatccaaca tcttctattt 780
agataattac tttaacaatc aatgacttaa tattctaatc ataaatttat acaaaaataa 840
aattacctcc aaaacattgc tacctttcct aaacattcag tcttgccaca gtttaataaa 900
aggaaagaac attaaaaagg ataagacact gtaatgatta gatgcttttt ataagcctaa 960
aggcattgtg attatttaga cagaagagaa gaaagtgaag tgaaaacctg atagttatgt 1020
agtctcatgg tttgctgttg agaggctgaa caccagctgc tttccttttc taggaagata 1080
ataaagtggg ctttggctac aacataaaga tgttgggtta gacagtttca ctacagtaag 1140
aacaacggga tgagttgccc aggaaattgt gaaatacttt ctaatgatct ttaaagatat 1200
aatgaacact aattcatctg gatttgttta ggtgtggtcc tggttaaagg caaagggaag 1260
gatcagataa cttcatgttt tttccattta acatacccaa tagattcttg attaagggga 1320
agggaaaatg agcaagatac agtccagtat tctaaaaaca atcagcctta cggggatcat 1380
ttccaaaagc atcctggttt ttggacctta aggcctttga taccttaacc caatttgacc 1440
taccaccagt ggaaaattct agtgcctggg ttttataggg tagaagaaag acatgcagtc 1500
aagtggccaa tacttcatgt ggaggataag caatgagatc cttcttgctg tctttccttt 1560
tgactgttct gggcaatatc aaattagttt cagtggcttg attctaggcc aagattctgg 1620
caacagattg tagtcttacc ttgtttttct tcaatctcac tggatctctc tctcttttta 1680
ccccccttag gctgagggta aaaagctggg attggtaggc tgggtccaga acacctgacc 1740
ggggcacagt gcaaggacaa ttggcaaggt cccatctccc aaggtgcgtc atatggcagg 1800
aatggcctga aacaagagaa gcctatcaaa aaccccctaa aactatcgga caatagcaaa 1860
cttcaacaat gaaaaagtca tcttgaagtt gggattacct cagactttcc aaattgttaa 1920
aataatgggc ctggaatttt aagtttccct aaggttaaac tccagtggtt tggtttttat 1980

tattaataga gatagaacta ttgtgtgtta atattaggca ttagtcaata agttatttta 2040
atgtcagatt ttttgaatgt tattattata ttacctgtat gatggaagga ttaccacttt 2100
tccccaattt tatccattaa aaacgttaga accttctgct tagagtactt ttaaaaa 2157

<210> 346
<211> 375
<212> DNA
<213> Homo sapiens

<220>
<221> misc_feature
<222> (1)...(375)
<223> n = a,t,c or g

<400> 346
cagacagtct tgattctgta aacaccaagc tacacataga aatagagcag gctttagaga 60
cctagtcaaa tttgttttcc ctttcccttt ttcatttgaa gggttttttt tgtttttttt 120
ttttagattt ttagatggag tctcgctctg tcgccaggct ggagtgcagt ggcacgatct 180
tggcttattg caacctctgc ctcccgggtt caagtgattc tcctgcctca gcctcacatc 240
tgtaatccca gctctcaggg aggcaagagg cgggaggata gcttgagccc aggagttcga 300
gacctgcctg ggcaatatag cgagacccca ttctccagnn nnnnnnnnnn nnnnnnnnnn 360
nnnnnnnnnn nnnnn 375

<210> 347
<211> 3031
<212> DNA
<213> Homo sapiens

<220>
<221> misc_feature
<222> (1)...(3031)
<223> n = a,t,c or g

<400> 347
atgatcgctg atgataagcc agctgcgatg gctggtgagt cagctggaca gtcatctgag 60
tctggagttg gggccaactt ctttggcatc acattccaga caacagaaac actgatgagc 120
acagggcatc tgaatggggc cgaatgcaaa gcaggtccag gcactgtgaa gaccctggcg 180
gtggaggaag aggcttcccg gctgtggagg aagccagacc cttacaacac aagacgagaa 240
ccagacctgc gtgggggagc tctggatgct acaggggctc aaggagggcc actggaccga 300
gcgcgcaaag aacctgagac cgcttgctct caccgccgca agtcggtcgc aggacagaca 360
ccagtgggca gcaacaaaaa aagaaaccgg gttccgggac acgtgccggc ggctggacta 420
acctcagcgg ctgcaaccaa ggagcgcgca caatgctccg atacaggggg tctggatccc 480
tactctgcgg gccatttctc cagagcgact ttgctcttct gtcctcccca cactcaccgc 540
tgcatctccc tcaccaaaag cgagaagtcg gagcgacaac agctctttct gcccaagccc 600
cagtcagctg ttttcgggtc cgagggaagg aggaccctgc gaaagctgcg acgactatct 660
tcccctgggg ccatggactc ggacgccagc ctgggtgtcc agccgcccgt cgtcgccaga 720
gcccgatgac ctttttctgc cggcccggag taagggcagc agcggcagcg ccgttcagct 780
gggggcaccg tgtcctcgtc caccccgagt gactgcgcgc cggagctgag tcgccgagtt 840
gcgcgccgct atgggctctg cgaggcgcgc attctgggac caagctagga ggcagtggat 900
tcaagtcgtc ctcgtccagc acctcgtcgt ctacgtcgtc gtcggctgcg tcgtcctcca 960
agaaggtcaa gaagcgaatg acagagccgg agctgcagca gctgcgtctc aagatcagca 1020
gccgcgagcg caagcgcatg cacgacctca acatcgccat ggatggcctc cgcgaggtca 1080
tgccgtacgc acacggccct tcggtgcgca agctttccaa gatcgccacg ctgcttgctg 1140
gcgcgcaact acatctccat gctcaccact tcgctggagg agatgaagcg actggtgagc 1200

gagatctacg gggggccacc cncgctggct tccacccgtc ggcctgcggc ggcctggcgc 1260
actccgcgcc cctgcccgcc gccaccgcgc acccggcagc agcagcgcac gccgcacatc 1320
accccgcggt gcaccacccc atcctgccgc ccgccgccgc agcggctgct gccgccgctg 1380
cagccgcggc tgtgtccagc gcctctctgc ccggatccgg gctgccgtcg gtcggctcca 1440
tccgtccacc gcacggccta ctcaagtctc cgtctgctgc cgcggccgcc ccgctggggg 1500
gcgggggcgg cggcagtggg gcgagcgggg gcttccagca ctggggcggc atgccctgcc 1560
cctgcagcat gtgccaggtg ccgccgccgc accaccacgt gtcggctatg ggcgccggca 1620
gcctgccgcg cctcacctcc gacgccaagt gagccgactg gcgccggcgc gttctggcga 1680
caggggagcc aggggccgcg gggaagcgag gactggcctg cgctgggctc gggagctctg 1740
tcgcgaggag gggcgcagga ccatggactg ggggtggggc atggtgggga ttccagcatc 1800
tgcgaaccca agcaatgggg gcgcccacag agcagtgggg agtgagggga tgttctctcc 1860
gggacctgat cgagcgctgt ctggctttaa cctgagctgg tccagtagac atcgttttat 1920
gaaaaggtac cgctgtgtgc attcctcact agaactcatc cgacccccga cccccacctc 1980
cgggaaaaga ttctaaaaac ttctttccct gagagcgtgg cctgacttgc agactcggct 2040
tgggcagcac ttcggggggg gagggggtgt tatgggaggg ggacacattg gggccttgct 2100
cctcttcctc ctttcttggc gggtgggaga ctccgggtag ccgcactgca gaagcaacag 2160
cccgaccgcg ccctccaggg ttcgtccctg ggcccaaggc caggggccac aagttagttg 2220
ggaagccggc gtttcggtat tcagaaggcg ctgatggtca tatccaatct caatattctg 2280
gggtcaatcc acaccctctt agaactgtgg ccgttcctcc ctgtctctcg ttgatttggg 2340
agaatatggt tttctaataa atctgtggat gttccttctt caacagtatg agcaagttta 2400
tagacattca gagtagaacc acttgtggat tggaataacc caaaactgcc gatttcaggg 2460
gcgggtgcat tgtagttatt attttaaaat agaaactacc ccaccgactc atctttcctt 2520
ctctaagcac aaagtgattt ggttattttg gtacctgaga acgtaacaga attaaaaagc 2580
aagtgcctgt ggaaaacaag ttggggttaa tttggggggt cctgttgggc tttttaaaaa 2640
tttccttttt tgggatgtgt aaatttatca aatgaatgag gtaaagtggc gcaatgccta 2700
aggctgtttg cctcacgtga actgccaagc ccccatcgga agtcctaagc cggctttccc 2760
tctattttgg gtttattttt gcccacgttt aaacacaaat ggtaaactcc tccacgtgct 2820
tcctgcgttc cgtgcaaagc cgcctcggcg ctgcctgcgt tgcaaactgg gctttgtagc 2880
gtctgccgtg taacaccctt cctctgatcg caccgcccct cgcagagagt gtatcatctg 2940
ttttattttt gtaaaaacaa agtgctaaat aatatttatt acttgtttgg ttgcaaaaac 3000
ggaataaatg actgagtgtt gagattttaa a 3031

<210> 348
<211> 450
<212> DNA
<213> Homo sapiens

<400> 348
tgagagcaag atgggtcacc agcagttgta ctggagccac ccgcgaaaat tcggccaggg 60
ttctcgctct tggtgagaaa tagtttgtga ttttggggaa gcgttgccgt ggagcgctag 120
gctgcttaaa atctcaagta gaggaggcca cgggcggcac gagggagcac gccgactggg 180
agtcgctggt gccgccatta caggcctgta ctgtggcttg ggagtcccgg gatgcgggta 240
gtagccgtct gagtgcggag tcttgtaatt tctatgacct gcaaaaccgc tgtctctttt 300
cttacaagtc gtgtctgttc aaaccggcac ggtctgatcc ggaaatatgg cctcaatatg 360
tgccgccagt gtttccgtca gtacgcgaag gatatcgggt tcattaagtt ggactaaatg 420
ctcttccttc agaggattat ccggggcatc 450

<210> 349
<211> 337
<212> DNA
<213> Homo sapiens

<400> 349
gcttgaataa gaaaaacaaa ttcttcctcc taagccatgg catatcagtt atacagaaat 60

actactttgg gaaacagtct tcaggagagc ctagatgagc tcatacaggt gggagactga 120
attaaaaatc aaacattttt atcttgatgg gtttacagta attcttgtca tatcacttag 180
gcaaagagat atgaggtaaa atatcttgga ctatttgacc cagcaagtca gtaacactga 240
ttaaattacc tcaaattctt gtttattaca ctgtcctgac aacaggacac aaaacatgat 300
tgaatgatat cattaaatat agctaaatct gggtaca 337

<210> 350
<211> 424
<212> DNA
<213> Homo sapiens

<220>
<221> misc_feature
<222> (1)...(424)
<223> n = a,t,c or g

<400> 350
ccgacttctg gagagagatg atgcgggcga gctggttggt gagacaggac agccggcacc 60
agcgaatcag acttccacat ttggaagcaa ttgtgagtgg gcgtggccca tagaccaaga 120
tcactgataa gaaatgttct cgacagcaag gtaggagcca atcccaccag cattgactca 180
gtcgtaattg ggaaggacca agaggtgaag ctgcagcctg gccaggttct ccacatggcg 240
aatgaacttt atccatatat tgtatagttc gaggaagagg caaagaaccc tggcctggaa 300
acacacagga agagaaagag atcacgcaac agcgattcta tagaaaggga tgctgctcat 360
gaagctgagg ctgggacagg gctggaacct gggagcaact ctggccaatg ctctgtgccc 420
ctan 424

<210> 351
<211> 479
<212> DNA
<213> Homo sapiens

<400> 351
cactttggtt ccagcatcct ggtccagcaa agaagccaat cagcccaaaa tgatacctgg 60
aggcttatca agaggccaaa cccgccactc cagaaatccc aggagattgt tgataaggtt 120
aaaccacagc ttgaagaaaa aaacaaatga gacttatgga aaattggaag ctgtgcagta 180
taaaactcaa gttgttgctg gaacaaatta ctacgattaa ggtacgagca ggtgataata 240
aatatatgca cttgaaagta ttcaaaaagt cttcccggac aaaatgagga cttggtactt 300
actggatacc aggttgacaa aaacaaggga tgacgagctg accgggcttt tagcagcatg 360
tacccaaagt gttctgattt ccttcaactg gctactggag tcatgatcct ttgctgataa 420
atataaccat caataaagaa gcattctttt ccaaagaaaa aaaaaaaaaa aaagtaaac 479

<210> 352
<211> 307
<212> DNA
<213> Homo sapiens

<400> 352
gcagcttccc gggcagacac tctctccctc aggaagaggt gccgccgagt caagcgcggg 60
gcagtgtgag cgccccgagg tgctttctca gttgaggaga ggtggggtta cagggcacag 120
gtgacagggc cggagaaaga tggagcagcc cggggcggcg gcgtcgggag cgggaggcgg 180
cagcgaggaa cccggtgggg gccggagcaa caagcggagc gcggggaacc gggccgccaa 240

tgaagaggaa acgaaaaaca aacccaaatt gcgtgaccga attacaagtt ttagaaaatc 300
tactgtc 307

<210> 353
<211> 1605
<212> DNA
<213> Homo sapiens

<400> 353
atgtgggaaa gtttggaact tcctagagac ttgttgaatg gctttgacca aaagcctgat 60
agcaatatgg acaataacgt ccaggctgag gtggtctcag atggagaaga ggaacttgtt 120
gggaactgga gaagtcctca caagttgaga gagtgtctcc ccctcatcat tttcctaagg 180
aacagactta agtatgccct gacaggagat gaagtaaaga tttgcatgca ggggttcatt 240
aaggttgatg gcaaggtacg aactgatata acctaccctg ctggattcat ggatgtcatc 300
agcattgaca agacgggaga taatttccat ctgatctatg acaccaaggg tcgctttgct 360
gtacatcgta ttacacccga ggaggctaag tacaaattgg gtaaagtgag aaaaatcttt 420
gtgggcacaa aaggaatccc tcatctggtg actcatgatg ctcacagcat ccgctacctt 480
gatcccctca tcaaggagca agccccaggc cccgctcgca actggggcaa tgccagcagc 540
tttcctcgat cagatgggaa gcccacagtc ctctctctca gagcagccac cacagggagc 600
acaggactgg acttactctg cctcaacaaa ttaatgctaa aagaaggaga agaccctaaa 660
agggttgcaa ccgggatctg gggcctgctg cctccaggaa cagtgggatt agtcctaggg 720
tggtctagcc tatctagtaa aggaattaat gtgctcaccg tggtaattga tagtgattat 780
cacagagaga tattggttat gatggactgt aaaggtctgc atattcttcc ccctggatca 840
aagatagctc agttactgat tttatcatac tgggtcccca gtctctatgg aaaggaaagg 900
gggaagggaa gttttgggag cacaggagcc acaggagtat attggaatca attaatcact 960
gatcaaggac ccatgaagaa attggaaata agaattttac tggcttactg ggcacagggg 1020
cagacatttc aatcattagt gatcaaaact ggccagaaac ttggctttgg atatttaacc 1080
cctgcagcaa aaagggaaat tgaggaaata gaacaagccg tctctcaggg gcagctagat 1140
cgcattgatc cacgttattc aatccaattg ttttatcttt ctcaccaaac actcccctac 1200
agggttaata ggacagatgg cccccgggct atgcttccta gaatgggttt tttgccacat 1260
accgggacta aaacactatc tccctatagt cagttactta ctaaagtcat ctattcaggc 1320
cacaaacaat gcaatcagtc actaggttat gaccctgatg tcatcaggat tcctttaagt 1380
aaaaagcaat tcaaagcagt attgccagta tctattaatc tgcaaatagc tttctctgat 1440
tacacaggac aaatagagca catacttcct gctgataaac tcctttattt cttatctcat 1500
accctggtaa tcttacccac aaaaatagtt cactccccca tacctaatgc tttaacactg 1560
tttactgatg gttctggtaa acatggaaaa gcagcagtct ggtag 1605

<210> 354
<211> 383
<212> DNA
<213> Homo sapiens

<400> 354
cttgcgtcag gaggggtagc ataccaagca atacaagctc ttaaaaacat gggtgaaatt 60
ctgaaagctg cgggctgtga cttatctaac gtggggacaa caactgttct tttgggtgac 120
ataaatgact tcaatactgc caatgaaatc tacagacagt atttgaagag taattttcct 180
gctagagctg cttaccaaga tgctgcttta ccccaaggca tccgaattga aattgaagca 240
gtagctatgc aaggaccact gacaacggca ttactataat ggggcccagt gctgagaaaa 300
ctgcaattga taacatttta atttttacaa ttgacgtaac atcttaatta accttttaat 360
tttcacaatc gacgacaggg tgg 383

<210> 355

<211> 385
<212> DNA
<213> Homo sapiens

<400> 355
atgtgtgact tcatgaacct cagcctggcg gactgccttc tggtcaacct ggcctacaag 60
tcctccgtat aaaggctggt ggtgggagaa tgctatcgct gccctgtttc ggagacacat 120
tcccgtcagc tggctgatcc gcgctaccct gagtgagtcg gaaaacttcg aagcagctgt 180
tggcaagttg gccaagactc cccttattgc tgatgtttat tacattgttg gtggcacgtc 240
cccccgggag ggggtggtca tcacgaggaa cagagatggc ccagcagaca tttggcctct 300
agatcctttg aatggagcgt ggttccgagt tgagacaaat tacgaccact ggaagccagc 360
acccaaggaa gatgaccgga gaaca 385

<210> 356
<211> 367
<212> DNA
<213> Homo sapiens

<400> 356
aggaagccca gtacattttt agttggtcgc ggcttgggct ccgctttggg gaggggcagc 60
aggtttattc actggatctc tgaataccca ggccccctcc accatggcca gccggggtgg 120
gggccggggt cgtggccggg gccagttgac cttcaacgtg gaggccgtgg gcattgggaa 180
aggggatgct ttgcccccac ccaccctgca gccttctcca ctcttcccta ttggcggcgt 240
ctaccccggg agctaaagat ccgagtgcgg aagctacaga aggaacggat tacaattctg 300
ctccccaaga ggccccctaa gaccacagaa gataaggagg aaacaataca gaaactagag 360
gtgagga 367

<210> 357
<211> 425
<212> DNA
<213> Homo sapiens

<400> 357
ctcatccagc gtggtggaat tccatcccaa agatctacca gttctcttct gacaaagcca 60
tgccatcctg aaatgatggt cctctggggc tggaggcagg gctcgagcca ggcctctgtt 120
ggggcaggga ggtgcctgat tgagtctgct ctgacctctc actctcccct tcttctccag 180
gctgctgctc ctgctgccct gtgggctgtg ccaagtgtgc ccagggctgc atctgcaaag 240
ggacgtcaga caagtgcagc tgctgtgcct gatgccagga cagctgtgct ctcagatgta 300
aatagagcaa cctatataaa cctggatttt tttttttttt tttttggac aaccctgacc 360
cgtttgctac atcttttttt ctatgaaata tgtgaatggc aataaattca tctagcctaa 420
aaaaa 425

<210> 358
<211> 1314
<212> DNA
<213> Homo sapiens

<400> 358
atgtggctgt gggaggacca gggcggcctc ctgggccctt tctccttcct gctgctagtg 60
ctgctgctgg tgacgcggag cccggtcaat gcctgcctcc tcaccggcag cctcttcgtt 120

ctactgcgcg tcttcagctt tgagccggtg ccctcttgca gggccctgca ggtgctcaag 180
ccccgggacc gcatttctgc catcgcccac cgtggcggca gccacgacgc gcccgagaac 240
acgctggcgg ccattcggca gggttccccg atcttctcag gacggccttc ccacttcctc 300
attcgtccac aaggcacact cccggccctt gatcgctccc agtgtctagg gatctccgga 360
cttgaggttt tatatttaca gaagacagca gatatgtata tagtttctgt gcgcctgacg 420
ctaaggttac atacagccag gaacaaggca gctaagaatg gagcaacagg cgtggagttg 480
gacattgagt ttacttctga cgggattcct gtcttaatgc acgataacac agtagatagg 540
acgactgatg ggactgggcg attgtgtgat ttgacatttg aacaaattag gaagctgaat 600
cctgcagcaa accacagact caggaatgat ttccctgatg aaaagatccc taccctaagg 660
gaagctgttg cagagtgcct aaaccataac ctcacaatct tctttgatgt caaaggccat 720
gcacacaagg ctactgaggc tctaaagaaa atgtatatgg aatttcctca actgtataat 780
aatagtgtgg tctgttcttt cttgccagaa gttatctaca agatgagaca aacagatcgg 840
gatgtaataa cagcattaac tcacagacct tggagcctaa gccatacagg agatgggaaa 900
ccacgctatg atactttctg gaaacatttt atatttgtta tgatggacat tttgctcgat 960
tggagcatgc ataatatctt gtggtacctg tgtggaattt cagctttcct catgcaaaag 1020
gattttgtat ccccggccta cttgaagaag tggtcagcta aaggaatcca ggttgttggt 1080
tggactgtta atacctttga tgaaaagagt tactacgaat cccatcttgg ttccagctat 1140
atcactgaca gcatgcttac cgcttctcac ctttcaaccc catctgtggc ctttgtccag 1200
gcccacagct tagccatggc ttccctcctg catccctgcc gtgggttgct ggcctcacac 1260
ttgcagcagc tggacagtga ttttagaagg ccaccagtcc ccatagctat gtga 1314

<210> 359
<211> 1464
<212> DNA
<213> Homo sapiens

<400> 359
atggcccccg ctccgccccc cgccgcctcc ttctcgccct ccgaggtcca gcggcgcctg 60
gcggccggcg cgtgctgggt ccgccgcggg gcccgcctct acgacctctc cagcttcgtg 120
cggcaccacc cggggggcga gcagctgctg cgggccaggg cgggccagga catcagcgcc 180
gacctggacg ggccgccgca caggcactcg gccaacgcgc gccgctggct ggagcagtac 240
tacgtgggag agctccgcgg ggagcagcag acaggtgata agcatcccat gcgctctgaa 300
acccaccaca tcacagaaac agcccttgct ggcgttacca ggaccttcgc cttcctccac 360
ccggtgggct ccatggagaa cgagcctgta gcccttgagg aaactcagaa gacagatcct 420
gctatggaac cacggttcaa agtggtggat tgggacaaga acacagccag tggctgcctg 480
actccccgtg ctgaagattg gctgagcatc caccgagaca ccctcatcct cactccgatt 540
gtgtgtggca gattggggcc caccagcatc acagatgagg gcactgaggg gctccgggag 600
gaaaagatgc acgtccagga tctcacagct ggtcaaaggc acagccaggc tctaatggac 660
ctggtggact ggcgaaagcc tctcctgtgg caggtgggcc acttgggaga gaagtacgat 720
gagtgggttc accagccggt gaccaggccc atccgcctct tccactcaga cctcattgag 780
ggcctctcta agactgtctg gtacagtgtc cccatcatct gggtgcccct ggtgctgtat 840
ctcagctggt cctactaccg aacctttgcc cagggcaacg tccgactctt cacgtcattt 900
acaacagagt acacggtggc agtgcccaag tccatgttcc ccgggctctt catgctgggg 960
acattcctct ggagcctcat cgagtacctc atccaccgct tcctgttcca catgaagccc 1020
cccagcgaca gctattacct catcatgctg cacttcgtca tgcacggcca gcaccacaag 1080
gcacccttcg acggctcccg cctggtcttc ccccctgtgc cagcctccct ggtgatcggc 1140
gtcttctact tgtgcatgca gctcatcctg cccgaggcag tagggggcac tgtgtttgcg 1200
gggggcctcc tgggctacgt cctctatgac atgacccatt actacctgca ctttggctcg 1260
ccgcacaagg gctcctacct gtacagcctg aaggcccacc acgtcaagca ccactttgca 1320
catcagaagt caggagggtc acatccactt ggtggccagg tggcccttgg tgacccactt 1380
cttcctggag cgtccctgcc tagagctcag cccacaggac tgcttcaggc cgtggccaca 1440
ggtagcagcc gcaaggggaa atga 1464

<210> 360
<211> 892

<212> DNA
<213> Homo sapiens

<400> 360
tttcgtggct ccgagccgag cgcgcggagc agctggggcc ggggcgcgga tgctggaagt 60
tcacatcccg tcggtggggc ccgaggccga ggggcccagg cagagcccgg agaaaagcca 120
catggtgttc cgagtggagg tgctgtgcag tgggcgcaga cacacggtgc caaggcgcta 180
cagcgagttc cacgcgctgc acaagcggat caagaagctg tacaaagtgc ccgacttccc 240
ctcgaaacgc ctgcccaact ggaggaccag agggttggaa cagcgccggc agggcttgga 300
ggcttacatc cagggcatcc tgtacctgaa ccaggaggtg cccaaggagt tactggaatt 360
cctgagactt cggcacttcc ccacagaccc caaggctagc aactggggca ccctgaggga 420
gttcctgcct ggcgacagca gctcccagca gcaccagcgg cctgtcctga gcttccatgt 480
ggatccctat gtttgcaacc cctccccaga gtcgctgccc aacgtggtgg tgaatggtgt 540
gctccagggc ctctacagct tcagcatcag cccagataaa gcccagccaa aggcggcctg 600
tcgccctgct cctctgccac cgatgccctg atcagtccag aggcctttgg ctgcctctta 660
agaaagtcat gtgcctctgt cctatgaact ccatataagg ctgggtcctc ctttggcctg 720
gacccaggac ttaattaccc agtgcccagt tgtgccacat tcccactcaa ggctcagaac 780
ttggctcgca ttggtagctg gaggtggtag aatttgtatg ctcttagagc ccaacagcca 840
aggcagggtc aagaagataa gtaataaaag agtaagtcag ccaggatgta tt 892

<210> 361
<211> 302
<212> DNA
<213> Homo sapiens

<220>
<221> misc_feature
<222> (1)...(302)
<223> n = a,t,c or g

<400> 361
cggcgcccga aggccaggtt catgncgtca cactgaagtc ccaggtcctc ggaggtgtcg 60
cgaaggatgc agtccacaag cacccacagg ttggccgagt ccagggcgct cgcgctgggc 120
gtggcacaga ttgtcctgtg tgaacttggt ccgggtctct ggggtggagg cgctgcgggg 180
ccgcttctca aaactcttgc acaataggtt gagggacttc tctacaccag gaccanagan 240
ttattcgttt cttgccgctc ttggaaacac ccagaggaca ctggaaacat agccacaggg 300
ac 302

<210> 362
<211> 822
<212> DNA
<213> Homo sapiens

<400> 362
   ccgcctgcct tttaattcag tgttcccatg agcgccaagg ccccattatt gcccccttgc 60
   tcccactcca tgctgcttct gggtgggacc taagatggct tgggagttgt tgggttcctg 120
   cgatcagaag tctaccccac cacctcctca ggaaactgct gcctccccta agaattttcc 180
   ttgccctgga gtagggggcc agagcacttt gatttccagc catttactcc aagtcctctc 240
   cccagctacc accagtccct tactctgttc tcccccagtg aaaaagagtc tgttgatttt 300
   cctcaaaact gctttattag gaatgtacca gggattgagt taggggagtt ggacagcccc 360
   ggctcctata ggagtcctac ttctctccag catcctgtgc catcctcttg acgtaatcgc 420
   tgtacattgt gtacacagca cctgtgtgag agaaaagaaa taatgcccct tggcatcaaa 480
   cccttcaccc gtcctctggc tctcaggcgt cccaggggtt cacctctagc tgctcccttg 540
   agagtccagc ttcccgctca tctttctgcc ccaggtccca acagctgctt caagcatctg 600
   accacacaac ctggggaaac cacttatctg acagttcacg aaacactttc acatgtattg 660
   tgtatctgag aaacctcaca gcagcctgca aggtaggcag gccaggggtg gttattgtca 720
   tgttacacat caggaaactg aggctcagtg agaaagattt gtccactgtc acacagttag 780
   caagtggctg tctgcttttt aatctcaaga aaaaaagaaa tt 822

<210> 363
<211> 402
<212> DNA
<213> Homo sapiens

<400> 363
aaggtgcaca tgaatggcct gcgatatgct ctatacatta tggggcctgc ccttacgaga 60
ggctgccacc aggtgcttat tatgctatgc tgcgaagtaa tagaacggat cttgacttga 120
tgatgaagga agcttacttt ggcgaagtgg atatcatgga cgacctccct actgagaaat 180
attagcgcta acgtcacagg caggctatac aagtgtgggg tgatcaacca cacagttgac 240
ctgcgccgta tgaaaacgga aaatggcaga ataatctgct tccatcccgc cagtttggtt 300
tcattgtact gacaacctca gctggcatca tggaccatga agaagcaaga cgaaaacaca 360
caggagggaa aatcctggga ttctttttct agggatgtaa ta 402

<210> 364
<211> 411
<212> DNA
<213> Homo sapiens

<220>
<221> misc_feature
<222> (1)...(411)
<223> n = a,t,c or g

<400> 364
gcgagcacta taagacggcg gtgtttccat tcggtgatca gcactgaaca cagaggactc 60
accatggagt ttgggctgag ctgggttttc ctcgttgctc ttttaagagg tggccagtgt 120
catgagcagc tggtggagtc tgggggaggc gtggtccagc ctgggaggtc cctaagactc 180
tcctgtgtag cgnctaaatt catgttcagt aannnnnnnn nnnnnnnnnn nnnnnnnnnn 240
nnnnnnnnnn nnnnnnnnnn nnnnnnnnnn nnnnnnnnnn nnnnnnntga taaacactat 300
gcagactctg tgaagggccg attcaccatc tccagagaca attccaagaa cacgctgtat 360
ctgcaaatga acagcctgag cgccgaggac acggctgtat attactgtgc g 411

<210> 365
<211> 3589
<212> DNA
<213> Homo sapiens

<400> 365
tttcgtgcca ggaggcaggg gaggctcggc gaccctgtca ggcgctgcgg gacgccgagg 60
actggggcgg cgtgctggag atccgcggcc aaccgtgggg tgctgctgtc gggactcata 120
ggtgtcgtct cctggaagag gcctctctcc cttgtgataa cctttttcat gctgctttct 180
gcagtgtgtg taatgctgaa tttggctggt tcaattctct cttgtcagaa tgctcagcta 240
gtcaactccc tagaaggctg ccagttgatt aagtttgaca gtgtggaggt gtgtgtctgc 300
tgtgagctgc agcaccagtc gtccggctgc agcaacctcg gggagacgct gaagctgaac 360

ccgctgcagg agaactgcaa cgctgtgagg ctgaccttga aggatctcct cttcagcgtg 420
tgtgccctca acgtcctgtc cactatcgtg tgtgcgctgg ccacagccat gtgctgtatg 480
cagatggtct cctccgatgt cctgcagatg ttccttccgc agaggtcaca tcctgccaac 540
cccacctgcg tgactcctca cggcaccgtt ctccaccaga ccctggattt cgacgagttc 600
atccccccac tcccgcctcc accctattac cccccagagt acacctgcac accgtccact 660
gaggcccaga ggggcctcca cctggacttt gctccgtctc cattcggcac tttgtatgac 720
gtggccatca atagccccgg cctgctctat cctgctgagc tccccctcc gtacgaggcg 780
gtggtgggcc agccccctgc cagccaggtt acaagtatag gtcagcaggt ggccgagtcc 840
agctccgggg acccaaacac cagtgctggc ttcagcactc cagtaccagc tgacagcaca 900
agcctcctgg tgtccgaggg cactgctacg ccaggttcca gcccatcccc cgatggccct 960
gtgggcgccc cagcaccctc cgaacctgcc cttccccctg gccgcgtgtc tccagaggat 1020
cctggcatgg gctcacaggt gcagccaggt cccgggcatg tgtcccgctc caccagcgac 1080
cccaccttgt gcacatctag catggcaggc gatgcctctt cacacaggcc gtcgtgtagc 1140
caggacctgg aagcggaact gtctgaggct gtgcctggga gcgcttccat gtctcgctct 1200
gccacggctg cctgtcgggc ccagctttca ccagcggggg acccagatac ctggaaaact 1260
gaccaacggc caacaccaga gcccttccca gcgacctcca aagagcggcc acgctcttta 1320
gtggacagca aggcctatgc ggacgccagg gttttggtgg ccaagttttt ggaacactca 1380
cactgtgccc tccccaccga ggcacagcac atggtgggtg ccatgcgctt ggctgtcacc 1440
aacgaggagc gcctcgagga ggaggccgtc ttcggcgctg atgttctgga ccaggtatga 1500
aggagccaca tttctgaccg tggacaccaa cgcctggcgc cacagatgtt cttggcagaa 1560
gccatcatgc ctgacaaaga ggccttcagg cccagcttgg cggaggataa atgtgggggg 1620
gacacccagg atgatctggg ggcggtgtgg gcagggaact gttacccct ctctcgaggg 1680
gagccgagca gtgtctcttt ggggattgca tttcctgcct agcccagtaa ttcggaaaga 1740
tgactttcac gtccacagtg aactctctgg ttttatctgg gcgtgcagca ccttgaactg 1800
aggcagtgtt gcacaaatgt gaatacagca acaggcgaca cttacgtcac ctaaagattc 1860
aaagtatctc caaaatcagg ggccttccaa aggcagggag tcttccccat cccccattta 1920
aacttagtgt ttcagttggg acagaggggt tgacatcgag ttcatggaaa cattttcttc 1980
atgtgggctc gatggctcta ttcttagggc gtttcatcac ggtctgggcc tatttcctgt 2040
gaaaggaaat aggtagtgtt agcacagatg gcagcctggg cccaactttc tgtccacagg 2100
gctattgtgc aaacaaatgt gcagtgcggg cctctgccat agcccggccc cccccaggga 2160
acaggccggt gttgcggggg ttggaggaac ccaccatctc ttgggccttg agctcggcct 2220
ccctccgtgg ggtgccttcc ccccctcctc ttgggccccc tttctccagg ctcccatcct 2280
gtccctggga gcagttgtgt gatccgtaac aacaagtgcc gattcccagc tcctttgtgt 2340
ctgagctcac ttgattgagc tcagcgggtc acagttcttg caggagagag gccggtttct 2400
ctgagaagta gcccttcctg attaaagcag gtccctttga cccagagctg acctttcccc 2460
gggcatgcgg cagcagacac acctgcgcag gaggatcata aaccacttac aaacccacgc 2520
gagaagggaa gcagacaccc aacgtgctgc ttatgggaga agcagacacg cccagcttgg 2580
ggacagcctg ggggaccttg ggactttttt agcccccatc ccttgttttc ttgttggcat 2640
cagtggcagc tgctcccaac aatggccgtg gaacagcgcc atccacgaga actttgtgca 2700
gtgatggaaa tgcctgttca gggccactag cagccacagc ccctgagctg cgttgggtgg 2760
gccctgcggt gaacaaaaca gcctagaggc caaggtctcc ccagagagcc catgagcctg 2820
gcatgggggc acagggggac cagagggtgt cttccactcc tgggtgccag ggcagggggc 2880
tcacgctgct gttccctcgg ctttgtgggg cttgtccttg agctccccca attccctaat 2940
gccactgtgg ccagtgtgct ggggagcttc tgtgtggctg gaagctggga aggggtgag 3000
gttccaatcc aggtgcccag agccttcctc cagtccttta tccctgccct gggggctgcg 3060
gtgtgggact ccttcagctg tggtcggctg aaaaggcttc gattgcttta gagagctgaa 3120
aacacataag ccaggccttt tgatgacaac ctgcactttt gcaggagaga cgtactagag 3180
atgaattttt gagcacttta ttcagactat aaaacaggaa atctctaatt ttcctcaagt 3240
gggggctttt gaattccctc aaggcaagtc cctttgcagg tgaaattaat tggtggattt 3300
tcagaatggc cgccctggag ccgagctttc agccacgcct gacgccctgg cctctgaatc 3360
tccactgtct ctgaggtgcg ctggggccac accgcagggg tttcacctgc ttgggggggt 3420
ccacactagg gcttgggggt tcacacagca ttgtccctgg gctgagccct tgggtgggct 3480
agagctgcag tcctcagaca tggctgcaat tccagcgagg ccaagaccac cagccaccca 3540
gtggacttcg ggtctctggg gtgttctcct catgcagccg gggcagagg 3589

<210> 366
<211> 291
<212> DNA

<213> Homo sapiens

<400> 366
tcttcaagca atcactatgt caacagacac aggtgtttcc cttccttcat atgaggaaga 60
tcagggatca aaactcattc gaaaagctaa agaggcacca ttcgtacccg ttgtttttgg 120
agacagtccc gctctgtcgc ccaggctgga gtgcagtggc aggatctcgg ctcactgcag 180
cctctgcctc ctgggttcaa gtgattctcc tacctcagct tcctgagtag ctgggactac 240
tcccgccacc acaccaggct gatttaatgt gtttttagta gagacggggt t 291

<210> 367
<211> 497
<212> DNA
<213> Homo sapiens

<400> 367
gagtacgtga ctgtacggag gattaaccta gaagcttgtt ccaatgagat ggtaacattc 60
ttgcagggcg agctgcatgt ctccaaactc ttcaaccatc ccaatatcgt gccatatcga 120
gccactttta ttgcagacaa tgagctgtgg gttgtcacat cattcatggc atacggttct 180
gcaaaagatc tcatctgtac acacttcatg gatggcatga atgagctggc gattgcttac 240
atcctgcagg gggtgctgaa ggccctcgac tacatccacc acatgggata tgtacacagg 300
agtgtcaaag ccagccacat cctgatctct gtggatggga aggtctacct gtctggtttg 360
cgcagcaacc tcagctgccc tggtgctttc tccaagggcc atcagtgtct tgcctagctt 420
gagggcttaa gtccttatgc tgtgttagtt tcgttgtcag aacaaattaa aattttcaga 480
gacgctgaaa aaaaaaa 497

<210> 368
<211> 1266
<212> DNA
<213> Homo sapiens

<400> 368
atgcctccag gcgtcttctc tggcttgacc ctcctcaggc taaacttgag gagtaaccac 60
ttcacctcct tgccagtgag tggagttttg gaccagctga agtcactcat ccaaatcgac 120
ctgcatgaca atccttggga ttgtacctgt gacattgtgg gcatgaagct gtgggtggag 180
cagctcaaag tgggcgtcct agtggacgag gtgatctgta aggcgcccaa aaaattcgct 240
gagaccgaca tgcgctccat taagtcggag ctgctgtgcc ctgactattc agatgtagta 300
gtttccacgc ccacaccctc ctctatccag gtccctgcga ggaccagcgc cgtgactcct 360
gcggtccggc ccaatagcac cggggccccc gcgagcttgg gcgcaggcgg aggggcgtcg 420
tcggtgccct tgtctgtgtt aattctcagc ctcctgctgg ttttcatcat gtccgtcttc 480
gtggccgccg ggctcttcgt gctgaaaccc atcgattatt atggggaaat ctgtgacaat 540
gcatgtctct gtgaggaaaa ggacggcatt ttaactgtga gctgtgaaaa ccgggggatc 600
atcagtctct ctgaaattag ccctccccgt ttcccaatct accacctctt gttgtccgga 660
aaccttttga accgtctcta tcccaatgag tttgtcaatt acactggggc ttcaattttg 720
catctaggta gcaatgttat ccaggacatt gagaccgggg ctttccatgg gctacggggt 780
ttgaggagat tgcatctaaa caataataaa ctggaacttc tgcgagatga taccttcctt 840
ggcttggaga acctccagta cctacaggtc gattacaact acatcagcgt cattgaaccc 900
aatgcttttg ggaaactgca tttgttgcag gtgcttatcc tcaatgacaa tcttttgtcc 960
agtttaccca acaatctttt ccgttttgtg cccttattgc acttggacct cccgcggaac 1020
cggctgaaac ttctgcccta cgtggggctc ttgcagcacg gggtcctttt agagtcgcac 1080
aaaacagggc attcaagccc tcactttcgg gacagcaggc tacgggaacc ggtgctctac 1140
agccccccga gtgctgtctt tgtagaaccc aaccggaacg aatatctgga gttaaaagca 1200
aaactaaacg ttgagccgga ctacctcgaa gtgctggaaa aacagaccac gtttagccag 1260
ttctaa 1266

<210> 369
<211> 1207
<212> DNA
<213> Homo sapiens

<400> 369
tgaggatctg tgaccccagc cataaggagc cttgccctcc ttgctgccat tctcctggtg 60
gccctgcagg ctcgggcgga gccactccag gcaattgctg atgaggctac agcccaggag 120
cagcctggag cagatgatca ggaagtggtt gattcctttg catgggatga aagagctcct 180
cttcaggttt cagggaagtc ctctcctgtt tgtgcacggc tgctcttgct acaggagacc 240
cgggacagag gactgctgtt tgccctccct cttcactctg cctaccttga ggatctgctc 300
aggcagagcc acttcaggca agagctgatg aagctgcagc ccaggagcag cctggagcag 360
atgatcagga aatggctcat gcctttacat ggcatgaaag tgccgctctt ccgctttcag 420
cctgataaaa tcattgtcct ctccaccctg attcctacag gagactactc accccataac 480
ctcaaaaacc tcttcatgag gatggtgacc cccagcccat gaggaccctc gccactcctt 540
gctgccaatt ctcccctggt gggccctgca ggccccaggc ttgagccgcg tccccaggaa 600
agagctgatg agcgttgctg cagcccccgg agcagattgc agcgggacat ccccagaagt 660
gggttgtttc cccttgcatg ggaccaaaag cttggctccc aaagcatccc aggctcaagg 720
gaaaaccatg gactgctatt gcagaatacc agcgtgcatt gcaggaggaa cgtcgcttat 780
ggaacctgca tcttaccagg ggaagacttt gggcattcct gctgctgagc ttgcagaaaa 840
agaaaaatga gctccaaatt tgactttgag agctacaggg aattgcctat tactcctgta 900
ccttcttgtt aaatttcctt tcctaatctc aaataaatgc cttgttacaa aggccctatc 960
tcaaaaaaaa agccacccgg cccgaaattc caaaattttg aatcggaaaa cctggaaaac 1020
cccggagggt ccttttaagg gggcattggg ccccatttaa atttttttaa tttaaaacac 1080
ggttgggcct ttttttaggg accaaacccc cttaaagtca attttgggca gggaccctcg 1140
gtttaaaaat ttttaagggc caaaataggc gccatttttt ttggcccaaa attttaggaa 1200
aaaaata 1207

<210> 370
<211> 497
<212> DNA
<213> Homo sapiens

<220>
<221> misc_feature
<222> (1)...(497)
<223> n = a,t,c or g

<400> 370
ggcacgagcc ttaatctagc agtaatttat caaaaaccca tatgataaga gaattatttt 60
ataatagtgt tggtaaagaa aatctaaaat agaaaaaaca tgccaacaat tgattggcta 120
atgaggtggg tgatgccaga tgcaagactg ctgggcctac cacaggagca agagagaagc 180
catgcattcc atctcaaggc atctgtgagc ccatgtagac accatgagca aagctcaccc 240
tcctgagttg aaaaaattta tagacaagaa gttatcattg aaattaaatg gtggcagaca 300
tgtccaagga atattgtggg gatttgatcc cttaatgaat cttgtgatag ataagtgtgt 360
ggagatggca accagcgggc aacagaagaa tattggaatg gtggtgatac gaagaaatag 420
tatcatcatg ttagaaacct tggaacaaga ataaataatg gctggtcagc agagaaattn 480
cacgtcccct cttcaaa 497

<210> 371
<211> 2004

<212> DNA
<213> Homo sapiens

<220>
<221> misc_feature
<222> (1)...(2004)
<223> n = a,t,c or g

<400> 371
agggattnnn nnatctggag ngactggaga actgtcncgg aannccnggg acgacccacg 60
cggacggcca accactcggg gcgacctcgg gtgcctcgct gaagatacag tcaagctccc 120
aaccgctcca gagcggccag gtgcttgcct ctgctacacc cactccatct gcacccccca 180
cctcccagca agagcttcag gccaaaatcc tcagcctctt caatagtggc acagtgacgg 240
ccaatagcaa gctctgcatc cccctcggtt gctgccggaa acaccccaaa ccagaatttt 300
tccacaagca agcaaacaag ccaagcctca acaaagatca caggcttctg gcaatcagcc 360
tccaagcatt ttgggacagg gaggatctgc tcagaacatg gggccccaga cctggggctc 420
cttaccaagg gctttttggc cagccttcca gtcgcctggc acctgctagc aacatgacta 480
gccagaggcc tgtgtctttc acaggtatca actttgacaa tccaagtgta cagaaggctc 540
tggataccct gatccagagt ggccctgctc tctcccacct ggttagccag accacagcac 600
agatggggca gccacaggcc cccatgggat cttaccagag gcattactga agctaaatct 660
ttcaactctc cccagtcccc tcatcccctg gcctcctccc acttacttgt tctaaataga 720
gctgtttgga ggatgttctc tgcgctccca ggccggcatc gagtgtcatc aatttctacc 780
acctgctctc tcttctgccc aaggctgtgt tgcttattcc ttacaaagtt tatactgcat 840
ttggggctgt atcttttttt gttttttgtt ttgtagaaaa taaatatctc cgggggcagt 900
acaggtgtct gggcttgtat ttgatggggt ttctccggtc cctgtttcta ctggatttgg 960
agccaggccc agctagccaa gtttggaatg gcatttgtca tgtcagtagc caccaccttt 1020
gttcattgtg aacctaccaa ggctttccag cttcatacac attgaccaga gctcaagctc 1080
ctgcctgcaa ctcctgccta gagttgaaga aaagcaaact agccttggca ggcacagtgt 1140
catcataccc tcaccccata tgtttggggt ctgcttgagg attcataaat cagccactct 1200
ggattgttga ggaatggcca tggcagccac agaaaaaaga atttttctct ctgagccaag 1260
gttgtttttt tgtttttttc tcttttcttt tttgttttca tttcattggg aagatctcca 1320
atgggactgg aacagctcca gttcaggcag gcagtttacc acaaaactgt ggaatctggg 1380
gccccaccac tctttccctg tttaaccagt ttctgttcag gcatccccct ctccagcaac 1440
actttcatga agttggttct gagactctgg ccgcgaacac ccgtttcttc agtgatttgt 1500
tttgggcttt tggctcaaaa ccccaggctc ttgtttttgt ctagactctt attctgtttc 1560
ctgagcagca ggaggtaggg accactttga tgtcagactt ctggtagctg gacatgttct 1620
cgagatgggt ggctgttccc cgactttttg tacccaagag tgaaattgtt nagaaaggag 1680
ggtttcctgg ctgtggttcc taaatggagc cccaggaaag ttgccttctc ccccagggtt 1740
ttgtgctcag taagaaccct gtggatcaca gtcttgaagg tcctctagca ggggtgaggg 1800
agagcagcga cttcagctga gtccctgccg agtggttaag caaacaatgg tttcaaaatt 1860
caaggtcccc aaatggcagc attttatgtt ctgacctgtt tgtgttatat agtggttttt 1920
ttttttcctc tttggaactc ttgtgttgtt aataaaatga aatgattact ttttaattaa 1980
aatgaaaaaa gtaaaaaaaa aaaa 2004

<210> 372
<211> 446
<212> DNA
<213> Homo sapiens

<400> 372
ccctgttctt ctttacctaa ctctatagca gtaattggta catttcttcc atcaaagttt 60
catattttta gcaaagtatc aagcctttcc gtacggaaag ctgcatccac aagattggag 120
ggaagcaggt ttctgttgta aaagaaagca ttctgtcagt attcttttaa aacaattttt 180
tagagatgag gtccctgttg cccaggctgg agtgcagtgg tgtgattgca gctcactgca 240
gccttgaact tttgggctca agtgatcctc ctgcctcagc ctcccaaagt gttgggacta 300
caggcgtgag ccatggtacc tgtcttcatc agtattctta aaattcagtg tgcaaagtat 360

ctgctttgag atgtccactt ctaaaaccct aacacagtgt tctctctcca cccctagctg 420
acactgcacc ttttgcttct cgtgcc 446

<210> 373
<211> 1816
<212> DNA
<213> Homo sapiens

<400> 373
cttctctgcc agaagatacc atttcaactt taacacagca tgatcgaaac atacaaccaa 60
acttctcccc gatctgcggc cactggactg cccatcagca tgaaaatttt tatgtattta 120
cttactgttt ttcttatcac ccagatgatt gggtcagcac tttttgctgt gtatcttcat 180
agaaggttgg acaagataga agatgaaagg aatcttcatg aagattttgt attcatgaaa 240
acgatacaga gatgcaacac aggagaaaga tccttatcct tactgaactg tgaggagatt 300
aaaagccagt ttgaaggctt tgtgaaggat ataatgttaa acaaagagga gacgaagaaa 360
gaaaacagct ttgaaatgca aaaaggtgat cagaatcctc aaattgcggc acatgtcata 420
agtgaggcca gcagtaaaac aacatctgtg ttacagtggg ctgaaaaagg atactacacc 480
atgagcaaca acttggtaac cctggaaaat gggaaacagc tgaccgttaa aagacaagga 540
ctctattata tctatgccca agtcaccttc tgttccaatc gggaagcttc gagtcaagct 600
ccatttatag ccagcctctg cctaaagtcc cccggtagat tcgagagaat cttactcaga 660
gctgcaaata cccacagttc cgccaaacct tgcgggcaac aatccattca cttgggagga 720
gtatttgaat tgcaaccagg tgcttcggtg tttgtcaatg tgactgatcc aagccaagtg 780
agccatggca ctggcttcac gtcctttggc ttactcaaac tctgaacagt gtcaccttgc 840
aggctgtggt ggagctgacg ctgggagtct tcataataca gcacagcggt taagcccacc 900
ccctgttaac tgcctattta taaccctagg atcctcctta tggagaacta tttattatac 960
actccaaggc atgtagaact gtaataagtg aattacaggt cacatgaaac caaaacgggc 1020
cctgctccat aagagcttat atatctgaag cagcaacccc actgatgcag acatccagag 1080
agtcctatga aaagacaagg ccattatgca caggttgaat tctgagtaaa cagcagataa 1140
cttgccaagt tcagttttgt ttctttgcgt gcagtgtctt tccatggata atgcatttga 1200
tttatcagtg aagatgcaga agggaaatgg ggagcctcag ctcacattca gttatggttg 1260
actctgggtt cctatggcct tgttggaggg ggccaggctc tagaacgtct aacacagtgg 1320
agaaccgaaa cccccccccc cccccccgcc accctctcgg acagttattc attctctttc 1380
aatctctctc tctccatctc tctctttcag tctctctctc tcaacctctt tcttccaatc 1440
tctctttctc aatctctctg tttccctttg tcagtctctt ccctccccca gtctctcttc 1500
tcaatccccc tttctaacac acacacacac acacacacac acacacacac acacacacac 1560
acacacacac acacacacac agagtcaggc cgttgctagt cagttctctt ctttccaccc 1620
tgtccctatc tctaccacta tagatgaggg tgaggagtag ggagtgcagc cctgagcctg 1680
cccactcctc attacgaaat gactgtattt aaaggaaatc tattgtatct acctgcagtc 1740
tccattgttt ccagagtgaa cttgtaatta tcttgttatt tattttttga ataataaaga 1800
cctcttaaca ttaaaa 1816

<210> 374
<211> 1104
<212> DNA
<213> Homo sapiens

<400> 374
gacagtttac tatatttcta cataatattg tgctttaatg aatcttcata atgatgagtt 60
tagtcatcaa gcaaagttta agtcataatc tcccctctag gaaaaatttc taagttcata 120
gcctacgtgt gtgtatgtgt gcgtgttgaa acagaagtgt gtgtgtgtgt gtgcatgtgt 180
ggcgcgtgct gcctgcacgg cgtgccagtt ttctgtgttt gtgtgtttga aaaccgtgtg 240
aggatttaac tgtggggttt tcccctgtat tcaagtatac gctttttttt tttggcgcat 300
tgggtcaaac ggttgttatt attagcctcc taactttgca ctgagtgtcc tcgcccttcc 360
tttagcttaa tcctatacat tacaggagga ttcctaggac aggggtgggc gacaattcat 420

gtgtaaatgt ttaactcaag ggacctctgt gactgcgttt aaaaaaggta cagtgtatat 480
ctctggagaa ataacatgta taccttacct ttagcagcct ttttattgtg gacctaatgc 540
caagaaaatt attccccaga gtattgcacc catttttccc atttgggaat ataaagttaa 600
agagaaaaaa aaaaaaaaac tgttgttgaa ctgtggccca tagatacttg taaagaatgg 660
atggttccgt ggaaggaaca gaaacaagca cttgggcgtg ggttgtgact gcttttgggg 720
gagcctgggg tttgggctcc cacctgttta cagacctttt tttctccttc agactttaaa 780
attttaaaaa tttaaaaagg aattaaaaaa aaaaaaaaag acttccgccg aaaagaaacc 840
tattttcaaa aggcatatgc gttcttttaa agctttggga cggaacaggg ttgttccctc 900
ggccatcggc ggggactttg tcgatttgct ttcgcccctt cgtcaaggtg acaggcgttg 960
tggccactgt cttgcacatg ccggacgcgc tctcaggaaa gccagggctc ccgagggcag 1020
ctccacacca tttcatgtat ttttaaaccc aactcctagc actgaagatg ttatttaaaa 1080
aaaaaaaaaa caaaacaaaa caaa 1104

<210> 375
<211> 840
<212> DNA
<213> Homo sapiens

<400> 375
agcggagccc tttattggaa tggcttccaa ccctgtgttt ttataatggt aaaggactag 60
aacagataga aagagataac agctttcata atgtggcaga agctacgttt acactcaagc 120
tgattcaatc actgattgca agtggaatag caggctctat gattggtgtg ataacattat 180
acaaatccca gatgtacaag ctttgtcatt tactcagtgc tgtggacttt caccatcctg 240
atattaaaac tgtgcaggtg tccacagtag atgctttcaa ggaagctgaa aaggagatca 300
ttattctgtc ctgtgtaagg acaagacaag taggattcat tgattcagaa aaaagaatga 360
atgttgcatt gactagagga aagaggcatt tgttgattgt gggaaattta gcctgtttga 420
ggaaaaatca actttgggga cgagtgatcc aacactgcga aggaagggaa gatggattgc 480
aacatgcaaa ccagtatgaa ccacagctga accatctcct taaagattat tttgaaaaac 540
aagtggaaga aaaacagaag aaaaagagtg aaaaagagaa atctaaagat aaatctcatt 600
cataaaaaga catggtgtaa atattttgta tttatgtaaa ttcagactca ttttacatga 660
tatatttttt atatttttat tactctaaac cctcttatta aaaatatgat atttaaataa 720
catagtaaac acatgtaaaa attttgttct tcaaaaaagt gtacacaagg tagtataaaa 780
tcctactcat caaaataagc ttttttctac acaaaaacaa acaaaacaca acaacaaaac 840

<210> 376
<211> 455
<212> DNA
<213> Homo sapiens

<400> 376
ggaccaccac cgcgcttggg ctgttgacca tccccatcat tatccacccc attgacaggt 60
cggtggattt cctcctggac tccagcctgc gcaagctcta cccaacagtg gggaagccca 120
gctcctcctg atcatactct ggtacctggc ctgtgcatcg gcctcctgct tcatgtcaac 180
ctcctactcc tgccagggaa tgtggacacc tggctccctg gtgtccaaag accctggcac 240
ctgggtgggt ttgagctgga cagaagctta gagacaaagg cttcaagaag cagtggctgc 300
agggagtcac agaagggcag gacctgaacg ctgtctgctt ccctggaatc caagatgctg 360
agtggaagtg gaccctgggt gggcccggcc ctgtcttttt caggacagat tctctggcca 420
ggcccttccc tgacccaata aatcctgaaa aggtt 455

<210> 377
<211> 501
<212> DNA

<213> Homo sapiens

<400> 377
tgccttcaga gttcgttgtg ccgagctctg gagatcctgc ggacaaggtc ctgcagatgc 60
ttcttcaccg tgtagcccag ctccttggtc cgcccctcca gcagggcgtg cttctcgcgg 120
ctgcggctca cacgctcgcc gtcgcccacg ctctctgcgt gcctcagctt ctcgtgcgca 180
atctccagct gcttctggta gtggttgggc ttccccaatt tggcctgcaa gttcttgaag 240
ctcttcgcga aacgcttcag cttcttgtcc gtgaagttgg cggactgcgc caggtcccac 300
aggtcaatca ccctgggctc ctcgaactca gcctcagtgc tgtagccctg gtggctgacc 360
ctgcgcaggc ggtccaggcc ctggttgatg ctgcgcagct tctccttcag ctccgtgtgc 420
ctgctgtgca ggacgctgca cttgatcttc acatcaagct gacagcacgt aagaggccca 480
tgccatgagc aatccccgag g 501

<210> 378
<211> 1867
<212> DNA
<213> Homo sapiens

<220>
<221> misc_feature
<222> (1)...(1867)
<223> n = a,t,c or g

<400> 378
ccttcctcac cccgtcccct gagtcccctg accagtggtc cagctcgtcc ccgcattcca 60
acgtctccga ctggtccgag ggcgtctcca gccctcccac cagcatgcag tcccagatcg 120
cccgcattcc ggaggccttc aagtaaacgg cgcgccccac gagaccccgg cttcctttcc 180
caagccttcg ggcgtctgtg tgcgctctgt ggatgccagg gccgaccaga ggagcctttt 240
taaaacacat gtttttatac aaaataagaa caaggatttt aatttttttt agtatttatt 300
tatggacttt tattttaacc cagaaacact gcctttttat ttatatgtac tgttttatct 360
ggccccaggt agaaactttt atctattctg agaaaacaag caagttctga gagccagggt 420
tttcctacgt aggatgaaaa gattcttctg tgtttataaa atataaacaa agattcatga 480
tttataaatg ccatttattt attgattcct cttttcaaaa tccaaaaaga aatgatgttg 540
ggagaaggga agttgaacga gcatagtcca aaaagctcct gggggcgtcc ngggccgcgc 600
ccttttcccc cgacgcccac cccaaccccc agcccagccc cggcccgctt ccaccagcat 660
caacctgccc tgtttaggga gaagctggca ttccagaggg caaacgggag ggcaaagctg 720
gcttcacctt tccggcacgg cggatttaat tttgcatctg aaaattaggg aaacaagttg 780
aagcatatgg ggttagatgt tgccatgtgt tttagatggg ttctttgcaa gcatgcttgt 840
ggaaaatgtg tttttcggag tgttgttatt gccaagagtt gcacccatgg gtaccaatca 900
tggaatcttt tgtttttcag gtttcagtat ttatgtagtt gttcgttggg ttatacaagt 960
tcttggtccc tccagaacca ccccggcccc ctgtccgttc ttgaaatgta ggcatcatgc 1020
atgtcaaaca tgagatgtgt ggactgtggc acttgcctgg gtcacacacg gaggcatcct 1080
acccttttct ggggaaagac actgcctggg ctgaccccgg tggcggcccc agcacctcag 1140
cctgcacagt gtcccccagg ttccgaagaa gatgctccag caacacagcc tgggccccag 1200
ctcgcgggac ccgacccccc gtggggctcc cgtgttttgt aggagacttg ccagagccgg 1260
gcacattgag ctgtgcaacg cccggggctg cgtcctttgg tccttgtccc cgcagccctg 1320
gcagggggca tgcggtcggg caggggctgg agggaggcgg gggctgccct tgggcccacc 1380
ccctcctagt ttgggaggag cagatttttg ccaataccaa gtattagcct atggcagaaa 1440
aaatgtctgt aaatatgtct ttaaaaagtg gcattttgtt taaaaaatct taatgaatgg 1500
agtctgttgt gtgtcatgcc agtgagggac gtccagactt ggctcagctc ggggagcctt 1560
agccggccca tgcactgggg aacgctccgc tgccgtgccg cctgcactcc tcagggcagc 1620
ctccccccgg tctctacggg ggtccgccgt ggtgcccatc cccagggggc atgacccaga 1680
tgcgtcccca agatgttgat ttttactgtg ttttataaaa tagagtgtag tttacagaaa 1740
aagactttaa aagtgatcta catgaaggaa ctgtagatga tagtaatata atcatctttt 1800
tttgttaact gatttgcaat aaaaatgata ctgatggtga tctggcttcc aaaaaaaaaa 1860
aaaaaaa 1867

<210> 379
<211> 1935
<212> DNA
<213> Homo sapiens

<400> 379
gggtcggaac aagcatcttc cgtttcgagc cacgcgtccg gagaatttag attcacgtca 60
gaagttccca ttcgatttga ttatcatggc aaacatgtat caatggatca ggggacgcta 120
gctgggattt tgattggtct ggctcagtta aactgctctg aactaaagct caagaggctt 180
tcctatcgac atggtttact aggcgttgac aaattattct catatgcaat cactgagtgg 240
cttaatgaca ttaagaagaa ccagctacca cgaatcctgg gaggtgttgg acctatgcat 300
tcactagtac aattagtaca aggcctaaag gacttggtct ggctcccaat agagcagtac 360
cggaaggatg gccgcattgt cagagggttt cagagaggcg ctgcttcctt tggtacctcg 420
acagcgatgg ctgctctaga actcacaaac agaatggttc aaaccataca ggcagctgca 480
gagactgctt atgatatggt gtctcctggt accctttcta tcgagcccaa gaagaccaaa 540
aggtttcctc atcaccggtt agcccaccag ccagtagacc tgagggaagg tgtggccaag 600
gcctacagtg ttgtgaaaga gggaatcaca gacacggctc agaccattta tgaaactgcg 660
gctcgagaac acgagagcag aggggtgact ggtgccgtgg gcgaggttct gcgccagatt 720
cctccggcag tggtgaaacc tctgattgtt gccacagaag caacgtcaaa cgtgctgggt 780
ggcatgagaa accaaattag gccagatgtc cggcaagacg agtcacagaa atggcgccac 840
ggggatgact gatggcttgg aacttgacag tgtgaagata aggagagtgg aacccaggag 900
ctcagagtcc tgacagcagc ttcagaggaa gctcgtttaa ttttattgtg ctcatctcag 960
gaacaaaagc attttttagt taaataattt aacatcaaaa caacatgcaa ccaaaaactt 1020
ctgacattta tagttgatac ttgcctatag aaatgtttgg tggctggtgt caaaggtcct 1080
taaagcattt gctgccaagt tagtggaagg ctcacttttg ttaagatgac tgtaattctc 1140
cttgttaccg acgagagatc attggaagct gccttctaac actttgtgta gctctgtgga 1200
gttggatttt cttaaggttt aaaaagaatc acagcttcgg aacttttaac tgaaaatgag 1260
agacagaagc cacaggggaa gcaaagcaaa taggattttc aatacaaata tcagtgtgga 1320
aaaataacct attctgttga atttagtgtt catgcacttg agaaccacca ttatttccat 1380
ttactccgaa aatccttctg tgggggtttg agaaagtgaa tgttgcagac atgttctgtt 1440
gtgttgcact ttatcctgtg tttatgtgta tgtgttttta gattaattca agttgtgtgc 1500
tatatttctt gtataattta caaagttaca caaaatatta aagagcagta aacattgtct 1560
gaaagttttt gggcaaaggg aaggtaactt ccaatgtaat taggcttccc tttaaggagt 1620
accgggaaaa tgccttctgt aatggaagtg gggcccctgt aattgtttat attttcagtt 1680
ttaagcaggt atagtgcagg cttgttagga atgtgtggaa gggaagattg gaagtgattt 1740
ttcctctttt aaaagtaaac aaaattcttc aaatatgccc tagttaacta tttcagcata 1800
cccattttta cttgggttaa ccagtgtaca ttttggataa ccctatccag ggaatggaat 1860
taaagtattt tttattttaa agggtggata ttgttttatg gcttcccctg gaatattttg 1920
gcttttcctt agggg 1935

<210> 380
<211> 329
<212> DNA
<213> Homo sapiens

<400> 380
ccgtctcacc ccagctcctt tggctccgca ccccacccac acctgctgcc caccaccccg 60
gcagcacctt tccctgccca ggcttcagag tgccctgttg ctgctgccac tgccccccac 120
actccagggc catgtcagag ctcccatcta ccctccacca gcatgccgct cctgaagatg 180
cccccaccat tctcggggtg cagccacccc tgcatcgggc actgtggtgg gcactgcagt 240
gggccttctc ctcctaccct ctagctgtga ggctgtccct aactctgaca ggtatgggcg 300
tcggatgagg gtcaatgtat cctcccacg 329

<210> 381
<211> 1417
<212> DNA
<213> Homo sapiens

<400> 381
ctgaactcaa aacttttagc tgttcattgc ttcatgatgg agatccaact taccagcgtt 60
tatttttgga ctgccttgta cattcattgc gggagcttca tacaggagat gttatgattt 120
tgcctgtact ttcttgcttc acaagattta tggctggttt gatctttgta ctccacagtt 180
gttttagatt catcactttt gtttgtccca catcctctga tcccctgagg acctgcgcag 240
tcctgctatg tgttggttat caggaccttc caaatccagt tttccgatat ttgcagagtg 300
tgaatgaatt gttgagcact ttgctcaact ctgactcacc ccagcaggtt ttacagtttg 360
tgcccaatgg gaggtactcc ttaaggcggc cctgcttgat tttttgtggg atttgaatgc 420
tgccattgct aaaaggcatt tgcatttcat tattcaaaga gagagagaag aaattatcaa 480
cagccttcag ttacaaaact gaacatatgc tttctgagat tcaactttat gatttcttat 540
aatttgccca gtatttgcat cctgttgctc tattaattta aaaacctttt atttggggga 600
aaggccaaca atttgcatca ttcaaagtct cattaattct ggaaaaccat ccattctgat 660
ctctagggta tatacaccca cagggcatag agctcttcca cgtgggtgga atctatgcaa 720
tgatagatat tcacactcta aatatgaggg tgtgtgtatg tgtatggggt ggccacagcc 780
atgcttacct atgccattta gttggtctta cttaatctgc ttaagatttg catctgtgta 840
cctttgttca gattagtttt tttttttcca gccgatttcc tcttagtggc taatgctgtt 900
agtgaatttt ccaactaatt tcctctcatt ggttaatgtt gttaatgaat tgagagaggt 960
aattgaggaa aggaaatgag taaatcactg ttcagcaaca ctgatttccg ttaacacatc 1020
agttatgaat ttcagggaat tcatctcgcc agattcttga taacatgcca ttcattgccc 1080
ttaggtgatt gaccctattt tcttacatgg ctcaaataaa actagtatgc tgttgtatga 1140
atcttttact gaccacacca tccaactata aagatataac gggacagctt taaaccaaag 1200
atcatgttta gaacaatgaa aaattatttg ttgtatctaa tacacgcctg tattgtgaaa 1260
agcttcattt agcaatgatg taataatttt taacttccag gaaataatct gtgaatggaa 1320
agatttttta acactctgag aatggggcta gcctctggct gggcacacct gaaggtgtct 1380
gaacatagtg aatactaaag aaaaacgctt aaaactt 1417

<210> 382
<211> 400
<212> DNA
<213> Homo sapiens

<400> 382
agctcatcta ctgaattttg acctgctccg ccgcgacgtg ttgtactaca agggccggct 60
ggacatggac ggcctggagg tggtggacct gaaggacggg aaggacagag acctccatgt 120
gagcatcagg aacgccttcc ggctgcaccg tggcgccaca ggggacagcc acctgctgtg 180
caccaggaag cccgagcaga agcagcgctg gatcaaggcc tttgccaggg agagggagca 240
ggtgcagctg gaccaggaga caggcttctc atcactgaac tgcagaggaa gcaggccatg 300
ctgaatgcca gcaagcactg atggccggcc ctgctacctg acgcgccaga agcacccagc 360
cctgcccagc aaccggaccc agcatcagga ctggtgctgg 400

<210> 383
<211> 337
<212> DNA
<213> Homo sapiens

<400> 383

ggcctgaaag ggcggggcct taaacattat ccaatcaggg atgctgggct gggaacgtcc 60
aatcaggcag gcagctggag gggacaggtc ggcttccggg atgtggtcgg tcctttgtct 120
ctcgctgcag ccggaggtca aggtctcgtc cttactgctg tgtgtcctct gctcctagag 180
tcccagcctc tgtggcccta tgacctgcag ggatcattga catttaggga tgtggccata 240
gaattctctc tggaggagtg gcaatgcctg gacactgcac agcagaattt atatagaaat 300
gtgatgttag agaactacag aaacctggtc ttcctgg 337

<210> 384
<211> 556
<212> DNA
<213> Homo sapiens

<400> 384
cctattgagg ccgtcggacc ggcccggatt cccggtcgac cccgcgtccg cccacgcgtc 60
cgcccacgcg tccgcccacg cgtccggtga ggggaggcag aaatggagga acgggcgttc 120
gtcaacccct tcccggacta cgaggccgcc gccggggcgc tgctcgcctc cggagcggcc 180
gaagagacag gctgtgttcg tcccccggcg accacggatg agcccggcct cccttttcat 240
caggacggga agatcattca taatttcata agacggatcc agaccaaaat taaagatctt 300
ctgcagcaaa tggaagaagg gctgaagacg gctgatcccc atgactgctc tgcttatact 360
ggctggacag gcatagccct tttgtacctg cagttgtacc gggtcacatg tgaccaaacc 420
tacctggctc cgatcccttg attaccgaaa aaaaaccact tcggaaattg aatggccgca 480
gggtcacctt cctctgtggg gatgctggcc ccctggctgt tggagctgtg atttatcaca 540
aactcagaag tgactg 556

<210> 385
<211> 423
<212> DNA
<213> Homo sapiens

<400> 385
acgcgtccga gaagatgttc ctatgtcaac tgaggaacag tataggatcc gtcaggtgaa 60
catttactat tatctagaag atgacagcat gtctgtcata gagcctgttg tagaaaattc 120
tggaatcctt caaggcaagt taataaaacg ccagcggcta gccaagaatg accggggtga 180
ccattaccat tggaaagacc taaatcgagg aataaacatc acaatttatg gcaaaacttt 240
ccgcgttggt gactgtgacc aattcacaca ggtattttta gaaagccaag gaattgagtt 300
aaatccacca gagaagatgg ctcttgatcc ttacactgaa ctccgaaaac agcctcttcg 360
taagtatgtc accccatcag actttgatca actcaagcaa tttctcacct ttgacgaaca 420
ggg 423

<210> 386
<211> 790
<212> DNA
<213> Homo sapiens

<400> 386
ctgaagacct gctacagttc tctgccaata ttcctgctct ttacaatagt aaacagcttg 60
aagacttttt caagggtgga ataattaatg atagttctga attaattggt cctgctgaag 120
ctcactcaga ttccctcatt gatacctttc ctgagtgtag tacggaaggc ttctccagtg 180
acagtgatct ggtatctctt actgttgatg tggattctct tgctgagtta gatgatggaa 240
tggcttccaa tcaaaattct cccattagaa cttttggtct caatctttct tcggattctt 300
cagcactagg ggctgttgct tctgacagtg aacagagcaa aacagaagaa gaacggggaa 360

gtcgtagcct ctttcctggc agtttaaagc cgaagcttgg caagagagat tatttggaga 420
aagcaggaga attaataaag ctggctttaa aaaaggaaga agaagacgac tatgaagctg 480
cttctgattt ttataggaag ggagttgatt tactcctaga aggtgttcaa ggagagtcaa 540
gccctacccg tcgagaagct gtgaagagaa gaacagccga gtacctcatg cgggcagaaa 600
gtatctctag tctttatggg aaacctcagc ttgatgatgt atcttatgtg tgtctccaac 660
ttctgatggc actcctactc atctgactcg gcccccccca ctcgtgccgg ctccctgagc 720
cccaatcgct agacacccgc tcctgttctt cctgcaactc acccgtctgc tctgtgcgtc 780
cgccgtctcg 790

<210> 387
<211> 462
<212> DNA
<213> Homo sapiens

<400> 387
ctgtccgtct ggcctgcccc gcggttccaa ccacagtggt gaagcagcgc ttgcagatgt 60
acaactcgca gcaccggtca gcaatcagct gcatccggac ggtgtggagg accgaggggt 120
tgggggcctt ctaccggagc tacaccacgc agctgaccat gaacatcccc ttccagtcca 180
tccacttcat ttttttttgc agggtgctgc ctatgggccc tctgctcccc aatgccttag 240
agagaggagg ggagccacgg ccgctcaccg gaaggctgtg tgcggggaca tccgaggtgg 300
tggtggacag gaaggacttg ggaaggggag cgagaaattg ctttttctct tcctccttgg 360
gcagaatgta gcttttctgc ttcactgtgg cagcctcctc cctggatcct tagatcccag 420
aggagggaag aaaatttgca gtgactgaaa acagtaaaaa aa 462

<210> 388
<211> 1827
<212> DNA
<213> Homo sapiens

<400> 388
cccggctcga gcggcgcggc agagtgcggc gcaccgcagc tcctgaggtt ttggttatga 60
acaggattcg gattcacgtc ttgccaacca atcgggggag gatcactcca gtgcccaggt 120
ctcaggaacc tctgtcttgt gcattcactc atcgtccatg ctctcaccct cgtctggagg 180
ggcaggagtt ttgcattaag catatccttg aagacaagaa tgcacccttc aagcagtgta 240
gttatatatc gacgaaggaa tggaaaaaga tgtcccaatg ctgccccaaa gccagagaag 300
aaagatgggg tgtccttctg tgctgaacat gtccgtagga atgccctggc acttcatgct 360
caaatgaaga agaccaaccc agggcctgtg ggtgaaacac tcctgtgcca gctgagctca 420
tatgctaaga cagagctggg ggtctcagac tccagaaagt agtcgcagtg aagccagccg 480
aattctattt gaagacagct ggagtgatgg ggagcaggaa cccattactg tggatcagac 540
atggagaggt gaccctgaca gtgaagctga tagcatagac agtgatcaag aagatcccct 600
aaaacatgct ggtgtctata catttttaag aagtggccct gattatgcgt gaaaagctaa 660
ttcgtttgca ggcgttgtat attgatcagt ttaaacgact tcagcatctg ctcaaggaga 720
agaagcgccg atacttacat aatcgcaaag tggaacatga agctctaggc agtagtctcc 780
tgactggccc agagggactt ttggccaaag aacgagagaa cttaaagcga ttaaaatgtc 840
tgcgacgata ccgccagcgc tatggagggg aagccttact gcataggcag ttgaaggaac 900
ggagaatgct ggccacagat ggtgctggcc aacaggccca taccactcgt tccagtcaga 960
ggtgcttggc ctttgtggat gatgttcgtt gttccaatca gtctcttcca atgaccagac 1020
actgccttac ccatatttgt caggatacga atcaggttct cttcaagtgc tgccagggat 1080
ctgaagaggt accctgcaac aaacctgttc ctgtaagcct ctctgaggat ccctgctgcc 1140
cactgcattt ccagttgcct cctcagatgt ataagcccga gcaggtactg tctgtgccag 1200
acgatctgga agccggcccc atggatctgt acttgagtgc tgctgagctt cagcccactg 1260
agagtttacc ttctgaggtc aaggatgact tggatgttgt gggtgatggt atgcagtgtc 1320
ctccttcacc tttgcttttt gatccttcac taacccttga agatcattta gtcaaagaaa 1380
ttgctgaaga cccagtggat attttgggcc agatgcagat ggctggagat gggtgcagat 1440

cccagggatc tcgaaattct gagaaagcct ctgcaccatt gtctcagagt ggattggcta 1500
ctgcaaatgg gaagccagaa cccacttcta ttagttgata gtttggggaa ccattttact 1560
ttggtggatt taaatttctc gttcttcaaa gaagtatttc tgatcatctc tcacaaaaca 1620
caggcaaccc agactacctt gtttttcttc tggattgagt attatagtca aacgtaaagc 1680
caaattttga ggacgtggac ccgtgggttt cttcccactc agggcaagga agagaaagag 1740
agataatccc tatcaaagct tctgggatta ttttgggact atacctaaaa ggaaagtgac 1800
atctttgtaa tgggatttcc caagtca 1827

<210> 389
<211> 1746
<212> DNA
<213> Homo sapiens

<400> 389
ggagcccggc gagggcgccg gtgctttgtt ctgtctgagg ccaggaagtt tgaccgcgct 60
gccatgccga accgtaaggc cagccggaat gcttactatt tcttcgtgca ggagaagatc 120
cccgtaacta cggcgacgag gcctgcctgt ggctcgcgtt gctgatgcca tcccttactg 180
ctccgcagac tgggcgcttc tgagggagga agaaaaggag aaatacgcag aaatggcttc 240
gagaatggag ggccgctcag ggaaaggacc ctgggccctc agagaagcag aaacctgttt 300
tcacaccact gaggaggcca ggcatgcttg taccaaagca gaatgtttca cctccagata 360
tgtcagcttt gtctttaaaa ggtgatcaag ctctccttgg aggcattttt tattttttga 420
acatttttag ccatggcgag ctacctcctc attgtgaaca gcgcttcctc ccttgtgaaa 480
ttggctgtgt taagtattct ctccaagaag gtattatggc agatttccac agttttataa 540
atcctggtga aattccacga ggattttgat ttcattgtca ggctgcaagt gattctagtc 600
acaagattcc tatttcaaat tttgaacgtg ggcataacca agcaactgtg ttacaaaacc 660
tttatagatt tattcatccc aacccaggga actggccacc tatctactgc aagtctgatg 720
atagaaccag agtcgactgg tgtttgaagc atatggcaaa ggcatcagaa atcaggcaag 780
atctacaact tctcactgta gaggaccttg tagtggggat ctaccaacaa aaatttctca 840
aggagccctc taagacttgg attcgaagcc tcctagatgt ggccatgtgg gattattcta 900
gcaacacaag gtgcaagtgg catgaagaaa atgatattct cttctgtgct ttagctgttt 960
gcaagaagat tgcgtactgc atcagtaatt ctctggccac tctctttgga atccagctca 1020
cagaggctca tgtaccacta caagattatg aggccagcaa tagtgtgaca cccaaaatgg 1080
ttgtattgga tgcagggcgt taccagaagc taagggttgg gagttcagga ttctctcatt 1140
tcaactcttc taatgaggaa caaagatcaa acacacccat tggtgactac ccatctaggg 1200
caaaaatttc tggccaaaac agcagcgttc ggggaagagg aattaccccg cccttactag 1260
agagcatttc caattcttcc agcaatatcc acaaattctc caactgtgac acttcactct 1320
caccttacat gtcccaaaaa gatggataca aatctttctc ttccttatct taatgatggt 1380
actcttttca atttctgaaa acagtaacca ggcccaactt ccctccttac tacagtcata 1440
ttaaacagat cacatcaatg acaaaatgtc actactataa aaactactta atttgtaagg 1500
aaattgtttc atagatttaa aaaaattgtg gttggagagc atcttggcat ttgtgctttt 1560
tttcttgagg gattgttctg cttcctggct gtatgatggg tatatcatta aagtttggag 1620
tcctatatga acaaaactga catttttaga gttgtacttt tgggaatgtt atagattgat 1680
cattctttct cctgataata aaggtattga atatctgtta tgaaaggtta aaaaaaaaaa 1740
aaaaaa 1746

<210> 390
<211> 1560
<212> DNA
<213> Homo sapiens

<400> 390
atgatctcca tcgtctgggt cctgtccttc accatctcct gcccactcct cttcggactc 60
aataacgcag gttattgggg tacaggtagc atttggttac ataagctgcc tgccgacact 120
acggccactg tcgaggacat gctgccttct gtcaccaccg tcaccactaa ctctgacact 180

atcaccgaaa cctttgccac tgctcagaac agccccacca gtgagaccac caccctgacc 240
tccagtattg ccccgccggc cacggccacg cctgactcaa actctgtacc ggctggccag 300
gccacccctt ccaaggggcc cagcgcctct gccccctccc cggccccagc ttcagccccc 360
aaggtcgccc ccctcgttga cctgagcgac accccgacct caacccctgc cgctagcaat 420
ttgtcttcta gtgtcctggc taaccaaggg gctgtcctca gcccaagcgc ccctgctggt 480
gtcggggagg cctctaaggc tcctccggcc agcaagccca cccctgcacc agtccccacc 540
ccgactgggg cagccagtcc tctagcagca gcggctgccc ctgccacaga agcccctcag 600
gccaagcagg aggctcccag caccaaaggc ccggacccgg agcccaccca gcccggagcc 660
gcgaagagcc cggccgaggc agccacagcc cttgctagcc cgaagagcga ggctgcctcc 720
gtcagcacca caaacccttc ccagggcgag gacggggatc ctctagagtc gaactgcggg 780
tttatgcaac ctaaccccct catttctgat ctaattaatg ttgctatctt gtctttttat 840
gccaagaagg cctctgacag cagggtaatt tcagatatta aaaagaaaaa tccaaatact 900
ataatcagtt ctttagctca aattcgaagt gttactttag agccacacag agctgctggt 960
gttctgagcc ggaccctttc ttcctctctg aaaattgttg aggaggcctt gcgagcagga 1020
gtggtaaaga aaatgatgaa attcctgaag tggcagagaa atgtggaggg ctctgagttc 1080
ctgcccccct ttgctctctt ccacgcaagt tcagctgacc gtcagcatct tctgaccaag 1140
ctcaacttca ggtctactgc cagacatgtg gccacgggac acagagagat catagcctcc 1200
tatggacctg tttatgaaat tccccttcac acgcacatgg ggtggctcat attaagactt 1260
tgtctatacg acaccaaaca tctggatgaa aatggaaaaa agaaaaagtt gagcgttatg 1320
atgaagctgc tcagctcgga ggatgaggtt ctggtgggca acgctgccct ctgccttggt 1380
aactgcatgg aggtgcccaa cgttgcgtct tccctgctaa agacggacct tttgcaggtc 1440
ttgttaaagc ttgcaggcag tgacacacag aagacggccg tgcaggtgaa cgcaggcatt 1500
gctctgggga agctgtgcac agctgagccc aggtatgctg tggacacgga gccaggctga 1560

<210> 391
<211> 1840
<212> DNA
<213> Homo sapiens

<220>
<221> misc_feature
<222> (1)...(1840)
<223> n = a,t,c or g

<400> 391
   agattgcaca ggaggaggcg gtagcggaac ctttctcctc cgcctggttc tcgcttccac 60
   ccgggcccag ttggggacgg acgcactaga ggcgacctaa acatggagac agcgggcgct 120
   gcaactgggc agccggcctc tgggctggta ggctccgggg tccacgaatg accggctttt 180
   cctggttaaa ggtggaattt tccttggtac cgttgctgca gcgggaatgc tagctggatt 240
   tattacaaca ttatcattgg ctaaaaagaa aagccctgaa tggttcaata agggaagtat 300
   ggccacggct gcattaccgg aaagcgggtc cttcccttgc cttgcgagcc tctgggctgg 360
   ggctccctgt aatgcatggt gtggggttgg ttgtgaataa cttcgcagtc tggaaagctt 420
   taggaagttc acagtaatga accgactttc cgaagtaaaa atgcaatcaa tatttccaac 480
   caattcccaa agaactcccg aatccggctg ttgagtggga ggaaacattg aaatccaaat 540
   gagatgagca tgggatgaat ttcaaaatgc ttggttacag aaaaggggtg gctctggaga 600
   cacccaatga cagccaaaag gactgggact gatttctccc aggaacatgg ggcagattgc 660
   tgactgaacc agtgcactgg atagcattca gcctcatcac aggagaagta tgggtgtgcg 720
   tgctggggga gggtaaagtt ttcccacagt taagaagact atttaaaaat agtaattaca 780
   ggaataactt ccttatgtgg aggggacccc ataggaaatg attctgtttg taacagttga 840
   agcaaatttc atactaaaaa agtttataat aaaagtatga taagaaaata tttataaaac 900
   agacccccta atagcaatag tactgcactc cgtctaatta aaggcctttc aagagccttt 960
   cttgtcaata tatcacacaa cttggaagtc ttattctgat tagccagact acaaagtgac 1020
   agtcacctgg taagtcctgt atggtaggaa gtgggtgtgg ctctcgcggt cctccatgtg 1080
   gtctgtgctt tacagccatc acacacatgc tagttattta cctgatgccc ttgcttgaag 1140
   gccctataaa ttcttattac ttaaaacaaa aagccagtaa tggagttgtg atcaccccaa 1200
   aagggcacag ttggggctaa aataatggat gatatcaggg ggtaaacata ttttttttga 1260
   ggaagttggg tctgggatct ctggaacact ggacaatttg gtccattttg gtcttcagtt 1320
   ggtncaaagt aaaaaaaggc ccagttgtca cccgtcttca aatattttta acccagaaag 1380
   tcaccctgag tacggagtcc ccaatataaa gtatttgggg accaccctcc ccaacaaagc 1440
   tgttagggta aattctgtgt gcacgcccca actccctcct gtcttcccac agtctcctac 1500
   aaaaaaaaac agatggagaa taatgtctac atgcaattgt ttttactgca attgtatttt 1560
   aaaatatttt caagagagag aaatacttta atatgtcaat atgtccagag aaaaaacctg 1620
   tagttaaata tcagttgtgc aatttcttcc tacttgccta ggctctaatt agtcagcaat 1680
   caggtttaat aaaggacatt tgaaatttct gttatcatga aaccttttat tctaagggag 1740
   atttttaaat gaaatgacaa gaacttaatg taatactttt ctctagacca tgttaccctg 1800
   ttaaaattgc tgtattgtta atgaaacagg tgtaatcagc 1840

<210> 392
<211> 1827
<212> DNA
<213> Homo sapiens

<400> 392
   gaattcggga ttcgggtgaa tgtagtgatt gtgggaaaac cttcagctgt agctctgccc 60
   tcattctgca tcggaggatc cacacggggg agaaacccta tgaatgtaat gagtgtggga 120
   agaccttcag ctggagctcc accctcaccc accatcagag aatccacact ggtgagaaac 180
   cctacgcctg caatgaatgt gggaaggcct tcagcaggag ctcaaccctt attcaccatc 240
   agagaatcca cactggagaa aaaccctatg aatgtaatga atgtgggaaa gccttcagcc 300
   agactcacac ctctatcagc accagagaat cccacactgg agagaagccc tacgaatgta 360
   tggaatgtgg aggaaagttt acctacaagt tcaggcctta ttcagcatca aagaatccac 420
   accggggaga acccctatga atgtagtgag tgtgggaaag ccttcaggta cagctcggct 480
   cttgttcgcc atcagagaat tcacactgga gagaagcctt tgaatgggat cggcatgagc 540
   aaaagctccc tcagagttac gaccgagtta aatatcagag agtccacgtg aaagagccac 600
   acacccattt tcctcacttt ccctgagtct caagagctct tgccttaccc tataaatctc 660
   aacagcttag gatgtgtccc tttcaactca gacttttcat tttagagaat ggggcagatg 720
   gggcaaatcc gttgaatttt cccagaaatc acaccagcca ttagaaagcg tcaaggcaag 780
   tgggatgggc gtgctgggga atagaaagca gctctgggac cagttacccc atttaggaaa 840
   ggagtttgca ctaaactgtt tttctcacag cagaggaacc tttcccaagg tggggatgga 900
   agcacagttc ggggacagaa ttccgatgga ttcccttagt ttggagtccg cgttagtcag 960
   gcacagacag cagtgggaaa ggacgctctg ggtcctgtta tttgctaggg agggtaaagg 1020
   gaagactatt tcaaaagcta ctgttcccta gtccaagctt taaagttcgg gtaaaaaaac 1080
   aatgctgttt ttgtttcatg atttcggtta aattaatgga aatttgggca attgagggga 1140
   ttattttatt gaggggtaga agagattccc agaatcatca tctgtgaatg aatggtgtcc 1200
   ctttaagggc tcttgggagc aggcccagac ccatgtttcc aagaagaaac cctggtgata 1260
   ttgccaagca agacccccct gcccatcccc ccccagttgt cccggggctg aatgggcaaa 1320
   tctgtcccaa acagcttagt aaccggctgt gaagggagag ggtcagaagc acttaggcgt 1380
   tgcccttctg attgctgtcc tctcttgtcc tcttcccact ccaatgatga aaatgatttt 1440
   ctctaaatgc ctgggtaagg atgctttcaa gggagctcac ttggcctgct tgccctgccc 1500
   tctcacctct gacacccagc cccaggagcc agaccactcc tgcctccacc tctgactctt 1560
   cagcagctga agattaatgc agagaaagag caaagcccaa aagggagaaa aaaaaaaaac 1620
   aaaaaaacaa aaaaacgaag cccagagaag gcaggctgtg cctgaggcct ggtagcagag 1680
   ctcgttgctg ggtgaggatg gcactccccg agttttccca accgacggga tgaccttcca 1740
   ttgtgttttc ccaaccctgc tcatcctgta gcataaatga agtgcactat taaacagaat 1800
   agtttttcag aaaaaaaaaa aaaaaaa 1827

<210> 393
<211> 410
<212> DNA
<213> Homo sapiens

<400> 393

aagaagggtg ttgtgttacc cactctatcg ccatttcaag ctggtgatga gggcctacag 60
ggacactata gatatattgc aactgggtaa aagtgcagct ttaaagtgtc tcctggatat 120
tcacaaaatt tttcaggaca atgacccagc gtacatactg aatgatctct acatctcata 180
ctactgtgtg tggattcata aagtcaaatc caaaaagttg tcagatcttg cagaagcctt 240
aaaggaagtc tcccttacaa aggcccagtt ggggttagaa ctgtaagaac tagaagcagc 300
agcactgctt gtccaggagg aagaaactgc attaaaagca gcccattcag tttctgggca 360
gcagacactt tgctccagct ctgaggcaag tgattcagag gactcatacg 410

<210> 394
<211> 2775
<212> DNA
<213> Homo sapiens

<400> 394
gagatgagga aaataggggg tcttatacag aggggcttca tgaaaatggg gtgtgctgca 60
gtgatcctct ttctctcttg gagtctcgaa tggaggtgga tgactatagt cagtatgagg 120
aagaaagtac agatgattcc tcctcttctg agggcgatga agaggaggat gactatgatg 180
atgactttga agacgatttt attcctcttc ctccagctaa gcgcttgagg ttaatagttg 240
gaaaagactc tatagatatt gacatttctt caaggagaag agaagatcag tctttaaggc 300
ttaatgccta agctcttggt cttaacttga cctgggataa ctactttaaa gaaataaaaa 360
attccagtca attattcctc aactgaaagt ttagtggcag cacttctatt tgtcccttca 420
cttatcagca tactattgta gaaagtgtac agcatactga ctcaattctt aagtctgatt 480
tgtgcaaatt tttatcgtac tttttaaaat agcccctccc taccgtgcaa ttcccgagtt 540
agagggtaaa gccccctgtt gtaaaataaa ggcctcaagc aaaattgtac cagtgatagc 600
aacctttccc cacacaggac cgttgaaaac agtaatgtgg cctacacagt ttttttaact 660
gtcagagcca tcagctgggc tctttaatat aatgactaaa caataatttt aaaacaattc 720
atagtagcag catattaagg gtttcttagt atgcctcata tcaccagcaa tgatctttgg 780
ctttttgatt tatttgctag atgtttcccc cttggagttt tgtcaagttt cacccctgtt 840
tgctggcccc caggtgtact gttgtggccc ttggttaata tcgcaaacca ttggttggga 900
gtcagattgg tttcttaaaa aaaaaaaaaa aaacgacata cgtgacagct cacttttcag 960
ttcattatat gtaccgaggg tagcagtgtg tgggatgagg ttcgatacag cgtatttatt 1020
gcttgtcatg taaattaaaa accttgtatt taactctatt caatcctttt agataaaatt 1080
gttctatgca agaatgattg gtgcttattt tttcaaaaat tatgctgtga acaacgtgat 1140
gacaacaagc aacatttatc taatgaacat acagctatct taatttggtt cttcaagttt 1200
tctgttgcac ttgtaaaatg ctacaaggaa tattaaaaaa atctattcac tttaacttat 1260
aatagtttat gaaataaaaa catgagtcac agcttttgtt ctgtggtaac ctataaaaaa 1320
agtttgtcct ttgagattca atgtaaagaa ctgaaaacaa tgtatatgtt gtaaatattt 1380
gtgtgttgtg agaaattttt gtcataagaa attaaaagaa cttaccagga aggtttttaa 1440
gtttagaaat attccatgcc aataaaatag gaaattataa atatatagtt ttaagcactg 1500
catcagtggg agttcttggc ttatgttagt ttatgttagt ttattatgaa aacatcgaag 1560
atttttttga ctatattatc agttaaacaa aaaggagtca gatttaattt gttttttgaa 1620
gcactttgag aaattaattt taattaactt aatgagcaaa tttttattac tactttatgt 1680
tcaataccag gttcttttca tttctctgga ttattttgca aatcattgga cagagaattt 1740
gggaatataa atctgtaaca ggtgtttgac accagtaggt ctctttattt ctgggaaatg 1800
tgtacctgta ctttctgata tacagtgttc ctaagtaaaa atcaattcag gggatttgta 1860
tagtgtctat aggtaaagta gcccatgtct tgaaatatga aaaggaatct gaaggtcatg 1920
aaaagtccag tggagaaaat ctcaatgctt actgttacta ctaattgatt cctactagtt 1980
tccaggtttg gggggatatt gtttcaatga cgctccttaa gactgttgat tgcccatagg 2040
ttccaaatag gaaattaaga ctcatggaac atttttagga aagtaggatt gttttctcct 2100
ggttctctaa ggaactactt ctgcagtctt acatagtctc atccttgttt gttgtggtgc 2160
agtcgaactc ctcaggcgtt tggaaagcat gtggtagacc ttcttccaca cccacccata 2220
cccccgttca ctgcgtctgg aggtcttcaa cagtgaagta gggcagccca cacagcctct 2280
caggagcacc tgtccgaggc acccggagca ctttgcagag cacgtccagc cctcatgggg 2340
tccctgcata gaaatgtgaa cccctgccac tgaggaagat gaaggtagac cctgtgtctg 2400
gaggtgctgg agggcagcgg gtcacctcct tgtattccca ccttagtttg ggggtgtttt 2460
gaagaggttc agagactaaa tcttaaacct tatttgaata ccaacgatag ctattttggg 2520
aatttcgatc ttaaaaagtg acaaaacaca tttcccattt tcatttttca gctgaatttt 2580

agtaacttat ttttgatgtt ttaattttat catggcctcc tctttggagc aaacccttcc 2640
catgggtctc aaagcagtga catttggtag taaatcactg cctctcagga gtcggtatgc 2700
acaagcactc agcagccact gttgatgcct tctagggaaa cctaatttcc gttggtaaag 2760
gtaggggcct cggaa 2775

<210> 395
<211> 497
<212> DNA
<213> Homo sapiens

<400> 395
gggacgcgga gggtcagagt gcagacctga gaccactgct caccgacttc agactccagt 60
ttatctgtgc cccagcttcc tcacttagtc tcagaagact taggctgagg cctcagaagg 120
agatctccat cctttgtcca gagcagaaca ggatggccaa tgtcccagga atcattgacc 180
ttcaaggacg tgtttgtgga cttcaccctg gaggagtggc agcaactgga ctctgcccag 240
aagaacctct acagggatgt catgcttgag aactacagcc acctggtgtc cgtggggtat 300
ctagttgcga agccagatgt gatcttcagg ttgggaccag gtgaagagtc ctggatggca 360
gatgggggga ccccggtacg gacctgtgca ggtgaggaca ggccagatgt atccattttt 420
gcctcattgt attttgaagt gctgttatta gaagtctggc aagttgatga gcagatagat 480
cactacaagg aaagcca 497

<210> 396
<211> 719
<212> DNA
<213> Homo sapiens

<400> 396
aaattggagc ttgccttgcc gatgttgatc caatgcaact agattcttca gtacgatttg 60
atagtgttgg tggcctgtct aatcatatag cagctctaaa agagatggtg gtgtttccat 120
tactttatcc agaagtcttt gaaaaattta aaattcaacc cccaagaggt tgtttgtttt 180
atgggccacc tggaactgga aagactctgg ttgccagagc acttgccaat gagtgcagtc 240
aaggggataa aagagtagca tttttcatga ggaaaggtgc tgattgtcta agtaactcgg 300
taggggaatc tcaaagacag ccacgattgc tgtttgatca ggcctatcag aggcgccaac 360
aatttatttc ttttgacgaa attgatggtc tggctccagt acggtcacgc aggcaagatc 420
agattcacag gtaaaacttg cttgatggca cttcaacgtt tcttcctatt ccctgagtgg 480
tattgatgtg tgagagatag aatgttaaat ggtgaaagaa actttataag ctagtagttc 540
ctttttttgg tagtagaacc ccagtatata gtacaaaaat aaaaaatgtg gatggttgca 600
gtggctcata cctgtattcc tggcactttg ggaggccaag gcaggcagat cacttgaggt 660
caagagtttg agaccagcct ggccaacatg gtgaaatcct gtctctacta aaaaaaaaa 719

<210> 397
<211> 828
<212> DNA
<213> Homo sapiens

<400> 397
gggtggttgg ttaccgcctt ttgcactagc agtagcaagg aaggggggtg ggcgctcttt 60
ctttttctct tagaagaggg tttagcacag gttttttcgt tctcacttcc acaccacctt 120
accgcctccc gaccccccct ctccccctcc ccacctatcg tcatgacgcc ctctccggat 180
tacttggtgg tgctttttgg gatcactgct gggcccaccg gggccaagct aggctcggat 240
gagaaggagt tgatcctgct gttctggaaa gtcgtggatc tggccaacaa gaaggtggga 300

cagttgcacg aagtgctagt tagaccggat cagttggaac tgacggagga ctgcaaagaa 360
gaaactaaaa tagacgtcga aagcctgtcc tcggcgtcgc agctggacca agccctccga 420
cagtttaacc agtcagtgag caatgaactg aatattggag tagggacttc cttctgtctc 480
tgtactgatg ggcagcttca tgtcaggcaa atcctgcatc ctgaggcttc caagaagaat 540
gtactattac ctgaatgctt ctattccttt tttgatcttc gaaaagaatt caagaaatgt 600
tgccctggtt cccctgatat tgacaaactg gacgttgccc caatgacaga gtgtatcctt 660
taaatcccta ctcaagaatc ctttgaaaaa atagttccag aaaaaaatgg taattcaaga 720
aactttcaaa cattttaaat tttgtcagca tatggataaa attggcctat ccaaaatttg 780
tttactctgc tgagaaacta aaatacaact atacccatct ttaaaaaa 828

<210> 398
<211> 588
<212> DNA
<213> Homo sapiens

<400> 398
ctagtgaggc ggaattcctc ctgtcatgat ctctggcagg aagaacaaaa gaaggtaatc 60
cacaaagaca cagcccttac ttagaaattc tgacaaagac agcaagacaa atctgctccc 120
cactcccaca caccacagac aaacacatct cagggcccct aaagaaggca tccccacccc 180
accctccgcc ccaagccata gaatcacagt tcagtggaat attctccctg gtactagatc 240
aatccctcca ccttccttcc tacccctttt tccttgacaa tcccaacact gtccccctgc 300
agcctccctg gccctcccaa agcagcatcc accgccagga agaggcagct gcacagtttt 360
agttccacct ccagagacct atttcttttc ctcagccctc ctagtctttc ggccgcctag 420
tctctatggt gggcctttta ttttgtatag ggaaagaaag agaaaaggga gaggtgggag 480
agatttccta gtgcaataca accaaaaaat atttttaaag tcttgaaaaa taaacataaa 540
gcagccttct tcccccaggc aactaaacag aaagaggttt ggttttgt 588

<210> 399
<211> 1944
<212> DNA
<213> Homo sapiens

<220>
<221> misc_feature
<222> (1)...(1944)
<223> n = a,t,c or g

<400> 399
ctttcaaagc cctttaaagt tccgccttgg gggaacccct gatttgaacc aaacctttta 60
aagctgggtt tcnnnnnnnn nnnnngttat ctggacagct tctttgagac tatttaaaaa 120
ctggtacaac aggtctctac aacgccaaga tctaactaag ctttaaaagg tcaagaagtt 180
ttatggctga caaaggactc gcgcaacgca gaaggccttt cccaccttaa gcttccgggg 240
atctgggaat tttaccccca ttctcttctg tttgtctgag tctcatctct ctgcaagcaa 300
gggctgaaat cattttgttt ggttgttttg agggagagag gcggggtggg ggggtgcaaa 360
tctgccagca gctcttacgt aaggcatgtt ttattgggga gggctgagct tttattttct 420
cctctccagt ggggttggct tttattgttt cttgtttggg tttggaatgg aaatatggat 480
agcagcataa agtactttta ttttgacaaa attcattttt ttcaacaatg gagacataga 540
tttgacccac aataacttct ccccctctct ttttactctg ctcaaaaagc atctctcctc 600
ccattaccca accttggtca taagtgtgcc tggctggttt gcagatattt gttctgcttt 660
gtaaaaattg gccattagtg catttattga gatgatctct aaagagctat gccctgacct 720
acccctgatt ctatgacatt ggggcccttc ttttgctgaa actgccttac gtaatggttt 780
tactcctttg aaagagattt gacggaatgc attttatgcc aagtgcttgc ctgcactgtt 840
tctgcaatat gtggtgtatg ctgtggtgat cttgctggga atgattataa gtgtgtgtgt 900
ggtgggggag tgggtattac atgcattgct gaagagtcat cctggtgttc ctcattcctc 960

ccaccttccc gtggtcattt taattacggg gcagtgtcac ccgcaaaggg aggaaactca 1020
aagccgaaag caaaattcca ggcctgattc tggcttttga ggttcctggt tcttgaagcc 1080
aggcctgacc cgactctcag atggggtcag tcccgtcgct ttgcagactg accctggaaa 1140
tctacaaaat gcagattttc ctgatttcct cttctcttgc ccagtttttt tttttttttt 1200
ttttttttta aagcctggat tgtaaccaga ttttcttttt tcccccttct cagctgtaga 1260
tatgatatct cctttcaggg ccccagctta agggcaaagt gagttaatgt gtagacaaag 1320
gcgagggcac aagagagagt tacatcatct atagcatggt gaatgagcca tggtgtgtgg 1380
tttcctggga accaccaaac acttgcaggt ttagcttttt cccaggggtt gacttacaag 1440
aaagaaaacc atgtttttgc aagattaaaa tgtggttgaa gtgtgcctaa attaaccatc 1500
cccattttta tcatatttcc accatcactt tcaggggttt taaggagtca gtgctcacct 1560
ggggcggagc tgggtaggta catttttgct tcttaggaaa ggctaagtcc cggggttccc 1620
gtccggattt aagggttcca gggaacttcc ccgaggaaca cccgattcgc agagggtaaa 1680
tttttctggg agttttgttt tgcagggata gctggggagt tatgggccac cctggttcca 1740
cggatgcggt aaatgaatcc cagcagaagt gggtgaagca gcgtttgcaa gatgtacaac 1800
tccgcagcac cggtcagcaa tcaggctgca tccggacggt gttggaggac cgaggggttg 1860
ggggccttct accggagcta caccacgcag ctgaacatga acatcccctt ccagtccatc 1920
cacttcatca cctatgagtc ctgc 1944

<210> 400
<211> 1958
<212> DNA
<213> Homo sapiens

<400> 400
   gaaattattc cttagatttg atctagatga agaaatgggg ccccctggca gcagcccaac 60
   accagagcct ccacaaaaag aagtgcagaa ggaaggggct gcggacccaa agcaggtcgg 120
   ggagcccagc gggcagaaga cccttgatgg atcctcacgg tctgcagagc tcagtccccc 180
   agggcgagga tgattctgag tatttggaga gagacgcccc tgcaggagat gctgggaggt 240
   ggctgcgtgt cagctttggt ttgtttggca gcgtttgggt gaacgatttc tccagagcca 300
   agaaagccaa caagaggggg gactggaggg acccttcccc gaggttggtc ctgcactttg 360
   gtggtggtgg cttcctggca ttttataatt gtcagttgtc ttggagctct tccccagtgg 420
   tcacacccac ctgtgacatc ctgtctgaga agttccatcg aggacaagcc ttagaagctc 480
   taggccaggc tcagcctgtc tgctatacac tgctggacca gagatacttc tcagggctag 540
   ggaacatcat taagaatgaa gccttgtaca gagctgggat ccatcccctt tctctttgtt 600
   cagttttgag tgcctcgcgt cgggaggtcc tggtggatca cgtggtggag ttcagtacag 660
   cctggctgca gggcaagttc caaggcagac cgcagcacac acaggtctac cagaaagaac 720
   agtgccctgc tggccaccag gtcatgaagg aggcgtttgg gcccgaagat gggttacaga 780
   ggctcacctg gtggtgcccg cagtgccagc cccagttgtc agaggagcca gagcagtgcc 840
   agttctccta aggagctggt ggtgctcctc acggaacctt gccgcttggg gaacctgacg 900
   tctaagtgtc cagaaaggag gatgtgggca gggacggggt acagaggata gtgtgggtca 960
   gaggtgccag tagtataata ttcatctccc tggagttatg ttgaaggcag agttttcata 1020
   gggttagatt ttttttatcc ttttctagtt cagttaattc atcctgttga attgcaccat 1080
   cgtgaaaaga tgggaaaaat cgtgatgatg ggtaagggga aaacttcccg gaaggcaatg 1140
   gggcaaggaa aaagaaagcc tatgggaaat ggctgtgctc ccaacatagc tttgcagatg 1200
   atgtgggttt tttttttttt ttttggttgt ttgttttgag agagagtctt gctctgtctc 1260
   cctgggctag ggtgtggtgg tgtgatcttg gctcacggca gccttgccct cccgggctca 1320
   agcagtcttc atctcagcct ccagagtagc tggggactta caggcatgtg atatgatgct 1380
   cggctgattt ttgtttactt ctttagagag atggggtctt gccatatttg ccaggctggt 1440
   ttgaactcct acaactcaag cattcctccc accttggtct cccaaaatgt tgggaccccg 1500
   ggtttgaagc caccgcgccc cagctagccc cctgtgtttt gtttttgttt tgtaaacttt 1560
   ggttgatgtt aaggccctcc attttggaaa gcaggaaaac aggatttttt ttttttttat 1620
   cttgttcccc tggaggaacc cagggatgga ggatagagtg gcctgagagc agtgcttgga 1680
   ttcagacctc ctgctgggtc cttctgctgg gatacaggcc cccaacaggc gggggtggag 1740
   cagggagctg cgcctttccg ggggtgcccg gtggtgtgta gagaaaagct gcttgtttta 1800
   ctccttaagt caatgtattg gtgactgttg atttgttgga acaatttcag ggaatcaagg 1860
   ggctgtgcga gaaacttccc tcatgttgtt tgggcaacag gtgaatgaac ctagagcggt 1920
   gacatggaaa ataaagctca ctgttactca aaaaaaaa 1958

<210> 401
<211> 810
<212> DNA
<213> Homo sapiens

<400> 401
cctgtctcca aactattata aagagacatc cctgttggcc gaggttccga aaacagttca 60
gttctctgag ggaaggcagt gccagcagct acatgcttag cttggctctc tcctgtatag 120
ttcttagtat tagaggatgg ctttggctta tcagaaagga gttcagggag gggtttccag 180
agaacaagct ccatggaagg acggctcata gactcaatca ttttctttgt aaaaacttca 240
tcaaattccc tcttcaaacc tgttttcatg gtatcagaaa ggacaagact ggggagatgg 300
ttaacatttc tgtcagcttc aacttcttca tcttcatcaa ttatcctgtc ctcaatctcc 360
tgaagcttcc ttcgggcaac ttcacactgt ggttactcca gttgtctgat ccatttcttc 420
acaaataaca tctaatatcc caggtatgga aaggccacta acactgggat ttactccatg 480
cccctctaca tcctgatgtg cacaaagaat ccagtcatta ggaccagcac agagctctgc 540
ttcagttatc cttttctttc ttactggaca atcatcatcc tcctcctcct ctcgcttgtg 600
tttcttttta cagtgaacag aaacacgtcc gcgcgccgca ctcccagcgg gactgctagg 660
gactgcgcga ggtcccggcc gcggggccaa ctcggcgccg tcagcccccg gcgggaaggc 720
ccggccgggg aaggccggct gcggcggctg caggaagtcc gagccgccgc tcagagggag 780
gccgctgaag ggccggccga gcgcagccat 810

<210> 402
<211> 1169
<212> DNA
<213> Homo sapiens

<400> 402
cacatatctt aaaagcacca cacgtcaact ggggataaca gtaggtcaac aggtaggtag 60
gtttaatcta gataagttga aagatagatt gctatcaaaa acagttctcc aagatgtgca 120
tagccaaact gggatagaag gcaaactccc ccaaagctac ctgctggttt tgaaaagggg 180
tggttaagaa catggctatt ccccaggagt agtagaaaat aatatgccct gactaccaac 240
agcctcaagt atgccttatt tgcacatcct agacttggtg tctgtaagac tcagttacca 300
cttctatttt cctgtaccta ggagttagca aaaggaaact ggggccttcc cagccgagcc 360
actaaacctg tcttatttgg aatggggatt gtccagcaaa gggagcaaac atgaattaga 420
tgttaagcta ttgagctgaa gaaaagaaag cagttcacat ttaggtgaaa tagatgatgt 480
tatcaggaag ccaggttccc accagagtcg gtgcttggta cctggtctct ccagtctcaa 540
cagactcagg tccaggtctc tcccccagga agcaaccact caataaaata gagaacatct 600
gagaattaca aatgtctatg cttgattgct cctctaaatc cagtgcatag gttaaccctg 660
catgcccatt tcttcctggg cttcttgatg gcaatgtgtt cttaaataac tggtcttgtg 720
ttcatgctaa agacaaactt acatgaagtt tttcagttta agacattcta gtgaatggct 780
gctatgtgtt tctgggcact cattcctaac caagtcttta gaggatttca gatgacctta 840
aagatgcaat atctttttct ttctttcttt ccttcttttt ttctggggac aagagttggc 900
gcccgttcgc ccgggtgggg gtgctgggag tgcagtggtg cgatctcagc tcactgcagc 960
ctctgcttcc caggttcaag tgtttctcct gcctcagcct tccgagtagc tgggattaca 1020
ggcatgtacc actatgcctg gctaattttt atttctatat ttttagtaga gatggggttt 1080
catcatgttt gccaggctgg tctcgaactc ctggcctcaa gtgatccgcc cacctcagcc 1140
tcccaaagtg ctgggattac aggcgcgag 1169

<210> 403
<211> 917
<212> DNA

<213> Homo sapiens

<400> 403
tttcgtcgca ggacctcact gcgccctctg cctttatcta ggcagggcta gaaatggtct 60
acttgcagtc cgtcttcctg cttcatccgg tggccaggca gagggaacgg cacgggcaag 120
ggcctggaag ggaatccgag tggcccatgt caaacgatgc ccataactca gccccgtagc 180
ggacgggggc acgaggaatc aggtgtagag cctgagtctc agggtcctga tctctaaccc 240
cagcgagttc tcggggatcc ctgggcagct ttgccgctct gatggctctg cttccaggct 300
gatcatcttt ggctttgagc ctcaggattc tatagaatca gaggagcgcg ctcttttaaa 360
gacagggtaa ctgaggcttg gaggagcctt ggactggccg agggaagagt gggaggggcg 420
ggctcaaagg ccaggaacct caagtcactt gagtctggca gcgtcaaggg acaccccatg 480
ctgggtgtgc cgtcttctct ttgcatagaa tcttgagctg atgatggttt tgtgggtttt 540
ttattaagag aaaaggctta ctgaacgctt tatatgcatc aattcatgag tccgtaccac 600
agcctatgag atgttacact catttcacag atggagaaac tgagggcaga agaggtgagc 660
ttacctgccc aaggcatcta gtgaccagag gggcagccag ggtgtgatcc tgcctcatct 720
gacttcgtag ttgggtacac atttaacttc cgccttcctg tgtgtgtttt ccaagagcat 780
cattaaaaag ttttggccgg gtgcggtggt cacgcgtgta atcccagcac tttgggaggc 840
cgacgtgagt agatcacctg aggtcaggat ttcgagacca gcctggccaa catggcaaaa 900
ctctgtctct actaaaa 917

<210> 404
<211> 1509
<212> DNA
<213> Homo sapiens

<400> 404
agtgcggtgg aattcgtggg aggcaaatag aatgatttcg gatcacctgc tggagtctga 60
aaaaaaccgt gattcatctg gaagatatta cagggccagc ttgccatatt ccaggcacac 120
gttatcaagt ttgggcctat tgtggcctct gacttctctt tcttcagcct tttgaccact 180
tattaattag tccatttgct agaagagtgg tcaagggaaa aacgagagat gaaatttagt 240
taagtctatg tgagcaagtg agagaaggtt aggtaagggg agaggatgga atgcttgcct 300
ccaatgaact ttggagcttg tatgtgagtc agattgctcc cctattgcta ttatctatta 360
ctcttgagag ctggctgtcc tttgaaagaa agaagtaatg ttctttgaaa gaaagaaaaa 420
tctcttgctg tgtcaaacct caaaatgttg ctattggggt tagaaggcct cctctttatg 480
ctttttaatg ctctttcaaa cgtgttcttt tagaccagtt ttctaataag ctttgtaaaa 540
tgtactatcc aaattagaag cggatttgga aatgcaaact aacgtgcact tagatatcca 600
agtgggtgag cttagccact cttacccatg ctctttccct ggttttccct gggagacctg 660
tcccgagatg atttccatat accagcatag aaaatcagaa tcaagagcaa actctgagac 720
tggcacaatc caagaagatt tcctggctct ggcttttagt aatatcgaga ctccaactgg 780
ccactgtact ggactgtaat ttataaatcc agtagctacg cagggtggag gctgggctga 840
ggattaccat aatgaaatgt actaaatctt catttaggta tgcaattgtg aagtgaaggc 900
atctgctttc tttacagtat cagagtccaa gaacaggatg ttcaccatag gatgaaaagc 960
ctcatgacaa agggcagaac taacactcca aatttaatgt gtttaaattg gtgggggcac 1020
cagcagaaaa tacttctagc tcagctttac tcttcttcca cactaggctg ggcccagcaa 1080
tacaggagag gatgaaggga ggagctccag gaggcgaggg aagagcccta gcagggcggc 1140
catcacaacc actcactgag agttgccctt gcttaaaaat gtattttatt ttagccagtg 1200
ggtcccttcc ctttctccct ttccctctct actgcctcaa gaacagattt gaggcccagg 1260
tgcggtgcct cacatctgta atcccaacac tttgggaggc tgagatgggt ggattgcttg 1320
agcccaggaa gttcaagacc agcactgggc aacacagcga gaccccatct cttaacaaat 1380
aacagacttg aggaacccct ctaccttcca taattcccct caaacaccgc caactccaaa 1440
caatcactca aacatgctca acaaataagt atacaagcag aaacaataaa ccaatctaga 1500
ttttaaaat 1509

<210> 405

<211> 6590
<212> DNA
<213> Homo sapiens

<400> 405
   ttttcgtcta tgtttctgat ctgctttcag gaagaattat tcatttttat cttcccaatc 60
   tgaatgtttt attgaatttt tattttccaa gttaataaaa cctttggtta ttatcctgtc 120
   tttgttttac agtgtcctgc tcttgatgcg tggatgcaga ttttgtctct ctgtggacgt 180
   taatgattgt ggtctgaggg gaagtctttt ctgctccctg tgttgccctc attttctctg 240
   agttctcttt attttggtgg tctcagtctc tatctttcac gttgtgaatt tttctcaaat 300
   atatggtgat cctatggatc tgttcatgtt ttaagagtga ggcatccaaa agctgattgg 360
   aagttgtgtg tgccaactgg tgagcttttc cactagggtc accaggtggg cacctggact 420
   catcattgga gaacactgcc tgtcagtatt tgcacgtgtt ttctctgggg ctcattctgt 480
   ttcttgagag atattcccac tactctcctt cctgggaaac gggcatacac agggctttta 540
   gcctatgctg agtactcatg tggtttcaaa aatggtgtcc catctgggca gaagtcccca 600
   tgagcacttg gcttgactgg caaaggacac cttttgcccc ttcctccaga catacccagc 660
   tctgagcttg gacaatgctc gaggaacaat gaaaaggcta gattttaact cctaattgcc 720
   ctctttagcc aagtgcctct gtgcaatgca caactgtagg accatatcca gagaccctgg 780
   tctatactca gtgtgcccga gtcctgaccg ctgagcaggc agcctcatca cccgtgagta 840
   tttagacttt cattaagttg cttcaacctt tgctgtgcct ggtgtccctg agttggagct 900
   gctctgattt ctccatggag taggagcaca gtggtcatct ggctgcatgg gaaggacagg 960
   ggacctcaga gccaataaat gccctggtct tcagccccat gtctcacccc atccctccta 1020
   ttccaatccc caagcctctc ctggaggcca cagggcatct ctgctgacca ctctgtggcc 1080
   tcccctctgc aggcatgtgg ttttaggatc cccctccctg ctcacttttc catcagttcc 1140
   attcctgcat aaataggttg ctagctcttg cttactgttc tcttctgtat tctgtgttca 1200
   tggttgtatt aatataactt tcattcctct gctcctattt tcatggggtt tcatgaggga 1260
   agaaacaggc tcatgtgttc agtataccat cttgatccat aaaccccatt tgagttatct 1320
   cacaggttgc ttagggggct tgctgctctg actggtttgt gaacgtgccc tgagtccagc 1380
   tgggactgca gcaacatcca tcctgttgag ctgtggtctg ctaagtgatt ttgtgtaatg 1440
   ctgtgccaag tgcaggagag ggatacatag catggctaag ctctgcttga atgccatgat 1500
   tagttattac cttccttcct tccccctggt tttgctgcct attgtcagta aatgatctta 1560
   gatctcctgc ctccccaaag atatagtcta tgtggtggtc tgggttctag gttttcttta 1620
   ccacccttgc ttcacttaag aaacagaaaa ctggctaggc acagtagtgg ctcacaccta 1680
   taatcccagc cctttgggag gccaaggcag gaggatcgct tgaggccagg aattcaagac 1740
   cagcctgcac aacatagcaa gactccatct ctacaaaaag ttaaatttaa attagtggtg 1800
   gcatgcactt atagtcccag ctactcggga ggatgaggca ggagaattgt ttgagcccat 1860
   gactttgagg ttactgtgag ctgtgatcct gctattttac tccacctgag tgacagagcg 1920
   agacgctgtc tcaaaaataa agaaacaagt agtccacaca ggtgtttgcc agcaaacaaa 1980
   cagcgcggat tctctgttga acgtggccaa cattgcctcg tcgaagtttg tatcttgcca 2040
   tagttgatga tcaggtggtc ggggcttcct ggtcagacct gacctttgtg gggagatgtt 2100
   ctcccaccaa ggctcagaag caggacactc ttgctgcccc tcgggcagcc acccagaaca 2160
   tgggcgtctc catttctgag gttcagcata gttcccccca ccctcttccc atgcagtgca 2220
   tgcctgctct ttatcaggat ggaacagagg gaaggcagag gcactggggg cagcctgtgt 2280
   tgttgatctc tgtaagtcac agactgttcg aaaacctcag gaattatgta agcgttcagt 2340
   gcctcattta agagatgaaa aactgaagat tagaaagagg aaacgactag tcgccctgct 2400
   agttgatgac ctccagatag ccatcttcat tcccagttaa gtttactaca aaaccacagc 2460
   ctcagctgct gaagctaacc tccctaccca ccatcgctag ggactcagaa caccatggag 2520
   ctcatctctc caacaaaagt cagaattagc aagtacagat gtgtcttacg aaaatgtcag 2580
   aatccatcat ggctgctttg gtgaggagct gttctccttc cgtggtcatg ccattggaag 2640
   gcagttgtat gacaagaatg ctttcacatg agctccgatt ttcttaactc tccagaagga 2700
   cttcagaagc ctctggagtt ttgagacgcg gcctctctct cccttctgct tggagacacc 2760
   ttcctggggg tctccactgc attccccgcc ggtattcctc tgtggagcct tgcgccctca 2820
   ggtcccagaa atgggctgtg tgggagcttc cagtcagcct ggtatctcgg gaccttctca 2880
   gccaggaaat gagactgcac aaagccctgt gaggcccagc tgtgttattt ctccttcccc 2940
   gacattgcat aatcactgct gacacagcat ttggggaaga tttaacgtct tgggccagca 3000
   gctgccatct ggaaccatca ccacatagca ggttgttctg tcccatccaa ctgatgaggg 3060
   ggctgcccta atgacaaggt ccaggacctg ggttgggtgg tggaaggctg ggagctggag 3120
   tgggcattat tgtgcttctg tgctgaaaat tgctatggct cttttcccct aacagccagc 3180
   gtgggagaac caaacatgag acacaagcat ccccttgagc tccacagatc ttgcgccttg 3240
   ctctcatcag gcatttcctt ggagaacagc agtcagcagc tcatggaagt gagcccggtg 3300
   cacagacttt gcatggactt tgcacaggtt ccatttccat cgtgtgcaga cacctgtatc 3360
   tttgggctcc acgtgagcac ctgccctctt cactctgatt ttttttttaa gagacagcat 3420
   cttactctgt caccgaggct ggagtacagt ggtgtgatca cagctcactg cagcctgaaa 3480
   ctcctgggct caggcaatcc tcctgcctca gcctcccgag tggctggtag tacaggtgca 3540
   tgccaccacg cccacctaat ttttttatct tttatagata caggtctcac tgttgctcag 3600
   gctggtctcg aactcctggc ctcaggtgat cctcctgcct tggcctacca aaatgctggg 3660
   attataggca tgaaccactg tgcctggcct tcactctgat ttttcttaag agtcctacag 3720
   gaccttcagg tcttgtcact tctctaccat tgtgtgtgaa gacagggcat ggggtgagct 3780
   tggtcaggtg tgggtcaaaa gcctggagac tcagagctgc tggttcccca agagcccaca 3840
   aaagttatca gagtttctga gctgagtgtt ctgcagttgc cagaccccac aatgctggga 3900
   atactggctt ctttgtgttt agaacttgtt ctgagaacct tgatgagaac acatcaagca 3960
   agcagttcct cccagccagg gctggagcaa catgccaaaa aacattcttg tctatgggaa 4020
   aaagacaacc agtattttgt actgtttgac caagttgttt ttaaatatac acatgcctca 4080
   acaatccacg gcctcaatcg cactgctaat ttgtttaaat ccctgacact cagatgtcaa 4140
   gaatagcttc atctagcagt gttctcttta aaggaaccct tttttttgtt ttcattttaa 4200
   tcactgcctt cttctctggg aggtattgtg ggctagagaa agaagtcagg tttgggagtc 4260
   agccagattt agattggagt cccagctctg tcaccagatc tctgggtgat cttggcaaac 4320
   taacttctga gcttctgtgc attataggca ttcagcacat atttgttgga tgaaaaatac 4380
   ttttcttgaa agtttatgaa gttgtaggat gcccagcagc ccccttttct ctatttcctt 4440
   cctcttgaag caagcgttga ctatacctct ttccctaacc cttagtgtac catagcttct 4500
   tccaacctct ctccaatcca ttactttttt ctgagctacc ctgtctcaaa tctaactcag 4560
   gcctttttgc aagaggaagt ttaagccatt gtttactcat taactcactt gacccttaga 4620
   acaatcgtgg gaggtgttta tttcggaaag gaagctaaag cacaggacag aaaaataagg 4680
   tcatagcgtg aactagtaag tagcagaact gggaacatag gactgttatt ttcaaacact 4740
   ctaaacttca ctgcagttgc aaatttgcaa ccaaaatggc cgccttgaaa gggtcttttg 4800
   cagtaaaatc tcagtgtcca acatcaaaga cagttcttca gggcctccaa ttaactatac 4860
   atcctgcccc cagctgctca tctctgcacc tctaacacgt acttcacaga gtaggaaatt 4920
   gggctttctc cttcaaaaag aaaagtagag aaggcctgag ggcagtgtaa ttaaaatgga 4980
   gaagtagaga ccacgaatta cttggatgaa ttcataactg gttggtctct gaggcaggga 5040
   ttattatttt tcctgtattt taagcttaaa agtagtgatt tttgcattgt tacaaaaaac 5100
   aaaatagcaa aatatcattt gatacttgta ttctgtttgc aatagctacc acttccttta 5160
   gccccagcaa aaatcaatag gtgtgtattc tctacaagcc catgctgtgc cacgaatgcc 5220
   aatattgcta aatgtcatgc aaggatttat acactttttt aaaggaaact ctggaagtgc 5280
   ctgttgtctg gagagttgac attgtccaaa gcaggccaac actggtatgg gtatggagaa 5340
   cccacgtcct acctgtgggt cacctgcaga tgccgtgaaa ttaactctct gggttggaag 5400
   tgagaagact ttttttaaaa gtcctttcta agtttagaaa tccaaaggtt acgaaggtag 5460
   tttttattta tcttcaataa gtggcctatt tgcttaaagc aagtgtacct ctgtatatca 5520
   agcccaaagc ttaggggcag tgcttcttgg ctttagtatc tgagaaaggt gcctttggga 5580
   agagtttggg tcagacagaa ggtagtctga atcttaaagg tttcagatga atgtttgaag 5640
   tagaacctgg gaaatctgct tcctagggct gttagaagga aattggtctc cttaaattac 5700
   gtcagcatct gaggcagttt cctaagggaa tttcttttaa attcaaaagt tcaactgcag 5760
   gaataaatag ctgtacagga ctagatcttc ccatccctca tcttttcccc ttccccgagt 5820
   aacgactcaa aaaggcttct gaaattctac tcataatcgg agccgtttgc attccaacaa 5880
   ggatccctta agccaaatgc ctggtgtgtt tgtgtagaat gtcccctggg gtagggggcg 5940
   gtagctgtgg ggagggaatg tggtgcaggg gacacctgcc cctctgccct tgaaattgca 6000
   ccataagatg ctgcctatgt cactcacagt ggtgctgata ctatccgcca aagacaaatt 6060
   gtgaacacgg aaaaatggtc gttccctact gtacatcctc aggacagact taactaaact 6120
   tggagacagg agattgttca gaccatattg tagtgggtgg tgatcattgt tttatttggg 6180
   gggagtctgg tctattgagc agattgaatg tttccttatt gtgcagggct taattgacta 6240
   tgtctgaaag tttttactga gagctctaag aaaactattg aggaaaatga aatgttattt 6300
   tgtagtacaa ggattatttt tgtctattta ggatatacat agctgtagct ttgtaaaaaa 6360
   aaaagatcct tttataacca atatatgaat gtgccgtctt acttattgat tactgtaaat 6420
   taagatataa atggctattt gcataattta tacctgtggg aattaactgg agtatttgtt 6480
   atttgactgt tttctattaa ggaatattag gcttggtgct atgatgaatg atcttgtaaa 6540
   atcatgtgta ttcttaacac catttttgaa tataaatttc ttgaactgac 6590

<210> 406

<211> 364
<212> DNA
<213> Homo sapiens

<400> 406
gcgtcgacct atcagattta aatgaaaaaa gagaaggaag aggctgattg ctggagtgtt 60
aaagaaatct tggccgggcg tggtggtcac gcttgtaatc ccagcacttt gggaggccga 120
ggcgggtgga tcacgaggtc aggagatcga gaccatcctg gctaacacgg tgaaaccccg 180
tctctactaa aaatacaaaa aaaaattagc cgggcgtgat ggcgggcgcc tgtagtccca 240
gctactcggg aggctgaggc aagagaatgg cgtgaacccg ggaggcggag cttgcagtga 300
gccgagattg cgccactgca ctcccgcctg ggccacagag acgaactccg tctcaaaaaa 360
aaaa 364

<210> 407
<211> 370
<212> DNA
<213> Homo sapiens

<400> 407
ggcagggctg gccaccacca aagccagaca ggcagagcac acaggcctgt ggtgcctgca 60
ctcagacact acaaaggcct tggtcacttt acatgttagt aaagccatgg ccttggagag 120
gggaccctgg gaggctgggc cccaggctct tgccaccacc tcggaaagac tgcccaaggt 180
ggttctcaca gaaggcagga gctcctggtc caccttggtc tcctggaggc atcatccctg 240
caatccctgc tcagacccca taaagatctt aggagactgc cctcccagag gccagcagac 300
tgtgttacag ggcacctggg agcactggtc agggtccgat aacgccttga atgtgtaagc 360
ctcagtaggc 370

<210> 408
<211> 700
<212> DNA
<213> Homo sapiens

<400> 408
gagaagtgac tgacggcctg caccaactta gacggtcaca gtcatgaaaa gtgcacgggt 60
gggattgccc gagttggcca tgaattaccg tgatggatcc atttggaaat gaagttggtt 120
gtgacatttc ttcaagtttt gtttcgcttg aagcttgact ggtagatgga gaattttggc 180
cttatgtatc ttatgtgact tgcattaccc agtccagtcc tctgccatta atctaaggtc 240
cgcaatgtat caggtatttt actgagaatc ttgcttacct aattctcaca aaaaaaaaaa 300
aaccttggga gatgagggtg gaaacaggat gagaggtcag gtgacttgtc tgagggctga 360
cggtttgtaa ttggaagagt cagggcctaa ccactggtct ctttgacaca aaaactcatg 420
ctcttagtgg caagcacagt gttctcaaga ttgggggaag ccagggaggg acagtgggtt 480
gtctgctact agtgggtcaa aattacatat ttagtcatga atggttcagc ttttaccatt 540
taaataacaa ataaatgttc tttctccata ggcattaaag actattccaa ctggcccacc 600
atcccgcaag tgtacctcaa tggcgagttt gtagggggct gtgacattct tctgcagatg 660
caccagaatg gggacttggt ggaagaactg aaaaagctgg 700

<210> 409
<211> 592
<212> DNA
<213> Homo sapiens

<400> 409
gcactctccc tcaggcacaa aagctgactg atgatcaggg ccccgtcctg atgtccactg 60
tagccatgcc tgtgtttagt aagcagaacg aaaccagatc gaagggcatt cttctgggag 120
tggttggcac agatgtccca gtgaaagaac ttctgaagac catccccaaa tacaagttag 180
ggattcacgg ttatgccttt gcaatcacaa ataatggata tatcctgacg catccggaac 240
tcaggctgct gtacgaagaa ggaaaaaagc gaaggaaacc taacctatag tagcgttgac 300
ctctcctgag gtggagtggg aagacccgag atgaccgtgt tgagaaatgc tatggtgaat 360
cgaaaagacc ggggaagttt tccatggagg tgaagaagac agtggacaaa gggaaacggg 420
ttttggtgat gacaaatgac tactattata cagacatcca agggtactcc tttcagttta 480
gggtgtggcg cttttccaga ggtcatggga aatatttctt cccgagggaa tgtaacccat 540
ccgaagaagg cctgcatgac ttagaacatc ccgatgtgtc ctttggcaga tg 592

<210> 410
<211> 483
<212> DNA
<213> Homo sapiens

<220>
<221> misc_feature
<222> (1)...(483)
<223> n = a,t,c or g

<400> 410
gtacgggaga tatgattgac gcctttaagc tagnagtaac actacgatta ctgaaaagaa 60
tccggccctc gaggccaaga aggcggaacg aggtaacaag cttgcttaga gatgcagcaa 120
atattacgga gatggatagt gaaaattctg caaaagaagg tgatcctgga acaatattct 180
tcttcaggga aggagctgct gtgtttcgga atgtgaaaga caaaactatg aagcatgtga 240
tgaacgttct acaaaaacat gaaattcagc cctatgaaat cgcactggta cactgggaaa 300
atgaagaact taactacata caaatacagg gacagtcaaa acttcacagg ggggaaatca 360
agttaaattc atagctggat ttacatgatg ccattctaga gaagtttgct ttctccaatg 420
ctctatgcct ttctgtaaaa ctggcaattt gggaagcatc actggataaa tttattgaat 480
cta 483

<210> 411
<211> 465
<212> DNA
<213> Homo sapiens

<400> 411
cccacgcgtc cggaaaaact gatcagtcaa atacagccag aggtggaccg agaaagagct 60
gttgtggaag tgaatatccc tgtagaatct gaagttaatc tgaagctgca gaatataatg 120
atgctacttc gtaaatgttg taatcatcca tatttgattg aatatcctat agaccctgtt 180
acacaagaat ttaagatcga tgaagaattg gtaacaaatt ctgggaagtt cttgattttg 240
gatcgaatgc tgccagaact aaaaaaaaga ggtcacaagg tgctgctttt ttcacaaatg 300
acaagcatgt tggacatttt gatggattac tgccatctca gagatttcaa cttcagcagg 360
cttgatgggt ccatgtctta ctcagagaga gaaaaaaaca tgcacagctt caacacggat 420
ccagaggtgt ttatcttctt agtgagtaca cgagctggtg gcctg 465

<210> 412
<211> 844
<212> DNA

<213> Homo sapiens

<400> 412
tcacattaat aaggtttctc cccagtatgg gttctctcga cgacataata agagctaagt 60
tttcacagaa gttttgcttg cattcaaggc actggtatat atgaccatct atctgaataa 120
tctcatgcat atgaataaaa atctttttac ccttccgagg gcatacatta tatattttcc 180
tttttgagt tctctgatta cgatttctct tctgaagtgt acattttcta tggctctctc 240
ctagggagtt ttccgctggt cttcttgacc catcgttgcc ctcacagtca ttttcttgat 300
aagaacttgg aagatacatc tgtatcaggc ctatcaatca tgagcctcta caagtatcca 360
gcatcatata tgttaattcc ttacttggta tttccaaccc tagaatccag atggaagaaa 420
acatacattc ctgatcctga caatttgact tctcaaagaa gatagcattc gtaacaagaa 480
agactcaaga ccatcttccc cctcctcctc aaaaggtctc tagttcagaa ctattcacaa 540
cactcaattc ttgcctctcc ctggcagccg ccgcgcctgc cctctaggcc ttggggacag 600
cacttccggt tactctccgg acactcgaat ctgggcttcc tggcggccgc agactctgcg 660
atagtagatc taagggccag gcttcgggaa ggaaagaaaa gcgaacccgg cacggacaag 720
acctaggagc aactcccttc gtgcgcctag actagaggag ttggggccag gccgcccaca 780
gagcgcttta acagagcgct tcaagggccc acaaggccgg ctacgcaaac cctttcaagc 840
tggc 844

<210> 413
<211> 1452
<212> DNA
<213> Homo sapiens

<400> 413
cttgtatctg aacatgcctt tgaaattcca gacaatgtta gacctggaca ccttattaaa 60
gaactttcta aagtaattcg agcaatagag gaggaaaacg gcaaaccagt taaatctcag 120
ggaattccta ttgtgtgtcc agtttcacga tcctcaaatg aagcaacttc cccataccat 180
tcccgaagaa agatgaggaa acttcgagat cataatgtcc gaactccttc taacctagac 240
atcctagagc tccacacaag ggaggacatc ttgagctcta aactgaatgg aaaattcaac 300
aaacatctcc aaccatcctc cacagtacct gaatggagag cgaaagataa tgatctacga 360
ttactgctga caaatggaag aataattaaa gatgaaaggc agccctttgc agatcaaagt 420
ctttatacag cagatagtga aaatgaagag gataaaagaa ggacaaaaaa ggcaaaaatg 480
aagatagaag agagttcagg agtagaggga gtggaacatg aagaatctca aaaaccactg 540
aatgggtttt ttacacgtgt gaaatcagaa ctcaggagta gatcatcagg atattctgat 600
atttctgagt cagaagactc cggacccgag tgcactgcac tgaaaagtat ctttaccact 660
gaagagtctg aaagttcagg tgatgaaaag aaacaagaaa taacatccaa ctttaaggag 720
gaatctaatg tgatgaggaa cttccttcaa aagagccaga agccatctag aagtgaaatt 780
ccaattaaaa gggaatgtcc tacctcgacg agcacagagg aagaagctat tcagggcatg 840
ctgtctatgg cagggttgca ctattccacg tgtttacaaa ggcaaataca aagcacagac 900
tgcagtggtg aaagaaactc tctccaggat cccagcagct gccatggcag taaccatgag 960
gttaggcagt tgtatcgcta tgataaacca gtggaatgtg gataccatgt caagactgaa 1020
gatccagact tgaggacttc ctcctggatt aaacagtttg atacttccag atttcatcct 1080
caggatctaa gtagaagcca gaaatgcatc agaaaggaag gttcatcaga aattagtcag 1140
agggtacaaa gtaggaatta tgtggacagc agcggctcaa gccttcagaa tggaaagtat 1200
atgcagaatt caaacctgac ttcgggggcg tgccagataa gtaatggcag tctaagccca 1260
gaaaggccag ttggtgaaac ttccttctcg gtgccccttc accccaccaa gagaccggca 1320
tcaaatccac cacctatcag caaccaggca acaaaaggta aacgtccaaa aaaaggaatg 1380
gcaacagcca aacaacgtct tgggaagatc cttaagttga acagaaatgg ccatgcacgt 1440
ttctttgtgt ga 1452

<210> 414
<211> 878
<212> DNA

<213> Homo sapiens

<220>
<221> misc_feature
<222> (1)...(878)
<223> n = a,t,c or g

<400> 414
caaacnccag tcagggccct gtaaaaaaaa ggcaccccat ttccctttta tcaagctttt 60
cggggtatgc tgtcggccct ccacaatgtg gttcaaaaac caggaaaaag tgtcttaact 120
ggaggcctta atgcgttgaa tttcatcggc aaaaaaccac ataatgtcct gccaaaaagt 180
gaccgcgttt taaccgggac aaagcggcca tgggagagac ctgttccctt gtctcagatg 240
ttaaggggaa gctaaggaga aggagaagca gagactggca ccagcagctc acgatggagg 300
agaacccgcg cactaccggg atgctgtttg atgaatatca aggcttgtca ccccctggaa 360
gccctggaaa ttctgtccaa tgaaagcgaa agcaaggttc agtcattttt agcatcactt 420
gatggagaga agctggaact cttaaaaaaa tgacctaatt tccattaaag acatctttgc 480
agccaaagaa ttagagaatg aagaaaatca agaagaacaa ggcttagaag aaaagggaga 540
agaatttgct cgcatgctta cagagcttct ctttgaatta catgtggcgg ccacacctga 600
caaactcaat aaggccatga agagggctca tgactgggtg gaagaggatc aaaccgtggt 660
gtcagtagat gtggcaaaaa gtgtccgaag aagaaacaaa gaaggaagaa aaggaagaga 720
aatctcaaga ccctcaagaa gacaaaaagg aggaaaagaa aactaagacc atagaggaag 780
tatacatgtc gtccattgaa agtctggcgg aggtaacagc gcgctgtatt gagcagcttc 840
ataaagtagc agaattaatt cttcatggac aagaagag 878

<210> 415
<211> 497
<212> DNA
<213> Homo sapiens

<220>
<221> misc_feature
<222> (1)...(497)
<223> n = a,t,c or g

<400> 415
ggcacgagcc ttaatctagc agtaatttat caaaaaccca tatgataaga gaattatttt 60
ataatagtgt tggtaaagaa aatctaaaat agaaaaaaca tgccaacaat tgattggcta 120
atgaggtggg tgatgccaga tgcaagactg ctgggcctac cacaggagca agagagaagc 180
catgcattcc atctcaaggc atctgtgagc ccatgtagac accatgagca aagctcaccc 240
tcctgagttg aaaaaattta tagacaagaa gttatcattg aaattaaatg gtggcagaca 300
tgtccaagga atattgtggg gatttgatcc cttaatgaat cttgtgatag ataagtgtgt 360
ggagatggca accagcgggc aacagaagaa tattggaatg gtggtgatac gaagaaatag 420
tatcatcatg ttagaaacct tggaacaaga ataaataatg gctggtcagc agagaaattn 480
cacgtcccct cttcaaa 497

<210> 416
<211> 819
<212> DNA
<213> Homo sapiens

<400> 416
atgcgggatg aagacaagcc tgaggacgag atggcccaaa agcgggccag cctgctggag 60
cggcagcagc ggcgagcaga ggaggcgcgg cggcgcaagc agtggcagga ggtggagaag 120

gaacagcgga gggaggaggc cgcgaggctg gcccaagagg aggccccggg cccagccccg 180
cttgtgtccg cagtcccgat ggcgactcca gcccctgctg cccgggctcc agccgaggag 240
gaggtgggcc cccggaaggg ggacttcacg cggcaggagt acgagcgccg ggcccagctg 300
aagctgatgg acgacctcga taagcacgcc gcgctgcgtg gtatccagcg ccttaccgac 360
tcggggcatc aagtcaataa cgtcgaggtt attgcccgtc gttgtgagcg ttttgcgggt 420
acggaaacag agcgtctgga agatctcaat tcccttgcca gactgactac ccccaacacc 480
atcgtgctgg ctgtacgcgg cggttacggt gccagtcgtt tactggcaga tattgactgg 540
caggcactgg tggctcgcca acaacatgac ccgttgctca tttgcggaca tagcgatttt 600
accgcaattc agtgcggtct tctggcgcat ggcaatgtca tcacctttag cggccccatg 660
ctggtagcaa actttggcgc ggatgaactg aacgccttta ccgagcacca tttctggctg 720
gcgttacgca atgaaacctt caccattgaa tggcaagggg aaggcccgaa atttcggggc 780
cgaaggcaca gttgtgggga ggcaatcttg cgatgctga 819

<210> 417
<211> 1170
<212> DNA
<213> Homo sapiens

<400> 417
tacaatagta aaccaagtct ttattctttg aagaatgtac cagagcattt agcaaaattg 60
ttttaaaatg gagggaggta aaaggcaaat gtaacttaaa ttcgggttat gctttcggag 120
tatgaaccat tgaggaaaga attatcaaaa tcccaaatca aatgcagtgt ccctcttgtc 180
tcacttgtgg ctagcaaata cagcagcagc cttgagaata aatacagatt tgctgcctac 240
tagctaccag ctggtcaagt ttgaagatat tgtgcatttt cctcagaaaa ctactgaaag 300
gattttgcc tttcttggaa ttcctttgtc tcctgctagt ttaaaccaaa tattgtttgc 360
cacctctaca aacctttttt accttcccta tgaaggggaa atatcaccaa ctaatactaa 420
tgtttggaaa cagaacttgc ctagagatga aattaaacta attgaaaaca tctgctggac 480
tctgatggat cgcctaggat atccaaagtt tatggactaa atgctgcagg tcagcagaaa 540
tttgcactaa taatacttac caacccactt tgtggatatg aatcagaaga gtttgtttat 600
tctttactgt gtgtgtgtgt gcgcacgcga gtatgtgttc aatgttgctc gcacagagag 660
attgttctaa aaaatggcac catatttggc ctaacaggat ttatttttat gtcatcacct 720
cccttgcctt tgtttctgaa aattttgtct gctaaaaaga ttctgctaca gagaggtaga 780
tgaagttata tcatggggtc aggggagatg ggaaaatttt aagtttttgt ctaactcccc 840
ttcatctgta actgtgctaa tctatctaga gacctcaaac actgctaaag gccttgcaat 900
tgctgcttta cccacgcatc tcttgctttc aagatggact acaaaagttc cttatccttt 960
tgaaaaggtc ttctgacaca cttatcttgc acaaagaaaa agaaaatttc ttttactgtg 1020
tttaatgttc agtgatatca ctgaggaaat ggtgaaagct cctatcagaa ctataggatt 1080
tcttctggga aatacagatg gaaatacaga atgaatatgt tttttgagg tcggaaactg 1140
actttaaaag cctccttgaa gtttttacg 1170

<210> 418
<211> 356
<212> DNA
<213> Homo sapiens

<400> 418
cggttcttga agaaacatat gaatgtattc gggggacaa gggcttccag ttttttcac 60
aacaggtcag ccaccatccg cctatctctg cgatgtcatg ctgagtctat aaatgttggt 120
tactggctag atgcgagatg gacttacttt actctgggcg caaatccatg caaattgttc 180
caattggcac aaaccatgtg actctgccac tttattgggg atcatattga gtggaacaaa 240
ctgacctctt gcatccatat catcttaagc gtacagaagt ggattgagca ctatggagag 300
attgccatca agaacctgct tgatgattgc tgctactgcc atacgaattt tataaa 356

<210> 419
<211> 535
<212> DNA
<213> Homo sapiens

<400> 419
ataaatacgt tattactttc aggggggaaa ggtggaaaaa ataagaaaaa taaaaactct 60
tctaaaccac agaaaaacaa tggatccact tgggccaatg tccctctacc tcccccccca 120
gtccagcccc ttcctggcac ggagctggaa cactatgcag tggaacaaca agaaaatggc 180
tatgacagtg atagctggtg cccaccattg ccagtacaaa cttacttaca ccaaggtctg 240
gaagatgaac tggaagaaga tgatgatagg gtcccaacac ctcctgttcg aggcgtggct 300
tctttctcct gctatctcct ttggacagca gtccactggc aactcttact ccattcccca 360
cgggaaggag atggcaaccc atggctggca ggcttcacct gggatggagt ttgacaagga 420
ggcctattca gttttgatta ttaggcaaaa caaacatggg cacatttcaa aggcaataat 480
tcaaccttcc acagccttcc agtttccacc gttaggttat tgttgttttg ggagc 535

<210> 420
<211> 2692
<212> DNA
<213> Homo sapiens

<400> 420
ctcgatttgt gccgtcagtc taacaacctg tgcttgcaaa gggaagagga tcttcagaga 60
acaagagatt accatgactg tatgaatgtt gttgaagtgt tcctagaaaa atttactaca 120
gaatgggata acttggccag atctgatgca gagagtacag ctgtccacct ggaagctttg 180
aaaaagttag cattggcatt gcaggagaga aagtatgcta ttgaagatct gaaagatcaa 240
aagcagaaaa tgatagagca tctgaattta gatgacaagg agttagtcaa agaacagacg 300
agtcatttag agcaacgttg gtttcagctt gaggacctca ttaaaaggaa aatccaagtg 360
tcagtcacca acttggagga gttaaatgtg gtgcagtcca gatttcagga gctaatggag 420
tgggcagaag agcaacaacc caacatcgcc gaggccctta agcagagccc tcctccagat 480
atggctcaga accttctcat ggatcacctg gccatctgca gtgaactgga ggccaagcag 540
atgctcctga aatcgcttat aaaggacgca gacagggtca tggcagatct tggtctcaat 600
gagcgacagg tcatccagaa ggctctctct gatgcacaaa gccacgtgaa ttgtctcagt 660
gacttagtgg gccagcgaag aaagtactta aacaaagcct tgtccgagaa aacccagttt 720
ctcatggcag tgttccaggc caccagccaa attcagcaac atgagcgaaa gataatgttc 780
cgtgaacaca tctgtctgtt accagatgat gtgagcaaac aagtcaaaac atgtaagagt 840
gcacaagcca gcctcaagac ttaccaaaat gaagtcactg gactttgggc ccagggtcgc 900
gaactaatga aggaagtcac agagcaggaa aagagtgaag tgctggggaa gcttcaggaa 960
ttgcagagtg tctatgacag tgttttacaa aagtgcagtc accggttaca agaactagag 1020
aagaatttgg tttctaggaa gcattttaag gaagattttg ataaagcttg ccactggcta 1080
aaacaagcag atattgttac atttcctgaa atcaacctaa tgaatgagag tactgagctt 1140
catacacaac tggctaaata ccaaaacatt cttgaacaat ctccagaata tgaaaatctt 1200
ctacttacgc tgcagagaac tggccagacc atattaccat cgctgaatga agtcgatcat 1260
tcctacctca gtgaaaagct aaatgctttg cctcgacaat ttaatgtaat tgttgccttg 1320
gctaaagaca agttctataa agtccaggaa gcaattcttg ctcggaagga atatgcttcc 1380
ttgattgagt tgacaaccca gtctctcagt gaacttgaag cccaattctt gaggatgagc 1440
aaagttccca ccgacctggc cgttgaggag gctctttctc tgcaagatgg ttgcagagcc 1500
attctggacg aggtggcggg ccttggggag gcggtggatg aactgaacca gaaaaaagaa 1560
ggttttcgca gcacaggtca gccttggcag ccagacaaga tgctgcacct tgtcacctta 1620
tatcacaggc tgaagcgaca aacagaacag agggttagct tattagaaga caccaccagt 1680
gcttaccaag aacacgagaa gatgtgccaa cagctggaga gacaactgaa gtctgtaaaa 1740
gaggagcagt ccaaagtgaa tgaggaaacg ctgcctgcag aggagaagct caaaatgtat 1800
cactccctgg caggaagtct ccaggactca gggattgtac tgaaacgagt aaccatacat 1860
cttgaagatc ttgccccaca ccttgacccc ttggcttatg agaaagccag gcatcagatc 1920
cagtcctggc aaggggagtt aaaactgttg acttctgcca ttggtgagac ggtgacagaa 1980

tgtgagagcc gaatggtgca gagtatagac ttccagactg agatgagtcg ctccctggac 2040
tggctgagga gagtgaaggc agagctcagt gggccggtgt acctagacct caacctgcag 2100
gacatccaag aggaaatcag aaaaatccaa attcatcagg aagaggtcca gtccagcttg 2160
agaatcatga atgcgctgag tcacaaggaa aaggagaagt tcacaaaggc caaggagctg 2220
atttctgcgg atttagaaca cagcctcgct gagctctcag agctggatgg agacatccag 2280
gaagccttac gcaccagaca ggctaccttg actgaaatat atagccagtg tcaaagggta 2340
ttatcaggta tttcaagcag ccaatgactg gcttgaggat gcccaagaaa tgttacagct 2400
ggcaggcaat ggcctagacg tggagagcgc agaggaaaat ctcaaaagcc acatggaatt 2460
tttcagtaca gaggatcagt tccatagtaa cctggaggag ctccacagcc tggtagccac 2520
cctggaccca ctcatcaagc caaccggcaa agaagaccta gaacagaaag tggcttctct 2580
ggaactcagg agccagagga tgagccggga ctctggtgcc caagtggatc tcttgcagag 2640
atgcacagct caatggcacg attaccagaa agcaagggaa gaggttattg at 2692

<210> 421
<211> 1551
<212> DNA
<213> Homo sapiens

<400> 421
ggtaaacatg ttgcttggaa gtatgctttt ccagtttcac aaaatgacac cagtggccac 60
agaagttagt accctttggc ctctgaaaat ttcgctttat gaagagaagg ctactaaaag 120
acatgtagga cttagggttc aaggtggcag actagaggag ctagtgtgta ctgctctcgt 180
ggagaggaaa tagtgacgag taaacatgga ctctctaagt ggatcatcta agaaaccacg 240
tcaggattca ggatgcatca agggagtaat gggacatgga gaacagagaa aatcgaagct 300
gggcagctgc ctgcctggga ccagcatgaa gacaggagaa gctcctcaac acagggaaag 360
aattagtgag taagagtcct caggggatcc atacttccca cagtgacctg tgcaatcctg 420
agaatgtgaa aaaccccatg actcccttgg gcctctagac tgatacagag agctacccag 480
agtttttgca gaggcaacac tcaagtccgt ggggaccccc ccaggccctg gactctggaa 540
cagcctggtt gaggtgccat ggccctgaca gaagctgcag tcatggtgct ggagatggac 600
agattgctct actccctctt gacagaaaac ctgctaaacc cctgtggttt aggagagctt 660
tgtgttcccc caggaagcac ttcgatggtg aattgggtga ctccactcat cctgctccta 720
actgggcagg attcaacttc accctgctcc taacttgggc agagccccaa gcagggggtg 780
gggaggacgc cctccctctc agaatactga aaaggctgga taacgcagat gaacaagcag 840
cccagatcag aagggaactt gatggccggc tgcaattggc agataaaatg gcaaagaaat 900
atgtgaatgt tatattggag gagaagaaaa gcaggtggcc tgagactctg ggactgatct 960
gtgaagtggt gccagatgtt ccttccaatc ccatgaatga actggtgcaa gaaagaaaat 1020
tccccaaatt tatagcaaaa gatatggaga atatgtatat agaagagttg cggtcttcag 1080
tgaatttgct aatggccaat ttggaaagtc ttccagtttc gaaaggtggt ccggaattta 1140
aattacaaaa attaaaacgt tcacagagac tctgcggttt tggacatagg agatgagaat 1200
gagattcagc tgtcaaagtc cgacgtggta ctgtcattca ccttagagat tgtcataatg 1260
gaagtgcaag gcctgaagtc agttgctccc aatcgaattg tttactgtac aatggaagtg 1320
gaaggagaaa aacttcagac agaccatgcc gaagcctcaa ggccacaatg ggggactcaa 1380
ggagatttca ccaccaccca tcctcgggct gtgtgtcaaa gtgaaactct tcacagaaag 1440
cactggagtt ctggccctgg aagataaaga actggtaagg gtgatattat acccaacttc 1500
taatagctcc aaatcagctg aattacaccg aatggtagtt ccaaaaaata c 1551

<210> 422
<211> 392
<212> DNA
<213> Homo sapiens

<400> 422
agctgagcgg cggggggcag tgtcgcctcg gtctagggac ggcggcggag tcggtggtcc 60
ctgcgcgatg gccacctcgg tgttgtgctg cctgcggtgc tgcagagacg gcgggactgg 120

ccacatccct ctgatggaga tgccggccgt gcatttgctc actcagctga tgggttctga 180
tgtttgcatt gtgcctagct gcctaccttt atgcgggatc tgagtctggt tattgctttg 240
tgcgatccct cgctgtctga tcttttttac cttctggtgg ggtgttgggt aatgtttctt 300
ttgagggtct cttggatccc tggtcgtctt gtttagcttt tatggctgtt ggcccttttg 360
tctctctggg ggatctgtca tttcctctac cg 392

<210> 423
<211> 665
<212> DNA
<213> Homo sapiens

<400> 423
aggggaaagc atgcagtgat tagaggagtc actctcataa actcggccag ttgtaggctt 60
gtcactctct gtggatgcta actgagactc agaagagtcc cttctcagtt ccatcaacaa 120
gcagggcatt gaagaaataa ctgtagaatc cgctacacca tctttctgaa cttgagattc 180
cagttctaca gatccatctt cctccttggc tgtctcttgt tcagatgctt ttggtggggc 240
ttcggactca atgagttctt caacttttcc cactgcctcc tccatcttca tttcagaatt 300
tcaagttaaa tcatggagta gagtctagga aatgatatca aagatgcaac aggaaagcaa 360
cctgcgctcc gcggcgactc cgtgccttct cgctcgcttc gcgcgcgccc cgtcggggca 420
cgcaccgaac ccccggccgg gactcttccc cgctccggtg actcaggacg cgacggccac 480
cgccgccttc tcctcctcgg aacaagcccc ggcagcgagc agtgggtcgc ggtcagagcc 540
cctgccggga ccaaagtgaa cctcgccgcc tccgcagcct ctaccaccaa gtcccttcag 600
tcgtccgcgc cgcccccaag aaaatgtcac cagaggccgt tccgctgggc gcctcacttc 660
cgccc 665

<210> 424
<211> 1269
<212> DNA
<213> Homo sapiens

<400> 424
ccgagacgca ctgggggccg gatgtagaat cctgcttatc tgtgaaatgc agttaacaca 60
tcagctggac ctatttcccg aatgcagggt aacccttctg ttatttaaag atgtaaaaaa 120
tgcgggagac ttgagaagaa aggccatgga aggcaccatc gatggatcac tgataaatcc 180
tacagtgttt cactcttgtt gcccaggctg gagtgcaatg gcgcgatctt ggctcacggc 240
aacctctgcc tcccgggttc aagcagttgt cctgcctcag cctcctgagt tgctgggatt 300
acagattgtt gatccatttt cagatacttg tggcacgcaa acaaagcagt tcaccctcta 360
caaacttggg aaaaatggaa gacaatgaac tctatctact gaaatttatt ttcaaccctt 420
tcccccaaat aaccaatatt tcacgaggct ttgaaaaaat tttggtatct cagcaaattg 480
acactttcaa ttcctaattg tttacattga agagggagaa aaacaaaata aatcaagaat 540
acctaatatc tcaagtagaa ggtcatcagg tttctctgaa aaatcctcct cgaaataatg 600
aataagaccg aaagtcgaca aagatatata aactctcttc acaagaagaa agtattggga 660
cattattgga tgctatcatt tgtagaatgt caacaaaaga tgttttatga aatgtccaga 720
aatattaaca aaaattctca gcattaaaga aaacaattga ttttcctttc cgtgcctata 780
caactaaaag tcaccaatag aaaagtttaa atcacagaat ggtattaaaa aaaaaaaaaa 840
aaggccaggc gcggtggctc acacctgtaa tcccagcacc ccgggaggcc aaggcaggca 900
gatcacctga ggtcgggagt tcgagaccag cctgaccaac atggagaaac cctgtctcta 960
ctaaaaatac aaaattagcc aggcatggtg gcgtatgcct gaaatcccag ctactcagga 1020
ggctgaggca ggcgaatctc ttgaacccgg gaggcggagg ttgcagtgag ccaagatcac 1080
accattgcac tccagcctgg gcaacaagag tgaaattcca tctcaaaaag aaaccaaaaa 1140
aacaaaaaaa aaacatgccg tttgagtact gtgtttttgg tgttgtccaa ggaaaattaa 1200
aaacctgtag catgaataat gtttgttttt catttcgaat cttgtgaatg tattaaatat 1260
atcgctctt 1269

<210> 425
<211> 1263
<212> DNA
<213> Homo sapiens

<400> 425
ggcttttaa atgacccagg cgtgggtaaa gcatcggtcc aggcatcggt ggcggggaca 60
ggcctaggca gcccgggcca cccagactgg cccctaatga gctcccaagt ggcctgtggg 120
gacttggcct cagttttgcc agctgtgaac tgggaccgtc gctgctaagg ccatggacac 180
cttcagcacc aagagcctgg ctctgcaggc gcagaagaag ctcctgagta agatggcgtc 240
caaggcagtg gtggccgtgc tggtggatga caccagcagt gaggtgctgg atgagctgta 300
ccgcgccacc agggagttca cgcgcagccg caaggaggcc cagaagatgc tcaagaacct 360
ggtcaaggtg gccctgaagc tgggactgct gctgcgtggg gaccagctgg gcggtgagga 420
gctggcgctg ctgcggcgct tccgccaccg ggcgcgctgc ctggccatga cggccgtcag 480
cttccaccag gtggacttca ccttcgaccg gcgcgtgctg gccgccgggc tgctcgagtg 540
ccgcgacctg ctgcaccagg ccgtgggtcc cctctctgat ctccaagtcc cacggccgca 600
tcaaccatcg tgttcggcca cctagccgac tgcgacttcc tggctgcgct ctacggcccc 660
gccgagccct accgctccca cctgcgcagg atctgcgagg gcctgggccg gatgctggac 720
gagggcagcc tctgaacccc ggcgccgccc aaccgcgccc ctcgcgcctt ttggggctct 780
cctgctgggc gcgggtgggg tttgtgggtt tttttccacc tcttttctcc caatcggact 840
ccggccaaac tcccctagac agatgggtga cctgtctcct ttgagaggat gctgaggcat 900
ctgtagcagc tgtttcaaac accaatgtca cctctcctcc tggcccccgc ccaatgggga 960
gaggaattcg gggccctact ctggggacca cctttcaccc gtttgtactt tctgggccac 1020
gccgacccct gggtcgcttg atgtaaaagc caaaagctgc tgcctcccac ttggatcatg 1080
tcgcctggga ttttcatccc tcgcacaagg actaggggtt cacacggtga actgggggaa 1140
gggaagtgtt agggggcaag tcgcggcacc cccccttcca taaactcacg tcctaacccc 1200
caggacctca gaagatgatc tgatttggaa ataggatcat tacagatgga attagttcaa 1260
aaa 1263

<210> 426
<211> 1542
<212> DNA
<213> Homo sapiens

<400> 426
ctaggctggt aagggccggg aaggcgcgaa cctgttcgca ggaaaagcag gcgagtgtgc 60
ttgggcaatg ctcagtacct tgaggcctgg cttgaccact gctgcagctc ggcgagccaa 120
gaacgccctg tcgcccacga cacgtgcgta ggcgcaagct ggaaagtgca agtcctgtgg 180
cttccagagg cagggaggca tggtgccggt tatctttagg tcctcgagcg tcagttcctt 240
gagcgggagt acgctagcac acgcagggcg cctaccagta gctgagcagg acctgtgcgg 300
ctcccactgt gagccgccgc acgcggagag ccttgaccgt tcagcatact gccgcgggtc 360
ggcgtccgca tcgacgcgct ttagaaacgc tctaggttga gaccgccggc atggatctcc 420
acctctactg cagacacaac tggaaggctt cggaccagtc gggctgaggt tcggagaagt 480
tgcagaccag cggaaatctt catcgtccag ctcacaaggt tctggcgtgg tcgcaggacg 540
tgcaccagcg gcagcagcag cagcaacaag caggacgcgc gctttgtcaa cgtccatggg 600
ttggtttctt tctggggctt ttccatcagt tctcttcctc tttctctttg ctggcaatct 660
cagctcgtac cttttctttt tcctccttcg tgcagctggg tcttcatcct catgtactcc 720
cttggcatct atggtgacgt gacttagaag gtggtgcaga ggtaggtgca gccctggggt 780
catgagaagt ttcctgaagt cttcattggt gagttttgat ctggaattgg cgacttcgat 840
attaacaagg atgcggcggc cgcagcaagt cgaggagtcg ggcaaagct gggcccttga 900
agagctgttg ctggagagca tacacacgat cctggagctg ggcctcttcc tgatgccttc 960
cagggcttgc agccctgact gctgcaggaa gacctgaaca gccattacgc tcaccacgtt 1020
gatactggca acagcagtga ggttgttgag gagccaaagg ccctcaggga gcagctgggc 1080
tgtttctgga aaaagaactg cagaaggata aataaggctg ccaaacacgc tcatctctga 1140

gctgcatttg ccctcccaca gtctccactg ctcctcagtt agcaggttgc ttagacatcg 1200
aagcacgggg catgccagcg gagaaggttt gctgtgtttc agggtgctgc aagcctcgac 1260
gaaggctgac cgagccccct ctctctcctt ttcctctgtc ccccgctggg cttgccgcag 1320
aactgctgca cctgcctccc gctcccgccg gcggcgccgt agctcctcac cccgcagacc 1380
gtgggcctga ggcggccctg ccgtcggctc cacatgggga gcaggaggag gaaagccgaa 1440
ggaaacagca gccacaacgc ggcggtcaga gtttaggtta tctctcttat tccctgaaca 1500
attgctgtta tcctgttctt tttcaaggtg cccggatttc at 1542

<210> 427
<211> 367
<212> DNA
<213> Homo sapiens

<400> 427
tctggctctt cctggagacc gggcagctcc ccaaagacag gagcacagat cagagaagct 60
aggagagctc cagcaggggc acagaggatt gggggcagga ggagtctgga acacagcctt 120
catgccccct gaccccaggc cgaccctccc cacaccctag ggtaccccag tcgtatcctc 180
tgtccgcatg tgtggccagg cctgacaaac acctgcagat ggctgctgcc ccaacctggg 240
acacggggtc gaaggggcct gtacactctg tcatttcctt tctagcccct gcatctccaa 300
caagtccaag gtgacagctg gtgctagggg cgtggggtta ataaatggct tatccttctc 360
tccaaaa 367

<210> 428
<211> 489
<212> DNA
<213> Homo sapiens

<400> 428
ggcacgagca catactcaca gagaacccac catggtgctg tctcctgccg acaagaccaa 60
cgtcaaggcc gcctggggta aggtcggcgc gcacgctggc gagtatggcg cggaggccct 120
ggagaggatg ctcctgtcct tccccaccac gccgacctac ttcccgcact tcgacctgaa 180
ccacggctct gcccatgtta agggccacgg caagaacgtg gacgacgcgc tgaccaacgc 240
cgtgacgcac gtgtactaca tgcccaactc gctgtacgcc ctcagcgacc tgcacccgca 300
caatcttcgg atggaccctg tcaacttcat gctcctaagc cactgtctgc tgtagaccct 360
ggtcgtccat cttcccgccg agctcactcc tgctgtgcac gcctccctga acaatgtcct 420
cgaatctgag aggactgagc tgacctctag cacctcatga ctaggctctt cggagtcaag 480
cctctgccg 489

<210> 429
<211> 489
<212> DNA
<213> Homo sapiens

<400> 429
ggcacgagca catactcaca gagaacccac catggtgctg tctcctgccg acaagaccaa 60
cgtcaaggcc gcctggggta aggtcggcgc gcacgctggc gagtatggcg cggaggccct 120
ggagaggatg ctcctgtcct tccccaccac gccgacctac ttcccgcact tcgacctgaa 180
ccacggctct gcccatgtta agggccacgg caagaacgtg gacgacgcgc tgaccaacgc 240
cgtgacgcac gtgtactaca tgcccaactc gctgtacgcc ctcagcgacc tgcacccgca 300
caatcttcgg atggaccctg tcaacttcat gctcctaagc cactgtctgc tgtagaccct 360
ggtcgtccat cttcccgccg agctcactcc tgctgtgcac gcctccctga acaatgtcct 420

cgaatctgag aggactgagc tgacctctag cacctcatga ctaggctctt cggagtcaag 480
cctctgccg 489

<210> 430
<211> 2231
<212> DNA
<213> Homo sapiens

<220>
<221> misc_feature
<222> (1)...(2231)
<223> n = a,t,c or g

<400> 430
tttttttttt ttttttttg tgtttaagag tttattagga taggcacata acagaaagct 60
ccaaaggaca acaaagctgc accttcctaa aggctgaccc tcaaagcacc atttgttag 120
aactcagagg agaagctcga tctctgagcc ttccctcctc acagtttggg aaacatgcac 180
aggatgaagg taaatcctgc atagaaatct tgtaattcca gaattagtca aacaaaattt 240
gaaatacaca catttaaaaa tacttacatt cntccatttt agcgttccat gaagtgatta 300
cttgacattt acaattacat cttgacattc acagagcaac gcctttggca ggggatctaa 360
ttccaagctt aagcccaaat ctatgcaaag gaccttcagc tccagaggaa tagtggggca 420
aaaatctggc tgaaaaggaa ctgcttgcca ccttggcaga taagagcaac agcacaataa 480
aggggattcc cagagctttc tacctaaaaa tagaagggca ttatctacaa gaacagaccc 540
aaaacacaat ttatttttaa acaagcccct tcagtgaagt catttgtcct caggaatcct 600
agtatgaaaa tagtgtcttt gattagtgaa tatatggaga attcggatta tcatctctat 660
ttttagttga gaaaatgcac aaaccatata gatttaaacc atgggttttg cagccgaagt 720
agagacagat gttttctggg atcatatctc agattcccaa gctaaagcta ttgttcaagg 780
tgatggaaat atgtaggggg cattaaacca ttttgcagct gctagaaagc tgtgttttcc 840
tgttttcctg atttagacaa tggagaagct aagaagagaa ataataggaa aggctttcct 900
atgttagttt taaaaagact gtgttatttt taaatccaat tttttttgtc caaacctcca 960
tctcctgttc tccctagttt tccacagaaa tatttaacag gaataaaggt tgaaaacaaa 1020
agtacctaat gatcatctcc catccatcga aagattgcct cagggataag acactcaaac 1080
aagggcttgt gatgaggatg gaaaagacaa agcaacttga agagaaaaaa aaaattcttt 1140
taaactcagt tcatcctgca aaatggacat aacacatgca tataaaggtt tgacttttta 1200
attccacttt tttgcctgat cgcaggggaa gatgggcatg tgggctgggc ccttcctgaa 1260
gccttgctcc ctccaccact gggcttaaca gacaggcctc caggtggcct tgcaaaggcc 1320
cgaacagtgg accgccagcc ccacttcccc cacaccctta aatccagagc ctccatgcag 1380
ttcatccccc acattacccc aaacataacg tccgtaagtg cctacacgta gatgtggttg 1440
catcatgatg ctatggcaat gcgtatgtcg cttaggtgtg tgggccaccg aaggcagagc 1500
ccagatgtgg ttggtcttgc ctggaagcca agctgttgag caggggtgta gtccctctac 1560
agaggatgct caccaagttc taatgcattg tcgataactg tccctgccct ggtttttgtg 1620
cattcctcag attatctgtg gtaagtgtcc tcctgcatcc tttctcaagt gacaaagaag 1680
aaagaaagag cagtgatggc acctgaaatt caggaatgag taggcatggc gagctgggtg 1740
tgtgagatgt tctctcttta cattgtctgc tttgagatgt gggatgaggg gagggagaga 1800
gttggtcctg aatatattca gtttttttcca agagtctccc agaattattg acctatttgg 1860
acaacaaatc ttccattgtc ttggtgtccc atctcccaaa gtgaaaactt cccccgtccc 1920
catggcccct ggcttgttct gctgctgaac aggtcaccct gggcagaaac actgtgtcta 1980
gcgagaagtt agcaggtggc agggtgcacc tctccccata ctgtgaccct tggattcccc 2040
actgtgccac gcccgagcaa gctggggccc ctcaggacaa ggacgatttg ttaatggcct 2100
cttggccggt gggcatttgt ttacccacta ctgtagcccc aagatccccc aaacaagaat 2160
ttccatgctt caaagtaaca gcaaaaataa acaggagaat aaagcacgtg tttgagccca 2220
gtggtttcca t 2231

<210> 431
<211> 1023

<212> DNA
<213> Homo sapiens

<220>
<221> misc_feature
<222> (1)...(1023)
<223> n = a,t,c or g

<400> 431
agcccccccn attgaaaatc gagagtctat gccacgngcg ggaattcgct atatttcatc 60
ttttatcttt ttctttgaca tttaccaatc accaagcatg caccaaacac tgctttaggc 120
actggggaca caaaggggac agagccatcc tcctttgaca cctggtcttc agttctgtgc 180
ccaacgtata tagttttgac aatgaccagg ttggactgtt taatgtcttt caacttacca 240
cgtaatcctc ttgtagggat cacatctttc tttatgatat tgtatttctc tacctctaac 300
agtaaaaatt ccattcaacc cttaaagctc acttcaaatt cttctttgag aagtttttcc 360
tttctccgca cccagaggta catatttgaa ctctctttgt acttggaggg cacttctttc 420
gtggtagttc ttttattttt attaatctct gtatccatag atagtcctcc aacaaccaaa 480
ggttgggact ctgtcttaca tatctgggng ccccctcata gtgcagtaat aagtaagttg 540
attatatccg agctatgtaa ccttatattt tttaatggtt ggatatcact gagttttttt 600
tttttaagaa tttttttatt gaggtaaact tcacataaca taaaattaac tattttaaag 660
tgagaagttc agtgccactt agtattgtta acaatgttgc ataaccacca cctttattta 720
aagttccaaa aaaaaatgtt ctcctctaaa aggaaacccc catcccatta agcagatact 780
ctccattcct tccttcctcc agcccccagc aaccaccaat ctgctttctg tctatatggt 840
tttatctatt cttgctattt tatataaatc gaattgtatg agaccttttg tgtctggctt 900
ctttcactta gtacaagttt ttgagattta tttacatagt agcatgtatc aacacttcat 960
ttttatggcc aaataaaatt gtattatgtg tttatagcac aatttaaaaa aaaaaaaaaa 1020
aaa 1023

<210> 432
<211> 481
<212> DNA
<213> Homo sapiens

<220>
<221> misc_feature
<222> (1)...(481)
<223> n = a,t,c or g

<400> 432
aaaaccataa aatgttcccc agttttgcag cacgttaaat ttcaaaaaat taaattgaac 60
cagagtcccc tggcaccgtt gaaggaaaaa taaatagaaa tgtgttagtg ccaagtagta 120
atctttggca ttaggggatt gatacaattt tgacattaaa tcagaattga agtttaaaaa 180
ccaggcaata gtttacatta ataatttttt tttttttttt tttttaagat ggagtcttgc 240
tctgtcgccc aggctggagt gcagtggcgc gatctcggct cactgcaagc tccgcctcgc 300
gggttcacgc cattctcctg cctcagcctc ccgagtagct gggactacag gcgcccgcta 360
ccacgcccgg ctaatttttt ctatttttag tagagacggg gtttcaccat gttagccagg 420
atggtctcaa tctcctgacc tcgtgatctg cctgccgaat tcctcgncac ggcatacaga 480
t 481

<210> 433
<211> 589
<212> DNA
<213> Homo sapiens

<400> 433
aatttgttcc acgccaagcc cgagcgccgg gactcggcca acctgtgggt gctggtggac 60
tgcatctttc gcgacacctc cgaggacctt ggatttcagt gtgacgccgt gaacctggcc 120
ttcgggcgcc gctgtgagga actggaggac gcgcggcaca agctgcagca ccacctgcac 180
aagatgctgc gggaaatcac agatcaggaa cacaacgtgg tggcactgaa ggaggccatc 240
aaggacaagg aggaacctct gcacatagcc cagacccggc tgtagctgct ctcacaccgg 300
cccaacatgc agttggcccc gaatgcagcc cagttcagcc ccagctcaca ccccccaaca 360
cccccaagct tctacaagcg gagcagtccc tgcgcaacct caaggacatc cacatgagcc 420
tggagaagga cgtcaccgcc atgaccaaca gtgtcttcat cgaccgccag aagtgcatgg 480
cccatcgtac ttgctacccc accattctgc agctggctgg ctaccagtga gcagcggcac 540
ggtgcttccc cccaatcccc caaataaaca gtgcgttagc attcaaaaa 589

<210> 434
<211> 589
<212> DNA
<213> Homo sapiens

<400> 434
aatttgttcc acgccaagcc cgagcgccgg gactcggcca acctgtgggt gctggtggac 60
tgcatctttc gcgacacctc cgaggacctt ggatttcagt gtgacgccgt gaacctggcc 120
ttcgggcgcc gctgtgagga actggaggac gcgcggcaca agctgcagca ccacctgcac 180
aagatgctgc gggaaatcac agatcaggaa cacaacgtgg tggcactgaa ggaggccatc 240
aaggacaagg aggaacctct gcacatagcc cagacccggc tgtagctgct ctcacaccgg 300
cccaacatgc agttggcccc gaatgcagcc cagttcagcc ccagctcaca ccccccaaca 360
cccccaagct tctacaagcg gagcagtccc tgcgcaacct caaggacatc cacatgagcc 420
tggagaagga cgtcaccgcc atgaccaaca gtgtcttcat cgaccgccag aagtgcatgg 480
cccatcgtac ttgctacccc accattctgc agctggctgg ctaccagtga gcagcggcac 540
ggtgcttccc cccaatcccc caaataaaca gtgcgttagc attcaaaaa 589

<210> 435
<211> 642
<212> DNA
<213> Homo sapiens

<220>
<221> misc_feature
<222> (1)...(642)
<223> n = a,t,c or g

<400> 435
gccttcaagc gcgggacnan canaagtcat ggaccgcaac ccctcgccgc cgccgccggg 60
tcgcgacang gnggaggagg aggaggtggc cggtggagac tgcataggga gcacggtcta 120
cagcaaacac tggctcttcg gcgtcctcag cggactcatc cagattgtnn ccctgaaaac 180
accaaatcta gctcagatga tgaggagcag ctgacggagc ttgatgaaga aatggagaat 240
gaaatttgca gagtatggga tatgtcaatg gatgaggacg tggctttatt tctccaagaa 300
tttaatgctc ctgatatatt catgggagta ctggccaagt ccaagtgtcc tcgattaaga 360
gaaatctgtg tgggaatttt aggtaatatg gcctgtttcc aggagatatg tgtgtccatc 420
agcagtgata aaaatcttgg gtgttattgc actgtttgta tgattcagac ccacctactc 480
tgctggaaac aagcagaagt ggccagtgtt tgggttgaaa ggatccagga acatccagct 540
atttatgata gcattgcctc attatgtcag ttcacaatgt tacttctgtn aagtgggagt 600
gtgacagctc ttattgatag aacnaagttg aatggcagaa tg 642

<210> 436
<211> 134
<212> DNA
<213> Homo sapiens

<400> 436
tcgagtaaac catgggacta aagcttggct ccaaagcatt caggctgaac gaaaaacatg 60
gactgctatt gcataatacc aacgtgcatt ggacgagaac gatgctatgg aacctgcata 120
ggcgacacgg tcgg 134

<210> 437
<211> 403
<212> DNA
<213> Homo sapiens

<400> 437
gtgttatcac tactatggga gtttgccctc gagccaagag ttcggcacga gctgactctg 60
tcactccttt tccgtaagcg gagagctgac atgccacggg tgttagtgtt tcgtgccctt 120
ttgatggtcc tcatctttct ctttgtggct tcctattggc ttttttacgg agtccgcatt 180
ttggactctc gggaccggaa ttaccacggc attgtgcaat atgcagtctc ccttgtggat 240
gccctcctct tcatccatta cctggccatc gtcctgctgg agctcaggca gctgcagccc 300
atgttcacgc tgcaagtggt ccgctccacc gatggcgagt cccgcttcta cagcctgtga 360
cacctgagta tccagcgagc agcattggtg gtcctagaaa att 403

<210> 438
<211> 599
<212> DNA
<213> Homo sapiens

<400> 438
tgattcggac gagccgcaca ggggtcagct tgctggtgcc caatggagcc attccccagg 60
gcaagttcta cgagatgtat ctactcatca acaaggcaga aagtaccctc ccgctttcag 120
aagggaccca gacagtattg agcccctcgg tgacctgtgg acccacaggc ctcctgctgt 180
gccgccccgt catcctcacc atgccccact gtgccgaagt cagtgcccgt gactggatct 240
ttcagctcaa gacccaggcc caccagggcc actgggagga ggtggtgacc ctggatgagg 300
agaccctgaa cacaccctgc tactgccagc tggagcccag ggcctgtcac atcctgctgg 360
accagctggg cacctacgtg ttcacgggcg agtcctattc ccgctcagca gtcaagcggc 420
tccagctggc cgtcttcgcc cccgccctct gcacctccct ggagtacagc ctccgggtct 480
actgcctgga ggacacgcct gtagcactga aggaggtgct ggagctggag cggactctgg 540
gcggatactt ggtggaggag ccgaaaccgc taatgttcaa ggacagttac cacaacctt 599

<210> 439
<211> 135
<212> PRT
<213> Homo sapiens

<220>
<221> misc_feature
<222> (1)...(135)
<223> Xaa = any amino acid or nothing

<400> 439
Met Lys Ser Ser Ser Ser Ser Ser Ser Pro Ser Phe Gln Ser Lys Thr
1               5                   10                  15
Thr Met Gly His Pro Leu Val Gln Thr Gly Leu Ser Ser Ser Xaa Pro
            20                  25                  30
Met Ser Leu Cys Ser Ser Leu Leu Ser Ser Cys Arg Ser Trp Asn Pro
        35                  40                  45
Phe Pro Ile Cys Tyr Met Leu Pro Leu Gly Thr Gly Gly His Ser Val
    50                  55                  60
Asn Gly Ser Cys Leu Gly Ile Ser Phe Gly Asn Ser Ser Leu Ser Arg
65                  70                  75                  80
Ala Pro Ser Cys Ser Pro Thr Ala Ala Ser Ser Ser Ser Ser Ser Ser
                85                  90                  95
Ser Ser Ser Ser Ser Ser Glu Ser Thr Lys Thr Met Thr Ser Ser Met
            100                 105                 110
Phe Cys Ile Val Arg Ile Arg Ala His Ile Phe Lys Ala Met Asn Ser
        115                 120                 125
Phe Ala Ile Ala Leu Thr Ile
    130                 135

<210> 440
<211> 136
<212> PRT
<213> Homo sapiens

<400> 440
Asp His Arg Gly Arg Ser Arg Thr Asn Ala His Gln Lys Met Thr Arg
1               5                   10                  15
Ser Ala Gly Ile Pro Met Phe Phe Phe Gln Met Gly Asn Ile Pro Pro
            20                  25                  30
Lys Ala Lys Thr Pro Leu Arg Cys Ile Leu Glu Asn Trp Asp Gln Phe
        35                  40                  45
Asp Pro Gln Met Leu Arg Lys Lys Leu Leu Ile Phe Phe Cys Ser Thr
    50                  55                  60
Ala Trp Pro Gln Tyr Pro Leu Gln Gly Gly Glu Thr Trp Pro Pro Lys
65                  70                  75                  80
Arg Ser Ile Asn Tyr Asn Ser Ile Leu Gln Leu Asp Leu Phe Cys Arg
                85                  90                  95
Lys Glu Gly Lys Trp Ser Glu Val Pro Tyr Val Gln Thr Phe Phe Ser
            100                 105                 110
Leu Arg Glu His Trp Gln Leu Tyr Asp Lys Cys Asp Leu Cys Pro Thr
        115                 120                 125
Gly Ser Pro Gln Ser Leu Pro Pro
    130                 135

<210> 441
<211> 30
<212> PRT
<213> Homo sapiens

<400> 441
Ser Thr Tyr Leu His Thr His Ile Tyr Val Tyr Ile Cys Met Cys Arg

1               5                   10                  15
Tyr Val Tyr Met Tyr Met Tyr Thr His Leu His Thr Gln Ala
            20                  25                  30

<210> 442
<211> 134
<212> PRT
<213> Homo sapiens

<220>
<221> misc_feature
<222> (1)...(134)
<223> Xaa = any amino acid or nothing

<400> 442
Ser His His Cys Thr Ser Ala Trp Ala Thr Gln Arg Asp Pro Val Ser
 1               5                   10                  15
Arg Lys Lys Lys Lys Arg Asp Lys Leu Pro Arg Asn Pro Ser Leu Pro
            20                  25                  30
His Met Glu Ala Leu Ala Asn Val Asn Phe Pro Arg Lys Ser Phe Arg
        35                  40                  45
Pro Glu Asp Ala Gly Lys Arg Ile Trp Leu Ser Arg Gly Gly Phe Cys
    50                  55                  60
Val Pro Ala Ala Arg Pro Gln Thr Met Asp Thr Gly Pro Ser Cys Ser
65                  70                  75                  80
Ser Pro Gly Leu Gln Asn Phe Ser Pro Gln Arg Lys Glu Asn Arg Ala
                85                  90                  95
Cys Ala Cys Xaa Gln Asn Ala Gly Pro Ala Pro Lys Asn Pro Met Cys
            100                 105                 110
Val Arg Leu Lys Ser Val Gly Arg Pro Gln Gly Phe Gln Arg Lys Leu
        115                 120                 125
Lys Glu Thr Gly Leu Cys
    130

<210> 443
<211> 169
<212> PRT
<213> Homo sapiens

<220>
<221> misc_feature
<222> (1)...(169)
<223> Xaa = any amino acid or nothing

<400> 443
Asn Ser Ala Arg Gly His Arg Leu Arg Glu Ser Gly Pro Trp Cys Ser
 1               5                   10                  15
Ser Pro Ala Asp Lys Thr Lys Ala Gln Arg Pro Pro Arg Leu Lys Leu
            20                  25                  30
Gly Ala Thr Pro Gly Glu Tyr Gly Gly Glu Pro Leu Glu Arg Met Phe
        35                  40                  45
Leu Ser Phe Pro Thr Pro Thr Lys Thr Tyr Phe Pro Lys Leu Arg Thr
    50                  55                  60
Leu Ser Gln Gly Ser Ala Pro Asn Gly Ser Ala Pro Gly Val Lys Gly

65                  70                  75                  80
His Gly Gln Glu Arg Trp Pro Asn Ala Leu Glu Pro Lys Ala Val Ala
                85                  90                  95
Ala Thr Trp Asn Glu His Ala Pro Asn Gly Ala Val Arg Pro Xaa Ser
            100                 105                 110
Asp Leu Ala Arg Arg His Lys Leu Arg Val Asp Pro Val Gln Phe Ser
        115                 120                 125
Lys Val Pro Lys Gly His Cys Leu Ala Trp Val Asp Pro Gly Ala Val
    130                 135                 140
His Leu Pro Arg Leu Glu Phe Gln Pro Leu Ser Val Ala Arg Leu Pro
145                 150                 155                 160
Leu Gly Gln Val Ser Trp Val Ser Cys
                165

<210> 444
<211> 149
<212> PRT
<213> Homo sapiens

<400> 444
Ser Ser Ala Glu Glu Phe Ala Gly Ala Ala Gly Pro Arg Ala Ala Gly
 1               5                  10                  15
Cys Gly Leu Ala Pro Ala Ala Asp Gln Lys Pro Asp His Arg Leu Ser
            20                  25                  30
Leu Gly Thr His Leu Val Gly Thr Ala Ala Ala Glu Leu Gly Trp Pro
        35                  40                  45
Leu Gly Leu Arg Gly His Gly Glu His Gly Gly Glu Val Pro Glu Ala
    50                  55                  60
Tyr Pro Pro Thr Ser Met Ser Arg Ser Pro Pro Thr Val Leu Val Ile
65                  70                  75                  80
Cys Gly Pro Gly Asn Asn Gly Gly Asp Gly Leu Val Cys Ala Arg His
                85                  90                  95
Leu Ile Leu Phe Gly Tyr Glu Pro Thr Ile Tyr Tyr Pro Arg Arg Pro
            100                 105                 110
Asn Lys Pro Leu Phe Thr Ala Leu Val Thr Gln Cys Gln Lys Met Asp
        115                 120                 125
Ile Pro Phe Leu Gly Glu Met Pro Ala Gln Pro Met Thr Ile Asp Glu
    130                 135                 140
Leu Tyr Glu Leu Val
145

<210> 445
<211> 105
<212> PRT
<213> Homo sapiens

<220>
<221> misc_feature
<222> (1)...(105)
<223> Xaa = any amino acid or nothing

<400> 445
Phe Lys Gln Ile Cys Leu Phe Leu Leu Leu Phe Phe Glu Met Glu Ser
 1               5                  10                  15

Cys Ser Val Thr Arg Leu Glu Cys Ser Gly Thr Ile Ser Ala His Cys
            20                  25                  30
Asn Leu His Leu Pro Gly Ser Ser Asp Ser Pro Ala Ser Ala Ser Pro
        35                  40                  45
Val Thr Gly Ile Thr Gly Thr Cys His His Ala Gln Leu Ile Phe Leu
    50                  55                  60
Tyr Phe Tyr Tyr Met Thr Gly Phe Arg His Val Ala Gln Val Gly Val
65                  70                  75                  80
Glu Leu Leu Gly Xaa Ala Ile His Leu Pro Trp Leu Pro Lys Val Leu
                85                  90                  95
Gly Leu Gln Ala Pro Ala Pro Asp Leu
            100                 105

<210> 446
<211> 395
<212> PRT
<213> Homo sapiens

<220>
<221> misc_feature
<222> (1)...(395)
<223> Xaa = any amino acid or nothing

<400> 446
Ala Leu Cys Glu Pro Gln Pro Phe Gln Gly Ser Gly Cys Val Ile Ala
1               5                   10                  15
Ile Leu Gly Arg Lys Met Phe Ser Ser Val Ala His Leu Ala Arg Ala
            20                  25                  30
Asn Pro Phe Asn Thr Pro His Leu Gln Leu Val His Asp Gly Leu Gly
        35                  40                  45
Asp Leu Arg Ser Ser Ser Pro Gly Pro Thr Gly Lys Pro Arg Arg Pro
    50                  55                  60
Arg Asn Leu Ala Ala Ala Ala Val Glu Glu Gln Tyr Ser Cys Asp Tyr
65                  70                  75                  80
Gly Ser Gly Arg Phe Phe Ile Leu Cys Gly Leu Gly Gly Ile Ile Ser
                85                  90                  95
Cys Gly Thr Thr His Thr Ala Leu Val Pro Leu Asp Leu Val Lys Cys
            100                 105                 110
Arg Met Lys Val Asp Pro Gln Lys Tyr Lys Gly Ile Phe Asn Gly Phe
        115                 120                 125
Ser Gln Val Thr Leu Lys Glu Asp Gly Val Arg Gly Leu Ala Lys Gly
    130                 135                 140
Leu Ala Pro Thr Phe Leu Gly Tyr Ser Met Gln Gly Leu Cys Lys Phe
145                 150                 155                 160
Gly Phe Tyr Glu Val Phe Lys Val Leu Tyr Arg His Met Leu Gly Glu
                165                 170                 175
Glu Asn Thr Tyr Leu Trp Arg Thr Ser Leu Tyr Leu Ala Ala Tyr Ala
            180                 185                 190
Arg Ala Glu Phe Phe Ala Asp Ile Ala Leu Ala Pro Met Glu Ala Ala
        195                 200                 205
Lys Val Arg Ile Gln Thr Gln Pro Gly Tyr Ala Asn Thr Leu Arg Gly
    210                 215                 220
Cys Ser Ser Pro Lys Cys Ile Lys Glu Glu Gly Leu Thr Ser Ile Leu
225                 230                 235                 240
Thr Arg Gly Val Leu Pro Leu Trp Met Arg Gln Ile Pro Tyr Thr Met
                245                 250                 255
Asn Xaa Ser Ser Pro Cys Leu Glu Arg Thr Val Glu Ala Leu Tyr Lys

260                 265                 270
Phe Val Val Pro Xaa Ala Pro Ala Val Lys Cys Ser Lys Pro Glu Gln
        275                 280                 285
Leu Gly Cys Asn His Leu Val Ala Gly Tyr Ile Ala Arg Val Phe Cys
    290                 295                 300
Ala Asn Cys Phe Ser Pro Arg Ala Asp Ser Val Val Ser Gly Val Glu
305                 310                 315                 320
Xaa Arg Lys Lys Val Ala Ser Ala Phe Ser Gly Pro Ser Lys Arg Leu
                325                 330                 335
Gly Ile Xaa Lys Val Xaa Trp Lys Gly Leu Phe Ala Arg Ile Ile Met
            340                 345                 350
Ile Gly Thr Leu Thr Ala Leu Gln Trp Phe Ile Tyr Asp Ser Val Lys
        355                 360                 365
Val Tyr Phe Arg Leu Pro Arg Pro Pro Pro Pro Glu Met Gln Glu Ser
    370                 375                 380
Leu Lys Lys Lys Leu Gly Val Asn Ser Ser Ser
385                 390                 395

<210> 447
<211> 96
<212> PRT
<213> Homo sapiens

<400> 447
Gly Thr Arg Trp Phe Cys Leu Leu Arg Pro Leu Phe Ala Leu Ser Val
1               5                   10                  15
His Phe Leu Gln Arg Ala Gly Arg Met Ala His Lys Gln Ile Tyr Tyr
            20                  25                  30
Ser Asp Lys Tyr Phe Asp Glu His Tyr Glu Tyr Arg His Val Met Leu
        35                  40                  45
Pro Arg Glu Leu Ser Lys Gln Val Pro Lys Thr His Leu Met Ser Glu
    50                  55                  60
Glu Glu Trp Arg Arg Leu Gly Val Gln Gln Ser Leu Gly Trp Val His
65                  70                  75                  80
Tyr Met Ile His Glu Pro Gly Lys Leu Cys Tyr Val Ile Val Lys Gln
                85                  90                  95

<210> 448
<211> 302
<212> PRT
<213> Homo sapiens

<220>
<221> misc_feature
<222> (1)...(302)
<223> Xaa = any amino acid or nothing

<400> 448
Arg Arg Ser Leu Ser Ala Phe Cys Ser Arg Leu Ala Ala Pro Pro Leu
1               5                   10                  15
Arg Ser Ser Arg Gly Ser Ser Arg Cys Gly Ser Ala Leu Ala Leu Ala
            20                  25                  30

Leu Leu Ala Leu Arg Pro Gly Pro Gly Pro Gly Pro Ala Pro Ala Met
        35                  40                  45
Glu Lys Thr Glu Leu Ile Gln Lys Ala Lys Leu Ala Asp Ala Arg Arg
    50                  55                  60
Ser Val Tyr Asp Asp Met Ala Thr Cys Met Lys Ala Val Thr Glu Gln
65                  70                  75                  80
Gly Ala Glu Leu Ser Asn Glu Glu Arg Asn Leu Leu Ser Val Ala Tyr
                85                  90                  95
Lys Asn Val Val Gly Gly Arg Lys Ser Arg Leu Gly Arg Val Ile Ser
            100                 105                 110
Ser Ile Glu Gln Lys Thr Asp Thr Ser Asp Lys Lys Leu Gln Leu Ile
        115                 120                 125
Lys Asp Tyr Arg Glu Lys Val Glu Ser Glu Leu Arg Ser Ile Cys Thr
    130                 135                 140
Thr Val Leu Glu Leu Leu Asp Lys Tyr Leu Ile Ala Asn Ala Thr Asn
145                 150                 155                 160
Pro Glu Ser Lys Val Phe Tyr Leu Lys Met Lys Gly Xaa Phe Thr Phe
                165                 170                 175
Arg Tyr Leu Cys Leu Glu Val Ala Cys Cys Asp Asp Arg Lys Gln Thr
            180                 185                 190
Ile Asp Asn Ser Gln Gly Ala Tyr Gln Gly Gly Ile Leu Ile Leu Arg
        195                 200                 205
Gln Glu Arg Arg Leu Gln Pro Thr His Pro Ile Arg Leu Gly Gly Leu
    210                 215                 220
Leu Phe Asn Phe Ser Val Phe Tyr Tyr Glu Ile Leu Asn Asn Pro Glu
225                 230                 235                 240
Ala Cys Leu His Ala Trp Leu Lys Thr Ala Phe Xaa Trp Arg Ala Ile
                245                 250                 255
Ala Xaa Leu Asp Thr Leu Asn Glu Asp Ser Tyr Lys Asp Ser Thr Leu
            260                 265                 270
Ile Met Gln Leu Leu Arg Asp Asn Pro Thr Thr Leu Trp Thr Ser Asp
        275                 280                 285
Ser Ala Gly Glu Glu Cys Asp Ala Ala Glu Gly Ala Glu Asn
    290                 295                 300

<210> 449
<211> 304
<212> PRT
<213> Homo sapiens

<400> 449
Met Glu Thr Ser Val Ile Glu Gly Gly Leu Asn Ile Thr Leu Thr Ile
1               5                   10                  15
Arg Leu Leu Met His Gly Lys Glu Val Gly Ser Ile Ile Gly Lys Lys
            20                  25                  30
Gly Glu Ser Val Lys Lys Met Arg Glu Glu Ser Gly Ala Pro Ile Asn
        35                  40                  45
Ile Ser Glu Gly Asn Cys Pro Glu Arg Ile Ile Thr Phe Ala Gly Pro
    50                  55                  60
Ala Asn Ala Val Phe Lys Gly Phe Ala Met Ile Ile Asp Lys Leu Glu
65                  70                  75                  80
Glu Asp Ile Ser Ser Ser Met Thr Ser Ser Thr Ala Ala Ser Arg Pro
                85                  90                  95
Pro Val Thr Leu Arg Leu Val Val Pro Ala Ser Gln Cys Gly Ser Leu
            100                 105                 110
Ile Gly Lys Gly Gly Cys Lys Ile Lys Glu Ile Arg Glu Ser Thr Gly
        115                 120                 125

Ala Gln Ala Ile Thr Ile Ala Gly Ile Pro Gln Ser Ile Ile Glu Cys
    130                 135                 140
Val Lys Gln Ile Cys Val Val Met Leu Glu Ser Pro Pro Lys Gly Val
145                 150                 155                 160
Thr Ile Pro Tyr Gln Leu Lys Pro Ser Ser Ser Pro Val Ile Phe Ala
                165                 170                 175
Gly Gly Gln Ala Tyr Thr Leu Gln Gly Gln Phe Ala Ile Pro Gln Pro
            180                 185                 190
Asp Leu Thr Met Leu His Gln Leu Ala Met Gln Gln Ser His Phe Pro
        195                 200                 205
Met Thr His Gly Asn Thr Gly Phe Ser Ala Gly Leu Asp Thr Ser Ala
    210                 215                 220
Gln Thr Thr Ser His Glu Leu Thr Ile Pro Asn Asp Leu Ile Gly Cys
225                 230                 235                 240
Ile Ile Gly Arg Gln Gly Ala Lys Ile Asn Glu Ile Arg Gln Met Ser
                245                 250                 255
Gly Ala Gln Ile Lys Ile Ala Asn Pro Val Glu Gly Ser Thr Asp Arg
            260                 265                 270
Gln Val Thr Ile Thr Gly Ser Ala Ala Ser Ile Ser Leu Ala Gln Tyr
        275                 280                 285
Leu Ile Asn Val Arg Leu Ser Ser Glu Thr Gly Gly Met Gly Ser Ser
    290                 295                 300

<210> 450
<211> 233
<212> PRT
<213> Homo sapiens

<220>
<221> misc_feature
<222> (1)...(233)
<223> Xaa = any amino acid or nothing

<400> 450
Arg Ile Pro Cys Ala Arg Trp Ala Lys Leu Cys Lys Val Ser Ser Phe
1               5                   10                  15
Ala Pro Asp Trp Thr Pro Met Phe Ala Leu Cys Pro Ser Ser Pro Pro
            20                  25                  30
Gly Leu Ala Glu Ala Thr Tyr Leu Lys Ser Gln Glu Asn Ser Cys Leu
        35                  40                  45
Pro Gly Ala Leu Pro Gly Thr Arg Ala Gln Gly Val Gly Ala Ala Pro
    50                  55                  60
Glu Arg Gly Ser His Ser Pro Pro Cys Arg Met Thr Val His Asn Leu
65                  70                  75                  80
Tyr Leu Phe Asp Arg Asn Gly Val Cys Leu His Tyr Ser Glu Trp His
                85                  90                  95
Arg Glu Glu Ala Ser Arg Gly Ser Pro Lys Glu Glu Glu Tyr Lys Ala
            100                 105                 110
Asp Val Arg Gly Trp Leu Phe Ser Ile Arg Ser Phe Val Ser Lys Met
        115                 120                 125
Ser Pro Leu Asp Met Lys Asp Gly Leu Pro Gly Pro Ser Lys Leu Ser
    130                 135                 140
Arg Tyr Lys Leu His Tyr Tyr Gly Asp Ala His Trp Gly Ser Lys Phe
145                 150                 155                 160
Val Met Asn Tyr Xaa Leu Xaa Ala Leu Gly Pro Ile Pro Arg Leu Leu

                165                 170                 175
Leu His Gln His Leu Thr Ser Ala Leu Val Cys Gly Ser Leu Val Val
            180                 185                 190
Lys Glu Ser Pro Leu Val Pro Leu Gly Pro Lys Thr Val Gln Ser Glu
        195                 200                 205
Ala Leu Phe Gly Ser Arg Thr Trp Thr Pro Tyr Val Arg Ser Leu Pro
    210                 215                 220
Phe Leu Ser Pro Ala Gln Gly Trp Ala
225                 230

<210> 451
<211> 63
<212> PRT
<213> Homo sapiens

<400> 451
Gln Val Ile Gln Gln Gly Arg Gln Ser Lys Gly Val Thr Gln Lys Asp
 1               5                  10                  15
Leu Ala Thr Lys Ile Asn Glu Lys Pro Gln Val Ile Ala Asp Tyr Glu
            20                  25                  30
Ser Gly Arg Ala Ile Pro Asn Asn Gln Val Leu Gly Lys Ile Glu Arg
        35                  40                  45
Ala Ile Asp Val Gly Thr Arg Ser Ala Arg Val Leu Arg Ala Gln
    50                  55                  60

<210> 452
<211> 115
<212> PRT
<213> Homo sapiens

<220>
<221> misc_feature
<222> (1)...(115)
<223> Xaa = any amino acid or nothing

<400> 452
Val Glu Ser Asn Thr Gly Arg Lys Trp Thr Glu Ala Ala Gly Thr Gly
 1               5                  10                  15
Asp Ile Gln Cys Leu Ala Trp Gly Ser Ser Gly Asp Gly Arg Gly Gly
            20                  25                  30
Asp Pro Arg Gly Arg Val Pro Ala Ala Ala Leu Gly Ala Ala Val Val
        35                  40                  45
Ala Ala Met Ala Ser Pro Asp Pro Trp Ala Pro Gly Pro Lys Gln Pro
    50                  55                  60
Gly Arg Trp Ala Asp Leu Ala Ala Leu Leu Leu Cys Gly Leu Arg Pro
65                  70                  75                  80
Ala Xaa Pro Pro Tyr Ser Pro Ala Ser Pro His Leu Ala Pro Lys Arg
                85                  90                  95
Ser His Arg Ala Ser Asp Leu Leu Glu Thr Arg Gln Gly Ala Glu Gly
            100                 105                 110
Arg Ala Arg
        115

<210> 453
<211> 90
<212> PRT
<213> Homo sapiens

<220>
<221> misc_feature
<222> (1)...(90)
<223> Xaa = any amino acid or nothing

<400> 453
Phe Phe Ala Ser Leu Leu Glu Ser Pro Val Ser Pro Arg Leu Ala Met
1               5                   10                  15
Asp Pro Asn Cys Ser Cys Ala Ala Gly Val Ser Cys Thr Cys Ala Gly
            20                  25                  30
Ser Cys Lys Cys Lys Glu Cys Lys Cys Thr Ser Cys Lys Lys Ser Glu
        35                  40                  45
Cys Glu Ala Ile Ser Met Val Trp Gly Cys Gly Xaa Gly Cys Cys Ser
    50                  55                  60
Cys Cys Pro Ala Ala Ala Ser Lys Cys Ala Gln Gly Cys Val Cys Lys
65                  70                  75                  80
Gly Ala Ser Glu Lys Cys Ser Cys Cys Asp
                85                  90

<210> 454
<211> 132
<212> PRT
<213> Homo sapiens

<220>
<221> misc_feature
<222> (1)...(132)
<223> Xaa = any amino acid or nothing

<400> 454
Asn Ser Ile Met Glu Glu Ile Gln Glu Leu Asn Glu Val Ala Arg His
1               5                   10                  15
Arg Pro Arg Ser Thr Leu Val Met Gly Ile Gln Gln Glu Asn Arg Gln
            20                  25                  30
Ile Arg Glu Leu Gln Gln Glu Asn Lys Glu Leu Arg Thr Ser Leu Glu
        35                  40                  45
Glu His Gln Ser Gly Leu Glu Glu Leu Ile Met Ser Lys Tyr Arg Glu
    50                  55                  60
Gln Met Leu Arg Leu Leu Met Ala Ser Lys Lys Asp Asp Pro Gly Ile
65                  70                  75                  80
Ile Met Lys Leu Lys Glu Gln His Ser Tyr Asp Xaa His Trp Tyr Ile
                85                  90                  95
Val Thr Ser Pro Lys Asp Ser Ser Leu Met His Leu Asp Thr Ser Leu
            100                 105                 110
Lys His Leu Asn Met Asp Trp Arg Glu Gly Thr Trp Lys Gln Ile Arg
        115                 120                 125
Met Tyr Thr Lys
    130

<210> 455
<211> 143
<212> PRT
<213> Homo sapiens

<400> 455
Arg Ala Gly Ala Lys Cys Asn Pro Gly Ser His Gly Cys Phe Gly Ala
1               5                   10                  15
Gln Asp Ser Lys Met Ala Glu Gln Leu Ser Pro Gly Lys Ala Val Asp
            20                  25                  30
Gln Val Cys Thr Phe Leu Phe Lys Lys Pro Gly Arg Lys Gly Ala Ala
        35                  40                  45
Gly Arg Arg Lys Arg Pro Ala Cys Asp Pro Glu Pro Gly Glu Ser Gly
    50                  55                  60
Ser Ser Ser Asp Glu Gly Cys Thr Val Val Arg Pro Glu Lys Lys Arg
65                  70                  75                  80
Val Thr His Asn Pro Met Ile Gln Lys Thr Arg Asp Ser Gly Lys Gln
                85                  90                  95
Lys Ala Ala Tyr Gly Asp Leu Ser Ser Glu Glu Glu Glu Glu Asn Glu
            100                 105                 110
Pro Glu Ser Leu Gly Val Val Tyr Lys Ser Thr Arg Ser Ala Lys Pro
        115                 120                 125
Val Gly Pro Glu Asp Met Gly Ala Thr Ala Glu Gly Gln Gly Gly
    130                 135                 140

<210> 456
<211> 86
<212> PRT
<213> Homo sapiens

<220>
<221> misc_feature
<222> (1)...(86)
<223> Xaa = any amino acid or nothing

<400> 456
Met Ser Glu Gly Pro Ser Val Arg Ser Glu Glu Ala Ile Cys Leu Tyr
1               5                   10                  15
Tyr Glu Glu Leu Gly Gly Gly Ala Arg Gln Thr His Val Arg Arg Pro
            20                  25                  30
Leu Ser Glu Cys Ser Pro Gly Asp Trp Ser His Ser Gly Val Ala Glu
        35                  40                  45
Gly Pro Xaa Cys Ile Gln Phe Leu His Ile Thr Ser His Gly Ala Lys
    50                  55                  60
Glu Ala Leu Ser Thr Trp Leu Gly Leu Leu Thr Ser Gly Pro Ala Thr
65                  70                  75                  80
Thr Ala Ala Val Leu Pro
                85

<210> 457
<211> 349
<212> PRT
<213> Homo sapiens

<220>
<221> misc_feature
<222> (1)...(349)
<223> Xaa = any amino acid or nothing

<400> 457
Ile Cys Ser Ala Ser Ser Cys Arg Cys Arg Ala Arg His Arg Val Leu
1               5                   10                  15
Ala Arg Arg Ser Gln Asn Thr Arg Ser Leu Pro Pro Pro Ser Leu Ser
            20                  25                  30
Pro Ala Ser Arg Ser Pro Arg Pro Thr Pro Ala Ala Thr Leu Arg Asp
        35                  40                  45
Ser Leu Ser Gly Gly Leu Cys Ser Ser Thr Ala Gly Leu Ile Arg Gly
    50                  55                  60
Thr Ala Ser Met Asp Leu Gln Gly Arg Gly Val Pro Ser Ile Asp Arg
65                  70                  75                  80
Leu Arg Val Leu Leu Met Leu Phe His Thr Met Ala Gln Ile Met Ala
                85                  90                  95
Glu Gln Glu Val Glu Asn Leu Ser Gly Leu Ser Thr Asn Pro Glu Lys
            100                 105                 110
Asp Ile Phe Val Val Arg Glu Asn Gly Thr Thr Cys Leu Met Ala Glu
        115                 120                 125
Phe Ala Ala Lys Phe Ile Val Pro Tyr Asp Val Trp Ala Ser Asn Tyr
    130                 135                 140
Val Asp Leu Ile Thr Glu Gln Ala Asp Ile Ala Leu Thr Arg Gly Ala
145                 150                 155                 160
Glu Val Lys Gly Arg Cys Gly His Ser Gln Ser Glu Leu Gln Val Phe
                165                 170                 175
Trp Val Asp Arg Ala Tyr Ala Leu Lys Met Leu Phe Val Lys Glu Ser
            180                 185                 190
His Asn Met Ser Lys Gly Pro Glu Ala Thr Leu Glu Ser Xaa Ala Lys
        195                 200                 205
Val Gln Phe Val Tyr Asp Ser Ser Glu Lys Thr His Phe Gln Arg Arg
    210                 215                 220
Ile Ser Cys Ser Gly Lys Pro Thr Ala Asn Leu His His Leu Ser Ala
225                 230                 235                 240
Leu Val Thr Pro Ala Gly Lys Ser Tyr Glu Cys Gln Ala Gln Gln Thr
                245                 250                 255
Ile Ser Leu Ala Ser Ser Asp Pro Gln Lys Thr Val Thr Met Ile Leu
            260                 265                 270
Ser Ala Val His Ile Gln Pro Phe Asp Ile Ile Ser Asp Phe Val Phe
        275                 280                 285
Ser Glu Glu His Lys Cys Pro Val Asp Glu Arg Glu Gln Leu Glu Glu
    290                 295                 300
Thr Leu Pro Leu Ile Leu Gly Leu Ile Leu Gly Leu Val Ile Met Val
305                 310                 315                 320
Thr Leu Ala Ile Tyr His Val His His Lys Met Thr Ala Asn Gln Val
                325                 330                 335
Gln Ile Pro Arg Asp Arg Ser Gln Tyr Lys His Met Gly
            340                 345

<210> 458
<211> 125
<212> PRT
<213> Homo sapiens

<400> 458
Lys Asp Gln Thr Ile Leu Glu Asn Lys Gln Gln Tyr Asp Ile Glu Ile
1               5                   10                  15
Thr Arg Ile Lys Ile Glu Leu Glu Glu Ala Leu Val Asn Val Lys Ser
            20                  25                  30
Ser Gln Phe Lys Leu Glu Thr Ala Glu Lys Glu Asn Gln Ile Leu Gly
        35                  40                  45
Ile Thr Leu Arg Gln Arg Asp Ala Glu Val Thr Arg Leu Lys Glu Leu
    50                  55                  60
Thr Arg Leu Phe Lys Thr Val Asp Leu Asn Val Leu Gln Ser Leu Thr
65                  70                  75                  80
Gln Lys Pro Asn Thr Val His Glu Leu Arg Lys Ser Arg Thr Ala Leu
                85                  90                  95
Asn Lys Glu Glu Val Gly Asp Leu Ser Pro Gly Pro Ser Thr His Phe
            100                 105                 110
Asn Phe Ser His Leu Leu Gly Ile Trp Gly Ser Ala Ala
        115                 120                 125

<210> 459
<211> 48
<212> PRT
<213> Homo sapiens

<220>
<221> misc_feature
<222> (1)...(48)
<223> Xaa = any amino acid or nothing

<400> 459
Gly Gly Arg Pro Glu Phe Leu Val Ser Ser Gln Lys Ile Ser Ile Trp
1               5                   10                  15
Gly Leu Glu Cys Gln Ser Gln Tyr Phe Leu Xaa Ala His Arg Arg Xaa
            20                  25                  30
Val Phe Gly Asp Trp Asn Val Asn Leu Asn Thr Ser Phe Leu Gly Lys
        35                  40                  45

<210> 460
<211> 529
<212> PRT
<213> Homo sapiens

<400> 460

<210> 461
<211> 97
<212> PRT
<213> Homo sapiens

<400> 461
Leu Val Val Met Asp Ala Leu Val Glu Asp Asp Ile Cys Ile Leu Asn
1               5                   10                  15
His Glu Thr Ala His Lys Arg Asp Thr Val Thr Pro Val Ser Ile Tyr
            20                  25                  30
Ser Gly Asp Glu Ser Val Ala Ser His Phe Ala Leu Val Thr Ala Tyr
        35                  40                  45
Glu Asp Ile Lys Lys Arg Leu Lys Asp Ser Glu Lys Glu Asn Ser Leu
    50                  55                  60
Leu Lys Lys Arg Ile Arg Phe Leu Glu Glu Lys Val Ile Phe Ile Phe
65                  70                  75                  80
Tyr Leu His Cys Val Cys Asp Ile Arg Asn Asn Ser Lys Tyr Leu Lys
                85                  90                  95
Phe

<210> 462
<211> 222
<212> PRT
<213> Homo sapiens

<220>
<221> misc_feature
<222> (1)...(222)
<223> Xaa = any amino acid or nothing

<400> 462
Gly Thr Arg Glu Gln Leu Arg His Thr Glu His Gln Leu Pro Pro Ala
1               5                   10                  15
Leu Lys Met Phe His Gln Ile Trp Ala Ala Leu Leu Tyr Phe Tyr Gly
            20                  25                  30
Ile Ile Leu Asn Ser Ile Tyr Gln Cys Pro Glu His Ser Gln Leu Thr
        35                  40                  45
Thr Leu Gly Val Asp Gly Lys Glu Phe Pro Glu Val His Leu Gly Gln
    50                  55                  60
Trp Tyr Phe Ile Ala Gly Ala Ala Pro Thr Lys Glu Glu Leu Ala Thr
65                  70                  75                  80
Phe Xaa Thr Leu Leu Asp Asn Ile Val Phe Gln Asn Met Gly Cys Trp
                85                  90                  95
Leu Leu Pro Pro Ile Ala Ala Ser Asn Leu Ser Val Leu Pro Ile Arg
            100                 105                 110
Met Xaa Val Gly Lys Met Gly Leu Leu Cys Ala Pro Gly Lys Trp Asp
        115                 120                 125
Leu Thr His Leu Thr Glu Gly Gly Gly Thr Asp Leu Gln Glu Thr Xaa
    130                 135                 140
Arg Gly Arg Pro Asp Met Lys Thr Glu Ala Leu Phe Pro Ala Ser Cys
145                 150                 155                 160
Pro Arg Val Gly Ile Met Leu Lys Xaa Glu Gln Ala Gln Gly Tyr Gln
                165                 170                 175

Arg Phe Leu Leu Tyr Asn Arg Ser Pro His Pro Pro Glu Lys Cys Val
            180                 185                 190
Glu Glu Phe Lys Ser Leu Thr Ser Cys Leu Asp Ser Lys Ala Phe Leu
        195                 200                 205
Leu Thr Pro Arg Asn Gln Glu Ala Cys Glu Leu Ser Asn Asn
    210                 215                 220

<210> 463
<211> 134
<212> PRT
<213> Homo sapiens

<400> 463
Ser Ser Met Ala Ser Ala Leu Ser Tyr Val Ser Lys Phe Lys Ser Phe
1               5                   10                  15
Val Ile Leu Phe Val Thr Pro Leu Leu Leu Leu Pro Leu Val Ile Leu
            20                  25                  30
Met Pro Ala Lys Ile Leu Asp Ser Arg Gln Val Cys Val Gln Tyr Met
        35                  40                  45
Lys Asp Thr Asn Met Leu Phe Leu Gly Gly Leu Ile Val Ala Val Ala
    50                  55                  60
Val Glu Arg Trp Asn Leu His Lys Arg Ile Ala Leu Arg Thr Leu Leu
65                  70                  75                  80
Trp Val Gly Ala Lys Pro Ala Arg Leu Met Leu Gly Phe Met Gly Val
                85                  90                  95
Thr Ala Leu Leu Ser Met Trp Ile Ser Asn Thr Ala Thr Thr Ala Met
            100                 105                 110
Met Val Pro Ile Val Glu Ala Ile Leu Gln Gln Met Glu Ala Thr Ser
        115                 120                 125
Ala Ala Thr Glu Ala Gly
    130

<210> 464
<211> 83
<212> PRT
<213> Homo sapiens

<400> 464
Pro Glu Asp Ala Pro Leu Glu Asn Val Glu Cys Asp Asn Met Asn Arg
1               5                   10                  15
Phe Asp Arg Pro Asp Arg Asn Val Arg Gln Pro Gln Glu Gly Phe Trp
            20                  25                  30
Lys Arg Pro Pro Gln Arg Trp Ser Gly Gln Glu His Tyr His Leu Ser
        35                  40                  45
His Pro Asp His Tyr His His His Gly Lys Ser Asp Leu Ser Ser Leu
    50                  55                  60
Lys Tyr Tyr Ser Leu Pro Ala Ala Phe Pro Asp Pro Gln Thr Thr Pro
65                  70                  75                  80
Arg Glu Asn

<210> 465

<211> 214
<212> PRT
<213> Homo sapiens

<220>
<221> misc_feature
<222> (1)...(214)
<223> Xaa = any amino acid or nothing

<400> 465
Val Val Arg Arg Ser Gly Phe Leu Phe Cys Leu Phe Val Leu Phe Leu
1               5                   10                  15
Ser Ser Met Asn Ser Ala Ser Val Asp Gly His Leu Ser Gly Cys Arg
            20                  25                  30
Leu Phe Leu Phe Leu Ser Pro Leu Phe Arg Phe Tyr Cys Asp Tyr Cys
        35                  40                  45
Asp Thr Tyr Leu Ser Pro His Asp Ser Pro Ser Val Arg Gln Thr His
    50                  55                  60
Cys Ser Gly Lys Lys His Ile Glu Asn Val Lys Asp Tyr Tyr Gln Lys
65                  70                  75                  80
Trp Met Glu Lys Gln Ser Gln Ser Leu Ile Asp Lys Thr Asn Gly Leu
                85                  90                  95
His Phe Gln Gln Gly Lys Asn Pro Pro Asn Pro Phe Ser Ala Pro Ser
            100                 105                 110
Phe Leu Pro Gly Ala Gly Xaa Tyr Gln Pro Ser Pro Pro Ser Leu Arg
        115                 120                 125
Gly Pro Pro Arg Pro Gly Met Asn Ala Gln Ala Pro Pro Tyr Trp Gly
    130                 135                 140
Gly Pro Ser His Gly Trp Pro Met Ile Gly Pro Pro Pro Ser Ser Trp
145                 150                 155                 160
Gly Leu Ile Ala Gln Trp Gly Pro Ala Ser Gly Glu Leu Arg Pro Pro
                165                 170                 175
Met Gly Gly His Tyr Cys Gln Leu Ile Ala Trp Gly Pro Pro Met Asp
            180                 185                 190
Val Gly Pro Ser Cys Pro Phe His Xaa Trp Cys Pro Leu Gly Pro Gly
        195                 200                 205
Met Thr Arg Pro Asp Arg
    210

<210> 466
<211> 114
<212> PRT
<213> Homo sapiens

<400> 466
Pro Thr Glu Tyr Glu Asn Leu Phe Pro Cys Ile Lys Glu Ala Phe Val
1               5                   10                  15
Val Glu Glu Trp Val Lys Glu Thr Leu Ala Val Leu Trp Pro Ala Lys
            20                  25                  30
Gln Tyr Pro Phe Val Thr Pro Ile Glu Glu Arg Ile Leu Met Glu Glu
        35                  40                  45
Gly Lys Ala Phe Pro Pro Ser Arg Ser Thr Ala Lys Gln Lys Leu Asp
    50                  55                  60
Gly Asn Pro Val Ser Pro Thr Pro Val Ile Gly Leu Ser Pro Thr Pro
65                  70                  75                  80
Asn Lys Glu Glu Lys His Leu Asn Leu Cys Pro Phe Glu Pro Thr Gly

85                 90                  95
His Leu Asp Gly Ala Arg Asp Thr Ala Gly Pro Ser Trp Leu His His
            100                 105                 110
Arg Phe

<210> 467
<211> 114
<212> PRT
<213> Homo sapiens

<400> 467

<210> 468
<211> 22
<212> PRT
<213> Homo sapiens

<400> 468
Met Arg Met Trp Pro Ala Cys Cys Leu Ile Leu Pro Arg Thr Gly Cys
 1               5                  10                  15
Thr Cys Cys Ser Leu Val
            20

<210> 469
<211> 236
<212> PRT
<213> Homo sapiens

<220>
<221> misc_feature
<222> (1)...(236)
<223> Xaa = any amino acid or nothing

<400> 469

Ala Leu Ser Thr Leu Ala Gly Asp Met Pro Arg Gly Arg Lys Ser Arg
1               5                   10                  15
Arg Arg Arg Asn Ala Arg Ala Ala Xaa Glu Asn Arg Asn Asn Arg Lys
            20                  25                  30
Ile Gln Ala Ser Glu Ala Ser Glu Thr Ser Met Ala Gly Ser Val Val
        35                  40                  45
Thr Ser Thr Pro Arg Arg Thr Thr Leu Ser Gly Pro Glu Glu Asp Pro
    50                  55                  60
Ser Thr Pro Glu Glu Gly Leu Leu Pro Pro Leu Lys Glu Ala Ser Ser
65                  70                  75                  80
Thr Ala Gln Ala Gln Lys Pro Phe Ser Cys Arg Arg Ser Asn Phe Ser
                85                  90                  95
Gly Ala Pro Arg Lys Val Phe Leu Met Ser Ile Leu Ala Leu Ile Phe
            100                 105                 110
Ile Met Gly Asn Ser Ala Lys Glu Ala Leu Val Trp Lys Val Leu Gly
        115                 120                 125
Lys Leu Gly Met Gln Pro Gly Arg Gln His Ser Ile Phe Gly Asp Pro
    130                 135                 140
Lys Lys Ile Val Pro Glu Glu Phe Val Arg Arg Gly Tyr Leu Ile Tyr
145                 150                 155                 160
Lys Pro Val Pro Arg Ser Ser Pro Val Glu Tyr Glu Phe Phe Trp Gly
                165                 170                 175
Ala Pro Ser Thr Arg Gly Asn Ser Ser Lys Leu Lys Val His Ala Phe
            180                 185                 190
Leu Trp Ala Arg Val Ser Xaa Thr Asp Cys Ser Lys Asp Trp Pro Cys
        195                 200                 205
Asn Tyr Asp Trp Asp Ser Asp Asp Asp Ala Glu Val Glu Ala Ile Leu
    210                 215                 220
Asn Ser Gly Ala Arg Gly Tyr Ser Ala Pro Leu Lys
225                 230                 235

<210> 470
<211> 137
<212> PRT
<213> Homo sapiens

<400> 470
Val Gly Gly Ala Gly Gly Gln Ser Ala Ala Ala Ala Ala Arg Arg Cys
1               5                   10                  15
Ser Glu Gly Glu Trp Ala Ser Gly Gly Val Arg Ser Phe Ser Arg Ala
            20                  25                  30
Ala Ala Ala Met Ala Pro Ile Lys Thr His Leu Pro Gly Phe Val Glu
        35                  40                  45
Gln Ala Glu Ala Leu Lys Ala Lys Gly Val Gln Val Val Ala Cys Leu
    50                  55                  60
Ser Val Asn Asp Ala Phe Val Thr Gly Glu Trp Gly Arg Ala His Lys
65                  70                  75                  80
Ala Glu Gly Lys Val Arg Leu Leu Ala Asp Pro Thr Gly Ala Phe Gly
                85                  90                  95
Lys Glu Thr Asp Leu Leu Leu Asp Asp Ser Leu Val Ser Ile Phe Gly
            100                 105                 110
Asn Arg Arg Leu Lys Arg Phe Ser Met Val Val Gln Asp Gly Ile Val
        115                 120                 125
Lys Ala Leu Asn Val Glu Pro Asp Gly
    130                 135

<210> 471
<211> 83
<212> PRT
<213> Homo sapiens

<400> 471
Ala Val Ala Ser Ala Met Ala Ala Ala Ser Gly Ser Val Leu Gln Arg
1               5                   10                  15
Cys Ile Val Ser Pro Ala Gly Arg His Ser Ala Ser Leu Ile Phe Leu
            20                  25                  30
His Gly Ser Gly Asp Ser Gly Gln Gly Leu Arg Met Trp Ile Lys Gln
        35                  40                  45
Val Leu Asn Gln Asp Leu Thr Phe Gln His Ile Lys Ile Ile Tyr Pro
    50                  55                  60
Thr Ala Pro Pro Arg Tyr Ala Val Ile Tyr Leu Thr Cys Gln Tyr Asn
65                  70                  75                  80
Ser Val Ala

<210> 472
<211> 74
<212> PRT
<213> Homo sapiens

<400> 472
Trp Pro Phe Phe Leu Pro Leu Lys Leu Phe Lys Phe Gln Val Ile Leu
1               5                   10                  15
Phe Leu Asp Val Ile Tyr Ile Leu Asn Phe Tyr Leu Tyr Arg Tyr Phe
            20                  25                  30
Leu Ala Ser Phe Tyr Thr Lys Tyr Asp Pro Thr His Phe Ile Leu Asn
        35                  40                  45
Thr Ala Ser Leu Leu Ser Val Leu Ile Pro Lys Met Pro Gln Leu His
    50                  55                  60
Gly Val Arg Ile Phe Gly Ile Asn Lys Tyr
65                  70

<210> 473
<211> 191
<212> PRT
<213> Homo sapiens

<220>
<221> misc_feature
<222> (1)...(191)
<223> Xaa = any amino acid or nothing

<400> 473
Ile Ser Trp Val Cys Leu Asn Cys Gln Ser Gln His Leu Leu Lys Ala
1               5                   10                  15
Pro Leu Ser Ser Ser Gly His Ser Gly Arg Ile Met Val Glu Thr His
            20                  25                  30
Gly Lys Asn Glu Thr Ala Asp Tyr Lys Arg Leu Gln Thr Phe Pro Leu

35                  40                  45
Val Met His Ser Asp Met Pro Gln Glu Met Arg Ala Glu Thr Met Glu
    50                  55                  60
Leu Cys Val Thr Ala Cys Glu Lys Phe Phe Gln Gln Gln Thr Glu Ser
65                  70                  75                  80
Ala Arg Gln Asp Cys Ser Lys Glu Thr Met Asp Lys Lys Phe Gly Ser
                85                  90                  95
Ser Trp His Val Val Ile Gly Glu Gly Phe Gly Phe Xaa Glu Ile Thr
            100                 105                 110
His Glu Val Glu Arg Thr Ser Ser Thr Leu Tyr Phe Gly Gly Thr Leu
        115                 120                 125
Ala Val Cys Val Trp Lys Leu Leu Leu Asp Thr Leu Ser Pro Ala Pro
    130                 135                 140
Ser Pro Ala Gly Pro Phe Pro Ala Thr His Leu Gly Trp Gly Ala Ala
145                 150                 155                 160
Leu Gly Arg Ser Trp Phe Phe Gln Gly Glu Leu Gly Ser Phe Leu Phe
                165                 170                 175
Val Phe Val Tyr Gln Phe Pro Glu Pro Arg Pro Val Cys Glu Thr
            180                 185                 190

<210> 474
<211> 79
<212> PRT
<213> Homo sapiens

<220>
<221> misc_feature
<222> (1)...(79)
<223> Xaa = any amino acid or nothing

<400> 474
Arg Lys Thr Gln Thr Thr Arg Arg Gly Pro Gly Leu Trp Ala Gly Pro
1               5                   10                  15
Gly Gly Xaa Arg Gly Gly Trp Trp Ser Arg Arg Leu Leu Leu Ala Ala
            20                  25                  30
Gly Phe Leu Gly Thr His Pro Gly Ser Thr His Pro Gly Leu Gln Gln
        35                  40                  45
Pro Arg Phe Lys Trp Asp His Thr Arg Ser Ser Gln Gly Ala Phe Ile
    50                  55                  60
Phe Thr Phe Phe Pro Arg Gly Gly Gln Glu His Ser Phe Thr Ser
65                  70                  75

<210> 475
<211> 69
<212> PRT
<213> Homo sapiens

<400> 475

<210> 476
<211> 101
<212> PRT
<213> Homo sapiens

<220>
<221> misc_feature
<222> (1)...(101)
<223> Xaa = any amino acid or nothing

<400> 476
Gln Lys Thr Ser Arg Thr Phe Leu Ala Ala Leu Cys Thr Val Cys Pro
 1               5                  10                  15
Arg Arg His Cys Leu Asp Pro Cys Phe Xaa Arg Arg Glu Leu Trp Gly
            20                  25                  30
Leu Pro Ala Pro Ala Pro Pro Pro Arg Glu Pro Thr Val Pro Pro Pro
        35                  40                  45
Leu Ser Pro Gly Pro Met Gly Pro Gly Ser Gly Cys Val Pro Cys Leu
    50                  55                  60
Arg Ala Leu Cys Leu Leu Pro Gln Met Arg Pro Val Pro Glu Arg Leu
 65                 70                  75                  80
Pro Val Gln Pro Gly Ser Val Trp Pro Gln Thr Gly Ile Gln Asn Pro
                85                  90                  95
Lys Lys Lys Lys Lys
            100

<210> 477
<211> 44
<212> PRT
<213> Homo sapiens

<400> 477
Ser Gly Val Leu Ala His Gly Gln Ala Glu Ala Glu Val Glu Thr Ser
 1               5                  10                  15
Cys Leu Phe Ile Thr Gly Met Arg Met Trp Pro Ala Cys Cys Leu Ile
            20                  25                  30
Leu Pro Arg Thr Gly Cys Thr Cys Cys Ser Leu Val
        35                  40

<210> 478
<211> 140
<212> PRT
<213> Homo sapiens

<220>
<221> misc_feature
<222> (1)...(140)

<223> Xaa = any amino acid or nothing

<400> 478
Cys Leu Pro Leu Cys Ser Thr Ala Phe Val Ser Lys Gln Glu Gly Ser
1               5                   10                  15
Glu Val Val Lys Arg Pro Arg Arg Tyr Leu Tyr Gln Trp Leu Gly Xaa
            20                  25                  30
Glu Lys Arg Gln Ser Trp Ala Lys Ala Leu Pro Leu Arg Asp Gly Leu
        35                  40                  45
Trp Gly Ser Cys Ser Arg Glu Trp Pro Leu Trp Val Val Val Gly Val
    50                  55                  60
Gln Ala Ala Cys Pro Gly Gly His Pro Gly Ala Pro Cys Val Gly Arg
65                  70                  75                  80
Gly Gly Met Gly Ile Leu His Gly Gly Xaa Cys His His Val Gly Cys
                85                  90                  95
Leu Arg Ala Pro Val Pro Tyr Pro Asp Pro Leu Glu Pro Arg Arg Glu
            100                 105                 110
Val Cys Glu Leu Asn Pro Asp Cys Asn Glu Leu Ala Asp His Ile Gly
        115                 120                 125
Phe Gln Glu Ala Tyr Arg Arg Phe Tyr Gly Pro Val
    130                 135                 140

<210> 479
<211> 92
<212> PRT
<213> Homo sapiens

<220>
<221> misc_feature
<222> (1)...(92)
<223> Xaa = any amino acid or nothing

<400> 479
Xaa Thr Ser Val Val Arg Pro Phe Ala Lys Leu Val Arg Pro Pro Val
1               5                   10                  15
Gln Val Tyr Gly Ile Glu Gly Arg Tyr Ala Thr Ala Leu Tyr Ser Val
            20                  25                  30
Leu Asn Pro Tyr Val Lys Arg Ser Ile Lys Val Lys Ser Leu Asn Asp
        35                  40                  45
Ile Thr Ala Lys Glu Arg Phe Ser Pro Leu Thr Thr Asn Leu Ile Asn
    50                  55                  60
Leu Leu Ala Glu Asn Gly Arg Leu Ser Asn Thr Gln Gly Val Val Ser
65                  70                  75                  80
Ala Phe Ser Thr Met Met Ser Val His Arg Gly Glu
                85                  90

<210> 480
<211> 169
<212> PRT
<213> Homo sapiens

<400> 480
Met Ser Met Leu Arg Leu Gln Lys Arg Leu Ala Ser Ser Val Leu Arg

1               5                   10                  15
Cys Gly Lys Lys Lys Val Trp Leu Asp Pro Asn Lys Thr Asn Glu Ile
                20                  25                  30
Ala Asn Ala Asn Ser His Gln Gln Ile Trp Lys Leu Ile Lys Asp Gly
            35                  40                  45
Leu Ile Ile Arg Lys Pro Val Thr Val His Ser Arg Ala Arg Cys Gln
    50                  55                  60
Lys Asn Thr Leu Ala His Trp Lys Gly Arg His Ile Gly Ile Asp Lys
65                  70                  75                  80
Arg Tyr Cys Glu Ser Lys Lys Ile Asp Arg His Met Tyr His Ser Leu
                85                  90                  95
Tyr Leu Lys Val Gln Gly Asn Val Phe Thr Asn Lys Pro Ile Leu Met
            100                 105                 110
Glu His Ser His Lys Leu Lys Ala Asp Lys Ala His Lys Lys Leu Leu
        115                 120                 125
Ala Asp Gln Ala Glu Ala Arg Arg Pro Lys Thr Lys Glu Ala Arg Lys
    130                 135                 140
Arg Ser Glu Glu Arg Leu Gln Ala Lys Lys Glu Glu Ile Ile Lys Thr
145                 150                 155                 160
Leu Phe Lys Glu Glu Asp Thr Lys Lys
                165

<210> 481
<211> 88
<212> PRT
<213> Homo sapiens

<220>
<221> misc_feature
<222> (1)...(88)
<223> Xaa = any amino acid or nothing

<400> 481
Asn Phe Leu Lys Asp Gly Val Leu Leu Cys Leu Pro Gly Trp Ile Glu
1               5                   10                  15
Tyr Ser Gly Met Ile Leu Ala His Cys Asn Leu Arg Leu Leu Gly Ser
                20                  25                  30
Asn Asn Ser Pro Ser Ala Ser Xaa Ile Ala Gly Ala Thr Gly Ala Cys
            35                  40                  45
His His Ala Trp Leu Ile Phe Xaa Asn Phe Ser Xaa Arg Trp Gly Leu
    50                  55                  60
Leu Gly Val Glu Ser Pro Ser Leu Gly Leu Leu Phe Leu Phe Ile Leu
65                  70                  75                  80
Lys Lys Asn Tyr Tyr Val Leu Tyr
                85

<210> 482
<211> 115
<212> PRT
<213> Homo sapiens

<400> 482
Ile Leu Val Leu Ile Lys Val His Ala Cys Gly Val Asn Pro Val Glu
1               5                   10                  15

Thr Tyr Ile Arg Ser Gly Thr Tyr Ser Arg Lys Pro Leu Leu Pro Tyr
            20                  25                  30
Thr Pro Gly Ser Asp Val Ala Gly Val Ile Glu Ala Val Gly Asp Asn
        35                  40                  45
Ala Ser Ala Phe Lys Lys Gly Asp Arg Val Phe Thr Ser Ser Thr Ile
    50                  55                  60
Ser Gly Gly Tyr Ala Glu Tyr Ala Leu Ala Ala Asp His Thr Val Tyr
65                  70                  75                  80
Lys Leu Pro Glu Lys Leu Asp Phe Lys Gln Gly Ala Ala Ile Gly Ile
                85                  90                  95
Pro Tyr Phe Thr Ala Tyr Arg Ala Leu Ile His Ser Ala Cys Val Lys
            100                 105                 110
Ala Gly Glu
        115

<210> 483
<211> 120
<212> PRT
<213> Homo sapiens

<400> 483
Leu Tyr Asp Asn Arg Thr Leu Thr Lys Gly Pro Asp Thr Val Gly Thr
1               5                   10                  15
Met Gly Gln Cys Arg Ser Ala Asn Ala Glu Asp Ala Gln Glu Phe Ser
            20                  25                  30
Asp Val Glu Arg Ala Ile Glu Thr Leu Ile Lys Asn Phe His Gln Tyr
        35                  40                  45
Ser Val Glu Gly Gly Lys Glu Thr Leu Thr Pro Ser Glu Leu Arg Asp
    50                  55                  60
Leu Val Thr Gln Gln Leu Pro His Leu Met Pro Ser Asn Cys Gly Leu
65                  70                  75                  80
Glu Glu Lys Ile Ala Asn Leu Gly Ser Cys Asn Asp Ser Lys Leu Glu
                85                  90                  95
Phe Arg Ser Ile Trp Glu Leu Ile Gly Glu Ala Ala Lys Ser Val Lys
            100                 105                 110
Leu Glu Arg Pro Val Arg Gly His
        115                 120

<210> 484
<211> 94
<212> PRT
<213> Homo sapiens

<400> 484

<210> 485
<211> 47
<212> PRT
<213> Homo sapiens

<400> 485
Gln Val Glu Ser Ser Pro Gln Pro Gly Leu Pro Ala Gly Glu Gln Leu
 1               5                  10                  15
Glu Gly Leu Lys His Ala Gln Asp Ser Asp Pro Arg Ser Pro Thr Leu
            20                  25                  30
Gly Ile Ala Arg Thr Pro Met Lys Thr Ser Ser Gly Gly Lys Arg
        35                  40                  45

<210> 486
<211> 112
<212> PRT
<213> Homo sapiens

<220>
<221> misc_feature
<222> (1)...(112)
<223> Xaa = any amino acid or nothing

<400> 486
Leu Glu Ala His Ser Glu Met Gly Ser Thr Glu Ile Leu Glu Lys Glu
 1               5                  10                  15
Thr Pro Glu Asn Leu Ser Asn Gly Thr Ser Ser Asn Val Glu Ala Ala
            20                  25                  30
Lys Arg Leu Ala Lys Arg Leu Tyr Gln Leu Asp Arg Phe Lys Arg Ser
        35                  40                  45
Asp Val Ala Lys His Leu Gly Lys Asn Asn Glu Phe Ser Lys Leu Val
    50                  55                  60
Ala Glu Glu Tyr Leu Lys Phe Phe Asp Phe Thr Gly Met Thr Leu Ile
65                  70                  75                  80
Ser His Ser Glu Ser Arg Ser Val Thr Gln Ala Gly Val Gln Xaa His
                85                  90                  95
His Leu Gly Ser Leu Gln Pro Pro Pro Ser Arg Phe Lys Gln Phe Ser
            100                 105                 110

<210> 487
<211> 132
<212> PRT
<213> Homo sapiens

<400> 487
Arg Pro Val Ile Pro Gly Ser Thr Ile Ser Trp Ala Leu Arg Gly Ala

1               5                   10                  15
Ala Lys Met Ala Ala Ser Arg Trp Ala Arg Lys Ala Val Val Leu Leu
            20                  25                  30
Cys Ala Ser Asp Leu Leu Leu Leu Leu Leu Leu Leu Pro Pro Pro Gly
        35                  40                  45
Ser Cys Ala Ala Glu Gly Ser Pro Gly Thr Pro Asp Glu Ser Thr Pro
    50                  55                  60
Pro Pro Arg Lys Lys Lys Lys Asp Ile Arg Asp Tyr Asn Asp Ala Asp
65                  70                  75                  80
Met Ala Arg Leu Leu Glu Gln Trp Glu Val Arg Gly Ala Arg Thr Pro
                85                  90                  95
Ser Arg Leu Phe Ser Val Arg Asp Pro Val Ala Tyr Asp Pro Leu Pro
            100                 105                 110
Val Leu Ser Trp Asp Leu Ser Asp Pro Leu Thr Ser Ser Thr His Ala
        115                 120                 125
Leu Asn Pro Tyr
    130

<210> 488
<211> 93
<212> PRT
<213> Homo sapiens

<400> 488
Phe Val Arg Ala Gly Phe Leu Pro Gly Phe Cys Arg Val Ser Pro Cys
1               5                   10                  15
Cys Ser Trp Val Val Glu Thr Leu Ala Lys Met Ala Cys Ala Ala Ala
            20                  25                  30
Arg Ser Pro Ala Asp Gln Asp Arg Phe Ile Cys Ile Tyr Pro Ala Tyr
        35                  40                  45
Leu Asn Asn Lys Lys Thr Ile Ala Glu Gly Arg Arg Ile Pro Ile Ser
    50                  55                  60
Lys Ala Val Glu Asn Pro Thr Ala Thr Glu Ile Gln Asp Val Cys Ser
65                  70                  75                  80
Ala Asp Gly Leu Asn Val Phe Leu Glu Val Lys Asn Val
                85                  90

<210> 489
<211> 78
<212> PRT
<213> Homo sapiens

<400> 489
Met Leu Thr Glu Leu Val Pro Thr Gln Ala Glu Pro Ala Asp Leu Glu
1               5                   10                  15
Leu Leu Gln Glu Leu Asn Arg Thr Cys Arg Ala Met Gln Gln Arg Val
            20                  25                  30
Leu Glu Leu Ile Pro Gln Ile Ala Asn Glu Gln Leu Thr Glu Glu Leu
        35                  40                  45
Leu Ile Val Asn Asp Asn Leu Asn Asn Val Phe Leu Arg His Glu Arg
    50                  55                  60
Phe Glu Arg Phe Arg Thr Gly Pro Met Ala Leu Ala Gly His
65                  70                  75

<210> 490
<211> 78
<212> PRT
<213> Homo sapiens

<400> 490
Pro Lys Arg Ser Val Leu Ser Lys Ser Val Pro Gly Tyr Lys Pro Lys
1               5                   10                  15
Val Ile Pro Asn Ala Ile Cys Gly Ile Cys Leu Lys Gly Lys Glu Ser
            20                  25                  30
Asn Lys Lys Gly Lys Ala Glu Ser Leu Ile His Cys Ser Gln Cys Glu
        35                  40                  45
Asn Ser Gly Arg Trp Ile Cys Asp Cys Cys Gln Arg Ala Pro Pro Thr
    50                  55                  60
Pro Arg Lys Val Gly Arg Arg Gly Lys Asn Ser Lys Glu Gly
65                  70                  75

<210> 491
<211> 82
<212> PRT
<213> Homo sapiens

<400> 491
Ala Glu Gly Lys Lys Leu Gly Leu Val Gly Trp Val Gln Asn Thr Asp
1               5                   10                  15
Arg Gly Thr Val Gln Gly Gln Leu Gln Gly Pro Ile Ser Lys Val Arg
            20                  25                  30
His Met Gln Glu Trp Pro Glu Thr Arg Glu Ala Tyr Gln Lys Pro Pro
        35                  40                  45
Lys Thr His Arg Thr Ile Ala Asn Phe Asn Asn Glu Lys Val Ile Leu
    50                  55                  60
Lys Leu Gly Leu Pro Gln Thr Phe Gln Ile Val Lys Ile Met Gly Leu
65                  70                  75                  80
Glu Phe

<210> 492
<211> 59
<212> PRT
<213> Homo sapiens

<220>
<221> misc_feature
<222> (1)...(59)
<223> Xaa = any amino acid or nothing

<400> 492
Ser Asn Leu Phe Ser Leu Ser Leu Phe His Leu Lys Gly Phe Phe Cys
1               5                   10                  15
Phe Phe Phe Leu Asp Phe Xaa Met Glu Ser Arg Ser Val Ala Arg Leu
            20                  25                  30

Glu Cys Ser Gly Thr Ile Leu Ala Tyr Cys Asn Leu Cys Leu Pro Gly
        35                  40                  45
Ser Ser Asp Ser Pro Ala Ser Ala Ser His Leu
    50                  55

<210> 493
<211> 548
<212> PRT
<213> Homo sapiens

<400> 493
Met Ile Ala Asp Asp Lys Pro Ala Ala Met Ala Gly Glu Ser Ala Gly
1               5                   10                  15
Gln Ser Ser Glu Ser Gly Val Gly Ala Asn Phe Phe Gly Ile Thr Phe
            20                  25                  30
Gln Thr Thr Glu Thr Leu Met Ser Thr Gly His Leu Asn Gly Ala Glu
        35                  40                  45
Cys Lys Ala Gly Pro Gly Thr Val Lys Thr Leu Ala Val Glu Glu Glu
    50                  55                  60
Ala Ser Arg Leu Trp Arg Lys Pro Asp Pro Tyr Asn Thr Arg Arg Glu
65                  70                  75                  80
Pro Asp Leu Arg Gly Gly Ala Leu Asp Ala Thr Gly Ala Gln Gly Gly
                85                  90                  95
Pro Leu Asp Arg Ala Arg Lys Glu Pro Glu Thr Ala Cys Ser His Arg
            100                 105                 110
Arg Lys Ser Val Ala Gly Gln Thr Pro Val Gly Ser Asn Lys Lys Arg
        115                 120                 125
Asn Arg Val Pro Gly His Val Pro Ala Ala Gly Leu Thr Ser Ala Ala
    130                 135                 140
Ala Thr Lys Glu Arg Ala Gln Cys Ser Asp Thr Gly Gly Leu Asp Pro
145                 150                 155                 160
Tyr Ser Ala Gly His Phe Ser Arg Ala Thr Leu Leu Phe Cys Pro Pro
                165                 170                 175
His Thr His Arg Cys Ile Ser Leu Thr Lys Ser Glu Lys Ser Glu Arg
            180                 185                 190
Gln Gln Leu Phe Leu Pro Lys Pro Gln Ser Ala Val Phe Gly Ser Glu
        195                 200                 205
Gly Arg Arg Thr Leu Arg Lys Leu Arg Arg Leu Ser Ser Pro Gly Ala
    210                 215                 220
Met Asp Ser Asp Ala Ser Leu Val Ser Ser Arg Pro Ser Ser Pro Glu
225                 230                 235                 240
Pro Asp Asp Leu Phe Leu Pro Ala Arg Ser Lys Gly Ser Ser Gly Ser
                245                 250                 255
Ala Phe Thr Gly Gly Thr Val Ser Ser Ser Thr Pro Ser Asp Cys Ala
            260                 265                 270
Pro Glu Leu Ser Ala Glu Leu Arg Ala Ala Met Gly Ser Ala Gly Ala
        275                 280                 285
His Ser Gly Thr Lys Leu Gly Gly Ser Gly Phe Lys Ser Ser Ser Ser
    290                 295                 300
Ser Thr Ser Ser Ser Thr Ser Ser Ser Ala Ala Ser Ser Ser Lys Lys
305                 310                 315                 320
Val Lys Lys Arg Met Thr Glu Pro Glu Leu Gln Gln Leu Arg Leu Lys
                325                 330                 335
Ile Ser Ser Arg Glu Arg Lys Arg Met His Asp Leu Asn Ile Ala Met
            340                 345                 350
Asp Gly Leu Arg Glu Val Met Pro Tyr Ala His Gly Pro Ser Val Arg
        355                 360                 365

Lys Leu Ser Lys Ile Ala Thr Leu Leu Leu Ala Arg Asn Tyr Ile Ser
    370                 375                 380
Met Leu Thr Thr Ser Leu Glu Glu Met Lys Arg Leu Val Ser Glu Ile
385                 390                 395                 400
Tyr Gly Gly His Thr Pro Ala Gly Phe His Pro Ser Ala Cys Gly Gly
                405                 410                 415
Leu Ala His Ser Ala Pro Leu Pro Ala Ala Thr Ala His Pro Ala Ala
            420                 425                 430
Ala Ala His Ala Ala His His Pro Ala Val His His Pro Ile Leu Pro
        435                 440                 445
Pro Ala Ala Ala Ala Ala Ala Ala Ala Ala Ala Ala Ala Ala Val Ser
    450                 455                 460
Ser Ala Ser Leu Pro Gly Ser Gly Leu Pro Ser Val Gly Ser Ile Arg
465                 470                 475                 480
Pro Pro His Gly Leu Leu Lys Ser Pro Ser Ala Ala Ala Ala Ala Pro
                485                 490                 495
Leu Gly Gly Gly Gly Gly Gly Ser Gly Ala Ser Gly Gly Phe Gln His
            500                 505                 510
Trp Gly Gly Met Pro Cys Pro Cys Ser Met Cys Gln Val Pro Pro Pro
        515                 520                 525
His His His Val Ser Ala Met Gly Ala Gly Ser Leu Pro Arg Leu Thr
    530                 535                 540
Ser Asp Ala Lys
545

<210> 494
<211> 54
<212> PRT
<213> Homo sapiens

<400> 494
Val Arg Ser Leu Val Ile Ser Met Thr Cys Lys Thr Ala Val Ser Phe
 1               5                  10                  15
Leu Thr Ser Arg Val Cys Ser Asn Arg His Gly Leu Ile Arg Lys Tyr
            20                  25                  30
Gly Leu Asn Met Cys Arg Gln Cys Phe Arg Gln Tyr Ala Lys Asp Ile
        35                  40                  45
Gly Phe Ile Lys Leu Asp
    50

<210> 495
<211> 61
<212> PRT
<213> Homo sapiens

<220>
<221> misc_feature
<222> (1)...(61)
<223> Xaa = any amino acid or nothing

<400> 495
Ala Met Ala Tyr Gln Leu Tyr Arg Asn Thr Thr Leu Gly Asn Ser Leu
 1               5                  10                  15
Gln Glu Ser Leu Asp Glu Leu Ile Gln Ser Gly Arg Leu Asn Xaa Lys

20                  25                  30
Ser Asn Ile Phe Ile Leu Met Gly Leu Gln Xaa Phe Leu Ser Tyr His
        35                  40                  45
Leu Gly Lys Glu Ile Xaa Gly Lys Ile Ser Trp Thr Ile
    50                  55                  60

<210> 496
<211> 140
<212> PRT
<213> Homo sapiens

<220>
<221> misc_feature
<222> (1)...(140)
<223> Xaa = any amino acid or nothing

<400> 496
Asp Phe Trp Arg Glu Met Met Arg Ala Ser Trp Leu Val Arg Gln Asp
 1               5                  10                  15
Ser Arg His Gln Arg Ile Arg Leu Pro His Leu Glu Ala Ile Val Ser
            20                  25                  30
Gly Arg Gly Pro Xaa Thr Lys Ile Thr Asp Lys Lys Cys Ser Arg Gln
        35                  40                  45
Gln Val Gly Ala Asn Pro Thr Ser Ile Asp Ser Val Val Ile Gly Lys
    50                  55                  60
Asp Gln Glu Val Lys Leu Gln Pro Gly Gln Val Leu His Met Ala Asn
65                  70                  75                  80
Glu Leu Tyr Pro Tyr Ile Val Xaa Phe Glu Glu Glu Ala Lys Asn Pro
                85                  90                  95
Gly Leu Glu Thr His Arg Lys Arg Lys Arg Ser Arg Asn Ser Asp Ser
            100                 105                 110
Ile Glu Arg Asp Ala Ala His Glu Ala Glu Ala Gly Thr Gly Leu Glu
        115                 120                 125
Pro Gly Ser Asn Ser Gly Gln Cys Ser Val Pro Leu
    130                 135                 140

<210> 497
<211> 132
<212> PRT
<213> Homo sapiens

<400> 497

<210> 498
<211> 61
<212> PRT
<213> Homo sapiens

<400> 498
Gln Gly Arg Arg Lys Met Glu Gln Pro Gly Ala Ala Ala Ser Gly Ala
1               5                   10                  15
Gly Gly Gly Ser Glu Glu Pro Gly Gly Gly Arg Ser Asn Lys Arg Ser
            20                  25                  30
Ala Gly Asn Arg Ala Ala Asn Glu Glu Glu Thr Lys Asn Lys Pro Lys
        35                  40                  45
Leu Arg Asp Arg Ile Thr Ser Phe Arg Lys Ser Thr Val
    50                  55                  60

<210> 499
<211> 534
<212> PRT
<213> Homo sapiens

<400> 499
Met Trp Glu Ser Leu Glu Leu Pro Arg Asp Leu Leu Asn Gly Phe Asp
1               5                   10                  15
Gln Lys Pro Asp Ser Asn Met Asp Asn Asn Val Gln Ala Glu Val Val
            20                  25                  30
Ser Asp Gly Glu Glu Glu Leu Val Gly Asn Trp Arg Ser Pro His Lys
        35                  40                  45
Leu Arg Glu Cys Leu Pro Leu Ile Ile Phe Leu Arg Asn Arg Leu Lys
    50                  55                  60
Tyr Ala Leu Thr Gly Asp Glu Val Lys Ile Cys Met Gln Gly Phe Ile
65                  70                  75                  80
Lys Val Asp Gly Lys Val Arg Thr Asp Ile Thr Tyr Pro Ala Gly Phe
                85                  90                  95
Met Asp Val Ile Ser Ile Asp Lys Thr Gly Asp Asn Phe His Leu Ile
            100                 105                 110
Tyr Asp Thr Lys Gly Arg Phe Ala Val His Arg Ile Thr Pro Glu Glu
        115                 120                 125
Ala Lys Tyr Lys Leu Gly Lys Val Arg Lys Ile Phe Val Gly Thr Lys
    130                 135                 140
Gly Ile Pro His Leu Val Thr His Asp Ala His Ser Ile Arg Tyr Leu
145                 150                 155                 160
Asp Pro Leu Ile Lys Glu Gln Ala Pro Gly Pro Ala Arg Asn Trp Gly
                165                 170                 175
Asn Ala Ser Ser Phe Pro Arg Ser Asp Gly Lys Pro Thr Val Leu Ser
            180                 185                 190
Leu Arg Ala Ala Thr Thr Gly Ser Thr Gly Leu Asp Leu Leu Cys Leu
        195                 200                 205

Asn Lys Leu Met Leu Lys Glu Gly Glu Asp Pro Lys Arg Val Ala Thr
    210                 215                 220
Gly Ile Trp Gly Leu Leu Pro Pro Gly Thr Val Gly Leu Val Leu Gly
225                 230                 235                 240
Trp Ser Ser Leu Ser Ser Lys Gly Ile Asn Val Leu Thr Val Val Ile
                245                 250                 255
Asp Ser Asp Tyr His Arg Glu Ile Leu Val Met Met Asp Cys Lys Gly
            260                 265                 270
Leu His Ile Leu Pro Pro Gly Ser Lys Ile Ala Gln Leu Leu Ile Leu
        275                 280                 285
Ser Tyr Trp Val Pro Ser Leu Tyr Gly Lys Glu Arg Gly Lys Gly Ser
    290                 295                 300
Phe Gly Ser Thr Gly Ala Thr Gly Val Tyr Trp Asn Gln Leu Ile Thr
305                 310                 315                 320
Asp Gln Gly Pro Met Lys Lys Leu Glu Ile Arg Ile Leu Leu Ala Tyr
                325                 330                 335
Trp Ala Gln Gly Gln Thr Phe Gln Ser Leu Val Ile Lys Thr Gly Gln
            340                 345                 350
Lys Leu Gly Phe Gly Tyr Leu Thr Pro Ala Ala Lys Arg Glu Ile Glu
        355                 360                 365
Glu Ile Glu Gln Ala Val Ser Gln Gly Gln Leu Asp Arg Ile Asp Pro
    370                 375                 380
Arg Tyr Ser Ile Gln Leu Phe Tyr Leu Ser His Gln Thr Leu Pro Tyr
385                 390                 395                 400
Arg Val Asn Arg Thr Asp Gly Pro Arg Ala Met Leu Pro Arg Met Gly
                405                 410                 415
Phe Leu Pro His Thr Gly Thr Lys Thr Leu Ser Pro Tyr Ser Gln Leu
            420                 425                 430
Leu Thr Lys Val Ile Tyr Ser Gly His Lys Gln Cys Asn Gln Ser Leu
        435                 440                 445
Gly Tyr Asp Pro Asp Val Ile Arg Ile Pro Leu Ser Lys Lys Gln Phe
    450                 455                 460
Lys Ala Val Leu Pro Val Ser Ile Asn Leu Gln Ile Ala Phe Ser Asp
465                 470                 475                 480
Tyr Thr Gly Gln Ile Glu His Ile Leu Pro Ala Asp Lys Leu Leu Tyr
                485                 490                 495
Phe Leu Ser His Thr Leu Val Ile Leu Pro Thr Lys Ile Val His Ser
            500                 505                 510
Pro Ile Pro Asn Ala Leu Thr Leu Phe Thr Asp Gly Ser Gly Lys His
        515                 520                 525
Gly Lys Ala Ala Val Trp
    530

<210> 500
<211> 92
<212> PRT
<213> Homo sapiens

<400> 500
Leu Ala Ser Gly Gly Val Ala Tyr Gln Ala Ile Gln Ala Leu Lys Asn
  1               5                  10                  15
Met Gly Glu Ile Leu Lys Ala Ala Gly Cys Asp Leu Ser Asn Val Gly
             20                  25                  30
Thr Thr Thr Val Leu Leu Gly Asp Ile Asn Asp Phe Asn Thr Ala Asn
         35                  40                  45
Glu Ile Tyr Arg Gln Tyr Leu Lys Ser Asn Phe Pro Ala Arg Ala Ala
     50                  55                  60

Tyr Gln Asp Ala Ala Leu Pro Gln Gly Ile Arg Ile Glu Ile Glu Ala
65                  70                  75                  80
Val Ala Met Gln Gly Pro Leu Thr Thr Ala Leu Leu
                85                  90

<210> 501
<211> 123
<212> PRT
<213> Homo sapiens

<400> 501
Thr Ser Ala Trp Arg Thr Ala Phe Trp Ser Thr Trp Pro Thr Ser Pro
1               5                   10                  15
Pro Tyr Lys Gly Trp Trp Trp Glu Asn Ala Ile Ala Ala Leu Phe Arg
            20                  25                  30
Arg His Ile Pro Val Ser Trp Leu Ile Arg Ala Thr Leu Ser Glu Ser
        35                  40                  45
Glu Asn Phe Glu Ala Ala Val Gly Lys Leu Ala Lys Thr Pro Leu Ile
    50                  55                  60
Ala Asp Val Tyr Tyr Ile Val Gly Gly Thr Ser Pro Arg Glu Gly Val
65                  70                  75                  80
Val Ile Thr Arg Asn Arg Asp Gly Pro Ala Asp Ile Trp Pro Leu Asp
                85                  90                  95
Pro Leu Asn Gly Ala Trp Phe Arg Val Glu Thr Asn Tyr Asp His Trp
            100                 105                 110
Lys Pro Ala Pro Lys Glu Asp Asp Arg Arg Thr
        115                 120

<210> 502
<211> 111
<212> PRT
<213> Homo sapiens

<220>
<221> misc_feature
<222> (1)...(111)
<223> Xaa = any amino acid or nothing

<400> 502
Leu Val Ala Ala Trp Ala Pro Leu Trp Gly Gly Ala Ala Gly Leu Phe
1               5                   10                  15
Thr Gly Ser Leu Asn Thr Gln Ala Pro Ser Thr Met Ala Ser Arg Gly
            20                  25                  30
Gly Gly Arg Gly Arg Gly Arg Gly Gln Leu Thr Phe Asn Val Glu Ala
        35                  40                  45
Val Gly Ile Gly Lys Gly Asp Ala Leu Pro Pro Pro Thr Leu Gln Pro
    50                  55                  60
Ser Pro Leu Phe Pro Ile Gly Gly Val Tyr Pro Gly Ser Xaa Arg Ser
65                  70                  75                  80
Glu Cys Gly Ser Tyr Arg Arg Asn Gly Leu Gln Phe Cys Ser Pro Arg
                85                  90                  95
Gly Pro Leu Arg Pro Gln Lys Ile Arg Arg Lys Gln Tyr Arg Asn
            100                 105                 110

<210> 503
<211> 42
<212> PRT
<213> Homo sapiens

<400> 503
Val Cys Ser Asp Leu Ser Leu Ser Pro Ser Ser Pro Gly Cys Cys Ser
1               5                   10                  15
Cys Cys Pro Val Gly Cys Ala Lys Cys Ala Gln Gly Cys Ile Cys Lys
            20                  25                  30
Gly Thr Ser Asp Lys Cys Ser Cys Cys Ala
        35                  40

<210> 504
<211> 437
<212> PRT
<213> Homo sapiens

<400> 504
Met Trp Leu Trp Glu Asp Gln Gly Gly Leu Leu Gly Pro Phe Ser Phe
1               5                   10                  15
Leu Leu Leu Val Leu Leu Leu Val Thr Arg Ser Pro Val Asn Ala Cys
            20                  25                  30
Leu Leu Thr Gly Ser Leu Phe Val Leu Leu Arg Val Phe Ser Phe Glu
        35                  40                  45
Pro Val Pro Ser Cys Arg Ala Leu Gln Val Leu Lys Pro Arg Asp Arg
    50                  55                  60
Ile Ser Ala Ile Ala His Arg Gly Gly Ser His Asp Ala Pro Glu Asn
65                  70                  75                  80
Thr Leu Ala Ala Ile Arg Gln Gly Ser Pro Ile Phe Ser Gly Arg Pro
                85                  90                  95
Ser His Phe Leu Ile Arg Pro Gln Gly Thr Leu Pro Ala Leu Asp Arg
            100                 105                 110
Ser Gln Cys Leu Gly Ile Ser Gly Leu Glu Val Leu Tyr Leu Gln Lys
        115                 120                 125
Thr Ala Asp Met Tyr Ile Val Ser Val Arg Leu Thr Leu Arg Leu His
    130                 135                 140
Thr Ala Arg Asn Lys Ala Ala Lys Asn Gly Ala Thr Gly Val Glu Leu
145                 150                 155                 160
Asp Ile Glu Phe Thr Ser Asp Gly Ile Pro Val Leu Met His Asp Asn
                165                 170                 175
Thr Val Asp Arg Thr Thr Asp Gly Thr Gly Arg Leu Cys Asp Leu Thr
            180                 185                 190
Phe Glu Gln Ile Arg Lys Leu Asn Pro Ala Ala Asn His Arg Leu Arg
        195                 200                 205
Asn Asp Phe Pro Asp Glu Lys Ile Pro Thr Leu Arg Glu Ala Val Ala
    210                 215                 220
Glu Cys Leu Asn His Asn Leu Thr Ile Phe Phe Asp Val Lys Gly His
225                 230                 235                 240
Ala His Lys Ala Thr Glu Ala Leu Lys Lys Met Tyr Met Glu Phe Pro
                245                 250                 255
Gln Leu Tyr Asn Asn Ser Val Val Cys Ser Phe Leu Pro Glu Val Ile
            260                 265                 270
Tyr Lys Met Arg Gln Thr Asp Arg Asp Val Ile Thr Ala Leu Thr His

275                 280                 285
Arg Pro Trp Ser Leu Ser His Thr Gly Asp Gly Lys Pro Arg Tyr Asp
    290                 295                 300
Thr Phe Trp Lys His Phe Ile Phe Val Met Met Asp Ile Leu Leu Asp
305                 310                 315                 320
Trp Ser Met His Asn Ile Leu Trp Tyr Leu Cys Gly Ile Ser Ala Phe
                325                 330                 335
Leu Met Gln Lys Asp Phe Val Ser Pro Ala Tyr Leu Lys Lys Trp Ser
            340                 345                 350
Ala Lys Gly Ile Gln Val Val Gly Trp Thr Val Asn Thr Phe Asp Glu
        355                 360                 365
Lys Ser Tyr Tyr Glu Ser His Leu Gly Ser Ser Tyr Ile Thr Asp Ser
    370                 375                 380
Met Leu Thr Ala Ser His Leu Ser Thr Pro Ser Val Ala Phe Val Gln
385                 390                 395                 400
Ala His Ser Leu Ala Met Ala Ser Leu Leu His Pro Cys Arg Gly Leu
                405                 410                 415
Leu Ala Ser His Leu Gln Gln Leu Asp Ser Asp Phe Arg Arg Pro Pro
            420                 425                 430
Val Pro Ile Ala Met
        435

<210> 505
<211> 487
<212> PRT
<213> Homo sapiens

<400> 505
Met Ala Pro Ala Pro Pro Pro Ala Ala Ser Phe Ser Pro Ser Glu Val
1               5                   10                  15
Gln Arg Arg Leu Ala Ala Gly Ala Cys Trp Val Arg Arg Gly Ala Arg
            20                  25                  30
Leu Tyr Asp Leu Ser Ser Phe Val Arg His His Pro Gly Gly Glu Gln
        35                  40                  45
Leu Leu Arg Ala Arg Ala Gly Gln Asp Ile Ser Ala Asp Leu Asp Gly
    50                  55                  60
Pro Pro His Arg His Ser Ala Asn Ala Arg Arg Trp Leu Glu Gln Tyr
65                  70                  75                  80
Tyr Val Gly Glu Leu Arg Gly Glu Gln Gln Thr Gly Asp Lys His Pro
                85                  90                  95
Met Arg Ser Glu Thr His His Ile Thr Glu Thr Ala Leu Ala Gly Val
            100                 105                 110
Thr Arg Thr Phe Ala Phe Leu His Pro Val Gly Ser Met Glu Asn Glu
        115                 120                 125
Pro Val Ala Leu Glu Glu Thr Gln Lys Thr Asp Pro Ala Met Glu Pro
    130                 135                 140
Arg Phe Lys Val Val Asp Trp Asp Lys Asn Thr Ala Ser Gly Cys Leu
145                 150                 155                 160
Thr Pro Arg Ala Glu Asp Trp Leu Ser Ile His Arg Asp Thr Leu Ile
                165                 170                 175
Leu Thr Pro Ile Val Cys Gly Arg Leu Gly Pro Thr Ser Ile Thr Asp
            180                 185                 190
Glu Gly Thr Glu Gly Leu Arg Glu Glu Lys Met His Val Gln Asp Leu
        195                 200                 205
Thr Ala Gly Gln Arg His Ser Gln Ala Leu Met Asp Leu Val Asp Trp
    210                 215                 220
Arg Lys Pro Leu Leu Trp Gln Val Gly His Leu Gly Glu Lys Tyr Asp

225                 230                 235                 240
Glu Trp Val His Gln Pro Val Thr Arg Pro Ile Arg Leu Phe His Ser
                245                 250                 255
Asp Leu Ile Glu Gly Leu Ser Lys Thr Val Trp Tyr Ser Val Pro Ile
            260                 265                 270
Ile Trp Val Pro Leu Val Leu Tyr Leu Ser Trp Ser Tyr Tyr Arg Thr
        275                 280                 285
Phe Ala Gln Gly Asn Val Arg Leu Phe Thr Ser Phe Thr Thr Glu Tyr
    290                 295                 300
Thr Val Ala Val Pro Lys Ser Met Phe Pro Gly Leu Phe Met Leu Gly
305                 310                 315                 320
Thr Phe Leu Trp Ser Leu Ile Glu Tyr Leu Ile His Arg Phe Leu Phe
                325                 330                 335
His Met Lys Pro Pro Ser Asp Ser Tyr Tyr Leu Ile Met Leu His Phe
            340                 345                 350
Val Met His Gly Gln His His Lys Ala Pro Phe Asp Gly Ser Arg Leu
        355                 360                 365
Val Phe Pro Pro Val Pro Ala Ser Leu Val Ile Gly Val Phe Tyr Leu
    370                 375                 380
Cys Met Gln Leu Ile Leu Pro Glu Ala Val Gly Gly Thr Val Phe Ala
385                 390                 395                 400
Gly Gly Leu Leu Gly Tyr Val Leu Tyr Asp Met Thr His Tyr Tyr Leu
                405                 410                 415
His Phe Gly Ser Pro His Lys Gly Ser Tyr Leu Tyr Ser Leu Lys Ala
            420                 425                 430
His His Val Lys His His Phe Ala His Gln Lys Ser Gly Gly Ser His
        435                 440                 445
Pro Leu Gly Gly Gln Val Ala Leu Gly Asp Pro Leu Leu Pro Gly Ala
    450                 455                 460
Ser Leu Pro Arg Ala Gln Pro Thr Gly Leu Leu Gln Ala Val Ala Thr
465                 470                 475                 480
Gly Ser Ser Arg Lys Gly Lys
                485

<210> 506
<211> 209
<212> PRT
<213> Homo sapiens

<400> 506
Phe Val Ala Pro Ser Arg Ala Arg Gly Ala Ala Gly Ala Gly Ala Arg
1               5                   10                  15
Met Leu Glu Val His Ile Pro Ser Val Gly Pro Glu Ala Glu Gly Pro
            20                  25                  30
Arg Gln Ser Pro Glu Lys Ser His Met Val Phe Arg Val Glu Val Leu
        35                  40                  45
Cys Ser Gly Arg Arg His Thr Val Pro Arg Arg Tyr Ser Glu Phe His
    50                  55                  60
Ala Leu His Lys Arg Ile Lys Lys Leu Tyr Lys Val Pro Asp Phe Pro
65                  70                  75                  80
Ser Lys Arg Leu Pro Asn Trp Arg Thr Arg Gly Leu Glu Gln Arg Arg
                85                  90                  95
Gln Gly Leu Glu Ala Tyr Ile Gln Gly Ile Leu Tyr Leu Asn Gln Glu
            100                 105                 110
Val Pro Lys Glu Leu Leu Glu Phe Leu Arg Leu Arg His Phe Pro Thr
        115                 120                 125
Asp Pro Lys Ala Ser Asn Trp Gly Thr Leu Arg Glu Phe Leu Pro Gly

130                 135                 140
Asp Ser Ser Ser Gln Gln His Gln Arg Pro Val Leu Ser Phe His Val
145                 150                 155                 160
Asp Pro Tyr Val Cys Asn Pro Ser Pro Glu Ser Leu Pro Asn Val Val
                165                 170                 175
Val Asn Gly Val Leu Gln Gly Leu Tyr Ser Phe Ser Ile Ser Pro Asp
            180                 185                 190
Lys Ala Gln Pro Lys Ala Ala Cys Arg Pro Ala Pro Leu Pro Pro Met
        195                 200                 205
Pro

<210> 507
<211> 46
<212> PRT
<213> Homo sapiens

<400> 507

<210> 508
<211> 78
<212> PRT
<213> Homo sapiens

<220>
<221> misc_feature
<222> (1)...(78)
<223> Xaa = any amino acid or nothing

<400> 508
Arg Asn Arg Cys Thr Leu Cys Thr Gln His Leu Cys Glu Arg Lys Glu
 1               5                  10                  15
Ile Met Pro Leu Gly Ile Lys Pro Phe Thr Arg Pro Leu Ala Leu Arg
            20                  25                  30
Arg Pro Arg Gly Ser Pro Leu Ala Ala Pro Leu Arg Val Xaa Cys Gln
        35                  40                  45
Gly Ala Leu Phe Leu Phe Ser His Thr Gly Ala Val Tyr Thr Met Tyr
    50                  55                  60
Ser Asp Tyr Val Lys Arg Met Ala Gln Asp Ala Gly Glu Lys
65                  70                  75

<210> 509
<211> 129
<212> PRT
<213> Homo sapiens

Gly Thr Pro Arg Thr Gly Ala Ala Cys Trp Arg Ser Ala Ala Asn Arg
20 25 30
Gly Val Leu Leu Ser Gly Leu Ile Gly Val Val Ser Trp Lys Arg Pro
35 40 45
Leu Ser Leu Val Ile Thr Phe Phe Met Leu Leu Ser Ala Val Cys Val
50 55 60
Met Leu Asn Leu Ala Gly Ser Ile Leu Ser Cys Gln Asn Ala Gln Leu
65 70 75 80
Val Asn Ser Leu Glu Gly Cys Gln Leu Ile Lys Phe Asp Ser Val Glu
85 90 95
Val Cys Val Cys Cys Glu Leu Gln His Gln Ser Ser Gly Cys Ser Asn
100 105 110
Leu Gly Glu Thr Leu Lys Leu Asn Pro Leu Gln Glu Asn Cys Asn Ala
115 120 125
Val Arg Leu Thr Leu Lys Asp Leu Leu Phe Ser Val Cys Ala Leu Asn
130 135 140
Val Leu Ser Thr Ile Val Cys Ala Leu Ala Thr Ala Met Cys Cys Met
145 150 155 160
Gln Met Val Ser Ser Asp Val Leu Gln Met Phe Leu Pro Gln Arg Ser
165 170 175
His Pro Ala Asn Pro Thr Cys Val Thr Pro His Gly Thr Val Leu His
180 185 190
Gln Thr Leu Asp Phe Asp Glu Phe Ile Pro Pro Leu Pro Pro Pro Pro
195 200 205
Tyr Tyr Pro Pro Glu Tyr Thr Cys Thr Pro Ser Thr Glu Ala Gln Arg
210 215 220
Gly Leu His Leu Asp Phe Ala Pro Ser Pro Phe Gly Thr Leu Tyr Asp
225 230 235 240
Val Ala Ile Asn Ser Pro Gly Leu Leu Tyr Pro Ala Glu Leu Pro Pro
245 250 255
Pro Tyr Glu Ala Val Val Gly Gln Pro Pro Ala Ser Gln Val Thr Ser
260 265 270
Ile Gly Gln Gln Val Ala Glu Ser Ser Ser Gly Asp Pro Asn Thr Ser
275 280 285
Ala Gly Phe Ser Thr Pro Val Pro Ala Asp Ser Thr Ser Leu Leu Val
290 295 300
Ser Glu Gly Thr Ala Thr Pro Gly Ser Ser Pro Ser Pro Asp Gly Pro
305 310 315 320
Val Gly Ala Pro Ala Pro Ser Glu Pro Ala Leu Pro Pro Gly Arg Val
325 330 335
Ser Pro Glu Asp Pro Gly Met Gly Ser Gln Val Gln Pro Gly Pro Gly
340 345 350
His Val Ser Arg Ser Thr Ser Asp Pro Thr Leu Cys Thr Ser Ser Met
355 360 365
Ala Gly Asp Ala Ser Ser His Arg Pro Ser Cys Ser Gln Asp Leu Glu
370 375 380
Ala Gly Leu Ser Glu Ala Val Pro Gly Ser Ala Ser Met Ser Arg Ser
385 390 395 400
Ala Thr Ala Ala Cys Arg Ala Gln Leu Ser Pro Ala Gly Asp Pro Asp
405 410 415
Thr Trp Lys Thr Asp Gln Arg Pro Thr Pro Glu Pro Phe Pro Ala Thr
420 425 430
Ser Lys Glu Arg Pro Arg Ser Leu Val Asp Ser Lys Ala Tyr Ala Asp
435 440 445
Ala Arg Val Leu Val Ala Lys Phe Leu Glu His Ser His Cys Ala Leu
450 455 460
Pro Thr Glu Ala Gln His Met Val Gly Ala Met Arg Leu Ala Val Thr
465 470 475 480
Asn Glu Glu Arg Leu Glu Glu Glu Ala Val Phe Gly Ala Asp Val Leu
485 490 495

<400> 509
Lys Val His Met Asn Gly Leu Arg Tyr Ala Leu Tyr Ile Met Gly Pro
1               5                   10                  15
Ala Leu Thr Arg Gly Ser Thr Arg Cys Leu Leu Ser Met Leu Arg Ser
            20                  25                  30
Asn Arg Thr Asp Leu Asp Leu Met Met Lys Glu Ala Tyr Phe Gly Glu
        35                  40                  45
Val Asp Ile Met Asp Asp Leu Pro Thr Gly Asn Ile Ser Ala Asn Val
    50                  55                  60
Thr Gly Arg Leu Tyr Lys Cys Gly Val Ile Asn His Thr Val Asp Leu
65                  70                  75                  80
Arg Arg Met Lys Thr Glu Lys Trp Gln Asn Asn Leu Leu Pro Ser Arg
                85                  90                  95
Gln Phe Gly Phe Ile Val Leu Thr Thr Ser Ala Gly Ile Met Asp His
            100                 105                 110
Glu Glu Ala Arg Arg Lys His Thr Gly Gly Lys Ile Leu Gly Phe Phe
        115                 120                 125
Phe

<210> 510
<211> 137
<212> PRT
<213> Homo sapiens

<400> 510
Ala Ser Thr Ile Arg Arg Arg Cys Phe His Ser Val Ile Ser Thr Glu
1               5                   10                  15
His Arg Gly Leu Thr Met Glu Phe Gly Leu Ser Trp Val Phe Leu Val
            20                  25                  30
Ala Leu Leu Arg Gly Gly Gln Cys His Glu Gln Leu Val Glu Ser Gly
        35                  40                  45
Gly Gly Val Val Gln Pro Gly Arg Ser Leu Arg Leu Ser Cys Val Ala
    50                  55                  60
Pro Lys Phe Met Phe Ser Asp Ser Ser Ser Ser Ser Ser Ser Ser Ser
65                  70                  75                  80
Ser Ser Ser Ser Ser Ser Ser Ser Ser Ser Ser Ser Ser Ser Ser Pro
                85                  90                  95
Asp Lys His Tyr Ala Asp Ser Val Lys Gly Arg Phe Thr Ile Ser Arg
            100                 105                 110
Asp Asn Ser Lys Asn Thr Leu Tyr Leu Gln Met Asn Ser Leu Ser Ala
        115                 120                 125
Glu Asp Thr Ala Val Tyr Tyr Cys Ala
    130                 135

<210> 511
<211> 499
<212> PRT
<213> Homo sapiens

<400> 511
Phe Arg Ala Arg Arg Gln Gly Arg Leu Gly Asp Pro Val Arg Arg Cys
1               5                   10                  15

Asp Gln Val

<210> 512
<211> 74
<212> PRT
<213> Homo sapiens

<400> 512
Leu Gln Ala Ile Thr Met Ser Thr Asp Thr Gly Val Ser Leu Pro Ser
1               5                   10                  15
Tyr Glu Glu Asp Gln Gly Ser Lys Leu Ile Arg Lys Ala Lys Glu Ala
            20                  25                  30
Pro Phe Val Pro Val Val Phe Gly Asp Ser Pro Ala Leu Ser Pro Arg
        35                  40                  45
Leu Glu Cys Ser Gly Arg Ile Ser Ala His Cys Ser Leu Cys Leu Leu
    50                  55                  60
Gly Ser Ser Asp Ser Pro Thr Ser Ala Ser
65                  70

<210> 513
<211> 138
<212> PRT
<213> Homo sapiens

<400> 513
Glu Tyr Val Thr Val Arg Arg Ile Asn Leu Glu Ala Cys Ser Asn Glu
1               5                   10                  15
Met Val Thr Phe Leu Gln Gly Glu Leu His Val Ser Lys Leu Phe Asn
            20                  25                  30
His Pro Asn Ile Val Pro Tyr Arg Ala Thr Phe Ile Ala Asp Asn Glu
        35                  40                  45
Leu Trp Val Val Thr Ser Phe Met Ala Tyr Gly Ser Ala Lys Asp Leu
    50                  55                  60
Ile Cys Thr His Phe Met Asp Gly Met Asn Glu Leu Ala Ile Ala Tyr
65                  70                  75                  80
Ile Leu Gln Gly Val Leu Lys Ala Leu Asp Tyr Ile His His Met Gly
                85                  90                  95
Tyr Val His Arg Ser Val Lys Ala Ser His Ile Leu Ile Ser Val Asp
            100                 105                 110
Gly Lys Val Tyr Leu Ser Gly Leu Arg Ser Asn Leu Ser Cys Pro Gly
        115                 120                 125
Ala Phe Ser Lys Gly His Gln Cys Leu Ala
    130                 135

<210> 514
<211> 423
<212> PRT
<213> Homo sapiens

<220>
<221> misc_feature

<222> (1)...(423)
<223> Xaa = any amino acid or nothing

<400> 514
Met Pro Pro Gly Val Phe Ser Gly Leu Thr Leu Leu Arg Leu Asn Leu
1               5                   10                  15
Arg Ser Asn His Phe Thr Ser Leu Pro Val Ser Gly Val Leu Asp Gln
            20                  25                  30
Leu Lys Ser Leu Ile Gln Ile Asp Leu His Asp Asn Pro Trp Asp Cys
        35                  40                  45
Thr Cys Asp Ile Val Gly Met Lys Leu Trp Val Glu Gln Leu Lys Val
    50                  55                  60
Gly Val Leu Val Asp Glu Val Ile Cys Lys Ala Pro Lys Lys Phe Ala
65                  70                  75                  80
Glu Thr Asp Met Arg Ser Ile Lys Ser Glu Leu Leu Cys Pro Asp Tyr
                85                  90                  95
Ser Asp Val Val Val Ser Thr Pro Thr Pro Ser Ser Ile Gln Val Pro
            100                 105                 110
Ala Arg Thr Ser Ala Val Thr Pro Ala Val Arg Pro Asn Ser Thr Gly
        115                 120                 125
Ala Pro Ala Ser Leu Gly Ala Gly Gly Gly Ala Ser Ser Val Pro Leu
    130                 135                 140
Ser Val Leu Ile Leu Ser Leu Leu Leu Val Phe Ile Met Ser Val Phe
145                 150                 155                 160
Val Ala Ala Gly Leu Phe Val Leu Lys Pro Ile Asp Tyr Tyr Gly Glu
                165                 170                 175
Ile Cys Asp Asn Ala Cys Leu Cys Glu Glu Lys Val Arg His Phe Asn
            180                 185                 190
Cys Glu Leu Xaa Lys Pro Gly Asp His Gln Ser Leu Xaa Asn Xaa Pro
        195                 200                 205
Ser Pro Phe Pro Asn Leu Pro Pro Leu Arg Cys Pro Glu Thr Phe Xaa
    210                 215                 220
Thr Val Ser Ile Pro Met Ser Leu Ser His Tyr Thr Gly Ala Ser Ile
225                 230                 235                 240
Leu His Leu Gly Ser Asn Val Ile Gln Asp Ile Glu Thr Gly Ala Phe
                245                 250                 255
His Gly Leu Arg Gly Leu Arg Arg Leu His Leu Asn Asn Asn Lys Leu
            260                 265                 270
Glu Leu Leu Gln Arg Xaa Tyr Leu Pro Trp Leu Gly Glu Pro Pro Val
        275                 280                 285
Pro Thr Gly Arg Leu Gln Leu His Gln Arg His Xaa Thr Gln Cys Phe
    290                 295                 300
Trp Glu Thr Ala Phe Val Ala Gly Ala Tyr Pro Arg Met Thr Ile Phe
305                 310                 315                 320
Cys Pro Val Tyr Pro Thr Ile Phe Ser Val Leu Cys Pro Tyr Cys Thr
                325                 330                 335
Trp Thr Ser Arg Gly Thr Gly Xaa Asn Phe Cys Pro Thr Trp Gly Ser
            340                 345                 350
Cys Ser Thr Gly Val Leu Xaa Ser Arg Thr Lys Gln Gly Ile Gln Arg
        355                 360                 365
Pro His Phe Arg Asp Ser Arg Leu Arg Glu Pro Val Leu Tyr Ser Pro
    370                 375                 380
Pro Ser Ala Val Phe Val Glu Pro Asn Arg Asn Glu Tyr Leu Glu Leu
385                 390                 395                 400
Lys Ala Lys Leu Asn Val Glu Pro Asp Tyr Leu Glu Val Leu Glu Lys
                405                 410                 415
Gln Thr Thr Phe Ser Gln Phe
            420

<210> 515
<211> 172
<212> PRT
<213> Homo sapiens

<400> 515
Gly Ser Val Thr Pro Ala Ile Arg Ser Leu Ala Leu Leu Ala Ala Ile
1               5                   10                  15
Leu Leu Val Ala Leu Gln Ala Arg Ala Glu Pro Leu Gln Ala Ile Ala
            20                  25                  30
Asp Glu Ala Thr Ala Gln Glu Gln Pro Gly Ala Asp Asp Gln Glu Val
        35                  40                  45
Val Asp Ser Phe Ala Trp Asp Glu Arg Ala Pro Leu Gln Val Ser Gly
    50                  55                  60
Lys Ser Ser Pro Val Cys Ala Arg Leu Leu Leu Leu Gln Glu Thr Arg
65                  70                  75                  80
Asp Arg Gly Leu Leu Phe Ala Leu Pro Leu His Ser Ala Tyr Leu Glu
                85                  90                  95
Asp Leu Leu Arg Gln Ser His Phe Arg Gln Glu Leu Met Lys Leu Gln
            100                 105                 110
Pro Arg Ser Ser Leu Glu Gln Met Ile Arg Lys Trp Leu Met Pro Leu
        115                 120                 125
His Gly Met Lys Val Pro Leu Phe Arg Phe Gln Pro Asp Lys Ile Ile
    130                 135                 140
Val Leu Ser Thr Leu Ile Pro Thr Gly Asp Tyr Ser Pro His Asn Leu
145                 150                 155                 160
Lys Asn Leu Phe Met Arg Met Val Thr Pro Ser Pro
                165                 170

<210> 516
<211> 81
<212> PRT
<213> Homo sapiens

<400> 516
Ala His Val Asp Thr Met Ser Lys Ala His Pro Pro Glu Leu Lys Lys
1               5                   10                  15
Phe Ile Asp Lys Lys Leu Ser Leu Lys Leu Asn Gly Gly Arg His Val
            20                  25                  30
Gln Gly Ile Leu Trp Gly Phe Asp Pro Leu Met Asn Leu Val Ile Asp
        35                  40                  45
Lys Cys Val Glu Met Ala Thr Ser Gly Gln Gln Lys Asn Ile Gly Met
    50                  55                  60
Val Val Ile Arg Arg Asn Ser Ile Ile Met Leu Glu Thr Leu Glu Gln
65                  70                  75                  80
Glu

<210> 517
<211> 121
<212> PRT
<213> Homo sapiens

<400> 517

<210> 518
<211> 56
<212> PRT
<213> Homo sapiens

<400> 518
Asn Asn Phe Leu Glu Met Arg Ser Leu Leu Pro Arg Leu Glu Cys Ser
1               5                   10                  15
Gly Val Ile Ala Ala His Cys Ser Leu Glu Leu Leu Gly Ser Ser Asp
            20                  25                  30
Pro Pro Ala Ser Ala Ser Gln Ser Val Gly Thr Thr Gly Val Ser His
        35                  40                  45
Gly Thr Cys Leu His Gln Tyr Ser
    50                  55

<210> 519
<211> 263
<212> PRT
<213> Homo sapiens

<400> 519
His Ser Met Ile Glu Thr Tyr Asn Gln Thr Ser Pro Arg Ser Ala Ala
1               5                   10                  15
Thr Gly Leu Pro Ile Ser Met Lys Ile Phe Met Tyr Leu Leu Thr Val
            20                  25                  30
Phe Leu Ile Thr Gln Met Ile Gly Ser Ala Leu Phe Ala Val Tyr Leu
        35                  40                  45
His Arg Arg Leu Asp Lys Ile Glu Asp Glu Arg Asn Leu His Glu Asp
    50                  55                  60
Phe Val Phe Met Lys Thr Ile Gln Arg Cys Asn Thr Gly Glu Arg Ser
65                  70                  75                  80
Leu Ser Leu Leu Asn Cys Glu Glu Ile Lys Ser Gln Phe Glu Gly Phe
                85                  90                  95
Val Lys Asp Ile Met Leu Asn Lys Glu Glu Thr Lys Lys Glu Asn Ser

                100                 105                 110
Phe Glu Met Gln Lys Gly Asp Gln Asn Pro Gln Ile Ala Ala His Val
        115                 120                 125
Ile Ser Glu Ala Ser Ser Lys Thr Thr Ser Val Leu Gln Trp Ala Glu
    130                 135                 140
Lys Gly Tyr Tyr Thr Met Ser Asn Asn Leu Val Thr Leu Glu Asn Gly
145                 150                 155                 160
Lys Gln Leu Thr Val Lys Arg Gln Gly Leu Tyr Tyr Ile Tyr Ala Gln
                165                 170                 175
Val Thr Phe Cys Ser Asn Arg Glu Ala Ser Ser Gln Ala Pro Phe Ile
            180                 185                 190
Ala Ser Leu Cys Leu Lys Ser Pro Gly Arg Phe Glu Arg Ile Leu Leu
        195                 200                 205
Arg Ala Ala Asn Thr His Ser Ser Ala Lys Pro Cys Gly Gln Gln Ser
    210                 215                 220
Ile His Leu Gly Gly Val Phe Glu Leu Gln Pro Gly Ala Ser Val Phe
225                 230                 235                 240
Val Asn Val Thr Asp Pro Ser Gln Val Ser His Gly Thr Gly Phe Thr
                245                 250                 255
Ser Phe Gly Leu Leu Lys Leu
            260

<210> 520
<211> 68
<212> PRT
<213> Homo sapiens

<220>
<221> misc_feature
<222> (1)...(68)
<223> Xaa = any amino acid or nothing

<400> 520
Pro Thr Cys Val Tyr Val Cys Val Leu Lys Gln Lys Cys Val Cys Val
 1               5                  10                  15
Cys Ala Cys Val Ala Arg Ala Ala Cys Thr Cys Val Pro Val Phe Val
            20                  25                  30
Cys Leu Cys Val Xaa Lys Pro Trp Xaa Gly Phe Asn Cys Gly Val Phe
        35                  40                  45
Pro Cys Ile Gln Val Tyr Ala Phe Phe Phe Trp Arg Ile Gly Ser Asn
    50                  55                  60
Gly Cys Tyr Tyr
65

<210> 521
<211> 211
<212> PRT
<213> Homo sapiens

<220>
<221> misc_feature
<222> (1)...(211)
<223> Xaa = any amino acid or nothing

<400> 521
Arg Ser Pro Leu Leu Glu Trp Leu Pro Thr Leu Cys Phe Tyr Asn Gly
1               5                   10                  15
Lys Gly Leu Glu Gln Ile Glu Arg Asp Asn Ser Phe His Asn Val Ala
            20                  25                  30
Glu Ala Thr Phe Thr Leu Lys Leu Ile Gln Ser Leu Ile Ala Ser Gly
        35                  40                  45
Ile Ala Gly Ser Met Ile Gly Val Ile Thr Leu Tyr Lys Ser Gln Met
    50                  55                  60
Tyr Lys Leu Cys His Leu Leu Ser Ala Val Asp Phe His His Pro Asp
65                  70                  75                  80
Ile Lys Thr Val Gln Val Ser Thr Val Asp Ala Phe Lys Glu Ala Glu
                85                  90                  95
Lys Glu Ile Ile Ile Leu Ser Cys Val Arg Thr Arg Gln Val Gly Phe
            100                 105                 110
Ile Asp Ser Glu Lys Arg Met Asn Val Ala Leu Thr Arg Gly Lys Arg
        115                 120                 125
His Leu Leu Ile Val Gly Asn Leu Ala Cys Leu Arg Lys Asn Gln Leu
    130                 135                 140
Trp Gly Arg Val Ile Gln His Cys Glu Gly Arg Glu Asp Gly Phe Asn
145                 150                 155                 160
Met Gln Thr Ser Met Asn His Ser Xaa Thr Ile Ser Leu Lys Ile Ile
                165                 170                 175
Leu Lys Asn Lys Trp Lys Lys Asn Arg Arg Lys Arg Val Lys Lys Arg
            180                 185                 190
Asn Leu Lys Ile Asn Leu Ile His Lys Lys Thr Trp Cys Lys Tyr Phe
        195                 200                 205
Val Phe Met
    210

<210> 522
<211> 42
<212> PRT
<213> Homo sapiens

<400> 522
Thr Thr Thr Ala Leu Gly Leu Leu Thr Ile Pro Ile Ile Ile His Pro
1               5                   10                  15
Ile Asp Arg Ser Val Asp Phe Leu Leu Asp Ser Ser Leu Arg Lys Leu
            20                  25                  30
Tyr Pro Thr Val Gly Lys Pro Ser Ser Ser
        35                  40

<210> 523
<211> 27
<212> PRT
<213> Homo sapiens

<400> 523

<210> 524
<211> 47
<212> PRT
<213> Homo sapiens

<400> 524
Phe Leu Thr Pro Ser Pro Glu Ser Pro Asp Gln Trp Ser Ser Ser Ser
1               5                   10                  15
Pro His Ser Asn Val Ser Asp Trp Ser Glu Gly Val Ser Ser Pro Pro
            20                  25                  30
Thr Ser Met Gln Ser Gln Ile Ala Arg Ile Pro Glu Ala Phe Lys
        35                  40                  45

<210> 525
<211> 283
<212> PRT
<213> Homo sapiens

<400> 525
Gly Ser Glu Gln Ala Ser Ser Val Ser Ser His Ala Ser Gly Glu Phe
1               5                   10                  15
Arg Phe Thr Ser Glu Val Pro Ile Arg Phe Asp Tyr His Gly Lys His
            20                  25                  30
Val Ser Met Asp Gln Gly Thr Leu Ala Gly Ile Leu Ile Gly Leu Ala
        35                  40                  45
Gln Leu Asn Cys Ser Glu Leu Lys Leu Lys Arg Leu Ser Tyr Arg His
    50                  55                  60
Gly Leu Leu Gly Val Asp Lys Leu Phe Ser Tyr Ala Ile Thr Glu Trp
65                  70                  75                  80
Leu Asn Asp Ile Lys Lys Asn Gln Leu Pro Arg Ile Leu Gly Gly Val
                85                  90                  95
Gly Pro Met His Ser Leu Val Gln Leu Val Gln Gly Leu Lys Asp Leu
            100                 105                 110
Val Trp Leu Pro Ile Glu Gln Tyr Arg Lys Asp Gly Arg Ile Val Arg
        115                 120                 125
Gly Phe Gln Arg Gly Ala Ala Ser Phe Gly Thr Ser Thr Ala Met Ala
    130                 135                 140
Ala Leu Glu Leu Thr Asn Arg Met Val Gln Thr Ile Gln Ala Ala Ala
145                 150                 155                 160
Glu Thr Ala Tyr Asp Met Val Ser Pro Gly Thr Leu Ser Ile Glu Pro
                165                 170                 175
Lys Lys Thr Lys Arg Phe Pro His His Arg Leu Ala His Gln Pro Val
            180                 185                 190
Asp Leu Arg Glu Gly Val Ala Lys Ala Tyr Ser Val Val Lys Glu Gly
        195                 200                 205
Ile Thr Asp Thr Ala Gln Thr Ile Tyr Glu Thr Ala Ala Arg Glu His
    210                 215                 220
Glu Ser Arg Gly Val Thr Gly Ala Val Gly Glu Val Leu Arg Gln Ile
225                 230                 235                 240
Pro Pro Ala Val Val Lys Pro Leu Ile Val Ala Thr Glu Ala Thr Ser
                245                 250                 255
Asn Val Leu Gly Gly Met Arg Asn Gln Ile Arg Pro Asp Val Arg Gln
            260                 265                 270
Asp Glu Ser Gln Lys Trp Arg His Gly Asp Asp

275                    280

<210> 526
<211> 110
<212> PRT
<213> Homo sapiens

<220>
<221> misc_feature
<222> (1)...(110)
<223> Xaa = any amino acid or nothing

<400> 526
Pro Ser His Pro Ser Ser Phe Gly Ser Ala Pro His Pro His Leu Leu
1               5                   10                  15
Pro Thr Thr Pro Ala Ala Pro Phe Pro Ala Gln Ala Ser Glu Cys Pro
            20                  25                  30
Val Ala Ala Ala Thr Ala Pro His Thr Pro Gly Pro Cys Gln Ser Ser
        35                  40                  45
His Leu Pro Ser Thr Ser Met Pro Pro Xaa Arg Cys Pro His His Ser
    50                  55                  60
Ser Gly Cys Ser His Pro Cys Ile Gly His Cys Gly Gly His Cys Ser
65                  70                  75                  80
Gly Pro Ser Pro Pro Thr Leu Xaa Leu Xaa Gly Cys Pro Xaa Leu Xaa
                85                  90                  95
Pro Gly Met Gly Val Gly Xaa Gly Ser Met Tyr Pro Pro Thr
            100                 105                 110

<210> 527
<211> 137
<212> PRT
<213> Homo sapiens

<400> 527
Glu Leu Lys Thr Phe Ser Cys Ser Leu Leu His Asp Gly Asp Pro Thr
1               5                   10                  15
Tyr Gln Arg Leu Phe Leu Asp Cys Leu Val His Ser Leu Arg Glu Leu
            20                  25                  30
His Thr Gly Asp Val Met Ile Leu Pro Val Leu Ser Cys Phe Thr Arg
        35                  40                  45
Phe Met Ala Gly Leu Ile Phe Val Leu His Ser Cys Phe Arg Phe Ile
    50                  55                  60
Thr Phe Val Cys Pro Thr Ser Ser Asp Pro Leu Arg Thr Cys Ala Val
65                  70                  75                  80
Leu Leu Cys Val Gly Tyr Gln Asp Leu Pro Asn Pro Val Phe Arg Tyr
                85                  90                  95
Leu Gln Ser Val Asn Glu Leu Leu Ser Thr Leu Leu Asn Ser Asp Ser
            100                 105                 110
Pro Gln Gln Val Leu Gln Phe Val Pro Asn Gly Arg Tyr Ser Leu Arg
        115                 120                 125
Arg Pro Cys Leu Ile Phe Cys Gly Ile
    130                 135

<210> 528
<211> 133
<212> PRT
<213> Homo sapiens

<220>
<221> misc_feature
<222> (1)...(133)
<223> Xaa = any amino acid or nothing

<400> 528
Leu Ile Tyr Cys Asn Phe Asp Leu Leu Arg Arg Asp Val Leu Tyr Tyr
1               5                   10                  15
Lys Gly Arg Leu Asp Met Asp Gly Leu Glu Val Val Asp Leu Lys Asp
            20                  25                  30
Gly Lys Asp Arg Asp Leu His Val Ser Ile Arg Asn Ala Phe Arg Leu
        35                  40                  45
His Arg Gly Ala Thr Gly Asp Ser His Leu Leu Cys Thr Arg Lys Pro
    50                  55                  60
Glu Gln Lys Gln Arg Trp Ile Lys Ala Phe Ala Arg Glu Arg Glu Gln
65                  70                  75                  80
Val Gln Leu Asp Gln Glu Thr Gly Phe Ser Ile Thr Glu Leu Gln Arg
                85                  90                  95
Lys Gln Ala Met Leu Asn Ala Ser Lys Gln Leu Met Ala Gly Pro Ala
            100                 105                 110
Thr Xaa Arg Ala Arg Ser Thr Gln Pro Cys Pro Ala Thr Gly Pro Ser
        115                 120                 125
Ile Arg Thr Gly Ala
    130

<210> 529
<211> 112
<212> PRT
<213> Homo sapiens

<400> 529
Gly Leu Lys Gly Arg Gly Leu Lys His Tyr Pro Ile Arg Asp Ala Gly
1               5                   10                  15
Leu Gly Thr Ser Asn Gln Ala Gly Ser Trp Arg Gly Gln Val Gly Phe
            20                  25                  30
Arg Asp Val Val Gly Pro Leu Ser Leu Ala Ala Ala Gly Gly Gln Gly
        35                  40                  45
Leu Val Leu Thr Ala Val Cys Pro Leu Leu Leu Glu Ser Gln Pro Leu
    50                  55                  60
Trp Pro Tyr Asp Leu Gln Gly Ser Leu Thr Phe Arg Asp Val Ala Ile
65                  70                  75                  80
Glu Phe Ser Leu Glu Glu Trp Gln Cys Leu Asp Thr Ala Gln Gln Asn
                85                  90                  95
Leu Tyr Arg Asn Val Met Leu Glu Asn Tyr Arg Asn Leu Val Phe Leu
            100                 105                 110

<210> 530
<211> 155
<212> PRT
<213> Homo sapiens

<400> 530
Gly Glu Ala Glu Met Glu Glu Arg Ala Phe Val Asn Pro Phe Pro Asp
1               5                   10                  15
Tyr Glu Ala Ala Ala Gly Ala Leu Leu Ala Ser Gly Ala Ala Glu Glu
            20                  25                  30
Thr Gly Cys Val Arg Pro Pro Ala Thr Thr Asp Glu Pro Gly Leu Pro
        35                  40                  45
Phe His Gln Asp Gly Lys Ile Ile His Asn Phe Ile Arg Arg Ile Gln
    50                  55                  60
Thr Lys Ile Lys Asp Leu Leu Gln Gln Met Glu Glu Gly Leu Lys Thr
65                  70                  75                  80
Ala Asp Pro His Asp Cys Ser Ala Tyr Thr Gly Trp Thr Gly Ile Ala
                85                  90                  95
Leu Leu Tyr Leu Gln Leu Tyr Arg Val Thr Cys Asp Gln Thr Tyr Leu
            100                 105                 110
Ala Pro Ile His Leu Ile Thr Glu Lys Lys Pro Leu Arg Lys Leu Asn
        115                 120                 125
Gly Arg Arg Val Thr Phe Leu Cys Gly Asp Ala Gly Pro Leu Ala Val
    130                 135                 140
Gly Ala Val Ile Tyr His Lys Leu Arg Ser Asp
145                 150                 155

<210> 531
<211> 73
<212> PRT
<213> Homo sapiens

<220>
<221> misc_feature
<222> (1)...(73)
<223> Xaa = any amino acid or nothing

<400> 531
Thr Phe Thr Ile Ile Xaa Lys Met Thr Ala Cys Leu Ser Xaa Ser Leu
1               5                   10                  15
Leu Xaa Lys Ile Leu Glu Ser Phe Lys Ala Ser Xaa Xaa Asn Ala Ser
            20                  25                  30
Gly Xaa Pro Arg Met Thr Gly Val Thr Ile Thr Ile Gly Lys Thr Xaa
        35                  40                  45
Ile Glu Glu Xaa Thr Ser Gln Phe Met Ala Lys Leu Ser Ala Leu Val
    50                  55                  60
Thr Val Thr Asn Ser His Arg Tyr Phe
65                  70

<210> 532
<211> 227
<212> PRT
<213> Homo sapiens

<400> 532
Glu Asp Leu Leu Gln Phe Ser Ala Asn Ile Pro Ala Leu Tyr Asn Ser
1               5                   10                  15
Lys Gln Leu Glu Asp Phe Phe Lys Gly Gly Ile Ile Asn Asp Ser Ser
            20                  25                  30
Glu Leu Ile Gly Pro Ala Glu Ala His Ser Asp Ser Leu Ile Asp Thr
        35                  40                  45
Phe Pro Glu Cys Ser Thr Glu Gly Phe Ser Ser Asp Ser Asp Leu Val
    50                  55                  60
Ser Leu Thr Val Asp Val Asp Ser Leu Ala Glu Leu Asp Asp Gly Met
65                  70                  75                  80
Ala Ser Asn Gln Asn Ser Pro Ile Arg Thr Phe Gly Leu Asn Leu Ser
                85                  90                  95
Ser Asp Ser Ser Ala Leu Gly Ala Val Ala Ser Asp Ser Glu Gln Ser
            100                 105                 110
Lys Thr Glu Glu Glu Arg Glu Ser Arg Ser Leu Phe Pro Gly Ser Leu
        115                 120                 125
Lys Pro Lys Leu Gly Lys Arg Asp Tyr Leu Glu Lys Ala Gly Glu Leu
    130                 135                 140
Ile Lys Leu Ala Leu Lys Lys Glu Glu Glu Asp Asp Tyr Glu Ala Ala
145                 150                 155                 160
Ser Asp Phe Tyr Arg Lys Gly Val Asp Leu Leu Leu Glu Gly Val Gln
                165                 170                 175
Gly Glu Ser Ser Pro Thr Arg Arg Glu Ala Val Lys Arg Arg Thr Ala
            180                 185                 190
Glu Tyr Leu Met Arg Ala Glu Ser Ile Ser Ser Leu Tyr Gly Lys Pro
        195                 200                 205
Gln Leu Asp Asp Val Ser Tyr Val Cys Leu Gln Leu Leu Met Ala Leu
    210                 215                 220
Leu Leu Ile
225

<210> 533
<211> 122
<212> PRT
<213> Homo sapiens

<400> 533

<210> 534
<211> 138
<212> PRT
<213> Homo sapiens

<400> 534

<210> 535
<211> 389
<212> PRT
<213> Homo sapiens

<220>
<221> misc_feature
<222> (1)...(389)
<223> Xaa = any amino acid or nothing

<400> 535
Gly Arg Lys Lys Arg Arg Asn Thr Gln Lys Trp Leu Arg Glu Trp Arg
1               5                   10                  15
Ala Ala Gln Gly Lys Asp Pro Gly Pro Ser Glu Lys Gln Lys Pro Val
            20                  25                  30
Phe Thr Pro Leu Arg Arg Pro Gly Met Leu Val Pro Lys Gln Asn Val
        35                  40                  45
Ser Pro Pro Asp Met Ser Ala Leu Ser Leu Lys Gly Asp Gln Ala Leu
    50                  55                  60
Leu Gly Gly Ile Phe Tyr Phe Leu Asn Ile Phe Ser His Gly Glu Leu
65                  70                  75                  80
Pro Pro His Cys Glu Gln Arg Phe Leu Pro Cys Glu Ile Gly Cys Val
                85                  90                  95
Lys Tyr Ser Leu Gln Glu Gly Ile Met Ala Asp Phe His Ser Phe Ile
            100                 105                 110
Asn Pro Gly Glu Ile Pro Arg Gly Phe Xaa Phe His Cys Gln Ala Ala
        115                 120                 125
Ser Asp Ser Ser His Lys Ile Pro Ile Ser Asn Phe Glu Arg Gly His
    130                 135                 140
Asn Gln Ala Thr Val Leu Gln Asn Leu Tyr Arg Phe Ile His Pro Asn

145                 150                 155                 160
Pro Gly Asn Trp Pro Pro Ile Tyr Cys Lys Ser Asp Asp Arg Thr Arg
                165                 170                 175
Val Asp Trp Cys Leu Lys His Met Ala Lys Ala Ser Glu Ile Arg Gln
            180                 185                 190
Asp Leu Gln Leu Leu Thr Val Glu Asp Leu Val Val Gly Ile Tyr Gln
        195                 200                 205
Gln Lys Phe Leu Lys Glu Pro Ser Lys Thr Trp Ile Arg Ser Leu Leu
    210                 215                 220
Asp Val Ala Met Trp Asp Tyr Ser Ser Asn Thr Arg Cys Lys Trp His
225                 230                 235                 240
Glu Glu Asn Asp Ile Leu Phe Cys Ala Leu Ala Val Cys Lys Lys Ile
                245                 250                 255
Ala Tyr Cys Ile Ser Asn Ser Leu Ala Thr Leu Phe Gly Ile Gln Leu
            260                 265                 270
Thr Glu Ala His Val Pro Leu Gln Asp Tyr Glu Ala Ser Asn Ser Val
        275                 280                 285
Thr Pro Lys Met Val Val Leu Asp Ala Gly Arg Tyr Gln Lys Leu Arg
    290                 295                 300
Val Gly Ser Ser Gly Phe Ser His Phe Asn Ser Ser Asn Glu Glu Gln
305                 310                 315                 320
Arg Ser Asn Thr Pro Ile Gly Asp Tyr Pro Ser Arg Ala Lys Ile Ser
                325                 330                 335
Gly Gln Asn Ser Ser Val Arg Gly Arg Gly Ile Thr Pro Pro Leu Leu
            340                 345                 350
Glu Ser Ile Ser Asn Ser Ser Ser Asn Ile His Lys Phe Ser Asn Cys
        355                 360                 365
Asp Thr Ser Leu Ser Pro Tyr Met Ser Gln Lys Asp Gly Tyr Lys Ser
    370                 375                 380
Phe Ser Ser Leu Ser
385

<210> 536
<211> 186
<212> PRT
<213> Homo sapiens

<220>
<221> misc_feature
<222> (1)...(186)
<223> Xaa = any amino acid or nothing

<400> 536
Ser Pro Ser Ser Gly Ser Cys Pro Ser Pro Ser Pro Ala His Ser Ser
 1               5                  10                  15
Ser Asp Ser Ile Thr Gln Val Ile Gly Val Gln Val Ala Phe Gly Tyr
            20                  25                  30
Ile Ser Cys Leu Pro Thr Leu Arg Pro Leu Ser Arg Thr Cys Cys Leu
        35                  40                  45
Leu Ser Pro Pro Ser Pro Leu Thr Leu Thr Leu Ser Pro Lys Pro Leu
    50                  55                  60
Pro Leu Leu Arg Thr Ala Pro Pro Val Arg Pro Pro Pro Xaa Pro Pro
65                  70                  75                  80
Val Leu Pro Arg Arg Pro Arg Pro Arg Leu Thr Gln Thr Leu Tyr Arg
                85                  90                  95
Leu Ala Arg Pro Pro Leu Pro Arg Gly Pro Ala Pro Leu Pro Pro Pro
            100                 105                 110

Arg Pro Gln Leu Gln Pro Pro Arg Ser Pro Pro Ser Leu Thr Xaa Ala
        115                 120                 125
Thr Pro Arg Pro Gln Pro Leu Pro Leu Ala Ile Cys Leu Leu Val Ser
    130                 135                 140
Trp Leu Thr Lys Gly Leu Ser Ser Ala Gln Ala Pro Leu Leu Val Ser
145                 150                 155                 160
Gly Arg Pro Leu Arg Leu Leu Arg Pro Ala Ser Pro Pro Leu His Gln
                165                 170                 175
Ser Pro Pro Arg Leu Gly Gln Pro Val Leu
            180                 185

<210> 537
<211> 66
<212> PRT
<213> Homo sapiens

<400> 537

<210> 538
<211> 195
<212> PRT
<213> Homo sapiens

<400> 538
Ile Arg Asp Ser Gly Glu Cys Ser Asp Cys Gly Lys Thr Phe Ser Cys
 1               5                  10                  15
Ser Ser Ala Leu Ile Leu His Arg Arg Ile His Thr Gly Glu Lys Pro
            20                  25                  30
Tyr Glu Cys Asn Glu Cys Gly Lys Thr Phe Ser Trp Ser Ser Thr Leu
        35                  40                  45
Thr His His Gln Arg Ile His Thr Gly Glu Lys Pro Tyr Ala Cys Asn
    50                  55                  60
Glu Cys Gly Lys Ala Phe Ser Arg Ser Ser Thr Leu Ile His His Gln
65                  70                  75                  80
Arg Ile His Thr Gly Glu Lys Pro Tyr Glu Cys Asn Glu Cys Gly Lys
                85                  90                  95
Ala Phe Ser Gln Asn Ser His Leu Tyr Gln His Gln Arg Ile His Thr
            100                 105                 110
Gly Glu Lys Pro Tyr Glu Cys Met Glu Cys Gly Gly Lys Phe Thr Tyr
        115                 120                 125
Ser Ser Gly Leu Ile Gln His Gln Arg Ile His Thr Gly Glu Asn Pro
    130                 135                 140
Tyr Glu Cys Ser Glu Cys Gly Lys Ala Phe Arg Tyr Ser Ser Ala Leu
145                 150                 155                 160

Val Arg His Gln Arg Ile His Thr Gly Glu Lys Pro Leu Asn Gly Ile
                165                 170                 175
Gly Met Ser Lys Ser Ser Leu Arg Val Thr Thr Glu Leu Asn Ile Arg
            180                 185                 190
Glu Ser Thr
        195

<210> 539
<211> 136
<212> PRT
<213> Homo sapiens

<220>
<221> misc_feature
<222> (1)...(136)
<223> Xaa = any amino acid or nothing

<400> 539
Arg Arg Val Leu Cys Tyr Pro Leu Tyr Arg His Phe Lys Leu Val Met
  1               5                  10                  15
Arg Ala Tyr Arg Asp Thr Ile Asp Ile Leu Gln Leu Gly Lys Ser Ala
             20                  25                  30
Ala Leu Lys Cys Leu Leu Asp Ile His Lys Ile Phe Gln Asp Asn Asp
         35                  40                  45
Pro Ala Tyr Ile Leu Asn Asp Leu Tyr Ile Ser Tyr Tyr Cys Val Trp
     50                  55                  60
Ile His Lys Val Lys Ser Lys Lys Leu Ser Asp Leu Ala Glu Ala Leu
 65                  70                  75                  80
Lys Glu Val Ser Leu Thr Lys Ala Gln Leu Gly Leu Glu Leu Xaa Glu
                 85                  90                  95
Leu Glu Ala Ala Ala Leu Leu Val Gln Glu Glu Glu Thr Ala Leu Lys
            100                 105                 110
Ala Ala His Ser Val Ser Gly Gln Gln Thr Leu Cys Ser Ser Ser Glu
        115                 120                 125
Ala Ser Asp Ser Glu Asp Ser Tyr
    130                 135

<210> 540
<211> 102
<212> PRT
<213> Homo sapiens

<400> 540
Asp Glu Glu Asn Arg Gly Ser Tyr Thr Glu Gly Leu His Glu Asn Gly
  1               5                  10                  15
Val Cys Cys Ser Asp Pro Leu Ser Leu Leu Glu Ser Arg Met Glu Val
             20                  25                  30
Asp Asp Tyr Ser Gln Tyr Glu Glu Glu Ser Thr Asp Asp Ser Ser Ser
         35                  40                  45
Ser Glu Gly Asp Glu Glu Glu Asp Asp Tyr Asp Asp Asp Phe Glu Asp
     50                  55                  60
Asp Phe Ile Pro Leu Pro Pro Ala Lys Arg Leu Arg Leu Ile Val Gly
 65                  70                  75                  80
Lys Asp Ser Ile Asp Ile Asp Ile Ser Ser Arg Arg Arg Glu Asp Gln

85                  90                  95
Ser Leu Arg Leu Asn Ala
            100

<210> 541
<211> 155
<212> PRT
<213> Homo sapiens

<220>
<221> misc_feature
<222> (1)...(155)
<223> Xaa = any amino acid or nothing

<400> 541
Asp His Cys Ser Pro Thr Ser Asp Ser Ser Leu Ser Val Pro Gln Leu
 1               5                  10                  15
Pro His Leu Val Ser Glu Asp Leu Gly Xaa Gly Leu Arg Arg Arg Ser
            20                  25                  30
Pro Ser Phe Val Gln Ser Arg Thr Gly Trp Pro Met Ser Gln Glu Ser
        35                  40                  45
Leu Thr Phe Lys Asp Val Phe Val Asp Phe Thr Leu Glu Glu Trp Gln
    50                  55                  60
Gln Leu Asp Ser Ala Gln Lys Asn Leu Tyr Arg Asp Val Met Leu Glu
65                  70                  75                  80
Asn Tyr Ser His Leu Val Ser Val Gly Tyr Leu Val Ala Lys Pro Asp
                85                  90                  95
Val Ile Phe Arg Leu Gly Pro Gly Glu Glu Ser Trp Met Ala Asp Gly
            100                 105                 110
Gly Thr Pro Val Arg Thr Cys Ala Gly Glu Asp Arg Pro Asp Val Ser
        115                 120                 125
Ile Phe Ala Ser Leu Tyr Phe Glu Val Leu Leu Leu Glu Val Trp Gln
    130                 135                 140
Val Asp Glu Gln Ile Asp His Tyr Lys Glu Ser
145                 150                 155

<210> 542
<211> 143
<212> PRT
<213> Homo sapiens

<400> 542
Ile Gly Ala Cys Leu Ala Asp Val Asp Pro Met Gln Leu Asp Ser Ser
 1               5                  10                  15
Val Arg Phe Asp Ser Val Gly Gly Leu Ser Asn His Ile Ala Ala Leu
            20                  25                  30
Lys Glu Met Val Val Phe Pro Leu Leu Tyr Pro Glu Val Phe Glu Lys
        35                  40                  45
Phe Lys Ile Gln Pro Pro Arg Gly Cys Leu Phe Tyr Gly Pro Pro Gly
    50                  55                  60
Thr Gly Lys Thr Leu Val Ala Arg Ala Leu Ala Asn Glu Cys Ser Gln
65                  70                  75                  80
Gly Asp Lys Arg Val Ala Phe Phe Met Arg Lys Gly Ala Asp Cys Leu
                85                  90                  95

Ser Asn Ser Val Gly Glu Ser Gln Arg Gln Pro Arg Leu Leu Phe Asp
            100                 105                 110
Gln Ala Tyr Gln Arg Arg Gln Gln Phe Ile Ser Phe Asp Glu Ile Asp
        115                 120                 125
Gly Leu Ala Pro Val Arg Ser Arg Arg Gln Asp Gln Ile His Arg
    130                 135                 140

<210> 543
<211> 220
<212> PRT
<213> Homo sapiens

<400> 543
Gly Trp Leu Val Thr Ala Phe Cys Thr Ser Ser Ser Lys Glu Gly Gly
 1               5                  10                  15
Trp Ala Leu Phe Leu Phe Leu Leu Glu Glu Gly Leu Ala Gln Val Phe
            20                  25                  30
Ser Phe Ser Leu Pro His His Leu Thr Ala Ser Arg Pro Pro Leu Ser
        35                  40                  45
Pro Ser Pro Pro Ile Val Met Thr Pro Ser Pro Asp Tyr Leu Val Val
    50                  55                  60
Leu Phe Gly Ile Thr Ala Gly Pro Thr Gly Ala Lys Leu Gly Ser Asp
65                  70                  75                  80
Glu Lys Glu Leu Ile Leu Leu Phe Trp Lys Val Val Asp Leu Ala Asn
                85                  90                  95
Lys Lys Val Gly Gln Leu His Glu Val Leu Val Arg Pro Asp Gln Leu
            100                 105                 110
Glu Leu Thr Glu Asp Cys Lys Glu Glu Thr Lys Ile Asp Val Glu Ser
        115                 120                 125
Leu Ser Ser Ala Ser Gln Leu Asp Gln Ala Leu Arg Gln Phe Asn Gln
    130                 135                 140
Ser Val Ser Asn Glu Leu Asn Ile Gly Val Gly Thr Ser Phe Cys Leu
145                 150                 155                 160
Cys Thr Asp Gly Gln Leu His Val Arg Gln Ile Leu His Pro Glu Ala
                165                 170                 175
Ser Lys Lys Asn Val Leu Leu Pro Glu Cys Phe Tyr Ser Phe Phe Asp
            180                 185                 190
Leu Arg Lys Glu Phe Lys Lys Cys Cys Pro Gly Ser Pro Asp Ile Asp
        195                 200                 205
Lys Leu Asp Val Ala Pro Met Thr Glu Cys Ile Leu
    210                 215                 220

<210> 544
<211> 155
<212> PRT
<213> Homo sapiens

<220>
<221> misc_feature
<222> (1)...(155)
<223> Xaa = any amino acid or nothing

<400> 544
Ser Thr Lys Thr Gln Pro Leu Leu Arg Asn Ser Asp Lys Asp Ser Lys

Thr Asn Leu Leu Pro Thr Pro Thr His His Arg Pro Asn Thr Ser Gln
1               5                   10                  15
Gly Pro Xaa Met Lys Ala Ser Pro Pro His Pro Pro Pro Gln Ala Lys
            20                  25                  30
Asn His Ser Ser Val Glu Tyr Ser Pro Gly Thr Arg Ser Ile Pro Pro
        35                  40                  45
Pro Ser Phe Leu Pro Leu Phe Pro Xaa Gln Ser Gln His Cys Pro Pro
    50                  55                  60
Ala Ala Ser Leu Ala Leu Pro Lys Gln His Pro Pro Pro Gly Arg Gly
65                  70                  75                  80
Ser Cys Thr Val Leu Val Pro Pro Pro Glu Thr Tyr Phe Phe Ser Ser
                85                  90                  95
Ala Leu Leu Val Phe Arg Pro Pro Ser Leu Tyr Gly Gly Pro Phe Ile
            100                 105                 110
Leu Tyr Arg Glu Arg Lys Arg Lys Gly Arg Gly Gly Arg Asp Phe Leu
        115                 120                 125
Val Gln Tyr Asn Gln Lys Ile Phe Leu Lys Ser
    130                 135                 140
145                 150                 155

<210> 545
<211> 62
<212> PRT
<213> Homo sapiens

<400> 545
Ile Pro Ala Glu Val Val Lys Gln Arg Leu Gln Asp Val Gln Leu Arg
1               5                   10                  15
Ser Thr Gly Gln Gln Ser Gly Cys Ile Arg Thr Val Trp Arg Thr Glu
            20                  25                  30
Gly Leu Gly Ala Phe Tyr Arg Ser Tyr Thr Thr Gln Leu Asn Met Asn
        35                  40                  45
Ile Pro Phe Gln Ser Ile His Phe Ile Thr Tyr Glu Ser Cys
    50                  55                  60

<210> 546
<211> 95
<212> PRT
<213> Homo sapiens

<220>
<221> misc_feature
<222> (1)...(95)
<223> Xaa = any amino acid or nothing

<400> 546
Glu Arg Val Leu Leu Cys Leu Pro Gly Leu Gly Cys Gly Gly Val Ile
1               5                   10                  15
Leu Ala His Gly Ser Leu Ala Leu Pro Gly Ser Ser Ser Leu His Leu
            20                  25                  30
Ser Leu Gln Ser Ser Trp Gly Leu Thr Gly Met Xaa Tyr Asp Ala Arg
        35                  40                  45
Leu Ile Phe Val Tyr Phe Phe Arg Glu Met Gly Ser Cys His Ile Cys
    50                  55                  60

Gln Ala Gly Leu Asn Ser Tyr Asn Ser Ser Ile Pro Pro Thr Leu Val
65                  70                  75                  80
Ser Gln Asn Val Gly Thr Pro Gly Leu Lys Pro Pro Arg Pro Ser
                85                  90                  95

<210> 547
<211> 64
<212> PRT
<213> Homo sapiens

<400> 547
Thr Glu Thr Arg Pro Arg Ala Ala Leu Pro Ala Gly Leu Leu Gly Thr
 1               5                  10                  15
Ala Arg Gly Pro Gly Arg Gly Ala Asn Ser Ala Pro Ser Ala Pro Gly
            20                  25                  30
Gly Lys Ala Arg Pro Gly Lys Ala Gly Cys Gly Gly Cys Arg Lys Ser
        35                  40                  45
Glu Pro Pro Leu Arg Gly Arg Pro Leu Lys Gly Arg Pro Ser Ala Ala
    50                  55                  60

<210> 548
<211> 131
<212> PRT
<213> Homo sapiens

<220>
<221> misc_feature
<222> (1)...(131)
<223> Xaa = any amino acid or nothing

<400> 548
Met Ala Ala Met Cys Phe Trp Ala Leu Ile Pro Asn Gln Val Phe Arg
 1               5                  10                  15
Gly Phe Gln Met Thr Leu Lys Met Gln Tyr Leu Phe Leu Ser Phe Phe
            20                  25                  30
Pro Ser Phe Phe Leu Gly Thr Arg Val Gly Ala Arg Ser Pro Gly Trp
        35                  40                  45
Gly Cys Trp Glu Cys Ser Gly Ala Ile Ser Ala His Cys Ser Leu Cys
    50                  55                  60
Phe Pro Gly Ser Ser Val Ser Pro Ala Ser Ala Phe Arg Val Ala Gly
65                  70                  75                  80
Ile Thr Gly Met Tyr His Tyr Ala Trp Leu Ile Phe Ile Ser Ile Phe
                85                  90                  95
Leu Val Glu Met Gly Phe His His Val Cys Gln Ala Gly Leu Glu Leu
            100                 105                 110
Leu Ala Xaa Val Ile Arg Pro Pro Gln Pro Pro Lys Val Leu Gly Leu
        115                 120                 125
Gln Ala Arg
    130

<210> 549
<211> 61
<212> PRT
<213> Homo sapiens

<220>
<221> misc_feature
<222> (1)...(61)
<223> Xaa = any amino acid or nothing

<400> 549
Leu Gly Thr His Leu Thr Ser Ala Phe Leu Cys Val Phe Ser Lys Ser
1               5                   10                  15
Ile Ile Lys Lys Phe Trp Pro Gly Ala Val Ala His Ala Cys Asn Pro
            20                  25                  30
Ser Thr Leu Gly Gly Arg Arg Glu Xaa Ile Thr Xaa Gly Gln Asp Phe
        35                  40                  45
Glu Thr Ser Leu Ala Asn Met Ala Lys Leu Cys Leu Tyr
    50                  55                  60

<210> 550
<211> 132
<212> PRT
<213> Homo sapiens

<400> 550
His Ser Lys Phe Asn Val Phe Lys Leu Val Gly Ala Pro Ala Glu Asn
1               5                   10                  15
Thr Ser Ser Ser Ala Leu Leu Phe Phe His Thr Arg Leu Gly Pro Ala
            20                  25                  30
Ile Gln Glu Arg Met Lys Gly Gly Ala Pro Gly Gly Glu Gly Arg Ala
        35                  40                  45
Leu Ala Gly Arg Pro Ser Gln Pro Leu Thr Glu Ser Cys Pro Cys Leu
    50                  55                  60
Lys Met Tyr Phe Ile Leu Ala Ser Gly Ser Leu Pro Phe Leu Pro Phe
65                  70                  75                  80
Pro Leu Tyr Cys Leu Lys Asn Arg Phe Glu Ala Gln Val Arg Cys Leu
                85                  90                  95
Thr Ser Val Ile Pro Thr Leu Trp Glu Ala Glu Met Gly Gly Leu Leu
            100                 105                 110
Glu Pro Arg Lys Phe Lys Thr Ser Thr Gly Gln His Ser Glu Thr Pro
        115                 120                 125
Ser Leu Asn Lys
    130

<210> 551
<211> 213
<212> PRT
<213> Homo sapiens

<400> 551

<210> 552
<211> 47
<212> PRT
<213> Homo sapiens

<400> 552
Met Lys Lys Glu Lys Glu Glu Ala Asp Cys Trp Ser Val Lys Glu Ile
1               5                   10                  15
Leu Ala Gly Arg Gly Gly His Ala Cys Asn Pro Ser Thr Leu Gly Gly
            20                  25                  30
Arg Gly Gly Trp Ile Thr Arg Ser Gly Asp Arg Asp His Pro Gly
        35                  40                  45

<210> 553
<211> 115
<212> PRT
<213> Homo sapiens

<220>
<221> misc_feature
<222> (1)...(115)
<223> Xaa = any amino acid or nothing

<400> 553
Gln Gly Trp Pro Pro Pro Lys Pro Asp Arg Gln Ser Thr Gln Ala Cys
1               5                   10                  15
Gly Ala Cys Thr Gln Thr Leu Gln Arg Pro Trp Ser Leu Tyr Met Leu

20                  25                  30
Val Lys Pro Trp Pro Trp Arg Gly Asp Pro Gly Arg Leu Gly Pro Arg
        35                  40                  45
Leu Leu Pro Pro Pro Arg Lys Asp Cys Pro Arg Trp Phe Ser Gln Lys
    50                  55                  60
Ala Gly Ala Pro Gly Pro Pro Trp Ser Pro Gly Gly Ile Ile Pro Ala
65                  70                  75                  80
Ile Pro Ala Gln Thr Pro Xaa Arg Ser Xaa Glu Thr Ala Leu Pro Glu
                85                  90                  95
Ala Ser Arg Leu Cys Tyr Arg Ala Pro Gly Ser Thr Gly Gln Gly Pro
            100                 105                 110
Ile Thr Pro
        115

<210> 554
<211> 52
<212> PRT
<213> Homo sapiens

<400> 554
Ile Thr Asn Lys Cys Ser Phe Ser Ile Gly Ile Lys Asp Tyr Ser Asn
1               5                   10                  15
Trp Pro Thr Ile Pro Gln Val Tyr Leu Asn Gly Glu Phe Val Gly Gly
            20                  25                  30
Cys Asp Ile Leu Leu Gln Met His Gln Asn Gly Asp Leu Val Glu Glu
        35                  40                  45
Leu Lys Lys Leu
    50

<210> 555
<211> 194
<212> PRT
<213> Homo sapiens

<400> 555
Thr Leu Pro Gln Ala Gln Lys Leu Thr Asp Asp Gln Gly Pro Val Leu
1               5                   10                  15
Met Ser Thr Val Ala Met Pro Val Phe Ser Lys Gln Asn Glu Thr Arg
            20                  25                  30
Ser Lys Gly Ile Leu Leu Gly Val Val Gly Thr Asp Val Pro Val Lys
        35                  40                  45
Glu Leu Leu Lys Thr Ile Pro Lys Tyr Lys Leu Gly Ile His Gly Tyr
    50                  55                  60
Ala Phe Ala Ile Thr Asn Asn Gly Tyr Ile Leu Thr His Pro Glu Leu
65                  70                  75                  80
Arg Leu Leu Tyr Glu Glu Gly Lys Lys Arg Arg Lys Pro Asn Tyr Ser
                85                  90                  95
Ser Val Asp Leu Ser Glu Val Glu Trp Glu Asp Arg Asp Asp Val Leu
            100                 105                 110
Arg Asn Ala Met Val Asn Arg Lys Asp Thr Gly Lys Phe Ser Met Glu
        115                 120                 125
Val Lys Lys Thr Val Asp Lys Gly Lys Arg Val Leu Val Met Thr Asn
    130                 135                 140
Asp Tyr Tyr Tyr Thr Asp Ile Lys Gly Thr Pro Phe Ser Leu Gly Val

145                 150                 155                 160
Ala Leu Ser Arg Gly His Gly Lys Tyr Phe Phe Pro Arg Glu Cys Asn
                165                 170                 175
Pro Ser Glu Glu Gly Leu His Asp Leu Glu His Pro Asp Val Ser Phe
            180                 185                 190
Gly Arg

<210> 556
<211> 149
<212> PRT
<213> Homo sapiens

<220>
<221> misc_feature
<222> (1)...(149)
<223> Xaa = any amino acid or nothing

<400> 556
Val Thr Leu Arg Leu Leu Lys Arg Ile Arg Pro Ser Arg Pro Arg Arg
 1               5                  10                  15
Arg Asn Glu Val Thr Ser Leu Leu Arg Asp Ala Ala Asn Ile Thr Glu
            20                  25                  30
Met Asp Ser Glu Asn Ser Ala Lys Glu Gly Asp Pro Gly Thr Ile Phe
        35                  40                  45
Phe Phe Arg Glu Gly Ala Ala Val Phe Arg Asn Val Lys Asp Lys Thr
    50                  55                  60
Met Lys His Val Met Asn Val Leu Gln Lys His Glu Ile Gln Pro Tyr
65                  70                  75                  80
Glu Ile Ala Leu Val His Trp Glu Asn Glu Glu Leu Asn Tyr Ile Gln
                85                  90                  95
Ile Gln Gly Gln Ser Lys Leu His Arg Gly Glu Ile Lys Leu Asn Ser
            100                 105                 110
Xaa Leu Asp Leu His Asp Ala Ile Leu Glu Lys Phe Ala Phe Ser Asn
        115                 120                 125
Ala Leu Cys Leu Ser Val Lys Leu Ala Ile Trp Glu Ala Ser Leu Asp
    130                 135                 140
Lys Phe Ile Glu Ser
145

<210> 557
<211> 155
<212> PRT
<213> Homo sapiens

<400> 557
Pro Thr Arg Pro Glu Lys Leu Ile Ser Gln Ile Gln Pro Glu Val Asp
 1               5                  10                  15
Arg Glu Arg Ala Val Val Glu Val Asn Ile Pro Val Glu Ser Glu Val
            20                  25                  30
Asn Leu Lys Leu Gln Asn Ile Met Met Leu Leu Arg Lys Cys Cys Asn
        35                  40                  45
His Pro Tyr Leu Ile Glu Tyr Pro Ile Asp Pro Val Thr Gln Glu Phe
    50                  55                  60

Lys Ile Asp Glu Glu Leu Val Thr Asn Ser Gly Lys Phe Leu Ile Leu
65                  70                  75                  80
Asp Arg Met Leu Pro Glu Leu Lys Lys Arg Gly His Lys Val Leu Leu
                85                  90                  95
Phe Ser Gln Met Thr Ser Met Leu Asp Ile Leu Met Asp Tyr Cys His
            100                 105                 110
Leu Arg Asp Phe Asn Phe Ser Arg Leu Asp Gly Ser Met Ser Tyr Ser
        115                 120                 125
Glu Arg Glu Lys Asn Met His Ser Phe Asn Thr Asp Pro Glu Val Phe
    130                 135                 140
Ile Phe Leu Val Ser Thr Arg Ala Gly Gly Leu
145                 150                 155

<210> 558
<211> 124
<212> PRT
<213> Homo sapiens

<220>
<221> misc_feature
<222> (1)...(124)
<223> Xaa = any amino acid or nothing

<400> 558
Leu Ile Gly Leu Ile Gln Met Tyr Leu Pro Ser Ser Tyr Gln Glu Asn
 1               5                  10                  15
Asp Cys Glu Gly Asn Asp Gly Ser Arg Arg Pro Ala Glu Asn Ser Leu
            20                  25                  30
Gly Glu Ser His Arg Lys Cys Thr Leu Gln Lys Arg Asn Arg Asn Gln
        35                  40                  45
Arg Thr Gln Lys Arg Lys Ile Tyr Asn Val Cys Pro Arg Lys Gly Lys
    50                  55                  60
Lys Ile Phe Ile His Met His Glu Ile Ile Gln Ile Asp Gly His Ile
65                  70                  75                  80
Tyr Gln Cys Leu Glu Cys Lys Gln Asn Phe Cys Glu Asn Leu Arg Ser
                85                  90                  95
Tyr Tyr Val Val Glu Arg Thr His Thr Gly Glu Lys Pro Tyr Xaa Cys
            100                 105                 110
Ile Gln Gly Thr Gly Ile Tyr Asp His Leu Ser Glu
        115                 120

<210> 559
<211> 483
<212> PRT
<213> Homo sapiens

<400> 559
Leu Val Ser Glu His Ala Phe Glu Ile Pro Asp Asn Val Arg Pro Gly
 1               5                  10                  15
His Leu Ile Lys Glu Leu Ser Lys Val Ile Arg Ala Ile Glu Glu Glu
            20                  25                  30
Asn Gly Lys Pro Val Lys Ser Gln Gly Ile Pro Ile Val Cys Pro Val
        35                  40                  45
Ser Arg Ser Ser Asn Glu Ala Thr Ser Pro Tyr His Ser Arg Arg Lys

50                  55                  60
Met Arg Lys Leu Arg Asp His Asn Val Arg Thr Pro Ser Asn Leu Asp
65                  70                  75                  80
Ile Leu Glu Leu His Thr Arg Glu Asp Ile Leu Ser Ser Lys Leu Asn
                85                  90                  95
Gly Lys Phe Asn Lys His Leu Gln Pro Ser Ser Thr Val Pro Glu Trp
            100                 105                 110
Arg Ala Lys Asp Asn Asp Leu Arg Leu Leu Leu Thr Asn Gly Arg Ile
        115                 120                 125
Ile Lys Asp Glu Arg Gln Pro Phe Ala Asp Gln Ser Leu Tyr Thr Ala
    130                 135                 140
Asp Ser Glu Asn Glu Glu Asp Lys Arg Arg Thr Lys Lys Ala Lys Met
145                 150                 155                 160
Lys Ile Glu Glu Ser Ser Gly Val Glu Gly Val Glu His Glu Glu Ser
                165                 170                 175
Gln Lys Pro Leu Asn Gly Phe Phe Thr Arg Val Lys Ser Glu Leu Arg
            180                 185                 190
Ser Arg Ser Ser Gly Tyr Ser Asp Ile Ser Glu Ser Glu Asp Ser Gly
        195                 200                 205
Pro Glu Cys Thr Ala Leu Lys Ser Ile Phe Thr Thr Glu Glu Ser Glu
    210                 215                 220
Ser Ser Gly Asp Glu Lys Lys Gln Glu Ile Thr Ser Asn Phe Lys Glu
225                 230                 235                 240
Glu Ser Asn Val Met Arg Asn Phe Leu Gln Lys Ser Gln Lys Pro Ser
                245                 250                 255
Arg Ser Glu Ile Pro Ile Lys Arg Glu Cys Pro Thr Ser Thr Ser Thr
            260                 265                 270
Glu Glu Glu Ala Ile Gln Gly Met Leu Ser Met Ala Gly Leu His Tyr
        275                 280                 285
Ser Thr Cys Leu Gln Arg Gln Ile Gln Ser Thr Asp Cys Ser Gly Glu
    290                 295                 300
Arg Asn Ser Leu Gln Asp Pro Ser Ser Cys His Gly Ser Asn His Glu
305                 310                 315                 320
Val Arg Gln Leu Tyr Arg Tyr Asp Lys Pro Val Glu Cys Gly Tyr His
                325                 330                 335
Val Lys Thr Glu Asp Pro Asp Leu Arg Thr Ser Ser Trp Ile Lys Gln
            340                 345                 350
Phe Asp Thr Ser Arg Phe His Pro Gln Asp Leu Ser Arg Ser Gln Lys
        355                 360                 365
Cys Ile Arg Lys Glu Gly Ser Ser Glu Ile Ser Gln Arg Val Gln Ser
    370                 375                 380
Arg Asn Tyr Val Asp Ser Ser Gly Ser Ser Leu Gln Asn Gly Lys Tyr
385                 390                 395                 400
Met Gln Asn Ser Asn Leu Thr Ser Gly Ala Cys Gln Ile Ser Asn Gly
                405                 410                 415
Ser Leu Ser Pro Glu Arg Pro Val Gly Glu Thr Ser Phe Ser Val Pro
            420                 425                 430
Leu His Pro Thr Lys Arg Pro Ala Ser Asn Pro Pro Pro Ile Ser Asn
        435                 440                 445
Gln Ala Thr Lys Gly Lys Arg Pro Lys Lys Gly Met Ala Thr Ala Lys
    450                 455                 460
Gln Arg Leu Gly Lys Ile Leu Lys Leu Asn Arg Asn Gly His Ala Arg
465                 470                 475                 480
Phe Phe Val

<210> 560
<211> 98

<212> PRT
<213> Homo sapiens

<220>
<221> misc_feature
<222> (1)...(98)
<223> Xaa = any amino acid or nothing

<400> 560
Xaa Ile Phe Ala Ala Lys Glu Leu Glu Asn Glu Glu Asn Gln Glu Glu
1               5                   10                  15
Gln Gly Leu Glu Glu Lys Gly Glu Glu Phe Ala Arg Met Leu Thr Glu
            20                  25                  30
Leu Leu Phe Glu Leu His Val Ala Ala Thr Pro Asp Lys Leu Asn Lys
        35                  40                  45
Ala Met Lys Arg Ala His Asp Trp Val Glu Glu Asp Gln Thr Val Val
    50                  55                  60
Ser Val Asp Val Ala Lys Ser Val Arg Arg Arg Asn Lys Glu Gly Arg
65                  70                  75                  80
Lys Gly Arg Glu Ile Ser Arg Pro Ser Arg Arg Gln Lys Gly Gly Lys
                85                  90                  95
Glu Asn

<210> 561
<211> 81
<212> PRT
<213> Homo sapiens

<400> 561
Ala His Val Asp Thr Met Ser Lys Ala His Pro Pro Glu Leu Lys Lys
1               5                   10                  15
Phe Ile Asp Lys Lys Leu Ser Leu Lys Leu Asn Gly Gly Arg His Val
            20                  25                  30
Gln Gly Ile Leu Trp Gly Phe Asp Pro Leu Met Asn Leu Val Ile Asp
        35                  40                  45
Lys Cys Val Glu Met Ala Thr Ser Gly Gln Gln Lys Asn Ile Gly Met
    50                  55                  60
Val Val Ile Arg Arg Asn Ser Ile Ile Met Leu Glu Thr Leu Glu Gln
65                  70                  75                  80
Glu

<210> 562
<211> 272
<212> PRT
<213> Homo sapiens

<400> 562

<210> 563
<211> 128
<212> PRT
<213> Homo sapiens

<400> 563
Gly Lys Asn Tyr Gln Asn Pro Lys Ser Asn Ala Val Ser Leu Leu Ser
1               5                   10                  15
His Leu Trp Leu Ala Asn Thr Ala Ala Ala Leu Arg Ile Asn Thr Asp
            20                  25                  30
Leu Leu Pro Thr Ser Tyr Gln Leu Val Lys Phe Glu Asp Ile Val His
        35                  40                  45
Phe Pro Gln Lys Thr Thr Glu Arg Ile Phe Ala Phe Leu Gly Ile Pro
    50                  55                  60
Leu Ser Pro Ala Ser Leu Asn Gln Ile Leu Phe Ala Thr Ser Thr Asn
65                  70                  75                  80
Leu Phe Tyr Leu Pro Tyr Glu Gly Glu Ile Ser Pro Thr Asn Thr Asn
                85                  90                  95
Val Trp Lys Gln Asn Leu Pro Arg Asp Glu Ile Lys Leu Ile Glu Asn
            100                 105                 110
Ile Cys Trp Thr Leu Met Asp Arg Leu Gly Tyr Pro Lys Phe Met Asp
        115                 120                 125

<210> 564
<211> 116
<212> PRT
<213> Homo sapiens

<400> 564
Val Leu Glu Glu Thr Tyr Glu Cys Ile Arg Gly Asp Lys Gly Phe Gln
1               5                   10                  15
Phe Phe Ser Gln Gln Val Ser His His Pro Pro Ile Ser Ala Cys His
            20                  25                  30
Ala Glu Ser Ile Asn Val Gly Tyr Trp Leu Asp Ala Arg Trp Thr Ser
        35                  40                  45
Leu Leu Trp Gly Lys Ser Met Gln Ile Val Pro Ile Gly Thr Asn His
    50                  55                  60
Val Thr Leu Pro Leu Phe Gly Asp His Ile Glu Trp Asn Lys Leu Thr
65                  70                  75                  80
Ser Cys Ile His Ile Ile Leu Ser Val Gln Lys Trp Ile Glu His Tyr
                85                  90                  95
Gly Glu Ile Ala Ile Lys Asn Leu Leu Asp Asp Cys Cys Tyr Cys His
            100                 105                 110
Thr Asn Phe Ile
        115

<210> 565
<211> 144
<212> PRT
<213> Homo sapiens

<400> 565
Ile Asn Thr Leu Leu Leu Ser Gly Gly Lys Gly Gly Lys Asn Lys Lys
1               5                   10                  15
Asn Lys Asn Ser Ser Lys Pro Gln Lys Asn Asn Gly Ser Thr Trp Ala
            20                  25                  30
Asn Val Pro Leu Pro Pro Pro Pro Val Gln Pro Leu Pro Gly Thr Glu
        35                  40                  45
Leu Glu His Tyr Ala Val Glu Gln Gln Glu Asn Gly Tyr Asp Ser Asp
    50                  55                  60
Ser Trp Cys Pro Pro Leu Pro Val Gln Thr Tyr Leu His Gln Gly Leu
65                  70                  75                  80
Glu Asp Glu Leu Glu Glu Asp Asp Asp Arg Val Pro Thr Pro Pro Val
                85                  90                  95
Arg Gly Val Ala Ser Ser Pro Ala Ile Ser Phe Gly Gln Gln Ser Thr
            100                 105                 110
Ala Thr Leu Thr Pro Phe Pro Thr Gly Arg Arg Trp Gln Pro Met Ala
        115                 120                 125
Gly Arg Leu His Leu Gly Trp Ser Leu Thr Arg Arg Pro Ile Gln Phe
    130                 135                 140

<210> 566

<211> 897
<212> PRT
<213> Homo sapiens

<400> 566
Leu Asp Leu Cys Arg Gln Ser Asn Asn Leu Cys Leu Gln Arg Glu Glu
1               5                   10                  15
Asp Leu Gln Arg Thr Arg Asp Tyr His Asp Cys Met Asn Val Val Glu
            20                  25                  30
Val Phe Leu Glu Lys Phe Thr Thr Glu Trp Asp Asn Leu Ala Arg Ser
        35                  40                  45
Asp Ala Glu Ser Thr Ala Val His Leu Glu Ala Leu Lys Lys Leu Ala
    50                  55                  60
Leu Ala Leu Gln Glu Arg Lys Tyr Ala Ile Glu Asp Leu Lys Asp Gln
65                  70                  75                  80
Lys Gln Lys Met Ile Glu His Leu Asn Leu Asp Asp Lys Glu Leu Val
                85                  90                  95
Lys Glu Gln Thr Ser His Leu Glu Gln Arg Trp Phe Gln Leu Glu Asp
            100                 105                 110
Leu Ile Lys Arg Lys Ile Gln Val Ser Val Thr Asn Leu Glu Glu Leu
        115                 120                 125
Asn Val Val Gln Ser Arg Phe Gln Glu Leu Met Glu Trp Ala Glu Glu
    130                 135                 140
Gln Gln Pro Asn Ile Ala Glu Ala Leu Lys Gln Ser Pro Pro Pro Asp
145                 150                 155                 160
Met Ala Gln Asn Leu Leu Met Asp His Leu Ala Ile Cys Ser Glu Leu
                165                 170                 175
Glu Ala Lys Gln Met Leu Leu Lys Ser Leu Ile Lys Asp Ala Asp Arg
            180                 185                 190
Val Met Ala Asp Leu Gly Leu Asn Glu Arg Gln Val Ile Gln Lys Ala
        195                 200                 205
Leu Ser Asp Ala Gln Ser His Val Asn Cys Leu Ser Asp Leu Val Gly
    210                 215                 220
Gln Arg Arg Lys Tyr Leu Asn Lys Ala Leu Ser Glu Lys Thr Gln Phe
225                 230                 235                 240
Leu Met Ala Val Phe Gln Ala Thr Ser Gln Ile Gln Gln His Glu Arg
                245                 250                 255
Lys Ile Met Phe Arg Glu His Ile Cys Leu Leu Pro Asp Asp Val Ser
            260                 265                 270
Lys Gln Val Lys Thr Cys Lys Ser Ala Gln Ala Ser Leu Lys Thr Tyr
        275                 280                 285
Gln Asn Glu Val Thr Gly Leu Trp Ala Gln Gly Arg Glu Leu Met Lys
    290                 295                 300
Glu Val Thr Glu Gln Glu Lys Ser Glu Val Leu Gly Lys Leu Gln Glu
305                 310                 315                 320
Leu Gln Ser Val Tyr Asp Ser Val Leu Gln Lys Cys Ser His Arg Leu
                325                 330                 335
Gln Glu Leu Glu Lys Asn Leu Val Ser Arg Lys His Phe Lys Glu Asp
            340                 345                 350
Phe Asp Lys Ala Cys His Trp Leu Lys Gln Ala Asp Ile Val Thr Phe
        355                 360                 365
Pro Glu Ile Asn Leu Met Asn Glu Ser Thr Glu Leu His Thr Gln Leu
    370                 375                 380
Ala Lys Tyr Gln Asn Ile Leu Glu Gln Ser Pro Glu Tyr Glu Asn Leu
385                 390                 395                 400
Leu Leu Thr Leu Gln Arg Thr Gly Gln Thr Ile Leu Pro Ser Leu Asn
                405                 410                 415
Glu Val Asp His Ser Tyr Leu Ser Glu Lys Leu Asn Ala Leu Pro Arg
            420                 425                 430

Gln Phe Asn Val Ile Val Ala Leu Ala Lys Asp Lys Phe Tyr Lys Val
        435                 440                 445
Gln Glu Ala Ile Leu Ala Arg Lys Glu Tyr Ala Ser Leu Ile Glu Leu
    450                 455                 460
Thr Thr Gln Ser Leu Ser Glu Leu Glu Ala Gln Phe Leu Arg Met Ser
465                 470                 475                 480
Lys Val Pro Thr Asp Leu Ala Val Glu Glu Ala Leu Ser Leu Gln Asp
                485                 490                 495
Gly Cys Arg Ala Ile Leu Asp Glu Val Ala Gly Leu Gly Glu Ala Val
            500                 505                 510
Asp Glu Leu Asn Gln Lys Lys Glu Gly Phe Arg Ser Thr Gly Gln Pro
        515                 520                 525
Trp Gln Pro Asp Lys Met Leu His Leu Val Thr Leu Tyr His Arg Leu
    530                 535                 540
Lys Arg Gln Thr Glu Gln Arg Val Ser Leu Leu Glu Asp Thr Thr Ser
545                 550                 555                 560
Ala Tyr Gln Glu His Glu Lys Met Cys Gln Gln Leu Glu Arg Gln Leu
                565                 570                 575
Lys Ser Val Lys Glu Glu Gln Ser Lys Val Asn Glu Glu Thr Leu Pro
            580                 585                 590
Ala Glu Glu Lys Leu Lys Met Tyr His Ser Leu Ala Gly Ser Leu Gln
        595                 600                 605
Asp Ser Gly Ile Val Leu Lys Arg Val Thr Ile His Leu Glu Asp Leu
    610                 615                 620
Ala Pro His Leu Asp Pro Leu Ala Tyr Glu Lys Ala Arg His Gln Ile
625                 630                 635                 640
Gln Ser Trp Gln Gly Glu Leu Lys Leu Leu Thr Ser Ala Ile Gly Glu
                645                 650                 655
Thr Val Thr Glu Cys Glu Ser Arg Met Val Gln Ser Ile Asp Phe Gln
            660                 665                 670
Thr Glu Met Ser Arg Ser Leu Asp Trp Leu Arg Arg Val Lys Ala Glu
        675                 680                 685
Leu Ser Gly Pro Val Tyr Leu Asp Leu Asn Leu Gln Asp Ile Gln Glu
    690                 695                 700
Glu Ile Arg Lys Ile Gln Ile His Gln Glu Glu Val Gln Ser Ser Leu
705                 710                 715                 720
Arg Ile Met Asn Ala Leu Ser His Lys Glu Lys Glu Lys Phe Thr Lys
                725                 730                 735
Ala Lys Glu Leu Ile Ser Ala Asp Leu Glu His Ser Leu Ala Glu Leu
            740                 745                 750
Ser Glu Leu Asp Gly Asp Ile Gln Glu Ala Leu Arg Thr Arg Gln Ala
        755                 760                 765
Thr Leu Thr Glu Ile Tyr Ser Gln Cys Gln Arg Tyr Tyr Gln Val Phe
    770                 775                 780
Gln Ala Ala Asn Asp Trp Leu Glu Asp Ala Gln Glu Met Leu Gln Leu
785                 790                 795                 800
Ala Gly Asn Gly Leu Asp Val Glu Ser Ala Glu Glu Asn Leu Lys Ser
                805                 810                 815
His Met Glu Phe Phe Ser Thr Glu Asp Gln Phe His Ser Asn Leu Glu
            820                 825                 830
Glu Leu His Ser Leu Val Ala Thr Leu Asp Pro Leu Ile Lys Pro Thr
        835                 840                 845
Gly Lys Glu Asp Leu Glu Gln Lys Val Ala Ser Leu Glu Leu Arg Ser
    850                 855                 860
Gln Arg Met Ser Arg Asp Ser Gly Ala Gln Val Asp Leu Leu Gln Arg
865                 870                 875                 880
Cys Thr Ala Gln Trp His Asp Tyr Gln Lys Ala Arg Glu Glu Val Ile
                885                 890                 895
Asp

<210> 567
<211> 363
<212> PRT
<213> Homo sapiens

<400> 567
Thr Asp Thr Glu Ser Tyr Pro Glu Phe Leu Gln Arg Gln His Ser Ser
1               5                   10                  15
Pro Trp Gly Pro Pro Gln Ala Leu Asp Ser Gly Thr Ala Trp Leu Arg
            20                  25                  30
Cys His Gly Pro Asp Arg Ser Cys Ser His Gly Ala Gly Asp Gly Gln
        35                  40                  45
Ile Ala Leu Leu Pro Leu Asp Arg Lys Pro Ala Lys Pro Leu Trp Phe
    50                  55                  60
Arg Arg Ala Leu Cys Ser Pro Arg Lys His Phe Asp Gly Glu Leu Gly
65                  70                  75                  80
Asp Ser Thr His Pro Ala Pro Asn Trp Ala Gly Phe Asn Phe Thr Leu
                85                  90                  95
Leu Leu Thr Trp Ala Glu Pro Gln Ala Gly Gly Gly Glu Asp Ala Leu
            100                 105                 110
Pro Leu Arg Ile Leu Lys Arg Leu Asp Asn Ala Asp Glu Gln Ala Ala
        115                 120                 125
Gln Ile Arg Arg Glu Leu Asp Gly Arg Leu Gln Leu Ala Asp Lys Met
    130                 135                 140
Ala Lys Lys Tyr Val Asn Val Ile Leu Glu Glu Lys Lys Ser Arg Trp
145                 150                 155                 160
Pro Glu Thr Leu Gly Leu Ile Cys Glu Val Val Pro Asp Val Pro Ser
                165                 170                 175
Asn Pro Met Asn Glu Leu Val Gln Glu Arg Lys Phe Pro Lys Phe Ile
            180                 185                 190
Ala Lys Asp Met Glu Asn Met Tyr Ile Glu Glu Leu Arg Ser Ser Val
        195                 200                 205
Asn Leu Leu Met Ala Asn Leu Glu Ser Leu Pro Val Ser Lys Gly Gly
    210                 215                 220
Pro Glu Phe Lys Leu Gln Lys Leu Lys Arg Ser Gln Asn Ser Ala Val
225                 230                 235                 240
Leu Asp Ile Gly Asp Glu Asn Glu Ile Gln Leu Ser Lys Ser Asp Val
                245                 250                 255
Val Leu Ser Phe Thr Leu Glu Ile Val Ile Met Glu Val Gln Gly Leu
            260                 265                 270
Lys Ser Val Ala Pro Asn Arg Ile Val Tyr Cys Thr Met Glu Val Glu
        275                 280                 285
Gly Glu Lys Leu Gln Thr Asp His Ala Glu Ala Ser Arg Pro Gln Trp
    290                 295                 300
Gly Thr Gln Gly Asp Phe Thr Thr Thr His Pro Arg Ala Val Val Lys
305                 310                 315                 320
Val Lys Leu Phe Thr Glu Ser Thr Gly Val Leu Ala Leu Glu Asp Lys
                325                 330                 335
Glu Leu Val Arg Val Ile Leu Tyr Pro Thr Ser Asn Ser Ser Lys Ser
            340                 345                 350
Ala Glu Leu His Arg Met Val Val Pro Lys Asn
        355                 360

<210> 568

<211> 96
<212> PRT
<213> Homo sapiens

<220>
<221> misc_feature
<222> (1)...(96)
<223> Xaa = any amino acid or nothing

<400> 568
Ala Glu Arg Arg Gly Ala Val Ser Pro Arg Ser Arg Asp Gly Gly Gly
1               5                   10                  15
Val Gly Gly Pro Cys Ala Met Ala Thr Ser Val Leu Cys Cys Leu Arg
            20                  25                  30
Cys Cys Arg Asp Gly Gly Thr Gly His Ile Pro Leu Met Glu Met Pro
        35                  40                  45
Ala Val His Leu Leu Thr Gln Leu Met Gly Ser Asp Val Cys Ile Val
    50                  55                  60
Pro Ser Cys Leu Pro Leu Cys Gly Ile Xaa Val Trp Leu Leu Leu Cys
65                  70                  75                  80
Ala Ile Pro Arg Cys Leu Ile Phe Phe Thr Phe Trp Trp Gly Val Gly
                85                  90                  95

<210> 569
<211> 110
<212> PRT
<213> Homo sapiens

<220>
<221> misc_feature
<222> (1)...(110)
<223> Xaa = any amino acid or nothing

<400> 569
Tyr Gln Arg Cys Asn Arg Lys Ala Thr Cys Ala Pro Arg Arg Leu Arg
1               5                   10                  15
Ala Phe Ser Leu Ala Ser Arg Ala Pro Arg Arg Gly Thr His Arg Thr
            20                  25                  30
Pro Gly Arg Asp Ser Ser Pro Leu Arg Xaa Leu Arg Thr Arg Arg Pro
        35                  40                  45
Pro Pro Pro Ser Pro Pro Arg Asn Lys Pro Arg Gln Arg Ala Val Gly
    50                  55                  60
Arg Gly Gln Ser Pro Cys Arg Asp Gln Ser Glu Pro Arg Arg Leu Arg
65                  70                  75                  80
Ser Leu Tyr His Gln Val Pro Ser Val Val Arg Ala Ala Pro Lys Lys
                85                  90                  95
Met Ser Pro Glu Ala Val Pro Leu Gly Ala Ser Leu Pro Pro
            100                 105                 110

<210> 570
<211> 234
<212> PRT

<213> Homo sapiens

<220>
<221> misc_feature
<222> (1)...(234)
<223> Xaa = any amino acid or nothing

<400> 570
Arg Asp Ala Leu Gly Ala Gly Cys Arg Ile Leu Leu Ile Cys Glu Met
1               5                   10                  15
Gln Leu Thr His Gln Leu Asp Leu Phe Pro Glu Cys Arg Val Thr Leu
            20                  25                  30
Leu Leu Phe Lys Asp Val Lys Asn Ala Gly Asp Leu Arg Arg Lys Ala
        35                  40                  45
Met Glu Gly Thr Ile Asp Gly Ser Leu Ile Asn Pro Thr Val Phe His
    50                  55                  60
Ser Cys Cys Pro Gly Trp Ser Ala Met Ala Arg Ser Trp Leu Thr Ala
65                  70                  75                  80
Thr Ser Ala Ser Arg Val Gln Ala Val Val Leu Pro Gln Pro Pro Glu
                85                  90                  95
Leu Leu Gly Leu Gln Ile Val Asp Pro Phe Gln Ile Leu Val Ala Ala
            100                 105                 110
Asn Lys Ala Val His Leu Tyr Lys Leu Gly Lys Asn Gly Arg Gln Xaa
        115                 120                 125
Thr Leu Ser Thr Glu Ile Tyr Phe Gln Pro Phe Pro Pro Asn Asn Gln
    130                 135                 140
Tyr Phe Thr Arg Leu Xaa Lys Asn Phe Gly Ile Ser Ala Asn Xaa His
145                 150                 155                 160
Phe Gln Phe Leu Ile Val Tyr Ile Glu Glu Gly Glu Lys Gln Ile Asn
                165                 170                 175
Gln Glu Tyr Leu Ile Ser Gln Val Glu Gly His Gln Val Ser Leu Lys
            180                 185                 190
Asn Pro Pro Glu Ile Met Asn Lys Thr Glu Met Ser Thr Lys Ile Tyr
        195                 200                 205
Lys Leu Ser Ser Gln Glu Glu Ser Ile Gly Thr Leu Leu Asp Ala Ile
    210                 215                 220
Ile Cys Arg Met Ser Thr Lys Asp Val Leu
225                 230

<210> 571
<211> 194
<212> PRT
<213> Homo sapiens

<400> 571
Thr Gly Thr Val Ala Ala Lys Ala Met Asp Thr Phe Ser Thr Lys Ser
1               5                   10                  15
Leu Ala Leu Gln Ala Gln Lys Lys Leu Leu Ser Lys Met Ala Ser Lys
            20                  25                  30
Ala Val Val Ala Val Leu Val Asp Asp Thr Ser Ser Glu Val Leu Asp
        35                  40                  45
Glu Leu Tyr Arg Ala Thr Arg Glu Phe Thr Arg Ser Arg Lys Glu Ala
    50                  55                  60
Gln Lys Met Leu Lys Asn Leu Val Lys Val Ala Leu Lys Leu Gly Leu
65                  70                  75                  80
Leu Leu Arg Gly Asp Gln Leu Gly Gly Glu Glu Leu Ala Leu Leu Arg

85                  90                  95
Arg Phe Arg His Arg Ala Arg Cys Leu Ala Met Thr Ala Val Ser Phe
            100                 105                 110
His Gln Val Asp Phe Thr Phe Asp Arg Arg Val Leu Ala Ala Gly Leu
        115                 120                 125
Leu Glu Cys Arg Asp Leu Leu His Gln Ala Val Gly Pro Ser Leu Ile
    130                 135                 140
Ser Lys Ser His Gly Arg Ile Asn His Val Phe Gly His Leu Ala Asp
145                 150                 155                 160
Cys Asp Phe Leu Ala Ala Leu Tyr Gly Pro Ala Glu Pro Tyr Arg Ser
                165                 170                 175
His Leu Arg Arg Ile Cys Glu Gly Leu Gly Arg Met Leu Asp Glu Gly
            180                 185                 190
Ser Leu

<210> 572
<211> 52
<212> PRT
<213> Homo sapiens

<220>
<221> misc_feature
<222> (1)...(52)
<223> Xaa = any amino acid or nothing

<400> 572
Ser Gly Ile Gly Asp Phe Asp Ile Asn Lys Asp Ala Ala Ala Ala Ala
 1               5                  10                  15
Ser Arg Gly Val Gly Ala Lys Leu Gly Pro Xaa Arg Ala Val Ala Gly
            20                  25                  30
Glu His Thr His Asp Pro Gly Ala Gly Pro Leu Pro Asp Ala Phe Gln
        35                  40                  45
Gly Leu Gln Pro
    50

<210> 573
<211> 43
<212> PRT
<213> Homo sapiens

<400> 573
Ser Gly Ser Ser Trp Arg Pro Gly Ser Ser Pro Lys Thr Gly Ala Gln
 1               5                  10                  15
Ile Arg Glu Ala Arg Arg Ala Pro Ala Gly Ala Gln Arg Ile Gly Gly
            20                  25                  30
Arg Arg Ser Leu Glu His Ser Leu His Ala Pro
        35                  40

<210> 574
<211> 152
<212> PRT

<213> Homo sapiens

<220>
<221> misc_feature
<222> (1)...(152)
<223> Xaa = any amino acid or nothing

<400> 574
Ala Arg Ala His Thr His Arg Glu Pro Thr Met Val Leu Ser Pro Ala
1               5                   10                  15
Asp Lys Thr Asn Val Lys Ala Ala Trp Gly Lys Val Gly Ala His Ala
            20                  25                  30
Gly Glu Tyr Gly Ala Glu Ala Leu Glu Arg Met Leu Leu Ser Phe Pro
        35                  40                  45
Thr Thr Pro Thr Tyr Phe Pro His Phe Asp Leu Asn His Gly Ser Ala
    50                  55                  60
His Val Lys Gly His Gly Lys Asn Val Asp Asp Ala Leu Thr Asn Ala
65                  70                  75                  80
Val Thr His Val Tyr Tyr Met Pro Asn Ser Leu Tyr Ala Leu Ser Asp
                85                  90                  95
Leu His Pro His Asn Leu Arg Met Asp Pro Val Asn Phe Met Leu Leu
            100                 105                 110
Ser His Cys Leu Leu Xaa Thr Leu Val Val His Leu Pro Ala Glu Leu
        115                 120                 125
Thr Pro Ala Val His Ala Ser Leu Asn Asn Val Leu Glu Ser Glu Arg
    130                 135                 140
Thr Glu Leu Thr Ser Ser Thr Ser
145                 150

<210> 575
<211> 152
<212> PRT
<213> Homo sapiens

<220>
<221> misc_feature
<222> (1)...(152)
<223> Xaa = any amino acid or nothing

<400> 575
Ala Arg Ala His Thr His Arg Glu Pro Thr Met Val Leu Ser Pro Ala
1               5                   10                  15
Asp Lys Thr Asn Val Lys Ala Ala Trp Gly Lys Val Gly Ala His Ala
            20                  25                  30
Gly Glu Tyr Gly Ala Glu Ala Leu Glu Arg Met Leu Leu Ser Phe Pro
        35                  40                  45
Thr Thr Pro Thr Tyr Phe Pro His Phe Asp Leu Asn His Gly Ser Ala
    50                  55                  60
His Val Lys Gly His Gly Lys Asn Val Asp Asp Ala Leu Thr Asn Ala
65                  70                  75                  80
Val Thr His Val Tyr Tyr Met Pro Asn Ser Leu Tyr Ala Leu Ser Asp
                85                  90                  95
Leu His Pro His Asn Leu Arg Met Asp Pro Val Asn Phe Met Leu Leu
            100                 105                 110
Ser His Cys Leu Leu Xaa Thr Leu Val Val His Leu Pro Ala Glu Leu
        115                 120                 125

Thr Pro Ala Val His Ala Ser Leu Asn Asn Val Leu Glu Ser Glu Arg
    130                 135                 140
Thr Glu Leu Thr Ser Ser Thr Ser
145                 150

<210> 576
<211> 109
<212> PRT
<213> Homo sapiens

<400> 576
Lys Leu Pro Pro Ser Pro Trp Pro Leu Ala Cys Ser Ala Ala Glu Gln
  1               5                  10                  15
Val Thr Leu Gly Arg Asn Thr Val Ser Ser Glu Lys Leu Ala Gly Gly
             20                  25                  30
Arg Val His Leu Ser Pro Tyr Cys Asp Pro Trp Ile Pro His Cys Ala
         35                  40                  45
Thr Pro Glu Gln Ala Gly Ala Pro Gln Asp Lys Asp Asp Leu Leu Met
    50                  55                  60
Ala Ser Trp Pro Val Gly Ile Cys Leu Pro Thr Thr Val Ala Pro Arg
 65                  70                  75                  80
Ser Pro Lys Gln Glu Phe Pro Cys Phe Lys Val Thr Ala Lys Ile Asn
                 85                  90                  95
Arg Arg Ile Lys His Val Phe Glu Pro Ser Gly Phe His
            100                 105

<210> 577
<211> 26
<212> PRT
<213> Homo sapiens

<400> 577
Ala Gly Ile Arg Tyr Ile Ser Ser Phe Ile Phe Phe Phe Asp Ile Tyr
  1               5                  10                  15
Gln Ser Pro Ser Met His Gln Thr Leu Leu
             20                  25

<210> 578
<211> 111
<212> PRT
<213> Homo sapiens

<220>
<221> misc_feature
<222> (1)...(111)
<223> Xaa = any amino acid or nothing

<400> 578
Gly Ile Asp Thr Ile Leu Thr Leu Asn Gln Asn Xaa Ser Leu Lys Thr
  1               5                  10                  15
Arg Gln Xaa Phe Thr Leu Ile Ile Phe Ile Phe Phe Phe Phe Leu Arg

20                  25                  30
Trp Ser Leu Asp Ser Val Ala Gln Ala Gly Val Gln Trp Arg Asp Leu
        35                  40                  45
Gly Ser Leu Gln Ala Pro Pro Arg Gly Phe Thr Pro Phe Ser Cys Leu
    50                  55                  60
Ser Leu Pro Ser Ser Trp Asp Tyr Arg Arg Pro Leu Pro Arg Pro Ala
65                  70                  75                  80
Asn Phe Phe Tyr Phe Xaa Xaa Arg Arg Gly Phe Thr Met Leu Ala Arg
                85                  90                  95
Met Val Ser Ile Ser Xaa Pro Arg Asp Leu Pro Ala Glu Phe Leu
            100                 105                 110

<210> 579
<211> 176
<212> PRT
<213> Homo sapiens

<220>
<221> misc_feature
<222> (1)...(176)
<223> Xaa = any amino acid or nothing

<400> 579
Asn Leu Phe His Ala Lys Pro Glu Arg Arg Asp Ser Ala Asn Leu Trp
1               5                   10                  15
Val Leu Val Asp Cys Ile Phe Arg Asp Thr Ser Glu Asp Leu Gly Phe
            20                  25                  30
Gln Cys Asp Ala Val Asn Leu Ala Phe Gly Arg Arg Cys Glu Glu Leu
        35                  40                  45
Glu Asp Ala Arg His Lys Leu Gln His His Leu His Lys Met Leu Arg
    50                  55                  60
Glu Ile Thr Asp Gln Glu His Asn Val Val Ala Leu Lys Glu Ala Ile
65                  70                  75                  80
Lys Asp Lys Glu Glu Pro Leu His Ile Ala Gln Thr Arg Leu Xaa Leu
                85                  90                  95
Leu Ser His Arg Pro Asn Met Gln Leu Ala Pro Asn Ala Ala Gln Phe
            100                 105                 110
Ser Pro Ser Ser Leu Pro Pro Asn Thr Pro Lys Leu Leu Gln Ala Glu
        115                 120                 125
Gln Ser Leu Arg Asn Leu Lys Asp Ile His Met Ser Leu Glu Lys Asp
    130                 135                 140
Val Thr Ala Met Thr Asn Ser Val Phe Ile Asp Arg Gln Lys Cys Met
145                 150                 155                 160
Ala His Arg Thr Cys Tyr Pro Thr Ile Leu Gln Leu Ala Gly Tyr Gln
                165                 170                 175

<210> 580
<211> 176
<212> PRT
<213> Homo sapiens

<220>
<221> misc_feature

<222> (1)...(176)
<223> Xaa = any amino acid or nothing

<400> 580
Asn Leu Phe His Ala Lys Pro Glu Arg Arg Asp Ser Ala Asn Leu Trp
1               5                   10                  15
Val Leu Val Asp Cys Ile Phe Arg Asp Thr Ser Glu Asp Leu Gly Phe
            20                  25                  30
Gln Cys Asp Ala Val Asn Leu Ala Phe Gly Arg Arg Cys Glu Glu Leu
        35                  40                  45
Glu Asp Ala Arg His Lys Leu Gln His His Leu His Lys Met Leu Arg
    50                  55                  60
Glu Ile Thr Asp Gln Glu His Asn Val Val Ala Leu Lys Glu Ala Ile
65                  70                  75                  80
Lys Asp Lys Glu Glu Pro Leu His Ile Ala Gln Thr Arg Leu Xaa Leu
                85                  90                  95
Leu Ser His Arg Pro Asn Met Gln Leu Ala Pro Asn Ala Ala Gln Phe
            100                 105                 110
Ser Pro Ser Ser Leu Pro Pro Asn Thr Pro Lys Leu Leu Gln Ala Glu
        115                 120                 125
Gln Ser Leu Arg Asn Leu Lys Asp Ile His Met Ser Leu Glu Lys Asp
    130                 135                 140
Val Thr Ala Met Thr Asn Ser Val Phe Ile Asp Arg Gln Lys Cys Met
145                 150                 155                 160
Ala His Arg Thr Cys Tyr Pro Thr Ile Leu Gln Leu Ala Gly Tyr Gln
                165                 170                 175

<210> 581
<211> 65
<212> PRT
<213> Homo sapiens

<220>
<221> misc_feature
<222> (1)...(65)
<223> Xaa = any amino acid or nothing

<400> 581
Xaa Ser Asp Asp Glu Glu Gln Leu Thr Glu Leu Asp Glu Glu Met Glu
1               5                   10                  15
Asn Glu Ile Cys Arg Val Trp Asp Met Ser Met Asp Glu Asp Val Ala
            20                  25                  30
Leu Phe Leu Gln Glu Phe Asn Ala Pro Asp Ile Phe Met Gly Val Leu
        35                  40                  45
Ala Lys Ser Lys Cys Pro Arg Leu Arg Glu Ile Cys Val Gly Ile Leu
    50                  55                  60
Xaa
65

<210> 582
<211> 44
<212> PRT

<213> Homo sapiens

<220>
<221> misc_feature
<222> (1)...(44)
<223> Xaa = any amino acid or nothing

<400> 582
Ser Ser Lys Pro Trp Asp Xaa Ser Leu Ala Pro Lys His Ser Gly Xaa
1               5                   10                  15
Thr Lys Asn Met Asp Cys Tyr Cys Ile Ile Pro Thr Cys Ile Gly Arg
            20                  25                  30
Glu Arg Cys Tyr Gly Thr Cys Ile Gly Asp Thr Val
        35                  40

<210> 583
<211> 134
<212> PRT
<213> Homo sapiens

<220>
<221> misc_feature
<222> (1)...(134)
<223> Xaa = any amino acid or nothing

<400> 583
Val Leu Ser Leu Leu Trp Glu Phe Ala Leu Glu Pro Arg Val Arg His
1               5                   10                  15
Glu Leu Thr Leu Ser Leu Leu Phe Arg Lys Arg Arg Ala Asp Met Pro
            20                  25                  30
Arg Val Leu Val Phe Arg Ala Leu Leu Met Val Leu Ile Phe Leu Phe
        35                  40                  45
Val Ala Ser Tyr Trp Leu Phe Tyr Gly Val Arg Ile Leu Asp Ser Arg
    50                  55                  60
Asp Arg Asn Tyr His Gly Ile Val Gln Tyr Ala Val Ser Leu Val Asp
65                  70                  75                  80
Ala Leu Leu Phe Ile His Tyr Leu Ala Ile Val Leu Leu Glu Leu Arg
                85                  90                  95
Gln Leu Gln Pro Met Phe Thr Leu Gln Val Val Arg Ser Thr Asp Gly
            100                 105                 110
Glu Ser Arg Phe Tyr Ser Leu Xaa His Leu Ser Ile Gln Arg Ala Ala
        115                 120                 125
Leu Val Val Leu Glu Asn
    130

<210> 584
<211> 199
<212> PRT
<213> Homo sapiens

<400> 584
Ile Arg Thr Ser Arg Thr Gly Val Ser Leu Leu Val Pro Asn Gly Ala
1               5                   10                  15

Ile Pro Gln Gly Lys Phe Tyr Glu Met Tyr Leu Leu Ile Asn Lys Ala
            20                  25                  30
Glu Ser Thr Leu Pro Leu Ser Glu Gly Thr Gln Thr Val Leu Ser Pro
        35                  40                  45
Ser Val Thr Cys Gly Pro Thr Gly Leu Leu Leu Cys Arg Pro Val Ile
    50                  55                  60
Leu Thr Met Pro His Cys Ala Glu Val Ser Ala Arg Asp Trp Ile Phe
65                  70                  75                  80
Gln Leu Lys Thr Gln Ala His Gln Gly His Trp Glu Glu Val Val Thr
                85                  90                  95
Leu Asp Glu Glu Thr Leu Asn Thr Pro Cys Tyr Cys Gln Leu Glu Pro
            100                 105                 110
Arg Ala Cys His Ile Leu Leu Asp Gln Leu Gly Thr Tyr Val Phe Thr
        115                 120                 125
Gly Glu Ser Tyr Ser Arg Ser Ala Val Lys Arg Leu Gln Leu Ala Val
    130                 135                 140
Phe Ala Pro Ala Leu Cys Thr Ser Leu Glu Tyr Ser Leu Arg Val Tyr
145                 150                 155                 160
Cys Leu Glu Asp Thr Pro Val Ala Leu Lys Glu Val Leu Glu Leu Glu
                165                 170                 175
Arg Thr Leu Gly Gly Tyr Leu Val Glu Glu Pro Lys Pro Leu Met Phe
            180                 185                 190
Lys Asp Ser Tyr His Asn Leu
        195

## Description

### 1. TECHNICAL FIELD

The present invention provides novel polynucleotides and proteins encoded by such polynucleotides, along with uses for these polynucleotides and proteins, for example in therapeutic, diagnostic and research methods.

### 2. BACKGROUND

Technology aimed at the discovery of protein factors (including e.g., cytokines, such as lymphokines, interferons, circulating soluble factors, chemokines, and interleukins) has matured rapidly over the past decade. The now routine hybridization cloning and expression cloning techniques clone novel polynucleotides "directly" in the sense that they rely on information directly related to the discovered protein (i.e., partial DNA/amino acid sequence of the protein in the case of hybridization cloning; activity of the protein in the case of expression cloning). More recent "indirect" cloning techniques such as signal sequence cloning, which isolates DNA sequences based on the presence of a now well-recognized secretory leader sequence motif, as well as various PCR-based or low stringency hybridization-based cloning techniques, have advanced the state of the art by making available large numbers of DNA/amino acid sequences for proteins that are known to have biological activity, for example, by virtue of their secreted nature in the case of leader sequence cloning, by virtue of their cell or tissue source in the case of PCR-based techniques, or by virtue of structural similarity to other genes of known biological activity.

Identified polynucleotide and polypeptide sequences have numerous applications in, for example, diagnostics, forensics, gene mapping; identification of mutations responsible for genetic disorders or other traits, to assess biodiversity, and to produce many other types of data and products dependent on DNA and amino acid sequences.

### 3. SUMMARY OF THE INVENTION

The compositions of the present invention include novel isolated polypeptides, novel isolated polynucleotides encoding such polypeptides, including recombinant DNA molecules, cloned genes or degenerate variants thereof, especially naturally occurring variants such as allelic variants, antisense polynucleotide molecules, and antibodies that specifically recognize one or more epitopes present on such polypeptides, as well as hybridomas producing such antibodies.

The compositions of the present invention additionally include vectors, including expression vectors, containing the polynucleotides of the invention, cells genetically engineered to contain such polynucleotides and cells genetically engineered to express such polynucleotides.

The present invention relates to a collection or library of at least one novel nucleic acid sequence assembled from expressed sequence tags (ESTs) isolated mainly by sequencing by hybridization (SBH), and in some cases, sequences obtained from one or more public databases. The invention relates also to the proteins encoded by such polynucleotides,along with therapeutic, diagnostic and research utilities for these polynucleotides and proteins. These nucleic acid sequences are designated as SEQ ID NO: 1-146, or 293-438. The polypeptides sequences are designated SEQ ID NO: 147-292, or 439-584. The nucleic acids and polypeptides are provided in the Sequence Listing. In the nucleic acids provided in the Sequence Listing, A is adenosine; C is cytosine; G is guanine; T is thymine; and N is unknown or any of the four bases. In the amino acids provided in the Sequence Listing, and Table 6, * corresponds to the stop codon.

The nucleic acid sequences of the present invention also include, nucleic acid sequences that hybridize to the complement of SEQ ID NO: 1-146, or 293-438 under stringent hybridization conditions; nucleic acid sequences which are allelic variants or species homologues of any of the nucleic acid sequences recited above, or nucleic acid sequences that encode a peptide comprising a specific domain or truncation of the peptides encoded by SEQ ID NO: 1-146, or 293-438. A polynucleotide comprising a nucleotide sequence having at least 90% identity to an identifying sequence of SEQ ID NO: 1-146, or 293-438 or a degenerate variant or fragment thereof. The identifying sequence can be 100 base pairs in length.

The nucleic acid sequences of the present invention also include the sequence information from the nucleic acid sequences of SEQ ID NO: 1-146, or 293-438. The sequence information can be a segment of any one of SEQ ID NO: 1-146, or 293-438 that uniquely identifies or represents the sequence information of SEQ ID NO: 1-146, or 293-438.

A collection as used in this application can be a collection of only one polynucleotide. The collection of sequence information or identifying information of each sequence can be provided on a nucleic acid array. In one embodiment, segments of sequence information are provided on a nucleic acid array to detect the polynucleotide that contains the segment. The array can be designed to detect full-match or mismatch to the polynucleotide that contains the segment. The collection can also be provided in a computer-readable format.

This invention also includes the reverse or direct complement of any of the nucleic acid sequences recited above; cloning or expression vectors containing the nucleic acid sequences; and host cells or organisms transformed with these expression vectors. Nucleic acid sequences (or their reverse or direct complements) according to the invention have numerous applications in a variety of techniques known to those skilled in the art of molecular biology, such as use as hybridization probes, use as primers for PCR, use in an array, use in computer-readablemedia, use in sequencing full-length genes, use for chromosome and gene mapping, use in the recombinant production of protein, and use in the generationof anti-sense DNA or RNA, their chemical analogs and the like.

In a preferred embodiment, the nucleic acid sequences of SEQ ID NO: 1-146, or 293-438 or novel segments or parts of the nucleic acids of the invention are used as primers in expression assays that are well known in the art. In a particularly preferred embodiment, the nucleic acid sequences of SEQ ID NO: 1-146, or 293-438 or novel segments or parts of the nucleic acids provided herein are used in diagnostics for identifying expressed genes or, as well known in the art and exemplified by Vollrath et al., Science 258:52-59 (1992), as expressed sequence tags for physical mapping of the human genome.

The isolated polynucleotides of the invention include, but are not limited to, a polynucleotide comprising any one of the nucleotide sequences set forth in SEQ ID NO: 1-146, or 293-438; a polynucleotide comprising any of the full length protein coding sequences of SEQ ID NO: 1-146, or 293-43 8; and a polynucleotide comprising any of the nucleotide sequences of the mature protein coding sequences of SEQ ID NO: 1-146, or 293-438. The polynucleotides of the present invention also include, but are not limited to, a polynucleotide that hybridizes under stringent hybridization conditions to (a) the complement of any one of the nucleotide sequences set forth in SEQ ID NO: 1-146, or 293-438; (b) a nucleotide sequence encoding any one of the amino acid sequences set forth in the Sequence Listing; (c) a polynucleotide which is an allelic variant of any polynucleotides recited above; (d) a polynucleotide which encodes a species homolog (e.g. orthologs) of any of the proteins recited above; or (e) a polynucleotide that encodes a polypeptide comprising a specific domain or truncation of any of the polypeptides comprising an amino acid sequence set forth in the Sequence Listing.

The isolated polypeptides of the invention include, but are not limited to, a polypeptide comprising any of the amino acid sequences set forth in SEQ ID NO: 147-292, or 439-584; or the corresponding full length or mature protein. Polypeptides of the invention also include polypeptides with biological activity that are encoded by (a) any of the polynucleotides having a nucleotide sequence set forth in SEQ ID NO: 1-146, or 293-438; or (b) polynucleotidesthat hybridize to the complement of the polynucleotidesof (a) under stringent hybridization conditions. Biologically or immunologically active variants of any of the polypeptide sequences in the Sequence Listing, and "substantial equivalents" thereof (e.g., with at least about 65%, 70%, 75%, 80%, 85%, 90%, 95%, 98% or 99% amino acid sequence identity) that preferably retain biological activity are also contemplated. The polypeptides of the invention may be wholly or partially chemically synthesized but are preferably produced by recombinant means using the genetically engineered cells (e.g. host cells) of the invention.

The invention also provides compositions comprising a polypeptide of the invention. Polypeptide compositions of the invention may further comprise an acceptable carrier, such as a hydrophilic, e.g., pharmaceutically acceptable, carrier.

The invention also provides host cells transformed or transfected with a polynucleotide of the invention.

The invention also relates to methods for producing a polypeptide of the invention comprising growing a culture of the host cells of the invention in a suitable culture medium under conditions permitting expression of the desired polypeptide, and purifying the polypeptide from the culture or from the host cells. Preferred embodiments include those in which the protein produced by such process is a mature form of the protein.

Polynucleotides according to the invention have numerous applications in a variety of techniques known to those skilled in the art of molecular biology. These techniques include use as hybridization probes, use as oligomers, or primers, for PCR, use for chromosome and gene mapping, use in the recombinant production of protein, and use in generation of anti-sense DNA or RNA, their chemical analogs and the like. For example, when the expression of an mRNA is largely restricted to a particular cell or tissue type, polynucleotides of the invention can be used as hybridization probes to detect the presence of the particular cell or tissue mRNA in a sample using, *e.g., in situ* hybridization.

In other exemplary embodiments, the polynucleotides are used in diagnostics as expressed sequence tags for identifying expressed genes or, as well known in the art and exemplified by Vollrath et al., Science 258:52-59 (1992), as expressed sequence tags for physical mapping of the human genome.

The polypeptides according to the invention can be used in a variety of conventional procedures and methods that are currently applied to other proteins. For example, a polypeptide of the invention can be used to generate an antibody that specifically binds the polypeptide. Such antibodies, particularly monoclonal antibodies, are useful for detecting or quantitating the polypeptide in tissue. The polypeptides of the invention can also be used as molecular weight markers, and as a food supplement.

Methods are also provided for preventing, treating, or ameliorating a medical condition which comprises the step of administering to a mammalian subject a therapeutically effective amount of a composition comprising a polypeptide of the present invention and a pharmaceutically acceptable carrier.

In particular, the polypeptides and polynucleotides of the invention can be utilized, for example, in methods for the prevention and/or treatment of disorders involving aberrant protein expression or biological activity.

The present invention further relates to methods for detecting the presence of the polynucleotides or polypeptides of the invention in a sample. Such methods can, for example, be utilized as part of prognostic and diagnostic evaluation of disorders as recited herein and for the identification of subjects exhibiting a predisposition to such conditions. The invention provides a method for detecting the polynucleotides of the invention in a sample, comprising contacting the sample with a compound that binds to and forms a complex with the polynucleotide of interest for a period sufficient to form the complex and under conditions sufficient to form a complex and detecting the complex such that if a complex is detected, the polynucleotide of interest is detected. The invention also provides a method for detecting the polypeptides of the invention in a sample comprising contacting the sample with a compound that binds to and forms a complex with the polypeptide under conditions and for a period sufficient to form the complex and detecting the formation of the complex such that if a complex is formed, the polypeptide is detected.

The invention also provides kits comprising polynucleotide probes and/or monoclonal antibodies, and optionally quantitative standards, for carrying out methods of the invention. Furthermore, the invention provides methods for evaluating the efficacy of drugs, and monitoring the progress of patients, involved in clinical trials for the treatment of disorders as recited above.

The invention also provides methods for the identification of compounds that modulate (i.e., increase or decrease) the expression or activity of the polynucleotides and/or polypeptides of the invention. Such methods can be utilized, for example, for the identification of compounds that can ameliorate symptoms of disorders as recited herein. Such methods can include, but are not limited to, assays for identifying compounds and other substances that interact with (*e*.*g*., bind to) the polypeptides of the invention. The invention provides a method for identifying a compound that binds to the polypeptides of the invention comprising contacting the compound with a polypeptide of the invention in a cell for a time sufficient to form a polypeptide/compound complex, wherein the complex drives expression of a reporter gene sequence in the cell; and detecting the complex by detecting the reporter gene sequence expression such that if expression of the reporter gene is detected the compound the binds to a polypeptide of the invention is identified.

The methods of the invention also provide methods for treatment which involve the administration of the polynucleotides or polypeptides of the invention to individuals exhibiting symptoms or tendencies. In addition, the invention encompasses methods for treating diseases or disorders as recited herein comprising administering compounds and other substances that modulate the overall activity of the target gene products. Compounds and other substances can effect such modulation either on the level of target gene/protein expression or target protein activity.

The polypeptides of the present invention and the polynucleotides encoding them are also useful for the same functions known to one of skill in the art as the polypeptides and polynucleotides to which they have homology (set forth in Table 2); for which they have a signature region (as set forth in Table 3); or for which they have homology to a gene family (as set forth in Table 4). If no homology is set forth for a sequence, then the polypeptides and polynucleotides of the present invention are useful for a variety of applications, as described herein, including use in arrays for detection.

### 4. DETAILED DESCRIPTION OF THE INVENTION

### 4.1 DEFINITIONS

It must be noted that as used herein and in the appended claims, the singular forms "a", "an" and "the" include plural references unless the context clearly dictates otherwise.

The term "active" refers to those forms of the polypeptide which retain the biologic and/or immunologic activities of any naturally occurring polypeptide. According to the invention, the terms "biologically active" or "biological activity" refer to a protein or peptide having structural, regulatory or biochemical functions of a naturally occurring molecule. Likewise "immunologically active" or "immunological activity" refers to the capability of the natural, recombinant or synthetic polypeptide to induce a specific immune response in appropriate animals or cells and to bind with specific antibodies.

The term "activated cells" as used in this application are those cells which are engaged in extracellular or intracellular membrane trafficking, including the export of secretory or enzymatic molecules as part of a normal or disease process.

The terms "complementary" or "complementarity" refer to the natural binding of polynucleotides by base pairing. For example, the sequence 5'-AGT-3' binds to the complementary sequence 3'-TCA-5'. Complementarity between two single-stranded molecules may be "partial" such that only some of the nucleic acids bind or it may be "complete" such that total complementarity exists between the single stranded molecules. The degree of complementarity between the nucleic acid strands has significant effects on the efficiency and strength of the hybridization between the nucleic acid strands.

The term "embryonic stem cells (ES)" refers to a cell that can give rise to many differentiated cell types in an embryo or an adult, including the germ cells. The term "germ line stem cells (GSCs)" refers to stem cells derived from primordial stem cells that provide a steady and continuous source of germ cells for the production of gametes. The term "primordial germ cells (PGCs)" refers to a small population of cells set aside from other cell lineages particularly from the yolk sac, mesenteries, or gonadal ridges during embryogenesis that have the potential to differentiate into germ cells and other cells. PGCs are the source from which GSCs and ES cells are derived. The PGCs, the GSCs and the ES cells are capable of self-renewal. Thus these cells not only populate the germ line and give rise to a plurality of terminally differentiated cells that comprise the adult specialized organs, but are able to regenerate themselves.

The term "expression modulating fragment," EMF, means a series of nucleotides which modulates the expression of an operably linked ORF or another EMF.

As used herein, a sequence is said to "modulate the expression of an operably linked sequence" when the expression of the sequence is altered by the presence of the EMF. EMFs include, but are not limited to, promoters, and promoter modulating sequences (inducible elements). One class of EMFs are nucleic acid fragments which induce the expression of an operably linked ORF in response to a specific regulatory factor or physiological event.

The terms "nucleotide sequence" or "nucleic acid" or "polynucleotide" or "oligonculeotide" are used interchangeably and refer to a heteropolymer of nucleotides or the sequence of these nucleotides. These phrases also refer to DNA or RNA of genomic or synthetic origin which may be single-stranded or double-stranded and may represent the sense or the antisense strand, to peptide nucleic acid (PNA) or to any DNA-like or RNA-like material. In the sequences herein A is adenine, C is cytosine, T is thymine, G is guanine and N is A, C, G or T (U). It is contemplated that where the polynucleotide is RNA, the T (thymine) in the sequences provided herein is substituted with U (uracil). Generally, nucleic acid segments provided by this invention may be assembled from fragments of the genome and short oligonucleotide linkers, or from a series of oligonucleotides, or from individual nucleotides, to provide a synthetic nucleic acid which is capable of being expressed in a recombinant transcriptional unit comprising regulatory elements derived from a microbial or viral operon, or a eukaryotic gene.

The terms "oligonucleotide fragment" or a "polynucleotide fragment", "portion," or "segment" or "probe" or "primer" are used interchangeably and refer to a sequence of nucleotide residues which are at least about 5 nucleotides, more preferably at least about 7 nucleotides, more preferably at least about 9 nucleotides, more preferably at least about 11 nucleotides and most preferably at least about 17 nucleotides. The fragment is preferably less than about 500 nucleotides, preferably less than about 200 nucleotides, more preferably less than about 100 nucleotides, more preferably less than about 50 nucleotides and most preferably less than 30 nucleotides. Preferably the probe is from about 6 nucleotides to about 200 nucleotides, preferably from about 15 to about 50 nucleotides, more preferably from about 17 to 30 nucleotides and most preferably from about 20 to 25 nucleotides. Preferably the fragments can be used in polymerase chain reaction (PCR), various hybridization procedures or microarray procedures to identify or amplify identical or related parts of mRNA or DNA molecules. A fragment or segment may uniquely identify each polynucleotide sequence of the present invention. Preferably the fragment comprises a sequence substantially similar to any one of SEQ ID NO: 1-146, or 293-438.

Probes may, for example, be used to determine whether specific mRNA molecules are present in a cell or tissue or to isolate similar nucleic acid sequences from chromosomal DNA as described by Walsh et al. (Walsh, P.S. et al., 1992, PCR Methods Appl 1:241-250). They may be labeled by nick translation, Klenow fill-in reaction, PCR, or other methods well known in the art. Probes of the present invention, their preparation and/or labeling are elaborated in Sambrook, J. et al., 1989, Molecular Cloning: A Laboratory Manual, Cold Spring Harbor Laboratory, NY; or Ausubel, F.M. et al., 1989, Current Protocols in Molecular Biology, John Wiley & Sons, New York NY, both of which are incorporated herein by reference in their entirety.

The nucleic acid sequences of the present invention also include the sequence information from the nucleic acid sequences of SEQ ID NO: 1-146, or 293-438. The sequence information can be a segment of any one of SEQ ID NO: 1-146, or 293-438 that uniquely identifies or represents the sequence information of that sequence of SEQ ID NO: 1-146, or 293-438. One such segment can be a twenty-mer nucleic acid sequence because the probability that a twenty-mer is fully matched in the human genome is I in 300. In the human genome, there are three billion base pairs in one set of chromosomes. Because 4²⁰ possible twenty-mers exist, there are 300 times more twenty-mers than there are base pairs in a set of human chromosomes. Using the same analysis, the probability for a seventeen-mer to be fully matched in the human genome is approximately 1 in 5. When these segments are used in arrays for expression studies, fifteen-mer segments can be used. The probability that the fifteen-mer is fully matched in the expressed sequences is also approximately one in five because expressed sequences comprise less than approximately 5% of the entire genome sequence.

Similarly, when using sequence information for detecting a single mismatch, a segment can be a twenty-five mer. The probability that the twenty-five mer would appear in a human genome with a single mismatch is calculated by multiplying the probability for a full match (1÷4²⁵) times the increased probability for mismatch at each nucleotide position (3 x 25). The probability that an eighteen mer with a single mismatch can be detected in an array for expression studies is approximately one in five. The probability that a twenty-mer with a single mismatch can be detected in a human genome is approximately one in five.

The term "open reading frame," ORF, means a series of nucleotide triplets coding for amino acids without any termination codons and is a sequence translatable into protein.

The terms "operably linked" or "operably associated" refer to functionally related nucleic acid sequences. For example, a promoter is operably associated or operably linked with a coding sequence if the promoter controls the transcription of the coding sequence. While operably linked nucleic acid sequences can be contiguous and in the same reading frame, certain genetic elements e.g. repressor genes are not contiguously linked to the coding sequence but still control transcription/translation of the coding sequence.

The term "pluripotent" refers to the capability of a cell to differentiate into a number of differentiated cell types that are present in an adult organism. A pluripotent cell is restricted in its differentiation capability in comparison to a totipotent cell.

The terms "polypeptide" or "peptide" or "amino acid sequence" refer to an oligopeptide, peptide, polypeptide or protein sequence or fragment thereof and to naturally occurring or synthetic molecules. A polypeptide "fragment," "portion," or "segment" is a stretch of amino acid residues of at least about 5 amino acids, preferably at least about 7 amino acids, more preferably at least about 9 amino acids and most preferably at least about 17 or more amino acids. The peptide preferably is not greater than about 500 amino acids, more preferably less than 200 amino acids more preferably less than 150 amino acids and most preferably less than 100 amino acids. Preferably the peptide is from about 5 to about 200 amino acids. To be active, any polypeptide must have sufficient length to display biological and/or immunological activity.

The term "naturally occurring polypeptide" refers to polypeptides produced by cells that have not been genetically engineered and specifically contemplates various polypeptides arising from post-translational modifications of the polypeptide including, but not limited to, acetylation, carboxylation, glycosylation, phosphorylation, lipidation and acylation.

The term "translated protein coding portion" means a sequence which encodes for the full length protein which may include any leader sequence or any processing sequence.

The term "mature protein coding sequence" means a sequence which encodes a peptide or protein without a signal or leader sequence. The "mature protein portion" means that portion of the protein which does not include a signal or leader sequence. The peptide may have been produced by processing in the cell which removes any leader/signal sequence. The mature protein portion may or may not include an initial methionine residue. The methionine residue may be removed from the protein during processing in the cell. The peptide may be produced synthetically or the protein may have been produced using a polynucleotide only encoding for the mature protein coding sequence.

The term "derivative" refers to polypeptides chemically modified by such techniques as ubiquitination, labeling (e.g., with radionuclides or various enzymes), covalent polymer attachment such as pegylation (derivatization with polyethylene glycol) and insertion or substitution by chemical synthesis of amino acids such as omithine, which do not normally occur in human proteins.

The term "variant"(or "analog") refers to any polypeptide differing from naturally occurring polypeptides by amino acid insertions, deletions, and substitutions, created using, e g., recombinant DNA techniques. Guidance in determining which amino acid residues may be replaced, added or deleted without abolishing activities of interest, may be found by comparing the sequence of the particular polypeptide with that of homologous peptides and minimizing the number of amino acid sequence changes made in regions of high homology (conserved regions) or by replacing amino acids with consensus sequence.

Alternatively, recombinant variants encoding these same or similar polypeptides may be synthesized or selected by making use of the "redundancy" in the genetic code. Various codon substitutions, such as the silent changes which produce various restriction sites, may be introduced to optimize cloning into a plasmid or viral vector or expression in a particular prokaryotic or eukaryotic system. Mutations in the polynucleotide sequence may be reflected in the polypeptide or domains of other peptides added to the polypeptide to modify the properties of any part of the polypeptide, to change characteristics such as ligand-binding affinities, interchain affinities, or degradation/turnover rate.

Preferably, amino acid "substitutions" are the result of replacing one amino acid with another amino acid having similar structural and/or chemical properties, *i. e.*, conservative amino acid replacements. "Conservative" amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid. "Insertions" or "deletions" are preferably in the range of about 1 to 20 amino acids, more preferably 1 to 10 amino acids. The variation allowed may be experimentally determined by systematically making insertions, deletions, or substitutions of amino acids in a polypeptide molecule using recombinant DNA techniques and assaying the resulting recombinant variants for activity.

Alternatively, where alteration of function is desired, insertions, deletions or non-conservative alterations can be engineered to produce altered polypeptides. Such alterations can, for example, alter one or more of the biological functions or biochemical characteristics of the polypeptides of the invention. For example, such alterations may change polypeptide characteristics such as ligand-binding affinities, interchain affinities, or degradation/turnover rate. Further, such alterations can be selected so as to generate polypeptides that are better suited for expression, scale up and the like in the host cells chosen for expression. For example, cysteine residues can be deleted or substituted with another amino acid residue in order to eliminate disulfide bridges.

The terms "purified" or "substantially purified" as used herein denotes that the indicated nucleic acid or polypeptide is present in the substantial absence of other biological macromolecules, e.g., polynucleotides, proteins, and the like. In one embodiment, the polynucleotide or polypeptide is purified such that it constitutes at least 95% by weight, more preferably at least 99% by weight, of the indicated biological macromolecules present (but water, buffers, and other small molecules, especially molecules having a molecular weight of less than 1000 daltons, can be present).

The term "isolated" as used herein refers to a nucleic acid or polypeptide separated from at least one other component (e.g., nucleic acid or polypeptide) present with the nucleic acid or polypeptide in its natural source. In one embodiment, the nucleic acid or polypeptide is found in the presence of (if anything) only a solvent, buffer, ion, or other component normally present in a solution of the same. The terms "isolated" and "purified" do not encompass nucleic acids or polypeptides present in their natural source.

The term "recombinant," when used herein to refer to a polypeptide or protein, means that a polypeptide or protein is derived from recombinant (*e*.*g*., microbial, insect, or mammalian) expression systems. "Microbial" refers to recombinant polypeptides or proteins made in bacterial or fungal (e.g., yeast) expression systems. As a product, "recombinant microbial" defines a polypeptide or protein essentially free of native endogenous substances and unaccompanied by associated native glycosylation. Polypeptides or proteins expressed in most bacterial cultures, *e*.*g*., *E. coli*, will be free of glycosylation modifications; polypeptides or proteins expressed in yeast will have a glycosylation pattern in general different from those expressed in mammalian cells.

The term "recombinant expression vehicle or vector" refers to a plasmid or phage or virus or vector, for expressing a polypeptide from a DNA (RNA) sequence. An expression vehicle can comprise a transcriptional unit comprising an assembly of (1) a genetic element or elements having a regulatory role in gene expression, for example, promoters or enhancers, (2) a structural or coding sequence which is transcribed into mRNA and translated into protein, and (3) appropriate transcription initiation and termination sequences. Structural units intended for use in yeast or eukaryotic expression systems preferably include a leader sequence enabling extracellular secretion of translated protein by a host cell. Alternatively, where recombinant protein is expressed without a leader or transport sequence, it may include an amino terminal methionine residue. This residue may or may not be subsequently cleaved from the expressed recombinant protein to provide a final product.

The term "recombinant expression system" means host cells which have stably integrated a recombinant transcriptional unit into chromosomal DNA or carry the recombinant transcriptional unit extrachromosomally. Recombinant expression systems as defined herein will express heterologous polypeptides or proteins upon induction of the regulatory elements linked to the DNA segment or synthetic gene to be expressed. This term also means host cells which have stably integrated a recombinant genetic element or elements having a regulatory role in gene expression, for example, promoters or enhancers. Recombinant expression systems as defined herein will express polypeptides or proteins endogenous to the cell upon induction of the regulatory elements linked to the endogenous DNA segment or gene to be expressed. The cells can be prokaryotic or eukaryotic.

The term "secreted" includes a protein that is transported across or through a membrane, including transport as a result of signal sequences in its amino acid sequence when it is expressed in a suitable host cell. "Secreted" proteins include without limitation proteins secreted wholly (*e.g.*, soluble proteins) or partially (*e.g.*, receptors) from the cell in which they are expressed. "Secreted" proteins also include without limitation proteins that are transported across the membrane of the endoplasmic reticulum. "Secreted" proteins are also intended to include proteins containing non-typical signal sequences (e.g. Interleukin-1 Beta, see Krasney, P.A. and Young, P.R. (1992) Cytokine 4(2): 134 -143) and factors released from damaged cells (e.g. Interleukin-1 Receptor Antagonist, see Arend, W.P. et al. (1998) Annu. Rev. Immunol. 16:27-55)

Where desired, an expression vector may be designed to contain a "signal or leader sequence" which will direct the polypeptide through the membrane of a cell. Such a sequence may be naturally present on the polypeptides of the present invention or provided from heterologous protein sources by recombinant DNA techniques.

The term "stringent" is used to refer to conditions that are commonly understood in the art as stringent. Stringent conditions can include highly stringent conditions (i.e., hybridization to filter-bound DNA in 0.5 M NaHPO₄, 7% sodium dodecyl sulfate (SDS), 1 mM EDTA at 65°C, and washing in 0.1X SSC/0.1% SDS at 68°C), and moderately stringent conditions (i.e., washing in 0.2X SSC/0.1% SDS at 42°C). Other exemplary hybridization conditions are described herein in the examples.

In instances of hybridization of deoxyoligonucleotides, additional exemplary stringent hybridization conditions include washing in 6X SSC/0.05% sodium pyrophosphate at 37°C (for 14-base oligonucleotides), 48°C (for 17-base oligos), 55°C (for 20-base oligonucleotides), and 60°C (for 23-base oligonucleotides).

As used herein, "substantially equivalent" can refer both to nucleotide and amino acid sequences, for example a mutant sequence, that varies from a reference sequence by one or more substitutions, deletions, or additions, the net effect of which does not result in an adverse functional dissimilarity between the reference and subject sequences. Typically, such a substantially equivalent sequence varies from one of those listed herein by no more than about 35% (*i*.*e*., the number of individual residue substitutions, additions, and/or deletions in a substantially equivalent sequence, as compared to the corresponding reference sequence, divided by the total number of residues in the substantially equivalent sequence is about 0.35 or less). Such a sequence is said to have 65% sequence identity to the listed sequence. In one embodiment, a substantially equivalent, *e*.*g*., mutant, sequence of the invention varies from a listed sequence by no more than 30% (70% sequence identity); in a variation of this embodiment, by no more than 25% (75% sequence identity); and in a further variation of this embodiment, by no more than 20% (80% sequence identity) and in a further variation of this embodiment, by no more than 10% (90% sequence identity) and in a further variation of this embodiment, by no more that 5% (95% sequence identity). Substantially equivalent, *e.g.,* mutant, amino acid sequences according to the invention preferably have at least 80% sequence identity with a listed amino acid sequence, more preferably at least 85% sequence identity, more preferably at least 90% sequence identity, more preferably at least 95% identity, more preferably at least 98% identity, and most preferably at least 99% identity. Substantially equivalent nucleotide sequences of the invention can have lower percent sequence identities, taking into account, for example, the redundancy or degeneracy of the genetic code. Preferably, nucleotide sequence has at least about 65% identity, more preferably at least about 75% identity, more preferably at least about 80% sequence identity, more preferably at least about 85% sequence identity, more preferably at least about 90% sequence identity, and most preferably at least about 95% identity, more preferably at least about 98% sequence identity, and most preferably at least about 99% sequence identity. For the purposes of the present invention, sequences having substantially equivalent biological activity and substantially equivalent expression characteristics are considered substantially equivalent. For the purposes of determining equivalence, truncation of the mature sequence (*e*.*g*., via a mutation which creates a spurious stop codon) should be disregarded. Sequence identity may be determined, e.g., using the Jotun Hein method (Hein, J. (1990) Methods Enzymol. 183:626-645). Identity between sequences can also be determined by other methods known in the art, e.g. by varying hybridization conditions.

The term "totipotent" refers to the capability of a cell to differentiate into all of the cell types of an adult organism.

The term "transformation" means introducing DNA into a suitable host cell so that the DNA is replicable, either as an extrachromosomal element, or by chromosomal integration. The term "transfection" refers to the taking up of an expression vector by a suitable host cell, whether or not any coding sequences are in fact expressed. The term "infection" refers to the introduction of nucleic acids into a suitable host cell by use of a virus or viral vector.

As used herein, an "uptake modulating fragment," UMF, means a series of nucleotides which mediate the uptake of a linked DNA fragment into a cell. UMFs can be readily identified using known UMFs as a target sequence or target motif with the computer-based systems described below. The presence and activity of a UMF can be confirmed by attaching the suspected UMF to a marker sequence. The resulting nucleic acid molecule is then incubated with an appropriate host under appropriate conditions and the uptake of the marker sequence is determined. As described above, a UMF will increase the frequency of uptake of a linked marker sequence.

Each of the above terms is meant to encompass all that is described for each, unless the context dictates otherwise.

### 4.2 NUCLEIC ACIDS OF THE INVENTION

Nucleotide sequences of the invention are set forth in the Sequence Listing.

The isolated polynucleotides of the invention include a polynucleotide comprising the nucleotide sequences of SEQ ID NO: 1-146, or 293-438; a polynucleotide encoding any one of the peptide sequences of SEQ ID NO: 147-292, or 439-584; and a polynucleotide comprising the nucleotide sequence encoding the mature protein coding sequence of the polypeptides of any one of SEQ ID NO: 147-292, or 439-584. The polynucleotides of the present invention also include, but are not limited to, a polynucleotide that hybridizes under stringent conditions to (a) the complement of any of the nucleotides sequences of SEQ ID NO: 1-146, or 293-438; (b) nucleotide sequences encoding any one of the amino acid sequences set forth in the Sequence Listing as SEQ ID NO: 147-292, or 439-584; (c) a polynucleotide which is an allelic variant of any polynucleotide recited above; (d) a polynucleotide which encodes a species homolog of any of the proteins recited above; or (e) a polynucleotide that encodes a polypeptide comprising a specific domain or truncation of the polypeptides of SEQ ID NO: 147-292, or 439-584. Domains of interest may depend on the nature of the encoded polypeptide; e.g., domains in receptor-like polypeptides include ligand-binding, extracellular, transmembrane, or cytoplasmic domains, or combinations thereof; domains in immunoglobulin-like proteins include the variable immunoglobulin-like domains; domains in enzyme-like polypeptides include catalytic and substrate binding domains; and domains in ligand polypeptides include receptor-binding domains.

The polynucleotides of the invention include naturally occurring or wholly or partially synthetic DNA, e.g., cDNA and genomic DNA, and RNA, e.g., mRNA. The polynucleotides may include all of the coding region of the cDNA or may represent a portion of the coding region of the cDNA.

The present invention also provides genes corresponding to the cDNA sequences disclosed herein. The corresponding genes can be isolated in accordance with known methods using the sequence information disclosed herein. Such methods include the preparation of probes or primers from the disclosed sequence information for identification and/or amplification of genes in appropriate genomic libraries or other sources of genomic materials. Further 5' and 3' sequence can be obtained using methods known in the art. For example, full length cDNA or genomic DNA that corresponds to any of the polynucleotidesof SEQ ID NO: 1-146, or 293-438 can be obtained by screening appropriate cDNA or genomic DNA libraries under suitable hybridization conditions using any of the polynucleotidesof SEQ ID NO: 1-146, or 293-438 or a portion thereof as a probe. Alternatively, the polynucleotides of SEQ ID NO: 1-146, or 293-438 may be used as the basis for suitable primer(s) that allow identification and/or amplification of genes in appropriate genomic DNA or cDNA libraries.

The nucleic acid sequences of the invention can be assembled from ESTs and sequences (including cDNA and genomic sequences) obtained from one or more public databases, such as dbEST, gbpri, and UniGene. The EST sequences can provide identifying sequence information, representative fragment or segment information, or novel segment information for the full-length gene.

The polynucleotides of the invention also provide polynucleotides including nucleotide sequences that are substantially equivalent to the polynucleotides recited above. Polynucleotides according to the invention can have, *e.g.*, at least about 65%, at least about 70%, at least about 75%, at least about 80%, 81%, 82%, 83%, 84%, more typically at least about 85%, 86%, 87%, 88%, 89%, more typically at least about 90%, 91%, 92%, 93%, 94%, and even more typically at least about 95%, 96%, 97%, 98%, 99%, sequence identity to a polynucleotide recited above.

Included within the scope of the nucleic acid sequences of the invention are nucleic acid sequence fragments that hybridize under stringent conditions to any of the nucleotide sequences of SEQ ID NO: 1-146, or 293-438, or complements thereof, which fragment is greater than about 5 nucleotides, preferably 7 nucleotides, more preferably greater than 9 nucleotides and most preferably greater than 17 nucleotides. Fragments of, e.g. 15, 17, or 20 nucleotides or more that are selective for (i.e. specifically hybridize to) any one of the polynucleotides of the invention are contemplated. Probes capable of specifically hybridizing to a polynucleotide can differentiate polynucleotide sequences of the invention from other polynucleotide sequences in the same family of genes or can differentiate human genes from genes of other species, and are preferably based on unique nucleotide sequences.

The sequences falling within the scope of the present invention are not limited to these specific sequences, but also include allelic and species variations thereof. Allelic and species variations can be routinely determined by comparing the sequence provided in SEQ ID NO: 1-146, or 293-438, a representative fragment thereof, or a nucleotide sequence at least 90% identical, preferably 95% identical, to SEQ ID NO: 1-146, or 293-438 with a sequence from another isolate of the same species. Furthermore, to accommodate codon variability, the invention includes nucleic acid molecules coding for the same amino acid sequences as do the specific ORFs disclosed herein. In other words, in the coding region of an ORF, substitution of one codon for another codon that encodes the same amino acid is expressly contemplated.

The nearest neighbor or homology result for the nucleic acids of the present invention, including SEQ ID NO: 1-146, or 293-438, can be obtained by searching a database using an algorithm or a program. Preferably, a BLAST which stands for Basic Local Alignment Search Tool is used to search for local sequence alignments (Altshul, S.F. J Mol. Evol. 36 290-300 (1993) and Altschut S.F. et al. J. Mol. Biol. 21:403-410 (1990)). Alternatively a FASTA version 3 search against Genpept, using Fastxy algorithm.

Species homologs (or orthologs) of the disclosed polynucleotides and proteins are also provided by the present invention. Species homologs may be isolated and identified by making suitable probes or primers from the sequences provided herein and screening a suitable nucleic acid source from the desired species.

The invention also encompasses allelic variants of the disclosed polynucleotides or proteins; that is, naturally-occurring alternative forms of the isolated polynucleotide which also encode proteins which are identical, homologous or related to that encoded by the polynucleotides.

The nucleic acid sequences of the invention are further directed to sequences which encode variants of the described nucleic acids. These amino acid sequence variants may be prepared by methods known in the art by introducing appropriate nucleotide changes into a native or variant polynucleotide. There are two variables in the construction of amino acid sequence variants: the location of the mutation and the nature of the mutation. Nucleic acids encoding the amino acid sequence variants are preferably constructed by mutating the polynucleotide to encode an amino acid sequence that does not occur in nature. These nucleic acid alterations can be made at sites that differ in the nucleic acids from different species (variable positions) or in highly conserved regions (constant regions). Sites at such locations will typically be modified in series, *e*.*g*., by substituting first with conservative choices (*e*.*g*., hydrophobic amino acid to a different hydrophobic amino acid) and then with more distant choices (e.g., hydrophobic amino acid to a charged amino acid), and then deletions or insertions may be made at the target site. Amino acid sequence deletions generally range from about 1 to 30 residues, preferably about 1 to 10 residues, and are typically contiguous. Amino acid insertions include amino- and/or carboxyl-terminal fusions ranging in length from one to one hundred or more residues, as well as intrasequence insertions of single or multiple amino acid residues. Intrasequence insertions may range generally from about 1 to 10 amino residues, preferably from 1 to 5 residues. Examples of terminal insertions include the heterologous signal sequences necessary for secretion or for intracellular targeting in different host cells and sequences such as FLAG or poly-histidine sequences useful for purifying the expressed protein.

In a preferred method, polynucleotides encoding the novel amino acid sequences are changed via site-directed mutagenesis. This method uses oligonucleotide sequences to alter a polynucleotide to encode the desired amino acid variant, as well as sufficient adjacent nucleotides on both sides of the changed amino acid to form a stable duplex on either side of the site of being changed. In general, the techniques of site-directed mutagenesis are well known to those of skill in the art and this technique is exemplified by publications such as, Edelman et al., *DNA* 2:183 (1983). A versatile and efficient method for producing site-specific changes in a polynucleotide sequence was published by Zoller and Smith, *Nucleic Acids Res.* 10:6487-6500 (1982). PCR may also be used to create amino acid sequence variants of the novel nucleic acids. When small amounts of template DNA are used as starting material, primer(s) that differs slightly in sequence from the corresponding region in the template DNA can generate the desired amino acid variant. PCR amplification results in a population of product DNA fragments that differ from the polynucleotide template encoding the polypeptide at the position specified by the primer. The product DNA fragments replace the corresponding region in the plasmid and this gives a polynucleotide encoding the desired amino acid variant.

A further technique for generating amino acid variants is the cassette mutagenesis technique described in Wells et al., *Gene* 34:315 (1985); and other mutagenesis techniques well known in the art, such as, for example, the techniques in Sambrook et al., supra, and *Current Protocols in Molecular Biology,* Ausubel et al. Due to the inherent degeneracy of the genetic code, other DNA sequences which encode substantially the same or a functionally equivalent amino acid sequence may be used in the practice of the invention for the cloning and expression of these novel nucleic acids. Such DNA sequences include those which are capable of hybridizing to the appropriate novel nucleic acid sequence under stringent conditions.

Polynucleotides encoding preferred polypeptide truncations of the invention can be used to generate polynucleotides encoding chimeric or fusion proteins comprising one or more domains of the invention and heterologous protein sequences.

The polynucleotides of the invention additionally include the complement of any of the polynucleotides recited above. The polynucleotide can be DNA (genomic, cDNA, amplified, or synthetic) or RNA. Methods and algorithms for obtaining such polynucleotides are well known to those of skill in the art and can include, for example, methods for determining hybridization conditions that can routinely isolate polynucleotides of the desired sequence identities.

In accordance with the invention, polynucleotide sequences comprising the mature protein coding sequences corresponding to any one of SEQ ID NO: 1-146, or 293-438, or functional equivalents thereof, may be used to generate recombinant DNA molecules that direct the expression of that nucleic acid, or a functional equivalent thereof, in appropriate host cells. Also included are the cDNA inserts of any of the clones identified herein.

A polynucleotide according to the invention can be joined to any of a variety of other nucleotide sequences by well-established recombinant DNA techniques (see Sambrook J et al., (1989) Molecular Cloning: A Laboratory Manual, Cold Spring Harbor Laboratory, NY). Useful nucleotide sequences for joining to polynucleotides include an assortment of vectors, e.g., plasmids, cosmids, lambda phage derivatives, phagemids, and the like, that are well known in the art. Accordingly, the invention also provides a vector including a polynucleotide of the invention and a host cell containing the polynucleotide. In general, the vector contains an origin of replication functional in at least one organism, convenient restriction endonuclease sites, and a selectable marker for the host cell. Vectors according to the invention include expression vectors, replication vectors, probe generation vectors, and sequencing vectors. A host cell according to the invention can be a prokaryotic or eukaryotic cell and can be a unicellular organism or part of a multicellular organism.

The present invention further provides recombinant constructs comprising a nucleic acid having any of the nucleotide sequences of SEQ ID NO: 1-146, or 293-438 or a fragment thereof or any other polynucleotides of the invention. In one embodiment, the recombinant constructs of the present invention comprise a vector, such as a plasmid or viral vector, into which a nucleic acid having any of the nucleotide sequences of SEQ ID NO: 1-146, or 293-438 or a fragment thereof is inserted, in a forward or reverse orientation. In the case of a vector comprising one of the ORFs of the present invention, the vector may further comprise regulatory sequences, including for example, a promoter, operably linked to the ORF. Large numbers of suitable vectors and promoters are known to those of skill in the art and are commercially available for generating the recombinant constructs of the present invention. The following vectors are provided by way of example. Bacterial: pBs, phagescript, PsiX174, pBluescript SK, pBs KS, pNH8a, pNH16a, pNH18a, pNH46a (Stratagene); pTrc99A, pKK223-3, pKK233-3, pDR540, pRIT5 (Pharmacia). Eukaryotic: pWLneo, pSV2cat, pOG44, PXTI, pSG (Stratagene) pSVK3, pBPV, pMSG, pSVL (Pharmacia).

The isolated polynucleotide of the invention may be operably linked to an expression control sequence such as the pMT2 or pED expression vectors disclosed in Kaufman et aL, *Nucleic Acids Res*. 19, 4485-4490 (1991), in order to produce the protein recombinantly. Many suitable expression control sequences are known in the art. General methods of expressing recombinant proteins are also known and are exemplified in R. Kaufman, *Methods in Enzymology* 185, 537-566 (1990). As defined herein "operably linked" means that the isolated polynucleotide of the invention and an expression control sequence are situated within a vector or cell in such a way that the protein is expressed by a host cell which has been transformed (transfected) with the ligated polynucleolide/expression control sequence.

Promoter regions can be selected from any desired gene using CAT (chloramphenicol transferase) vectors or other vectors with selectable markers. Two appropriate vectors are pKK232-8 and pCM7. Particular named bacterial promoters include lacI, lacZ, T3, T7, gpt, lambda PR, and trc. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art. Generally, recombinant expression vectors will include origins of replication and selectable markers permitting transformation of the host cell, *e*.*g*., the ampicillin resistance gene of *E*. *coli* and *S. cerevisiae* TRP1 gene, and a promoter derived from a highly-expressed gene to direct transcription of a downstream structural sequence. Such promoters can be derived from operons encoding glycolytic enzymes such as 3-phosphoglycerate kinase (PGK), a-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an amino terminal identification peptide imparting desired characteristics, *e.g*., stabilization or simplified purification of expressed recombinant product. Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include *E. coli, Bacillus subtilis, Salmonella typhimurium* and various species within the genera *Pseudomonas, Streptomyces,* and *Staphylococcus,* although others may also be employed as a matter of choice.

As a representative but non-limiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM 1 (Promega Biotech, Madison, WI, USA). These pBR322 "backbone" sections are combined with an appropriate promoter and the structural sequence to be expressed. Following transformation of a suitable host strain and growth of the host strain to an appropriate cell density, the selected *promoter* is induced or derepressed by appropriate means (e.g., temperature shift or chemical induction) and cells are cultured for an additional period. Cells are typically harvested by centrifugation, disrupted by physical or chemical means, and the resulting crude extract retained for further purification.

Polynucleotides of the invention can also be used to induce immune responses. For example, as described in Fan et al., *Nat. Biotech.* 17:870-872 (1999), incorporated herein by reference, nucleic acid sequences encoding a polypeptide may be used to generate antibodies against the encoded polypeptide following topical administration of naked plasmid DNA or following injection, and preferably intramuscular injection of the DNA. The nucleic acid sequences are preferably inserted in a recombinant expression vector and may be in the form of naked DNA.

### 4.3 ANTISENSE NUCLEIC ACIDS

Another aspect of the invention pertains to isolated antisense nucleic acid molecules that are hybridizable to or complementary to the nucleic acid molecule comprising the nucleotide sequence of SEQ ID NO: 1-146, or 293-438, or fragments, analogs or derivatives thereof. An "antisense" nucleic acid comprises a nucleotide sequence that is complementary to a "sense" nucleic acid encoding a protein, *e*.*g*., complementary to the coding strand of a double-stranded cDNA molecule or complementary to an mRNA sequence. In specific aspects, antisense nucleic acid molecules are provided that comprise a sequence complementary to at least about 10, 25, 50, 100, 250 or 500 nucleotides or an entire coding strand, or to only a portion thereof. Nucleic acid molecules encoding fragments, homologs, derivatives and analogs of a protein of any of SEQ ID NO: 147-292, or 439-584 or antisense nucleic acids complementary to a nucleic acid sequence of SEQ ID NO: 1-146, or 293-438 are additionally provided.

In one embodiment, an antisense nucleic acid molecule is antisense to a "coding region" of the coding strand of a nucleotide sequence of the invention. The term "coding region" refers to the region of the nucleotide sequence comprising codons which are translated into amino acid residues. In another embodiment, the antisense nucleic acid molecule is antisense to a "noncoding region" of the coding strand of a nucleotide sequence of the invention. The term "noncoding region" refers to 5' and 3' sequences which flank the coding region that are not translated into amino acids (*i*.*e*., also referred to as 5' and 3' untranslated regions).

Given the coding strand sequences encoding a nucleic acid disclosed herein (*e.g.*, SEQ ID NO: 1-146, or 293-438), antisense nucleic acids of the invention can be designed according to the rules of Watson and Crick or Hoogsteen base pairing. The antisense nucleic acid molecule can be complementary to the entire coding region of an mRNA, but more preferably is an oligonucleotide that is antisense to only a portion of the coding or noncoding region of a mRNA. For example, the antisense oligonucleotide can be complementary to the region surrounding the translation start site of a mRNA. An antisense oligonucleotide can be, for example, about 5, 10, 15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e*.*g*., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, *e*.*g*., phosphorothioate derivatives and acridine substituted nucleotides can be used.

Examples of modified nucleotides that can be used to generate the antisense nucleic acid include: 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (*i*.*e*., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding a protein according to the invention to.thereby inhibit expression of the protein, *e.g*., by inhibiting transcription and/or translation. The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, *e.g.*, by linking the antisense nucleic acid molecules to peptides or antibodies that bind to cell surface receptors or antigens. The antisense nucleic acid molecules can also be delivered to cells using the vectors described herein. To achieve sufficient intracellular concentrations of antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier *et al*. (1987) *Nucleic Acids Res* 15: 6625-6641). The antisense nucleic acid molecule can also comprise a 2'-o-methylribonucleotide (Inoue *et al*. (1987) *Nucleic Acids Res* 15: 6131-6148) or a chimeric RNA -DNA analogue (Inoue *et al*. (1987) *FEBS Lett* 215: 327-330).

### 4.4 RIBOZYMES AND PNA MOIETIES

In still another embodiment, an antisense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes *(e.g.,* hammerhead ribozymes (described in Haselhoff and Gerlach (1988) *Nature* 334:585-591)) can be used to catalytically cleave a mRNA transcripts to thereby inhibit translation of a mRNA. A ribozyme having specificity for a nucleic acid of the invention can be designed based upon the nucleotide sequence of a DNA disclosed herein (*i.e*., SEQ ID NO: 1-146, or 293-438). For example, a derivative of a Tetrahymena L-19 IVS RNA can be constructed in which the nucleotide sequence of the active site is complementary to the nucleotide sequence to be cleaved in an mRNA of SEQ ID NO: 1-146, or 293-438 (see, *e.g.,* Cech *et al*. U.S. Pat. No. 4,987,071; and Cech *et al*. U.S. Pat. No. 5,116,742). Alternatively, polynucleotides of the invention can be used to select a catalytic RNA having a specific ribonuclease activity from a pool of RNA molecules. See, *e.g.,* Bartel *et al*., (1993) *Science* 261:1411-1418.

Alternatively, gene expression can be inhibited by targeting nucleotide sequences complementary to the regulatory region (*e*.*g*., promoter and/or enhancers) to form triple helical structures that prevent transcription of the gene in target cells. See generally, Helene. (1991) *Anticancer Drug Des.* 6: 569-84; Helene. *et al*. (1992) *Ann. N.Y. Acad. Sci*. 660:27-36; and Maher (1992) *Bioassays* 14: 807-15.

In various embodiments, the nucleic acids of the invention can be modified at the base moiety, sugar moiety or phosphate backbone to improve, *e.g.*, the stability, hybridization, or solubility of the molecule. For example, the deoxyribose phosphate backbone of the nucleic acids can be modified to generate peptide nucleic acids (see Hyrup *et al*. (1996) *Bioorg Med Chem* 4: 5-23). As used herein, the terms "peptide nucleic acids" or "PNAs" refer to nucleic acid mimics, *e.g.*, DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup *et al*. (1996) above; Perry-O'Keefe *et al*. (1996) *PNAS* 93: 14670-675.

PNAs of the invention can be used in therapeutic and diagnostic applications. For example, PNAs can be used as antisense or antigene agents for sequence-specific modulation of gene expression by, *e.g*., inducing transcription or translation arrest or inhibiting replication. PNAs of the invention can also be used, *e.g.,* in the analysis of single base pair mutations in a gene by, *e.g.,* PNA directed PCR clamping; as artificial restriction enzymes when used in combination with other enzymes, *e.g.,* S1 nucleases (Hyrup B. (1996) above); or as probes or primers for DNA sequence and hybridization (Hyrup *et al*. (1996), above; Perry-O'Keefe (1996), above).

In another embodiment, PNAs of the invention can be modified, *e.g*., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras can be generated that may combine the advantageous properties of PNA and DNA. Such chimeras allow DNA recognition enzymes, *e*.*g*., RNase H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup (1996) above). The synthesis of PNA-DNA chimeras can be performed as described in Hyrup (1996) above and Finn *et al*. (1996) *Nucl Acids Res 24:* 3357-63. For example, a DNA chain can be synthesized on a solid support using standard phosphoramidite coupling chemistry, and modified nucleoside analogs, *e.g*., 5'-(4-methoxytrityl)amino-5'-deoxy-thymidine phosphoramidite, can be used between the PNA and the 5' end of DNA (Mag *et al*. (1989) *Nucl Acid Res* 17: 5973-88). PNA monomers are then coupled in a stepwise manner to produce a chimeric molecule with a 5' PNA segment and a 3' DNA segment (Finn *et al*. (1996) above). Alternatively, chimeric molecules can be synthesized with a 5' DNA segment and a 3' PNA segment. See, Petersen *et al*. (1975) *Bioorg Med Chem Lett* 5: 1119-11124.

In other embodiments, the oligonucleotide may include other appended groups such as peptides (*e*.*g*., for targeting host cell receptors *in vivo*), or agents facilitating transport across the cell membrane (see, *e*.*g*., Letsinger *et al*., 1989, *Proc. Natl. Acad Sci. U.S.A*. 86:6553-6556; Lemaitre *et al*., 1987, Proc. *Natl. Acad. Sci*. 84:648-652; PCT Publication No. W088/09810) or the blood-brain barrier (see, *e*.*g*., PCT Publication No. W089/10134). In addition, oligonucleotides can be modified with hybridization triggered cleavage agents (See, *e*.*g*., Krol *et al*., 1988, *BioTechniques* 6:958-976) or intercalating agents. (See, *e.g.*, Zon, 1988, *Pharm. Res.* 5: 539-549). To this end, the oligonucleotide may be conjugated to another molecule, *e*.*g*., a peptide, a hybridization triggered cross-linking agent, a transport agent, a hybridization-triggered cleavage agent, etc.

### 4.5 HOSTS

The present invention further provides host cells genetically engineered to contain the polynucleotides of the invention. For example, such host cells may contain nucleic acids of the invention introduced into the host cell using known transformation, transfection or infection methods. The present invention still further provides host cells genetically engineered to express the polynucleotides of the invention, wherein such polynucleotides are in operative association with a regulatory sequence heterologous to the host cell which drives expression of the polynucleotides in the cell.

Knowledge of nucleic acid sequences allows for modification of cells to permit, or increase, expression of endogenous polypeptide. Cells can be modified (e.g., by homologous recombination) to provide increased polypeptide expression by replacing, in whole or in part, the naturally occurring promoter with all or part of a heterologous promoter so that the cells express the polypeptide at higher levels. The heterologous promoter is inserted in such a manner that it is operatively linked to the encoding sequences. See, for example, PCT International Publication No. WO94/12650, PCT International Publication No. WO92/20808, and PCT International Publication No. WO91/09955. It is also contemplated that, in addition to heterologous promoter DNA, amplifiable marker DNA (e.g., ada, dhfr, and the multifunctional CAD gene which encodes carbamyl phosphate synthase, aspartate transcarbamylase, and dihydroorotase) and/or intron DNA may be inserted along with the heterologous promoter DNA. If linked to the coding sequence, amplification of the marker DNA by standard selection methods results in co-amplification of the desired protein coding sequences in the cells.

The host cell can be a higher eukaryotic host cell, such as a mammalian cell, a lower eukaryotic host cell, such as a yeast cell, or the host cell can be a prokaryotic cell, such as a bacterial cell. Introduction of the recombinant construct into the host cell can be effected by calcium phosphate transfection, DEAE-dextran mediated transfection, or electroporation (Davis, L. et al., *Basic Methods in Molecular Biology* (1986)). The host cells containing one of the polynucleotides of the invention, can be used in conventional manners to produce the gene product encoded by the isolated fragment (in the case of an ORF) or can be used to produce a heterologous protein under the control of the EMF.

Any host/vector system can be used to express one or more of the ORFs of the present invention. These include, but are not limited to, eukaryotic hosts such as HeLa cells, Cv-1 cell, COS cells, 293 cells, and Sf9 cells, as well as prokaryotic host such as *E. coli* and *B. subtilis.* The most preferred cells are those which do not normally express the particular polypeptide or protein or which expresses the polypeptide or protein at low natural level. Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., in Molecular Cloning: A Laboratory Manual, Second Edition, Cold Spring Harbor, New York (1989), the disclosure of which is hereby incorporated by reference.

Various mammalian cell culture systems can also be employed to express recombinant protein. Examples of mammalian expression systems include the COS-7 lines of monkey kidney fibroblasts, described by Gluzman, Cell 23:175 (1981). Other cell lines capable of expressing a compatible vector are, for example, the C127, monkey COS cells, Chinese Hamster Ovary (CHO) cells, human kidney 293 cells, human epidermal A431 cells, human Colo205 cells, 3T3 cells, CV-1 cells, other transformed primate cell lines, normal diploid cells, cell strains derived from *in vitro* culture of primary tissue, primary explants, HeLa cells, mouse L cells, BHK, HL-60, U937, HaK or Jurkat cells. Mammalian expression vectors will comprise an origin of replication, a suitable promoter and also any necessary ribosome binding sites, polyadenylation site, splice donor and acceptor sites, transcriptional termination sequences, and 5' flanking nontranscribed sequences. DNA sequences derived from the SV40 viral genome, for example, SV40 origin, early promoter, enhancer, splice, and polyadenylation sites may be used to provide the required nontranscribed genetic elements. Recombinant polypeptides and proteins produced in bacterial culture are usually isolated by initial extraction from cell pellets, followed by one or more salting-out, aqueous ion exchange or size exclusion chromatography steps. Protein refolding steps can be used, as necessary, in completing configuration of the mature protein. Finally, high performance liquid chromatography (HPLC) can be employed for final purification steps. Microbial cells employed in expression of proteins can be disrupted by any convenient method, including freeze-thaw cycling, sonication, mechanical disruption, or use of cell lysing agents.

Alternatively, it may be possible to produce the protein in lower eukaryotes such as yeast or insects or in prokaryotes such as bacteria. Potentially suitable yeast strains include *Saccharomyces cerevisiae, Schizosaccharomyces pombe, Kluyveromyces* strains, *Candida*, or any yeast strain capable of expressing heterologous proteins. Potentially suitable bacterial strains include *Escherichia coli, Bacillus subtilis, Salmonella typhimurium*, or any bacterial strain capable of expressing heterologous proteins. If the protein is made in yeast or bacteria, it may be necessary to modify the protein produced therein, for example by phosphorylation or glycosylation of the appropriate sites, in order to obtain the functional protein. Such covalent attachments may be accomplished using known chemical or enzymatic methods.

In another embodiment of the present invention, cells and tissues may be engineered to express an endogenous gene comprising the polynucleotides of the invention under the control of inducible regulatory elements, in which case the regulatory sequences of the endogenous gene may be replaced by homologous recombination. As described herein, gene targeting can be used to replace a gene's existing regulatory region with a regulatory sequence isolated from a different gene or a novel regulatory sequence synthesized by genetic engineering methods. Such regulatory sequences may be comprised of promoters, enhancers, scaffold-attachment regions, negative regulatory elements, transcriptional initiation sites, regulatory protein binding sites or combinations of said sequences. Alternatively, sequences which affect the structure or stability of the RNA or protein produced may be replaced, removed, added, or otherwise modified by targeting. These sequence include polyadenylation signals, mRNA stability elements, splice sites, leader sequences for enhancing or modifying transport or secretion properties of the protein, or other sequences which alter or improve the function or stability of protein or RNA molecules.

The targeting event may be a simple insertion of the regulatory sequence, placing the gene under the control of the new regulatory sequence, *e*.*g*., inserting a new promoter or enhancer or both upstream of a gene. Alternatively, the targeting event may be a simple deletion of a regulatory element, such as the deletion of a tissue-specific negative regulatory element. Alternatively, the targeting event may replace an existing element; for example, a tissue-specific enhancer can be replaced by an enhancer that has broader or different cell-type specificity than the naturally occurring elements. Here, the naturally occurring sequences are deleted and new sequences are added. In all cases, the identification of the targeting event may be facilitated by the use of one or more selectable marker genes that are contiguous with the targeting DNA, allowing for the selection of cells in which the exogenous DNA has integrated into the host cell genome. The identification of the targeting event may also be facilitated by the use of one or more marker genes exhibiting the property of negative selection, such that the negatively selectable marker is linked to the exogenous DNA, but configured such that the negatively selectable marker flanks the targeting sequence, and such that a correct homologous recombination event with sequences in the host cell genome does not result in the stable integration of the negatively selectable marker. Markers useful for this purpose include the Herpes Simplex Virus thymidine kinase (TK) gene or the bacterial xanthine-guanine phosphoribosyl-transferase (gpt) gene.

The gene targeting or gene activation techniques which can be used in accordance with this aspect of the invention are more particularly described in U.S. Patent No. 5,272,071 to Chappel; U.S. Patent No. 5,578,461 to Sherwin et al.; International Application No. PCT/US92/09627 (WO93/09222) by Selden et al.; and International Application No. PCT/US90/06436 (WO91/06667) by Skoultchi et al., each of which is incorporated by reference herein in its entirety.

### 4.6 POLYPEPTIDES OF THE INVENTION

The isolated polypeptides of the invention include, but are not limited to, a polypeptide comprising: the amino acid sequences set forth as any one of SEQ ID NO: 147-292, or 439-584 or an amino acid sequence encoded by any one of the nucleotide sequences SEQ ID NO: 1-146, or 293-438 or the corresponding full length or mature protein. Polypeptides of the invention also include polypeptides preferably with biological or immunological activity that are encoded by: (a) a polynucleotide having any one of the nucleotide sequences set forth in SEQ ID NO: 1-146, or 293-438 or (b) polynucleotides encoding any one of the amino acid sequences set forth as SEQ ID NO: 147-292, or 439-584 or (c) polynucleotides that hybridize to the complement of the polynucleotides of either (a) or (b) under stringent hybridization conditions. The invention also provides biologically active or immunologically active variants of any of the amino acid sequences set forth as SEQ ID NO: 147-292, or 439-584 or the corresponding full length or mature protein; and "substantial equivalents" thereof *(e.g.,* with at least about 65%, at least about 70%, at least about 75%, at least about 80%, at least about 85%, 86%, 87%, 88%, 89%, at least about 90%, 91%, 92%, 93%, 94%, typically at least about 95%, 96%, 97%, more typically at least about 98%, or most typically at least about 99% amino acid identity) that retain biological activity. Polypeptides encoded by allelic variants may have a similar, increased, or decreased activity compared to polypeptides comprising SEQ ID NO: 147-292, or 439-584.

Fragments of the proteins of the present invention which are capable of exhibiting biological activity are also encompassed by the present invention. Fragments of the protein may be in linear form or they may be cyclized using known methods, for example, as described in H. U. Saragovi, et al., Bio/Technology 10, 773-778 (1992) and in R. S. McDowell, et al., J. Amer. Chem. Soc. 114, 9245-9253 (1992), both of which are incorporated herein by reference. Such fragments may be fused to carrier molecules such as immunoglobulins for many purposes, including increasing the valency of protein binding sites.

The present invention also provides both full-length and mature forms (for example, without a signal sequence or precursor sequence) of the disclosed proteins. The protein coding sequence is identified in the sequence listing by translation of the disclosed nucleotide sequences. The mature form of such protein may be obtained by expression of a full-length polynucleotide in a suitable mammalian cell or other host cell. The sequence of the mature form of the protein is also determinable from the amino acid sequence of the full-length form. Where proteins of the present invention are membrane bound, soluble forms of the proteins are also provided. In such forms, part or all of the regions causing the proteins to be membrane bound are deleted so that the proteins are fully secreted from the cell in which they are expressed.

Protein compositions of the present invention may further comprise an acceptable carrier, such as a hydrophilic, *e*.*g*., pharmaceutically acceptable, carrier.

The present invention further provides isolated polypeptides encoded by the nucleic acid fragments of the present invention or by degenerate variants of the nucleic acid fragments of the present invention. By "degenerate variant" is intended nucleotide fragments which differ from a nucleic acid fragment of the present invention *(e.g.,* an ORF) by nucleotide sequence but, due to the degeneracy of the genetic code, encode an identical polypeptide sequence. Preferred nucleic acid fragments of the present invention are the ORFs that encode proteins.

A variety of methodologies known in the art can be utilized to obtain any one of the isolated polypeptides or proteins of the present invention. At the simplest level, the amino acid sequence can be synthesized using commercially available peptide synthesizers. The synthetically-constructed protein sequences, by virtue of sharing primary, secondary or tertiary structural and/or conformational characteristics with proteins may possess biological properties in common therewith, including protein activity. This technique is particularly useful in producing small peptides and fragments of larger polypeptides. Fragments are useful, for example, in generating antibodies against the native polypeptide. Thus, they may be employed as biologically active or immunological substitutes for natural, purified proteins in screening of therapeutic compounds and in immunological processes for the development of antibodies.

The polypeptides and proteins of the present invention can alternatively be purified from cells which have been altered to express the desired polypeptide or protein. As used herein, a cell is said to be altered to express a desired polypeptide or protein when the cell, through genetic manipulation, is made to produce a polypeptide or protein which it normally does not produce or which the cell normally produces at a lower level. One skilled in the art can readily adapt procedures for introducing and expressing either recombinant or synthetic sequences into eukaryotic or prokaryotic cells in order to generate a cell which produces one of the polypeptides or proteins of the present invention.

The invention also relates to methods for producing a polypeptide comprising growing a culture of host cells of the invention in a suitable culture medium, and purifying the protein from the cells or the culture in which the cells are grown. For example, the methods of the invention include a process for producing a polypeptide in which a host cell containing a suitable expression vector that includes a polynucleotide of the invention is cultured under conditions that allow expression of the encoded polypeptide. The polypeptide can be recovered from the culture, conveniently from the culture medium, or from a lysate prepared from the host cells and further purified. Preferred embodiments include those in which the protein produced by such process is a full length or mature form of the protein.

In an alternative method, the polypeptide or protein is purified from bacterial cells which naturally produce the polypeptide or protein. One skilled in the art can readily follow known methods for isolating polypeptides and proteins in order to obtain one of the isolated polypeptides or proteins of the present invention. These include, but are not limited to, immunochromatography, HPLC, size-exclusion chromatography, ion-exchange chromatography, and immuno-affinity chromatography. See, *e*.*g*., Scopes, *Protein Purification: Principles and Practice,* Springer-Verlag (1994); Sambrook, et al., in Molecular Cloning: *A Laboratory Manual*; Ausubel et al., *Current Protocols in Molecular Biology.* Polypeptide fragments that retain biological/immunological activity include fragments comprising greater than about 100 amino acids, or greater than about 200 amino acids, and fragments that encode specific protein domains.

The purified polypeptides can be used in *in vitro* binding assays which are well known in the art to identify molecules which bind to the polypeptides. These molecules include but are not limited to, for e.g., small molecules, molecules from combinatorial libraries, antibodies or other proteins. The molecules identified in the binding assay are then tested for antagonist or agonist activity in *in vivo* tissue culture or animal models that are well known in the art. In brief, the molecules are titrated into a plurality of cell cultures or animals and then tested for either cell/animal death or prolonged survival of the animal/cells.

In addition, the peptides of the invention or molecules capable of binding to the peptides may be complexed with toxins, e.g., ricin or cholera, or with other compounds that are toxic to cells. The toxin-binding molecule complex is then targeted to a tumor or other cell by the specificity of the binding molecule for SEQ ID NO: 147-292, or 439-584.

The protein of the invention may also be expressed as a product of transgenic animals, e.g., as a component of the milk of transgenic cows, goats, pigs, or sheep which are characterized by somatic or germ cells containing a nucleotide sequence encoding the protein.

The proteins provided herein also include proteins characterized by amino acid sequences similar to those of purified proteins but into which modification are naturally provided or deliberately engineered. For example, modifications, in the peptide or DNA sequence, can be made by those skilled in the art using known techniques. Modifications of interest in the protein sequences may include the alteration, substitution, replacement, insertion or deletion of a selected amino acid residue in the coding sequence. For example, one or more of the cysteine residues may be deleted or replaced with another amino acid to alter the conformation of the molecule. Techniques for such alteration, substitution, replacement, insertion or deletion are well known to those skilled in the art (see, *e.g.*, U.S. Pat. No. 4,518,584). Preferably, such alteration, substitution, replacement, insertion or deletion retains the desired activity of the protein. Regions of the protein that are important for the protein function can be determined by various methods known in the art including the alanine-scanning method which involved systematic substitution of single or strings of amino acids with alanine, followed by testing the resulting alanine-containing variant for biological activity. This type of analysis determines the importance of the substituted amino acid(s) in biological activity. Regions of the protein that are important for protein function may be determined by the eMATRIX program.

Other fragments and derivatives of the sequences of proteins which would be expected to retain protein activity in whole or in part and are useful for screening or other immunological methodologies may also be easily made by those skilled in the art given the disclosures herein. Such modifications are encompassed by the present invention.

The protein may also be produced by operably linking the isolated polynucleotide of the invention to suitable control sequences in one or more insect expression vectors, and employing an insect expression system. Materials and methods for baculovirus/insect cell expression systems are commercially available in kit form from, *e.g*., Invitrogen, San Diego, Calif., U.S.A. (the MaxBat™ kit), and such methods are well known in the art, as described in Summers and Smith, Texas Agricultural Experiment Station Bulletin No. 1555 (1987), incorporated herein by reference. As used herein, an insect cell capable of expressing a polynucleotide of the present invention is "transformed."

The protein of the invention may be prepared by culturing transformed host cells under culture conditions suitable to express the recombinant protein. The resulting expressed protein may then be purified from such culture (*i*.*e*., from culture medium or cell extracts) using known purification processes, such as gel filtration and ion exchange chromatography. The purification of the protein may also include an affinity column containing agents which will bind to the protein; one or more column steps over such affinity resins as concanavalin A-agarose, heparin-toyopearl™ or Cibacrom blue 3GA Sepharose™; one or more steps involving hydrophobic interaction chromatography using such resins as phenyl ether, butyl ether, or propyl ether; or immunoaffinity chromatography.

Alternatively, the protein of the invention may also be expressed in a form which will facilitate purification. For example, it may be expressed as a fusion protein, such as those of maltose binding protein (MBP), glutathione-S-transferase (GST) or thioredoxin (TRX), or as a His tag. Kits for expression and purification of such fusion proteins are commercially available from New England BioLab (Beverly, Mass.), Pharmacia (Piscataway, N.J.) and Invitrogen, respectively. The protein can also be tagged with an epitope and subsequently purified by using a specific antibody directed to such epitope. One such epitope ("FLAG®") is commercially available from Kodak (New Haven, Conn.).

Finally, one or more reverse-phase high performance liquid chromatography (RP- HPLC) steps employing hydrophobic RP-HPLC media, *e.g.*, silica gel having pendant methyl or other aliphatic groups, can be employed to further purify the protein. Some or all of the foregoing purification steps, in various combinations, can also be employed to provide a substantially homogeneous isolated recombinant protein. The protein thus purified is substantially free of other mammalian proteins and is defined in accordance with the present invention as an "isolated protein."

The polypeptides of the invention include analogs (variants). This embraces fragments, as well as peptides in which one or more amino acids has been deleted, inserted, or substituted. Also, analogs of the polypeptides of the invention embrace fusions of the polypeptides or modifications of the polypeptides of the invention, wherein the polypeptide or analog is fused to another moiety or moieties, e.g., targeting moiety or another therapeutic agent. Such analogs may exhibit improved properties such as activity and/or stability. Examples of moieties which may be fused to the polypeptide or an analog include, for example, targeting moieties which provide for the delivery of polypeptide to pancreatic cells, e.g., antibodies to pancreatic cells, antibodies to immune cells such as T-cells, monocytes, dendritic cells, granulocytes, etc., as well as receptor and ligands expressed on pancreatic or immune cells. Other moieties which may be fused to the polypeptide include therapeutic agents which are used for treatment, for example, immunosuppressive drugs such as cyclosporin, SK506, azathioprine, CD3 antibodies and steroids. Also, polypeptides may be fused to immune modulators, and other cytokines such as alpha or beta interferon.

### 4.6.1 DETERMINING POLYPEPTIDE AND POLYNUCLEOTIDE IDENTITY AND SIMILARITY

Preferred identity and/or similarity are designed to give the largest match between the sequences tested. Methods to determine identity and similarity are codified in computer programs including, but are not limited to, the GCG program package, including GAP (Devereux, J., et al., Nucleic Acids Research 12(1):387 (1984); Genetics Computer Group, University of Wisconsin, Madison, WI), BLASTP, BLASTN, BLASTX, FASTA (Altschul, S.F. et al., J. Molec. Biol. 215:403-410 (1990), PSI-BLAST (Altschul S.F. et al., Nucleic Acids Res. vol. 25, pp. 3389-3402, herein incorporated by reference), eMatrix software (Wu et al., J. Comp. Biol., Vol. 6, pp. 219-235 (1999), herein incorporated by reference), eMotif software (Nevill-Manning et al, ISMB-97, Vol. 4, pp. 202-209, herein incorporated by reference), pFam software (Sonnhammer et al., Nucleic Acids Res., Vol. 26(1), pp. 320-322 (1998), herein incorporated by reference) and the Kyte-Doolittle hydrophobocity prediction algorithm (J. Mol Biol, 157, pp. 105-31 (1982), incorporated herein by reference). The BLAST programs are publicly available from the National Center for Biotechnology Information (NCBI) and other sources (BLAST Manual, Altschul, S., et al. NCB NLM NIH Bethesda, MD 20894; Altschul, S., et al., J. Mol. Biol. 215:403-410 (1990).

### 4.7 CHIMERIC AND FUSION PROTEINS

The invention also provides chimeric or fusion proteins. As used herein, a "chimeric protein" or "fusion protein" comprises a polypeptide of the invention operatively linked to another polypeptide. Within a fusion protein the polypeptide according to the invention can correspond to all or a portion of a protein according to the invention. In one embodiment, a fusion protein comprises at least one biologically active portion of a protein according to the invention. In another embodiment, a fusion protein comprises at least two biologically active portions of a protein according to the invention. Within the fusion protein, the term "operatively linked" is intended to indicate that the polypeptide according to the invention and the other polypeptide are fused in-frame to each other. The polypeptide can be fused to the N-terminus or C-terminus.

For example, in one embodiment a fusion protein comprises a polypeptide according to the invention operably linked to the extracellular domain of a second protein. In another embodiment, the fusion protein is a GST-fusion protein in which the polypeptide sequences of the invention are fused to the C-terminus of the GST (i.e., glutathione S-transferase) sequences.

In another embodiment, the fusion protein is an immunoglobulin fusion protein in which the polypeptide sequences according to the invention comprise one or more domains fused to sequences derived from a member of the immunoglobulin protein family. The immunoglobulin fusion proteins of the invention can be incorporated into pharmaceutical compositions and administered to a subject to inhibit an interaction between a ligand and a protein of the invention on the surface of a cell, to thereby suppress signal transduction *in vivo*. The immunoglobulin fusion proteins can be used to affect the bioavailability of a cognate ligand. Inhibition of the ligand/protein interaction may be useful therapeutically for both the treatment of proliferative and differentiative disorders, *e*,*g*., cancer as well as modulating *(e.g.,* promoting or inhibiting) cell survival. Moreover, the immunoglobulin fusion proteins of the invention can be used as immunogens to produce antibodies in a subject, to purify ligands, and in screening assays to identify molecules that inhibit the interaction of a polypeptide of the invention with a ligand.

A chimeric or fusion protein of the invention can be produced by standard recombinant DNA techniques. For example, DNA fragments coding for the different polypeptide sequences are ligated together in-frame in accordance with conventional techniques, *e.g.*, by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence (see, for example, Ausubel et al. (eds.) CURRENT PROTOCOLS IN MOLECULAR BIOLOGY, John Wiley & Sons, 1992). Moreover, many expression vectors are commercially available that already encode a fusion moiety (*e.g*., a GST polypeptide). A nucleic acid encoding a polypeptide of the invention can be cloned into such an expression vector such that the fusion moiety is linked in-frame to the protein of the invention.

### 4.8 GENE THERAPY

Mutations in the polynucleotides of the invention may result in loss of normal function of the encoded protein. The invention thus provides gene therapy to restore normal activity of the polypeptides of the invention; or to treat disease states involving polypeptides of the invention. Delivery of a functional gene encoding polypeptides of the invention to appropriate cells is effected *ex vivo*, *in situ*, or *in vivo* by use of vectors, and more particularly viral vectors (e.g., adenovirus, adeno-associated virus, or a retrovirus), or *ex vivo* by use of physical DNA transfer methods (e.g., liposomes or chemical treatments). See, for example, Anderson, Nature, supplement to vol. 392, no. 6679, pp.25-20 (1998). For additional reviews of gene therapy technology see Friedmann, Science, 244: 1275-1281 (1989); Verma, Scientific American: 68-84 (1990); and Miller, Nature, 357: 455-460 (1992). Introduction of any one of the nucleotides of the present invention or a gene encoding the polypeptides of the present invention can also be accomplished with extrachromosomal substrates (transient expression) or artificial chromosomes (stable expression). Cells may also be cultured *ex vivo* in the presence of proteins of the present invention in order to proliferate or to produce a desired effect on or activity in such cells. Treated cells can then be introduced *in vivo* for therapeutic purposes. Alternatively, it is contemplated that in other human disease states, preventing the expression of or inhibiting the activity of polypeptides of the invention will be useful in treating the disease states. It is contemplated that antisense therapy or gene therapy could be applied to negatively regulate the expression of polypeptides of the invention.

Other methods inhibiting expression of a protein include the introduction of antisense molecules to the nucleic acids of the present invention, their complements, or their translated RNA sequences, by methods known in the art. Further, the polypeptides of the present invention can be inhibited by using targeted deletion methods, or the insertion of a negative regulatory element such as a silencer, which is tissue specific.

The present invention still further provides cells genetically engineered *in vivo* to express the polynucleotides of the invention, wherein such polynucleotides are in operative association with a regulatory sequence heterologous to the host cell which drives expression of the polynucleotides in the cell. These methods can be used to increase or decrease the expression of the polynucleotides of the present invention.

Knowledge of DNA sequences provided by the invention allows for modification of cells to permit, increase, or decrease, expression of endogenous polypeptide. Cells can be modified (e.g., by homologous recombination) to provide increased polypeptide expression by replacing, in whole or in part, the naturally occurring promoter with all or part of a heterologous promoter so that the cells express the protein at higher levels. The heterologous promoter is inserted in such a manner that it is operatively linked to the desired protein encoding sequences. See, for example, PCT International Publication No. WO 94/12650, PCT International Publication No. WO 92/20808, and PCT International PublicationNo. WO 91/09955. It is also contemplated that, in addition to heterologous promoter DNA, amplifiable marker DNA (e.g., ada, dhfr, and the multifunctional CAD gene which encodes carbamyl phosphate synthase, aspartate transcarbamylase, and dihydroorotase) and/or intron DNA may be inserted along with the heterologous promoter DNA. If linked to the desired protein coding sequence, amplification of the marker DNA by standard selection methods results in co-amplification of the desired protein coding sequences in the cells.

In another embodiment of the present invention, cells and tissues may be engineered to express an endogenous gene comprising the polynucleotides of the invention under the control of inducible regulatory elements, in which case the regulatory sequences of the endogenous gene may be replaced by homologous recombination. As described herein, gene targeting can be used to replace a gene's existing regulatory region with a regulatory sequence isolated from a different gene or a novel regulatory sequence synthesized by genetic engineering methods. Such regulatory sequences may be comprised of promoters, enhancers, scaffold-attachment regions, negative regulatory elements, transcriptional initiation sites, regulatory protein binding sites or combinations of said sequences. Alternatively, sequences which affect the structure or stability of the RNA or protein produced may be replaced, removed, added, or otherwise modified by targeting. These sequences include polyadenylation signals, mRNA stability elements, splice sites, leader sequences for enhancing or modifying transport or secretion properties of the protein, or other sequences which alter or improve the function or stability of protein or RNA molecules.

The targeting event may be a simple insertion of the regulatory sequence, placing the gene under the control of the new regulatory sequence, e.g., inserting a new promoter or enhancer or both upstream of a gene. Alternatively, the targeting event may be a simple deletion of a regulatory element, such as the deletion of a tissue-specific negative regulatory element. Alternatively, the targeting event may replace an existing element; for example, a tissue-specific enhancer can be replaced by an enhancer that has broader or different cell-type specificity than the naturally occurring elements. Here, the naturally occurring sequences are deleted and new sequences are added. In all cases, the identification of the targeting event may be facilitated by the use of one or more selectable marker genes that are contiguous with the targeting DNA, allowing for the selection of cells in which the exogenous DNA has integrated into the cell genome. The identification of the targeting event may also be facilitated by the use of one or more marker genes exhibiting the property of negative selection, such that the negatively selectable marker is linked to the exogenous DNA, but configured such that the negatively selectable marker flanks the targeting sequence, and such that a correct homologous recombination event with sequences in the host cell genome does not result in the stable integration of the negatively selectable marker. Markers useful for this purpose include the Herpes Simplex Virus thymidine kinase (TK) gene or the bacterial xanthine-guanine phosphoribosyl-transferase(gpt) gene.

The gene targeting or gene activation techniques which can be used in accordance with this aspect of the invention are more particularly described in U.S. Patent No. 5,272,071 to Chappel; U.S. Patent No. 5,578,461 to Sherwin et al.; International Application No. PCT/US92/09627 (WO93/09222) by Selden et al.; and International Application No. PCT/US90/06436 (WO91/06667) by Skoultchi et al., each of which is incorporated by reference herein in its entirety.

### 4.9 TRANSGENIC ANIMALS

In preferred methods to determine biological functions of the polypeptides of the invention in vivo, one or more genes provided by the invention are either over expressed or inactivated in the germ line of animals using homologous recombination [Capecchi, Science 244:1288-1292 (1989)]. Animals in which the gene is over expressed, under the regulatory control of exogenous or endogenous promoter elements, are known as transgenic animals. Animals in which an endogenous gene has been inactivated by homologous recombination are referred to as "knockout" animals. Knockout animals, preferably non-human mammals, can be prepared as described in U.S. Patent No. 5,557,032, incorporated herein by reference. Transgenic animals are useful to determine the roles polypeptides of the invention play in biological processes, and preferably in disease states. Transgenic animals are useful as model systems to identify compounds that modulate lipid metabolism. Transgenic animals, preferably non-human mammals, are produced using methods as described in U.S. Patent No 5,489,743 and PCT Publication No. WO94/28122, incorporated herein by reference.

Transgenic animals can be prepared wherein all or part of a promoter of the polynucleotides of the invention is either activated or inactivated to alter the level of expression of the polypeptides of the invention. Inactivation can be carried out using homologous recombination methods described above. Activation can be achieved by supplementing or even replacing the homologous promoter to provide for increased protein expression. The homologous promoter can be supplemented by insertion of one or more heterologous enhancer elements known to confer promoter activation in a particular tissue.

The polynucleotides of the present invention also make possible the development, through, e.g., homologous recombination or knock out strategies, of animals that fail to express polypeptides of the invention or that express a variant polypeptide. Such animals are useful as models for studying the *in vivo* activities of polypeptide as well as for studying modulators of the polypeptides of the invention.

In preferred methods to determine biological functions of the polypeptides of the invention *in vivo*, one or more genes provided by the invention are either over expressed or inactivated in the germ line of animals using homologous recombination [Capecchi, Science 244:1288-1292 (1989)]. Animals in which the gene is over expressed, under the regulatory control of exogenous or endogenous promoter elements, are known as transgenic animals. Animals in which an endogenous gene has been inactivated by homologous recombination are referred to as "knockout" animals. Knockout animals, preferably non-human mammals, can be prepared as described in U.S. Patent No. 5,557,032, incorporated herein by reference. Transgenic animals are useful to determine the roles polypeptides of the invention play in biological processes, and preferably in disease states. Transgenic animals are useful as model systems to identify compounds that modulate lipid metabolism. Transgenic animals, preferably non-human mammals, are produced using methods as described in U.S. Patent No 5,489,743 and PCT Publication No. WO94/28122, incorporated herein by reference.

Transgenic animals can be prepared wherein all or part of the polynucleotides of the invention promoter is either activated or inactivated to alter the level of expression of the polypeptides of the invention. Inactivation can be carried out using homologous recombination methods described above. Activation can be achieved by supplementing or even replacing the homologous promoter to provide for increased protein expression. The homologous promoter can be supplemented by insertion of one or more heterologous enhancer elements known to confer promoter activation in a particular tissue.

### 4.10 USES AND BIOLOGICAL ACTIVITY

The polynucleotides and proteins of the present invention are expected to exhibit one or more of the uses or biological activities (including those associated with assays cited herein) identified herein. Uses or activities described for proteins of the present invention may be provided by administration or use of such proteins or of polynucleotides encoding such proteins (such as, for example, in gene therapies or vectors suitable for introduction of DNA). The mechanism underlying the particular condition or pathology will dictate whether the polypeptides of the invention, the polynucleotides of the invention or modulators (activators or inhibitors) thereof would be beneficial to the subject in need of treatment. Thus, "therapeutic compositions of the invention" include compositions comprising isolated polynucleotides (including recombinant DNA molecules, cloned genes and degenerate variants thereof) or polypeptides of the invention (including full length protein, mature protein and truncations or domains thereof), or compounds and other substances that modulate the overall activity of the target gene products, either at the level of target gene/protein expression or target protein activity. Such modulators include polypeptides, analogs, (variants), including fragments and fusion proteins, antibodies and other binding proteins; chemical compounds that directly or indirectly activate or inhibit the polypeptides of the invention (identified, e.g., via drug screening assays as described herein); antisense polynucleotides and polynucleotides suitable for triple helix formation; and in particular antibodies or other binding partners that specifically recognize one or more epitopes of the polypeptides of the invention.

The polypeptides of the present invention may likewise be involved in cellular activation or in one of the other physiological pathways described herein.

### 4.10.1 RESEARCH USES AND UTILITIES

The polynucleotides provided by the present invention can be used by the research community for various purposes. The polynucleotides can be used to express recombinant protein for analysis, characterization or therapeutic use; as markers for tissues in which the corresponding protein is preferentially expressed (either constitutively or at a particular stage of tissue differentiation or development or in disease states); as molecular weight markers on gels; as chromosome markers or tags (when labeled) to identify chromosomes or to map related gene positions; to compare with endogenous DNA sequences in patients to identify potential genetic disorders; as probes to hybridize and thus discover novel, related DNA sequences; as a source of information to derive PCR primers for genetic fingerprinting; as a probe to "subtract-out" known sequences in the process of discovering other novel polynucleotides; for selecting and making oligomers for attachment to a "gene chip" or other support, including for examination of expression patterns; to raise anti-protein antibodies using DNA immunization techniques; and as an antigen to raise anti-DNA antibodies or elicit another immune response. Where the polynucleotide encodes a protein which binds or potentially binds to another protein (such as, for example, in a receptor-ligand interaction), the polynucleotide can also be used in interaction trap assays (such as, for example, that described in Gyuris et al., Cell 75:791-803 (1993)) to identify polynucleotides encoding the other protein with which binding occurs or to identify inhibitors of the binding interaction.

The polypeptides provided by the present invention can similarly be used in assays to determine biological activity, including in a panel of multiple proteins for high-throughput screening; to raise antibodies or to elicit another immune response; as a reagent (including the labeled reagent) in assays designed to quantitatively determine levels of the protein (or its receptor) in biological fluids; as markers for tissues in which the corresponding polypeptide is preferentially expressed (either constitutively or at a particular stage of tissue differentiation or development or in a disease state); and, of course, to isolate correlative receptors or ligands. Proteins involved in these binding interactions can also be used to screen for peptide or small molecule inhibitors or agonists of the binding interaction.

Any or all of these research utilities are capable of being developed into reagent grade or kit format for commercialization as research products.

Methods for performing the uses listed above are well known to those skilled in the art. References disclosing such methods include without limitation "Molecular Cloning: A Laboratory Manual", 2d ed., Cold Spring Harbor Laboratory Press, Sambrook, J., E. F. Fritsch and T. Maniatis eds., 1989, and "Methods in Enzymology: Guide to Molecular Cloning Techniques", Academic Press, Berger, S. L. and A. R. Kimmel eds., 1987.

### 4.10.2 NUTRITIONAL USES

Polynucleotides and polypeptides of the present invention can also be used as nutritional sources or supplements. Such uses include without limitation use as a protein or amino acid supplement, use as a carbon source, use as a nitrogen source and use as a source of carbohydrate. In such cases the polypeptide or polynucleotide of the invention can be added to the feed of a particular organism or can be administered as a separate solid or liquid preparation, such as in the form of powder, pills, solutions, suspensions or capsules. In the case of microorganisms, the polypeptide or polynucleotide of the invention can be added to the medium in or on which the microorganism is cultured.

### 4.10.3 CYTOKINE AND CELL PROLIFERATION/DIFFERENTIATION ACTIVITY

A polypeptide of the present invention may exhibit activity relating to cytokine, cell proliferation (either inducing or inhibiting) or cell differentiation (either inducing or inhibiting) activity or may induce production of other cytokines in certain cell populations. A polynucleotide of the invention can encode a polypeptide exhibiting such attributes. Many protein factors discovered to date, including all known cytokines, have exhibited activity in one or more factor-dependent cell proliferation assays, and hence the assays serve as a convenient confirmation of cytokine activity. The activity of therapeutic compositions of the present invention is evidenced by any one of a number of routine factor dependent cell proliferation assays for cell lines including, without limitation, 32D, DA2, DA1G, T10, B9, B9/11, BaF3, MC9/G, M+(preB M+), 2E8, RB5, DA1, 123, T1165, HT2, CTLL2, TF-1, Mo7e, CMK, HUVEC, and Caco. Therapeutic compositions of the invention can be used in the following:

Assays for T-cell or thymocyte proliferation include without limitation those described in: Current Protocols in Immunology, Ed by J. E. Coligan, A. M. Kruisbeek, D. H. Margulies, E. M. Shevach, W. Strober, Pub. Greene Publishing Associates and Wiley-Interscience (Chapter 3, *In Vitro* assays for Mouse Lymphocyte Function 3.1-3.19; Chapter 7, Immunologic studies in Humans); Takai et al., J. Immunol. 137:3494-3500, 1986; Bertagnolli et al., J. Immunol. 145:1706-1712, 1990; Bertagnolli et al., Cellular Immunology 133:327-341, 1991; Bertagnolli, et al., I. Immunol. 149:3778-3783, 1992; Bowman et al., I. Immunol. 152:1756-1761, 1994.

Assays for cytokine production and/or proliferation of spleen cells, lymph node cells or thymocytes include, without limitation, those described in: Polyclonal T cell stimulation, Kruisbeek, A. M. and Shevach, E. M. In Current Protocols in Immunology. J. E. e.a. Coligan eds. Vol 1 pp. 3.12.1-3.12.14, John Wiley and Sons, Toronto. 1994; and Measurement of mouse and human interleukin-γ, Schreiber, R. D. In Current Protocols in Immunology. J. E. e.a. Coligan eds. Vol I pp. 6.8.1-6.8.8, John Wiley and Sons, Toronto. 1994.

Assays for proliferation and differentiation of hematopoietic and lymphopoietic cells include, without limitation, those described in: Measurement of Human and Murine Interleukin 2 and Interleukin 4, Bottomly, K., Davis, L. S. and Lipsky, P. E. In Current Protocols in Immunology. J. E. e.a. Coligan eds. Vol 1 pp. 6.3.1-6.3.12, John Wiley and Sons, Toronto. 1991; deVries et al., J. Exp. Med. 173:1205-1211, 1991; Moreau et al., Nature 336:690-692, 1988; Greenberger et al., Proc. Natl. Acad. Sci. U.S.A. 80:2931-2938,1983; Measurement of mouse and human interleukin 6-Nordan, R. In Current Protocols in Immunology. J. E. Coligan eds. Vol 1 pp. 6.6.1-6.6.5, John Wiley and Sons, Toronto. 1991; Smith et al., Proc. Natl. Aced. Sci. U.S.A. 83:1857-1861, 1986; Measurement of human Interleukin 11-Bennett, F., Giannotti, J., Clark, S. C. and Turner, K. J. In Current Protocols in Immunology. J. E. Coligan eds. Vol 1 pp. 6.15.1 John Wiley and Sons, Toronto. 1991; Measurement of mouse and human Interleukin 9--Ciarletta, A., Giannotti, J., Clark, S. C. and Turner, K. J. In Current Protocols in Immunology. J. E. Coligan eds. Vol I pp. 6.13.1, John Wiley and Sons, Toronto. 1991.

Assays for T-cell clone responses to antigens (which will identify, among others, proteins that affect APC-T cell interactions as well as direct T-cell effects by measuring proliferation and cytokine production) include, without limitation, those described in: Current Protocols in Immunology, Ed by J. E. Coligan, A. M. Kruisbeek, D. H. Margulies, E. M. Shevach, W Strober, Pub. Greene Publishing Associates and Wiley-Interscience (Chapter *3, In Vitro* assays for Mouse Lymphocyte Function; Chapter 6, Cytokines and their cellular receptors; Chapter 7, Immunologic studies in Humans); Weinberger et al., Proc. Natl. Acad. Sci. USA 77:6091-6095, 1980; Weinberger et al., Eur. J. Immun. 11:405-411, 1981; Takai et al., J. Immunol. 137:3494-3500, 1986; Takai et al., J. Immunol. 140:508-512, 1988.

### 4.10.4 STEM CELL GROWTH FACTOR ACTIVITY

A polypeptide of the present invention may exhibit stem cell growth factor activity and be involved in the proliferation, differentiation and survival of pluripotent and totipotent stem cells including primordial germ cells, embryonic stem cells, hematopoietic stem cells and/or germ line stem cells. Administration of the polypeptide of the invention to stem cells *in vivo* or *ex vivo* is expected to maintain and expand cell populations in a totipotential or pluripotential state which would be useful for re-engineering damaged or diseased tissues, transplantation, manufacture of bio-pharmaceuticals and the development of bio-sensors. The ability to produce large quantities of human cells has important working applications for the production of human proteins which currently must be obtained from non-human sources or donors, implantation of cells to treat diseases such as Parkinson's, Alzheimer's and other neurodegenerative diseases; tissues for grafting such as bone marrow, skin, cartilage, tendons, bone, muscle (including cardiac muscle), blood vessels, cornea, neural cells, gastrointestinal cells and others; and organs for transplantation such as kidney, liver, pancreas (including islet cells), heart and lung.

It is contemplated that multiple different exogenous growth factors and/or cytokines may be administered in combination with the polypeptide of the invention to achieve the desired effect, including any of the growth factors listed herein, other stem cell maintenance factors, and specifically including stem cell factor (SCF), leukemia inhibitory factor (LIF), Flt-3 ligand (Flt-3L), any of the interleukins, recombinant soluble EL-6 receptor fused to IL-6, macrophage inflammatory protein 1-alpha (MIP-1-alpha), G-CSF, GM-CSF, thrombopoietin (TPO), platelet factor 4 (PF-4), platelet-derived growth factor (PDGF), neural growth factors and basic fibroblast growth factor (bFGF).

Since totipotent stem cells can give rise to virtually any mature cell type, expansion of these cells in culture will facilitate the production of large quantities of mature cells. Techniques for culturing stem cells are known in the art and administration of polypeptides of the invention, optionally with other growth factors and/or cytokines, is expected to enhance the survival and proliferation of the stem cell populations. This can be accomplished by direct administration of the polypeptide of the invention to the culture medium. Alternatively, stroma cells transfected with a polynucleotide that encodes for the polypeptide of the invention can be used as a feeder layer for the stem cell populations in culture or in vivo. Stromal support cells for feeder layers may include embryonic bone marrow fibroblasts, bone marrow stromal cells, fetal liver cells, or cultured embryonic fibroblasts (see U.S. Patent No. 5,690,926).

Stem cells themselves can be transfected with a polynucleotide of the invention to induce autocrine expression of the polypeptide of the invention. This will allow for generation of undifferentiated totipotential/pluripotential stem cell lines that are useful as is or that can then be differentiated into the desired mature cell types. These stable cell lines can also serve as a source of undifferentiated totipotential/pluripotential mRNA to create cDNA libraries and templates for polymerase chain reaction experiments. These studies would allow for the isolation and identification of differentially expressed genes in stem cell populations that regulate stem cell proliferation and/or maintenance.

Expansion and maintenance of totipotent stem cell populations will be useful in the treatment of many pathological conditions. For example, polypeptides of the present invention may be used to manipulate stem cells in culture to give rise to neuroepithelial cells that can be used to augment or replace cells damaged by illness, autoimmune disease, accidental damage or genetic disorders. The polypeptide of the invention may be useful for inducing the proliferation of neural cells and for the regeneration of nerve and brain tissue, i.e. for the treatment of central and peripheral nervous system diseases and neuropathies, as well as mechanical and traumatic disorders which involve degeneration, death or trauma to neural cells or nerve tissue. In addition, the expanded stem cell populations can also be genetically altered for gene therapy purposes and to decrease host rejection of replacement tissues after grafting or implantation.

Expression of the polypeptide of the invention and its effect on stem cells can also be manipulated to achieve controlled differentiation of the stem cells into more differentiated cell types. A broadly applicable method of obtaining pure populations of a specific differentiated cell type from undifferentiated stem cell populations involves the use of a cell-type specific promoter driving a selectable marker. The selectable marker allows only cells of the desired type to survive. For example, stem cells can be induced to differentiate into cardiomyocytes (Wobus et al., Differentiation, 48: 173-182, (1991); Klug et al., J. Clin. Invest., 98(1): 216-224, (1998)) or skeletal muscle cells (Browder, L. W. In: *Principles of Tissue Engineering eds.* Lanza et al., Academic Press (1997)). Alternatively, directed differentiation of stem cells can be accomplished by culturing the stem cells in the presence of a differentiation factor such as retinoic acid and an antagonist of the polypeptide of the invention which would inhibit the effects of endogenous stem cell factor activity and allow differentiation to proceed.

*In vitro* cultures of stem cells can be used to determine if the polypeptide of the invention exhibits stem cell growth factor activity. Stem cells are isolated from any one of various cell sources (including hematopoietic stem cells and embryonic stem cells) and cultured on a feeder layer, as described by Thompson et al. Proc. Natl. Acad. Sci, U.S.A., 92: 7844-7848 (1995), in the presence of the polypeptide of the invention alone or in combination with other growth factors or cytokines. The ability of the polypeptide of the invention to induce stem cells proliferation is determined by colony formation on semi-solid support e.g. as described by Bernstein et al., Blood, 77: 2316-2321 (1991).

### 4.10.5 HEMATOPOIESIS REGULATING ACTIVITY

A polypeptide of the present invention may be involved in regulation of hematopoiesis and, consequently, in the treatment of myeloid or lymphoid cell disorders. Even marginal biological activity in support of colony forming cells or of factor-dependent cell lines.indicates involvement in regulating hematopoiesis, e.g. in supporting the growth and proliferation of erythroid progenitor cells alone or in combination with other cytokines, thereby indicating utility, for example, in treating various anemias or for use in conjunction with irradiation/chemotherapy to stimulate the production of erythroid precursors and/or erythroid cells; in supporting the growth and proliferation of myeloid cells such as granulocytes and monocytes/macrophages (i.e., traditional CSF activity) useful, for example, in conjunction with chemotherapy to prevent or treat consequent myelo-suppression; in supporting the growth and proliferation of megakaryocytes and consequently of platelets thereby allowing prevention or treatment of various platelet disorders such as thrombocytopenia, and generally for use in place of or complimentary to platelet transfusions; and/or in supporting the growth and proliferation of hematopoietic stem cells which are capable of maturing to any and all of the above-mentioned hematopoietic cells and therefore find therapeutic utility in various stem cell disorders (such as those usually treated with transplantation, including, without limitation, aplastic anemia and paroxysmal nocturnal hemoglobinuria), as well as in repopulating the stem cell compartment post irradiation/chemotherapy, either *in-vivo* or *ex-vivo* (i.e., in conjunction with bone marrow transplantation or with peripheral progenitor cell transplantation (homologous or heterologous)) as normal cells or genetically manipulated for gene therapy.

Therapeutic compositions of the invention can be used in the following:

Suitable assays for proliferation and differentiation of various hematopoietic lines are cited above.

Assays for embryonic stem cell differentiation (which will identify, among others, proteins that influence embryonic differentiation hematopoiesis) include, without limitation, those described in: Johansson et al. Cellular Biology 15:141-151, 1995; Keller et al., Molecular and Cellular Biology 13:473-486, 1993; McClanahan et al., Blood 81:2903-2915, 1993.

Assays for stem cell survival and differentiation (which will identify, among others, proteins that regulate lympho-hematopoiesis) include, without limitation, those described in: Methylcellulose colony forming assays, Freshney, M. G. In Culture of Hematopoietic Cells. R I. Freshney, et al. eds. Vol pp. 265-268, Wiley-Liss, Inc., New York, N.Y. 1994; Hirayama et al., Proc. Natl. Acad. Sci. USA 89:5907-5911, 1992; Primitive hematopoietic colony forming cells with high proliferative potential, McNiece, I. K. and Briddell, R. A. In Culture of Hematopoietic Cells. R. I. Freshney, et al. eds. Vol pp. 23-39, Wiley-Liss, Inc., New York, N.Y. 1994; Neben et al., Experimental Hematology 22:353-359, 1994; Cobblestone area forming cell assay, Ploemacher, R. E. In Culture of Hematopoietic Cells. R. I. Freshney, et al. eds. Vol pp. 1-21, Wiley-Liss, Inc., New York, N.Y. 1994; Long term bone marrow cultures in the presence of stromal cells, Spooncer, E., Dexter, M. and Allen, T. In Culture of Hematopoietic Cells. R I. Freshney, et al. eds. Vol pp. 163-179, Wiley-Liss, Inc., New York, N.Y. 1994; Long term culture initiating cell assay, Sutherland, H. J. In Culture of Hematopoietic Cells. R. I. Freshney, et al. eds. Vol pp. 139-162, Wiley-Liss, Inc., New York, N.Y. 1994.

### 4.10.6 TISSUE GROWTH ACTIVITY

A polypeptide of the present invention also may be involved in bone, cartilage, tendon, ligament and/or nerve tissue growth or regeneration, as well as in wound healing and tissue repair and replacement, and in healing of burns, incisions and ulcers.

A polypeptide of the present invention which induces cartilage and/or bone growth in circumstances where bone is not normally formed, has application in the healing of bone fractures and cartilage damage or defects in humans and other animals. Compositions of a polypeptide, antibody, binding partner, or other modulator of the invention may have prophylactic use in closed as well as open fracture reduction and also in the improved fixation of artificial joints. De novo bone formation induced by an osteogenic agent contributes to the repair of congenital, trauma induced, or oncologic resection induced craniofacial defects, and also is useful in cosmetic plastic surgery.

A polypeptide of this invention may also be involved in attracting bone-forming cells, stimulating growth of bone-forming cells, or inducing differentiation of progenitors of bone-forming cells. Treatment of osteoporosis, osteoarthritis, bone degenerative disorders, or periodontal disease, such as through stimulation of bone and/or cartilage repair or by blocking inflammation or processes of tissue destruction (collagenase activity, osteoclast activity, etc.) mediated by inflammatory processes may also be possible using the composition of the invention.

Another category of tissue regeneration activity that may involve the polypeptide of the present invention is tendon/ligament formation. Induction of tendon/ligament-like tissue or other tissue formation in circumstances where such tissue is not normally formed, has application in the heating of tendon or ligament tears, deformities and other tendon or ligament defects in humans and other animals. Such a preparation employing a tendon/ligament-like tissue inducing protein may have prophylactic use in preventing damage to tendon or ligament tissue, as well as use in the improved fixation of tendon or ligament to bone or other tissues, and in repairing defects to tendon or ligament tissue. De novo tendon/ligament-like tissue formation induced by a composition of the present invention contributes to the repair of congenital, trauma induced, or other tendon or ligament defects of other origin, and is also useful in cosmetic plastic surgery for attachment or repair of tendons or ligaments. The compositions of the present invention may provide environment to attract tendon- or ligament-forming cells, stimulate growth of tendon- or ligament-forming cells, induce differentiation of progenitors of tendon- or ligament-forming cells, or induce growth of tendon/ligament cells or progenitors *ex vivo* for return *in vivo* to effect tissue repair. The compositions of the invention may also be useful in the treatment of tendinitis, carpal tunnel syndrome and other tendon or ligament defects. The compositions may also include an appropriate matrix and/or sequestering agent as a carrier as is well known in the art.

The compositions of the present invention may also be useful for proliferation of neural cells and for regeneration of nerve and brain tissue, i.e. for the treatment of central and peripheral nervous system diseases and neuropathies, as well as mechanical and traumatic disorders, which involve degeneration, death or trauma to neural cells or nerve tissue. More specifically, a composition may be used in the treatment of diseases of the peripheral nervous system, such as peripheral nerve injuries, peripheral neuropathy and localized neuropathies, and central nervous system diseases, such as Alzheimer's, Parkinson's disease, Huntington's disease, amyotrophic lateral sclerosis, and Shy-Drager syndrome. Further conditions which may be treated in accordance with the present invention include mechanical and traumatic disorders, such as spinal cord disorders, head trauma and cerebrovascular diseases such as stroke. Peripheral neuropathies resulting from chemotherapy or other medical therapies may also be treatable using a composition of the invention.

Compositions of the invention may also be useful to promote better or faster closure of non-healing wounds, including without limitation pressure ulcers, ulcers associated with vascular insufficiency, surgical and traumatic wounds, and the like.

Compositions of the present invention may also be involved in the generation or regeneration of other tissues, such as organs (including, for example, pancreas, liver, intestine, kidney, skin, endothelium), muscle (smooth, skeletal or cardiac) and vascular (including vascular endothelium) tissue, or for promoting the growth of cells comprising such tissues. Part of the desired effects may be by inhibition or modulation of fibrotic scarring may allow normal tissue to regenerate. A polypeptide of the present invention may also exhibit angiogenic activity.

A composition of the present invention may also be useful for gut protection or regeneration and treatment of lung or liver fibrosis, reperfusion injury in various tissues, and conditions resulting from systemic cytokine damage.

A composition of the present invention may also be useful for promoting or inhibiting differentiation of tissues described above from precursor tissues or cells; or for inhibiting the growth of tissues described above.

Therapeutic compositions of the invention can be used in the following:

Assays for tissue generation activity include, without limitation, those described in: International Patent Publication No. WO95/16035 (bone, cartilage, tendon); International Patent Publication No. WO95/05846 (nerve, neuronal); International Patent Publication No. WO91/07491 (skin, endothelium).

Assays for wound healing activity include, without limitation, those described in: Winter, Epidermal Wound Healing, pps. 71-112 (Maibach, H. 1. and Rovee, D. T., eds.), Year Book Medical Publishers, Inc., Chicago, as modified by Eaglstein and Mertz, J. Invest. Dermatol 71:382-84 (1978).

### 4.10.7 IMMUNE STIMULATING OR SUPPRESSING ACTIVITY

A polypeptide of the present invention may also exhibit immune stimulating or immune suppressing activity, including without limitation the activities for which assays are described herein. A polynucleotide of the invention can encode a polypeptide exhibiting such activities. A protein may be useful in the treatment of various immune deficiencies and disorders (including severe combined immunodeficiency (SCID)), e.g., in regulating (up or down) growth and proliferation of T and/or B lymphocytes, as well as effecting the cytolytic activity ofNK cells and other cell populations. These immune deficiencies may be genetic or be caused by viral (e.g., HIV) as well as bacterial or fungal infections, or may result from autoimmune disorders. More specifically, infectious diseases causes by viral, bacterial, fungal or other infection may be treatable using a protein of the present invention, including infections by HIV, hepatitis viruses, herpes viruses, mycobacteria, Leishmania spp., malaria spp. and various fungal infections such as candidiasis. Of course, in this regard, proteins of the present invention may also be useful where a boost to the immune system generally may be desirable, i.e., in the treatment of cancer.

Autoimmune disorders which may be treated using a protein of the present invention include, for example, connective tissue disease, multiple sclerosis, systemic lupus erythematosus, rheumatoid arthritis, autoimmune pulmonary inflammation, Guillain-Barre syndrome, autoimmune thyroiditis, insulin dependent diabetes mellitis, myasthenia gravis, graft-versus-host disease and autoimmune inflammatory eye disease. Such a protein (or antagonists thereof, including antibodies) of the present invention may also to be useful in the treatment of allergic reactions and conditions (*e*.*g*., anaphylaxis, serum sickness, drug reactions, food allergies, insect venom allergies, mastocytosis, allergic rhinitis, hypersensitivity pneumonitis, urticaria, angioedema, eczema, atopic dermatitis, allergic contact dermatitis, erythema multiforme, Stevens-Johnson syndrome, allergic conjunctivitis, atopic keratoconjunctivitis, venereal keratoconjunctivitis, giant papillary conjunctivitis and contact allergies), such as asthma (particularly allergic asthma) or other respiratory problems. Other conditions, in which immune suppression is desired (including, for example, organ transplantation), may also be treatable using a protein (or antagonists thereof) of the present invention. The therapeutic effects of the polypeptides or antagonists thereof on allergic reactions can be evaluated by in vivo animals models such as the cumulative contact enhancement test (Lastbom et al., Toxicology 125: 59-66, 1998), skin prick test (Hoffmann et al., Allergy 54: 446-54, 1999), guinea pig skin sensitization test (Vohr et al., Arch. Toxocol. 73: 501-9), and murine local lymph node assay (Kimber et al., J. Toxicol. Environ. Health 53: 563-79).

Using the proteins of the invention it may also be possible to modulate immune responses, in a number of ways. Down regulation may be in the form of inhibiting or blocking an immune response already in progress or may involve preventing the induction of an immune response. The functions of activated T cells may be inhibited by suppressing T cell responses or by inducing specific tolerance in T cells, or both. Immunosuppression of T cell responses is generally an active, non-antigen-specific, process which requires continuous exposure of the T cells to the suppressive agent. Tolerance, which involves inducing non-responsiveness or anergy in T cells, is distinguishable from immunosuppression in that it is generally antigen-specific and persists after exposure to the tolerizing agent has ceased. Operationally, tolerance can be demonstrated by the lack of a T cell response upon reexposure to specific antigen in the absence of the tolerizing agent.

Down regulating or preventing one or more antigen functions (including without limitation B lymphocyte antigen functions (such as, for example, B7)), e.g., preventing high level lymphokine synthesis by activated T cells, will be useful in situations of tissue, skin and organ transplantation and in graft-versus-host disease (GVHD). For example, blockage of T cell function should result in reduced tissue destruction in tissue transplantation. Typically, in tissue transplants, rejection of the transplant is initiated through its recognition as foreign by T cells, followed by an immune reaction that destroys the transplant. The administration of a therapeutic composition of the invention may prevent cytokine synthesis by immune cells, such as T cells, and thus acts as an immunosuppressant. Moreover, a lack of costimulation may also be sufficient to anergize the T cells, thereby inducing tolerance in a subject. Induction of long-term tolerance by B lymphocyte antigen-blocking reagents may avoid the necessity of repeated administration of these blocking reagents. To achieve sufficient immunosuppression or tolerance in a subject, it may also be necessary to block the function of a combination of B lymphocyte antigens.

The efficacy of particular therapeutic compositions in preventing organ transplant rejection or GVHD can be assessed using animal models that are predictive of efficacy in humans. Examples of appropriate systems which can be used include allogeneic cardiac grafts in rats and xenogeneic pancreatic islet cell grafts in mice, both of which have been used to examine the immunosuppressive effects of CTLA4Ig fusion proteins in vivo as described in Lenschow et al., Science 257:789-792 (1992) and Turka et al., Proc. Natl. Acad. Sci USA, 89:11102-11105 (1992). In addition, murine models of GVHD (see Paul ed., Fundamental Immunology, Raven Press, New York, 1989, pp. 846-847) can be used to determine the effect of therapeutic compositions of the invention on the development of that disease.

Blocking antigen function may also be therapeutically useful for treating autoimmune diseases. Many autoimmune disorders are the result of inappropriate activation of T cells that are reactive against self tissue and which promote the production of cytokines and autoantibodies involved in the pathology of the diseases. Preventing the activation of autoreactive T cells may reduce or eliminate disease symptoms. Administration of reagents which block stimulation of T cells can be used to inhibit T cell activation and prevent production of autoantibodies or T cell-derived cytokines which may be involved in the disease process. Additionally, blocking reagents may induce antigen-specific tolerance of autoreactive T cells which could lead to long-term relief from the disease. The efficacy of blocking reagents in preventing or alleviating autoimmune disorders can be determined using a number of well-characterized animal models of human autoimmune diseases. Examples include murine experimental autoimmune encephalitis, systemic lupus erythmatosis in MRL/lpr/lpr mice or NZB hybrid mice, murine autoimmune collagen arthritis, diabetes mellitus in NOD mice and BB rats, and murine experimental myasthenia gravis (see Paul ed., Fundamental Immunology, Raven Press, New York, 1989, pp. 840-856).

Upregulation of an antigen function (e.g., a B lymphocyte antigen function), as a means of up regulating immune responses, may also be useful in therapy. Upregulation of immune responses may be in the form of enhancing an existing immune response or eliciting an initial immune response. For example, enhancing an immune response may be useful in cases of viral infection, including systemic viral diseases such as influenza, the common cold, and encephalitis.

Alternatively, anti-viral immune responses may be enhanced in an infected patient by removing T cells from the patient, costimulating the T cells in vitro with viral antigen-pulsed APCs either expressing a peptide of the present invention or together with a stimulatory form of a soluble peptide of the present invention and reintroducing the in vitro activated T cells into the patient. Another method of enhancing anti-viral immune responses would be to isolate infected cells from a patient, transfect them with a nucleic acid encoding a protein of the present invention as described herein such that the cells express all or a portion of the protein on their surface, and reintroduce the transfected cells into the patient. The infected cells would now be capable of delivering a costimulatory signal to, and thereby activate, T cells in vivo.

A polypeptide of the present invention may provide the necessary stimulation signal to T cells to induce a T cell mediated immune response against the transfected tumor cells. In addition, tumor cells which lack MHC class I or MHC class II molecules, or which fail to reexpress sufficient mounts of MHC class I or MHC class II molecules, can be transfected with nucleic acid encoding all or a portion of (e.g., a cytoplasmic-domain truncated portion) of an MHC class I alpha chain protein and β₂ microglobulin protein or an MHC class II alpha chain protein and an MHC class II beta chain protein to thereby express MHC class I or MHC class II proteins on the cell surface. Expression of the appropriate class I or class II MHC in conjunction with a peptide having the activity of a B lymphocyte antigen (e.g., B7-1, B7-2, B7-3) induces a T cell mediated immune response against the transfected tumor cell. Optionally, a gene encoding an antisense construct which blocks expression of an MHC class II associated protein, such as the invariant chain, can also be cotransfected with a DNA encoding a peptide having the activity of a B lymphocyte antigen to promote presentation of tumor associated antigens and induce tumor specific immunity. Thus, the induction of a T cell mediated immune response in a human subject may be sufficient to overcome tumor-specific tolerance in the subject.

The activity of a protein of the invention may, among other means, be measured by the following methods:

Suitable assays for thymocyte or splenocyte cytotoxicity include, without limitation, those described in: Current Protocols in Immunology, Ed by J. E. Coligan, A. M. Kruisbeek, D. H. Margulies, E. M. Shevach, W. Strober, Pub. Greene Publishing Associates and Wiley-Interscience (Chapter 3, In Vitro assays for Mouse Lymphocyte Function 3.1-3.19; Chapter 7, Immunologic studies in Humans); Herrmann et al., Proc. Natl. Acad. Sci. USA 78:2488-2492, 1981; Herrmann et al., J. Immunol. 128:1968-1974, 1982; Handa et al.; J. Immunol. 135:1564-1572, 1985; Takai et al., I. Immunol. 137:3494-3500, 1986; Takai et al., J. Immunol. 140:508-512, 1988; Bowman et al., J. Virology 61:1992-1998; Bertagnolli et al., Cellular Immunology 133:327-341, 1991; Brown et al., J. Immunol. 153:3079-3092, 1994.

Assays for T-cell-dependent immunoglobulin responses and isotype switching (which will identify, among others, proteins that modulate T-cell dependent antibody responses and that affect Th1/Th2 profiles) include, without limitation, those described in: Maliszewski, J. Immunol. 144:3028-3033, 1990; and Assays for B cell function: In vitro antibody production, Mond, J. J. and Brunswick, M. In Current Protocols in Immunology. J. E. e.a. Coligan eds. Vol 1 pp. 3.8.1-3.8.16, John Wiley and Sons, Toronto. 1994.

Mixed lymphocyte reaction (MLR) assays (which will identify, among others, proteins that generate predominantly Th1 and CTL responses) include, without limitation, those described in: Current Protocols in Immunology, Ed by J. E. Coligan, A. M. Kruisbeek, D. H. Margulies, E. M. Shevach, W. Strober, Pub. Greene Publishing Associates and Wiley-Interscience (Chapter 3, In Vitro assays for Mouse Lymphocyte Function 3.1-3.19; Chapter 7, Immunologic studies in Humans); Takai et al., J. Immunol. 137:3494-3500, 1986; Takai et al., J. Immunol. 140:508-512, 1988; Bertagnolli et al., J. Immunol. 149:3778-3783, 1992.

Dendritic cell-dependent assays (which will identify, among others, proteins expressed by dendritic cells that activate naive T-cells) include, without limitation, those described in: Guery et al., J. Immunol. 134:536-544, 1995; Inaba et al., Journal of Experimental Medicine 173:549-559, 1991; Macatonia et al., Journal of Immunology 154:5071-5079, 1995; Porgador et al., Journal of Experimental Medicine 182:255-260, 1995; Nair et al., Journal of Virology 67:4062-4069, 1993; Huang et al., Science 264:961-965, 1994; Macatonia et al., Journal of Experimental Medicine 169:1255-1264, 1989; Bhardwaj et al., Journal of Clinical Investigation 94:797-807, 1994; and Inaba et al., Journal of Experimental Medicine 172:631-640,1990.

Assays for lymphocyte survival/apoptosis (which will identify, among others, proteins that prevent apoptosis after superantigen induction and proteins that regulate lymphocyte homeostasis) include, without limitation, those described in: Darzynkiewicz et al., Cytometry 13:795-808, 1992; Gorczyca et al., Leukemia 7:659-670,1993; Gorczyca et al., Cancer Research 53:1945-1951, 1993; Itoh et al., Cell 66:233-243, 1991; Zacharchuk, Journal of Immunology 145:4037-4045, 1990; Zamai et al., Cytometry 14:891-897, 1993; Gorczyca et al., International Journal of Oncology 1:639-648, 1992.

Assays for proteins that influence early steps of T-cell commitment and development include, without limitation, those described in: Antica et al., Blood 84:111-117, 1994; Fine et al., Cellular Immunology 155:111-122, 1994; Galy et al., Blood 85:2770-2778, 1995; Toki et al., Proc. Nat. Acad Sci. USA 88:7548-7551,1991.

### 4.10.8 ACTIVIN/INHIBIN ACTIVITY

A polypeptide of the present invention may also exhibit activin- or inhibin-related activities. A polynucleotide of the invention may encode a polypeptide exhibiting such characteristics. Inhibins are characterized by their ability to inhibit the release of follicle stimulating hormone (FSH), while activins and are characterized by their ability to stimulate the release of follicle stimulating hormone (FSH). Thus, a polypeptide of the present invention, alone or in heterodimers with a member of the inhibin family, may be useful as a contraceptive based on the ability of inhibins to decrease fertility in female mammals and decrease spermatogenesis in male mammals. Administration of sufficient amounts of other inhibins can induce infertility in these mammals. Alternatively, the polypeptide of the invention, as a homodimer or as a heterodimer with other protein subunits of the inhibin group, may be useful as a fertility inducing therapeutic, based upon the ability of activin molecules in stimulating FSH release from cells of the anterior pituitary. See, for example, U.S. Pat. No. 4,798,885. A polypeptide of the invention may also be useful for advancement of the onset of fertility in sexually immature mammals, so as to increase the lifetime reproductive performance of domestic animals such as, but not limited to, cows, sheep and pigs.

The activity of a polypeptide of the invention may, among other means, be measured by the following methods.

Assays for activin/inhibin activity include, without limitation, those described in: Vale et al., Endocrinology 91:562-572, 1972; Ling et al., Nature 321:779-782, 1986; Vale et al., Nature 321:776-779, 1986; Mason et al., Nature 318:659-663, 1985; Forage et al., Proc. Natl. Acad. Sci. USA 83:3091-3095, 1986.

### 4.10.9 CHEMOTACTIC/CHEMOKINETIC ACTIVITY

A polypeptide of the present invention may be involved in chemotactic or chemokinetic activity for mammalian cells, including, for example, monocytes, fibroblasts, neutrophils, T-cells, mast cells, eosinophils, epithelial and/or endothelial cells. A polynucleotide of the invention can encode a polypeptide exhibiting such attributes. Chemotactic and chemokinetic receptor activation can be used to mobilize or attract a desired cell population to a desired site of action. Chemotactic or chemokinetic compositions (e.g. proteins, antibodies, binding partners, or modulators of the invention) provide particular advantages in treatment of wounds and other trauma to tissues, as well as in treatment of localized infections. For example, attraction of lymphocytes, monocytes or neutrophils to tumors or sites of infection may result in improved immune responses against the tumor or infecting agent.

A protein or peptide has chemotactic activity for a particular cell population if it can stimulate, directly or indirectly, the directed orientation or movement of such cell population. Preferably, the protein or peptide has the ability to directly stimulate directed movement of cells. Whether a particular protein has chemotactic activity for a population of cells can be readily determined by employing such protein or peptide in any known assay for cell chemotaxis.

Therapeutic compositions of the invention can be used in the following:

Assays for chemotactic activity (which will identify proteins that induce or prevent chemotaxis) consist of assays that measure the ability of a protein to induce the migration of cells across a membrane as well as the ability of a protein to induce the adhesion of one cell population to another cell population. Suitable assays for movement and adhesion include, without limitation, those described in: Current Protocols in Immunology, Ed by J. E. Coligan, A. M. Kruisbeek, D. H. Marguiles, E. M. Shevach, W. Strober, Pub. Greene Publishing Associates and Wiley-Interscience (Chapter 6.12, Measurement of alpha and beta Chemokines 6.12.1-6.12.28; Taub et al. J. Clin. Invest. 95:1370-1376, 1995; Lind et al. APMIS 103:140-146, 1995; Muller et al Eur. J. Immunol. 25:1744-1748; Gruber et al. J. of Immunol. 152:5860-5867, 1994; Johnston et al. J. of Immunol. 153:1762-1768, 1994.

### 4.10.10 HEMOSTATIC AND THROMBOLYTIC ACTIVITY

A polypeptide of the invention may also be involved in hemostasis or thrombolysis or thrombosis. A polynucleotide of the invention can encode a polypeptide exhibiting such attributes. Compositions may be useful in treatment of various coagulation disorders (including hereditary disorders, such as hemophilias) or to enhance coagulation and other hemostatic events in treating wounds resulting from trauma, surgery or other causes. A composition of the invention may also be useful for dissolving or inhibiting formation of thromboses and for treatment and prevention of conditions resulting therefrom (such as, for example, infarction of cardiac and central nervous system vessels (e.g., stroke).

Therapeutic compositions of the invention can be used in the following:

Assay for hemostatic and thrombolytic activity include, without limitation, those described in: Linet et al., J. Clin. Pharmacol. 26:131-140, 1986; Burdick et al., Thrombosis Res. 45:413-419, 1987; Humphrey et al., Fibrinolysis 5:71-79 (1991); Schaub, Prostaglandins 35:467-474, 1988.

### 4.10.11 CANCER DIAGNOSIS AND THERAPY

Polypeptides of the invention may be involved in cancer cell generation, proliferation or metastasis. Detection of the presence or amount of polynucleotides or polypeptides of the invention may be useful for the diagnosis and/or prognosis of one or more types of cancer. For example, the presence or increased expression of a polynucleotide/polypeptide of the invention may indicate a hereditary risk of cancer, a precancerous condition, or an ongoing malignancy. Conversely, a defect in the gene or absence of the polypeptide may be associated with a cancer condition. Identification of single nucleotide polymorphisms associated with cancer or a predisposition to cancer may also be useful for diagnosis or prognosis.

Cancer treatments promote tumor regression by inhibiting tumor cell proliferation, inhibiting angiogenesis (growth of new blood vessels that is necessary to support tumor growth) and/or prohibiting metastasis by reducing tumor cell motility or invasiveness. Therapeutic compositions of the invention may be effective in adult and pediatric oncology including in solid phase tumors/malignancies, locally advanced tumors, human soft tissue sarcomas, metastatic cancer, including lymphatic metastases, blood cell malignancies including multiple myeloma, acute and chronic leukemias, and lymphomas, head and neck cancers including mouth cancer, larynx cancer and thyroid cancer, lung cancers including small cell carcinoma and non-small cell cancers, breast cancers including small cell carcinoma and ductal carcinoma, gastrointestinal cancers including esophageal cancer, stomach cancer, colon cancer, colorectal cancer and polyps associated with colorectal neoplasia, pancreatic cancers, liver cancer, urologic cancers including bladder cancer and prostate cancer, malignancies of the female genital tract including ovarian carcinoma, uterine (including endometrial) cancers, and solid tumor in the ovarian follicle, kidney cancers including renal cell carcinoma, brain cancers including intrinsic brain tumors, neuroblastoma, astrocytic brain tumors, gliomas, metastatic tumor cell invasion in the central nervous system, bone cancers including osteomas, skin cancers including malignant melanoma, tumor progression of human skin keratinocytes, squamous cell carcinoma, basal cell carcinoma, hemangiopericytoma and Karposi's sarcoma.

Polypeptides, polynucleotides, or modulators of polypeptides of the invention (including inhibitors and stimulators of the biological activity of the polypeptide of the invention) may be administered to treat cancer. Therapeutic compositions can be administered in therapeutically effective dosages alone or in combination with adjuvant cancer therapy such as surgery, chemotherapy, radiotherapy, thermotherapy, and laser therapy, and may provide a beneficial effect, e.g. reducing tumor size, slowing rate of tumor growth, inhibiting metastasis, or otherwise improving overall clinical condition, without necessarily eradicating the cancer.

The composition can also be administered in therapeutically effective amounts as a portion of an anti-cancer cocktail. An anti-cancer cocktail is a mixture of the polypeptide or modulator of the invention with one or more anti-cancer drugs in addition to a pharmaceutically acceptable carrier for delivery. The use of anti-cancer cocktails as a cancer treatment is routine. Anti-cancer drugs that are well known in the art and can be used as a treatment in combination with the polypeptide or modulator of the invention include: Actinomycin D, Aminoglutethimide, Asparaginase, Bleomycin, Busulfan, Carboplatin, Carmustine, Chlorambucil, Cisplatin (cis-DDP), Cyclophosphamide, Cytarabine HCl (Cytosine arabinoside), Dacarbazine, Dactinomycin, Daunorubicin HCl, Doxorubicin HCl, Estramustine phosphate sodium, Etoposide (V16-213), Floxuridine, 5-Fluorouracil (5-Fu), Flutamide, Hydroxyurea (hydroxycarbamide), Ifosfamide, Interferon Alpha-2a, Interferon Alpha-2b, Leuprolide acetate (LHRH-releasing factor analog), Lomustine, Mechlorethamine HCl (nitrogen mustard), Melphalan, Mercaptopurine, Mesna, Methotrexate (MTX), Mitomycin, Mitoxantrone HCl, Octreotide, Plicamycin, Procarbazine HCl, Streptozocin, Tamoxifen citrate, Thioguanine, Thiotepa, Vinblastine sulfate, Vincristine sulfate, Amsacrine, Azacitidine, Hexamethylmelamine, Interleukin-2, Mitoguazone, Pentostatin, Semustine, Teniposide, and Vindesine sulfate.

In addition, therapeutic compositions of the invention may be used for prophylactic treatment of cancer. There are hereditary conditions and/or environmental situations (e.g. exposure to carcinogens) known in the art that predispose an individual to developing cancers. Under these circumstances, it may be beneficial to treat these individuals with therapeutically effective doses of the polypeptide of the invention to reduce the risk of developing cancers.

*In vitro* models can be used to determine the effective doses of the polypeptide of the invention as a potential cancer treatment. These *in vitro* models include proliferation assays of cultured tumor cells, growth of cultured tumor cells in soft agar (see Freshney, (1987) Culture of Animal Cells: A Manual of Basic Technique, Wily-Liss, New York, NY Ch 18 and Ch 21), tumor systems in nude mice as described in Giovanella et al., J. Natl. Can. Inst., 52: 921-30 (1974), mobility and invasive potential of tumor cells in Boyden Chamber assays as described in Pilkington et al., Anticancer Res., 17: 4107-9 (1997), and angiogenesis assays such as induction of vascularization of the chick chorioallantoic membrane or induction of vascular endothelial cell migration as described in Ribatta et al., Intl. J. Dev. Biol., 40: 1189-97 (1999) and Li et al., Clin. Exp. Metastasis, 17:423-9 (1999), respectively. Suitable tumor cells lines are available, e.g. from American Type Tissue Culture Collection catalogs.

### 4.10.12 RECEPTOR/LIGAND ACTIVITY

A polypeptide of the present invention may also demonstrate activity as receptor, receptor ligand or inhibitor or agonist of receptor/ligand interactions. A polynucleotide of the invention can encode a polypeptide exhibiting such characteristics. Examples of such receptors and ligands include, without limitation, cytokine receptors and their ligands, receptor kinases and their ligands, receptor phosphatases and their ligands, receptors involved in cell-cell interactions and their ligands (including without limitation, cellular adhesion molecules (such as selectins, integrins and their ligands) and receptor/ligand pairs involved in antigen presentation, antigen recognition and development of cellular and humoral immune responses. Receptors and ligands are also useful for screening of potential peptide or small molecule inhibitors of the relevant receptor/ligand interaction. A protein of the present invention (including, without limitation, fragments of receptors and ligands) may themselves be useful as inhibitors of receptor/ligand interactions.

The activity of a polypeptide of the invention may, among other means, be measured by the following methods:

Suitable assays for receptor-ligand activity include without limitation those described in: Current Protocols in Immunology, Ed by J. E. Coligan, A. M. Kruisbeek, D. H. Margulies, E. M. Shevach, W. Strober, Pub. Greene Publishing Associates and Wiley- Interscience (Chapter 7.28, Measurement of Cellular Adhesion under static conditions 7.28.1- 7.28.22), Takai et al., Proc. Natl. Acad. Sci. USA 84:6864-6868, 1987; Bierer et al., *J.* Exp. Med. 168:1145-1156, 1988; Rosenstein et al., J. Exp. Med. 169:149-160 1989; Stoltenborg et al., J. Immunol. Methods 175:59-68, 1994; Stitt et al., Cell 80:661-670, 1995.

By way of example, the polypeptides of the invention may be used as a receptor for a ligand(s) thereby transmitting the biological activity of that ligand(s). Ligands may be identified through binding assays, affinity chromatography, dihybrid screening assays, BIAcore assays, gel overlay assays, or other methods known in the art.

Studies characterizing drugs or proteins as agonist or antagonist or partial agonists or a partial antagonist require the use of other proteins as competing ligands. The polypeptides of the present invention or ligand(s) thereof may be labeled by being coupled to radioisotopes, colorimetric molecules or a toxin molecules by conventional methods. ("Guide to Protein Purification" Murray P. Deutscher (ed) Methods in Enzymology Vol. 182 (1990) Academic Press, Inc. San Diego). Examples of radioisotopes include, but are not limited to, tritium and carbon-14 . Examples of colorimetric molecules include, but are not limited to, fluorescent molecules such as fluorescamine, or rhodamine or other colorimetric molecules. Examples of toxins include, but are not limited, to ricin.

### 4.10.13 DRUG SCREENING

This invention is particularly useful for screening chemical compounds by using the novel polypeptides or binding fragments thereof in any of a variety of drug screening techniques. The polypeptides or fragments employed in such a test may either be free in solution, affixed to a solid support, bome on a cell surface or located intracellularly. One method of drug screening utilizes eukaryotic or prokaryotic host cells which are stably transformed with recombinant nucleic acids expressing the polypeptide or a fragment thereof. Drugs are screened against such transformed cells in competitive binding assays. Such cells, either in viable or fixed form, can be used for standard binding assays. One may measure, for example, the formation of complexes between polypeptides of the invention or fragments and the agent being tested or examine the diminution in complex formation between the novel polypeptides and an appropriate cell line, which are well known in the art.

Sources for test compounds that may be screened for ability to bind to or modulate (i.e., increase or decrease) the activity of polypeptides of the invention include (1) inorganic and organic chemical libraries, (2) natural product libraries, and (3) combinatorial libraries comprised of either random or mimetic peptides, oligonucleotides or organic molecules.

Chemical libraries may be readily synthesized or purchased from a number of commercial sources, and may include structural analogs of known compounds or compounds that are identified as "hits" or "leads" via natural product screening.

The sources of natural product libraries are microorganisms (including bacteria and fungi), animals, plants or other vegetation, or marine organisms, and libraries of mixtures for screening may be created by: (1) fermentation and extraction of broths from soil, plant or marine microorganisms or (2) extraction of the organisms themselves. Natural product libraries include polyketides, non-ribosomal peptides, and (non-naturally occurring) variants thereof. For a review, see *Science 282*:63-68 (1998).

Combinatorial libraries are composed of large numbers of peptides, oligonucleotides or organic compounds and can be readily prepared by traditional automated synthesis methods, PCR, cloning or proprietary synthetic methods. Of particular interest are peptide and oligonucleotide combinatorial libraries. Still other libraries of interest include peptide, protein, peptidomimetic, multiparallel synthetic collection, recombinatorial, and polypeptide libraries. For a review of combinatorial chemistry and libraries created therefrom, see Myers, *Curr. Opin. Biotechnol*. 8:701-707 (1997). For reviews and examples of peptidomimetic libraries, see Al-Obeidi et al., *Mol. Biotechnol,* 9(3):205-23 (1998); Hruby et al., *Curr Opin Chem Biol*, 1(1):114-19 (1997); Dorner et al., *Bioorg Med Chem,* 4(5):709-15 (1996) (alkylated dipeprides).

Identification of modulators through use of the various libraries described herein permits modification of the candidate "hit" (or "lead") to optimize the capacity of the "hit" to bind a polypeptide of the invention. The molecules identified in the binding assay are then tested for antagonist or agonist activity in *in vivo* tissue culture or animal models that are well known in the art. In brief, the molecules are titrated into a plurality of cell cultures or animals and then tested for either cell/animal death or prolonged survival of the animal/cells.

The binding molecules thus identified may be complexed with toxins, e.g., ricin or cholera, or with other compounds that are toxic to cells such as radioisotopes. The toxin-binding molecule complex is then targeted to a tumor or other cell by the specificity of the binding molecule for a polypeptide of the invention. Alternatively, the binding molecules may be complexed with imaging agents for targeting and imaging purposes.

### 4.10.14 ASSAY FOR RECEPTOR ACTIVITY

The invention also provides methods to detect specific binding of a polypeptide e.g. a ligand or a receptor. The art provides numerous assays particularly useful for identifying previously unknown binding partners for receptor polypeptides of the invention. For example, expression cloning using mammalian or bacterial cells, or dihybrid screening assays can be used to identify polynucleotides encoding binding partners. As another example, affinity chromatography with the appropriate immobilized polypeptide of the invention can be used to isolate polypeptides that recognize and bind polypeptides of the invention. There are a number of different libraries used for the identification of compounds, and in particular small molecules, that modulate (*i*.*e*., increase or decrease) biological activity of a polypeptide of the invention. Ligands for receptor polypeptides of the invention can also be identified by adding exogenous ligands, or cocktails of ligands to two cells populations that are genetically identical except for the expression of the receptor of the invention: one cell population expresses the receptor of the invention whereas the other does not. The response of the two cell populations to the addition of ligands(s) are then compared. Alternatively, an expression library can be co-expressed with the polypeptide of the invention in cells and assayed for an autocrine response to identify potential ligand(s). As still another example, BIAcore assays, gel overlay assays, or other methods known in the art can be used to identify binding partner polypeptides, including, (1) organic and inorganic chemical libraries, (2) natural product libraries, and (3) combinatorial libraries comprised of random peptides, oligonucleotides or organic molecules.

The role of downstream intracellular signaling molecules in the signaling cascade of the polypeptide of the invention can be determined. For example, a chimeric protein in which the cytoplasmic domain of the polypeptide of the invention is fused to the extracellular portion of a protein, whose ligand has been identified, is produced in a host cell. The cell is then incubated with the ligand specific for the extracellular portion of the chimeric protein, thereby activating the chimeric receptor. Known downstream proteins involved in intracellular signaling can then be assayed for expected modifications i.e. phosphorylation. Other methods known to those in the art can also be used to identify signaling molecules involved in receptor activity.

### 4.10.15 ANTI-INFLAMMATORY ACTIVITY

Compositions of the present invention may also exhibit anti-inflammatory activity. The anti-inflammatory activity may be achieved by providing a stimulus to cells involved in the inflammatory response, by inhibiting or promoting cell-cell interactions (such as, for example, cell adhesion), by inhibiting or promoting chemotaxis of cells involved in the inflammatory process, inhibiting or promoting cell extravasation, or by stimulating or suppressing production of other factors which more directly inhibit or promote an inflammatory response. Compositions with such activities can be used to treat inflammatory conditions including chronic or acute conditions), including without limitation intimation associated with infection (such as septic shock, sepsis or systemic inflammatory response syndrome (SIRS)), ischemia-reperfusion injury, endotoxin lethality, arthritis, complement-mediated hyperacute rejection, nephritis, cytokine or chemokine-induced lung injury, inflammatory bowel disease, Crohn's disease or resulting from over production of cytoldnes such as TNF or IL-1. Compositions of the invention may also be useful to treat anaphylaxis and hypersensitivity to an antigenic substance or material. Compositions of this invention may be utilized to prevent or treat conditions such as, but not limited to, sepsis, acute pancreatitis, endotoxin shock, cytokine induced shock, rheumatoid arthritis, chronic inflammatory arthritis, pancreatic cell damage from diabetes mellitus type 1, graft versus host disease, inflammatory bowel disease, inflamation associated with pulmonary disease, other autoimmune disease or inflammatory disease, an antiproliferative agent such as for acute or chronic mylegenous leukemia or in the prevention of premature labor secondary to intrauterine infections.

### 4.10.16 LEUKEMIAS

Leukemias and related disorders may be treated or prevented by administration of a therapeutic that promotes or inhibits function of the polynucleotides and/or polypepudes of the invention. Such leukemias and related disorders include but are not limited to acute leukemia, acute lymphocytic leukemia, acute myelocytic leukemia, myeloblastic, promyelocytic, myelomonocytic, monocytic, erythroleukemia, chronic leukemia, chronic myelocytic (granulocytic) leukemia and chronic lymphocytic leukemia (for a review of such disorders, see Fishman et al., 1985, Medicine, 2d Ed., J.B. Lippincott Co., Philadelphia).

### 4.10.17 NERVOUS SYSTEM DISORDERS

Nervous system disorders, involving cell types which can be tested for efficacy of intervention with compounds that modulate the activity of the polynucleotides and/or polypeptides of the invention, and which can be treated upon thus observing an indication of therapeutic utility, include but are not limited to nervous system injuries, and diseases or disorders which result in either a disconnection of axons, a diminution or degeneration of neurons, or demyelination. Nervous system lesions which may be treated in a patient (including human and non-human mammalian patients) according to the invention include but are not limited to the following lesions of either the central (including spinal cord, brain) or peripheral nervous systems:
(i) traumatic lesions, including lesions caused by physical injury or associated with surgery, for example, lesions which sever a portion of the nervous system, or compression injuries;
(ii) ischemic lesions, in which a lack of oxygen in a portion of the nervous system results in neuronal injury or death, including cerebral infarction or ischemia, or spinal cord infarction or ischemia;
(iii) infectious lesions, in which a portion of the nervous system is destroyed or injured as a result of infection, for example, by an abscess or associated with infection by human immunodeficiency virus, herpes zoster, or herpes simplex virus or with Lyme disease, tuberculosis, syphilis;
(iv) degenerative lesions, in which a portion of the nervous system is destroyed or injured as a result of a degenerative process including but not limited to degeneration associated with Parkinson's disease, Alzheimer's disease, Huntington's chorea, or amyotrophic lateral sclerosis;
(v) lesions associated with nutritional diseases or disorders, in which a portion of the nervous system is destroyed or injured by a nutritional disorder or disorder of metabolism including but not limited to, vitamin B12 deficiency, folic acid deficiency, Wemicke disease, tobacco-alcohol amblyopia, Marchiafava-Bignami disease (primary degeneration of the corpus callosum), and alcoholic cerebellar degeneration;
(vi) neurological lesions associated with systemic diseases including but not limited to diabetes (diabetic neuropathy, Bell's palsy), systemic lupus erythematosus, carcinoma, or sarcoidosis;
(vii) lesions caused by toxic substances including alcohol, lead, or particular neurotoxins; and
(viii) demyelinated lesions in which a portion of the nervous system is destroyed or injured by a demyelinating disease including but not limited to multiple sclerosis, human immunodeficiency virus-associated myelopathy, transverse myelopathy or various etiologies, progressive multifocal leukoencephalopathy, and central pontine myelinolysis.

Therapeutics which are useful according to the invention for treatment of a nervous system disorder may be selected by testing for biological activity in promoting the survival or differentiation of neurons. For example, and not by way of limitation, therapeutics which elicit any of the following effects may be useful according to the invention:
(i) increased survival time of neurons in culture;
(ii) increased sprouting of neurons in culture or in vivo;
(iii) increased production of a neuron-associated molecule in culture or *in vivo*, *e*.*g*., choline acetyltransferase or acetylcholinesterase with respect to motor neurons; or
(iv) decreased symptoms of neuron dysfunction in vivo.

Such effects may be measured by any method known in the art. In preferred, non-limiting embodiments, increased survival of neurons may be measured by the method set forth in Arakawa et al. (1990, J. Neurosci. 10:3507-3515); increased sprouting of neurons may be detected by methods set forth in Pestronk et al. (1980, Exp. Neurol. 70:65-82) or Brown et al. (1981, Ann. Rev. Neurosci. 4:17-42); increased production of neuron-associated molecules may be measured by bioassay, enzymatic assay, antibody binding, Northern blot assay, *etc*., depending on the molecule to be measured; and motor neuron dysfunction may be measured by assessing the physical manifestation of motor neuron disorder, *e.g.*, weakness, motor neuron conduction velocity, or functional disability.

In specific embodiments, motor neuron disorders that may be treated according to the invention include but are not limited to disorders such as infarction, infection, exposure to toxin, trauma, surgical damage, degenerative disease or malignancy that may affect motor neurons as well as other components of the nervous system, as well as disorders that selectively affect neurons such as amyotrophic lateral sclerosis, and including but not limited to progressive spinal muscular atrophy, progressive bulbar palsy, primary lateral sclerosis, infantile and juvenile muscular atrophy, progressive bulbar paralysis of childhood (Fazio-Londe syndrome), poliomyelitis and the post polio syndrome, and Hereditary Motorsensory Neuropathy (Charcot-Marie-Tooth Disease).

### 4.10.18 OTHER ACTIVITIES

A polypeptide of the invention may also exhibit one or more of the following additional activities or effects: inhibiting the growth, infection or function of, or killing, infectious agents, including, without limitation, bacteria, viruses, fungi and other parasites; effecting (suppressing or enhancing) bodily characteristics, including, without limitation, height, weight, hair color, eye color, skin, fat to lean ratio or other tissue pigmentation, or organ or body part size or shape (such as, for example, breast augmentation or diminution, change in bone form or shape); effecting biorhythms or circadian cycles or rhythms; effecting the fertility of male or female subjects; effecting the metabolism, catabolism, anabolism, processing, utilization, storage or elimination of dietary fat, lipid, protein, carbohydrate, vitamins, minerals, co-factors or other nutritional factors or component(s); effecting behavioral characteristics, including, without limitation, appetite, libido, stress, cognition (including cognitive disorders), depression (including depressive disorders) and violent behaviors; providing analgesic effects or other pain reducing effects; promoting differentiation and growth of embryonic stem cells in lineages other than hematopoietic lineages; hormonal or endocrine activity; in the case of enzymes, correcting deficiencies of the enzyme and treating deficiency-related diseases; treatment of hyperproliferative disorders (such as, for example, psoriasis); immunoglobulin-like activity (such as, for example, the ability to bind antigens or complement); and the ability to act as an antigen in a vaccine composition to raise an immune response against such protein or another material or entity which is cross-reactive with such protein.

### 4.10.19 IDENTIFICATION OF POLYMORPHISMS

The demonstration of polymorphisms makes possible the identification of such polymorphisms in human subjects and the pharmacogenetic use of this information for diagnosis and treatment. Such polymorphisms may be associated with, e.g., differential predisposition or susceptibility to various disease states (such as disorders involving inflammation or immune response) or a differential response to drug administration, and this genetic information can be used to tailor preventive or therapeutic treatment appropriately. For example, the existence of a polymorphism associated with a predisposition to inflammation or autoimmune disease makes possible the diagnosis of this condition in humans by identifying the presence of the polymorphism.

Polymorphisms can be identified in a variety of ways known in the art which all generally involve obtaining a sample from a patient, analyzing DNA from the sample, optionally involving isolation or amplification of the DNA, and identifying the presence of the polymorphism in the DNA. For example, PCR may be used to amplify an appropriate fragment of genomic DNA which may then be sequenced. Alternatively, the DNA may be subjected to allele-specific oligonucleotide hybridization (in which appropriate oligonucleotides are hybridized to the DNA under conditions permitting detection of a single base mismatch) or to a single nucleotide extension assay (in which an oligonucleotide that hybridizes immediately adjacent to the position of the polymorphism is extended with one or more labeled nucleotides). In addition, traditional restriction fragment length polymorphism analysis (using restriction enzymes that provide differential digestion of the genomic DNA depending on the presence or absence of the polymorphism) may be performed. Arrays with nucleotide sequences of the present invention can be used to detect polymorphisms. The array can comprise modified nucleotide sequences of the present invention in order to detect the nucleotide sequences of the present invention. In the alternative, any one of the nucleotide sequences of the present invention can be placed on the array to detect changes from those sequences.

Alternatively a polymorphism resulting in a change in the amino acid sequence could also be detected by detecting a corresponding change in amino acid sequence of the protein, e.g., by an antibody specific to the variant sequence.

### 4.10.20 ARTHRITIS AND INFLAMMATION

The immunosuppressive effects of the compositions of the invention against rheumatoid arthritis is determined in an experimental animal model system. The experimental model system is adjuvant induced arthritis in rats, and the protocol is described by J. Holoshitz, et at., 1983, Science, 219:56, or by B. Waksman et al., 1963, Int. Arch. Allergy Appl. Immunol., 23:129. Induction of the disease can be caused by a single injection, generally intradermally, of a suspension of killed Mycobacterium tuberculosis in complete Freund's adjuvant (CFA). The route of injection can vary, but rats may be injected at the base of the tail with an adjuvant mixture. The polypeptide is administered in phosphate buffered solution (PBS) at a dose of about 1-5 mg/kg. The control consists of administering PBS only.

The procedure for testing the effects of the test compound would consist of intradermally injecting killed Mycobacterium tuberculosis in CFA followed by immediately administering the test compound and subsequent treatment every other day until day 24. At 14,15,18,20,22, and 24 days after injection of Mycobacterium CFA, an overall arthritis score may be obtained as described by J. Holoskitz above. An analysis of the data would reveal that the test compound would have a dramatic affect on the swelling of the joints as measured by a decrease of the arthritis score.

### 4.11 THERAPEUTIC METHODS

The compositions (including polypeptide fragments, analogs, variants and antibodies or other binding partners or modulators including antisense polynucleotides) of the invention have numerous applications in a variety of therapeutic methods. Examples of therapeutic applications include, but are not limited to, those exemplified herein.

### 4.11.1 EXAMPLE

One embodiment of the invention is the administration of an effective amount of the polypeptides or other composition of the invention to individuals affected by a disease or disorder that can be modulated by regulating the peptides of the invention. While the mode of administration is not particularly important, parenteral administration is preferred. An exemplary mode of administration is to deliver an intravenous bolus. The dosage of the polypeptides or other composition of the invention will normally be determined by the prescribing physician. It is to be expected that the dosage will vary according to the age, weight, condition and response of the individual patient. Typically, the amount of polypeptide administered per dose will be in the range of about 0.01 µg/kg to 100 mg/kg of body weight, with the preferred dose being about 0.1 µg/kg to 10 mg/kg of patient body weight. For parenteral administration, polypeptides of the invention will be formulated in an injectable form combined with a pharmaceutically acceptable parenteral vehicle. Such vehicles are well known in the art and examples include water, saline, Ringer's solution, dextrose solution, and solutions consisting of small amounts of the human serum albumin. The vehicle may contain minor amounts of additives that maintain the isotonicity and stability of the polypeptide or other active ingredient. The preparation of such solutions is within the skill of the art.

### 4.12 PHARMACEUTICAL FORMULATIONS AND ROUTES OF ADMINISTRATION

A protein or other composition of the present invention (from whatever source derived, including without limitation from recombinant and non-recombinant sources and including antibodies and other binding partners of the polypeptides of the invention) may be administered to a patient in need, by itself, or in pharmaceutical compositions where it is mixed with suitable carriers or excipient(s) at doses to treat or ameliorate a variety of disorders. Such a composition may optionally contain (in addition to protein or other active ingredient and a carrier) diluents, fillers, salts, buffers, stabilizers, solubilizers, and other materials well known in the art. The term "pharmaceutically acceptable" means a non-toxic material that does not interfere with the effectiveness of the biological activity of the active ingredient(s). The characteristics of the carrier will depend on the route of administration. The pharmaceutical composition of the invention may also contain cytokines, lymphokines, or other hematopoietic factors such as M-CSF, GM-CSF, TNF, IL-1, IL-2, IL-3, IL-4, IL-5, IL-6, IL-7, IL-8, IL-9, IL-10, II,-11, IL-12, IL-13, IL-14, IL-15, IFN, TNF0, TNF1, TNF2, G-CSF, Meg-CSF, thrombopoietin, stem cell factor, and erythropoietin. In further compositions, proteins of the invention may be combined with other agents beneficial to the treatment of the disease or disorder in question. These agents include various growth factors such as epidermal growth factor (EGF), platelet-derived growth factor (PDGF), transforming growth factors (TGF-α and TGF-β), insulin-like growth factor (IGF), as well as cytokines described herein.

The pharmaceutical composition may further contain other agents which either enhance the activity of the protein or other active ingredient or complement its activity or use in treatment. Such additional factors and/or agents may be included in the pharmaceutical composition to produce a synergistic effect with protein or other active ingredient of the invention, or to minimize side effects. Conversely, protein or other active ingredient of the present invention may be included in formulations of the particular clotting factor, cytokine, lymphokine, other hematopoietic factor, thrombolytic or anti-thrombotic factor, or anti-inflammatory agent to minimize side effects of the clotting factor, cytokine, lymphokine, other hematopoietic factor, thrombolytic or anti-thrombotic factor, or anti-inflammatory agent (such as IL-1Ra, IL-1 Hyl, IL-1 Hy2, anti-TNF, corticosteroids, immunosuppressive agents). A protein of the present invention may be active in multimers (e.g., heterodimers or homodimers) or complexes with itself or other proteins. As a result, pharmaceutical compositions of the invention may comprise a protein of the invention in such multimeric or complexed form.

As an alternative to being included in a pharmaceutical composition of the invention including a first protein, a second protein or a therapeutic agent may be concurrently administered with the first protein (e.g., at the same time, or at differing times provided that therapeutic concentrations of the combination of agents is achieved at the treatment site). Techniques for formulation and administration of the compounds of the instant application may be found in "Remington's Pharmaceutical Sciences," Mack Publishing Co., Easton, PA, latest edition. A therapeutically effective dose further refers to that amount of the compound sufficient to result in amelioration of symptoms, *e.g*., treatment, healing, prevention or amelioration of the relevant medical condition, or an increase in rate of treatment, healing, prevention or amelioration of such conditions. When applied to an individual active ingredient, administered alone, a therapeutically effective dose refers to that ingredient alone. When applied to a combination, a therapeutically effective dose refers to combined amounts of the active ingredients that result in the therapeutic effect, whether administered in combination, serially or simultaneously.

In practicing the method of treatment or use of the present invention, a therapeutically effective amount of protein or other active ingredient of the present invention is administered to a mammal having a condition to be treated. Protein or other active ingredient of the present invention may be administered in accordance with the method of the invention either alone or in combination with other therapies such as treatments employing cytokines, lymphokines or other hematopoietic factors. When co- administered with one or more cytokines, lymphokines or other hematopoietic factors, protein or other active ingredient of the present invention may be administered either simultaneously with the cytokine(s), lymphokine(s), other hematopoietic factor(s), thrombolytic or anti-thrombotic factors, or sequentially. If administered sequentially, the attending physician will decide on the appropriate sequence of administering protein or other active ingredient of the present invention in combination with cytokine(s), lymphokine(s), other hematopoietic factor(s), thrombolytic or anti-thrombotic factors.

### 4.12.1 ROUTES OF ADMINISTRATION

Suitable routes of administration may, for example, include oral, rectal, transmucosal, or intestinal administration; parenteral delivery, including intramuscular, subcutaneous, intramedullary injections, as well as intrathecal, direct intraventricular, intravenous, intraperitoneal, intranasal, or intraocular injections. Administration of protein or other active ingredient of the present invention used in the pharmaceutical composition or to practice the method of the present invention can be carried out in a variety of conventional ways, such as oral ingestion, inhalation, topical application or cutaneous, subcutaneous, intraperitoneal, parenteral or intravenous injection. Intravenous administration to the patient is preferred.

Alternately, one may administer the compound in a local rather than systemic manner, for example, via injection of the compound directly into a arthritic joints or in fibrotic tissue, often in a depot or sustained release formulation. In order to prevent the scarring process frequently occurring as complication of glaucoma surgery, the compounds may be administered topically, for example, as eye drops. Furthermore, one may administer the drug in a targeted drug delivery system, for example, in a liposome coated with a specific antibody, targeting, for example, arthritic or fibrotic tissue. The liposomes will be targeted to and taken up selectively by the afflicted tissue.

The polypeptides of the invention are administered by any route that delivers an effective dosage to the desired site of action. The determination of a suitable route of administration and an effective dosage for a particular indication is within the level of skill in the art. Preferably for wound treatment, one administers the therapeutic compound directly to the site. Suitable dosage ranges for the polypeptides of the invention can be extrapolated from these dosages or from similar studies in appropriate animal models. Dosages can then be adjusted as necessary by the clinician to provide maximal therapeutic benefit.

### 4.12.2 COMPOSITIONS/FORMULATIONS

Pharmaceutical compositions for use in accordance with the present invention thus may be formulated in a conventional manner using one or more physiologically acceptable carriers comprising excipients and auxiliaries which facilitate processing of the active compounds into preparations which can be used pharmaceutically. These pharmaceutical compositions may be manufactured in a manner that is itself known, *e*.*g*., by means of conventional mixing, dissolving, granulating, dragee-making, levigating, emulsifying, encapsulating, entrapping or lyophilizing processes. Proper formulation is dependent upon the route of administration chosen. When a therapeutically effective amount of protein or other active ingredient of the present invention is administered orally, protein or other active ingredient of the present invention will be in the form of a tablet, capsule, powder, solution or elixir. When administered in tablet form, the pharmaceutical composition of the invention may additionally contain a solid carrier such as a gelatin or an adjuvant. The tablet, capsule, and powder contain from about 5 to 95% protein or other active ingredient of the present invention, and preferably from about 25 to 90% protein or other active ingredient of the present invention. When administered in liquid form, a liquid carrier such as water, petroleum, oils of animal or plant origin such as peanut oil, mineral oil, soybean oil, or sesame oil, or synthetic oils may be added. The liquid form of the pharmaceutical composition may further contain physiological saline solution, dextrose or other saccharide solution, or glycols such as ethylene glycol, propylene glycol or polyethylene glycol. When administered in liquid form, the pharmaceutical composition contains from about 0.5 to 90% by weight of protein or other active ingredient of the present invention, and preferably from about 1 to 50% protein or other active ingredient of the present invention.

When a therapeutically effective amount of protein or other active ingredient of the present invention is administered by intravenous, cutaneous or subcutaneous injection, protein or other active ingredient of the present invention will be in the form of a pyrogen-free, parenterally acceptable aqueous solution. The preparation of such parenterally acceptable protein or other active ingredient solutions, having due regard to pH, isotonicity, stability, and the like, is within the skill in the art. A preferred pharmaceutical composition for intravenous, cutaneous, or subcutaneous injection should contain, in addition to protein or other active ingredient of the present invention, an isotonic vehicle such as Sodium Chloride Injection, Ringer's Injection, Dextrose Injection, Dextrose and Sodium Chloride Injection, Lactated Ringer's Injection, or other vehicle as known in the art. The pharmaceutical composition of the present invention may also contain stabilizers, preservatives, buffers, antioxidants, or other additives known to those of skill in the art For injection, the agents of the invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained from a solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration. For buccal administration, the compositions may take the form of tablets or lozenges formulated in conventional manner.

For administration by inhalation, the compounds for use according to the present invention are conveniently delivered in the form of an aerosol spray presentation from pressurized packs or a nebuliser, with the use of a suitable propellant, *e.g.*, dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, *e.g.*, gelatin for use in an inhaler or insufflator may be formulated containing a powder mix of the compound and a suitable powder base such as lactose or starch. The compounds may be formulated for parenteral administration by injection, *e.g*., by bolus injection or continuous infusion. Formulations for injection may be presented in unit dosage form, *e.g.*, in ampules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions. Alternatively, the active ingredient may be in powder form for constitution with a suitable vehicle, *e.g*., sterile pyrogen-free water, before use.

The compounds may also be formulated in rectal compositions such as suppositories or retention enemas, *e.g*., containing conventional suppository bases such as cocoa butter or other glycerides. In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

A pharmaceutical carrier for the hydrophobic compounds of the invention is a co-solvent system comprising benzyl alcohol, a nonpolar surfactant, a water-miscible organic polymer, and an aqueous phase. The co-solvent system may be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:5W) consists of VPD diluted 1:1 with a 5% dextrose in water solution. This co-solvent system dissolves hydrophobic compounds well, and itself produces low toxicity upon systemic administration. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of polysorbate 80; the fraction size of polyethylene glycol may be varied; other biocompatible polymers may replace polyethylene glycol, e.g. polyvinyl pyrrolidone; and other sugars or polysaccharides may substitute for dextrose. Alternatively, other delivery systems for hydrophobic pharmaceutical compounds may be employed. Liposomes and emulsions are well known examples of delivery vehicles or carriers for hydrophobic drugs. Certain organic solvents such as dimethylsulfoxide also may be employed, although usually at the cost of greater toxicity. Additionally, the compounds may be delivered using a sustained-release system, such as semipermeable matrices of solid hydrophobic polymers containing the therapeutic agent. Various types of sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein or other active ingredient stabilization may be employed.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols. Many of the active ingredients of the invention may be provided as salts with pharmaceutically compatible counter ions. Such pharmaceutically acceptable base addition salts are those salts which retain the biological effectiveness and properties of the free acids and which are obtained by reaction with inorganic or organic bases such as sodium hydroxide, magnesium hydroxide, ammonia, trialkylamine, dialkylamine, monoalkylamine, dibasic amino acids, sodium acetate, potassium benzoate, triethanol amine and the like.

The pharmaceutical composition of the invention may be in the form of a complex of the protein(s) or other active ingredient(s) of present invention along with protein or peptide antigens. The protein and/or peptide antigen will deliver a stimulatory signal to both B and T lymphocytes. B lymphocytes will respond to antigen through their surface immunoglobulin receptor. T lymphocytes will respond to antigen through the T cell receptor (TCR) following presentation of the antigen by MHC proteins. MHC and structurally related proteins including those encoded by class I and class II MHC genes on host cells will serve to present the peptide antigen(s) to T lymphocytes. The antigen components could also be supplied as purified MHC-peptide complexes alone or with co-stimulatory molecules that can directly signal T cells. Alternatively antibodies able to bind surface immunoglobulin and other molecules on B cells as well as antibodies able to bind the TCR and other molecules on T cells can be combined with the pharmaceutical composition of the invention.

The pharmaceutical composition of the invention may be in the form of a liposome in which protein of the present invention is combined, in addition to other pharmaceutically acceptable carriers, with amphipathic agents such as lipids which exist in aggregated form as micelles, insoluble monolayers, liquid crystals, or lamellar layers in aqueous solution. Suitable lipids for liposomal formulation include, without limitation, monoglycerides, diglycerides, sulfatides, lysolecithins, phospholipids, saponin, bile acids, and the like. Preparation of such liposomal formulations is within the level of skill in the art, as disclosed, for example, in U.S. Patent Nos. 4,235,871; 4,501,728; 4,837,028; and 4,737,323, all of which are incorporated herein by reference.

The amount of protein or other active ingredient of the present invention in the pharmaceutical composition of the present invention will depend upon the nature and severity of the condition being treated, and on the nature of prior treatments which the patient has undergone. Ultimately, the attending physician will decide the amount of protein or other active ingredient of the present invention with which to treat each individual patient. Initially, the attending physician will administer low doses of protein or other active ingredient of the present invention and observe the patient's response. Larger doses of protein or other active ingredient of the present invention may be administered until the optimal therapeutic effect is obtained for the patient, and at that point the dosage is not increased further. It is contemplated that the various pharmaceutical compositions used to practice the method of the present invention should contain about 0.01 µg to about 100 mg (preferably about 0.1 µg to about 10 mg, more preferably about 0.1 µg to about I mg) of protein or other active ingredient of the present invention per kg body weight. For compositions of the present invention which are useful for bone, cartilage, tendon or ligament regeneration, the therapeutic method includes administering the composition topically, systematically, or locally as an implant or device. When administered, the therapeutic composition for use in this invention is, of course, in a pyrogen-free, physiologically acceptable form. Further, the composition may desirably be encapsulated or injected in a viscous form for delivery to the site of bone, cartilage or tissue damage. Topical administration may be suitable for wound healing and tissue repair. Therapeutically useful agents other than a protein or other active ingredient of the invention which may also optionally be included in the composition as described above, may alternatively or additionally, be administered simultaneously or sequentially with the composition in the methods of the invention. Preferably for bone and/or cartilage formation, the composition would include a matrix capable of delivering the protein-containing or other active ingredient-containing composition to the site of bone and/or cartilage damage, providing a structure for the developing bone and cartilage and optimally capable of being resorbed into the body. Such matrices may be formed of materials presently in use for other implanted medical applications.

The choice of matrix material is based on biocompatibility, biodegradability, mechanical properties, cosmetic appearance and interface properties. The particular application of the compositions will define the appropriate formulation. Potential matrices for the compositions may be biodegradable and chemically defined calcium sulfate, tricalcium phosphate, hydroxyapatite, polylactic acid, polyglycolic acid and polyanhydrides. Other potential materials are biodegradable and biologically well-defined, such as bone or dermal collagen. Further matrices are comprised of pure proteins or extracellular matrix components. Other potential matrices are nonbiodegradable and chemically defined, such as sintered hydroxyapatite, bioglass, aluminates, or other ceramics. Matrices may be comprised of combinations of any of the above mentioned types of material, such as polylactic acid and hydroxyapatite or collagen and tricalcium phosphate. The bioceramics may be altered in composition, such as in calcium-aluminate-phosphate and processing to alter pore size, particle size, particle shape, and biodegradability. Presently preferred is a 50:50 (mole weight) copolymer of lactic acid and glycolic acid in the form of porous particles having diameters ranging from 150 to 800 microns. In some applications, it will be useful to utilize a sequestering agent, such as carboxymethyl cellulose or autologous blood clot, to prevent the protein compositions from disassociating from the matrix.

A preferred family of sequestering agents is cellulosic materials such as alkylcelluloses (including hydroxyalkylcelluloses), including methylcellulose, ethylcellulose, hydroxyethylcellulose, hydroxypropylcellulose, hydroxypropyl-methylcellulose, and carboxymethylcellulose, the most preferred being cationic salts of carboxymethylcellulose (CMC). Other preferred sequestering agents include hyaluronic acid, sodium alginate, poly(ethylene glycol), polyoxyethylene oxide, carboxyvinyl polymer and poly(vinyl alcohol). The amount of sequestering agent useful herein is 0.5-20 wt %, preferably 1-10 wt % based on total formulation weight, which represents the amount necessary to prevent desorption of the protein from the polymer matrix and to provide appropriate handling of the composition, yet not so much that the progenitor cells are prevented from infiltrating the matrix, thereby providing the protein the opportunity to assist the osteogenic activity of the progenitor cells. In further compositions, proteins or other active ingredients of the invention may be combined with other agents beneficial to the treatment of the bone and/or cartilage defect, wound, or tissue in question. These agents include various growth factors such as epidermal growth factor (EGF), platelet derived growth factor (PDGF), transforming growth factors (TGF-α and TGF-β), and insulin-like growth factor (IGF).

The therapeutic compositions are also presently valuable for veterinary applications. Particularly domestic animals and thoroughbred horses, in addition to humans, are desired patients for such treatment with proteins or other active ingredients of the present invention. The dosage regimen of a protein-containing pharmaceutical composition to be used in tissue regeneration will be determined by the attending physician considering various factors which modify the action of the proteins, *e*.*g*., amount of tissue weight desired to be formed, the site of damage, the condition of the damaged tissue, the size of a wound, type of damaged tissue (*e*.*g*., bone), the patient's age, sex, and diet, the severity of any infection, time of administration and other clinical factors. The dosage may vary with the type of matrix used in the reconstitution and with inclusion of other proteins in the pharmaceutical composition. For example, the addition of other known growth factors, such as IGF I (insulin like growth factor I), to the final composition, may also effect the dosage. Progress can be monitored by periodic assessment of tissue/bone growth and/or repair, for example, X-rays, histomorphoroetric determinations and tetracycline labeling.

Polynucleotides of the present invention can also be used for gene therapy. Such polynucleotides can be introduced either in vivo or ex vivo into cells for expression in a mammalian subject. Polynucleotides of the invention may also be administered by other known methods for introduction of nucleic acid into a cell or organism (including, without limitation, in the form of viral vectors or naked DNA). Cells may also be cultured ex vivo in the presence of proteins of the present invention in order to proliferate or to produce a desired effect on or activity in such cells. Treated cells can then be introduced in vivo for therapeutic purposes.

### 4.12.3 EFFECTIVE DOSAGE

Pharmaceutical compositions suitable for use in the present invention include compositions wherein the active ingredients are contained in an effective amount to achieve its intended purpose. More specifically, a therapeutically effective amount means an amount effective to prevent development of or to alleviate the existing symptoms of the subject being treated. Determination of the effective amount is well within the capability of those skilled in the art, especially in light of the detailed disclosure provided herein. For any compound used in the method of the invention, the therapeutically effective dose can be estimated initially from appropriate in vitro assays. For example, a dose can be formulated in animal models to achieve a circulating concentration range that can be used to more accurately determine useful doses in humans. For example, a dose can be formulated in animal models to achieve a circulating concentration range that includes the IC₅₀ as determined in cell culture (*i*.*e*., the concentration of the test compound which achieves a half-maximal inhibition of the protein's biological activity). Such information can be used to more accurately determine useful doses in humans.

A therapeutically effective dose refers to that amount of the compound that results in amelioration of symptoms or a prolongation of survival in a patient. Toxicity and therapeutic efficacy of such compounds can be determined by standard pharmaceutical procedures in cell cultures or experimental animals, *e*.*g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. Compounds which exhibit high therapeutic indices are preferred. The data obtained from these cell culture assays and animal studies can be used in formulating a range of dosage for use in human. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. See, *e.g.,* Fingl et al., 1975, in "The Pharmacological Basis of Therapeutics", Ch. 1 p.l. Dosage amount and interval may be adjusted individually to provide plasma levels of the active moiety which are sufficient to maintain the desired effects, or minimal effective concentration (MEC). The MEC will vary for each compound but can be estimated from *in vitro* data. Dosages necessary to achieve the MEC will depend on individual characteristics and route of administration. However, HPLC assays or bioassays can be used to determine plasma concentrations.

Dosage intervals can also be determined using MEC value. Compounds should be administered using a regimen which maintains plasma levels above the MEC for 10-90% of the time, preferably between 30-90% and most preferably between 50-90%. In cases of local administration or selective uptake, the effective local concentration of the drug may not be related to plasma concentration.

An exemplary dosage regimen for polypeptides or other compositions of the invention will be in the range of about 0.01 µg/kg to 100 mg/kg of body weight daily, with the preferred dose being about 0.1 µg/kg to 25 mg/kg of patient body weight daily, varying in adults and children. Dosing may be once daily, or equivalent doses may be delivered at longer or shorter intervals.

The amount of composition administered will, of course, be dependent on the subject being treated, on the subject's age and weight, the severity of the affliction, the manner of administration and the judgment of the prescribing physician.

### 4.12.4 PACKAGING

The compositions may, if desired, be presented in a pack or dispenser device which may contain one or more unit dosage forms containing the active ingredient. The pack may, for example, comprise metal or plastic foil, such as a blister pack. The pack or dispenser device may be accompanied by instructions for administration. Compositions comprising a compound of the invention formulated in a compatible pharmaceutical carrier may also be prepared, placed in an appropriate container, and labeled for treatment of an indicated condition.

### 4.13 ANTIBODIES

Also included in the invention are antibodies to proteins, or fragments of proteins of the invention. The term "antibody" as used herein refers to immunoglobulin molecules and immunologically active portions of immunoglobulin (Ig) molecules, i.e., molecules that contain an antigen binding site that specifically binds (immunoreacts with) an antigen. Such antibodies include, but are not limited to, polyclonal, monoclonal, chimeric, single chain, F_{ab}, F_{ab'} and F_{(ab')2} fragments, and an F_{ab} expression library. In general, an antibody molecule obtained from humans relates to any of the classes IgG, IgM, IgA, IgE and IgD, which differ from one another by the nature of the heavy chain present in the molecule. Certain classes have subclasses as well, such as IgG₁, IgG₂, and others. Furthermore, in humans, the light chain may be a kappa chain or a lambda chain. Reference herein to antibodies includes a reference to all such classes, subclasses and types of human antibody species.

An isolated related protein of the invention may be intended to serve as an antigen, or a portion or fragment thereof, and additionally can be used as an immunogen to generate antibodies that immunospecifically bind the antigen, using standard techniques for polyclonal and monoclonal antibody preparation. The full-length protein can be used or, alternatively, the invention provides antigenic peptide fragments of the antigen for use as immunogens. An antigenic peptide fragment comprises at least 6 amino acid residues of the amino acid sequence of the full length protein, such as the amino acid sequences shown in SEQ ID NO: 147-292, or 439-584, and encompasses an epitope thereof such that an antibody raised against the peptide forms a specific immune complex with the full length protein or with any fragment that contains the epitope. Preferably, the antigenic peptide comprises at least 10 amino acid residues, or at least 15 amino acid residues, or at least 20 amino acid residues, or at least 30 amino acid residues. Preferred epitopes encompassed by the antigenic peptide are regions of the protein that are located on its surface; commonly these are hydrophilic regions.

In certain embodiments of the invention, at least one epitope encompassed by the antigenic peptide is a region of -related protein that is located on the surface of the protein, *e*.*g*., a hydrophilic region. A hydrophobicity analysis of the human related protein sequence will indicate which regions of a related protein are particularly hydrophilic and, therefore, are likely to encode surface residues useful for targeting antibody production. As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation. See, *e*.*g*., Hopp and Woods, 1981, *Proc. Nat. Acad. Sci. USA* 78: 3824-3828; Kyte and Doolittle 1982, *J*. *Mol. Biol*. 157: 105-142, each of which is incorporated herein by reference in its entirety. Antibodies that are specific for one or more domains within an antigenic protein, or derivatives, fragments, analogs or homologs thereof, are also provided herein.

A protein of the invention, or a derivative, fragment, analog, homolog or ortholog thereof, may be utilized as an immunogen in the generation of antibodies that immunospecifically bind these protein components.

Various procedures known within the art may be used for the production of polyclonal or monoclonal antibodies directed against a protein of the invention, or against derivatives, fragments, analogs homologs or orthologs thereof (see, for example, Antibodies: A Laboratory Manual, Harlow E, and Lane D, 1988, Cold Spring Harbor Laboratory Press, Cold Spring Harbor, NY, incorporated herein by reference). Some of these antibodies are discussed below.

### 4.13.1 POLYCLONAL ANTIBODIES

For the production of polyclonal antibodies, various suitable host animals (e.g., rabbit, goat, mouse or other mammal) may be immunized by one or more injections with the native protein, a synthetic variant thereof, or a derivative of the foregoing. An appropriate immunogenic preparation can contain, for example, the naturally occurring immunogenic protein, a chemically synthesized polypeptide representing the immunogenic protein, or a recombinantly expressed immunogenic protein. Furthermore, the protein may be conjugated to a second protein known to be immunogenic in the mammal being immunized. Examples of such immunogenic proteins include but are not limited to keyhole limpet hemocyanin, serum albumin, bovine thyroglobulin, and soybean trypsin inhibitor. The preparation can further include an adjuvant. Various adjuvants used to increase the immunological response include, but are not limited to, Freund's (complete and incomplete), mineral gels (e.g., aluminum hydroxide), surface active substances (e.g., lysolecithin, pluronic polyols; polyanions, peptides, oil emulsions, dinitrophenol, etc.), adjuvants usable in humans such as Bacille Calmette-Guerin and Corynebacterium parvum, or similar immunostimulatory agents. Additional examples of adjuvants which can be employed include MPL-TDM adjuvant (monophosphoryl Lipid A, synthetic trehalose dicorynomycolate).

The polyclonal antibody molecules directed against the immunogenic protein can be isolated from the mammal (e.g., from the blood) and further purified by well known techniques, such as affinity chromatography using protein A or protein G, which provide primarily the IgG fraction of immune serum. Subsequently, or alternatively, the specific antigen which is the target of the immunoglobulin sought, or an epitope thereof, may be immobilized on a column to purify the immune specific antibody by immunoaffinity chromatography. Purification of immunoglobulins is discussed, for example, by D. Wilkinson (The Scientist, published by The Scientist, Inc., Philadelphia PA, Vol. 14, No. 8 (April 17, 2000), pp. 25-28).

### 4.13.2 MONOCLONAL ANTIBODIES

The term "monoclonal antibody" (MAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs thus contain an antigen binding site capable of immunoreacting with a particular epitope of the antigen characterized by a unique binding affinity for it.

Monoclonal antibodies can be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes can be immunized in vitro. The immunizing agent will typically include the protein antigen, a fragment thereof or a fusion protein thereof. Generally, either peripheral blood lymphocytes are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies (Kozbor, J. Immunol., 133:3001 (1984); Brodeur et al., Monoclonal Antibody Production Techniques and Applications, Marcel Dekker, Inc., New York, (1987) pp. 51-63).

The culture medium in which the hybridoma cells are cultured can then be assayed for the presence of monoclonal antibodies directed against the antigen. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980). Preferably, antibodies having a high degree of specificity and a high binding affinity for the target antigen are isolated.

After the desired hybridoma cells are identified, the clones can be subcloned by limiting dilution procedures and grown by standard methods. Suitable culture media for this purpose include, for example, Dulbecco's Modified Eagle's Medium and RPMI-1640 medium. Alternatively, the hybridoma cells can be grown in vivo as ascites in a mammal. The monoclonal antibodies secreted by the subclones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies can also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal, antibodies in the recombinant host cells. The DNA also can be modified, for example, by substituting the coding sequence for human heavy and light chain constant domains in place of the homologous murine sequences (U.S. Patent No. 4,816,567; Monison, Nature 368, 812-13 (1994)) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

### 4.133 HUMANIZED ANTIBODIES

The antibodies directed against the protein antigens of the invention can further comprise humanized antibodies or human antibodies. These antibodies are suitable for administration to humans without engendering an immune response by the human against the administered immunoglobulin. Humanized forms of antibodies are chimeric immunoglobulins, immunoglobulin chains or fragments thereof (such as Fv, Fab, Fab', F(ab')₂ or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al., Nature, 321 :522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. (See also U.S. Patent No. 5,225,539.) In some instances, Fv framework residues of the human immunoglobulin are replaced by corresponding non-human residues. Humanized antibodies can also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also will comprise at least a portion of an immunoglobulin constant region (Fc), typically that of a human immunoglobulin (Jones et al., 1986; Riechmann et al., 1988; and Presta, Curr. Op. Struct. Biol., 2:593-596 (1992)).

### 4.13.4 HUMAN ANTIBODIES

Fully human antibodies relate to antibody molecules in which essentially the entire sequences ofboth the light chain and the heavy chain, including the CDRs, arise from human genes. Such antibodies are termed "human antibodies", or "fully human antibodies" herein. Human monoclonal antibodies can be prepared by the trioma technique; the human B-cell hybridoma technique (see Kozbor, et al., 1983 Immunol Today 4: 72) and the EBV hybridoma technique to produce human monoclonal antibodies (see Cole, et al., 1985 In: MONOCLONAL ANTIBODIES AND CANCER THERAPY, Alan R Liss, Inc., pp. 77-96). Human monoclonal antibodies may be utilized in the practice of the present invention and may be produced by using human hybridomas (see Cote, et al., 1983. Proc Natl Acad Sci USA 80: 2026-2030) or by transforming human B-cells with Epstein Barr Virus in vitro (see Cole, et al., 1985 In: MONOCLONAL ANTIBODIES AND CANCER THERAPY, Alan R Liss, Inc., pp. 77-96).

In addition, human antibodies can also be produced using additional techniques, including phage display libraries (Hoogenboom and Winter, J. Mol. Biol., 227:381 (1991); Marks et al., J. Mol. Biol., 222:581 (1991)). Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in Marks et al. (Bio/Technology 10, 779-783 (1992)); Lonberg et al. (Nature 368 856-859 (1994)); Morrison (Nature 368, 812-13 (1994)); Fishwild et al,(Nature Biotechnology 14, 845-51 (1996)); Neuberger (Nature Biotechnology 14, 826 (1996)); and Lonberg and Huszar (Intern. Rev. Immunol. 13 65-93 (1995)).

Human antibodies may additionally be produced using transgenic nonhuman animals which are modified so as to produce fully human antibodies rather than the animal's endogenous antibodies in response to challenge by an antigen. (See PCT publication WO94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the Xenomouse™ as disclosed in PCT publications WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

An example of a method of producing a nonhuman host, exemplified as a mouse, lacking expression of an endogenous immunoglobulin heavy chain is disclosed in U.S. Patent No. 5,939,598. It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable marker; and producing from the embryonic stem cell a transgenic mouse whose somatic and germ cells contain the gene encoding the selectable marker.

A method for producing an antibody of interest, such as a human antibody, is disclosed in U.S. Patent No. 5,916,771. It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

In a further improvement on this procedure, a method for identifying a clinically relevant epitope on an immunogen, and a correlative method for selecting an antibody that binds immunospecifically to the relevant epitope with high affinity, are disclosed in PCT publication WO 99/53049.

### 4.13.5 F_{ab} FRAGMENTS AND SINGLE CHAIN ANTIBODIES

According to the invention, techniques can be adapted for the production of single-chain antibodies specific to an antigenic protein of the invention (see e.g., U.S. Patent No. 4,946,778). In addition, methods can be adapted for the construction of F_{ab} expression libraries (see e.g., Huse, et al., 1989 Science 246: 1275-1281) to allow rapid and effective identification of monoclonal F_{ab} fragments with the desired specificity for a protein or derivatives, fragments, analogs or homologs thereof. Antibody fragments that contain the idiotypes to a protein antigen may be produced by techniques known in the art including, but not limited to: (i) an F_{(ab')2} fragment produced by pepsin digestion of an antibody molecule; (ii) an F_{ab} fragment generated by reducing the disulfide bridges of an F_{(ab')2} fragment; (iii) an F_{ab} fragment generated by the treatment of the antibody molecule with papain and a reducing agent and (iv) Fᵥ fragments.

### 4.13.6 BISPECIFIC ANTIBODIES

Bispecific antibodies are monoclonal, preferably human or humanized, antibodies that have binding specificities for at least two different antigens. In the present case, one of the binding specificities is for an antigenic protein of the invention. The second binding target is any other antigen, and advantageously is a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker *et al*., 1991 *EMBO J.,* 10:3655-3659.

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Additionally, Fab' fragments can be directly recovered from E. coli and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from E. coli and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994).

Antibodies with more than two valencies are contemplated. For example, trispecific antibodies can be prepared. Tutt et al., J. Immunol. 147:60 (1991).
Exemplary bispecific antibodies can bind to two different epitopes, at least one of which originates in the protein antigen of the invention. Alternatively, an anti-antigenic arm of an immunoglobulin molecule can be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular antigen. Bispecific antibodies can also be used to direct cytotoxic agents to cells which express a particular antigen. These antibodies possess an antigen-binding arm and an arm which binds a cytotoxic agent or a radionuclide chelator, such as EOTUBE, DPTA, DOTA, or TETA. Another bispecific antibody of interest binds the protein antigen described herein and further binds tissue factor (TF).

### 4.13.7 HETEROCONJUGATE ANTIBODIES

Heteroconjugate antibodies are also within the scope of the present invention.
Heteroconjugate antibodies are composed of two covalently joined antibodies. Such antibodies have, for example, been proposed to target immune system cells to unwanted cells (U.S. Patent No. 4,676,980), and for treatment of HIV infection (WO 91/00360; WO 92/200373; EP 03089). It is contemplated that the antibodies can be prepared in vitro using known methods in synthetic protein chemistry, including those involving crosslinking agents. For example, immunotoxins can be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 4.13.8 EFFECTOR FUNCTION ENGINEERING

It can be desirable to modify the antibody of the invention with respect to effector function, so as to enhance, e.g., the effectiveness of the antibody in treating cancer. For example, cysteine residue(s) can be introduced into the Fc region, thereby allowing interchain disulfide bond formation in this region. The homodimeric antibody thus generated can have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced antitumor activity can also be prepared using heterobifunctional cross-linkers as described in Wolff et al. Cancer Research, 53: 2560-2565 (1993). Alternatively, an antibody can be engineered that has dual Fc regions and can thereby have enhanced complement lysis and ADCC capabilities. See Stevenson et al., Anti-Cancer Drug Design, 3: 219-230 (1989).

### 4.13.9 IMMUNOCONJUGATES

The invention also pertains to immunoconjugates comprising an antibody conjugated to a cytotoxic agent such as a chemotherapeutic agent, toxin (e.g., an enzymatically active toxin of bacterial, fimgal, plant, or animal origin, or fragments thereof), or a radioactive isotope (i.e., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody can be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (e.g., avidin) that is in turn conjugated to a cytotoxic agent.

### 4.14 COMPUTER READABLE SEQUENCES

In one application of this embodiment, a nucleotide sequence of the present invention can be recorded on computer readable media. As used herein, "computer readable media" refers to any medium which can be read and accessed directly by a computer. Such media include, but are not limited to: magnetic storage media, such as floppy discs, hard disc storage medium, and magnetic tape; optical storage media such as CD-ROM; electrical storage media such as RAM and ROM; and hybrids of these categories such as magnetic/optical storage media. A skilled artisan can readily appreciate how any of the presently known computer readable mediums can be used to create a manufacture comprising computer readable medium having recorded thereon a nucleotide sequence of the present invention. As used herein, "recorded" refers to a process for storing information on computer readable medium. A skilled artisan can readily adopt any of the presently known methods for recording information on computer readable medium to generate manufactures comprising the nucleotide sequence information of the present invention.

A variety of data storage structures are available to a skilled artisan for creating a computer readable medium having recorded thereon a nucleotide sequence of the present invention. The choice of the data storage structure will generally be based on the means chosen to access the stored information. In addition, a variety of data processor programs and formats can be used to store the nucleotide sequence information of the present invention on computer readable medium. The sequence information can be represented in a word processing text file, formatted in commercially-available software such as WordPerfect and Microsoft Word, or represented in the form of an ASCII file, stored in a database application, such as DB2, Sybase, Oracle, or the like. A skilled artisan can readily adapt any number of data processor structuring formats (e.g. text file or database) in order to obtain computer readable medium having recorded thereon the nucleotide sequence information of the present invention.

By providing any of the nucleotide sequences SEQ ID NO: 1-146, or 293-438 or a representative fragment thereof; or a nucleotide sequence at least 95% identical to any of the nucleotide sequences of SEQ ID NO: 1-146, or 293-438 in computer readable form, a skilled artisan can routinely access the sequence information for a variety of purposes. Computer software is publicly available which allows a skilled artisan to access sequence information provided in a computer readable medium. The examples which follow demonstrate how software which implements the BLAST (Altschul et al., J. Mol. Biol. 215:403-410 (1990)) and BLAZE (Brutlag et al., Comp. Chem. 17:203-207 (1993)) search algorithms on a Sybase system is used to identify open reading frames (ORFs) within a nucleic acid sequence. Such ORFs may be protein encoding fragments and may be useful in producing commercially important proteins such as enzymes used in fermentation reactions and in the production of commercially useful metabolites.

As used herein, "a computer-based system" refers to the hardware means, software means, and data storage means used to analyze the nucleotide sequence information of the present invention. The minimum hardware means of the computer-based systems of the present invention comprises a central processing unit (CPU), input means, output means, and data storage means. A skilled artisan can readily appreciate that any one of the currently available computer-based systems are suitable for use in the present invention. As stated above, the computer-based systems of the present invention comprise a data storage means having stored therein a nucleotide sequence of the present invention and the necessary hardware means and software means for supporting and implementing a search means. As used herein, "data storage means" refers to memory which can store nucleotide sequence information of the present invention, or a memory access means which can access manufactures having recorded thereon the nucleotide sequence information of the present invention.

As used herein, "search means" refers to one or more programs which are implemented on the computer-based system to compare a target sequence or target structural motif with the sequence information stored within the data storage means. Search means are used to identify fragments or regions of a known sequence which match a particular target sequence or target motif A variety of known algorithms are disclosed publicly and a variety of commercially available software for conducting search means are and can be used in the computer-based systems of the present invention. Examples of such software includes, but is not limited to, Smith-Waterman, MacPattern (EMBL), BLASTN and BLASTA (NPOLYPEPTIDEIA). A skilled artisan can readily recognize that any one of the available algorithms or implementing software packages for conducting homology searches can be adapted for use in the present computer-based systems. As used herein, a "target sequence" can be any nucleic acid or amino acid sequence of six or more nucleotides or two or more amino acids. A skilled artisan can readily recognize that the longer a target sequence is, the less likely a target sequence will be present as a random occurrence in the database. The most preferred sequence length of a target sequence is from about 10 to 300 amino acids, more preferably from about 30 to 100 nucleotide residues. However, it is well recognized that searches for commercially important fragments, such as sequence fragments involved in gene expression and protein processing, may be of shorter length.

As used herein, "a target structural motif," or "target motif," refers to any rationally selected sequence or combination of sequences in which the sequence(s) are chosen based on a three-dimensional configuration which is formed upon the folding of the target motif. There are a variety of target motifs known in the art. Protein target motifs include, but are not limited to, enzyme active sites and signal sequences. Nucleic acid target motifs include, but are not limited to, promoter sequences, hairpin structures and inducible expression elements (protein binding sequences).

### 4.15 TRIPLE HELIX FORMATION

In addition, the fragments of the present invention, as broadly described, can be used to control gene expression through triple helix formation or antisense DNA or RNA, both of which methods are based on the binding of a polynucleotide sequence to DNA or RNA. Polynucleotides suitable for use in these methods are preferably 20 to 40 bases in length and are designed to be complementary to a region of the gene involved in transcription (triple helix - see Lee et al., Nucl. Acids Res. 6:3073 (1979); Cooney et al., Science 15241:456 (1988); and Dervan et al., Science 251:1360 (1991)) or to the mRNA itself (antisense - Olmno, J. Neurochem. 56:560 (1991); Oligodeoxynucleotides as Antisense Inhibitors of Gene Expression, CRC Press, Boca Raton, FL (1988)). Triple helix-formation optimally results in a shut-off of RNA transcription from DNA, while antisense RNA hybridization blocks translation of an mRNA molecule into polypeptide. Both techniques have been demonstrated to be effective in model systems. Information contained in the sequences of the present invention is necessary for the design of an antisense or triple helix oligonucleotide.

### 4.16 DIAGNOSTIC ASSAYS AND KITS

The present invention further provides methods to identify the presence or expression of one of the ORFs of the present invention, or homolog thereof, in a test sample, using a nucleic acid probe or antibodies of the present invention, optionally conjugated or otherwise associated with a suitable label.

In general, methods for detecting a polynucleotide of the invention can comprise contacting a sample with a compound that binds to and forms a complex with the polynucleotide for a period sufficient to form the complex, and detecting the complex, so that if a complex is detected, a polynucleotide of the invention is detected in the sample. Such methods can also comprise contacting a sample under stringent hybridization conditions with nucleic acid primers that anneal to a polynucleotide of the invention under such conditions, and amplifying annealed polynucleotides, so that if a polynucleotide is amplified, a polynucleotide of the invention is detected in the sample.

In general, methods for detecting a polypeptide of the invention can comprise contacting a sample with a compound that binds to and forms a complex with the polypeptide for a period sufficient to form the complex, and detecting the complex, so that if a complex is detected, a polypeptide of the invention is detected in the sample.

In detail, such methods comprise incubating a test sample with one or more of the antibodies or one or more of the nucleic acid probes of the present invention and assaying for binding of the nucleic acid probes or antibodies to components within the test sample.

Conditions for incubating a nucleic acid probe or antibody with a test sample vary. Incubation conditions depend on the format employed in the assay, the detection methods employed, and the type and nature of the nucleic acid probe or antibody used in the assay. One skilled in the art will recognize that any one of the commonly available hybridization, amplification or immunological assay formats can readily be adapted to employ the nucleic acid probes or antibodies of the present invention. Examples of such assays can be found in Chard, T., An Introduction to Radioimmunoassay and Related Techniques, Elsevier Science Publishers, Amsterdam, The Netherlands (1986); Bullock, G.R. et al., Techniques in Immunocytochemistry, Academic Press, Orlando, FL Vol. 1 (1982), Vol. 2 (1983), Vol. 3 (1985); Tijssen, P., Practice and Theory of immunoassays: Laboratory Techniques in Biochemistry and Molecular Biology, Elsevier Science Publishers, Amsterdam, The Netherlands (1985). The test samples of the present invention include cells, protein or membrane extracts of cells, or biological fluids such as sputum, blood, serum, plasma, or urine. The test sample used in the above-described method will vary based on the assay format, nature of the detection method and the tissues, cells or extracts used as the sample to be assayed. Methods for preparing protein extracts or membrane extracts of cells are well known in the art and can be readily be adapted in order to obtain a sample which is compatible with the system utilized.

In another embodiment of the present invention, kits are provided which contain the necessary reagents to carry out the assays of the present invention. Specifically, the invention provides a compartment kit to receive, in close confinement, one or more containers which comprises: (a) a first container comprising one of the probes or antibodies of the present invention; and (b) one or more other containers comprising one or more of the following: wash reagents, reagents capable of detecting presence of a bound probe or antibody.

In detail, a compartment kit includes any kit in which reagents are contained in separate containers. Such containers include small glass containers, plastic containers or strips of plastic or paper. Such containers allows one to efficiently transfer reagents from one compartment to another compartment such that the samples and reagents are not cross-contaminated, and the agents or solutions of each container can be added in a quantitative fashion from one compartment to another. Such containers will include a container which will accept the test sample, a container which contains the antibodies used in the assay, containers which contain wash reagents (such as phosphate buffered saline, Tris-buffers, etc.), and containers which contain the reagents used to detect the bound antibody or probe. Types of detection reagents include labeled nucleic acid probes, labeled secondary antibodies, or in the alternative, if the primary antibody is labeled, the enzymatic, or antibody binding reagents which are capable of reacting with the labeled antibody. One skilled in the art will readily recognize that the disclosed probes and antibodies of the present invention can be readily incorporated into one of the established kit formats which are well known in the art.

### 4.17 MEDICAL IMAGING

The novel polypeptides and binding partners of the invention are useful in medical imaging of sites expressing the molecules of the invention (e.g., where the polypeptide of the invention is involved in the immune response, for imaging sites of inflammation or infection). See, e.g., Kunkel et al., U.S. Pat. NO. 5,413,778. Such methods involve chemical attachment of a labeling or imaging agent, administration of the labeled polypeptide to a subject in a pharmaceutically acceptable carrier, and imaging the labeled polypeptide in vivo at the target site.

### 4.18 SCREENING ASSAYS

Using the isolated proteins and polynucleotides of the invention, the present invention further provides methods of obtaining and identifying agents which bind to a polypeptide encoded by an ORF corresponding to any of the nucleotide sequences set forth in SEQ ID NO: 1-146, or 293-438, or bind to a specific domain of the polypeptide encoded by the nucleic acid. In detail, said method comprises the steps of:
(a) contacting an agent with an isolated protein encoded by an ORF of the present invention, or nucleic acid of the invention; and
(b) determining whether the agent binds to said protein or said nucleic acid.

In general, therefore, such methods for identifying compounds that bind to a polynucleotide of the invention can comprise contacting a compound with a polynucleotide of the invention for a time sufficient to form a polynucleotide/compound complex, and detecting the complex, so that if a polynucleotide/compound complex is detected, a compound that binds to a polynucleotide of the invention is identified.

Likewise, in general, therefore, such methods for identifying compounds that bind to a polypeptide of the invention can comprise contacting a compound with a polypeptide of the invention for a time sufficient to form a polypeptide/compound complex, and detecting the complex, so that if a polypeptide/compound complex is detected, a compound that binds to a polynucleotide of the invention is identified.

Methods for identifying compounds that bind to a polypeptide of the invention can also comprise contacting a compound with a polypeptide of the invention in a cell for a time sufficient to form a polypeptide/compound complex, wherein the complex drives expression of a receptor gene sequence in the cell, and detecting the complex by detecting reporter gene sequence expression, so that if a polypeptide/compound complex is detected, a compound that binds a polypeptide of the invention is identified.

Compounds identified via such methods can include compounds which modulate the activity of a polypeptide of the invention (that is, increase or decrease its activity, relative to activity observed in the absence of the compound). Alternatively, compounds identified via such methods can include compounds which modulate the expression of a polynucleotide of the invention (that is, increase or decrease expression relative to expression levels observed in the absence of the compound). Compounds, such as compounds identified via the methods of the invention, can be tested using standard assays well known to those of skill in the art for their ability to modulate activity/expression.

The agents screened in the above assay can be, but are not limited to, peptides, carbohydrates, vitamin derivatives, or other pharmaceutical agents. The agents can be selected and screened at random or rationally selected or designed using protein modeling techniques.

For random screening, agents such as peptides, carbohydrates, pharmaceutical agents and the like are selected at random and are assayed for their ability to bind to the protein encoded by the ORF of the present invention. Alternatively, agents may be rationally selected or designed. As used herein, an agent is said to be "rationally selected or designed" when the agent is chosen based on the configuration of the particular protein. For example, one skilled in the art can readily adapt currently available procedures to generate peptides, pharmaceutical agents and the like, capable of binding to a specific peptide sequence, in order to generate rationally designed antipeptide peptides, for example see Hurby et al., Application of Synthetic Peptides: Antisense Peptides," In Synthetic Peptides, A User's Guide, W.H. Freeman, NY (1992), pp. 289-307, and Kaspczak et al., Biochemistry 28:9230-8 (1989), or pharmaceutical agents, or the like.

In addition to the foregoing, one class of agents of the present invention, as broadly described, can be used to control gene expression through binding to one of the ORFs or EMFs of the present invention. As described above, such agents can be randomly screened or rationally designed/selected. Targeting the ORF or EMF allows a skilled artisan to design sequence specific or element specific agents, modulating the expression of either a single ORF or multiple ORFs which rely on the same EMF for expression control. One class of DNA binding agents are agents which contain base residues which hybridize or form a triple helix formation by binding to DNA or RNA. Such agents can be based on the classic phosphodiester, ribonucleic acid backbone, or can be a variety of sulfhydryl or polymeric derivatives which have base attachment capacity.

Agents suitable for use in these methods preferably contain 20 to 40 bases and are designed to be complementary to a region of the gene involved in transcription (triple helix - see Lee et al., Nucl. Acids Res. 6:3073 (1979); Cooney et al., Science 241:456 (1988); and Dervan et al., Science 251:1360 (1991)) or to the mRNA itself (antisense - Okano, J. Neurochem. 56:560 (1991); Oligodeoxynucleotides as Antisense Inhibitors of Gene Expression, CRC Press, Boca Raton, FL (1988)). Triple helix-formation optimally results in a shut-off of RNA transcription from DNA, while antisense RNA hybridization blocks translation of an mRNA molecule into polypeptide. Both techniques have been demonstrated to be effective in model systems. Information contained in the sequences of the present invention is necessary for the design of an antisense or triple helix oligonucleotide and other DNA binding agents.

Agents which bind to a protein encoded by one of the ORFs of the present invention can be used as a diagnostic agent. Agents which bind to a protein encoded by one of the ORFs of the present invention can be formulated using known techniques to generate a pharmaceutical composition.

### 4.19 USE OF NUCLEIC ACIDS AS PROBES

Another aspect of the subject invention is to provide for polypeptide-specific nucleic acid hybridization probes capable of hybridizing with naturally occurring nucleotide sequences. The hybridization probes of the subject invention may be derived from any of the nucleotide sequences SEQ ID NO: 1-146, or 293-438. Because the corresponding gene is only expressed in a limited number of tissues, a hybridization probe derived from any of the nucleotide sequences SEQ ID NO: 1-146, or 293-438 can be used as an indicator of the presence of RNA of cell type of such a tissue in a sample.

Any suitable hybridization technique can be employed, such as, for example, in situ hybridization. PCR as described in US Patents Nos. 4,683,195 and 4,965,188 provides additional uses for oligonucleotides based upon the nucleotide sequences. Such probes used in PCR may be of recombinant origin, may be chemically synthesized, or a mixture of both. The probe will comprise a discrete nucleotide sequence for the detection of identical sequences or a degenerate pool of possible sequences for identification of closely related genomic sequences.

Other means for producing specific hybridization probes for nucleic acids include the cloning of nucleic acid sequences into vectors for the production of mRNA probes. Such vectors are known in the art and are commercially available and may be used to synthesize RNA probes *in vitro* by means of the addition of the appropriate RNA polymerase as T7 or SP6 RNA polymerase and the appropriate radioactively labeled nucleotides. The nucleotide sequences may be used to construct hybridization probes for mapping their respective genomic sequences. The nucleotide sequence provided herein may be mapped to a chromosome or specific regions of a chromosome using well known genetic and/or chromosomal mapping techniques. These techniques include in situ hybridization, linkage analysis against known chromosomal markers, hybridization screening with libraries or flow-sorted chromosomal preparations specific to known chromosomes, and the like. The technique of fluorescent in situ hybridization of chromosome spreads has been described, among other places, in Verma et al (1988) Human Chromosomes: A Manual of Basic Techniques, Pergamon Press, New York NY.

Fluorescent *in situ* hybridization of chromosomal preparations and other physical chromosome mapping techniques may be correlated with additional genetic map data. Examples of genetic map data can be found in the 1994 Genome Issue of Science (265:1981f). Correlation between the location of a nucleic acid on a physical chromosomal map and a specific disease (or predisposition to a specific disease) may help delimit the region of DNA associated with that genetic disease. The nucleotide sequences of the subject invention may be used to detect differences in gene sequences between normal, carrier or affected individuals.

### 4.20 PREPARATION OF SUPPORT BOUND OLIGONUCLEOTIDES

Oligonucleotides, i.e., small nucleic acid segments, may be readily prepared by, for example, directly synthesizing the oligonucleotide by chemical means, as is commonly practiced using an automated oligonucleotide synthesizer.

Support bound oligonucleotides may be prepared by any of the methods known to those of skill in the art using any suitable support such as glass, polystyrene or Teflon. One strategy is to precisely spot oligonucleotides synthesized by standard synthesizers. Immobilization can be achieved using passive adsorption (Inouye & Hondo, (1990) J. Clin. Microbiol. 28(6) 1469-72); using UV light (Nagata *et al*., 1985; Dahlen *et al*., 1987; Morrissey & Collins, (1989) Mol. Cell Probes 3(2) 189-207) or by covalent binding of base modified DNA (Keller *et al*., 1988; 1989); all references being specifically incorporated herein.

Another strategy that may be employed is the use of the strong biotin-streptavidin interaction as a linker. For example, Broude *et al*. (1994) Proc. Natl. Acad. Sci. USA 91(8) 3072-6, describe the use of biotinylated probes, although these are duplex probes, that are immobilized on streptavidin-coated magnetic beads. Streptavidin-coated beads may be purchased from Dynal, Oslo. Of course, this same linking chemistry is applicable to coating any surface with streptavidin. Biotinylated probes may be purchased from various sources, such as, e.g., Operon Technologies (Alameda, CA).

Nunc Laboratories (Naperville, IL) is also selling suitable material that could be used. Nunc Laboratories have developed a method by which DNA can be covalently bound to the microwell surface termed Covalink NH. CovaLink NH is a polystyrene surface grafted with secondary amino groups (>NH) that serve as bridge-heads for further covalent coupling. CovaLink Modules may be purchased from Nunc Laboratories. DNA molecules may be bound to CovaLink exclusively at the 5'-end by a phosphoramidate bond, allowing immobilization of more than I pmol of DNA (Rasmussen *et al*., (1991) Anal. Biochem. 198(1) 138-42).

The use of CovaLink NH strips for covalent binding of DNA molecules at the 5'-end has been described (Rasmussen et al., (1991). In this technology, a phosphoramidate bond is employed (Chu et al., (1983) Nucleic Acids Res. 11 (8) 6513-29). This is beneficial as immobilization using only a single covalent bond is preferred. The phosphoramidate bond joins the DNA to the CovaLink NH secondary amino groups that are positioned at the end of spacer arms covalently grafted onto the polystyrene surface through a 2 nm long spacer arm. To link an oligonucleotide to CovaLink NH via an phosphoramidate bond, the oligonucleotide terminus must have a 5'-end phosphate group. It is, perhaps, even possible for biotin to be covalently bound to CovaLink and then streptavidin used to bind the probes.

More specifically, the linkage method includes dissolving DNA in water (7.5 ng/µl) and denaturing for 10 min. at 95°C and cooling on ice for 10 min. Ice-cold 0.1 M 1-methylimidazole, pH 7.0 (1-Melm₇), is then added to a final concentration of 10 mM 1-MeIm₇. The single-stranded DNA solution is then dispensed into CovaLinkNH strips (75 µl/well) standing on ice.

Carbodiimide 0.2 M 1-ethyl-3-(3-dimethylaminopropyl)-carbodiimide (EDC), dissolved in 10 mM 1-MeIm₇, is made fresh and 25 µl added per well. The strips are incubated for 5 hours at 50°C. After incubation the strips are washed using, e.g., Nunc-Immuno Wash; first the wells are washed 3 times, then they are soaked with washing solution for 5 min., and finally they are washed 3 times (where in the washing solution is 0.4 N NaOH, 0.25% SDS heated to 50°C).

It is contemplated that a further suitable method for use with the present invention is that described in PCT Patent Application WO 90/03382 (Southern & Maskos), incorporated herein by reference. This method of preparing an oligonucleotide bound to a support involves attaching a nucleoside 3'-reagent through the phosphate group by a covalent phosphodiester link to aliphatic hydroxyl groups carried by the support The oligonucleotide is then synthesized on the supported nucleoside and protecting groups removed from the synthetic oligonucleotide chain under standard conditions that do not cleave the oligonucleotide from the support. Suitable reagents include nucleoside phosphoramidite and nucleoside hydrogen phosphorate.

An on-chip strategy for the preparation of DNA probe for the preparation of DNA probe arrays may be employed. For example, addressable laser-activated photodeprotection may be employed in the chemical synthesis of oligonucleotides directly on a glass surface, as described by Fodor *et al*. (1991) Science 251 (4995) 767-73, incorporated herein by reference. Probes may also be immobilized on nylon supports as described by Van Ness *et al*. (1991) Nucleic Acids Res. 19(12) 3345-50; or linked to Teflon using the method of Duncan & Cavalier (1988) Anal. Biochem. 169(1) 104-8; all references being specifically incorporated herein.

To link an oligonucleotide to a nylon support, as described by Van Ness *et al*. (1991), requires activation of the nylon surface via alkylation and selective activation of the 5'-amine of oligonucleotides with cyanuric chloride.

One particular way to prepare support bound oligonucleotides is to utilize the light-generated synthesis described by Pease *et al.,* (1994) PNAS USA 91(11) 5022-6, incorporated herein by reference). These authors used current photolithographic techniques to generate arrays of immobilized oligonucleotide probes (DNA chips). These methods, in which light is used to direct the synthesis of oligonucleotide probes in high-density, miniaturized arrays, utilize photolabile 5'-protected *N*-acyl-deoxynucleoside phosphoramidites, surface linker chemistry and versatile combinatorial synthesis strategies. A matrix of 256 spatially defined oligonucleotide probes may be generated in this manner.

### 4.21 PREPARATION OF NUCLEIC ACID FRAGMENTS

The nucleic acids may be obtained from any appropriate source, such as cDNAs, genomic DNA, chromosomal DNA, microdissected chromosome bands, cosmid or YAC inserts, and RNA, including mRNA without any amplification steps. For example, Sambrook *et al.* (1989) describes three protocols for the isolation of high molecular weight DNA from mammalian cells (p. 9.14-9.23).

DNA fragments may be prepared as clones in M13, plasmid or lambda vectors and/or prepared directly from genomic DNA or cDNA by PCR or other amplification methods. Samples may be prepared or dispensed in multiwell plates. About 100-1000 ng of DNA samples may be prepared in 2-500 ml of final volume.

The nucleic acids would then be fragmented by any of the methods known to those of skill in the art including, for example, using restriction enzymes as described at 9.24-9.28 of Sambrook *et al*. (1989), shearing by ultrasound and NaOH treatment.

Low pressure shearing is also appropriate, as described by Schriefer *et al.* (1990) Nucleic-Acids Res. 18(24) 7455-6, incorporated herein by reference). In this method, DNA samples are passed through a small French pressure cell at a variety of low to intermediate pressures. A lever device allows controlled application of low to intermediate pressures to the cell. The results of these studies indicate that low-pressure shearing is a useful alternative to sonic and enzymatic DNA fragmentation methods.

One particularly suitable way for fragmenting DNA is contemplated to be that using the two base recognition endonuclease, *Cvi*JI, described by Fitzgerald *et al*. (1992) Nucleic Acids Res. 20(14)3753-62. These authors described an approach for the rapid fragmentation and fractionation of DNA into particular sizes that they contemplated to be suitable for shotgun cloning and sequencing.

The restriction endonuclease *Cvi*JI normally cleaves the recognition sequence PuGCPy between the G and C to leave blunt ends. Atypical reaction conditions, which alter the specificity of this enzyme (*Cvi*JI**), yield a quasi-random distribution of DNA fragments form the small molecule pUC19 (2688 base pairs). Fitzgerald *et al.* (1992) quantitatively evaluated the randomness of this fragmentation strategy, using a *Cvi*JI** digest of pUC 19 that was size fractionated by a rapid gel filtration method and directly ligated, without end repair, to a lac Z minus M13 cloning vector. Sequence analysis of 76 clones showed that *Cvi*JI** restricts pyGCPy and PuGCPu, in addition to PuGCPy sites, and that new sequence data is accumulated at a rate consistent with random fragmentation.

As reported in the literature, advantages of this approach compared to sonication and agarose gel fractionation include: smaller amounts of DNA are required (0.2-0.5 µg instead of 2-5 µg); and fewer steps are involved (no preligation, end repair, chemical extraction, or agarose gel electrophoresis and elution are needed

Irrespective of the manner in which the nucleic acid fragments are obtained or prepared, it is important to denature the DNA to give single stranded pieces available for hybridization. This is achieved by incubating the DNA solution for 2-5 minutes at 80-90°C. The solution is then cooled quickly to 2°C to prevent renaturation of the DNA fragments before they are contacted with the chip. Phosphate groups must also be removed from genomic DNA by methods known in the art.

### 4.22 PREPARATION OF DNA ARRAYS

Arrays may be prepared by spotting DNA samples on a support such as a nylon membrane. Spotting may be performed by using arrays of metal pins (the positions of which correspond to an array of wells in a microtiter plate) to repeated by transfer of about 20 nl of a DNA solution to a nylon membrane. By offset printing, a density of dots higher than the density of the wells is achieved. One to 25 dots may be accommodated in 1 mm², depending on the type of label used. By avoiding spotting in some preselected number of rows and columns, separate subsets (subarrays) may be formed. Samples in one subarray may be the same genomic segment of DNA (or the same. gene) from different individuals, or may be different, overlapped genomic clones. Each of the subarrays may represent replica spotting of the same samples. In one example, a selected gene segment may be amplified from 64 patients. For each patient, the amplified gene segment may be in one 96-well plate (all 96 wells containing the same sample). A plate for each of the 64 patients is prepared. By using a 96-pin device, all samples may be spotted on one 8 x 12 cm membrane. Subarraysmay contain 64 samples, one from each patient. Where the 96 subarrays are identical, the dot span may be 1 mm² and there may be a 1 mm space between subarrays.

Another approach is to use membranes or plates (available from NUNC, Naperville, Illinois) which may be partitioned by physical spacers e.g. a plastic grid molded over the membrane, the grid being similar to the sort of membrane applied to the bottom of multiwell plates, or hydrophobic strips. A fixed physical spacer is not preferred for imaging by exposure to flat phosphor-storage screens or x-ray films

The present invention is illustrated in the following examples. Upon consideration of the present disclosure, one of skill in the art will appreciate that many other embodiments and variations may be made in the scope of the present invention. Accordingly, it is intended that the broader aspects of the present invention not be limited to the disclosure of the following examples. The present invention is not to be limited in scope by the exemplified embodiments which are intended as illustrations of single aspects of the invention, and compositions and methods which are functionally equivalent are within the scope of the invention. Indeed, numerous modifications and variations in the practice of the invention are expected to occur to those skilled in the art upon consideration of the present preferred embodiments. Consequently, the only limitations which should be placed upon the scope of the invention are those which appear in the appended claims.

All references cited within the body of the instant specification are hereby incorporated by reference in their entirety.

### 5. EXAMPLES

### 5.1 EXAMPLE 1

### Novel Nucleic Acid Sequences Obtained From Various Libraries

A plurality of novel nucleic acids were obtained from cDNA libraries prepared from various human tissues and in some cases isolated from a genomic library derived from human chromosome using standard PCR, SBH sequence signature analysis and Sanger sequencing techniques. The inserts of the library were amplified with PCR using primers specific for the vector sequences which flank the inserts. Clones from cDNA libraries were spotted on nylon membrane filters and screened with oligonucleotide probes (e.g., 7-mers) to obtain signature sequences. The clones were clustered into groups of similar or identical sequences. Representative clones were selected for sequencing.

In some cases, the 5' sequence of the amplified inserts was then deduced using a typical Sanger sequencing protocol. PCR products were purified and subjected to fluorescent dye terminator cycle sequencing. Single pass gel sequencing was done using a 377 Applied Biosystems (ABI) sequencerto obtain the novel nucleic acid sequences. In some cases RACE (Rapid Amplification of cDNA Ends) was performed to further extend the sequence in the 5' direction.

### 5.2 EXAMPLE 2

### Assemblage of Novel Nucleic Acids

The contigs or nucleic acids of the present invention, designated as SEQ ID NO: 293-438 were assembled using an EST sequence as a seed. Then a recursive algorithm was used to extend the seed EST into an extended assemblage, by pulling additional sequences from different databases (i.e., Hyseq's database containing EST sequences, dbEST version 115, gb pri 115, and UniGene version 103, and exons from public domain genomic sequences predicated by GenScan) that belong to this assemblage. The algorithm terminated when there was no additional sequences from the above databases that would extend the assemblage. Further, inclusion of component sequences into the assemblage was based on a BLASTN hit to the extending assemblage with BLAST score greater than 300 and percent identity greater than 95%.

Table 6 sets forth the novel predicted polypeptides (including proteins) encoded by the novel polynucleotides (SEQ ID NO: 293-438) of the present invention, and their corresponding translation start and stop nucleotide locations to each of SEQ ID NO: 293-438. Table 6 also indicates the method by which the polypeptide was predicted. Method A refers to a polypeptide obtained by using a software program called FASTY (available from http://fasta.bioch.virginia,edu) which selects a polypeptide based on a comparison of the translated novel polynucleotide to known polynucleotides(W.R. Pearson, Methods in Enzymology, 183:63-98 (1990), herein incorporated by reference). Method B refers to a polypeptide obtained by using a software program called GenScan for human/vertebrate sequences (available from Stanford University, Office of Technology Licensing) that predicts the polypeptide based on a probabilistic model of gene structure/compositionalproperties (C. Burge and S. Karlin, J. Mol. Biol., 268:78-94 (1997), incorporated herein by reference). Method C refers to a polypeptide obtained by using a Hyseq proprietary software program that translates the novel polynucleotide and its complementary strand into six possible amino acid sequences (forward and reverse frames) and chooses the polypeptide with the longest open reading frame.

### 5.3 EXAMPLE 3

### Novel Nucleic Acids

Using PHRAP (Univ. of Washington) or CAP4 (Paracel), full-length gene cDNA sequences and their corresponding protein sequences were generated from the assemblage. Any frame shifts and incorrect stop codons were corrected by hand editing. During editing, the sequence was checked using FASTXY algorithm against Genbank (i.e., dbEST version 118, gb pri 118, UniGene version 118, Genpept release 118). Other computer programs which may have been used in the editing process were phredPhrap and Consed ((University of Washington) and ed-ready, ed-ext and gc-zip-2 (Hyseq, Inc.)). The full-length nucleotide sequences are shown in the Sequence Listing as SEQ ID NO: 1-90. The corresponding polypeptide sequences are SEQ ID NO: 147-236.

Table 1 shows the various tissue sources of SEQ ID NO: 1-90.

The nearest neighbor results for SEQ ID NO: 1-90 were obtained by a BLASTP (version 2.0al 19MP-WashU) search against Genpept release 122 and Geneseq release 200105 (Derwent), using BLAST algorithm. The nearest neighbor result showed the closest homologue for SEQ ID NO: 1-90 from Genpept. The translated amino acid sequences for which the nucleic acid sequence encodes are shown in the Sequence Listing. The homologs with identifiable functions for SEQ ID NO: 1-90 are shown in Table 2 below.

Using eMatrix software package (Stanford University, Stanford, CA) (Wu et al., J. Comp. Biol., Vol. 6 pp. 219-235 (1999) herein incorporated by reference), all the sequences were examined to determine whether they had identifiable signature regions. Table 3 shows the signature region found in the indicated polypeptide sequences, the description of the signature, the eMatrix p-value(s) and the position(s) of the signature within the polypeptide sequence.

Using the pFam software program (Sonnhammer et al., Nucleic Acids Res., Vol. 26(1) pp. 320-322 (1998) herein incorporated by reference) all the polypeptide sequences were examined for domains with homology to certain peptide domains. Table 4 shows the name of the domain found, the description, the p-value and the pFam score for the identified domain within the sequence.

The nucleotide sequence within the sequences that codes for signal peptide sequences and their cleavage sites can be determine from using Neural Network SignalP V1.1 program (from Center for Biological Sequence Analysis, The Technical University of Denmark). The process for identifying prokaryotic and eukaryotic signal peptides and their cleavage sites are also disclosed by Henrik Nielson, Jacob Engelbrecht, Soren Brunak, and Gunnar von Heijne in the publication " Identification of prokaryotic and eukaryotic signal peptides and prediction of their cleavage sites" Protein Engineering, Vol. 10, no. 1,pp. 1-6 (1997), incorporated herein by reference. A maximum S score and a mean S score, as described in the Nielson et al, as reference, was obtained for the polypeptide sequences. Table 7 shows the position of the signal peptide in each of the polypeptides and the maximum score and mean score associated with that signal peptide.

### 5.4 EXAMPLE 4

### Novel Nucleic Acids

Using PHRAP (Univ. of Washington) or CAP4 (Paracel), a full length gene cDNA sequence and its correspondingprotein sequence were generated from the assemblage. Any frame shifts and incorrect stop codons were corrected by hand editing. During editing, the sequence was checked using FASTY and/or BLAST against Genbank (i.e. dbEST version 119, gb pri 119, UniGene version 119, Genpept release 119) Other computer programs which may have been used in the editing process were phredPhrap and Consed (University of Washington) and ed-ready, ed-ext and gc-zip-2 (Hyseq, Inc.). The full-length nucleotide, including splice variants resulting from these procedures are shown in the Sequence Listing as SEQ ID NO: 91-105. The corresponding polypeptide sequences are SEQ ID NO: 237-251.

Table 1 shows the various tissue sources of SEQ ID NO: 91-105.

The nearest neighbor results for SEQ ID NO: 91-105 were obtained by a BLASTP version 2.0al 19MP-WashU search against Genpept release 122 and Geneseq release 200105 (Derwent), using BLAST algorithm. The nearest neighbor result showed the closest homologue for SEQ ID NO: 91-105 from Genpept. The homologs with identifiable functions for SEQ ID NO: 91-105 are shown in Table 2 below.

Using eMatrix software package (Stanford University, Stanford, CA) (Wu et al., J. Comp. Biol., Vol. 6 pp. 219-235 (1999) herein incorporated by reference), all the sequences were examined to determine whether they had identifiable signature regions. Table 3 shows the signature region found in the indicated polypeptide sequences, the description of the signature, the eMatrix p-value(s) and the position(s) of the signature within the polypeptide sequence.

Using the pFam software program (Sonnhammer et al., Nucleic Acids Res., Vol. 26(1) pp. 320-322 (1998) herein incorporated by reference) all the polypeptide sequences were examined for domains with homology to certain peptide domains. Table 4 shows the name of the domain found, the description, the p-value and the pFam score for the identified domain within the sequence.

The nucleotide sequence within the sequences that codes for signal peptide sequences and their cleavage sites can be determine from using Neural Network SignalP V1.1 program (from Center for Biological Sequence Analysis, The Technical University of Denmark). The process for identifying prokaryotic and eukaryotic signal peptides and their cleavage sites are also disclosed by Henrik Nielson, Jacob Engelbrecht, Soren Brunak, and Gunnar von Heijne in the publication " Identification of prokaryotic and eukaryotic signal peptides and prediction of their cleavage sites" Protein Engineering, Vol. 10, no. 1, pp. 1-6 (1997), incorporated herein by reference. A maximum S score and a mean S score, as described in the Nielson et al as reference, was obtained for the polypeptide sequences. Table 7 shows the position of the signal peptide in each of the polypeptides and the maximum score and mean score associated with that signal peptide.

### 5.5 EXAMPLE 5

### Novel Nucleic Acids

Using PHRAP (Univ. of Washington) or CAP4 (Paracel), a full-length gene cDNA sequence and its corresponding protein sequence were generated from the assemblage. Any frame shifts and incorrect stop codons were corrected by hand editing. During editing, the sequence was checked using FASTY and/or BLAST against Genbank (i.e., dbEST version 120, gb pri 120, UniGene version 120, Genpept release 120). Other computerprograms which may have been used in the editing process were phredPhrap and Consed (University of Washington) and ed-ready, ed-ext and gc-zip-2 (Hyseq, Inc.). The full-length nucleotide, including splice variants resulting from these procedures are shown in the Sequence Listing as SEQ ID NO:106-119. The corresponding polypeptide sequences are SEQ ID NO: 252-265.

Table 1 shows the various tissue sources of SEQ ID NO: 106-119.

The homology results for SEQ ID NO: 106-119 were obtained by a BLASTP version 2.0al 19MP-WashU search against Genpept release 122 and Geneseq release 200105 (Derwent), using BLAST algorithm. The nearest neighbor result showed the homologs for SEQ ID NO: 106-119 from Genpept. The translated amino acid sequences for which the nucleic acid sequence encodes are shown in the Sequence Listing. The homologues with identifiable functions for SEQ ID NO: 106-119 are shown in Table 2 below.

Using eMatrix software package (Stanford University, Stanford, CA) (Wu et al., J. Comp. Biol., Vol. 6 pp. 219-235 (1999) herein incorporated by reference), all the sequences were examined to determine whether they had identifiable signature regions. Table 3 shows the signature region found in the indicated polypeptide sequences, the description of the signature, the eMatrix p-value(s) and the position(s) of the signature within the polypeptide sequence.

Using the pFam software program (Sonnhammer et al., Nucleic Acids Res., Vol. 26(1) pp. 320-322 (1998) herein incorporated by reference) all the polypeptide sequences were examined for domains with homology to certain peptide domains. Table 4 shows the name of the domain found, the description, the p-value and the pFam score for the identified domain within the sequence.

The nucleotide sequence within the sequences that codes for signal peptide sequences and their cleavage sites can be determine from using Neural Network SignalP V 1.1 program (from Center for Biological Sequence Analysis, The Technical University of Denmark). The process for identifying prokaryotic and eukaryotic signal peptides and their cleavage sites are also disclosed by Henrik Nielson, Jacob Engelbrecht, Soren Brunak, and Gunnar von Heijne in the publication " Identification of prokaryotic and eukaryotic signal peptides and prediction of their cleavage sites" Protein Engineering, Vol. 10, no. 1, pp. 1-6 (1997), incorporated herein by reference. A maximum S score and a mean S score, as described in the Nielson et al as reference, was obtained for the polypeptide sequences. Table 7 shows the position of the signal peptide in each of the polypeptides and the maximum score and mean score associated with that signal peptide.

### 5.6 EXAMPLE 6

### Novel Nucleic Acids

Using PHRAP (Univ. of Washington) or CAP4 (Paracel), a full-length gene cDNA sequence and its correspondingprotein sequence were generated from the assemblage. Any frame shifts and incorrect stop codons were corrected by hand editing. During editing, the sequence was checked using FASTY and/or BLAST against Genbank (i.e., dbEST version 121, gb pri 121, UniGene version 121, Genpept release 121). Other computer programs which may have been used in the editing process were phredPhrap and Consed (University of Washington) and ed-ready, ed-ext and gc-zip-2 (Hyseq, Inc.). The full-length nucleotide, including splice variants resulting from these procedures are shown in the Sequence Listing as SEQ ID NO: 120-128. The corresponding amino acid sequences are SEQ ID NO: 266-274.

Table I shows the various tissue sources of SEQ ID NO: 120-128.

The homology results for SEQ ID NO: 120-128 were obtained by a BLASTP version 2.0al 19MP-WashU search against Genpept release 122 and Geneseq release 200105 (Derwent), using BLAST algorithm. The nearest neighbor result showed the homologs for SEQ ID NO: 120-128 from Genpept. The translated amino acid sequences for which the nucleic acid sequence encodes are shown in the Sequence Listing. The homologues with identifiable functions for SEQ ID NO: 120-128 are shown in Table 2 below.

Using eMatrix software package (Stanford University, Stanford, CA) (Wu et al., J. Comp. Biol., Vol. 6 pp. 219-235 (1999) herein incorporated by reference), all the sequences were examined to determine whether they had identifiable signature regions. Table 3 shows the signature region found in the indicated polypeptide sequences, the description of the signature, the eMatrix p-value(s) and the position(s) of the signature within the polypeptide sequence.

Using the pFam software program (Sonnhammer et al., Nucleic Acids Res., Vol. 26(1) pp. 320-322 (1998) herein incorporated by reference) all the polypeptide sequences were examined for domains with homology to certain peptide domains. Table 4 shows the name of the domain found, the description, the p-value and the pFam score for the identified domain within the sequence.

The nucleotide sequence within the sequences that codes for signal peptide sequences and their cleavage sites can be determine from using Neural Network SignalP V1.1 program (from Center for Biological Sequence Analysis, The Technical University of Denmark). The process for identifying prokaryotic and eukaryotic signal peptides and their cleavage sites are also disclosed by Henrik Nielson, Jacob Engelbrecht, Soren Brunak, and Gunnar von Heijne in the publication " Identification of prokaryotic and eukaryotic signal peptides and prediction of their cleavage sites" Protein Engineering, Vol. 10, no. 1, pp. 1-6 (1997), incorporated herein by reference. A maximum S score and a mean S score, as described in the Nielson et al as reference, was obtained for the polypeptide sequences. Table 7 shows the position of the signal peptide in each of the polypeptides and the maximum score and mean score associated with that signal peptide.

### 5.7 EXAMPLE 7

### Novel Nucleic Acids

Using PHRAP (Univ. of Washington) or CAP4 (Paracel), a full-length gene cDNA sequence and its corresponding protein sequence were generated from the assemblage. Any frame shifts and incorrect stop codons were corrected by hand editing. During editing, the sequence was checked using FASTY and/or BLAST against Genbank (i.e., dbEST version, gb pri, UniGene, Genpept). Other computer programs which may have been used in the editing process were phredPhrap and Consed (University of Washington) and ed-ready, ed-ext and gc-zip-2 (Hyseq, Inc.). The full-length nucleotide, including splice variants resulting from these procedures are shown in the Sequence Listing as SEQ ID NO: 129-146. The corresponding amino acid sequences are SEQ ID NO: 275-292.

Table 1 shows the various tissue sources of SEQ ID NO: 129-146.

The homology results for SEQ ID NO: 129-146 were obtained by a BLASTP version 2.0al 19MP-WashU search against Genpept release 122 and Geneseq release 200105 (Derwent), using BLAST algorithm. The nearest neighbor result showed the homologs for SEQ ID NO: 129-146 from Genpept. The translated amino acid sequences for which the nucleic acid sequence encodes are shown in the Sequence Listing. The homologues with identifiable functions for SEQ ID NO: 129-146 are shown in Table 2 below.

Using eMatrix software package (Stanford University, Stanford, CA) (Wu et al., J. Comp. Biol., Vol. 6 pp. 219-235 (1999) herein incorporated by reference), all the sequences were examined to determine whether they had identifiable signature regions. Table 3 shows the signature region found in the indicated polypeptide sequences, the description of the signature, the eMatrix p-value(s) and the position(s) of the signature within the polypeptide sequence.

Using the pFam software program (Sonnhammer et al., Nucleic Acids Res., Vol. 26(1) pp. 320-322 (1998) herein incorporated by reference) all the polypeptide sequences were examined for domains with homology to certain peptide domains. Table 4 shows the name of the domain found, the description, the p-value and the pFam score for the identified domain within the sequence.

The nucleotide sequence within the sequences that codes for signal peptide sequences and their cleavage sites can be determine from using Neural Network SignalP V1.1 program (from Center for Biological Sequence Analysis, The Technical University of Denmark). The process for identifying prokaryotic and eukaryotic signal peptides and their cleavage sites are also disclosed by Henrik Nielson, Jacob Engelbrecht, Soren Brunak, and Gunnar von Heijne in the publication " Identification of prokaryotic and eukaryotic signal peptides and prediction of their cleavage sites" Protein Engineering, Vol. 10, no. 1, pp. 1-6 (1997), incorporated herein by reference. A maximum S score and a mean S score, as described in the Nielson et al as reference, was obtained for the polypeptide sequences. Table 7 shows the position of the signal peptide in each of the polypeptides and the maximum score and mean score associated with that signal peptide.

Table 5 is a correlation table of all of the sequences and SEQ ID Nos.

**Table 1**

| **Tissue origin** | **RNA Source** | **Library Name** | **SEQ ID NOS:** |
|---|---|---|---|
| adult brain | GIBCO | AB3001 | 10 16 26 32 36 38 52-53 67 69 87 90 102 112 |
| adult brain | GIBCO | ABD003 | 10 19-20 23 28 32 37 41-42 44 49 51 53 55-56 61-64 69 71 75 81 83 87 89-90 99 102 110 118 133 135 139 |
| adult brain | Clontech | ABR001 | 23 82 90 99 102 112 129 |
| adult brain | Clontech | ABR006 | 10 90 96 109-110 120 131 141-142 |
| adult brain | Clontech | ABR008 | 6 10-13 16-17 19-23 25-27 29 31-36 45 47 49-50 61 64 68-69 73 75 79 85 89-90 92-94 99 101 103-104 107-108 112 117 122-123 126-127 130-131 135-137 139 141-142 146 |
| adult brain | Clontech | ABR011 | 121 |
| adult brain | BioChain | ABR012 | 37 |
| adult brain | Invitrogen | ABR013 | 36 |
| adult brain | Invitrogen | ABR014 | 64 136-137 144 |
| adult brain | Invitrogen | ABR015 | 99 141-142 |
| adult brain | Invitrogen | ABT004 | 610 17 22 27 31-32 36 38 51 64 67 90 95 98 141-142 |
| cultured preadipocytes | Stratagene | ADP001 | 4 12-13 22 37 42 71 101-102 106 126 141-142 |
| adrenal gland | Clontech | ADR002 | 8 10 22 27 32-33 37 42 48-49 51 54 61 66 69 81 84 95-96 100 110 116-117 122 128 134-135 141-142 |
| adult heart | GIBCO | AHR001 | 4 6 8 10-11 13 17 22-23 25 27-29 31-34 37 41-42 44 48 50 61-62 64 66 69-71 74-75 85 89 95-99 103-104 110 112 116 118 132 136-137 |
| adult kidney | GIBCO | AKD001 | 3-4 6 8 10-11 13 15 17 20-24 27-29 32-37 41-42 44 49-51 53 56 60-66 69-71 74 79 84-89 92-93 96 98-99 102-103 110 113 116 118-119 125 130 132 135 138 145 |
| adult kidney | Invitrogen | AKT002 | 3 8 27 29 32 37-38 52 60-61 71 74 84 899395101 110 113 118 126 130 135 138 140 146 |
| adult lung | GIBCO | ALG001 | 6 17-18 23 27 33 37 42 45 49 63 71 99 132 138 |
| lymph node | Clontech | ALN001 | 8 17 29 37 42 53 56 61 64 110 118 125-126 |
| young liver | GIBCO | ALV001 | 4 6 8 10 18 22 29 32 48-49 62 74 92 100 102 118 124 |
| adult liver | Invitrogen | ALV002 | 6 8 11 18 22 24-25 27 32-33 36 41 49 63-65 99-101 113 116 134 |
| adult liver | Clontech | ALV003 | 136-137 |
| adult ovary | Invitrogen | AOV001 | 1 3-4 6 8-13 16-17 19-23 27-29 31-35 37-38 40 42 44 48-52 56-58 60-61 63-64 66 69-71 74-75 79 81 84-85 87-88 92-93 98-99 101-104 107 110 112 116 118 120 122 124 126 128 130 132-133 138 141-142 |
| adult placenta | Clontech | APL001 | 4 29 49 92 139 |
| placenta | Invitrogen | APL002 | 35 44 50-51 88 98 141-142 |
| adult spleen | GIBCO | ASP001 | 6 10-11 16-17 32 37 41 47 51 56 61 63 71-72 75 77 96101 112 118 122 134 136-137 |
| testis | GIBCO | ATS001 | 4 6 10-12 23 27 33-34 37 61 71 79 99 118 120 126 130 132 135 |
| adult bladder | Invitrogen | BLD001 | 4 22 27 48 72 86 90 133 |
| bone marrow | Clontech | BMD001 | 13 5 8-9 20-21 23 26-27 30 32 37-38 40 42 44 50-53 56 58-61 64 71-72 74-75 77 83 86 88-89 93 95 100 104 107 111 116 118-121 126 131-132 135-137 139-140 144 |
| bone marrow | Clontech | BMD002 | 2-3 5 11 13 26 37 42 47 50 56 61 67-68 71-72 77 79 91 95 100-101 104 107 110 113-114 116 119 121-122 136-137 144 |
| bone marrow | Clontech | BMD004 | 77 |
| bone marrow | Clonetech | BMD007 | 77 136-137 |
| colon | Invitrogen | CLN001 | 32 35 51 87 |
| Mixture of 16 tissues -mRNAs* | Various Vendors* | CTL021 | 5 49 71 77 |
| adult cervix | BioChain | CVX001 | 1 4 8 11 13 19-20 26 28 32 41 44 51-52 59 6166 7174 80 83 101 104 118 122 125 132-133 141-142 |
| endothelial cells | Stratagene | EDT001 | 4 6 8-13 16-17 21-24 27-29 32-33 35 37 41-42 44 46-47 49-50 52 56 58 61 64 66 69-71 74 82 84 86 99-101 110 112 116 118 121 133 135 138 |
| fetal brain | Clontech | FBR001 | 124 |
| fetal brain | Clontech | FBR004 | 120 127 |
| fetal brain | Clontech | FBR006 | 8 13 22 25 29 35 41-42 49 64 69 73 75-76 79 92-93 100-101 112 122 124-125 127130135 |
| fetal brain | Invitrogen | FBT002 | 22 29 34 49-50 89 104 134 |
| fetal heart | Invitrogen | FHR001 | 79118 122 131 146 |
| fetal kidney | Clontech | FKD001 | 12 32 37 41-42 49 71 105 119 132 |
| fetal kidney | Clontech | FKD002 | 61 143 |
| fetal kidney | Invitrogen | FKD007 | 12 |
| fetal lung | Clontech | FLG001 | 3 34 56 61 77 95 |
| fetal lung | Invitrogen | FLG003 | 4 56 122 124 136-137 |
| fetal lung | Clontech | FLG004 | 61 |
| fetal liver-spleen | Columbia University | FLS001 | 1-9 11-25 27 29-30 32 35-38 41-51 56 58 60-66 68-71 74-75 77-82 84-88 91-99101 103-105 107-116 118-119 122- |
| | | | 127 129-138 141-143 145-146 |
| fetal liver-spleen | Columbia University | FLS002 | 3-4 7 911 14-15 17-20 22-23 25-27 29-30 32 38 41-43 45 47 49-51 53 56 58 63 66 68-71 74-75 77 79 83 85-87 93 95 97-98 100 104-105 107 110 112-114 116 118-119 122 124-127 132 134 136-138 141-142 145 |
| fetal liver-spleen | Columbia University | FLS003 | 5 83 85 104 124 126 136-137 |
| fetal liver | Invitrogen | FLV001 | 5-6 14-15 18 25 27 29 32 36 47 51 68 84 88 95 107 115-116 136-137 |
| fetal liver | Clontech | FLV002 | 136-137 |
| fetal liver | Clontech | FLV004 | 27 47 95 106 113 |
| fetal muscle | Invitrogen | FMS001 | 27 41 47 61 71 77 86 88 99-100 122 133 136-137 145 |
| fetal muscle | Invitrogen | FMS002 | 1 61 88 91 121 145-146 |
| fetal skin | Invitrogen | FSK001 | 1 3-6 8 12 14 19 29 35-36 41 47 51 61 69 71 81 84 86-88 91 95 102 104 111-113 124 138 |
| fetal skin | Invitrogen | FSK002 | 99 102 110 127-128 |
| fetal spleen | BioChain | FSP001 | 61 |
| umbilical cord | BioChain | FUC001 | 4-5 9-12 14 23 28-29 32 37-38 42 45 51 56 61 66 71 82 84-85 90 97 102 104 106 113 116 125 136-137 139 |
| fetal brain | GIBCO | HFB001 | 6-8 10-13 16-17 19 23 26-27 29 31-33 35-36 41-42 48 50 55-56 58 61 66 71 73-74 76 86 90 92 96 99 103 110117 125-126128 131 138-139 143 |
| macrophage | Invitrogen | HMP001 | 21 132 |
| infant brain | Columbia University | IB2002 | 5-7 10-11 19 22-23 25-26 31 36 38 48-49 51 54-55 61 64 69 73-75 85-87 90 92-93 96 99-100103 108 110118 125 127 134136-137 |
| infant brain | Columbia University | IB2003 | 38 51 61 85-86 88-90 99 117 123 125-126 |
| infant brain | Columbia University | IBM002 | 86 |
| infant brain | Columbia University | IBS001 | 58 90 96 100 117 |
| lung, fibroblast | Stratagene | LFB001 | 4 8 10 13 27 34 37 42 52 56 63 71 96 116 122 126 |
| fibroblast | Stratagene | LFB001 | 4 8 10 13 27 34 37 42 52 56 63 71 96 116 122 126 |
| lung tumor | Invitrogen | LGT002 | 4 7-9 11 13 17 19-20 23 31-32 35 37 41-42 49-51 55-56 60 62-64 71 74-75 78 84-85 88 93 96 99 101-102 105 112 118 124 126 130 139 |
| lymphocytes | ATCC | LPC001 | 10 13 21 34-35 49 58 61 85-86 89 101 103 112 121 128 136-137 |
| leukocyte | GIBCO | LUC001 | 6-11 13 17 23 26 29 32-35 37 41-42 44 51-52 56 58-61 63-64 66 71-72 74-75 85 87-89 96 103-104 107 110 112-113 117-118 121 126 130 132 135-139 |
| leukocyte | Clontech | LUC003 | 13 15 17 37 51 61 139 |
| melanoma from cell line ATCC#CRL 1424 | Clontech | MEL004 | 8 13 28 40 42 49-50 62 69 71 86 126 134 |
| mammary gland | Invitrogen | MMG001 | 10-11 13-15 17-18 22-23 27 29 32 35-37 41-42 45 49-51 58 61 63 71-72 85-88 93 95 98 104 107 110 122 124-125 130-131 133-135 141-142 |
| induced neuron cells | Stratagene | NTD001 | 6 10 12 31 41 56 61 81 102 110 127 |
| retinoic acid induced neuronal cells | Stratagene | NTR001 | 145 |
| neuronal cells | Stratagene | NTU001 | 8 47 49 61 124 129 135 146 |
| pituitary gland | Clontech | PIT004 | 52 81-82 132 135-137 |
| placenta | Clontech | PLA003 | 139 |
| prostate | Clontech | PRT001 | 10 23 28 49 54 60-61 64 69 133 135 138 |
| rectum | Invitrogen | REC001 | 8 28-29 32 36 38 47 72 84-85 90 110 132-133 135 |
| salivary gland | Clontech | SAL001 | 6 44 51-52 56 64 110 141-142 |
| small intestine | Clontech | SIN001 | 4 38 60 71-72 74 89 94 112 117-118 120 125-126 133 135 138 141-142 |
| skeletal muscle | Clontech | SKM001 | 8 70 130 |
| spinal cord | Clontech | SPC001 | 1 3 14 63 70 93 99 112118 122 126 136-137 139 146 |
| adult spleen | Clontech | SPLc01 | 36 42 49 61 89 120122 141-142 |
| stomach | Clontech | STO001 | 48 50 88 102 110 120 122 |
| thalamus | Clontech | THA002 | 8 22 42 54 88 90 117 |
| thymus | Clonetech | THM001 | 7 9 20 22 33 36-37 41 57 61 71 74 77 81 85-86 88 93 96 114 132-134 136-137 |
| thymus | Clontech | THMc02 | 10-112933 37 50 53 59 77 79 88 91 100 103 111-112 118-119 141-142 |
| thyroid gland | Clontech | THR001 | 1 4 810 23 25 28-29 34 38 44-45 51-52 61 63-64 71 76 82 87 93 105 110 112 124 126 129 132 135 143 |
| trachea | Clontech | TRC001 | 8 23 42 56 59 71 132 139 |
| uterus | Clontech | UTR001 | 10 32 41 53 59 61-62 99 145 |

| | | | |
|---|---|---|---|
| *The 16 tissue-mRNAs and their vendor source, are as follows: 1) Normal adult brain mRNA (Invitrogen), 2) normal adult kidney mRNA (Invitrogen), 3) normal adult liver mRNA (Invitrogen), 4) normal fetal brain mRNA (Invitrogen), 5) normal fetal kidney mRNA (Invitrogen), 6) normal fetal liver mRNA (Invitrogen), 7) normal fetal skin mRNA (Invitrogen), 8) human adrenal gland mRNA (Clontech), 9) human bone marrow mRNA (Clontech), 10) human leukemia lymphablastic mRNA (Clontech), 11) human thymus mRNA (Clontech), 12) human lymph node mRNA (Clontech), 13) human spinal cord mRNA (Clontech), 14) human thyroid mRNA (Clontech), 15) human esophagus mRNA (BioChain), 16) human conceptional umbilical cord mRNA (BioChain). | | | |

**Table 2**

| **SEQ ID NO:** | **Accession Number** | **Species** | **Description** | **Smith- Waterman Score** | **% Identity** |
|---|---|---|---|---|---|
| 1 | U11031 | Rattus norvegicus | BIG-1 protein | 213 | 25 |
| 2 | AF156961 | Homo sapiens | gag | 1762 | 91 |
| 4 | Y70744 | Homo sapiens | PSEQ-2 protein encoded by NSEQ gene associated with matrix remodelling. | 539 | 100 |
| 5 | V00488 | Homo sapiens | alpha globin | 733 | 100 |
| 6 | Y79507 | Homo sapiens | Human carbohydrate-associated protein CRBAP-3. | 1521 | 100 |
| 7 | B45366 | Homo sapiens | Human secreted protein sequence encoded by gene 25 SEQ ID NO:118. | 221 | 65 |
| 8 | X60036 | Homo sapiens | phosphate carrier protein | 1897 | 100 |
| 9 | X54942 | Homo sapiens | Cksl protein homologue | 439 | 100 |
| 10 | X56468 | Homo sapiens | 14.3.3 protein | 1246 | 100 |
| 11 | X78136 | Homo sapiens | hnRNP-E2 | 1850 | 99 |
| 12 | AF129332 | Homo sapiens | MUM2 | 767 | 100 |
| 13 | AJ005259 | Homo sapiens | homologous to Bombyx mori multiprotein bridging factor (EMBL: AB001078) | 749 | 100 |
| 14 | B43515 | Homo sapiens | Human cancer associated protein sequence SEQ ID NO:960. | 640 | 99 |
| 15 | K03473 | Homo sapiens | metallothionein I-F | 380 | 100 |
| 16 | AF161472 | Homo sapiens | HSPC123 | 496 | 78 |
| 17 | X98253 | Homo sapiens | ZNF183 | 1860 | 100 |
| 18 | M36803 | Homo sapiens | hemopexin | 2603 | 100 |
| 19 | AL031652 | Homo sapiens | dJ1119D9.3 (novel protein) | 1442 | 100 |
| 20 | AF019980 | Dictyosteli um discoideu m | ZipA | 173 | 24 |
| 21 | AF292100 | Homo sapiens | RP42 protein | 1365 | 99 |
| 22 | J02888 | Homo sapiens | quinone oxidoreductase | 513 | 95 |
| 23 | W67891 | Homo sapiens | Human secreted protein encoded by gene 85 clone HBXFG80. | 2044 | 100 |
| 24 | AF129756 | Homo sapiens | Apo M | 1016 | 100 |
| 25 | U87318 | Xenopus laevis | NaDC-2 | 1161 | 55 |
| 26 | AF241785 | Homo sapiens | NPD012 | 1418 | 100 |
| 27 | X12517 | Homo sapiens | C protein (AA 1-159) | 918 | 100 |
| 28 | X70476 | Homo sapiens | subunit of coatomer complex | 4751 | 100 |
| 29 | BC001029 | Homo sapiens | N-Acetylglucosamine kinase | 1249 | 99 |
| 30 | X08055 | Homo sapiens | preglycophorin B | 314 | 100 |
| 31 | AF320912 | Homo sapiens | MAGE-H1 | 1140 | 100 |
| 32 | AF197952 | Homo sapiens | thioredoxin peroxidase PMP20 | 345 | 94 |
| 33 | W88457 | Homo sapiens | Human lysophospholipase IHLP. | 1243 | 100 |
| 34 | AF151036 | Homo sapiens | HSPC202 | 808 | 100 |
| 35 | AL008583 | Homo sapiens | dJ327J16.1 (dynein, axonemal, light polypeptide 4) | 565 | 100 |
| 36 | L76200 | Homo sapiens | guanylate kinase | 198 | 100 |
| 37 | DC001773 | Homo sapiens | Similar to ribosomal protein L34 | 591 | 100 |
| 38 | AF216962 | Homo sapiens | ancient conserved domain protein 2 | 1743 | 93 |
| 39 | U05255 | Homo sapiens | glycophorin HeP2 | 245 | 97 |
| 40 | X51699 | Homo sapiens | bone Gla precursor (100 AA) | 526 | 100 |
| 41 | X83218 | Homo sapiens | ATP synthase, oligomycin sensitivity conferring protein | 1032 | 99 |
| 42 | X63527 | Homo sapiens | ribosomal protein L19 | 990 | 100 |
| 43 | B37410 | Homo sapiens | Human secreted protein BLAST search protein SEQ ID NO: 120. | 116 | 55 |
| 44 | AF149414 | Arabidopsi s thaliana | contains similarity to Pfam family PF00145 (C-5 cytosine-specific DNA methylase); score=10.4. E=0.051, N=1 | 299 | 37 |
| 45 | AY007220 | Homo sapiens | S100-type calcium binding protein A14 | 541 | 100 |
| 46 | X92896 | Homo sapiens | ITBA2 | 568 | 99 |
| 47 | U47924 | Homo sapiens | C8 | 1400 | 100 |
| 48 | AF243495 | Homo sapiens | hepatocellular carcinoma-associated antigen 67 | 2530 | 99 |
| 49 | AF311213 | Mus musculus | MESDC2 | 1004 | 83 |
| 50 | X12791 | Homo sapiens | 19kD SRP-protein (AA 1 -144) | 742 | 100 |
| 51 | AJ006973 | Homo sapiens | TOM1 | 2515 | 100 |
| 52 | Y57608 | Homo sapiens | Human apoptosis associated protein HAPOP-3. | 338 | 98 |
| 53 | X84194 | Homo sapiens | acylphosphatase | 200 | 100 |
| 54 | G00352 | Homo sapiens | Human secreted protein, SEQ ID NO: 4433. | 228 | 68 |
| 55 | AF221520 | Homo sapiens | basic helix-loop-helix protein class B I | 1772 | 99 |
| 56 | U14973 | Homo sapiens | ribosomal protein S29 | 313 | 98 |
| 57 | AF000944 | Rattus norvegicus | TFIIA small subunit | 269 | 77 |
| 58 | W69784 | Homo sapiens | Protein Kinase C Inhibitor-like Protein (IPKC-2). | 628 | 100 |
| 59 | M27784 | synthetic construct | stefin A | 508 | 100 |
| 60 | Y15909 | Homo sapiens | DIA-156 protein | 5638 | 99 |
| 61 | M58458 | Homo sapiens | ribosomal protein S4X isoform | 660 | 99 |
| 62 | X95384 | Homo sapiens | 14.5 kDa translational inhibitor protein, p14.5 | 675 | 100 |
| 63 | M92449 | Homo sapiens | putative | 1285 | 98 |
| 64 | U93868 | Homo sapiens | RNA polymerase III subunit | 470 | 47 |
| 65 | X76717 | Homo sapiens | MT-11 protein | 382 | 100 |
| 66 | D00678 | Homo sapiens | Human Hydrolase protein-3 (HYDRL-3) encoding cDNA. | 1775 | 100 |
| 67 | AJ278219 | Homo sapiens | fatty acid hydroxylase | 1247 | 100 |
| 68 | G03032 | Homo sapiens | Human secreted protein, SEQ ID NO: 7113. | 246 | 56 |
| 69 | AF174593 | Homo sapiens | F-box protein Fb17 | 2355 | 100 |
| 70 | AB038021 | Homo sapiens | CLST 11240 protein | 516 | 100 |
| 71 | X77953 | Rattus norvegicus | ribosomal protein S15a | 671 | 100 |
| 72 | U24080 | Homo sapiens | immunoglobulin heavy chain VH3 | 624 | 93 |
| 73 | AF023268 | Homo sapiens | cotel | 470 | 41 |
| 74 | Y95008 | Homo sapiens | Human secreted protein vf3_1, SEQ ID NO:56. | 472 | 45 |
| 75 | AF308302 | Homo sapiens | serologically defined breast cancer antigen NY-BR-96 | 2003 | 97 |
| 76 | AL109653 | Homo sapiens | bG115M3.1 (novel protein) | 1874 | 55 |
| 77 | R15222 | Homo sapiens | Chronic myelogenous leukaemia-derived myeloid-related protein. | 507 | 98 |
| 78 | X85373 | Homo sapiens | Sm protein G | 387 | 100 |
| 79 | AF230533 | Homo sapiens | nuclear receptor coactivator CIA | 3006 | 100 |
| 80 | G03789 | Homo sapiens | Human secreted protein, SEQ ID NO: 7870. | 156 | 73 |
| 81 | AL035461 | Homo sapiens | dJ967N21.5 (novel MCM2/3/5 family member) | 3096 | 100 |
| 82 | AK000022 | Homo sapiens | unnamed protein product | 493 | 100 |
| 83 | AF074016 | Homo sapiens | nonsense-mediated mRNA decay trans-acting factor | 334 | 37 |
| 84 | AF151076 | Homo sapiens | HSPC242 | 591 | 94 |
| 85 | AJ277841 | Homo sapiens | ELG protein | 1744 | 100 |
| 86 | AK000012 | Homo sapiens | unnamed protein product | 816 | 99 |
| 87 | AF309871 | Pichia pastoris | Gsa11p | 182 | 30 |
| 88 | Y22498 | Homo sapiens | Human secreted protein sequence clone hb1041_2. | 2355 | 99 |
| 89 | AF117649 | Drosophila melanogas ter | Adrift | 766 | 39 |
| 90 | AF249745 | Homo sapiens | RhoGEF | 3389 | 99 |
| 91 | AF325191 | Homo sapiens | KRAB zinc finger protein HZF26 | 332 | 85 |
| 92 | AB035966 | Homo sapiens | testis-specific adriamycin sensitivity protein | 2312 | 100 |
| 93 | AL136125 | Homo sapiens | dJ304B14.2.1 (novel protein isoform 1) | 2257 | 100 |
| 94 | W23317 | Homo sapiens | Human prostate protein HPA32. | 888 | 100 |
| 95 | AF116640 | Homo sapiens | PRO1584 | 420 | 100 |
| 96 | AF182319 | Xenopus laevis | 4g2 | 550 | 82 |
| 97 | Y88618 | Homo sapiens | Human PTAN-1 amino acid sequence. | 2312 | 99 |
| 98 | AF056198 | Drosophila melanogas ter | Hsp70/Hsp90 organizing protein homolog | 222 | 32 |
| 99 | AF161405 | Homo sapiens | HSPC287 | 724 | 100 |
| 100 | B21006 | Homo sapiens | Human nucleic acid-binding protein, NuABP-10. | 2397 | 100 |
| 101 | AB004538 | Schizosacc haromyces pombe | HYPOTHETICAL 59.2KD PROTEIN IN PFK26-SGA1 INTERGENIC REGION | 581 | 34 |
| 102 | W74763 | Homo sapiens | Human secreted protein encoded by gene 33 clone HTOJN06. | 132 | 100 |
| 103 | AF226869 | Homo sapiens | RB-associated KRAB repressor | 292 | 54 |
| 104 | AF118088 | Homo sapiens | PRO2000 | 1865 | 100 |
| 105 | Y84440 | Homo sapiens | Amino acid sequence of a human RNA-associated protein. | 2659 | 100 |
| 106 | U32855 | Drosophila melanogas ter | cytoplasmic dynein light chain 1 | 465 | 96 |
| 107 | AF116630 | Homo sapiens | PRO1278 | 508 | 98 |
| 108 | AB046628 | Macaca fascicularis | hypothetical protein | 233 | 93 |
| 109 | AL023494 | Homo sapiens | dJ366L4.2 (novel protein) | 669 | 100 |
| 110 | G02872 | Homo sapiens | Human secreted protein, SEQ ID NO: 6953. | 288 | 58 |
| 112 | AF151363 | Mus musculus | Cdc42 GTPase-activating protein | 3204 | 78 |
| 113 | AF130079 | Homo sapiens | PRO2852 | 272 | 65 |
| 114 | AK001575 | Homo sapiens | unnamed protein product | 1550 | 100 |
| 116 | B34974 | Homo sapiens | Human secreted protein sequence encoded by gene 46 SEQ ID NO: 178. | 434 | 100 |
| 117 | AJ272268 | Homo sapiens | calcium channel alpha2-delta3 subunit | 5241 | 100 |
| 118 | Z82083 | Caenorhab ditis elegans | ZK1010.2 | 330 | 30 |
| 119 | AF155827 | Homo sapiens | proliferation-associated SNF2-like protein | 4300 | 99 |
| 120 | X17617 | Mus musculus | zinc finger protein (AA 1-580) | 1007 | 49 |
| 121 | AF043725 | Homo sapiens | PHD-finger protein | 298 | 41 |
| 122 | G03522 | Homo sapiens | Human secreted protein, SEQ ID NO: 7603. | 735 | 100 |
| 123 | X85373 | Homo sapiens | Sm protein G | 339 | 89 |
| 124 | M24496 | Mus musculus | neurofilament largest subunit | 233 | 26 |
| 125 | AF098066 | Homo sapiens | squamous cell carcinoma antigen recognized by T cell | 1747 | 51 |
| 126 | AY008372 | Homo sapiens | oxysterol binding protein-related protein 3 | 3491 | 100 |
| 127 | AF182037 | Rattus norvegicus | Robo2 | 5093 | 93 |
| 128 | AF150755 | Mus musculus | microtubule-actin crosslinking factor | 506 | 19 |
| 129 | U16802 | Rattus norvegicus | Ca2+-dependent activator protein; calcium-dependent actin-binding protein | 839 | 78 |
| 130 | Z50110 | Caenorhab ditis elegans | F18H3.1 | 282 | 40 |
| 131 | Y21847 | Homo sapiens | Human signal peptide-contianing protein (SIGP) (clone ID 1864292). | 1184 | 84 |
| 132 | AF151879 | Homo sapiens | CGI-121 protein | 629 | 100 |
| 133 | AF099935 | Homo sapiens | MDC-3.13 isoform 2 | 590 | 56 |
| 134 | AF119848 | Homo sapiens | PRO1580 | 1275 | 99 |
| 135 | G04018 | Homo sapiens | Human secreted protein, SEQ ID NO: 8099. | 397 | 98 |
| 136 | V00489 | Homo sapiens | alpha globin | 648 | 99 |
| 137 | V00488 | Homo sapiens | alpha globin | 706 | 93 |
| 138 | W59929 | 791 | Human AAA-associated immunoglobulin reactive protein | 219 | 93 |
| 139 | Y87218 | Homo sapiens | Human secreted protein sequence SEQ ID NO:257. | 314 | 98 |
| 140 | Y02666 | Homo sapiens | Human secreted protein encoded by gene 17 clone HLTA194. | 221 | 100 |
| 141 | M97188 | Strongyloc entrotus purpuratus | tektin A1 | 246 | 45 |
| 142 | M97188 | Strongyloc entrotus purpuratus | tektin A1 | 179 | 35 |
| 143 | Y36212 | 791 | Human secreted protein | 1033 | 99 |
| 144 | R15222 | Homo sapiens | Chronic myelogenous leukaemia-derived myeloid-related protein. | 614 | 99 |
| 145 | Y73493 | Homo sapiens | Human secreted protein clone yk224_1 protein sequence SEQ ID NO:208. | 2165 | 100 |
| 146 | U87306 | Rattus norvegicus | transmembrane receptor UNC5H2 | 4708 | 91 |

**Table 3**

| **SEQ ID NO:** | **Accession Number** | **Description** | **Results*** |
|---|---|---|---|
| 1 | PR00761 | BINDIN PRECURSOR SIGNATURE | PR00761E 14.32 4.500e-10 460-479 PR00761E 14.32 7.253e-09 459-478 |
| 5 | BL01033 | Globins profile. | BL01033A 16.94 3.250e-20 74-96 BL01033B 13.81 7.000e-14 136-148 |
| 8 | BL00215 | Mitochondrial energy transfer proteins. | BL00215A 15.82 8.500e-17 69-94 BL00215B 10.44 8.714e-12 206-219 BL00215A 15.82 7.319e-11 166-191 |
| 9 | BL00944 | Cyclin-dependent kinases regulatory subunits proteins. | BL00944 17.47 1.000e-40 32-74 |
| 10 | BL00796 | 14-3-3 proteins. | BL00796C 17.44 1.000e-40 97-147 BL00796D 17.39 1.000e-40 148-194 BL00796E 14.15 1.000e-40 196-232 BL00796B 10.67 4.484e-37 35-68 BL00796A 10.52 3.571e-25 3-30 |
| 11 | PF00013 | KH domain proteins family of RNA binding proteins. | PF00013 5.78 4.150e-09 112-124 |
| 15 | PR00860 | VERTEBRATE METALLOTHIONEIN SIGNATURE | PR00860B 7.04 2.929e-20 27-41 PR00860A 5.46 3.842e-15 5-18 PR00860C 9.61 5.500e-15 41-51 |
| 17 | PF00642 | Zinc finger C-x8-C-x5-C-x3-H type (and similar). | PF00642 11.59 8.200e-11 211-222 |
| 18 | BL00546 | Matrixins cysteine switch. | BL00546G 16.84 3.053e-09 110-130 |
| 19 | BL00310 | Lysosome-associated membrane glycoproteins duplicated domain proteins. | BL00310A 14.05 2.102e-11 56-71 |
| 25 | BL01271 | Sodium:sulfate symporter family proteins. | BL01271D 25.26 5.154e-38 480-535 BL01271B 12.02 6.400e-24 208-233 BL01271A 8.06 7.955e-23 132-152 BL01271C 13.62 7.429e-20 407-429 |
| 27 | DM00215 | PROLINE-RICH PROTEIN 3. | DM00215 19.43 3.898e-09 78-111 |
| 28 | PR00962 | LETHAL(2) GIANT LARVAE PROTEIN SIGNATURE | PR00962D 10.40 9.511e-10 241-265 |
| 29 | BL00237 | G-protein coupled receptors proteins. | BL00237D 11.23 1.000e-09 261-278 |
| 30 | BL00312 | Glycophorin A proteins. | BL00312B 9.22 7.517e-32 37-66 |
| 33 | PF00756 | Putative esterase. | PF00756C 14.12 7.692e-10 119-149 |
| 35 | BL01239 | Dynein light chain type 1 proteins. | BL01239 16.10 1.099e-13 32-86 |
| 36 | BL00856 | Guanylate kinase proteins. | BL00856A 17.25 4.176e-11 8-21 |
| 37 | BL01145 | Ribosomal protein L34e proteins. | BL01145A 13.73 1.000e-40 3-45 BL01145B 14.65 2.636e-20 88-111 |
| 40 | PR00002 | BONE MATRIX GLA DOMAIN SIGNATURE | PR00002A 11.56 7.000e-20 68-85 PR00002B 8.36 4.316e-13 87-98 |
| 41 | BL00389 | ATP synthase delta (OSCP) subunit proteins. | BL00389C 20.13 6.760e-25 168-206 BL00389B 17.02 6.211e-13 107-129 BL00389A 11.02 7.188e-1037-50 |
| 42 | BL00526 | Ribosomal protein L19e proteins. | BL00526A 19.50 1.000e-40 4-47 BL00526B 26.53 1.000e-40 53-100 BL00526C 20.60 1.000e-40 100-143 |
| 48 | PR00683 | SPECTRIN PLECKSTRIN HOMOLOGY DOMAIN SIGNATURE | PR00683B 16.62 5.558e-09 250-272 |
| 52 | PF00628 | PHD-finger. | PF00628 15.84 5.125e-11 37-52 |
| 53 | PR00112 | ACYLPHOSPHATASE SIGNATURE | PR00112C 18.81 5.725e-23 4-25 |
| 55 | BL00038 | Myc-type, 'helix-loop-helix' dimerization domain proteins. | BL00038B 16.97 5.395e-09 367-388 |
| 58 | BL00892 | HIT family proteins. | BL00892A 18.17 2.125e-10 3-34 |
| 59 | BL00287 | Cysteine proteases inhibitors proteins. | BL00287 17.35 7.429e-19 39-63 |
| 60 | PR00219 | SYNAPTOBREVIN SIGNATURE | PR00219B 5.46 2.901e-09 528-548 |
| 61 | BL00528 | Ribosomal protein S4e proteins. | BL00528B 24.75 1.000e-40 47-101 BL00528A 16.12 5.000e-36 3-36 |
| 62 | BL01094 | Hypothetical YER057c/yjjV family proteins. | BL01094B 20.31 1.0006-40 49-99 BL01094A 16.79 7.188e-35 9-42 BL01094C 18.20 5.821e-28 99-129 |
| 64 | PF00992 | Troponin. | PF00992A 16.67 6.451e-10 159-194 |
| 65 | PR00860 | VERTEBRATE METALLOTHIONEIN SIGNATURE | PR00860B 7.04 2.929e-20 27-41 PR00860C 9.61 1.474e-14 41-51 PR00860A 5.46 5.034e-13 5-18 |
| 66 | PD01922 | PROTEIN PHOSPHODIESTERAS E HYDROL. | PD01922B 21.83 7.000e-22 78-114 |
| 71 | BL00053 | Ribosomal protein S8 proteins. | BL00053C 16.715.500e-26 98-131 BL00053B 14.56 4.789e-14 58-76 BL00053A 8.83 5.320e-12 5-18 |
| 72 | DM00031 | IMMUNOGLOBULIN V REGION. | DM00031A 16.80 1.000e-40 20-68 DM00031B 15.41 1.000e-40 84-118 |
| 73 | PR00806 | VINCULIN SIGNATURE | PR00806A 6.63 6.055e-09 142-153 |
| 75 | BL00107 | Protein kinases ATP-binding region proteins. | BL00107A 18.39 8.920e-13 148-179 |
| 76 | PR00209 | ALPHA/BETA GLIADIN FAMILY SIGNATURE | PR00209B 4.88 1.978e-10 793-812 PR00209B 4.88 3.739e-10 791-810 PR00209B 4.88 8.500e-09 792-811 |
| 77 | BL00269 | Mammalian defensins proteins. | BL00269C 16.52 6.786e-26 352-381 BL00269A 8.53 2.607e-20 287-307 BL00269B 19.17 2.800e-18 148-177 BL00269B 19.17 5.500e-17 314-343 BL00269A 8.53 2.73le-14 122-142 |
| 78 | PD01861 | PROTEIN NUCLEAR RIBONUCLEOPROTEI N SMALL MRNA RNA. | PD01861A 14.06 1.265e-19 24-48 PD01861B 8.80 2.241e-11 58-71 |
| 81 | BL00717 | Sigma-54 factors family proteins. | BL00717B 10.07 3.538e-09 729-740 |
| 85 | BL00412 | Neuromodulin(GAP-43) proteins. | BL00412D 16.54 8.773e-11 46-97 |
| 86 | BL00415 | Synapsins proteins. | BL00415N 4.29 4.153e-09 37-81 |
| 90 | BL00741 | Guanine-nucleotide dissociation stimulators CDC24 family sign. | BL00741B 14.27 8.138e-13 416-439 |
| 91 | PD01066 | PROTEIN ZINC FINGER ZINC-FINGER METAL-BINDING NU. | PD01066 19.43 9.438e-37 6-45 |
| 100 | PD01066 | PROTEIN ZINC FINGER ZINC-FINGER METAL-BINDING NU. | PD01066 19.43 2.915e-29 53-92 |
| 103 | PD01066 | PROTEIN ZINC FINGER ZINC-FINGER METAL-BINDING NU. | PD01066 19.43 9.750e-35 10-49 |
| 104 | PR00503 | BROMODOMAIN SIGNATURE | PR00503B 9.96 4.800e-12 336-353 |
| 106 | BL01239 | Dynein light chain type 1 proteins. | BL01239 16.10 1.000e-40 17-71 |
| 112 | PD00930 | PROTEIN GTPASE DOMAIN ACTIVATION. | PD00930B 33.72 9.769e-2 138-179 |
| 116 | BL00157 | Ribulose bisphosphate carboxylase large chain proteins. | BL00157F 7.84 9.757e-10 27-81 |
| 117 | PD01101 | INHIBITOR HEAVY CHAIN CHANNEL IN. | PD01101B 21.53 1.692e-23 144-197 |
| 119 | BL00039 | DEAD-box subfamily ATP-dependent helicases proteins. | BL00039D 21.67 4.732e-11 670-716 |
| 120 | PD00066 | PROTEIN ZINC-FINGER METAL-BINDI. | PD00066 13.92 4.462e-15 257-270 PD00066 13.92 1.600e-14 229-242 PD00066 13.92 3.400e-14 145-158 PD00066 13.92 4.600e-14 201-214 PD00066 13.92 8.800e-14 173-186 PD00066 13.92 2200e-09 89-102 PD00066 13.92 3.700e-09 369-382 |
| 122 | PR00652 | 5-HYDROXYTRYPTAM INE 7 RECEPTOR SIGNATURE | PR00652B 5.29 9.505e-09 119-140 |
| 123 | PD01861 | PROTEIN NUCLEAR RIBONUCLEOPROTEI N SMALL MRNA RNA. | PD01861A 14.06 1.000e-15 24-48 |
| 124 | PF00992 | Troponin. | PF00992A 16.67 1.380e-10 596-631 PF00992A 16.67 4.316e-09 602-637 |
| 125 | DM00396 | 5 kw INTRON COI ND4L ND5. | DM00396A 5.97 7.429e-09 301-309 |
| 126 | BL01013 | Oxysterol-binding protein family proteins. | BL01013A 25.14 5.500e-21 329-365 BL01013D 26.81 2.161e-18 599-643 BL01013C 9.97 4.231e-13 417-427 BL01013B 11.33 3.017e-11 395-406 |
| 127 | DM00179 | w KINASE ALPHA ADHESION T-CELL. | DM00179 13.97 9.053e-10 481-491 |
| 136 | BL01033 | Globins profile. | BL01033B 13.81 7.000e-14 123-135 |
| 137 | BL01033 | Globins profile. | BL01033A 16.94 3.250e-20 74-96 BL01033B 13.81 7.000e-14 146-158 |
| 141 | PR00511 | TEKTIN SIGNATURE | PR00511A 13.59 3.700e-14 113-130 |
| 144 | BL00269 | Mammalian defensins proteins. | BL00269C 16.52 6.786e-26 96-125 BL00269A 8.53 2.607e-20 31-51 BL00269B 19.17 5.500e-17 58-87 |
| 146 | PD01719 | PRECURSOR GLYCOPROTEIN SIGNAL RE. | PD01719A 12.89 9.143e-14 249-277 |

| | | | |
|---|---|---|---|
| *Results include in order: accession number subtype; raw score; p-value; position of signature in amino acid sequence | | | |

**Table 4**

| **SEQ ID NO:** | **pFam Name** | **Description** | **p-value** | **pFam Score** |
|---|---|---|---|---|
| 1 | ig | Immunoglobulin domain | 2.8e-22 | 76.9 |
| 2 | Gag_p30 | Gag P30 core shell protein | 9.2e-19 | 69.2 |
| 5 | globin | Globin | 4.6e-57 | 203.0 |
| 8 | mito_carr | Mitochondrial carrier proteins | 1.5e-112 | 387.3 |
| 9 | CKS | Cyclin-dependent kinase regulatory subunit | 8.5e-50 | 178.9 |
| 10 | 14-3-3 | 14-3-3 proteins | 1.1e-147 | 504.0 |
| 11 | KH-domain | KH domain | 1.6e-49 | 177.9 |
| 13 | HTH_3 | Helix-turn-helix | 6.5e-13 | 56.3 |
| 15 | metalthio | Metallothionein | 1.2e-24 | 95.4 |
| 17 | zf-CCCH | Zinc finger C-x8-C-x5-C-x3-H type | 9.1 e-10 | 39.9 |
| 18 | hemopexin | Hemopexin | 2.4e-58 | 207.3 |
| 25 | Na_sulph_sy mp | Sodium:sulfate symporter transmembrane | 7.3e-136 | 464.8 |
| 28 | WD40 | WD domain, G-beta repeat | 7.5e-54 | 192.3 |
| 29 | 7tm_1 | 7 transmembrane receptor (rhodopsin family) | 4.5e-10 | 34.2 |
| 30 | Glycophorin _A | Glycophorin A | 2.3e-20 | 71.7 |
| 31 | MAGE | MAGE family | 0.05 | -101.0 |
| 33 | abhydrolase_ 2 | Phospholipase/Carboxylesterase | 8.6e-30 | 112.4 |
| 35 | Dynein_light | Dynein light chain type 1 | 1.3e-12 | 55.3 |
| 37 | Ribosomal_ L34e | Ribosomal protein L34e | 1e-65 | 231.7 |
| 38 | cNMP_bindi ng | Cyclic nucleotide-binding domain | 0.05 | 10.1 |
| 40 | gla | Vitamin K-dependent carboxylation/gamma-carb | 2.2e-15 | 64.6 |
| 41 | OSCP | ATP synthase delta (OSCP) subunit | 2.7e-75 | 257.7 |
| 42 | Ribosomal_ L19e | Ribosomal protein L19e | 3.6e-104 | 359.5 |
| 45 | S_100 | S-100/ICaBP type calcium binding domain | 2.9e-08 | 40.9 |
| 48 | Sec7 | Sec7 domain | 3.7e-65 | 229.9 |
| 50 | SRP19 | SRP19 protein | 1.2e-25 | 98.7 |
| 51 | VHS | VHS domain | 9.6e-71 | 248.4 |
| 52 | PHD | PHD-finger | 0.00025 | 23.2 |
| 53 | Acylphospha tase | Acylphosphatase | 0.014 | -6.9 |
| 55 | HLH | Helix-loop-helix DNA-binding domain | 5e-05 | 30.1 |
| 56 | Ribosomal_ S14 | Ribosomal protein S14p/S29e | 7.5e-20 | 68.3 |
| 57 | TFIIA_gam ma | Transcription initiation factor IIA, gamm | 2.6e-31 | 117.5 |
| 58 | HIT | HIT family | 0.0001 | 4.1 |
| 59 | cystatin | Cystatin domain | 5.7e-28 | 100.2 |
| 60 | FH2 | Formin Homology 2 Domain | 1.3e-187 | 636.7 |
| 61 | Ribosomal_ S4e | Ribosomal family S4e | 4.6e-08 | 40.2 |
| 62 | UPF0076 | Domain of unknown function UPF0076 | 9.9e-68 | 238.4 |
| 65 | metalthio | Metallothionein | 2.2e-23 | 91.1 |
| 68 | PX | PX domain | 1.7e-09 | 45.0 |
| 69 | F-box | F-box domain. | 3.2e-09 | 44.1 |
| 71 | Ribosomal_ S8 | Ribosomal protein S8 | 6e-58 | 192.1 |
| 72 | ig | Immunoglobulin domain | 3.1e-12 | 44.5 |
| 75 | pkinase | Eukaryotic protein kinase domain | 1.1e-30 | 114.5 |
| 76 | LRR | Leucine Rich Repeat | 1.1e-30 | 115.4 |
| 77 | Defensin_pr opep | Defensin propeptide | 2.5e-46 | 167.3 |
| 78 | Sm | Sm protein | 1.1e-24 | 95.5 |
| 81 | MCM | MCM2/3/5 family | 1.1e-118 | 407.7 |
| 89 | FtsJ | FtsJ cell division protein | 0.00037 | -37.0 |
| 90 | RhoGEF | RhoGEF domain | 7e-39 | 142.6 |
| 91 | KRAB | KRAB box | 1e-40 | 148.7 |
| 97 | HMG_box | HMG (high mobility group) box | 0.078 | -8.3 |
| 98 | TPR | TPR Domain | 4.1e-15 | 63.6 |
| 100 | zf-C2H2 | Zinc finger, C2H2 type | 1.3e-65 | 231.4 |
| 103 | KRAB | KRAB box | 7.4e-37 | 135.9 |
| 104 | bromodomai n | Bromodomain | 4.2e-18 | 67.2 |
| 105 | rrm | RNA recognition motif. | 1.2e-09 | 45.5 |
| 106 | Dynein_light | Dynein light chain type 1 | 3.5e-66 | 233.3 |
| 112 | RhoGAP | RhoGAP domain | 6.7e-49 | 175.9 |
| 119 | SNF2_N | SNF2 and others N-terminal domain | 2.2e-104 | 360.2 |
| 120 | zf-C2H2 | Zinc finger, C2H2 type | 8.7e-57 | 202.1 |
| 123 | Sm | Sm protein | 3.9e-20 | 80.3 |
| 126 | Oxysterol_B P | Oxysterol-binding protein | 3.4e-78 | 273.2 |
| 127 | ig | Immunoglobulin domain | 2.8e-53 | 176.7 |
| 136 | globin | Globin | 8.8e-42 | 150.1 |
| 137 | globin | Globin | 3.4e-53 | 189.9 |
| 144 | Defensin_pr opep | Defensin propeptide | 3e-25 | 97.3 |
| 146 | ZU5 | ZU5 domain | 9.3e-57 | 202.0 |

**Table 5**

| **SEQ ID NO: of full-length nucleotide sequence** | **SEQ ID NO: of full-length peptide sequence** | **SEQ ID NO: of contig nucleotide sequence** | **SEQ ID NO: of contig peptide sequence** | **Priority docket number_corresponding SEQ ID NO: in priority application** | **SEQ ID NO: in USSN 09/552,929** | **SEQ ID NO: in USSN 09/770,160** |
|---|---|---|---|---|---|---|
| 1 | 147 | 293 | 439 | 791CIP2A_1 | 151 | 151 |
| 2 | 148 | 294 | 440 | 791CIP2A_2 | 608 | 608 |
| 3 | 149 | 295 | 441 | 791CIP2A_3 | 649 | 649 |
| 4 | 150 | 296 | 442 | 791CIP2A_4 | 940 | 940 |
| 5 | 151 | 297 | 443 | 791CIP2A_5 | 954 | 954 |
| 6 | 152 | 298 | 444 | 791CIP2A_7 | 1009 1010 | 1009 1010 |
| 7 | 153 | 299 | 445 | 791CIP2A_8 | 1030 | 1030 |
| 8 | 154 | 300 | 446 | 791CIP2A_9 | 1033 | 1033 |
| 9 | 155 | 301 | 447 | 791CIP2A_10 | 1036 1037 | 1036 1037 |
| 10 | 156 | 302 | 448 | 791CIP2A_11 | 1051 | 1051 |
| 11 | 157 | 303 | 449 | 791CIP2A_12 | 1070 1071 1072 1073 1074 | 1070 1071 1072 1073 1074 |
| 12 | 158 | 304 | 450 | 791CIP2A_13 | 1091 | 1091 |
| 13 | 159 | 305 | 451 | 791CIP2A_14 | 1092 1093 | 1092 1093 |
| 14 | 160 | 306 | 452 | 791CIP2A_15 | 1108 1109 1110 | 1108 1109 1110 |
| 15 | 161 | 307 | 453 | 791CIP2A_16 | 1136 1137 1138 1139 | 1136 1137 1138 1139 1332 |
| 16 | 162 | 308 | 454 | 791CIP2A_17 | 1181 | 1181 |
| 17 | 163 | 309 | 455 | 791CIP2A_18 | 1197 1198 | 1197 1198 |
| 18 | 164 | 310 | 456 | 791CIP2A_19 | 1220 1221 | 1220 1221 |
| 19 | 165 | 311 | 457 | 791CIP2A_20 | 1247 | 1247 |
| 20 | 166 | 312 | 458 | 791CIP2A_21 | 1268 1269 | 1268 1269 |
| 21 | 167 | 313 | 459 | 791CIP2A_22 | 1328 1329 | 1328 1329 |
| 22 | 168 | 314 | 460 | 791CIP2A_23 | 1330 1331 | 1330 1331 1332 |
| 23 | 169 | 315 | 461 | 791CIP2A_24 | 1360 1361 1362 | 1360 1361 1362 |
| 24 | 170 | 316 | 462 | 791CIP2A_25 | 1390 | 1390 |
| 25 | 171 | 317 | 463 | 791CIP2A_26 | 1393 1394 1395 1396 1397 1398 1399 1400 | 1393 1394 1395 1396 1397 1398 1399 1400 |
| 26 | 172 | 318 | 464 | 791CIP2A_27 | 1412 1413 1414 | 1412 1413 1414 |
| 27 | 173 | 319 | 465 | 791CIP2A_28 | 1416 | 1416 |
| 28 | 174 | 320 | 466 | 791CIP2A_29 | 1435 1436 | 1435 1436 |
| 29 | 175 | 321 | 467 | 791CIP2A_30 | 1437 1438 1439 1440 1441 1442 1443 1444 1445 | 1437 1438 1439 1440 1441 1442 1443 1444 1445 |
| 30 | 176 | 322 | 468 | 791CIP2A_31 | 1474 1475 1476 | 1474 1475 1476 |
| 31 | 177 | 323 | 469 | 791CIP2A_32 | 1508 | 1508 |
| 32 | 178 | 324 | 470 | 791CIP2A_33 | 1517 1518 1519 | 1517 1518 1519 |
| 33 | 179 | 325 | 471 | 791CIP2A_34 | 1528 1529 | 1528 1529 |
| 34 | 180 | 326 | 472 | 791CIP2A_35 | 1543 1544 | 1543 1544 |
| 35 | 181 | 327 | 473 | 791CIP2A_36 | 1596 | 1596 |
| 36 | 182 | 328 | 474 | 791CIP2A_37 | 1609 1610 1611 | 1609 1610 1611 |
| 37 | 183 | 329 | 475 | 791CIP2A_38 | 1619 1620 | 1619 1620 |
| 38 | 184 | 330 | 476 | 791CIP2A_39 | 1644 | 1644 |
| 39 | 185 | 331 | 477 | 791CIP2A_40 | 1698 1699 1700 | 1698 1699 1700 |
| 40 | 186 | 332 | 478 | 791CIP2A_41 | 1714 | 1714 |
| 41 | 187 | 333 | 479 | 791CIP2A_42 | 1743 1744 | 1743 1744 |
| 42 | 188 | 334 | 480 | 791CIP2A_43 | 1834 1835 | 1834 1835 |
| 43 | 189 | 335 | 481 | 791CIP2A_44 | 1847 | 1847 |
| 44 | 190 | 336 | 482 | 791CIP2A_45 | 1887 1888 1889 1890 1891 1892 | 1887 1888 1889 1890 1891 1892 |
| 45 | 191 | 337 | 483 | 791CIP2A_46 | 1981 | 1981 |
| 46 | 192 | 338 | 484 | 791CIP2A_47 | 2033 2034 | 2033 2034 |
| 47 | 193 | 339 | 485 | 791CIP2A_48 | 2063 2064 | 2063 2064 |
| 48 | 194 | 340 | 486 | 791CIP2A_49 | 2119 2120 2121 2122 | 2119 2120 2121 2122 |
| 49 | 195 | 341 | 487 | 791CIP2A_50 | 2138 2139 2140 | 2138 2139 2140 |
| 50 | 196 | 342 | 488 | 791CIP2A_51 | 2141 2142 2143 | 2141 2142 2143 |
| 51 | 197 | 343 | 489 | 791CIP2A_52 | 2189 2190 2191 2192 | 2189 2190 2191 2192 |
| 52 | 198 | 344 | 490 | 791CIP2A_53 | 2194 2195 2196 | 2194 2195 2196 |
| 53 | 199 | 345 | 491 | 791CIP2A_54 | 2200 | 2200 |
| 54 | 200 | 346 | 492 | 791CIP2A_55 | 2247 2248 2249 2250 2251 2252 2253 | 2247 2248 2249 2250 2251 2252 2253 |
| 55 | 201 | 347 | 493 | 791CIP2A_56 | 2255 | 2255 |
| 56 | 202 | 348 | 494 | 791CIP2A_57 | 2288 2289 2290 | 2288 2289 2290 |
| 57 | 203 | 349 | 495 | 791CIP2A_58 | 2311 2312 | 2311 2312 |
| 58 | 204 | 350 | 496 | 791CIP2A_59 | 2324 2325 | 2324 2325 |
| 59 | 205 | 351 | 497 | 791CIP2A_60 | 2334 | 2334 |
| 60 | 206 | 352 | 498 | 791CIP2A_61 | 2340 2341 | 2340 2341 |
| 61 | 207 | 353 | 499 | 791CIP2A_62 | 2353 2354 2355 | 2353 2354 2355 |
| 62 | 208 | 354 | 500 | 791CIP2A_63 | 2373 2374 | 2373 2374 |
| 63 | 209 | 355 | 501 | 791CIP2A_64 | 2401 2402 | 2401 2402 |
| 64 | 210 | 356 | 502 | 791CIP2A_65 | 2403 2404 2405 2406 | 2403 2404 2405 2406 |
| 65 | 211 | 357 | 503 | 791CIP2A_66 | 2424 2425 | 2424 2425 |
| 66 | 212 | 358 | 504 | 791CIP2A_67 | 2756 2757 | 2756 2757 |
| 67 | 213 | 359 | 505 | 791CIP2A_68 | 2811 | 2811 |
| 68 | 214 | 360 | 506 | 791CIP2A_69 | 2844 | 2844 |
| 69 | 215 | 361 | 507 | 791CIP2A_70 | 2854 2855 2856 2857 2858 2859 2860 | 2854 2855 2856 2857 2858 2859 2860 |
| 70 | 216 | 362 | 508 | 791CIP2A_71 | 2861 2862 2863 | 2861 2862 2863 |
| 71 | 217 | 363 | 509 | 791CIP2A_72 | 2882 2883 2884 | 2882 2883 2884 |
| 72 | 218 | 364 | 510 | 791CIP2A_73 | 2899 2900 2901 2902 2903 | 2899 2900 2901 2902 2903 |
| 73 | 219 | 365 | 511 | 791CIP2A_74 | 2938 | 2938 |
| 74 | 220 | 366 | 512 | 791CIP2A_75 | 2974 2975 2976 2977 | 2974 2975 2976 2977 |
| 75 | 221 | 367 | 513 | 791CIP2A_76 | 2980 2981 2982 2983 2984 2985 2986 | 2980 2981 2982 2983 2984 2985 2986 |
| 76 | 222 | 368 | 514 | 791CIP2A_77 | 3000 3001 | 3000 3001 |
| 77 | 223 | 369 | 515 | 791CIP2A_78 | 3045 | 3045 |
| 78 | 224 | 370 | 516 | 791CIP2A_79 | 3083 | 3083 |
| 79 | 225 | 371 | 517 | 791CIP2A_80 | 3111 | 3111 |
| 80 | 226 | 372 | 518 | 791CIP2A_81 | 3138 | 3138 |
| 81 | 227 | 373 | 519 | 791CIP2A_82 | 3160 3161 | 3160 3161 |
| 82 | 228 | 374 | 520 | 791CIP2A_83 | 3382 | 3382 |
| 83 | 229 | 375 | 521 | 791CIP2A_84 | 3503 | 3503 |
| 84 | 230 | 376 | 522 | 791CIP2A_85 | 3934 3935 3936 3937 | 3934 3935 3936 3937 |
| 85 | 231 | 377 | 523 | 791CIP2A_86 | 4214 4215 4216 | 4214 4215 4216 |
| 86 | 232 | 378 | 524 | 791CIP2A_87 | 4341 | 4341 |
| 87 | 233 | 379 | 525 | 791CIP2A_88 | 4385 | 4385 |
| 88 | 234 | 380 | 526 | 791CIP2A_89 | 4874 | 4874 |
| 89 | 235 | 381 | 527 | 791CIP2A_90 | 5591 | 5591 |
| 90 | 236 | 382 | 528 | 791CIP2A_91 | 5597 5598 5599 5600 5601 5602 | 5597 5598 5599 5600 5601 5602 |
| 91 | 237 | 383 | 529 | 791CIP2B_1 | 282 283 284 | 282 283 284 |
| 92 | 238 | 384 | 530 | 791CIP2B_2 | 305 | 305 |
| 93 | 239 | 385 | 531 | 791CIP2B_3 | 657 658 659 660 | 657 658 659 660 |
| 94 | 240 | 386 | 532 | 791CIP2B_4 | 803 | 803 |
| 95 | 241 | 387 | 533 | 791CIP2B_5 | 906 907 908 909 | 906 907 908 909 |
| 96 | 242 | 388 | 534 | 791CIP2B_6 | 2265 | 2265 |
| 97 | 243 | 389 | 535 | 791CIP2B_7 | 2474 | 2474 |
| 98 | 244 | 390 | 536 | 791CIP2B_8 | 2758 2759 2760 | 2758 2759 2760 |
| 99 | 245 | 391 | 537 | 791CIP2B_9 | 2892 | 2892 |
| 100 | 246 | 392 | 538 | 791CIP2B_11 | 3724 | 3724 |
| 101 | 247 | 393 | 539 | 791CIP2B_12 | 4059 | 4059 |
| 102 | 248 | 394 | 540 | 791CIP2B_13 | 4258 | 4258 |
| 103 | 249 | 395 | 541 | 791CIP2B_14 | 5253 | 5253 |
| 104 | 250 | 396 | 542 | 791CIP2B_15 | 5509 | 5509 |
| 105 | 251 | 397 | 543 | 791CIP2B_16 | 5526 | 5526 |
| 106 | 252 | 398 | 544 | 791CIP2C_1 | 70 | 70 |
| 107 | 253 | 399 | 545 | 791CIP2C_2 | 239 | 239 |
| 108 | 254 | 400 | 546 | 791CIP2C_3 | 376 | 376 |
| 109 | 255 | 401 | 547 | 791CIP2C_4 | 862 | 862 |
| 110 | 256 | 402 | 548 | 791CIP2C_5 | 1753 | 1753 |
| 111 | 257 | 403 | 549 | 791CIP2C_6 | 1820 | 1820 |
| 112 | 258 | 404 | 550 | 791CIP2C_7 | 2841 | 2841 |
| 113 | 259 | 405 | 551 | 791CIP2C_8 | 2904 | 2904 |
| 114 | 260 | 406 | 552 | 791CIP2C_9 | 3619 3620 3621 | 3619 3620 3621 |
| 115 | 261 | 407 | 553 | 791CIP2C_10 | 4069 | 4069 |
| 116 | 262 | 408 | 554 | 791CIP2C_11 | 4503 4504 | 4503 4504 |
| 117 | 263 | 409 | 555 | 791CIP2C_12 | 5083 | 5083 |
| 118 | 264 | 410 | 556 | 791CIP2C_13 | 5202 | 5202 |
| 119 | 265 | 411 | 557 | 791CIP2C_14 | 5436 | 5436 |
| 120 | 266 | 412 | 558 | 791CIP2D_1 | 1410 | 1410 |
| 121 | 267 | 413 | 559 | 791CIP2D_2 | 2209 2210 | 2209 2210 |
| 122 | 268 | 414 | 560 | 791CIP2D_3 | 2570 | 2570 |
| 123 | 269 | 415 | 561 | 791CIP2D_4 | 3083 791CIP2 A_79 | 3083 791CIP2A _79 |
| 124 | 270 | 416 | 562 | 791CIP2D_5 | 3638 3639 | 3638 3639 |
| 125 | 271 | 417 | 563 | 791CIP2D_6 | 4616 | 4616 |
| 126 | 272 | 418 | 564 | 791CIP2D_7 | 5046 | 5046 |
| 127 | 273 | 419 | 565 | 791CIP2D_8 | 5048 | 5048 |
| 128 | 274 | 420 | 566 | 791CIP2D_9 | 5092 | 5092 |
| 129 | 275 | 421 | 567 | 791CIP2E_1 | 237 | 237 |
| 130 | 276 | 422 | 568 | 791CIP2E_2 | 387 | 387 |
| 131 | 277 | 423 | 569 | 791CIP2E_3 | 458 | 458 |
| 132 | 278 | 424 | 570 | 791CIP2E_4 | 1482 | 1482 |
| 133 | 279 | 425 | 571 | 791CIP2E_5 | 2368 | 2368 |
| 134 | 280 | 426 | 572 | 791CIP2E_6 | 2429 2430 2431 | 2429 2430 2431 |
| 135 | 281 | 427 | 573 | 791CIP2E_7 | 2483 2484 2485 | 2483 2484 2485 |
| 136 | 282 | 428 | 574 | 791CIP2E_8 | 2675 2676 | 2675 2676 |
| 137 | 283 | 429 | 575 | 791CIP2E_9 | 2675 2676 | 2675 2676 |
| 138 | 284 | 430 | 576 | 791CIP2E_10 | 2741 | 2741 |
| 139 | 285 | 431 | 577 | 791CIP2E_11 | 3058 | 3058 |
| 140 | 286 | 432 | 578 | 791CIP2E_12 | 4078 4079 | 4078 4079 |
| 141 | 287 | 433 | 579 | 791CIP2E_13 | 4367 4368 4369 4370 4371 4372 | 4367 4368 4369 4370 4371 4372 |
| 142 | 288 | 434 | 580 | 791CIP2E_14 | 4367 4368 4369 4370 4371 4372 | 4367 4368 4369 4370 4371 4372 |
| 143 | 289 | 435 | 581 | 791CIP2E_15 | 5140 5141 | 5140 5141 |
| 144 | 290 | 436 | 582 | 791CIP2E_16 | 5273 | 5273 |
| 145 | 291 | 437 | 583 | 791CIP2E_17 | 5299 | 5299 |
| 146 | 292 | 438 | 584 | 791CIP2E_18 | 5805 | 5805 |

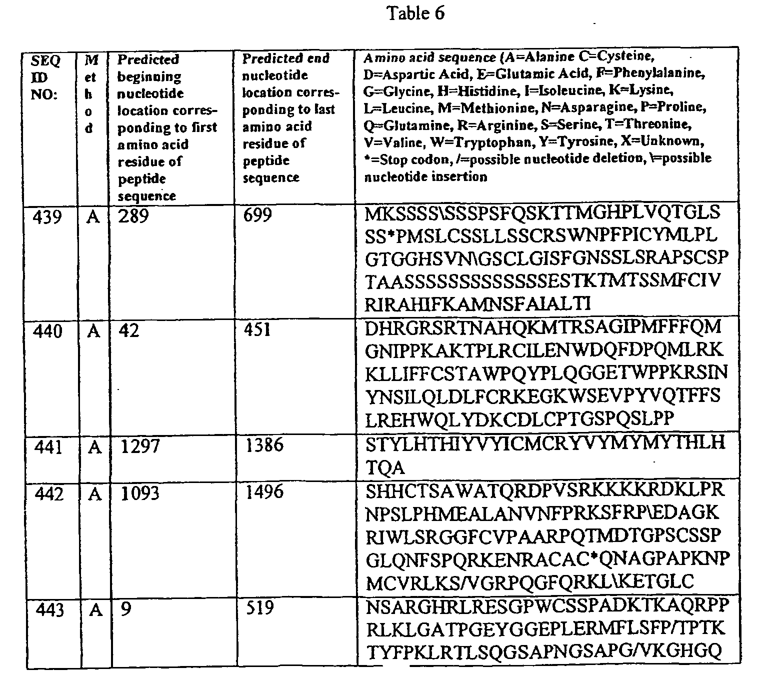

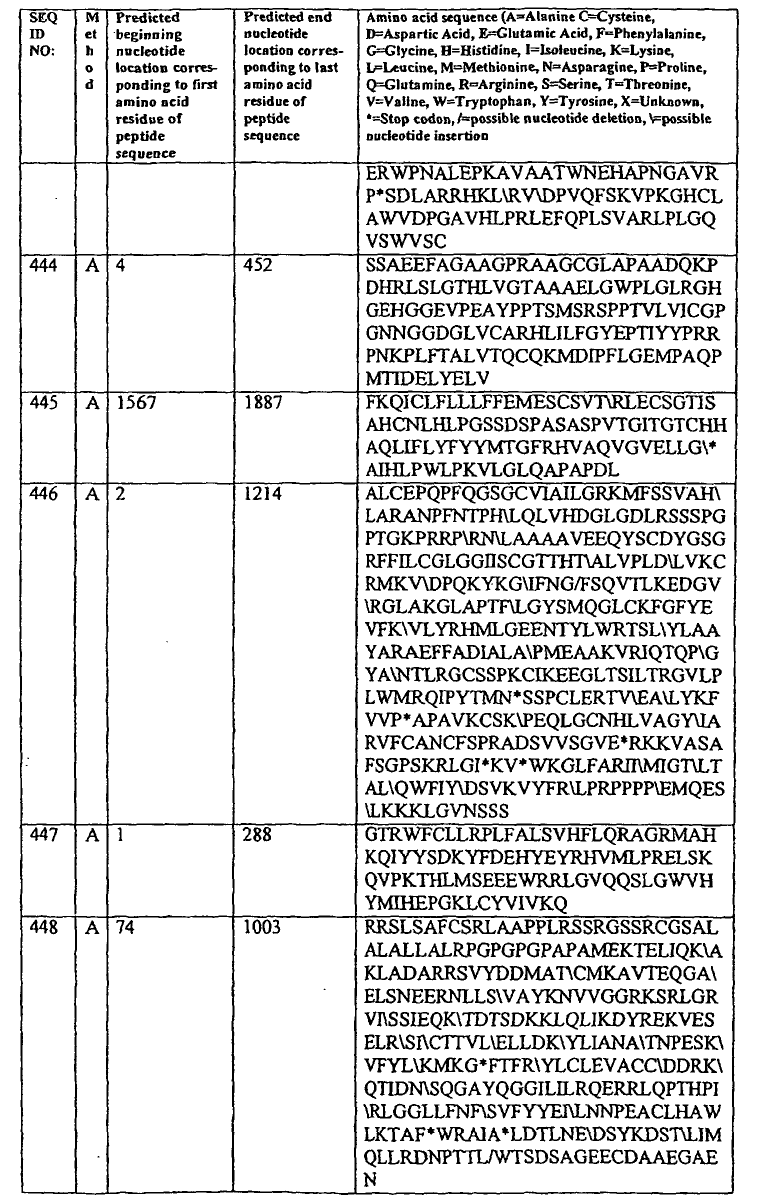

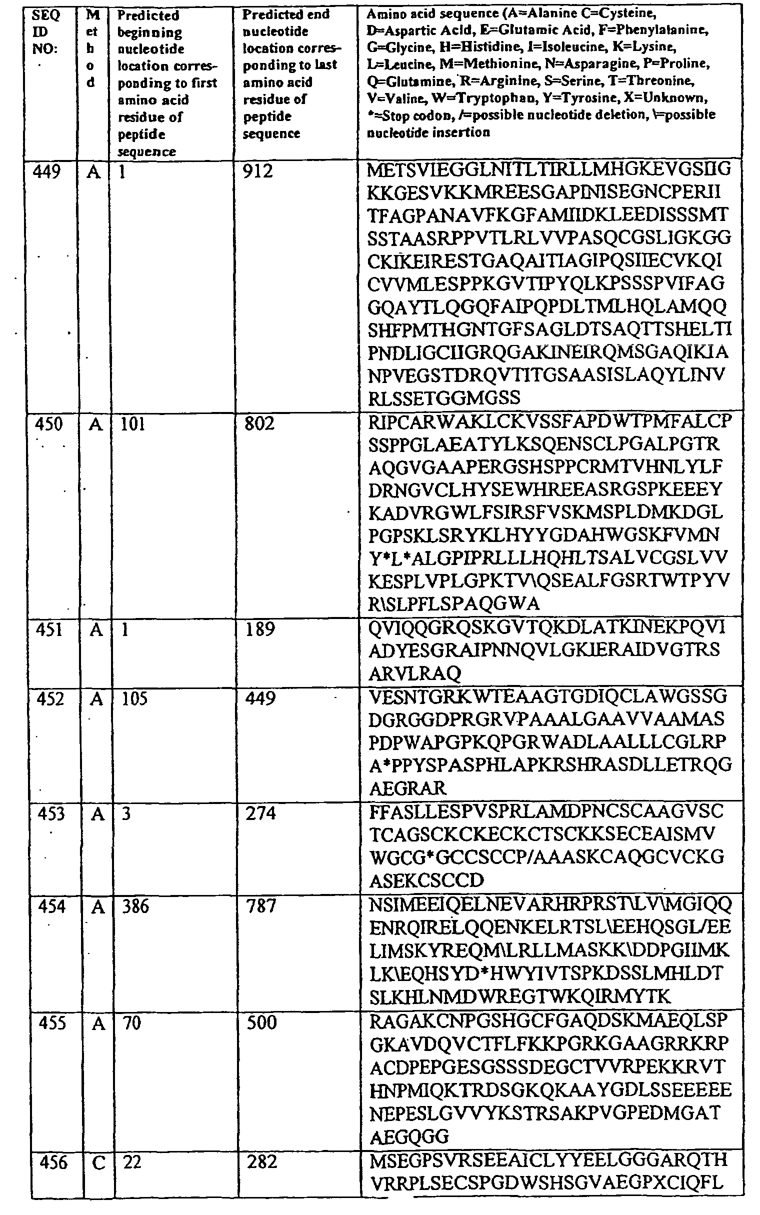

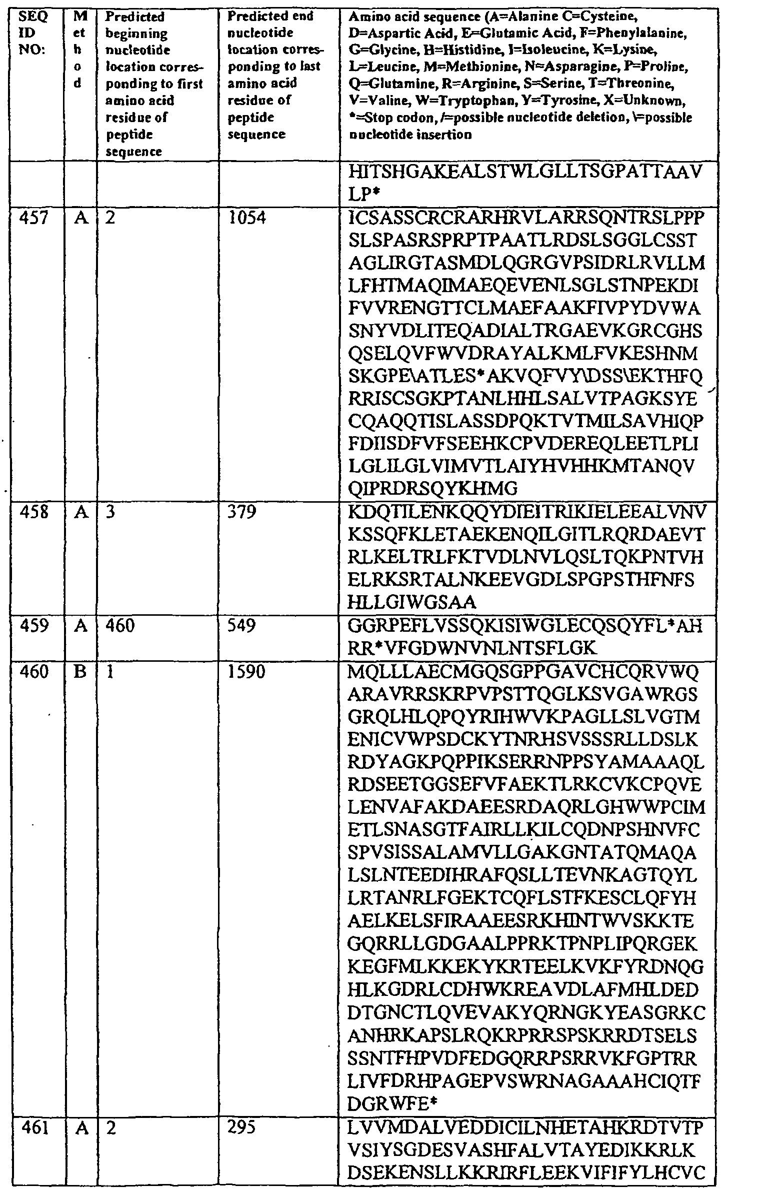

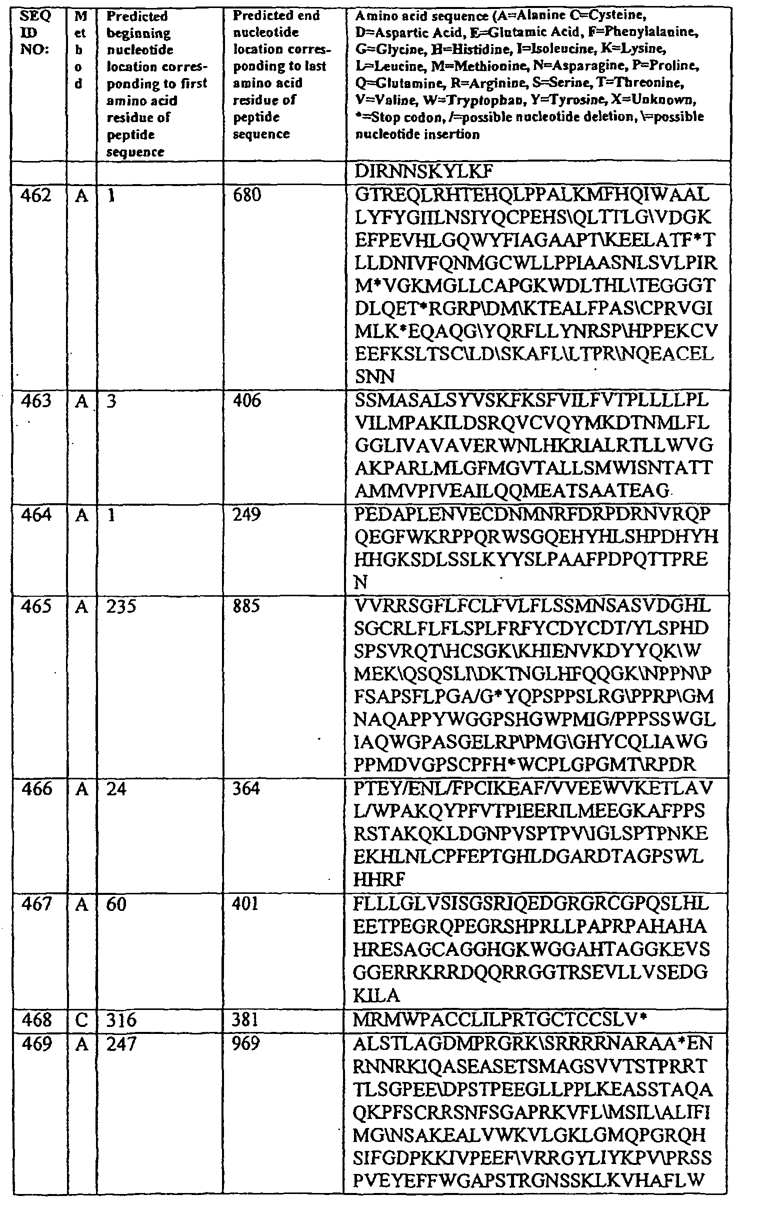

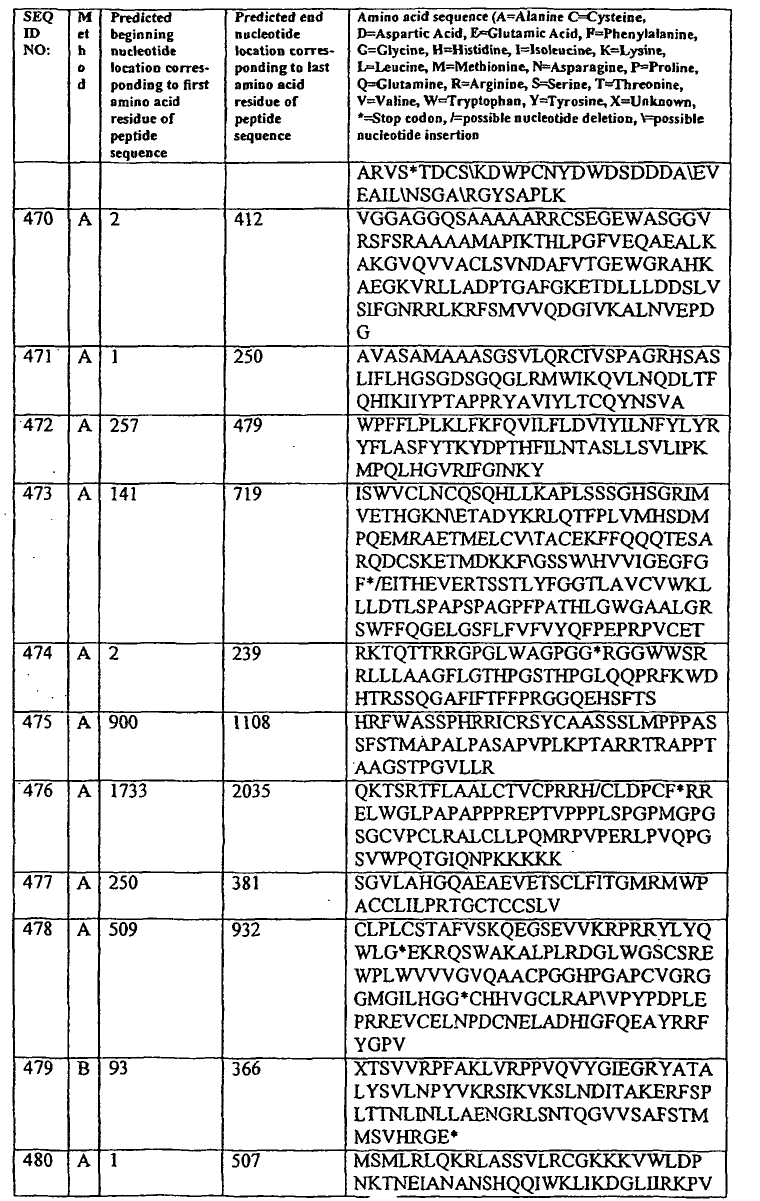

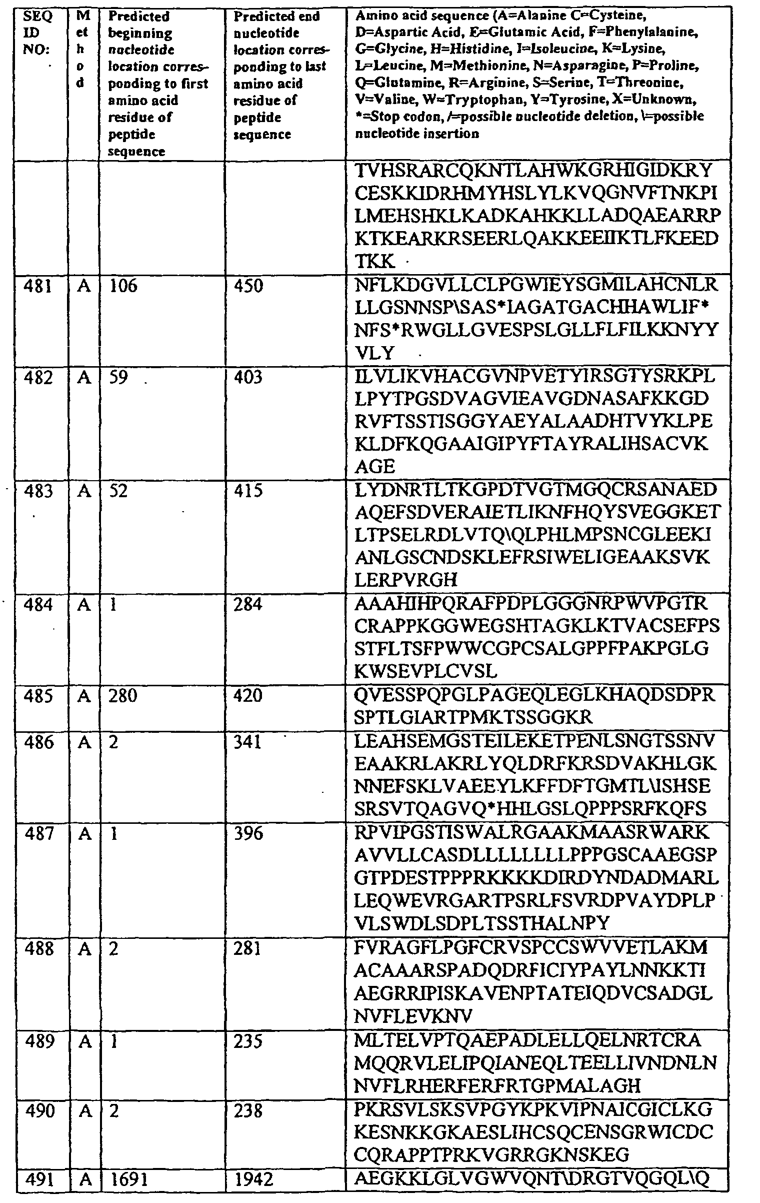

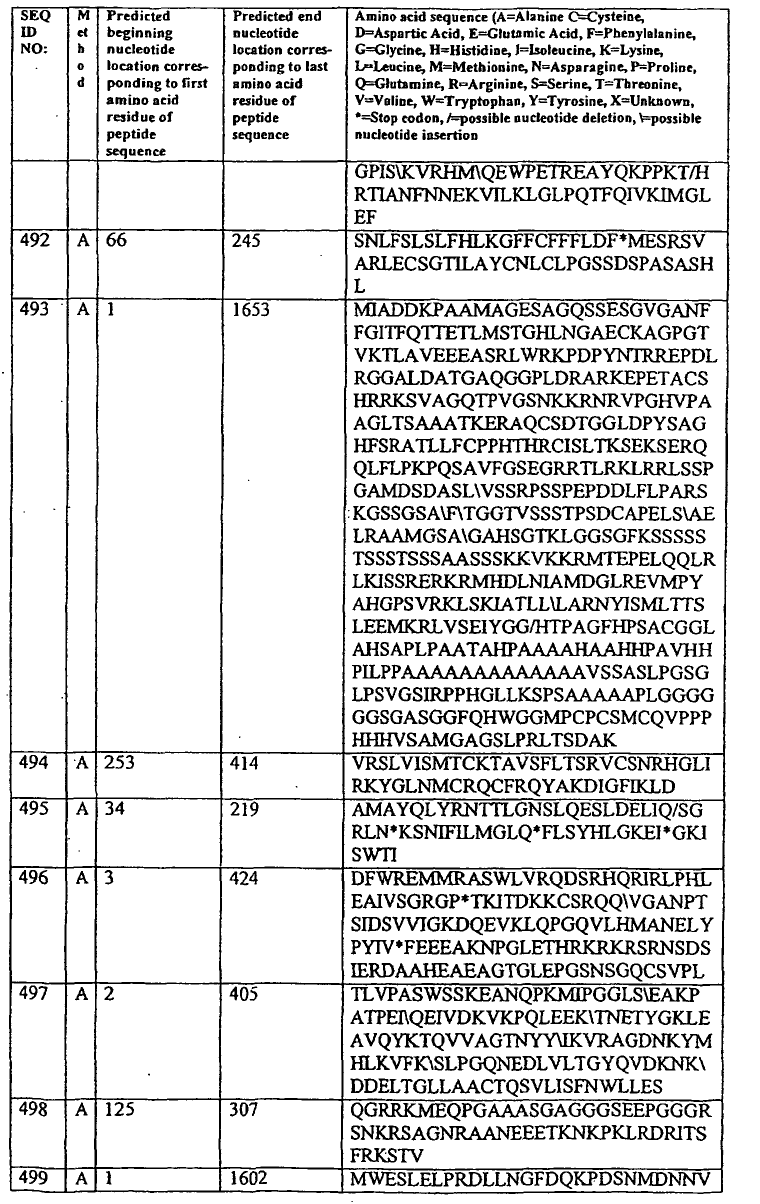

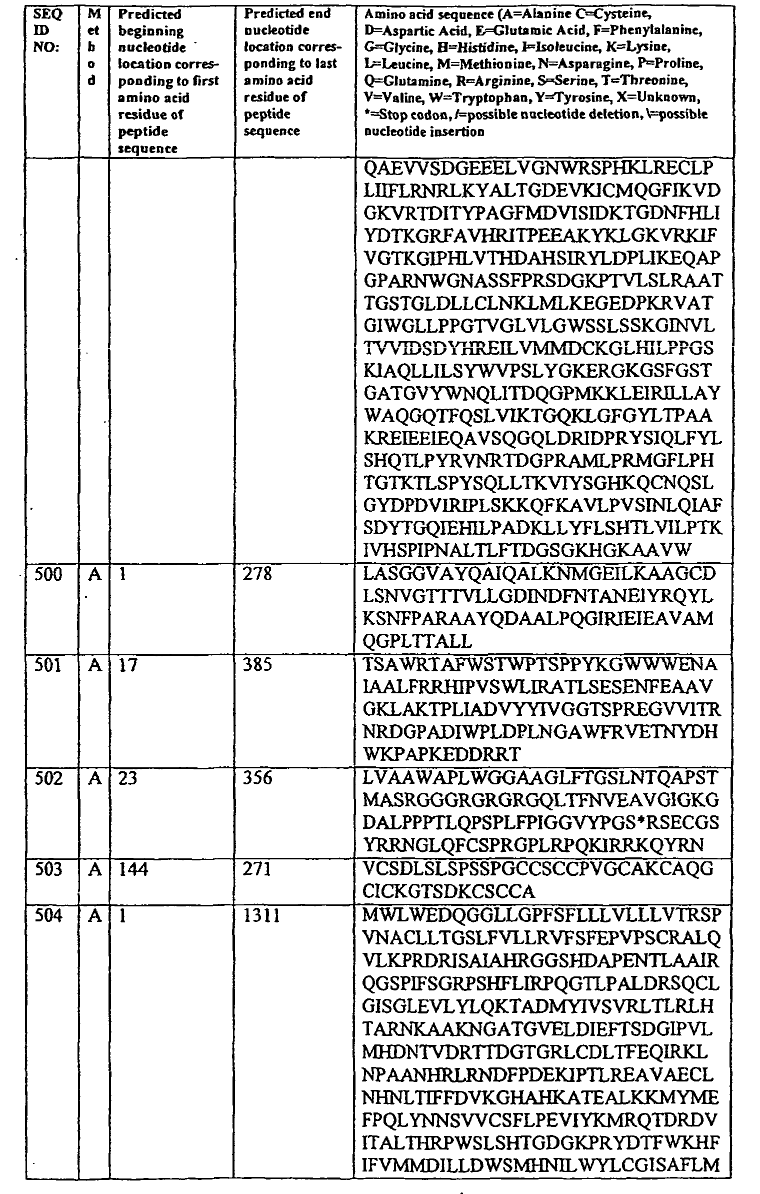

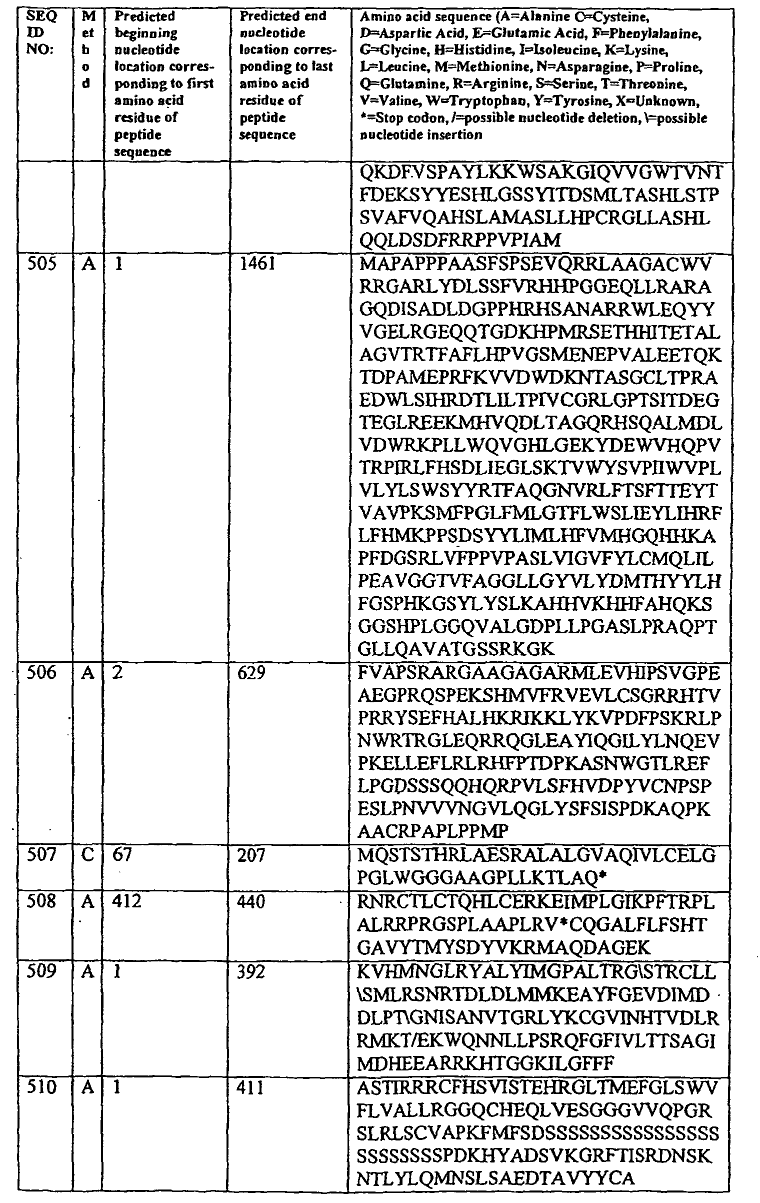

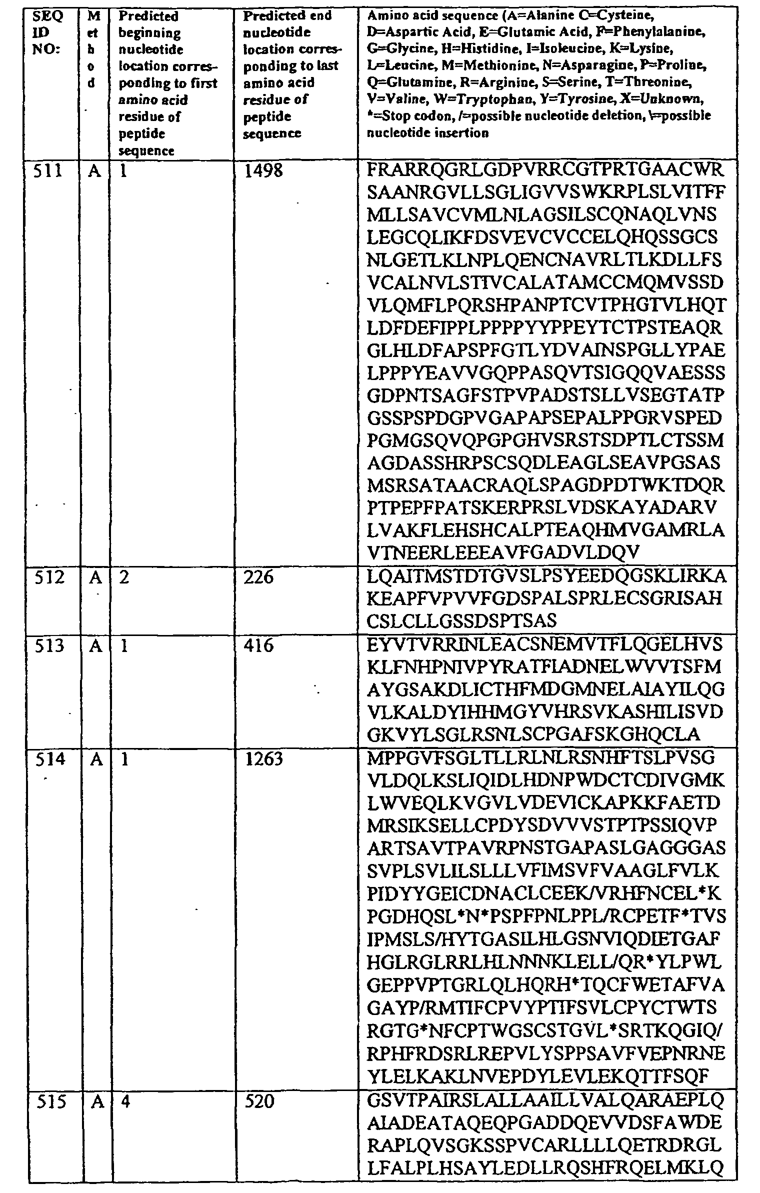

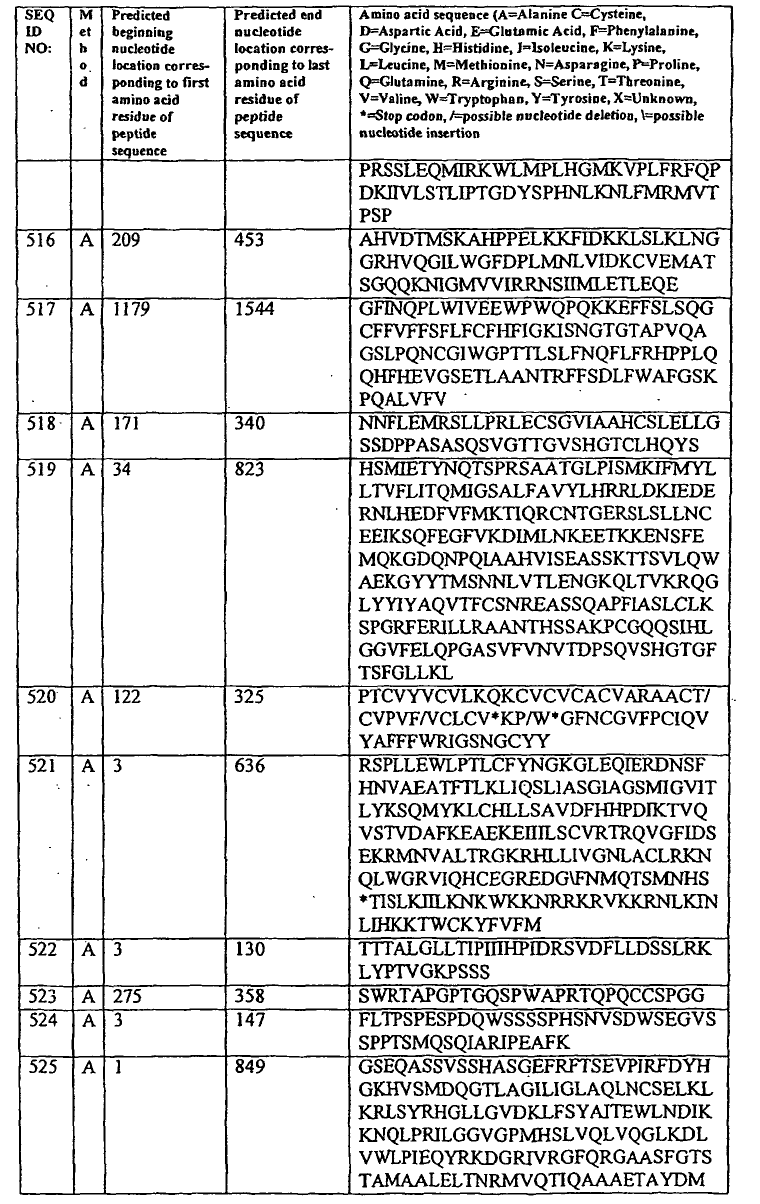

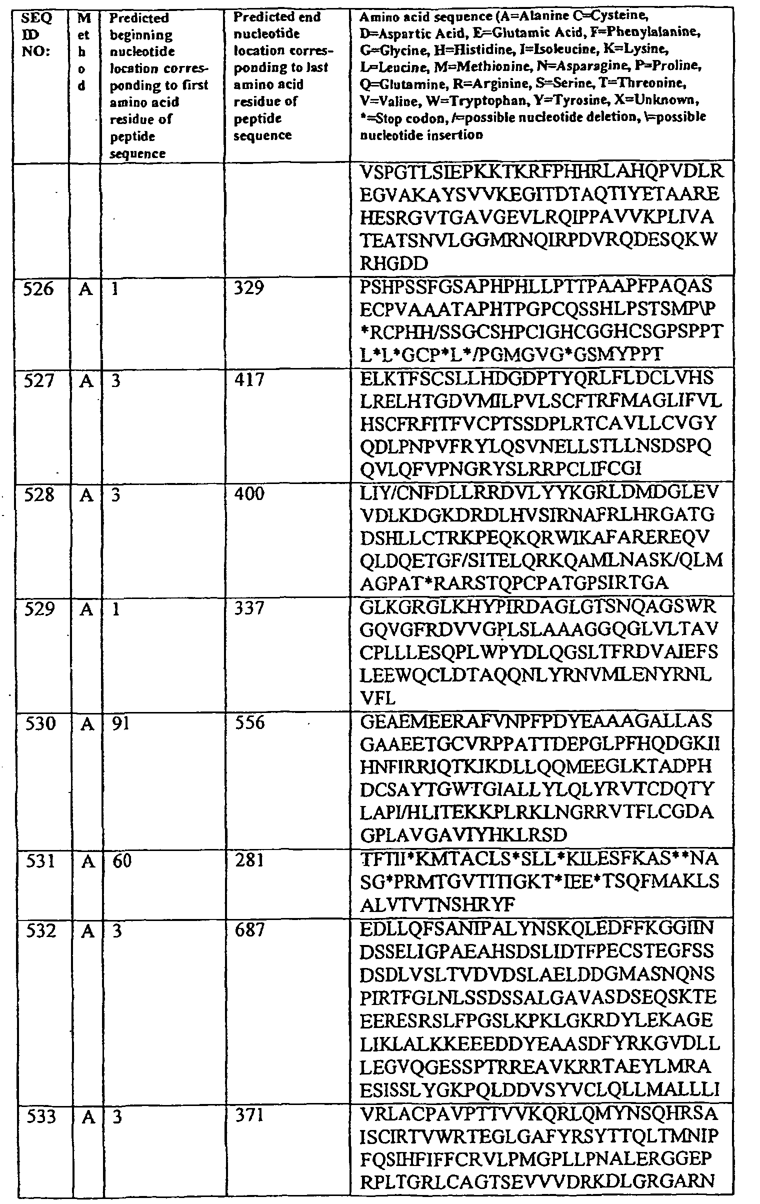

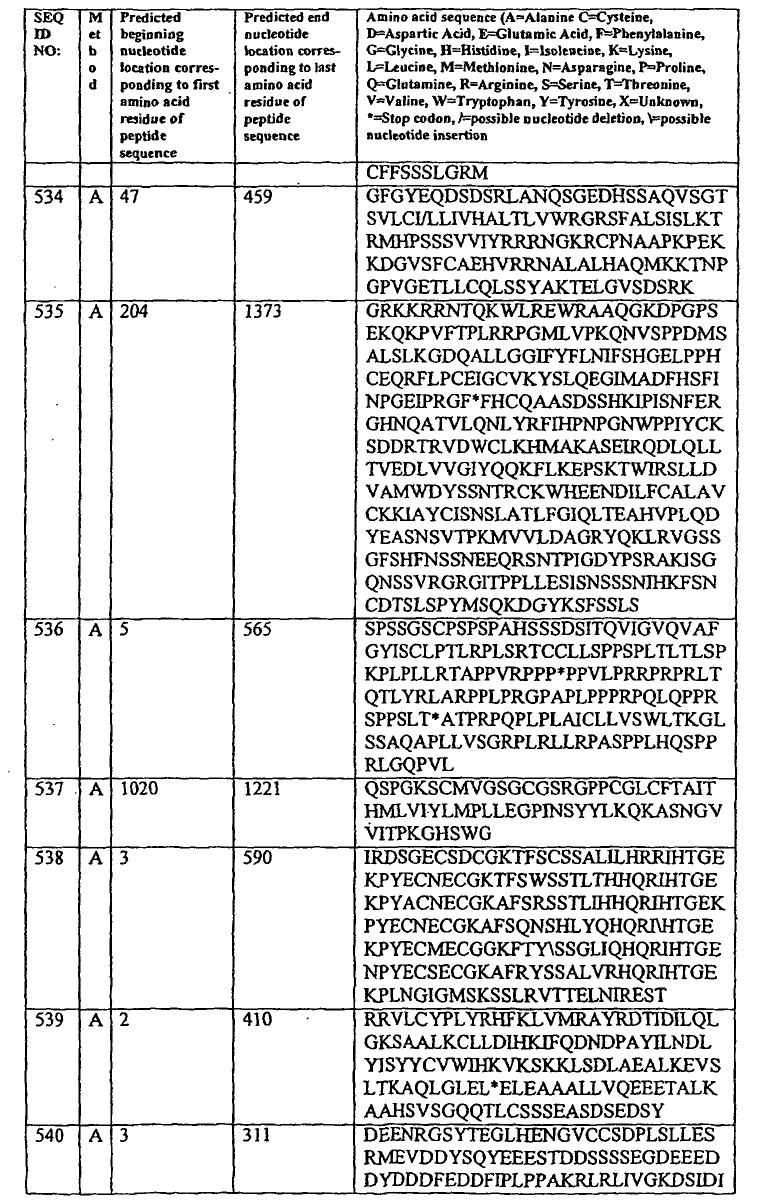

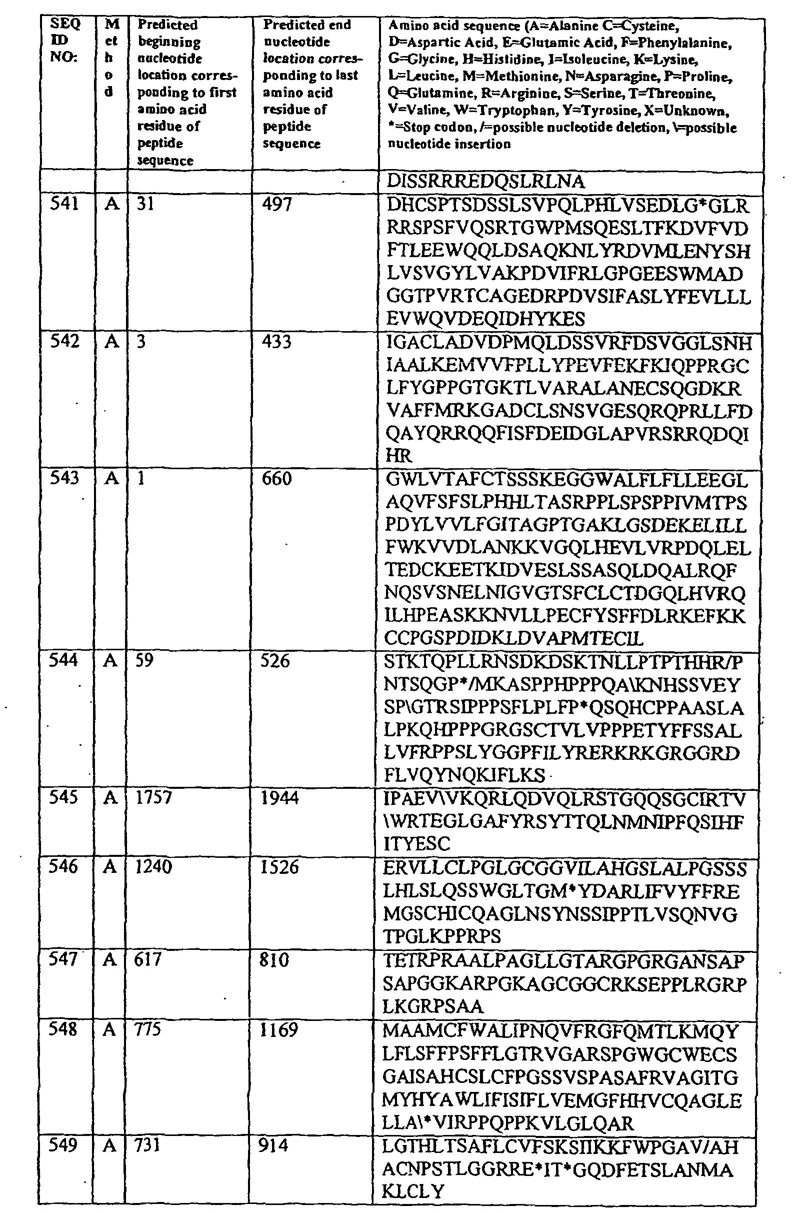

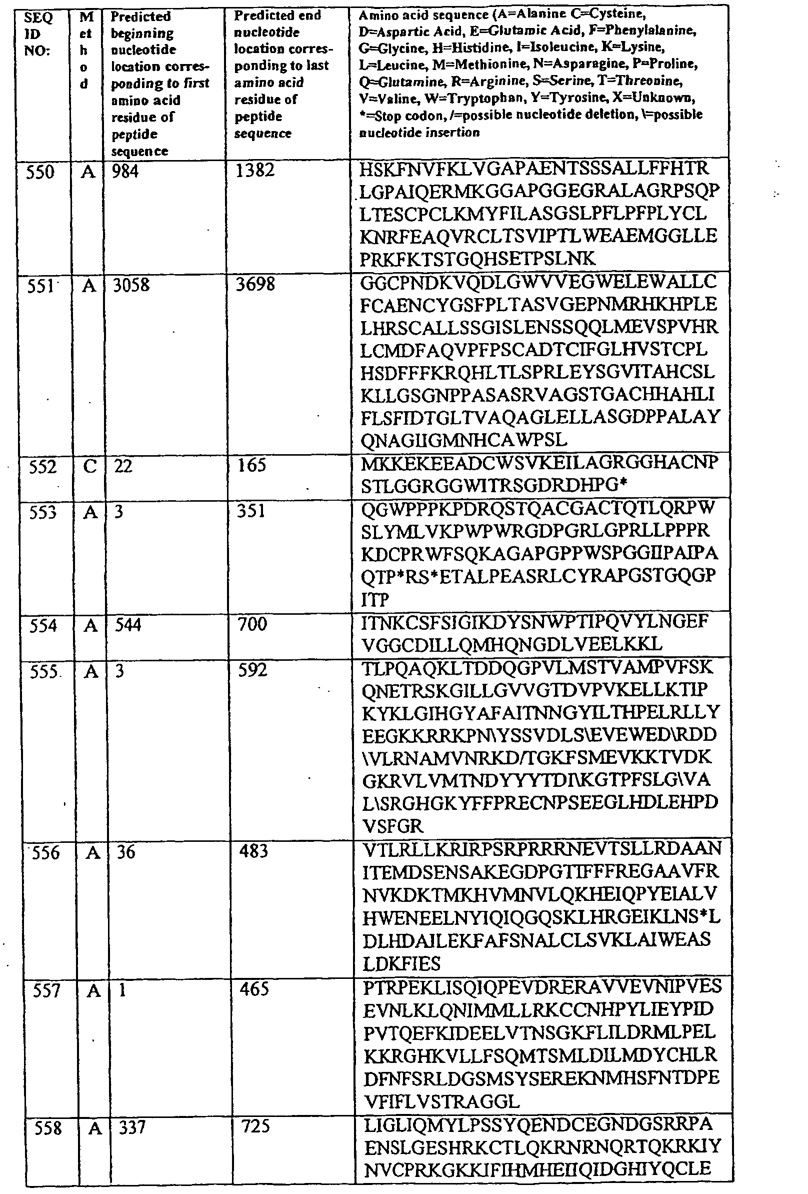

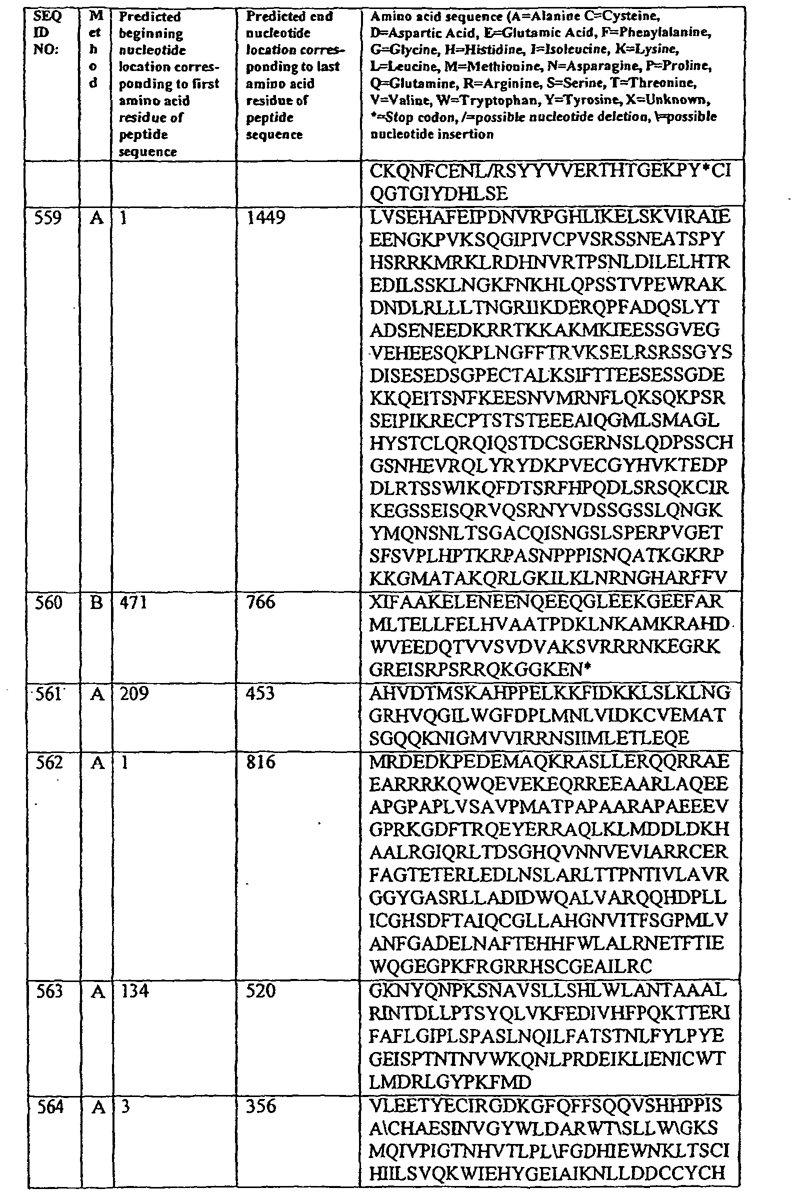

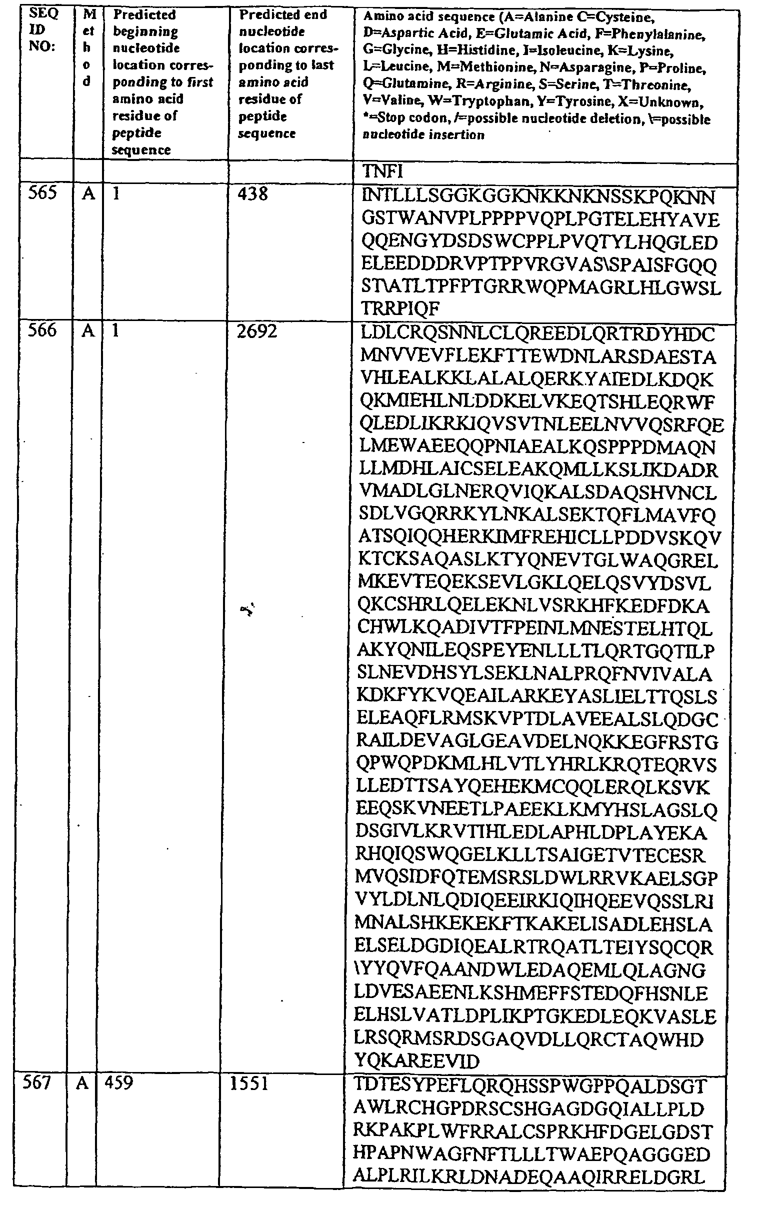

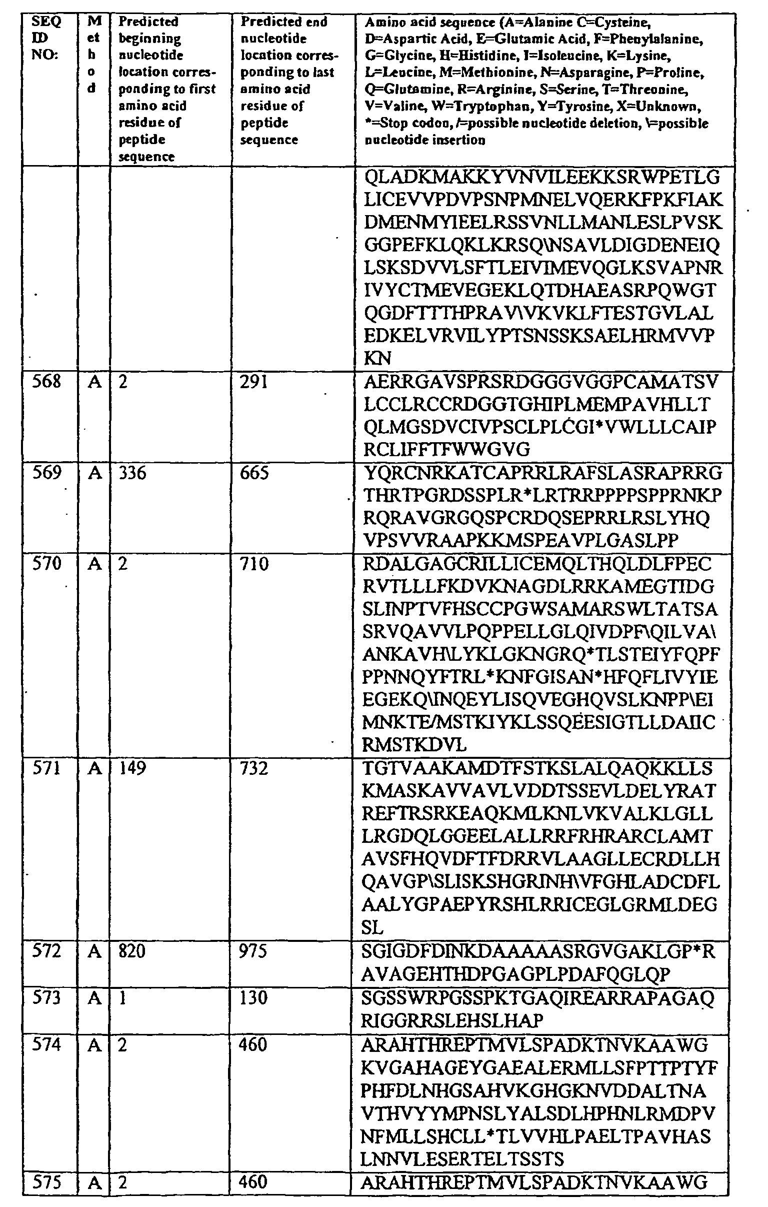

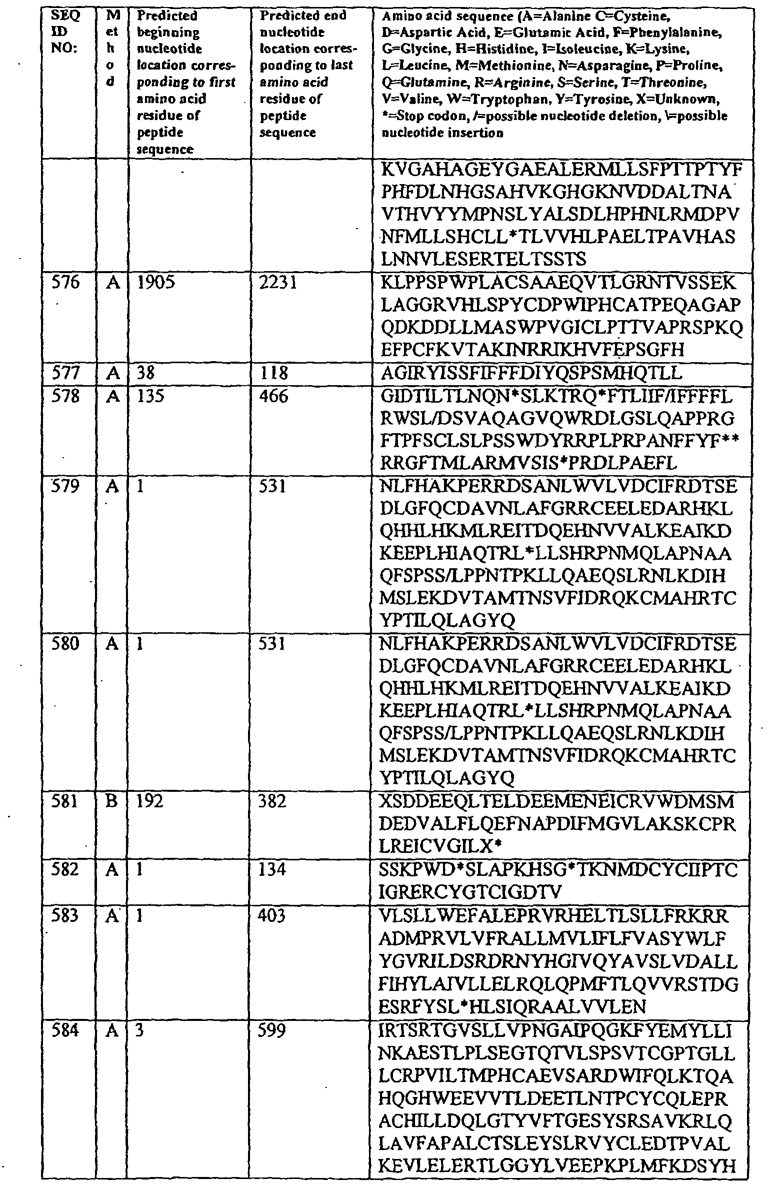

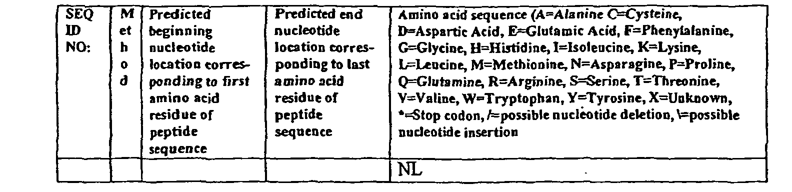

**Table 7**

| **SEQ ID NO:** | **Position of end of signal in amino acid sequence** | **MaxS (maximum score)** | **MeanS (mean score)** |
|---|---|---|---|
| 1 | 30 | 0.928 | 0.784 |
| 2 | 22 | 0.960 | 0.789 |
| 4 | 16 | 0.991 | 0.955 |
| 5 | 33 | 0.972 | 0.922 |
| 6 | 24 | 0.962 | 0.730 |
| 7 | 33 | 0.931 | 0.710 |
| 18 | 31 | 0.987 | 0.804 |
| 24 | 22 | 0.960 | 0.789 |
| 25 | 30 | 0.986 | 0.858 |
| 39 | 16 | 0.991 | 0.955 |
| 40 | 23 | 0.989 | 0.917 |
| 49 | 33 | 0.972 | 0.922 |
| 63 | 28 | 0.962 | 0.806 |
| 66 | 31 | 0.987 | 0.852 |
| 72 | 19 | 0.956 | 0.866 |
| 73 | 18 | 0.966 | 0.843 |
| 110 | 19 | 0.969 | 0.933 |
| 125 | 20 | 0.995 | 0.971 |
| 127 | 21 | 0.984 | 0.854 |
| 129 | 17 | 0.992 | 0.957 |
| 135 | 44 | 0.916 | 0.654 |
| 140 | 17 | 0.992 | 0.957 |
| 142 | 16 | 0.907 | 0.567 |
| 144 | 49 | 0.973 | 0.705 |
| 146 | 26 | 0.962 | 0.874 |

## Claims

1. An isolated polynucleotide comprising a nucleotide sequence selected from the group consisting of SEQ ID NO: 1-146, or 293-438, a mature protein coding portion of SEQ ID NO: 1-146, or 293-438, an active domain coding portion of SEQ ID NO: 1-146, or 293-438, and complementary sequences thereof.

2. An isolated polynucleotide encoding a polypeptide with biological activity, wherein said polynucleotide hybridizes to the polynucleotide of claim 1 under stringent hybridization conditions.

3. An isolated polynucleotide encoding a polypeptide with biological activity, wherein said polynucleotide has greater than about 90% sequence identity with the polynucleotide of claim 1.

4. The polynucleotide of claim 1 wherein said polynucleotide is DNA.

5. An isolated polynucleotide of claim 1 wherein said polynucleotide comprises the complementary sequences.

6. A vector comprising the polynucleotide of claim 1.

7. An expression vector comprising the polynucleotide of claim 1.

8. A host cell genetically engineered to comprise the polynucleotide of claim 1.

9. A host cell genetically engineered to comprise the polynucleotide of claim 1 operatively associated with a regulatory sequence that modulates expression of the polynucleotide in the host cell.

10. An isolated polypeptide, wherein the polypeptide is selected from the group consisting of:
(a) a polypeptide encoded by any one of the polynucleotides of claim 1; and
(b) a polypeptide encoded by a polynucleotide hybridizing under stringent conditions with any one of SEQ ID NO: 1-146, or 293-438.

11. A composition comprising the polypeptide of claim 10 and a carrier.

12. An antibody directed against the polypeptide of claim 10.

13. A method for detecting the polynucleotide of claim 1 in a sample, comprising:
a) contacting the sample with a compound that binds to and forms a complex with the polynucleotide of claim 1 for a period sufficient to form the complex; and
b) detecting the complex, so that if a complex is detected, the polynucleotide of claim 1 is detected.

14. A method for detecting the polynucleotide of claim 1 in a sample, comprising:
a) contacting the sample under stringent hybridization conditions with nucleic acid primers that anneal to the polynucleotide of claim 1 under such conditions;
b) amplifying a product comprising at least a portion of the polynucleotide of claim 1; and
c) detecting said product and thereby the polynucleotide of claim 1 in the sample.

15. The method of claim 14, wherein the polynucleotide is an RNA molecule and the method further comprises reverse transcribing an annealed RNA molecule into a cDNA polynucleotide.

16. A method for detecting the polypeptide of claim 10 in a sample, comprising:
a) contacting the sample with a compound that binds to and forms a complex with the polypeptide under conditions and for a period sufficient to form the complex; and
b) detecting formation of the complex, so that if a complex formation is detected, the polypeptide of claim 10 is detected.

17. A method for identifying a compound that binds to the polypeptide of claim 10, comprising:
a) contacting the compound with the polypeptide of claim 10 under conditions sufficient to form a polypeptide/compound complex; and
b) detecting the complex, so that if the polypeptide/compound complex is detected, a compound that binds to the polypeptide of claim 10 is identified.

18. A method for identifying a compound that binds to the polypeptide of claim 10, comprising:
a) contacting the compound with the polypeptide of claim 10, in a cell, under conditions sufficient to form a polypeptide/compound complex, wherein the complex drives expression of a reporter gene sequence in the cell; and
b) detecting the complex by detecting reporter gene sequence expression, so that if the polypeptide/compound complex is detected, a compound that binds to the polypeptide of claim 10 is identified.

19. A method of producing the polypeptide of claim 10, comprising,
a) culturing a host cell comprising a polynucleotide sequence selected from SEQ ID NO: 1-146, or 293-438, a mature protein coding portion of SEQ ID NO: 1-146, or 293-438, an active domain coding portion of SEQ ID NO: 1-146, or 293-438, complementary sequences thereof and a polynucleotide sequence hybridizing under stringent conditions to SEQ ID NO: 1-146, or 293-438, under conditions sufficient to express the polypeptide in said cell; and
b) isolating the polypeptide from the cell culture or cells of step (a).

20. An isolated polypeptide comprising an amino acid sequence selected from the group consisting of any one of the polypeptides SEQ ID NO: 147-292, or 439-584, the mature protein portion thereof, or the active domain thereof.

21. The polypeptide of claim 20 wherein the polypeptide is provided on a polypeptide array.

22. A collection of polynucleotides, wherein the collection comprising the sequence information of at least one of SEQ ID NO: 1-146, or 293-438.

23. The collection of claim 22, wherein the collection is provided on a nucleic acid array.

24. The collection of claim 23, wherein the array detects full-matches to any one of the polynucleotides in the collection.

25. The collection of claim 23, wherein the array detects mismatches to any one of the polynucleotides in the collection.

26. The collection of claim 22, wherein the collection is provided in a computer-readable format.

27. A method of treatment comprising administering to a mammalian subject in need thereof a therapeutic amount of a composition comprising a polypeptide of claim 10 or 20 and a pharmaceutically acceptable carrier.

28. A method of treatment comprising administering to a mammalian subject in need thereof a therapeutic amount of a composition comprising an antibody that specifically binds to a polypeptide of claim 10 or 20 and a pharmaceutically acceptable carrier.
